(19) Europäisches Patentamt
European Patent Office
Office européen des brevets

(11) EP 2 508 610 A1

(12) **EUROPEAN PATENT APPLICATION**

(43) Date of publication:
**10.10.2012 Bulletin 2012/41**

(51) Int Cl.:
***C12N 15/82*** *(2006.01)* ***A01H 5/00*** *(2006.01)*

(21) Application number: **12168728.9**

(22) Date of filing: **30.06.2008**

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority:
**02.05.2008 US 57921 P**
**29.06.2007 EP 07111425**
**17.07.2007 EP 07112614**
**17.07.2007 EP 07112616**
**18.07.2007 EP 07112724**
**18.07.2007 EP 07112725**
**02.06.2008 EP 08157433**

(62) Document number(s) of the earlier application(s) in accordance with Art. 76 EPC:
**08774524.6 / 2 078 090**

(27) Previously filed application:
**30.06.2008 PCT/EP2008/058370**

(71) Applicant: **BASF Plant Science GmbH**
**67056 Ludwigshafen (DE)**

(72) Inventors:
- **Frankard, Valerie**
  **1410 Waterloo (BE)**
- **Reuzeau, Christophe**
  **24350 Tocan Saint Apre (FR)**
- **Sanz Molinero, Ana Isabel**
  **9050 Gentbrugge (BE)**

(74) Representative: **Fitzner, Uwe**
**Dres. Fitzner & Münch**
**Rechts- und Patentanwälte**
**Hauser Ring 10**
**40878 Ratingen (DE)**

Remarks:
This application was filed on 21-05-2012 as a divisional application to the application mentioned under INID code 62.

# (54) Plants having enhanced yield-related traits and a method for making the same

(57) The present invention relates generally to the field of molecular biology and concerns a method for enhancing various economically important yield-related traits in plants. More specifically, the present invention concerns a method for enhancing yield-related traits in plants by modulating expression in a plant of a nucleic acid encoding a yield increasing polypeptide. The present invention also concerns plants having modulated expression of a nucleic acid encoding a yield increasing polypeptide, which plants have enhanced yield-related traits relative to control plants.

MTDYRLQPTMNLWTTDDNASMMEAFMSSSDISTLWPPASTT

TTTATTETTPTPAMEIPAQAGFNQETLQQRLQALIEGTHEG
4
WTYAIFWQPSYDFSGASVLGWGDGYYKGEEDKANPRRRSSS
1
PPFSTPADQEYRKKVLRELNSLISGGVAPSDDAVDEEVTDT

EWFFLVSMTQSFACGAGLAGKAFATGNAVWVSGSDQLSGSG

CERAKQGGVFGMHTIACIPSANGVVEVGSTEPIRQSSDLIN
2
KVRILFNFDGGAGDLSGLNWNLDPDQGENDPSMWINDPIGT

PGSNEPGNGAPSSSSQLFSKSIQFENGSSSTITENPNLDPT

PSPVHSQTQNPKFNNTFSRELNFSTSSSTLVKPRSGEILNF

GDEGKRSSGNPDPSSYSGQTQFENKRKRSMVLNEDKVLSFG

DKTAGESDHSDLEASVVKEVAVEKRPKKRGRKPANGREEPL

NH**VEAERQRREKLNQRFYALRAVVPNVSKMDKASLLGDAIA**
3
**YINELKSKVVK**TESEKLQIKNQLEEVKLELAGRRASASGGD
3
MSSSCSSIKPVGMEIEVKIIGWDAMIRVESSKRNHPAARLM
5
SALMDLELEVNHASMSVVNDLMIQQATVKMGFRIYTQEQLR
6
ASLISKIG

**FIGURE 1**

EP 2 508 610 A1

SEQUENCE LISTING

<110> BASF Plant Science GmbH

<120> Plants having enhanced yield-related traits and a method for making the same

<130> PF59348

<160> 320

<170> PatentIn version 3.3

<210> 1
<211> 1872
<212> DNA
<213> Arabidopsis thaliana

<400> 1
```
atgactgatt accggctaca accaacgatg aatctttgga ccaccgacga caacgcttct      60
atgatggaag ctttcatgag ctcttccgat atctcaactt tatggcctcc ggcgtcgacg     120
acaaccacga cggcgacgac tgaaacaact ccgacgccgg cgatggagat tccggcacag     180
gcgggattta atcaagagac tcttcagcaa cgtttacaag ctttgattga aggaacacac     240
gaaggttgga cctacgctat attctggcaa ccgtcgtatg atttctccgg cgcctccgtg     300
ctcggatggg gagatggtta ttacaaaggt gaagaagata aagcaaaccc gagacggaga     360
tcgagttcgc cgccgttttc tactccggcg gatcaggagt acaggaaaaa agtgttgaga     420
gagcttaact cgttgatctc cggtggtgtt gctccgtcgg atgacgctgt tgatgaggag     480
gtgacggata cggaatggtt tttcttggtt tcgatgacgc agagcttcgc ttgcggtgcg     540
ggattagctg gtaaagcgtt tgcaacgggt aacgcggttt gggtttccgg gtcagatcaa     600
ttatccgggt cgggttgtga acgggctaag caaggaggag tgtttgggat gcatactatt     660
gcgtgtattc cttcggcgaa cggagttgtg gaagtcgggt caacggagcc gatccgacag     720
agttcggacc ttattaacaa ggttcgaatt cttttcaatt tcgacggcgg agctggagat     780
ttatcgggtc ttaattggaa tcttgacccg gatcaaggtg agaacgaccc gtctatgtgg     840
attaatgacc cgattggaac acctggatct aacgaaccgg gtaacggagc tccaagttct     900
agctcccagc ttttttcaaa gtctattcag tttgagaacg gtagctcaag cacaataacc     960
gaaaacccga atctggatcc gactccgagt ccggttcatt ctcagaccca gaatccgaaa    1020
ttcaataaca ctttctcccg agaacttaat ttttcgacgt caagttctac tttagtgaaa    1080
ccaagatccg gcgagatatt aaacttcggc gatgaaggta aacgaagctc cggaaacccg    1140
gatccaagtt cttattcggg tcaaacacaa ttcgaaaaca aaagaaagag gtcgatggtt    1200
ttgaacgaag ataaagttct atcattcgga gataaaaccg ccggagaatc agatcactcc    1260
gatctagaag cttccgtcgt gaaagaagta gcagtagaga aacgtccaaa gaaacgagga    1320
agaaagccag caaacggtag agaagagcca ctaaaccacg tcgaagcaga gagacaaaga    1380
cgcgagaaac taaaccaaag attctacgcg ttacgagcgg ttgtaccaaa cgtttcaaaa    1440
atggataaag cttcgttact cggtgacgca atcgcttaca tcaacgagct taaatccaaa    1500
```

```
gtagtcaaaa cagagtcaga gaaactccaa atcaagaacc agctcgagga agtgaaactc   1560
gagctcgccg gaagaaaagc gagtgctagt ggaggagata tgtcgtcttc gtgttcttcg   1620
attaaaccgg tggggatgga gattgaagtg aagataattg gttgggacgc aatgattaga   1680
gttgaatcta gtaagaggaa tcatccggcg gcgaggttga tgtcggcgtt gatggatttg   1740
gagttggaag tgaatcacgc gagtatgtcg gtggttaacg atttgatgat tcaacaagcg   1800
acggtgaaga tgggttttag gatctatacg caagaacagc tcagagcaag tttgatttca   1860
aaaatcggtt aa                                                        1872
```

```
<210>  2
<211>  623
<212>  PRT
<213>  Arabidopsis thaliana

<400>  2

Met Thr Asp Tyr Arg Leu Gln Pro Thr Met Asn Leu Trp Thr Thr Asp
1               5                   10                  15

Asp Asn Ala Ser Met Met Glu Ala Phe Met Ser Ser Ser Asp Ile Ser
            20                  25                  30

Thr Leu Trp Pro Pro Ala Ser Thr Thr Thr Thr Thr Ala Thr Thr Glu
        35                  40                  45

Thr Thr Pro Thr Pro Ala Met Glu Ile Pro Ala Gln Ala Gly Phe Asn
    50                  55                  60

Gln Glu Thr Leu Gln Gln Arg Leu Gln Ala Leu Ile Glu Gly Thr His
65                  70                  75                  80

Glu Gly Trp Thr Tyr Ala Ile Phe Trp Gln Pro Ser Tyr Asp Phe Ser
                85                  90                  95

Gly Ala Ser Val Leu Gly Trp Gly Asp Gly Tyr Tyr Lys Gly Glu Glu
            100                 105                 110

Asp Lys Ala Asn Pro Arg Arg Arg Ser Ser Ser Pro Pro Phe Ser Thr
        115                 120                 125

Pro Ala Asp Gln Glu Tyr Arg Lys Lys Val Leu Arg Glu Leu Asn Ser
    130                 135                 140

Leu Ile Ser Gly Gly Val Ala Pro Ser Asp Asp Ala Val Asp Glu Glu
145                 150                 155                 160

Val Thr Asp Thr Glu Trp Phe Phe Leu Val Ser Met Thr Gln Ser Phe
                165                 170                 175

Ala Cys Gly Ala Gly Leu Ala Gly Lys Ala Phe Ala Thr Gly Asn Ala
            180                 185                 190
```

Val Trp Val Ser Gly Ser Asp Gln Leu Ser Gly Ser Gly Cys Glu Arg
195 200 205

Ala Lys Gln Gly Gly Val Phe Gly Met His Thr Ile Ala Cys Ile Pro
210 215 220

Ser Ala Asn Gly Val Val Glu Val Gly Ser Thr Glu Pro Ile Arg Gln
225 230 235 240

Ser Ser Asp Leu Ile Asn Lys Val Arg Ile Leu Phe Asn Phe Asp Gly
245 250 255

Gly Ala Gly Asp Leu Ser Gly Leu Asn Trp Asn Leu Asp Pro Asp Gln
260 265 270

Gly Glu Asn Asp Pro Ser Met Trp Ile Asn Asp Pro Ile Gly Thr Pro
275 280 285

Gly Ser Asn Glu Pro Gly Asn Gly Ala Pro Ser Ser Ser Ser Gln Leu
290 295 300

Phe Ser Lys Ser Ile Gln Phe Glu Asn Gly Ser Ser Ser Thr Ile Thr
305 310 315 320

Glu Asn Pro Asn Leu Asp Pro Thr Pro Ser Pro Val His Ser Gln Thr
325 330 335

Gln Asn Pro Lys Phe Asn Asn Thr Phe Ser Arg Glu Leu Asn Phe Ser
340 345 350

Thr Ser Ser Ser Thr Leu Val Lys Pro Arg Ser Gly Glu Ile Leu Asn
355 360 365

Phe Gly Asp Glu Gly Lys Arg Ser Ser Gly Asn Pro Asp Pro Ser Ser
370 375 380

Tyr Ser Gly Gln Thr Gln Phe Glu Asn Lys Arg Lys Arg Ser Met Val
385 390 395 400

Leu Asn Glu Asp Lys Val Leu Ser Phe Gly Asp Lys Thr Ala Gly Glu
405 410 415

Ser Asp His Ser Asp Leu Glu Ala Ser Val Val Lys Glu Val Ala Val
420 425 430

Glu Lys Arg Pro Lys Lys Arg Gly Arg Lys Pro Ala Asn Gly Arg Glu
435 440 445

Glu Pro Leu Asn His Val Glu Ala Glu Arg Gln Arg Arg Glu Lys Leu
450 455 460

Asn Gln Arg Phe Tyr Ala Leu Arg Ala Val Val Pro Asn Val Ser Lys
465 470 475 480

Met Asp Lys Ala Ser Leu Leu Gly Asp Ala Ile Ala Tyr Ile Asn Glu
485 490 495

Leu Lys Ser Lys Val Val Lys Thr Glu Ser Glu Lys Leu Gln Ile Lys
500 505 510

Asn Gln Leu Glu Glu Val Lys Leu Glu Leu Ala Gly Arg Lys Ala Ser
515 520 525

Ala Ser Gly Gly Asp Met Ser Ser Ser Cys Ser Ser Ile Lys Pro Val
530 535 540

Gly Met Glu Ile Glu Val Lys Ile Ile Gly Trp Asp Ala Met Ile Arg
545 550 555 560

Val Glu Ser Ser Lys Arg Asn His Pro Ala Ala Arg Leu Met Ser Ala
565 570 575

Leu Met Asp Leu Glu Leu Glu Val Asn His Ala Ser Met Ser Val Val
580 585 590

Asn Asp Leu Met Ile Gln Gln Ala Thr Val Lys Met Gly Phe Arg Ile
595 600 605

Tyr Thr Gln Glu Gln Leu Arg Ala Ser Leu Ile Ser Lys Ile Gly
610 615 620

<210> 3
<211> 11
<212> PRT
<213> Artificial sequence

<220>
<223> Motif 1

<220>
<221> VARIANT
<222> (2)..(2)
<223> /replace = "Glu" /replace = "Thr"

<220>
<221> UNSURE
<222> (4)..(4)
<223> Unknown amino acid

<220>
<221> VARIANT
<222> (5)..(5)
<223> /replace = "Glu"

<220>
<221> VARIANT
<222> (6)..(6)
<223> /replace = "Ser"

<220>
<221> UNSURE
<222> (7)..(7)
<223> Unknown amino acid

<220>
<221> VARIANT
<222> (8)..(8)
<223> /replace = "Phe"

<220>
<221> VARIANT
<222> (9)..(9)
<223> /replace = "Asn"

<220>
<221> VARIANT
<222> (11)..(11)
<223> /replace = "Asp"

<400> 3

```
Leu Gly Trp Xaa Asp Gly Xaa Tyr Lys Gly Glu
1               5                   10
```

<210> 4
<211> 9
<212> PRT
<213> Artificial sequence

<220>
<223> Motif 2

<220>
<221> VARIANT
<222> (2)..(2)
<223> /replace = "Ile"

<220>
<221> VARIANT
<222> (3)..(3)
<223> /replace = "Ile" /replace = "Leu"

<220>
<221> VARIANT
<222> (5)..(5)
<223> /replace = "Leu" /replace = "Ile"

<220>
<221> VARIANT
<222> (6)..(6)
<223> /replace = "Ala"

<220>
<221> VARIANT
<222> (7)..(7)
<223> /replace = "Val" /replace = "Thr" /replace = "Ala"

<220>
<221> VARIANT
<222> (8)..(8)
<223> /replace = "Leu" /replace = "Ser"

<220>
<221> VARIANT
<222> (9)..(9)
<223> /replace = "Asp" /replace = "Ser"

<400> 4

Gly Val Val Glu Val Gly Ser Thr Glu
1 5

<210> 5
<211> 7
<212> PRT
<213> Artificial sequence

<220>
<223> Motif 3

<220>

<221> VARIANT
<222> (1)..(1)
<223> /replace = "Glu"

<220>
<221> VARIANT
<222> (3)..(3)
<223> /replace = "Val" /replace = "Ile"

<220>
<221> VARIANT
<222> (5)..(5)
<223> /replace = "Ile" /replace = "Val"

<220>
<221> VARIANT
<222> (7)..(7)
<223> /replace = "Asp" /replace = "Glu" /replace = "Ala"

<400> 5

Asp Lys Ala Ser Leu Leu Gly
1 5

<210> 6
<211> 7
<212> PRT
<213> Artificial sequence

<220>
<223> Motif 4

<220>
<221> VARIANT
<222> (1)..(1)
<223> /replace = "Asn" /replace = "Ser"

<220>
<221> VARIANT
<222> (4)..(4)
<223> /replace = "His"

<400> 6

Thr Leu Gln Gln Arg Leu Gln
1 5

<210> 7

<211> 12
<212> PRT
<213> Artificial sequence

<220>
<223> Motif 5

<220>
<221> VARIANT
<222> (1)..(1)
<223> /replace = "Phe"

<220>
<221> VARIANT
<222> (2)..(2)
<223> /replace = "Phe" /replace = "Val"

<220>
<221> VARIANT
<222> (3)..(3)
<223> /replace = "Leu" /replace = "Val" /replace = "Met" /replace = "Ser"

<220>
<221> VARIANT
<222> (5)..(5)
<223> /replace = "Leu" /replace = "Arg" /replace = "Asn" /replace = "Glu"

<220>
<221> VARIANT
<222> (6)..(6)
<223> /replace = "Asp"

<220>
<221> VARIANT
<222> (9)..(9)
<223> /replace = "Val"

<220>
<221> VARIANT
<222> (10)..(10)
<223> /replace = "Arg"

<220>
<221> VARIANT
<222> (11)..(11)
<223> /replace = "Ile"

<220>
<221> VARIANT
<222> (12)..(12)
<223> /replace = "Asn" /replace = "Glu" /replace = "Tyr"

<400> 7

```
Lys Ile Ile Gly Trp Glu Ala Met Ile Gly Val Gln
1               5                   10
```

<210> 8
<211> 14
<212> PRT
<213> Artificial sequence

<220>
<223> Motif 6

<220>
<221> VARIANT
<222> (1)..(1)
<223> /replace = "Tyr"

<220>
<221> VARIANT
<222> (2)..(2)
<223> /replace = "Ser"

<220>
<221> VARIANT
<222> (3)..(3)
<223> /replace = "Asn"

<220>
<221> VARIANT
<222> (4)..(4)
<223> /replace = "Cys" /replace = "Met" /replace = "Leu" /replace = "Thr"

<220>
<221> VARIANT
<222> (5)..(5)
<223> /replace = "Gln"

<220>
<221> VARIANT
<222> (6)..(6)
<223> /replace = "Cys" /replace = "Ser"

<220>
<221> VARIANT
<222> (7)..(7)
<223> /replace = "Met" /replace = "Phe"

<220>
<221> VARIANT
<222> (8)..(8)
<223> /replace = "Asn" /replace = "Ser"

<220>
<221> VARIANT
<222> (9)..(9)
<223> /replace = "Cys" /replace = "Glu"

<220>
<221> VARIANT
<222> (10)..(10)
<223> /replace = "Gln" /replace = "Phe" /replace = "Met"

<220>
<221> VARIANT
<222> (11)..(11)
<223> /replace = "Arg" /replace = "Leu"

<220>
<221> VARIANT
<222> (12)..(12)
<223> /replace = "Phe" /replace = "Leu"

<220>
<221> VARIANT
<222> (13)..(13)
<223> /replace = "Aps" /replace = "His"

<220>
<221> VARIANT

<222> (14)..(14)
<223> /replace = "Asp" /replace = "Val"

<400> 8

His Ala Ser Val Ser Val Val Lys Asp Leu Met Ile Gln Gln
1 5 10

<210> 9
<211> 70
<212> PRT
<213> Artificial sequence

<220>
<223> Motif 7

<400> 9

Pro Lys Lys Arg Gly Arg Lys Pro Ala Asn Gly Arg Glu Glu Pro Leu
1 5 10 15

Asn His Val Glu Ala Glu Arg Gln Arg Arg Glu Lys Leu Asn Gln Arg
20 25 30

Phe Tyr Ala Leu Arg Ala Val Val Pro Asn Val Ser Lys Met Asp Lys
35 40 45

Ala Ser Leu Leu Gly Asp Ala Ile Ala Tyr Ile Asn Glu Leu Lys Ser
50 55 60

Lys Val Val Lys Thr Glu
65 70

<210> 10
<211> 56
<212> DNA
<213> Artificial sequence

<220>
<223> primer: prm06515

<400> 10
ggggacaagt ttgtacaaaa aagcaggctt aaacaatgac tgattaccgg ctacaa 56

<210> 11
<211> 50
<212> DNA
<213> Artificial sequence

<220>
<223> primer: prm06516

<400> 11
ggggaccact ttgtacaaga aagctgggta caccctttta accgattttt 50

<210> 12
<211> 2194
<212> DNA
<213> Oryza sativa

<400> 12

aatccgaaaa gtttctgcac cgttttcacc ccctaactaa caatataggg aacgtgtgct 60
aaatataaaa tgagacctta tatatgtagc gctgataact agaactatgc aagaaaaact 120
catccaccta ctttagtggc aatcgggcta aataaaaaag agtcgctaca ctagtttcgt 180
tttccttagt aattaagtgg gaaaatgaaa tcattattgc ttagaatata cgttcacatc 240
tctgtcatga agttaaatta ttcgaggtag ccataattgt catcaaactc ttcttgaata 300
aaaaaatctt tctagctgaa ctcaatgggt aaagagagag atttttttta aaaaaataga 360
atgaagatat tctgaacgta ttggcaaaga tttaaacata taattatata attttatagt 420
ttgtgcattc gtcatatcgc acatcattaa ggacatgtct tactccatcc caatttttat 480
ttagtaatta aagacaattg acttattttt attatttatc ttttttcgat tagatgcaag 540
gtacttacgc acacactttg tgctcatgtg catgtgtgag tgcacctcct caatacacgt 600
tcaactagca acacatctct aatatcactc gcctatttaa tacatttagg tagcaatatc 660
tgaattcaag cactccacca tcaccagacc acttttaata atatctaaaa tacaaaaaat 720
aattttacag aatagcatga aaagtatgaa acgaactatt taggtttttc acatacaaaa 780
aaaaaaagaa ttttgctcgt gcgcgagcgc caatctccca tattgggcac acaggcaaca 840
acagagtggc tgcccacaga acaacccaca aaaaacgatg atctaacgga ggacagcaag 900
tccgcaacaa ccttttaaca gcaggctttg cggccaggag agaggaggag aggcaaagaa 960
aaccaagcat cctccttctc ccatctataa attcctcccc ccttttcccc tctctatata 1020
ggaggcatcc aagccaagaa gagggagagc accaaggaca cgcgactagc agaagccgag 1080
cgaccgcctt ctcgatccat atcttccggt cgagttcttg gtcgatctct tccctcctcc 1140
acctcctcct cacagggtat gtgcctccct tcggttgttc ttggatttat tgttctaggt 1200
tgtgtagtac gggcgttgat gttaggaaag gggatctgta tctgtgatga ttcctgttct 1260
tggatttggg atagaggggt tcttgatgtt gcatgttatc ggttcggttt gattagtagt 1320
atggttttca atcgtctgga gagctctatg gaaatgaaat ggtttaggga tcggaatctt 1380
gcgattttgt gagtaccttt tgtttgaggt aaaatcagag caccggtgat tttgcttggt 1440
gtaataaagt acggttgttt ggtcctcgat tctggtagtg atgcttctcg atttgacgaa 1500
gctatccttt gtttattccc tattgaacaa aaataatcca actttgaaga cggtcccgtt 1560
gatgagattg aatgattgat tcttaagcct gtccaaaatt tcgcagctgg cttgtttaga 1620
tacagtagtc cccatcacga aattcatgga aacagttata atcctcagga acaggggatt 1680
ccctgttctt ccgatttgct ttagtcccag aatttttttt cccaaatatc ttaaaaagtc 1740
actttctggt tcagttcaat gaattgattg ctacaaataa tgcttttata gcgttatcct 1800
agctgtagtt cagttaatag gtaatacccc tatagtttag tcaggagaag aacttatccg 1860
atttctgatc tccattttta attatatgaa atgaactgta gcataagcag tattcatttg 1920
gattattttt tttattagct ctcacccctt cattattctg agctgaaagt ctggcatgaa 1980
ctgtcctcaa ttttgttttc aaattcacat cgattatcta tgcattatcc tcttgtatct 2040
acctgtagaa gtttcttttt ggttattcct tgactgcttg attacagaaa gaaatttatg 2100

aagctgtaat cgggatagtt atactgcttg ttcttatgat tcatttcctt tgtgcagttc 2160

ttggtgtagc ttgccacttt caccagcaaa gttc 2194

<210> 13
<211> 1929
<212> DNA
<213> Phaseolus vulgaris

<400> 13
atgacggagt accggtcgcc gcccacaatg aatctctgga ccgacgacaa cgcttccgtc 60

atggaggcct tcatgagctc ctccgatttc tcctcccttt ggctgccaac accgcaatcc 120

gctgcctcta ccaccactcc gggagccgac actgccagag ccctgccgcc gcctccgccg 180

tcgcagtccc agtccctttt taaccaggag accttacaac agcggctgca aacgctgatc 240

gaaggcgccg aagagagctg gacctacgcc attttctggc aatcttctta tgactactcc 300

tccagcactt cccttctcgg ttggggtgac ggatactaca agggagagga ggacaaagga 360

aaaggaaaag cccccaaaga gatgtcgtcg gcggagcagg accaccgtaa gaaggttctc 420

cgcgagctca attcgttgat ttccggccct ttccgttccg ctgatgacgt cgacgaagag 480

gtatccgaca ctgagtggtt ctttctagtg tccatgactc agtcctttct cagcggaagc 540

gggctcccgg gccaggcttt tcttaattcc agcccggttt gggtcgccgg ggctgaccgg 600

ttatccgatt cgacgtcgga gcgggctcgc caggggcagg tctttggggt tcagactctg 660

gtttgcatac cgtccgcaaa cggcgtcgtt gagctggctt ctacggaggt gattttccag 720

aactccgatc tcatgaagaa agtgcgggat ttgttcaact tcaacaaccc agacgcgggt 780

ttctggccgt tgaatcaggg cgagaacgac ccttcctcac tctggctcaa cccttcttcc 840

tcgattgaaa tcaaggacac ctctaacgcc gttgcactcg tttcagcgaa tgcatcactg 900

agcaaaacca tgccgttcga aacccctggt tccagcactc taacagaaac ccctagtgcc 960

gccgccgcag ctcacgtccc caatcccaag aaccagggtt tcttccccag agaattgaac 1020

ttctcgaact ccttaaaacc cgaatccggt gaaattctga gcttcggaga gagcaagaag 1080

agctcttaca acgggagtta cttccccggt gttgcggctg aggagaccaa caagaagaga 1140

aggtctcctg cttctcgcag cagcattgac gatggaatgc tgtccttcac ctccggcgtg 1200

attataccgg cttcgaatat aaaatctggt gccgttgctg gtggtggtgc cagcggcggt 1260

gattccgaga actcggatct ggaagcttcc gtggtgaaag aggcggacag cagggtggtg 1320

gagccggaga aacggccccg gaagaggggt cggaagccgg ggaacggcag agaggagccg 1380

ctgaatcacg tggaagcgga gaggcagagg agggaaaagc tgaatcaacg gttctacgcg 1440

cttcgtgcgg tggttcccaa cgtgtcgaag atggacaaag catcgctctt aggcgatgcc 1500

atatcctaca taaacgagct gaagtcgaag ctgagtgagc tggaatcaga gaaaggggaa 1560

ttggagaagc aattggagtt ggtgaagaag gagcttgagc tggcaactaa aagcccttct 1620

cctccacctg ggccacctcc atccaacaaa gaagcgaagg aaacgacgag caagctgatt 1680

gatttggagt tagaggtgaa gatcataggg tgggacgcca tgataaggat ccaatgcagc 1740

aagaaaaacc accctgcggc caggttgatg gctgcgttga aggagctgga ccttgacgtg 1800

aatcatgcca gcgtatccgt ggtgaatgat ttgatgatcc aacaagccac cgtgaatatg 1860

ggaaaccggt tttacactca ggaacagctc cggtcggcgc gctcctccaa aattggcaat 1920

gcactatag 1929

<210> 14
<211> 642
<212> PRT
<213> Phaseolus vulgaris

<400> 14

```
Met Thr Glu Tyr Arg Ser Pro Pro Thr Met Asn Leu Trp Thr Asp Asp
1               5                   10                  15

Asn Ala Ser Val Met Glu Ala Phe Met Ser Ser Ser Asp Phe Ser Ser
            20                  25                  30

Leu Trp Leu Pro Thr Pro Gln Ser Ala Ala Ser Thr Thr Thr Pro Gly
        35                  40                  45

Ala Asp Thr Ala Arg Ala Leu Pro Pro Pro Pro Pro Ser Gln Ser Gln
    50                  55                  60

Ser Leu Phe Asn Gln Glu Thr Leu Gln Gln Arg Leu Gln Thr Leu Ile
65                  70                  75                  80

Glu Gly Ala Glu Glu Ser Trp Thr Tyr Ala Ile Phe Trp Gln Ser Ser
                85                  90                  95

Tyr Asp Tyr Ser Ser Ser Thr Ser Leu Leu Gly Trp Gly Asp Gly Tyr
            100                 105                 110

Tyr Lys Gly Glu Glu Asp Lys Gly Lys Gly Lys Ala Pro Lys Glu Met
        115                 120                 125

Ser Ser Ala Glu Gln Asp His Arg Lys Lys Val Leu Arg Glu Leu Asn
    130                 135                 140

Ser Leu Ile Ser Gly Pro Phe Arg Ser Ala Asp Asp Val Asp Glu Glu
145                 150                 155                 160

Val Ser Asp Thr Glu Trp Phe Phe Leu Val Ser Met Thr Gln Ser Phe
                165                 170                 175

Leu Ser Gly Ser Gly Leu Pro Gly Gln Ala Phe Leu Asn Ser Ser Pro
            180                 185                 190

Val Trp Val Ala Gly Ala Asp Arg Leu Ser Asp Ser Thr Ser Glu Arg
        195                 200                 205
```

Ala Arg Gln Gly Gln Val Phe Gly Val Gln Thr Leu Val Cys Ile Pro
    210                 215                 220

Ser Ala Asn Gly Val Val Glu Leu Ala Ser Thr Glu Val Ile Phe Gln
225                 230                 235                 240

Asn Ser Asp Leu Met Lys Lys Val Arg Asp Leu Phe Asn Phe Asn Asn
                245                 250                 255

Pro Asp Ala Gly Phe Trp Pro Leu Asn Gln Gly Glu Asn Asp Pro Ser
            260                 265                 270

Ser Leu Trp Leu Asn Pro Ser Ser Ser Ile Glu Ile Lys Asp Thr Ser
        275                 280                 285

Asn Ala Val Ala Leu Val Ser Ala Asn Ala Ser Leu Ser Lys Thr Met
    290                 295                 300

Pro Phe Glu Thr Pro Gly Ser Ser Thr Leu Thr Glu Thr Pro Ser Ala
305                 310                 315                 320

Ala Ala Ala Ala His Val Pro Asn Pro Lys Asn Gln Gly Phe Phe Pro
                325                 330                 335

Arg Glu Leu Asn Phe Ser Asn Ser Leu Lys Pro Glu Ser Gly Glu Ile
            340                 345                 350

Leu Ser Phe Gly Glu Ser Lys Lys Ser Ser Tyr Asn Gly Ser Tyr Phe
        355                 360                 365

Pro Gly Val Ala Ala Glu Glu Thr Asn Lys Lys Arg Arg Ser Pro Ala
    370                 375                 380

Ser Arg Ser Ser Ile Asp Asp Gly Met Leu Ser Phe Thr Ser Gly Val
385                 390                 395                 400

Ile Ile Pro Ala Ser Asn Ile Lys Ser Gly Ala Val Ala Gly Gly Gly
                405                 410                 415

Ala Ser Gly Gly Asp Ser Glu Asn Ser Asp Leu Glu Ala Ser Val Val
            420                 425                 430

Lys Glu Ala Asp Ser Arg Val Val Glu Pro Glu Lys Arg Pro Arg Lys
        435                 440                 445

Arg Gly Arg Lys Pro Gly Asn Gly Arg Glu Glu Pro Leu Asn His Val
    450                 455                 460

Glu Ala Glu Arg Gln Arg Arg Glu Lys Leu Asn Gln Arg Phe Tyr Ala
465                 470                 475                 480

Leu Arg Ala Val Val Pro Asn Val Ser Lys Met Asp Lys Ala Ser Leu
485 490 495

Leu Gly Asp Ala Ile Ser Tyr Ile Asn Glu Leu Lys Ser Lys Leu Ser
500 505 510

Glu Leu Glu Ser Glu Lys Gly Glu Leu Glu Lys Gln Leu Glu Leu Val
515 520 525

Lys Lys Glu Leu Glu Leu Ala Thr Lys Ser Pro Ser Pro Pro Pro Gly
530 535 540

Pro Pro Pro Ser Asn Lys Glu Ala Lys Glu Thr Thr Ser Lys Leu Ile
545 550 555 560

Asp Leu Glu Leu Glu Val Lys Ile Ile Gly Trp Asp Ala Met Ile Arg
565 570 575

Ile Gln Cys Ser Lys Lys Asn His Pro Ala Ala Arg Leu Met Ala Ala
580 585 590

Leu Lys Glu Leu Asp Leu Asp Val Asn His Ala Ser Val Ser Val Val
595 600 605

Asn Asp Leu Met Ile Gln Gln Ala Thr Val Asn Met Gly Asn Arg Phe
610 615 620

Tyr Thr Gln Glu Gln Leu Arg Ser Ala Arg Ser Ser Lys Ile Gly Asn
625 630 635 640

Ala Leu

<210> 15
<211> 2100
<212> DNA
<213> Catharanthus roseus

<400> 15
atgacggact ataggctaca accgaaaatg aacctatggg gtacgacgac caacaccgca 60
gcttcaccaa taattacttc agatgataat agttcgatga tggaggcttt tatgacctca 120
tcagatccga tttctttgtg gccgccgtca atgtctgtga atcatcacca tccaccaact 180
cctacttctt ccgccgtaac aactgcggtg gactccgcta aatctatgcc tgcccaacct 240
gctttttca atcaagaaaa tctccaacag cgccttcaaa ctctaattga tggtgctagg 300
gagagttgga cttatgccat attttggcag tcgtctgttg tcgaattcgc cggtccttcg 360
gtcttgggtt ggggcgatgg atattataag ggagaagaag ataaagggaa gaggaagaat 420
tcgtcttccg cgagttcttt tgcagaacag gaacacagaa agaaagtcct tagagagctc 480
aattctttga ttgctgggcc acaaggcacc gccgatgatg cagttgatga agaggtgacc 540

gataccgaat ggtttttctt aatttcaatg actcagtcat ttgtttccgg gagcggtctt 600
ccagggcagg ccttatacaa ttcaaacccg gtatgggtta ccggagcagg gaggctggcg 660
gtttcacact gcgaccgggc caggcaggct caaagttttg ggcttcagac cttagtttgt 720
attccctccg caaacggcgt tgtggagctg ggttcaacgg aattgatttt tcagagctcc 780
gatctcatga ataaggttag gatactgttc aattttaata atatagattt gggttcgagc 840
tctggacctt ggcctgagaa cgatccttct tctctgtggc ttactgatcc atcgccctca 900
ggggtagggg ttaaggaggg ggtgaatact aataataata ctagtgttca agggaattca 960
attccttctg gtaataagca gcaacttgtg tttggaaata atgataatca tccaaccacg 1020
agtactttga ctgatcatcc cggggctggg gctgtaaata gttataataa ttcatctcag 1080
aatgcccagc agcctcaagg tagtttcttt acaagggaat tgaatttttc agaatacggg 1140
tttgaaagga gtagtgtaaa gaatgggaat tgtaagccag aatcgggaga aatattgaac 1200
tttggtggtg aatctgttac caagaagaat tctgtaagtg gaaatgggaa cttgttttca 1260
gtacaatcac agtttggagc tggggaggag aacaagaaca agaaaaggcc atctcctgtg 1320
tcaaggggga gcaatgatga ggggatgctt tcttttactt ctggggtagt tttgccctct 1380
actggtgtag tgaagtctag tggtggtggt ggtggtggag actccgatca ttctgatctt 1440
gaggcctcag tggtgaagga ggcagagagt agtagggttg tggatcccga gaaacggcct 1500
aggaagaggg ggagaaagcc tgcaaatgga agggaagagc cattgaatca tgtggaggca 1560
gagaggcaga ggagggagaa gttgaaccag aggttctatg ctcttagagc cgtggttcct 1620
aatgtttcaa aaatggataa agcttcactt cttggtgatg ctatttctta tatcaatgag 1680
ttgaaagcta aacttcaaac tacagaaaca gataaagatg aattgaagaa ccaattggac 1740
tcattaaaga aggaattagc aagtaaagaa tctcggcttt tatcatcacc agatcaagat 1800
ctcaagtcct caaacaagca gtcagtgggt aacttggata tggatattga tgtgaaaatc 1860
attggtcggg aagcaatgat tcgagtccaa tccagtaaaa acaaccaccc tgcagcaaga 1920
gtaatgggag cactaaagga tcttgatctt gaattactcc atgctagtgt ctcagtggta 1980
aatgatttga tgatccagca aaatacagtg agaatgggga gccgatttta tactcaggag 2040
cagcttagaa tagcattgac atccagaata gccggaaact cgatgaggct cttggtatga 2100

<210> 16
<211> 699
<212> PRT
<213> Catharanthus roseus

<400> 16

Met Thr Asp Tyr Arg Leu Gln Pro Lys Met Asn Leu Trp Gly Thr Thr
1 5 10 15

Thr Asn Thr Ala Ala Ser Pro Ile Ile Thr Ser Asp Asp Asn Ser Ser
20 25 30

Met Met Glu Ala Phe Met Thr Ser Ser Asp Pro Ile Ser Leu Trp Pro

35 40 45

Pro Ser Met Ser Val Asn His His His Pro Pro Thr Pro Thr Ser Ser
50 55 60

Ala Val Thr Thr Ala Val Asp Ser Ala Lys Ser Met Pro Ala Gln Pro
65 70 75 80

Ala Phe Phe Asn Gln Glu Asn Leu Gln Gln Arg Leu Gln Thr Leu Ile
85 90 95

Asp Gly Ala Arg Glu Ser Trp Thr Tyr Ala Ile Phe Trp Gln Ser Ser
100 105 110

Val Val Glu Phe Ala Gly Pro Ser Val Leu Gly Trp Gly Asp Gly Tyr
115 120 125

Tyr Lys Gly Glu Glu Asp Lys Gly Lys Arg Lys Asn Ser Ser Ser Ala
130 135 140

Ser Ser Phe Ala Glu Gln Glu His Arg Lys Lys Val Leu Arg Glu Leu
145 150 155 160

Asn Ser Leu Ile Ala Gly Pro Gln Gly Thr Ala Asp Asp Ala Val Asp
165 170 175

Glu Glu Val Thr Asp Thr Glu Trp Phe Phe Leu Ile Ser Met Thr Gln
180 185 190

Ser Phe Val Ser Gly Ser Gly Leu Pro Gly Gln Ala Leu Tyr Asn Ser
195 200 205

Asn Pro Val Trp Val Thr Gly Ala Gly Arg Leu Ala Val Ser His Cys
210 215 220

Asp Arg Ala Arg Gln Ala Gln Ser Phe Gly Leu Gln Thr Leu Val Cys
225 230 235 240

Ile Pro Ser Ala Asn Gly Val Val Glu Leu Gly Ser Thr Glu Leu Ile
245 250 255

Phe Gln Ser Ser Asp Leu Met Asn Lys Val Arg Ile Leu Phe Asn Phe
260 265 270

Asn Asn Ile Asp Leu Gly Ser Ser Ser Gly Pro Trp Pro Glu Asn Asp
275 280 285

Pro Ser Ser Leu Trp Leu Thr Asp Pro Ser Pro Ser Gly Val Gly Val
290 295 300

Lys Glu Gly Val Asn Thr Asn Asn Asn Thr Ser Val Gln Gly Asn Ser
305 310 315 320

Ile Pro Ser Gly Asn Lys Gln Gln Leu Val Phe Gly Asn Asn Asp Asn
325 330 335

His Pro Thr Thr Ser Thr Leu Thr Asp His Pro Gly Ala Gly Ala Val
340 345 350

Asn Ser Tyr Asn Asn Ser Ser Gln Asn Ala Gln Gln Pro Gln Gly Ser
355 360 365

Phe Phe Thr Arg Glu Leu Asn Phe Ser Glu Tyr Gly Phe Glu Arg Ser
370 375 380

Ser Val Lys Asn Gly Asn Cys Lys Pro Glu Ser Gly Glu Ile Leu Asn
385 390 395 400

Phe Gly Gly Glu Ser Val Thr Lys Lys Asn Ser Val Ser Gly Asn Gly
405 410 415

Asn Leu Phe Ser Val Gln Ser Gln Phe Gly Ala Gly Glu Glu Asn Lys
420 425 430

Asn Lys Lys Arg Pro Ser Pro Val Ser Arg Gly Ser Asn Asp Glu Gly
435 440 445

Met Leu Ser Phe Thr Ser Gly Val Val Leu Pro Ser Thr Gly Val Val
450 455 460

Lys Ser Ser Gly Gly Gly Gly Gly Gly Asp Ser Asp His Ser Asp Leu
465 470 475 480

Glu Ala Ser Val Val Lys Glu Ala Glu Ser Ser Arg Val Val Asp Pro
485 490 495

Glu Lys Arg Pro Arg Lys Arg Gly Arg Lys Pro Ala Asn Gly Arg Glu
500 505 510

Glu Pro Leu Asn His Val Glu Ala Glu Arg Gln Arg Arg Glu Lys Leu
515 520 525

Asn Gln Arg Phe Tyr Ala Leu Arg Ala Val Val Pro Asn Val Ser Lys
530 535 540

Met Asp Lys Ala Ser Leu Leu Gly Asp Ala Ile Ser Tyr Ile Asn Glu
545 550 555 560

Leu Lys Ala Lys Leu Gln Thr Thr Glu Thr Asp Lys Asp Glu Leu Lys
565 570 575

Asn Gln Leu Asp Ser Leu Lys Lys Glu Leu Ala Ser Lys Glu Ser Arg
580 585 590

```
Leu Leu Ser Ser Pro Asp Gln Asp Leu Lys Ser Ser Asn Lys Gln Ser
        595                 600                 605

Val Gly Asn Leu Asp Met Asp Ile Asp Val Lys Ile Ile Gly Arg Glu
    610                 615                 620

Ala Met Ile Arg Val Gln Ser Ser Lys Asn Asn His Pro Ala Ala Arg
625                 630                 635                 640

Val Met Gly Ala Leu Lys Asp Leu Asp Leu Glu Leu Leu His Ala Ser
                645                 650                 655

Val Ser Val Val Asn Asp Leu Met Ile Gln Gln Asn Thr Val Arg Met
            660                 665                 670

Gly Ser Arg Phe Tyr Thr Gln Glu Gln Leu Arg Ile Ala Leu Thr Ser
        675                 680                 685

Arg Ile Ala Gly Asn Ser Met Arg Leu Leu Val
    690                 695

<210>  17
<211>  2079
<212>  DNA
<213>  Solanum tuberosum

<400>  17
atgactgaat acagcttacc caccatgaat ttgtggaaca atagtactag cgatgataac      60
gtgtctatga tggaagcttt tatgtcttct gatctttctt tttgggctac tactaattct     120
actactacta attctgcttc tgctgctgtg gttggcgtca attcaaatct tcttcatact     180
aataataata atccgtctgt ttttcctcta tcttcttcta catctgtatc cgcggctgcg     240
gctgttgatg ctaccaaatc tatgccgttt ttcaaccaag aaacccttca gcagcgtctt     300
caagctctta tcgatggtgc tagagagacg tggacttacg ctatcttttg gcaatcgtcg     360
gttgttgatt tctcgagtcc gtctgtgttg ggttggggag atggttatta caaaggcgaa     420
gaagataaag ccaaaaggaa attagcggtt tcttctcctg cttatattgc tgagcaggag     480
caccggaaga aggttctccg ggagctgaat tcgttgattt caggggcacc agctgggacc     540
gatgatgctg ttgatgaaga agttaccgac accgaatggt tctttcttat ctccatgacc     600
caatcgtttg ttaatggaag tgggcttcct ggtcaggcgt tgtatagttc cagccctatt     660
tgggtcgccg gaactgagaa attggcagct tcccactgcg aacgggttag acaagcacaa     720
gggttcgggc ttcagactat tgtctgtatt ccttcagcta acggcgtggt tgaattgggc     780
tcgacagagt tgattgttga aagttctgat ctcatgaaca aggttagagt attgtttaac     840
ttcagtaatg atttggggtc tggttcatgg gctgtgcagc cggagagcga cccgtcggcg     900
ctttggctca ctgaaccatc gtcctcaggt atggaagtta gagagtcttt aaatacagta     960
caaacaaatt cagttccatc aagtaatagt aataagcaaa ttgcttatgc aaatgagaat    1020
```

aatcatcaat ctggaaatgg tcagagttgt tacaatctgc agcaacaaca gaacaatcct 1080

cctcaacaac aaacacaagg atttttcacg agggagttga atttttcgga attcgggttc 1140

gatggaagta gcaataggaa tgggaatgca tcgctctctt gcaaacctga atcaggagaa 1200

atcttgaatt ttggtgatag tactaaaaaa agtgcttcca gtgcaaatgt gaacttgttt 1260

acgggtcagt cccaatttgg ggcagtggag gagaataaca ataacaagaa caagaaaaga 1320

tcagctactt ccaggggaag caatgaagaa ggaatgcttt catttgtttc aggtacagtt 1380

ttgccttctt cgggtatgaa gtcaggtgga ggtggaggcg aagactctga acattcagat 1440

cttgaggctt cagtggtgaa agaagctgat agtagtagag tggtagaacc agaaaagagg 1500

ccaaggaagc gagggagaaa gccagcgaat ggacgagagg agcctttgaa tcacgtcgag 1560

gcagagaggc aaaggaggga gaaattgaac caaagattct acgcgcttag agctgttgta 1620

ccaaatgtgt ctaagatgga caaggcatca cttcttggag atgcaatttc atatataaac 1680

gagttgaaat caaagcttca aaatacagag tcagataaag aagacttgaa gagccaaata 1740

gaagatttaa agaaagaatc aaggcgcccc ggtcctccac caccaaacca agatctcaag 1800

attggaggca agattgtaga cgtggatata gacgttaaga taatcggatg ggatgcaatg 1860

attggtatac aatgtaataa aaagaatcat ccagctgcaa ggttaatggc agccctcatg 1920

gaattagacc tagacgtgca ccatgccagt gtttcagttg tcaacgattt gatgatccaa 1980

caagccacag tgaaaatggg tagcagacat tacactgaag agcagcttag ggtagcattg 2040

aaatcgaaaa ttgctgaaac cccccttagaa agtagatag 2079

<210> 18
<211> 692
<212> PRT
<213> Solanum tuberosum

<400> 18

Met Thr Glu Tyr Ser Leu Pro Thr Met Asn Leu Trp Asn Asn Ser Thr
1 5 10 15

Ser Asp Asp Asn Val Ser Met Met Glu Ala Phe Met Ser Ser Asp Leu
20 25 30

Ser Phe Trp Ala Thr Thr Asn Ser Thr Thr Thr Asn Ser Ala Ser Ala
35 40 45

Ala Val Val Gly Val Asn Ser Asn Leu Leu His Thr Asn Asn Asn Asn
50 55 60

Pro Ser Val Phe Pro Leu Ser Ser Ser Thr Ser Val Ser Ala Ala Ala
65 70 75 80

Ala Val Asp Ala Thr Lys Ser Met Pro Phe Phe Asn Gln Glu Thr Leu
85 90 95

Gln Gln Arg Leu Gln Ala Leu Ile Asp Gly Ala Arg Glu Thr Trp Thr

100 105 110

Tyr Ala Ile Phe Trp Gln Ser Ser Val Val Asp Phe Ser Ser Pro Ser
115 120 125

Val Leu Gly Trp Gly Asp Gly Tyr Tyr Lys Gly Glu Glu Asp Lys Ala
130 135 140

Lys Arg Lys Leu Ala Val Ser Ser Pro Ala Tyr Ile Ala Glu Gln Glu
145 150 155 160

His Arg Lys Lys Val Leu Arg Glu Leu Asn Ser Leu Ile Ser Gly Ala
165 170 175

Pro Ala Gly Thr Asp Asp Ala Val Asp Glu Glu Val Thr Asp Thr Glu
180 185 190

Trp Phe Phe Leu Ile Ser Met Thr Gln Ser Phe Val Asn Gly Ser Gly
195 200 205

Leu Pro Gly Gln Ala Leu Tyr Ser Ser Ser Pro Ile Trp Val Ala Gly
210 215 220

Thr Glu Lys Leu Ala Ala Ser His Cys Glu Arg Val Arg Gln Ala Gln
225 230 235 240

Gly Phe Gly Leu Gln Thr Ile Val Cys Ile Pro Ser Ala Asn Gly Val
245 250 255

Val Glu Leu Gly Ser Thr Glu Leu Ile Val Glu Ser Ser Asp Leu Met
260 265 270

Asn Lys Val Arg Val Leu Phe Asn Phe Ser Asn Asp Leu Gly Ser Gly
275 280 285

Ser Trp Ala Val Gln Pro Glu Ser Asp Pro Ser Ala Leu Trp Leu Thr
290 295 300

Glu Pro Ser Ser Ser Gly Met Glu Val Arg Glu Ser Leu Asn Thr Val
305 310 315 320

Gln Thr Asn Ser Val Pro Ser Ser Asn Ser Asn Lys Gln Ile Ala Tyr
325 330 335

Ala Asn Glu Asn Asn His Gln Ser Gly Asn Gly Gln Ser Cys Tyr Asn
340 345 350

Leu Gln Gln Gln Gln Asn Asn Pro Pro Gln Gln Gln Thr Gln Gly Phe
355 360 365

Phe Thr Arg Glu Leu Asn Phe Ser Glu Phe Gly Phe Asp Gly Ser Ser

```
            370                 375                 380

Asn Arg Asn Gly Asn Ala Ser Leu Ser Cys Lys Pro Glu Ser Gly Glu
385                 390                 395                 400

Ile Leu Asn Phe Gly Asp Ser Thr Lys Lys Ser Ala Ser Ser Ala Asn
                405                 410                 415

Val Asn Leu Phe Thr Gly Gln Ser Gln Phe Gly Ala Val Glu Glu Asn
            420                 425                 430

Asn Asn Asn Lys Asn Lys Lys Arg Ser Ala Thr Ser Arg Gly Ser Asn
        435                 440                 445

Glu Glu Gly Met Leu Ser Phe Val Ser Gly Thr Val Leu Pro Ser Ser
    450                 455                 460

Gly Met Lys Ser Gly Gly Gly Gly Gly Glu Asp Ser Glu His Ser Asp
465                 470                 475                 480

Leu Glu Ala Ser Val Val Lys Glu Ala Asp Ser Ser Arg Val Val Glu
                485                 490                 495

Pro Glu Lys Arg Pro Arg Lys Arg Gly Arg Lys Pro Ala Asn Gly Arg
            500                 505                 510

Glu Glu Pro Leu Asn His Val Glu Ala Glu Arg Gln Arg Arg Glu Lys
        515                 520                 525

Leu Asn Gln Arg Phe Tyr Ala Leu Arg Ala Val Val Pro Asn Val Ser
    530                 535                 540

Lys Met Asp Lys Ala Ser Leu Leu Gly Asp Ala Ile Ser Tyr Ile Asn
545                 550                 555                 560

Glu Leu Lys Ser Lys Leu Gln Asn Thr Glu Ser Asp Lys Glu Asp Leu
                565                 570                 575

Lys Ser Gln Ile Glu Asp Leu Lys Lys Glu Ser Arg Arg Pro Gly Pro
            580                 585                 590

Pro Pro Pro Asn Gln Asp Leu Lys Ile Gly Gly Lys Ile Val Asp Val
        595                 600                 605

Asp Ile Asp Val Lys Ile Ile Gly Trp Asp Ala Met Ile Gly Ile Gln

    610                 615                 620

Cys Asn Lys Lys Asn His Pro Ala Ala Arg Leu Met Ala Ala Leu Met
625                 630                 635                 640

Glu Leu Asp Leu Asp Val His His Ala Ser Val Ser Val Val Asn Asp
```

```
            645                 650                 655

Leu Met Ile Gln Gln Ala Thr Val Lys Met Gly Ser Arg His Tyr Thr
            660                 665                 670

Glu Glu Gln Leu Arg Val Ala Leu Lys Ser Lys Ile Ala Glu Thr Pro
        675                 680                 685

Leu Glu Ser Arg
    690
```

<210> 19
<211> 1941
<212> DNA
<213> Pisum sativum

<400> 19

```
atgacagatt accgctcact cccgacaatg aataatagta tctggacaga cgacaactcc      60
tccgtcatgg aagctttcat gagcaccacc gccgaccttt cttctatctg gcttccacca     120
ccaaattccg ctgcatcaac caccacacca ggacccgata caaccaaacc tccaccacaa     180
caacagccac tcttcaacca agaaactctc caacaccgtc ttcaagcttt aatcgaagac     240
gcaaaagaga attggactta cgccatcttc tggcaaacct cttacgacta ctctacaagc     300
agacagctcc ttggttgggg agacggttat tacaaaggcg aagatgacaa agaaaaagct     360
aaaaaagtta tcttgcctga acaacaagct catcgcaata aagtcctccg tgaacttaac     420
gccttaattt ccggctcatc tagctcagat gatgtcgttg acgaagatgt caccgacact     480
gagtggttct ttctcacgtc gatgacacac tccttcgtca acggaagcgg tttactgagc     540
caggcttact ttaattcttc cccagtatgg atcaacgata ggctttccat gtccacatgt     600
gaaagaaccc gtgcagcaca tgttcacggg cttcagactt tggtatatat accagcaccg     660
tcttcaaacg gtgtcgttga gctcgcatct actgagataa ttccacatag cgctggtata     720
atggagaagg ttcgtttttt gtttgatttc aataatccag aagcacggtc ttggccgttg     780
aattccgccg acaacgatcc ttcttccatg tggttggata tccccggctc cggtggaatt     840
gaaattagag actccatcaa cactgttagt gccgtcagtg taacggcttc agcaaatgca     900
acaatcccta aaaaatcgcc gtttgaaatt cacggtgctt ctactactct accggaatca     960
tccaccaccg ttaatatttc aactgctcag cgccaaatcc agaatcaaaa ccagaaccag    1020
agcttctttc caagagaact gaatttttca ggttctttca aaccggaatc cggcgagatt    1080
ctgaactttg ggagagtaa aaagagttct tacagctctg ccaatggtaa tttcttttcc     1140
ggtccgtcac cattcgctgc taatgaagaa aacagaaaaa gaagatcccc ggtgtctaga    1200
agtagtatcg aggacggaat tctttcattc agttctggca aactgttaca tggttcaact    1260
ataaaatccg gtggcggaga ttccgatcat tcagatctgg aagtttctgt ggtgaagaaa    1320
actgtgagca gcagagttat cgaaccagag aagcggcctc ggaagcgagg tagaaaacca    1380
gccaacggaa gagaagaacc cttgaaccac gtggaagcag agaggcagcg acgggagaaa    1440
```

```
ctgaaccaga gattctacgc tttacgagct gtggttccta atgtttctaa aatggacaaa   1500
gcttcgcttc ttggagacgc gatttcttac ataaacgagt tgaaattgaa gctgcaaggg   1560
cttgaatcgt caaaagatga gttggagaaa gaactagata caacccgaaa ggaactagaa   1620
attgcaacta aaaaaccagt tcggttgaac gaagaagaaa aagagaaacc agaaaacaac   1680
tctaagttga ttgatttgga catagatgtg aaaatcatgg gatgggatgc tatgattagg   1740
attcagtgta gcaagaagaa tcaccctgct gcgaaattaa tggcggcttt aaaagaattg   1800
gatcttgatg tgaatcatgc tagtgtttct gttgttaatg acttaatgat tcaacaggct   1860
tctattaaca tgggaagtag attttacaca caagaacagc ttttatctgt tctttcttcc   1920
aaaattggtg atacacaatg a                                             1941
```

```
<210>  20
<211>  646
<212>  PRT
<213>  Pisum sativum

<400>  20

Met Thr Asp Tyr Arg Ser Leu Pro Thr Met Asn Asn Ser Ile Trp Thr
1               5                   10                  15

Asp Asp Asn Ser Ser Val Met Glu Ala Phe Met Ser Thr Thr Ala Asp
            20                  25                  30

Leu Ser Ser Ile Trp Leu Pro Pro Pro Asn Ser Ala Ala Ser Thr Thr
        35                  40                  45

Thr Pro Gly Pro Asp Thr Thr Lys Pro Pro Pro Gln Gln Gln Pro Leu
    50                  55                  60

Phe Asn Gln Glu Thr Leu Gln His Arg Leu Gln Ala Leu Ile Glu Asp
65                  70                  75                  80

Ala Lys Glu Asn Trp Thr Tyr Ala Ile Phe Trp Gln Thr Ser Tyr Asp
                85                  90                  95

Tyr Ser Thr Ser Arg Gln Leu Leu Gly Trp Gly Asp Gly Tyr Tyr Lys
            100                 105                 110

Gly Glu Asp Asp Lys Glu Lys Ala Lys Lys Val Ile Leu Pro Glu Gln
        115                 120                 125

Gln Ala His Arg Asn Lys Val Leu Arg Glu Leu Asn Ala Leu Ile Ser
    130                 135                 140

Gly Ser Ser Ser Ser Asp Asp Val Val Asp Glu Asp Val Thr Asp Thr
145                 150                 155                 160

Glu Trp Phe Phe Leu Thr Ser Met Thr His Ser Phe Val Asn Gly Ser
                165                 170                 175
```

Gly Leu Leu Ser Gln Ala Tyr Phe Asn Ser Ser Pro Val Trp Ile Asn
180 185 190

Asp Arg Leu Ser Met Ser Thr Cys Glu Arg Thr Arg Ala Ala His Val
195 200 205

His Gly Leu Gln Thr Leu Val Tyr Ile Pro Ala Pro Ser Ser Asn Gly
210 215 220

Val Val Glu Leu Ala Ser Thr Glu Ile Ile Pro His Ser Ala Gly Ile
225 230 235 240

Met Glu Lys Val Arg Phe Leu Phe Asp Phe Asn Asn Pro Glu Ala Arg
245 250 255

Ser Trp Pro Leu Asn Ser Ala Asp Asn Asp Pro Ser Ser Met Trp Leu
260 265 270

Asp Ile Pro Gly Ser Gly Gly Ile Glu Ile Arg Asp Ser Ile Asn Thr
275 280 285

Val Ser Ala Val Ser Val Thr Ala Ser Ala Asn Ala Thr Ile Pro Lys
290 295 300

Lys Ser Pro Phe Glu Ile His Gly Ala Ser Thr Thr Leu Pro Glu Ser
305 310 315 320

Ser Thr Thr Val Asn Ile Ser Thr Ala Gln Arg Gln Ile Gln Asn Gln
325 330 335

Asn Gln Asn Gln Ser Phe Phe Pro Arg Glu Leu Asn Phe Ser Gly Ser
340 345 350

Phe Lys Pro Glu Ser Gly Glu Ile Leu Asn Phe Gly Glu Ser Lys Lys
355 360 365

Ser Ser Tyr Ser Ser Ala Asn Gly Asn Phe Phe Ser Gly Pro Ser Pro
370 375 380

Phe Ala Ala Asn Glu Glu Asn Arg Lys Arg Arg Ser Pro Val Ser Arg
385 390 395 400

Ser Ser Ile Glu Asp Gly Ile Leu Ser Phe Ser Ser Gly Lys Leu Leu
405 410 415

His Gly Ser Thr Ile Lys Ser Gly Gly Gly Asp Ser Asp His Ser Asp
420 425 430

Leu Glu Val Ser Val Val Lys Lys Thr Val Ser Ser Arg Val Ile Glu
435 440 445

Pro Glu Lys Arg Pro Arg Lys Arg Gly Arg Lys Pro Ala Asn Gly Arg
450 455 460

Glu Glu Pro Leu Asn His Val Glu Ala Glu Arg Gln Arg Arg Glu Lys
465 470 475 480

Leu Asn Gln Arg Phe Tyr Ala Leu Arg Ala Val Val Pro Asn Val Ser
485 490 495

Lys Met Asp Lys Ala Ser Leu Leu Gly Asp Ala Ile Ser Tyr Ile Asn
500 505 510

Glu Leu Lys Leu Lys Leu Gln Gly Leu Glu Ser Ser Lys Asp Glu Leu
515 520 525

Glu Lys Glu Leu Asp Thr Thr Arg Lys Glu Leu Glu Ile Ala Thr Lys
530 535 540

Lys Pro Val Arg Leu Asn Glu Glu Glu Lys Glu Lys Pro Glu Asn Asn
545 550 555 560

Ser Lys Leu Ile Asp Leu Asp Ile Asp Val Lys Ile Met Gly Trp Asp
565 570 575

Ala Met Ile Arg Ile Gln Cys Ser Lys Lys Asn His Pro Ala Ala Lys
580 585 590

Leu Met Ala Ala Leu Lys Glu Leu Asp Leu Asp Val Asn His Ala Ser
595 600 605

Val Ser Val Val Asn Asp Leu Met Ile Gln Gln Ala Ser Ile Asn Met
610 615 620

Gly Ser Arg Phe Tyr Thr Gln Glu Gln Leu Leu Ser Val Leu Ser Ser
625 630 635 640

Lys Ile Gly Asp Thr Gln
645

<210> 21
<211> 1761
<212> DNA
<213> Brassica oleracea

<400> 21
tcaaactctc tccattagag cagccttgag ctgatcctgc gtgaaaaact gattccccat 60

cttcacagtc gcttgttgga tcatgaactc attcactacc gataaactcg cgtgattcac 120

ttccaattcc agctccttga gtgcttccat gaacttagca ccaggatgat tcttcttagc 180

gcattggatc ctaatcatcg catcccaacc aatgatcttc acatctatct ccacctctat 240

ggacacacct gaatcttgat ccaaacactt ttgatcttta accaaacttt tcacattccc 300

atcccccacc tctttgctca tcccatcaat ctgcttctgc aactcctctt tatcagcttc 360

agccttttgc agcttagctt taagctcatt gatatacgag atcgcgtctc ctagaagaga 420
agctttatcc atcttagaca cattaggaac cacagctctc agagagtaga atctctggtt 480
cagcttctct cttctctgtc tctctgcctc cacgtgattc aacggctcct ctcttccgtt 540
agccggtttc ctccctcgtt tcctcggttt cttctccgtc tcagccacga ttctaccact 600
ctccgcttct ttaacaaccg aagcttcgag atcagagcga ttcgagtcgt tcgatttcgt 660
aggaagtgga agaacagaag tgaaagacag catctcttct tccttgtcgg aaaccaaaca 720
tctcttcttc ttgttgctgt ctccctccac aaacccagaa ctcgagattt gcgggttctg 780
ctgctgctta gggtgttcca ctgagcttcc attacaaagc ttcgaagccg tctgagaatc 840
agagttagaa gtcgccggag ccccaagaac cggttcgatc ccggttacaa tcggttcatt 900
aatccacgtg gcggaatcat tctctccttg atccgggttc aaattaaact cccaagaacc 960
cgactcgcct ccaccaccgc caccgttgaa actgaaaaag ctgttgactt tctcaacgag 1020
atccgagctc tgatgtataa tctccaacga accaagctca acgacgccgt tttgcgtcgg 1080
tatacaaacc atcgtctcca acccgtaaac ctgaccctgg cgagctctct cgcagctcga 1140
tccagccaag gcgttcgagc cagagagcca aatggtccgc gagctcgaga aagctcggcc 1200
gggcaaacca gagccgttga cgaagctctg cgtcatcgaa accaagaaga accattccgt 1260
atccgacacg tcttcgtctc ccgcctcgtc ggaggagccg ccgctgctgc tgactgtccc 1320
tcctccgccg gagatcagag agttgagctc tcgaatcact ctcttccgat gctcttgctc 1380
cgccgcgctg acgggattgg gcttcctctt cctcgacttc ctctcctcct cgcctttgta 1440
gtagccatcg ccccaggtta gcagcgccgc cgtgttgctg atgtcttctc cggcgaagtc 1500
gtgggatagt tgccagaaca cggcgtacgt ccagctttct ctagctcctt cgatcaatgc 1560
ttggagtcgt tgctggagag tgtcttcggt gacgtgagga ggaggaggag tgagaggggg 1620
ttgaggccag agagaagagt gttcggagcc gatgagaggt tccattacag acgcattgtc 1680
ttcgattgtt gaccagaggt ttgtgccgtt tgttgactgg ttgaggtggt ggtcggttag 1740
ttgaacattt gtcgaagaca t 1761

<210> 22
<211> 586
<212> PRT
<213> Brassica oleracea

<400> 22

Met Ser Ser Thr Asn Val Gln Leu Thr Asp His His Leu Asn Gln Ser
1 5 10 15

Thr Asn Gly Thr Asn Leu Trp Ser Thr Ile Glu Asp Asn Ala Ser Val
20 25 30

Met Glu Pro Leu Ile Gly Ser Glu His Ser Ser Leu Trp Pro Gln Pro
35 40 45

```
Pro Leu Thr Pro Pro Pro Pro His Val Thr Glu Asp Thr Leu Gln Gln
    50                  55                  60

Arg Leu Gln Ala Leu Ile Glu Gly Ala Arg Glu Ser Trp Thr Tyr Ala
65                  70                  75                  80

Val Phe Trp Gln Leu Ser His Asp Phe Ala Gly Glu Asp Ile Ser Asn
                85                  90                  95

Thr Ala Ala Leu Leu Thr Trp Gly Asp Gly Tyr Tyr Lys Gly Glu Glu
            100                 105                 110

Glu Arg Lys Ser Arg Lys Arg Lys Pro Asn Pro Val Ser Ala Ala Glu
        115                 120                 125

Gln Glu His Arg Lys Arg Val Ile Arg Glu Leu Asn Ser Leu Ile Ser
    130                 135                 140

Gly Gly Gly Gly Thr Val Ser Ser Ser Gly Gly Ser Ser Asp Glu Ala
145                 150                 155                 160

Gly Asp Glu Asp Val Ser Asp Thr Glu Trp Phe Phe Leu Val Ser Met
                165                 170                 175

Thr Gln Ser Phe Val Asn Gly Ser Gly Leu Pro Gly Arg Ala Phe Ser
            180                 185                 190

Ser Ser Arg Thr Ile Trp Leu Ser Gly Ser Asn Ala Leu Ala Gly Ser
        195                 200                 205

Ser Cys Glu Arg Ala Arg Gln Gly Gln Val Tyr Gly Leu Glu Thr Met
    210                 215                 220

Val Cys Ile Pro Thr Gln Asn Gly Val Val Glu Leu Gly Ser Leu Glu
225                 230                 235                 240

Ile Ile His Gln Ser Ser Asp Leu Val Glu Lys Val Asn Ser Phe Phe
                245                 250                 255

Ser Phe Asn Gly Gly Gly Gly Gly Gly Glu Ser Gly Ser Trp Glu Phe
            260                 265                 270

Asn Leu Asn Pro Asp Gln Gly Glu Asn Asp Ser Ala Thr Trp Ile Asn
        275                 280                 285

Glu Pro Ile Val Thr Gly Ile Glu Pro Val Leu Gly Ala Pro Ala Thr
    290                 295                 300

Ser Asn Ser Asp Ser Gln Thr Ala Ser Lys Leu Cys Asn Gly Ser Ser
305                 310                 315                 320
```

```
Val Glu His Pro Lys Gln Gln Gln Asn Pro Gln Ile Ser Ser Ser Gly
                325                 330                 335

Phe Val Glu Gly Asp Ser Asn Lys Lys Lys Arg Cys Leu Val Ser Asp
            340                 345                 350

Lys Glu Glu Glu Met Leu Ser Phe Thr Ser Val Leu Pro Leu Pro Thr
        355                 360                 365

Lys Ser Asn Asp Ser Asn Arg Ser Asp Leu Glu Ala Ser Val Val Lys
    370                 375                 380

Glu Ala Glu Ser Gly Arg Ile Val Ala Glu Thr Glu Lys Lys Pro Arg
385                 390                 395                 400

Lys Arg Gly Arg Lys Pro Ala Asn Gly Arg Glu Glu Pro Leu Asn His
                405                 410                 415

Val Glu Ala Glu Arg Gln Arg Arg Glu Lys Leu Asn Gln Arg Phe Tyr
            420                 425                 430

Ser Leu Arg Ala Val Val Pro Asn Val Ser Lys Met Asp Lys Ala Ser
        435                 440                 445

Leu Leu Gly Asp Ala Ile Ser Tyr Ile Asn Glu Leu Lys Ala Lys Leu
    450                 455                 460

Gln Lys Ala Glu Ala Asp Lys Glu Glu Leu Gln Lys Gln Ile Asp Gly
465                 470                 475                 480

Met Ser Lys Glu Val Gly Asp Gly Asn Val Lys Ser Leu Val Lys Asp
                485                 490                 495

Gln Lys Cys Leu Asp Gln Asp Ser Gly Val Ser Ile Glu Val Glu Ile
            500                 505                 510

Asp Val Lys Ile Ile Gly Trp Asp Ala Met Ile Arg Ile Gln Cys Ala
        515                 520                 525

Lys Lys Asn His Pro Gly Ala Lys Phe Met Glu Ala Leu Lys Glu Leu
    530                 535                 540

Glu Leu Glu Val Asn His Ala Ser Leu Ser Val Val Asn Glu Phe Met
545                 550                 555                 560

Ile Gln Gln Ala Thr Val Lys Met Gly Asn Gln Phe Phe Thr Gln Asp
                565                 570                 575

Gln Leu Lys Ala Ala Leu Met Glu Arg Val
            580                 585
```

<210> 23
<211> 2100
<212> DNA
<213> Oryza sativa

<400> 23
atgaaccttt ggacggacga caacgcgtcg atgatggagg cgttcatggc ctccgccgac 60
ctcccggcct tcccgtgggg ggcggcctcc accccgccgc cgccgccgcc gccgcctcac 120
caccaccacc aacagcagca gcagcaggtc ctgccgccgc ctgcagcagc tccggcggcg 180
gcggcgttca accaggacac gctgcagcag cggctgcagt cgatcatcga ggggtctcgg 240
gagacgtgga cgtacgccat cttctggcag tcctccatcg acgtctccac cggcgcctcc 300
ctcctcggct ggggcgacgg ctactacaag ggctgcgacg acgacaagcg caagcagcgc 360
tcctccaccc ccgccgccgc cgccgagcag gagcaccgca agcgcgtcct ccgcgagctc 420
aactccctca tcgccggcgc aggcgccgcc cccgacgagg ccgtcgagga ggaggtcacc 480
gacaccgagt ggttcttcct cgtctccatg acccagtcct tccccaacgg cctcggcctc 540
cccggccagg ccctcttcgc cgcccagccc acctggatcg ccaccggctt gtcctccgcc 600
ccctgtgacc gcgccaggca ggcctacacc ttcggcctcc gcaccatggt ctgcctcccc 660
ctcgccaccg gcgtcctcga gctcggctcc accgacgtca tcttccagac cggggacagc 720
atccccagga tccgcgccct cttcaacctc agcgccgccg ccgcctcctc ctggcccccc 780
caccccgacg ccgcctccgc cgatccctcc gtgctctggc tcgccgacgc gccccccatg 840
gacatgaagg actccatctc cgccgcagac atctccgtct ccaagccacc acccccgccg 900
ccgcaccaga tccagcactt cgagaacggg agcaccagca cgctcacgga gaatcccagc 960
ccgtcggtgc acgcgccgac gccctcgcag ccggccgcgc cgccccagcg gcagcagcag 1020
cagcagcaga gcagccaggc tcagcagggc cccttccgcc gggagctcaa cttctccgac 1080
ttcgcgtcca acggcggcgc cgcggccccg cctttcttca agcccgagac cggcgagatc 1140
ctaaacttcg gcaacgacag cagcagtggc cggaggaatc cgtcgccggc gcccccggcc 1200
gccaccgcca gcctcaccac cgcgccgggg agcctgttct cgcagcacac gccgacgctg 1260
acggcggcgg cgaacgacgc caagagcaac aaccagaaga ggtccatgga ggccacctcc 1320
cgcgccagca acaccaacaa ccacccggcg gcgacggcga acgaggggat gctgtccttc 1380
tcgtcggcgc cgacgacgcg gccgtcgacg ggacgggcg ccccggccaa gtcggagtcg 1440
gaccactcgg acctggaggc gtcggtgcgg gaggtggaga gcagccgcgt ggtggcgccg 1500
ccgccggagg cggagaagcg gccgcggaag cgcgggcgga agccggcgaa cgggcgggag 1560
gagccgctga accacgtgga ggcggagcgg cagcggcggg agaagctcaa ccagcgcttc 1620
tacgcgctgc gcgccgtggt gcccaacgtg tccaagatgg acaaggcgtc gctgctgggc 1680
gacgccatct cctacatcaa cgagctccgg gggaagctca cggcgctgga gacggacaag 1740
gagacgctgc agtcgcagat ggagtcgctc aagaaggagc gggacgcgcg gccgccggcg 1800
ccgtcgggcg gcggcggaga cggcggggcg cggtgccacg cggtggagat cgaggccaag 1860

atccttgggc tggaggccat gatccgcgtg cagtgccaca agcggaacca cccggcggcg 1920
cggctgatga cggcgctgcg ggagctggac ctggacgtgt accatgccag cgtctccgtg 1980
gtcaaggacc tcatgatcca gcaggtggcc gtcaagatgg ccagccgcgt ctactcgcag 2040
gaccagctca acgccgcgct ctacacccgc atcgccgagc ctggcaccgc cgcccggtaa 2100

<210> 24
<211> 699
<212> PRT
<213> Oryza sativa

<400> 24

Met Asn Leu Trp Thr Asp Asp Asn Ala Ser Met Met Glu Ala Phe Met
1 5 10 15

Ala Ser Ala Asp Leu Pro Ala Phe Pro Trp Gly Ala Ala Ser Thr Pro
20 25 30

Pro Pro Pro Pro Pro Pro Pro His His His His Gln Gln Gln Gln Gln
35 40 45

Gln Val Leu Pro Pro Pro Ala Ala Ala Pro Ala Ala Ala Ala Phe Asn
50 55 60

Gln Asp Thr Leu Gln Gln Arg Leu Gln Ser Ile Ile Glu Gly Ser Arg
65 70 75 80

Glu Thr Trp Thr Tyr Ala Ile Phe Trp Gln Ser Ser Ile Asp Val Ser
85 90 95

Thr Gly Ala Ser Leu Leu Gly Trp Gly Asp Gly Tyr Tyr Lys Gly Cys
100 105 110

Asp Asp Asp Lys Arg Lys Gln Arg Ser Ser Thr Pro Ala Ala Ala Ala
115 120 125

Glu Gln Glu His Arg Lys Arg Val Leu Arg Glu Leu Asn Ser Leu Ile
130 135 140

Ala Gly Ala Gly Ala Ala Pro Asp Glu Ala Val Glu Glu Glu Val Thr
145 150 155 160

Asp Thr Glu Trp Phe Phe Leu Val Ser Met Thr Gln Ser Phe Pro Asn
165 170 175

Gly Leu Gly Leu Pro Gly Gln Ala Leu Phe Ala Ala Gln Pro Thr Trp
180 185 190

Ile Ala Thr Gly Leu Ser Ser Ala Pro Cys Asp Arg Ala Arg Gln Ala
195 200 205

Tyr Thr Phe Gly Leu Arg Thr Met Val Cys Leu Pro Leu Ala Thr Gly

210 215 220

Val Leu Glu Leu Gly Ser Thr Asp Val Ile Phe Gln Thr Gly Asp Ser
225 230 235 240

Ile Pro Arg Ile Arg Ala Leu Phe Asn Leu Ser Ala Ala Ala Ala Ser
245 250 255

Ser Trp Pro Pro His Pro Asp Ala Ala Ser Ala Asp Pro Ser Val Leu
260 265 270

Trp Leu Ala Asp Ala Pro Pro Met Asp Met Lys Asp Ser Ile Ser Ala
275 280 285

Ala Asp Ile Ser Val Ser Lys Pro Pro Pro Pro Pro Pro His Gln Ile
290 295 300

Gln His Phe Glu Asn Gly Ser Thr Ser Thr Leu Thr Glu Asn Pro Ser
305 310 315 320

Pro Ser Val His Ala Pro Thr Pro Ser Gln Pro Ala Ala Pro Pro Gln
325 330 335

Arg Gln Gln Gln Gln Gln Gln Ser Ser Gln Ala Gln Gln Gly Pro Phe
340 345 350

Arg Arg Glu Leu Asn Phe Ser Asp Phe Ala Ser Asn Gly Gly Ala Ala
355 360 365

Ala Pro Pro Phe Phe Lys Pro Glu Thr Gly Glu Ile Leu Asn Phe Gly
370 375 380

Asn Asp Ser Ser Ser Gly Arg Arg Asn Pro Ser Pro Ala Pro Pro Ala
385 390 395 400

Ala Thr Ala Ser Leu Thr Thr Ala Pro Gly Ser Leu Phe Ser Gln His
405 410 415

Thr Pro Thr Leu Thr Ala Ala Ala Asn Asp Ala Lys Ser Asn Asn Gln
420 425 430

Lys Arg Ser Met Glu Ala Thr Ser Arg Ala Ser Asn Thr Asn Asn His
435 440 445

Pro Ala Ala Thr Ala Asn Glu Gly Met Leu Ser Phe Ser Ser Ala Pro
450 455 460

Thr Thr Arg Pro Ser Thr Gly Thr Gly Ala Pro Ala Lys Ser Glu Ser
465 470 475 480

Asp His Ser Asp Leu Glu Ala Ser Val Arg Glu Val Glu Ser Ser Arg
485 490 495

```
Val Val Ala Pro Pro Pro Glu Ala Glu Lys Arg Pro Arg Lys Arg Gly
            500                 505                 510

Arg Lys Pro Ala Asn Gly Arg Glu Glu Pro Leu Asn His Val Glu Ala
        515                 520                 525

Glu Arg Gln Arg Arg Glu Lys Leu Asn Gln Arg Phe Tyr Ala Leu Arg
    530                 535                 540

Ala Val Val Pro Asn Val Ser Lys Met Asp Lys Ala Ser Leu Leu Gly
545                 550                 555                 560

Asp Ala Ile Ser Tyr Ile Asn Glu Leu Arg Gly Lys Leu Thr Ala Leu
                565                 570                 575

Glu Thr Asp Lys Glu Thr Leu Gln Ser Gln Met Glu Ser Leu Lys Lys
            580                 585                 590

Glu Arg Asp Ala Arg Pro Pro Ala Pro Ser Gly Gly Gly Gly Asp Gly
        595                 600                 605

Gly Ala Arg Cys His Ala Val Glu Ile Glu Ala Lys Ile Leu Gly Leu
    610                 615                 620

Glu Ala Met Ile Arg Val Gln Cys His Lys Arg Asn His Pro Ala Ala
625                 630                 635                 640

Arg Leu Met Thr Ala Leu Arg Glu Leu Asp Leu Asp Val Tyr His Ala
                645                 650                 655

Ser Val Ser Val Val Lys Asp Leu Met Ile Gln Gln Val Ala Val Lys
            660                 665                 670

Met Ala Ser Arg Val Tyr Ser Gln Asp Gln Leu Asn Ala Ala Leu Tyr
        675                 680                 685

Thr Arg Ile Ala Glu Pro Gly Thr Ala Ala Arg
    690                 695
```

<210> 25
<211> 2163
<212> DNA
<213> Rheum australe

<400> 25

```
atgatgaacc tctggagttc cgatgataac gttgctttcg ccgacgcttt tatgtccttg      60
gactcttcct ccgccgccgc ctggcctccg gcgccttcct cccaccaata caaccaccag     120
tacaacctcc aacaccagca acaactgcag catcaaccac aacaacaaca acaacaacaa     180
cagatggcca attcggccgg cggtggttgg caacaagcga tggccggcgg cgctcagatg     240
aatccggcgc agatgatgaa ccaggacagc ctacagcagc ggctgcaggc gctgatcgac     300
```

```
gacgccaggg agagctggac ctacgccatc ttctggcagt gcaacgtcga gcccaccggc     360
cagtctctcc ttggctgggg cgacggatac tataagggcg atgactccgc caataagaac     420
gcctcctccg ccgcccccgc cgctggatcc aggccgccga agaaccccgc agaacaggag     480
cacaggagga gagtcctccg ggaactcaat tccctgatct ccggctcctc ttcccctcaa     540
aacgacgccg ttgacgacga cgtcaccgac accgagtggt tcttccttat ctccatgacg     600
caggctttcc ccttcggcgt cgatctcccc ggccaagcca tcctcggatc caaccctatc     660
tgggcgtacg gctccgaccg tctcgccggc tccccgtggg atcgggccag acaaggggct     720
gcgttcgggt tgcagacgat cgtctgcatc cccagcggca ccggcgtcct cgaattaggc     780
tccaccgaac tcgtcttcaa cagttccgtt ttgatgaata aggttagggt tttgttcaat     840
ttcggatctg gagatgcgag tctcctgacc gccgcagcct cctcctccgc ccctgccgcc     900
gctcctccac cgccgatctc aacggcgacg accgatgagg cggagaacga ccccgccgcg     960
ctgtggatca gcgatccgtc ttcctccgca gccgaggtga aggaggcatt gaaccctagg    1020
atcaccgtcc gtgagagctc gattccgatc ggatcgaatt cgattcccgt ccaccagccg    1080
gccaaaccgc cgcaattgga cgttcaaagc tcaatcggat tgacggagaa ttcaattggt    1140
atccatagcc agaagagtca caatcagcca ctccaacatc aaggattttt caccaaagag    1200
ttgaatttct ccgagtttgc gatgaagcca gaatccggag agatcctcaa ctttggggag    1260
agtaagagga attccctcgg caacggaaac ggattgaatt cgcaattcct tgtggaagag    1320
agcaacaaaa acatcatcag caagaagcga tctccgacct caagaggaag cgcagaggaa    1380
ggcatgcttt cattcacttc aagcgtggtc ttgccttctt ccatggcggt gaagtcaagc    1440
gccaccggcg caggggactc cgaccactct gacctggaag cttcggtggt gaaggaagcc    1500
gacagcagcc gggtggtgga ccccgagaag cggccccgaa agcgtggccg gaagccagca    1560
aacggcaggg aggagcccct gaaccatgtc gaagcggaga ggcagaggag ggagaagctc    1620
aaccagagat tctacgccct cagggcggtt gtccccaatg tgtcaaaaat ggacaaagca    1680
tcactcttag gagacgcaat ctccttcatt aacgagctaa aatcgaagct ccaaaacgtg    1740
gaatcagaga aggaaacatt gctgagccag gtggagtgtt tgaagacaga ggtgttagca    1800
agcagagatc atcaatcaag gtcctccaat ggaggaggag gagtacagaa ccaccaccat    1860
ccaagcctag aacaggacat gaatatgctg aatggaagct gtaagcagtc agatttggac    1920
gtggacgtca agatcattgg aagggatgcc atggttagag tgaactgcag caagagcaac    1980
cacccagctg caaggctaat ggtggcattg aaggagcttg acttggaagt tacgcacgcg    2040
agcgtttcgg ttgtcaatga tctgatgatc cagcaggcga cggtgaggat ggggagccgg    2100
tattacagcc cggatcatct taggatggtc ttagaagcca aggtttctga tatcagggga    2160
tga                                                                  2163
```

<210> 26
<211> 720
<212> PRT

<213> Rheum australe

<400> 26

Met Met Asn Leu Trp Ser Ser Asp Asp Asn Val Ala Phe Ala Asp Ala
1               5                   10                  15

Phe Met Ser Leu Asp Ser Ser Ser Ala Ala Ala Trp Pro Pro Ala Pro
            20                  25                  30

Ser Ser His Gln Tyr Asn His Gln Tyr Asn Leu Gln His Gln Gln Gln
        35                  40                  45

Leu Gln His Gln Pro Gln Gln Gln Gln Gln Gln Gln Gln Met Ala Asn
    50                  55                  60

Ser Ala Gly Gly Gly Trp Gln Gln Ala Met Ala Gly Gly Ala Gln Met
65                  70                  75                  80

Asn Pro Ala Gln Met Met Asn Gln Asp Ser Leu Gln Gln Arg Leu Gln
                85                  90                  95

Ala Leu Ile Asp Asp Ala Arg Glu Ser Trp Thr Tyr Ala Ile Phe Trp
            100                 105                 110

Gln Cys Asn Val Glu Pro Thr Gly Gln Ser Leu Leu Gly Trp Gly Asp
        115                 120                 125

Gly Tyr Tyr Lys Gly Asp Asp Ser Ala Asn Lys Asn Ala Ser Ser Ala
    130                 135                 140

Ala Pro Ala Ala Gly Ser Arg Pro Pro Lys Asn Pro Ala Glu Gln Glu
145                 150                 155                 160

His Arg Arg Arg Val Leu Arg Glu Leu Asn Ser Leu Ile Ser Gly Ser
                165                 170                 175

Ser Ser Pro Gln Asn Asp Ala Val Asp Asp Asp Val Thr Asp Thr Glu
            180                 185                 190

Trp Phe Phe Leu Ile Ser Met Thr Gln Ala Phe Pro Phe Gly Val Asp
        195                 200                 205

Leu Pro Gly Gln Ala Ile Leu Gly Ser Asn Pro Ile Trp Ala Tyr Gly
    210                 215                 220

Ser Asp Arg Leu Ala Gly Ser Pro Trp Asp Arg Ala Arg Gln Gly Ala
225                 230                 235                 240

Ala Phe Gly Leu Gln Thr Ile Val Cys Ile Pro Ser Gly Thr Gly Val
                245                 250                 255

Leu Glu Leu Gly Ser Thr Glu Leu Val Phe Asn Ser Ser Val Leu Met

260 265 270

Asn Lys Val Arg Val Leu Phe Asn Phe Gly Ser Gly Asp Ala Ser Leu
275 280 285

Leu Thr Ala Ala Ala Ser Ser Ser Ala Pro Ala Ala Ala Pro Pro Pro
290 295 300

Pro Ile Ser Thr Ala Thr Thr Asp Glu Ala Glu Asn Asp Pro Ala Ala
305 310 315 320

Leu Trp Ile Ser Asp Pro Ser Ser Ser Ala Ala Glu Val Lys Glu Ala
325 330 335

Leu Asn Pro Arg Ile Thr Val Arg Glu Ser Ser Ile Pro Ile Gly Ser
340 345 350

Asn Ser Ile Pro Val His Gln Pro Ala Lys Pro Pro Gln Leu Asp Val
355 360 365

Gln Ser Ser Ile Gly Leu Thr Glu Asn Ser Ile Gly Ile His Ser Gln
370 375 380

Lys Ser His Asn Gln Pro Leu Gln His Gln Gly Phe Phe Thr Lys Glu
385 390 395 400

Leu Asn Phe Ser Glu Phe Ala Met Lys Pro Glu Ser Gly Glu Ile Leu
405 410 415

Asn Phe Gly Glu Ser Lys Arg Asn Ser Leu Gly Asn Gly Asn Gly Leu
420 425 430

Asn Ser Gln Phe Leu Val Glu Glu Ser Asn Lys Asn Ile Ile Ser Lys
435 440 445

Lys Arg Ser Pro Thr Ser Arg Gly Ser Ala Glu Glu Gly Met Leu Ser
450 455 460

Phe Thr Ser Ser Val Val Leu Pro Ser Ser Met Ala Val Lys Ser Ser
465 470 475 480

Ala Thr Gly Ala Gly Asp Ser Asp His Ser Asp Leu Glu Ala Ser Val
485 490 495

Val Lys Glu Ala Asp Ser Ser Arg Val Val Asp Pro Glu Lys Arg Pro
500 505 510

Arg Lys Arg Gly Arg Lys Pro Ala Asn Gly Arg Glu Glu Pro Leu Asn
515 520 525

His Val Glu Ala Glu Arg Gln Arg Arg Glu Lys Leu Asn Gln Arg Phe
530 535 540

```
Tyr Ala Leu Arg Ala Val Val Pro Asn Val Ser Lys Met Asp Lys Ala
545                 550                 555                 560

Ser Leu Leu Gly Asp Ala Ile Ser Phe Ile Asn Glu Leu Lys Ser Lys
                565                 570                 575

Leu Gln Asn Val Glu Ser Glu Lys Glu Thr Leu Leu Ser Gln Val Glu
            580                 585                 590

Cys Leu Lys Thr Glu Val Leu Ala Ser Arg Asp His Gln Ser Arg Ser
        595                 600                 605

Ser Asn Gly Gly Gly Gly Val Gln Asn His His His Pro Ser Leu Glu
    610                 615                 620

Gln Asp Met Asn Met Leu Asn Gly Ser Cys Lys Gln Ser Asp Leu Asp
625                 630                 635                 640

Val Asp Val Lys Ile Ile Gly Arg Asp Ala Met Val Arg Val Asn Cys
                645                 650                 655

Ser Lys Ser Asn His Pro Ala Ala Arg Leu Met Val Ala Leu Lys Glu
            660                 665                 670

Leu Asp Leu Glu Val Thr His Ala Ser Val Ser Val Val Asn Asp Leu
        675                 680                 685

Met Ile Gln Gln Ala Thr Val Arg Met Gly Ser Arg Tyr Tyr Ser Pro
    690                 695                 700

Asp His Leu Arg Met Val Leu Glu Ala Lys Val Ser Asp Ile Arg Gly
705                 710                 715                 720
```

<210> 27
<211> 2109
<212> DNA
<213> Zea mays

<400> 27

```
ggcacgaggg acgacaacgc ctccatgatg gaggccttca tggcttctgc cgacctcccc      60
accttcccct ggggagcgcc ggccgggggc ggcaactcgt cggccgccgc ggcgtcgccg     120
ccgccgcaga tgccagcggc gatggctccc gggttcaacc aggacacgct gcagcagagg     180
ctacaagcca tgatagaggg gtcgagggag acctggacct acgccatctt ctggcagtcc     240
tcccttgact ccgccaccgg cgcctcgctg ctcggctggg gggacggcta ctacaagggc     300
tgcgacgaag acaagcgcaa gcagaagccg ctcacgccct ccgcccaggc cgagcaggag     360
caccgcaagc gcgtgctccg cgagctcaac tccctcatct ccggcgcggc ggcggctccc     420
gacgaggccg tcgaggagga ggtcaccgat accgagtggt tcttcctcgt ctccatgacg     480
cagtcgtttc tcaacggttc gggcctcccc gggcaggcgc tcttcgccgg gcagcccaca     540
```

```
tggatcgcct ctggcctctc ctccgcgccg tgcgagcgcg cgcgtcaggc ctacaacttc     600
ggcctccgca ccatggtatg cttccccgtc ggcaccgggg tgctcgagct cggctccacc     660
gacgtcgtgt tcaagaccgc cgagagcatg gctaagatcc gctcgctctt tgggggcgga     720
gcagggggtg gctcgtggcc gcccgtgcag cctcaggcgc cgtcgtcgca gcagcccgcc     780
gccggagccg accacgcgga gacggacccg tccatgctat ggctcgccga cgcgccggtc     840
atggacatca aggattcctt gtcgcacccg tcggccgaga tctccgtctc caagcctccg     900
ccgcatccgc cgcagatcca ttttgagaac gggagcacga gcacgctcac ggagaacccc     960
agcccctccg tgcacgcgcc gccacccccg ccggcgccgg ctgctccaca gcagcggcag    1020
caccagcacc agaatcaggc gcaccagggc ccgttccgcc gggagctcaa tttctcggac    1080
ttcgcgtcca ctccttcctt ggcggcgacc ccgccgttct tcaagcccga gtccggcgag    1140
atcctcagct tcggcgccga cagcaacgcc cggaggaacc cgtcgccggt acctcccgcc    1200
gccaccgcca gcctcaccac cgctcccggg agcctcttct cgcagcacac ggcgaccatg    1260
acggccgccg cggcgaacga cgcgaagaac aacaacaagc gctcaatgga ggccacctcc    1320
cgggcgagca acaccaacca ccacccggcg gcgacggcga acgagggcat gctgtccttc    1380
tcgtcggcgc cgactacgcg gccgtcgacg ggcacgggcg cgccggccaa gtcggagtcc    1440
gaccactcgg acctggacgc gtctgtgcgc gaggtggaga gcagccgcgt ggtggcgccg    1500
ccaccggagg cggagaagcg gccgcgaaag cgcgggcgga agcccgcgaa cgggcgcgag    1560
gagccgctga accacgtgga ggcggagcgg cagcggcggg agaagctgaa ccagcgcttc    1620
tacgcgctgc gcgccgtggt gcccaacgtg tcgaagatgg acaaggcgtc gctgctcggc    1680
gacgccatct cctacatcaa cgagctccgg ggcaagctga cgtcgctgga gaccgacaag    1740
gagacgctgc agacccaggt ggaggcgctc aagaaggagc gcgacgcgcg gccgccctcg    1800
cactccgctg ggctcggcgg gcacgacggc gggccgcgct gccacgcggt ggagatcgac    1860
gccaagatcc tggggctgga ggccatgatc cgcgtgcagt gccacaagcg caaccacccg    1920
tcggcgcggc tgatgacagc gctccgcgag ctcgacctgg acgtgtacca cgccagcgtg    1980
tccgtcgtca aggacctcat gatccagcag gtcgccgtca agatggccag ccgcgtgtac    2040
acgcaggacc agctcagcgc cgcgctctac agccgcctcg cggagcccgg gtctgccatg    2100
ggcaggtaa                                                            2109
```

<210> 28
<211> 702
<212> PRT
<213> Zea mays

<400> 28

```
Gly Thr Arg Asp Asp Asn Ala Ser Met Met Glu Ala Phe Met Ala Ser
1               5                   10                  15

Ala Asp Leu Pro Thr Phe Pro Trp Gly Ala Pro Ala Gly Gly Gly Asn
            20                  25                  30
```

Ser Ser Ala Ala Ala Ala Ser Pro Pro Pro Gln Met Pro Ala Ala Met
35 40 45

Ala Pro Gly Phe Asn Gln Asp Thr Leu Gln Gln Arg Leu Gln Ala Met
50 55 60

Ile Glu Gly Ser Arg Glu Thr Trp Thr Tyr Ala Ile Phe Trp Gln Ser
65 70 75 80

Ser Leu Asp Ser Ala Thr Gly Ala Ser Leu Leu Gly Trp Gly Asp Gly
85 90 95

Tyr Tyr Lys Gly Cys Asp Glu Asp Lys Arg Lys Gln Lys Pro Leu Thr
100 105 110

Pro Ser Ala Gln Ala Glu Gln Glu His Arg Lys Arg Val Leu Arg Glu
115 120 125

Leu Asn Ser Leu Ile Ser Gly Ala Ala Ala Ala Pro Asp Glu Ala Val
130 135 140

Glu Glu Glu Val Thr Asp Thr Glu Trp Phe Phe Leu Val Ser Met Thr
145 150 155 160

Gln Ser Phe Leu Asn Gly Ser Gly Leu Pro Gly Gln Ala Leu Phe Ala
165 170 175

Gly Gln Pro Thr Trp Ile Ala Ser Gly Leu Ser Ser Ala Pro Cys Glu
180 185 190

Arg Ala Arg Gln Ala Tyr Asn Phe Gly Leu Arg Thr Met Val Cys Phe
195 200 205

Pro Val Gly Thr Gly Val Leu Glu Leu Gly Ser Thr Asp Val Val Phe
210 215 220

Lys Thr Ala Glu Ser Met Ala Lys Ile Arg Ser Leu Phe Gly Gly Gly
225 230 235 240

Ala Gly Gly Gly Ser Trp Pro Pro Val Gln Pro Gln Ala Pro Ser Ser
245 250 255

Gln Gln Pro Ala Ala Gly Ala Asp His Ala Glu Thr Asp Pro Ser Met
260 265 270

Leu Trp Leu Ala Asp Ala Pro Val Met Asp Ile Lys Asp Ser Leu Ser
275 280 285

His Pro Ser Ala Glu Ile Ser Val Ser Lys Pro Pro Pro His Pro Pro
290 295 300

Gln Ile His Phe Glu Asn Gly Ser Thr Ser Thr Leu Thr Glu Asn Pro
305                 310                 315                 320

Ser Pro Ser Val His Ala Pro Pro Pro Pro Pro Ala Pro Ala Ala Pro
                325                 330                 335

Gln Gln Arg Gln His Gln His Gln Asn Gln Ala His Gln Gly Pro Phe
            340                 345                 350

Arg Arg Glu Leu Asn Phe Ser Asp Phe Ala Ser Thr Pro Ser Leu Ala
        355                 360                 365

Ala Thr Pro Pro Phe Phe Lys Pro Glu Ser Gly Glu Ile Leu Ser Phe
    370                 375                 380

Gly Ala Asp Ser Asn Ala Arg Arg Asn Pro Ser Pro Val Pro Pro Ala
385                 390                 395                 400

Ala Thr Ala Ser Leu Thr Thr Ala Pro Gly Ser Leu Phe Ser Gln His
                405                 410                 415

Thr Ala Thr Met Thr Ala Ala Ala Ala Asn Asp Ala Lys Asn Asn Asn
            420                 425                 430

Lys Arg Ser Met Glu Ala Thr Ser Arg Ala Ser Asn Thr Asn His His
        435                 440                 445

Pro Ala Ala Thr Ala Asn Glu Gly Met Leu Ser Phe Ser Ser Ala Pro
    450                 455                 460

Thr Thr Arg Pro Ser Thr Gly Thr Gly Ala Pro Ala Lys Ser Glu Ser
465                 470                 475                 480

Asp His Ser Asp Leu Asp Ala Ser Val Arg Glu Val Glu Ser Ser Arg
                485                 490                 495

Val Val Ala Pro Pro Pro Glu Ala Glu Lys Arg Pro Arg Lys Arg Gly
            500                 505                 510

Arg Lys Pro Ala Asn Gly Arg Glu Glu Pro Leu Asn His Val Glu Ala
        515                 520                 525

Glu Arg Gln Arg Arg Glu Lys Leu Asn Gln Arg Phe Tyr Ala Leu Arg
    530                 535                 540

Ala Val Val Pro Asn Val Ser Lys Met Asp Lys Ala Ser Leu Leu Gly
545                 550                 555                 560

Asp Ala Ile Ser Tyr Ile Asn Glu Leu Arg Gly Lys Leu Thr Ser Leu
                565                 570                 575

Glu Thr Asp Lys Glu Thr Leu Gln Thr Gln Val Glu Ala Leu Lys Lys
580 585 590

Glu Arg Asp Ala Arg Pro Pro Ser His Ser Ala Gly Leu Gly Gly His
595 600 605

Asp Gly Gly Pro Arg Cys His Ala Val Glu Ile Asp Ala Lys Ile Leu
610 615 620

Gly Leu Glu Ala Met Ile Arg Val Gln Cys His Lys Arg Asn His Pro
625 630 635 640

Ser Ala Arg Leu Met Thr Ala Leu Arg Glu Leu Asp Leu Asp Val Tyr
645 650 655

His Ala Ser Val Ser Val Val Lys Asp Leu Met Ile Gln Gln Val Ala
660 665 670

Val Lys Met Ala Ser Arg Val Tyr Thr Gln Asp Gln Leu Ser Ala Ala
675 680 685

Leu Tyr Ser Arg Leu Ala Glu Pro Gly Ser Ala Met Gly Arg
690 695 700

<210> 29
<211> 1731
<212> DNA
<213> Physcomitrella patens

<400> 29
atgtcgcagt tattgaatgc gtgggaagta gcggactcag ctatgatcga ggcgttcatg 60

ggaacggggt ataactgcgt ggaaggcttc gaagtgcagg atgatccgga cgggcagctc 120

cacttgaacg agtcggtgtt gctgcggagg ctgcactcgc tggtcgaaga gagtaccgtg 180

gattggacgt atgcaatctt ttggcagctg tcagccttac gtgaagggga gatgatgctc 240

ggttggggtg atgggtactt caggagtgct aaggagaatg aaatcaacga cgcgaggaac 300

atgaagggtg gttctcaaga ggaggatcaa cagatgaggc gaaaggtgtt gcgagagctg 360

caggctctag tcaatggctc tgaagatgat gttagcgact atgtcacgga tacggaatgg 420

ttttaccttg tttcgatgtc tcattcttat gcagccggag tggggactcc tggacgagca 480

ttagcatctg acagacctgt gtggctaatt ggagcgaaca aagctcctga taataactgc 540

agtcgtgttc aattggctaa ggtacacagt tcaatgatcc ttcagacaat cctttgtatt 600

ccgtcgaaat ctggtgtcgt tgagcttgga tcgacagatc tggcaaaaag ctgggaagtt 660

gttcagaatg tcaagatggt tttgatgaa ccaatgatgt gggcagccca tgaaatccaa 720

gcggtggcgc attccttgcc tttgagtagc gacgcgacta gtatgcgacc gtcaagccca 780

agtcttatgt caatagcaac aataagtgct tcaatttcaa atgcaagtca gattggcaga 840

tgttcaaatc caagtcaaga tcatgaagca cattttgttg gaaggaagaa tccaccctcg 900

tcaagacccc cgatgaggtt ctacaaacca cgggtggaag aattgagctc ggacactcca 960

gaaacaaact tggttgataa taaatatatt gtagggaagt cagtatcttt ccaccacctt 1020

tctaagatag gtcagagagg aatgcctgga cctcctacca ctgcaaatag gttaccatgt 1080

ttcgctccgt ccgaggtaga ttgtcatgaa tcagaagccg acgtttccgt caaagaaaat 1140

gttgtggaat caagtacaaa tctcgagcct aaaccacgca aaagagggcg caagccggca 1200

aacgataggg aagagccact aaatcatgtg caagctgaac ggcagcggcg agaaaaactc 1260

aatcagaaat tttatgctct acggtctgta gtgccaaacg tgtccaagat ggataaggca 1320

tccttattag aagatgccat tacctacatt aatgagcttc aagaaaagct acaaaaagca 1380

gaggctgaat tgaaggtttt ccaaaggcaa gtgcttgcat caactggcga atctaaaaaa 1440

cctaatccat ctcgcaggga ttcgaccgaa agttctgatg aagaacgttt cagacttcaa 1500

gagagtggtc agagatcggc acctcttgtt cacacttctg aaaataaacc tgtaattagt 1560

gtttttgttc ttggagaaga agctatgatt cgagtttatt gcacaagaca ctctaacttt 1620

atagttcata tgatgtcggc attggaaaag ctacgcttag aggttataca ttcaaacact 1680

tcatccatga aggatatgct tcttcacgtc gtgattgtca aggtgcgctg a 1731

<210> 30
<211> 576
<212> PRT
<213> Physcomitrella patens

<400> 30

```
Met Ser Gln Leu Leu Asn Ala Trp Glu Val Ala Asp Ser Ala Met Ile
1               5                   10                  15

Glu Ala Phe Met Gly Thr Gly Tyr Asn Cys Val Glu Gly Phe Glu Val
            20                  25                  30

Gln Asp Asp Pro Asp Gly Gln Leu His Leu Asn Glu Ser Val Leu Leu
        35                  40                  45

Arg Arg Leu His Ser Leu Val Glu Glu Ser Thr Val Asp Trp Thr Tyr
    50                  55                  60

Ala Ile Phe Trp Gln Leu Ser Ala Leu Arg Glu Gly Glu Met Met Leu
65                  70                  75                  80

Gly Trp Gly Asp Gly Tyr Phe Arg Ser Ala Lys Glu Asn Glu Ile Asn
                85                  90                  95

Asp Ala Arg Asn Met Lys Gly Gly Ser Gln Glu Glu Asp Gln Gln Met
            100                 105                 110

Arg Arg Lys Val Leu Arg Glu Leu Gln Ala Leu Val Asn Gly Ser Glu
        115                 120                 125

Asp Asp Val Ser Asp Tyr Val Thr Asp Thr Glu Trp Phe Tyr Leu Val
    130                 135                 140
```

```
Ser Met Ser His Ser Tyr Ala Ala Gly Val Gly Thr Pro Gly Arg Ala
145                 150                 155                 160

Leu Ala Ser Asp Arg Pro Val Trp Leu Ile Gly Ala Asn Lys Ala Pro
                165                 170                 175

Asp Asn Asn Cys Ser Arg Val Gln Leu Ala Lys Val His Ser Ser Met
            180                 185                 190

Ile Leu Gln Thr Ile Leu Cys Ile Pro Ser Lys Ser Gly Val Val Glu
        195                 200                 205

Leu Gly Ser Thr Asp Leu Ala Lys Ser Trp Glu Val Val Gln Asn Val
    210                 215                 220

Lys Met Val Phe Asp Glu Pro Met Met Trp Ala Ala His Glu Ile Gln
225                 230                 235                 240

Ala Val Ala His Ser Leu Pro Leu Ser Ser Asp Ala Thr Ser Met Arg
                245                 250                 255

Pro Ser Ser Pro Ser Leu Met Ser Ile Ala Thr Ile Ser Ala Ser Ile
            260                 265                 270

Ser Asn Ala Ser Gln Ile Gly Arg Cys Ser Asn Pro Ser Gln Asp His
        275                 280                 285

Glu Ala His Phe Val Gly Arg Lys Asn Pro Pro Ser Ser Arg Pro Pro
    290                 295                 300

Met Arg Phe Tyr Lys Pro Arg Val Glu Glu Leu Ser Ser Asp Thr Pro
305                 310                 315                 320

Glu Thr Asn Leu Val Asp Asn Lys Tyr Ile Val Gly Lys Ser Val Ser
                325                 330                 335

Phe His His Leu Ser Lys Ile Gly Gln Arg Gly Met Pro Gly Pro Pro
            340                 345                 350

Thr Thr Ala Asn Arg Leu Pro Cys Phe Ala Pro Ser Glu Val Asp Cys
        355                 360                 365

His Glu Ser Glu Ala Asp Val Ser Val Lys Glu Asn Val Val Glu Ser
    370                 375                 380

Ser Thr Asn Leu Glu Pro Lys Pro Arg Lys Arg Gly Arg Lys Pro Ala
385                 390                 395                 400

Asn Asp Arg Glu Glu Pro Leu Asn His Val Gln Ala Glu Arg Gln Arg
                405                 410                 415
```

```
Arg Glu Lys Leu Asn Gln Lys Phe Tyr Ala Leu Arg Ser Val Val Pro
            420                 425                 430

Asn Val Ser Lys Met Asp Lys Ala Ser Leu Leu Glu Asp Ala Ile Thr
        435                 440                 445

Tyr Ile Asn Glu Leu Gln Glu Lys Leu Gln Lys Ala Glu Ala Glu Leu
    450                 455                 460

Lys Val Phe Gln Arg Gln Val Leu Ala Ser Thr Gly Glu Ser Lys Lys
465                 470                 475                 480

Pro Asn Pro Ser Arg Arg Asp Ser Thr Glu Ser Ser Asp Glu Glu Arg
                485                 490                 495

Phe Arg Leu Gln Glu Ser Gly Gln Arg Ser Ala Pro Leu Val His Thr
            500                 505                 510

Ser Glu Asn Lys Pro Val Ile Ser Val Phe Val Leu Gly Glu Glu Ala
        515                 520                 525

Met Ile Arg Val Tyr Cys Thr Arg His Ser Asn Phe Ile Val His Met
    530                 535                 540

Met Ser Ala Leu Glu Lys Leu Arg Leu Glu Val Ile His Ser Asn Thr
545                 550                 555                 560

Ser Ser Met Lys Asp Met Leu Leu His Val Val Ile Val Lys Val Arg
                565                 570                 575
```

<210> 31
<211> 981
<212> DNA
<213> Lycopersicon esculentum

<400> 31

```
agtggaaatg gtcagagttg ttacaatcag caacaacaga agaatcctcc tcagcaacaa      60
acacaaggat tcttcacgag ggagttgaat ttttcggaat tcggtttcga tggaagtagt     120
aataggaatg gaaattcatc ggtttcttgc aagcctgaat caggagaaat cttgaatttt     180
ggtgatagta ctaaaaaaag tgcttccagt gccaatgtga acttgtttac aggtcagtcc     240
caattttggg gcctggggga ggagaataat aacaagaacc aagaaaagat cagctacttc     300
aggggaagca atgaagaagg aatgctttca tttgtttcag gtacagtttg cttcttcggg     360
catgaagtca ggtggaggcg gaggcaagac tctgaacatt cagatctcga ggcttcagtg     420
gtgaaagaag ctgatagtag tagagtggta gagcctgaaa agaggccaag gaagcgaggt     480
agaaagccag cgaatggacg ggaggagcca ttgaatcacg tcgaggcaga gaggcaaagg     540
agggagaaat tgaaccaaag attcttctct cttagagctg ttgtaccaaa tgtgtctaag     600
atggacaagg catcactcct tggagatgct atttcctata taaacgagtt gaaatcgaag     660
```

```
cttcaaaata cagagtcaga taaagaagac ttgaagagcc aaatagaaga tttaaagaaa     720
gaatcaaggc gccccggtcc tcctccacca ccaaatcaag atctcaagat gtctagccac     780
actggaggca agattgtaga cgtggatata gacgttaaga tcatcggatg ggatgcaatg     840
attcgtatac aatgtaataa aaagaatcat ccagcgaagg ctaatggcag cgctcatgga     900
attagaccta gacgtgcatc atgccagtgt ttcagttgtc aacgatttga tgatccaaca     960
agccacagtg aaaatgggta g                                               981
```

<210> 32
<211> 326
<212> PRT
<213> Lycopersicon esculentum

<400> 32

```
Ser Gly Asn Gly Gln Ser Cys Tyr Asn Gln Gln Gln Gln Lys Asn Pro
1               5                   10                  15

Pro Gln Gln Gln Thr Gln Gly Phe Phe Thr Arg Glu Leu Asn Phe Ser
            20                  25                  30

Glu Phe Gly Phe Asp Gly Ser Ser Asn Arg Asn Gly Asn Ser Ser Val
        35                  40                  45

Ser Cys Lys Pro Glu Ser Gly Glu Ile Leu Asn Phe Gly Asp Ser Thr
    50                  55                  60

Lys Lys Ser Ala Ser Ser Ala Asn Val Asn Leu Phe Thr Gly Gln Ser
65                  70                  75                  80

Gln Phe Trp Gly Leu Gly Glu Glu Asn Asn Asn Lys Asn Gln Glu Lys
                85                  90                  95

Ile Ser Tyr Phe Arg Gly Ser Asn Glu Glu Gly Met Leu Ser Phe Val
            100                 105                 110

Ser Gly Thr Val Cys Phe Phe Gly His Glu Val Arg Trp Arg Arg Arg
        115                 120                 125

Gln Asp Ser Glu His Ser Asp Leu Glu Ala Ser Val Val Lys Glu Ala
    130                 135                 140

Asp Ser Ser Arg Val Val Glu Pro Glu Lys Arg Pro Arg Lys Arg Gly
145                 150                 155                 160

Arg Lys Pro Ala Asn Gly Arg Glu Glu Pro Leu Asn His Val Glu Ala
                165                 170                 175

Glu Arg Gln Arg Arg Glu Lys Leu Asn Gln Arg Phe Phe Ser Leu Arg
            180                 185                 190

Ala Val Val Pro Asn Val Ser Lys Met Asp Lys Ala Ser Leu Leu Gly
```

195 200 205

Asp Ala Ile Ser Tyr Ile Asn Glu Leu Lys Ser Lys Leu Gln Asn Thr
210 215 220

Glu Ser Asp Lys Glu Asp Leu Lys Ser Gln Ile Glu Asp Leu Lys Lys
225 230 235 240

Glu Ser Arg Arg Pro Gly Pro Pro Pro Pro Pro Asn Gln Asp Leu Lys
245 250 255

Met Ser Ser His Thr Gly Gly Lys Ile Val Asp Val Asp Ile Asp Val
260 265 270

Lys Ile Ile Gly Trp Asp Ala Met Ile Arg Ile Gln Cys Asn Lys Lys
275 280 285

Asn His Pro Ala Lys Ala Asn Gly Ser Ala His Gly Ile Arg Pro Arg
290 295 300

Arg Ala Ser Cys Gln Cys Phe Ser Cys Gln Arg Phe Asp Asp Pro Thr
305 310 315 320

Ser His Ser Glu Asn Gly
325

<210> 33
<211> 1476
<212> DNA
<213> Populus trichocarpa

<400> 33
atggaggaac tcattatttc tccatcttca tcatcttcac cggtctcctt atcccaagaa 60
accccaccga ctctccaaca aaggcttcaa ttcatagtcc aaaaccaacc agattggtgg 120
tcttatgcca tattttggca aacatcaaac gatgacagcg gccgaatctt cctaggctgg 180
ggtgatggcc attttcaagg ctctaaagat acttctccca aaccaaacac cttcagcaat 240
agccgcatga cgatatcaaa ctccgagagg aaaagggtca tgatgaaggg aatccaatcc 300
ctgatcggtg aatgtcacga tcttgatatg tctctgatgg atggtaacga tgctaccgac 360
tctgagtggt tctatgtcat gtcccttact cgatctttct cacctggaga tggcatcctt 420
ggcaaagctt atacaactgg ttctttgatt tggttaactg gtggccatga acttcaattc 480
tacaactgtg aaagagtcaa agaagcacaa atgcatggca ttgagaccct ggtttgcata 540
cctacatcgt gtggggttct tgaattagga tcctcttctg tgatcagaga aaattggggt 600
cttgttcaac aagccaagtc tctttttggg tcagatctta gcgcttattt ggtgcccaaa 660
ggccctaata actccagtga agaaccgacc cagtttcttg acaggagcat ttcttttgcg 720
gatatgggca taatagctgg acttcaagaa gattgtgcag ttgatcgtga acagaagaac 780
gctcgtgaaa cagaagaagc aaataaacgt aacgctaata aaccaggcct gtcttatttg 840

aactcagagc attcagactc ggactttcct ctacttgcta tgcatatgga gaaaagaatt 900
ccaaagaaaa gagggagaaa gcctggcctg ggcagagacg ccccactgaa ccatgtagag 960
gctgagaggc agcggagaga gaagttgaac caccggtttt acgcgctgcg tgcggtggtc 1020
ccaaacgtgt ctagaatgga caaagcatca ctattatctg atgctgtatc ctacatcaat 1080
gaattgaagg cgaaggttga tgaattagag tcacaactag aaagggaatc caagaaagtg 1140
aaattggaag ttgccgataa tttggacaat caaagcacca ccacttctgt ggaccaatca 1200
gcctgcaggc cgaatagtgc tggtggcgct gggcttgcac ttgaagttga gatcaagttt 1260
gtgggtaatg atgcaatgat tagagtccaa tcggagaatg tgaactatcc agcttccagg 1320
ttaatgtgtg cgctccgtga actggagttt caggttcacc atgctagtat gtcctgtgtt 1380
aacgagctta tgctccaaga tgtggtagtt agggttcctg atggactgag aaccgaagag 1440
gccttgaaat ctgctcttct tggaagacta gaataa 1476

<210> 34
<211> 491
<212> PRT
<213> Populus trichocarpa

<400> 34

Met Glu Glu Leu Ile Ile Ser Pro Ser Ser Ser Ser Ser Pro Val Ser
1 5 10 15

Leu Ser Gln Glu Thr Pro Pro Thr Leu Gln Gln Arg Leu Gln Phe Ile
20 25 30

Val Gln Asn Gln Pro Asp Trp Trp Ser Tyr Ala Ile Phe Trp Gln Thr
35 40 45

Ser Asn Asp Asp Ser Gly Arg Ile Phe Leu Gly Trp Gly Asp Gly His
50 55 60

Phe Gln Gly Ser Lys Asp Thr Ser Pro Lys Pro Asn Thr Phe Ser Asn
65 70 75 80

Ser Arg Met Thr Ile Ser Asn Ser Glu Arg Lys Arg Val Met Met Lys
85 90 95

Gly Ile Gln Ser Leu Ile Gly Glu Cys His Asp Leu Asp Met Ser Leu
100 105 110

Met Asp Gly Asn Asp Ala Thr Asp Ser Glu Trp Phe Tyr Val Met Ser
115 120 125

Leu Thr Arg Ser Phe Ser Pro Gly Asp Gly Ile Leu Gly Lys Ala Tyr
130 135 140

Thr Thr Gly Ser Leu Ile Trp Leu Thr Gly Gly His Glu Leu Gln Phe
145 150 155 160

Tyr Asn Cys Glu Arg Val Lys Glu Ala Gln Met His Gly Ile Glu Thr
165 170 175

Leu Val Cys Ile Pro Thr Ser Cys Gly Val Leu Glu Leu Gly Ser Ser
180 185 190

Ser Val Ile Arg Glu Asn Trp Gly Leu Val Gln Gln Ala Lys Ser Leu
195 200 205

Phe Gly Ser Asp Leu Ser Ala Tyr Leu Val Pro Lys Gly Pro Asn Asn
210 215 220

Ser Ser Glu Glu Pro Thr Gln Phe Leu Asp Arg Ser Ile Ser Phe Ala
225 230 235 240

Asp Met Gly Ile Ile Ala Gly Leu Gln Glu Asp Cys Ala Val Asp Arg
245 250 255

Glu Gln Lys Asn Ala Arg Glu Thr Glu Glu Ala Asn Lys Arg Asn Ala
260 265 270

Asn Lys Pro Gly Leu Ser Tyr Leu Asn Ser Glu His Ser Asp Ser Asp
275 280 285

Phe Pro Leu Leu Ala Met His Met Glu Lys Arg Ile Pro Lys Lys Arg
290 295 300

Gly Arg Lys Pro Gly Leu Gly Arg Asp Ala Pro Leu Asn His Val Glu
305 310 315 320

Ala Glu Arg Gln Arg Arg Glu Lys Leu Asn His Arg Phe Tyr Ala Leu
325 330 335

Arg Ala Val Val Pro Asn Val Ser Arg Met Asp Lys Ala Ser Leu Leu
340 345 350

Ser Asp Ala Val Ser Tyr Ile Asn Glu Leu Lys Ala Lys Val Asp Glu
355 360 365

Leu Glu Ser Gln Leu Glu Arg Glu Ser Lys Lys Val Lys Leu Glu Val
370 375 380

Ala Asp Asn Leu Asp Asn Gln Ser Thr Thr Thr Ser Val Asp Gln Ser
385 390 395 400

Ala Cys Arg Pro Asn Ser Ala Gly Gly Ala Gly Leu Ala Leu Glu Val
405 410 415

Glu Ile Lys Phe Val Gly Asn Asp Ala Met Ile Arg Val Gln Ser Glu
420 425 430

Asn Val Asn Tyr Pro Ala Ser Arg Leu Met Cys Ala Leu Arg Glu Leu
435 440 445

Glu Phe Gln Val His His Ala Ser Met Ser Cys Val Asn Glu Leu Met
450 455 460

Leu Gln Asp Val Val Val Arg Val Pro Asp Gly Leu Arg Thr Glu Glu
465 470 475 480

Ala Leu Lys Ser Ala Leu Leu Gly Arg Leu Glu
485 490

<210> 35
<211> 878
<212> DNA
<213> Triticum aestivum

<400> 35
atgaaaggcc gtgggaacag aggactgctg gtggagggag ctcattgctt cccaatgtcc 60

agaaaggatt gcagagtttc acttggagtc aggcccgggg cctgaattct caccagcaga 120

agtttggcaa tggtatactg atagtgagta atgaagctac acacggcaac aatagaaccg 180

cggacagctc cactacaaca cagtttcagc ttcagaaagc acctcagctc cagaaactac 240

cacttcttca gaaaccacca cagctagtga agccgctgca gatggtcaac cagcaacagc 300

tgcagccaca ggcgcctagg caaatagatt ttagtgcagg gaccagttcc aagtctggtg 360

tcctggttac aagagcagct gttcttgatg gagatagttc agaggtgaat ggcttgtgta 420

aagaggaagg gacaacacct gtcatagagg accgacggcc aaggaagagg ggaagaaagc 480

ctgcgaatgg gagagaggag ccgctgaatc atgttgaggc tgagcgtcaa aggagggaga 540

agctcaacca gcggttctat gcgttgagag ccgttgtgcc caacatctcg aaaatggaca 600

aggcttccct gctgggcgat gcaatagcat acatcactga ccttcagaag aagctcaaag 660

atatggagac ggagagagaa cgatttcttg agtctggtat ggtggatcca agggagcgag 720

cccctagacc agaggttgac atccaggtgg tgcaagacga ggttctggtt cgagttatgt 780

ctccattgga gaaccatccg gtaaagaagg tctttgaagc gtttgaagag gcggacgtcc 840

gggtagggga gtcgaaactc acaggcaaca atggaacg 878

<210> 36
<211> 292
<212> PRT
<213> Triticum aestivum

<400> 36

Glu Arg Pro Trp Glu Gln Arg Thr Ala Gly Gly Gly Ser Ser Leu Leu
1 5 10 15

Pro Asn Val Gln Lys Gly Leu Gln Ser Phe Thr Trp Ser Gln Ala Arg
20 25 30

Gly Leu Asn Ser His Gln Gln Lys Phe Gly Asn Gly Ile Leu Ile Val

35 40 45

Ser Asn Glu Ala Thr His Gly Asn Asn Arg Thr Ala Asp Ser Ser Thr
50 55 60

Thr Thr Gln Phe Gln Leu Gln Lys Ala Pro Gln Leu Gln Lys Leu Pro
65 70 75 80

Leu Leu Gln Lys Pro Pro Gln Leu Val Lys Pro Leu Gln Met Val Asn
85 90 95

Gln Gln Gln Leu Gln Pro Gln Ala Pro Arg Gln Ile Asp Phe Ser Ala
100 105 110

Gly Thr Ser Ser Lys Ser Gly Val Leu Val Thr Arg Ala Ala Val Leu
115 120 125

Asp Gly Asp Ser Ser Glu Val Asn Gly Leu Cys Lys Glu Glu Gly Thr
130 135 140

Thr Pro Val Ile Glu Asp Arg Arg Pro Arg Lys Arg Gly Arg Lys Pro
145 150 155 160

Ala Asn Gly Arg Glu Glu Pro Leu Asn His Val Glu Ala Glu Arg Gln
165 170 175

Arg Arg Glu Lys Leu Asn Gln Arg Phe Tyr Ala Leu Arg Ala Val Val
180 185 190

Pro Asn Ile Ser Lys Met Asp Lys Ala Ser Leu Leu Gly Asp Ala Ile
195 200 205

Ala Tyr Ile Thr Asp Leu Gln Lys Lys Leu Lys Asp Met Glu Thr Glu
210 215 220

Arg Glu Arg Phe Leu Glu Ser Gly Met Val Asp Pro Arg Glu Arg Ala
225 230 235 240

Pro Arg Pro Glu Val Asp Ile Gln Val Val Gln Asp Glu Val Leu Val
245 250 255

Arg Val Met Ser Pro Leu Glu Asn His Pro Val Lys Lys Val Phe Glu
260 265 270

Ala Phe Glu Glu Ala Asp Val Arg Val Gly Glu Ser Lys Leu Thr Gly
275 280 285

Asn Asn Gly Thr
290

<210> 37
<211> 2054

<212> DNA
<213> Populus sp.

<400> 37
tgaaaaaaaa aaacacatct gctttctggg atgactgatt accgactacc gccgacaatg 60
aatctatgga cggacgacaa cgcgtctgta atggaagcat ttatgaactc ctccgatctc 120
tcttctcttt gggcaccgcc tccgcaatct tccgcctcca cttccacgcc atcagcagca 180
gcacagccat ctgagaaaac tatgttgaac caagaaacac tccagcaacg ccttcagaca 240
ttaattgaag gcgcgtgtga gggctgggct tatgctattt tctggcaatc ctcttatgat 300
tattctggcg cctccgtcct aggatggggc gacggttatt ataaagggga ggaagataaa 360
ggaaaaacta gaacgagaaa ttcggcatca tcagctgttg aacaggagca tcgtaaaacg 420
gtgctccgta agctgaattc gttgattgct ggacctaatt ctgttactga tgatgctatc 480
gatgaagagg ttactgatac tgagtggttt tttcttgtta gcatgactca gtctttcgtt 540
aatggaagtg ggttacctgg tcaagctctt ttcaatggga gtccggtttg ggttgctggg 600
tcggagaggt tgggggcttc gccgtgtgaa agagccaggc agggtcaggt tttgggttta 660
cagactttag tttgtatacc gtctgccagt ggtgttgttg aattgggttc tactgaattg 720
atttttcaaa gctcggatct gatgaataag gttagggttt tgttcgattt taatagtttg 780
gaggtggttt cttggccgat tggaactaca aatactgatc agggtgagaa cgacccgtct 840
tcgttttggc ttactgatcc agaaactaaa gatggaaatg gtgggattcc ttggaatctg 900
aacgggagtg atcaaaacaa gaacaatcat catagttcta atcagagttc gagtagcttg 960
actgatcatc tgggtggaat tcatcatgct cagaatcatc aacagcagcc gattcatgct 1020
cgaagtcttt tcactcgaga gttgaatttt ggggagtgca gcacatatga tgggagtagt 1080
gttagaaatg gaaattccca tttgacgaag ccagaatctg gagagatatt gaattttggg 1140
gagagtaaga ggactgcttc cagtgcaaat gggaatttct attcgggttt ggtgacagag 1200
gagaataata agaagaagag atcagttggt aatgaagaag ggatgctttc atttacttct 1260
ggtgtgattt taccttcttc ttgtatcctg aaatccagtg gcggtacagg aggggattca 1320
gatcactctg atcttgaggc ttcggttgta aaagaggcgg atagcagcag ggttgtggaa 1380
ccagaaaaga ggccacgaaa acgagggaga aaacctgcca atggaagaga agagcctttg 1440
aatcatgttg aagcagagag gcaaagaaga gagaaactta atcagaggtt ttatgcactg 1500
cgagctgtgg ttcctaatgt atcaaagatg gacaaagctt cacttcttgg ggatgccata 1560
tcatatatcg acgagcttag aacgaagctg caatctgccg agtctagcaa ggaggagttg 1620
gagaagcaag tggaatcaat gaagagggag ttagtaagca aggattcaag tcctccacct 1680
aaagaggagc tcaagatgtc aaataacgaa ggagttaaac tgatagacat ggatatcgat 1740
gtgaagataa gtggatggga tgcgatgata cgaatccaat gttgtaaaaa gaaccaccct 1800
gctgctaggc taatgtcggc tttgagagac ctggaccttg atgttcagta tgccaatgtg 1860
tctgtgatga atgatttgat gatccagcaa gccacggtga agatggggag tcggttttac 1920
acgcaagaag agcttagggt agccatatca acaaatgttg gtggcgtcca ttaggcattg 1980

aacagaacaa caaatgcaat tagcagttgg gatagatcct ggtaaagatt acaggttccg 2040

actttattta ttaa 2054

<210> 38
<211> 647
<212> PRT
<213> Populus sp.

<400> 38

Met Thr Asp Tyr Arg Leu Pro Pro Thr Met Asn Leu Trp Thr Asp Asp
1 5 10 15

Asn Ala Ser Val Met Glu Ala Phe Met Asn Ser Ser Asp Leu Ser Ser
20 25 30

Leu Trp Ala Pro Pro Pro Gln Ser Ser Ala Ser Thr Ser Thr Pro Ser
35 40 45

Ala Ala Ala Gln Pro Ser Glu Lys Thr Met Leu Asn Gln Glu Thr Leu
50 55 60

Gln Gln Arg Leu Gln Thr Leu Ile Glu Gly Ala Cys Glu Gly Trp Ala
65 70 75 80

Tyr Ala Ile Phe Trp Gln Ser Ser Tyr Asp Tyr Ser Gly Ala Ser Val
85 90 95

Leu Gly Trp Gly Asp Gly Tyr Tyr Lys Gly Glu Glu Asp Lys Gly Lys
100 105 110

Thr Arg Thr Arg Asn Ser Ala Ser Ser Ala Val Glu Gln Glu His Arg
115 120 125

Lys Thr Val Leu Arg Lys Leu Asn Ser Leu Ile Ala Gly Pro Asn Ser
130 135 140

Val Thr Asp Asp Ala Ile Asp Glu Glu Val Thr Asp Thr Glu Trp Phe
145 150 155 160

Phe Leu Val Ser Met Thr Gln Ser Phe Val Asn Gly Ser Gly Leu Pro
165 170 175

Gly Gln Ala Leu Phe Asn Gly Ser Pro Val Trp Val Ala Gly Ser Glu
180 185 190

Arg Leu Gly Ala Ser Pro Cys Glu Arg Ala Arg Gln Gly Gln Val Phe
195 200 205

Gly Leu Gln Thr Leu Val Cys Ile Pro Ser Ala Ser Gly Val Val Glu
210 215 220

Leu Gly Ser Thr Glu Leu Ile Phe Gln Ser Ser Asp Leu Met Asn Lys
225 230 235 240

Val Arg Val Leu Phe Asp Phe Asn Ser Leu Glu Val Val Ser Trp Pro
245 250 255

Ile Gly Thr Thr Asn Thr Asp Gln Gly Glu Asn Asp Pro Ser Ser Phe
260 265 270

Trp Leu Thr Asp Pro Glu Thr Lys Asp Gly Asn Gly Gly Ile Pro Trp
275 280 285

Asn Leu Asn Gly Ser Asp Gln Asn Lys Asn Asn His His Ser Ser Asn
290 295 300

Gln Ser Ser Ser Ser Leu Thr Asp His Leu Gly Gly Ile His His Ala
305 310 315 320

Gln Asn His Gln Gln Gln Pro Ile His Ala Arg Ser Leu Phe Thr Arg
325 330 335

Glu Leu Asn Phe Gly Glu Cys Ser Thr Tyr Asp Gly Ser Ser Val Arg
340 345 350

Asn Gly Asn Ser His Leu Thr Lys Pro Glu Ser Gly Glu Ile Leu Asn
355 360 365

Phe Gly Glu Ser Lys Arg Thr Ala Ser Ser Ala Asn Gly Asn Phe Tyr
370 375 380

Ser Gly Leu Val Thr Glu Glu Asn Asn Lys Lys Lys Arg Ser Val Gly
385 390 395 400

Asn Glu Glu Gly Met Leu Ser Phe Thr Ser Gly Val Ile Leu Pro Ser
405 410 415

Ser Cys Ile Leu Lys Ser Ser Gly Gly Thr Gly Gly Asp Ser Asp His
420 425 430

Ser Asp Leu Glu Ala Ser Val Val Lys Glu Ala Asp Ser Ser Arg Val
435 440 445

Val Glu Pro Glu Lys Arg Pro Arg Lys Arg Gly Arg Lys Pro Ala Asn
450 455 460

Gly Arg Glu Glu Pro Leu Asn His Val Glu Ala Glu Arg Gln Arg Arg
465 470 475 480

Glu Lys Leu Asn Gln Arg Phe Tyr Ala Leu Arg Ala Val Val Pro Asn
485 490 495

Val Ser Lys Met Asp Lys Ala Ser Leu Leu Gly Asp Ala Ile Ser Tyr

500 505 510

Ile Asp Glu Leu Arg Thr Lys Leu Gln Ser Ala Glu Ser Ser Lys Glu
515 520 525

Glu Leu Glu Lys Gln Val Glu Ser Met Lys Arg Glu Leu Val Ser Lys
530 535 540

Asp Ser Ser Pro Pro Pro Lys Glu Glu Leu Lys Met Ser Asn Asn Glu
545 550 555 560

Gly Val Lys Leu Ile Asp Met Asp Ile Asp Val Lys Ile Ser Gly Trp
565 570 575

Asp Ala Met Ile Arg Ile Gln Cys Cys Lys Lys Asn His Pro Ala Ala
580 585 590

Arg Leu Met Ser Ala Leu Arg Asp Leu Asp Leu Asp Val Gln Tyr Ala
595 600 605

Asn Val Ser Val Met Asn Asp Leu Met Ile Gln Gln Ala Thr Val Lys
610 615 620

Met Gly Ser Arg Phe Tyr Thr Gln Glu Glu Leu Arg Val Ala Ile Ser
625 630 635 640

Thr Asn Val Gly Gly Val His
645

<210> 39
<211> 1827
<212> DNA
<213> Vitis vinifera

<400> 39
atgaaaactg aaatgggtat gggaggaggg gcttggacgg aggaggataa agcgatggtg 60
gcggctgttt tgggaactcg agcatttgat tacttgatga ccagctccgt tgtgagtgag 120
aatctgttaa tggcagtggg gagtgatgaa aatttgcaga caaagctttc ggatctcgta 180
gaccgaccaa atgcatccaa tttcagttgg aattatgcaa ttttctggca aatttcacag 240
tccaagtctg ggattgggt gcttggatgg ggagatgggt cttgtaggga gcccagggaa 300
ggggaggaat ccgaagtgac ccgaatttta aatattcggc tcgaggatgc aacccagcag 360
aggatgagga aaagggtgct tcagaagctg catacattgt ttggaggctc ggatgaggac 420
agttatgctt ttggattgga ccgagtcact gatactgaaa tgttttttct cgcttccatg 480
tacttctcat ttaccagagg agaaggtggc ccagggaaat cttttggatc tgggaagcat 540
ttgtggctgt ctgatgcatt gaaatctcca tcagattatt gtgttcgatc attccttgcc 600
aaatctgctg gaattcagac cattgtgtta atcccaactg atgttggggt tgtagagctg 660
ggttctgtga ggtcattacc tgaaagctta gagatgttgc agactataag atcatcattc 720

tcgatgtatt tgccattcat caggggcaag ccagccttac cagtgttgaa cgagaagaaa 780
aatgaaagtg ctcctttttc caatctgggg actggggagc gagtagaggg aattccaaag 840
atttttgggc aggatttgaa ctcaggtcat tcccatttta gggagaaact tgctgttcga 900
aaggcagaag agaggccatg ggacagctac caaaacggga acaggcttcc ttttacgaac 960
actagaaatg gttttcatgg ttcgggctgg ccacacatgc agggtgtgaa accagcaagt 1020
acggcagaaa tgtacagtcc tcaggtccca atcaataatc tacatgagat ggttaatggg 1080
gttagagagg agtttcggct tagccagttc cagcctccca agcaggtgca gatgcaaatt 1140
gattttgcag ggctgcttc aaggtctacc atgttggctc ggccaattag tgtggagtct 1200
gagcattcag atgtcgaggc ttcatgcaag gatgagcggc caggcccagc tgatgaaagg 1260
aggccccgga aaaggggcag aaagcctgca aatggaagag aagaacccct caatcatgtg 1320
gaagcagaga ggcagaggcg tgagaaactg aaccagcggt tttatgcatt gcgagctgtt 1380
gtgcccaaca tctccaagat ggacaaagcc tccttgctgg gagatgcaat tacttacatc 1440
accgagctcc agaagaaact caaggacatg gaatcagagc gggagaaatt tgggagcact 1500
tcaagagatg cattatcttt ggaaactaac accgaggcag aaactcatat acaggcgtct 1560
gatgtcgata tccaagcagc caatgatgaa gttattgtga gagtaagctg cccactggat 1620
acccaccctg tatcaagagt catccaaaca ttcaaggagg cacagatcac agtgatcgag 1680
tcgaaacttg ctacagacaa tgatactgtg ttacatactt ttgtaatcaa gtcccaggga 1740
tccgagcagc tgatgaagga aaagctcaca gctgcatttt cgcgcgaatc caattcttta 1800
cagcctttgt catcatcagt tgggtaa 1827

<210> 40
<211> 608
<212> PRT
<213> Vitis vinifera

<400> 40

Met Lys Thr Glu Met Gly Met Gly Gly Gly Ala Trp Thr Glu Glu Asp
1 5 10 15

Lys Ala Met Val Ala Ala Val Leu Gly Thr Arg Ala Phe Asp Tyr Leu
20 25 30

Met Thr Ser Ser Val Val Ser Glu Asn Leu Leu Met Ala Val Gly Ser
35 40 45

Asp Glu Asn Leu Gln Thr Lys Leu Ser Asp Leu Val Asp Arg Pro Asn
50 55 60

Ala Ser Asn Phe Ser Trp Asn Tyr Ala Ile Phe Trp Gln Ile Ser Gln
65 70 75 80

Ser Lys Ser Gly Asp Trp Val Leu Gly Trp Gly Asp Gly Ser Cys Arg
85 90 95

Glu Pro Arg Glu Gly Glu Glu Ser Glu Val Thr Arg Ile Leu Asn Ile
100 105 110

Arg Leu Glu Asp Ala Thr Gln Gln Arg Met Arg Lys Arg Val Leu Gln
115 120 125

Lys Leu His Thr Leu Phe Gly Gly Ser Asp Glu Asp Ser Tyr Ala Phe
130 135 140

Gly Leu Asp Arg Val Thr Asp Thr Glu Met Phe Phe Leu Ala Ser Met
145 150 155 160

Tyr Phe Ser Phe Thr Arg Gly Glu Gly Gly Pro Gly Lys Ser Phe Gly
165 170 175

Ser Gly Lys His Leu Trp Leu Ser Asp Ala Leu Lys Ser Pro Ser Asp
180 185 190

Tyr Cys Val Arg Ser Phe Leu Ala Lys Ser Ala Gly Ile Gln Thr Ile
195 200 205

Val Leu Ile Pro Thr Asp Val Gly Val Val Glu Leu Gly Ser Val Arg
210 215 220

Ser Leu Pro Glu Ser Leu Glu Met Leu Gln Thr Ile Arg Ser Ser Phe
225 230 235 240

Ser Met Tyr Leu Pro Phe Ile Arg Gly Lys Pro Ala Leu Pro Val Leu
245 250 255

Asn Glu Lys Lys Asn Glu Ser Ala Pro Phe Ser Asn Leu Gly Thr Gly
260 265 270

Glu Arg Val Glu Gly Ile Pro Lys Ile Phe Gly Gln Asp Leu Asn Ser
275 280 285

Gly His Ser His Phe Arg Glu Lys Leu Ala Val Arg Lys Ala Glu Glu
290 295 300

Arg Pro Trp Asp Ser Tyr Gln Asn Gly Asn Arg Leu Pro Phe Thr Asn
305 310 315 320

Thr Arg Asn Gly Phe His Gly Ser Gly Trp Pro His Met Gln Gly Val
325 330 335

Lys Pro Ala Ser Thr Ala Glu Met Tyr Ser Pro Gln Val Pro Ile Asn
340 345 350

Asn Leu His Glu Met Val Asn Gly Val Arg Glu Glu Phe Arg Leu Ser
355 360 365

Gln Phe Gln Pro Pro Lys Gln Val Gln Met Gln Ile Asp Phe Ala Gly
370 375 380

Ala Ala Ser Arg Ser Thr Met Leu Ala Arg Pro Ile Ser Val Glu Ser
385 390 395 400

Glu His Ser Asp Val Glu Ala Ser Cys Lys Asp Glu Arg Pro Gly Pro
405 410 415

Ala Asp Glu Arg Arg Pro Arg Lys Arg Gly Arg Lys Pro Ala Asn Gly
420 425 430

Arg Glu Glu Pro Leu Asn His Val Glu Ala Glu Arg Gln Arg Arg Glu
435 440 445

Lys Leu Asn Gln Arg Phe Tyr Ala Leu Arg Ala Val Val Pro Asn Ile
450 455 460

Ser Lys Met Asp Lys Ala Ser Leu Leu Gly Asp Ala Ile Thr Tyr Ile
465 470 475 480

Thr Glu Leu Gln Lys Lys Leu Lys Asp Met Glu Ser Glu Arg Glu Lys
485 490 495

Phe Gly Ser Thr Ser Arg Asp Ala Leu Ser Leu Glu Thr Asn Thr Glu
500 505 510

Ala Glu Thr His Ile Gln Ala Ser Asp Val Asp Ile Gln Ala Ala Asn
515 520 525

Asp Glu Val Ile Val Arg Val Ser Cys Pro Leu Asp Thr His Pro Val
530 535 540

Ser Arg Val Ile Gln Thr Phe Lys Glu Ala Gln Ile Thr Val Ile Glu
545 550 555 560

Ser Lys Leu Ala Thr Asp Asn Asp Thr Val Leu His Thr Phe Val Ile
565 570 575

Lys Ser Gln Gly Ser Glu Gln Leu Met Lys Glu Lys Leu Thr Ala Ala
580 585 590

Phe Ser Arg Glu Ser Asn Ser Leu Gln Pro Leu Ser Ser Ser Val Gly
595 600 605

<210> 41
<211> 663
<212> PRT
<213> Oryza sativa

<400> 41

Met Glu Ala Phe Met Ala Ser Ala Asp Leu Pro Ala Phe Pro Trp Gly
1 5 10 15

Ala Ala Ser Thr Pro Pro Pro Pro Pro Pro Pro Pro His His His His
20 25 30

Gln Gln Gln Gln Gln Gln Val Leu Pro Pro Pro Ala Ala Ala Pro Ala
35 40 45

Ala Ala Ala Phe Asn Gln Asp Thr Leu Gln Gln Arg Leu Gln Ser Ile
50 55 60

Ile Glu Gly Ser Arg Glu Thr Trp Thr Tyr Ala Ile Phe Trp Gln Ser
65 70 75 80

Ser Ile Asp Val Ser Thr Gly Ala Ser Leu Leu Gly Trp Gly Asp Gly
85 90 95

Tyr Tyr Lys Gly Cys Asp Asp Asp Lys Arg Lys Gln Arg Ser Ser Thr
100 105 110

Pro Ala Ala Ala Ala Glu Gln Glu His Arg Lys Arg Val Leu Arg Glu
115 120 125

Leu Asn Ser Leu Ile Ala Gly Ala Gly Ala Ala Pro Asp Glu Ala Val
130 135 140

Glu Glu Glu Ala Leu Phe Ala Ala Gln Pro Thr Trp Ile Ala Thr Gly
145 150 155 160

Leu Ser Ser Ala Pro Cys Asp Arg Ala Arg Gln Ala Tyr Thr Phe Gly
165 170 175

Leu Arg Thr Met Val Cys Leu Pro Leu Ala Thr Gly Val Leu Glu Leu
180 185 190

Gly Ser Thr Asp Val Ile Phe Gln Thr Gly Asp Ser Ile Pro Arg Ile
195 200 205

Arg Ala Leu Phe Asn Leu Ser Ala Ala Ala Ala Ser Ser Trp Pro Pro
210 215 220

His Pro Asp Ala Ala Ser Ala Asp Pro Ser Val Leu Trp Leu Ala Asp
225 230 235 240

Ala Pro Pro Met Asp Met Lys Asp Ser Ile Ser Ala Ala Asp Ile Ser
245 250 255

Val Ser Lys Pro Pro Pro Pro Pro Pro His Gln Ile Gln His Phe Glu
260 265 270

Asn Gly Ser Thr Ser Thr Leu Thr Glu Asn Pro Ser Pro Ser Val His
275 280 285

Ala Pro Thr Pro Ser Gln Pro Ala Ala Pro Pro Gln Arg Gln Gln Gln
290 295 300

Gln Gln Gln Ser Ser Gln Ala Gln Gln Gly Pro Phe Arg Arg Glu Leu
305 310 315 320

Asn Phe Ser Asp Phe Ala Ser Asn Gly Gly Ala Ala Ala Pro Pro Phe
325 330 335

Phe Lys Pro Glu Thr Gly Glu Ile Leu Asn Phe Gly Asn Asp Ser Ser
340 345 350

Thr Gly Arg Arg Asn Pro Ser Pro Ala Pro Pro Ala Ala Thr Ala Ser
355 360 365

Leu Thr Thr Ala Pro Gly Ser Leu Phe Ser Gln His Thr Pro Thr Leu
370 375 380

Thr Ala Ala Ala Asn Asp Ala Lys Ser Asn Asn Gln Lys Arg Ser Met
385 390 395 400

Glu Ala Thr Ser Arg Ala Ser Asn Thr Asn Asn His Pro Ala Ala Thr
405 410 415

Ala Asn Glu Gly Met Leu Ser Phe Ser Ser Ala Pro Thr Thr Arg Pro
420 425 430

Ser Thr Gly Thr Gly Ala Pro Ala Lys Ser Glu Ser Asp His Ser Asp
435 440 445

Leu Glu Ala Ser Val Arg Glu Val Glu Ser Ser Arg Val Val Ala Pro
450 455 460

Pro Pro Glu Ala Glu Lys Arg Pro Arg Lys Arg Gly Arg Lys Pro Ala
465 470 475 480

Asn Gly Arg Glu Glu Pro Leu Asn His Val Glu Ala Glu Arg Gln Arg
485 490 495

Arg Glu Lys Leu Asn Gln Arg Phe Tyr Ala Leu Arg Ala Val Val Pro
500 505 510

Asn Val Ser Lys Met Asp Lys Ala Ser Leu Leu Gly Asp Ala Ile Ser
515 520 525

Tyr Ile Asn Glu Leu Arg Gly Lys Leu Thr Ala Leu Glu Thr Asp Lys
530 535 540

Glu Thr Leu Gln Ser Gln Met Glu Ser Leu Lys Lys Glu Arg Asp Ala
545 550 555 560

Arg Pro Pro Ala Pro Ser Gly Gly Gly Gly Asp Gly Gly Ala Arg Cys
565 570 575

His Ala Val Glu Ile Glu Ala Lys Ile Leu Gly Leu Glu Ala Met Ile
580 585 590

Arg Val Gln Cys His Lys Arg Asn His Pro Ala Ala Arg Leu Met Thr
595 600 605

Ala Leu Arg Glu Leu Asp Leu Asp Val Tyr His Ala Ser Val Ser Val
610 615 620

Val Lys Asp Leu Met Ile Gln Gln Val Ala Val Lys Met Ala Ser Arg
625 630 635 640

Val Tyr Ser Gln Asp Gln Leu Asn Ala Ala Leu Tyr Thr Arg Ile Ala
645 650 655

Glu Pro Gly Thr Ala Ala Arg
660

<210> 42
<211> 70
<212> PRT
<213> Artificial sequence

<220>
<223> peptide sequence corresponding to Motif 7 in NP_001065478

<400> 42

Pro Arg Lys Arg Gly Arg Lys Pro Ala Asn Gly Arg Glu Glu Pro Leu
1 5 10 15

Asn His Val Glu Ala Glu Arg Gln Arg Arg Glu Lys Leu Asn Gln Arg
20 25 30

Phe Tyr Ala Leu Arg Ala Val Val Pro Asn Val Ser Lys Met Asp Lys
35 40 45

Ala Ser Leu Leu Gly Asp Ala Ile Ser Tyr Ile Asn Glu Leu Arg Gly
50 55 60

Lys Leu Thr Ala Leu Glu
65 70

<210> 43
<211> 70
<212> PRT
<213> Artificial sequence

<220>
<223> peptide sequence corresponding to Motif 7 in EAY79619

<400> 43

Pro Arg Lys Arg Gly Arg Lys Pro Ala Asn Gly Arg Glu Glu Pro Leu

1 5 10 15

Asn His Val Glu Ala Glu Arg Gln Arg Arg Glu Lys Leu Asn Gln Arg
20 25 30

Phe Tyr Ala Leu Arg Ala Val Val Pro Asn Val Ser Lys Met Asp Lys
35 40 45

Ala Ser Leu Leu Gly Asp Ala Ile Ser Tyr Ile Asn Glu Leu Arg Gly
50 55 60

Lys Leu Thr Ala Leu Glu
65 70

<210> 44
<211> 70
<212> PRT
<213> Artificial sequence

<220>
<223> peptide sequence corresponding to Motif 7 in AAD15818

<400> 44

Pro Arg Lys Arg Gly Arg Lys Pro Ala Asn Gly Arg Glu Glu Pro Leu
1 5 10 15

Asn His Val Glu Ala Glu Arg Gln Arg Arg Glu Lys Leu Asn Gln Arg
20 25 30

Phe Tyr Ala Leu Arg Ala Val Val Pro Asn Val Ser Lys Met Asp Lys
35 40 45

Ala Ser Leu Leu Gly Asp Ala Ile Ser Tyr Ile Asn Glu Leu Arg Gly
50 55 60

Lys Leu Thr Ser Leu Glu
65 70

<210> 45
<211> 70
<212> PRT
<213> Artificial sequence

<220>
<223> peptide sequence corresponding to Motif 7 in AAB00686

<400> 45

Pro Arg Lys Arg Gly Arg Lys Pro Gly Asn Gly Arg Glu Glu Pro Leu
1 5 10 15

Asn His Val Glu Ala Glu Arg Gln Arg Arg Glu Lys Leu Asn Gln Arg
20 25 30

Phe Tyr Ala Leu Arg Ala Val Val Pro Asn Val Ser Lys Met Asp Lys
35 40 45

Ala Ser Leu Leu Gly Asp Ala Ile Ser Tyr Ile Asn Glu Leu Lys Ser
50 55 60

Lys Leu Ser Glu Leu Glu
65 70

<210> 46
<211> 70
<212> PRT
<213> Artificial sequence

<220>
<223> peptide sequence corresponding to Motif 7 in ABD59338

<400> 46

Pro Arg Lys Arg Gly Arg Lys Pro Ala Asn Gly Arg Glu Glu Pro Leu
1 5 10 15

Asn His Val Glu Ala Glu Arg Gln Arg Arg Glu Lys Leu Asn Gln Arg
20 25 30

Phe Tyr Ala Leu Arg Ala Val Val Pro Asn Val Ser Lys Met Asp Lys
35 40 45

Ala Ser Leu Leu Gly Asp Ala Ile Ser Tyr Ile Asn Glu Leu Lys Leu
50 55 60

Lys Leu Gln Gly Leu Glu
65 70

<210> 47
<211> 70
<212> PRT
<213> Artificial sequence

<220>
<223> peptide sequence corresponding to Motif 7 in Pt29.195#1

<400> 47

Pro Arg Lys Arg Gly Arg Lys Pro Ala Asn Gly Arg Glu Glu Pro Leu
1 5 10 15

Asn His Val Glu Ala Glu Arg Gln Arg Arg Glu Lys Leu Asn Gln Arg
20 25 30

Phe Tyr Ala Leu Arg Ala Val Val Pro Asn Val Ser Lys Met Asp Lys
35 40 45

Ala Ser Leu Leu Gly Asp Ala Ile Ser Tyr Ile Asp Glu Leu Arg Thr
50 55 60

Lys Leu Gln Ser Ala Glu
65 70

<210> 48
<211> 70
<212> PRT
<213> Artificial sequence

<220>
<223> peptide sequence corresponding to Motif 7 in CAF74710

<400> 48

```
Pro Arg Lys Arg Gly Arg Lys Pro Ala Asn Gly Arg Glu Glu Pro Leu
1               5                   10                  15

Asn His Val Glu Ala Glu Arg Gln Arg Arg Glu Lys Leu Asn Gln Arg
            20                  25                  30

Phe Tyr Ala Leu Arg Ala Val Val Pro Asn Val Ser Lys Met Asp Lys
        35                  40                  45

Ala Ser Leu Leu Gly Asp Ala Ile Ser Tyr Ile Asn Glu Leu Lys Ser
    50                  55                  60

Lys Leu Gln Asn Thr Glu
65                  70
```

<210> 49
<211> 70
<212> PRT
<213> Artificial sequence

<220>
<223> peptide sequence corresponding to Motif 7 in AAF04917

<400> 49

```
Pro Arg Lys Arg Gly Arg Lys Pro Ala Asn Gly Arg Glu Glu Pro Leu
1               5                   10                  15

Asn His Val Glu Ala Glu Arg Gln Arg Arg Glu Lys Leu Asn Gln Arg
            20                  25                  30

Phe Phe Ser Leu Arg Ala Val Val Pro Asn Val Ser Lys Met Asp Lys
        35                  40                  45

Ala Ser Leu Leu Gly Asp Ala Ile Ser Tyr Ile Asn Glu Leu Lys Ser
    50                  55                  60

Lys Leu Gln Asn Thr Glu
65                  70
```

<210> 50
<211> 70
<212> PRT
<213> Artificial sequence

<220>
<223> peptide sequence corresponding to Motif 7 in AAQ14332

<400> 50

Pro Arg Lys Arg Gly Arg Lys Pro Ala Asn Gly Arg Glu Glu Pro Leu
1 5 10 15

Asn His Val Glu Ala Glu Arg Gln Arg Arg Glu Lys Leu Asn Gln Arg
20 25 30

Phe Tyr Ala Leu Arg Ala Val Val Pro Asn Val Ser Lys Met Asp Lys
35 40 45

Ala Ser Leu Leu Gly Asp Ala Ile Ser Tyr Ile Asn Glu Leu Lys Ala
50 55 60

Lys Leu Gln Thr Thr Glu
65 70

<210> 51
<211> 70
<212> PRT
<213> Artificial sequence

<220>
<223> peptide sequence corresponding to Motif 7 in AAY90122

<400> 51

Pro Arg Lys Arg Gly Arg Lys Pro Ala Asn Gly Arg Glu Glu Pro Leu
1 5 10 15

Asn His Val Glu Ala Glu Arg Gln Arg Arg Glu Lys Leu Asn Gln Arg
20 25 30

Phe Tyr Ala Leu Arg Ala Val Val Pro Asn Val Ser Lys Met Asp Lys
35 40 45

Ala Ser Leu Leu Gly Asp Ala Ile Ser Phe Ile Asn Glu Leu Lys Ser
50 55 60

Lys Leu Gln Asn Val Glu
65 70

<210> 52
<211> 70
<212> PRT
<213> Artificial sequence

<220>
<223> peptide sequence corresponding to Motif 7 in AAL55713

<400> 52

Pro Lys Lys Arg Gly Arg Lys Pro Ala Asn Gly Arg Glu Glu Pro Leu
1 5 10 15

Asn His Val Glu Ala Glu Arg Gln Arg Arg Glu Lys Leu Asn Gln Arg
20 25 30

Phe Tyr Ala Leu Arg Ala Val Val Pro Asn Val Ser Lys Met Asp Lys
35 40 45

Ala Ser Leu Leu Gly Asp Ala Ile Ala Tyr Ile Asn Glu Leu Lys Ser
50 55 60

Lys Val Val Lys Thr Glu
65 70

<210> 53
<211> 70
<212> PRT
<213> Artificial sequence

<220>
<223> peptide sequence corresponding to Motif 7 in ABD65632

<400> 53

Pro Arg Lys Arg Gly Arg Lys Pro Ala Asn Gly Arg Glu Glu Pro Leu
1 5 10 15

Asn His Val Glu Ala Glu Arg Gln Arg Arg Glu Lys Leu Asn Gln Arg
20 25 30

Phe Tyr Ser Leu Arg Ala Val Val Pro Asn Val Ser Lys Met Asp Lys
35 40 45

Ala Ser Leu Leu Gly Asp Ala Ile Ser Tyr Ile Asn Glu Leu Lys Ala
50 55 60

Lys Leu Gln Lys Ala Glu
65 70

<210> 54
<211> 70
<212> PRT
<213> Artificial sequence

<220>
<223> peptide sequence corresponding to Motif 7 in ABK94979

<400> 54

Pro Lys Lys Arg Gly Arg Lys Pro Gly Leu Gly Arg Asp Ala Pro Leu
1 5 10 15

Asn His Val Glu Ala Glu Arg Gln Arg Arg Glu Lys Leu Asn His Arg
20 25 30

Phe Tyr Ala Leu Arg Ala Val Val Pro Asn Val Ser Arg Met Asp Lys
35 40 45

Ala Ser Leu Leu Ser Asp Ala Val Ser Tyr Ile Asn Glu Leu Lys Ala
50 55 60

Lys Val Asp Glu Leu Glu
65 70

<210> 55
<211> 70
<212> PRT
<213> Artificial sequence

<220>
<223> peptide sequence corresponding to Motif 7 in EDQ91347

<400> 55

Pro Arg Lys Arg Gly Arg Lys Pro Ala Asn Asp Arg Glu Glu Pro Leu
1 5 10 15

Asn His Val Gln Ala Glu Arg Gln Arg Arg Glu Lys Leu Asn Gln Lys
20 25 30

Phe Tyr Ala Leu Arg Ser Val Val Pro Asn Val Ser Lys Met Asp Lys
35 40 45

Ala Ser Leu Leu Glu Asp Ala Ile Thr Tyr Ile Asn Glu Leu Gln Glu
50 55 60

Lys Leu Gln Lys Ala Glu
65 70

<210> 56
<211> 4120
<212> DNA
<213> Artificial sequence

<220>
<223> expression cassette

<400> 56
aatccgaaaa gtttctgcac cgttttcacc ccctaactaa caatataggg aacgtgtgct 60

aaatataaaa tgagacctta tatatgtagc gctgataact agaactatgc aagaaaaact 120

catccaccta ctttagtggc aatcgggcta aataaaaaag agtcgctaca ctagtttcgt 180

tttccttagt aattaagtgg gaaaatgaaa tcattattgc ttagaatata cgttcacatc 240

tctgtcatga agttaaatta ttcgaggtag ccataattgt catcaaactc ttcttgaata 300

aaaaaatctt tctagctgaa ctcaatgggt aaagagagag atttttttta aaaaaataga 360

atgaagatat tctgaacgta ttggcaaaga tttaaacata taattatata attttatagt 420

ttgtgcattc gtcatatcgc acatcattaa ggacatgtct tactccatcc caatttttat 480

ttagtaatta aagacaattg acttattttt attatttatc ttttttcgat tagatgcaag 540

gtacttacgc acacactttg tgctcatgtg catgtgtgag tgcacctcct caatacacgt 600

tcaactagca acacatctct aatatcactc gcctatttaa tacatttagg tagcaatatc 660

tgaattcaag cactccacca tcaccagacc acttttaata atatctaaaa tacaaaaaat 720

aattttacag aatagcatga aaagtatgaa acgaactatt taggtttttc acatacaaaa 780

aaaaaaagaa ttttgctcgt gcgcgagcgc caatctccca tattgggcac acaggcaaca 840
acagagtggc tgcccacaga acaacccaca aaaaacgatg atctaacgga ggacagcaag 900
tccgcaacaa ccttttaaca gcaggctttg cggccaggag agaggaggag aggcaaagaa 960
aaccaagcat cctcctcctc ccatctataa attcctcccc ccttttcccc tctctatata 1020
ggaggcatcc aagccaagaa gagggagagc accaaggaca cgcgactagc agaagccgag 1080
cgaccgcctt cttcgatcca tatcttccgg tcgagttctt ggtcgatctc ttccctcctc 1140
cacctcctcc tcacagggta tgtgcccttc ggttgttctt ggatttattg ttctaggttg 1200
tgtagtacgg gcgttgatgt taggaaaggg gatctgtatc tgtgatgatt cctgttcttg 1260
gatttgggat agaggggttc ttgatgttgc atgttatcgg ttcggtttga ttagtagtat 1320
ggttttcaat cgtctggaga gctctatgga aatgaaatgg tttagggtac ggaatcttgc 1380
gattttgtga gtaccttttg tttgaggtaa aatcagagca ccggtgattt tgcttggtgt 1440
aataaaagta cggttgtttg gtcctcgatt ctggtagtga tgcttctcga tttgacgaag 1500
ctatcctttg tttattccct attgaacaaa aataatccaa ctttgaagac ggtcccgttg 1560
atgagattga atgattgatt cttaagcctg tccaaaattt cgcagctggc ttgtttagat 1620
acagtagtcc ccatcacgaa attcatggaa acagttataa tcctcaggaa caggggattc 1680
cctgttcttc cgatttgctt tagtcccaga atttttttc ccaaatatct taaaaagtca 1740
ctttctggtt cagttcaatg aattgattgc tacaaataat gcttttatag cgttatccta 1800
gctgtagttc agttaatagg taatacccct atagtttagt caggagaaga acttatccga 1860
tttctgatct ccatttttaa ttatatgaaa tgaactgtag cataagcagt attcatttgg 1920
attatttttt ttattagctc tcaccccttc attattctga gctgaaagtc tggcatgaac 1980
tgtcctcaat tttgttttca aattcacatc gattatctat gcattatcct cttgtatcta 2040
cctgtagaag tttctttttg gttattcctt gactgcttga ttacagaaag aaatttatga 2100
agctgtaatc gggatagtta tactgcttgt tcttatgatt catttccttt gtgcagttct 2160
tggtgtagct tgccactttc accagcaaag ttcatttaaa tcaactaggg atatcacaag 2220
tttgtacaaa aaagcaggct taaacaatga ctgattaccg gctacaacca acgatgaatc 2280
tttggaccac cgacgacaac gcttctatga tggaagcttt catgagctct tccgatatct 2340
caactttatg gcctccggcg tcgacgacaa ccacgacggc gacgactgaa acaactccga 2400
cgccggcgat ggagattccg gcacaggcgg gatttaatca agagactctt cagcaacgtt 2460
tacaagcttt gattgaagga acacacgaag gttggaccta cgctatattc tggcaaccgt 2520
cgtatgattt ctccggcgcc tccgtgctcg gatggggaga tggttattac aaaggtgaag 2580
aagataaagc aaacccgaga cggagatcga gttcgccgcc gttttctact ccggcggatc 2640
aggagtacag gaaaaaagtg ttgagagagc ttaactcgtt gatctccggt ggtgttgctc 2700
cgtcggatga cgctgttgat gaggaggtga cggatacgga atggtttttc ttggtttcga 2760
tgacgcagag cttcgcttgc ggtgcgggat tagctggtaa agcgtttgca acgggtaacg 2820
cggtttgggt ttccgggtca gatcaattat ccgggtcggg ttgtgaacgg gctaagcaag 2880

gaggagtgtt tgggatgcat actattgcgt gtattccttc ggcgaacgga gttgtggaag 2940
tcgggtcaac ggagccgatc cgacagagtt cggaccttat taacaaggtt cgaattcttt 3000
tcaatttcga cggcggagct ggagatttat cgggtcttaa ttggaatctt gacccggatc 3060
aaggtgagaa cgacccgtct atgtggatta atgacccgat tggaacacct ggatctaacg 3120
aaccgggtaa cggagctcca agttctagct cccagctttt ttcaaagtct attcagtttg 3180
agaacggtag ctcaagcaca ataaccgaaa acccgaatct ggatccgact ccgagtccgg 3240
ttcattctca gacccagaat ccgaaattca ataacacttt ctcccgagaa cttaattttt 3300
cgacgtcaag ttctacttta gtgaaaccaa gatccggcga gatattaaac ttcggcgatg 3360
aaggtaaacg aagctccgga aacccggatc caagttctta ttcgggtcaa acacaattcg 3420
aaaacaaaag aaagaggtcg atggttttga acgaagataa agttctatca ttcggagata 3480
aaaccgccgg agaatcagat cactccgatc tagaagcttc cgtcgtgaaa gaagtagcag 3540
tagagaaacg tccaaagaaa cgaggaagaa agccagcaaa cggtagagaa gagccactaa 3600
accacgtcga agcagagaga caaagacgcg agaaactaaa ccaaagattc tacgcgttac 3660
gagcggttgt accaaacgtt tcaaaaatgg ataaagcttc gttactcggt gacgcaatcg 3720
cttacatcaa cgagcttaaa tccaaagtag tcaaaacaga gtcagagaaa ctccaaatca 3780
agaaccagct cgaggaagtg aaactcgagc tcgccggaag aaaagcgagt gctagtggag 3840
gagatatgtc gtcttcgtgt tcttcgatta aaccggtggg gatggagatt gaagtgaaga 3900
taattggttg ggacgcaatg attagagttg aatctagtaa gaggaatcat ccggcggcga 3960
ggttgatgtc ggcgttgatg gatttggagt tggaagtgaa tcacgcgagt atgtcggtgg 4020
ttaacgattt gatgattcaa caagcgacgg tgaagatggg ttttaggatc tatacgcaag 4080
aacagctcag agcaagtttg atttcaaaaa tcggttaaaa 4120

<210> 57
<211> 687
<212> DNA
<213> Nicotiana tabacum

<400> 57
atggctccca ttgaaggtgt tattcaagta cagggtgata taacaaatgc taaaacagct 60
gaagtggtta ttagacattt tgacggatgc aaggctgaca ttgttgtctg tgatggtgca 120
cctgatgtaa cgggacttca tgacatggat gaatttgttc agtcccagct gatattggcg 180
ggcctaacca ttgtcactca catactaaaa ggaggcggaa agttcatagc aaaaattttt 240
cgaggaaaag acacaagcct tctttactgt cagctaaaac tatttttcac agaagtgact 300
tttgctaagc caaaaagcag tcggaattct agcatagagg catttgcagt ttgcgagaat 360
tactctccac ctgaaggatt taatgagaaa gatcttcatc gccttcttga aaagattgga 420
agtccatctg gcacagagga cctagattgc agtagtgcat ggctggaagg tcctaataag 480
gtgtatattc catttctggc ttgtggagac cttagcgggt acgattcaga ccgttcatat 540
ccacttccta aagctgcaga tggaacctat cagtgtttag atcctgtaca acctccaatt 600

gcaccgccat ataaacgagc tcttgaaatg aagaaagcgt cgaatcaagg aatccaaaac 660

ctagacaagc tttctcttag ctcctaa 687

<210> 58
<211> 228
<212> PRT
<213> Nicotiana tabacum

<400> 58

```
Met Ala Pro Ile Glu Gly Val Ile Gln Val Gln Gly Asp Ile Thr Asn
1               5                   10                  15

Ala Lys Thr Ala Glu Val Val Ile Arg His Phe Asp Gly Cys Lys Ala
            20                  25                  30

Asp Ile Val Val Cys Asp Gly Ala Pro Asp Val Thr Gly Leu His Asp
        35                  40                  45

Met Asp Glu Phe Val Gln Ser Gln Leu Ile Leu Ala Gly Leu Thr Ile
    50                  55                  60

Val Thr His Ile Leu Lys Gly Gly Gly Lys Phe Ile Ala Lys Ile Phe
65                  70                  75                  80

Arg Gly Lys Asp Thr Ser Leu Leu Tyr Cys Gln Leu Lys Leu Phe Phe
                85                  90                  95

Thr Glu Val Thr Phe Ala Lys Pro Lys Ser Ser Arg Asn Ser Ser Ile
            100                 105                 110

Glu Ala Phe Ala Val Cys Glu Asn Tyr Ser Pro Pro Glu Gly Phe Asn
        115                 120                 125

Glu Lys Asp Leu His Arg Leu Leu Glu Lys Ile Gly Ser Pro Ser Gly
    130                 135                 140

Thr Glu Asp Leu Asp Cys Ser Ser Ala Trp Leu Glu Gly Pro Asn Lys
145                 150                 155                 160

Val Tyr Ile Pro Phe Leu Ala Cys Gly Asp Leu Ser Gly Tyr Asp Ser
                165                 170                 175

Asp Arg Ser Tyr Pro Leu Pro Lys Ala Ala Asp Gly Thr Tyr Gln Cys
            180                 185                 190

Leu Asp Pro Val Gln Pro Pro Ile Ala Pro Pro Tyr Lys Arg Ala Leu
        195                 200                 205

Glu Met Lys Lys Ala Ser Asn Gln Gly Ile Gln Asn Leu Asp Lys Leu
    210                 215                 220
```

Ser Leu Ser Ser
225

<210> 59
<211> 951
<212> DNA
<213> Nicotiana tabacum

<400> 59
atgggaaaag catcgaggga caaaagagat atttactatc ggaaagcaaa ggaagaagga 60
tggcgtgccc gaagtgcctt caagctcctt cagattgatg aggaatttaa catctttgaa 120
ggtgtgaagc gagttgtgga tttgtgtgca gcaccaggca gctggagtca ggttttaagc 180
cggaagttat atctcccagc aaagttgtca cctgatacca aggacgacga tctaccactt 240
attgtggcta ttgacttaca gcctatggct cccattgaag gtgttattca agtacagggt 300
gatataacaa atgctaaaac agctgaagtg gttattagac attttgacgg atgcaaggct 360
gacattgttg tctgtgatgg tgcacctgat gtaacgggac ttcatgacat ggatgaattt 420
gttcagtccc agctgatatt ggcgggccta accattgtca ctcacatact aaaaggaggc 480
ggaaagttca tagcaaaaat ttttcgagga aaagacacaa gccttcttta ctgtcagcta 540
aaactatttt tcacagaagt gacttttgct aagccaaaaa gcagtcggaa ttctagcata 600
gaggcatttg cagtttgcga gaattactct ccacctgaag gatttaatga gaaagatctt 660
catcgccttc ttgaaaagat tggaagtcca tctggcacag aggacctaga ttgcagtagt 720
gcatggctgg aaggtcctaa taaggtgtat attccatttc tggcttgtgg agaccttagc 780
gggtacgatt cagaccgttc atatccactt cctaaagctg cagatggaac ctatcagtgt 840
ttagatcctg tacaacctcc aattgcaccg ccatataaac gagctcttga aatgaagaaa 900
gcgtcgaatc aaggaatcca aaacctagac aagctttctc ttagctccta a 951

<210> 60
<211> 316
<212> PRT
<213> Nicotiana tabacum

<400> 60

Met Gly Lys Ala Ser Arg Asp Lys Arg Asp Ile Tyr Tyr Arg Lys Ala
1 5 10 15

Lys Glu Glu Gly Trp Arg Ala Arg Ser Ala Phe Lys Leu Leu Gln Ile
20 25 30

Asp Glu Glu Phe Asn Ile Phe Glu Gly Val Lys Arg Val Val Asp Leu
35 40 45

Cys Ala Ala Pro Gly Ser Trp Ser Gln Val Leu Ser Arg Lys Leu Tyr
50 55 60

Leu Pro Ala Lys Leu Ser Pro Asp Thr Lys Asp Asp Asp Leu Pro Leu
65 70 75 80

Ile Val Ala Ile Asp Leu Gln Pro Met Ala Pro Ile Glu Gly Val Ile
85 90 95

Gln Val Gln Gly Asp Ile Thr Asn Ala Lys Thr Ala Glu Val Val Ile
100 105 110

Arg His Phe Asp Gly Cys Lys Ala Asp Ile Val Val Cys Asp Gly Ala
115 120 125

Pro Asp Val Thr Gly Leu His Asp Met Asp Glu Phe Val Gln Ser Gln
130 135 140

Leu Ile Leu Ala Gly Leu Thr Ile Val Thr His Ile Leu Lys Gly Gly
145 150 155 160

Gly Lys Phe Ile Ala Lys Ile Phe Arg Gly Lys Asp Thr Ser Leu Leu
165 170 175

Tyr Cys Gln Leu Lys Leu Phe Phe Thr Glu Val Thr Phe Ala Lys Pro
180 185 190

Lys Ser Ser Arg Asn Ser Ser Ile Glu Ala Phe Ala Val Cys Glu Asn
195 200 205

Tyr Ser Pro Pro Glu Gly Phe Asn Glu Lys Asp Leu His Arg Leu Leu
210 215 220

Glu Lys Ile Gly Ser Pro Ser Gly Thr Glu Asp Leu Asp Cys Ser Ser
225 230 235 240

Ala Trp Leu Glu Gly Pro Asn Lys Val Tyr Ile Pro Phe Leu Ala Cys
245 250 255

Gly Asp Leu Ser Gly Tyr Asp Ser Asp Arg Ser Tyr Pro Leu Pro Lys
260 265 270

Ala Ala Asp Gly Thr Tyr Gln Cys Leu Asp Pro Val Gln Pro Pro Ile
275 280 285

Ala Pro Pro Tyr Lys Arg Ala Leu Glu Met Lys Lys Ala Ser Asn Gln
290 295 300

Gly Ile Gln Asn Leu Asp Lys Leu Ser Leu Ser Ser
305 310 315

<210> 61
<211> 1128
<212> DNA
<213> Arabidopsis thaliana

<400> 61
gaatttctac tttggtgctg ctcttcttca catacctagc tgctgtatac tatacgtgaa 60

ccaactccgt attctacttt agaggccatg ggaaaagctt ctcgagacaa aagggatatc 120
tattatcgta aagctaaaga agaaggatgg cgtgcaagaa gtgcttttaa gcttcttcag 180
attgatgaag aatttaatat ttttgaaggt gtgaagaggg tcgtagattt atgtgctgca 240
cctggtagct ggagtcaggt cttgagtcgt caattatatc ttcctgcaaa gtcctcagct 300
gagtcaaaag atggggatct tcctcttata gtagccatcg atttgcagcc tatggctcca 360
attgaaggtg tcatccaggt tcaaggggac ataactaatg ctcggacaga agtggtcatt 420
agacactttg acggctgcaa agcggacctg gttgtctgtg atggtgctcc agatgttact 480
ggtttgcatg acatggatga atttgtccag tcccaactca tactagcagg attaacgatt 540
gtaactcata ttcttaaaga aggtggaaaa tttattgcaa agatattccg tggaaaagat 600
acaagtctct tgtactgtca actgaaattg tttttcccaa ccgtaacttt tgcgaaaccg 660
aaaagcagcc gcaattcaag tatagaggct tttgcagtct gcgagaatta ctctccgcca 720
gaaggattta acccgagaga tctgcatcgt ctcttggaga aagtcggaag cccttcaggt 780
ggaagcgatc tcgattgcag tagtggttgg cttgaaggac ccaacaaagt ttatatacca 840
ttcttggcat gtggtgactt aaccggttat gactcagacc ggtcttaccc actccctaga 900
gaagcagatg gatcatcata ccagagtttg gacccgattc aacctcccat cgcaccgcct 960
tataaacgag ctcttgagct caagaaagct tcagcacaaa gcttcaactc ttgaagaagg 1020
tgataccaaa aaagaaaaag gaacaaactt ttcattggat tctgtagcct gtcgtaatga 1080
tatattcagg gacctacaat ttctataacg cttaatgttt ttgttatg 1128

<210> 62
<211> 309
<212> PRT
<213> Arabidopsis thaliana

<400> 62

Met Gly Lys Ala Ser Arg Asp Lys Arg Asp Ile Tyr Tyr Arg Lys Ala
1 5 10 15

Lys Glu Glu Gly Trp Arg Ala Arg Ser Ala Phe Lys Leu Leu Gln Ile
20 25 30

Asp Glu Glu Phe Asn Ile Phe Glu Gly Val Lys Arg Val Val Asp Leu
35 40 45

Cys Ala Ala Pro Gly Ser Trp Ser Gln Val Leu Ser Arg Gln Leu Tyr
50 55 60

Leu Pro Ala Lys Ser Ser Ala Glu Ser Lys Asp Gly Asp Leu Pro Leu
65 70 75 80

Ile Val Ala Ile Asp Leu Gln Pro Met Ala Pro Ile Glu Gly Val Ile
85 90 95

Gln Val Gln Gly Asp Ile Thr Asn Ala Arg Thr Ala Glu Val Val Ile

100 105 110

Arg His Phe Asp Gly Cys Lys Ala Asp Leu Val Val Cys Asp Gly Ala
115 120 125

Pro Asp Val Thr Gly Leu His Asp Met Asp Glu Phe Val Gln Ser Gln
130 135 140

Leu Ile Leu Ala Gly Leu Thr Ile Val Thr His Ile Leu Lys Glu Gly
145 150 155 160

Gly Lys Phe Ile Ala Lys Ile Phe Arg Gly Lys Asp Thr Ser Leu Leu
165 170 175

Tyr Cys Gln Leu Lys Leu Phe Phe Pro Thr Val Thr Phe Ala Lys Pro
180 185 190

Lys Ser Ser Arg Asn Ser Ser Ile Glu Ala Phe Ala Val Cys Glu Asn
195 200 205

Tyr Ser Pro Pro Glu Gly Phe Asn Pro Arg Asp Leu His Arg Leu Leu
210 215 220

Glu Lys Val Gly Ser Pro Ser Gly Gly Ser Asp Leu Asp Cys Ser Ser
225 230 235 240

Gly Trp Leu Glu Gly Pro Asn Lys Val Tyr Ile Pro Phe Leu Ala Cys
245 250 255

Gly Asp Leu Thr Gly Tyr Asp Ser Asp Arg Ser Tyr Pro Leu Pro Arg
260 265 270

Glu Ala Asp Gly Ser Ser Tyr Gln Ser Leu Asp Pro Ile Gln Pro Pro
275 280 285

Ile Ala Pro Pro Tyr Lys Arg Ala Leu Glu Leu Lys Lys Ala Ser Ala
290 295 300

Gln Ser Phe Asn Ser
305

<210> 63
<211> 951
<212> DNA
<213> Lycopersicon esculentum

<400> 63
atgggaaaag catctaggga taagagggat atttactacc ggaaagcaaa ggaagaaggg 60

tggcgtgccc gaagtgcctt caagctcctt cagatagatg aggagtttaa tatctttgaa 120

ggtgcgaagc gcgttgtgga tttatgtgct gctccaggca gctggagtca ggttttaagc 180

cggaagttat atctcccagc aaagctgtca ccgggtacca aggatgatga tctaccactt 240

atcgtggcta ttgacttaca gcctatggct ccaattgaag gtgttattca agtacagggt 300
gatataacaa atgctaaaac agttgaagtg gttattagac attttgatgg atgcaaagct 360
gaccttgttg tctgtgatgg tgcacctgat gtaacgggac ttcatgacat ggatgaattt 420
gttcaatccc aactgatatt ggcgggccta accatagtca ctcacatact aaaagaaggt 480
ggaaagttca tagcaaaaat ttttcgagga aaagacacaa gtctccttta ctgtcaacta 540
aaactatttt tcacggaagt gacttttgct aagccaaaaa gtagtcgcaa ttctagcata 600
gaggcatttg cagtttgtga aaactactct ccacctgaag gattcaatga gaaagacctt 660
tatcgccttc ttgaacaagt tggaagtcca tcaggcgctg aggacttaga ttgcagtagt 720
ggatggcttg aaggtcgtaa caaggtgtat atcccatttc tggcttgcgg agacctgagt 780
ggatatgatt cagatcgttc atatccgctt cctaaatctg cagatggaac ctatcaatgt 840
ttagatcctg tacaacctcc gattgcacca ccgtataaac gagctcttga aatgaaaaaa 900
gcttcaagtc aaggaattca taatctagac aaactttctc ttgacccctg a 951

<210> 64
<211> 316
<212> PRT
<213> Lycopersicon esculentum

<400> 64

Met Gly Lys Ala Ser Arg Asp Lys Arg Asp Ile Tyr Tyr Arg Lys Ala
1 5 10 15

Lys Glu Glu Gly Trp Arg Ala Arg Ser Ala Phe Lys Leu Leu Gln Ile
20 25 30

Asp Glu Glu Phe Asn Ile Phe Glu Gly Ala Lys Arg Val Val Asp Leu
35 40 45

Cys Ala Ala Pro Gly Ser Trp Ser Gln Val Leu Ser Arg Lys Leu Tyr
50 55 60

Leu Pro Ala Lys Leu Ser Pro Gly Thr Lys Asp Asp Asp Leu Pro Leu
65 70 75 80

Ile Val Ala Ile Asp Leu Gln Pro Met Ala Pro Ile Glu Gly Val Ile
85 90 95

Gln Val Gln Gly Asp Ile Thr Asn Ala Lys Thr Val Glu Val Val Ile
100 105 110

Arg His Phe Asp Gly Cys Lys Ala Asp Leu Val Val Cys Asp Gly Ala
115 120 125

Pro Asp Val Thr Gly Leu His Asp Met Asp Glu Phe Val Gln Ser Gln
130 135 140

Leu Ile Leu Ala Gly Leu Thr Ile Val Thr His Ile Leu Lys Glu Gly
145                 150                 155                 160

Gly Lys Phe Ile Ala Lys Ile Phe Arg Gly Lys Asp Thr Ser Leu Leu
                165                 170                 175

Tyr Cys Gln Leu Lys Leu Phe Phe Thr Glu Val Thr Phe Ala Lys Pro
            180                 185                 190

Lys Ser Ser Arg Asn Ser Ser Ile Glu Ala Phe Ala Val Cys Glu Asn
        195                 200                 205

Tyr Ser Pro Pro Glu Gly Phe Asn Glu Lys Asp Leu Tyr Arg Leu Leu
    210                 215                 220

Glu Gln Val Gly Ser Pro Ser Gly Ala Glu Asp Leu Asp Cys Ser Ser
225                 230                 235                 240

Gly Trp Leu Glu Gly Arg Asn Lys Val Tyr Ile Pro Phe Leu Ala Cys
                245                 250                 255

Gly Asp Leu Ser Gly Tyr Asp Ser Asp Arg Ser Tyr Pro Leu Pro Lys
            260                 265                 270

Ser Ala Asp Gly Thr Tyr Gln Cys Leu Asp Pro Val Gln Pro Pro Ile
        275                 280                 285

Ala Pro Pro Tyr Lys Arg Ala Leu Glu Met Lys Lys Ala Ser Ser Gln
    290                 295                 300

Gly Ile His Asn Leu Asp Lys Leu Ser Leu Asp Pro
305                 310                 315

<210> 65
<211> 951
<212> DNA
<213> Medicago truncatula

<400> 65
atgggcaaag cttcaaggga taagagggat atatattacc gaaaagcgaa agaagaaggt 60
tggcgtgctc gaagtgcttt taaacttctt cagatagatg aggaatttaa catttttgaa 120
ggggtgaaac gtgttgtaga tttatgtgct gccccaggca gttggagtca ggttttgagt 180
cgaaaactgt accttccagc caagcttgca cctgatgcaa aggatgaaaa tcttcctctt 240
attgtagcta ttgatttgca gccaatggct ccgattgaag gtgttatcca ggtgcagggt 300
gatataacta atgctcggac cgctgaagtg gtcattagac atttcgatgg ttgcaaggcc 360
aaccttgttg tgtgtgatgg tgctcctgat gttaccggac ttcatgacat ggatgaattt 420
gttcaatccc aactcatact tgcagggttg acaattgtta ctcatgtact gaaggaagga 480
gggaagttca ttgcgaagat atttagagga aaggacacaa gccttctata ctgtcagcta 540
aaattatttt tccctgtggt gactttcgca aagccaaaaa gtagccgtaa ttccagcata 600

gaggcatttg cagtttgtga aaactactcc cctcctgaag gattcaaccc gaaagatctt 660

caccgacttc ttgagaaggt tggaagtcca tcaggcgtag atgatacaga ttgtgttagt 720

gggtggttgg aaggccctaa taaggtgtat atcccatttc tagcttgcgg ggacctcact 780

ggatatgatt ctgataggtc atatccactg cctaaagttg ctgggggaac atatcagagc 840

ttggatccag tacaacctcc tattgcccct ccttacaaga gagctctaga gttaaaaaaa 900

gcatcacctc aaggattccg agaacttgaa aatctctccc tggattcctg a 951

<210> 66
<211> 316
<212> PRT
<213> Medicago truncatula

<220>
<221> UNSURE
<222> (236)..(236)
<223> Xaa can be any naturally occurring amino acid

<400> 66

Met Gly Lys Ala Ser Arg Asp Lys Arg Asp Ile Tyr Tyr Arg Lys Ala
1 5 10 15

Lys Glu Glu Gly Trp Arg Ala Arg Ser Ala Phe Lys Leu Leu Gln Ile
20 25 30

Asp Glu Glu Phe Asn Ile Phe Glu Gly Val Lys Arg Val Val Asp Leu
35 40 45

Cys Ala Ala Pro Gly Ser Trp Ser Gln Val Leu Ser Arg Lys Leu Tyr
50 55 60

Leu Pro Ala Lys Leu Ala Pro Asp Ala Lys Asp Glu Asn Leu Pro Leu
65 70 75 80

Ile Val Ala Ile Asp Leu Gln Pro Met Ala Pro Ile Glu Gly Val Ile
85 90 95

Gln Val Gln Gly Asp Ile Thr Asn Ala Arg Thr Ala Glu Val Val Ile
100 105 110

Arg His Phe Asp Gly Cys Lys Ala Asn Leu Val Val Cys Asp Gly Ala
115 120 125

Pro Asp Val Thr Gly Leu His Asp Met Asp Glu Phe Val Gln Ser Gln
130 135 140

Leu Ile Leu Ala Gly Leu Thr Ile Val Thr His Val Leu Lys Glu Gly
145 150 155 160

Gly Lys Phe Ile Ala Lys Ile Phe Arg Gly Lys Asp Thr Ser Leu Leu
165 170 175

Tyr Cys Gln Leu Lys Leu Phe Phe Pro Val Val Thr Phe Ala Lys Pro
180 185 190

Lys Ser Ser Arg Asn Ser Ser Ile Glu Ala Phe Ala Val Cys Glu Asn
195 200 205

Tyr Ser Pro Pro Glu Gly Phe Asn Pro Lys Asp Leu His Arg Leu Leu
210 215 220

Glu Lys Val Gly Ser Pro Ser Gly Val Asp Asp Xaa Asp Cys Val Ser
225 230 235 240

Gly Trp Leu Glu Gly Pro Asn Lys Val Tyr Ile Pro Phe Leu Ala Cys
245 250 255

Gly Asp Leu Thr Gly Tyr Asp Ser Asp Arg Ser Tyr Pro Leu Pro Lys
260 265 270

Val Ala Gly Gly Thr Tyr Gln Ser Leu Asp Pro Val Gln Pro Pro Ile
275 280 285

Ala Pro Pro Tyr Lys Arg Ala Leu Glu Leu Lys Lys Ala Ser Pro Gln
290 295 300

Gly Phe Arg Glu Leu Glu Asn Leu Ser Leu Asp Ser
305 310 315

<210> 67
<211> 1018
<212> DNA
<213> Oryza sativa

<400> 67
atgggcaagg cctccaaaga caagagggac atctactacc gcaaggcgaa ggaggagggg 60

tggagagctc gtagcgcctt caagcttctg cagatcgacc aggagttcaa catcttccac 120

ggagtgaagc gtgttgttga tctgtgcgct gctcccggaa gctggagtca ggttttgagt 180

cggaacctgt atgttccagc aaaacaatct cctgattgca aagaaggtga ccttcctctt 240

attgttgcca ttgatttgca accaatggct ccgatagaag gcgttataca agttcaaggg 300

gacatcacta atgctcgaac agcagaagtg gttattaggc attttgatgg atgcaaagca 360

gatttggttg tctgcgatgg tgctcctgat gttaccggcc ttcatgatat ggacgagttt 420

gttcagtccc agcttatact ggcggcattg acaattgtga ctcacgtact taaagttggt 480

gggaaatttg ttgcgaagat tttccgtgga aaagatacaa gtctgttgta ttgccagctg 540

aaactattct tctcacaagt tacctttgca aagcccaaaa gtagccggaa ctcaagcatc 600

gaggcgtttg cagtttgtga gaattattcg cctcccgaag gatttaagga aaaagattta 660

tatcacctgc tagagaaagt ggggactcct tctggggctg atgatttaga ctgcagaagt 720

ggatggttag agggaccaaa caaggtctac atcccatttc tggcttgcgg tgacctcagt 780

ggttacgatt cggatcgttc ttacccacta acaagcacag agggaggatc ctaccagagc 840

ttggatccag tccagcctcc aattgctcca ccttacaaaa ccgcactgga gatgaaaaag 900

gtcgccagcc atggcattgg agcagatatc agcaaattat ccctcgactc ctgaactaca 960

ctctggatcg ctgtttgtgt tcttgtaact gccaaccctg tggaatttgc aaattgta 1018

<210> 68
<211> 317
<212> PRT
<213> Oryza sativa

<400> 68

Met Gly Lys Ala Ser Lys Asp Lys Arg Asp Ile Tyr Tyr Arg Lys Ala
1 5 10 15

Lys Glu Glu Gly Trp Arg Ala Arg Ser Ala Phe Lys Leu Leu Gln Ile
20 25 30

Asp Gln Glu Phe Asn Ile Phe His Gly Val Lys Arg Val Val Asp Leu
35 40 45

Cys Ala Ala Pro Gly Ser Trp Ser Gln Val Leu Ser Arg Asn Leu Tyr
50 55 60

Val Pro Ala Lys Gln Ser Pro Asp Cys Lys Glu Gly Asp Leu Pro Leu
65 70 75 80

Ile Val Ala Ile Asp Leu Gln Pro Met Ala Pro Ile Glu Gly Val Ile
85 90 95

Gln Val Gln Gly Asp Ile Thr Asn Ala Arg Thr Ala Glu Val Val Ile
100 105 110

Arg His Phe Asp Gly Cys Lys Ala Asp Leu Val Val Cys Asp Gly Ala
115 120 125

Pro Asp Val Thr Gly Leu His Asp Met Asp Glu Phe Val Gln Ser Gln
130 135 140

Leu Ile Leu Ala Ala Leu Thr Ile Val Thr His Val Leu Lys Val Gly
145 150 155 160

Gly Lys Phe Val Ala Lys Ile Phe Arg Gly Lys Asp Thr Ser Leu Leu
165 170 175

Tyr Cys Gln Leu Lys Leu Phe Phe Ser Gln Val Thr Phe Ala Lys Pro
180 185 190

Lys Ser Ser Arg Asn Ser Ser Ile Glu Ala Phe Ala Val Cys Glu Asn
195 200 205

```
Tyr Ser Pro Pro Glu Gly Phe Lys Glu Lys Asp Leu Tyr His Leu Leu
    210                 215                 220

Glu Lys Val Gly Thr Pro Ser Gly Ala Asp Asp Leu Asp Cys Arg Ser
225                 230                 235                 240

Gly Trp Leu Glu Gly Pro Asn Lys Val Tyr Ile Pro Phe Leu Ala Cys
                245                 250                 255

Gly Asp Leu Ser Gly Tyr Asp Ser Asp Arg Ser Tyr Pro Leu Thr Ser
            260                 265                 270

Thr Glu Gly Gly Ser Tyr Gln Ser Leu Asp Pro Val Gln Pro Pro Ile
        275                 280                 285

Ala Pro Pro Tyr Lys Thr Ala Leu Glu Met Lys Lys Val Ala Ser His
    290                 295                 300

Gly Ile Gly Ala Asp Ile Ser Lys Leu Ser Leu Asp Ser
305                 310                 315
```

<210> 69
<211> 894
<212> DNA
<213> Ostreococcus lucimarinus

<400> 69

```
atggggaagt cgagcaagga taagcgcgat atatactacc gcaaggcgaa agaggaggga      60
tggcgcgcga ggtcggcgtt caagttgctg caaattgacg agtcgttcga tatttttcga     120
gacgtgcgac acgtcgtgga tctgtgcgcg gcgcccgggt cgtggtcgca ggttttgagt     180
cgaaagttgt acctacccgc gttggcgcga ggggtcgagg aggaagagct gccgaagatt     240
gtcgccatcg acttgcaacc gatggcgccg atcgagggcg tgacgacgat acagggcgat     300
atcacgtcga tggacaaagt gcgcgaggtc ctgtcgcatt tcgatgggaa acacgcggat     360
ttgatcgtcg gcgatggagc gcccgacgtc acgggtttgc acgatttgga tgagtttatg     420
caggcgcagc tcatactcgc cggcttgacg gtggcgacgc acatattgaa accaggcgga     480
acgttcatag cgaagatatt tcgggggaaa gacatcagtt tgctctactc acaactgaaa     540
atttcttccc ccgaagtcac gtgcgccaag ccgaaaagta gccggaattc tagcatagaa     600
gcattcatag tgtgtcaggg ttactcccct ccagagggat tcgaaccaca tcaattgact     660
cgtattttag aagcgcgtgc gacgtctcac gccgctggag acgagggcgc ggccgtggga     720
accaagtcct ggccgaacaa tgtgctcgtg ccgtttttgg cgtgcggcga tttgtcggga     780
tacgacgccg atcagagcta tgcgttggat gacaccaaag tcagactaaa accggtgcaa     840
ccgccgacaa caccggcgta tatgaacgca atcaagctct taaagagaaa gtga           894
```

<210> 70
<211> 297
<212> PRT

<213> Ostreococcus lucimarinus

<400> 70

Met Gly Lys Ser Ser Lys Asp Lys Arg Asp Ile Tyr Tyr Arg Lys Ala
1               5                   10                  15

Lys Glu Glu Gly Trp Arg Ala Arg Ser Ala Phe Lys Leu Leu Gln Ile
            20                  25                  30

Asp Glu Ser Phe Asp Ile Phe Arg Asp Val Arg His Val Val Asp Leu
        35                  40                  45

Cys Ala Ala Pro Gly Ser Trp Ser Gln Val Leu Ser Arg Lys Leu Tyr
    50                  55                  60

Leu Pro Ala Leu Ala Arg Gly Val Glu Glu Glu Glu Leu Pro Lys Ile
65                  70                  75                  80

Val Ala Ile Asp Leu Gln Pro Met Ala Pro Ile Glu Gly Val Thr Thr
                85                  90                  95

Ile Gln Gly Asp Ile Thr Ser Met Asp Lys Val Arg Glu Val Leu Ser
            100                 105                 110

His Phe Asp Gly Lys His Ala Asp Leu Ile Val Gly Asp Gly Ala Pro
        115                 120                 125

Asp Val Thr Gly Leu His Asp Leu Asp Glu Phe Met Gln Ala Gln Leu
    130                 135                 140

Ile Leu Ala Gly Leu Thr Val Ala Thr His Ile Leu Lys Pro Gly Gly
145                 150                 155                 160

Thr Phe Ile Ala Lys Ile Phe Arg Gly Lys Asp Ile Ser Leu Leu Tyr
                165                 170                 175

Ser Gln Leu Lys Ile Phe Phe Pro Glu Val Thr Cys Ala Lys Pro Lys
            180                 185                 190

Ser Ser Arg Asn Ser Ser Ile Glu Ala Phe Ile Val Cys Gln Gly Tyr
        195                 200                 205

Ser Pro Pro Glu Gly Phe Glu Pro His Gln Leu Thr Arg Ile Leu Glu
    210                 215                 220

Ala Arg Ala Thr Ser His Ala Ala Gly Asp Glu Gly Ala Ala Val Gly
225                 230                 235                 240

Thr Lys Ser Trp Pro Asn Asn Val Leu Val Pro Phe Leu Ala Cys Gly
                245                 250                 255

Asp Leu Ser Gly Tyr Asp Ala Asp Gln Ser Tyr Ala Leu Asp Asp Thr

260 265 270

Lys Val Arg Leu Lys Pro Val Gln Pro Pro Thr Thr Pro Ala Tyr Met
275 280 285

Asn Ala Ile Lys Leu Leu Lys Arg Lys
290 295

<210> 71
<211> 951
<212> DNA
<213> Populus tremuloides

<400> 71
atggggagag cttcaaggga taaaagggat atatactata gaaaagcaaa ggaagaaggt 60

tggcgtgctc gaagtgcctt taagctcatt cagatagatg aggaattcaa tatttttgaa 120

ggagtgaagc gtgtggtgga tttatgtgct gcacctggta gctggagtca ggttttgagc 180

cgtaaactat atttaccagc aaaactttca cctgattcaa gggataatga tcttcctctt 240

attgtggcca ttgatttgca acctatggct ctcattgaag gtgttatcca agtccagggt 300

gatattacca atgcccgaac tgctgaagtg gtcattagac attttgatgg cagcaaggct 360

gacttggttg tgtgtgatgg tgcccctgat gttaccggac tccatgacat ggatgaattt 420

gttcagtctc aactcatact agcgggttta acaattgtca cacatgtact caaagaaggt 480

ggaaaattta ttgcgaagat atttcgtgga aaagatacaa gtcttctgta ttgccagctc 540

aaattatttt ttcctgtggt gacttttgcc aaaccaaaaa gtagccgcaa ttccagcata 600

gaggcatttg cagtttgtga gaattactct cctcctgagg gatttgatcc gaaagacttg 660

cgtcgccttt tggaaaaggt gggaagcccc tctggtgcag atgacctaga ttgcagtagc 720

gggtggttag aaggggcaag taaggtgtat attccatttc tagcttgcgg tgaccttagt 780

gggtatgact ctgaccgatc atatccacta ccaaaagatg ccgatggcac atatcagagc 840

ttggatcctg tacaaccccc aattgcccct ccttataaaa gagcccttga aatgaagaaa 900

gcttctagtc atggtgtaaa agagcttgaa aagctctctt tggattcttg a 951

<210> 72
<211> 316
<212> PRT
<213> Populus tremuloides

<400> 72

Met Gly Arg Ala Ser Arg Asp Lys Arg Asp Ile Tyr Tyr Arg Lys Ala
1 5 10 15

Lys Glu Glu Gly Trp Arg Ala Arg Ser Ala Phe Lys Leu Ile Gln Ile
20 25 30

Asp Glu Glu Phe Asn Ile Phe Glu Gly Val Lys Arg Val Val Asp Leu
35 40 45

Cys Ala Ala Pro Gly Ser Trp Ser Gln Val Leu Ser Arg Lys Leu Tyr
50 55 60

Leu Pro Ala Lys Leu Ser Pro Asp Ser Arg Asp Asn Asp Leu Pro Leu
65 70 75 80

Ile Val Ala Ile Asp Leu Gln Pro Met Ala Leu Ile Glu Gly Val Ile
85 90 95

Gln Val Gln Gly Asp Ile Thr Asn Ala Arg Thr Ala Glu Val Val Ile
100 105 110

Arg His Phe Asp Gly Ser Lys Ala Asp Leu Val Val Cys Asp Gly Ala
115 120 125

Pro Asp Val Thr Gly Leu His Asp Met Asp Glu Phe Val Gln Ser Gln
130 135 140

Leu Ile Leu Ala Gly Leu Thr Ile Val Thr His Val Leu Lys Glu Gly
145 150 155 160

Gly Lys Phe Ile Ala Lys Ile Phe Arg Gly Lys Asp Thr Ser Leu Leu
165 170 175

Tyr Cys Gln Leu Lys Leu Phe Phe Pro Val Val Thr Phe Ala Lys Pro
180 185 190

Lys Ser Ser Arg Asn Ser Ser Ile Glu Ala Phe Ala Val Cys Glu Asn
195 200 205

Tyr Ser Pro Pro Glu Gly Phe Asp Pro Lys Asp Leu Arg Arg Leu Leu
210 215 220

Glu Lys Val Gly Ser Pro Ser Gly Ala Asp Asp Leu Asp Cys Ser Ser
225 230 235 240

Gly Trp Leu Glu Gly Ala Ser Lys Val Tyr Ile Pro Phe Leu Ala Cys
245 250 255

Gly Asp Leu Ser Gly Tyr Asp Ser Asp Arg Ser Tyr Pro Leu Pro Lys
260 265 270

Asp Ala Asp Gly Thr Tyr Gln Ser Leu Asp Pro Val Gln Pro Pro Ile
275 280 285

Ala Pro Pro Tyr Lys Arg Ala Leu Glu Met Lys Lys Ala Ser Ser His
290 295 300

Gly Val Lys Glu Leu Glu Lys Leu Ser Leu Asp Ser
305 310 315

<210> 73
<211> 954
<212> DNA
<213> Saccharum officinarum

<400> 73
atgggcaagg cctccaagga caagagggac atctattacc ggaaggccaa agaggaaggg 60

tggagagctc gcagcgcctt caagctcctc cagatagacc aggagttcaa catattccat 120

ggagtgaagc gtgtggttga cctgtgtgct gctcccggaa gctggagcca ggttttgagc 180

cggaacctgt atgtaccagc aaaacaatct cctgattgca aggaaggcga ccttcctctc 240

atcgtcgcaa ttgacttgca accaatggcc ccgatagaag gtgttataca agtgcagggc 300

gacatcacca atgctcggac agcggaagtg gttattaggc attttgatgg atgcaaagca 360

gacttggttg tttgcgatgg agctccggat gttactggcc ttcatgatat ggacgaattt 420

gttcagtccc agcttatact cgcggcattg actatcgtga ctcatgtact caaagttggt 480

ggaaaatttg tcgcgaagat atttcggggt aaagatacaa gtctactgta ctgccagcta 540

aaactgttct tctcacaagt tacatttgcg aaaccaaaaa gtagccggaa ctcaagcatt 600

gaggcgtttg cagtttgtga gaactattca cctccagaag gtttcaagga ggaagatcta 660

taccacctgc tagagaaagt ggggactcct tcaggaggtg acgatttaga ttgcagaagc 720

ggatggctcg agggaccaaa caaggtgtac atcccgttcc ttgcctgcgg ggatctcagc 780

ggctacgact ctgatcgctc ataccggctc ccgagcacgg aaggtgggtc ctaccggagc 840

ttggaccccg tccagcctcc catcgccccg ccttacaaaa ccgctctgca gatgaagaag 900

gtttccagcc acagcgccag cgcggacgcc atgaaaccat ccacagattc ttga 954

<210> 74
<211> 317
<212> PRT
<213> Saccharum officinarum

<400> 74

Met Gly Lys Ala Ser Lys Asp Lys Arg Asp Ile Tyr Tyr Arg Lys Ala
1               5                   10                  15

Lys Glu Glu Gly Trp Arg Ala Arg Ser Ala Phe Lys Leu Leu Gln Ile
            20                  25                  30

Asp Gln Glu Phe Asn Ile Phe His Gly Val Lys Arg Val Val Asp Leu
        35                  40                  45

Cys Ala Ala Pro Gly Ser Trp Ser Gln Val Leu Ser Arg Asn Leu Tyr
    50                  55                  60

Val Pro Ala Lys Gln Ser Pro Asp Cys Lys Glu Gly Asp Leu Pro Leu
65                  70                  75                  80

Ile Val Ala Ile Asp Leu Gln Pro Met Ala Pro Ile Glu Gly Val Ile
                85                  90                  95

```
Gln Val Gln Gly Asp Ile Thr Asn Ala Arg Thr Ala Glu Val Val Ile
            100                 105                 110

Arg His Phe Asp Gly Cys Lys Ala Asp Leu Val Val Cys Asp Gly Ala
        115                 120                 125

Pro Asp Val Thr Gly Leu His Asp Met Asp Glu Phe Val Gln Ser Gln
    130                 135                 140

Leu Ile Leu Ala Ala Leu Thr Ile Val Thr His Val Leu Lys Val Gly
145                 150                 155                 160

Gly Lys Phe Val Ala Lys Ile Phe Arg Gly Lys Asp Thr Ser Leu Leu
                165                 170                 175

Tyr Cys Gln Leu Lys Leu Phe Phe Ser Gln Val Thr Phe Ala Lys Pro
            180                 185                 190

Lys Ser Ser Arg Asn Ser Ser Ile Glu Ala Phe Ala Val Cys Glu Asn
        195                 200                 205

Tyr Ser Pro Pro Glu Gly Phe Lys Glu Glu Asp Leu Tyr His Leu Leu
    210                 215                 220

Glu Lys Val Gly Thr Pro Ser Gly Gly Asp Asp Leu Asp Cys Arg Ser
225                 230                 235                 240

Gly Trp Leu Glu Gly Pro Asn Lys Val Tyr Ile Pro Phe Leu Ala Cys
                245                 250                 255

Gly Asp Leu Ser Gly Tyr Asp Ser Asp Arg Ser Tyr Arg Leu Pro Ser
            260                 265                 270

Thr Glu Gly Gly Ser Tyr Arg Ser Leu Asp Pro Val Gln Pro Pro Ile
        275                 280                 285

Ala Pro Pro Tyr Lys Thr Ala Leu Gln Met Lys Lys Val Ser Ser His
    290                 295                 300

Ser Ala Ser Ala Asp Ala Met Lys Pro Ser Thr Asp Ser
305                 310                 315

<210>  75
<211>  945
<212>  DNA
<213>  Triticum aestivum

<220>
<221>  misc_feature
<222>  (637)..(637)
<223>  n is a, c, g, or t
```

<220>
<221> misc_feature
<222> (673)..(673)
<223> n is a, c, g, or t

<220>
<221> misc_feature
<222> (775)..(775)
<223> n is a, c, g, or t

<220>
<221> misc_feature
<222> (829)..(829)
<223> n is a, c, g, or t

<220>
<221> misc_feature
<222> (837)..(837)
<223> n is a, c, g, or t

<400> 75
atgggcaagg cttcgaaaga caagcgggac atctactacc ggaaggccaa ggaggagggg 60
tggagggctc gcagcgcctt caagctcatg cagatcgacc aggagttcaa catcttccac 120
ggagtgaagc gtgccgttga cctgtgcgcc gctcctggga gctggagcca ggttttgagc 180
cgcaatttgt atttaccggc gaagctatca tctgacggca aagatggtgg ccttcctctc 240
atcgtcgcaa tcgatctgca acctatggct ccgatagaag gtgtcataca agtgcagggc 300
gacatcacca atgctcgaac agcggaagtg gttatcaggc actttgatgg atgcaaagcg 360
gacttggttg tctgtgatgg tgcccctgat gttactggac ttcatgatat ggacgagttt 420
gttcagtccc agcttatatt ggcggcactg acaattgtga ctcatgtact taaagttggt 480
ggaaaatttg ttgcgaagat tttccggggt aaagatacaa gtctcctgta ttgccagtta 540
aagctgttct tctcacaagt tacatttgca aagccaaaaa gcagccgcaa ctcaagtatc 600
gaggcatttg cagtttgcga gaactactca cctccangag ggcttcaaga gaaagattta 660
tatcacctgt tgnagaaagt gggaactcct tctggggctg atgatttaga ttgcagaagc 720
ggatggttgg aggggaccaa acaggtctac atcccgtttc tggcttgtgg cgacntcagg 780
ggttacgatc ggaccgttca taccccccctc cgagcacaga ggcggcaant accagancta 840
gatccagtca gccctccatt gcccggccgt aaaaactgcg ttgaaattag aaagggtcta 900
accagggtct ggcgcaatac agcaattatc ttcgacctta aataa 945

<210> 76
<211> 314
<212> PRT
<213> Triticum aestivum

<220>
<221> UNSURE
<222> (213)..(213)
<223> Xaa can be any naturally occurring amino acid

<220>
<221> UNSURE

<222> (225)..(225)
<223> Xaa can be any naturally occurring amino acid

<220>
<221> UNSURE
<222> (259)..(259)
<223> Xaa can be any naturally occurring amino acid

<220>
<221> UNSURE
<222> (277)..(277)
<223> Xaa can be any naturally occurring amino acid

<220>
<221> UNSURE
<222> (279)..(279)
<223> Xaa can be any naturally occurring amino acid

<400> 76

```
Met Gly Lys Ala Ser Lys Asp Lys Arg Asp Ile Tyr Tyr Arg Lys Ala
1               5                   10                  15

Lys Glu Glu Gly Trp Arg Ala Arg Ser Ala Phe Lys Leu Met Gln Ile
            20                  25                  30

Asp Gln Glu Phe Asn Ile Phe His Gly Val Lys Arg Ala Val Asp Leu
        35                  40                  45

Cys Ala Ala Pro Gly Ser Trp Ser Gln Val Leu Ser Arg Asn Leu Tyr
    50                  55                  60

Leu Pro Ala Lys Leu Ser Ser Asp Gly Lys Asp Gly Gly Leu Pro Leu
65                  70                  75                  80

Ile Val Ala Ile Asp Leu Gln Pro Met Ala Pro Ile Glu Gly Val Ile
                85                  90                  95

Gln Val Gln Gly Asp Ile Thr Asn Ala Arg Thr Ala Glu Val Val Ile
            100                 105                 110

Arg His Phe Asp Gly Cys Lys Ala Asp Leu Val Val Cys Asp Gly Ala
        115                 120                 125

Pro Asp Val Thr Gly Leu His Asp Met Asp Glu Phe Val Gln Ser Gln
    130                 135                 140

Leu Ile Leu Ala Ala Leu Thr Ile Val Thr His Val Leu Lys Val Gly
145                 150                 155                 160

Gly Lys Phe Val Ala Lys Ile Phe Arg Gly Lys Asp Thr Ser Leu Leu
                165                 170                 175

Tyr Cys Gln Leu Lys Leu Phe Phe Ser Gln Val Thr Phe Ala Lys Pro
            180                 185                 190

Lys Ser Ser Arg Asn Ser Ser Ile Glu Ala Phe Ala Val Cys Glu Asn
```

195 200 205

Tyr Ser Pro Pro Xaa Gly Leu Gln Glu Lys Asp Leu Tyr His Leu Leu
210 215 220

Xaa Lys Val Gly Thr Pro Ser Gly Ala Asp Asp Leu Asp Cys Arg Ser
225 230 235 240

Gly Trp Leu Glu Gly Thr Lys Gln Val Tyr Ile Pro Phe Leu Ala Cys
245 250 255

Gly Asp Xaa Arg Gly Tyr Asp Arg Thr Val His Thr Pro Leu Arg Ala
260 265 270

Gln Arg Arg Gln Xaa Pro Xaa Leu Asp Pro Val Ser Pro Pro Leu Pro
275 280 285

Gly Arg Lys Asn Cys Val Glu Ile Arg Lys Gly Leu Thr Arg Val Trp
290 295 300

Arg Asn Thr Ala Ile Ile Phe Asp Leu Lys
305 310

<210> 77
<211> 1246
<212> DNA
<213> Apis mellifera

<400> 77
taaagctaac caaaacgtat aacaacaata aacaacaagc attgtacatt agtcaataat 60
gggaaaaaca tcaaaagata aacgagatat atattatcga cgagcaaaag aagaaggatg 120
gagagcaagg agtgctttta aattgcttca aatagataac gaatgtcata ttttagatgg 180
agtaaacaaa gctgtggatt tgtgcgctgc acccgggagc tggagtcaag ttttatcacg 240
aagattaaat gagaattata aaaaagcttt ggaaacagga aatgcaatac ctccaaaaat 300
agttgcagtt gatttacaag ctatggcacc attggaagga gtgattcaaa ttcaaggaga 360
cattacgaat attgatactg caaaacaaat tatctctcat tttgataatg aacaagctga 420
tttagtagtt tgcgatggag cacctgatgt gacaggttta catgatatgg atatttatat 480
ccaatctcag ttattattag cagctctgaa tattactact catatattaa agcaaggagg 540
cacatttgtt gcaaaaatat ttagagctaa agatgtgact ttgctctatg ctcaactaaa 600
aatatttttc ccctatgtct attgtacgaa acctagcagt tctcgtaact ctagtattga 660
agcatttgta gtttgtaaag attactcacc accagaaggt tataaacctc atatgatgaa 720
tcctctttta acacataaac catgtgattt taatgacctt acaggaatta atagagttat 780
tgttccattt gttgtttgtg gtgatcttag tcagcctgat tccgatacgt gttatccatt 840
ggattttgaa ggaaaaacat atacttatca cgaaccggtc caaacaccga tttcaccacc 900
ttacaaagaa gcactcagtt tgatggaaga tagagatacc gatttcagaa gattcgatgt 960

taatgtagtt gtagataatt taagttcatt gactattgag gactttaaaa aacaagcgaa 1020

gcaaaaacat aaagagataa atgaaactaa aaggatcgaa ttgcaagata ataaaaaaga 1080

tgatagcgag gaagatatat tgggacttga caagttaatg cccaccgaat tatttgatga 1140

atctgaaaat aaaaataatg ataatatgta aaaatgttat gattttatat tatacatata 1200

atttgagtta taaatgtttt taatatatta ataataactt ataaat 1246

<210> 78
<211> 370
<212> PRT
<213> Apis mellifera

<400> 78

Met Gly Lys Thr Ser Lys Asp Lys Arg Asp Ile Tyr Tyr Arg Arg Ala
1 5 10 15

Lys Glu Glu Gly Trp Arg Ala Arg Ser Ala Phe Lys Leu Leu Gln Ile
20 25 30

Asp Asn Glu Cys His Ile Leu Asp Gly Val Asn Lys Ala Val Asp Leu
35 40 45

Cys Ala Ala Pro Gly Ser Trp Ser Gln Val Leu Ser Arg Arg Leu Asn
50 55 60

Glu Asn Tyr Lys Lys Ala Leu Glu Thr Gly Asn Ala Ile Pro Pro Lys
65 70 75 80

Ile Val Ala Val Asp Leu Gln Ala Met Ala Pro Leu Glu Gly Val Ile
85 90 95

Gln Ile Gln Gly Asp Ile Thr Asn Ile Asp Thr Ala Lys Gln Ile Ile
100 105 110

Ser His Phe Asp Asn Glu Gln Ala Asp Leu Val Val Cys Asp Gly Ala
115 120 125

Pro Asp Val Thr Gly Leu His Asp Met Asp Ile Tyr Ile Gln Ser Gln
130 135 140

Leu Leu Leu Ala Ala Leu Asn Ile Thr Thr His Ile Leu Lys Gln Gly
145 150 155 160

Gly Thr Phe Val Ala Lys Ile Phe Arg Ala Lys Asp Val Thr Leu Leu
165 170 175

Tyr Ala Gln Leu Lys Ile Phe Phe Pro Tyr Val Tyr Cys Thr Lys Pro
180 185 190

Ser Ser Ser Arg Asn Ser Ser Ile Glu Ala Phe Val Val Cys Lys Asp
195 200 205

Tyr Ser Pro Pro Glu Gly Tyr Lys Pro His Met Met Asn Pro Leu Leu
210 215 220

Thr His Lys Pro Cys Asp Phe Asn Asp Leu Thr Gly Ile Asn Arg Val
225 230 235 240

Ile Val Pro Phe Val Val Cys Gly Asp Leu Ser Gln Pro Asp Ser Asp
245 250 255

Thr Cys Tyr Pro Leu Asp Phe Glu Gly Lys Thr Tyr Thr Tyr His Glu
260 265 270

Pro Val Gln Thr Pro Ile Ser Pro Pro Tyr Lys Glu Ala Leu Ser Leu
275 280 285

Met Glu Asp Arg Asp Thr Asp Phe Arg Arg Phe Asp Val Asn Val Val
290 295 300

Val Asp Asn Leu Ser Ser Leu Thr Ile Glu Asp Phe Lys Lys Gln Ala
305 310 315 320

Lys Gln Lys His Lys Glu Ile Asn Glu Thr Lys Arg Ile Glu Leu Gln
325 330 335

Asp Asn Lys Lys Asp Asp Ser Glu Glu Asp Ile Leu Gly Leu Asp Lys
340 345 350

Leu Met Pro Thr Glu Leu Phe Asp Glu Ser Glu Asn Lys Asn Asn Asp
355 360 365

Asn Met
370

<210> 79
<211> 1093
<212> DNA
<213> Caenorhabditis elegans

<400> 79
atgggaaaaa catctcgtga caaaagggac atttactatc gtctggcaaa ggagaacaaa 60
tggcgagcac ggagtgcatt caaattgatg cagattgatg atgaatttca gattctgaaa 120
ggagtccgtc gagctgtaga tttgtgtgca gcacctggaa gttggtcaca agtgctatcg 180
aaaaggttat acgaggaaga tcaagaggca aaaattgttg cgattgatct tcagccgatg 240
gcaccgattc caggagttat tcaacttcaa ggagatatta cttccgttga tacagctaat 300
caggtcatca aacacttttc cggagaaaaa tcagatattg tgatttgcga cggagcccca 360
gatgtaaccg gaattcattc tctggacgaa ttcatgcaag ccgaactaat cctcgctgcc 420
ttcaacatca caagtcacgt tctcaaagaa ggcggcaact ttctcgcgaa aattttccga 480
tcccgaaact cctctcttct ctatgcacaa atgaagaagt attttaaaaa agtatatcta 540

gccaaaccac gaagttcccg tcaatccagt tgtgaagctt ttgtcctgtg tctcgactat 600
tcaccgcccg agggatttgt tccaacaatg ggaaagactt cattagacgc aactgatgct 660
tctgctattt cacctgatat tatcgatgga ttcgtgactt gtggcgatct aagtggatgg 720
gacagcgaga agtcttatcc gttggatatt gatgcttgtt ttcccaaagg agagattgat 780
gaagagcaga aaaaacgata cgagttcaaa gacgtcgttc agccaccaac agatcctgcc 840
tacaaagcag ctctcgataa gaagaaaagt ggagtttttg caaaaatgtc tgcagatttg 900
aacagacagc tgaaagctga acttagtcgt ggaaaggatc agaagaagac tccagcagag 960
aatgttccaa gcgtagaaga attggagaaa gctgctgaaa aatttcaact ataatttggt 1020
tatttttgtt ctcattgtca gttaattgat tttttttcac ttttttcgag tctcattatt 1080
agtatttgtt ttg 1093

<210> 80
<211> 337
<212> PRT
<213> Caenorhabditis elegans

<400> 80

Met Gly Lys Thr Ser Arg Asp Lys Arg Asp Ile Tyr Tyr Arg Leu Ala
1 5 10 15

Lys Glu Asn Lys Trp Arg Ala Arg Ser Ala Phe Lys Leu Met Gln Ile
20 25 30

Asp Asp Glu Phe Gln Ile Leu Lys Gly Val Arg Arg Ala Val Asp Leu
35 40 45

Cys Ala Ala Pro Gly Ser Trp Ser Gln Val Leu Ser Lys Arg Leu Tyr
50 55 60

Glu Glu Asp Gln Glu Ala Lys Ile Val Ala Ile Asp Leu Gln Pro Met
65 70 75 80

Ala Pro Ile Pro Gly Val Ile Gln Leu Gln Gly Asp Ile Thr Ser Val
85 90 95

Asp Thr Ala Asn Gln Val Ile Lys His Phe Ser Gly Glu Lys Ser Asp
100 105 110

Ile Val Ile Cys Asp Gly Ala Pro Asp Val Thr Gly Ile His Ser Leu
115 120 125

Asp Glu Phe Met Gln Ala Glu Leu Ile Leu Ala Ala Phe Asn Ile Thr
130 135 140

Ser His Val Leu Lys Glu Gly Gly Asn Phe Leu Ala Lys Ile Phe Arg
145 150 155 160

Ser Arg Asn Ser Ser Leu Leu Tyr Ala Gln Met Lys Lys Tyr Phe Lys
165 170 175

Lys Val Tyr Leu Ala Lys Pro Arg Ser Ser Arg Gln Ser Ser Cys Glu
180 185 190

Ala Phe Val Leu Cys Leu Asp Tyr Ser Pro Pro Glu Gly Phe Val Pro
195 200 205

Thr Met Gly Lys Thr Ser Leu Asp Ala Thr Asp Ala Ser Ala Ile Ser
210 215 220

Pro Asp Ile Ile Asp Gly Phe Val Thr Cys Gly Asp Leu Ser Gly Trp
225 230 235 240

Asp Ser Glu Lys Ser Tyr Pro Leu Asp Ile Asp Ala Cys Phe Pro Lys
245 250 255

Gly Glu Ile Asp Glu Glu Gln Lys Lys Arg Tyr Glu Phe Lys Asp Val
260 265 270

Val Gln Pro Pro Thr Asp Pro Ala Tyr Lys Ala Ala Leu Asp Lys Lys
275 280 285

Lys Ser Gly Val Phe Ala Lys Met Ser Ala Asp Leu Asn Arg Gln Leu
290 295 300

Lys Ala Glu Leu Ser Arg Gly Lys Asp Gln Lys Lys Thr Pro Ala Glu
305 310 315 320

Asn Val Pro Ser Val Glu Glu Leu Glu Lys Ala Ala Glu Lys Phe Gln
325 330 335

Leu

<210> 81
<211> 1433
<212> DNA
<213> Danio rerio

<400> 81
gaagaagaag aactgtcacg ttgcggcgaa aacaagcagc agtccaacaa taagtgagtg 60
aaggtgaaat ggggcgctca tccaaagaca aacgagatat ttactacaga cttgcgaagg 120
aggaaggatg gagggcgaga agtgccttca aactcctgca gctggatgag gagttcaagc 180
tgtttagagg tgttagtcgt gctgtggatc tgtgtgcagc acctggcagc tggagtcaag 240
tcctcagccg caaactacgt ggaaaagaca agagtgaaga ggtcaagatt gtggcagttg 300
atctgcaagc catggcacct ttaccgggcg tcacacaaat tcaaggagac atcaccaaga 360
tttctacagc agaggaaatt attcgacatt ttgaaggaga gtctgcagac ttggttgtgt 420
gtgacggtgc tcctgatgtg acagggcttc atgatgtaga tgagtatatt caggcacagc 480

```
tcctgttggc agctctcaat atcaccacac atgttctcaa accaggcggc aactttgttg    540
caaaaatttt caggggaaaa gatgtgaccc tgctgtactc gcagctaaag atcttcttca    600
gctttgtgac ctgtgccaaa ccacccagca gccgcaactc tagcattgag gcatttgtgg    660
tgtgtcagaa ttactctcca cctgagggtt acgttcccaa catgtccaac cctttactgg    720
atcactccta tgatgtcgac tttaaccagt tagagggacc aaaccgaata atagtccctt    780
tccttgcttg tggagatctc agtgggtttg attcagacag aacgtatcct cttcagcttg    840
attctagcaa agagtatcag tatttaccac caacccaacc tccaattcgg cctccctacc    900
agcaagcctg tcaattgcgc aagagtaacc tgcttgccaa agaggactcg ccgtccggtg    960
cgctggatga agccttgacc gctttagatc tcaacacaaa accagacaca agcacgacta   1020
caccaggagc ctcagaataa tggaattgta cagcttcaag ttttgcttga cactgtcaag   1080
aatgtgtttg tcacgggaac atctttgtac tttatttaca cctacatttt gatagtatta   1140
acaaacccac agaaattttt actcctagaa gatatttatt tagaggtaaa taaggtacaa   1200
aggtttctat gattctgtct ctgtgtaaag cttttattta atttttcatg catttctgga   1260
ttgtaattaa tgtaactttt aggcatttta ttctttctga ctgtattaca tttaccattc   1320
acaggattgt ttcagtaagt cgccgaaaca acctgatgac ttttaacctc agctttacat   1380
ggtaataaaa cactcaaatc taaaaaaaaa aaaaaaaaaa aaaaaaaaaa aaa          1433
```

<210> 82
<211> 323
<212> PRT
<213> Danio rerio

<400> 82

```
Met Gly Arg Ser Ser Lys Asp Lys Arg Asp Ile Tyr Tyr Arg Leu Ala
1               5                   10                  15

Lys Glu Glu Gly Trp Arg Ala Arg Ser Ala Phe Lys Leu Leu Gln Leu
            20                  25                  30

Asp Glu Glu Phe Lys Leu Phe Arg Gly Val Ser Arg Ala Val Asp Leu
        35                  40                  45

Cys Ala Ala Pro Gly Ser Trp Ser Gln Val Leu Ser Arg Lys Leu Arg
    50                  55                  60

Gly Lys Asp Lys Ser Glu Glu Val Lys Ile Val Ala Val Asp Leu Gln
65                  70                  75                  80

Ala Met Ala Pro Leu Pro Gly Val Thr Gln Ile Gln Gly Asp Ile Thr
                85                  90                  95

Lys Ile Ser Thr Ala Glu Glu Ile Ile Arg His Phe Glu Gly Glu Ser
            100                 105                 110
```

```
Ala Asp Leu Val Val Cys Asp Gly Ala Pro Asp Val Thr Gly Leu His
        115                 120                 125

Asp Val Asp Glu Tyr Ile Gln Ala Gln Leu Leu Leu Ala Ala Leu Asn
    130                 135                 140

Ile Thr Thr His Val Leu Lys Pro Gly Gly Asn Phe Val Ala Lys Ile
145                 150                 155                 160

Phe Arg Gly Lys Asp Val Thr Leu Leu Tyr Ser Gln Leu Lys Ile Phe
                165                 170                 175

Phe Ser Phe Val Thr Cys Ala Lys Pro Pro Ser Ser Arg Asn Ser Ser
            180                 185                 190

Ile Glu Ala Phe Val Val Cys Gln Asn Tyr Ser Pro Pro Glu Gly Tyr
        195                 200                 205

Val Pro Asn Met Ser Asn Pro Leu Leu Asp His Ser Tyr Asp Val Asp
    210                 215                 220

Phe Asn Gln Leu Glu Gly Pro Asn Arg Ile Ile Val Pro Phe Leu Ala
225                 230                 235                 240

Cys Gly Asp Leu Ser Gly Phe Asp Ser Asp Arg Thr Tyr Pro Leu Gln
                245                 250                 255

Leu Asp Ser Ser Lys Glu Tyr Gln Tyr Leu Pro Pro Thr Gln Pro Pro
            260                 265                 270

Ile Arg Pro Pro Tyr Gln Gln Ala Cys Gln Leu Arg Lys Ser Asn Leu
        275                 280                 285

Leu Ala Lys Glu Asp Ser Pro Ser Gly Ala Leu Asp Glu Ala Leu Thr
    290                 295                 300

Ala Leu Asp Leu Asn Thr Lys Pro Asp Thr Ser Thr Thr Thr Pro Gly

305                 310                 315                 320

Ala Ser Glu


<210>  83
<211>  1984
<212>  DNA
<213>  Homo sapiens

<400>  83
ggatacgtcg acaatagatg acggggctca cgttgtacgt tcacatcagg tcccggcccg      60

ccggaacctg ggcgatccac gatgccgagt ttgccacgct gcgacagccc ataggcttgc     120

ccccccgggc attcgggtgg actacgaaca caaactgaag ccctaggact tgtcgcccgt     180
```

ttgcgctctc gccgaggcac aggctgctcg cggaccaccc tgctccgaaa actaagaagg 240
ggaccagtag acggggtgga ttcgaggtgg ggacatggga cgcagcggtc ggagccgcct 300
ggaggggtac ccggtggtcc cgatggagct atcatctttg agaggtaggt ggtagcccat 360
tcatctggtt actgatactg gccggcatca gtggactgtc ggcaggtcct tgagcaactg 420
gtgtgtgaaa tgggacggac gtcaaaggac aagcgggatg tctactaccg cctggccaag 480
gagaatggct ggcgtgctcg cagcgccttc aaactgctac aactggataa ggaattccaa 540
ctcttccaag gcgtgacacg ggcagttgac ctgtgtgcag ccccaggcag ctggagccag 600
gtgctgagcc ggaagatcgg gggccaaggg tccggccacg tggtggctgt ggacctgcag 660
gctatggctc cactaccagg tgtggtacag atccaggggg acatcaccca gctgtccact 720
gccaaggaga tcatccagca ctttaagggc tgccctgcgg acctagtggt gtgtgacggg 780
gctcctgatg taaccggtct ccatgatgtt gatgagtata tgcaggccca gctcctccta 840
gctgctctga acattgctac acatgtcctg aagccagggg gctgctttgt ggccaagata 900
ttccgaggcc gggatgtgac gctcctctac agccagctgc aggtcttctt ctccagcgtg 960
ctgtgtgcca agcccaggag cagccggaac tctagcatcg aggccttcgc tgtctgtcag 1020
ggctatgacc ctcccgaggg cttcatcccg gacctgagca aacccctgct ggaccattct 1080
tacgatttca accagctgga tggtcccacc cgcatcattg tgccttttgt gacctgtggg 1140
gacctgagct cctatgattc ggaccgcagt tacccactgg acctagaggg cggctcagag 1200
tacaagtaca ctccacccac acagcccccc atctcgccac cataccagga ggcctgcacg 1260
ttgaagagga aggggcagct ggccaaggag atccgccccc aggactgccc catcagcaga 1320
gtggacacgt ttccccagcc cctggccgcc cctcagtgcc acaccctgct ggcccctgag 1380
atggaagaca atgaaatgag ttgttcacct taacccatta cgctggcaaa ctgatacaac 1440
tcagaaactg ctcaatgtca ggaaaaccgg aacttgttga tgaacttgtt ttcaaaatat 1500
catctcatca acgaggcctg gacaaacaag ccctgaggag ggatctgcaa caaccctgaa 1560
gacaacaagg aaagaaacca tgaaagtctg tctgtactgc agtgggaatt cttgagtgag 1620
gtcttacctc ttctttaaac ctcttcagga gtgtcctgat tatgtccaga attttcccta 1680
aaggcaggga ttcttaacct ggatagaagc caggggtaac catgaacttg atggaagaaa 1740
atgttacatc tttattttca gcaatgaaac tgaaatttag ccttactccc aagttataaa 1800
tgctggcaac aaatcacagt agtaaaagca gtacctggga ttttcccacc aatacaaacc 1860
agttacagca actgtaatgt aacataaaaa gggcatcttg aagtattgtt catatttgtc 1920
acctcttgga atgatagcag atcctgtaag ttgatgtatt aattaaaagc ccatatatta 1980
ctgc 1984

<210> 84
<211> 327
<212> PRT
<213> Homo sapiens

<400> 84

Met Gly Arg Thr Ser Lys Asp Lys Arg Asp Val Tyr Tyr Arg Leu Ala
1               5                   10                  15

Lys Glu Asn Gly Trp Arg Ala Arg Ser Ala Phe Lys Leu Leu Gln Leu
            20                  25                  30

Asp Lys Glu Phe Gln Leu Phe Gln Gly Val Thr Arg Ala Val Asp Leu
        35                  40                  45

Cys Ala Ala Pro Gly Ser Trp Ser Gln Val Leu Ser Arg Lys Ile Gly
    50                  55                  60

Gly Gln Gly Ser Gly His Val Val Ala Val Asp Leu Gln Ala Met Ala
65                  70                  75                  80

Pro Leu Pro Gly Val Val Gln Ile Gln Gly Asp Ile Thr Gln Leu Ser
                85                  90                  95

Thr Ala Lys Glu Ile Ile Gln His Phe Lys Gly Cys Pro Ala Asp Leu
            100                 105                 110

Val Val Cys Asp Gly Ala Pro Asp Val Thr Gly Leu His Asp Val Asp
        115                 120                 125

Glu Tyr Met Gln Ala Gln Leu Leu Leu Ala Ala Leu Asn Ile Ala Thr
    130                 135                 140

His Val Leu Lys Pro Gly Gly Cys Phe Val Ala Lys Ile Phe Arg Gly
145                 150                 155                 160

Arg Asp Val Thr Leu Leu Tyr Ser Gln Leu Gln Val Phe Phe Ser Ser
                165                 170                 175

Val Leu Cys Ala Lys Pro Arg Ser Ser Arg Asn Ser Ser Ile Glu Ala
            180                 185                 190

Phe Ala Val Cys Gln Gly Tyr Asp Pro Pro Glu Gly Phe Ile Pro Asp
        195                 200                 205

Leu Ser Lys Pro Leu Leu Asp His Ser Tyr Asp Phe Asn Gln Leu Asp
    210                 215                 220

Gly Pro Thr Arg Ile Ile Val Pro Phe Val Thr Cys Gly Asp Leu Ser
225                 230                 235                 240

Ser Tyr Asp Ser Asp Arg Ser Tyr Pro Leu Asp Leu Glu Gly Gly Ser
                245                 250                 255

Glu Tyr Lys Tyr Thr Pro Pro Thr Gln Pro Pro Ile Ser Pro Pro Tyr
            260                 265                 270

Gln Glu Ala Cys Thr Leu Lys Arg Lys Gly Gln Leu Ala Lys Glu Ile
        275                 280                 285

Arg Pro Gln Asp Cys Pro Ile Ser Arg Val Asp Thr Phe Pro Gln Pro
    290                 295                 300

Leu Ala Ala Pro Gln Cys His Thr Leu Leu Ala Pro Glu Met Glu Asp
305                 310                 315                 320

Asn Glu Met Ser Cys Ser Pro
                325

<210> 85
<211> 2466
<212> DNA
<213> Arabidopsis thaliana

<400> 85
atgggtaagg tcaaaggaaa gcatcgtttg gataagtact atcgtctcgc taaggaacgt 60
ggattccgtt ctcgagcttc ctacaagctt cttcagctcg acgccaagta ctctcttctc 120
cactcagctc atgccgttct cgatctctgc gccgcccctg gtggttggat gcaagtcgcc 180
gtcgagaaag ttcccgtcgg cagcctcgtt ctcggtatcg atcttgtccc gattctccct 240
gttcgtggct gtgttactat gacgcaagat atcacaagga ctgagtgtaa atcgaagatc 300
aagcaagtga tggagcagca tggtgttagt gcttttaatt tggttctgca cgatgggtct 360
ccaaatgttg gtggtgcgtg ggcacaagag gctatgagcc agaacgcttt ggttattgat 420
tctgttagat tagccactga gttcttagct cgcaacggga atttagtcac caaggttttc 480
aggtctcgag actacaactc tgtgctctac tgtcttggga ggctgtttga aaaagttgaa 540
gtgtttaagc cgccggctag tcgttcagca tctgcagaaa catatcttgt gggtttgaaa 600
tacttagccc ctgccaagat tgatccccgt cttcttgatt atagacatct ctttaaggag 660
tcagcagaac ccacgagaaa ggtggtggat gtgcttggag gatcaaaaca aaagagaaac 720
cgtgatgggt atgaagatgg ggagtccatt ttgagaagag ttgcatctgc tgctgatttc 780
atttggtctg aaaatcctct tgacgttctt ggcacgacta cttccatatc atttgatgat 840
caagcctctc tacctttaaa ggaacatgat ttgacgacag aggagattaa aattctatgc 900
gatgatcttc ctgtcttggg gaagaatgac ttcaagcata tcctaaaatg gcgaatgcaa 960
atcaggaaag ctctgactcc tgagaaaaag gaagttgcta aacctgagcc tgatgtggga 1020
aaggaagatg aggaaaatga agatgataaa ctactcaatg aattggaaga gctgacaaac 1080
acagttgacc gcaagaagaa acaggcaaag aagattcttg caaaacgaag ggctaaggac 1140
aaggcgcgga aggcgactgg tccacagatg gatgtacttg aagatggttt cgtcgacaat 1200
gaattgttct ctcttaatgc tatcaaggga aagaaagatc taatggcagt tgacaatgat 1260
gaagatgata atggcaatgc cgttgacagt gagaacgaag accatggcga aggagcttca 1320
gatgactcca aagacagtga cagagattct gatgaagaac gtcagaaata cactgaacaa 1380

atggaagaga tttttgagca ggcatatgag cggtatatgg tgaagaaaga aggaagcgca 1440
aagcaaagga agcgggctag gcaggcccat gctgaaaagc tggaggaagg tgatggagat 1500
gaagaaatga agatcgatta cgattcagat atgaatgaag aaaaggatga ggcaaacccct 1560
cttgttgtac cacttgatga tggagtggtc caaacaaagg aggaaatatc caaccagtgg 1620
ttcagtcaga atatatttgc agaagccgtg gaagaaggag acttgggaaa agatgacagt 1680
gaagacgaaa tagcaaataa gaaaaagagc aaaaatctgt ctaaaccaga caagtcaaag 1740
cagaaggctt caaaagcaag tgtgttgtct gatcagagct tgcccaatag ctccaagaag 1800
gaagatgagt ttgaggtagt ccctgcacca gccacagatt ctgactcaga ttcatcctct 1860
gaagatgatg ttcataccaa ggccgagata ctggcatgtg ccaaaaaaat gctgaggaag 1920
aagcagagag aacagatgct tgatgatgca tataacaaac acatgtttgt agatgaaggt 1980
ttaccaaaat ggtttgtgga tgacgagaag cagcacagac aaccaatgaa acctgttacg 2040
aaagatgaag ttaatgcaat gaaagcacag ttcaaagaga tcaatgctcg tccagccaag 2100
aaggtggcag aggccaaggc acggaagaag cgagctgccc agaaaagatt ggagaaagtg 2160
aggaagaagg caaacacaat atctgacaca gcagatatat cagaccgttc aaaggacaag 2220
atgatagaca agctatacaa gaaagcagca gagccaagaa agccaaggaa agaactggtt 2280
gtgtctaaga aaggagttgg ggttaaagtt ggtaagggac agaagagagt ggataggagg 2340
atgaagagtg atgataggaa acgtggggga ggaaagcccg gtaggaacgg gcagaagggc 2400
actggtaaag caggccagaa aggaaaaagg cctgctggga aacctcgggg aaggaaaccc 2460
gggtaa 2466

<210> 86
<211> 821
<212> PRT
<213> Arabidopsis thaliana

<400> 86

Met Gly Lys Val Lys Gly Lys His Arg Leu Asp Lys Tyr Tyr Arg Leu
1 5 10 15

Ala Lys Glu Arg Gly Phe Arg Ser Arg Ala Ser Tyr Lys Leu Leu Gln
20 25 30

Leu Asp Ala Lys Tyr Ser Leu Leu His Ser Ala His Ala Val Leu Asp
35 40 45

Leu Cys Ala Ala Pro Gly Gly Trp Met Gln Val Ala Val Glu Lys Val
50 55 60

Pro Val Gly Ser Leu Val Leu Gly Ile Asp Leu Val Pro Ile Leu Pro
65 70 75 80

Val Arg Gly Cys Val Thr Met Thr Gln Asp Ile Thr Arg Thr Glu Cys
85 90 95

Lys Ser Lys Ile Lys Gln Val Met Glu Gln His Gly Val Ser Ala Phe
100 105 110

Asn Leu Val Leu His Asp Gly Ser Pro Asn Val Gly Gly Ala Trp Ala
115 120 125

Gln Glu Ala Met Ser Gln Asn Ala Leu Val Ile Asp Ser Val Arg Leu
130 135 140

Ala Thr Glu Phe Leu Ala Arg Asn Gly Asn Leu Val Thr Lys Val Phe
145 150 155 160

Arg Ser Arg Asp Tyr Asn Ser Val Leu Tyr Cys Leu Gly Arg Leu Phe
165 170 175

Glu Lys Val Glu Val Phe Lys Pro Pro Ala Ser Arg Ser Ala Ser Ala
180 185 190

Glu Thr Tyr Leu Val Gly Leu Lys Tyr Leu Ala Pro Ala Lys Ile Asp
195 200 205

Pro Arg Leu Leu Asp Tyr Arg His Leu Phe Lys Glu Ser Ala Glu Pro
210 215 220

Thr Arg Lys Val Val Asp Val Leu Gly Gly Ser Lys Gln Lys Arg Asn
225 230 235 240

Arg Asp Gly Tyr Glu Asp Gly Glu Ser Ile Leu Arg Arg Val Ala Ser
245 250 255

Ala Ala Asp Phe Ile Trp Ser Glu Asn Pro Leu Asp Val Leu Gly Thr
260 265 270

Thr Thr Ser Ile Ser Phe Asp Asp Gln Ala Ser Leu Pro Leu Lys Glu
275 280 285

His Asp Leu Thr Thr Glu Glu Ile Lys Ile Leu Cys Asp Asp Leu Pro
290 295 300

Val Leu Gly Lys Asn Asp Phe Lys His Ile Leu Lys Trp Arg Met Gln
305 310 315 320

Ile Arg Lys Ala Leu Thr Pro Glu Lys Lys Glu Val Ala Lys Pro Glu
325 330 335

Pro Asp Val Gly Lys Glu Asp Glu Glu Asn Glu Asp Asp Lys Leu Leu
340 345 350

Asn Glu Leu Glu Glu Leu Thr Asn Thr Val Asp Arg Lys Lys Lys Gln
355 360 365

Ala Lys Lys Ile Leu Ala Lys Arg Arg Ala Lys Asp Lys Ala Arg Lys
370 375 380

Ala Thr Gly Pro Gln Met Asp Val Leu Glu Asp Gly Phe Val Asp Asn
385 390 395 400

Glu Leu Phe Ser Leu Asn Ala Ile Lys Gly Lys Lys Asp Leu Met Ala
405 410 415

Val Asp Asn Asp Glu Asp Asp Asn Gly Asn Ala Val Asp Ser Glu Asn
420 425 430

Glu Asp His Gly Glu Gly Ala Ser Asp Asp Ser Lys Asp Ser Asp Arg
435 440 445

Asp Ser Asp Glu Glu Arg Gln Lys Tyr Thr Glu Gln Met Glu Glu Ile
450 455 460

Phe Glu Gln Ala Tyr Glu Arg Tyr Met Val Lys Lys Glu Gly Ser Ala
465 470 475 480

Lys Gln Arg Lys Arg Ala Arg Gln Ala His Ala Glu Lys Leu Glu Glu
485 490 495

Gly Asp Gly Asp Glu Glu Met Lys Ile Asp Tyr Asp Ser Asp Met Asn
500 505 510

Glu Glu Lys Asp Glu Ala Asn Pro Leu Val Val Pro Leu Asp Asp Gly
515 520 525

Val Val Gln Thr Lys Glu Glu Ile Ser Asn Gln Trp Phe Ser Gln Asn
530 535 540

Ile Phe Ala Glu Ala Val Glu Glu Gly Asp Leu Gly Lys Asp Asp Ser
545 550 555 560

Glu Asp Glu Ile Ala Asn Lys Lys Lys Ser Lys Asn Leu Ser Lys Pro
565 570 575

Asp Lys Ser Lys Gln Lys Ala Ser Lys Ala Ser Val Leu Ser Asp Gln
580 585 590

Ser Leu Pro Asn Ser Ser Lys Lys Glu Asp Glu Phe Glu Val Val Pro
595 600 605

Ala Pro Ala Thr Asp Ser Asp Ser Asp Ser Ser Ser Glu Asp Asp Val
610 615 620

His Thr Lys Ala Glu Ile Leu Ala Cys Ala Lys Lys Met Leu Arg Lys
625 630 635 640

Lys Gln Arg Glu Gln Met Leu Asp Asp Ala Tyr Asn Lys His Met Phe
645 650 655

Val Asp Glu Gly Leu Pro Lys Trp Phe Val Asp Asp Glu Lys Gln His
660 665 670

Arg Gln Pro Met Lys Pro Val Thr Lys Asp Glu Val Asn Ala Met Lys
675 680 685

Ala Gln Phe Lys Glu Ile Asn Ala Arg Pro Ala Lys Lys Val Ala Glu
690 695 700

Ala Lys Ala Arg Lys Lys Arg Ala Ala Gln Lys Arg Leu Glu Lys Val
705 710 715 720

Arg Lys Lys Ala Asn Thr Ile Ser Asp Thr Ala Asp Ile Ser Asp Arg
725 730 735

Ser Lys Asp Lys Met Ile Asp Lys Leu Tyr Lys Lys Ala Ala Glu Pro
740 745 750

Arg Lys Pro Arg Lys Glu Leu Val Val Ser Lys Lys Gly Val Gly Val
755 760 765

Lys Val Gly Lys Gly Gln Lys Arg Val Asp Arg Arg Met Lys Ser Asp
770 775 780

Asp Arg Lys Arg Gly Gly Gly Lys Pro Gly Arg Asn Gly Gln Lys Gly
785 790 795 800

Thr Gly Lys Ala Gly Gln Lys Gly Lys Arg Pro Ala Gly Lys Pro Arg
805 810 815

Gly Arg Lys Pro Gly
820

<210> 87
<211> 675
<212> DNA
<213> Arabidopsis thaliana

<400> 87
atgagtgggg caggtgtacc ggactttttc tacagagaag ctcaacgtct tggctatgtt 60
gctcgctcag ctttcaagct tctacagatt caaaaacagt acaagctcat caaaccaggc 120
tcctctgttc ttgacctagg ttgcgcgcct ggtgcttggc ttcaggttgc ttgccaaagc 180
ttaggcccac ttagaagtgg tggaattgtc gttggtatgg atataaagaa ggtgaaggtt 240
cctccacagt gtgattctcg agtgcaaacc attgctgctg atgttttaaa ctttcccaga 300
caaaagattc gggagttatc acctcagcaa ttgggatttt cagtcattct ttcagatatg 360
tgtcactcag tatctggaat aaccactaga gatgcagctc tgtctgctga attgggaatg 420
cgagcacttg atttggctgt tggtcaagct gccatctctc agtcacctaa tgatgatgat 480

ggaggcccaa acgaaagtcg tcctggtgta cttaggcatg gtggccatct tgtgatcaag 540

cttctagaaa gcgaggatgc tcaagatttt gctcgaattt gcaagcctat cttcaacaag 600

gcatcttggt tgaggccaaa agctacaaga ccatcatctc gagaaattta cttgatttgc 660

cagggatttc gataa 675

<210> 88
<211> 224
<212> PRT
<213> Arabidopsis thaliana

<400> 88

Met Ser Gly Ala Gly Val Pro Asp Phe Phe Tyr Arg Glu Ala Gln Arg
1 5 10 15

Leu Gly Tyr Val Ala Arg Ser Ala Phe Lys Leu Leu Gln Ile Gln Lys
20 25 30

Gln Tyr Lys Leu Ile Lys Pro Gly Ser Ser Val Leu Asp Leu Gly Cys
35 40 45

Ala Pro Gly Ala Trp Leu Gln Val Ala Cys Gln Ser Leu Gly Pro Leu
50 55 60

Arg Ser Gly Gly Ile Val Val Gly Met Asp Ile Lys Lys Val Lys Val
65 70 75 80

Pro Pro Gln Cys Asp Ser Arg Val Gln Thr Ile Ala Ala Asp Val Leu
85 90 95

Asn Phe Pro Arg Gln Lys Ile Arg Glu Leu Ser Pro Gln Gln Leu Gly
100 105 110

Phe Ser Val Ile Leu Ser Asp Met Cys His Ser Val Ser Gly Ile Thr
115 120 125

Thr Arg Asp Ala Ala Leu Ser Ala Glu Leu Gly Met Arg Ala Leu Asp
130 135 140

Leu Ala Val Gly Gln Ala Ala Ile Ser Gln Ser Pro Asn Asp Asp Asp
145 150 155 160

Gly Gly Pro Asn Glu Ser Arg Pro Gly Val Leu Arg His Gly Gly His
165 170 175

Leu Val Ile Lys Leu Leu Glu Ser Glu Asp Ala Gln Asp Phe Ala Arg
180 185 190

Ile Cys Lys Pro Ile Phe Asn Lys Ala Ser Trp Leu Arg Pro Lys Ala
195 200 205

Thr Arg Pro Ser Ser Arg Glu Ile Tyr Leu Ile Cys Gln Gly Phe Arg
210 215 220

<210> 89
<211> 1212
<212> DNA
<213> Oryza sativa

<400> 89
atgagcggcg cgggcggcac cgccgacttc ttctaccgcg aggcgcagcg cctcggctac 60
gtcgcccgct ccgcgttcaa gctgatccag atacagaagc agcacaagct catcgccccc 120
ggcgccgccg tcctcgacct cggctgcgcc cccggcgcgt ggctccaggt ggcgtgccag 180
aacctgggtc cgctcgagaa gggcggggtg atcgtcggcg tcgatgtcaa gaaggtgaag 240
gttccatctg cgcactgtga ctcgagggtc aggaccgttt gcgccgatgt gatggctctg 300
atgaagcagc aagctcgggc aatgtcgcca caggaacgag gattctctgt aatattgtca 360
gacatgtgcc cagtagtttc tggaattaca accaaagatg cggctatatc ttgcgagctg 420
ggcatgcgtg ctctttcatt ggcagttggt aagatgaaag caaaagattc agattgcatt 480
gcaattttag agaagtttca gagctccact gagccagatc ctgatgaaga tggcattctc 540
cgtcgtggag gcagccttgt aattaagttt cttgagaatg aggatatacc aggttttggc 600
aaattttgca aagagaagtt caagaaagtg tccttattga gacccaaggc gacaagatct 660
agttcgaggg agattttcat ggtctgtgaa ggccgcggct tctcggtcgc gccacgctgg 720
catccacttt ctcatggccc ggcgacgtac gggacggct ccacgtgtca cgccgccgcc 780
cacagcttgg tcgaattgag aactgttggg cttcaaagag aggctcccaa gtgcagtgac 840
ctgaaagctc aagctcaacc tgcggcgttc gtgcggattc cgcggggcgc gtcccttccc 900
gccacccgca acgccgctgg actccactcc actctcctcg tcgcttcgtt gctgcacatc 960
accacccacc gcggtggcac tcacttctat attagcttaa gcgtggggtg ggagacgccg 1020
aggagggagg agtgccgcat cccggtggtg ccgccgcagt gcccggcgcc gccgaggaag 1080
aggccggtgg cgctgccgga gctggggaag gagcggcggg agccgcccaa gggcgggtac 1140
ttccagccgc cggacctcga gtcgctcttc gtgctcgcgc cgccgcggag gcaggcgtcc 1200
agctgcgcgt ag 1212

<210> 90
<211> 403
<212> PRT
<213> Oryza sativa

<400> 90

Met Ser Gly Ala Gly Gly Thr Ala Asp Phe Phe Tyr Arg Glu Ala Gln
1 5 10 15

Arg Leu Gly Tyr Val Ala Arg Ser Ala Phe Lys Leu Ile Gln Ile Gln
20 25 30

```
Lys Gln His Lys Leu Ile Ala Pro Gly Ala Ala Val Leu Asp Leu Gly
        35                  40                  45

Cys Ala Pro Gly Ala Trp Leu Gln Val Ala Cys Gln Asn Leu Gly Pro
    50                  55                  60

Leu Glu Lys Gly Gly Val Ile Val Gly Val Asp Val Lys Lys Val Lys
65                  70                  75                  80

Val Pro Ser Ala His Cys Asp Ser Arg Val Arg Thr Val Cys Ala Asp
                85                  90                  95

Val Met Ala Leu Met Lys Gln Gln Ala Arg Ala Met Ser Pro Gln Glu
            100                 105                 110

Arg Gly Phe Ser Val Ile Leu Ser Asp Met Cys Pro Val Val Ser Gly
        115                 120                 125

Ile Thr Thr Lys Asp Ala Ala Ile Ser Cys Glu Leu Gly Met Arg Ala
    130                 135                 140

Leu Ser Leu Ala Val Gly Lys Met Lys Ala Lys Asp Ser Asp Cys Ile
145                 150                 155                 160

Ala Ile Leu Glu Lys Phe Gln Ser Ser Thr Glu Pro Asp Pro Asp Glu
                165                 170                 175

Asp Gly Ile Leu Arg Arg Gly Gly Ser Leu Val Ile Lys Phe Leu Glu
            180                 185                 190

Asn Glu Asp Ile Pro Gly Phe Gly Lys Phe Cys Lys Glu Lys Phe Lys
        195                 200                 205

Lys Val Ser Leu Leu Arg Pro Lys Ala Thr Arg Ser Ser Ser Arg Glu
    210                 215                 220

Ile Phe Met Val Cys Glu Gly Arg Gly Phe Ser Val Ala Pro Arg Trp
225                 230                 235                 240

His Pro Leu Ser His Gly Pro Ala Thr Tyr Gly Thr Gly Ser Thr Cys
                245                 250                 255

His Ala Ala Ala His Ser Leu Val Glu Leu Arg Thr Val Gly Leu Gln
            260                 265                 270

Arg Glu Ala Pro Lys Cys Ser Asp Leu Lys Ala Gln Ala Gln Pro Ala
        275                 280                 285

Ala Phe Val Arg Ile Pro Arg Gly Ala Ser Leu Pro Ala Thr Arg Asn
    290                 295                 300

Ala Ala Gly Leu His Ser Thr Leu Leu Val Ala Ser Leu Leu His Ile
```

305 310 315 320

Thr Thr His Arg Gly Gly Thr His Phe Tyr Ile Ser Leu Ser Val Gly
325 330 335

Trp Glu Thr Pro Arg Arg Glu Glu Cys Arg Ile Pro Val Val Pro Pro
340 345 350

Gln Cys Pro Ala Pro Pro Arg Lys Arg Pro Val Ala Leu Pro Glu Leu
355 360 365

Gly Lys Glu Arg Arg Glu Pro Pro Lys Gly Gly Tyr Phe Gln Pro Pro
370 375 380

Asp Leu Glu Ser Leu Phe Val Leu Ala Pro Pro Arg Arg Gln Ala Ser
385 390 395 400

Ser Cys Ala

<210> 91
<211> 1187
<212> DNA
<213> Oryza sativa

<400> 91
ggtcagccaa tacattgatc cgttgccaat catgcaaagt attttggctg tggccgagtg 60
ccggaattga taattgtgtt ctgactaaat taaatgacca gaagtcgcta tcttccaatg 120
tatccgaaac ctggattaaa caatcctgtt ctgttctcta gcccctcctg catggccgga 180
ttgttttttt gacatgtttt cttgactgag gcctgtttgt tctaaacttt ttcttcaaac 240
ttttaacttt ttcatcacat cagaactttt ctacacacat aaacttttaa cttttctgtc 300
acatcgttcc aatttcaatc aaacttttaa ttttggcttg aactaaacac accctgagtc 360
ttttattgct cctccatacg ggttggctgg ttgagaatag gtattttcag agagaaaatc 420
tggatattgg gaggaggaac ttggcatgaa tggccactat atttagagca attctacggt 480
ctttgaggag gtaccatgag gtaccaaaat tttagtgtaa attttagtat cttcttaagg 540
accgtaaaat tgctcctata tttaagggat gtttatatct atccatatcc ataatttgat 600
tttgataaga aaaaatgtga gcacaccaag catgtccatg accttgcact cttggctcac 660
tcgtcaactg tgaagaacct aaaaaatgct caatatagct acaggtgcct gaaaaaataa 720
ctttaaagtt ttgaacatcg atttcactaa acaacaatta ttatctccct ctgaaatgtt 780
gctacctaag atgatagttt agaactctag aatcattgtc ggcggagaaa gtaaattatt 840
ttccccaaat ttccagctat gaaaaaaccc tcaccaaaca ccatcaaaca agagttcacc 900
aaaccgccca tgcggccatg ctgtcacgca acgcaccgca ttgcctgatg gccgctcgat 960
gcatgcatgc ttccccgtgc acatatccga cagacgcgcc gtgtcagcga gctcctcgac 1020
cgacctgtgt agcccatgca agcatccacc cccgccacgt acaccccctc ctcctcccta 1080

cgtgtcaccg ctctctccac ctatatatgc ccacctggcc cctctcctcc catctccact 1140

tcacccgatc gcttcttctt cttcttcgtt gcattcatct tgctagc 1187

<210> 92
<211> 951
<212> DNA
<213> Hordeum vulgare

<400> 92
atgggcaagg cctccaaaga caagcgggac atatactatc ggaaggccaa ggaggagggg 60

tggagggctc gcagcgcctt caagctaatg cagatcgacc aggagttcaa catcttccac 120

ggagtgaagc gcgccgttga cctgtgcgct gcccctggga gctggagcca ggttttgagc 180

cgcaatttgt acctgccggc aaagctatca tctgacggca aagatggcgg ccttcctctc 240

atcgtcgcaa tcgatctgca gccgatggct cctatagaag gtgtcataca agtgcagggc 300

gacatcacca atgctcgaac agcggaagtg gttatcaggc acttcgatgg atgcaaagcg 360

gacttggttg tctgtgatgg tgcccctgat gttactgggc ttcatgatat ggatgagttt 420

gttcagtccc agcttatatt ggcggcactg acaattgtga ctcatgtact caaagttggt 480

ggaaaatttg ttgcgaagat tttccggggt aaagatacaa gtctcctgta ttgccagtta 540

aagttgttct tctcacaagt tacatttgca aagccaaaaa gcagccgtaa ctcaagtatc 600

gaggcatttg cagtttgcga gaactactca cctccagagg gcttcaaaga gaaagacttg 660

tatcacctgc tggagaaagt gggaactcct tctggggctg atgatttaga ttgccgaagc 720

ggatggttgg agggaccaaa caaggtctac attccgtttc tggcttgcgg tgacctcagc 780

ggttatgatt cggaccgttc atacccccctc ccgagcacag aaggtggcac ctaccagagc 840

ctagatccag tccagcctcc catcgcgccg ccatacaaaa ctgcgttgga gatgaagaag 900

gcgtctagcc acggcgccgg cgcagatact agcaaatcat atgtcgaccc c 951

<210> 93
<211> 317
<212> PRT
<213> Hordeum vulgare

<400> 93

Met Gly Lys Ala Ser Lys Asp Lys Arg Asp Ile Tyr Tyr Arg Lys Ala
1 5 10 15

Lys Glu Glu Gly Trp Arg Ala Arg Ser Ala Phe Lys Leu Met Gln Ile
20 25 30

Asp Gln Glu Phe Asn Ile Phe His Gly Val Lys Arg Ala Val Asp Leu
35 40 45

Cys Ala Ala Pro Gly Ser Trp Ser Gln Val Leu Ser Arg Asn Leu Tyr
50 55 60

Leu Pro Ala Lys Leu Ser Ser Asp Gly Lys Asp Gly Gly Leu Pro Leu
65 70 75 80

Ile Val Ala Ile Asp Leu Gln Pro Met Ala Pro Ile Glu Gly Val Ile
85 90 95

Gln Val Gln Gly Asp Ile Thr Asn Ala Arg Thr Ala Glu Val Val Ile
100 105 110

Arg His Phe Asp Gly Cys Lys Ala Asp Leu Val Val Cys Asp Gly Ala
115 120 125

Pro Asp Val Thr Gly Leu His Asp Met Asp Glu Phe Val Gln Ser Gln
130 135 140

Leu Ile Leu Ala Ala Leu Thr Ile Val Thr His Val Leu Lys Val Gly
145 150 155 160

Gly Lys Phe Val Ala Lys Ile Phe Arg Gly Lys Asp Thr Ser Leu Leu
165 170 175

Tyr Cys Gln Leu Lys Leu Phe Phe Ser Gln Val Thr Phe Ala Lys Pro
180 185 190

Lys Ser Ser Arg Asn Ser Ser Ile Glu Ala Phe Ala Val Cys Glu Asn
195 200 205

Tyr Ser Pro Pro Glu Gly Phe Lys Glu Lys Asp Leu Tyr His Leu Leu
210 215 220

Glu Lys Val Gly Thr Pro Ser Gly Ala Asp Asp Leu Asp Cys Arg Ser
225 230 235 240

Gly Trp Leu Glu Gly Pro Asn Lys Val Tyr Ile Pro Phe Leu Ala Cys
245 250 255

Gly Asp Leu Ser Gly Tyr Asp Ser Asp Arg Ser Tyr Pro Leu Pro Ser
260 265 270

Thr Glu Gly Gly Thr Tyr Gln Ser Leu Asp Pro Val Gln Pro Pro Ile
275 280 285

Ala Pro Pro Tyr Lys Thr Ala Leu Glu Met Lys Lys Ala Ser Ser His
290 295 300

Gly Ala Gly Ala Asp Thr Ser Lys Ser Tyr Val Asp Pro
305 310 315

<210> 94
<211> 1414
<212> DNA
<213> Sorghum bicolor

<400> 94
gcacgagggc ccagtgggct caaaacactt ggcggcgcca cctcgccctc ctccgactac 60
tcgctcgctc gtcccgcccc gtcttccgcc cccgcctccg cccccgccgc ccttggctcg 120
caccagcgcc acctcgccct cctccgacta ctcgctcgct cgtcccgccc cgtcttccgc 180
ctccgcctcc gccgccgccg cccttggttc gcctcatcgt cgtcgccatg ggcaaggcct 240
ccaaggacaa gagagacatc tactaccgga aggccaaaga ggagggatgg agagctcgca 300
gcgccttcaa gctcctccaa atagaccagg agttcaacat cttccatgga gtgaagcgcg 360
tggttgatct gtgtgctgct cccggaagct ggagccaggt tttgagccgg aacttgtatg 420
taccagcaaa acagtctcct gattgcaagg aaggtgacct tcctctcatc gttgcaattg 480
acttgcaacc aatggccccg atagaaggtg ttatacaagt gcagggtgac atcaccaatg 540
ctcggacagc ggaagtggtt attaggcatt ttgatggatg caaagcagac ttggttgttt 600
gcgatggagc tccggatgtt actggccttc atgatatgga cgaatttgtt cagtcccagc 660
ttatacttgc ggcattgact atcgtgactc atgtactcaa agttggtgga aaatttgtcg 720
caaagatatt tcggggtaaa gatacaagtc tcctgtactg ccagctaaaa ctgttcttct 780
cacaagttac atttgcgaag ccaaaaagta gccggaactc aagcattgag gcatttgcag 840
tttgtgagaa ctattcacct ccagaagggt tcaaggagga agatctatac cacctgctag 900
agaaagtagg gactccttca ggagttgacg atttagattg cagaagcgga tggctcgagg 960

gaccaaacaa ggtgtatatc ccgttccttg cctgtgggga tctcagtggc tacgactccg 1020
accgctcata cccgctccca atcacagacg gtggctccta ccggagcttg gaccccgtcc 1080
agcctcccat cgccccgcct tacaaaaccg cccttcagat gaagaaggcc tccagccaca 1140
gcgccagcag ggacgccatg aaaccatcca cagactcttg agctgcaacc tgggccagcc 1200
tgaaccccca ggcgatgatg tttgtcccac actggtagca tcgctgggtc ctggacctgg 1260
ctctctcctg agcctcgtct attaaaaagg aaaaagaaaa tttgggaaca ttttgtgtcg 1320
aggtgggaca ttttgtggta gttttgagct gtggctccgc agcatgtacc tgatcactac 1380
tgattacgca gagatcctgc ttgtgtcctt gtgc 1414

<210> 95
<211> 317
<212> PRT
<213> Sorghum bicolor

<400> 95

Met Gly Lys Ala Ser Lys Asp Lys Arg Asp Ile Tyr Tyr Arg Lys Ala
1 5 10 15

Lys Glu Glu Gly Trp Arg Ala Arg Ser Ala Phe Lys Leu Leu Gln Ile
20 25 30

Asp Gln Glu Phe Asn Ile Phe His Gly Val Lys Arg Val Val Asp Leu
35 40 45

Cys Ala Ala Pro Gly Ser Trp Ser Gln Val Leu Ser Arg Asn Leu Tyr
50 55 60

Val Pro Ala Lys Gln Ser Pro Asp Cys Lys Glu Gly Asp Leu Pro Leu
65 70 75 80

Ile Val Ala Ile Asp Leu Gln Pro Met Ala Pro Ile Glu Gly Val Ile
85 90 95

Gln Val Gln Gly Asp Ile Thr Asn Ala Arg Thr Ala Glu Val Val Ile
100 105 110

Arg His Phe Asp Gly Cys Lys Ala Asp Leu Val Val Cys Asp Gly Ala
115 120 125

Pro Asp Val Thr Gly Leu His Asp Met Asp Glu Phe Val Gln Ser Gln
130 135 140

Leu Ile Leu Ala Ala Leu Thr Ile Val Thr His Val Leu Lys Val Gly
145 150 155 160

Gly Lys Phe Val Ala Lys Ile Phe Arg Gly Lys Asp Thr Ser Leu Leu
165 170 175

Tyr Cys Gln Leu Lys Leu Phe Phe Ser Gln Val Thr Phe Ala Lys Pro
180 185 190

Lys Ser Ser Arg Asn Ser Ser Ile Glu Ala Phe Ala Val Cys Glu Asn
195 200 205

Tyr Ser Pro Pro Glu Gly Phe Lys Glu Glu Asp Leu Tyr His Leu Leu
210 215 220

Glu Lys Val Gly Thr Pro Ser Gly Val Asp Asp Leu Asp Cys Arg Ser
225 230 235 240

Gly Trp Leu Glu Gly Pro Asn Lys Val Tyr Ile Pro Phe Leu Ala Cys
245 250 255

Gly Asp Leu Ser Gly Tyr Asp Ser Asp Arg Ser Tyr Pro Leu Pro Ile
260 265 270

Thr Asp Gly Gly Ser Tyr Arg Ser Leu Asp Pro Val Gln Pro Pro Ile
275 280 285

Ala Pro Pro Tyr Lys Thr Ala Leu Gln Met Lys Lys Ala Ser Ser His
290 295 300

Ser Ala Ser Arg Asp Ala Met Lys Pro Ser Thr Asp Ser
305 310 315

<210> 96
<211> 1008
<212> DNA
<213> Brassica napus

<400> 96
atgggaaaag cttctcggga taaaagggat atatactatc ggaaagccaa agaagaaggg 60

tggcgtgcaa gaagtgcctt taagcttctt cagattgatg aggagttcaa catttttcaa 120

ggagtgaaga gggttgtaga tttatgtgct gcacctggta gctggagtca ggttctgagt 180

cgtcaactgt atcttcctgc aaagtattca gctgagtcaa aagaagaaga tcttcctctt 240

attgtggcca tagatttgca gcctatggct ccaatcgaag gtgtaatcca agttcaaggg 300

gacataacta atgctcggac tgccgaagtg gtcattagac attttgacgg ttgcaaggct 360

gacctggttg tgtgtgatgg tgctccagat gttaccggtt tgcatgacat ggatgaattt 420

gtccagtccc aactcatact agcgggctta acgattgtaa cccatattct taaagaaggt 480

ggaaaattca ttgcaaagat attccgtgga aaagatacaa gtctcttgta ctgtcagctg 540

aagttgtttt tcccaactgt gacttttgcg aaacctaaaa gcagccgcaa ttctagtata 600

gaagcttttg ctgtctgcga aaattactcc ccaccagaag gatttaaccc gagagatctg 660

catcttctct tggagaaagt cggaagccct tcaggtggaa gcgatctcga ttgcagtagt 720

ggttggctcg agggacccaa caaagtttat attccattct tggcatgtgg tgacttaagc 780

ggttatgact cggaccggtc ttacccactc ccaaaagagg cagatggatc gtcataccga 840

agcctggacc cgattcagcc tccgatcgca ccgccttata aacgagctat tgagctcaag 900

aaagcttcag cacaaagcct caactcttaa agctaggctc gataacaata ataatgctaa 960

acaaagacac agaaagaagt ttttcattcg attcttagaa ggctgtag 1008

<210> 97
<211> 309
<212> PRT
<213> Brassica napus

<400> 97

Met Gly Lys Ala Ser Arg Asp Lys Arg Asp Ile Tyr Tyr Arg Lys Ala
1 5 10 15

Lys Glu Glu Gly Trp Arg Ala Arg Ser Ala Phe Lys Leu Leu Gln Ile
20 25 30

Asp Glu Glu Phe Asn Ile Phe Gln Gly Val Lys Arg Val Val Asp Leu
35 40 45

Cys Ala Ala Pro Gly Ser Trp Ser Gln Val Leu Ser Arg Gln Leu Tyr
50 55 60

Leu Pro Ala Lys Tyr Ser Ala Glu Ser Lys Glu Glu Asp Leu Pro Leu
65 70 75 80

Ile Val Ala Ile Asp Leu Gln Pro Met Ala Pro Ile Glu Gly Val Ile
85 90 95

Gln Val Gln Gly Asp Ile Thr Asn Ala Arg Thr Ala Glu Val Val Ile
100 105 110

Arg His Phe Asp Gly Cys Lys Ala Asp Leu Val Val Cys Asp Gly Ala
115 120 125

Pro Asp Val Thr Gly Leu His Asp Met Asp Glu Phe Val Gln Ser Gln
130 135 140

Leu Ile Leu Ala Gly Leu Thr Ile Val Thr His Ile Leu Lys Glu Gly
145 150 155 160

Gly Lys Phe Ile Ala Lys Ile Phe Arg Gly Lys Asp Thr Ser Leu Leu
165 170 175

Tyr Cys Gln Leu Lys Leu Phe Phe Pro Thr Val Thr Phe Ala Lys Pro
180 185 190

Lys Ser Ser Arg Asn Ser Ser Ile Glu Ala Phe Ala Val Cys Glu Asn
195 200 205

Tyr Ser Pro Pro Glu Gly Phe Asn Pro Arg Asp Leu His Leu Leu Leu
210 215 220

Glu Lys Val Gly Ser Pro Ser Gly Gly Ser Asp Leu Asp Cys Ser Ser
225 230 235 240

Gly Trp Leu Glu Gly Pro Asn Lys Val Tyr Ile Pro Phe Leu Ala Cys
245 250 255

Gly Asp Leu Ser Gly Tyr Asp Ser Asp Arg Ser Tyr Pro Leu Pro Lys
260 265 270

Glu Ala Asp Gly Ser Ser Tyr Arg Ser Leu Asp Pro Ile Gln Pro Pro
275 280 285

Ile Ala Pro Pro Tyr Lys Arg Ala Ile Glu Leu Lys Lys Ala Ser Ala
290 295 300

Gln Ser Leu Asn Ser
305

<210> 98
<211> 1446
<212> DNA
<213> Glycine max

<400> 98
gagaagcaaa caaaccctag cgcagagaag agatattctt cttcgcagaa tcattgcagt 60

```
tgcgagcagg ccgaagaaac ctaacctgca ttcgaaaatc agcccccttg gaaatcccaa     120
cactagtgtg gtacctgtgc tcgacgattg ggtgttcaag gggaattagc ttcgtctcgc     180
agaacttcaa cgcatcattc gtgaccttcg caaacgcggg gtttgttctt atttggtttt     240
gccagttgtt gccatcaggg gtttgtttct gaaatttgtg gcagggcaag tgagcgataa     300
accatgggga gagcttcaag ggataagagg gatatctatt accggaaagc aaaagaagaa     360
ggttggcgag ctcgaagtgc gtttaaactc cttcagatag acgaggaatt caaccttttt     420
gatggagtga agcgtgttgt agatttatgt gctgccccag gtagctggag tcaggttttg     480
agtcgtaaat tgtatctccc agccaagctt gcacctgatg caaaggatga aaatcttcct     540
cttattgtag ctattgattt gcaaccaatg gctccaattg aaggtgtcat ccaggtgcag     600
ggtgatataa ctaacgctcg gacagctgaa gtggtcatta gacattttga tggttgcaag     660
gctgacctag ttgtgtgtga tggtgcgcct gatgttactg gccttcatga catggatgaa     720
tttgtacaat cccaactctt acttgcaggg ttgacaattg ttactcatgt acttaaggaa     780
ggagggaagt ttattgcaaa gatatttaga ggaaaggaca caagccttct atattgtcag     840
ctaaaattat ttttccctgt agtgacgttc gcaaaaccaa aaagcagccg taattccagc     900
atagaggcat ttgcagtttg tgaaaactat tcccctcctg aaggattcaa tccaaaagat     960
cttcatcgcc ttcttgagaa ggttggaagt ccctcagggg tagatgatac agattgttgt    1020
agtggttggc tggaaggccc taataaggtg tatatcccat ttctagcttg tggtgacctc    1080
agtgggtatg attctgatcg ctcatatcca ctacctaaag ttgccggggg aacatatcag    1140
agcttggatc ctgtgcagcc ccctattgcc ccaccttaca agagagcttt ggagttaaaa    1200
aaagcttcca gtcaaggatt ccgtgaactt gaaaacctct cattggattc ttgatcgtgc    1260
tatgcactgt ttattcatca tgtcgtttgg aattttagtt tttgttacaa tgatatgatt    1320
tgtgttttga taattattag cgtacgtgtg aactttaaca ctttttctc ttgtaccatc     1380
attgcatgcc tagcgagtct tttgttatcg caatttttg aaatgaatac aactatttta     1440
taaaaa                                                               1446
```

```
<210>  99
<211>  316
<212>  PRT
<213>  Glycine max

<400>  99

Met Gly Arg Ala Ser Arg Asp Lys Arg Asp Ile Tyr Tyr Arg Lys Ala
1               5                   10                  15

Lys Glu Glu Gly Trp Arg Ala Arg Ser Ala Phe Lys Leu Leu Gln Ile
            20                  25                  30

Asp Glu Glu Phe Asn Leu Phe Asp Gly Val Lys Arg Val Val Asp Leu
        35                  40                  45

Cys Ala Ala Pro Gly Ser Trp Ser Gln Val Leu Ser Arg Lys Leu Tyr
```

50 55 60

Leu Pro Ala Lys Leu Ala Pro Asp Ala Lys Asp Glu Asn Leu Pro Leu
65 70 75 80

Ile Val Ala Ile Asp Leu Gln Pro Met Ala Pro Ile Glu Gly Val Ile
85 90 95

Gln Val Gln Gly Asp Ile Thr Asn Ala Arg Thr Ala Glu Val Val Ile
100 105 110

Arg His Phe Asp Gly Cys Lys Ala Asp Leu Val Val Cys Asp Gly Ala
115 120 125

Pro Asp Val Thr Gly Leu His Asp Met Asp Glu Phe Val Gln Ser Gln
130 135 140

Leu Leu Leu Ala Gly Leu Thr Ile Val Thr His Val Leu Lys Glu Gly
145 150 155 160

Gly Lys Phe Ile Ala Lys Ile Phe Arg Gly Lys Asp Thr Ser Leu Leu
165 170 175

Tyr Cys Gln Leu Lys Leu Phe Phe Pro Val Val Thr Phe Ala Lys Pro
180 185 190

Lys Ser Ser Arg Asn Ser Ser Ile Glu Ala Phe Ala Val Cys Glu Asn
195 200 205

Tyr Ser Pro Pro Glu Gly Phe Asn Pro Lys Asp Leu His Arg Leu Leu
210 215 220

Glu Lys Val Gly Ser Pro Ser Gly Val Asp Asp Thr Asp Cys Cys Ser
225 230 235 240

Gly Trp Leu Glu Gly Pro Asn Lys Val Tyr Ile Pro Phe Leu Ala Cys
245 250 255

Gly Asp Leu Ser Gly Tyr Asp Ser Asp Arg Ser Tyr Pro Leu Pro Lys
260 265 270

Val Ala Gly Gly Thr Tyr Gln Ser Leu Asp Pro Val Gln Pro Pro Ile
275 280 285

Ala Pro Pro Tyr Lys Arg Ala Leu Glu Leu Lys Lys Ala Ser Ser Gln
290 295 300

Gly Phe Arg Glu Leu Glu Asn Leu Ser Leu Asp Ser
305 310 315

<210> 100

<211> 1201
<212> DNA
<213> Zea mays

<400> 100
ccacgtcctc ctccggctac tcgctcgttc cgccccggcg tcccgccctc agccgccgcc 60

gtcgcccccct ccctcggttc gcctcgtcgt cgtcatgggc aaggcctcca aggacaagag 120

ggacatctac taccggaagg ccaaagagga aggctggaga gctcgcagcg ccttcaagct 180

cctccagata gaccaggagt tcaacatctt ccatggagtg aagcatgtgg ttgacctgtg 240

tgctgctccc ggaagttgga gccaggtttt gagccggaac ctgtatgtac cagcaaaaca 300

gtcttctgat tgcaaggaag gtgatcttcc tctcatcgtc gcaattgact tgcaaccaat 360

ggccccaata gaaggtgtta tacaagtgca gggcgacatc accaatgctc ggacagcaga 420

cgtggttatt aggcattttg atggatgcaa agcagacttg gttgtttgcg atggagcccc 480

ggatgttact ggccttcatg atatggatga atttgttcag tcccagctta tactcgcggc 540

attggctatc gtgactcatg tactcaaagt tggtggaaaa tttgtggcga agatatttcg 600

gggtaaagat acaagtctcc tgtactgcca gctaaaactg ttcttctcgc aagttacgtt 660

cgccaagcca aaaagtagcc ggaactcaag catcgaggcg tttgcggtct gtgagaacta 720

ctcacctcct gaagggttca aggaggaaga cctataccac ctgctagaga aagtgggtac 780

tccctcagga gctggcgatc tagattgcag aagcggatgg ctggagggac caaacaaggt 840

gtacatcccg ttcctggcct gcggggatct cagcggctac gactccgacc gctcgtaccc 900

gctgcccagc agcacggacg gtggctcgta ccggagcctg gaccccgtgc agcctcccat 960

cgccccgcct tacaagaccg ccctgcagat gaagaaggct tccagccacg gcgccggcgc 1020

ggacgccatc aagccgtccg cagactcctg agccgcagac cgtttccgtt taccaccttg 1080

cgcggtggaa gttgctttcg ctgggtcctg gacctggctc tctagtctct ctctacagaa 1140

acagaacctt catctacgaa actaaaaaaa aaggaaaaag gattggaaaa aaaaaaaaaa 1200

a 1201

<210> 101
<211> 318
<212> PRT
<213> Zea mays

<400> 101

Met Gly Lys Ala Ser Lys Asp Lys Arg Asp Ile Tyr Tyr Arg Lys Ala
1 5 10 15

Lys Glu Glu Gly Trp Arg Ala Arg Ser Ala Phe Lys Leu Leu Gln Ile
20 25 30

Asp Gln Glu Phe Asn Ile Phe His Gly Val Lys His Val Val Asp Leu
35 40 45

Cys Ala Ala Pro Gly Ser Trp Ser Gln Val Leu Ser Arg Asn Leu Tyr
50 55 60

```
Val Pro Ala Lys Gln Ser Ser Asp Cys Lys Glu Gly Asp Leu Pro Leu
65                  70                  75                  80

Ile Val Ala Ile Asp Leu Gln Pro Met Ala Pro Ile Glu Gly Val Ile
                85                  90                  95

Gln Val Gln Gly Asp Ile Thr Asn Ala Arg Thr Ala Asp Val Val Ile
            100                 105                 110

Arg His Phe Asp Gly Cys Lys Ala Asp Leu Val Val Cys Asp Gly Ala
        115                 120                 125

Pro Asp Val Thr Gly Leu His Asp Met Asp Glu Phe Val Gln Ser Gln
    130                 135                 140

Leu Ile Leu Ala Ala Leu Ala Ile Val Thr His Val Leu Lys Val Gly
145                 150                 155                 160

Gly Lys Phe Val Ala Lys Ile Phe Arg Gly Lys Asp Thr Ser Leu Leu
                165                 170                 175

Tyr Cys Gln Leu Lys Leu Phe Phe Ser Gln Val Thr Phe Ala Lys Pro
            180                 185                 190

Lys Ser Ser Arg Asn Ser Ser Ile Glu Ala Phe Ala Val Cys Glu Asn
        195                 200                 205

Tyr Ser Pro Pro Glu Gly Phe Lys Glu Glu Asp Leu Tyr His Leu Leu
    210                 215                 220

Glu Lys Val Gly Thr Pro Ser Gly Ala Gly Asp Leu Asp Cys Arg Ser
225                 230                 235                 240

Gly Trp Leu Glu Gly Pro Asn Lys Val Tyr Ile Pro Phe Leu Ala Cys
                245                 250                 255

Gly Asp Leu Ser Gly Tyr Asp Ser Asp Arg Ser Tyr Pro Leu Pro Ser
            260                 265                 270

Ser Thr Asp Gly Gly Ser Tyr Arg Ser Leu Asp Pro Val Gln Pro Pro
        275                 280                 285

Ile Ala Pro Pro Tyr Lys Thr Ala Leu Gln Met Lys Lys Ala Ser Ser
    290                 295                 300

His Gly Ala Gly Ala Asp Ala Ile Lys Pro Ser Ala Asp Ser
305                 310                 315

<210>  102
<211>  732
<212>  DNA
```

<213> Tagetes erecta

<400> 102
aaagcaaaag aagaaggttg gcgtgcacga agtgcgttta agctccttca aattgatgag 60

gagtttaaca tctttgaagg agtgaagcgt gtggtcgatt tatgcgcagc acctggtagc 120

tggagtcagg ttttaagtcg taagctgtat ctcccagcta aacagtcatc tgaccttccg 180

cttatcgtgg ctattgattt acaacccatg gctccaattg aaggtgtgat ccaagttcaa 240

ggagatatta caaatgctag aaccgctgaa gtggttatta gacattttga cggatgcaag 300

gctgacctag ttgtctgtga cggtgctcct gatgtaactg gacttcatga catggacgaa 360

tttgttcagt cacaactgat attggcgggg ttgacaattg tcactcacat tctcaagaaa 420

ggtggaaagt ttattgcaaa gatcttcagg ggaaaggata cgagcctctt atattgtcag 480

ctaaaactat ttttcacaga ggttacgctt gcaaaaccaa aaagtagtcg aaattcaagc 540

atagaggcat ttgctgtttg tgagaattat acacctccag aaggattcaa tgaaaaagat 600

ttgcatcgcc tcctagagaa ggtcggaact ccatctggtg cagacaattt agattgtagt 660

agtggatggt tagaagggcc aaacaaggtt tatataccat ttcttgcttg tggggacctt 720

agtggttatg ac 732

<210> 103
<211> 244
<212> PRT
<213> Tagetes erecta

<400> 103

Lys Ala Lys Glu Glu Gly Trp Arg Ala Arg Ser Ala Phe Lys Leu Leu
1 5 10 15

Gln Ile Asp Glu Glu Phe Asn Ile Phe Glu Gly Val Lys Arg Val Val
20 25 30

Asp Leu Cys Ala Ala Pro Gly Ser Trp Ser Gln Val Leu Ser Arg Lys
35 40 45

Leu Tyr Leu Pro Ala Lys Gln Ser Ser Asp Leu Pro Leu Ile Val Ala
50 55 60

Ile Asp Leu Gln Pro Met Ala Pro Ile Glu Gly Val Ile Gln Val Gln
65 70 75 80

Gly Asp Ile Thr Asn Ala Arg Thr Ala Glu Val Val Ile Arg His Phe
85 90 95

Asp Gly Cys Lys Ala Asp Leu Val Val Cys Asp Gly Ala Pro Asp Val
100 105 110

Thr Gly Leu His Asp Met Asp Glu Phe Val Gln Ser Gln Leu Ile Leu
115 120 125

Ala Gly Leu Thr Ile Val Thr His Ile Leu Lys Lys Gly Gly Lys Phe
130 135 140

Ile Ala Lys Ile Phe Arg Gly Lys Asp Thr Ser Leu Leu Tyr Cys Gln
145 150 155 160

Leu Lys Leu Phe Phe Thr Glu Val Thr Leu Ala Lys Pro Lys Ser Ser
165 170 175

Arg Asn Ser Ser Ile Glu Ala Phe Ala Val Cys Glu Asn Tyr Thr Pro
180 185 190

Pro Glu Gly Phe Asn Glu Lys Asp Leu His Arg Leu Leu Glu Lys Val
195 200 205

Gly Thr Pro Ser Gly Ala Asp Asn Leu Asp Cys Ser Ser Gly Trp Leu
210 215 220

Glu Gly Pro Asn Lys Val Tyr Ile Pro Phe Leu Ala Cys Gly Asp Leu
225 230 235 240

Ser Gly Tyr Asp

<210> 104
<211> 1770
<212> DNA
<213> Arabidopsis thaliana

<400> 104
atggctccga cgaatgttca agtaaccgat taccatctca accaatcaaa aacggataca 60
acaaatctct ggtcaaccga cgacgatgca tcggtaatgg aagctttcat cggcggcggc 120
tccgatcatt cttctctttt tcctccactt cctcctcctc ctcttcctca agtcaacgaa 180
gataatctcc agcaacgtct ccaagcttta atcgaaggag caaacgagaa ctggacttac 240
gccgtgttct ggcaatcatc tcacggtttc gccggagaag acaacaacaa caacaacaca 300
gtgttgttag gttggggaga tggttattac aaaggagaag aagagaagtc tagaaagaag 360
aaatcaaatc cagctagtgc agctgaacaa gagcatcgta agagagtgat tagagagctc 420
aactctttaa tctccggtgg tgtaggagga ggagatgaag ctggagatga agaagttaca 480
gatactgaat ggttcttctt agtttcaatg acacagagct ttgtcaaggg tactggttta 540
cctggtcaag ctttctcaaa ttcagacacg atttggttat ctggttctaa tgctttagct 600
ggatcaagtt gtgagagagc tcgtcaaggt cagatttatg ggttacaaac aatggtgtgt 660
gtagcgacag agaatggtgt cgttgagctt ggttcgtcgg agattattca tcaaagttca 720
gatcttgttg ataaagttga cacctttttc aattttaaca atggtggtgg tgaatttggt 780
tcttgggcgt ttaatttgaa tccagatcaa ggagagaatg atccaggttt gtggattagt 840
gaacctaatg gtgttgactc tggtcttgta gctgctccgg tgatgaataa tggtggaaat 900

gactcaactt ctaattctga ttctcaacca atttctaagc tttgtaatgg aagctctgtt 960
gaaaacccta accctaaagt tctgaaatct tgtgaaatgg tgaatttcaa gaatgggatt 1020
gagaatggtc aagaagaaga tagtagtaat aagaagagat caccggtttc gaataatgaa 1080
gaagggatgc tttcttttac ctctgttctt ccatgtgact cgaatcactc tgatcttgaa 1140
gcttcagtgg ctaaagaagc tgagagtaac agagttgtgg ttgaaccgga gaagaaaccg 1200
aggaaacgag ggagaaaacc ggcgaatgga agagaagagc ctttgaatca tgtagaggca 1260
gagagacaga gaagagagaa gttgaatcag agattctatt ctttaagagc tgtggttcct 1320
aatgtgtcta agatggataa agcttctcta ttaggagatg ctatttcgta tatcagtgag 1380
cttaagtcta agttgcaaaa ggctgaatct gataaagaag agttgcagaa gcagattgat 1440
gtgatgaata aagaagcggg aaatgcgaaa agttcggtaa aagatcgaaa atgtttgaat 1500
caagaatcga gtgtgttgat agagatggag gttgatgtga agattattgg ttgggatgca 1560
atgataagga ttcaatgtag taagaggaat catcctggtg ctaagttcat ggaagcactt 1620
aaggagttgg atttggaagt gaatcatgcg agtttatcgg tagtgaatga tcttatgatc 1680
caacaagcga ctgtgaaaat ggggaatcag tttttcacgc aagatcaact caaggttgct 1740
ctaacggaga aagttggaga atgtccatga 1770

<210> 105
<211> 589
<212> PRT
<213> Arabidopsis thaliana

<400> 105

Met Ala Pro Thr Asn Val Gln Val Thr Asp Tyr His Leu Asn Gln Ser
1 5 10 15

Lys Thr Asp Thr Thr Asn Leu Trp Ser Thr Asp Asp Asp Ala Ser Val
20 25 30

Met Glu Ala Phe Ile Gly Gly Gly Ser Asp His Ser Ser Leu Phe Pro
35 40 45

Pro Leu Pro Pro Pro Pro Leu Pro Gln Val Asn Glu Asp Asn Leu Gln
50 55 60

Gln Arg Leu Gln Ala Leu Ile Glu Gly Ala Asn Glu Asn Trp Thr Tyr
65 70 75 80

Ala Val Phe Trp Gln Ser Ser His Gly Phe Ala Gly Glu Asp Asn Asn
85 90 95

Asn Asn Asn Thr Val Leu Leu Gly Trp Gly Asp Gly Tyr Tyr Lys Gly
100 105 110

Glu Glu Glu Lys Ser Arg Lys Lys Lys Ser Asn Pro Ala Ser Ala Ala
115 120 125

```
Glu Gln Glu His Arg Lys Arg Val Ile Arg Glu Leu Asn Ser Leu Ile
    130                 135                 140

Ser Gly Gly Val Gly Gly Gly Asp Glu Ala Gly Asp Glu Glu Val Thr
145                 150                 155                 160

Asp Thr Glu Trp Phe Phe Leu Val Ser Met Thr Gln Ser Phe Val Lys
                165                 170                 175

Gly Thr Gly Leu Pro Gly Gln Ala Phe Ser Asn Ser Asp Thr Ile Trp
            180                 185                 190

Leu Ser Gly Ser Asn Ala Leu Ala Gly Ser Ser Cys Glu Arg Ala Arg
        195                 200                 205

Gln Gly Gln Ile Tyr Gly Leu Gln Thr Met Val Cys Val Ala Thr Glu
    210                 215                 220

Asn Gly Val Val Glu Leu Gly Ser Ser Glu Ile Ile His Gln Ser Ser
225                 230                 235                 240

Asp Leu Val Asp Lys Val Asp Thr Phe Phe Asn Phe Asn Asn Gly Gly
                245                 250                 255

Gly Glu Phe Gly Ser Trp Ala Phe Asn Leu Asn Pro Asp Gln Gly Glu
            260                 265                 270

Asn Asp Pro Gly Leu Trp Ile Ser Glu Pro Asn Gly Val Asp Ser Gly
        275                 280                 285

Leu Val Ala Ala Pro Val Met Asn Asn Gly Gly Asn Asp Ser Thr Ser
    290                 295                 300

Asn Ser Asp Ser Gln Pro Ile Ser Lys Leu Cys Asn Gly Ser Ser Val
305                 310                 315                 320

Glu Asn Pro Asn Pro Lys Val Leu Lys Ser Cys Glu Met Val Asn Phe
                325                 330                 335

Lys Asn Gly Ile Glu Asn Gly Gln Glu Glu Asp Ser Ser Asn Lys Lys
            340                 345                 350

Arg Ser Pro Val Ser Asn Asn Glu Glu Gly Met Leu Ser Phe Thr Ser
        355                 360                 365

Val Leu Pro Cys Asp Ser Asn His Ser Asp Leu Glu Ala Ser Val Ala
    370                 375                 380

Lys Glu Ala Glu Ser Asn Arg Val Val Val Glu Pro Glu Lys Lys Pro
385                 390                 395                 400
```

Arg Lys Arg Gly Arg Lys Pro Ala Asn Gly Arg Glu Glu Pro Leu Asn
405 410 415

His Val Glu Ala Glu Arg Gln Arg Arg Glu Lys Leu Asn Gln Arg Phe
420 425 430

Tyr Ser Leu Arg Ala Val Val Pro Asn Val Ser Lys Met Asp Lys Ala
435 440 445

Ser Leu Leu Gly Asp Ala Ile Ser Tyr Ile Ser Glu Leu Lys Ser Lys
450 455 460

Leu Gln Lys Ala Glu Ser Asp Lys Glu Glu Leu Gln Lys Gln Ile Asp
465 470 475 480

Val Met Asn Lys Glu Ala Gly Asn Ala Lys Ser Ser Val Lys Asp Arg
485 490 495

Lys Cys Leu Asn Gln Glu Ser Ser Val Leu Ile Glu Met Glu Val Asp
500 505 510

Val Lys Ile Ile Gly Trp Asp Ala Met Ile Arg Ile Gln Cys Ser Lys
515 520 525

Arg Asn His Pro Gly Ala Lys Phe Met Glu Ala Leu Lys Glu Leu Asp
530 535 540

Leu Glu Val Asn His Ala Ser Leu Ser Val Val Asn Asp Leu Met Ile
545 550 555 560

Gln Gln Ala Thr Val Lys Met Gly Asn Gln Phe Phe Thr Gln Asp Gln
565 570 575

Leu Lys Val Ala Leu Thr Glu Lys Val Gly Glu Cys Pro
580 585

<210> 106
<211> 1130
<212> DNA
<213> Oryza sativa

<400> 106
catgcggcta atgtagatgc tcactgcgct agtagtaagg tactccagta cattatggaa 60

tatacaaagc tgtaatactc gtatcagcaa gagagaggca cacaagttgt agcagtagca 120

caggattaga aaaacgggac gacaaatagt aatggaaaaa caaaaaaaaa caaggaaaca 180

catggcaata taaatggaga aatcacaaga ggaacagaat ccgggcaata cgctgcgaaa 240

gtactcgtac gtaaaaaaaa gaggcgcatt catgtgtgga cagcgtgcag cagaagcagg 300

gatttgaaac cactcaaatc caccactgca aaccttcaaa cgaggccatg gtttgaagca 360

tagaaagcac aggtaagaag cacaacgccc tcgctctcca ccctcccacc caatcgcgac 420

gcacctcgcg gatcggtgac gtggcctcgc cccccaaaaa tatcccgcgg cgtgaagctg 480
acaccccggg cccacccacc tgtcacgttg gcacatgttg gttatggttc ccggccgcac 540
caaaatatca acgcggcgcg gcccaaaatt tccaaaatcc cgcccaagcc cctggcgcgt 600
gccgctcttc cacccaggtc cctctcgtaa tccataatgg cgtgtgtacc ctcggctggt 660
tgtacgtggg cgggttaccc tgggggtgtg ggtggatgac gggtgggccc ggaggaggtc 720
cggccccgcg cgtcatcgcg gggcggggtg tagcgggtgc gaaaaggagg cgatcggtac 780
gaaaattcaa attaggaggt ggggggcggg gcccttggag aataagcgga atcgcagata 840
tgcccctgac ttggcttggc tcctcttctt cttatccctt gtcctcgcaa ccccgcttcc 900
ttctctcctc tcctcttctc ttctcttctc tggtggtgtg ggtgtgtccc tgtctcccct 960
ctccttcctc ctctcctttc ccctcctctc ttcccccctc tcacaagaga gagagcgcca 1020
gactctcccc aggtgaggtg agaccagtct ttttgctcga ttcgacgcgc ctttcacgcc 1080
gcctcgcgcg gatctgaccg cttccctcgg ccttctcgca ggattcagcc 1130

<210> 107
<211> 53
<212> DNA
<213> Artificial sequence

<220>
<223> primer: prm06763

<400> 107
ggggacaagt ttgtacaaaa aagcaggctt aaacaatggc tccgacgaat gtt 53

<210> 108
<211> 50
<212> DNA
<213> Artificial sequence

<220>
<223> primer: prm06764

<400> 108
ggggaccact ttgtacaaga aagctgggtt gctgacttca attcatggac 50

<210> 109
<211> 1779
<212> DNA
<213> Arabidopsis thaliana

<400> 109
atgaacggca caacatcatc aatcaacttc ttgacctccg acgatgacgc gtcggcggcg 60
gctatggaag ctttcattgg aacaaaccac cactcatctc tctttcctcc accaccacaa 120
caaccacctc agcctcagtt caacgaagat actcttcaac aacgtctcca agctttaata 180
gaatccgccg gagaaaactg gacttacgct atcttctggc agatctcaca cgacttcgat 240
tcatccaccg gagataacac agtgatcctc ggctggggag atggttacta caaaggagag 300
gaagataaag agaagaagaa gaacaacacc aacacggcgg agcaagagca tcggaaaaga 360

gtaatacgtg agcttaactc gttaatctcc ggcggaattg gggtttccga tgaatcaaac 420
gatgaagaag taacagatac tgaatggttc ttcttagttt cgatgactca aagcttcgtt 480
aacggtgttg gtctccccgg agaatctttc ttaaactctc gtgtgatttg gttatccggg 540
tctggtgctt taaccgggtc gggttgtgaa agagcgggtc aaggtcagat ttacgggtta 600
aagacgatgg tgtgtatcgc gactcaaaac ggcgtcgttg agcttggttc gtcggaggtt 660
ataagtcaaa gctcagatct gatgcataaa gttaacaact tgtttaattt caacaacggt 720
ggtggaaaca atggtgttga agcttcttcg tggggtttta atctgaatcc agatcaagga 780
gagaatgatc cagctttgtg gattagtgaa ccgacgaaca ccggaatcga atctccggcg 840

agggttaata atggtaataa ctcgaattct aattctaagt ctgattctca tcaaatttct 900
aagcttgaga agaatgatat tagctctgta gagaatcaga atcgtcaaag ttcgtgtctt 960
gtcgagaaag atttgacctt tcaaggtggg ttgttgaaat ctaatgagac tttgagtttc 1020
tgtggtaatg agagtagtaa gaagagaact tcggtatcta aagggagtaa taatgatgaa 1080
gggatgcttt cgtttagtac tgtggttaga tcagctgcga atgattcgga tcattctgat 1140
cttgaagcat ctgttgttaa ggaagcgatt gttgttgagc caccggagaa gaagccgagg 1200
aaacggggga ggaaaccggc gaatgggaga gaagagccgt tgaatcatgt tgaagcagag 1260
aggcagagaa gagagaagtt aaaccagaga ttctactctt tgagagctgt tgttcctaac 1320
gtttcgaaga tggataaagc ttcgcttctc ggagacgcga tttcgtatat caatgagctt 1380
aagtcgaagc tgcagcaagc ggagtctgat aaagaggaga ttcagaagaa gctagatggg 1440
atgagtaagg aagggaataa tgggaaaggt tgcgggtcaa gggcaaaaga acggaaaagt 1500
tcgaatcaag attctacggc gagttctata gaaatggaga ttgatgttaa gatcataggt 1560
tgggatgtga tgatacgtgt acaatgcggc aagaaagatc atcccggtgc taggttcatg 1620
gaagcactta aggaattgga tttggaagtg aatcatgcga gtttatccgt tgtgaatgat 1680
ttgatgattc aacaagctac ggtgaagatg gggagccaat tttcaatcal tgaccagctc 1740
aaagttgctt tgatgacgaa agtcggagaa aactattga 1779

<210> 110
<211> 592
<212> PRT
<213> Arabidopsis thaliana

<400> 110

Met Asn Gly Thr Thr Ser Ser Ile Asn Phe Leu Thr Ser Asp Asp Asp
1 5 10 15

Ala Ser Ala Ala Ala Met Glu Ala Phe Ile Gly Thr Asn His His Ser
20 25 30

Ser Leu Phe Pro Pro Pro Pro Gln Gln Pro Pro Gln Pro Gln Phe Asn
35 40 45

Glu Asp Thr Leu Gln Gln Arg Leu Gln Ala Leu Ile Glu Ser Ala Gly
50 55 60

Glu Asn Trp Thr Tyr Ala Ile Phe Trp Gln Ile Ser His Asp Phe Asp
65 70 75 80

Ser Ser Thr Gly Asp Asn Thr Val Ile Leu Gly Trp Gly Asp Gly Tyr
85 90 95

Tyr Lys Gly Glu Glu Asp Lys Glu Lys Lys Lys Asn Asn Thr Asn Thr
100 105 110

Ala Glu Gln Glu His Arg Lys Arg Val Ile Arg Glu Leu Asn Ser Leu
115 120 125

Ile Ser Gly Gly Ile Gly Val Ser Asp Glu Ser Asn Asp Glu Glu Val
130 135 140

Thr Asp Thr Glu Trp Phe Phe Leu Val Ser Met Thr Gln Ser Phe Val
145 150 155 160

Asn Gly Val Gly Leu Pro Gly Glu Ser Phe Leu Asn Ser Arg Val Ile
165 170 175

Trp Leu Ser Gly Ser Gly Ala Leu Thr Gly Ser Gly Cys Glu Arg Ala
180 185 190

Gly Gln Gly Gln Ile Tyr Gly Leu Lys Thr Met Val Cys Ile Ala Thr
195 200 205

Gln Asn Gly Val Val Glu Leu Gly Ser Ser Glu Val Ile Ser Gln Ser
210 215 220

Ser Asp Leu Met His Lys Val Asn Asn Leu Phe Asn Phe Asn Asn Gly
225 230 235 240

Gly Gly Asn Asn Gly Val Glu Ala Ser Ser Trp Gly Phe Asn Leu Asn
245 250 255

Pro Asp Gln Gly Glu Asn Asp Pro Ala Leu Trp Ile Ser Glu Pro Thr
260 265 270

Asn Thr Gly Ile Glu Ser Pro Ala Arg Val Asn Asn Gly Asn Asn Ser
275 280 285

Asn Ser Asn Ser Lys Ser Asp Ser His Gln Ile Ser Lys Leu Glu Lys
290 295 300

Asn Asp Ile Ser Ser Val Glu Asn Gln Asn Arg Gln Ser Ser Cys Leu
305 310 315 320

Val Glu Lys Asp Leu Thr Phe Gln Gly Gly Leu Leu Lys Ser Asn Glu

325 330 335

Thr Leu Ser Phe Cys Gly Asn Glu Ser Ser Lys Lys Arg Thr Ser Val
340 345 350

Ser Lys Gly Ser Asn Asn Asp Glu Gly Met Leu Ser Phe Ser Thr Val
355 360 365

Val Arg Ser Ala Ala Asn Asp Ser Asp His Ser Asp Leu Glu Ala Ser
370 375 380

Val Val Lys Glu Ala Ile Val Val Glu Pro Pro Glu Lys Lys Pro Arg
385 390 395 400

Lys Arg Gly Arg Lys Pro Ala Asn Gly Arg Glu Glu Pro Leu Asn His
405 410 415

Val Glu Ala Glu Arg Gln Arg Arg Glu Lys Leu Asn Gln Arg Phe Tyr
420 425 430

Ser Leu Arg Ala Val Val Pro Asn Val Ser Lys Met Asp Lys Ala Ser
435 440 445

Leu Leu Gly Asp Ala Ile Ser Tyr Ile Asn Glu Leu Lys Ser Lys Leu
450 455 460

Gln Gln Ala Glu Ser Asp Lys Glu Glu Ile Gln Lys Lys Leu Asp Gly
465 470 475 480

Met Ser Lys Glu Gly Asn Asn Gly Lys Gly Cys Gly Ser Arg Ala Lys
485 490 495

Glu Arg Lys Ser Ser Asn Gln Asp Ser Thr Ala Ser Ser Ile Glu Met
500 505 510

Glu Ile Asp Val Lys Ile Ile Gly Trp Asp Val Met Ile Arg Val Gln
515 520 525

Cys Gly Lys Lys Asp His Pro Gly Ala Arg Phe Met Glu Ala Leu Lys
530 535 540

Glu Leu Asp Leu Glu Val Asn His Ala Ser Leu Ser Val Val Asn Asp
545 550 555 560

Leu Met Ile Gln Gln Ala Thr Val Lys Met Gly Ser Gln Phe Phe Asn
565 570 575

His Asp Gln Leu Lys Val Ala Leu Met Thr Lys Val Gly Glu Asn Tyr
580 585 590

<210> 111
<211> 1833

<212> DNA
<213> Brassica napus

<400> 111
atgacggagc cgacgatgaa tctctggacc accgacgata acgcctctat gatggaagct 60
ttcatgagct cctcctccga catctcagcc ttatggccac cggtaacgac gacggcaacg 120
gcgtcgacta cagctccggc accggcggga ttcaacgagg agactctaca gcaaaggcta 180
caagcactga ttgaagggac gaacgaaggc tggacctacg ctatattctg gcagccgtcg 240
tacgacttct ccggtgcctc cgtgctcgga tggggcgatg gttattacaa aggagaagaa 300
gacaaggcaa agcccagaca gagaacgtct ccaccgccgt tttcaactcc ggcggatcag 360
gagtatcgta agaaggtgtt gcgtgagctc aactcgttga tctccggcgg aggtggtccg 420
acggatgatg ccgtcgatga agaggtgacg gatacggagt ggtttttctt ggtttcgatg 480
acgcagagct tcgcttgcgg ttctgggttg gcgggtaagg cgttctcgac gggtaacgca 540
gtttgggttt atgggtcgga tcagttaacc gggtcgggtt gtgagcgggc gaagcaagga 600
ggagtgtttg ggatgcagac catcgcgtgt attccttcgg cgaacggggt tgttgaactc 660
gggccaacgg agcagatccg acagagttcg gatcttatga acaaggtacg agtacttttc 720
aatttcaacg gtggcgctgg agatttatcg tgtcttaact ggaatcttga cccggatcaa 780
ggcgagaacg atccgtctat gtggattaac gacccgattg gagtacccga gcagggtaac 840
ggagctccga gctctagctc ccagcttttt gccaagtcga tccagtttga gaatggtggt 900
agttcaagca ccatcatcga aaacccgaat ccggatccag ctccagctcc gagcccggtt 960
cattcccaga cccagaatcc gaaattcagc aacaatttct cccgcgaatt aaatttctcc 1020
acgtcgagca ccaccttggt gaaaccaaga cccgccgaga tattgagctt cggcgatgag 1080
ggtaaacgga gctccgtgaa cccggatccg agttcttatt cgggtcagac tcagttcgag 1140
aataagagaa agaagtccat aggtatgagc gatgacaagg ttctaacttt cggaaccggc 1200
ggaggagaat ccgatcactc cgatctagaa gcctccgtcg tgaaagagat accggagaaa 1260
cgtcccaaga aacgtggaag aaaaccggcc aacggtagag aagagccgct taaccacgtc 1320
gaagcagaga gacagagacg cgagaaacta aaccagcgat tctacgcgtt acgtgcggtt 1380
gtaccaaacg tctcgaaaat ggataaagct tctttgctcg gagacgcgat tgcttacatc 1440
aacgagctga agtctaaggt gaccaagacg gaatctgaga aaactcagat caaaacccag 1500
ctcgaggaag tgaaaatgga gctcgccgga agaaaagcaa gcgccggtgg agatctatca 1560
tcctcttgct cgttgacagc gatcaaaccc gtggggatgg agatcgaggt gaagattatc 1620
ggttgggatg cgatgataag ggtggaatcg agcaagagga atcatccggc agcgaggttg 1680
atgtcggcgt tgatggatct agagcttgag gtgaatcacg cgagtatgtc ggtggttaac 1740
gatctgatga tacagcaagc gacggtgaag atgggattca gaatatacac gcaggaacag 1800
ctccgggcga gtttaatctc aaagattggt taa 1833

<210> 112
<211> 610

<212> PRT
<213> Brassica napus

<400> 112

```
Met Thr Glu Pro Thr Met Asn Leu Trp Thr Thr Asp Asp Asn Ala Ser
1               5                   10                  15

Met Met Glu Ala Phe Met Ser Ser Ser Ser Asp Ile Ser Ala Leu Trp
            20                  25                  30

Pro Pro Val Thr Thr Thr Ala Thr Ala Ser Thr Thr Ala Pro Ala Pro
        35                  40                  45

Ala Gly Phe Asn Glu Glu Thr Leu Gln Gln Arg Leu Gln Ala Leu Ile
    50                  55                  60

Glu Gly Thr Asn Glu Gly Trp Thr Tyr Ala Ile Phe Trp Gln Pro Ser
65                  70                  75                  80

Tyr Asp Phe Ser Gly Ala Ser Val Leu Gly Trp Gly Asp Gly Tyr Tyr
                85                  90                  95

Lys Gly Glu Glu Asp Lys Ala Lys Pro Arg Gln Arg Thr Ser Pro Pro
            100                 105                 110

Pro Phe Ser Thr Pro Ala Asp Gln Glu Tyr Arg Lys Lys Val Leu Arg
        115                 120                 125

Glu Leu Asn Ser Leu Ile Ser Gly Gly Gly Gly Pro Thr Asp Asp Ala
    130                 135                 140

Val Asp Glu Glu Val Thr Asp Thr Glu Trp Phe Phe Leu Val Ser Met
145                 150                 155                 160

Thr Gln Ser Phe Ala Cys Gly Ser Gly Leu Ala Gly Lys Ala Phe Ser
                165                 170                 175

Thr Gly Asn Ala Val Trp Val Tyr Gly Ser Asp Gln Leu Thr Gly Ser
            180                 185                 190

Gly Cys Glu Arg Ala Lys Gln Gly Gly Val Phe Gly Met Gln Thr Ile
        195                 200                 205

Ala Cys Ile Pro Ser Ala Asn Gly Val Val Glu Leu Gly Pro Thr Glu
    210                 215                 220

Gln Ile Arg Gln Ser Ser Asp Leu Met Asn Lys Val Arg Val Leu Phe
225                 230                 235                 240

Asn Phe Asn Gly Gly Ala Gly Asp Leu Ser Cys Leu Asn Trp Asn Leu
                245                 250                 255
```

Asp Pro Asp Gln Gly Glu Asn Asp Pro Ser Met Trp Ile Asn Asp Pro
260 265 270

Ile Gly Val Pro Glu Gln Gly Asn Gly Ala Pro Ser Ser Ser Ser Gln
275 280 285

Leu Phe Ala Lys Ser Ile Gln Phe Glu Asn Gly Gly Ser Ser Ser Thr
290 295 300

Ile Ile Glu Asn Pro Asn Pro Asp Pro Ala Pro Ala Pro Ser Pro Val
305 310 315 320

His Ser Gln Thr Gln Asn Pro Lys Phe Ser Asn Asn Phe Ser Arg Glu
325 330 335

Leu Asn Phe Ser Thr Ser Ser Thr Thr Leu Val Lys Pro Arg Pro Ala
340 345 350

Glu Ile Leu Ser Phe Gly Asp Glu Gly Lys Arg Ser Ser Val Asn Pro
355 360 365

Asp Pro Ser Ser Tyr Ser Gly Gln Thr Gln Phe Glu Asn Lys Arg Lys

370 375 380

Lys Ser Ile Gly Met Ser Asp Asp Lys Val Leu Thr Phe Gly Thr Gly
385 390 395 400

Gly Gly Glu Ser Asp His Ser Asp Leu Glu Ala Ser Val Val Lys Glu
405 410 415

Ile Pro Glu Lys Arg Pro Lys Lys Arg Gly Arg Lys Pro Ala Asn Gly
420 425 430

Arg Glu Glu Pro Leu Asn His Val Glu Ala Glu Arg Gln Arg Arg Glu
435 440 445

Lys Leu Asn Gln Arg Phe Tyr Ala Leu Arg Ala Val Val Pro Asn Val
450 455 460

Ser Lys Met Asp Lys Ala Ser Leu Leu Gly Asp Ala Ile Ala Tyr Ile
465 470 475 480

Asn Glu Leu Lys Ser Lys Val Thr Lys Thr Glu Ser Glu Lys Thr Gln
485 490 495

Ile Lys Thr Gln Leu Glu Glu Val Lys Met Glu Leu Ala Gly Arg Lys
500 505 510

Ala Ser Ala Gly Gly Asp Leu Ser Ser Ser Cys Ser Leu Thr Ala Ile
515 520 525

Lys Pro Val Gly Met Glu Ile Glu Val Lys Ile Ile Gly Trp Asp Ala
530 535 540

Met Ile Arg Val Glu Ser Ser Lys Arg Asn His Pro Ala Ala Arg Leu
545 550 555 560

Met Ser Ala Leu Met Asp Leu Glu Leu Glu Val Asn His Ala Ser Met
565 570 575

Ser Val Val Asn Asp Leu Met Ile Gln Gln Ala Thr Val Lys Met Gly
580 585 590

Phe Arg Ile Tyr Thr Gln Glu Gln Leu Arg Ala Ser Leu Ile Ser Lys
595 600 605

Ile Gly
610

<210> 113
<211> 1959
<212> DNA
<213> Lotus japonicus

<400> 113
atgaatctct ggagcgacga caactcctcc gtcatggagg ccttcatgac ctcctccgac 60
ttatcctccg tctggccacc agcgccgcct cctccctctc aatcctccgt cgccgtcctc 120
aaccaggaca ctctccagca acgcctccag gccctaatcg aaggcgcaag agagacctgg 180
acctacgcca ttttctggca accttcctac gactactccg gcacctccct cctcggctgg 240
ggcgacggtt actacaaagg cgaagaggac aaagccaagg caaaagcaaa agccaaagcc 300
aaagccacct cctccgctga acaagaacac cgccggaagg tgcttcgtga cttgaattcc 360
ctcatctccg gttcctccgc ccccgcttct gatgacgctg tcgacgaaga ggtcaccgac 420
acggaatggt tcttccttgt ttccatgacc cagtcgtttg tcaacggcgg cgggctagct 480
ggtcaggcgt atttcaactc cacccctgtt tgggtcgccg gcgctgatcg cctcgcagcc 540
tccgcctgcg agagggccag gcaggggcag ctcttcggaa tccagacgct cgtctgcgtg 600
ccgtctgcaa acggcgtcgt tgaactcgga tccactgagc tgatttacca gaactccgat 660
ctgatgaaca aggtgaaggt gctgttcaat ttcagcaaca gcaacctcga tttgggttct 720
tcctggacat tgggttccac caccaccgcc gagaatgacc cttccgccct ctggctcgcc 780
gaccccgacc ctgacggcag agattccgtc agcaccgtcg ctcccaccac cgcttccgtt 840
tcgattccca gccaccacaa caacgatcag agcattgcga agacgttgca gttcgaaacc 900
cctgtttcga gtaccttgac tgaaaccccg agtgctgtta atctcacaaa ccagccatcg 960
cagaaccaga gaaaccagag cagcttcttc tccagggaga tgaatttctc cgagtacagt 1020
ttcgatgcga agaacggcag cagtaaccac cagcatctga agccggaatc cggtgagatt 1080
ctgagcttcg gtgacagcaa gagaaccccc aatttcttct ccggccagtc gcagttcgtt 1140
ccggctgtcg aggagaacaa caacgggaag aagaggtcac cgaattcgag aagcagcaac 1200

gacgacggga tgctttcatt cacctccggc gtgattctcc cgtcctcgaa tttgaaatcc 1260
tccaccggcg gcggcgattc ggagcattca gatctggaag cttcggtggt gaaggaagcc 1320
gacagcagca ggttggtgga gccggagaag cgacctagga agagggggag gaaaccggcg 1380
aacggcagag aggaaccgtt gaaccacgtt gaagcagaga ggcagaggag ggagaagctt 1440
aaccagagat tctacgcgct tcgcgcggtg gttccgaacg tgtcgaagat ggacaaagct 1500
tcactgctag gagacgctat atcctacatc acggagctga agaccaagct tcagagttca 1560
gaatcggata aaactgggtt gcagaagcaa tttgatgcga tgaagaagga gctggagaaa 1620
accagtgaac agtcttcttc tccaactcca cctccaccta acaagaacaa gtccttctcc 1680
tcatcctcct cctcttccaa tcagattcta gtggaggaca ttgacgtgaa gatcattggg 1740
tgggatgcaa tgataagggt ccaatgcagc aagaagaacc acccggccgc gattttgatg 1800
gcggcgttga tggagctgga ccttgaggtg aaccatgcta gtgtgtcagt ggtgaatgat 1860
accatgatcc agcaagcaac agtgaagatg ggaagccgct tttacactca ggagcagctt 1920
cgatcagcac tctcttccaa atttggggat gctccttag 1959

<210> 114
<211> 652
<212> PRT
<213> Lotus japonicus

<400> 114

Met Asn Leu Trp Ser Asp Asp Asn Ser Ser Val Met Glu Ala Phe Met
1 5 10 15

Thr Ser Ser Asp Leu Ser Ser Val Trp Pro Pro Ala Pro Pro Pro Pro
20 25 30

Ser Gln Ser Ser Val Ala Val Leu Asn Gln Asp Thr Leu Gln Gln Arg
35 40 45

Leu Gln Ala Leu Ile Glu Gly Ala Arg Glu Thr Trp Thr Tyr Ala Ile
50 55 60

Phe Trp Gln Pro Ser Tyr Asp Tyr Ser Gly Thr Ser Leu Leu Gly Trp
65 70 75 80

Gly Asp Gly Tyr Tyr Lys Gly Glu Glu Asp Lys Ala Lys Ala Lys Ala
85 90 95

Lys Ala Lys Ala Lys Ala Thr Ser Ser Ala Glu Gln Glu His Arg Arg
100 105 110

Lys Val Leu Arg Asp Leu Asn Ser Leu Ile Ser Gly Ser Ser Ala Pro
115 120 125

Ala Ser Asp Asp Ala Val Asp Glu Glu Val Thr Asp Thr Glu Trp Phe
130 135 140

```
Phe Leu Val Ser Met Thr Gln Ser Phe Val Asn Gly Gly Gly Leu Ala
145                 150                 155                 160

Gly Gln Ala Tyr Phe Asn Ser Thr Pro Val Trp Val Ala Gly Ala Asp
                165                 170                 175

Arg Leu Ala Ala Ser Ala Cys Glu Arg Ala Arg Gln Gly Gln Leu Phe
            180                 185                 190

Gly Ile Gln Thr Leu Val Cys Val Pro Ser Ala Asn Gly Val Val Glu
        195                 200                 205

Leu Gly Ser Thr Glu Leu Ile Tyr Gln Asn Ser Asp Leu Met Asn Lys
    210                 215                 220

Val Lys Val Leu Phe Asn Phe Ser Asn Ser Asn Leu Asp Leu Gly Ser
225                 230                 235                 240

Ser Trp Thr Leu Gly Ser Thr Thr Thr Ala Glu Asn Asp Pro Ser Ala
                245                 250                 255

Leu Trp Leu Ala Asp Pro Asp Pro Asp Gly Arg Asp Ser Val Ser Thr
            260                 265                 270

Val Ala Pro Thr Thr Ala Ser Val Ser Ile Pro Ser His His Asn Asn
        275                 280                 285

Asp Gln Ser Ile Ala Lys Thr Leu Gln Phe Glu Thr Pro Val Ser Ser
    290                 295                 300

Thr Leu Thr Glu Thr Pro Ser Ala Val Asn Leu Thr Asn Gln Pro Ser
305                 310                 315                 320

Gln Asn Gln Arg Asn Gln Ser Ser Phe Phe Ser Arg Glu Met Asn Phe
                325                 330                 335

Ser Glu Tyr Ser Phe Asp Ala Lys Asn Gly Ser Ser Asn His Gln His
            340                 345                 350

Leu Lys Pro Glu Ser Gly Glu Ile Leu Ser Phe Gly Asp Ser Lys Arg
        355                 360                 365

Thr Pro Asn Phe Phe Ser Gly Gln Ser Gln Phe Val Pro Ala Val Glu
    370                 375                 380

Glu Asn Asn Asn Gly Lys Lys Arg Ser Pro Asn Ser Arg Ser Ser Asn
385                 390                 395                 400

Asp Asp Gly Met Leu Ser Phe Thr Ser Gly Val Ile Leu Pro Ser Ser
                405                 410                 415
```

Asn Leu Lys Ser Ser Thr Gly Gly Gly Asp Ser Glu His Ser Asp Leu
            420                 425                 430

Glu Ala Ser Val Val Lys Glu Ala Asp Ser Ser Arg Leu Val Glu Pro
        435                 440                 445

Glu Lys Arg Pro Arg Lys Arg Gly Arg Lys Pro Ala Asn Gly Arg Glu
    450                 455                 460

Glu Pro Leu Asn His Val Glu Ala Glu Arg Gln Arg Arg Glu Lys Leu
465                 470                 475                 480

Asn Gln Arg Phe Tyr Ala Leu Arg Ala Val Val Pro Asn Val Ser Lys
                485                 490                 495

Met Asp Lys Ala Ser Leu Leu Gly Asp Ala Ile Ser Tyr Ile Thr Glu
            500                 505                 510

Leu Lys Thr Lys Leu Gln Ser Ser Glu Ser Asp Lys Thr Gly Leu Gln
        515                 520                 525

Lys Gln Phe Asp Ala Met Lys Lys Glu Leu Glu Lys Thr Ser Glu Gln
    530                 535                 540

Ser Ser Ser Pro Thr Pro Pro Pro Pro Asn Lys Asn Lys Ser Phe Ser
545                 550                 555                 560

Ser Ser Ser Ser Ser Ser Asn Gln Ile Leu Val Glu Asp Ile Asp Val
                565                 570                 575

Lys Ile Ile Gly Trp Asp Ala Met Ile Arg Val Gln Cys Ser Lys Lys
            580                 585                 590

Asn His Pro Ala Ala Ile Leu Met Ala Ala Leu Met Glu Leu Asp Leu
        595                 600                 605

Glu Val Asn His Ala Ser Val Ser Val Val Asn Asp Thr Met Ile Gln
    610                 615                 620

Gln Ala Thr Val Lys Met Gly Ser Arg Phe Tyr Thr Gln Glu Gln Leu
625                 630                 635                 640

Arg Ser Ala Leu Ser Ser Lys Phe Gly Asp Ala Pro
                645                 650

<210> 115
<211> 2070
<212> DNA

<213> Lycopersicon esculentum

<400> 115
atgactgaat acagcttgcc caccatgaat ttgtggaaca atagtactag cgatgataac 60

gtttctatga tggaagcttt tatgtcttct gatctttctt tttgggctac taataattct 120
acttctgctg ctgcggttgg tgtcaattca aatcttcctc atgctagtag taatactccc 180
tctgtttttg caccatcttc ttctacatct gcatctactt tatccgcagc tgcgactgtg 240
gatgcttcca aatctatgcc gtttttcaac caagaaaccc ttcagcagcg tcttcaagct 300
cttattgatg gtgctagaga gacgtggact tatgctatct tttggcaatc gtcggttgtt 360
gatttctcaa gtccgtctgt gttgggttgg ggagatggtt attacaaagg ggaagaagat 420
aaagcaaaaa ggaaattatc ggtgtcatca cctgcttata ttgctgagca ggagcatcgg 480
aagaaggttc tacgggagct gaattcgttg atttccgggg caccacccgg aacggatgat 540
gcggttgatg aagaagttac cgacaccgaa tggttctttc ttatctccat gacccaatcg 600
tttgttaatg gaagtgggct tcctggtcag gcgttgtata gttccagccc gatttgggtc 660
gccggaactg agaaattggc agcttcacac tgtgaacgtg tgaggcaagc acaagggttc 720
gggctccaga cgattgtctg tattccttca gctaacggcg tggttgaatt gggctcgacg 780
gagttgattg ttcaaagttc tgatcttatg aacaaggtta gagtattgtt taacttcagt 840
aatgatttgg gttctggttc atgggctgtg cagccggaga gcgacccatc ggcgctctgg 900
ctcactgatc catcgtcctc aggtatggaa gttagagagt ctttaaatac agttcaaaca 960
aattcagttc catctagtaa tagtaataag caaattgctt atggaaatga gaataatcat 1020
ccatctggaa atggtcagag ttgttacaat cagcaacaac agaagaatcc tcctcagcaa 1080
caaacacaag gactcttcac gagggagttg aatttttcgg aattcggttt cgatggaagt 1140
agtaatagga atggaaattc atcggtttct tgcaagcctg aatcaggaga aatcttgaat 1200
tttggtgata gtactaaaaa aagtgcttcc agtgccaatg tgaacttgtt tacaggtcag 1260
tcccaatttg gggctgggga ggagaataat aacaagaaca agaaaagatc agctacttcc 1320
aggggaagca atgaagaagg aatgctttca tttgtttcag gtacagtggt gccttcttcg 1380
ggcatgaagt caggtggagg cggaggcgaa gactctgaac attcagatct cgaggcttca 1440
gtggtgaaag aagctgatag tagtagagtg gtagagcctg aaaagaggcc aaggaagcga 1500
ggtagaaagc cagcgaatgg acgggaggag ccattgaatc acgtcgaggc agagaggcaa 1560
aggagggaga aattgaacca aagattctac gcgcttagag ctgttgtacc aaatgtgtct 1620
aagatggaca aggcatcact ccttggagat gctatttcct atataaacga gttgaaatcg 1680
aagcttcaaa atacagagtc agataaagaa gacttgaaga gccaaataga agatttaaag 1740
aaagaatcaa ggcgccccgg tcctcctcca ccaccaaatc aagatctcaa gatgtctagc 1800
cacactggag gcaagattgt agacgtggat atagacgtta agatcatcgg atgggatgca 1860
atgattcgta tacaatgtaa taaaaagaat catccagccg caaggctaat ggcagcgctc 1920
atggaattag acctagacgt gcatcatgcc agtgtttcag ttgtcaacga tttgatgatc 1980
caacaagcca cagtgaaaat gggtagcagg aattacactg aagagcagct tagggtagcg 2040
ttgacatcga aaattgctga aacacactaa 2070

<210> 116
<211> 689
<212> PRT
<213> Lycopersicon esculentum

<400> 116

Met Thr Glu Tyr Ser Leu Pro Thr Met Asn Leu Trp Asn Asn Ser Thr
1 5 10 15

Ser Asp Asp Asn Val Ser Met Met Glu Ala Phe Met Ser Ser Asp Leu
20 25 30

Ser Phe Trp Ala Thr Asn Asn Ser Thr Ser Ala Ala Ala Val Gly Val
35 40 45

Asn Ser Asn Leu Pro His Ala Ser Ser Asn Thr Pro Ser Val Phe Ala
50 55 60

Pro Ser Ser Ser Thr Ser Ala Ser Thr Leu Ser Ala Ala Ala Thr Val
65 70 75 80

Asp Ala Ser Lys Ser Met Pro Phe Phe Asn Gln Glu Thr Leu Gln Gln
85 90 95

Arg Leu Gln Ala Leu Ile Asp Gly Ala Arg Glu Thr Trp Thr Tyr Ala
100 105 110

Ile Phe Trp Gln Ser Ser Val Val Asp Phe Ser Ser Pro Ser Val Leu
115 120 125

Gly Trp Gly Asp Gly Tyr Tyr Lys Gly Glu Glu Asp Lys Ala Lys Arg
130 135 140

Lys Leu Ser Val Ser Ser Pro Ala Tyr Ile Ala Glu Gln Glu His Arg
145 150 155 160

Lys Lys Val Leu Arg Glu Leu Asn Ser Leu Ile Ser Gly Ala Pro Pro
165 170 175

Gly Thr Asp Asp Ala Val Asp Glu Glu Val Thr Asp Thr Glu Trp Phe
180 185 190

Phe Leu Ile Ser Met Thr Gln Ser Phe Val Asn Gly Ser Gly Leu Pro
195 200 205

Gly Gln Ala Leu Tyr Ser Ser Ser Pro Ile Trp Val Ala Gly Thr Glu
210 215 220

Lys Leu Ala Ala Ser His Cys Glu Arg Val Arg Gln Ala Gln Gly Phe
225 230 235 240

Gly Leu Gln Thr Ile Val Cys Ile Pro Ser Ala Asn Gly Val Val Glu
245 250 255

Leu Gly Ser Thr Glu Leu Ile Val Gln Ser Ser Asp Leu Met Asn Lys
260 265 270

Val Arg Val Leu Phe Asn Phe Ser Asn Asp Leu Gly Ser Gly Ser Trp
275 280 285

Ala Val Gln Pro Glu Ser Asp Pro Ser Ala Leu Trp Leu Thr Asp Pro
290 295 300

Ser Ser Ser Gly Met Glu Val Arg Glu Ser Leu Asn Thr Val Gln Thr
305 310 315 320

Asn Ser Val Pro Ser Ser Asn Ser Asn Lys Gln Ile Ala Tyr Gly Asn
325 330 335

Glu Asn Asn His Pro Ser Gly Asn Gly Gln Ser Cys Tyr Asn Gln Gln
340 345 350

Gln Gln Lys Asn Pro Pro Gln Gln Gln Thr Gln Gly Leu Phe Thr Arg
355 360 365

Glu Leu Asn Phe Ser Glu Phe Gly Phe Asp Gly Ser Ser Asn Arg Asn
370 375 380

Gly Asn Ser Ser Val Ser Cys Lys Pro Glu Ser Gly Glu Ile Leu Asn
385 390 395 400

Phe Gly Asp Ser Thr Lys Lys Ser Ala Ser Ser Ala Asn Val Asn Leu
405 410 415

Phe Thr Gly Gln Ser Gln Phe Gly Ala Gly Glu Glu Asn Asn Asn Lys
420 425 430

Asn Lys Lys Arg Ser Ala Thr Ser Arg Gly Ser Asn Glu Glu Gly Met
435 440 445

Leu Ser Phe Val Ser Gly Thr Val Val Pro Ser Ser Gly Met Lys Ser
450 455 460

Gly Gly Gly Gly Gly Glu Asp Ser Glu His Ser Asp Leu Glu Ala Ser
465 470 475 480

Val Val Lys Glu Ala Asp Ser Ser Arg Val Val Glu Pro Glu Lys Arg
485 490 495

Pro Arg Lys Arg Gly Arg Lys Pro Ala Asn Gly Arg Glu Glu Pro Leu
500 505 510

Asn His Val Glu Ala Glu Arg Gln Arg Arg Glu Lys Leu Asn Gln Arg
        515                 520                 525

Phe Tyr Ala Leu Arg Ala Val Val Pro Asn Val Ser Lys Met Asp Lys
    530                 535                 540

Ala Ser Leu Leu Gly Asp Ala Ile Ser Tyr Ile Asn Glu Leu Lys Ser
545                 550                 555                 560

Lys Leu Gln Asn Thr Glu Ser Asp Lys Glu Asp Leu Lys Ser Gln Ile
                565                 570                 575

Glu Asp Leu Lys Lys Glu Ser Arg Arg Pro Gly Pro Pro Pro Pro Pro
            580                 585                 590

Asn Gln Asp Leu Lys Met Ser Ser His Thr Gly Gly Lys Ile Val Asp
        595                 600                 605

Val Asp Ile Asp Val Lys Ile Ile Gly Trp Asp Ala Met Ile Arg Ile
    610                 615                 620

Gln Cys Asn Lys Lys Asn His Pro Ala Ala Arg Leu Met Ala Ala Leu
625                 630                 635                 640

Met Glu Leu Asp Leu Asp Val His His Ala Ser Val Ser Val Val Asn
                645                 650                 655

Asp Leu Met Ile Gln Gln Ala Thr Val Lys Met Gly Ser Arg Asn Tyr
            660                 665                 670

Thr Glu Glu Gln Leu Arg Val Ala Leu Thr Ser Lys Ile Ala Glu Thr
        675                 680                 685

His

<210> 117
<211> 1992
<212> DNA
<213> Vitis vinifera

<400> 117
atgacggaat atcgtgtgcc aaccatgaat ctgtggaccg acgataacgc ttctatgatg 60

gaggctttta taagctctga tctctcttcc tttagttggg ggccatcctc cgctgcttct 120

acgtctactc ctgcgcctga cccgtcaagg aatctcgccc aatctcagcc ttccatggcc 180

gtcttcaacc aggagaccct ccagcagcga cttcaagccc taattgaagg cgcccgagag 240

agctggacct acgcaatttt ttggcagtcc agcgttgatt tctcgggtgc ctctttgtta 300

ggatggggtg acggctacta taaaggtgag gaagataaag ggaagaggaa gatgaccccg 360

tcgtctgttt ccgagcagga gcaccggaag aaagtgctcc gggagctgaa ttcgctcatt 420

tctgggacgg cgtcgtcgtc ggacgacgct gtcgatgagg aggtcaccga caccgagtgg 480

ttcttccttg tatcgatgac tcagtcgttt gtgaacggcg ctgggttgcc gggtcaggcg 540
ctcttcaact cgagtccggt gtgggttgtc ggaaccgaac ggctcatgag ttctccttgc 600
gagagagccc ggcaggcgca ggtgttcggg ttacagacta tggtttgtat accatcggct 660
aacggcgtcg tcgaactggg ctccaccgaa ttgatttacc agagctcaga tctgatgaac 720
aaggttagag ttttgttcaa tttcaataac cttgaagttg gttcatggcc gatcggagcc 780
gccgctcccg accagggcga gagcgatcct tcgtcgctct ggatctccga tccgacctct 840
aatgtcgaaa tcaaggattc tgtgaatgct acagctactg gagcttccaa tcccattggt 900
aatcagcaga attccaagtc aattcaattt gagaacccta gctcaagtag tttaactgaa 960
aaccctagta ttatgcataa tcctcagcag caacagcaga ttcacacgca gggtttttc 1020
actagggagt tgaatttctc agaatttgga tttgatggaa ayaatgggag aaatggtaac 1080
ttgcattccc tgaagcccga gtctggggaa attttgaatt ttggagacag taagaggagt 1140
tcttgtagtg cgaatgggaa tatgttttca ggtcattcac aggtagttgc agaggaaaat 1200
aagaagagga ggtcaccgac ttcaagagga agtgccgaag aaggcatgct ttcgtttact 1260
tcaggtgtga tattgccatc ctcttgtgtg gtgaagtcaa gtggtggtgg tggagattct 1320
gatcactcgg atcttgaagc ttcagtggtt cgggaggcag atagtagtag agtggtggaa 1380
ccagaaaaaa ggcctaggaa gcgtgggaga aaacctgcaa atggaaggga agagccattg 1440
aatcatgtgg aggcagagcg gcagaggagg gagaagctta accagaggtt ttatgccctc 1500
cgagctgtgg ttccaaatgt gtcaaagatg gataaagcct cccttcttgg tgatgcaatt 1560
tcatatatca atgagctcag gacaaagcta caaagtgcag agtcggacaa ggaggacctt 1620
cagaaggaag tgaattcaat gaagaaggag ttggctagta aagatwcaca gtactccggt 1680
tcatctcgac cacctccaga ccaagatctc aagatgtcga atcaccacgg tagcaaattg 1740
gtagaaatgg atattgatgt gaagataatc gggtgggatg ccatgattcg aattcagtgt 1800

agtaaaaaga accatcctgc tgcaaagctt atgggggctc tcaaggagct ggacctagat 1860
gtgaaccacg cgagtgtgtc ggtggtgaat gatttgatga tccaacaggc aaccgtgaag 1920
atgggaagta ggttttacac tcaagatcag ctgaggctag ccctgtcatc caaatttgca 1980
gacagcaggt ag 1992

<210> 118
<211> 663
<212> PRT
<213> Vitis vinifera

<220>
<221> misc_feature
<222> (556)..(556)
<223> Xaa can be any naturally occurring amino acid

<400> 118

Met Thr Glu Tyr Arg Val Pro Thr Met Asn Leu Trp Thr Asp Asp Asn

1 5 10 15

Ala Ser Met Met Glu Ala Phe Ile Ser Ser Asp Leu Ser Ser Phe Ser
20 25 30

Trp Gly Pro Ser Ser Ala Ala Ser Thr Ser Thr Pro Ala Pro Asp Pro
35 40 45

Ser Arg Asn Leu Ala Gln Ser Gln Pro Ser Met Ala Val Phe Asn Gln
50 55 60

Glu Thr Leu Gln Gln Arg Leu Gln Ala Leu Ile Glu Gly Ala Arg Glu
65 70 75 80

Ser Trp Thr Tyr Ala Ile Phe Trp Gln Ser Ser Val Asp Phe Ser Gly
85 90 95

Ala Ser Leu Leu Gly Trp Gly Asp Gly Tyr Tyr Lys Gly Glu Glu Asp
100 105 110

Lys Gly Lys Arg Lys Met Thr Pro Ser Ser Val Ser Glu Gln Glu His
115 120 125

Arg Lys Lys Val Leu Arg Glu Leu Asn Ser Leu Ile Ser Gly Thr Ala
130 135 140

Ser Ser Ser Asp Asp Ala Val Asp Glu Glu Val Thr Asp Thr Glu Trp
145 150 155 160

Phe Phe Leu Val Ser Met Thr Gln Ser Phe Val Asn Gly Ala Gly Leu
165 170 175

Pro Gly Gln Ala Leu Phe Asn Ser Ser Pro Val Trp Val Val Gly Thr
180 185 190

Glu Arg Leu Met Ser Ser Pro Cys Glu Arg Ala Arg Gln Ala Gln Val
195 200 205

Phe Gly Leu Gln Thr Met Val Cys Ile Pro Ser Ala Asn Gly Val Val
210 215 220

Glu Leu Gly Ser Thr Glu Leu Ile Tyr Gln Ser Ser Asp Leu Met Asn
225 230 235 240

Lys Val Arg Val Leu Phe Asn Phe Asn Asn Leu Glu Val Gly Ser Trp
245 250 255

Pro Ile Gly Ala Ala Ala Pro Asp Gln Gly Glu Ser Asp Pro Ser Ser
260 265 270

Leu Trp Ile Ser Asp Pro Thr Ser Asn Val Glu Ile Lys Asp Ser Val
275 280 285

```
Asn Ala Thr Ala Thr Gly Ala Ser Asn Pro Ile Gly Asn Gln Gln Asn
    290                 295                 300

Ser Lys Ser Ile Gln Phe Glu Asn Pro Ser Ser Ser Ser Leu Thr Glu
305                 310                 315                 320

Asn Pro Ser Ile Met His Asn Pro Gln Gln Gln Gln Gln Ile His Thr
                325                 330                 335

Gln Gly Phe Phe Thr Arg Glu Leu Asn Phe Ser Glu Phe Gly Phe Asp
            340                 345                 350

Gly Asn Asn Gly Arg Asn Gly Asn Leu His Ser Leu Lys Pro Glu Ser
        355                 360                 365

Gly Glu Ile Leu Asn Phe Gly Asp Ser Lys Arg Ser Ser Cys Ser Ala
    370                 375                 380

Asn Gly Asn Met Phe Ser Gly His Ser Gln Val Val Ala Glu Glu Asn
385                 390                 395                 400

Lys Lys Arg Arg Ser Pro Thr Ser Arg Gly Ser Ala Glu Glu Gly Met
                405                 410                 415

Leu Ser Phe Thr Ser Gly Val Ile Leu Pro Ser Ser Cys Val Val Lys
            420                 425                 430

Ser Ser Gly Gly Gly Gly Asp Ser Asp His Ser Asp Leu Glu Ala Ser
        435                 440                 445

Val Val Arg Glu Ala Asp Ser Ser Arg Val Val Glu Pro Glu Lys Arg
    450                 455                 460

Pro Arg Lys Arg Gly Arg Lys Pro Ala Asn Gly Arg Glu Glu Pro Leu
465                 470                 475                 480

Asn His Val Glu Ala Glu Arg Gln Arg Arg Glu Lys Leu Asn Gln Arg
                485                 490                 495

Phe Tyr Ala Leu Arg Ala Val Val Pro Asn Val Ser Lys Met Asp Lys
            500                 505                 510

Ala Ser Leu Leu Gly Asp Ala Ile Ser Tyr Ile Asn Glu Leu Arg Thr
        515                 520                 525

Lys Leu Gln Ser Ala Glu Ser Asp Lys Glu Asp Leu Gln Lys Glu Val
    530                 535                 540

Asn Ser Met Lys Lys Glu Leu Ala Ser Lys Asp Xaa Gln Tyr Ser Gly
545                 550                 555                 560
```

```
Ser Ser Arg Pro Pro Pro Asp Gln Asp Leu Lys Met Ser Asn His His
                565                 570                 575

Gly Ser Lys Leu Val Glu Met Asp Ile Asp Val Lys Ile Ile Gly Trp
            580                 585                 590

Asp Ala Met Ile Arg Ile Gln Cys Ser Lys Lys Asn His Pro Ala Ala
        595                 600                 605

Lys Leu Met Gly Ala Leu Lys Glu Leu Asp Leu Asp Val Asn His Ala
    610                 615                 620

Ser Val Ser Val Val Asn Asp Leu Met Ile Gln Gln Ala Thr Val Lys
625                 630                 635                 640

Met Gly Ser Arg Phe Tyr Thr Gln Asp Gln Leu Arg Leu Ala Leu Ser
                645                 650                 655

Ser Lys Phe Ala Asp Ser Arg
            660

<210>  119
<211>  2145
<212>  DNA
<213>  Zea mays

<400>  119
atgaacctgt ggacggacga caacgcctcc atgatggagg ccttcatggc ttctgccgac      60
ctccccacct tcccctgggg agcgccggcc gggggcggca actcgtcggc cgccgcggcg     120
tcgccgccgc cgcagatgcc agcggcgatg gctcccgggt tcaaccagga cacgctgcag     180
cagaggctac aagccatgat agaggggtcg agggagacct ggacctacgc catcttctgg     240
cagtcctccc ttgactccgc caccggcgcc tcgctgctcg gctgggggga cggctactac     300
aagggctgcg acgaagacaa gcgcaagcag aagccgctca cgccctccgc ccaggccgag     360
caggagcacc gcaagcgcgt gctccgcgag ctcaactccc tcatctccgg cgcggcggcg     420
gctcccgacg aggccgtcga ggaggaggtc accgataccg agtggttctt cctcgtctcc     480
atgacgcagt cgtttctcaa cggttcgggc ctccccgggc aggcgctctt cgccgggcag     540
cccacatgga tcgcctctgg cctctcctcc gcgccgtgcg agcgcgcgcg tcaggcctac     600
aacttcggcc tccgcaccat ggtatgcttc cccgtcggca ccggggtgct cgagctcggc     660
tccaccgacg tcgtgttcaa gaccgccgag agcatggcta agatccgctc gctctttggg     720
ggcggagcag gggtggctcc gtggccgccc gtgcagcctc aggcgccgtc gtcgcagcag     780
cccgccgccg gagccgacca cgcggagacg gacccgtcca tgctatggct cgccgacgcg     840
ccggtcatgg acatcaagga ttccttgtcg cacccgtcgg ccgagatctc cgtctccaag     900
cctccgccgc atccgccgca gatccatttt gagaacggga gcacgagcac gctcacggag     960
aaccccagcc cctccgtgca cgcgccgcca cccccgccgg cgccggctgc tccacagcag    1020
```

```
cggcagcacc agcaccagaa tcaggcgcac cagggcccgt tccgccggga gctcaatttc    1080
tcggacttcg cgtccactcc ttccttggcg gcgaccccgc cgttcttcaa gcccgagtcc    1140
ggcgagatcc tcagcttcgg cgccgacagc aacgcccgga ggaacccgtc gccggtacct    1200
cccgccgcca ccgccagcct caccaccgct cccgggagcc tcttctcgca gcacacggcg    1260
accatgacgg ccgccgcggc gaacgacgcg aagaacaaca acaagcgctc aatggaggcc    1320
acctcccggg cgagcaacac caaccaccac ccggcggcga cggcgaacga gggcatgctg    1380
tccttctcgt cggcgccgac tacgcggccg tcgacgggca cgggcgcgcc ggccaagtcg    1440
gagtccgacc actcggacct ggacgcgtct gtgcgcgagg tggagagcag ccgcgtggtg    1500
gcgccgccac cggaggcgga gaagcggccg cgaaagcgcg ggcggaagcc cgcgaacggg    1560
cgcgaggagc cgctgaacca cgtggaggcg gagcggcagc ggcgggagaa gctgaaccag    1620
cgcttctacg cgctgcgcgc cgtggtgccc aacgtgtcga agatggacaa ggcgtcgctg    1680
ctcggcgacg ccatctccta catcaacgag ctccggggca agctgacgtc gctggagacc    1740
gacaaggaga cgctgcagac ccaggtggag gcgctcaaga aggagcgcga cgcgcggccg    1800
ccctcgcact ccgctgggct cggcgggcac gacggcgggc cgcgctgcca cgcggtggag    1860
atcgacgcca agatcctggg gctggaggcc atgatccgcg tgcagtgcca caagcgcaac    1920
cacccgtcgg cgcggctgat gacagcgctc cgcgagctcg acctggacgt gtaccacgcc    1980

agcgtgtccg tcgtcaagga cctcatgatc cagcaggtcg ccgtcaagat ggccagccgc    2040
gtgtacacgc aggaccagct cagcgccgcg ctctacagcc gcctcgcgga gcccgggtct    2100
gccatgggca ggtaatgaag gaggagagct gctcagctcg gctcg                   2145
```

<210> 120
<211> 704

<212> PRT
<213> Zea mays

<400> 120

```
Met Asn Leu Trp Thr Asp Asp Asn Ala Ser Met Met Glu Ala Phe Met
1               5                   10                  15

Ala Ser Ala Asp Leu Pro Thr Phe Pro Trp Gly Ala Pro Ala Gly Gly
            20                  25                  30

Gly Asn Ser Ser Ala Ala Ala Ala Ser Pro Pro Pro Gln Met Pro Ala
        35                  40                  45

Ala Met Ala Pro Gly Phe Asn Gln Asp Thr Leu Gln Gln Arg Leu Gln
    50                  55                  60

Ala Met Ile Glu Gly Ser Arg Glu Thr Trp Thr Tyr Ala Ile Phe Trp
65                  70                  75                  80
```

```
Gln Ser Ser Leu Asp Ser Ala Thr Gly Ala Ser Leu Leu Gly Trp Gly
                85                  90                  95

Asp Gly Tyr Tyr Lys Gly Cys Asp Glu Asp Lys Arg Lys Gln Lys Pro
            100                 105                 110

Leu Thr Pro Ser Ala Gln Ala Glu Gln Glu His Arg Lys Arg Val Leu
        115                 120                 125

Arg Glu Leu Asn Ser Leu Ile Ser Gly Ala Ala Ala Ala Pro Asp Glu
    130                 135                 140

Ala Val Glu Glu Glu Val Thr Asp Thr Glu Trp Phe Phe Leu Val Ser
145                 150                 155                 160

Met Thr Gln Ser Phe Leu Asn Gly Ser Gly Leu Pro Gly Gln Ala Leu
                165                 170                 175

Phe Ala Gly Gln Pro Thr Trp Ile Ala Ser Gly Leu Ser Ser Ala Pro
            180                 185                 190

Cys Glu Arg Ala Arg Gln Ala Tyr Asn Phe Gly Leu Arg Thr Met Val
        195                 200                 205

Cys Phe Pro Val Gly Thr Gly Val Leu Glu Leu Gly Ser Thr Asp Val
    210                 215                 220

Val Phe Lys Thr Ala Glu Ser Met Ala Lys Ile Arg Ser Leu Phe Gly
225                 230                 235                 240

Gly Gly Ala Gly Gly Gly Ser Trp Pro Pro Val Gln Pro Gln Ala Pro
                245                 250                 255

Ser Ser Gln Gln Pro Ala Ala Gly Ala Asp His Ala Glu Thr Asp Pro
            260                 265                 270

Ser Met Leu Trp Leu Ala Asp Ala Pro Val Met Asp Ile Lys Asp Ser
        275                 280                 285

Leu Ser His Pro Ser Ala Glu Ile Ser Val Ser Lys Pro Pro Pro His
    290                 295                 300

Pro Pro Gln Ile His Phe Glu Asn Gly Ser Thr Ser Thr Leu Thr Glu
305                 310                 315                 320

Asn Pro Ser Pro Ser Val His Ala Pro Pro Pro Pro Pro Ala Pro Ala
                325                 330                 335

Ala Pro Gln Gln Arg Gln His Gln His Gln Asn Gln Ala His Gln Gly
            340                 345                 350

Pro Phe Arg Arg Glu Leu Asn Phe Ser Asp Phe Ala Ser Thr Pro Ser
```

355 360 365

Leu Ala Ala Thr Pro Pro Phe Phe Lys Pro Glu Ser Gly Glu Ile Leu
370 375 380

Ser Phe Gly Ala Asp Ser Asn Ala Arg Arg Asn Pro Ser Pro Val Pro
385 390 395 400

Pro Ala Ala Thr Ala Ser Leu Thr Thr Ala Pro Gly Ser Leu Phe Ser
405 410 415

Gln His Thr Ala Thr Met Thr Ala Ala Ala Ala Asn Asp Ala Lys Asn
420 425 430

Asn Asn Lys Arg Ser Met Glu Ala Thr Ser Arg Ala Ser Asn Thr Asn
435 440 445

His His Pro Ala Ala Thr Ala Asn Glu Gly Met Leu Ser Phe Ser Ser
450 455 460

Ala Pro Thr Thr Arg Pro Ser Thr Gly Thr Gly Ala Pro Ala Lys Ser
465 470 475 480

Glu Ser Asp His Ser Asp Leu Asp Ala Ser Val Arg Glu Val Glu Ser
485 490 495

Ser Arg Val Val Ala Pro Pro Pro Glu Ala Glu Lys Arg Pro Arg Lys
500 505 510

Arg Gly Arg Lys Pro Ala Asn Gly Arg Glu Glu Pro Leu Asn His Val
515 520 525

Glu Ala Glu Arg Gln Arg Arg Glu Lys Leu Asn Gln Arg Phe Tyr Ala
530 535 540

Leu Arg Ala Val Val Pro Asn Val Ser Lys Met Asp Lys Ala Ser Leu
545 550 555 560

Leu Gly Asp Ala Ile Ser Tyr Ile Asn Glu Leu Arg Gly Lys Leu Thr
565 570 575

Ser Leu Glu Thr Asp Lys Glu Thr Leu Gln Thr Gln Val Glu Ala Leu
580 585 590

Lys Lys Glu Arg Asp Ala Arg Pro Pro Ser His Ser Ala Gly Leu Gly
595 600 605

Gly His Asp Gly Gly Pro Arg Cys His Ala Val Glu Ile Asp Ala Lys
610 615 620

Ile Leu Gly Leu Glu Ala Met Ile Arg Val Gln Cys His Lys Arg Asn
625 630 635 640

His Pro Ser Ala Arg Leu Met Thr Ala Leu Arg Glu Leu Asp Leu Asp
645 650 655

Val Tyr His Ala Ser Val Ser Val Val Lys Asp Leu Met Ile Gln Gln
660 665 670

Val Ala Val Lys Met Ala Ser Arg Val Tyr Thr Gln Asp Gln Leu Ser
675 680 685

Ala Ala Leu Tyr Ser Arg Leu Ala Glu Pro Gly Ser Ala Met Gly Arg
690 695 700

<210> 121
<211> 1683
<212> DNA
<213> Brassica napus

<400> 121
atggatgatc atcggaatcg tgtctctctc tctccaccgg atgctcacat gagcaactac 60
ttcctcaacc agtcaacagc caccgacgac gataacgcct ccgcctcgat ggaagctttc 120
atcggaacaa atcactggtc acaacaacca tcccttcctc ctcctccgtc attgtctcag 180
ttcaacgaag atactctcca gcaacgtctc caaacgctaa tcgaatccgc gggtgagaga 240
tggacgtacg cgatcttctg gcagatctcg cacgatttcg actctcccgc cggagagagc 300
acggtgattc tcgggtgggg agacgggtac tacagaggag aggaagacaa agagaagaag 360
aagaaacaga gctcgagctc gaatccggcg gagcaggagc atcggaaaag agtaatccgg 420
gagcttaact cgttaatcgc cggcggagcc ggagtttccg acgaagccaa cgacgaggaa 480
gttacggata cagagtggtt cttcttggtt tcgatgactc agagcttcgt aaacggcgtt 540
gggctccccg gagagtcttt tttaaactcc cgcgtgattt ggttatccgg gtcgggtgcg 600
ttaaccgggt caggctgcga gcgggcgagg caaggggagg tttacgggtt gcagacgata 660
gtgtgtatcg ctgcggaaaa cggcgtcgtt gagcttggtt cgtcggaggt gataagtcaa 720
agctcagatc tgatggataa agtcaacggt cttttcaacg gtaacggtaa cggggaagct 780
tcttcttggg ggtttaatct caatcctgat caaggtgaga acgatcctgc tttgtggtta 840
tctgaaccga acataaccgg aatcgaaccg gttcaggaaa cgcccgcgat tgagaacgcc 900
gctgatttga acttctcgag ctcagggctg aaccaaaacg gtaactttaa acaagggtcg 960
tcaagcaaca agaagagatc tccggttggg aaagacgaag aaatgctttc tttcagcacg 1020
gtggttcgat ccgcggcgaa gtcagtggac tctgatcatt ccgatatcga agcatcggtg 1080
gttaaggagg cgattatcgt cgaaccggag aagaaaccga ggaaacgggg gagaaaaccg 1140
gctaacggga gagaagagcc gttgaaccat gtggaagcag agaggcagcg aagggagaag 1200
ctaaaccaga gattctactc gttgagagct gtggttccca acgtttcgaa aatggacaag 1260
gcttctcttc ttggagacgc catttcgtat atcaacgagc tgaaaacgaa gctgcagcaa 1320

gcggagaccg ataaggaaga ggttcagaag cagctagatc ggatgagcaa ggaaggtggc 1380
gggtctagga gggcgaaaga gcgaaaatcg aacctagatt ccgcgagttc tgtggaaatg 1440
gagattgatg tgaagatcat aggttgggat gtgatgatac gtgtacaatg cggcaagaag 1500
aatcatcctg gtgcgaggtt catggaagcg cttaaggagt tggatttgga agtgaatcat 1560
gctagtttat ctatggtgaa tgatttgatg attcaacaag ctactgtgaa gatgggaagc 1620
cagtttttca atcatgatca gctcaaggct gctttaatgt tgaaagttgg agaagacagt 1680
tga 1683

<210> 122
<211> 560
<212> PRT
<213> Brassica napus

<400> 122

Met Asp Asp His Arg Asn Arg Val Ser Leu Ser Pro Pro Asp Ala His
1 5 10 15

Met Ser Asn Tyr Phe Leu Asn Gln Ser Thr Ala Thr Asp Asp Asp Asn
20 25 30

Ala Ser Ala Ser Met Glu Ala Phe Ile Gly Thr Asn His Trp Ser Gln
35 40 45

Gln Pro Ser Leu Pro Pro Pro Pro Ser Leu Ser Gln Phe Asn Glu Asp
50 55 60

Thr Leu Gln Gln Arg Leu Gln Thr Leu Ile Glu Ser Ala Gly Glu Arg
65 70 75 80

Trp Thr Tyr Ala Ile Phe Trp Gln Ile Ser His Asp Phe Asp Ser Pro
85 90 95

Ala Gly Glu Ser Thr Val Ile Leu Gly Trp Gly Asp Gly Tyr Tyr Arg
100 105 110

Gly Glu Glu Asp Lys Glu Lys Lys Lys Lys Gln Ser Ser Ser Ser Asn
115 120 125

Pro Ala Glu Gln Glu His Arg Lys Arg Val Ile Arg Glu Leu Asn Ser
130 135 140

Leu Ile Ala Gly Gly Ala Gly Val Ser Asp Glu Ala Asn Asp Glu Glu
145 150 155 160

Val Thr Asp Thr Glu Trp Phe Phe Leu Val Ser Met Thr Gln Ser Phe
165 170 175

Val Asn Gly Val Gly Leu Pro Gly Glu Ser Phe Leu Asn Ser Arg Val
180 185 190

```
Ile Trp Leu Ser Gly Ser Gly Ala Leu Thr Gly Ser Gly Cys Glu Arg
        195                 200                 205

Ala Arg Gln Gly Glu Val Tyr Gly Leu Gln Thr Ile Val Cys Ile Ala
    210                 215                 220

Ala Glu Asn Gly Val Val Glu Leu Gly Ser Ser Glu Val Ile Ser Gln
225                 230                 235                 240

Ser Ser Asp Leu Met Asp Lys Val Asn Gly Leu Phe Asn Gly Asn Gly
                245                 250                 255

Asn Gly Glu Ala Ser Ser Trp Gly Phe Asn Leu Asn Pro Asp Gln Gly
            260                 265                 270

Glu Asn Asp Pro Ala Leu Trp Leu Ser Glu Pro Asn Ile Thr Gly Ile
        275                 280                 285

Glu Pro Val Gln Glu Thr Pro Ala Ile Glu Asn Ala Ala Asp Leu Asn
    290                 295                 300

Phe Ser Ser Ser Gly Leu Asn Gln Asn Gly Asn Phe Lys Gln Gly Ser
305                 310                 315                 320

Ser Ser Asn Lys Lys Arg Ser Pro Val Gly Lys Asp Glu Glu Met Leu
                325                 330                 335

Ser Phe Ser Thr Val Val Arg Ser Ala Ala Lys Ser Val Asp Ser Asp
            340                 345                 350

His Ser Asp Ile Glu Ala Ser Val Val Lys Glu Ala Ile Ile Val Glu
        355                 360                 365

Pro Glu Lys Lys Pro Arg Lys Arg Gly Arg Lys Pro Ala Asn Gly Arg
    370                 375                 380

Glu Glu Pro Leu Asn His Val Glu Ala Glu Arg Gln Arg Arg Glu Lys
385                 390                 395                 400

Leu Asn Gln Arg Phe Tyr Ser Leu Arg Ala Val Val Pro Asn Val Ser
                405                 410                 415

Lys Met Asp Lys Ala Ser Leu Leu Gly Asp Ala Ile Ser Tyr Ile Asn
            420                 425                 430

Glu Leu Lys Thr Lys Leu Gln Gln Ala Glu Thr Asp Lys Glu Glu Val
        435                 440                 445

Gln Lys Gln Leu Asp Arg Met Ser Lys Glu Gly Gly Gly Ser Arg Arg
    450                 455                 460
```

Ala Lys Glu Arg Lys Ser Asn Leu Asp Ser Ala Ser Ser Val Glu Met
465 470 475 480

Glu Ile Asp Val Lys Ile Ile Gly Trp Asp Val Met Ile Arg Val Gln
485 490 495

Cys Gly Lys Lys Asn His Pro Gly Ala Arg Phe Met Glu Ala Leu Lys
500 505 510

Glu Leu Asp Leu Glu Val Asn His Ala Ser Leu Ser Met Val Asn Asp
515 520 525

Leu Met Ile Gln Gln Ala Thr Val Lys Met Gly Ser Gln Phe Phe Asn
530 535 540

His Asp Gln Leu Lys Ala Ala Leu Met Leu Lys Val Gly Glu Asp Ser
545 550 555 560

<210> 123
<211> 1899
<212> DNA
<213> Glycine max

<400> 123
atgaatctgt ggacggacga gaactcctcg gtgatggagg ccttcatgag ctcctccgac 60
ctctcctccc tctggctccc cacgccgcaa tccgccgcct ctactaccac tcctggactg 120
gaaaccaccc gagcaccgcc gcctcagtcc cactccctcc tcaaccagga gaccctccag 180
cagcgcctcc agacacttat cgaaggcgcc cgagagagct ggacctacgc aatcttctgg 240
cagtcttcct acgactattc ctccggcacc tcccttctcg gctggggaga cggttattac 300
aagggtgagg aggacaaagt caaagccaaa ggcaaaaccc ccaaaacgac gtcgtccgcg 360
gagcaggacc accgcaaaaa agtcctccgc gaactcaatt ccttaatctc tggcccctcc 420
gcttccgttg acgacgtcga cgaagaagtc accgacaccg agtggttctt cctcgtgtcg 480
atgactcagt ccttcgtcaa cggaagcggc ctcccgggtc aggctttttt taactcgagc 540
ccggtctggg ttgccgggcc ggaccggctg tccgagtcgg tctgcgaacg ggcccaccag 600
gggcagatgt tcgggctcca gactctggtc tgcataccgt ccgcaaatgg cgtcgttgag 660
ctcgcctcca cggaggtgat tttccagaac cctgatctga tgaacaaggt gcgcgatttg 720
ttcaacttca acaacaaccc tgaaactggt tcctgggcgt tgaattgcgt tgccaccacc 780
gatcaggggg agaacgaccc ttcctcactc tggctcaacc ctgaaatcag agactcctct 840
accgttgcgc cgccgaattc aacggttaac aagactctgc agttcgaaac ccctggttcc 900
agtaccttaa ccgatacccc tagtgctgct gctgttcatg tacctaaatc taatggccag 960
ggtttcttct cgagggaatt gaacttttcg aattcattga agcctgaatc tggtgaaatt 1020
ctgagctttg gagagagcaa gaagagctct tacaacggga gtttctttcc cggtgttgtt 1080
gcaattgagg agaacaacaa gaagaggtct cccgtttctc ggagcagcat tgacgatggg 1140

atgctgtcct tcacatccct gccggctgca aatataaaat ctggtagtgg tggtgccggt 1200
gccggtggtg gtgattcgga tcactcggat ctggaagctt ccatggtgaa acaggcagac 1260
agcagagtga tggagccgga gaaacggcct cggaagaggg ggcgaaagcc ggccaacggc 1320
agagaggagc cgctgaatca cgtggaagcc gagagacaaa ggagagaaaa gctgaatcaa 1380
agattctatg ctctacgagc tgtagttcca aatgtatcca agatggacaa ggcatcgttg 1440
ttaggtgacg ccatatcgta cataaatgag ctgaaattga agctcaacgg gctggattca 1500
gagaaagggg aattggagaa gcaattggat tcagccaaga aggaacttga gctcgcgacc 1560
aaaaaccctc ctcctcctcc accaccacca cctggactac ctccatctaa taacgaagaa 1620
gcaaagaaga caacgaccaa gctcgctgat ttggagatag aggtgaagat aattggttgg 1680
gatgcgatga taagaatcca atgcagcaag aagaaccacc ctgcagcaag gttgatggca 1740
gcattgaagg acctggactt ggaagtgcat cacgcaagcg tgtccgtggt gaatgattta 1800
atgatccaac aagccacggt gaacatggga aacaagtttt acacgcagga gcagcttctg 1860
tcggcactct cgtccaaagt tggcgatgaa ctacgatag 1899

<210> 124
<211> 632
<212> PRT
<213> Glycine max

<400> 124

Met Asn Leu Trp Thr Asp Glu Asn Ser Ser Val Met Glu Ala Phe Met
1               5                   10                  15

Ser Ser Ser Asp Leu Ser Ser Leu Trp Leu Pro Thr Pro Gln Ser Ala
            20                  25                  30

Ala Ser Thr Thr Thr Pro Gly Leu Glu Thr Thr Arg Ala Pro Pro Pro
        35                  40                  45

Gln Ser His Ser Leu Leu Asn Gln Glu Thr Leu Gln Gln Arg Leu Gln
    50                  55                  60

Thr Leu Ile Glu Gly Ala Arg Glu Ser Trp Thr Tyr Ala Ile Phe Trp
65                  70                  75                  80

Gln Ser Ser Tyr Asp Tyr Ser Ser Gly Thr Ser Leu Leu Gly Trp Gly
                85                  90                  95

Asp Gly Tyr Tyr Lys Gly Glu Glu Asp Lys Val Lys Ala Lys Gly Lys
            100                 105                 110

Thr Pro Lys Thr Thr Ser Ser Ala Glu Gln Asp His Arg Lys Lys Val
        115                 120                 125

Leu Arg Glu Leu Asn Ser Leu Ile Ser Gly Pro Ser Ala Ser Val Asp
    130                 135                 140

Asp Val Asp Glu Glu Val Thr Asp Thr Glu Trp Phe Phe Leu Val Ser
145 150 155 160

Met Thr Gln Ser Phe Val Asn Gly Ser Gly Leu Pro Gly Gln Ala Phe
165 170 175

Phe Asn Ser Ser Pro Val Trp Val Ala Gly Pro Asp Arg Leu Ser Glu
180 185 190

Ser Val Cys Glu Arg Ala His Gln Gly Gln Met Phe Gly Leu Gln Thr
195 200 205

Leu Val Cys Ile Pro Ser Ala Asn Gly Val Val Glu Leu Ala Ser Thr
210 215 220

Glu Val Ile Phe Gln Asn Pro Asp Leu Met Asn Lys Val Arg Asp Leu
225 230 235 240

Phe Asn Phe Asn Asn Asn Pro Glu Thr Gly Ser Trp Ala Leu Asn Cys
245 250 255

Val Ala Thr Thr Asp Gln Gly Glu Asn Asp Pro Ser Ser Leu Trp Leu
260 265 270

Asn Pro Glu Ile Arg Asp Ser Ser Thr Val Ala Pro Pro Asn Ser Thr
275 280 285

Val Asn Lys Thr Leu Gln Phe Glu Thr Pro Gly Ser Ser Thr Leu Thr
290 295 300

Asp Thr Pro Ser Ala Ala Ala Val His Val Pro Lys Ser Asn Gly Gln
305 310 315 320

Gly Phe Phe Ser Arg Glu Leu Asn Phe Ser Asn Ser Leu Lys Pro Glu
325 330 335

Ser Gly Glu Ile Leu Ser Phe Gly Glu Ser Lys Lys Ser Ser Tyr Asn
340 345 350

Gly Ser Phe Phe Pro Gly Val Val Ala Ile Glu Glu Asn Asn Lys Lys
355 360 365

Arg Ser Pro Val Ser Arg Ser Ser Ile Asp Asp Gly Met Leu Ser Phe
370 375 380

Thr Ser Leu Pro Ala Ala Asn Ile Lys Ser Gly Ser Gly Gly Ala Gly
385 390 395 400

Ala Gly Gly Gly Asp Ser Asp His Ser Asp Leu Glu Ala Ser Met Val
405 410 415

Lys Gln Ala Asp Ser Arg Val Met Glu Pro Glu Lys Arg Pro Arg Lys
420 425 430

Arg Gly Arg Lys Pro Ala Asn Gly Arg Glu Glu Pro Leu Asn His Val
435 440 445

Glu Ala Glu Arg Gln Arg Arg Glu Lys Leu Asn Gln Arg Phe Tyr Ala
450 455 460

Leu Arg Ala Val Val Pro Asn Val Ser Lys Met Asp Lys Ala Ser Leu
465 470 475 480

Leu Gly Asp Ala Ile Ser Tyr Ile Asn Glu Leu Lys Leu Lys Leu Asn
485 490 495

Gly Leu Asp Ser Glu Lys Gly Glu Leu Glu Lys Gln Leu Asp Ser Ala
500 505 510

Lys Lys Glu Leu Glu Leu Ala Thr Lys Asn Pro Pro Pro Pro Pro Pro
515 520 525

Pro Pro Pro Gly Leu Pro Pro Ser Asn Asn Glu Glu Ala Lys Lys Thr
530 535 540

Thr Thr Lys Leu Ala Asp Leu Glu Ile Glu Val Lys Ile Ile Gly Trp
545 550 555 560

Asp Ala Met Ile Arg Ile Gln Cys Ser Lys Lys Asn His Pro Ala Ala
565 570 575

Arg Leu Met Ala Ala Leu Lys Asp Leu Asp Leu Glu Val His His Ala
580 585 590

Ser Val Ser Val Val Asn Asp Leu Met Ile Gln Gln Ala Thr Val Asn
595 600 605

Met Gly Asn Lys Phe Tyr Thr Gln Glu Gln Leu Leu Ser Ala Leu Ser
610 615 620

Ser Lys Val Gly Asp Glu Leu Arg
625 630

<210> 125
<211> 1457
<212> DNA
<213> Glycine max

<400> 125
atgaccgagt accggatgaa cctgtggacc gacgacaact cctccgtcat ggaggccttc 60

atgagctcct ccgacctctc ctccctctgg cttgccacgc cgcagtccgc cacctccacc 120

acaactccag gcacggcaaa agcccctcct cctcctcctc ctcctccgcc gccgccggcg 180

cagtcccagt ccctcctcaa ccaggagact ctccagcaac gcctccagac tctcatcgaa 240
ggcgcctgcg agagctggac ctacgcaatc ttctggcaat cttcctacga ctattcctcc 300
ggcacctccc ttctcggctg gggagacggt tactacaagg gcgaggagga caaagacaaa 360
gtcaaaacca aagcccccaa aacgaggtcg tccgcagagc aggaccatcg taagaaggtc 420
ctccgcgaac ttaattcctt aatttccggc ccttccgctt cagctgacga cattgacgaa 480
gaagtcaccg acaccgagtg gttcttcctg gtgtcgatga ctcagtcctt cgtcaacggt 540
agcgggctgc cgggccaggc ttttttaac tcgagcccgg tctgggtcgc cgggcccgaa 600
agactttccg aatcggcctg cgaacgggcc cgccaggggc agctcttcgg gctccagact 660
cttgtctgta taccgtccgc aaacggcgtc gtcgagctcg cctccgcgga ggtcattttt 720
cagaaccccg atctgatgaa caaggtgcgt gatttgttca acttcaacaa caacaacaac 780
aacaacaacc ctgaaacgtg ttcctgggcg ttgaattgcg ttgccaccac cgatcaaggc 840
gagaacgacc cttcctcgct ctggctcaac cctgaaatca aagactcctc caccgtctcg 900
ccgccgaatt ccacggttaa taagacgatg catttcgaaa cccctggttc cagtacccta 960
accgaaaccc ctagcgctgc tgctgctgtt cacgtcccca attctaaaag ccagggattc 1020
ttcccgaggg aattgaactt ttcgaattca ttgaaacctg aatccggtga aattctgagc 1080
tttggggaga gcaagaagag ctcttacaat ggggctttct ttcccggtgt tgttgcggtt 1140
gaggagaaca acaacaataa caagaacaag aagaagaggt ccccggtggt ttctcggagc 1200
agcattgacg atggaatgct ttccttcact tccctgccgg ctgcaaatat aaaatcagtt 1260
aacggtgctt gtgtcggtgc cggtgattcg gatcactcgg atctggaagc ttccgtggcg 1320
aaacaggtgg tggagcccga gaaacggccc cggaagagag ggcgaaagcc ggctaacgga 1380
agagaggaac cactaaatca tgtggaagcc gagagacaga ggagagaaaa gctgaaccaa 1440
agattctatg ctctacg 1457

<210> 126
<211> 654
<212> PRT
<213> Glycine max

<400> 126

Met Thr Glu Tyr Arg Met Asn Leu Trp Thr Asp Asp Asn Ser Ser Val
1 5 10 15

Met Glu Ala Phe Met Ser Ser Ser Asp Leu Ser Ser Leu Trp Leu Ala
20 25 30

Thr Pro Gln Ser Ala Thr Ser Thr Thr Thr Pro Gly Thr Ala Lys Ala
35 40 45

Pro Pro Pro Pro Pro Pro Pro Pro Pro Pro Pro Ala Gln Ser Gln Ser
50 55 60

Leu Leu Asn Gln Glu Thr Leu Gln Gln Arg Leu Gln Thr Leu Ile Glu

```
65                  70                  75                  80

Gly Ala Cys Glu Ser Trp Thr Tyr Ala Ile Phe Trp Gln Ser Ser Tyr
                85                  90                  95

Asp Tyr Ser Ser Gly Thr Ser Leu Leu Gly Trp Gly Asp Gly Tyr Tyr
            100                 105                 110

Lys Gly Glu Glu Asp Lys Asp Lys Val Lys Thr Lys Ala Pro Lys Thr
        115                 120                 125

Arg Ser Ser Ala Glu Gln Asp His Arg Lys Lys Val Leu Arg Glu Leu
    130                 135                 140

Asn Ser Leu Ile Ser Gly Pro Ser Ala Ser Ala Asp Asp Ile Asp Glu
145                 150                 155                 160

Glu Val Thr Asp Thr Glu Trp Phe Phe Leu Val Ser Met Thr Gln Ser
                165                 170                 175

Phe Val Asn Gly Ser Gly Leu Pro Gly Gln Ala Phe Phe Asn Ser Ser
            180                 185                 190

Pro Val Trp Val Ala Gly Pro Glu Arg Leu Ser Glu Ser Ala Cys Glu
        195                 200                 205

Arg Ala Arg Gln Gly Gln Leu Phe Gly Leu Gln Thr Leu Val Cys Ile
    210                 215                 220

Pro Ser Ala Asn Gly Val Val Glu Leu Ala Ser Ala Glu Val Ile Phe
225                 230                 235                 240

Gln Asn Pro Asp Leu Met Asn Lys Val Arg Asp Leu Phe Asn Phe Asn
                245                 250                 255

Asn Asn Asn Asn Asn Asn Asn Pro Glu Thr Cys Ser Trp Ala Leu Asn
            260                 265                 270

Cys Val Ala Thr Thr Asp Gln Gly Glu Asn Asp Pro Ser Ser Leu Trp
        275                 280                 285

Leu Asn Pro Glu Ile Lys Asp Ser Ser Thr Val Ser Pro Pro Asn Ser
    290                 295                 300

Thr Val Asn Lys Thr Met His Phe Glu Thr Pro Gly Ser Ser Thr Leu
305                 310                 315                 320

Thr Glu Thr Pro Ser Ala Ala Ala Ala Val His Val Pro Asn Ser Lys
                325                 330                 335

Ser Gln Gly Phe Phe Pro Arg Glu Leu Asn Phe Ser Asn Ser Leu Lys
            340                 345                 350
```

Pro Glu Ser Gly Glu Ile Leu Ser Phe Gly Glu Ser Lys Lys Ser Ser
355 360 365

Tyr Asn Gly Ala Phe Phe Pro Gly Val Val Ala Val Glu Glu Asn Asn
370 375 380

Asn Asn Asn Lys Asn Lys Lys Lys Arg Ser Pro Val Val Ser Arg Ser
385 390 395 400

Ser Ile Asp Asp Gly Met Leu Ser Phe Thr Ser Leu Pro Ala Ala Asn
405 410 415

Ile Lys Ser Val Asn Gly Ala Cys Val Gly Ala Gly Asp Ser Asp His
420 425 430

Ser Asp Leu Glu Ala Ser Val Ala Lys Gln Val Val Glu Pro Glu Lys
435 440 445

Arg Pro Arg Lys Arg Gly Arg Lys Pro Ala Asn Gly Arg Glu Glu Pro
450 455 460

Leu Asn His Val Glu Ala Glu Arg Gln Arg Arg Glu Lys Leu Asn Gln
465 470 475 480

Arg Phe Tyr Ala Leu Arg Ala Val Val Pro Asn Val Ser Lys Met Asp
485 490 495

Lys Ala Ser Leu Leu Gly Asp Ala Ile Leu Tyr Ile Asn Glu Leu Lys
500 505 510

Ser Lys Leu Asn Val Leu Asp Ser Glu Lys Thr Glu Leu Glu Lys Gln
515 520 525

Leu Asp Ser Thr Lys Lys Glu Leu Glu Leu Ala Thr Lys Asn Pro Pro
530 535 540

Pro Pro Pro Pro Pro Pro Pro Pro Pro Gly Pro Pro Pro Ser Asn Ser
545 550 555 560

Val Glu Pro Lys Lys Thr Thr Ser Lys Leu Ala Asp Leu Glu Leu Glu
565 570 575

Val Lys Ile Ile Gly Trp Asp Ala Met Val Arg Ile Gln Cys Ser Lys
580 585 590

Lys Asn His Pro Ala Ala Arg Leu Met Ala Ala Leu Lys Asp Leu Asp
595 600 605

Leu Glu Val His His Ala Ser Val Ser Val Val Asn Asp Leu Met Ile
610 615 620

Gln Gln Ala Thr Val Asn Met Gly Asn Lys Phe Tyr Thr Gln Glu Gln
625 630 635 640

Leu Leu Ser Ala Leu Ser Ser Lys Val Gly Asp Glu Leu Arg
645 650

<210> 127
<211> 2025
<212> DNA
<213> Hordeum vulgare

<400> 127
atgatggagg ccctcatggc ctccgccgac atgccggcgt tcccctgggg cgcggcggcc 60

accccgccgc cgccggccgc cgtgccggcc ttcaaccagg acacgctcca gcagcgcctg 120

caggccatca tcgagggctc cagggagacc tggacctacg ccatcttctg gcagtcctcc 180

accgacgccg gcgcctcgct cctcggctgg ggcgacggct attacaaggg ctgcgacgac 240

gccgacaagc gccgccagca gcccaccccg gcctccgccg ccgagcagga gcaccgcaag 300

cgcgtcctca gggagctcaa ctcgctcata gccgggggcg gcgccgccgc gcccgacgag 360

gccgtcgagg aggagggcac ggacaccgag tgggtcttcc tcgtctccat gacccagtcc 420

ttccccaacg ggatgggctt gccgggccag gctctcttcg ccggccagcc tatctggatc 480

gccaccgggc tcgccagcgc gccctgcgag cgggccaagc aggcctacac cttcggcctc 540

cgcaccatgg tctgcatccc cctcggcacc ggcgtgctcg agctcggcgc caccgaggtc 600

atcttccaga ccaccgatag cttggggcgg atccgctcgc tcttcagcct caacggcgga 660

ggagggggct ctggatcctg gccgcccgtg gcgccgccgc cccaggaggc ggagacggat 720

ccgtccgtgc tctggctcgc cgacgcgccg gccggggaca tgaaggagtc gccgccgtcc 780

gtcgagatct ccgtctccaa gccgccgccg tcacagccgc cgcagatcca tcacttcgag 840

aacgggagca ccagcacgct cacggagaac cccagcctct ccgtgcacgc gcagcagcct 900

ctgccgcagc agcaggcggc ggcggcggcg cagaggcaga accagctcca gctccagcac 960

cagcacaacc agggtccttt ccgccgggag ctcaatttct cagatttcgc gtccaaccca 1020

tccgtcacgg tgaccccgcc tttcttcaag cctgagtctg gtgagatcct aaactttggc 1080

gctgacagca ccagccggag gaacccttcg ccggcgcccc ccgccgcgac ggccagcctc 1140

accaccgcgc cggggagcct cttctcccag cacacggcga cggtgacggc cccatcaaac 1200

gacgccaaga acaacccgaa gcggtccatg gaggccacct cccgcgcgag caacaccaac 1260

caccaccaga ccgcgacagc caacgagggg atgctgtcct tctcgtccgc gccgacgacg 1320

cggccgtcca ccggcacggg cgcaccagcc aagtcggagt ccgaccattc cgacctggag 1380

gcgtcggtcc gcgaggtgga gagcagccgc gtggtgcctc cgccggagga gaagcggccg 1440

cgcaagcgcg ggcgcaagcc ggcgaacggg cgcgaggagc cactgaacca cgtggaggcg 1500

gagcggcagc ggcgggagaa gctgaaccag cggttctacg cgctccgcgc cgtggtgccc 1560

aacgtgtcca agatggacaa ggcctcactg ctgggcgacg ccatctccta catcaacgag 1620

ctccgcggta agatgacggc gctggagtcg gacaaggaga cgctccactc ccaaattgag 1680

gcgctgaaga aggagcgcga cgcccggccg gccgcgccgt cgtcgtcggg aatgcacgac 1740

aacggggcgc ggtgccacgc ggtggagatc gaggccaaga tcctggggct ggaggcgatg 1800

atccgcgtgc agtgccacaa gcgcaaccac ccggcggcga agctgatgac ggcgctgcgg 1860

gagctggacc tggacgtgta ccacgccagc gtctcggtgg tgaaggacat catgatccag 1920

caggtggcgg tgaagatggc cacccgggtc tactcccagg aacagctcaa cgcggcgctc 1980

tacggccgcc tcgccgagcc gggcgccgcg atgcaaatcc ggtaa 2025

<210> 128
<211> 674
<212> PRT
<213> Hordeum vulgare

<400> 128

Met Met Glu Ala Leu Met Ala Ser Ala Asp Met Pro Ala Phe Pro Trp
1 5 10 15

Gly Ala Ala Ala Thr Pro Pro Pro Pro Ala Ala Val Pro Ala Phe Asn
20 25 30

Gln Asp Thr Leu Gln Gln Arg Leu Gln Ala Ile Ile Glu Gly Ser Arg
35 40 45

Glu Thr Trp Thr Tyr Ala Ile Phe Trp Gln Ser Ser Thr Asp Ala Gly
50 55 60

Ala Ser Leu Leu Gly Trp Gly Asp Gly Tyr Tyr Lys Gly Cys Asp Asp
65 70 75 80

Ala Asp Lys Arg Arg Gln Gln Pro Thr Pro Ala Ser Ala Ala Glu Gln
85 90 95

Glu His Arg Lys Arg Val Leu Arg Glu Leu Asn Ser Leu Ile Ala Gly
100 105 110

Gly Gly Ala Ala Ala Pro Asp Glu Ala Val Glu Glu Glu Gly Thr Asp
115 120 125

Thr Glu Trp Val Phe Leu Val Ser Met Thr Gln Ser Phe Pro Asn Gly
130 135 140

Met Gly Leu Pro Gly Gln Ala Leu Phe Ala Gly Gln Pro Ile Trp Ile
145 150 155 160

Ala Thr Gly Leu Ala Ser Ala Pro Cys Glu Arg Ala Lys Gln Ala Tyr
165 170 175

Thr Phe Gly Leu Arg Thr Met Val Cys Ile Pro Leu Gly Thr Gly Val
180 185 190

Leu Glu Leu Gly Ala Thr Glu Val Ile Phe Gln Thr Thr Asp Ser Leu
        195                 200                 205

Gly Arg Ile Arg Ser Leu Phe Ser Leu Asn Gly Gly Gly Gly Gly Ser
    210                 215                 220

Gly Ser Trp Pro Pro Val Ala Pro Pro Pro Gln Glu Ala Glu Thr Asp
225                 230                 235                 240

Pro Ser Val Leu Trp Leu Ala Asp Ala Pro Ala Gly Asp Met Lys Glu
                245                 250                 255

Ser Pro Pro Ser Val Glu Ile Ser Val Ser Lys Pro Pro Pro Ser Gln
            260                 265                 270

Pro Pro Gln Ile His His Phe Glu Asn Gly Ser Thr Ser Thr Leu Thr
        275                 280                 285

Glu Asn Pro Ser Leu Ser Val His Ala Gln Gln Pro Leu Pro Gln Gln
    290                 295                 300

Gln Ala Ala Ala Ala Ala Gln Arg Gln Asn Gln Leu Gln Leu Gln His
305                 310                 315                 320

Gln His Asn Gln Gly Pro Phe Arg Arg Glu Leu Asn Phe Ser Asp Phe
                325                 330                 335

Ala Ser Asn Pro Ser Val Thr Val Thr Pro Pro Phe Phe Lys Pro Glu
            340                 345                 350

Ser Gly Glu Ile Leu Asn Phe Gly Ala Asp Ser Thr Ser Arg Arg Asn
        355                 360                 365

Pro Ser Pro Ala Pro Pro Ala Ala Thr Ala Ser Leu Thr Thr Ala Pro
    370                 375                 380

Gly Ser Leu Phe Ser Gln His Thr Ala Thr Val Thr Ala Pro Ser Asn
385                 390                 395                 400

Asp Ala Lys Asn Asn Pro Lys Arg Ser Met Glu Ala Thr Ser Arg Ala
                405                 410                 415

Ser Asn Thr Asn His His Gln Thr Ala Thr Ala Asn Glu Gly Met Leu
            420                 425                 430

Ser Phe Ser Ser Ala Pro Thr Thr Arg Pro Ser Thr Gly Thr Gly Ala
        435                 440                 445

Pro Ala Lys Ser Glu Ser Asp His Ser Asp Leu Glu Ala Ser Val Arg
    450                 455                 460

Glu Val Glu Ser Ser Arg Val Val Pro Pro Pro Glu Glu Lys Arg Pro
465 470 475 480

Arg Lys Arg Gly Arg Lys Pro Ala Asn Gly Arg Glu Glu Pro Leu Asn
485 490 495

His Val Glu Ala Glu Arg Gln Arg Arg Glu Lys Leu Asn Gln Arg Phe
500 505 510

Tyr Ala Leu Arg Ala Val Val Pro Asn Val Ser Lys Met Asp Lys Ala
515 520 525

Ser Leu Leu Gly Asp Ala Ile Ser Tyr Ile Asn Glu Leu Arg Gly Lys
530 535 540

Met Thr Ala Leu Glu Ser Asp Lys Glu Thr Leu His Ser Gln Ile Glu
545 550 555 560

Ala Leu Lys Lys Glu Arg Asp Ala Arg Pro Ala Ala Pro Ser Ser Ser
565 570 575

Gly Met His Asp Asn Gly Ala Arg Cys His Ala Val Glu Ile Glu Ala
580 585 590

Lys Ile Leu Gly Leu Glu Ala Met Ile Arg Val Gln Cys His Lys Arg
595 600 605

Asn His Pro Ala Ala Lys Leu Met Thr Ala Leu Arg Glu Leu Asp Leu
610 615 620

Asp Val Tyr His Ala Ser Val Ser Val Val Lys Asp Ile Met Ile Gln
625 630 635 640

Gln Val Ala Val Lys Met Ala Thr Arg Val Tyr Ser Gln Glu Gln Leu
645 650 655

Asn Ala Ala Leu Tyr Gly Arg Leu Ala Glu Pro Gly Ala Ala Met Gln
660 665 670

Ile Arg

<210> 129
<211> 2112
<212> DNA
<213> Zea mays

<400> 129
atgaacctgt ggacggacga caacgcctcc atgatggagg ccttcatggc ctccgccgac 60

ctccccgcct acccctgggg ggcgccggcc gggggcggca acccgccgcc gccgcagatg 120

ccgccggcga tggcgatggc gccggggttc aaccaggaca cgctgcagca gcggctgcag 180

gccatgatcg aggggtccag ggagacctgg acctacgcca tcttctggca gtcctccctg 240
gacgccgcca ccggcgcgtc gctgctaggc tggggcgacg gctactacaa gggctgcgac 300
gacgacaagc gcaggcaccg cccgccgctc acgcccgccg cgcaggccga gcaggagcac 360
cgcaagcgcg tgctccgcga gctcaactcc ctcatctccg gcggcgcctc cgccgcgccc 420
gcgcccgcgc ccgacgaggc cgtcgaggag gaggtcaccg acaccgagtg gttcttcctc 480
gtctccatga cgcagtcctt cctcaacggc tcgggcctcc ccggccaggc gctcttcgcg 540
ggccaccaca cctggatcgc cgccggcctc tcctccgcgc cgtgcgaccg cgcgcgccag 600
gcctacaact tcggcctccg caccatggtc tgcttccccg tcggcacggg cgtgctcgag 660
ctcggctcca ccgacgtcgt gttccagacc gccgagacca tggccaagat ccgctcgctc 720
ttcgggggcg ggccgggggg tggctcgtgg ccgcccgtgc agccccaggc ggcgccgcag 780
cagcagcacg ccgccgaagc cgaccaggcg gcggagacgg acccatccgt gctgtggctc 840
gccgacgcgc cggtcgtgga catcaaggat tcctactcgc acccgtcggc ggccgagatc 900
tccgtctcca aaccgcctcc gcctccgcct ccgccgcaga ttcacttcga gaacgggagc 960
acgagcacgc tcacggagaa ccccagcccc tccgtgcacg cgccgccagc cccgccggcg 1020
ccaccgcagc ggcagcagca gaatcagggc ccgttccgcc gggagctcaa cttctcggat 1080
ttcgcgtcca atccttcctt ggcggcggcc ccgccgttct tcaagcccga gtccggcgag 1140
atcctcagct tcggcgtcga cagcaacgcc cagaggaacc cgtcgccggc gcctcccgcc 1200
agcctcacca ccgctcccgg cagcctattc tcgcagtcgc agcacacggc gaccgccgcg 1260
gcgaacgacg cgaagaacaa caacaacaac aacaagcgtt cgatggaagc cacctccctg 1320
gcgagcaaca ccaaccacca cccggcggcg gcggcgaacg agggcatgct gtccttctcg 1380
tcggcgccga ccgcgcggcc gtcggcaggc acgggcgcgc cggccaagtc ggagtccgac 1440
cactcggacc tggacgcgtc cgtgcgcgag gtggagagca gccgcgtcgt ggcgccgccg 1500
ccggaggcgg agaagcggcc gcggaagcgc gggcggaagc ccgcgaacgg gcgcgaggag 1560
ccgctgaacc acgtggaggc ggagcggcag cggcgggaga agctgaacca gcgcttctac 1620
gcgctgcgcg ccgtggtgcc caacgtgtcc aagatggaca aggcgtcgct gctcggcgac 1680
gccatctcct acatcaacga gctccgcggc aagctgacgt cgctggagtc ggacagggag 1740
acgctgcagg cccaggtcga ggcgctcaag aaggagcgcg acgcgcggcc gcacccgcac 1800
cccgctgccg ggctcggcgg gcacgacgcc ggcgggccgc gctgccacgc ggtagagatc 1860
gacgccaaga tcctggggct ggaggccatg atccgcgtgc agtgccacaa gcgcaaccac 1920
ccgtcggcgc ggctgatgac ggcgctccgc gagctcgacc tggacgtgta ccacgccagc 1980
gtctccgtcg tcaaggacct catgatccag caggtcgccg tcaagatggc cagccgcatg 2040
tactcgcagg accagctcag cgccgcgctc tacagccgcc ttgcggagcc cgggtctgtc 2100
atgggcaggt aa 2112

<210> 130

<211> 703
<212> PRT
<213> Zea mays

<400> 130

Met Asn Leu Trp Thr Asp Asp Asn Ala Ser Met Met Glu Ala Phe Met
1               5                   10                  15

Ala Ser Ala Asp Leu Pro Ala Tyr Pro Trp Gly Ala Pro Ala Gly Gly
            20                  25                  30

Gly Asn Pro Pro Pro Pro Gln Met Pro Pro Ala Met Ala Met Ala Pro
        35                  40                  45

Gly Phe Asn Gln Asp Thr Leu Gln Gln Arg Leu Gln Ala Met Ile Glu
    50                  55                  60

Gly Ser Arg Glu Thr Trp Thr Tyr Ala Ile Phe Trp Gln Ser Ser Leu
65                  70                  75                  80

Asp Ala Ala Thr Gly Ala Ser Leu Leu Gly Trp Gly Asp Gly Tyr Tyr
                85                  90                  95

Lys Gly Cys Asp Asp Asp Lys Arg Arg His Arg Pro Pro Leu Thr Pro
            100                 105                 110

Ala Ala Gln Ala Glu Gln Glu His Arg Lys Arg Val Leu Arg Glu Leu
        115                 120                 125

Asn Ser Leu Ile Ser Gly Gly Ala Ser Ala Ala Pro Ala Pro Ala Pro
    130                 135                 140

Asp Glu Ala Val Glu Glu Glu Val Thr Asp Thr Glu Trp Phe Phe Leu
145                 150                 155                 160

Val Ser Met Thr Gln Ser Phe Leu Asn Gly Ser Gly Leu Pro Gly Gln
                165                 170                 175

Ala Leu Phe Ala Gly His His Thr Trp Ile Ala Ala Gly Leu Ser Ser
            180                 185                 190

Ala Pro Cys Asp Arg Ala Arg Gln Ala Tyr Asn Phe Gly Leu Arg Thr
        195                 200                 205

Met Val Cys Phe Pro Val Gly Thr Gly Val Leu Glu Leu Gly Ser Thr
    210                 215                 220

Asp Val Val Phe Gln Thr Ala Glu Thr Met Ala Lys Ile Arg Ser Leu
225                 230                 235                 240

Phe Gly Gly Gly Pro Gly Gly Gly Ser Trp Pro Pro Val Gln Pro Gln
                245                 250                 255

Ala Ala Pro Gln Gln Gln His Ala Ala Glu Ala Asp Gln Ala Ala Glu
260 265 270

Thr Asp Pro Ser Val Leu Trp Leu Ala Asp Ala Pro Val Val Asp Ile
275 280 285

Lys Asp Ser Tyr Ser His Pro Ser Ala Ala Glu Ile Ser Val Ser Lys
290 295 300

Pro Pro Pro Pro Pro Pro Pro Pro Gln Ile His Phe Glu Asn Gly Ser
305 310 315 320

Thr Ser Thr Leu Thr Glu Asn Pro Ser Pro Ser Val His Ala Pro Pro
325 330 335

Ala Pro Pro Ala Pro Pro Gln Arg Gln Gln Gln Asn Gln Gly Pro Phe
340 345 350

Arg Arg Glu Leu Asn Phe Ser Asp Phe Ala Ser Asn Pro Ser Leu Ala
355 360 365

Ala Ala Pro Pro Phe Phe Lys Pro Glu Ser Gly Glu Ile Leu Ser Phe
370 375 380

Gly Val Asp Ser Asn Ala Gln Arg Asn Pro Ser Pro Ala Pro Pro Ala
385 390 395 400

Ser Leu Thr Thr Ala Pro Gly Ser Leu Phe Ser Gln Ser Gln His Thr
405 410 415

Ala Thr Ala Ala Ala Asn Asp Ala Lys Asn Asn Asn Asn Asn Asn Lys
420 425 430

Arg Ser Met Glu Ala Thr Ser Leu Ala Ser Asn Thr Asn His His Pro
435 440 445

Ala Ala Ala Ala Asn Glu Gly Met Leu Ser Phe Ser Ser Ala Pro Thr
450 455 460

Ala Arg Pro Ser Ala Gly Thr Gly Ala Pro Ala Lys Ser Glu Ser Asp
465 470 475 480

His Ser Asp Leu Asp Ala Ser Val Arg Glu Val Glu Ser Ser Arg Val
485 490 495

Val Ala Pro Pro Pro Glu Ala Glu Lys Arg Pro Arg Lys Arg Gly Arg
500 505 510

Lys Pro Ala Asn Gly Arg Glu Glu Pro Leu Asn His Val Glu Ala Glu
515 520 525

Arg Gln Arg Arg Glu Lys Leu Asn Gln Arg Phe Tyr Ala Leu Arg Ala
530 535 540

Val Val Pro Asn Val Ser Lys Met Asp Lys Ala Ser Leu Leu Gly Asp
545 550 555 560

Ala Ile Ser Tyr Ile Asn Glu Leu Arg Gly Lys Leu Thr Ser Leu Glu
565 570 575

Ser Asp Arg Glu Thr Leu Gln Ala Gln Val Glu Ala Leu Lys Lys Glu
580 585 590

Arg Asp Ala Arg Pro His Pro His Pro Ala Ala Gly Leu Gly Gly His
595 600 605

Asp Ala Gly Gly Pro Arg Cys His Ala Val Glu Ile Asp Ala Lys Ile
610 615 620

Leu Gly Leu Glu Ala Met Ile Arg Val Gln Cys His Lys Arg Asn His
625 630 635 640

Pro Ser Ala Arg Leu Met Thr Ala Leu Arg Glu Leu Asp Leu Asp Val
645 650 655

Tyr His Ala Ser Val Ser Val Val Lys Asp Leu Met Ile Gln Gln Val
660 665 670

Ala Val Lys Met Ala Ser Arg Met Tyr Ser Gln Asp Gln Leu Ser Ala
675 680 685

Ala Leu Tyr Ser Arg Leu Ala Glu Pro Gly Ser Val Met Gly Arg
690 695 700

<210> 131
<211> 1074
<212> DNA
<213> Arabidopsis thaliana

<400> 131
atgacagaac tcaacttcca cctcctccca ataatctccg atcgcttcac gacgacgacg 60
acaacatcac cgtcgttctc gtcacattct tcttcttctt cttctcttct ctctttcacc 120
aaacgaagac gaaaacacca acctttagta tcatccatac gcatggaaca gtcacggtca 180
cggaatcgga aagacaaagt cgtcgtcatt ttaggagcaa ccggcgccgg aaaatcaaga 240
ctttccgtcg atctcgctac tcgtttccct tcagagatca taaactccga taaaatccaa 300
gtctacgaag gattagagat cacaacgaat cagattacgt tacaagaccg tcgcggcgtt 360
cctcaccatc tcctcggcgt catcaacccc gaacacggcg aactaaccgc cggagagttt 420
cgctccgccg cttcaaacgt cgtcaaagtg ataacttctc gtcaaaaggt tccgattatc 480
gccggtggat ctaactcttt cgtccacgca ctcttagctc aacgattcga cccaaagttc 540

gatcctttt catccgggtc gtgtttaatc agctccgatt tgcgttacga gtgttgtttc 600
atctgggtcg atgtatcgga gactgttctc tacgagtatc ttctcagaag agtcgacgaa 660
atgatggatt caggtatgtt cgaagagctg tctagattct acgacccggt taaatccggt 720
ttagaaaccc ggtttgggat taggaaagct ataggtgtac cggagtttga cggttacttc 780
aaagagtatc caccggagaa gaagatgata aagtgggacg ctttaagaaa agcggcgtac 840
gataaggcgg ttgatgatat caaaaggaac acgtggacgt tagcgaagag acaagtgaag 900
aagattgaga tgctaaaaga cgctggttgg gaaatagaaa gagttgatgc aacggcgtcg 960
tttaaagcag tgatgatgaa gagttcgtcg gagaagaagt ggagagagaa ttgggaagag 1020
caagtgttgg agccaagcgt aaagattgtg aagcggcatt tggtgcaaaa ttag 1074

<210> 132
<211> 357
<212> PRT
<213> Arabidopsis thaliana

<400> 132

Met Thr Glu Leu Asn Phe His Leu Leu Pro Ile Ile Ser Asp Arg Phe
1 5 10 15

Thr Thr Thr Thr Thr Thr Ser Pro Ser Phe Ser Ser His Ser Ser Ser
20 25 30

Ser Ser Ser Leu Leu Ser Phe Thr Lys Arg Arg Arg Lys His Gln Pro
35 40 45

Leu Val Ser Ser Ile Arg Met Glu Gln Ser Arg Ser Arg Asn Arg Lys
50 55 60

Asp Lys Val Val Val Ile Leu Gly Ala Thr Gly Ala Gly Lys Ser Arg
65 70 75 80

Leu Ser Val Asp Leu Ala Thr Arg Phe Pro Ser Glu Ile Ile Asn Ser
85 90 95

Asp Lys Ile Gln Val Tyr Glu Gly Leu Glu Ile Thr Thr Asn Gln Ile
100 105 110

Thr Leu Gln Asp Arg Arg Gly Val Pro His His Leu Leu Gly Val Ile
115 120 125

Asn Pro Glu His Gly Glu Leu Thr Ala Gly Glu Phe Arg Ser Ala Ala
130 135 140

Ser Asn Val Val Lys Val Ile Thr Ser Arg Gln Lys Val Pro Ile Ile
145 150 155 160

Ala Gly Gly Ser Asn Ser Phe Val His Ala Leu Leu Ala Gln Arg Phe
165 170 175

Asp Pro Lys Phe Asp Pro Phe Ser Ser Gly Ser Cys Leu Ile Ser Ser
180 185 190

Asp Leu Arg Tyr Glu Cys Cys Phe Ile Trp Val Asp Val Ser Glu Thr
195 200 205

Val Leu Tyr Glu Tyr Leu Leu Arg Arg Val Asp Glu Met Met Asp Ser
210 215 220

Gly Met Phe Glu Glu Leu Ser Arg Phe Tyr Asp Pro Val Lys Ser Gly
225 230 235 240

Leu Glu Thr Arg Phe Gly Ile Arg Lys Ala Ile Gly Val Pro Glu Phe
245 250 255

Asp Gly Tyr Phe Lys Glu Tyr Pro Pro Glu Lys Lys Met Ile Lys Trp
260 265 270

Asp Ala Leu Arg Lys Ala Ala Tyr Asp Lys Ala Val Asp Asp Ile Lys
275 280 285

Arg Asn Thr Trp Thr Leu Ala Lys Arg Gln Val Lys Lys Ile Glu Met
290 295 300

Leu Lys Asp Ala Gly Trp Glu Ile Glu Arg Val Asp Ala Thr Ala Ser
305 310 315 320

Phe Lys Ala Val Met Met Lys Ser Ser Ser Glu Lys Lys Trp Arg Glu
325 330 335

Asn Trp Glu Glu Gln Val Leu Glu Pro Ser Val Lys Ile Val Lys Arg
340 345 350

His Leu Val Gln Asn
355

<210> 133
<211> 654
<212> DNA
<213> Oryza sativa

<400> 133
cttctacatc ggcttaggtg tagcaacacg actttattat tattattatt attattatta 60

ttattttaca aaaatataaa atagatcagt ccctcaccac aagtagagca agttggtgag 120

ttattgtaaa gttctacaaa gctaatttaa aagttattgc attaacttat ttcatattac 180

aaacaagagt gtcaatggaa caatgaaaac catatgacat actataattt tgtttttatt 240

attgaaatta tataattcaa agagaataaa tccacatagc cgtaaagttc tacatgtggt 300

gcattaccaa aatatatata gcttacaaaa catgacaagc ttagtttgaa aaattgcaat 360

ccttatcaca ttgacacata aagtgagtga tgagtcataa tattattttt cttgctaccc 420

atcatgtata tatgatagcc acaaagttac tttgatgatg atatcaaaga acatttttag 480

gtgcacctaa cagaatatcc aaataatatg actcacttag atcataatag agcatcaagt 540

aaaactaaca ctctaaagca accgatggga aagcatctat aaatagacaa gcacaatgaa 600

aatcctcatc atccttcacc acaattcaaa tattatagtt gaagcatagt agta 654

<210> 134
<211> 55
<212> DNA
<213> Artificial sequence

<220>
<223> primer: prm03095

<400> 134
ggggacaagt ttgtacaaaa aagcaggctt cacaatgaca gaactcaact tccac 55

<210> 135
<211> 49
<212> DNA
<213> Artificial sequence

<220>
<223> primer: prm03096

<400> 135
ggggaccact ttgtacaaga aagctgggta actaattttg caccaaatg 49

<210> 136
<211> 957
<212> DNA
<213> Arabidopsis thaliana

<400> 136
atgaagtgta atgacaaaat ggttgtgatc atgggtgcca ccggttctgg caagtcatca 60

ctctctgttg atctcgcttt acattttaaa gccgagatca tcaactctga caaaatgcag 120

ttctacgatg gcttgaagat caccacgaat caatcgacca ttgaagatcg acgtggagtg 180

ccacatcacc ttctcggtga actaaacccg gaggctggag aagtcacagc ggcagaattt 240

cgcgttatgg cggctgaagc catctccgag attactcaac gtaaaaagct cccaatcctt 300

gccggtggat ccaactcata cattcatgct ctccttgcaa aatcttatga ccctgaaaac 360

tatccgtttt ctgatcacaa gggctcaatc tgctccgagt tgaaatatga ttgttgtttc 420

atttggatag atgtggatca gtctgtgtta ttcgagtatc tttctttacg tttggatctt 480

atgatgaagt caggtatgtt cgaggagatc gctgagttcc accgctctaa gaaggccccg 540

aaagagccat tggggatatg gaaggctata ggagtgcaag agtttgatga ctacctcaaa 600

atgtacaagt gggacaatga catggataaa tgggacccta tgagaaagga ggcttatgag 660

aaggcggtga gagccatcaa agaaaacaca tttcagctca caaaggatca aatcacgaag 720

atcaacaagc tgagaaatgc cgggtgggac ataaagaagg tggatgctac agcatcgttt 780

cgagaggcaa ttagggcagc caaggaaggc gaaggtgtag ccgagatgca gagaaagata 840

tggaacaagg aagtgttgga accgtgtgtg aagattgtca ggagccactt ggaccaaccg 900

atcaactatt attattatta cttttattta ctaaaaagat ttttaagtct taactag 957

<210> 137
<211> 318
<212> PRT
<213> Arabidopsis thaliana

<400> 137

Met Lys Cys Asn Asp Lys Met Val Val Ile Met Gly Ala Thr Gly Ser
1 5 10 15

Gly Lys Ser Ser Leu Ser Val Asp Leu Ala Leu His Phe Lys Ala Glu
20 25 30

Ile Ile Asn Ser Asp Lys Met Gln Phe Tyr Asp Gly Leu Lys Ile Thr
35 40 45

Thr Asn Gln Ser Thr Ile Glu Asp Arg Arg Gly Val Pro His His Leu
50 55 60

Leu Gly Glu Leu Asn Pro Glu Ala Gly Glu Val Thr Ala Ala Glu Phe
65 70 75 80

Arg Val Met Ala Ala Glu Ala Ile Ser Glu Ile Thr Gln Arg Lys Lys
85 90 95

Leu Pro Ile Leu Ala Gly Gly Ser Asn Ser Tyr Ile His Ala Leu Leu
100 105 110

Ala Lys Ser Tyr Asp Pro Glu Asn Tyr Pro Phe Ser Asp His Lys Gly
115 120 125

Ser Ile Cys Ser Glu Leu Lys Tyr Asp Cys Cys Phe Ile Trp Ile Asp
130 135 140

Val Asp Gln Ser Val Leu Phe Glu Tyr Leu Ser Leu Arg Leu Asp Leu
145 150 155 160

Met Met Lys Ser Gly Met Phe Glu Glu Ile Ala Glu Phe His Arg Ser
165 170 175

Lys Lys Ala Pro Lys Glu Pro Leu Gly Ile Trp Lys Ala Ile Gly Val
180 185 190

Gln Glu Phe Asp Asp Tyr Leu Lys Met Tyr Lys Trp Asp Asn Asp Met
195 200 205

Asp Lys Trp Asp Pro Met Arg Lys Glu Ala Tyr Glu Lys Ala Val Arg
210 215 220

Ala Ile Lys Glu Asn Thr Phe Gln Leu Thr Lys Asp Gln Ile Thr Lys

225 230 235 240

Ile Asn Lys Leu Arg Asn Ala Gly Trp Asp Ile Lys Lys Val Asp Ala
245 250 255

Thr Ala Ser Phe Arg Glu Ala Ile Arg Ala Ala Lys Glu Gly Glu Gly
260 265 270

Val Ala Glu Met Gln Arg Lys Ile Trp Asn Lys Glu Val Leu Glu Pro
275 280 285

Cys Val Lys Ile Val Arg Ser His Leu Asp Gln Pro Ile Asn Tyr Tyr
290 295 300

Tyr Tyr Tyr Phe Tyr Leu Leu Lys Arg Phe Leu Ser Leu Asn
305 310 315

<210> 138
<211> 1029
<212> DNA
<213> Arabidopsis thaliana

<400> 138
atgcaacaac tcatgacctt gttatcacca ccactctctc attcttctct ccttcccacc 60
gtcactacca aattcgggtc accacgatta gtcactacgt gcatgggcca tgcagggcgt 120
aaaaatatca aggataaggt ggttctcatc acaggtacaa caggcacagg caagtcacgc 180
ctctcagtcg atcttgccac ccgttttttt cccgccgaga tcataaactc ggacaaaatg 240
caaatctaca agggattcga gattgtcaca aatctaatcc cactgcatga gcaaggagga 300
gtcccgcacc atcttctagg tcagttccac ccacaagacg gtgaactcac ccctgcagag 360
ttccgttctt tggcgacact gtccatctct aaactaattt ctagcaagaa actcccgatt 420
gtagttggtg gatccaactc cttcaatcac gctctactcg ccgagcgttt tgacccggat 480
attgatccat tctctcccgg atcgagtctt tcaacgatct gctctgacct aaggtacaaa 540
tgttgcatct tatgggttga tgttttagag ccggttctgt tccaacactt gtgcaatcgt 600
gtcgaccaaa tgatcgagtc gggattggtc gagcagcttg ccgaattgta cgaccctgtt 660
gtagattcgg gtcgacgact aggggttcgg aagacgatag gagtagagga gttcgaccga 720
tactttagag tataccctaa ggagatggac aagggaattt gggacttagc gagaaaggcg 780
gcgtacgagg agacagtgaa ggggatgaaa gagaggacat gtcggttggt gaagaagcag 840
aaagagaaga tcatgaagct gataagaggt ggttgggaga ttaagaggct tgacgctacg 900
gcggcaatta tggctgagct gaatcaaagt acggcaaagg gagaaggaaa gaatgggaga 960
gagatttggg aaaaacacat tgtggatgaa agtgtcgaga ttgtcaagaa gtttttgttg 1020
gaagtttag 1029

<210> 139
<211> 342

<212> PRT
<213> Arabidopsis thaliana

<400> 139

Met Gln Gln Leu Met Thr Leu Leu Ser Pro Pro Leu Ser His Ser Ser
1               5                   10                  15

Leu Leu Pro Thr Val Thr Thr Lys Phe Gly Ser Pro Arg Leu Val Thr
            20                  25                  30

Thr Cys Met Gly His Ala Gly Arg Lys Asn Ile Lys Asp Lys Val Val
        35                  40                  45

Leu Ile Thr Gly Thr Thr Gly Thr Gly Lys Ser Arg Leu Ser Val Asp
    50                  55                  60

Leu Ala Thr Arg Phe Phe Pro Ala Glu Ile Ile Asn Ser Asp Lys Met
65                  70                  75                  80

Gln Ile Tyr Lys Gly Phe Glu Ile Val Thr Asn Leu Ile Pro Leu His
                85                  90                  95

Glu Gln Gly Gly Val Pro His His Leu Leu Gly Gln Phe His Pro Gln
            100                 105                 110

Asp Gly Glu Leu Thr Pro Ala Glu Phe Arg Ser Leu Ala Thr Leu Ser
        115                 120                 125

Ile Ser Lys Leu Ile Ser Ser Lys Lys Leu Pro Ile Val Val Gly Gly
    130                 135                 140

Ser Asn Ser Phe Asn His Ala Leu Leu Ala Glu Arg Phe Asp Pro Asp
145                 150                 155                 160

Ile Asp Pro Phe Ser Pro Gly Ser Ser Leu Ser Thr Ile Cys Ser Asp
                165                 170                 175

Leu Arg Tyr Lys Cys Cys Ile Leu Trp Val Asp Val Leu Glu Pro Val
            180                 185                 190

Leu Phe Gln His Leu Cys Asn Arg Val Asp Gln Met Ile Glu Ser Gly
        195                 200                 205

Leu Val Glu Gln Leu Ala Glu Leu Tyr Asp Pro Val Val Asp Ser Gly
    210                 215                 220

Arg Arg Leu Gly Val Arg Lys Thr Ile Gly Val Glu Glu Phe Asp Arg
225                 230                 235                 240

Tyr Phe Arg Val Tyr Pro Lys Glu Met Asp Lys Gly Ile Trp Asp Leu
                245                 250                 255

```
Ala Arg Lys Ala Ala Tyr Glu Glu Thr Val Lys Gly Met Lys Glu Arg
            260                 265                 270

Thr Cys Arg Leu Val Lys Lys Gln Lys Glu Lys Ile Met Lys Leu Ile
        275                 280                 285

Arg Gly Gly Trp Glu Ile Lys Arg Leu Asp Ala Thr Ala Ala Ile Met
    290                 295                 300

Ala Glu Leu Asn Gln Ser Thr Ala Lys Gly Glu Gly Lys Asn Gly Arg
305                 310                 315                 320

Glu Ile Trp Glu Lys His Ile Val Asp Glu Ser Val Glu Ile Val Lys
                325                 330                 335

Lys Phe Leu Leu Glu Val
            340
```

<210> 140
<211> 1137
<212> DNA
<213> Humulus lupulus

<400> 140

```
agccggaaga agaccagggt gggcacgcat ggactacgca tccgttgcca tggctgccgc     60
gcccaccaca acaaccacta ccaacgtatc actccgtcgc cagcgacacc ggaaagagaa    120
gcttctcgtt ctaatgggcg ccaccggtac cgggaagtcc cgtctctcca tcgacctcgc    180
ggcgcacttc cctctcgaag tcataaactc ggataaaatg caggtttata aaggactgga    240
tatcacgacg aacaagatat cggtaccgga ccggggcggc gtgccacacc atctcctcgg    300
tgaggttgac ccggctcgag gtgagttgac tcctgccgat ttcaggtctt tggctggaaa    360
agccgtgtct gaaatcactg ggaggagaaa gcttcccgtc ctggtgggtg ggtccaactc    420
gttcatacat gcgctgttgg tggaccggtt tgactcgtcg ggcccgggcg tgttcgagga    480
agggtcccac tcggtggtgt cttctgagtt gagatacgac tgttgctttc tctgggttga    540
cgtgtcggta aaggtattga ccgattactt ggcgaagcga gtcgacgaca tgttggagct    600
ggggatgttc gatgagttgg ccgagttcta tagcccggag gacgaggacc acgacgaaga    660
ctctgcgact cggaccgggt tgagaaaagc catcggagtt cccgagtttg accggtattt    720
cgaaaagttc agacctggcg acgtggaggg ggaggacccc gggagggatc gggtgcggag    780
gggcgcattc gaagaggcgg tgagggcgat caaggagaac acgtgtcatc tagcgaagag    840
acaaataggg aagatcttac gattaaaagg cgctgggtgg gacctacggc ggctggacgc    900
aacagagtcg ttccgggcgg cgatgacgtc agactccggc gagaagtgca cggagatttg    960
ggagaagcag gtattggaac caagcgtgaa gattgtgagt cgcttcttgg acgagtaggt   1020
tttgccagca atttccagct catttttttct tttattttt tgagtttgtt aaaaaaaaat   1080
tttggtagtt agttttccca cggaaaaatt ttcaacttgt cacaattaca aattaat      1137
```

<210> 141
<211> 329
<212> PRT
<213> Humulus lupulus

<400> 141

Met Asp Tyr Ala Ser Val Ala Met Ala Ala Ala Pro Thr Thr Thr Thr
1               5                   10                  15

Thr Thr Asn Val Ser Leu Arg Arg Gln Arg His Arg Lys Glu Lys Leu
            20                  25                  30

Leu Val Leu Met Gly Ala Thr Gly Thr Gly Lys Ser Arg Leu Ser Ile
        35                  40                  45

Asp Leu Ala Ala His Phe Pro Leu Glu Val Ile Asn Ser Asp Lys Met
    50                  55                  60

Gln Val Tyr Lys Gly Leu Asp Ile Thr Thr Asn Lys Ile Ser Val Pro
65                  70                  75                  80

Asp Arg Gly Gly Val Pro His His Leu Leu Gly Glu Val Asp Pro Ala
                85                  90                  95

Arg Gly Glu Leu Thr Pro Ala Asp Phe Arg Ser Leu Ala Gly Lys Ala
            100                 105                 110

Val Ser Glu Ile Thr Gly Arg Arg Lys Leu Pro Val Leu Val Gly Gly
        115                 120                 125

Ser Asn Ser Phe Ile His Ala Leu Leu Val Asp Arg Phe Asp Ser Ser
    130                 135                 140

Gly Pro Gly Val Phe Glu Glu Gly Ser His Ser Val Val Ser Ser Glu
145                 150                 155                 160

Leu Arg Tyr Asp Cys Cys Phe Leu Trp Val Asp Val Ser Val Lys Val
                165                 170                 175

Leu Thr Asp Tyr Leu Ala Lys Arg Val Asp Asp Met Leu Glu Leu Gly
            180                 185                 190

Met Phe Asp Glu Leu Ala Glu Phe Tyr Ser Pro Glu Asp Glu Asp His
        195                 200                 205

Asp Glu Asp Ser Ala Thr Arg Thr Gly Leu Arg Lys Ala Ile Gly Val
    210                 215                 220

Pro Glu Phe Asp Arg Tyr Phe Glu Lys Phe Arg Pro Gly Asp Val Glu
225                 230                 235                 240

Gly Glu Asp Pro Gly Arg Asp Arg Val Arg Arg Gly Ala Phe Glu Glu
                245                 250                 255

```
Ala Val Arg Ala Ile Lys Glu Asn Thr Cys His Leu Ala Lys Arg Gln
            260                 265                 270

Ile Gly Lys Ile Leu Arg Leu Lys Gly Ala Gly Trp Asp Leu Arg Arg
        275                 280                 285

Leu Asp Ala Thr Glu Ser Phe Arg Ala Ala Met Thr Ser Asp Ser Gly
    290                 295                 300

Glu Lys Cys Thr Glu Ile Trp Glu Lys Gln Val Leu Glu Pro Ser Val
305                 310                 315                 320

Lys Ile Val Ser Arg Phe Leu Asp Glu
                325


<210>  142
<211>  1335
<212>  DNA
<213>  Lotus japonicus

<400>  142
ctctctttct tcttcttcct atgagacttt cctctctctc acctcacccc caccaccacc      60

accactacac cacacactac cattaccatt accaccaccc ctcttcactc gccatggacg     120

gccaccgccg catagacaag gtggttgtca tcatgggcgc caccggctcc ggcaaatccc     180

gcctctccat cgacctcgcc accctctttc ccttctcaga gatcatcaac tccgataaaa     240

tgcaagtcta caaaggactc gacaccacca ccaacaagat cccacctcac caacgcaaca     300

acgtccccca ccacctcctc ggcgacgtcg acccttctct cggtgatttc accccctccg     360

acttccgccg ccgcgccggt gacctcatct ccgacatcac ccgccgcaga aagctcccca     420

tcttcgtcgg cggctccaac tccttcgtcc acgccctcct tgtcgaccga ttcgaccccg     480

agtccaacgt cttccgcgac gattcaccct cgccggtttc ctccgagttg agataccggt     540

gctgcttcct ctggatggac attgcgttcc ccgtgctgtc ggagtatttg ctgaagcgag     600

tcgacgacat gcttgactcg ggaatggtgg acgagttggc tcagttcttc gactcggaca     660

cagcgaacca gaccgggttg agaaaagcca tcggtgtacc cgagttcgac cggtttttca     720

aggatccggt gcgggagggt gcagcgtacg aggaagcggt gagggcgatt aaggagaaca     780

cgtgtcagct agcgaagagg cagattggga agatcatgcg gttaaaaaga gcagggtggg     840

acttacggag gatcgacgcc acggaagcgt tccgggtggc gcttgtggca gacggcggcg     900

gagagagatt ttccgatgaa tggaagaggc aagtgttgga accaagcgtg aagattgtga     960

agcggttctt gatggagtag gtttgggttt tccagctaaa tccagctatt ccacttcctc    1020

tttaaatttt tctttttttt ttcttcttct ctttttttgc gtttatttca cccccaaata    1080

agacaaaaaa tgaaagtggg aaattggggg gaaaatgtac aaaaatggga gtggaaattg    1140

ttaatttgaa tccactagtt tggttgagtg agtgatttca gagagggaat gagcatggat    1200
```

cattaaattt ttggtcttgg agttactagc aactagcaac tagcaactag caacaaggct 1260

tggctcatgt gagattccac aatttttctc aaggcaatag aaatggatat ttagtataaa 1320

aaaaaaaaaa aaaaa 1335

<210> 143
<211> 319
<212> PRT
<213> Lotus japonicus

<400> 143

Met Arg Leu Ser Ser Leu Ser Pro His Pro His His His His His Tyr
1 5 10 15

Thr Thr His Tyr His Tyr His Tyr His His Pro Ser Ser Leu Ala Met
20 25 30

Asp Gly His Arg Arg Ile Asp Lys Val Val Val Ile Met Gly Ala Thr
35 40 45

Gly Ser Gly Lys Ser Arg Leu Ser Ile Asp Leu Ala Thr Leu Phe Pro
50 55 60

Phe Ser Glu Ile Ile Asn Ser Asp Lys Met Gln Val Tyr Lys Gly Leu
65 70 75 80

Asp Thr Thr Thr Asn Lys Ile Pro Pro His Gln Arg Asn Asn Val Pro
85 90 95

His His Leu Leu Gly Asp Val Asp Pro Ser Leu Gly Asp Phe Thr Pro
100 105 110

Ser Asp Phe Arg Arg Arg Ala Gly Asp Leu Ile Ser Asp Ile Thr Arg
115 120 125

Arg Arg Lys Leu Pro Ile Phe Val Gly Gly Ser Asn Ser Phe Val His
130 135 140

Ala Leu Leu Val Asp Arg Phe Asp Pro Glu Ser Asn Val Phe Arg Asp
145 150 155 160

Asp Ser Pro Ser Pro Val Ser Ser Glu Leu Arg Tyr Arg Cys Cys Phe
165 170 175

Leu Trp Met Asp Ile Ala Phe Pro Val Leu Ser Glu Tyr Leu Leu Lys
180 185 190

Arg Val Asp Asp Met Leu Asp Ser Gly Met Val Asp Glu Leu Ala Gln
195 200 205

Phe Phe Asp Ser Asp Thr Ala Asn Gln Thr Gly Leu Arg Lys Ala Ile
210 215 220

Gly Val Pro Glu Phe Asp Arg Phe Phe Lys Asp Pro Val Arg Glu Gly
225 230 235 240

Ala Ala Tyr Glu Glu Ala Val Arg Ala Ile Lys Glu Asn Thr Cys Gln
245 250 255

Leu Ala Lys Arg Gln Ile Gly Lys Ile Met Arg Leu Lys Arg Ala Gly
260 265 270

Trp Asp Leu Arg Arg Ile Asp Ala Thr Glu Ala Phe Arg Val Ala Leu
275 280 285

Val Ala Asp Gly Gly Gly Glu Arg Phe Ser Asp Glu Trp Lys Arg Gln
290 295 300

Val Leu Glu Pro Ser Val Lys Ile Val Lys Arg Phe Leu Met Glu
305 310 315

<210> 144
<211> 1120
<212> DNA
<213> Medicago truncatula

<400> 144
atgccaacaa caacaacaac accttcattt ctctcacctc ctcactctca acgtcattat 60
cataaaccca ttcaatttca ccactattca cacacccttt ttactccttc ctcttcctcc 120
gccacccaca ccaggcggcg gccccactgc cctcgcatgg aaatctcccc ctcacgccac 180
cgtcggaaag acaaagtcct cattatagtc ggtgcaaccg gctccggaaa atcacgtctc 240
tccgttgaac tcgccaccct cttcccttac tcagaaataa ttaattccga caaaatccaa 300
gtgtacagag gactcgatat caccaccaac aagatcccat ttcatcaacg caacaacgtt 360
ccacatcatc ttctcggcga cattgatcct tctcacggtg agttctcacc ttcagatttc 420
cgccgccacg ccggagatat catctccgat attacttccc ggagaaaact tcccattatc 480
gttggtgggt ccaactcatt cattcacgct cttcttgtag aacgattcga ccctgagtca 540
aacgttttcg acgagtcatc atcattgtca acatcgatat cctcggattt aaggtacaaa 600
tgttgttttc tttggatgga tatttcgttt cctgtgttgt cggaatattt gctgaaacga 660
gtcgatgata tgtttgactc gggaatggtg aacgagttag ctgagtttta tgaaccggat 720
gcagataacc aaaccggttt aagaaaagca atcggtgtac ccgagttcga ccggtttttt 780
aaacaatatc caccacaggt tggaccagat gaatctgaac gtcataatcc aatgcgggaa 840
ggtgcatata ttgaagcagt gaaggcgatt aaagataaca cgtgtcagtt agctaagaga 900
cagataggga agatcttacg gttaaaaaga gctgggtggg acctacaacg gattgatgcc 960
acggaggcgt ttagggcggt gctgacgtca gagtctaacg gcggtggaga agaatttacc 1020
ggtgtatgga aaaaacaagt attggaacca agcgtgaaga ttgtgaagcg tttcttgatg 1080
gagtaggtct tccagctaaa tccagcttag ctaatccaac 1120

<210> 145
<211> 361
<212> PRT
<213> Medicago truncatula

<400> 145

Met Pro Thr Thr Thr Thr Thr Pro Ser Phe Leu Ser Pro Pro His Ser
1 5 10 15

Gln Arg His Tyr His Lys Pro Ile Gln Phe His His Tyr Ser His Thr
20 25 30

Leu Phe Thr Pro Ser Ser Ser Ser Ala Thr His Thr Arg Arg Arg Pro
35 40 45

His Cys Pro Arg Met Glu Ile Ser Pro Ser Arg His Arg Arg Lys Asp
50 55 60

Lys Val Leu Ile Ile Val Gly Ala Thr Gly Ser Gly Lys Ser Arg Leu
65 70 75 80

Ser Val Glu Leu Ala Thr Leu Phe Pro Tyr Ser Glu Ile Ile Asn Ser
85 90 95

Asp Lys Ile Gln Val Tyr Arg Gly Leu Asp Ile Thr Thr Asn Lys Ile
100 105 110

Pro Phe His Gln Arg Asn Asn Val Pro His His Leu Leu Gly Asp Ile
115 120 125

Asp Pro Ser His Gly Glu Phe Ser Pro Ser Asp Phe Arg Arg His Ala
130 135 140

Gly Asp Ile Ile Ser Asp Ile Thr Ser Arg Arg Lys Leu Pro Ile Ile
145 150 155 160

Val Gly Gly Ser Asn Ser Phe Ile His Ala Leu Leu Val Glu Arg Phe
165 170 175

Asp Pro Glu Ser Asn Val Phe Asp Glu Ser Ser Ser Leu Ser Thr Ser
180 185 190

Ile Ser Ser Asp Leu Arg Tyr Lys Cys Cys Phe Leu Trp Met Asp Ile
195 200 205

Ser Phe Pro Val Leu Ser Glu Tyr Leu Leu Lys Arg Val Asp Asp Met
210 215 220

Phe Asp Ser Gly Met Val Asn Glu Leu Ala Glu Phe Tyr Glu Pro Asp
225 230 235 240

Ala Asp Asn Gln Thr Gly Leu Arg Lys Ala Ile Gly Val Pro Glu Phe
245 250 255

Asp Arg Phe Phe Lys Gln Tyr Pro Pro Gln Val Gly Pro Asp Glu Ser
260 265 270

Glu Arg His Asn Pro Met Arg Glu Gly Ala Tyr Ile Glu Ala Val Lys
275 280 285

Ala Ile Lys Asp Asn Thr Cys Gln Leu Ala Lys Arg Gln Ile Gly Lys
290 295 300

Ile Leu Arg Leu Lys Arg Ala Gly Trp Asp Leu Gln Arg Ile Asp Ala
305 310 315 320

Thr Glu Ala Phe Arg Ala Val Leu Thr Ser Glu Ser Asn Gly Gly Gly
325 330 335

Glu Glu Phe Thr Gly Val Trp Lys Lys Gln Val Leu Glu Pro Ser Val
340 345 350

Lys Ile Val Lys Arg Phe Leu Met Glu
355 360

<210> 146
<211> 1105
<212> DNA
<213> Morus alba

<400> 146
gcccgggcag gtccggccac tacctctggc ccacgagaca ggtgggcgcg gatggagttt 60
tcctcctccg ccgcccgccg ccaccgccac catcccaagg acaagctcct cgtcatcatg 120
ggcgccaccg gcaccggcaa atcccgactc tccgtcgagc tcgccaccca cttcaacggc 180
gagatcatca actccgacaa aatgcaagtc tacaagggac tcgacacaac gaccaacaag 240
attcccctcg acgaccgcct cggtgtcccc caccaccttc tcggcgaggt cgactcggaa 300
gctcacggcg agttgactcg ctccgagttc cgctccttgg ccggaaaggc cgtgtcggaa 360
atcaccgcca ggaggaaact cccagtcctc gccggcggct ccaactcctt cattcacgcc 420
ctgctcgtgg accggttcga cccggaatac gacgttttcg acgagcggtc cgaccaaccg 480
gcggattcgt cgaaggttct cctccgctac aactgttgct tcctttgggt ggatgtttcg 540
ttgagggttt tggaagatta tttattgaag cgagtcgatg acatgctcaa ctcggggatg 600
ttcgacgagt tggccgagtt ctacgacccg gaggaggatc acggaccggc gaactggacc 660
ggattgagga aggctatagg cgtgcccgag tttgaccggt atttcgagag gtgcagaccg 720
ggggagaaag gagagtggga tcgggtgcgg agggaagcat acgaggaggc ggtgagggaa 780
ataaaggaga acacgtgtca gctggcgaag agacagattg gaaagattct gagattaaag 840
aagttggggt gggacctacg gaggttggac gcgacggagg cgtttagggc ggtgatgacg 900

tcagattccg gtaagaggtg ttcggaaatt tgggagaggc aggtattgga accaagcgtg 960
aagattgtga agcgcttctt ggacgagtag taggttttgc cagcactttc cagctaattt 1020
ttatttttat tatttttttg ttgttttttt aaaaaaattt tggtagttag ttttccaact 1080
tcaacttcag ctcaaaaaaa aaaaa 1105

<210> 147
<211> 312
<212> PRT
<213> Morus alba

<400> 147

Met Glu Phe Ser Ser Ser Ala Ala Arg Arg His Arg His His Pro Lys
1 5 10 15

Asp Lys Leu Leu Val Ile Met Gly Ala Thr Gly Thr Gly Lys Ser Arg
20 25 30

Leu Ser Val Glu Leu Ala Thr His Phe Asn Gly Glu Ile Ile Asn Ser
35 40 45

Asp Lys Met Gln Val Tyr Lys Gly Leu Asp Thr Thr Thr Asn Lys Ile
50 55 60

Pro Leu Asp Asp Arg Leu Gly Val Pro His His Leu Leu Gly Glu Val
65 70 75 80

Asp Ser Glu Ala His Gly Glu Leu Thr Arg Ser Glu Phe Arg Ser Leu
85 90 95

Ala Gly Lys Ala Val Ser Glu Ile Thr Ala Arg Arg Lys Leu Pro Val
100 105 110

Leu Ala Gly Gly Ser Asn Ser Phe Ile His Ala Leu Leu Val Asp Arg
115 120 125

Phe Asp Pro Glu Tyr Asp Val Phe Asp Glu Arg Ser Asp Gln Pro Ala
130 135 140

Asp Ser Ser Lys Val Leu Leu Arg Tyr Asn Cys Cys Phe Leu Trp Val
145 150 155 160

Asp Val Ser Leu Arg Val Leu Glu Asp Tyr Leu Leu Lys Arg Val Asp
165 170 175

Asp Met Leu Asn Ser Gly Met Phe Asp Glu Leu Ala Glu Phe Tyr Asp
180 185 190

Pro Glu Glu Asp His Gly Pro Ala Asn Trp Thr Gly Leu Arg Lys Ala
195 200 205

Ile Gly Val Pro Glu Phe Asp Arg Tyr Phe Glu Arg Cys Arg Pro Gly

```
    210                 215                 220

Glu Lys Gly Glu Trp Asp Arg Val Arg Arg Glu Ala Tyr Glu Glu Ala
225                 230                 235                 240

Val Arg Glu Ile Lys Glu Asn Thr Cys Gln Leu Ala Lys Arg Gln Ile
                245                 250                 255

Gly Lys Ile Leu Arg Leu Lys Lys Leu Gly Trp Asp Leu Arg Arg Leu
            260                 265                 270

Asp Ala Thr Glu Ala Phe Arg Ala Val Met Thr Ser Asp Ser Gly Lys
        275                 280                 285

Arg Cys Ser Glu Ile Trp Glu Arg Gln Val Leu Glu Pro Ser Val Lys
    290                 295                 300

Ile Val Lys Arg Phe Leu Asp Glu
305                 310

<210>  148
<211>  1083
<212>  DNA
<213>  Oryza sativa

<400>  148
atgaccagcg ttgccaccag gattgccacg ctcgtgcggg ccgcggcggc ggcgagccgg      60
ccattgcggc tccaccgccg gcccggcggc gaggatacga ggatggtggt gatcgtcggc     120
gccacgggca ccgggaagac caagctgtcc atcgacgccg ccaaggtgat cggcggcgag     180
gttgtcaacg ccgacaagat tcagctctat gacggcctcg acgtgaccac caacaaggtg     240
agcctcgccg accgccgtgg cgtgccgcac cacctcctcg gagccatccg ccccgaggcc     300
ggcgagctcc cgccgtcgtc cttccggtcc ctcgccgccg ccacggccgc gtcgatcgcg     360
gcgaggcggc tcgtgccggt catcgccggt gggtcgaact ccctcatcca cgccctcctc     420
gccgaccact tcgacgcctc cgctggcgat cccttctccc ccgccgccgc cttccgccac     480
taccgcccgg cgctccggtt cccgtgctgc ctgctctggg tccacgtcga tgaggcgctc     540
ctcgacgagt acctcgaccg ccgcgtggac gacatggtgg acgctggcat ggtcgaggag     600
ctccgggagt acttcgccac gacaaccgcc gcggagcgcg ccgcgcactc cgggctgggc     660
aaggccatcg gcgtccccga gctcggcgac tacttcgccg ggcgcaagac cttctccgag     720
gcgatcgacg acatcaaagc caacacccgc gtcctcgccg ccgcgcaggt gtccaagatc     780
cgccgcatgt ccgacgcctg ggctggccc atccaccgcc tcgacgcctc cgacacagtc     840
cgcgccaggc tcacgcgggc gggctccgcc gccgagtccg cctcctggga gcgcgacgtg     900
cgcggcccag gcctcgccac catccgcagc ttcctcgccg atcagtcacc gccaccgcgc     960
agcgagggca ccaacgacta cctgtacgcc atggagacgg aaccagagcc gccgccgccg    1020
ccgacgttgc cgccgcggct gctccggttg ccgcggatgc agtactgcga catggtgggg    1080
```

tga 1083

<210> 149
<211> 360
<212> PRT
<213> Oryza sativa

<400> 149

Met Thr Ser Val Ala Thr Arg Ile Ala Thr Leu Val Arg Ala Ala Ala
1 5 10 15

Ala Ala Ser Arg Pro Leu Arg Leu His Arg Arg Pro Gly Gly Glu Asp
20 25 30

Thr Arg Met Val Val Ile Val Gly Ala Thr Gly Thr Gly Lys Thr Lys
35 40 45

Leu Ser Ile Asp Ala Ala Lys Val Ile Gly Gly Glu Val Val Asn Ala
50 55 60

Asp Lys Ile Gln Leu Tyr Asp Gly Leu Asp Val Thr Thr Asn Lys Val
65 70 75 80

Ser Leu Ala Asp Arg Arg Gly Val Pro His His Leu Leu Gly Ala Ile
85 90 95

Arg Pro Glu Ala Gly Glu Leu Pro Pro Ser Ser Phe Arg Ser Leu Ala
100 105 110

Ala Ala Thr Ala Ala Ser Ile Ala Ala Arg Arg Leu Val Pro Val Ile
115 120 125

Ala Gly Gly Ser Asn Ser Leu Ile His Ala Leu Leu Ala Asp His Phe
130 135 140

Asp Ala Ser Ala Gly Asp Pro Phe Ser Pro Ala Ala Ala Phe Arg His
145 150 155 160

Tyr Arg Pro Ala Leu Arg Phe Pro Cys Cys Leu Leu Trp Val His Val
165 170 175

Asp Glu Ala Leu Leu Asp Glu Tyr Leu Asp Arg Arg Val Asp Asp Met
180 185 190

Val Asp Ala Gly Met Val Glu Glu Leu Arg Glu Tyr Phe Ala Thr Thr
195 200 205

Thr Ala Ala Glu Arg Ala Ala His Ser Gly Leu Gly Lys Ala Ile Gly
210 215 220

Val Pro Glu Leu Gly Asp Tyr Phe Ala Gly Arg Lys Thr Phe Ser Glu
225 230 235 240

```
Ala Ile Asp Asp Ile Lys Ala Asn Thr Arg Val Leu Ala Ala Ala Gln
                245                 250                 255

Val Ser Lys Ile Arg Arg Met Ser Asp Ala Trp Gly Trp Pro Ile His
            260                 265                 270

Arg Leu Asp Ala Ser Asp Thr Val Arg Ala Arg Leu Thr Arg Ala Gly
        275                 280                 285

Ser Ala Ala Glu Ser Ala Ser Trp Glu Arg Asp Val Arg Gly Pro Gly
    290                 295                 300

Leu Ala Thr Ile Arg Ser Phe Leu Ala Asp Gln Ser Pro Pro Pro Arg
305                 310                 315                 320

Ser Glu Gly Thr Asn Asp Tyr Leu Tyr Ala Met Glu Thr Glu Pro Glu
                325                 330                 335

Pro Pro Pro Pro Pro Thr Leu Pro Pro Arg Leu Leu Arg Leu Pro Arg
            340                 345                 350

Met Gln Tyr Cys Asp Met Val Gly
        355                 360

<210>  150
<211>  1053
<212>  DNA
<213>  Petunia x hybrida

<400>  150
atgttaattg tagtacatat tattagcatc acacgcatca tattcatcac cttaacccat      60
aatcatctcc atttccttat gtttagatca ttatcataca atcacaagca cctcaaattc     120
cttacaaacc cgaccacacg ggtactccga agaaacatgt cgtcatccac tgtagtaaca     180
atacccggcc ccacacaaaa aaacaaaaac aaaatcatag taataatggg tgcaacaggt     240
tcaggaaaat caaaactctc aatagacctc gtcacacgtc actatccttt ttccgaaatc     300
attaactccg acaaaatcca aattaccaaa ggtttaaaca taaccacaaa caaaatcact     360
gtacccgacc gacgtggcgt agttcatcat ttactcggcg agattgaccc cgactttaac     420
ttttctcctt ctcatttccg gtcaattgct ggtcaacgca ttaactccat tattaatcgc     480
cataaactcc cattcctcgt tggtgggtcc aactcatata tctacgcttt attaacaaac     540
cggttcgacc cggattttaa ccctgattca aacccggttc attttatatc caacgagtta     600
cgctacaact gttgttttat ttgggtcgat gtattaaacc cggttttgaa tgagtatttg     660
gataaacggg tcgatgagat gatgaactcg ggtatgtatg aagaactgga acagtttttt     720
aaagaaaaca ggttttcgga tccgggtttg gaaccgggtc gggccaccgg gttgaggaaa     780
gcgatagggg taccggaaat ggagaggtat tttaagaaga gctgtacgta tgaggaagca     840
gtgagggaaa taaaagaaaa cacgtggcgg ttagcgaaga agcagatgtg gaagatccaa     900
```

```
cggttgagag aagcagggtg ggacctacaa agagtagatg ccacggaggc atttgtggag     960
gcgatgagta ataagaagga aaagggaatt atttgggaaa aacaagtagt ggaaccaagt    1020
gtcaagattg tgaaccgttt tttgttggac tga                                 1053

<210>  151
<211>  350
<212>  PRT
<213>  Petunia x hybrida

<400>  151

Met Leu Ile Val Val His Ile Ile Ser Ile Thr Arg Ile Ile Phe Ile
1               5                   10                  15

Thr Leu Thr His Asn His Leu His Phe Leu Met Phe Arg Ser Leu Ser
            20                  25                  30

Tyr Asn His Lys His Leu Lys Phe Leu Thr Asn Pro Thr Thr Arg Val
        35                  40                  45

Leu Arg Arg Asn Met Ser Ser Ser Thr Val Val Thr Ile Pro Gly Pro
    50                  55                  60

Thr Gln Lys Asn Lys Asn Lys Ile Ile Val Ile Met Gly Ala Thr Gly
65                  70                  75                  80

Ser Gly Lys Ser Lys Leu Ser Ile Asp Leu Val Thr Arg His Tyr Pro
                85                  90                  95

Phe Ser Glu Ile Ile Asn Ser Asp Lys Ile Gln Ile Thr Lys Gly Leu
            100                 105                 110

Asn Ile Thr Thr Asn Lys Ile Thr Val Pro Asp Arg Arg Gly Val Val
        115                 120                 125

His His Leu Leu Gly Glu Ile Asp Pro Asp Phe Asn Phe Ser Pro Ser
    130                 135                 140

His Phe Arg Ser Ile Ala Gly Gln Arg Ile Asn Ser Ile Ile Asn Arg
145                 150                 155                 160

His Lys Leu Pro Phe Leu Val Gly Gly Ser Asn Ser Tyr Ile Tyr Ala
                165                 170                 175

Leu Leu Thr Asn Arg Phe Asp Pro Asp Phe Asn Pro Asp Ser Asn Pro
            180                 185                 190

Val His Phe Ile Ser Asn Glu Leu Arg Tyr Asn Cys Cys Phe Ile Trp
        195                 200                 205

Val Asp Val Leu Asn Pro Val Leu Asn Glu Tyr Leu Asp Lys Arg Val
    210                 215                 220
```

Asp Glu Met Met Asn Ser Gly Met Tyr Glu Glu Leu Glu Gln Phe Phe
225 230 235 240

Lys Glu Asn Arg Phe Ser Asp Pro Gly Leu Glu Pro Gly Arg Ala Thr
245 250 255

Gly Leu Arg Lys Ala Ile Gly Val Pro Glu Met Glu Arg Tyr Phe Lys
260 265 270

Lys Ser Cys Thr Tyr Glu Glu Ala Val Arg Glu Ile Lys Glu Asn Thr
275 280 285

Trp Arg Leu Ala Lys Lys Gln Met Trp Lys Ile Gln Arg Leu Arg Glu
290 295 300

Ala Gly Trp Asp Leu Gln Arg Val Asp Ala Thr Glu Ala Phe Val Glu
305 310 315 320

Ala Met Ser Asn Lys Lys Glu Lys Gly Ile Ile Trp Glu Lys Gln Val
325 330 335

Val Glu Pro Ser Val Lys Ile Val Asn Arg Phe Leu Leu Asp
340 345 350

<210> 152
<211> 1199
<212> DNA
<213> Populus tremuloides

<400> 152
cttactctgt ttttttttt ttcgttgcct atgaaaattc ccttcccaaa gagtaccaat 60
cagcctcttt acactgccct taaaacccaa ccacttcacc ccattaacat ttctataccc 120
ttcaataagc cacgcccacc accaatagca gtccgtatgg acacggactc ctctaccacc 180
acctccaccg ccgtctaccg ccataaaaaa gacaagattc tcgtaataat gggagcgact 240
gggtgtggca aaacaagggt atctattgat ctagctacac gcttccaatc cgaaatcatc 300
aactccgaca aaatgcaagt ctacgaaggt cttgacatca ccaccaacaa aatcaccatt 360
caagaccgtc taggtgttcc tcaccattta ctcggtgagt tcgacccgga tgatggtgag 420
ttgactccct ccgagtatcg gttagctggt ggattggcta tctcaggtat tgtttcaagg 480
caaaatcttc ctattgtggt tggtgggtcc aactcactta ttcacgcttt ggttgttgac 540
cggtttaatc ccgagttaaa cgtttttgat gggtgtaacc cagtttcaac ccagttaaga 600
tataactgtt gtttttgtg ggtggatgtg tcattacctg ttttgtgcga ttacttgtgt 660
aagcgagtcg acgaaatgct cgactccggg atgctcgatg agctgtcaga gtattatggc 720
tcagttgatg cagcgagtca aatcgggttg aggaaagcga ttggggtgcc tgagtttgat 780
cggtatttca aaaagtaccc acctgggtct gggtgtggca gaggtattgg tgtggaatgg 840
gatcgggtac ggaggggagt atacgaggtc tgtgtgaggg agataaagga gaacacgtgt 900

cagcttgcga aaaggcagat cggcaagatc ttgagattaa aaggggcagg gtgggaccta 960

aaaagagttg atgcgactga gagctttagg gaggtgatga cggtgacgtc agatgatcat 1020

atcaagaaaa gaaagaagaa gaggtggatg gaggtctggg ggagagatgt gatggagcca 1080

agcatgaaaa ttgtgaaacg cttcttggag gaggagtagg agtaggtttt acagtcagtc 1140

agccagtcaa tccatcaatc aatcaatcca gctttttcc cgctaccgtt ctggttttc 1199

<210> 153
<211> 362
<212> PRT
<213> Populus tremuloides

<400> 153

Met Lys Ile Pro Phe Pro Lys Ser Thr Asn Gln Pro Leu Tyr Thr Ala
1 5 10 15

Leu Lys Thr Gln Pro Leu His Pro Ile Asn Ile Ser Ile Pro Phe Asn
20 25 30

Lys Pro Arg Pro Pro Pro Ile Ala Val Arg Met Asp Thr Asp Ser Ser
35 40 45

Thr Thr Thr Ser Thr Ala Val Tyr Arg His Lys Lys Asp Lys Ile Leu
50 55 60

Val Ile Met Gly Ala Thr Gly Cys Gly Lys Thr Arg Val Ser Ile Asp
65 70 75 80

Leu Ala Thr Arg Phe Gln Ser Glu Ile Ile Asn Ser Asp Lys Met Gln
85 90 95

Val Tyr Glu Gly Leu Asp Ile Thr Thr Asn Lys Ile Thr Ile Gln Asp
100 105 110

Arg Leu Gly Val Pro His His Leu Leu Gly Glu Phe Asp Pro Asp Asp
115 120 125

Gly Glu Leu Thr Pro Ser Glu Tyr Arg Leu Ala Gly Gly Leu Ala Ile
130 135 140

Ser Gly Ile Val Ser Arg Gln Asn Leu Pro Ile Val Val Gly Gly Ser
145 150 155 160

Asn Ser Leu Ile His Ala Leu Val Val Asp Arg Phe Asn Pro Glu Leu
165 170 175

Asn Val Phe Asp Gly Cys Asn Pro Val Ser Thr Gln Leu Arg Tyr Asn
180 185 190

Cys Cys Phe Leu Trp Val Asp Val Ser Leu Pro Val Leu Cys Asp Tyr
195 200 205

Leu Cys Lys Arg Val Asp Glu Met Leu Asp Ser Gly Met Leu Asp Glu
210 215 220

Leu Ser Glu Tyr Tyr Gly Ser Val Asp Ala Ala Ser Gln Ile Gly Leu
225 230 235 240

Arg Lys Ala Ile Gly Val Pro Glu Phe Asp Arg Tyr Phe Lys Lys Tyr
245 250 255

Pro Pro Gly Ser Gly Cys Gly Arg Gly Ile Gly Val Glu Trp Asp Arg
260 265 270

Val Arg Arg Gly Val Tyr Glu Val Cys Val Arg Glu Ile Lys Glu Asn
275 280 285

Thr Cys Gln Leu Ala Lys Arg Gln Ile Gly Lys Ile Leu Arg Leu Lys
290 295 300

Gly Ala Gly Trp Asp Leu Lys Arg Val Asp Ala Thr Glu Ser Phe Arg
305 310 315 320

Glu Val Met Thr Val Thr Ser Asp Asp His Ile Lys Lys Arg Lys Lys
325 330 335

Lys Arg Trp Met Glu Val Trp Gly Arg Asp Val Met Glu Pro Ser Met
340 345 350

Lys Ile Val Lys Arg Phe Leu Glu Glu Glu
355 360

<210> 154
<211> 1094
<212> DNA
<213> Populus tremuloides

<400> 154
ctcaacgaca acgtctcagg aaattcaaca atgaccatga ggctttcttt gaccgcctac 60
aaacaagtac agcctcgtgg gaatttccaa ggggggctaa acatgaaacc tttctttcgt 120
cgaaaagata aggttgtgtt cgttgttgga ccgactggca caggcaaatc aaggctggct 180
attgacctgg caacccgttt cccagccgag gttgtcaatt gtgacaaaat gcaagtttat 240
aaaggcctcg atatagtcac aaacaaggtc actgaagagg agtgtcgcgg tgtgcctcat 300
catttacttg gcatagcaga ccctaatgca aatttcactt ccgatgactt caggcaccat 360
gcatcacttg ttgtcgaatc aatcgtcaca cgtgatcggc taccgatcat cgccggtgga 420
tcaaattcct acatcgaggc tttagcaaat gatgatcctg aatttcgatt aaggtatgaa 480
tgttgttttc tttgggtgga cgtgtcactc ccaatacttt attcattcgt atcagagcgg 540
gtcgatcgaa tggtggaagc aggcttgatt gatgaggtga gagatatgtt tgatcctaat 600
aaatttgatg attattcaca aggaatcaaa cgggcaattg gggttcctga attggatcat 660

```
tttttacgaa atgaggcgat tgtggatgct aaaactcgta gaaagcttct tgatgaggcc     720
attgataaaa ttaaagaaaa tacttgtatg ctagcctctc gacaattaca aaaaatccat     780
cggcttcata gcatatggaa ttggaacgtg caccgaatcg atgccacccc agttttccta     840
acgagtggaa aggaggttga caatctttgg gacaaacttg tggcaggacc aagcaccatg     900
attgtgaacc agtttctttg tgacaaaaat tatgcatctc ccattatacc atcagaatca     960
atcaagatgg agccaatctc tgtcccggcc ctggctgttg gcgccactcg atagaggcct    1020
gtcaggtcat caaaatcacc atcaatcaca ctagtgctat tatgccaatg ggcgcttttg    1080
ccacgtggca gggt                                                      1094
```

```
<210>  155
<211>  327
<212>  PRT
<213>  Populus tremuloides

<400>  155

Met Thr Met Arg Leu Ser Leu Thr Ala Tyr Lys Gln Val Gln Pro Arg
1               5                   10                  15

Gly Asn Phe Gln Gly Gly Leu Asn Met Lys Pro Phe Phe Arg Arg Lys
            20                  25                  30

Asp Lys Val Val Phe Val Val Gly Pro Thr Gly Thr Gly Lys Ser Arg
        35                  40                  45

Leu Ala Ile Asp Leu Ala Thr Arg Phe Pro Ala Glu Val Val Asn Cys
    50                  55                  60

Asp Lys Met Gln Val Tyr Lys Gly Leu Asp Ile Val Thr Asn Lys Val
65                  70                  75                  80

Thr Glu Glu Glu Cys Arg Gly Val Pro His His Leu Leu Gly Ile Ala
                85                  90                  95

Asp Pro Asn Ala Asn Phe Thr Ser Asp Asp Phe Arg His His Ala Ser
            100                 105                 110

Leu Val Val Glu Ser Ile Val Thr Arg Asp Arg Leu Pro Ile Ile Ala
        115                 120                 125

Gly Gly Ser Asn Ser Tyr Ile Glu Ala Leu Ala Asn Asp Asp Pro Glu
    130                 135                 140

Phe Arg Leu Arg Tyr Glu Cys Cys Phe Leu Trp Val Asp Val Ser Leu
145                 150                 155                 160

Pro Ile Leu Tyr Ser Phe Val Ser Glu Arg Val Asp Arg Met Val Glu
                165                 170                 175
```

Ala Gly Leu Ile Asp Glu Val Arg Asp Met Phe Asp Pro Asn Lys Phe
180 185 190

Asp Asp Tyr Ser Gln Gly Ile Lys Arg Ala Ile Gly Val Pro Glu Leu
195 200 205

Asp His Phe Leu Arg Asn Glu Ala Ile Val Asp Ala Lys Thr Arg Arg
210 215 220

Lys Leu Leu Asp Glu Ala Ile Asp Lys Ile Lys Glu Asn Thr Cys Met
225 230 235 240

Leu Ala Ser Arg Gln Leu Gln Lys Ile His Arg Leu His Ser Ile Trp
245 250 255

Asn Trp Asn Val His Arg Ile Asp Ala Thr Pro Val Phe Leu Thr Ser
260 265 270

Gly Lys Glu Val Asp Asn Leu Trp Asp Lys Leu Val Ala Gly Pro Ser
275 280 285

Thr Met Ile Val Asn Gln Phe Leu Cys Asp Lys Asn Tyr Ala Ser Pro
290 295 300

Ile Ile Pro Ser Glu Ser Ile Lys Met Glu Pro Ile Ser Val Pro Ala
305 310 315 320

Leu Ala Val Gly Ala Thr Arg
325

<210> 156
<211> 1094
<212> DNA
<213> Populus tremuloides

<400> 156
gtcaacgaca acgtcccagc acattcaaca atgaccatga ggctttcttt gtctgcctac 60
aaacaagtac agcctcgtgt gaatttccaa ggggggctca acatgaaacc tttctttcgt 120
cgaaaagata aggttgtgtt cgttcttgga ccgactggca ctggcaaatc aaggctggct 180
attgacctag caacccactt cccagccgag gttgtcaatt gtgacaaaat gcaagtttat 240
aagggcctag atatagtcac aaacaaggtg acggaagagg agtgtcgagg cgtgccccat 300
catttacttg gcatagcaga ccctaatgca gatttcactt ccgatgactt caggcaccac 360
gcatcacttg ttgtcgaatc aatcgtcaca cgtgatcggc tgccgatcat tgccggcgga 420
tccaattcgt acgtggaggc tttagcaaat gatgatcctg agtttcgatt aaggtatgaa 480
tgttgctttc tttgggtgga tgtgtcacta cctctactcc actcattcgt atcagatcgg 540
gttgatcgaa tggtaagagc aggcttgatt gatgaggtga gagatgtgtt tgatccaaca 600
aaatttgatg attattcaca aggaatcaag cgggcaattg ggttcctga attagatcaa 660
tttctacgaa acgagacgat tgtggatgct aaaacacgta gaaagcttct tgatgaggcc 720

attgaaaaaa tcaaagaaaa tacttgtatg ctagctcgtc gacaattaca aaaaatccgt 780

cgacttcata gcatatggaa ttggaaaatg caccggatcg atgccacacc agttttccta 840

gcaagtggaa aggaggctga caatctttgg gaccaaattg tggcaggacc aagcaccatg 900

attgtgaacc aatttctttg tgaagaaaat catgtatccc ccattgtacc atcagagtcg 960

atcaatatgg tgccaatttc tgtcccggct atggccgccg tggctagtcg atagaggtaa 1020

aggattcttg gttaatacca aaattcatct acaaatcatt accatcatca taatcaccag 1080

ctgtgattat aaat 1094

<210> 157
<211> 327
<212> PRT
<213> Populus tremuloides

<400> 157

Met Thr Met Arg Leu Ser Leu Ser Ala Tyr Lys Gln Val Gln Pro Arg
1 5 10 15

Val Asn Phe Gln Gly Gly Leu Asn Met Lys Pro Phe Phe Arg Arg Lys
20 25 30

Asp Lys Val Val Phe Val Leu Gly Pro Thr Gly Thr Gly Lys Ser Arg
35 40 45

Leu Ala Ile Asp Leu Ala Thr His Phe Pro Ala Glu Val Val Asn Cys
50 55 60

Asp Lys Met Gln Val Tyr Lys Gly Leu Asp Ile Val Thr Asn Lys Val
65 70 75 80

Thr Glu Glu Glu Cys Arg Gly Val Pro His His Leu Leu Gly Ile Ala
85 90 95

Asp Pro Asn Ala Asp Phe Thr Ser Asp Asp Phe Arg His His Ala Ser
100 105 110

Leu Val Val Glu Ser Ile Val Thr Arg Asp Arg Leu Pro Ile Ile Ala
115 120 125

Gly Gly Ser Asn Ser Tyr Val Glu Ala Leu Ala Asn Asp Asp Pro Glu
130 135 140

Phe Arg Leu Arg Tyr Glu Cys Cys Phe Leu Trp Val Asp Val Ser Leu
145 150 155 160

Pro Leu Leu His Ser Phe Val Ser Asp Arg Val Asp Arg Met Val Arg
165 170 175

Ala Gly Leu Ile Asp Glu Val Arg Asp Val Phe Asp Pro Thr Lys Phe
180 185 190

```
Asp Asp Tyr Ser Gln Gly Ile Lys Arg Ala Ile Gly Val Pro Glu Leu
        195                 200                 205

Asp Gln Phe Leu Arg Asn Glu Thr Ile Val Asp Ala Lys Thr Arg Arg
    210                 215                 220

Lys Leu Leu Asp Glu Ala Ile Glu Lys Ile Lys Glu Asn Thr Cys Met
225                 230                 235                 240

Leu Ala Arg Arg Gln Leu Gln Lys Ile Arg Arg Leu His Ser Ile Trp
                245                 250                 255

Asn Trp Lys Met His Arg Ile Asp Ala Thr Pro Val Phe Leu Ala Ser
            260                 265                 270

Gly Lys Glu Ala Asp Asn Leu Trp Asp Gln Ile Val Ala Gly Pro Ser
        275                 280                 285

Thr Met Ile Val Asn Gln Phe Leu Cys Glu Glu Asn His Val Ser Pro
    290                 295                 300

Ile Val Pro Ser Glu Ser Ile Asn Met Val Pro Ile Ser Val Pro Ala

305                 310                 315                 320

Met Ala Ala Val Ala Ser Arg
                325
```

<210> 158
<211> 1046
<212> DNA
<213> Populus tremuloides

<400> 158

```
tgtaagtccc tgtgcccaca aacaagtcgg atgcttgata tccctcctgg tatgctcaaa      60
atggacatgc taagtcccag aagccggcaa aaggagaagg ttgtgatagt aatggggcct     120
actggaactg gcaagtctcg gctctctatc gaacttgcaa cccagttccc tgcagaaatc     180
ataaactccg acaaaatgca ggtttacaaa ggacttgaca tcgtcactaa caaagttgct     240
gaggaagaga aatctggtgt ccctcaccat ttgttaggaa tagcaaatcc tactgtggac     300
tttacagcaa caaattactg tcacacggct tccttggcgg tcgaatcaat ttcgactcga     360
ggattgctcc cgatcattgt tggaggttcc aattcataca ttgaggcctt gatggatgat     420
gaggatttta ggttacggtt gaactatgat tgttgcttcc tttgggtaga tgtatcaatg     480
cctgtgctac ataaatttgt ttcgaggcga gtcgagcaga tggttagtgt ggggatgatc     540
gatgaggtca gaaacatttt cgatccctac gctgattatt ctacggggat caggaggtca     600
attggagttc ctgaattcga caagtacttt agagctgaag catttttgga tgaagaaaac     660
cgtgccagac tacttcatga agcaatatgt gatgtcaaaa agaatacatg taagttagct     720
```

tgccgtcaat gggaaaagat taatcggctt aggaagatca aggggtggga catccataga 780
cttgatgcca ctgaagtatt ccaaaagtcg ggaaaggaag cagatcatgc ctgggaaatg 840
cttgtggcca gacccagcac tgccattgtg ggacaactcc tctgtggtgt tcctgctgat 900
aaggtccccg ccatagctag cgtagcaaaa aacatgggct atattcgaca atgccttgtg 960
gcatagccgt tcaatatggc taattaacga ctttagaaag tggtatcata tacaatatta 1020
tctaagtaga gggctatcaa ggcagg 1046

<210> 159
<211> 311
<212> PRT
<213> Populus tremuloides

<400> 159

Met Leu Asp Ile Pro Pro Gly Met Leu Lys Met Asp Met Leu Ser Pro
1 5 10 15

Arg Ser Arg Gln Lys Glu Lys Val Val Ile Val Met Gly Pro Thr Gly
20 25 30

Thr Gly Lys Ser Arg Leu Ser Ile Glu Leu Ala Thr Gln Phe Pro Ala
35 40 45

Glu Ile Ile Asn Ser Asp Lys Met Gln Val Tyr Lys Gly Leu Asp Ile
50 55 60

Val Thr Asn Lys Val Ala Glu Glu Glu Lys Ser Gly Val Pro His His
65 70 75 80

Leu Leu Gly Ile Ala Asn Pro Thr Val Asp Phe Thr Ala Thr Asn Tyr
85 90 95

Cys His Thr Ala Ser Leu Ala Val Glu Ser Ile Ser Thr Arg Gly Leu
100 105 110

Leu Pro Ile Ile Val Gly Gly Ser Asn Ser Tyr Ile Glu Ala Leu Met
115 120 125

Asp Asp Glu Asp Phe Arg Leu Arg Leu Asn Tyr Asp Cys Cys Phe Leu
130 135 140

Trp Val Asp Val Ser Met Pro Val Leu His Lys Phe Val Ser Arg Arg
145 150 155 160

Val Glu Gln Met Val Ser Val Gly Met Ile Asp Glu Val Arg Asn Ile
165 170 175

Phe Asp Pro Tyr Ala Asp Tyr Ser Thr Gly Ile Arg Arg Ser Ile Gly
180 185 190

Val Pro Glu Phe Asp Lys Tyr Phe Arg Ala Glu Ala Phe Leu Asp Glu

195 200 205

Glu Asn Arg Ala Arg Leu Leu His Glu Ala Ile Cys Asp Val Lys Lys
210 215 220

Asn Thr Cys Lys Leu Ala Cys Arg Gln Trp Glu Lys Ile Asn Arg Leu
225 230 235 240

Arg Lys Ile Lys Gly Trp Asp Ile His Arg Leu Asp Ala Thr Glu Val
245 250 255

Phe Gln Lys Ser Gly Lys Glu Ala Asp His Ala Trp Glu Met Leu Val
260 265 270

Ala Arg Pro Ser Thr Ala Ile Val Gly Gln Leu Leu Cys Gly Val Pro
275 280 285

Ala Asp Lys Val Pro Ala Ile Ala Ser Val Ala Lys Asn Met Gly Tyr
290 295 300

Ile Arg Gln Cys Leu Val Ala
305 310

<210> 160
<211> 1112
<212> DNA
<213> Populus tremuloides

<400> 160
ttacaccatt ctcaaataat tacacgaatt atggaaatga ttcctctaca accaaagctt 60
gcctcaagta tgagaactat gccaatggct gcctctctcc ccttaagaat ggagcgggaa 120
atcaggcacc gcaacaacct ccaacagatc actagttcac tttattccat tgacgacaag 180
aagaagcaaa aagctttgtt cgtaatggga acaactgcca ctggaaaatc aaaactctcc 240
attgatttag ccactcattt tcaaggtgaa atcatcaatt cagacaaaat tcaggtttac 300
aagggccttg acatgttgac caacaaaatt tcggaaattg aacgccgagg tgtgcctcac 360
catttgctag gatttgtgga acctggagaa gagtttacca cacaagattt ttgcaatcat 420
gtacacaagg ccatgaaaca tattacaggg gatggaagca tccccattat cgccggcggc 480
tcgaatagat acatcgaagc actcgtcgag gatccgcttt tcaacttcaa ggatagttat 540
gatacttgtt ttctatgggt ggatgttgcc ttgccaatct tatttgatcg cgcagcaaaa 600
agggttgatg agatgcttga tgctggtctt gttgaagagg ttcgaggtat gtttattcca 660
gggatagatc acaatagcgg gatttggcgg gctattggga ttgcagagat ggaaccatat 720
tttcaagctg aaatggaaat ggctgatgag gtcaccatga aaatattgct tgaaactggt 780
attaaagaaa tgaaagaaaa taccaagaag ctaatcaata aacaactaac gaaaatcaaa 840
tatttggcta acaagaaagg atggaaattc catcggattg atgctacttg tgtgtatgag 900
agaagtgcaa aagttgatga agatgtttgg gacaagaagg ttttgagacc tagcttggag 960

atagttacta attttctccg ggaagacgag aaagcagaag aagtggcaga cagttttcta 1020

gttacaagct agcagctgca tctaatcaat tgagatatac cgattagttc aattttctat 1080

cgcatcttta cttctcagca cacatcaaaa ta 1112

<210> 161
<211> 333
<212> PRT
<213> Populus tremuloides

<400> 161

Met Glu Met Ile Pro Leu Gln Pro Lys Leu Ala Ser Ser Met Arg Thr
1 5 10 15

Met Pro Met Ala Ala Ser Leu Pro Leu Arg Met Glu Arg Glu Ile Arg
20 25 30

His Arg Asn Asn Leu Gln Gln Ile Thr Ser Ser Leu Tyr Ser Ile Asp
35 40 45

Asp Lys Lys Lys Gln Lys Ala Leu Phe Val Met Gly Thr Thr Ala Thr
50 55 60

Gly Lys Ser Lys Leu Ser Ile Asp Leu Ala Thr His Phe Gln Gly Glu
65 70 75 80

Ile Ile Asn Ser Asp Lys Ile Gln Val Tyr Lys Gly Leu Asp Met Leu
85 90 95

Thr Asn Lys Ile Ser Glu Ile Glu Arg Arg Gly Val Pro His His Leu
100 105 110

Leu Gly Phe Val Glu Pro Gly Glu Glu Phe Thr Thr Gln Asp Phe Cys
115 120 125

Asn His Val His Lys Ala Met Lys His Ile Thr Gly Asp Gly Ser Ile
130 135 140

Pro Ile Ile Ala Gly Gly Ser Asn Arg Tyr Ile Glu Ala Leu Val Glu
145 150 155 160

Asp Pro Leu Phe Asn Phe Lys Asp Ser Tyr Asp Thr Cys Phe Leu Trp
165 170 175

Val Asp Val Ala Leu Pro Ile Leu Phe Asp Arg Ala Ala Lys Arg Val
180 185 190

Asp Glu Met Leu Asp Ala Gly Leu Val Glu Glu Val Arg Gly Met Phe
195 200 205

Ile Pro Gly Ile Asp His Asn Ser Gly Ile Trp Arg Ala Ile Gly Ile

```
    210                 215                 220

Ala Glu Met Glu Pro Tyr Phe Gln Ala Glu Met Glu Met Ala Asp Glu
225                 230                 235                 240

Val Thr Met Lys Ile Leu Leu Glu Thr Gly Ile Lys Glu Met Lys Glu
                245                 250                 255

Asn Thr Lys Lys Leu Ile Asn Lys Gln Leu Thr Lys Ile Lys Tyr Leu
            260                 265                 270

Ala Asn Lys Lys Gly Trp Lys Phe His Arg Ile Asp Ala Thr Cys Val
        275                 280                 285

Tyr Glu Arg Ser Ala Lys Val Asp Glu Asp Val Trp Asp Lys Lys Val
    290                 295                 300

Leu Arg Pro Ser Leu Glu Ile Val Thr Asn Phe Leu Arg Glu Asp Glu
305                 310                 315                 320

Lys Ala Glu Glu Val Ala Asp Ser Phe Leu Val Thr Ser
                325                 330
```

<210> 162
<211> 1325
<212> DNA
<213> Populus tremuloides

<400> 162

```
ggcagtacaa agcaaaaagc tttgttcgta atgggaacaa ctgccactgg aaaatcaaaa     60
ctctccatcg atttagccac tcattttcaa ggtgagatca tcaactcgga caaaattcag    120
gtttacaagg gccttgacat attgaccaac aaagtttcgg aagatgaaag ccgaggtgtg    180
cctcaccact tgctaggatt tgtggaacct ggggaagagt ttaccacaca agatttttgc    240
aaccatgtac atatggccat gagacatatt atagggaatg gaaacatccc cattattgcc    300
ggcggctcaa atagatacat cgaagcactc gtcgaggatc cactgttcaa ggataattac    360
gatacttgtt ttctatgggt ggatgttgcc ttgccaattt tatttgttcg cgcagcaaag    420
agggttgata agatgctcga tgctggtctt gtcgacgagg tccgaggcat gtttattcca    480
gggatagatc acaatagcgg gatttggcgg gctattggga ttccagagct ggaaccatat    540
tttcaagctg aaatggaaat ggccgatgag gtgaccagaa aaatgttact tgacactggt    600
atcaaggaaa tgaaagaaaa taccaagaag ctaatcaata aacaactgag gaaaatcaaa    660
tatttggcga atgagaaagg atggaaatta catcggattg atgctacttt tgtgtacgag    720
agaagtggaa acgtagatga agatgtttgg gacgacaagg ttttgagacc tagcctggag    780
atgctcacta actttctcca ggaagatggg aaagcagaag aatttgtgga tgctagtggt    840
ttgcctggta gctttgaatg gagagagaaa ggagttgtca ctgatgtcaa gattcagggt    900
gcttgtggat catgctgggc ttttagcacc actggatctg ttgaaggagc aaattttatt    960
```

gcaacaagga agcttctcaa ccttagtgaa caacagcttg ttgattgtga cagtgtgaca 1020
gacaagactt cctgtggtga tggttgcggt ggagggttca tgaccaatgc ctacaggtgt 1080
ttgatcgagg caggggcgtt acaagaggag actggattga atgcaatatt aatgcaaact 1140
tgtattagag gggtctcgtg cccagttatt cgcggcaaga aatggctcaa ccgtggtgtt 1200
ctacttgttg ggaatggtgc aagaggttac tccattctta attaggtctg gctacaagcc 1260
gtactggatc atcaggaact catggggtga gcattgggga gagaagggac atcaccttct 1320
ttgca 1325

<210> 163
<211> 404
<212> PRT
<213> Populus tremuloides

<400> 163

Met Gly Thr Thr Ala Thr Gly Lys Ser Lys Leu Ser Ile Asp Leu Ala
1 5 10 15

Thr His Phe Gln Gly Glu Ile Ile Asn Ser Asp Lys Ile Gln Val Tyr
20 25 30

Lys Gly Leu Asp Ile Leu Thr Asn Lys Val Ser Glu Asp Glu Ser Arg
35 40 45

Gly Val Pro His His Leu Leu Gly Phe Val Glu Pro Gly Glu Glu Phe
50 55 60

Thr Thr Gln Asp Phe Cys Asn His Val His Met Ala Met Arg His Ile
65 70 75 80

Ile Gly Asn Gly Asn Ile Pro Ile Ile Ala Gly Gly Ser Asn Arg Tyr
85 90 95

Ile Glu Ala Leu Val Glu Asp Pro Leu Phe Lys Asp Asn Tyr Asp Thr
100 105 110

Cys Phe Leu Trp Val Asp Val Ala Leu Pro Ile Leu Phe Val Arg Ala
115 120 125

Ala Lys Arg Val Asp Lys Met Leu Asp Ala Gly Leu Val Asp Glu Val
130 135 140

Arg Gly Met Phe Ile Pro Gly Ile Asp His Asn Ser Gly Ile Trp Arg
145 150 155 160

Ala Ile Gly Ile Pro Glu Leu Glu Pro Tyr Phe Gln Ala Glu Met Glu
165 170 175

Met Ala Asp Glu Val Thr Arg Lys Met Leu Leu Asp Thr Gly Ile Lys
180 185 190

Glu Met Lys Glu Asn Thr Lys Lys Leu Ile Asn Lys Gln Leu Arg Lys
195 200 205

Ile Lys Tyr Leu Ala Asn Glu Lys Gly Trp Lys Leu His Arg Ile Asp
210 215 220

Ala Thr Phe Val Tyr Glu Arg Ser Gly Asn Val Asp Glu Asp Val Trp
225 230 235 240

Asp Asp Lys Val Leu Arg Pro Ser Leu Glu Met Leu Thr Asn Phe Leu
245 250 255

Gln Glu Asp Gly Lys Ala Glu Glu Phe Val Asp Ala Ser Gly Leu Pro
260 265 270

Gly Ser Phe Glu Trp Arg Glu Lys Gly Val Val Thr Asp Val Lys Ile
275 280 285

Gln Gly Ala Cys Gly Ser Cys Trp Ala Phe Ser Thr Thr Gly Ser Val
290 295 300

Glu Gly Ala Asn Phe Ile Ala Thr Arg Lys Leu Leu Asn Leu Ser Glu
305 310 315 320

Gln Gln Leu Val Asp Cys Asp Ser Val Thr Asp Lys Thr Ser Cys Gly
325 330 335

Asp Gly Cys Gly Gly Gly Phe Met Thr Asn Ala Tyr Arg Cys Leu Ile
340 345 350

Glu Ala Gly Ala Leu Gln Glu Glu Thr Gly Leu Asn Ala Ile Leu Met
355 360 365

Gln Thr Cys Ile Arg Gly Val Ser Cys Pro Val Ile Arg Gly Lys Lys
370 375 380

Trp Leu Asn Arg Gly Val Leu Leu Val Gly Asn Gly Ala Arg Gly Tyr
385 390 395 400

Ser Ile Leu Asn

<210> 164
<211> 1233
<212> DNA
<213> Vitis vinifera

<400> 164
atgccatctc tgtcgkaatg tgaygtatct gtgcaggact gcacaaacca ccccaccaaa 60

acgaccgtca cttctattcc tatgaaactc cccgtcccca ccggttatca tccatattgc 120

agcaaagttc aaacgctccc actcataccg gcgataaagc ccaccttccg aagatccggg 180

```
tgggctcgca tggattccac ggyccgccgt aatcgcmcca agcacaaggt cgtcgttatc     240
atgggagcca ccggcaccgg aaaatccaaa ctctccatcg acctcgccac acgcttcccc     300
gccgagatca tcaactctga caaaatacaa atctacagtg gccttgacat caccactaac     360
aagatccaaa tgcacgagcg acaaggtgta ccccaccacc tgctcggaga tttcgactcc     420
tcccacgctg agataacccc ttcccagttc cgctcggtgg ctgccgctgc tatctcagac     480
atctcttctc gccgcaaact gccagtcctc gctggtgggt ccaattcctt tattcacgca     540
ctcctcgtcg accggtttga ctccgagtct gaccccttya atggttcgga ctcggtctcc     600
accgaattac gttaccgctg ttgtttccta tgggttgacg tttcatttgc tgttctctcc     660
gactacttgt cgaagcgagt cgatgaaatg cttggctcag ggatgcttga agagttagct     720
aagttctacg acccggatga agacgagtct agacccaaaa ctgggttgag aaaagcgata     780
ggagtccccg agttcgacag gcatttccga aagtaccctc ccgtcgacca aggaataatc     840
gctggaaatc ccaagaagaa gaaggatgat ccagaaatgg aaagttttga agaggcagtg     900
aaggccatca aggacaacac gtgtcatcta gcgaagaagc agatagagaa gatcctacgg     960
atgaggggag ccgggtggga cctcaagaga ctggacgcca cagaggcgtt cagggtgctg    1020
ctgtcgtcag attccggcaa gaagtcgtca gaaatatggg agaagcaggt agtggaaccg    1080
agcgtgaagt ttgtgaggcg cttcttagag gagtaggttg ttcagctttt tctacccgtc    1140
ctgttttcct atgatccaaa attagttacc taaattactt tcatttcccc cttttttttgg   1200
atttactctt tccctctcta tttgaaaaaa aaa                                 1233
```

<210> 165
<211> 371
<212> PRT
<213> Vitis vinifera

<220>
<221> misc_feature
<222> (6)..(6)
<223> Xaa can be any naturally occurring amino acid

<220>
<221> misc_feature
<222> (68)..(68)
<223> Xaa can be any naturally occurring amino acid

<220>
<221> misc_feature
<222> (73)..(73)
<223> Xaa can be any naturally occurring amino acid

<400> 165

```
Met Pro Ser Leu Ser Xaa Cys Asp Val Ser Val Gln Asp Cys Thr Asn
1               5                   10                  15

His Pro Thr Lys Thr Thr Val Thr Ser Ile Pro Met Lys Leu Pro Val
            20                  25                  30

Pro Thr Gly Tyr His Pro Tyr Cys Ser Lys Val Gln Thr Leu Pro Leu
```

35 40 45

Ile Pro Ala Ile Lys Pro Thr Phe Arg Arg Ser Gly Trp Ala Arg Met
50 55 60

Asp Ser Thr Xaa Arg Arg Asn Arg Xaa Lys His Lys Val Val Val Ile
65 70 75 80

Met Gly Ala Thr Gly Thr Gly Lys Ser Lys Leu Ser Ile Asp Leu Ala
85 90 95

Thr Arg Phe Pro Ala Glu Ile Ile Asn Ser Asp Lys Ile Gln Ile Tyr
100 105 110

Ser Gly Leu Asp Ile Thr Thr Asn Lys Ile Gln Met His Glu Arg Gln
115 120 125

Gly Val Pro His His Leu Leu Gly Asp Phe Asp Ser Ser His Ala Glu
130 135 140

Ile Thr Pro Ser Gln Phe Arg Ser Val Ala Ala Ala Ala Ile Ser Asp
145 150 155 160

Ile Ser Ser Arg Arg Lys Leu Pro Val Leu Ala Gly Gly Ser Asn Ser
165 170 175

Phe Ile His Ala Leu Leu Val Asp Arg Phe Asp Ser Glu Ser Asp Pro
180 185 190

Phe Asn Gly Ser Asp Ser Val Ser Thr Glu Leu Arg Tyr Arg Cys Cys
195 200 205

Phe Leu Trp Val Asp Val Ser Phe Ala Val Leu Ser Asp Tyr Leu Ser
210 215 220

Lys Arg Val Asp Glu Met Leu Gly Ser Gly Met Leu Glu Glu Leu Ala
225 230 235 240

Lys Phe Tyr Asp Pro Asp Glu Asp Glu Ser Arg Pro Lys Thr Gly Leu
245 250 255

Arg Lys Ala Ile Gly Val Pro Glu Phe Asp Arg His Phe Arg Lys Tyr
260 265 270

Pro Pro Val Asp Gln Gly Ile Ile Ala Gly Asn Pro Lys Lys Lys Lys
275 280 285

Asp Asp Pro Glu Met Glu Ser Phe Glu Glu Ala Val Lys Ala Ile Lys
290 295 300

Asp Asn Thr Cys His Leu Ala Lys Lys Gln Ile Glu Lys Ile Leu Arg
305 310 315 320

```
Met Arg Gly Ala Gly Trp Asp Leu Lys Arg Leu Asp Ala Thr Glu Ala
                325                 330                 335

Phe Arg Val Leu Leu Ser Ser Asp Ser Gly Lys Lys Ser Ser Glu Ile
            340                 345                 350

Trp Glu Lys Gln Val Val Glu Pro Ser Val Lys Phe Val Arg Arg Phe
        355                 360                 365

Leu Glu Glu
    370
```

<210> 166
<211> 1167
<212> DNA
<213> Zea mays

<400> 166

```
atgaccaccc tcctcgccaa taggatcact acgctcgtgc gcgcccctcc tcctcccatg      60
gccgccgccg ccgtcgcggg agcgcggagg ccattgcacc ggaccttggc gcacccgcca     120
ccgcccgagg aggacgagca tcagcagcag cgcgcgtgcc gcagcagggg atcctcgtcc     180
tcctgctcgg cttcctcgtc atcgacgccc gcccggcccc ggggcacggg gatggtggtg     240
atcgtcggcg ccacgggcac cgggaagacc aagctgtcca tcgacgccgc ggaggcggtc     300
ggcggggagg tggtgaacgc ggataagatc cagctctacg ccgggctgga cgtgaccacg     360
aacaaggtgg cccccgcgga ccgccgcggc gtgccgcacc acctcctcgg cgccatccgc     420
cccgaggccg gcgagctccc gccctccacg ttccgctccc tcgccgccgc cacggccgcc     480
tcgatcgccg cgcgcggccg cctgccggtc gtcgcgggcg gctccaactc cctcatccac     540
gcgctcctcg ccgaccgcct cgacgccggc gccgccgacc ccttctccgc tccaccgcag     600
ccggcgccgc cgcggtgggg ccgccggccc gcgctccgat ccccgtgctg tctcctctgg     660
gtccacgtcg acgccgcgct cctcgcggag tacctggacc ggcgcgtgga cgacatggtg     720
cgcggcggca tggtggagga gctgcgggag tacttcgccg cgaccaccgc cgccgagcgc     780
gccgcgcacg ccgcggggct gggcagggcc atcggcgtgc ccgagctggg cgcctgcttc     840
gcggggcgcg ccagcttccg cgccgcgatc gacgacatca aggccaacac gcgggacctg     900
gcggccgcgc aggtgcgcaa gatccgacgc atggccgatg cctggggctg gcccatccag     960
cggctcgacg cgtcggccac agtccgcgcg cgcctccgcg gcgcggggcc cgacgcggag    1020
tcggcgtgct gggagcgcga cgtgcgcgcg cccgggctcg ccgccatccg gagcttcctt    1080
ctagagctgg acggcggcag cgtcgtcgac ggcgctgtgg tggaggaggt ggagccgcgg    1140
gtgcgatgct gcgacgtggt ggggtga                                        1167
```

<210> 167
<211> 388
<212> PRT
<213> Zea mays

<400> 167

Met Thr Thr Leu Leu Ala Asn Arg Ile Thr Thr Leu Val Arg Ala Pro
1 5 10 15

Pro Pro Pro Met Ala Ala Ala Ala Val Ala Gly Ala Arg Arg Pro Leu
20 25 30

His Arg Thr Leu Ala His Pro Pro Pro Pro Glu Glu Asp Glu His Gln
35 40 45

Gln Gln Arg Ala Cys Arg Ser Arg Gly Ser Ser Ser Ser Cys Ser Ala
50 55 60

Ser Ser Ser Ser Thr Pro Ala Arg Pro Arg Gly Thr Gly Met Val Val
65 70 75 80

Ile Val Gly Ala Thr Gly Thr Gly Lys Thr Lys Leu Ser Ile Asp Ala
85 90 95

Ala Glu Ala Val Gly Gly Glu Val Val Asn Ala Asp Lys Ile Gln Leu
100 105 110

Tyr Ala Gly Leu Asp Val Thr Thr Asn Lys Val Ala Pro Ala Asp Arg
115 120 125

Arg Gly Val Pro His His Leu Leu Gly Ala Ile Arg Pro Glu Ala Gly
130 135 140

Glu Leu Pro Pro Ser Thr Phe Arg Ser Leu Ala Ala Ala Thr Ala Ala
145 150 155 160

Ser Ile Ala Ala Arg Gly Arg Leu Pro Val Val Ala Gly Gly Ser Asn
165 170 175

Ser Leu Ile His Ala Leu Leu Ala Asp Arg Leu Asp Ala Gly Ala Ala
180 185 190

Asp Pro Phe Ser Ala Pro Pro Gln Pro Ala Pro Pro Arg Trp Gly Arg
195 200 205

Arg Pro Ala Leu Arg Ser Pro Cys Cys Leu Leu Trp Val His Val Asp
210 215 220

Ala Ala Leu Leu Ala Glu Tyr Leu Asp Arg Arg Val Asp Asp Met Val
225 230 235 240

Arg Gly Gly Met Val Glu Glu Leu Arg Glu Tyr Phe Ala Ala Thr Thr
245 250 255

Ala Ala Glu Arg Ala Ala His Ala Ala Gly Leu Gly Arg Ala Ile Gly
260 265 270

```
Val Pro Glu Leu Gly Ala Cys Phe Ala Gly Arg Ala Ser Phe Arg Ala
        275                 280                 285

Ala Ile Asp Asp Ile Lys Ala Asn Thr Arg Asp Leu Ala Ala Ala Gln
    290                 295                 300

Val Arg Lys Ile Arg Arg Met Ala Asp Ala Trp Gly Trp Pro Ile Gln
305                 310                 315                 320

Arg Leu Asp Ala Ser Ala Thr Val Arg Ala Arg Leu Arg Gly Ala Gly
                325                 330                 335

Pro Asp Ala Glu Ser Ala Cys Trp Glu Arg Asp Val Arg Ala Pro Gly
            340                 345                 350

Leu Ala Ala Ile Arg Ser Phe Leu Leu Glu Leu Asp Gly Gly Ser Val
        355                 360                 365

Val Asp Gly Ala Val Val Glu Glu Val Glu Pro Arg Val Arg Cys Cys
    370                 375                 380

Asp Val Val Gly
385
```

<210> 168
<211> 753
<212> DNA
<213> Oryza sativa

<400> 168

```
atgaaggtgc agtgcgacgt gtgcgcggcc gaggccgcct cggtcttctg ctgcgccgac     60
gaggccgcgc tgtgcgacgc gtgcgaccgc cgcgtccaca gcgcgaacaa gctcgccggg    120
aagcaccgcc gattctccct cctccaaccg ttggcgtcgt cgtcgtccgc ccagaagcca    180
ccgctctgcg acatctgtca ggagaagagg gggttcttgt tctgcaagga ggacagggcg    240
atcctgtgcc gggagtgcga cgtcacggtg cacaccacga gcgagctgac gaggcggcac    300
ggccggttcc tcctcaccgg cgtgcgcctc tcgtcggcgc cgatggactc ccccgcgccg    360
tcggaggaag aggaggagga agcaggggag gactacagct gcagccccag cagcgtcgcc    420
ggcaccgccg cggggagcgc gagcgacggg agcagcatct ccgagtacct caccaagacg    480
ctgcccggtt ggcacgtcga ggacttcctc gtcgacgagg ccaccgccgc ctcctcctcc    540
tcagacgggc tatttcaggg tgggctgctg gctcagatcg gtggggtgcc ggacggttac    600
gcggcgtggg ccggccggga gcagctgcac agtggcgtcg ctgtcgccgc cgacgagcgg    660
gccagccgcg agcggtgggt gccgcagatg aacgcggagt ggggcgccgg cagcaagcga    720
cccagggcgt cgcctccctg cttgtactgg tga                                 753
```

<210> 169
<211> 250
<212> PRT
<213> Oryza sativa

<400> 169

Met Lys Val Gln Cys Asp Val Cys Ala Ala Glu Ala Ala Ser Val Phe
1               5                   10                  15

Cys Cys Ala Asp Glu Ala Ala Leu Cys Asp Ala Cys Asp Arg Arg Val
            20                  25                  30

His Ser Ala Asn Lys Leu Ala Gly Lys His Arg Arg Phe Ser Leu Leu
        35                  40                  45

Gln Pro Leu Ala Ser Ser Ser Ser Ala Gln Lys Pro Pro Leu Cys Asp
    50                  55                  60

Ile Cys Gln Glu Lys Arg Gly Phe Leu Phe Cys Lys Glu Asp Arg Ala
65                  70                  75                  80

Ile Leu Cys Arg Glu Cys Asp Val Thr Val His Thr Thr Ser Glu Leu
                85                  90                  95

Thr Arg Arg His Gly Arg Phe Leu Leu Thr Gly Val Arg Leu Ser Ser
            100                 105                 110

Ala Pro Met Asp Ser Pro Ala Pro Ser Glu Glu Glu Glu Glu Glu Ala
        115                 120                 125

Gly Glu Asp Tyr Ser Cys Ser Pro Ser Ser Val Ala Gly Thr Ala Ala
    130                 135                 140

Gly Ser Ala Ser Asp Gly Ser Ser Ile Ser Glu Tyr Leu Thr Lys Thr
145                 150                 155                 160

Leu Pro Gly Trp His Val Glu Asp Phe Leu Val Asp Glu Ala Thr Ala
                165                 170                 175

Ala Ser Ser Ser Ser Asp Gly Leu Phe Gln Gly Gly Leu Leu Ala Gln
            180                 185                 190

Ile Gly Gly Val Pro Asp Gly Tyr Ala Ala Trp Ala Gly Arg Glu Gln
        195                 200                 205

Leu His Ser Gly Val Ala Val Ala Ala Asp Glu Arg Ala Ser Arg Glu
    210                 215                 220

Arg Trp Val Pro Gln Met Asn Ala Glu Trp Gly Ala Gly Ser Lys Arg
225                 230                 235                 240

Pro Arg Ala Ser Pro Pro Cys Leu Tyr Trp

245 250

<210> 170
<211> 1074
<212> DNA
<213> Oryza sativa

<400> 170
atgaaggtgc tgtgctccgc gtgcgaggcg gcggaggcgc gggtgctctg ctgcgccgac 60

gacgccgccc tctgcgcgcg ctgcgacctc cacgtccacg ccgccaaccg cctcgccggc 120

aagcaccacc gcctccccct cctctcctcc tcctcttctt cctcctctcc ctcccccccg 180

acctgcgaca tctgccagga cgcccacgcc tacttcttct gcgtcgagga ccgcgccctc 240

ctctgccgcg cctgcgacgt cgccgtccac accgccaacg ccctcgtctc cgcccaccgc 300

cgcttcctcc tcaccggcgt ccacgtcggc cttgacgccg ccgccgacga cgacgacaaa 360

caccccccac acccccttgtc gtcgtcgctg ccgcgcaaca cggcaccgcc cccgcagccg 420

ccgccgaagc gcagcccctc gccgatctac agcgatgacg acgtcatcga ctgggccacc 480

ggtggccacg acatcggcat caccggcaac ctgcccgact ggtcgctcgt cgacgagcag 540

ttcaacaccc ctgcgctgcc gccggtggtg accaagaccc cgccgaagcg ggcctcccgt 600

ggccccgtca cggccggcac cgccgcggcg gtgttcggca acctcgccgg cggatcgccg 660

gactggccgc tcaacgagtt cttcggcttc gccgacttca gctccggctt cggcttcgcc 720

gagaacggca cgtccaaggc ggacagcggc aagatcggga gcatggacgg ctcgccgaac 780

ggcggcaggt cgtcgtcgtc gtcctcctcc tcctccgccg ccgccgccgg cggcggcggc 840

ggcggccagg acttcttcgg ccaggtgccg gaagttcact gggccgtgcc ggagctcccc 900

tcgccgccca cggcgtcagg gctccactgg caacgggacc cgcgctacgg tggcggcgcc 960

accgacgcca gcgcggtgtt cgtgccggac atctcctcgc cggagaaccc cttccgttgc 1020

ttcgccgccg ccgccgccgg tgaccatacc atgaaacgcc ggaggagatg ctaa 1074

<210> 171
<211> 357
<212> PRT
<213> Oryza sativa

<400> 171

Met Lys Val Leu Cys Ser Ala Cys Glu Ala Ala Glu Ala Arg Val Leu
1 5 10 15

Cys Cys Ala Asp Asp Ala Ala Leu Cys Ala Arg Cys Asp Leu His Val
20 25 30

His Ala Ala Asn Arg Leu Ala Gly Lys His His Arg Leu Pro Leu Leu
35 40 45

Ser Ser Ser Ser Ser Ser Ser Ser Pro Ser Pro Pro Thr Cys Asp Ile
50 55 60

Cys Gln Asp Ala His Ala Tyr Phe Phe Cys Val Glu Asp Arg Ala Leu
65 70 75 80

Leu Cys Arg Ala Cys Asp Val Ala Val His Thr Ala Asn Ala Leu Val
85 90 95

Ser Ala His Arg Arg Phe Leu Leu Thr Gly Val His Val Gly Leu Asp
100 105 110

Ala Ala Ala Asp Asp Asp Asp Lys His Pro Pro His Pro Leu Ser Ser
115 120 125

Ser Leu Pro Arg Asn Thr Ala Pro Pro Pro Gln Pro Pro Pro Lys Arg
130 135 140

Ser Pro Ser Pro Ile Tyr Ser Asp Asp Asp Val Ile Asp Trp Ala Thr
145 150 155 160

Gly Gly His Asp Ile Gly Ile Thr Gly Asn Leu Pro Asp Trp Ser Leu
165 170 175

Val Asp Glu Gln Phe Asn Thr Pro Ala Leu Pro Pro Val Val Thr Lys
180 185 190

Thr Pro Pro Lys Arg Ala Ser Arg Gly Pro Val Thr Ala Gly Thr Ala
195 200 205

Ala Ala Val Phe Gly Asn Leu Ala Gly Gly Ser Pro Asp Trp Pro Leu
210 215 220

Asn Glu Phe Phe Gly Phe Ala Asp Phe Ser Ser Gly Phe Gly Phe Ala
225 230 235 240

Glu Asn Gly Thr Ser Lys Ala Asp Ser Gly Lys Ile Gly Ser Met Asp
245 250 255

Gly Ser Pro Asn Gly Gly Arg Ser Ser Ser Ser Ser Ser Ser Ser Ser
260 265 270

Ala Ala Ala Ala Gly Gly Gly Gly Gly Gly Gln Asp Phe Phe Gly Gln
275 280 285

Val Pro Glu Val His Trp Ala Val Pro Glu Leu Pro Ser Pro Pro Thr
290 295 300

Ala Ser Gly Leu His Trp Gln Arg Asp Pro Arg Tyr Gly Gly Gly Ala
305 310 315 320

Thr Asp Ala Ser Ala Val Phe Val Pro Asp Ile Ser Ser Pro Glu Asn
325 330 335

Pro Phe Arg Cys Phe Ala Ala Ala Ala Ala Gly Asp His Thr Met Lys

340 345 350

Arg Arg Arg Arg Cys
355

<210> 172
<211> 816
<212> DNA
<213> Oryza sativa

<400> 172
atgaagatcc agtgcgacgc gtgcgagagc gcggcggcgg cggtggtgtg ctgcgcggac 60

gaggcggcgc tgtgcgcggc gtgcgacgtg gaggtgcacg cggcgaacaa gctggccggg 120

aagcaccagc ggctgccgct ggaggcgctc tcggcgaggc tcccgcgctg cgacgtgtgc 180

caggagaagg cggcgttcat cttctgcgtg gaggaccgcg cgctcttctg ccgcgactgc 240

gacgagccca tccacgtccc cggcacgctc tccggcaacc accagcgcta cctcgccacc 300

ggcatccgcg tcggcttcgc ctccgcctcg ccctgcgacg gcggcagcga cgcccatgac 360

tccgaccacc acgccccgcc catgggctcc tccgagcatc atcaccatca tcagcagccg 420

gccccgaccg tcgccgtcga cacgccctcg ccgcagttcc tgccgcaggg ctgggccgtc 480

gacgagctcc tccagttctc cgactacgag accggcgaca agctgcagaa ggagtcgtcg 540

ccgccgctcg ggttccagga gctggagtgg ttcgccgaca tcgacctgtt ccacaaccag 600

gcgcccaagg gcggcgccgc cgccggccgg acgacggcgg aggtccccga gctcttcgct 660

tcgcaggcgg ccaacgacgt ggcgtactac aggccgccga ccaggaccgc cgccgccgcc 720

ttcaccgcgg ccaccggctt ccgccagagc aagaaggccc gcgtcgagct ccccgacgac 780

gaggaggatt acctcatcgt ccctgatctt ggttga 816

<210> 173
<211> 271
<212> PRT
<213> Oryza sativa

<400> 173

Met Lys Ile Gln Cys Asp Ala Cys Glu Ser Ala Ala Ala Ala Val Val
1 5 10 15

Cys Cys Ala Asp Glu Ala Ala Leu Cys Ala Ala Cys Asp Val Glu Val
20 25 30

His Ala Ala Asn Lys Leu Ala Gly Lys His Gln Arg Leu Pro Leu Glu
35 40 45

Ala Leu Ser Ala Arg Leu Pro Arg Cys Asp Val Cys Gln Glu Lys Ala
50 55 60

Ala Phe Ile Phe Cys Val Glu Asp Arg Ala Leu Phe Cys Arg Asp Cys
65 70 75 80

Asp Glu Pro Ile His Val Pro Gly Thr Leu Ser Gly Asn His Gln Arg
85 90 95

Tyr Leu Ala Thr Gly Ile Arg Val Gly Phe Ala Ser Ala Ser Pro Cys
100 105 110

Asp Gly Gly Ser Asp Ala His Asp Ser Asp His His Ala Pro Pro Met
115 120 125

Gly Ser Ser Glu His His His His His Gln Gln Pro Ala Pro Thr Val
130 135 140

Ala Val Asp Thr Pro Ser Pro Gln Phe Leu Pro Gln Gly Trp Ala Val
145 150 155 160

Asp Glu Leu Leu Gln Phe Ser Asp Tyr Glu Thr Gly Asp Lys Leu Gln
165 170 175

Lys Glu Ser Ser Pro Pro Leu Gly Phe Gln Glu Leu Glu Trp Phe Ala
180 185 190

Asp Ile Asp Leu Phe His Asn Gln Ala Pro Lys Gly Gly Ala Ala Ala
195 200 205

Gly Arg Thr Thr Ala Glu Val Pro Glu Leu Phe Ala Ser Gln Ala Ala
210 215 220

Asn Asp Val Ala Tyr Tyr Arg Pro Pro Thr Arg Thr Ala Ala Ala Ala
225 230 235 240

Phe Thr Ala Ala Thr Gly Phe Arg Gln Ser Lys Lys Ala Arg Val Glu
245 250 255

Leu Pro Asp Asp Glu Glu Asp Tyr Leu Ile Val Pro Asp Leu Gly
260 265 270

<210> 174
<211> 810
<212> DNA
<213> Oryza sativa

<400> 174
atgaaggtgc agtgcgacgt gtgcgcggcc gaggcggcgt cggtgttctg ctgcgccgac 60

gaggccgcgc tgtgcgacgc gtgcgaccac cgggtgcacc gggccaacaa gctcgccggg 120

aagcaccgcc ggttctcgct gctcaacccc tcggcgtccg gccgctcgcc gacatcgacg 180

acggcgccgc tctgcgacat ctgccaggag aagaggggtt tcctgttctg caaggaggac 240

cgggcgatcc tgtgccgcga gtgcgacgtg ccggtgcaca cggcgagcga gctcaccatg 300

cgccacagcc ggtacctcct caccggcgtg cggctctcct cggagcctgc cgcgtccccg 360

gcgccgccgt cggaggagga gaacagcagc agcttctgct gcagcgccga cgacgccgtg 420

ccggccccgg cggcgcccgc cacgagccac ggcgggagca gcggcagcag cagcatctcc 480

gagtacctca ccacgctgcc cgggtggcac gtcgaggact tcctcgtcga cgacgccact 540

gccgaggccg ccgccgccgc cgccgccacc tcttccggca tctccgcgaa cgggccgtgt 600

cagggggtaa cacggatcgg agggctgcaa gaatccgccg gctaccctgc gtggatggcg 660

cagcagcagc tgtgctgcga cggcctcgtc gccggcgacg cgtcgccggc tagccgggag 720

cggtgggtgc cgcagatgta cgcggatcag cttgccgccg gcagcaagag atccaggacg 780

tccactgctt cttcctactc ctactggtga 810

<210> 175
<211> 269
<212> PRT
<213> Oryza sativa

<400> 175

Met Lys Val Gln Cys Asp Val Cys Ala Ala Glu Ala Ala Ser Val Phe
1 5 10 15

Cys Cys Ala Asp Glu Ala Ala Leu Cys Asp Ala Cys Asp His Arg Val
20 25 30

His Arg Ala Asn Lys Leu Ala Gly Lys His Arg Arg Phe Ser Leu Leu
35 40 45

Asn Pro Ser Ala Ser Gly Arg Ser Pro Thr Ser Thr Thr Ala Pro Leu
50 55 60

Cys Asp Ile Cys Gln Glu Lys Arg Gly Phe Leu Phe Cys Lys Glu Asp
65 70 75 80

Arg Ala Ile Leu Cys Arg Glu Cys Asp Val Pro Val His Thr Ala Ser
85 90 95

Glu Leu Thr Met Arg His Ser Arg Tyr Leu Leu Thr Gly Val Arg Leu
100 105 110

Ser Ser Glu Pro Ala Ala Ser Pro Ala Pro Pro Ser Glu Glu Glu Asn
115 120 125

Ser Ser Ser Phe Cys Cys Ser Ala Asp Asp Ala Val Pro Ala Pro Ala
130 135 140

Ala Pro Ala Thr Ser His Gly Gly Ser Ser Gly Ser Ser Ser Ile Ser
145 150 155 160

Glu Tyr Leu Thr Thr Leu Pro Gly Trp His Val Glu Asp Phe Leu Val
165 170 175

Asp Asp Ala Thr Ala Glu Ala Ala Ala Ala Ala Ala Ala Thr Ser Ser
180 185 190

Gly Ile Ser Ala Asn Gly Pro Cys Gln Gly Val Thr Arg Ile Gly Gly
195 200 205

Leu Gln Glu Ser Ala Gly Tyr Pro Ala Trp Met Ala Gln Gln Gln Leu
210 215 220

Cys Cys Asp Gly Leu Val Ala Gly Asp Ala Ser Pro Ala Ser Arg Glu
225 230 235 240

Arg Trp Val Pro Gln Met Tyr Ala Asp Gln Leu Ala Ala Gly Ser Lys
245 250 255

Arg Ser Arg Thr Ser Thr Ala Ser Ser Tyr Ser Tyr Trp
260 265

<210> 176
<211> 774
<212> DNA
<213> Oryza sativa

<400> 176
atgaggatcc agtgcgacgc gtgcgaggcc gcggcggcca cggtggtgtg ctgcgcggac 60
gaggcggcgc tgtgcgcgcg ctgcgacgtc gagatccacg ccgccaacaa gctcgccagc 120
aagcaccagc gcctcccgct cgacgccgcg ctccccgccg ccctcccgcg ctgcgacgtc 180
tgccaggaga aggcggcgtt catcttctgc gtggaggaca gggcgctctt ctgccgggac 240
tgcgacgagc ccatccacgt cccggggacg ctctccggca accaccagcg ctacctcacc 300
accggcatcc gcgtcgggtt cagctccgtc tgtagcgcca acgccgacca cctcccgccg 360
ccagcgccca aggggaactc caagccgccg gcaagcggca tcgctgctgc tgctgctccc 420
aagccggccg tgtccgcggc ggcgcaggag gtgccgtcgt caccgttctt gccgccgtcg 480
ggctgggccg tcgaggatct cctgcagctc tccgactacg agtccagcga caagaagggc 540
tctcctattg ggttcaagga tctggagtgg ctcgatgaca tcgacctgtt ccatgtccag 600
tcgccggcca agggaggcag cacggcggcg gaggtgcctg agctcttcgc ctcgccgcag 660
ccagcgagca acatggggct ctacaaggcg agcggtgcac gccaaagcaa gaagccacgg 720
gtggagatac ccgatgacga cgaggacttc ttcatcgttc ctgatcttgg atga 774

<210> 177
<211> 257
<212> PRT
<213> Oryza sativa

<400> 177

Met Arg Ile Gln Cys Asp Ala Cys Glu Ala Ala Ala Ala Thr Val Val
1 5 10 15

Cys Cys Ala Asp Glu Ala Ala Leu Cys Ala Arg Cys Asp Val Glu Ile
20 25 30

His Ala Ala Asn Lys Leu Ala Ser Lys His Gln Arg Leu Pro Leu Asp

35 40 45

Ala Ala Leu Pro Ala Ala Leu Pro Arg Cys Asp Val Cys Gln Glu Lys
50 55 60

Ala Ala Phe Ile Phe Cys Val Glu Asp Arg Ala Leu Phe Cys Arg Asp
65 70 75 80

Cys Asp Glu Pro Ile His Val Pro Gly Thr Leu Ser Gly Asn His Gln
85 90 95

Arg Tyr Leu Thr Thr Gly Ile Arg Val Gly Phe Ser Ser Val Cys Ser
100 105 110

Ala Asn Ala Asp His Leu Pro Pro Pro Ala Pro Lys Gly Asn Ser Lys
115 120 125

Pro Pro Ala Ser Gly Ile Ala Ala Ala Ala Ala Pro Lys Pro Ala Val
130 135 140

Ser Ala Ala Ala Gln Glu Val Pro Ser Ser Pro Phe Leu Pro Pro Ser
145 150 155 160

Gly Trp Ala Val Glu Asp Leu Leu Gln Leu Ser Asp Tyr Glu Ser Ser
165 170 175

Asp Lys Lys Gly Ser Pro Ile Gly Phe Lys Asp Leu Glu Trp Leu Asp
180 185 190

Asp Ile Asp Leu Phe His Val Gln Ser Pro Ala Lys Gly Gly Ser Thr
195 200 205

Ala Ala Glu Val Pro Glu Leu Phe Ala Ser Pro Gln Pro Ala Ser Asn
210 215 220

Met Gly Leu Tyr Lys Ala Ser Gly Ala Arg Gln Ser Lys Lys Pro Arg
225 230 235 240

Val Glu Ile Pro Asp Asp Asp Glu Asp Phe Phe Ile Val Pro Asp Leu
245 250 255

Gly

<210> 178
<211> 1137
<212> DNA
<213> Oryza sativa

<400> 178
atgtcgcctc ctcctccacc atattaccac cacctcctcc tcctccgctc ctcgcccacc 60

accactggag gaggagctcg ggttcttgcc gcggcggagc tcgcacgcat gaagctactg 120

tgcagcgcgt gcgaggcggc ggaggccagc gtcctctgct gcgccgacga ggccgccctg 180
tgcgcgcgct gcgaccgcga catccacgcc gccaaccgcc tcgccgggaa gcacctccgc 240
ctccctctcc tctcccccgc ctcctcctcc tcctcctccg ccgccgccct cgcgccgccg 300
ccgccgtcgc cgcccaagtg cgacatatgc caggagagcc acgcgtactt cttctgcctc 360
gaggaccgcg cgctgctgtg ccggagctgc gacgtggcgg tgcacacggc caacgccttc 420
gtctccgcgc accgccgttt cctcctcacc ggcgtgcagg tcgggcagga gcaggacgag 480
cactccccctg acccgcctga gccgtctcct cctccgccgc cgccgccgcc tgcatccaag 540
agcgaccacc cggcgccgct ctacggcgag ggcggaggag ggttcagctg ggacgccgcc 600
gactcgccgg ccgcgggcgg cctccccgac tggtcggccg tcgtcgacca gttcggctcc 660
ccgccgccgc cgcgccacac ggacaccgcg accgtgacga ccccgccgcc gaccaagagg 720
agcccacgcg cgccggcgtt cggcggccag ggcggcatga tggattggcc cctcggcgag 780

ttcttcggcg gcttcaccga cttcaccggc ggctttggct tcggcttcgg cgacagtggc 840
acctccaagg ctgacagcgg gaagctggga gggagcacgg acggctcgcc gtactaccgg 900
tcgtcatcgg aagatgaccg gaacgccgac gagctcttcg ggcaggtacc agagatccag 960
tggtcggtgc cggagctccc ctcgccgccg acggcctccg gcctccactg gcaacgccat 1020
ccagccgcca ctcacggcgg cggcggcggc ggacccgaca ccaccgcctt cgtccccgac 1080
atctgctccc ccgacagctg cttcccggcc accacctcca aacgccggag gcaataa 1137

<210> 179
<211> 378
<212> PRT
<213> Oryza sativa

<400> 179

Met Ser Pro Pro Pro Pro Pro Tyr Tyr His His Leu Leu Leu Leu Arg
1 5 10 15

Ser Ser Pro Thr Thr Thr Gly Gly Gly Ala Arg Val Leu Ala Ala Ala
20 25 30

Glu Leu Ala Arg Met Lys Leu Leu Cys Ser Ala Cys Glu Ala Ala Glu
35 40 45

Ala Ser Val Leu Cys Cys Ala Asp Glu Ala Ala Leu Cys Ala Arg Cys
50 55 60

Asp Arg Asp Ile His Ala Ala Asn Arg Leu Ala Gly Lys His Leu Arg
65 70 75 80

Leu Pro Leu Leu Ser Pro Ala Ser Ser Ser Ser Ser Ser Ala Ala Ala
85 90 95

Leu Ala Pro Pro Pro Pro Ser Pro Pro Lys Cys Asp Ile Cys Gln Glu

```
                100                 105                 110

Ser His Ala Tyr Phe Phe Cys Leu Glu Asp Arg Ala Leu Leu Cys Arg
        115                 120                 125

Ser Cys Asp Val Ala Val His Thr Ala Asn Ala Phe Val Ser Ala His
    130                 135                 140

Arg Arg Phe Leu Leu Thr Gly Val Gln Val Gly Gln Glu Gln Asp Glu
145                 150                 155                 160

His Ser Pro Asp Pro Pro Glu Pro Ser Pro Pro Pro Pro Pro Pro Pro
                165                 170                 175

Pro Ala Ser Lys Ser Asp His Pro Ala Pro Leu Tyr Gly Glu Gly Gly
            180                 185                 190

Gly Gly Phe Ser Trp Asp Ala Ala Asp Ser Pro Ala Ala Gly Gly Leu
        195                 200                 205

Pro Asp Trp Ser Ala Val Val Asp Gln Phe Gly Ser Pro Pro Pro Pro
    210                 215                 220

Arg His Thr Asp Thr Ala Thr Val Thr Thr Pro Pro Pro Thr Lys Arg
225                 230                 235                 240

Ser Pro Arg Ala Pro Ala Phe Gly Gly Gln Gly Gly Met Met Asp Trp
                245                 250                 255

Pro Leu Gly Glu Phe Phe Gly Gly Phe Thr Asp Phe Thr Gly Gly Phe
            260                 265                 270

Gly Phe Gly Phe Gly Asp Ser Gly Thr Ser Lys Ala Asp Ser Gly Lys
        275                 280                 285

Leu Gly Gly Ser Thr Asp Gly Ser Pro Tyr Tyr Arg Ser Ser Ser Glu
    290                 295                 300

Asp Asp Arg Asn Ala Asp Glu Leu Phe Gly Gln Val Pro Glu Ile Gln
305                 310                 315                 320

Trp Ser Val Pro Glu Leu Pro Ser Pro Pro Thr Ala Ser Gly Leu His
                325                 330                 335

Trp Gln Arg His Pro Ala Ala Thr His Gly Gly Gly Gly Gly Gly Pro
            340                 345                 350

Asp Thr Thr Ala Phe Val Pro Asp Ile Cys Ser Pro Asp Ser Cys Phe
        355                 360                 365

Pro Ala Thr Thr Ser Lys Arg Arg Arg Gln
    370                 375
```

<210> 180
<211> 1083
<212> DNA
<213> Oryza sativa

<400> 180
atgaagatcc agtgcaacgc gtgcggcgcg gcggaggcgc gggtgctgtg ctgcgccgac 60
gaggcagcgc tctgcacggc gtgcgacgag gaggtgcacg ccgccaacaa gctcgccggg 120
aagcaccagc gggtgccgct gctctccgac gacggcggcg ccgcgcccgc cgccgccgcc 180
ccggccgtgc ccaagtgcga catctgccag gaggcttctg gatacttctt ctgcctggag 240
gaccgtgcac ttctttgcag agattgtgat gtttctatac acacagtaaa ctcctttgtt 300
tcagtacacc aaagattcct actaacaggt gttcaagttg gccttgatcc tgctgatcca 360
gttccacctg ttgctgacaa gcatgttaag agtgctggtg gttcagtgga ttcagcaact 420
aaacatttgc aaaggaatcc tacagactta tctggtgaaa acagtgcatc tttgcccagc 480
caaaatgtaa tcaatggtaa ttattctagg cagagttctg ttacaatggc caagacagga 540
caggtcaatt ggactatgag caacaacaca attagatcaa tagaccctcc acccaagtat 600
tcatcagagg aaagtccagc acttctgcta gctagccaca ctagcaccat ggcagcgtac 660
tccagtcaaa tcagtaagga tagtgatcgg atctacaact taccattcac aggtggtaat 720
gggtcagata gtctacatga ttggcatgtt gatgagttct ttagtaactc agaatttggc 780
tttgctgagc atggttcttc taagggtgac aacgctaagc cagggagtgc tggtggatct 840
ccgcagtgcc gtctggctga aggcctgttt gtcgaaggac ttctaggtca agtgcctgac 900
aatccatgga cagtgcctga ggtcccctcg ccaccgacag cctctggtct ctattggcaa 960
aataatttgc tttgcccttc gtacgacagc accatgttcg tccctgagat ttcctccttg 1020
gagaactctc agaacaactt cactgtatct gctggtttga agcgccgaag gaggcagttt 1080
tga 1083

<210> 181
<211> 360
<212> PRT
<213> Oryza sativa

<400> 181

Met Lys Ile Gln Cys Asn Ala Cys Gly Ala Ala Glu Ala Arg Val Leu
1 5 10 15

Cys Cys Ala Asp Glu Ala Ala Leu Cys Thr Ala Cys Asp Glu Glu Val
20 25 30

His Ala Ala Asn Lys Leu Ala Gly Lys His Gln Arg Val Pro Leu Leu
35 40 45

Ser Asp Asp Gly Gly Ala Ala Pro Ala Ala Ala Ala Pro Ala Val Pro
50 55 60

Lys Cys Asp Ile Cys Gln Glu Ala Ser Gly Tyr Phe Phe Cys Leu Glu
65 70 75 80

Asp Arg Ala Leu Leu Cys Arg Asp Cys Asp Val Ser Ile His Thr Val
85 90 95

Asn Ser Phe Val Ser Val His Gln Arg Phe Leu Leu Thr Gly Val Gln
100 105 110

Val Gly Leu Asp Pro Ala Asp Pro Val Pro Pro Val Ala Asp Lys His
115 120 125

Val Lys Ser Ala Gly Gly Ser Val Asp Ser Ala Thr Lys His Leu Gln
130 135 140

Arg Asn Pro Thr Asp Leu Ser Gly Glu Asn Ser Ala Ser Leu Pro Ser
145 150 155 160

Gln Asn Val Ile Asn Gly Asn Tyr Ser Arg Gln Ser Ser Val Thr Met
165 170 175

Ala Lys Thr Gly Gln Val Asn Trp Thr Met Ser Asn Asn Thr Ile Arg
180 185 190

Ser Ile Asp Pro Pro Pro Lys Tyr Ser Ser Glu Glu Ser Pro Ala Leu
195 200 205

Leu Leu Ala Ser His Thr Ser Thr Met Ala Ala Tyr Ser Ser Gln Ile
210 215 220

Ser Lys Asp Ser Asp Arg Ile Tyr Asn Leu Pro Phe Thr Gly Gly Asn
225 230 235 240

Gly Ser Asp Ser Leu His Asp Trp His Val Asp Glu Phe Phe Ser Asn
245 250 255

Ser Glu Phe Gly Phe Ala Glu His Gly Ser Ser Lys Gly Asp Asn Ala
260 265 270

Lys Pro Gly Ser Ala Gly Gly Ser Pro Gln Cys Arg Leu Ala Glu Gly
275 280 285

Leu Phe Val Glu Gly Leu Leu Gly Gln Val Pro Asp Asn Pro Trp Thr
290 295 300

Val Pro Glu Val Pro Ser Pro Pro Thr Ala Ser Gly Leu Tyr Trp Gln
305 310 315 320

Asn Asn Leu Leu Cys Pro Ser Tyr Asp Ser Thr Met Phe Val Pro Glu
325 330 335

Ile Ser Ser Leu Glu Asn Ser Gln Asn Asn Phe Thr Val Ser Ala Gly
340 345 350

Leu Lys Arg Arg Arg Arg Gln Phe
355 360

<210> 182
<211> 927
<212> DNA
<213> Oryza sativa

<400> 182
atgcgggtgc agtgcgacgt ctgcgccgcc gagccggccg cggtgctctg ctgcgccgac 60
gaggccgcgc tctgctccgc ctgcgaccgc cgcgtccacc gcgccaaccg cctcgccagc 120
aagcaccgcc gcctcccgct cgtccacccg tcctcctcct cctccggcga cggtggcgcc 180
gccgccgcgc cgctgtgcga cgtgtgcagg gagaagaggg gcctcgtgtt ctgcgtcgag 240
gaccgcgcca tcctgtgcgc cgactgcgac gagcccatcc actccgccaa cgacctcacc 300
gccaagcaca cccgcttcct cctcgtcggc gccaagctct cccccgccgc gctcgccgaa 360
cagccgctcc cgtcctccga ctgcagctcc gacgacgacg ccgccgccgc cgccaccgag 420
gaggagtacc actcctccgc cgcatccacc ggcgccgcag tgagcgcgcc tcttgacgcc 480
tccagcaacg gcgccggtgg cggaggagga gtaggaggga gcagcatctc cgactacctc 540
accaccatct gccccggctg gcgcgtcgag gacctcctcc ccgacgacga cgccttcgcc 600
gccgccgccg cgcaggcggg gaaagagaag gacgagcgcg tgccgttcct cgacgccgac 660
ctgttcgacg tggtcgccgg ccggccggag aagaagggcg gcgcgtgggc gccgcacgtg 720
ccgcacctgc cggcgtggtg cctcgacgag gtgccggtcg tcgtcgccgc gtcggcggcg 780
ccagcggcga cacctgtaaa ggcgaagcag ggacacgtgc gggacagcca ctggagcgac 840
agcgacgcgt tcgccgtgcc ggagttctcg ccgccgccgc cgccggccaa gagggcgcgg 900
cccagctcgc agttctggtg cttctga 927

<210> 183
<211> 308
<212> PRT
<213> Oryza sativa

<400> 183

Met Arg Val Gln Cys Asp Val Cys Ala Ala Glu Pro Ala Ala Val Leu
1 5 10 15

Cys Cys Ala Asp Glu Ala Ala Leu Cys Ser Ala Cys Asp Arg Arg Val
20 25 30

His Arg Ala Asn Arg Leu Ala Ser Lys His Arg Arg Leu Pro Leu Val
35 40 45

His Pro Ser Ser Ser Ser Ser Gly Asp Gly Gly Ala Ala Ala Ala Pro
50 55 60

Leu Cys Asp Val Cys Arg Glu Lys Arg Gly Leu Val Phe Cys Val Glu
65 70 75 80

Asp Arg Ala Ile Leu Cys Ala Asp Cys Asp Glu Pro Ile His Ser Ala
85 90 95

Asn Asp Leu Thr Ala Lys His Thr Arg Phe Leu Leu Val Gly Ala Lys
100 105 110

Leu Ser Pro Ala Ala Leu Ala Glu Gln Pro Leu Pro Ser Ser Asp Cys
115 120 125

Ser Ser Asp Asp Asp Ala Ala Ala Ala Ala Thr Glu Glu Glu Tyr His
130 135 140

Ser Ser Ala Ala Ser Thr Gly Ala Ala Val Ser Ala Pro Leu Asp Ala
145 150 155 160

Ser Ser Asn Gly Ala Gly Gly Gly Gly Gly Val Gly Gly Ser Ser Ile
165 170 175

Ser Asp Tyr Leu Thr Thr Ile Cys Pro Gly Trp Arg Val Glu Asp Leu
180 185 190

Leu Pro Asp Asp Asp Ala Phe Ala Ala Ala Ala Ala Gln Ala Gly Lys
195 200 205

Glu Lys Asp Glu Arg Val Pro Phe Leu Asp Ala Asp Leu Phe Asp Val
210 215 220

Val Ala Gly Arg Pro Glu Lys Lys Gly Gly Ala Trp Ala Pro His Val
225 230 235 240

Pro His Leu Pro Ala Trp Cys Leu Asp Glu Val Pro Val Val Val Ala
245 250 255

Ala Ser Ala Ala Pro Ala Ala Thr Pro Val Lys Ala Lys Gln Gly His
260 265 270

Val Arg Asp Ser His Trp Ser Asp Ser Asp Ala Phe Ala Val Pro Glu
275 280 285

Phe Ser Pro Pro Pro Pro Pro Ala Lys Arg Ala Arg Pro Ser Ser Gln
290 295 300

Phe Trp Cys Phe
305

<210> 184
<211> 636
<212> DNA

<213> Oryza sativa

<400> 184
atgcggacga tctgcgacgt gtgcgagagc gcgccggcgg tgctcttctg cgtggccgac 60
gaggccgcgc tctgccggtc ctgcgacgag aaggtgcata tgtgtaacaa gcttgctagg 120
cggcacgtga gagttgggct tgcagaccct aataaagttc aacgctgtga tatatgtgaa 180
aatgcccccg ccttcttcta ttgcgagata gatggtacat cactttgcct tagttgtgat 240
atgactgttc atgttggtgg gaaacgaacc catggaagat acctgctcct aaggcaacgg 300
gttgaatttc caggagataa accaggtcat atggatgatg ttgctatgca acagaaagat 360
cctgaaaacc ggacggatca aaagaaggcc cctcactcag taacaaagga gcaaatggca 420
aaccatcata atgtgtctga tgatccagcc tcagatggca actgcgatga ccagggtaac 480
atcgattcca aaatgattga tcttaatatg agacccgtcc gtactcatgg acaaggttca 540
aactcacaga ctcagggcgt ggatgttagc gtcaacaatc atgattctcc aggagtggtg 600
ccaacatgta atttcgaacg agaagccaac aaataa 636

<210> 185
<211> 211
<212> PRT
<213> Oryza sativa

<400> 185

Met Arg Thr Ile Cys Asp Val Cys Glu Ser Ala Pro Ala Val Leu Phe
1 5 10 15

Cys Val Ala Asp Glu Ala Ala Leu Cys Arg Ser Cys Asp Glu Lys Val
20 25 30

His Met Cys Asn Lys Leu Ala Arg Arg His Val Arg Val Gly Leu Ala
35 40 45

Asp Pro Asn Lys Val Gln Arg Cys Asp Ile Cys Glu Asn Ala Pro Ala
50 55 60

Phe Phe Tyr Cys Glu Ile Asp Gly Thr Ser Leu Cys Leu Ser Cys Asp
65 70 75 80

Met Thr Val His Val Gly Gly Lys Arg Thr His Gly Arg Tyr Leu Leu
85 90 95

Leu Arg Gln Arg Val Glu Phe Pro Gly Asp Lys Pro Gly His Met Asp
100 105 110

Asp Val Ala Met Gln Gln Lys Asp Pro Glu Asn Arg Thr Asp Gln Lys
115 120 125

Lys Ala Pro His Ser Val Thr Lys Glu Gln Met Ala Asn His His Asn
130 135 140

Val Ser Asp Asp Pro Ala Ser Asp Gly Asn Cys Asp Asp Gln Gly Asn
145 150 155 160

Ile Asp Ser Lys Met Ile Asp Leu Asn Met Arg Pro Val Arg Thr His
165 170 175

Gly Gln Gly Ser Asn Ser Gln Thr Gln Gly Val Asp Val Ser Val Asn
180 185 190

Asn His Asp Ser Pro Gly Val Val Pro Thr Cys Asn Phe Glu Arg Glu
195 200 205

Ala Asn Lys
210

<210> 186
<211> 633
<212> DNA
<213> Oryza sativa

<400> 186
atgaagatcg ggtgcgacgc gtgcgagcag gcggaggcgg cggtgctgtg ctgcgccgac 60
gaggccgcgc tctgccgccg ctgcgacgcc gccgtccact ccgccaacag gctcgccggc 120
aagcacaccc gcgtcgcgct cctcctcccc tcctcctcct ccgccgccgc cggcgacgac 180
gaccaccacc ccacctgcga catctgccag gagaagacgg gctacttctt ctgcctcgag 240
gaccgcgccc tgctctgccg gagctgcgac gtcgccgtcc acaccgccac tgcgcacgcc 300
gccgcccacc gccgcttcct catcaccggc gtccgcatcg gcggcagcgt cgacgccgcc 360
gccgcggccg acgtcatcgt cagcccaaca agcagcagca tcgcgccggc cggctcggcc 420
agcagcaacc acgccggcgc cgccggcaac aacaatggcc ggtcgccggc gccggtgagg 480
ttctcagggg gagacggcgg cgttgagccg gagcagcagt ggccgtggag tgacgtcttc 540
gccgccgacg acgacgatga cgtcagcgcc gccatggagc agtgctacta tcatggcatc 600
tctgaacctc actcctccag cctcactgga tga 633

<210> 187
<211> 210
<212> PRT
<213> Oryza sativa

<400> 187

Met Lys Ile Gly Cys Asp Ala Cys Glu Gln Ala Glu Ala Ala Val Leu
1 5 10 15

Cys Cys Ala Asp Glu Ala Ala Leu Cys Arg Arg Cys Asp Ala Ala Val
20 25 30

His Ser Ala Asn Arg Leu Ala Gly Lys His Thr Arg Val Ala Leu Leu
35 40 45

```
Leu Pro Ser Ser Ser Ser Ala Ala Ala Gly Asp Asp Asp His His Pro
    50                  55                  60

Thr Cys Asp Ile Cys Gln Glu Lys Thr Gly Tyr Phe Phe Cys Leu Glu
65                  70                  75                  80

Asp Arg Ala Leu Leu Cys Arg Ser Cys Asp Val Ala Val His Thr Ala
                85                  90                  95

Thr Ala His Ala Ala Ala His Arg Arg Phe Leu Ile Thr Gly Val Arg
            100                 105                 110

Ile Gly Gly Ser Val Asp Ala Ala Ala Ala Ala Asp Val Ile Val Ser
        115                 120                 125

Pro Thr Ser Ser Ser Ile Ala Pro Ala Gly Ser Ala Ser Ser Asn His
    130                 135                 140

Ala Gly Ala Ala Gly Asn Asn Asn Gly Arg Ser Pro Ala Pro Val Arg
145                 150                 155                 160

Phe Ser Gly Gly Asp Gly Gly Val Glu Pro Glu Gln Gln Trp Pro Trp
                165                 170                 175

Ser Asp Val Phe Ala Ala Asp Asp Asp Asp Asp Val Ser Ala Ala Met
            180                 185                 190

Glu Gln Cys Tyr Tyr His Gly Ile Ser Glu Pro His Ser Ser Ser Leu
        195                 200                 205

Thr Gly
    210
```

<210> 188
<211> 597
<212> DNA
<213> Saccharum officinarum

<400> 188

```
atgaagatcc agtgcgacgc ttgcgagggc gcggctgcca cggtggtgtg ctgcgccgac      60
gaggccgcgc tgtgcgcgcg ctgcgacgtc gagatccacg ccgccaacaa gctcgccagc     120
aagcaccagc gcctcccgct cgaggcgctc tcggccaagc tcccgcgctg cgacgtctgc     180
caggagaaag cggcgttcat cttctgcgtg gaggaccggg cgctcttctg ccgggactgc     240
gacgagccca tccacgtccc gggcacgctc tccgggaacc accagcgcta cctcgccacc     300
ggcatccgcg tcggcttcgc ctccgcctcc gcctgcagcg acggcgcctg cgacgcccac     360
gactccgacc accacgcccc gcccaaggcc accatcgagc caccgcaggc caccgtctcc     420
gccgcggcgc agcaggtgcc ctcgccgccg cagttcctgc cgcagggctg ggccgtcgac     480
gagctcctgc agttctccga ctacgagtcc agcgacaagc tgcacaagga gtccccgctc     540
```

gggttcggag ctggagtggt tcgccgacat cgacctcttc cacgagcagg cgcctaa 597

<210> 189
<211> 198
<212> PRT
<213> Saccharum officinarum

<400> 189

Met Lys Ile Gln Cys Asp Ala Cys Glu Gly Ala Ala Ala Thr Val Val
1 5 10 15

Cys Cys Ala Asp Glu Ala Ala Leu Cys Ala Arg Cys Asp Val Glu Ile
20 25 30

His Ala Ala Asn Lys Leu Ala Ser Lys His Gln Arg Leu Pro Leu Glu
35 40 45

Ala Leu Ser Ala Lys Leu Pro Arg Cys Asp Val Cys Gln Glu Lys Ala
50 55 60

Ala Phe Ile Phe Cys Val Glu Asp Arg Ala Leu Phe Cys Arg Asp Cys
65 70 75 80

Asp Glu Pro Ile His Val Pro Gly Thr Leu Ser Gly Asn His Gln Arg
85 90 95

Tyr Leu Ala Thr Gly Ile Arg Val Gly Phe Ala Ser Ala Ser Ala Cys
100 105 110

Ser Asp Gly Ala Cys Asp Ala His Asp Ser Asp His His Ala Pro Pro
115 120 125

Lys Ala Thr Ile Glu Pro Pro Gln Ala Thr Val Ser Ala Ala Ala Gln
130 135 140

Gln Val Pro Ser Pro Pro Gln Phe Leu Pro Gln Gly Trp Ala Val Asp
145 150 155 160

Glu Leu Leu Gln Phe Ser Asp Tyr Glu Ser Ser Asp Lys Leu His Lys
165 170 175

Glu Ser Pro Leu Gly Phe Gly Ala Gly Val Val Arg Arg His Arg Pro
180 185 190

Leu Pro Arg Ala Gly Ala
195

<210> 190
<211> 534
<212> DNA
<213> Arabidopsis thaliana

<400> 190
atgaagatac agtgtgatgt gtgtgagaaa gctccggcga cggtgatttg ttgcgccgac 60

gaagctgctc tctgtcctca atgcgacatc gagattcacg ccgctaacaa actcgctagc 120

aagcaccaac gtcttcatct taattccctc tccaccaaat tccctcgttg cgatatctgc 180

caagagaagg cagctttcat tttctgtgta gaggatagag ctctgctttg cagggactgc 240

gatgaatcca tccacgtggc taattctcga tctgctaatc accagaggtt cttagccact 300

gggatcaaag tagctctgac ctcaactata tgtagtaaag aaattgagaa gaatcaacct 360

gagccttcca acaaccaaca gaaggctaat cagattcctg ctaaatccac aagccagcag 420

caacaacaac cttcttctgc tactccactt ccctgggctg ttgacgattt ctttcacttc 480

tctgatattg aatccaccga caaggtagtg atgagaattg agattcaatg ttag 534

<210> 191
<211> 177
<212> PRT
<213> Arabidopsis thaliana

<400> 191

Met Lys Ile Gln Cys Asp Val Cys Glu Lys Ala Pro Ala Thr Val Ile
1 5 10 15

Cys Cys Ala Asp Glu Ala Ala Leu Cys Pro Gln Cys Asp Ile Glu Ile
20 25 30

His Ala Ala Asn Lys Leu Ala Ser Lys His Gln Arg Leu His Leu Asn
35 40 45

Ser Leu Ser Thr Lys Phe Pro Arg Cys Asp Ile Cys Gln Glu Lys Ala
50 55 60

Ala Phe Ile Phe Cys Val Glu Asp Arg Ala Leu Leu Cys Arg Asp Cys
65 70 75 80

Asp Glu Ser Ile His Val Ala Asn Ser Arg Ser Ala Asn His Gln Arg
85 90 95

Phe Leu Ala Thr Gly Ile Lys Val Ala Leu Thr Ser Thr Ile Cys Ser
100 105 110

Lys Glu Ile Glu Lys Asn Gln Pro Glu Pro Ser Asn Asn Gln Gln Lys
115 120 125

Ala Asn Gln Ile Pro Ala Lys Ser Thr Ser Gln Gln Gln Gln Gln Pro
130 135 140

Ser Ser Ala Thr Pro Leu Pro Trp Ala Val Asp Asp Phe Phe His Phe
145 150 155 160

Ser Asp Ile Glu Ser Thr Asp Lys Val Val Met Arg Ile Glu Ile Gln
165 170 175

Cys

<210> 192
<211> 747
<212> DNA
<213> Arabidopsis thaliana

<400> 192
atgaagatac agtgtgatgt gtgtgagaaa gctccggcga cggtgatttg ttgcgccgac 60

gaagctgctc tctgtcctca atgcgacatc gagattcacg ccgctaacaa actcgctagc 120

aagcaccaac gtcttcatct taattccctc tccaccaaat tccctcgttg cgatatctgc 180

caagagaagg cagctttcat tttctgtgta gaggatagag ctctgctttg cagggactgc 240

gatgaatcca tccacgtggc taattctcga tctgctaatc accagaggtt cttagccact 300

gggatcaaag tagctctgac ctcaactata tgtagtaaag aaattgagaa gaatcaacct 360

gagccttcca acaaccaaca gaaggctaat cagattcctg ctaaatccac aagccagcag 420

caacaacaac cttcttctgc tactccactt ccctgggctg ttgacgattt ctttcacttc 480

tctgatattg aatccaccga caagaaagga cagcttgatc ttggggcagg ggagttggat 540

tggttttcag acatgggatt cttcggtgat cagattaatg acaaggctct tcctgcagct 600

gaagttcctg agctttctgt ttcgcattta ggtcatgttc attcatacaa acctatgaag 660

tcaaatgttt cacacaagaa gccgaggttt gagaccagat atgatgatga tgatgaggaa 720

cacttcattg tccctgatct tggctaa 747

<210> 193
<211> 248
<212> PRT
<213> Arabidopsis thaliana

<400> 193

Met Lys Ile Gln Cys Asp Val Cys Glu Lys Ala Pro Ala Thr Val Ile
1               5                   10                  15

Cys Cys Ala Asp Glu Ala Ala Leu Cys Pro Gln Cys Asp Ile Glu Ile
            20                  25                  30

His Ala Ala Asn Lys Leu Ala Ser Lys His Gln Arg Leu His Leu Asn
        35                  40                  45

Ser Leu Ser Thr Lys Phe Pro Arg Cys Asp Ile Cys Gln Glu Lys Ala
    50                  55                  60

Ala Phe Ile Phe Cys Val Glu Asp Arg Ala Leu Leu Cys Arg Asp Cys
65                  70                  75                  80

Asp Glu Ser Ile His Val Ala Asn Ser Arg Ser Ala Asn His Gln Arg
                85                  90                  95

```
Phe Leu Ala Thr Gly Ile Lys Val Ala Leu Thr Ser Thr Ile Cys Ser
            100                 105                 110

Lys Glu Ile Glu Lys Asn Gln Pro Glu Pro Ser Asn Asn Gln Gln Lys
        115                 120                 125

Ala Asn Gln Ile Pro Ala Lys Ser Thr Ser Gln Gln Gln Gln Gln Pro
    130                 135                 140

Ser Ser Ala Thr Pro Leu Pro Trp Ala Val Asp Asp Phe Phe His Phe
145                 150                 155                 160

Ser Asp Ile Glu Ser Thr Asp Lys Lys Gly Gln Leu Asp Leu Gly Ala
                165                 170                 175

Gly Glu Leu Asp Trp Phe Ser Asp Met Gly Phe Phe Gly Asp Gln Ile
            180                 185                 190

Asn Asp Lys Ala Leu Pro Ala Ala Glu Val Pro Glu Leu Ser Val Ser
        195                 200                 205

His Leu Gly His Val His Ser Tyr Lys Pro Met Lys Ser Asn Val Ser
    210                 215                 220

His Lys Lys Pro Arg Phe Glu Thr Arg Tyr Asp Asp Asp Asp Glu Glu
225                 230                 235                 240

His Phe Ile Val Pro Asp Leu Gly
                245
```

<210> 194
<211> 996
<212> DNA
<213> Arabidopsis thaliana

<400> 194

```
atgaagatca ggtgcgacgt ctgcgataaa gaagaagcgt cggtgttttg cacggccgac      60
gaagcatctc tctgcggcgg ctgcgaccac caagtccacc acgctaacaa actcgcctct     120
aaacatctcc gtttctctct cctttatcct tcttcttcca acacctcctc tcctctctgc     180
gacatctgtc aggataaaaa agctctgttg ttctgtcaac aagatagagc tattttatgc     240
aaagattgcg attcatcgat ccacgctgcg aacgaacaca caaagaaaca cgataggttt     300
cttcttacag ggtttaagct ctctgcaaca tcgtctgttt acaaacctac ttcgaaatct     360
tcttcttctt cttcaagcaa ccaagatttc tctgtccctg gatcatcaat ctctaatcct     420
cctcctctca agaaacctct ctcagctcct cctcagagca acaagatcca accctttttcg    480
aagatcaacg gcggtgatgc gtcggtgaat cagtggggat ccacaagcac gatttctgag     540
tatttgatgg atacgttacc tggttggcac gttgaggatt tcctcgattc ctctcttcct     600
acttatggtt tctctaagag tggtgatgat gatggagtgt taccatatat ggaaccagaa     660
```

gatgacaaca acactaagag aaacaacaac aacaacaaca acaacaacaa caatacagtg 720

tcacttccat ctaagaattt agggatttgg gtccctcaga ttccacaaac tcttccttct 780

tcatacccaa atcaatactt ttctcaagac aacaacatac agtttgggat gtacaacaaa 840

gaaacatcac cagaagtagt gtcttttgct ccaatacaaa acatgaaaca acaaggacag 900

aacaacaaga gatggtatga tgatggtggc ttcactgtcc cacagatcac tcctcctcct 960

ctttcttcta ataaaaagtt tagatctttc tggtaa 996

<210> 195
<211> 331
<212> PRT
<213> Arabidopsis thaliana

<400> 195

Met Lys Ile Arg Cys Asp Val Cys Asp Lys Glu Glu Ala Ser Val Phe
1 5 10 15

Cys Thr Ala Asp Glu Ala Ser Leu Cys Gly Gly Cys Asp His Gln Val
20 25 30

His His Ala Asn Lys Leu Ala Ser Lys His Leu Arg Phe Ser Leu Leu
35 40 45

Tyr Pro Ser Ser Ser Asn Thr Ser Ser Pro Leu Cys Asp Ile Cys Gln
50 55 60

Asp Lys Lys Ala Leu Leu Phe Cys Gln Gln Asp Arg Ala Ile Leu Cys
65 70 75 80

Lys Asp Cys Asp Ser Ser Ile His Ala Ala Asn Glu His Thr Lys Lys
85 90 95

His Asp Arg Phe Leu Leu Thr Gly Val Lys Leu Ser Ala Thr Ser Ser
100 105 110

Val Tyr Lys Pro Thr Ser Lys Ser Ser Ser Ser Ser Ser Ser Asn Gln
115 120 125

Asp Phe Ser Val Pro Gly Ser Ser Ile Ser Asn Pro Pro Pro Leu Lys
130 135 140

Lys Pro Leu Ser Ala Pro Pro Gln Ser Asn Lys Ile Gln Pro Phe Ser
145 150 155 160

Lys Ile Asn Gly Gly Asp Ala Ser Val Asn Gln Trp Gly Ser Thr Ser
165 170 175

Thr Ile Ser Glu Tyr Leu Met Asp Thr Leu Pro Gly Trp His Val Glu
180 185 190

```
Asp Phe Leu Asp Ser Ser Leu Pro Thr Tyr Gly Phe Ser Lys Ser Gly
        195                 200                 205

Asp Asp Asp Gly Val Leu Pro Tyr Met Glu Pro Glu Asp Asp Asn Asn
    210                 215                 220

Thr Lys Arg Asn Asn Asn Asn Asn Asn Asn Asn Asn Asn Asn Thr Val
225                 230                 235                 240

Ser Leu Pro Ser Lys Asn Leu Gly Ile Trp Val Pro Gln Ile Pro Gln
                245                 250                 255

Thr Leu Pro Ser Ser Tyr Pro Asn Gln Tyr Phe Ser Gln Asp Asn Asn
            260                 265                 270

Ile Gln Phe Gly Met Tyr Asn Lys Glu Thr Ser Pro Glu Val Val Ser
        275                 280                 285

Phe Ala Pro Ile Gln Asn Met Lys Gln Gln Gly Gln Asn Asn Lys Arg
    290                 295                 300

Trp Tyr Asp Asp Gly Gly Phe Thr Val Pro Gln Ile Thr Pro Pro Pro
305                 310                 315                 320

Leu Ser Ser Asn Lys Lys Phe Arg Ser Phe Trp
                325                 330
```

<210> 196
<211> 900
<212> DNA
<213> Arabidopsis thaliana

<400> 196

```
atgaagattc agtgtaacgt ttgtgaggcg gcggaagcga cggttctatg ttgcgccgac     60
gaggctgctc tttgttgggc ttgcgatgag aaaattcacg ccgctaataa actcgccgga    120
aaacatcaga gagtccctct ctctgcctct gcctcttcca tacccaaatg tgacatttgt    180
caggaagcat ctggattctt cttttgtctg caagatagag ctttgctatg taggaaatgt    240
gatgttgcaa tccacactgt gaatcctcat gtttcagctc accagagatt tcttctcact    300
ggaatcaaag ttggtcttga atctatagac actggtcctt ctactaaatc ctcacctacc    360
aatgatgata aaaccatgga gaccaaacct tttgttcaat ctatacctga gcctcaaaag    420
atggccttcg atcatcatca tcaccagcag cagcaggaac agcaggaagg agttataccg    480
ggaactaaag tcaatgatca gacatcgaca aagcttcctc tcgtaagtag cggatcaact    540
actggaagca ttcctcagtg gcaaatagag gagattttcg ggctaaccga ctttgatcag    600
agctatgaat acatggagaa taatggatca tctaaggcgg atactagtag acgaggagat    660
tcagacagtt cttcgatgat gagatctgca gaagaagatg gagaagataa caataactgc    720
ttgggaggtg agacatcatg ggcggttcca cagattcagt ctccacctac agcgtctggt    780
ctaaactggc ctaagcattt tcaccaccac tctgtgtttg ttccggacat aacttcttca    840
```

actccttata ccggttcatc cccgaatcaa agggttggga aacggcggcg acggttctag 900

<210> 197
<211> 299
<212> PRT
<213> Arabidopsis thaliana

<400> 197

Met Lys Ile Gln Cys Asn Val Cys Glu Ala Ala Glu Ala Thr Val Leu
1 5 10 15

Cys Cys Ala Asp Glu Ala Ala Leu Cys Trp Ala Cys Asp Glu Lys Ile
20 25 30

His Ala Ala Asn Lys Leu Ala Gly Lys His Gln Arg Val Pro Leu Ser
35 40 45

Ala Ser Ala Ser Ser Ile Pro Lys Cys Asp Ile Cys Gln Glu Ala Ser
50 55 60

Gly Phe Phe Phe Cys Leu Gln Asp Arg Ala Leu Leu Cys Arg Lys Cys
65 70 75 80

Asp Val Ala Ile His Thr Val Asn Pro His Val Ser Ala His Gln Arg
85 90 95

Phe Leu Leu Thr Gly Ile Lys Val Gly Leu Glu Ser Ile Asp Thr Gly
100 105 110

Pro Ser Thr Lys Ser Ser Pro Thr Asn Asp Asp Lys Thr Met Glu Thr
115 120 125

Lys Pro Phe Val Gln Ser Ile Pro Glu Pro Gln Lys Met Ala Phe Asp
130 135 140

His His His His Gln Gln Gln Gln Glu Gln Gln Glu Gly Val Ile Pro
145 150 155 160

Gly Thr Lys Val Asn Asp Gln Thr Ser Thr Lys Leu Pro Leu Val Ser
165 170 175

Ser Gly Ser Thr Thr Gly Ser Ile Pro Gln Trp Gln Ile Glu Glu Ile
180 185 190

Phe Gly Leu Thr Asp Phe Asp Gln Ser Tyr Glu Tyr Met Glu Asn Asn
195 200 205

Gly Ser Ser Lys Ala Asp Thr Ser Arg Arg Gly Asp Ser Asp Ser Ser
210 215 220

Ser Met Met Arg Ser Ala Glu Glu Asp Gly Glu Asp Asn Asn Asn Cys
225 230 235 240

Leu Gly Gly Glu Thr Ser Trp Ala Val Pro Gln Ile Gln Ser Pro Pro
245 250 255

Thr Ala Ser Gly Leu Asn Trp Pro Lys His Phe His His His Ser Val
260 265 270

Phe Val Pro Asp Ile Thr Ser Ser Thr Pro Tyr Thr Gly Ser Ser Pro
275 280 285

Asn Gln Arg Val Gly Lys Arg Arg Arg Arg Phe
290 295

<210> 198

<211> 717
<212> DNA
<213> Arabidopsis thaliana

<400> 198
atgaagatac aatgtgatgt gtgtgagaaa gctccggcca cgcttatatg ttgtgctgat 60

gaagctgctc tctgcgctaa atgtgacgtt gaggttcatg ctgctaataa actcgctagc 120

aaacaccaac gcctttttct tgactctctc tcaactaaat tccctccctg cgacatctgc 180

cttgagaagg cagctttcat attctgtgta gaggataggg ctctgctctg cagagattgc 240

gatgaggcga cccatgcgcc aaatactcgc tctgctaatc accagaggtt cttagccact 300

ggaatccgag ttgctcttag ttccactagt tgcaatcaag aagtggaaaa gaatcacttt 360

gacccatcta atcagcagag tctctctaaa ccgccaactc agcaacccgc tgctccatct 420

cctttgtggg ctaccgatga attcttcagc tactctgatc ttgactgcag taataaggag 480

aaagagcaac tcgatctcgg ggagctggat tggcttgcag agatgggtct gtttggtgac 540

cagcctgatc aagaggctct accggtagcc gaagttcccg agctttcctt ttcacatttg 600

gctcatgctc attcctacaa cagacctatg aagtccaatg tacccaacaa gaagcagagg 660

cttgagtacc ggtatgatga tgaagaagag cacttcctag tccccgacct aggctaa 717

<210> 199
<211> 238
<212> PRT
<213> Arabidopsis thaliana

<400> 199

Met Lys Ile Gln Cys Asp Val Cys Glu Lys Ala Pro Ala Thr Leu Ile
1 5 10 15

Cys Cys Ala Asp Glu Ala Ala Leu Cys Ala Lys Cys Asp Val Glu Val
20 25 30

His Ala Ala Asn Lys Leu Ala Ser Lys His Gln Arg Leu Phe Leu Asp
35 40 45

Ser Leu Ser Thr Lys Phe Pro Pro Cys Asp Ile Cys Leu Glu Lys Ala
50 55 60

Ala Phe Ile Phe Cys Val Glu Asp Arg Ala Leu Leu Cys Arg Asp Cys
65 70 75 80

Asp Glu Ala Thr His Ala Pro Asn Thr Arg Ser Ala Asn His Gln Arg
85 90 95

Phe Leu Ala Thr Gly Ile Arg Val Ala Leu Ser Ser Thr Ser Cys Asn
100 105 110

Gln Glu Val Glu Lys Asn His Phe Asp Pro Ser Asn Gln Gln Ser Leu
115 120 125

Ser Lys Pro Pro Thr Gln Gln Pro Ala Ala Pro Ser Pro Leu Trp Ala
130 135 140

Thr Asp Glu Phe Phe Ser Tyr Ser Asp Leu Asp Cys Ser Asn Lys Glu
145 150 155 160

Lys Glu Gln Leu Asp Leu Gly Glu Leu Asp Trp Leu Ala Glu Met Gly
165 170 175

Leu Phe Gly Asp Gln Pro Asp Gln Glu Ala Leu Pro Val Ala Glu Val
180 185 190

Pro Glu Leu Ser Phe Ser His Leu Ala His Ala His Ser Tyr Asn Arg
195 200 205

Pro Met Lys Ser Asn Val Pro Asn Lys Lys Gln Arg Leu Glu Tyr Arg
210 215 220

Tyr Asp Asp Glu Glu Glu His Phe Leu Val Pro Asp Leu Gly
225 230 235

<210> 200
<211> 489
<212> DNA
<213> Arabidopsis thaliana

<400> 200
atgaagatac aatgtgaagt gtgtgaaaag gctgaagcag aagtactttg ttgttcagat 60
gaagctgtac tatgcaagcc atgtgacatt aaggttcatg aagctaataa acttttccaa 120
agacatcacc gagtcgcctt acaaaaagat gcagcctcag ccaccacagc ttctggagct 180
cctctatgtg atatctgcca ggagagaaaa gggtacttct tctgcttaga ggatagagca 240
atgctgtgca atgattgtga tgaagccatt cacacttgca attctcacca aagattctta 300
ctttctggag tacaagtttc tgatcagtct ttgactgaaa actccgaatg cagcactagt 360
tttagctctg aaacttacca gattcagtca aaagtgtctc tgaatagtca gtactctagt 420

gaggaaactg aagctggaaa ctcaggtgag atagtacaca agaatccttc tgtaatctta 480

agtccttag 489

<210> 201
<211> 162
<212> PRT
<213> Arabidopsis thaliana

<400> 201

Met Lys Ile Gln Cys Glu Val Cys Glu Lys Ala Glu Ala Glu Val Leu
1 5 10 15

Cys Cys Ser Asp Glu Ala Val Leu Cys Lys Pro Cys Asp Ile Lys Val
20 25 30

His Glu Ala Asn Lys Leu Phe Gln Arg His His Arg Val Ala Leu Gln
35 40 45

Lys Asp Ala Ala Ser Ala Thr Thr Ala Ser Gly Ala Pro Leu Cys Asp
50 55 60

Ile Cys Gln Glu Arg Lys Gly Tyr Phe Phe Cys Leu Glu Asp Arg Ala
65 70 75 80

Met Leu Cys Asn Asp Cys Asp Glu Ala Ile His Thr Cys Asn Ser His
85 90 95

Gln Arg Phe Leu Leu Ser Gly Val Gln Val Ser Asp Gln Ser Leu Thr
100 105 110

Glu Asn Ser Glu Cys Ser Thr Ser Phe Ser Ser Glu Thr Tyr Gln Ile
115 120 125

Gln Ser Lys Val Ser Leu Asn Ser Gln Tyr Ser Ser Glu Glu Thr Glu
130 135 140

Ala Gly Asn Ser Gly Glu Ile Val His Lys Asn Pro Ser Val Ile Leu
145 150 155 160

Ser Pro

<210> 202
<211> 729
<212> DNA
<213> Arabidopsis thaliana

<400> 202
atgaagattt ggtgtgctgt ttgtgataaa gaagaagctt cggtgttttg ttgtgcggat 60

gaagcagctc tttgtaatgg ttgcgatcgc catgttcatt tcgccaataa actagccggg 120

aaacatctcc ggttctctct cacttctcct actttcaaag atgctcctct ttgtgatatt 180

```
tgcggggaga ggcgtgcatt attattttgc caagaagaca gagcaatact atgcagagaa     240
tgtgacattc caatacatca agctaatgag cacactaaga aacacaatag attcctcctt     300
accggcgtta agatctctgc ctccccgtca gcctacccaa gagcctccaa ttccaactct     360
gctgctgcat ttggtcgagc caaaacccga ccaaaatcag tatcgagcga ggtcccgagc     420
tcggcctcca atgaggtatt tacgagctct tcttcgacga ccacgagcaa ttgctattat     480
gggatagaag aaaactacca tcacgtgagc gattcggggt cgggatcggg ttgtacaggt     540
agtatatccg agtatttgat ggagacatta ccgggttgga gagtggagga tttgcttgaa     600
cacccttctt gtgtctccta tgaggataac attattacta ataacaataa cagtgagtct     660
tatagggttt atgatggttc ttcacaattc catcatcaag ggttttggga tcacaaaccc     720
ttctcttga                                                             729
```

```
<210>  203
<211>  242
<212>  PRT
<213>  Arabidopsis thaliana

<400>  203

Met Lys Ile Trp Cys Ala Val Cys Asp Lys Glu Glu Ala Ser Val Phe
1               5                   10                  15

Cys Cys Ala Asp Glu Ala Ala Leu Cys Asn Gly Cys Asp Arg His Val
            20                  25                  30

His Phe Ala Asn Lys Leu Ala Gly Lys His Leu Arg Phe Ser Leu Thr
        35                  40                  45

Ser Pro Thr Phe Lys Asp Ala Pro Leu Cys Asp Ile Cys Gly Glu Arg
    50                  55                  60

Arg Ala Leu Leu Phe Cys Gln Glu Asp Arg Ala Ile Leu Cys Arg Glu
65                  70                  75                  80

Cys Asp Ile Pro Ile His Gln Ala Asn Glu His Thr Lys Lys His Asn
                85                  90                  95

Arg Phe Leu Leu Thr Gly Val Lys Ile Ser Ala Ser Pro Ser Ala Tyr
            100                 105                 110

Pro Arg Ala Ser Asn Ser Asn Ser Ala Ala Ala Phe Gly Arg Ala Lys
        115                 120                 125

Thr Arg Pro Lys Ser Val Ser Ser Glu Val Pro Ser Ser Ala Ser Asn
    130                 135                 140

Glu Val Phe Thr Ser Ser Ser Ser Thr Thr Thr Ser Asn Cys Tyr Tyr
145                 150                 155                 160

Gly Ile Glu Glu Asn Tyr His His Val Ser Asp Ser Gly Ser Gly Ser
```

165 170 175

Gly Cys Thr Gly Ser Ile Ser Glu Tyr Leu Met Glu Thr Leu Pro Gly
180 185 190

Trp Arg Val Glu Asp Leu Leu Glu His Pro Ser Cys Val Ser Tyr Glu
195 200 205

Asp Asn Ile Ile Thr Asn Asn Asn Asn Ser Glu Ser Tyr Arg Val Tyr
210 215 220

Asp Gly Ser Ser Gln Phe His His Gln Gly Phe Trp Asp His Lys Pro
225 230 235 240

Phe Ser

<210> 204
<211> 700
<212> DNA
<213> Medicago truncatula

<400> 204
atgaagatcc agtgtgatgc atgtcacaaa caagaggcct ccttgttttg ccctgcagat 60
gaagcagctc tctgcaatca atgtgatcgc aatatccact atgccaacaa ggtctctgct 120
aaacacaaac gcttcactct tcaccacccc acttccaaag acacccctct ctgtgatatc 180
tgcaaggaga ggcgtgcata tctattttgc aaagaagaca gagcaatact ttgcagggaa 240
tgtgacattc ctatccatga aatcaacaaa cttaccaagc aacacaacag gtttcttctc 300
acaggcgtaa agatcggtgc ttcttcttct tgttcaaatc caacaatttc caatggctca 360
gaactaagaa cctcaagtcc aagaccaagt tcattttcaa gtgaaaataa tagttgttct 420
caaagttcat tcaaagagaa catggtttgt gacacagttt caaccagtag catttccgaa 480
tacttgattg aaacgattcc tggttactgc atggaggacc tttttgatgc ttcttttgca 540
cctaataacg ttttctgtaa taaggattat tatgaacaga accaagatct tcaagttata 600
aacatgtctg actgggtacc acaatctcaa gttagattcc ctcagcttag tgctaattcg 660
aatgttccca attgattctt tagatggggt tatgaaaatg 700

<210> 205
<211> 224
<212> PRT
<213> Medicago truncatula

<400> 205

Met Lys Ile Gln Cys Asp Ala Cys His Lys Gln Glu Ala Ser Leu Phe
1 5 10 15

Cys Pro Ala Asp Glu Ala Ala Leu Cys Asn Gln Cys Asp Arg Asn Ile
20 25 30

His Tyr Ala Asn Lys Val Ser Ala Lys His Lys Arg Phe Thr Leu His
35 40 45

His Pro Thr Ser Lys Asp Thr Pro Leu Cys Asp Ile Cys Lys Glu Arg
50 55 60

Arg Ala Tyr Leu Phe Cys Lys Glu Asp Arg Ala Ile Leu Cys Arg Glu
65 70 75 80

Cys Asp Ile Pro Ile His Glu Ile Asn Lys Leu Thr Lys Gln His Asn
85 90 95

Arg Phe Leu Leu Thr Gly Val Lys Ile Gly Ala Ser Ser Ser Cys Ser
100 105 110

Asn Pro Thr Ile Ser Asn Gly Ser Glu Leu Arg Thr Ser Ser Pro Arg
115 120 125

Pro Ser Ser Phe Ser Ser Glu Asn Asn Ser Cys Ser Gln Ser Ser Phe
130 135 140

Lys Glu Asn Met Val Cys Asp Thr Val Ser Thr Ser Ser Ile Ser Glu
145 150 155 160

Tyr Leu Ile Glu Thr Ile Pro Gly Tyr Cys Met Glu Asp Leu Phe Asp
165 170 175

Ala Ser Phe Ala Pro Asn Asn Val Phe Cys Asn Lys Asp Tyr Tyr Glu
180 185 190

Gln Asn Gln Asp Leu Gln Val Ile Asn Met Ser Asp Trp Val Pro Gln
195 200 205

Ser Gln Val Arg Phe Pro Gln Leu Ser Ala Asn Ser Asn Val Pro Asn
210 215 220

<210> 206
<211> 702
<212> DNA
<213> Lycopersicon esculentum

<400> 206
atgaagatac agtgtgatgt gtgtgagaaa gctcaagcta ctgtgatttg ctgtgctgat 60

gaggctgctc tgtgtgcaaa atgtgatatt gaagttcatg ctgctaataa attagcaagc 120

aagcaccaaa ggcttcatct tcagtgccta tctaacaagc ttcctccttg tgatatttgc 180

caagataaag cagccttcat cttctgtgtt gaggatagag ctctcttttg caaggactgt 240

gacgaagcaa ttcattcagc cagcagcctc gctaagaacc accaacgctt cttagccact 300

ggaatccgtg tagccttgag ctcaagctgt aataaggaat cagtaaaaaa ccaactgcag 360

ccacaaccac ctcagcagaa ttcccaacaa gttggcttga aaatgcctcc acagcagttg 420

tcctgtataa catcaccatc ttggcctgtc gatgatttac taggatttcc agattatgag 480

tcgagtgaca agaaggatct acttgagctt ggtgaatttg agtggttagg gggcattgat 540

ctctttggtg aacaaacagc agctgaagtg cccgagctat cagtacctca gtcgagcaat 600

acaaatattt acaggacaac caaatatcaa atgccttaca agaagtccag aattgaaatc 660

ccagatgatg atgagtattt tactgtcccc gatcttggtt ga 702

<210> 207
<211> 233
<212> PRT
<213> Lycopersicon esculentum

<400> 207

```
Met Lys Ile Gln Cys Asp Val Cys Glu Lys Ala Gln Ala Thr Val Ile
1               5                   10                  15

Cys Cys Ala Asp Glu Ala Ala Leu Cys Ala Lys Cys Asp Ile Glu Val
            20                  25                  30

His Ala Ala Asn Lys Leu Ala Ser Lys His Gln Arg Leu His Leu Gln
        35                  40                  45

Cys Leu Ser Asn Lys Leu Pro Pro Cys Asp Ile Cys Gln Asp Lys Ala
    50                  55                  60

Ala Phe Ile Phe Cys Val Glu Asp Arg Ala Leu Phe Cys Lys Asp Cys
65                  70                  75                  80

Asp Glu Ala Ile His Ser Ala Ser Ser Leu Ala Lys Asn His Gln Arg
                85                  90                  95

Phe Leu Ala Thr Gly Ile Arg Val Ala Leu Ser Ser Ser Cys Asn Lys
            100                 105                 110

Glu Ser Val Lys Asn Gln Leu Gln Pro Gln Pro Pro Gln Gln Asn Ser
        115                 120                 125

Gln Gln Val Gly Leu Lys Met Pro Pro Gln Gln Leu Ser Cys Ile Thr
    130                 135                 140

Ser Pro Ser Trp Pro Val Asp Asp Leu Leu Gly Phe Pro Asp Tyr Glu
145                 150                 155                 160

Ser Ser Asp Lys Lys Asp Leu Leu Glu Leu Gly Glu Phe Glu Trp Leu
                165                 170                 175

Gly Gly Ile Asp Leu Phe Gly Glu Gln Thr Ala Ala Glu Val Pro Glu
            180                 185                 190

Leu Ser Val Pro Gln Ser Ser Asn Thr Asn Ile Tyr Arg Thr Thr Lys
        195                 200                 205
```

```
Tyr Gln Met Pro Tyr Lys Lys Ser Arg Ile Glu Ile Pro Asp Asp Asp
    210                 215                 220

Glu Tyr Phe Thr Val Pro Asp Leu Gly
225                 230

<210>  208
<211>  702
<212>  DNA
<213>  Solanum tuberosum

<400>  208
atgaagatcc agtgtgatgt gtgtgagaaa gctcaagcta ctgtgatttg ctgtgctgat      60

gaggctgctt tgtgtgcaaa atgtgatatt gaagttcatg ctgctaataa attagcaagt     120

aagcatcaaa ggcttcatct tcagtgcctg tctaacaagc ttcctccttg tgatatttgc     180

caagataaag cagccttcat cttctgtgtt gaggatagag ctctcttttg caaggactgt     240

gacgaagcaa ttcattcagc cagcagcctc gctaagaacc accaacgctt cttagccact     300

ggaatccgtg tagccttgag ctcaagctgc aataaggaag cagtaaaaaa ccaactggag     360

ccacaaccac ctcagcagaa ttcccaacaa gttggcttga aaatgcctcc acagcaattg     420

tccggtatca catcaccatc ttggcctgtt gatgatttac taggatttcc agattatgaa     480

tcgagtgaca agaaggatct acttgagctt ggtgaatttg agtggttagg aggtattgat     540

ctctttggtg aacaaacagc agctgaagta cccgagctat cagtacctca gtcaagcaac     600

acaaatattt accggacaac caaatatcaa atgccttaca agaagcccag aattgaaatc     660

ccagatgatg atgagtattt taccgtccca gatcttggtt ga                        702

<210>  209
<211>  233
<212>  PRT
<213>  Solanum tuberosum

<400>  209

Met Lys Ile Gln Cys Asp Val Cys Glu Lys Ala Gln Ala Thr Val Ile
1               5                   10                  15

Cys Cys Ala Asp Glu Ala Ala Leu Cys Ala Lys Cys Asp Ile Glu Val
            20                  25                  30

His Ala Ala Asn Lys Leu Ala Ser Lys His Gln Arg Leu His Leu Gln
        35                  40                  45

Cys Leu Ser Asn Lys Leu Pro Pro Cys Asp Ile Cys Gln Asp Lys Ala
    50                  55                  60

Ala Phe Ile Phe Cys Val Glu Asp Arg Ala Leu Phe Cys Lys Asp Cys
65                  70                  75                  80

Asp Glu Ala Ile His Ser Ala Ser Ser Leu Ala Lys Asn His Gln Arg
```

85 90 95

Phe Leu Ala Thr Gly Ile Arg Val Ala Leu Ser Ser Ser Cys Asn Lys
100 105 110

Glu Ala Val Lys Asn Gln Leu Glu Pro Gln Pro Pro Gln Gln Asn Ser
115 120 125

Gln Gln Val Gly Leu Lys Met Pro Pro Gln Gln Leu Ser Gly Ile Thr
130 135 140

Ser Pro Ser Trp Pro Val Asp Asp Leu Leu Gly Phe Pro Asp Tyr Glu
145 150 155 160

Ser Ser Asp Lys Lys Asp Leu Leu Glu Leu Gly Glu Phe Glu Trp Leu
165 170 175

Gly Gly Ile Asp Leu Phe Gly Glu Gln Thr Ala Ala Glu Val Pro Glu
180 185 190

Leu Ser Val Pro Gln Ser Ser Asn Thr Asn Ile Tyr Arg Thr Thr Lys
195 200 205

Tyr Gln Met Pro Tyr Lys Lys Pro Arg Ile Glu Ile Pro Asp Asp Asp
210 215 220

Glu Tyr Phe Thr Val Pro Asp Leu Gly
225 230

<210> 210
<211> 933
<212> DNA
<213> Populus trichocarpa

<400> 210
atgaagatcc agtgcgatgt gtgtaacaaa gaagaggcat cggtgttttg caccgctgac 60
gaggcagctc tttgcgacac ctgtgaccac cgtgttcacc atgccaacaa gcttgcttca 120
aagcaccaac gtttttccct tctccatcct tcctcaaaaa acttccccat ctgtgatatc 180
tgccaggaga aacgggcttt cttgttctgt caacaagaca gggcgatttt atgtagagag 240
tgtgatggtc caatacacac agcaaacgag catacccaga agcacaacag gtttcttctc 300
acaggagtca agctctctgc tacatctgct gtttatatat cttcttcctc tgtcaccaac 360
agtggtggtg atctcgttcc tgattcaaag tctcaacagc agcagcagca gcagcaatca 420
atcaagaaac ctgtgtttga tgctccagtg aattccaatc cacctacagt tcccagtacc 480
ttatctacaa acacagaagt aaacaagggt ggggataatt tggtaacaaa tgaagggttt 540
ggttcaacaa caagtagtac tatatcagag tacttgatgg agactcttcc tggctggcat 600
gttgaagact ttcttgattc ctctactact ccctttggtt tctgtaagat tgatgatggt 660
ctattgccgt ttatggatgc tcatgatctt gagagcaaca tgagttcttt ctcatcagaa 720

agtttggggc tttgggtccc tcaagcacca tctactccat acacatctca acagtattat 780

tatccacagt tggtagggca aagtgggttc aaggagataa aagagaccac aaacatgaaa 840

gctaacagaa ggttggcaga tgatgtcttc actgttccac agatcagcct cccggccaat 900

ataagctcta agagatctag gcccttatgg tag 933

<210> 211
<211> 310
<212> PRT
<213> Populus trichocarpa

<400> 211

Met Lys Ile Gln Cys Asp Val Cys Asn Lys Glu Glu Ala Ser Val Phe
1 5 10 15

Cys Thr Ala Asp Glu Ala Ala Leu Cys Asp Thr Cys Asp His Arg Val
20 25 30

His His Ala Asn Lys Leu Ala Ser Lys His Gln Arg Phe Ser Leu Leu
35 40 45

His Pro Ser Ser Lys Asn Phe Pro Ile Cys Asp Ile Cys Gln Glu Lys
50 55 60

Arg Ala Phe Leu Phe Cys Gln Gln Asp Arg Ala Ile Leu Cys Arg Glu
65 70 75 80

Cys Asp Gly Pro Ile His Thr Ala Asn Glu His Thr Gln Lys His Asn
85 90 95

Arg Phe Leu Leu Thr Gly Val Lys Leu Ser Ala Thr Ser Ala Val Tyr
100 105 110

Ile Ser Ser Ser Ser Val Thr Asn Ser Gly Gly Asp Leu Val Pro Asp
115 120 125

Ser Lys Ser Gln Gln Gln Gln Gln Gln Gln Gln Ser Ile Lys Lys Pro
130 135 140

Val Phe Asp Ala Pro Val Asn Ser Asn Pro Pro Thr Val Pro Ser Thr
145 150 155 160

Leu Ser Thr Asn Thr Glu Val Asn Lys Gly Gly Asp Asn Leu Val Thr
165 170 175

Asn Glu Gly Phe Gly Ser Thr Thr Ser Ser Thr Ile Ser Glu Tyr Leu
180 185 190

Met Glu Thr Leu Pro Gly Trp His Val Glu Asp Phe Leu Asp Ser Ser
195 200 205

Thr Thr Pro Phe Gly Phe Cys Lys Ile Asp Asp Gly Leu Leu Pro Phe
210 215 220

Met Asp Ala His Asp Leu Glu Ser Asn Met Ser Ser Phe Ser Ser Glu
225 230 235 240

Ser Leu Gly Leu Trp Val Pro Gln Ala Pro Ser Thr Pro Tyr Thr Ser
245 250 255

Gln Gln Tyr Tyr Tyr Pro Gln Leu Val Gly Gln Ser Gly Phe Lys Glu
260 265 270

Ile Lys Glu Thr Thr Asn Met Lys Ala Asn Arg Arg Leu Ala Asp Asp
275 280 285

Val Phe Thr Val Pro Gln Ile Ser Leu Pro Ala Asn Ile Ser Ser Lys
290 295 300

Arg Ser Arg Pro Leu Trp
305 310

<210> 212
<211> 618
<212> DNA
<213> Vitis vinifera

<400> 212
atgaagatac cgtgtgatat atgtgggaat gtggaggctg aggttctatg tagtgctgat 60
gaggcagtac tctgttgggg atgcgatgaa agggtgcaca cagctaacaa gctgtcccag 120
aagcatcaac gtgtccctct tctcaaacac ccaccctcca cttcmtcttc tcagctgcct 180
ccttgtgata tctgccagga gaaaagcggg tattttttct gcctggagga cagggcatta 240
ctctgcaaga actgtgatgt ttcaactcat tcaacaaact cttacgtgtc gtcacaccga 300
cgttttgtca tatcaggaat caaagttgct cttcaatccg taactaacaa ctacagaact 360
ggctgcaaca gcagaaccta ccctctcgat atgccaaact caaatagttc ttcagtcaac 420
ttcccaatgg atagggagaa gaagccagaa atgaccacag aagttgcatc cacatcctca 480
gacatggtag ccatgttctc aggtgaaatc catttggcaa ctggacctga atggacatta 540
gatgaaatcc ttgggagcaa tgattttgac tattatgagt tttcggacat ggggcaatcc 600
aggattagca gccaatga 618

<210> 213
<211> 205
<212> PRT
<213> Vitis vinifera

<400> 213

Met Lys Ile Pro Cys Asp Ile Cys Gly Asn Val Glu Ala Glu Val Leu
1 5 10 15

Cys Ser Ala Asp Glu Ala Val Leu Cys Trp Gly Cys Asp Glu Arg Val
20 25 30

His Thr Ala Asn Lys Leu Ser Gln Lys His Gln Arg Val Pro Leu Leu
35 40 45

Lys His Pro Pro Ser Thr Ser Ser Ser Gln Leu Pro Pro Cys Asp Ile
50 55 60

Cys Gln Glu Lys Ser Gly Tyr Phe Phe Cys Leu Glu Asp Arg Ala Leu
65 70 75 80

Leu Cys Lys Asn Cys Asp Val Ser Thr His Ser Thr Asn Ser Tyr Val
85 90 95

Ser Ser His Arg Arg Phe Val Ile Ser Gly Ile Lys Val Ala Leu Gln
100 105 110

Ser Val Thr Asn Asn Tyr Arg Thr Gly Cys Asn Ser Arg Thr Tyr Pro
115 120 125

Leu Asp Met Pro Asn Ser Asn Ser Ser Ser Val Asn Phe Pro Met Asp
130 135 140

Arg Glu Lys Lys Pro Glu Met Thr Thr Glu Val Ala Ser Thr Ser Ser
145 150 155 160

Asp Met Val Ala Met Phe Ser Gly Glu Ile His Leu Ala Thr Gly Pro
165 170 175

Glu Trp Thr Leu Asp Glu Ile Leu Gly Ser Asn Asp Phe Asp Tyr Tyr
180 185 190

Glu Phe Ser Asp Met Gly Gln Ser Arg Ile Ser Ser Gln
195 200 205

<210> 214
<211> 717
<212> DNA
<213> Glycine max

<400> 214
atgaaaattc agtgcgatgt gtgtgagaaa gccccggcaa ccgtgatttg ctgcgcagat 60

gaggcagctt tgtgtgccaa atgtgacgtt gaagttcatg ctgcaaacaa gcttgcaagc 120

aagcaccaga ggcttctcct tcaatctgta tctaacaagc ttcccagatg tgacatatgt 180

caagataagc cagctttcat attttgtgtt gaggacagag cactcttctg taaagactgt 240

gatgaaccta ttcatttagc cagtagcctt tctgcaaacc accagcgctt ccttgctact 300

ggtatccggg tggctttggg ttctaattgc accaaaggca atgaaaaagg tcacgtggaa 360

ccatctaaac caaaagcaca agaagttcct gcgaaaattc cttctcagca agtgcctagc 420

```
ttcacatcct cttgggcagt tgatgacttg ttggaattaa cagactttga atcaccagac    480
aaagttcaga agcaatccct tgagtttgga gaacttgaat ggctagcaga tgtaggcctt    540
tttggtgaac agtttcctca tgaagcttta gcggcggctg aagttcctca gcttccaatg    600
actagcagtg ttggctcaca caaagccccc aaatccttgt tgtcttacaa aaagcctagg    660
attgaagtcc tagatgaaga tgatgatgag cacttcaccg taccagatct cggataa       717

<210>  215
<211>  238
<212>  PRT
<213>  Glycine max

<400>  215

Met Lys Ile Gln Cys Asp Val Cys Glu Lys Ala Pro Ala Thr Val Ile
1               5                   10                  15

Cys Cys Ala Asp Glu Ala Ala Leu Cys Ala Lys Cys Asp Val Glu Val
            20                  25                  30

His Ala Ala Asn Lys Leu Ala Ser Lys His Gln Arg Leu Leu Leu Gln
        35                  40                  45

Ser Val Ser Asn Lys Leu Pro Arg Cys Asp Ile Cys Gln Asp Lys Pro
    50                  55                  60

Ala Phe Ile Phe Cys Val Glu Asp Arg Ala Leu Phe Cys Lys Asp Cys
65                  70                  75                  80

Asp Glu Pro Ile His Leu Ala Ser Ser Leu Ser Ala Asn His Gln Arg
                85                  90                  95

Phe Leu Ala Thr Gly Ile Arg Val Ala Leu Gly Ser Asn Cys Thr Lys
            100                 105                 110

Gly Asn Glu Lys Gly His Val Glu Pro Ser Lys Pro Lys Ala Gln Glu
        115                 120                 125

Val Pro Ala Lys Ile Pro Ser Gln Gln Val Pro Ser Phe Thr Ser Ser
    130                 135                 140

Trp Ala Val Asp Asp Leu Leu Glu Leu Thr Asp Phe Glu Ser Pro Asp
145                 150                 155                 160

Lys Val Gln Lys Gln Ser Leu Glu Phe Gly Glu Leu Glu Trp Leu Ala
                165                 170                 175

Asp Val Gly Leu Phe Gly Glu Gln Phe Pro His Glu Ala Leu Ala Ala
            180                 185                 190

Ala Glu Val Pro Gln Leu Pro Met Thr Ser Ser Val Gly Ser His Lys
        195                 200                 205
```

Ala Pro Lys Ser Leu Leu Ser Tyr Lys Lys Pro Arg Ile Glu Val Leu
    210                 215                 220

Asp Glu Asp Asp Asp Glu His Phe Thr Val Pro Asp Leu Gly
225                 230                 235

<210> 216
<211> 52
<212> DNA
<213> Artificial sequence

<220>
<223> primer 1

<400> 216
ggggacaagt ttgtacaaaa aagcaggctt aaacaatgaa ggtgcagtgc ga 52

<210> 217
<211> 49
<212> DNA
<213> Artificial sequence

<220>
<223> primer 2

<400> 217
ggggaccact ttgtacaaga aagctgggtt caccagtaca agcagggag 49

<210> 218
<211> 2194
<212> DNA
<213> Oryza sativa

<400> 218
aatccgaaaa gtttctgcac cgttttcacc ccctaactaa caatataggg aacgtgtgct 60

aaatataaaa tgagacctta tatatgtagc gctgataact agaactatgc aagaaaaact 120

catccaccta ctttagtggc aatcgggcta aataaaaaag agtcgctaca ctagtttcgt 180

tttccttagt aattaagtgg gaaaatgaaa tcattattgc ttagaatata cgttcacatc 240

tctgtcatga agttaaatta ttcgaggtag ccataattgt catcaaactc ttcttgaata 300

aaaaaatctt tctagctgaa ctcaatgggt aaagagagag attttttta aaaaaataga 360

atgaagatat tctgaacgta ttggcaaaga tttaaacata taattatata attttatagt 420

ttgtgcattc gtcatatcgc acatcattaa ggacatgtct tactccatcc caatttttat 480

ttagtaatta aagacaattg acttattttt attatttatc ttttttcgat tagatgcaag 540

gtacttacgc acacactttg tgctcatgtg catgtgtgag tgcacctcct caatacacgt 600

tcaactagca acacatctct aatatcactc gcctatttaa tacatttagg tagcaatatc 660

tgaattcaag cactccacca tcaccagacc acttttaata atatctaaaa tacaaaaaat 720

aattttacag aatagcatga aaagtatgaa acgaactatt taggtttttc acatacaaaa 780

aaaaaaagaa ttttgctcgt gcgcgagcgc caatctccca tattgggcac acaggcaaca 840

acagagtggc tgcccacaga acaacccaca aaaaacgatg atctaacgga ggacagcaag 900

tccgcaacaa ccttttaaca gcaggctttg cggccaggag agaggaggag aggcaaagaa 960
aaccaagcat cctccttctc ccatctataa attcctcccc ccttttcccc tctctatata 1020
ggaggcatcc aagccaagaa gagggagagc accaaggaca cgcgactagc agaagccgag 1080
cgaccgcctt ctcgatccat atcttccggt cgagttcttg gtcgatctct tccctcctcc 1140
acctcctcct cacagggtat gtgcctccct tcggttgttc ttggatttat tgttctaggt 1200
tgtgtagtac gggcgttgat gttaggaaag gggatctgta tctgtgatga ttcctgttct 1260
tggatttggg atagaggggt tcttgatgtt gcatgttatc ggttcggttt gattagtagt 1320
atggttttca atcgtctgga gagctctatg gaaatgaaat ggtttaggga tcggaatctt 1380
gcgattttgt gagtaccttt tgtttgaggt aaaatcagag caccggtgat tttgcttggt 1440
gtaataaagt acggttgttt ggtcctcgat tctggtagtg atgcttctcg atttgacgaa 1500
gctatccttt gtttattccc tattgaacaa aaataatcca actttgaaga cggtcccgtt 1560
gatgagattg aatgattgat tcttaagcct gtccaaaatt tcgcagctgg cttgtttaga 1620
tacagtagtc cccatcacga aattcatgga aacagttata atcctcagga acaggggatt 1680
ccctgttctt ccgatttgct ttagtcccag aatttttttt cccaaatatc ttaaaaagtc 1740
actttctggt tcagttcaat gaattgattg ctacaaataa tgcttttata gcgttatcct 1800
agctgtagtt cagttaatag gtaatacccc tatagtttag tcaggagaag aacttatccg 1860
atttctgatc tccattttta attatatgaa atgaactgta gcataagcag tattcatttg 1920
gattattttt tttattagct ctcacccctt cattattctg agctgaaagt ctggcatgaa 1980
ctgtcctcaa ttttgttttc aaattcacat cgattatcta tgcattatcc tcttgtatct 2040

acctgtagaa gtttcttttt ggttattcct tgactgcttg attacagaaa gaaatttatg 2100
aagctgtaat cgggatagtt atactgcttg ttcttatgat tcatttcctt tgtgcagttc 2160
ttggtgtagc ttgccacttt caccagcaaa gttc 2194

<210> 219
<211> 47
<212> PRT
<213> Artificial sequence

<220>
<223> B-box 1 in SEQ ID NO: 2

<400> 219

Met Lys Val Gln Cys Asp Val Cys Ala Ala Glu Ala Ala Ser Val Phe
1 5 10 15

Cys Cys Ala Asp Glu Ala Ala Leu Cys Asp Ala Cys Asp Arg Arg Val
20 25 30

His Ser Ala Asn Lys Leu Ala Gly Lys His Arg Arg Phe Ser Leu
35 40 45

<210> 220

<211> 48
<212> PRT
<213> Artificial sequence

<220>
<223> B-box 2 in SEQ ID NO: 2

<400> 220

Gln Lys Pro Pro Leu Cys Asp Ile Cys Gln Glu Lys Arg Gly Phe Leu
1 5 10 15

Phe Cys Lys Glu Asp Arg Ala Ile Leu Cys Arg Glu Cys Asp Val Thr
20 25 30

Val His Thr Thr Ser Glu Leu Thr Arg Arg His Gly Arg Phe Leu Leu
35 40 45

<210> 221
<211> 24
<212> PRT
<213> Artificial sequence

<220>
<223> consensus sequence of B-box

<220>
<221> UNSURE
<222> (2)..(3)
<223> Xaa can be any naturally occurring amino acid

<220>
<221> UNSURE
<222> (5)..(20)
<223> Xaa can be any naturally occurring amino acid

<220>
<221> UNSURE
<222> (22)..(23)
<223> Xaa can be any naturally occurring amino acid

<400> 221

Cys Xaa Xaa Cys Xaa Xaa Xaa Xaa Xaa Xaa Xaa Xaa Xaa Xaa Xaa Xaa
1 5 10 15

Xaa Xaa Xaa Xaa Cys Xaa Xaa Cys
20

<210> 222
<211> 1245
<212> DNA
<213> Zea mays

<400> 222
cccgtccggt ctagctagga ctagtaagta gcataccatt accatcctcc gcgcggctag 60

ctccgactcg ctttcatcca tacgattgcc cggagctcaa gccctgagct gagccgccag 120

agcgcgcgcg atcatcgctt gagggaaaag cagagtggag tggccgagat gaaggtgcag 180

```
tgcgacgtgt gcgcggccga ggcggccgag gtgttctgct gcgccgacga ggcggcgctg     240
tgcgacgcgt gcgaccgccg cgtgcaccgc gccaacaagc tcgccggcaa gcaccgccgc     300
ttctcgctgc tcagcccggc gccaccgccg ccgccgccgc tctgcgacat ctgccaggac     360
aagcgggggc tcctgttctg caaggaggac cgcgccatcc tgtgccgcga ctgcgacgtg     420
tcggtgcaca cggccagcga cctgaccatg cgccacgccc ggttcctgct cacgggcgtc     480
cgcctctccg ccgagcccgc cgccgcgtgc ccggcgccgg aggatgagga ggaggaggac     540
gacgagaaca gcagcggcag cttctgctgc agcgccggcg acgcagccgc acatcctcct     600
cctcttccgt cgtcggcgcc cgccaccagc cacgggagcg acagcagcag catctccgag     660
tacctcacca agacgctgcc cgggtggcac gtcgaggact tcctcattga cgacgcgtcc     720
gctggcgacg tcggcgcctg ctcggatggc ctctatcagg gacaaaatgg acagatcagt     780
ggggtgctgc aagaagctta cctgccgtgg acggagcggg agcaggtgca aactgacgtc     840
gcagacgagc gggccagctg ggagcggtgg gtgccacaga tgcatgcgga gttcggcggc     900
ggcggcaagc gacctagagc gtcgccttcg cctccctgtt cgtactggtg attggtgaaa     960
agttcgccgt cttcagcaag atgatttgta ttgggctaat tagtagagct actgtaggaa    1020
cgaccgaaaa atgagccaaa aaagtaacgg cctccatcga gctcgtttta aattattctc    1080
tgcctaaagc caaactttca gaaaggagaa cggtattttc ctgttcgatt aattaggtcg    1140
atttcgatcg aactcgttcc aaaatcccaa ttgtttgcta cctaaagaca gacttcctga    1200
atatagttga gtaacagggg aatttgccga aggaaaaaaa aaaaa                    1245

<210>  223
<211>  260
<212>  PRT
<213>  Zea mays

<400>  223

Met Lys Val Gln Cys Asp Val Cys Ala Ala Glu Ala Ala Glu Val Phe
1               5                   10                  15

Cys Cys Ala Asp Glu Ala Ala Leu Cys Asp Ala Cys Asp Arg Arg Val
            20                  25                  30

His Arg Ala Asn Lys Leu Ala Gly Lys His Arg Arg Phe Ser Leu Leu
        35                  40                  45

Ser Pro Ala Pro Pro Pro Pro Pro Pro Leu Cys Asp Ile Cys Gln Asp
    50                  55                  60

Lys Arg Gly Leu Leu Phe Cys Lys Glu Asp Arg Ala Ile Leu Cys Arg
65                  70                  75                  80

Asp Cys Asp Val Ser Val His Thr Ala Ser Asp Leu Thr Met Arg His
                85                  90                  95

Ala Arg Phe Leu Leu Thr Gly Val Arg Leu Ser Ala Glu Pro Ala Ala
```

100 105 110

Ala Cys Pro Ala Pro Glu Asp Glu Glu Glu Glu Asp Asp Glu Asn Ser
115 120 125

Ser Gly Ser Phe Cys Cys Ser Ala Gly Asp Ala Ala Ala His Pro Pro
130 135 140

Pro Leu Pro Ser Ser Ala Pro Ala Thr Ser His Gly Ser Asp Ser Ser
145 150 155 160

Ser Ile Ser Glu Tyr Leu Thr Lys Thr Leu Pro Gly Trp His Val Glu
165 170 175

Asp Phe Leu Ile Asp Asp Ala Ser Ala Gly Asp Val Gly Ala Cys Ser
180 185 190

Asp Gly Leu Tyr Gln Gly Gln Asn Gly Gln Ile Ser Gly Val Leu Gln
195 200 205

Glu Ala Tyr Leu Pro Trp Thr Glu Arg Glu Gln Val Gln Thr Asp Val
210 215 220

Ala Asp Glu Arg Ala Ser Trp Glu Arg Trp Val Pro Gln Met His Ala
225 230 235 240

Glu Phe Gly Gly Gly Gly Lys Arg Pro Arg Ala Ser Pro Ser Pro Pro
245 250 255

Cys Ser Tyr Trp
260

<210> 224
<211> 1065
<212> DNA
<213> Oryza sativa

<400> 224
atggtttcgg ccttgtttcg gacgatcctg gtgacgggcg gcgccggcta catcggcagc 60
cacaccgtcc tccagcttct ccaactcggc ttccgcgttg tcgtcctcga caacctcgac 120
aacgcctccg agctcgccat cctccgcgtc agggaactcg ccggacacaa cgccaacaac 180
ctcgacttcc gcaaggttga cctccgcgac aagcaagcgt tggaccaaat cttctcctct 240
caaaggtttg aggctgtcat ccattttgcc gggctgaaag ctgttggcga gagcgtgcag 300
aagcccctgc tttactacga caacaacctc atcggcacca tcactctcct gcaggtcatg 360
gccgcacatg gctgcaccaa gctggtgttc tcatcatccg caactgtcta cgggtggccc 420
aaggaggtgc cctgcactga agaatcccca ctttgtgcaa tgaaccccta cggcagaaca 480
aagctggtaa tcgaagacat gtgccgggat ctgcatgcct cagacccaaa ctggaagatc 540
atactgctcc gatacttcaa ccctgttgga gctcacccaa gcgggtacat tggtgaggac 600

```
ccctgcggca tcccaaacaa cctcatgccc ttcgtccagc aggtcgctgt tggcaggagg     660
ccggccctta ccgtctatgg aaccgactac aacaccaagg atggaactgg ggttcgtgac     720
tatatccatg ttgttgatct agcggatggt catatcgccg cgttaaggaa gctctatgaa     780
gattctgata gaataggatg tgaggtgtac aatctgggca ctggaaaggg gacatctgtg     840
ctggaaatgg ttgcagcatt cgagaaagct tctggaaaga aaatcccgct tgtatttgct     900
ggacgaaggc ctggagatgc cgagatcgtt tacgctcaaa ctgccaaagc tgagaaggaa     960
ctgaaatgga aggcaaaata cggggtagag gagatgtgca gggacctgtg gaattgggcg    1020
agcaagaacc cctacgggta tggatcgccg gacagtagca actga                    1065
```

```
<210>  225
<211>  354
<212>  PRT
<213>  Oryza sativa

<400>  225

Met Val Ser Ala Leu Phe Arg Thr Ile Leu Val Thr Gly Gly Ala Gly
1               5                   10                  15

Tyr Ile Gly Ser His Thr Val Leu Gln Leu Leu Gln Leu Gly Phe Arg
            20                  25                  30

Val Val Val Leu Asp Asn Leu Asp Asn Ala Ser Glu Leu Ala Ile Leu
        35                  40                  45

Arg Val Arg Glu Leu Ala Gly His Asn Ala Asn Asn Leu Asp Phe Arg
    50                  55                  60

Lys Val Asp Leu Arg Asp Lys Gln Ala Leu Asp Gln Ile Phe Ser Ser
65                  70                  75                  80

Gln Arg Phe Glu Ala Val Ile His Phe Ala Gly Leu Lys Ala Val Gly
                85                  90                  95

Glu Ser Val Gln Lys Pro Leu Leu Tyr Tyr Asp Asn Asn Leu Ile Gly
            100                 105                 110

Thr Ile Thr Leu Leu Gln Val Met Ala Ala His Gly Cys Thr Lys Leu
        115                 120                 125

Val Phe Ser Ser Ser Ala Thr Val Tyr Gly Trp Pro Lys Glu Val Pro
    130                 135                 140

Cys Thr Glu Glu Ser Pro Leu Cys Ala Met Asn Pro Tyr Gly Arg Thr
145                 150                 155                 160

Lys Leu Val Ile Glu Asp Met Cys Arg Asp Leu His Ala Ser Asp Pro
                165                 170                 175
```

Asn Trp Lys Ile Ile Leu Leu Arg Tyr Phe Asn Pro Val Gly Ala His
180 185 190

Pro Ser Gly Tyr Ile Gly Glu Asp Pro Cys Gly Ile Pro Asn Asn Leu
195 200 205

Met Pro Phe Val Gln Gln Val Ala Val Gly Arg Arg Pro Ala Leu Thr
210 215 220

Val Tyr Gly Thr Asp Tyr Asn Thr Lys Asp Gly Thr Gly Val Arg Asp
225 230 235 240

Tyr Ile His Val Val Asp Leu Ala Asp Gly His Ile Ala Ala Leu Arg
245 250 255

Lys Leu Tyr Glu Asp Ser Asp Arg Ile Gly Cys Glu Val Tyr Asn Leu
260 265 270

Gly Thr Gly Lys Gly Thr Ser Val Leu Glu Met Val Ala Ala Phe Glu
275 280 285

Lys Ala Ser Gly Lys Lys Ile Pro Leu Val Phe Ala Gly Arg Arg Pro
290 295 300

Gly Asp Ala Glu Ile Val Tyr Ala Gln Thr Ala Lys Ala Glu Lys Glu
305 310 315 320

Leu Lys Trp Lys Ala Lys Tyr Gly Val Glu Glu Met Cys Arg Asp Leu
325 330 335

Trp Asn Trp Ala Ser Lys Asn Pro Tyr Gly Tyr Gly Ser Pro Asp Ser
340 345 350

Ser Asn

<210> 226
<211> 1176
<212> DNA
<213> Oryza sativa

<400> 226
atggacttcg gtgattctag acgcaagcct aatgtcgtcg ggaagttcac cgtagctgtt 60

gcactcacgg tcatgtgcat cattgtgttg aaacagtcgc ctggttttac cagtacaagt 120

gtgttctctc gccatgaaat tggtgtgact catgtgttgg tgactggagg agctggatac 180

attgggtcac atgctactct tcgtctactt agggacaact accgagttac cattgtggat 240

aacctttcta gagggaacat gggagctgtc agagtccttc aacgtttgtt tccagagcct 300

gggaggcttc agtttattta tgctgattta ggcgatgcga aagctgttaa caaaatattt 360

tcagaaaatg cattcgatgc tgttatgcac tttgctgccg ttgcttacgt tggtgagagc 420

acgttggagc cactgaggta ctaccacaac ataacatcaa atacattgac agtgcttgag 480

gcaatggcag catataatgt aaaaactctg atatactcaa gtacttgtgc aacatatggt 540

gaacctgaca caatgcctat cacagaagca acccctcaga atcctatcaa tccatatggg 600

aaggcaaaaa agatggcaga ggacatcatt ttagatttct ctaagagatc agaaatggca 660

gtcatgatct taagatactt caacgtcatt ggatcagacc caggggggcg gttaggggaa 720

gctccacgac cagagctgcg tgagcatgga cggatttctg gtgcttgctt tgatgcagca 780

ctgggaatca ttccaggttt gaaggttcgg ggaactgatt accctacagc tgatggaact 840

tgcataaggg actatattga tgtcacggat ctcgttgatg ctcatgtgaa agctcttgat 900

aaagctcagc ctggcaaagt tggaatctac aatgttggca caggacatgg tagatcagtg 960

aaggagtttg tagaagcatg taagagcgca acaggagcta gcatcaaggt ttcattcctt 1020

acccggagac caggagacta tgctgaagtt tacagtgacc catccaaaat ccatgacgag 1080

ctgaactgga cagcccgtta cattgatctt cgcgaaagcc tttcaactgc atggaaatgg 1140

cagaaagcac acccgaatgg gtatggatcg gcctga 1176

<210> 227
<211> 391
<212> PRT
<213> Oryza sativa

<400> 227

```
Met Asp Phe Gly Asp Ser Arg Arg Lys Pro Asn Val Val Gly Lys Phe
1               5                   10                  15

Thr Val Ala Val Ala Leu Thr Val Met Cys Ile Ile Val Leu Lys Gln
            20                  25                  30

Ser Pro Gly Phe Thr Ser Thr Ser Val Phe Ser Arg His Glu Ile Gly
        35                  40                  45

Val Thr His Val Leu Val Thr Gly Gly Ala Gly Tyr Ile Gly Ser His
    50                  55                  60

Ala Thr Leu Arg Leu Leu Arg Asp Asn Tyr Arg Val Thr Ile Val Asp
65                  70                  75                  80

Asn Leu Ser Arg Gly Asn Met Gly Ala Val Arg Val Leu Gln Arg Leu
                85                  90                  95

Phe Pro Glu Pro Gly Arg Leu Gln Phe Ile Tyr Ala Asp Leu Gly Asp
            100                 105                 110

Ala Lys Ala Val Asn Lys Ile Phe Ser Glu Asn Ala Phe Asp Ala Val
        115                 120                 125

Met His Phe Ala Ala Val Ala Tyr Val Gly Glu Ser Thr Leu Glu Pro
    130                 135                 140
```

Leu Arg Tyr Tyr His Asn Ile Thr Ser Asn Thr Leu Thr Val Leu Glu
145 150 155 160

Ala Met Ala Ala Tyr Asn Val Lys Thr Leu Ile Tyr Ser Ser Thr Cys
165 170 175

Ala Thr Tyr Gly Glu Pro Asp Thr Met Pro Ile Thr Glu Ala Thr Pro
180 185 190

Gln Asn Pro Ile Asn Pro Tyr Gly Lys Ala Lys Lys Met Ala Glu Asp
195 200 205

Ile Ile Leu Asp Phe Ser Lys Arg Ser Glu Met Ala Val Met Ile Leu
210 215 220

Arg Tyr Phe Asn Val Ile Gly Ser Asp Pro Gly Gly Arg Leu Gly Glu
225 230 235 240

Ala Pro Arg Pro Glu Leu Arg Glu His Gly Arg Ile Ser Gly Ala Cys
245 250 255

Phe Asp Ala Ala Leu Gly Ile Ile Pro Gly Leu Lys Val Arg Gly Thr
260 265 270

Asp Tyr Pro Thr Ala Asp Gly Thr Cys Ile Arg Asp Tyr Ile Asp Val
275 280 285

Thr Asp Leu Val Asp Ala His Val Lys Ala Leu Asp Lys Ala Gln Pro
290 295 300

Gly Lys Val Gly Ile Tyr Asn Val Gly Thr Gly His Gly Arg Ser Val
305 310 315 320

Lys Glu Phe Val Glu Ala Cys Lys Ser Ala Thr Gly Ala Ser Ile Lys
325 330 335

Val Ser Phe Leu Thr Arg Arg Pro Gly Asp Tyr Ala Glu Val Tyr Ser
340 345 350

Asp Pro Ser Lys Ile His Asp Glu Leu Asn Trp Thr Ala Arg Tyr Ile
355 360 365

Asp Leu Arg Glu Ser Leu Ser Thr Ala Trp Lys Trp Gln Lys Ala His
370 375 380

Pro Asn Gly Tyr Gly Ser Ala
385 390

<210> 228
<211> 1065
<212> DNA

<213> Oryza sativa

<400> 228
atggtttcgg ccttgttgcg gacgatcctg gtgacgggcg gcgccggcta catcggcagc 60
cacaccgtcc tccagcttct ccaactcggc ttccgcgttg tcgtcctcga caacctcgac 120
aacgcctccg agctcgccat cctccgcgtc agggaactcg ccggacacaa cgccaacaac 180
ctcgacttcc gcaaggttga cctccgcgac aagcaagcgt tggaccaaat cttctcctct 240
caaaggtttg aggctgtcat ccattttgcc gggctgaaag ctgttggcga gagcgtgcag 300
aagcccctgc tttactacga caacaacctc atcggcacca tcactctcct gcaggtcatg 360
gccgcacatg gctgcaccaa gctggtgttc tcatcatccg caactgtcta cgggtggccc 420
aaggaggtgc cctgcactga agaatcccca ctttgtgcaa tgaaccccta cggcagaaca 480
aagctggtaa tcgaagacat gtgccgggat ctgcatgcct cagacccaaa ctggaagatc 540
atactgctcc gatacttcaa ccctgttgga gctcacccaa gcgggtacat tggtgaggac 600
ccctgcggca tcccaaacaa cctcatgccc ttcgtccagc aggtcgctgt tggcaggagg 660
ccggccctta ccgtctatgg aaccgactac aacaccaagg atggaactgg ggttcgtgac 720
tatatccatg ttgttgatct agcggatggt catatcgccg cgttaaggaa gctctatgaa 780
gattctgata gaataggatg tgaggtgtac aatctgggca ctggaaaggg gacatctgtg 840
ctggaaatgg ttgcagcatt cgagaaagct tctggaaaga aaatcccgct tgtatttgct 900
ggacgaaggc ctggagatgc cgagatcgtt tacgctcaaa ctgccaaagc tgagaaggaa 960
ctgaaatgga aggcaaaata cggggtagag gagatgtgca gggacctgtg gaattgggcg 1020
agcaagaacc cctacgggta tggatcgccg gacagtagca actga 1065

<210> 229
<211> 354
<212> PRT
<213> Oryza sativa

<400> 229

Met Val Ser Ala Leu Leu Arg Thr Ile Leu Val Thr Gly Gly Ala Gly
1 5 10 15

Tyr Ile Gly Ser His Thr Val Leu Gln Leu Leu Gln Leu Gly Phe Arg
20 25 30

Val Val Val Leu Asp Asn Leu Asp Asn Ala Ser Glu Leu Ala Ile Leu
35 40 45

Arg Val Arg Glu Leu Ala Gly His Asn Ala Asn Asn Leu Asp Phe Arg
50 55 60

Lys Val Asp Leu Arg Asp Lys Gln Ala Leu Asp Gln Ile Phe Ser Ser
65 70 75 80

Gln Arg Phe Glu Ala Val Ile His Phe Ala Gly Leu Lys Ala Val Gly
85 90 95

Glu Ser Val Gln Lys Pro Leu Leu Tyr Tyr Asp Asn Asn Leu Ile Gly
100 105 110

Thr Ile Thr Leu Leu Gln Val Met Ala Ala His Gly Cys Thr Lys Leu
115 120 125

Val Phe Ser Ser Ser Ala Thr Val Tyr Gly Trp Pro Lys Glu Val Pro
130 135 140

Cys Thr Glu Glu Ser Pro Leu Cys Ala Met Asn Pro Tyr Gly Arg Thr
145 150 155 160

Lys Leu Val Ile Glu Asp Met Cys Arg Asp Leu His Ala Ser Asp Pro
165 170 175

Asn Trp Lys Ile Ile Leu Leu Arg Tyr Phe Asn Pro Val Gly Ala His
180 185 190

Pro Ser Gly Tyr Ile Gly Glu Asp Pro Cys Gly Ile Pro Asn Asn Leu
195 200 205

Met Pro Phe Val Gln Gln Val Ala Val Gly Arg Arg Pro Ala Leu Thr
210 215 220

Val Tyr Gly Thr Asp Tyr Asn Thr Lys Asp Gly Thr Gly Val Arg Asp
225 230 235 240

Tyr Ile His Val Val Asp Leu Ala Asp Gly His Ile Ala Ala Leu Arg
245 250 255

Lys Leu Tyr Glu Asp Ser Asp Arg Ile Gly Cys Glu Val Tyr Asn Leu
260 265 270

Gly Thr Gly Lys Gly Thr Ser Val Leu Glu Met Val Ala Ala Phe Glu
275 280 285

Lys Ala Ser Gly Lys Lys Ile Pro Leu Val Phe Ala Gly Arg Arg Pro
290 295 300

Gly Asp Ala Glu Ile Val Tyr Ala Gln Thr Ala Lys Ala Glu Lys Glu
305 310 315 320

Leu Lys Trp Lys Ala Lys Tyr Gly Val Glu Glu Met Cys Arg Asp Leu
325 330 335

Trp Asn Trp Ala Ser Lys Asn Pro Tyr Gly Tyr Gly Ser Pro Asp Ser
340 345 350

Ser Asn

<210> 230
<211> 1266
<212> DNA
<213> Oryza sativa

<400> 230

```
atgcttccca ccaacaggaa caggccccag cagaggcctg ccagatcttg gtatttcatc      60
tcagacatgg acttctccga tccgaagcgc aagccgcggt acctcagtaa gatcctcatg     120
gtggcgctcc tcaccgccat gtgcgtcgtc atgctcacac agccgccctg ccataggagg     180
actcccagtg tgttctccat ccatgaaccc ggagtcacgc atgttctagt gacgggcggc     240
gctggatata tcggttcgca cgctgcgctt cggctgctga aagattcctt cagagtcacc     300
atagtggata atctttcaag aggaaatatg ggggcaatca aggttcttca gaacttgttt     360
tcagagcctg ggcggttgca gttcatctat gctgatttag gcgatccaaa agctgttaat     420
agaatatttg cagaaaatgc ttttgatgct gtcatgcact ttgctgctgt cgcttatgtt     480
ggtgagagca cacttgagcc tttgaggtac tatcataaca tcacctcaaa cactttagtt     540
gtgctagagg ctatggcagc acacaatgtg agaaccctga tctactctag cacatgtgcc     600
acctatggag aacccgagaa gatgcctatc accgaaggaa ctccccagtt tccgatcaac     660
ccatatggta aagccaagaa gatggcagag gacatcatct tggatttttc caaatccaag     720
aaggcagaca tggctgtgat gattctaaga tacttcaacg tcattggttc tgaccctgaa     780
ggaaggcttg gcgaggctcc aaaacctgaa ttacgggagc atggccgtat atctggtgca     840
tgctttgatg ctgcactggg gataattcct ggtttgaagg ttaaaggtac tgactatgaa     900
acacctgacg gtacttgtgt aagagattac atcgatgtca ctgacctcgt tgacgcccat     960
gtgaaggcgc tcaacaaggc ggaaaggggc aaagttggca tatacaatgt tggcacaggc    1020
aaaggtaggt cagtgaaaga atttgtcgaa gcttgcaaga aggcaacagg agttgacatc    1080
aaggtcgact acttccctcg tcgacccggt gactatgccg aagtgtacag cgatcccgca    1140
aagatcaaca gcgagctgaa ctggactgct caacataccg atctcctgga gagcctcagg    1200
gttgcctgga catggcagaa gaagcaccgg agcggctacg gaccccctca ggccatggtt    1260
ttgtga                                                               1266
```

<210> 231
<211> 421
<212> PRT
<213> Oryza sativa

<400> 231

```
Met Leu Pro Thr Asn Arg Asn Arg Pro Gln Gln Arg Pro Ala Arg Ser
1               5                   10                  15

Trp Tyr Phe Ile Ser Asp Met Asp Phe Ser Asp Pro Lys Arg Lys Pro
            20                  25                  30

Arg Tyr Leu Ser Lys Ile Leu Met Val Ala Leu Leu Thr Ala Met Cys
        35                  40                  45
```

Val Val Met Leu Thr Gln Pro Pro Cys His Arg Arg Thr Pro Ser Val
50 55 60

Phe Ser Ile His Glu Pro Gly Val Thr His Val Leu Val Thr Gly Gly
65 70 75 80

Ala Gly Tyr Ile Gly Ser His Ala Ala Leu Arg Leu Leu Lys Asp Ser
85 90 95

Phe Arg Val Thr Ile Val Asp Asn Leu Ser Arg Gly Asn Met Gly Ala
100 105 110

Ile Lys Val Leu Gln Asn Leu Phe Ser Glu Pro Gly Arg Leu Gln Phe
115 120 125

Ile Tyr Ala Asp Leu Gly Asp Pro Lys Ala Val Asn Arg Ile Phe Ala
130 135 140

Glu Asn Ala Phe Asp Ala Val Met His Phe Ala Ala Val Ala Tyr Val
145 150 155 160

Gly Glu Ser Thr Leu Glu Pro Leu Arg Tyr Tyr His Asn Ile Thr Ser
165 170 175

Asn Thr Leu Val Val Leu Glu Ala Met Ala Ala His Asn Val Arg Thr
180 185 190

Leu Ile Tyr Ser Ser Thr Cys Ala Thr Tyr Gly Glu Pro Glu Lys Met
195 200 205

Pro Ile Thr Glu Gly Thr Pro Gln Phe Pro Ile Asn Pro Tyr Gly Lys
210 215 220

Ala Lys Lys Met Ala Glu Asp Ile Ile Leu Asp Phe Ser Lys Ser Lys
225 230 235 240

Lys Ala Asp Met Ala Val Met Ile Leu Arg Tyr Phe Asn Val Ile Gly
245 250 255

Ser Asp Pro Glu Gly Arg Leu Gly Glu Ala Pro Lys Pro Glu Leu Arg
260 265 270

Glu His Gly Arg Ile Ser Gly Ala Cys Phe Asp Ala Ala Leu Gly Ile
275 280 285

Ile Pro Gly Leu Lys Val Lys Gly Thr Asp Tyr Glu Thr Pro Asp Gly
290 295 300

Thr Cys Val Arg Asp Tyr Ile Asp Val Thr Asp Leu Val Asp Ala His
305 310 315 320

Val Lys Ala Leu Asn Lys Ala Glu Arg Gly Lys Val Gly Ile Tyr Asn
325 330 335

Val Gly Thr Gly Lys Gly Arg Ser Val Lys Glu Phe Val Glu Ala Cys
340 345 350

Lys Lys Ala Thr Gly Val Asp Ile Lys Val Asp Tyr Phe Pro Arg Arg
355 360 365

Pro Gly Asp Tyr Ala Glu Val Tyr Ser Asp Pro Ala Lys Ile Asn Ser
370 375 380

Glu Leu Asn Trp Thr Ala Gln His Thr Asp Leu Leu Glu Ser Leu Arg
385 390 395 400

Val Ala Trp Thr Trp Gln Lys Lys His Arg Ser Gly Tyr Gly Pro Pro
405 410 415

Gln Ala Met Val Leu
420

<210> 232
<211> 1227
<212> DNA
<213> Oryza sativa

<400> 232
atggcggtgg agaagacggt gccgggcggg gtgaggacgg tgctggtgac gggcggggcg 60
gggtacatcg gcagccacgc cgtgctgcag ctcctcctcg ccggattccg cgccgtcgtc 120
gtcgacaacc tcaacaactc ctccgagctc gccgtccgcc gcgtcgccgc cctcgccggc 180
gaccactccc ggaacctcgc cttccacaag gttgacctcc gtgacaaggg agcactggaa 240
aaggtttttg cttcaacaag attcgatgct gtagttcact ttgctgggct aaaagctgtg 300
ggcgaaagtg tgcagaagcc attgctttat tacgacaaca atgttaatgg aacagtaaat 360
cttctggaag ttatgtctgc ccatggatgt aagaagttgg tgttctcatc atcagctgca 420
gtttatggat caccgaagaa ctcaccctgc acagaagagt ttcctctcac tccaaacaat 480
ccatatggca aaacaaagct tgttgttgaa gatatttgcc gtgatatcta ccgtacagat 540
cctgaatgga agattattct acttaggtac ttcaatccag ttggggctca tcctagcgga 600
taccttgggg aagatccatg tggcattccc aacaatctta tgccttatgt tcagcaagtt 660
gctgttggca ggaggccagc tctgacaata ctaggaaatg attatgcaac cagagatggt 720
accggggtcc gagattacat ccatgtggtt gaccttgctg atggacatat tgcggcattg 780
cagaaactct ttgagagctc cagcataggg tgtgaagcat acaaccttgg aactggtaaa 840
ggtacctctg tgctggagat agtcaaggca tttgagaagg cttctgggaa gaaaattcct 900
ctgattattg gcccaagacg ccctggtgat gcggagattc tattttcatt acctgctaaa 960
gcagagaagg aactcaactg gaaagcgaaa tttggcatcg atgaaatgtg tagggatcaa 1020

tggaactggg ctagcaagaa cccttatgga tacgggtcac ttgactccac caagcagaac 1080

ggacaccact cgtacggatc aattggctct cccaagcaga atggccactg cacaaatgga 1140

ttttctgaat ccactaggca taatggtcac aacggatatg gactggtcga ctctgccaag 1200

cataacggca acggccactt ccactga 1227

<210> 233
<211> 408
<212> PRT
<213> Oryza sativa

<400> 233

```
Met Ala Val Glu Lys Thr Val Pro Gly Gly Val Arg Thr Val Leu Val
1               5                   10                  15

Thr Gly Gly Ala Gly Tyr Ile Gly Ser His Ala Val Leu Gln Leu Leu
            20                  25                  30

Leu Ala Gly Phe Arg Ala Val Val Val Asp Asn Leu Asn Asn Ser Ser
        35                  40                  45

Glu Leu Ala Val Arg Arg Val Ala Ala Leu Ala Gly Asp His Ser Arg
    50                  55                  60

Asn Leu Ala Phe His Lys Val Asp Leu Arg Asp Lys Gly Ala Leu Glu
65                  70                  75                  80

Lys Val Phe Ala Ser Thr Arg Phe Asp Ala Val Val His Phe Ala Gly
                85                  90                  95

Leu Lys Ala Val Gly Glu Ser Val Gln Lys Pro Leu Leu Tyr Tyr Asp
            100                 105                 110

Asn Asn Val Asn Gly Thr Val Asn Leu Leu Glu Val Met Ser Ala His
        115                 120                 125

Gly Cys Lys Lys Leu Val Phe Ser Ser Ser Ala Ala Val Tyr Gly Ser
    130                 135                 140

Pro Lys Asn Ser Pro Cys Thr Glu Glu Phe Pro Leu Thr Pro Asn Asn
145                 150                 155                 160

Pro Tyr Gly Lys Thr Lys Leu Val Val Glu Asp Ile Cys Arg Asp Ile
                165                 170                 175

Tyr Arg Thr Asp Pro Glu Trp Lys Ile Ile Leu Leu Arg Tyr Phe Asn
            180                 185                 190

Pro Val Gly Ala His Pro Ser Gly Tyr Leu Gly Glu Asp Pro Cys Gly
        195                 200                 205
```

```
Ile Pro Asn Asn Leu Met Pro Tyr Val Gln Gln Val Ala Val Gly Arg
    210                 215                 220

Arg Pro Ala Leu Thr Ile Leu Gly Asn Asp Tyr Ala Thr Arg Asp Gly
225                 230                 235                 240

Thr Gly Val Arg Asp Tyr Ile His Val Val Asp Leu Ala Asp Gly His
                245                 250                 255

Ile Ala Ala Leu Gln Lys Leu Phe Glu Ser Ser Ser Ile Gly Cys Glu
            260                 265                 270

Ala Tyr Asn Leu Gly Thr Gly Lys Gly Thr Ser Val Leu Glu Ile Val
        275                 280                 285

Lys Ala Phe Glu Lys Ala Ser Gly Lys Lys Ile Pro Leu Ile Ile Gly
    290                 295                 300

Pro Arg Arg Pro Gly Asp Ala Glu Ile Leu Phe Ser Leu Pro Ala Lys
305                 310                 315                 320

Ala Glu Lys Glu Leu Asn Trp Lys Ala Lys Phe Gly Ile Asp Glu Met
                325                 330                 335

Cys Arg Asp Gln Trp Asn Trp Ala Ser Lys Asn Pro Tyr Gly Tyr Gly
            340                 345                 350

Ser Leu Asp Ser Thr Lys Gln Asn Gly His His Ser Tyr Gly Ser Ile
        355                 360                 365

Gly Ser Pro Lys Gln Asn Gly His Cys Thr Asn Gly Phe Ser Glu Ser
    370                 375                 380

Thr Arg His Asn Gly His Asn Gly Tyr Gly Leu Val Asp Ser Ala Lys
385                 390                 395                 400

His Asn Gly Asn Gly His Phe His
                405
```

<210> 234
<211> 1110
<212> DNA
<213> Oryza sativa

<400> 234

```
atggcggggg gcgaggtggt ggcggtggcg gcggcggcgg cggggacgag caggacggtg      60
ctggtgacgg gaggggccgg gtacatcggg agccacaccg tgctgcagct gctcgccgcg     120
gggttccgcg tcgtcgtcgc cgacagcctc gggaactcct ccgagctcgc cgtccgccgc     180
gtcgccgcgc tcgccgggga caaggcgcgg aacctctcct tacacaaggt tgatattcgt     240
gataagggtg gactggaaaa ggtttttcct tcgacaagat ttgatgctgt cgttcatttt     300
gctggtctga aagctgtggg tgaaagtgtg cagaagccat tgctttatta tgaccataat     360
```

gttgctggga caataattct tctagaagtt atggcagccc atggatgtaa aaagttggtg 420
ttttcatcgt cagctgcagt ttatggatca cctaaaaact caccctgcac agaagagttt 480
cctctcactc cacacaatcc atatggcaga accaagctca tagcagagga gatttgccgt 540
gatatatacc attcagattc tgaatggagc ataattttac ttaggtactt caatcctgtt 600
ggagctcatc ccagtggata ccttggtgaa gacccatgtg gaattcctaa caacctcatg 660
cccttcgtcc agcaagttgc tgtcgggagg agaccgtcac ttacaatatt tggaaatgac 720
tatgcaacaa aagatgggac aggggtacgt gattatattc atgtagttga tcttgctgag 780
gggcatattg ctgcactacg gaagctgttt gaaagctcaa taggatgtca agcatacaac 840
cttggaacag gaaagggaac atcagtgttg gaaatagtta atgcatttga gaaagtttct 900
ggaaagaaaa tacctttggt tattggtcca cgacgccctg gtgatgctga gattctcttt 960
tcgtcagctg ctaaagcaga gagagaattc aagtggaagg caaaatatgg catcgaagaa 1020
atgtgcagag accaatggaa ctgggcgagc aagaatcctt tcgggtacgc ctcgcccgac 1080
tccaccaagc agaatggtca ttcacactga 1110

<210> 235
<211> 369
<212> PRT
<213> Oryza sativa

<400> 235

Met Ala Gly Gly Glu Val Val Ala Val Ala Ala Ala Ala Ala Gly Thr
1 5 10 15

Ser Arg Thr Val Leu Val Thr Gly Gly Ala Gly Tyr Ile Gly Ser His
20 25 30

Thr Val Leu Gln Leu Leu Ala Ala Gly Phe Arg Val Val Val Ala Asp
35 40 45

Ser Leu Gly Asn Ser Ser Glu Leu Ala Val Arg Arg Val Ala Ala Leu
50 55 60

Ala Gly Asp Lys Ala Arg Asn Leu Ser Leu His Lys Val Asp Ile Arg
65 70 75 80

Asp Lys Gly Gly Leu Glu Lys Val Phe Ser Ser Thr Arg Phe Asp Ala
85 90 95

Val Val His Phe Ala Gly Leu Lys Ala Val Gly Glu Ser Val Gln Lys
100 105 110

Pro Leu Leu Tyr Tyr Asp His Asn Val Ala Gly Thr Ile Ile Leu Leu
115 120 125

Glu Val Met Ala Ala His Gly Cys Lys Lys Leu Val Phe Ser Ser Ser
130 135 140

Ala Ala Val Tyr Gly Ser Pro Lys Asn Ser Pro Cys Thr Glu Glu Phe
145 150 155 160

Pro Leu Thr Pro His Asn Pro Tyr Gly Arg Thr Lys Leu Ile Ala Glu
165 170 175

Glu Ile Cys Arg Asp Ile Tyr His Ser Asp Ser Glu Trp Ser Ile Ile
180 185 190

Leu Leu Arg Tyr Phe Asn Pro Val Gly Ala His Pro Ser Gly Tyr Leu
195 200 205

Gly Glu Asp Pro Cys Gly Ile Pro Asn Asn Leu Met Pro Phe Val Gln
210 215 220

Gln Val Ala Val Gly Arg Arg Pro Ser Leu Thr Ile Phe Gly Asn Asp
225 230 235 240

Tyr Ala Thr Lys Asp Gly Thr Gly Val Arg Asp Tyr Ile His Val Val
245 250 255

Asp Leu Ala Glu Gly His Ile Ala Ala Leu Arg Lys Leu Phe Glu Ser
260 265 270

Ser Ile Gly Cys Gln Ala Tyr Asn Leu Gly Thr Gly Lys Gly Thr Ser
275 280 285

Val Leu Glu Ile Val Asn Ala Phe Glu Lys Val Ser Gly Lys Lys Ile
290 295 300

Pro Leu Val Ile Gly Pro Arg Arg Pro Gly Asp Ala Glu Ile Leu Phe
305 310 315 320

Ser Ser Ala Ala Lys Ala Glu Arg Glu Phe Lys Trp Lys Ala Lys Tyr
325 330 335

Gly Ile Glu Glu Met Cys Arg Asp Gln Trp Asn Trp Ala Ser Lys Asn
340 345 350

Pro Phe Gly Tyr Ala Ser Pro Asp Ser Thr Lys Gln Asn Gly His Ser
355 360 365

His

<210> 236
<211> 1497
<212> DNA
<213> Oryza sativa

<400> 236
atgccggcgg acgcggcggc gaaggggatg aagctggaga ggtacgcgag cggcgcgggc 60

gcgatgctgc tgctgcggcg ggcggcgagc gggaaggtcg tgtcggcgtc gtcgcacctg 120
ctgttccgcg ccaccgtgct cgccacgatg gcgctcgtgt tcctcttcac gttccactac 180
ccgtcgctgc tgtcgcgctc cttcacgctc tcctccggcg ccggcgccgg cgaggggga 240
gccgcggcgc acgcctcgca ccggagcttg ctcatgtcgt cgtcgtcggc gtcggcgtcg 300
gcggcgtcgg tgtacggggg cgcggcgtgg gagaaggagg tgaggcggag cgcgaagccg 360
aggaaggacg gggggatagc cgtgctggtg accggcgcgg cggggttcgt cggcacgcac 420
tgctcgctgg cgctgagggc gcgcggcgac ggcgtgctgg ggctcgacaa cttcaacgcc 480
tactacgacc cggagctgaa gcgcgcgcgg cagaggctcc tcgccggccg cggcgtgctc 540
gtgctcgacg ccgacatcaa cgacgcgctc ctgctcgaga agctgttcga cctggtgccg 600
ttcacccacg tgctgcacct cgccgcgcag gccggcgtgc gctacgccat ggaggcgccg 660
cagacgtacg tggcgtccaa cgtggccggg ctcgtgaccg tcctcgaggt cgccgccaag 720
cacgccgacc cgcagccggc gatcgtctgg gcgtcgtcgt cgtcggtgta cggcctcaac 780
accgacgcac ccttctccga ggagcaccgc accgaccggc cggcgtcgct gtacgcggcg 840
accaagaagg ccggcgaggc catcgcgcac acgtacaacc acatctacgg cctctccatc 900
accggcctcc gcttcttcac cgtgtacggg ccatggggcc gccccgacat ggcctacttc 960
ttcttcgcca agagcatcgt ctccggcgag cccatcacgc tgttccgcgc cgccgacggc 1020
gccgacgcgc gccgcgattt cacctacatc gacgacgtcg tcaagggctg cctcggcgcg 1080
ctcgacacct ccggcaagag caccggctcc tccaagtccg gcaagaagtc cggcccggcg 1140
ccgctccgcg tctacaacct cggcaacacc tcgccggtgc cggtcacccg catggtcgcc 1200
atcctcgaga agctcctcgg caagaaggcc aacaagcgca tcgtcgccat gcccagcaat 1260
ggcgacgtgc ccttcaccca cgccaacgtc acccacgccg cccacgactt cggctaccgc 1320
cccaccacct ccctcgacgc cggcctccgc cacttcgtcg actggttcgc cgactactac 1380
aagctcaagc tcgacgtccc caagatcgcc gccaaggtcg ccggcgccgg caagccctcc 1440
tcctcgtcgg cctccaagaa gaagaagaag gccgcggcga tgtcggcgtc atcatga 1497

<210> 237
<211> 498
<212> PRT
<213> Oryza sativa

<400> 237

Met Pro Ala Asp Ala Ala Ala Lys Gly Met Lys Leu Glu Arg Tyr Ala
1 5 10 15

Ser Gly Ala Gly Ala Met Leu Leu Leu Arg Arg Ala Ala Ser Gly Lys
20 25 30

Val Val Ser Ala Ser Ser His Leu Leu Phe Arg Ala Thr Val Leu Ala
35 40 45

Thr Met Ala Leu Val Phe Leu Phe Thr Phe His Tyr Pro Ser Leu Leu
50 55 60

Ser Arg Ser Phe Thr Leu Ser Ser Gly Ala Gly Ala Gly Glu Gly Gly
65 70 75 80

Ala Ala Ala His Ala Ser His Arg Ser Leu Leu Met Ser Ser Ser Ser
85 90 95

Ala Ser Ala Ser Ala Ala Ser Val Tyr Gly Gly Ala Ala Trp Glu Lys
100 105 110

Glu Val Arg Arg Ser Ala Lys Pro Arg Lys Asp Gly Gly Ile Ala Val
115 120 125

Leu Val Thr Gly Ala Ala Gly Phe Val Gly Thr His Cys Ser Leu Ala
130 135 140

Leu Arg Ala Arg Gly Asp Gly Val Leu Gly Leu Asp Asn Phe Asn Ala
145 150 155 160

Tyr Tyr Asp Pro Glu Leu Lys Arg Ala Arg Gln Arg Leu Leu Ala Gly
165 170 175

Arg Gly Val Leu Val Leu Asp Ala Asp Ile Asn Asp Ala Leu Leu Leu
180 185 190

Glu Lys Leu Phe Asp Leu Val Pro Phe Thr His Val Leu His Leu Ala
195 200 205

Ala Gln Ala Gly Val Arg Tyr Ala Met Glu Ala Pro Gln Thr Tyr Val
210 215 220

Ala Ser Asn Val Ala Gly Leu Val Thr Val Leu Glu Val Ala Ala Lys
225 230 235 240

His Ala Asp Pro Gln Pro Ala Ile Val Trp Ala Ser Ser Ser Ser Val
245 250 255

Tyr Gly Leu Asn Thr Asp Ala Pro Phe Ser Glu Glu His Arg Thr Asp
260 265 270

Arg Pro Ala Ser Leu Tyr Ala Ala Thr Lys Lys Ala Gly Glu Ala Ile
275 280 285

Ala His Thr Tyr Asn His Ile Tyr Gly Leu Ser Ile Thr Gly Leu Arg
290 295 300

Phe Phe Thr Val Tyr Gly Pro Trp Gly Arg Pro Asp Met Ala Tyr Phe
305 310 315 320

```
Phe Phe Ala Lys Ser Ile Val Ser Gly Glu Pro Ile Thr Leu Phe Arg
                325                 330                 335

Ala Ala Asp Gly Ala Asp Ala Arg Arg Asp Phe Thr Tyr Ile Asp Asp
            340                 345                 350

Val Val Lys Gly Cys Leu Gly Ala Leu Asp Thr Ser Gly Lys Ser Thr
        355                 360                 365

Gly Ser Ser Lys Ser Gly Lys Lys Ser Gly Pro Ala Pro Leu Arg Val
    370                 375                 380

Tyr Asn Leu Gly Asn Thr Ser Pro Val Pro Val Thr Arg Met Val Ala
385                 390                 395                 400

Ile Leu Glu Lys Leu Leu Gly Lys Lys Ala Asn Lys Arg Ile Val Ala
                405                 410                 415

Met Pro Ser Asn Gly Asp Val Pro Phe Thr His Ala Asn Val Thr His
            420                 425                 430

Ala Ala His Asp Phe Gly Tyr Arg Pro Thr Thr Ser Leu Asp Ala Gly
        435                 440                 445

Leu Arg His Phe Val Asp Trp Phe Ala Asp Tyr Tyr Lys Leu Lys Leu
    450                 455                 460

Asp Val Pro Lys Ile Ala Ala Lys Val Ala Gly Ala Gly Lys Pro Ser
465                 470                 475                 480

Ser Ser Ser Ala Ser Lys Lys Lys Lys Lys Ala Ala Ala Met Ser Ala
                485                 490                 495

Ser Ser


<210>  238
<211>  1122
<212>  DNA
<213>  Oryza sativa

<400>  238
atggtgagcg gcggaggagt agcggcggag aacggcgaga tggtggggaa cggggagggg      60

aggaagggtg cgggggcgag cgtgctggtg acggggggag cggggtacat cgggacgcac     120

acggtgctgc ggctgctgga gaaggggttc gcggtcaccg tcgtcgacaa cttccacaac     180

tccgtcccgg aggcgctcga ccgcgtccgc ctcatcgccg gcgccgccct ctccgcccgc     240

ctcgacttca tcgccgggga tctcaagagc aaggacgaca tggagaaggt gttcgccgcc     300

aagaggtatg acgccgtgat ccacttcgcc gggctgaagg cggtggggga gagcgtcgcg     360

cacccgcaga tgtactacga gaacaacgtc gccggcacca tgaacctcta ctccgccatg     420

accaagtacg gctgcaagaa gatagtgttc tcgtcgtcgg cgacggtgta cggccagccg     480
```

gagaagaccc cctgcgtcga ggattccaag ctgagcgctc tcaacccata cggcaccacc 540

aagctcgtcc tggagaacta cttccggcag gtgcaggccg ccgacccgga gatgagggtg 600

atcctgctca ggtacttcaa ccccatcggc gctcaccgga gcggcgacat cggggaggac 660

cccaggggca tccccaacaa ccttcttccg tacatccagc aggtcgccgt cggccgccgc 720

cccgagctca acgtctacgg cgtcgactac ccaaccaggg acggcaccgc gatcagggat 780

tacatacatg tagtggacct tgccgatggc cacattgccg cactggagaa gctcttcgct 840

actcctgaca ttggttgtgt ggcttacaat ctaggaacag ggtgtggaac aacggtgctc 900

gaggtggtga aggcgttcga ggaggcgtcc ggaaagaaaa ttcctatcaa gatttgcccc 960

agaagacctg gagattgcac tgaggtttac gcttccactg acaaggccaa gaaggagctc 1020

ggatggagtg ctcggtttgg aatagaggac atgtgcaggg accagtggaa ttgggccaag 1080

aagaatccgt acggatacag cgccaatgct gagcagaatt ag 1122

<210> 239
<211> 373
<212> PRT
<213> Oryza sativa

<400> 239

Met Val Ser Gly Gly Gly Val Ala Ala Glu Asn Gly Glu Met Val Gly
1 5 10 15

Asn Gly Glu Gly Arg Lys Gly Ala Gly Ala Ser Val Leu Val Thr Gly
20 25 30

Gly Ala Gly Tyr Ile Gly Thr His Thr Val Leu Arg Leu Leu Glu Lys
35 40 45

Gly Phe Ala Val Thr Val Val Asp Asn Phe His Asn Ser Val Pro Glu
50 55 60

Ala Leu Asp Arg Val Arg Leu Ile Ala Gly Ala Ala Leu Ser Ala Arg
65 70 75 80

Leu Asp Phe Ile Ala Gly Asp Leu Lys Ser Lys Asp Asp Met Glu Lys
85 90 95

Val Phe Ala Ala Lys Arg Tyr Asp Ala Val Ile His Phe Ala Gly Leu
100 105 110

Lys Ala Val Gly Glu Ser Val Ala His Pro Gln Met Tyr Tyr Glu Asn
115 120 125

Asn Val Ala Gly Thr Met Asn Leu Tyr Ser Ala Met Thr Lys Tyr Gly
130 135 140

Cys Lys Lys Ile Val Phe Ser Ser Ser Ala Thr Val Tyr Gly Gln Pro

```
145                 150                 155                 160

Glu Lys Thr Pro Cys Val Glu Asp Ser Lys Leu Ser Ala Leu Asn Pro
                165                 170                 175

Tyr Gly Thr Thr Lys Leu Val Leu Glu Asn Tyr Phe Arg Gln Val Gln
            180                 185                 190

Ala Ala Asp Pro Glu Met Arg Val Ile Leu Leu Arg Tyr Phe Asn Pro
        195                 200                 205

Ile Gly Ala His Arg Ser Gly Asp Ile Gly Glu Asp Pro Arg Gly Ile
    210                 215                 220

Pro Asn Asn Leu Leu Pro Tyr Ile Gln Gln Val Ala Val Gly Arg Arg
225                 230                 235                 240

Pro Glu Leu Asn Val Tyr Gly Val Asp Tyr Pro Thr Arg Asp Gly Thr
                245                 250                 255

Ala Ile Arg Asp Tyr Ile His Val Val Asp Leu Ala Asp Gly His Ile
            260                 265                 270

Ala Ala Leu Glu Lys Leu Phe Ala Thr Pro Asp Ile Gly Cys Val Ala
        275                 280                 285

Tyr Asn Leu Gly Thr Gly Cys Gly Thr Thr Val Leu Glu Val Val Lys
    290                 295                 300

Ala Phe Glu Glu Ala Ser Gly Lys Lys Ile Pro Ile Lys Ile Cys Pro
305                 310                 315                 320

Arg Arg Pro Gly Asp Cys Thr Glu Val Tyr Ala Ser Thr Asp Lys Ala
                325                 330                 335

Lys Lys Glu Leu Gly Trp Ser Ala Arg Phe Gly Ile Glu Asp Met Cys
            340                 345                 350

Arg Asp Gln Trp Asn Trp Ala Lys Lys Asn Pro Tyr Gly Tyr Ser Ala
        355                 360                 365

Asn Ala Glu Gln Asn
    370
```

<210> 240
<211> 1056
<212> DNA
<213> Arabidopsis thaliana

<400> 240

```
atgggttctt ctgtggagca gaacattctt gttactggtg gtgctggctt tatcgggacg      60

catactgttg ttcaacttct caaagatggt tttaaggttt cgatcatcga taattttgat     120
```

aactctgtta tcgaagctgt tgatagagtt agggagcttg ttggtcctga tctctccaag 180
aagctcgact tcaatctggg tgatctaaga aacaaagggg acattgagaa actattctcc 240
aagcagagat ttgatgctgt gattcatttt gcgggtctta aagctgtggg tgagagtgtt 300
gaaaaccctc gccgctactt tgacaataac ttggttggaa caatcaatct atatgagacc 360
atggcaaagt acaactgcaa aatgatggtg ttttcatctt ctgccactgt ttatggacaa 420
cctgaaaaga ttccatgcat ggaagacttt gaattaaagg ctatgaatcc ttatggtcgt 480
actaagctct ttcttgaaga aatagctaga gatattcaaa aggcagaacc ggaatggaga 540
attattctgc tgaggtactt caatcctgta ggagcacatg agagtggcag tattggtgag 600
gatccaaaag gcatccccaa taacctcatg ccttacatcc aacaagtggc cgttggacgt 660
ttaccggaac tcaatgtcta tggacatgac tatcccaccg aggatggtag tgcggtaaga 720
gactacatcc atgtgatgga tttagcagat ggccatatcg ctgcgctcag gaagctattt 780
gctgatccaa agattggttg tactgcttac aatctaggga ctggtcaagg aacgtctgtg 840
ttagaaatgg ttgcagcttt tgaaaaagct tccggcaaga aaatcccgat taagctctgt 900
ccgagaaggt caggagatgc aacagcagtt tatgcttcaa cagagaaggc tgagaaagaa 960
cttggctgga aggcaaaata tggagtggat gagatgtgca gagatcagtg gaaatgggca 1020
aacaataatc catggggtta ccagaataag ctttga 1056

<210> 241
<211> 351
<212> PRT
<213> Arabidopsis thaliana

<400> 241

Met Gly Ser Ser Val Glu Gln Asn Ile Leu Val Thr Gly Gly Ala Gly
1 5 10 15

Phe Ile Gly Thr His Thr Val Val Gln Leu Leu Lys Asp Gly Phe Lys
20 25 30

Val Ser Ile Ile Asp Asn Phe Asp Asn Ser Val Ile Glu Ala Val Asp
35 40 45

Arg Val Arg Glu Leu Val Gly Pro Asp Leu Ser Lys Lys Leu Asp Phe
50 55 60

Asn Leu Gly Asp Leu Arg Asn Lys Gly Asp Ile Glu Lys Leu Phe Ser
65 70 75 80

Lys Gln Arg Phe Asp Ala Val Ile His Phe Ala Gly Leu Lys Ala Val
85 90 95

Gly Glu Ser Val Glu Asn Pro Arg Arg Tyr Phe Asp Asn Asn Leu Val
100 105 110

Gly Thr Ile Asn Leu Tyr Glu Thr Met Ala Lys Tyr Asn Cys Lys Met

```
        115                 120                 125

Met Val Phe Ser Ser Ser Ala Thr Val Tyr Gly Gln Pro Glu Lys Ile
    130                 135                 140

Pro Cys Met Glu Asp Phe Glu Leu Lys Ala Met Asn Pro Tyr Gly Arg
145                 150                 155                 160

Thr Lys Leu Phe Leu Glu Glu Ile Ala Arg Asp Ile Gln Lys Ala Glu
                165                 170                 175

Pro Glu Trp Arg Ile Ile Leu Leu Arg Tyr Phe Asn Pro Val Gly Ala
            180                 185                 190

His Glu Ser Gly Ser Ile Gly Glu Asp Pro Lys Gly Ile Pro Asn Asn
        195                 200                 205

Leu Met Pro Tyr Ile Gln Gln Val Ala Val Gly Arg Leu Pro Glu Leu
    210                 215                 220

Asn Val Tyr Gly His Asp Tyr Pro Thr Glu Asp Gly Ser Ala Val Arg
225                 230                 235                 240

Asp Tyr Ile His Val Met Asp Leu Ala Asp Gly His Ile Ala Ala Leu
                245                 250                 255

Arg Lys Leu Phe Ala Asp Pro Lys Ile Gly Cys Thr Ala Tyr Asn Leu
            260                 265                 270

Gly Thr Gly Gln Gly Thr Ser Val Leu Glu Met Val Ala Ala Phe Glu
        275                 280                 285

Lys Ala Ser Gly Lys Lys Ile Pro Ile Lys Leu Cys Pro Arg Arg Ser
    290                 295                 300

Gly Asp Ala Thr Ala Val Tyr Ala Ser Thr Glu Lys Ala Glu Lys Glu
305                 310                 315                 320

Leu Gly Trp Lys Ala Lys Tyr Gly Val Asp Glu Met Cys Arg Asp Gln
                325                 330                 335

Trp Lys Trp Ala Asn Asn Asn Pro Trp Gly Tyr Gln Asn Lys Leu
            340                 345                 350

<210>  242
<211>  1056
<212>  DNA
<213>  Arabidopsis thaliana

<400>  242
atgggttctt ctgtggaaca gaacattttg gtgactggtg gtgctggatt cattggaaca      60

catactgttg ttcagctttt gaatcagggt tttaaggtta cgatcattga taatcttgat     120
```

aactctgtcg ttgaagctgt tcatagggtt agggaacttg ttggtcctga tctctctacc 180

aagcttgaat tcaatctggg tgatctaaga aacaaaggag atattgagaa actcttttcc 240

aatcagagat ttgatgctgt gattcacttt gctggtctta aagctgtggg agaaagtgtt 300

ggaaaccctc gtcgttactt tgataataac ttagttggaa ctatcaatct atatgagacc 360

atggcaaaat acaactgcaa aatgatggtg ttttcgtcgt ctgcaacagt ttatggtcaa 420

cctgaaatag tcccatgtgt ggaagacttt gagttacagg ctatgaatcc ttatggtcgt 480

actaagctgt ttcttgaaga gatagctaga gacattcacg ctgcggaacc agaatggaag 540

ataattcttc tgaggtactt caatccggtt ggagctcacg agagtggaag aattggagaa 600

gatccaaagg gcataccgaa taatctcatg ccttatatcc aacaagtggc ggttggaaga 660

ttacctgaac tcaatgtatt tggacatgat tatcctacca tggatggtag cgcggttaga 720

gactacatcc atgtaatgga tttagcagat ggccatgtgg ctgcgttaaa caaattgttt 780

tcagactcaa agattggctg tactgcttat aatcttggca ctggtcaagg aacttctgtc 840

ttagaaatgg tttcttcttt tgaaaaagct tctggcaaga aaatacctat caagctatgt 900

ccaagaagag ctggagatgc aacagctgtt tatgcttcaa ctcagaaagc tgagaaggaa 960

cttggctgga aggcaaaata tggagttgat gagatgtgca gagatcaatg gaactgggca 1020

aataagaatc catggggttt ccagaagaag ccttga 1056

<210> 243
<211> 351
<212> PRT
<213> Arabidopsis thaliana

<400> 243

Met Gly Ser Ser Val Glu Gln Asn Ile Leu Val Thr Gly Gly Ala Gly
1 5 10 15

Phe Ile Gly Thr His Thr Val Val Gln Leu Leu Asn Gln Gly Phe Lys
20 25 30

Val Thr Ile Ile Asp Asn Leu Asp Asn Ser Val Val Glu Ala Val His
35 40 45

Arg Val Arg Glu Leu Val Gly Pro Asp Leu Ser Thr Lys Leu Glu Phe
50 55 60

Asn Leu Gly Asp Leu Arg Asn Lys Gly Asp Ile Glu Lys Leu Phe Ser
65 70 75 80

Asn Gln Arg Phe Asp Ala Val Ile His Phe Ala Gly Leu Lys Ala Val
85 90 95

Gly Glu Ser Val Gly Asn Pro Arg Arg Tyr Phe Asp Asn Asn Leu Val
100 105 110

```
Gly Thr Ile Asn Leu Tyr Glu Thr Met Ala Lys Tyr Asn Cys Lys Met
        115                 120                 125

Met Val Phe Ser Ser Ser Ala Thr Val Tyr Gly Gln Pro Glu Ile Val
    130                 135                 140

Pro Cys Val Glu Asp Phe Glu Leu Gln Ala Met Asn Pro Tyr Gly Arg
145                 150                 155                 160

Thr Lys Leu Phe Leu Glu Glu Ile Ala Arg Asp Ile His Ala Ala Glu
                165                 170                 175

Pro Glu Trp Lys Ile Ile Leu Leu Arg Tyr Phe Asn Pro Val Gly Ala
            180                 185                 190

His Glu Ser Gly Arg Ile Gly Glu Asp Pro Lys Gly Ile Pro Asn Asn
        195                 200                 205

Leu Met Pro Tyr Ile Gln Gln Val Ala Val Gly Arg Leu Pro Glu Leu
    210                 215                 220

Asn Val Phe Gly His Asp Tyr Pro Thr Met Asp Gly Ser Ala Val Arg
225                 230                 235                 240

Asp Tyr Ile His Val Met Asp Leu Ala Asp Gly His Val Ala Ala Leu
                245                 250                 255

Asn Lys Leu Phe Ser Asp Ser Lys Ile Gly Cys Thr Ala Tyr Asn Leu
            260                 265                 270

Gly Thr Gly Gln Gly Thr Ser Val Leu Glu Met Val Ser Ser Phe Glu
        275                 280                 285

Lys Ala Ser Gly Lys Lys Ile Pro Ile Lys Leu Cys Pro Arg Arg Ala
    290                 295                 300

Gly Asp Ala Thr Ala Val Tyr Ala Ser Thr Gln Lys Ala Glu Lys Glu
305                 310                 315                 320

Leu Gly Trp Lys Ala Lys Tyr Gly Val Asp Glu Met Cys Arg Asp Gln
                325                 330                 335

Trp Asn Trp Ala Asn Lys Asn Pro Trp Gly Phe Gln Lys Lys Pro
            340                 345                 350

<210>  244
<211>  1047
<212>  DNA
<213>  Arabidopsis thaliana

<400>  244
atggttggga atattctggt gaccggtggt gctggttaca tcggaagtca cacggttctt      60
```

```
cagcttcttc tcggaggcta taacaccgtc gttatagaca acctcgacaa ttcctctctc    120
gtttcgatcc aacgcgtcaa ggatctcgcc ggagatcatg gacaaaatct caccgtccac    180
caggtggacc ttcgcgataa acccgcactt gagaaggttt tctccgaaac aaagtttgat    240
gcagtaatgc attttgctgg attgaaagca gttggtgaga gcgtggcgaa accacttctg    300
tattataaca ataacttgat tgcgactatt acacttttgg aagtaatggc tgcacacgga    360
tgtaaaaagc ttgtattttc ttcgtccgct actgtgtatg gctggccaaa ggaggttcct    420
tgtacagaag agtctcccct gtctggaatg agtccttatg gacggacaaa gctgttcata    480
gaggacattt gccgtgatgt acaacgtggt gatcctgaat ggagaatcat aatgctgagg    540
tactttaacc ctgtgggagc tcaccctagc ggtcgcattg gtgaggatcc ttgtgggact    600
ccaaataatc tcatgcctta tgtccagcaa gtcgttgttg ggaggctacc taacctaaaa    660
atttatggaa ctgactatac cactaaagat ggcactggtg tacgagacta tattcatgtt    720
gttgatctag cagatggcca tatatgtgcg cttcaaaagc tagacgatac tgaaataggt    780
tgtgaggtat acaacctggg aaccggaaaa ggaacaacag tgttggagat ggttgatgca    840
tttgagaaag cttctggaat gaaaatccca ctggtgaagg ttggaaggag accaggtgat    900
gcagaaaccg tctatgcgtc aacagaaaaa gctgaacgcg aactaaactg gaaggcaaat    960
tttggaatcg aagaaatgtg tagggatcag tggaactggg caagcaacaa tcctttcggt   1020
tacggttctt caccaaactc aacataa                                        1047
```

```
<210>  245
<211>  348
<212>  PRT
<213>  Arabidopsis thaliana

<400>  245

Met Val Gly Asn Ile Leu Val Thr Gly Gly Ala Gly Tyr Ile Gly Ser
1               5                   10                  15

His Thr Val Leu Gln Leu Leu Leu Gly Gly Tyr Asn Thr Val Val Ile
            20                  25                  30

Asp Asn Leu Asp Asn Ser Ser Leu Val Ser Ile Gln Arg Val Lys Asp
        35                  40                  45

Leu Ala Gly Asp His Gly Gln Asn Leu Thr Val His Gln Val Asp Leu
    50                  55                  60

Arg Asp Lys Pro Ala Leu Glu Lys Val Phe Ser Glu Thr Lys Phe Asp
65                  70                  75                  80

Ala Val Met His Phe Ala Gly Leu Lys Ala Val Gly Glu Ser Val Ala
                85                  90                  95

Lys Pro Leu Leu Tyr Tyr Asn Asn Asn Leu Ile Ala Thr Ile Thr Leu
            100                 105                 110
```

```
Leu Glu Val Met Ala Ala His Gly Cys Lys Lys Leu Val Phe Ser Ser
        115                 120                 125

Ser Ala Thr Val Tyr Gly Trp Pro Lys Glu Val Pro Cys Thr Glu Glu
    130                 135                 140

Ser Pro Leu Ser Gly Met Ser Pro Tyr Gly Arg Thr Lys Leu Phe Ile
145                 150                 155                 160

Glu Asp Ile Cys Arg Asp Val Gln Arg Gly Asp Pro Glu Trp Arg Ile
                165                 170                 175

Ile Met Leu Arg Tyr Phe Asn Pro Val Gly Ala His Pro Ser Gly Arg
            180                 185                 190

Ile Gly Glu Asp Pro Cys Gly Thr Pro Asn Asn Leu Met Pro Tyr Val
        195                 200                 205

Gln Gln Val Val Val Gly Arg Leu Pro Asn Leu Lys Ile Tyr Gly Thr
    210                 215                 220

Asp Tyr Thr Thr Lys Asp Gly Thr Gly Val Arg Asp Tyr Ile His Val
225                 230                 235                 240

Val Asp Leu Ala Asp Gly His Ile Cys Ala Leu Gln Lys Leu Asp Asp
                245                 250                 255

Thr Glu Ile Gly Cys Glu Val Tyr Asn Leu Gly Thr Gly Lys Gly Thr
            260                 265                 270

Thr Val Leu Glu Met Val Asp Ala Phe Glu Lys Ala Ser Gly Met Lys
        275                 280                 285

Ile Pro Leu Val Lys Val Gly Arg Arg Pro Gly Asp Ala Glu Thr Val
    290                 295                 300

Tyr Ala Ser Thr Glu Lys Ala Glu Arg Glu Leu Asn Trp Lys Ala Asn
305                 310                 315                 320

Phe Gly Ile Glu Glu Met Cys Arg Asp Gln Trp Asn Trp Ala Ser Asn
                325                 330                 335

Asn Pro Phe Gly Tyr Gly Ser Ser Pro Asn Ser Thr
            340                 345
```

<210> 246
<211> 1056
<212> DNA
<213> Arabidopsis thaliana

<400> 246
atgatggcta gaaacgttct agtaagcggt ggtgctggat acatcggtag tcacacggtg 60

```
cttcaacttc ttctcggtgg ttactccgtc gtagttgttg ataatctcga taattcctcc     120
gccgtctctc ttcaacgtgt gaagaaactc gcggctgaac acggagaacg cctctccttc     180
caccaggtgg atcttcgaga caggtctgct cttgagaaga tattctcaga aacaaagttt     240
gatgcagtga tacattttgc tggacttaaa gcagttggtg agagtgtaga gaagcctttg     300
ctttattata ataataatct tgttggtact attactcttt tggaagttat ggctcaacat     360
ggctgcaaaa atcttgtgtt ttcatcatca gcaactgtgt atggctctcc aaaagaagtt     420
ccttgtacag aggagtttcc aatttctgct ttgaacccat atggacgaac aaagctattc     480
attgaggaaa tctgccgcga tgtatacggt tctgacccgg aatggaagat tatattgctt     540
aggtatttca atcctgttgg tgcacatcct agtggtgaca ttggtgaaga ccctcgtggt     600
attccaaaca atctcatgcc ctttgtacaa caagtggctg ttggtaggag acctcatctc     660
actgtctttg gaaatgatta caatacaaaa gatggaacag ggttcgaga ttacattcat     720
gttattgatt tagctgatgg acacatagcg gctctacgta agctagaaga ttgcaagatc     780
ggttgtgaag tgtacaatct cggaacagga aatggaacat cagttcttga aatggttgat     840
gcttttgaaa aagcatccgg aaagaaaatc cctctggtga tcgcgggacg tcgaccggga     900
gatgctgaag ttgtatacgc ctcgacggaa agagcagaaa gtgaattgaa ttggaaggcc     960
aaatatggga ttgaggagat gtgtagagat ttgtggaact gggctagtaa taatccttat    1020
ggatatgatt cctcttctga agacaactct cattaa                              1056
```

<210> 247
<211> 351
<212> PRT
<213> Arabidopsis thaliana

<400> 247

```
Met Met Ala Arg Asn Val Leu Val Ser Gly Gly Ala Gly Tyr Ile Gly
1               5                   10                  15

Ser His Thr Val Leu Gln Leu Leu Leu Gly Gly Tyr Ser Val Val Val
            20                  25                  30

Val Asp Asn Leu Asp Asn Ser Ser Ala Val Ser Leu Gln Arg Val Lys
        35                  40                  45

Lys Leu Ala Ala Glu His Gly Glu Arg Leu Ser Phe His Gln Val Asp
    50                  55                  60

Leu Arg Asp Arg Ser Ala Leu Glu Lys Ile Phe Ser Glu Thr Lys Phe
65                  70                  75                  80

Asp Ala Val Ile His Phe Ala Gly Leu Lys Ala Val Gly Glu Ser Val
                85                  90                  95

Glu Lys Pro Leu Leu Tyr Tyr Asn Asn Asn Leu Val Gly Thr Ile Thr
            100                 105                 110
```

Leu Leu Glu Val Met Ala Gln His Gly Cys Lys Asn Leu Val Phe Ser
        115                 120                 125

Ser Ser Ala Thr Val Tyr Gly Ser Pro Lys Glu Val Pro Cys Thr Glu
    130                 135                 140

Glu Phe Pro Ile Ser Ala Leu Asn Pro Tyr Gly Arg Thr Lys Leu Phe
145                 150                 155                 160

Ile Glu Glu Ile Cys Arg Asp Val Tyr Gly Ser Asp Pro Glu Trp Lys
                165                 170                 175

Ile Ile Leu Leu Arg Tyr Phe Asn Pro Val Gly Ala His Pro Ser Gly
            180                 185                 190

Asp Ile Gly Glu Asp Pro Arg Gly Ile Pro Asn Asn Leu Met Pro Phe
        195                 200                 205

Val Gln Gln Val Ala Val Gly Arg Arg Pro His Leu Thr Val Phe Gly
    210                 215                 220

Asn Asp Tyr Asn Thr Lys Asp Gly Thr Gly Val Arg Asp Tyr Ile His
225                 230                 235                 240

Val Ile Asp Leu Ala Asp Gly His Ile Ala Ala Leu Arg Lys Leu Glu
                245                 250                 255

Asp Cys Lys Ile Gly Cys Glu Val Tyr Asn Leu Gly Thr Gly Asn Gly
            260                 265                 270

Thr Ser Val Leu Glu Met Val Asp Ala Phe Glu Lys Ala Ser Gly Lys
        275                 280                 285

Lys Ile Pro Leu Val Ile Ala Gly Arg Arg Pro Gly Asp Ala Glu Val
    290                 295                 300

Val Tyr Ala Ser Thr Glu Arg Ala Glu Ser Glu Leu Asn Trp Lys Ala
305                 310                 315                 320

Lys Tyr Gly Ile Glu Glu Met Cys Arg Asp Leu Trp Asn Trp Ala Ser
                325                 330                 335

Asn Asn Pro Tyr Gly Tyr Asp Ser Ser Ser Glu Asp Asn Ser His
            340                 345                 350

<210> 248
<211> 1053
<212> DNA
<213> Arabidopsis thaliana

<400> 248
atggcgaaga gtgttttggt taccggcggc gctggctata tcggaagtca tactgttctt 60

```
caactgcttg aaggtggtta ctccgccgtc gttgttgaca attacgacaa ttcctctgct     120
gcttcgcttc agcgcgtgaa gaaactcgcc ggcgaaaacg gaaaccgcct ctccttccac     180
caggtggatc tccgagacag gcctgcgctt gagaagatat tctcagaaac aaagtttgat     240
gcagtgatac actttgctgg acttaaagca gtaggtgaga gtgtggagaa gcctttgctc     300
tattataata ataacattgt tggcactgtt actcttttgg aggttatggc tcaatacggc     360
tgcaaaaatc ttgtattttc atcatcagct actgtctatg gctggcctaa ggaggttcct     420
tgcactgagg agtcaccaat ttctgccact aacccatatg gccgtacaaa gctttttatc     480
gaagagattt gccgggatgt acatcgttct gactcggaat ggaagatcat attgcttaga     540
tacttcaacc ctgttggtgc acatcctagt ggttacattg gtgaagatcc tcttggagtt     600
cccaacaatc tcatgcctta tgtccaacaa gttgcagttg gccggagacc tcaccttact     660
gtctttggaa ccgactacaa gacaaaggat ggtacagggg ttagagatta cattcatgtc     720
atggatttag cagatggaca catagctgct ctgcgtaagc tagatgatct caaaatcagt     780
tgtgaggtat acaatcttgg aacgggtaat ggaacatcgg tcctagaaat ggtcgctgcc     840
tttgaaaaag catccggaaa gaaaatccct ttggtgatgg ctggacgacg tcctggagac     900
gctgaagttg tttacgcctc aacggaaaaa gcagagcgcg aactaaattg gaaggctaag     960
aatgggatag aggagatgtg tagggatcta tggaattggg caagcaataa cccctacggc    1020
tacaattcct cctccaatgg ctcctcttca taa                                 1053
```

<210> 249
<211> 350
<212> PRT
<213> Arabidopsis thaliana

<400> 249

```
Met Ala Lys Ser Val Leu Val Thr Gly Gly Ala Gly Tyr Ile Gly Ser
1               5                   10                  15

His Thr Val Leu Gln Leu Leu Glu Gly Gly Tyr Ser Ala Val Val Val
            20                  25                  30

Asp Asn Tyr Asp Asn Ser Ser Ala Ala Ser Leu Gln Arg Val Lys Lys
        35                  40                  45

Leu Ala Gly Glu Asn Gly Asn Arg Leu Ser Phe His Gln Val Asp Leu
    50                  55                  60

Arg Asp Arg Pro Ala Leu Glu Lys Ile Phe Ser Glu Thr Lys Phe Asp
65                  70                  75                  80

Ala Val Ile His Phe Ala Gly Leu Lys Ala Val Gly Glu Ser Val Glu
                85                  90                  95

Lys Pro Leu Leu Tyr Tyr Asn Asn Asn Ile Val Gly Thr Val Thr Leu
            100                 105                 110
```

Leu Glu Val Met Ala Gln Tyr Gly Cys Lys Asn Leu Val Phe Ser Ser
        115                 120                 125

Ser Ala Thr Val Tyr Gly Trp Pro Lys Glu Val Pro Cys Thr Glu Glu
    130                 135                 140

Ser Pro Ile Ser Ala Thr Asn Pro Tyr Gly Arg Thr Lys Leu Phe Ile
145                 150                 155                 160

Glu Glu Ile Cys Arg Asp Val His Arg Ser Asp Ser Glu Trp Lys Ile
                165                 170                 175

Ile Leu Leu Arg Tyr Phe Asn Pro Val Gly Ala His Pro Ser Gly Tyr
            180                 185                 190

Ile Gly Glu Asp Pro Leu Gly Val Pro Asn Asn Leu Met Pro Tyr Val
        195                 200                 205

Gln Gln Val Ala Val Gly Arg Arg Pro His Leu Thr Val Phe Gly Thr
    210                 215                 220

Asp Tyr Lys Thr Lys Asp Gly Thr Gly Val Arg Asp Tyr Ile His Val
225                 230                 235                 240

Met Asp Leu Ala Asp Gly His Ile Ala Ala Leu Arg Lys Leu Asp Asp
                245                 250                 255

Leu Lys Ile Ser Cys Glu Val Tyr Asn Leu Gly Thr Gly Asn Gly Thr
            260                 265                 270

Ser Val Leu Glu Met Val Ala Ala Phe Glu Lys Ala Ser Gly Lys Lys
        275                 280                 285

Ile Pro Leu Val Met Ala Gly Arg Arg Pro Gly Asp Ala Glu Val Val
    290                 295                 300

Tyr Ala Ser Thr Glu Lys Ala Glu Arg Glu Leu Asn Trp Lys Ala Lys
305                 310                 315                 320

Asn Gly Ile Glu Glu Met Cys Arg Asp Leu Trp Asn Trp Ala Ser Asn
                325                 330                 335

Asn Pro Tyr Gly Tyr Asn Ser Ser Ser Asn Gly Ser Ser Ser
            340                 345                 350

<210> 250
<211> 1137
<212> DNA
<213> Populus tremuloides

<400> 250
atggattatc tggattcaag acgaaagagc agatgtgctg gaaaaattat tgcagctgcg 60

```
ctctgcatta cattctccca gcatgagctc ggcgtaacgc atgtcttagt gactggaggt   120
gctgggtata ttggctctca tgctgcactt cgactcttga aggattcata ccgtgtaact   180
aaagtggaca atctttctcg agggaatctg ggtgctgtta aagttctcca agatctattt   240
ccggagcctg ggcggttaca gttcatatac gcagacttgg gagatgcaaa agctgtaaac   300
aaaatctttg ctgaaaatgc atttgatgct gtgatgcatt tcgctgctgt tgcatatgtt   360
ggtgaaagta caatggaacc tctcagatat tatcacaaca tcacatcaaa taccttggta   420
gttttagaag caatggcagc acataaggtg aagacattga tttattctag cacttgtgca   480
acttacggcg agcctgttaa gatgccgatt acagaacaaa ctcctcagct tccaatcaac   540
ccctatggaa aagccaagaa aatggcagaa gatatcataa ttgacttctc aaaaaccact   600
gacatggctg tcatgatcct aagatacttc aatgttattg ggtctgatcc agaaggaaga   660
ttaggggaag ctccgcaacc tgaacttcgt gagcatggcc gaatctctgg cgcttgtttt   720
gatgcagctc gaggaataat accaggacta aaacagagaa tcacaattca gtggatagac   780
tataaaactg ctgatggcac gtgtgtacgg gactatatcg atgtcactga cctggtcgat   840
gcccatgtga aagctcttgc ccatgcaaag cctcgaaaag ttggcattta caatgttgga   900
actggaaaag gtagatcagt gaaggagttt gtcgaggcat gtaagaaggc aacaggtgtg   960
gacataaagg ttgaatacct caatcgtcgg ccaggagact atgctgaggt cttcagtgac  1020
ccttccaaaa taaaacaaga gctaaactgg aaagctcaat atactgacct taagaagagt  1080
ttgcagattg catggaaatg gcagaagtca catttgaatg gttacagtca cagttaa     1137
```

<210> 251
<211> 378
<212> PRT
<213> Populus tremuloides

<400> 251

```
Met Asp Tyr Leu Asp Ser Arg Arg Lys Ser Arg Cys Ala Gly Lys Ile
1               5                   10                  15

Ile Ala Ala Ala Leu Cys Ile Thr Phe Ser Gln His Glu Leu Gly Val
            20                  25                  30

Thr His Val Leu Val Thr Gly Gly Ala Gly Tyr Ile Gly Ser His Ala
        35                  40                  45

Ala Leu Arg Leu Leu Lys Asp Ser Tyr Arg Val Thr Lys Val Asp Asn
    50                  55                  60

Leu Ser Arg Gly Asn Leu Gly Ala Val Lys Val Leu Gln Asp Leu Phe
65                  70                  75                  80

Pro Glu Pro Gly Arg Leu Gln Phe Ile Tyr Ala Asp Leu Gly Asp Ala
                85                  90                  95
```

Lys Ala Val Asn Lys Ile Phe Ala Glu Asn Ala Phe Asp Ala Val Met
100 105 110

His Phe Ala Ala Val Ala Tyr Val Gly Glu Ser Thr Met Glu Pro Leu
115 120 125

Arg Tyr Tyr His Asn Ile Thr Ser Asn Thr Leu Val Val Leu Glu Ala
130 135 140

Met Ala Ala His Lys Val Lys Thr Leu Ile Tyr Ser Ser Thr Cys Ala
145 150 155 160

Thr Tyr Gly Glu Pro Val Lys Met Pro Ile Thr Glu Gln Thr Pro Gln
165 170 175

Leu Pro Ile Asn Pro Tyr Gly Lys Ala Lys Lys Met Ala Glu Asp Ile
180 185 190

Ile Ile Asp Phe Ser Lys Thr Thr Asp Met Ala Val Met Ile Leu Arg
195 200 205

Tyr Phe Asn Val Ile Gly Ser Asp Pro Glu Gly Arg Leu Gly Glu Ala
210 215 220

Pro Gln Pro Glu Leu Arg Glu His Gly Arg Ile Ser Gly Ala Cys Phe
225 230 235 240

Asp Ala Ala Arg Gly Ile Ile Pro Gly Leu Lys Gln Arg Ile Thr Ile
245 250 255

Gln Trp Ile Asp Tyr Lys Thr Ala Asp Gly Thr Cys Val Arg Asp Tyr
260 265 270

Ile Asp Val Thr Asp Leu Val Asp Ala His Val Lys Ala Leu Ala His
275 280 285

Ala Lys Pro Arg Lys Val Gly Ile Tyr Asn Val Gly Thr Gly Lys Gly
290 295 300

Arg Ser Val Lys Glu Phe Val Glu Ala Cys Lys Lys Ala Thr Gly Val
305 310 315 320

Asp Ile Lys Val Glu Tyr Leu Asn Arg Arg Pro Gly Asp Tyr Ala Glu
325 330 335

Val Phe Ser Asp Pro Ser Lys Ile Lys Gln Glu Leu Asn Trp Lys Ala
340 345 350

Gln Tyr Thr Asp Leu Lys Lys Ser Leu Gln Ile Ala Trp Lys Trp Gln
355 360 365

Lys Ser His Leu Asn Gly Tyr Ser His Ser

370 375

<210> 252
<211> 1254
<212> DNA
<213> Populus tremuloides

<400> 252
atgctaaact ttggcaggac cagagctcag acaaggtcca atagatctat atctcttgga 60
ggcatggatt attcagatcc aaaaaggaaa aacaacgttg taggaaagat tcttttggct 120
gctaccctca cagctttatg tataattatg ctaaagcaat ctccgacctt ttattctcca 180
agcccgttct ctttgcatga agaaggggtg atccatgtcc tagtgacagg tggtgctggc 240
tacattggtt cccatgctgc attgcgactt ttgaaggatg gttaccgagt aaccatagtg 300
gacaaccttt ctcgaggaaa cataggtgca gtcaaggttt tacaagaatt atttccagag 360
cccgggaggc ttcagtttat atatgctgac ctgggagatc ctaaaactgt taacattatc 420
ttctcacaaa atgcatttga tgctgtgatg cattttgcgg cagttgcata tgttggggaa 480
agcaccatgg aacccctgaa gtactatcac aatatcacat caaatacctt ggtagttttg 540
gaggcaatgg ctgcaaatga tgtaaagact ttgatatatt caagtacatg tgcaacatat 600
ggggagcctg aaaagatgcc gattactgaa gtaactccac aggtacccat taatccatat 660
ggaaaagcta agaagatggc agaagatatc atccttgatt tttctaaaaa ctcagacatg 720
gcaattatga tattgagata cttcaatgtg attggatcag atccagatgg caggttaggt 780
gaggctccta gacctgaact gcgtgagcat ggacgaattt ccggtgcttg tttcgatgca 840
gctcgtggta ttattgctgg gctgaaggtt aaaggaacag actataagac gcatgatgga 900
acttgtataa gagattatat tgacgttact gatcttgttg atgctcatgt taaagcactt 960
gaaaaggcaa tgcctggtaa agtaggaatc tacaatgttg gcactggaaa gggtagatca 1020
gtcaaggagt ttgttaaggc atgtaaaaag gcaactggtg tagatatcaa agttgactat 1080
ttacctcgcc gtcctggtga ctacgctgaa gtattcagtg acccatcaaa aatttaccgc 1140
gagctgaact ggacagcgca atacacggat ctccagaaga gcttacagac tgcatggaga 1200
tggcaaaaat cacatcaaaa tgggtatgga tccccttttgg tgatggcttc ttga 1254

<210> 253
<211> 417
<212> PRT
<213> Populus tremuloides

<400> 253

Met Leu Asn Phe Gly Arg Thr Arg Ala Gln Thr Arg Ser Asn Arg Ser
1 5 10 15

Ile Ser Leu Gly Gly Met Asp Tyr Ser Asp Pro Lys Arg Lys Asn Asn
20 25 30

Val Val Gly Lys Ile Leu Leu Ala Ala Thr Leu Thr Ala Leu Cys Ile
35 40 45

```
Ile Met Leu Lys Gln Ser Pro Thr Phe Tyr Ser Pro Ser Pro Phe Ser
    50                  55                  60

Leu His Glu Glu Gly Val Ile His Val Leu Val Thr Gly Gly Ala Gly
65                  70                  75                  80

Tyr Ile Gly Ser His Ala Ala Leu Arg Leu Leu Lys Asp Gly Tyr Arg
                85                  90                  95

Val Thr Ile Val Asp Asn Leu Ser Arg Gly Asn Ile Gly Ala Val Lys
            100                 105                 110

Val Leu Gln Glu Leu Phe Pro Glu Pro Gly Arg Leu Gln Phe Ile Tyr
        115                 120                 125

Ala Asp Leu Gly Asp Pro Lys Thr Val Asn Ile Ile Phe Ser Gln Asn
    130                 135                 140

Ala Phe Asp Ala Val Met His Phe Ala Ala Val Ala Tyr Val Gly Glu
145                 150                 155                 160

Ser Thr Met Glu Pro Leu Lys Tyr Tyr His Asn Ile Thr Ser Asn Thr
                165                 170                 175

Leu Val Val Leu Glu Ala Met Ala Ala Asn Asp Val Lys Thr Leu Ile
            180                 185                 190

Tyr Ser Ser Thr Cys Ala Thr Tyr Gly Glu Pro Glu Lys Met Pro Ile
        195                 200                 205

Thr Glu Val Thr Pro Gln Val Pro Ile Asn Pro Tyr Gly Lys Ala Lys
    210                 215                 220

Lys Met Ala Glu Asp Ile Ile Leu Asp Phe Ser Lys Asn Ser Asp Met
225                 230                 235                 240

Ala Ile Met Ile Leu Arg Tyr Phe Asn Val Ile Gly Ser Asp Pro Asp
                245                 250                 255

Gly Arg Leu Gly Glu Ala Pro Arg Pro Glu Leu Arg Glu His Gly Arg
            260                 265                 270

Ile Ser Gly Ala Cys Phe Asp Ala Ala Arg Gly Ile Ile Ala Gly Leu
        275                 280                 285

Lys Val Lys Gly Thr Asp Tyr Lys Thr His Asp Gly Thr Cys Ile Arg
    290                 295                 300

Asp Tyr Ile Asp Val Thr Asp Leu Val Asp Ala His Val Lys Ala Leu
305                 310                 315                 320
```

```
Glu Lys Ala Met Pro Gly Lys Val Gly Ile Tyr Asn Val Gly Thr Gly
                325                 330                 335

Lys Gly Arg Ser Val Lys Glu Phe Val Lys Ala Cys Lys Lys Ala Thr
            340                 345                 350

Gly Val Asp Ile Lys Val Asp Tyr Leu Pro Arg Arg Pro Gly Asp Tyr
        355                 360                 365

Ala Glu Val Phe Ser Asp Pro Ser Lys Ile Tyr Arg Glu Leu Asn Trp
    370                 375                 380

Thr Ala Gln Tyr Thr Asp Leu Gln Lys Ser Leu Gln Thr Ala Trp Arg
385                 390                 395                 400

Trp Gln Lys Ser His Gln Asn Gly Tyr Gly Ser Pro Leu Val Met Ala
                405                 410                 415

Ser


<210>  254
<211>  1233
<212>  DNA
<213>  Populus tremuloides

<400>  254
atggtgcaca agggctctgg tatggatttc ctggattcaa gaagaaagag caattctgct      60
ggaaaagtta ttgcagttgc attcttcatt gcagtatgca ttgtcatgct caagcaagtg     120
tactcaccta gttataccag ccccgacatg ttctcccaac atgagctagg cgtaacgcat     180
gtgttggtga ccggaggtgc tggttacata ggttctcacg ctgcacttcg actcttgaag     240
gattcatacc gagtaactat agtggacaat ctctctcgag ggaatctcgg tgcggttaaa     300
gttcttcaag agctatttcc agagcctggg agattgcagt tcatatatgc tgacctagga     360
gatgcaaaag ctgtaaacaa aatctttgcc gaaaatgcat ttgatgctgt gatgcatttt     420
gctgctgttg catatgttgg tgaaagtaca atagagcccc ttagatatta tcacaatatc     480
acatcaaata ccttggttgt cttggaagca atggctgcac ataatgtgaa gacattaatc     540
tattcgagca cttgtgcaac gtacggagaa cctattaaga tgccgattag agaagaaact     600
cctcagcttc caatcaaccc ctatggaaaa gccaagaaaa tggcagaaga tatcataatt     660
gacttctcca acaccactga catggctgtc atgatcctaa gcatccagca ttctgatcaa     720
aataatgcct atgtttacag atacttcaat gttattgggt ctgacccgga aggaagatta     780
ggggaagctc cacgacctga gcttcgtgag catggccgaa tctctggtgc ttgttttgat     840
gcagctcgag ggataacacc tggactcaag gttaaaggga cagactataa aacagctgat     900
ggcacatgtg tacgcgacta tatcgatgtc actgacctgg ttgatgccca tgtgaaagct     960
cttgcccacg caaaacctcg gaaagttggc atttacaatg ttggaactgg aaaaggcaga    1020
```

```
tcagtgaagg agtttgttga tgcgtgtaag aaggcaacag gcgtggacat aaaggttgaa   1080
taccttgatc gccggccagg agactatgct gaggtcttca gtgatccttc caaaataaaa   1140
caagagctaa gctggacagc tcaatatact gaccttcaga agagtttaca gattgcttgg   1200
aaatggcaga agtcacattt gaatggttat tga                                1233

<210>  255
<211>  410
<212>  PRT
<213>  Populus tremuloides

<400>  255

Met Val His Lys Gly Ser Gly Met Asp Phe Leu Asp Ser Arg Arg Lys
1               5                   10                  15

Ser Asn Ser Ala Gly Lys Val Ile Ala Val Ala Phe Phe Ile Ala Val
            20                  25                  30

Cys Ile Val Met Leu Lys Gln Val Tyr Ser Pro Ser Tyr Thr Ser Pro
        35                  40                  45

Asp Met Phe Ser Gln His Glu Leu Gly Val Thr His Val Leu Val Thr
    50                  55                  60

Gly Gly Ala Gly Tyr Ile Gly Ser His Ala Ala Leu Arg Leu Leu Lys
65                  70                  75                  80

Asp Ser Tyr Arg Val Thr Ile Val Asp Asn Leu Ser Arg Gly Asn Leu
                85                  90                  95

Gly Ala Val Lys Val Leu Gln Glu Leu Phe Pro Glu Pro Gly Arg Leu
            100                 105                 110

Gln Phe Ile Tyr Ala Asp Leu Gly Asp Ala Lys Ala Val Asn Lys Ile
        115                 120                 125

Phe Ala Glu Asn Ala Phe Asp Ala Val Met His Phe Ala Ala Val Ala
    130                 135                 140

Tyr Val Gly Glu Ser Thr Ile Glu Pro Leu Arg Tyr Tyr His Asn Ile
145                 150                 155                 160

Thr Ser Asn Thr Leu Val Val Leu Glu Ala Met Ala Ala His Asn Val
                165                 170                 175

Lys Thr Leu Ile Tyr Ser Ser Thr Cys Ala Thr Tyr Gly Glu Pro Ile
            180                 185                 190

Lys Met Pro Ile Arg Glu Glu Thr Pro Gln Leu Pro Ile Asn Pro Tyr
        195                 200                 205
```

Gly Lys Ala Lys Lys Met Ala Glu Asp Ile Ile Ile Asp Phe Ser Asn
    210                 215                 220

Thr Thr Asp Met Ala Val Met Ile Leu Ser Ile Gln His Ser Asp Gln
225                 230                 235                 240

Asn Asn Ala Tyr Val Tyr Arg Tyr Phe Asn Val Ile Gly Ser Asp Pro
                245                 250                 255

Glu Gly Arg Leu Gly Glu Ala Pro Arg Pro Glu Leu Arg Glu His Gly
            260                 265                 270

Arg Ile Ser Gly Ala Cys Phe Asp Ala Ala Arg Gly Ile Thr Pro Gly
        275                 280                 285

Leu Lys Val Lys Gly Thr Asp Tyr Lys Thr Ala Asp Gly Thr Cys Val
    290                 295                 300

Arg Asp Tyr Ile Asp Val Thr Asp Leu Val Asp Ala His Val Lys Ala
305                 310                 315                 320

Leu Ala His Ala Lys Pro Arg Lys Val Gly Ile Tyr Asn Val Gly Thr
                325                 330                 335

Gly Lys Gly Arg Ser Val Lys Glu Phe Val Asp Ala Cys Lys Lys Ala
            340                 345                 350

Thr Gly Val Asp Ile Lys Val Glu Tyr Leu Asp Arg Arg Pro Gly Asp
        355                 360                 365

Tyr Ala Glu Val Phe Ser Asp Pro Ser Lys Ile Lys Gln Glu Leu Ser
    370                 375                 380

Trp Thr Ala Gln Tyr Thr Asp Leu Gln Lys Ser Leu Gln Ile Ala Trp
385                 390                 395                 400

Lys Trp Gln Lys Ser His Leu Asn Gly Tyr
                405                 410

<210> 256
<211> 627
<212> DNA
<213> Populus tremuloides

<400> 256
atggctggac aaacaattct tgtgactggt ggggctgggt ttattggcac tcacacagta 60

gtacagctcc taaaggaagg ttttaaggtt tcaatcattg acaatcttga taattctgtc 120

actgaagctg ttgatcgtgt taaggaagtg gtgggtcctc agctttctaa gaatcttgaa 180

ttcaatctgg gtgatcttag aaacaaggat gatttggaga agttgttttc aagaactaag 240

ttcgatgctg tgatccattt tgctggtctc aaggcagttg gggagagtgt tgctaatcca 300

cgtagatatt ttgacaataa tctggttggc accatcaatc tctatgaagt tatggcaaaa 360

```
tacaattgca agaagatggt tttctcatca tcagcaactg tttatggcca acctgaaaaa    420
attccttgtg ttgaggactt caacctaatg gcaatgaatc cttatggacg taccaagaaa    480
ttgctcgaga tattcaaaag gcagaaccag aatggagtat cattttactt aggtacttca    540
accccgtggg agctcatgag agtggtaaac ttggtgaaga tcccaagggt atcccaaaca    600
atctcatgcc ctacatacaa caagtag                                          627
```

<210> 257
<211> 208
<212> PRT
<213> Populus tremuloides

<400> 257

```
Met Ala Gly Gln Thr Ile Leu Val Thr Gly Gly Ala Gly Phe Ile Gly
1               5                   10                  15

Thr His Thr Val Val Gln Leu Leu Lys Glu Gly Phe Lys Val Ser Ile
            20                  25                  30

Ile Asp Asn Leu Asp Asn Ser Val Thr Glu Ala Val Asp Arg Val Lys
        35                  40                  45

Glu Val Val Gly Pro Gln Leu Ser Lys Asn Leu Glu Phe Asn Leu Gly
    50                  55                  60

Asp Leu Arg Asn Lys Asp Asp Leu Glu Lys Leu Phe Ser Arg Thr Lys
65                  70                  75                  80

Phe Asp Ala Val Ile His Phe Ala Gly Leu Lys Ala Val Gly Glu Ser
                85                  90                  95

Val Ala Asn Pro Arg Arg Tyr Phe Asp Asn Asn Leu Val Gly Thr Ile
            100                 105                 110

Asn Leu Tyr Glu Val Met Ala Lys Tyr Asn Cys Lys Lys Met Val Phe
        115                 120                 125

Ser Ser Ser Ala Thr Val Tyr Gly Gln Pro Glu Lys Ile Pro Cys Val
    130                 135                 140

Glu Asp Phe Asn Leu Met Ala Met Asn Pro Tyr Gly Arg Thr Lys Lys
145                 150                 155                 160

Leu Leu Glu Ile Phe Lys Arg Gln Asn Gln Asn Gly Val Ser Phe Tyr
                165                 170                 175

Leu Gly Thr Ser Thr Pro Trp Glu Leu Met Arg Val Val Asn Leu Val
            180                 185                 190

Lys Ile Pro Arg Val Ser Gln Thr Ile Ser Cys Pro Thr Tyr Asn Lys
        195                 200                 205
```

<210> 258
<211> 1047
<212> DNA
<213> Populus tremuloides

<400> 258
atggcgaaga gtatcttggt taccggcggt gccggttaca tagggagcca tacggtgctg 60
cagcttttac ttggcggtta cagcatcgtg gttgttgata atctcgacaa ctcctctgat 120
attgctctta aaagagttaa agaactcgcc ggtgatttcg gtaaaaacct cgtctttcac 180
caggttgatc tccgggataa gccagcactg gaaaaaattt ttgccaggac aaaatttgat 240
gctgtcattc actttgctgg gctgaaagcg gttggtgaga gtgtgcagaa accattgctt 300
tactttaaca acaatctcat tggaacaatt accctgctag aagttatgac ttcccatgga 360
tgcaagcagt tggtgttttc atcttcggct actgtttatg gttgcccaaa ggaggttcca 420
tgtacagaag agtttccttt gtctgctgcc agcccatacg gaagaaccaa gctcttcatt 480
gaagggatct gctgtgatat ccaccgttca gactctgaat ggaagatcat tttgcttaga 540
tacttcaatc cagttggtgc acatccaagt ggtcatattg gtgaggatcc acttggaatt 600
ccaaacaatc tcatgcccta tgtgcagcaa gttgctgttg gcaggcggcc tcatctaacc 660
gtttatggaa ctgattattc aactaaagat ggcactgggg tacgtgatta cattcatgtt 720
gttgatttag cggatgggca cattgctgca ttgcgtaagc tctctgatgc taatataggt 780
tgtgaagtgt acaacttggg aacaggaaaa ggtacatccg ttctggagat ggttgcggca 840
tttgaaaagg catctagaaa gaaaattccc cttgtaatgg ccgctcggcg acctggtgat 900
gctgaaattg tgtatgcagc aacagagaag gcagaacgtg aattgaattg gaaggcaaaa 960
tatggcattg atgagatgtg tagggatcaa tggaactggg ccggcaagaa cccttatggc 1020
tatggatctt ctgacagcac taactaa 1047

<210> 259
<211> 348
<212> PRT
<213> Populus tremuloides

<400> 259

Met Ala Lys Ser Ile Leu Val Thr Gly Gly Ala Gly Tyr Ile Gly Ser
1 5 10 15

His Thr Val Leu Gln Leu Leu Leu Gly Gly Tyr Ser Ile Val Val Val
20 25 30

Asp Asn Leu Asp Asn Ser Ser Asp Ile Ala Leu Lys Arg Val Lys Glu
35 40 45

Leu Ala Gly Asp Phe Gly Lys Asn Leu Val Phe His Gln Val Asp Leu
50 55 60

Arg Asp Lys Pro Ala Leu Glu Lys Ile Phe Ala Arg Thr Lys Phe Asp
65 70 75 80

Ala Val Ile His Phe Ala Gly Leu Lys Ala Val Gly Glu Ser Val Gln
85 90 95

Lys Pro Leu Leu Tyr Phe Asn Asn Asn Leu Ile Gly Thr Ile Thr Leu
100 105 110

Leu Glu Val Met Thr Ser His Gly Cys Lys Gln Leu Val Phe Ser Ser
115 120 125

Ser Ala Thr Val Tyr Gly Cys Pro Lys Glu Val Pro Cys Thr Glu Glu
130 135 140

Phe Pro Leu Ser Ala Ala Ser Pro Tyr Gly Arg Thr Lys Leu Phe Ile
145 150 155 160

Glu Gly Ile Cys Cys Asp Ile His Arg Ser Asp Ser Glu Trp Lys Ile
165 170 175

Ile Leu Leu Arg Tyr Phe Asn Pro Val Gly Ala His Pro Ser Gly His
180 185 190

Ile Gly Glu Asp Pro Leu Gly Ile Pro Asn Asn Leu Met Pro Tyr Val
195 200 205

Gln Gln Val Ala Val Gly Arg Arg Pro His Leu Thr Val Tyr Gly Thr
210 215 220

Asp Tyr Ser Thr Lys Asp Gly Thr Gly Val Arg Asp Tyr Ile His Val
225 230 235 240

Val Asp Leu Ala Asp Gly His Ile Ala Ala Leu Arg Lys Leu Ser Asp
245 250 255

Ala Asn Ile Gly Cys Glu Val Tyr Asn Leu Gly Thr Gly Lys Gly Thr
260 265 270

Ser Val Leu Glu Met Val Ala Ala Phe Glu Lys Ala Ser Arg Lys Lys
275 280 285

Ile Pro Leu Val Met Ala Ala Arg Arg Pro Gly Asp Ala Glu Ile Val
290 295 300

Tyr Ala Ala Thr Glu Lys Ala Glu Arg Glu Leu Asn Trp Lys Ala Lys
305 310 315 320

Tyr Gly Ile Asp Glu Met Cys Arg Asp Gln Trp Asn Trp Ala Gly Lys
325 330 335

Asn Pro Tyr Gly Tyr Gly Ser Ser Asp Ser Thr Asn
340 345

<210> 260
<211> 990
<212> DNA
<213> Populus tremuloides

<400> 260
atggcctata atattctggt tacaggcggt gctggttaca taggaagcca cacggtgctg 60
caacttttat taggcggcta caacactgtg gttgttgata acctcgacaa cgcctctgat 120
attgctctta aaagagttaa agaactcgcc ggtgatttcg gcaaaaacct cgtctttcac 180
caggttgatc tccgggacaa gccggctctg gaaaatgttt tcgccgagac aaagtttgat 240
gctgtcattc actttgctgg gctgaaagca gttggtgaga gtatgcagaa accattgctt 300
tatttcaaca ataatctcat tggaacaatt actctgctag aagttatggc tgctcatgga 360
tgcaagcagg taattttctc taggttgctt tgcattggtg ttttcatctt cagctactgt 420
ttatggttgg ccgaaggagg ttccatgtac agaagagttc cctttgtctg ctgcaaaccc 480
atatggaaga accaagagat ctgccgcgat atctacagtt cagattctga atggaagatc 540
atattactca gatactttaa tccagttggt gcacatccaa gtggctatat tggtgaggat 600
cctcgtggaa ttccaaacaa tctcatgccc tatgtgcagc aagttgctgt tggcaggagg 660
cctcatctaa cagtttttgg aactgattat ccaacaaaag acggtaccgg ggtacgtgat 720
tacattcatg ttgtcgattt agcagatggg cacattgctg cattgcgtaa gctctctgaa 780
gctaatatag gttgtgaagt gtacaacttg ggaacaggga aaggtacatc agttctggag 840
atggtcgcag catttgaaaa ggcatctgga aagagtccca tcttcgtgtg ccagaaaatt 900
cctcttgtaa tggctgatcg gcgacctggt gatgctgaaa ctgtgtatgc agcaacagag 960
aaggcagaac gtgaattgag ttggaagtaa 990

<210> 261
<211> 329
<212> PRT
<213> Populus tremuloides

<400> 261

Met Ala Tyr Asn Ile Leu Val Thr Gly Gly Ala Gly Tyr Ile Gly Ser
1 5 10 15

His Thr Val Leu Gln Leu Leu Leu Gly Gly Tyr Asn Thr Val Val Val
20 25 30

Asp Asn Leu Asp Asn Ala Ser Asp Ile Ala Leu Lys Arg Val Lys Glu
35 40 45

Leu Ala Gly Asp Phe Gly Lys Asn Leu Val Phe His Gln Val Asp Leu
50 55 60

Arg Asp Lys Pro Ala Leu Glu Asn Val Phe Ala Glu Thr Lys Phe Asp

65 70 75 80

Ala Val Ile His Phe Ala Gly Leu Lys Ala Val Gly Glu Ser Met Gln
85 90 95

Lys Pro Leu Leu Tyr Phe Asn Asn Asn Leu Ile Gly Thr Ile Thr Leu
100 105 110

Leu Glu Val Met Ala Ala His Gly Cys Lys Gln Val Ile Phe Ser Arg
115 120 125

Leu Leu Cys Ile Gly Val Phe Ile Phe Ser Tyr Cys Leu Trp Leu Ala
130 135 140

Glu Gly Gly Ser Met Tyr Arg Arg Val Pro Phe Val Cys Cys Lys Pro
145 150 155 160

Ile Trp Lys Asn Gln Glu Ile Cys Arg Asp Ile Tyr Ser Ser Asp Ser
165 170 175

Glu Trp Lys Ile Ile Leu Leu Arg Tyr Phe Asn Pro Val Gly Ala His
180 185 190

Pro Ser Gly Tyr Ile Gly Glu Asp Pro Arg Gly Ile Pro Asn Asn Leu
195 200 205

Met Pro Tyr Val Gln Gln Val Ala Val Gly Arg Arg Pro His Leu Thr
210 215 220

Val Phe Gly Thr Asp Tyr Pro Thr Lys Asp Gly Thr Gly Val Arg Asp
225 230 235 240

Tyr Ile His Val Val Asp Leu Ala Asp Gly His Ile Ala Ala Leu Arg
245 250 255

Lys Leu Ser Glu Ala Asn Ile Gly Cys Glu Val Tyr Asn Leu Gly Thr
260 265 270

Gly Lys Gly Thr Ser Val Leu Glu Met Val Ala Ala Phe Glu Lys Ala
275 280 285

Ser Gly Lys Ser Pro Ile Phe Val Cys Gln Lys Ile Pro Leu Val Met
290 295 300

Ala Asp Arg Arg Pro Gly Asp Ala Glu Thr Val Tyr Ala Ala Thr Glu
305 310 315 320

Lys Ala Glu Arg Glu Leu Ser Trp Lys
325

<210> 262
<211> 1254

<212> DNA
<213> Populus tremuloides

<400> 262
atgctaagtt ttggcaggac cagaactcag ccaaggtcca atagatctat gtcccttgga 60

ggcatggatt tttcagatcc aaaaagaaaa aataatgttg taggaaagat tcttttggcc 120

gcttccctaa cagctgtatg tataattatg ctgaaacaat ctccaacctt taattctcca 180

agcccgttct ctttgcgtga agatggggtg atccatgtcc ttgtgacagg tggtgctggc 240

tacattggtt cccatgcagc attgcgactt ttgaaggatg gttaccgagt aaccatagtg 300

gacaaccttt ctcgaggaaa cttaggtgca gtcaaggttt tacaagagtt atttcctgag 360

cctgggaggc ttcagtttat atatgctgac ttgggagagc ctaaaactgt taacagcatc 420

ttttcacaaa atgcatttga tgctgtgatg cattttgcag cagttgcata tgttggggaa 480

agcaccgtgt acccccttaa gtactatcac aacattacat caaataccttt ggtagtgttg 540

gagtcaatgg ctgcaaatga tgtaaagact ttgatatatt caagcacatg tgcaacatat 600

ggggagcctg aaaagatgcc tattactgaa gacactccac aggtacccat taatccatat 660

ggaaaagcta agaagatggc agaagatatc atccttgact tctctaaaaa ttcagacatg 720

gcaattatga tattgagata cttcaatgtg attggatcag atccagatgg aaggttaggt 780

gaggctccta gacctgaact gcgtgagcac ggacgaattt ctggtgcttg ttttgatgct 840

gctcgtggta ttgttgctgg actaaaggtt aaaggaacgg actataagac acacgatgga 900

acttgtataa gagattatat cgatgttact gatcttgttg atgctcatgt taaagcactt 960

gaaaaggcaa tgcctgggaa agttggaatc tacaatgttg gcactggaat gggtagatca 1020

gtcaacgagt ttgtacatgc atgtaaaaag gcaaccggtg tagatatcaa agtagactat 1080

ttacctcgcc ggcctggtga ctatgctgaa gtgtttagtg acccatcaaa aattaaccgt 1140

gagctgaact ggacagcaca atacactgat ctccaaaaga gtttacaggt tgcatggaga 1200

tggcaaaaat cacatcaaaa tgggtatgga tccccttttgg tgatggcttc ttga 1254

<210> 263
<211> 417
<212> PRT
<213> Populus tremuloides

<400> 263

Met Leu Ser Phe Gly Arg Thr Arg Thr Gln Pro Arg Ser Asn Arg Ser
1 5 10 15

Met Ser Leu Gly Gly Met Asp Phe Ser Asp Pro Lys Arg Lys Asn Asn
20 25 30

Val Val Gly Lys Ile Leu Leu Ala Ala Ser Leu Thr Ala Val Cys Ile
35 40 45

Ile Met Leu Lys Gln Ser Pro Thr Phe Asn Ser Pro Ser Pro Phe Ser
50 55 60

```
Leu Arg Glu Asp Gly Val Ile His Val Leu Val Thr Gly Gly Ala Gly
65                  70                  75                  80

Tyr Ile Gly Ser His Ala Ala Leu Arg Leu Leu Lys Asp Gly Tyr Arg
                85                  90                  95

Val Thr Ile Val Asp Asn Leu Ser Arg Gly Asn Leu Gly Ala Val Lys
            100                 105                 110

Val Leu Gln Glu Leu Phe Pro Glu Pro Gly Arg Leu Gln Phe Ile Tyr
        115                 120                 125

Ala Asp Leu Gly Glu Pro Lys Thr Val Asn Ser Ile Phe Ser Gln Asn
    130                 135                 140

Ala Phe Asp Ala Val Met His Phe Ala Ala Val Ala Tyr Val Gly Glu
145                 150                 155                 160

Ser Thr Val Tyr Pro Leu Lys Tyr Tyr His Asn Ile Thr Ser Asn Thr
                165                 170                 175

Leu Val Val Leu Glu Ser Met Ala Ala Asn Asp Val Lys Thr Leu Ile
            180                 185                 190

Tyr Ser Ser Thr Cys Ala Thr Tyr Gly Glu Pro Glu Lys Met Pro Ile
        195                 200                 205

Thr Glu Asp Thr Pro Gln Val Pro Ile Asn Pro Tyr Gly Lys Ala Lys
    210                 215                 220

Lys Met Ala Glu Asp Ile Ile Leu Asp Phe Ser Lys Asn Ser Asp Met
225                 230                 235                 240

Ala Ile Met Ile Leu Arg Tyr Phe Asn Val Ile Gly Ser Asp Pro Asp
                245                 250                 255

Gly Arg Leu Gly Glu Ala Pro Arg Pro Glu Leu Arg Glu His Gly Arg
            260                 265                 270

Ile Ser Gly Ala Cys Phe Asp Ala Ala Arg Gly Ile Val Ala Gly Leu
        275                 280                 285

Lys Val Lys Gly Thr Asp Tyr Lys Thr His Asp Gly Thr Cys Ile Arg
    290                 295                 300

Asp Tyr Ile Asp Val Thr Asp Leu Val Asp Ala His Val Lys Ala Leu
305                 310                 315                 320

Glu Lys Ala Met Pro Gly Lys Val Gly Ile Tyr Asn Val Gly Thr Gly
                325                 330                 335
```

Met Gly Arg Ser Val Asn Glu Phe Val His Ala Cys Lys Lys Ala Thr
340 345 350

Gly Val Asp Ile Lys Val Asp Tyr Leu Pro Arg Arg Pro Gly Asp Tyr
355 360 365

Ala Glu Val Phe Ser Asp Pro Ser Lys Ile Asn Arg Glu Leu Asn Trp
370 375 380

Thr Ala Gln Tyr Thr Asp Leu Gln Lys Ser Leu Gln Val Ala Trp Arg
385 390 395 400

Trp Gln Lys Ser His Gln Asn Gly Tyr Gly Ser Pro Leu Val Met Ala
405 410 415

Ser

<210> 264
<211> 1575
<212> DNA
<213> Populus tremuloides

<400> 264
atgattctgg tcacaggcgg cgccggcttc atcggctcgc atagctgcgt ggaactcgcc 60
gctgcgggcg agccctacct gatctacgac aacttcagca acagcagccc cgatgtgctc 120
gaacgcatgg aacgcatcac gggccggcgc ccgctgtgcg tcgagggaga cgtgcgcgac 180
cgcgccgcgc tggaccggct gtttgccgag cactccatcc gcgaggtgat ccacttcgcc 240
gcgctcaagg ccgtgggcga gtccgtggcc cagccgctgc gctactacga acacaacgtg 300
ggcggcacgg tggccctgct gcaggccatg cggacggcgg gcgtgcgcag cctggtgttc 360
tcctcctcgg ccacggtcta cggcgatccg gccagcctgc cgatccgcga agactttccg 420
ctgtccgcca ccaaccccta cggccgcagc aagctgtgga tcgaggaaat gctggccgac 480
ctggaccggg ccgaggccgg ccagtggagc ctggcacggc tgcgctactt caaccccgtg 540
ggcgcccatg aaagcggcct gatcggcgag gacccgcgcg acatccccaa caacctcatg 600
ccctatgtgt cgcaggtcgc catcggccag cgccagcagc tcagtgtcta tggcgatgac 660
tatgcgacgc cggacggcac gggcgtgcgc gactacatcc acgtcaccga cctggcgcgc 720
ggccacctgg cggcgctgcg ctatctgcgc gagcagcagg gcctgctgac cgtgaacctg 780
ggcacggggc gcccggtctc ggtgctggag atggtcaagg ccttcgagcg cgccagcggc 840
cgccccgtgc cctaccagat cgtggcgcgc cgacccggcg acgtggccca gtgctgggcc 900
gaccccgccg aggccgagcg gctgctgggc tggaaggcca tgctggacct ggaccgcatc 960
gcctgcgggc gccgagggcg tggtatcttt tcgcccttgt ttcacgccct agccaagagt 1020
ctgcccatgc ttgccaaacg catcatcccc tgtcttgacg tcacgggagg gcgcgtcgtc 1080
aaaggcgtga acttcgtgga actgcgcgat gcgggcgacc cggtggagat cgccgcgcgc 1140

tacaacgccc agggcgcgga cgagctgacc ttcctggaca tcacggccac cagcgacggg 1200

cgcgacctga tcctgcccat catcgaatcg gtggccagcc aggtcttcat tccgctgacc 1260

gtgggtggtg gcgtgcgcac cgtcgaggac gtgcgccgcc tgctcaacgc gggcgccgac 1320

aagaccagct tcaactcggc cgccatcgcc aaccccgacg tgatcaatgc ggcctcggac 1380

aagtacggcg cgcaatgcat cgtcgtggcc atcgatgcca agcggcgcac ggccgaggac 1440

gagcagcgca tcggcgccga tggccgcgct gccggccccg gctgggacgt gtacagccac 1500

ggcggacgca aaaacaccgg cctggacgcc gtgcactcac gcgcgccgtc agcgacgccg 1560

tgcccgtgcc cgtga 1575

<210> 265
<211> 524
<212> PRT
<213> Populus tremuloides

<400> 265

Met Ile Leu Val Thr Gly Gly Ala Gly Phe Ile Gly Ser His Ser Cys
1 5 10 15

Val Glu Leu Ala Ala Ala Gly Glu Pro Tyr Leu Ile Tyr Asp Asn Phe
20 25 30

Ser Asn Ser Ser Pro Asp Val Leu Glu Arg Met Glu Arg Ile Thr Gly
35 40 45

Arg Arg Pro Leu Cys Val Glu Gly Asp Val Arg Asp Arg Ala Ala Leu
50 55 60

Asp Arg Leu Phe Ala Glu His Ser Ile Arg Glu Val Ile His Phe Ala
65 70 75 80

Ala Leu Lys Ala Val Gly Glu Ser Val Ala Gln Pro Leu Arg Tyr Tyr
85 90 95

Glu His Asn Val Gly Gly Thr Val Ala Leu Leu Gln Ala Met Arg Thr
100 105 110

Ala Gly Val Arg Ser Leu Val Phe Ser Ser Ser Ala Thr Val Tyr Gly
115 120 125

Asp Pro Ala Ser Leu Pro Ile Arg Glu Asp Phe Pro Leu Ser Ala Thr
130 135 140

Asn Pro Tyr Gly Arg Ser Lys Leu Trp Ile Glu Glu Met Leu Ala Asp
145 150 155 160

Leu Asp Arg Ala Glu Ala Gly Gln Trp Ser Leu Ala Arg Leu Arg Tyr
165 170 175

Phe Asn Pro Val Gly Ala His Glu Ser Gly Leu Ile Gly Glu Asp Pro

180 185 190

Arg Asp Ile Pro Asn Asn Leu Met Pro Tyr Val Ser Gln Val Ala Ile
195 200 205

Gly Gln Arg Gln Gln Leu Ser Val Tyr Gly Asp Asp Tyr Ala Thr Pro
210 215 220

Asp Gly Thr Gly Val Arg Asp Tyr Ile His Val Thr Asp Leu Ala Arg
225 230 235 240

Gly His Leu Ala Ala Leu Arg Tyr Leu Arg Glu Gln Gln Gly Leu Leu
245 250 255

Thr Val Asn Leu Gly Thr Gly Arg Pro Val Ser Val Leu Glu Met Val
260 265 270

Lys Ala Phe Glu Arg Ala Ser Gly Arg Pro Val Pro Tyr Gln Ile Val
275 280 285

Ala Arg Arg Pro Gly Asp Val Ala Gln Cys Trp Ala Asp Pro Ala Glu
290 295 300

Ala Glu Arg Leu Leu Gly Trp Lys Ala Met Leu Asp Leu Asp Arg Ile
305 310 315 320

Ala Cys Gly Arg Arg Gly Arg Gly Ile Phe Ser Pro Leu Phe His Ala
325 330 335

Leu Ala Lys Ser Leu Pro Met Leu Ala Lys Arg Ile Ile Pro Cys Leu
340 345 350

Asp Val Thr Gly Gly Arg Val Val Lys Gly Val Asn Phe Val Glu Leu
355 360 365

Arg Asp Ala Gly Asp Pro Val Glu Ile Ala Ala Arg Tyr Asn Ala Gln
370 375 380

Gly Ala Asp Glu Leu Thr Phe Leu Asp Ile Thr Ala Thr Ser Asp Gly
385 390 395 400

Arg Asp Leu Ile Leu Pro Ile Ile Glu Ser Val Ala Ser Gln Val Phe
405 410 415

Ile Pro Leu Thr Val Gly Gly Gly Val Arg Thr Val Glu Asp Val Arg
420 425 430

Arg Leu Leu Asn Ala Gly Ala Asp Lys Thr Ser Phe Asn Ser Ala Ala
435 440 445

Ile Ala Asn Pro Asp Val Ile Asn Ala Ala Ser Asp Lys Tyr Gly Ala

450 455 460

Gln Cys Ile Val Val Ala Ile Asp Ala Lys Arg Arg Thr Ala Glu Asp
465 470 475 480

Glu Gln Arg Ile Gly Ala Asp Gly Arg Ala Ala Gly Pro Gly Trp Asp
485 490 495

Val Tyr Ser His Gly Gly Arg Lys Asn Thr Gly Leu Asp Ala Val His
500 505 510

Ser Arg Ala Pro Ser Ala Thr Pro Cys Pro Cys Pro
515 520

<210> 266
<211> 1059
<212> DNA
<213> Ostreococcus tauri

<400> 266
atgacgtgga acgtcctcgt caccggtggc gccggataca tcggctcgca cacgtgcgtg 60
cggctcctgc aagcgggcgc gcgcgtcacc gtcgtggata actttgacaa ctcgtgcgcg 120
gaatctttaa agcgcgtgcg aaacatcgtg ggcgaggacg cgggggcgcg tttgacgcac 180
cacgaggttg attgctgcga taaagtcgcg ctcgatggcg tcttcgcgtc ggcgggggtg 240
acgttcgatg cggtgataca cttcgccggg ttgaaggcgg tgggcgagag cgtggccgag 300
ccgatgaagt attacgagaa taacatcgtg agcacgctgg tgctgtgcga gacgatggcg 360
aaacacgggt gtaagacgat tatttttagc tcgagcgcga ccgtgtacgg ggagccggcg 420
tcggtgccgt gcacggaaga tttcccgacg gcggcgttga acccgtacgg acggaccaag 480
ttgttcatcg aacacatttt gagcgatctc tacgtgagtg ataaggagtg gaaggtggcg 540
ctgttgcgat actttaaccc ggtcggtgcg cacgagagtg ggacgctggg ggaggatccc 600
aagggaatcc cgaataactt gatgccattc gtgcagcaag tcgccgtcgg tcgacgaccc 660
gagctcaacg tgtttgggaa cgactatcca accaaggatg gaaccggtcg acgtgattac 720
attcatgtcg tcgatttggc cgacggacac gtcgcggcgg taaagaaact cacgagtgat 780
cccgacgccg gtttgctcac cgtcaacctc gggacgggga agagcacgag cgtcttagag 840
ctcgtcgcgg cgttcgagaa ggcgtccggt aagaaaattc cgtgcaagat tgtcgatcgt 900
cgcgcaggtg acgccgcaga ggtgtacggg gcgacagata aggcgttcaa ggttctcggt 960
tggcgtgcac ttcgcaccat cgaagactgc tgcatcgatc agtggaagtg ggcgagctcg 1020
aacccgtacg ggtacgcagg taagcccgac gacgcgtaa 1059

<210> 267
<211> 352
<212> PRT
<213> Ostreococcus tauri

<400> 267

Met Thr Trp Asn Val Leu Val Thr Gly Gly Ala Gly Tyr Ile Gly Ser
1               5                   10                  15

His Thr Cys Val Arg Leu Leu Gln Ala Gly Ala Arg Val Thr Val Val
            20                  25                  30

Asp Asn Phe Asp Asn Ser Cys Ala Glu Ser Leu Lys Arg Val Arg Asn
        35                  40                  45

Ile Val Gly Glu Asp Ala Gly Ala Arg Leu Thr His His Glu Val Asp
    50                  55                  60

Cys Cys Asp Lys Val Ala Leu Asp Gly Val Phe Ala Ser Ala Gly Val
65                  70                  75                  80

Thr Phe Asp Ala Val Ile His Phe Ala Gly Leu Lys Ala Val Gly Glu
                85                  90                  95

Ser Val Ala Glu Pro Met Lys Tyr Tyr Glu Asn Asn Ile Val Ser Thr
            100                 105                 110

Leu Val Leu Cys Glu Thr Met Ala Lys His Gly Cys Lys Thr Ile Ile
        115                 120                 125

Phe Ser Ser Ser Ala Thr Val Tyr Gly Glu Pro Ala Ser Val Pro Cys
    130                 135                 140

Thr Glu Asp Phe Pro Thr Ala Ala Leu Asn Pro Tyr Gly Arg Thr Lys
145                 150                 155                 160

Leu Phe Ile Glu His Ile Leu Ser Asp Leu Tyr Val Ser Asp Lys Glu
                165                 170                 175

Trp Lys Val Ala Leu Leu Arg Tyr Phe Asn Pro Val Gly Ala His Glu
            180                 185                 190

Ser Gly Thr Leu Gly Glu Asp Pro Lys Gly Ile Pro Asn Asn Leu Met
        195                 200                 205

Pro Phe Val Gln Gln Val Ala Val Gly Arg Arg Pro Glu Leu Asn Val
    210                 215                 220

Phe Gly Asn Asp Tyr Pro Thr Lys Asp Gly Thr Gly Arg Arg Asp Tyr
225                 230                 235                 240

Ile His Val Val Asp Leu Ala Asp Gly His Val Ala Ala Val Lys Lys
                245                 250                 255

Leu Thr Ser Asp Pro Asp Ala Gly Leu Leu Thr Val Asn Leu Gly Thr
            260                 265                 270

Gly Lys Ser Thr Ser Val Leu Glu Leu Val Ala Ala Phe Glu Lys Ala

```
                275                 280                 285

Ser Gly Lys Lys Ile Pro Cys Lys Ile Val Asp Arg Arg Ala Gly Asp
    290                 295                 300

Ala Ala Glu Val Tyr Gly Ala Thr Asp Lys Ala Phe Lys Val Leu Gly
305                 310                 315                 320

Trp Arg Ala Leu Arg Thr Ile Glu Asp Cys Cys Ile Asp Gln Trp Lys
                325                 330                 335

Trp Ala Ser Ser Asn Pro Tyr Gly Tyr Ala Gly Lys Pro Asp Asp Ala
            340                 345                 350
```

```
<210>  268
<211>  2100
<212>  DNA
<213>  Ostreococcus tauri

<400>  268
atgacagctc agttacaaag tgaaagtact tctaaaattg ttttggttac aggtggtgct      60
ggatacattg gttcacacac tgtggtagag ctaattgaga atggatatga ctgtgttgtt     120
gctgataacc tgtcgaattc aacttatgat tctgtagcca ggttagaggt cttgaccaag     180
catcacattc ccttctatga ggttgatttg tgtgaccgaa aggtctgga aaaggttttc      240
aaagaatata aaattgattc ggtaattcac tttgctggtt taaaggctgt aggtgaatct     300
acacaaatcc cgctgagata ctatcacaat aacattttgg gaactgtcgt tttattagag     360
ttaatgcaac aatacaacgt ttccaaattt gttttttcat cttctgctac tgtctatggt     420
gatgctacga gattcccaaa tatgattcct atcccagaag aatgtccctt agggcctact     480
aatccgtatg gtcatacgaa atacgccatt gagaatatct tgaatgatct ttacaatagc     540
gacaaaaaaa gttggaagtt tgctatcttg cgttatttta acccaattgg cgcacatccc     600
tctggattaa tcggagaaga tccgctaggt ataccaaaca atttgttgcc atatatggct     660
caagtagctg ttggtaggcg cgagaagctt tacatcttcg gagacgatta tgattccaga     720
gatggtaccc cgatcaggga ttatatccac gtagttgatc tagcaaaagg tcatattgca     780
gccctgcaat acctagaggc ctacaatgaa aatgaaggtt tgtgtcgtga gtggaacttg     840
ggttccggta aaggttctac agtttttgaa gtttatcatg cattctgcaa agcttctggt     900
attgatcttc catacaaagt tacgggcaga agagcaggtg atgttttgaa cttgacggct     960
aaaccagata gggccaaacg cgaactgaaa tggcagaccg agttgcaggt tgaagactcc    1020
tgcaaggatt tatggaaatg gactactgag aatccttttg gttaccagtt aaggggtgtc    1080
gaggccagat tttccgctga agatatgcgt tatgacgcaa gatttgtgac tattggtgcc    1140
ggcaccagat ttcaagccac gtttgccaat ttgggcgcca gcattgttga cctgaaagtg    1200
aacggacaat cagttgttct tggctatgaa aatgaggaag ggtatttgaa tcctgatagt    1260
gcttatatag gcgccacgat cggcaggtat gctaatcgta tttcgaaggg taagtttagt    1320
```

```
ttatgcaaca aagactatca gttaaccgtt aataacggcg ttaatgcgaa tcatagtagt   1380
atcggttctt tccacagaaa aagatttttg ggacccatca ttcaaaatcc ttcaaaggat   1440
gtttttaccg ccgagtacat gctgatagat aatgagaagg acaccgaatt tccaggtgat   1500
ctattggtaa ccatacagta tactgtgaac gttgcccaaa aaagtttgga aatggtatat   1560
aaaggtaaat tgactgctgg tgaagcgacg ccaataaatt taacaaatca tagttatttc   1620
aatctgaaca agccatatgg agacactatt gagggtacgg agattatggt gcgttcaaaa   1680
aaatctgttg atgtcgacaa aaacatgatt cctacgggta atatcgtcga tagagaaatt   1740
gctaccttta actctacaaa gccaacggtc ttaggcccca aaaatcccca gtttgattgt   1800
tgttttgtgg tggatgaaaa tgctaagcca agtcaaatca atactctaaa caatgaattg   1860
acgcttattg tcaaggcttt tcatcccgat tccaatatta cattagaagt tttaagtaca   1920
gagccaactt atcaatttta taccggtgat ttcttgtctg ctggttacga agcaagacaa   1980
ggttttgcaa ttgagcctgg tagatacatt gatgctatca atcaagagaa ctggaaagat   2040
tgtgtaacct tgaaaaacgg tgaaacttac gggtccaaga ttgtctacag attttcctga   2100
```

<210> 269
<211> 699
<212> PRT
<213> Ostreococcus tauri

<400> 269

```
Met Thr Ala Gln Leu Gln Ser Glu Ser Thr Ser Lys Ile Val Leu Val
1               5                   10                  15

Thr Gly Gly Ala Gly Tyr Ile Gly Ser His Thr Val Val Glu Leu Ile
            20                  25                  30

Glu Asn Gly Tyr Asp Cys Val Val Ala Asp Asn Leu Ser Asn Ser Thr
        35                  40                  45

Tyr Asp Ser Val Ala Arg Leu Glu Val Leu Thr Lys His His Ile Pro
    50                  55                  60

Phe Tyr Glu Val Asp Leu Cys Asp Arg Lys Gly Leu Glu Lys Val Phe
65                  70                  75                  80

Lys Glu Tyr Lys Ile Asp Ser Val Ile His Phe Ala Gly Leu Lys Ala
                85                  90                  95

Val Gly Glu Ser Thr Gln Ile Pro Leu Arg Tyr Tyr His Asn Asn Ile
            100                 105                 110

Leu Gly Thr Val Val Leu Leu Glu Leu Met Gln Gln Tyr Asn Val Ser
        115                 120                 125

Lys Phe Val Phe Ser Ser Ser Ala Thr Val Tyr Gly Asp Ala Thr Arg
    130                 135                 140
```

Phe Pro Asn Met Ile Pro Ile Pro Glu Glu Cys Pro Leu Gly Pro Thr
145 150 155 160

Asn Pro Tyr Gly His Thr Lys Tyr Ala Ile Glu Asn Ile Leu Asn Asp
165 170 175

Leu Tyr Asn Ser Asp Lys Lys Ser Trp Lys Phe Ala Ile Leu Arg Tyr
180 185 190

Phe Asn Pro Ile Gly Ala His Pro Ser Gly Leu Ile Gly Glu Asp Pro
195 200 205

Leu Gly Ile Pro Asn Asn Leu Leu Pro Tyr Met Ala Gln Val Ala Val
210 215 220

Gly Arg Arg Glu Lys Leu Tyr Ile Phe Gly Asp Asp Tyr Asp Ser Arg
225 230 235 240

Asp Gly Thr Pro Ile Arg Asp Tyr Ile His Val Val Asp Leu Ala Lys
245 250 255

Gly His Ile Ala Ala Leu Gln Tyr Leu Glu Ala Tyr Asn Glu Asn Glu
260 265 270

Gly Leu Cys Arg Glu Trp Asn Leu Gly Ser Gly Lys Gly Ser Thr Val
275 280 285

Phe Glu Val Tyr His Ala Phe Cys Lys Ala Ser Gly Ile Asp Leu Pro
290 295 300

Tyr Lys Val Thr Gly Arg Arg Ala Gly Asp Val Leu Asn Leu Thr Ala
305 310 315 320

Lys Pro Asp Arg Ala Lys Arg Glu Leu Lys Trp Gln Thr Glu Leu Gln
325 330 335

Val Glu Asp Ser Cys Lys Asp Leu Trp Lys Trp Thr Thr Glu Asn Pro
340 345 350

Phe Gly Tyr Gln Leu Arg Gly Val Glu Ala Arg Phe Ser Ala Glu Asp
355 360 365

Met Arg Tyr Asp Ala Arg Phe Val Thr Ile Gly Ala Gly Thr Arg Phe
370 375 380

Gln Ala Thr Phe Ala Asn Leu Gly Ala Ser Ile Val Asp Leu Lys Val
385 390 395 400

Asn Gly Gln Ser Val Val Leu Gly Tyr Glu Asn Glu Glu Gly Tyr Leu
405 410 415

```
Asn Pro Asp Ser Ala Tyr Ile Gly Ala Thr Ile Gly Arg Tyr Ala Asn
            420                 425                 430

Arg Ile Ser Lys Gly Lys Phe Ser Leu Cys Asn Lys Asp Tyr Gln Leu
        435                 440                 445

Thr Val Asn Asn Gly Val Asn Ala Asn His Ser Ser Ile Gly Ser Phe
    450                 455                 460

His Arg Lys Arg Phe Leu Gly Pro Ile Ile Gln Asn Pro Ser Lys Asp
465                 470                 475                 480

Val Phe Thr Ala Glu Tyr Met Leu Ile Asp Asn Glu Lys Asp Thr Glu
                485                 490                 495

Phe Pro Gly Asp Leu Leu Val Thr Ile Gln Tyr Thr Val Asn Val Ala
            500                 505                 510

Gln Lys Ser Leu Glu Met Val Tyr Lys Gly Lys Leu Thr Ala Gly Glu
        515                 520                 525

Ala Thr Pro Ile Asn Leu Thr Asn His Ser Tyr Phe Asn Leu Asn Lys
    530                 535                 540

Pro Tyr Gly Asp Thr Ile Glu Gly Thr Glu Ile Met Val Arg Ser Lys
545                 550                 555                 560

Lys Ser Val Asp Val Asp Lys Asn Met Ile Pro Thr Gly Asn Ile Val
                565                 570                 575

Asp Arg Glu Ile Ala Thr Phe Asn Ser Thr Lys Pro Thr Val Leu Gly
            580                 585                 590

Pro Lys Asn Pro Gln Phe Asp Cys Cys Phe Val Val Asp Glu Asn Ala
        595                 600                 605

Lys Pro Ser Gln Ile Asn Thr Leu Asn Asn Glu Leu Thr Leu Ile Val
    610                 615                 620

Lys Ala Phe His Pro Asp Ser Asn Ile Thr Leu Glu Val Leu Ser Thr
625                 630                 635                 640

Glu Pro Thr Tyr Gln Phe Tyr Thr Gly Asp Phe Leu Ser Ala Gly Tyr
                645                 650                 655

Glu Ala Arg Gln Gly Phe Ala Ile Glu Pro Gly Arg Tyr Ile Asp Ala
            660                 665                 670

Ile Asn Gln Glu Asn Trp Lys Asp Cys Val Thr Leu Lys Asn Gly Glu
        675                 680                 685
```

```
Thr Tyr Gly Ser Lys Ile Val Tyr Arg Phe Ser
    690                 695
```

<210> 270
<211> 1024
<212> DNA
<213> Escherichia coli

<400> 270

```
atggctattc tcgttacagg tggtgctggt tatattgcat cacatactat actgagtttg      60
atagagcagg gtaatgaagt aattattatt gataatttat caaattcata ttatgattcg     120
ttattgaaaa taaaagagat tactggatgt gattgtcttt tttataaagg agatatcctt     180
gacaaaaaac tcttgctaaa aatatttgat gaaaataata tcactgctgt gatgcatttt     240
gctggtgcaa agtctgtaag tgaatctgtt atcagacctt tgcaatacta tcgaaataat     300
gtctctggca ctttttcttt agttgaggca atggaggagt ctggtgtaga aaatttaata     360
tttagctctt cagcaactgt ctatggtgag ccaaagatta ttcctgtgaa tgaaagttgt     420
gctataggtg gtactacaaa tccgtatgga acgtccaagt tatttgtaga gaactttttg     480
cgggattatt ctaaggcatc ccctaatttt aaaactatag tattaagata ttttaaccct     540
atcggtgctc actcatctgg aaaaataggt gaagatccta acggaattcc gaataacttg     600
atgcccttta tatgccaggt tgctatcggt aaacaaaaaa cgctcaagat ctacggaaat     660
gattatccta ctaaagatgg tactgggatt cgcgattata ttcatgttat ggatcttgct     720
gaaggtcatg ttgctgcatt aaataatatt aatcaaggag ctaattatag agtttacaac     780
cttggaacag gaattggata ctctgtgttg gaattattag aagcatttca gaaagttaca     840
actcggagag ttccctatgt ttttactaaa aggcgttcag gtgatattgc tgaatgttgg     900
tctgatccta ccaaagcgta tgaagaatta ggatggaaag caaggcgagg gttagaagat     960
atgattcgtg atgcatggaa ttggcagcaa aaaaatccaa atggatttaa gaaagtttaa    1020
gtaa                                                                 1024
```

<210> 271
<211> 339
<212> PRT
<213> Escherichia coli

<400> 271

```
Met Ala Ile Leu Val Thr Gly Gly Ala Gly Tyr Ile Ala Ser His Thr
1               5                   10                  15

Ile Leu Ser Leu Ile Glu Gln Gly Asn Glu Val Ile Ile Ile Asp Asn
            20                  25                  30

Leu Ser Asn Ser Tyr Tyr Asp Ser Leu Leu Lys Ile Lys Glu Ile Thr
        35                  40                  45

Gly Cys Asp Cys Leu Phe Tyr Lys Gly Asp Ile Leu Asp Lys Lys Leu
    50                  55                  60
```

Leu Leu Lys Ile Phe Asp Glu Asn Asn Ile Thr Ala Val Met His Phe
65 70 75 80

Ala Gly Ala Lys Ser Val Ser Glu Ser Val Ile Arg Pro Leu Gln Tyr
85 90 95

Tyr Arg Asn Asn Val Ser Gly Thr Phe Ser Leu Val Glu Ala Met Glu
100 105 110

Glu Ser Gly Val Glu Asn Leu Ile Phe Ser Ser Ser Ala Thr Val Tyr
115 120 125

Gly Glu Pro Lys Ile Ile Pro Val Asn Glu Ser Cys Ala Ile Gly Gly
130 135 140

Thr Thr Asn Pro Tyr Gly Thr Ser Lys Leu Phe Val Glu Asn Phe Leu
145 150 155 160

Arg Asp Tyr Ser Lys Ala Ser Pro Asn Phe Lys Thr Ile Val Leu Arg
165 170 175

Tyr Phe Asn Pro Ile Gly Ala His Ser Ser Gly Lys Ile Gly Glu Asp
180 185 190

Pro Asn Gly Ile Pro Asn Asn Leu Met Pro Phe Ile Cys Gln Val Ala
195 200 205

Ile Gly Lys Gln Lys Thr Leu Lys Ile Tyr Gly Asn Asp Tyr Pro Thr
210 215 220

Lys Asp Gly Thr Gly Ile Arg Asp Tyr Ile His Val Met Asp Leu Ala
225 230 235 240

Glu Gly His Val Ala Ala Leu Asn Asn Ile Asn Gln Gly Ala Asn Tyr
245 250 255

Arg Val Tyr Asn Leu Gly Thr Gly Ile Gly Tyr Ser Val Leu Glu Leu
260 265 270

Leu Glu Ala Phe Gln Lys Val Thr Thr Arg Arg Val Pro Tyr Val Phe
275 280 285

Thr Lys Arg Arg Ser Gly Asp Ile Ala Glu Cys Trp Ser Asp Pro Thr
290 295 300

Lys Ala Tyr Glu Glu Leu Gly Trp Lys Ala Arg Arg Gly Leu Glu Asp
305 310 315 320

Met Ile Arg Asp Ala Trp Asn Trp Gln Gln Lys Asn Pro Asn Gly Phe
325 330 335

Lys Lys Val

<210> 272
<211> 53
<212> DNA
<213> Artificial sequence

<220>
<223> primer 1

<400> 272
ggggacaagt ttgtacaaaa aagcaggctt aaacaatggt ttcggccttg ttg 53

<210> 273
<211> 49
<212> DNA
<213> Artificial sequence

<220>
<223> primer 2

<400> 273
ggggaccact ttgtacaaga aagctgggtg ctgctgctac tggaggatt 49

<210> 274
<211> 2194
<212> DNA
<213> Oryza sativa

<400> 274
aatccgaaaa gtttctgcac cgttttcacc ccctaactaa caatataggg aacgtgtgct 60

aaatataaaa tgagacctta tatatgtagc gctgataact agaactatgc aagaaaaact 120

catccaccta ctttagtggc aatcgggcta aataaaaaag agtcgctaca ctagtttcgt 180

tttccttagt aattaagtgg gaaaatgaaa tcattattgc ttagaatata cgttcacatc 240

tctgtcatga agttaaatta ttcgaggtag ccataattgt catcaaactc ttcttgaata 300

aaaaaatctt tctagctgaa ctcaatgggt aaagagagag atttttttta aaaaaataga 360

atgaagatat tctgaacgta ttggcaaaga tttaaacata taattatata attttatagt 420

ttgtgcattc gtcatatcgc acatcattaa ggacatgtct tactccatcc caatttttat 480

ttagtaatta aagacaattg acttattttt attatttatc tttttcgat tagatgcaag 540

gtacttacgc acacactttg tgctcatgtg catgtgtgag tgcacctcct caatacacgt 600

tcaactagca acacatctct aatatcactc gcctatttaa tacatttagg tagcaatatc 660

tgaattcaag cactccacca tcaccagacc acttttaata atatctaaaa tacaaaaaat 720

aattttacag aatagcatga aaagtatgaa acgaactatt taggtttttc acatacaaaa 780

aaaaaaagaa ttttgctcgt gcgcgagcgc caatctccca tattgggcac acaggcaaca 840

acagagtggc tgcccacaga acaacccaca aaaaacgatg atctaacgga ggacagcaag 900

tccgcaacaa ccttttaaca gcaggctttg cggccaggag agaggaggag aggcaaagaa 960

aaccaagcat cctccttctc ccatctataa attcctcccc ccttttcccc tctctatata 1020

ggaggcatcc aagccaagaa gagggagagc accaaggaca cgcgactagc agaagccgag 1080
cgaccgcctt ctcgatccat atcttccggt cgagttcttg gtcgatctct tccctcctcc 1140
acctcctcct cacagggtat gtgcctccct tcggttgttc ttggatttat tgttctaggt 1200
tgtgtagtac gggcgttgat gttaggaaag gggatctgta tctgtgatga ttcctgttct 1260
tggatttggg atagaggggt tcttgatgtt gcatgttatc ggttcggttt gattagtagt 1320
atggttttca atcgtctgga gagctctatg gaaatgaaat ggtttaggga tcggaatctt 1380
gcgattttgt gagtaccttt tgtttgaggt aaaatcagag caccggtgat tttgcttggt 1440
gtaataaagt acggttgttt ggtcctcgat tctggtagtg atgcttctcg atttgacgaa 1500
gctatccttt gtttattccc tattgaacaa aaataatcca actttgaaga cggtcccgtt 1560
gatgagattg aatgattgat tcttaagcct gtccaaaatt tcgcagctgg cttgtttaga 1620
tacagtagtc cccatcacga aattcatgga aacagttata atcctcagga acaggggatt 1680
ccctgttctt ccgatttgct ttagtcccag aatttttttt cccaaatatc ttaaaaagtc 1740
actttctggt tcagttcaat gaattgattg ctacaaataa tgcttttata gcgttatcct 1800
agctgtagtt cagttaatag gtaatacccc tatagtttag tcaggagaag aacttatccg 1860
atttctgatc tccattttta attatatgaa atgaactgta gcataagcag tattcatttg 1920
gattattttt tttattagct ctcacccctt cattattctg agctgaaagt ctggcatgaa 1980
ctgtcctcaa ttttgttttc aaattcacat cgattatcta tgcattatcc tcttgtatct 2040
acctgtagaa gtttcttttt ggttattcct tgactgcttg attacagaaa gaaatttatg 2100
aagctgtaat cgggatagtt atactgcttg ttcttatgat tcatttcctt tgtgcagttc 2160
ttggtgtagc ttgccacttt caccagcaaa gttc 2194

<210> 275
<211> 265
<212> PRT
<213> Artificial sequence

<220>
<223> epimerase domain in SEQ ID NO: 2

<400> 275

Ile Leu Val Thr Gly Gly Ala Gly Tyr Ile Gly Ser His Thr Val Leu
1 5 10 15

Gln Leu Leu Gln Leu Gly Phe Arg Val Val Val Leu Asp Asn Leu Asp
20 25 30

Asn Ala Ser Glu Leu Ala Ile Leu Arg Val Arg Glu Leu Ala Gly His
35 40 45

Asn Ala Asn Asn Leu Asp Phe Arg Lys Val Asp Leu Arg Asp Lys Gln
50 55 60

Ala Leu Asp Gln Ile Phe Ser Ser Gln Arg Phe Glu Ala Val Ile His
65 70 75 80

Phe Ala Gly Leu Lys Ala Val Gly Glu Ser Val Gln Lys Pro Leu Leu
85 90 95

Tyr Tyr Asp Asn Asn Leu Ile Gly Thr Ile Thr Leu Leu Gln Val Met
100 105 110

Ala Ala His Gly Cys Thr Lys Leu Val Phe Ser Ser Ser Ala Thr Val
115 120 125

Tyr Gly Trp Pro Lys Glu Val Pro Cys Thr Glu Glu Ser Pro Leu Cys
130 135 140

Ala Met Asn Pro Tyr Gly Arg Thr Lys Leu Val Ile Glu Asp Met Cys
145 150 155 160

Arg Asp Leu His Ala Ser Asp Pro Asn Trp Lys Ile Ile Leu Leu Arg
165 170 175

Tyr Phe Asn Pro Val Gly Ala His Pro Ser Gly Tyr Ile Gly Glu Asp
180 185 190

Pro Cys Gly Ile Pro Asn Asn Leu Met Pro Phe Val Gln Gln Val Ala
195 200 205

Val Gly Arg Arg Pro Ala Leu Thr Val Tyr Gly Thr Asp Tyr Asn Thr
210 215 220

Lys Asp Gly Thr Gly Val Arg Asp Tyr Ile His Val Val Asp Leu Ala
225 230 235 240

Asp Gly His Ile Ala Ala Leu Arg Lys Leu Tyr Glu Asp Ser Asp Arg
245 250 255

Ile Gly Cys Glu Val Tyr Asn Leu Gly
260 265

<210> 276
<211> 7
<212> PRT
<213> Artificial sequence

<220>
<223> Motif 1: NAD+ binding

<220>
<221> UNSURE
<222> (2)..(3)
<223> Xaa can be any naturally occurring amino acid

<220>
<221> UNSURE
<222> (5)..(6)
<223> Xaa can be any naturally occurring amino acid

<400> 276

Gly Xaa Xaa Gly Xaa Xaa Gly
1 5

<210> 277
<211> 4
<212> PRT
<213> Artificial sequence

<220>
<223> Motif 2: active site

<220>
<221> UNSURE
<222> (4)..(4)
<223> /replace = "Ser"

<400> 277

Tyr Gly Arg Thr
1

<210> 278
<211> 12
<212> PRT
<213> Artificial sequence

<220>
<223> Motif 3: conserved region

<400> 278

Leu Val Thr Gly Gly Ala Gly Tyr Ile Gly Ser His
1 5 10

<210> 279
<211> 5
<212> PRT
<213> Artificial sequence

<220>
<223> Motif 4: conserved region

<220>
<221> UNSURE
<222> (2)..(2)
<223> Xaa can be any naturally occurring amino acid

<220>
<221> VARIANT
<222> (4)..(4)
<223> /replace = "Leu"

<400> 279

Val Xaa His Phe Ala
1 5

<210> 280
<211> 11
<212> PRT
<213> Artificial sequence

<220>
<223> Motif 5: conserved region

<400> 280

Asp Gly Thr Gly Val Arg Asp Tyr Ile His Val
1 5 10

<210> 281
<211> 1047
<212> DNA
<213> Brassica napus

<400> 281
atggtgaaga acgttctcgt cacaggcggc gctggctaca tcggaagcca cacggtgctt 60

caactgctca acggtggata ctccgccgta gtcgtcgaca atcacgacaa ttcatccgct 120

gtttctctcc aacgcgtcaa aaaactcgcc ggcgataacg gaaaccgcct ctccttccac 180

caggtggatc tcagagacag gcctgcgctt gagaagattt tctctgaaac taagtttgat 240

gcagtgatac actttgctgg acttaaagca gtaggtgaga gtgtggagaa gcctttgctc 300

tattataata ataacctttt tggcactgtt atccttttgg aggttatgtc tcaattcggc 360

tgcaaaaatc ttgtgttttc gtcatcagct actgtctatg gctggccaaa agaggttcct 420

tgcaccgagg agtccccaat ttctgcaacc aacccttatg gccgtaccaa gctttttatc 480

gaggaaattt gccgggatgt acatcgctcc gaccctgaat ggaagatcat attgcttaga 540

tacttcaacc ctgttggtgc acatcctagt ggttacattg gtgaagatcc tcttggggtt 600

ccaaacaacc tcatgcctta tgtccaacaa gttgcagttg gccggagacc tcacctcacc 660

gtctttggaa ctgactataa cacaaaggat ggcacagggg tgagagatta cattcatgtg 720

attgacttag cagatggaca tatagccgct ctgcgcaagc tggatgatct caaaatcagt 780

tgtgaggtat acaatcttgg aacgggtaat ggaacatcag ttctagaaat ggttgctgcc 840

tttgagaagg catccggaaa gaaaatccct ttggtgttgg ctggacgacg tcctggagac 900

gctgagattg tttacgcctc aaccgagaaa gcagaacgcg agctaaactg gaaggctaag 960

tatgggattg aagacatgtg tagggatcta tggaactggg ccagcaataa tccttacggc 1020

tacgcctcct ccaatggctc ctcttaa 1047

<210> 282
<211> 348
<212> PRT
<213> Brassica napus

<400> 282

Met Val Lys Asn Val Leu Val Thr Gly Gly Ala Gly Tyr Ile Gly Ser
1 5 10 15

His Thr Val Leu Gln Leu Leu Asn Gly Gly Tyr Ser Ala Val Val Val
20 25 30

Asp Asn His Asp Asn Ser Ser Ala Val Ser Leu Gln Arg Val Lys Lys
35 40 45

Leu Ala Gly Asp Asn Gly Asn Arg Leu Ser Phe His Gln Val Asp Leu
50 55 60

Arg Asp Arg Pro Ala Leu Glu Lys Ile Phe Ser Glu Thr Lys Phe Asp
65 70 75 80

Ala Val Ile His Phe Ala Gly Leu Lys Ala Val Gly Glu Ser Val Glu
85 90 95

Lys Pro Leu Leu Tyr Tyr Asn Asn Asn Leu Phe Gly Thr Val Ile Leu
100 105 110

Leu Glu Val Met Ser Gln Phe Gly Cys Lys Asn Leu Val Phe Ser Ser
115 120 125

Ser Ala Thr Val Tyr Gly Trp Pro Lys Glu Val Pro Cys Thr Glu Glu
130 135 140

Ser Pro Ile Ser Ala Thr Asn Pro Tyr Gly Arg Thr Lys Leu Phe Ile
145 150 155 160

Glu Glu Ile Cys Arg Asp Val His Arg Ser Asp Pro Glu Trp Lys Ile
165 170 175

Ile Leu Leu Arg Tyr Phe Asn Pro Val Gly Ala His Pro Ser Gly Tyr
180 185 190

Ile Gly Glu Asp Pro Leu Gly Val Pro Asn Asn Leu Met Pro Tyr Val
195 200 205

Gln Gln Val Ala Val Gly Arg Arg Pro His Leu Thr Val Phe Gly Thr
210 215 220

Asp Tyr Asn Thr Lys Asp Gly Thr Gly Val Arg Asp Tyr Ile His Val
225 230 235 240

Ile Asp Leu Ala Asp Gly His Ile Ala Ala Leu Arg Lys Leu Asp Asp
245 250 255

Leu Lys Ile Ser Cys Glu Val Tyr Asn Leu Gly Thr Gly Asn Gly Thr
260 265 270

Ser Val Leu Glu Met Val Ala Ala Phe Glu Lys Ala Ser Gly Lys Lys
275 280 285

Ile Pro Leu Val Leu Ala Gly Arg Arg Pro Gly Asp Ala Glu Ile Val
290 295 300

Seite 295

Tyr Ala Ser Thr Glu Lys Ala Glu Arg Glu Leu Asn Trp Lys Ala Lys
305                 310                 315                 320

Tyr Gly Ile Glu Asp Met Cys Arg Asp Leu Trp Asn Trp Ala Ser Asn
                325                 330                 335

Asn Pro Tyr Gly Tyr Ala Ser Ser Asn Gly Ser Ser
            340                 345

<210> 283
<211> 1047
<212> DNA
<213> Brassica napus

<400> 283
atggtgaaga acgttctggt cacaggcggc gctggctaca tcggtagcca cacggtgctt 60
caacttctca acggaggcta ctccgtcgta gtcgtcgaca atcacgacaa ttcatccgct 120
gtttctctcc aacgcgtcaa aaaactcgcc ggcgataacg gaaaccgcct ctccttccac 180
caggtggatc tcagagacag gcctgcgctt gagaagattt tctcagaaac taagtttgat 240
gcagtgatac actttgctgg acttaaagca gtaggtgaga gtgtggagaa gccccctgctt 300
tattataata ataacttatt tggcactgtt atccttttgg aggttatgtc tcagttcggc 360
tgcaaaaatc ttgtgttttc gtcatcagct actgtctatg gctggccaaa agaggttcct 420
tgtaccgagg agtccccaat ttctgcaacc aacccttatg gccgtaccaa gctttttatc 480
gaggaaattt gccgggatgt acatcgctcc gaccctgaat ggaagatcat attgctcaga 540
tacttcaacc ctgttggtgc acatcctagt ggttacattg gtgaagatcc tcttggggtt 600
ccaaacaacc tcatgcctta tgtccaacaa gttgcagttg gccggagacc tcacctcacc 660
gtctttggaa ctgactataa cacaaaggat ggcacagggg tgagagatta cattcatgtg 720
attgacttag cagatggaca tatagccgct ctgcgcaagc tggatgatct caaaatcagt 780
tgtgaggtat acaatcttgg aacgggtaat ggaacatcag ttctagaaat ggttgctgcc 840
tttgagaagg catccggaaa gaaaatccct ttggtgttgg ctggacgacg tcctggagac 900
gctgagattg tttacgcctc aaccgagaaa gcagaacgcg agctaaactg gaaggctaag 960
tatgggattg aagacatgtg tagggatcta tggaactggg ccagcaataa tccttacggc 1020
tacgcctcct ccaatggctc ctcttaa 1047

<210> 284
<211> 348
<212> PRT
<213> Brassica napus

<400> 284

Met Val Lys Asn Val Leu Val Thr Gly Gly Ala Gly Tyr Ile Gly Ser
1               5                   10                  15

His Thr Val Leu Gln Leu Leu Asn Gly Gly Tyr Ser Val Val Val Val
            20                  25                  30

Asp Asn His Asp Asn Ser Ser Ala Val Ser Leu Gln Arg Val Lys Lys
35 40 45

Leu Ala Gly Asp Asn Gly Asn Arg Leu Ser Phe His Gln Val Asp Leu
50 55 60

Arg Asp Arg Pro Ala Leu Glu Lys Ile Phe Ser Glu Thr Lys Phe Asp
65 70 75 80

Ala Val Ile His Phe Ala Gly Leu Lys Ala Val Gly Glu Ser Val Glu
85 90 95

Lys Pro Leu Leu Tyr Tyr Asn Asn Asn Leu Phe Gly Thr Val Ile Leu
100 105 110

Leu Glu Val Met Ser Gln Phe Gly Cys Lys Asn Leu Val Phe Ser Ser
115 120 125

Ser Ala Thr Val Tyr Gly Trp Pro Lys Glu Val Pro Cys Thr Glu Glu
130 135 140

Ser Pro Ile Ser Ala Thr Asn Pro Tyr Gly Arg Thr Lys Leu Phe Ile

145 150 155 160

Glu Glu Ile Cys Arg Asp Val His Arg Ser Asp Pro Glu Trp Lys Ile
165 170 175

Ile Leu Leu Arg Tyr Phe Asn Pro Val Gly Ala His Pro Ser Gly Tyr
180 185 190

Ile Gly Glu Asp Pro Leu Gly Val Pro Asn Asn Leu Met Pro Tyr Val
195 200 205

Gln Gln Val Ala Val Gly Arg Arg Pro His Leu Thr Val Phe Gly Thr
210 215 220

Asp Tyr Asn Thr Lys Asp Gly Thr Gly Val Arg Asp Tyr Ile His Val
225 230 235 240

Ile Asp Leu Ala Asp Gly His Ile Ala Ala Leu Arg Lys Leu Asp Asp
245 250 255

Leu Lys Ile Ser Cys Glu Val Tyr Asn Leu Gly Thr Gly Asn Gly Thr
260 265 270

Ser Val Leu Glu Met Val Ala Ala Phe Glu Lys Ala Ser Gly Lys Lys
275 280 285

Ile Pro Leu Val Leu Ala Gly Arg Arg Pro Gly Asp Ala Glu Ile Val
290 295 300

Tyr Ala Ser Thr Glu Lys Ala Glu Arg Glu Leu Asn Trp Lys Ala Lys
305 310 315 320

Tyr Gly Ile Glu Asp Met Cys Arg Asp Leu Trp Asn Trp Ala Ser Asn
325 330 335

Asn Pro Tyr Gly Tyr Ala Ser Ser Asn Gly Ser Ser
340 345

<210> 285
<211> 1068
<212> DNA
<213> Zea mays

<400> 285
atggtgtccg ccgtgctccg gaccatcctc gtgacgggcg gcgccgggta catcggcagc 60

cacacggtgc tgcagctgct gcagcagggc ttccgcgtcg tcgtcgtcga caacctcgac 120

aacgcctccg aggccgccct cgcccgcgtc gccgagctcg ccgggcacga cggcgccaac 180

ctcgtcttcc acaaggttga ccttcgcgac aggcacgcgt tggtggacat cttctcgtcg 240

cacaggttcg aggctgtcat tcactttgct gggctcaagg ctgttgggga gagcgtgcac 300

aagcccctac tttactacga caacaacctg gtcggcacca tcaccctcct ggaggtgatg 360

gctgcgaacg gctgcaagaa gctggtgttc tcgtcatctg caactgtcta tgggtggccc 420

aaggaagtac cctgcaccga agaattcccg ctctgcgcca ccaatcccta tgggcggaca 480

aagcttgtga ttgaagacat ctgccgcgac gtccaccgct ccgaccccga ctggaagatc 540

atactgctca ggtacttcaa ccccgttggc gctcatccaa gcgggtacat cggcgaagac 600

ccctgcggtg tcccgaacaa cctgatgccc tacgtgcagc aagtcgctgt tgggaggtta 660

cctcacctca cggtctacgg gacggactac agcaccaagg atgggactgg ggtgcgtgat 720

tacatccacg ttgtcgacct ggctgacggc cacatagcag ccctgaggaa gctctacgaa 780

gactccgaca aaataggctg tgaagtgtac aacttgggga ctggaaaggg gacgtccgtg 840

ttggaaatgg tggctgcatt cgagaaggct tctgggaaga aaatccctct ggtgttcgct 900

gggcgaagac ccggagacgc agagatcgtc tacgccgcaa ctgccaaggc agagaaggag 960

ctcaaatgga aggccaagta cgggatcgag gagatgtgca gagatctgtg gaactgggcg 1020

agcaagaacc cgtacgggta cgctgggtca cgcgacaaca gcaaatga 1068

<210> 286
<211> 355
<212> PRT
<213> Zea mays

<400> 286

Met Val Ser Ala Val Leu Arg Thr Ile Leu Val Thr Gly Gly Ala Gly
1 5 10 15

Tyr Ile Gly Ser His Thr Val Leu Gln Leu Leu Gln Gln Gly Phe Arg
20 25 30

Val Val Val Val Asp Asn Leu Asp Asn Ala Ser Glu Ala Ala Leu Ala
35 40 45

Arg Val Ala Glu Leu Ala Gly His Asp Gly Ala Asn Leu Val Phe His
50 55 60

Lys Val Asp Leu Arg Asp Arg His Ala Leu Val Asp Ile Phe Ser Ser
65 70 75 80

His Arg Phe Glu Ala Val Ile His Phe Ala Gly Leu Lys Ala Val Gly
85 90 95

Glu Ser Val His Lys Pro Leu Leu Tyr Tyr Asp Asn Asn Leu Val Gly
100 105 110

Thr Ile Thr Leu Leu Glu Val Met Ala Ala Asn Gly Cys Lys Lys Leu
115 120 125

Val Phe Ser Ser Ser Ala Thr Val Tyr Gly Trp Pro Lys Glu Val Pro
130 135 140

Cys Thr Glu Glu Phe Pro Leu Cys Ala Thr Asn Pro Tyr Gly Arg Thr
145 150 155 160

Lys Leu Val Ile Glu Asp Ile Cys Arg Asp Val His Arg Ser Asp Pro
165 170 175

Asp Trp Lys Ile Ile Leu Leu Arg Tyr Phe Asn Pro Val Gly Ala His
180 185 190

Pro Ser Gly Tyr Ile Gly Glu Asp Pro Cys Gly Val Pro Asn Asn Leu
195 200 205

Met Pro Tyr Val Gln Gln Val Ala Val Gly Arg Leu Pro His Leu Thr
210 215 220

Val Tyr Gly Thr Asp Tyr Ser Thr Lys Asp Gly Thr Gly Val Arg Asp
225 230 235 240

Tyr Ile His Val Val Asp Leu Ala Asp Gly His Ile Ala Ala Leu Arg
245 250 255

Lys Leu Tyr Glu Asp Ser Asp Lys Ile Gly Cys Glu Val Tyr Asn Leu
260 265 270

Gly Thr Gly Lys Gly Thr Ser Val Leu Glu Met Val Ala Ala Phe Glu
275 280 285

Lys Ala Ser Gly Lys Lys Ile Pro Leu Val Phe Ala Gly Arg Arg Pro
290 295 300

Gly Asp Ala Glu Ile Val Tyr Ala Ala Thr Ala Lys Ala Glu Lys Glu
305 310 315 320

Leu Lys Trp Lys Ala Lys Tyr Gly Ile Glu Glu Met Cys Arg Asp Leu
325 330 335

Trp Asn Trp Ala Ser Lys Asn Pro Tyr Gly Tyr Ala Gly Ser Arg Asp
340 345 350

Asn Ser Lys
355

<210> 287
<211> 1068
<212> DNA
<213> Zea mays

<400> 287
atggtgtccg ccgtgctccg gaccatcctc gtgacgggcg gcgccgggta catcggcagc 60
cacacggtgc tgcagctgct gcagcagggc ttccgcgtcg tcgtcgtcga caacctcgac 120
aacgcctccg aggccgccct cgcccgcgtc gccgagctcg ccgggcacga cggcgccaac 180
ctcgtcttcc acaaggttga ccttcgcgac aggcacgcgt tggtggacat cttctcgtcg 240
cacaggttcg aggctgtcat tcactttgct gggctcaagg ctgttgggga gagcgtgcac 300
aagcccctac tttactacga caacaacctg gtcggcacca tcaccctcct cgaggtgatg 360
gctgcgaacg gctgcaagaa gctggtgttc tcgtcatctg caactgtcta tgggtggccc 420
aaggaagtac catgcaccga agaattcccg ctctgcgcca ccaatcccta tgggcggaca 480
aagcttgtga ttgaagacat ctgccgcgac gtccaccgct ccgaccccga ctggaagatc 540
atactgctca ggtacttcaa ccccgttggc gctcatccaa gcgggtacat cggcgaagac 600
ccctgcggtg tcccgaacaa cctgatgccc tacgtgcagc aagtcgctgt tgggaagtta 660
cctcacctca cggtctacgg gacggactac agcaccaagg atgggactgg ggtgcgtgat 720
tacatccacg ttgtcgacct ggctgacggc cacatagcag ccctgaggaa gctctacgaa 780
gactccgaca aaataggctg tgaagtgtac aacttgggga ctggaaaggg gacgtccgtg 840
ttggaaatgg tggctgcatt cgagaaggct tctgggaaga aaatccctct ggtgttcgct 900
gggcgaagac ccggagacgc agagatcgtc tacgccgcaa ctgccaaggc agagaaggag 960
ctcaaatgga aggccaagta cgggatcgag gagatgtgca gagatctgtg gaactgggcg 1020
agcaagaacc cgtacgggta cgctgggtca cgcgacaaca gcaaatga 1068

<210> 288
<211> 355
<212> PRT
<213> Zea mays

<400> 288

Met Val Ser Ala Val Leu Arg Thr Ile Leu Val Thr Gly Gly Ala Gly
1 5 10 15

Tyr Ile Gly Ser His Thr Val Leu Gln Leu Leu Gln Gln Gly Phe Arg
20 25 30

Val Val Val Val Asp Asn Leu Asp Asn Ala Ser Glu Ala Ala Leu Ala
35 40 45

Arg Val Ala Glu Leu Ala Gly His Asp Gly Ala Asn Leu Val Phe His
50 55 60

Lys Val Asp Leu Arg Asp Arg His Ala Leu Val Asp Ile Phe Ser Ser
65 70 75 80

His Arg Phe Glu Ala Val Ile His Phe Ala Gly Leu Lys Ala Val Gly
85 90 95

Glu Ser Val His Lys Pro Leu Leu Tyr Tyr Asp Asn Asn Leu Val Gly
100 105 110

Thr Ile Thr Leu Leu Glu Val Met Ala Ala Asn Gly Cys Lys Lys Leu
115 120 125

Val Phe Ser Ser Ser Ala Thr Val Tyr Gly Trp Pro Lys Glu Val Pro
130 135 140

Cys Thr Glu Glu Phe Pro Leu Cys Ala Thr Asn Pro Tyr Gly Arg Thr
145 150 155 160

Lys Leu Val Ile Glu Asp Ile Cys Arg Asp Val His Arg Ser Asp Pro
165 170 175

Asp Trp Lys Ile Ile Leu Leu Arg Tyr Phe Asn Pro Val Gly Ala His
180 185 190

Pro Ser Gly Tyr Ile Gly Glu Asp Pro Cys Gly Val Pro Asn Asn Leu
195 200 205

Met Pro Tyr Val Gln Gln Val Ala Val Gly Lys Leu Pro His Leu Thr
210 215 220

Val Tyr Gly Thr Asp Tyr Ser Thr Lys Asp Gly Thr Gly Val Arg Asp
225 230 235 240

Tyr Ile His Val Val Asp Leu Ala Asp Gly His Ile Ala Ala Leu Arg
245 250 255

Lys Leu Tyr Glu Asp Ser Asp Lys Ile Gly Cys Glu Val Tyr Asn Leu
260 265 270

Gly Thr Gly Lys Gly Thr Ser Val Leu Glu Met Val Ala Ala Phe Glu
275 280 285

Lys Ala Ser Gly Lys Lys Ile Pro Leu Val Phe Ala Gly Arg Arg Pro
    290                 295                 300

Gly Asp Ala Glu Ile Val Tyr Ala Ala Thr Ala Lys Ala Glu Lys Glu
305                 310                 315                 320

Leu Lys Trp Lys Ala Lys Tyr Gly Ile Glu Glu Met Cys Arg Asp Leu
                325                 330                 335

Trp Asn Trp Ala Ser Lys Asn Pro Tyr Gly Tyr Ala Gly Ser Arg Asp
            340                 345                 350

Asn Ser Lys
        355

<210> 289
<211> 1068
<212> DNA
<213> Zea mays

<400> 289
atggtgtccg ccgtgctccg gaccatcctc gtgacgggcg gcgccgggta catcggcagc 60
cacacggtgc tgcagctgct gcagcagggc ttccgcgtcg tcgtcgtcga caacctcgac 120
aacgcctccg aggccgccct cgcccgcgtc gccgagctcg ccgggcacga cggcgccaac 180
ctcgtcttcc acaaggttga ccttcgcgac aggcacgcgt tggtggacat cttctcgtcg 240
cacaggttcg aggctgtcat tcactttgct gggctcaagg ctgttgggga gagcgtgcac 300
aagcccctac tttactacga caacaacctg gtcggcacca tcaccctcct cgaggtgatg 360
gctgcgaacg gctgcaagaa gctggtgttc tcgtcatctg caactgtcta tgggtggccc 420
aaggaagtac catgcaccga agaattcccg ctctgcgcca ccaatcccta tgggcggaca 480
aagcttgtga ttgaagacat ctgccgcgac gtccaccgct ccgaccccga ctggaagatc 540
atactgctca ggtacttcaa ccccgttggc gctcatccaa gcgggtacat cggcgaagac 600
ccctgcggtg tcccgaacaa cctgatgccc tacgtgcagc aagtcgctgt tgggaggtta 660
cctcacctca cggtctacgg gacggactac agcaccaagg atgggactgg ggtgcgtgat 720
tacatccacg ttgtcgacct ggctgacggc cacatagcag ccctgaggaa gctctacgaa 780
gactccgaca aaataggctg tgaagtgtac aacttgggga ctggaaaggg gacgtcggtg 840
ttggaaatgg tggctgcatt cgagaaggct tctgggaaga aaatccctct ggtgttcgct 900
gggcgaagac ccggagacgc agagatcgtc tacgccgcaa ctgccaaggc agagaaggag 960
ctcaaatgga aggccaagta cgggatcgag gagatgtgca gagatctgtg gaactgggcg 1020
agcaagaacc cgtacgggta cgctgggtca cgcgacaaca gcaaatga 1068

<210> 290
<211> 355
<212> PRT
<213> Zea mays

<400> 290

Met Val Ser Ala Val Leu Arg Thr Ile Leu Val Thr Gly Gly Ala Gly
1 5 10 15

Tyr Ile Gly Ser His Thr Val Leu Gln Leu Leu Gln Gln Gly Phe Arg
20 25 30

Val Val Val Val Asp Asn Leu Asp Asn Ala Ser Glu Ala Ala Leu Ala
35 40 45

Arg Val Ala Glu Leu Ala Gly His Asp Gly Ala Asn Leu Val Phe His
50 55 60

Lys Val Asp Leu Arg Asp Arg His Ala Leu Val Asp Ile Phe Ser Ser
65 70 75 80

His Arg Phe Glu Ala Val Ile His Phe Ala Gly Leu Lys Ala Val Gly
85 90 95

Glu Ser Val His Lys Pro Leu Leu Tyr Tyr Asp Asn Asn Leu Val Gly
100 105 110

Thr Ile Thr Leu Leu Glu Val Met Ala Ala Asn Gly Cys Lys Lys Leu
115 120 125

Val Phe Ser Ser Ser Ala Thr Val Tyr Gly Trp Pro Lys Glu Val Pro
130 135 140

Cys Thr Glu Glu Phe Pro Leu Cys Ala Thr Asn Pro Tyr Gly Arg Thr
145 150 155 160

Lys Leu Val Ile Glu Asp Ile Cys Arg Asp Val His Arg Ser Asp Pro
165 170 175

Asp Trp Lys Ile Ile Leu Leu Arg Tyr Phe Asn Pro Val Gly Ala His
180 185 190

Pro Ser Gly Tyr Ile Gly Glu Asp Pro Cys Gly Val Pro Asn Asn Leu
195 200 205

Met Pro Tyr Val Gln Gln Val Ala Val Gly Arg Leu Pro His Leu Thr
210 215 220

Val Tyr Gly Thr Asp Tyr Ser Thr Lys Asp Gly Thr Gly Val Arg Asp
225 230 235 240

Tyr Ile His Val Val Asp Leu Ala Asp Gly His Ile Ala Ala Leu Arg
245 250 255

Lys Leu Tyr Glu Asp Ser Asp Lys Ile Gly Cys Glu Val Tyr Asn Leu
260 265 270

Gly Thr Gly Lys Gly Thr Ser Val Leu Glu Met Val Ala Ala Phe Glu
        275                 280                 285

Lys Ala Ser Gly Lys Lys Ile Pro Leu Val Phe Ala Gly Arg Arg Pro
    290                 295                 300

Gly Asp Ala Glu Ile Val Tyr Ala Ala Thr Ala Lys Ala Glu Lys Glu
305                 310                 315                 320

Leu Lys Trp Lys Ala Lys Tyr Gly Ile Glu Glu Met Cys Arg Asp Leu
                325                 330                 335

Trp Asn Trp Ala Ser Lys Asn Pro Tyr Gly Tyr Ala Gly Ser Arg Asp
            340                 345                 350

Asn Ser Lys
        355

<210> 291
<211> 1272
<212> DNA
<213> Zea mays

<400> 291
atggcggtgg agaagacgct gcccggcgcg agtgcgggga ggaccgtgct ggtcacgggc 60
ggggcgggct acatcggtag ccacgccgtg ctgcagctcc tcctcgcggg cttccgcgcc 120
gtcgtcatcg acaacctcaa caactcctcc gagctggccg tccgccgcgt cgccgcgctc 180
gcgggggacc actcccgcaa cctctctttc cacaagattg atctccgtga caagggagca 240
ctggaaatgg tttttgcttc tacaagattt gaagctgtca ttcacttcgc tggattgaaa 300
gctgtgggtg aaagcgtaca gaagccatta ctttattatg acaacaacgt cattggcacg 360
ataaatcttc tagaagttat gtctgttcac ggttgcaaga agttggtgtt ctcatcatca 420
gctgcagttt atggatcacc caaaaactca ccctgcacag aaaattttcc tcttactcca 480
aacaatccat atggcaaaac aaagctcgtt gttgaagata tttgccggga tatctaccgt 540
tcagatcctg aatggaagat cattttactt aggtacttca atccagttgg tgctcatcct 600
agtggatatc ttggcgagga cccacgagga attcccaaca atcttatgcc ctatgttcag 660
caagttgcgg ttggtaggag gccagctcta acagttttag gaaatgacta tgcaacaaga 720
gatgggactg gggtccgaga ttacatccat gtggttgacc ttgctgacgg acatattgct 780
gcattgcaga agctttttga gaactctagc atagggtgtg aagcgtacaa ccttggaacc 840
ggaagaggta catctgtgct ggagattgtt aaagcatttg agaaggcttc tgggaagaaa 900
atacctctga tttttggtga aagacgccca ggtgatgcag agattctgtt ttcagagact 960
actaaagcag agagggagct taactggaaa gcaaaatacg gtattgaaga gatgtgccgc 1020
gaccaatgga actgggccag caagaaccct tatggctatg gatcacctga ctctatcaag 1080
cagaatggtc accaaacaaa cggatccgct gactcctcca agcagaatgg ccaccgcaca 1140

aacggttcaa ctgactcacc caagcggaac ggccaccatg cgtatgggtc tgctgactca 1200

cccaagcgca acgggcactg cgtttttgga tcatcagacc tcaagccgaa tggtaatggc 1260

cacctgcgct ga 1272

<210> 292
<211> 423
<212> PRT
<213> Zea mays

<400> 292

Met Ala Val Glu Lys Thr Leu Pro Gly Ala Ser Ala Gly Arg Thr Val
1 5 10 15

Leu Val Thr Gly Gly Ala Gly Tyr Ile Gly Ser His Ala Val Leu Gln
20 25 30

Leu Leu Leu Ala Gly Phe Arg Ala Val Val Ile Asp Asn Leu Asn Asn
35 40 45

Ser Ser Glu Leu Ala Val Arg Arg Val Ala Ala Leu Ala Gly Asp His
50 55 60

Ser Arg Asn Leu Ser Phe His Lys Ile Asp Leu Arg Asp Lys Gly Ala
65 70 75 80

Leu Glu Met Val Phe Ala Ser Thr Arg Phe Glu Ala Val Ile His Phe
85 90 95

Ala Gly Leu Lys Ala Val Gly Glu Ser Val Gln Lys Pro Leu Leu Tyr
100 105 110

Tyr Asp Asn Asn Val Ile Gly Thr Ile Asn Leu Leu Glu Val Met Ser
115 120 125

Val His Gly Cys Lys Lys Leu Val Phe Ser Ser Ser Ala Ala Val Tyr
130 135 140

Gly Ser Pro Lys Asn Ser Pro Cys Thr Glu Asn Phe Pro Leu Thr Pro
145 150 155 160

Asn Asn Pro Tyr Gly Lys Thr Lys Leu Val Val Glu Asp Ile Cys Arg
165 170 175

Asp Ile Tyr Arg Ser Asp Pro Glu Trp Lys Ile Ile Leu Leu Arg Tyr
180 185 190

Phe Asn Pro Val Gly Ala His Pro Ser Gly Tyr Leu Gly Glu Asp Pro
195 200 205

Arg Gly Ile Pro Asn Asn Leu Met Pro Tyr Val Gln Gln Val Ala Val

210 215 220

Gly Arg Arg Pro Ala Leu Thr Val Leu Gly Asn Asp Tyr Ala Thr Arg
225 230 235 240

Asp Gly Thr Gly Val Arg Asp Tyr Ile His Val Val Asp Leu Ala Asp
245 250 255

Gly His Ile Ala Ala Leu Gln Lys Leu Phe Glu Asn Ser Ser Ile Gly
260 265 270

Cys Glu Ala Tyr Asn Leu Gly Thr Gly Arg Gly Thr Ser Val Leu Glu
275 280 285

Ile Val Lys Ala Phe Glu Lys Ala Ser Gly Lys Lys Ile Pro Leu Ile
290 295 300

Phe Gly Glu Arg Arg Pro Gly Asp Ala Glu Ile Leu Phe Ser Glu Thr
305 310 315 320

Thr Lys Ala Glu Arg Glu Leu Asn Trp Lys Ala Lys Tyr Gly Ile Glu
325 330 335

Glu Met Cys Arg Asp Gln Trp Asn Trp Ala Ser Lys Asn Pro Tyr Gly
340 345 350

Tyr Gly Ser Pro Asp Ser Ile Lys Gln Asn Gly His Gln Thr Asn Gly
355 360 365

Ser Ala Asp Ser Ser Lys Gln Asn Gly His Arg Thr Asn Gly Ser Thr
370 375 380

Asp Ser Pro Lys Arg Asn Gly His His Ala Tyr Gly Ser Ala Asp Ser
385 390 395 400

Pro Lys Arg Asn Gly His Cys Val Phe Gly Ser Ser Asp Leu Lys Pro
405 410 415

Asn Gly Asn Gly His Leu Arg
420

<210> 293
<211> 1272
<212> DNA
<213> Zea mays

<400> 293
atggcggtgg agaagacgct gcccggcgcg agtgcgggga ggaccgtgct ggtcacgggc 60

ggggcgggct acatcggtag ccacgccgtg ctgcagctcc tcctcgcggg cttccgcgcc 120

gtcgtcatcg acaacctcaa caactcctcc gagctggccg tccgccgcgt cgccgcgctc 180

gcgggggacc actcccgcaa cctctctttc cacaagattg atctccgtga caagggagca 240

ctggaaatgg tttttgcttc tacaagattt gaagctgtca ttcacttcgc tggattgaaa 300
gctgtgggtg aaagcgtaca gaagccatta ctttattatg acaacaacgt cattggcacg 360
ataaatcttc tagaagttat gtctgttcac ggttgcaaga agttggtgtt ctcatcatca 420
gctgcagttt atggatcacc caaaaactca ccctgcacag aaaattttcc tcttactcca 480
aacaatccat atggcaaaac aaagctcgtt gttgaagata tttgccggga tatctaccgt 540
tcagatcctg aatggaagat cattttactt aggtacttca atccagttgg tgctcatcct 600
agtggatatc ttggcgagga cccacgagga attcccaaca atcttatgcc ctatgttcag 660
caagttgcgg ttggtaggag gccagctcta acagttttag gaaatgacta tgcaacaaga 720
gatgggactg gggtccgaga ttacatccat gtggttgacc ttgctgacgg acatattgct 780
gcattgcaga agctttttga gaactctagc atagggtgtg aagcgtacaa ccttggaacc 840
ggaagaggta catctgtgct ggagattgtt aaagcatttg agaaggcttc tgggaagaaa 900
atacctctga tttttggtga aagacgccca ggtgatgcag agattctgtt ttcagagact 960
actaaagcag agagggagct taactggaaa gcaaaatacg gtattgaaga gatgtgccgc 1020
gaccaatgga actgggccag caagaaccct tatggctatg gatcacctga ctctatcaag 1080
cagaatggtc accaaacaaa cggatccgct gactcctcca agcagaatgg ccaccgcaca 1140
aacggttcaa ctgactcacc caagcggaac ggccaccatg cgtatgggtc tgctgactca 1200
cccaagcgca acgggcactg cgtttttgga tcatcagacc tcaagccgaa tggtaatggc 1260
cacctgcgct ga 1272

<210> 294
<211> 423
<212> PRT
<213> Zea mays

<400> 294

Met Ala Val Glu Lys Thr Leu Pro Gly Ala Ser Ala Gly Arg Thr Val
1 5 10 15

Leu Val Thr Gly Gly Ala Gly Tyr Ile Gly Ser His Ala Val Leu Gln
20 25 30

Leu Leu Leu Ala Gly Phe Arg Ala Val Val Ile Asp Asn Leu Asn Asn
35 40 45

Ser Ser Glu Leu Ala Val Arg Arg Val Ala Ala Leu Ala Gly Asp His
50 55 60

Ser Arg Asn Leu Ser Phe His Lys Ile Asp Leu Arg Asp Lys Gly Ala
65 70 75 80

Leu Glu Met Val Phe Ala Ser Thr Arg Phe Glu Ala Val Ile His Phe
85 90 95

Ala Gly Leu Lys Ala Val Gly Glu Ser Val Gln Lys Pro Leu Leu Tyr

100 105 110

Tyr Asp Asn Asn Val Ile Gly Thr Ile Asn Leu Leu Glu Val Met Ser
115 120 125

Val His Gly Cys Lys Lys Leu Val Phe Ser Ser Ser Ala Ala Val Tyr
130 135 140

Gly Ser Pro Lys Asn Ser Pro Cys Thr Glu Asn Phe Pro Leu Thr Pro
145 150 155 160

Asn Asn Pro Tyr Gly Lys Thr Lys Leu Val Val Glu Asp Ile Cys Arg
165 170 175

Asp Ile Tyr Arg Ser Asp Pro Glu Trp Lys Ile Ile Leu Leu Arg Tyr
180 185 190

Phe Asn Pro Val Gly Ala His Pro Ser Gly Tyr Leu Gly Glu Asp Pro
195 200 205

Arg Gly Ile Pro Asn Asn Leu Met Pro Tyr Val Gln Gln Val Ala Val
210 215 220

Gly Arg Arg Pro Ala Leu Thr Val Leu Gly Asn Asp Tyr Ala Thr Arg
225 230 235 240

Asp Gly Thr Gly Val Arg Asp Tyr Ile His Val Val Asp Leu Ala Asp
245 250 255

Gly His Ile Ala Ala Leu Gln Lys Leu Phe Glu Asn Ser Ser Ile Gly
260 265 270

Cys Glu Ala Tyr Asn Leu Gly Thr Gly Arg Gly Thr Ser Val Leu Glu
275 280 285

Ile Val Lys Ala Phe Glu Lys Ala Ser Gly Lys Lys Ile Pro Leu Ile
290 295 300

Phe Gly Glu Arg Arg Pro Gly Asp Ala Glu Ile Leu Phe Ser Glu Thr
305 310 315 320

Thr Lys Ala Glu Arg Glu Leu Asn Trp Lys Ala Lys Tyr Gly Ile Glu
325 330 335

Glu Met Cys Arg Asp Gln Trp Asn Trp Ala Ser Lys Asn Pro Tyr Gly
340 345 350

Tyr Gly Ser Pro Asp Ser Ile Lys Gln Asn Gly His Gln Thr Asn Gly
355 360 365

Ser Ala Asp Ser Ser Lys Gln Asn Gly His Arg Thr Asn Gly Ser Thr
370 375 380

Asp Ser Pro Lys Arg Asn Gly His His Ala Tyr Gly Ser Ala Asp Ser
385 390 395 400

Pro Lys Arg Asn Gly His Cys Val Phe Gly Ser Ser Asp Leu Lys Pro
405 410 415

Asn Gly Asn Gly His Leu Arg
420

<210> 295
<211> 1272
<212> DNA
<213> Zea mays

<400> 295
atggcggtgg agaagacgct gcccggcgcg agtgcgggga ggaccgtgct ggtcacgggc 60
ggggcgggct acatcggtag ccacgccgtg ctgcagctcc tcctcgcggg cttccgcgcc 120
gtcgtcatcg acaacctcaa caactcctcc gagctggccg tccgccgcgt cgccgcgctc 180
gcgggggacc actcccgcaa cctctctttc cacaagattg atctccgtga caagggagca 240
ctggaaatgg tttttgcttc tacaagattt gaagctgtca ttcacttcgc tggattgaaa 300
gctgtgggtg aaagcgtaca gaagccatta ctttattatg acaacaacgt cattggcacg 360
ataaatcttc tagaagttat gtctgttcac ggttgcaaga agttggtgtt ctcatcatca 420
gctgcagttt atggatcacc caaaaactca ccctgcacag aaaattttcc tcttactcca 480
aacaatccat atggcaaaac aaagctcgtt gttgaagata tttgccggga tatctaccgt 540
tcagatcctg aatggaagat cattttactt aggtacttca atccagttgg tgctcatcct 600
agtggatatc ttggcgagga cccacgagga attcccaaca atcttatgcc ctatgttcag 660
caagttgcgg ttggtaggag gccagctcta acagttttag gaaatgacta tgcaacaaga 720
gatgggactg ggtccgaga ttacatccat gtggttgacc ttgctgacgg acatattgct 780
gcattgcaga agctttttga gaactctagc atagggtgtg aagcgtacaa ccttggaacc 840
ggaagaggta catctgtgct ggagattgtt aaagcatttg agaaggcttc tgggaagaaa 900
atacctctga tttttggtga aagacgccca ggtgatgcag agattctgtt ttcagagact 960
actaaagcag agagggagct taactggaaa gcaaaatacg gtattgaaga gatgtgccgc 1020
gaccaatgga actgggccag caagaaccct tatggctatg gatcacctga ctctatcaag 1080
cagaatggtc accaaacaaa cggatccgct gactcctcca agcagaatgg ccaccgcaca 1140
aacggttcaa ctgactcacc caagcggaac ggccaccatg cgtatgggtc tgctgactca 1200
cccaagcgca acgggcactg cgtttttgga tcatcagacc tcaagccgaa tggtaatggc 1260
cacctgcgct ga 1272

<210> 296
<211> 423
<212> PRT
<213> Zea mays

<400> 296

Met Ala Val Glu Lys Thr Leu Pro Gly Ala Ser Ala Gly Arg Thr Val
1               5                   10                  15

Leu Val Thr Gly Gly Ala Gly Tyr Ile Gly Ser His Ala Val Leu Gln
            20                  25                  30

Leu Leu Leu Ala Gly Phe Arg Ala Val Val Ile Asp Asn Leu Asn Asn
        35                  40                  45

Ser Ser Glu Leu Ala Val Arg Arg Val Ala Ala Leu Ala Gly Asp His
    50                  55                  60

Ser Arg Asn Leu Ser Phe His Lys Ile Asp Leu Arg Asp Lys Gly Ala
65                  70                  75                  80

Leu Glu Met Val Phe Ala Ser Thr Arg Phe Glu Ala Val Ile His Phe
                85                  90                  95

Ala Gly Leu Lys Ala Val Gly Glu Ser Val Gln Lys Pro Leu Leu Tyr
            100                 105                 110

Tyr Asp Asn Asn Val Ile Gly Thr Ile Asn Leu Leu Glu Val Met Ser
        115                 120                 125

Val His Gly Cys Lys Lys Leu Val Phe Ser Ser Ser Ala Ala Val Tyr
    130                 135                 140

Gly Ser Pro Lys Asn Ser Pro Cys Thr Glu Asn Phe Pro Leu Thr Pro
145                 150                 155                 160

Asn Asn Pro Tyr Gly Lys Thr Lys Leu Val Val Glu Asp Ile Cys Arg
                165                 170                 175

Asp Ile Tyr Arg Ser Asp Pro Glu Trp Lys Ile Ile Leu Leu Arg Tyr
            180                 185                 190

Phe Asn Pro Val Gly Ala His Pro Ser Gly Tyr Leu Gly Glu Asp Pro
        195                 200                 205

Arg Gly Ile Pro Asn Asn Leu Met Pro Tyr Val Gln Gln Val Ala Val
    210                 215                 220

Gly Arg Arg Pro Ala Leu Thr Val Leu Gly Asn Asp Tyr Ala Thr Arg
225                 230                 235                 240

Asp Gly Thr Gly Val Arg Asp Tyr Ile His Val Val Asp Leu Ala Asp
                245                 250                 255

Gly His Ile Ala Ala Leu Gln Lys Leu Phe Glu Asn Ser Ser Ile Gly
            260                 265                 270

Cys Glu Ala Tyr Asn Leu Gly Thr Gly Arg Gly Thr Ser Val Leu Glu
275 280 285

Ile Val Lys Ala Phe Glu Lys Ala Ser Gly Lys Lys Ile Pro Leu Ile
290 295 300

Phe Gly Glu Arg Arg Pro Gly Asp Ala Glu Ile Leu Phe Ser Glu Thr
305 310 315 320

Thr Lys Ala Glu Arg Glu Leu Asn Trp Lys Ala Lys Tyr Gly Ile Glu
325 330 335

Glu Met Cys Arg Asp Gln Trp Asn Trp Ala Ser Lys Asn Pro Tyr Gly
340 345 350

Tyr Gly Ser Pro Asp Ser Ile Lys Gln Asn Gly His Gln Thr Asn Gly
355 360 365

Ser Ala Asp Ser Ser Lys Gln Asn Gly His Arg Thr Asn Gly Ser Thr
370 375 380

Asp Ser Pro Lys Arg Asn Gly His His Ala Tyr Gly Ser Ala Asp Ser
385 390 395 400

Pro Lys Arg Asn Gly His Cys Val Phe Gly Ser Ser Asp Leu Lys Pro
405 410 415

Asn Gly Asn Gly His Leu Arg
420

<210> 297
<211> 1272
<212> DNA
<213> Zea mays

<400> 297
atggcggtgg agaagacgct gcccggcgcg agtgctggga ggaccgtgct ggtcacgggc 60
ggggcgggct acatcggtag ccacgccgtg ctgcagctcc tcctcgcggg cttccgcgcc 120
gtcgtcgtcg acaacctcaa caactcctcc gagctggccg tccgccgcgt cgccgcgctc 180
gcgggggacc actcccgcaa cctctctttc cacaagattg atctccgtga caagggagca 240
ctggaaatgg tttttgcttc tacaagattt gaagctgtca ttcacttcgc tggattgaaa 300
gctgtgggtg aaagcgtaca gaagccatta ctttattatg acaacaacgt cattggcacg 360
ataaatcttc tagaagttat gtctgttcac ggttgcaaga agttggtgtt ctcatcatca 420
gctgcagttt atggatcacc caaaaactca ccctgcacag aaaatttttcc tcttactcca 480
aacaatccat atggcaaaac aaagctcgtt gttgaagata tttgccggga tatctaccgt 540
tcagatcctg aatggaagat cattttactt aggtacttca atccagttgg tgctcatcct 600

agtggatatc ttggcgagga cccacgagga attcccaaca atcttatgcc ctatgttcag 660

caagttgcgg ttggtaggag gccagctcta acagttttag gaaatgacta tgcaacaaga 720

gatgggactg gggtccgaga ttacatccat gtggttgacc ttgctgacgg acatattgct 780

gcattgcaga agctttttga gaactctagc atagggtgtg aagcgtacaa ccttggaacc 840

ggaagaggta catctgtgct ggagattgtt aaagcatttg agaaggcttc tgggaagaaa 900

atacctctga tttttggtga aagacgccca ggtgatgcag agattctgtt ttcagagact 960

actaaagcag agagggagct taactggaaa gcaaaatacg gtattgaaga gatgtgccgc 1020

gaccaatgga actgggccag caagaaccct tatggctatg gatcacctga ctctatcaag 1080

cagaatggtc accaaacaaa cggatccgct gactcctcca agcagaatgg ccaccgcaca 1140

aacggttcaa ctgactcacc caagcggaac ggccaccatg cgtatgggtc tgctgactca 1200

cccaagcgca acgggcactg cgtttttgga tcatcagacc tcaagccgaa tggtaatggc 1260

cacctgcgct ga 1272

<210> 298
<211> 423
<212> PRT
<213> Zea mays

<400> 298

Met Ala Val Glu Lys Thr Leu Pro Gly Ala Ser Ala Gly Arg Thr Val
1               5                   10                  15

Leu Val Thr Gly Gly Ala Gly Tyr Ile Gly Ser His Ala Val Leu Gln
            20                  25                  30

Leu Leu Leu Ala Gly Phe Arg Ala Val Val Val Asp Asn Leu Asn Asn
        35                  40                  45

Ser Ser Glu Leu Ala Val Arg Arg Val Ala Ala Leu Ala Gly Asp His
    50                  55                  60

Ser Arg Asn Leu Ser Phe His Lys Ile Asp Leu Arg Asp Lys Gly Ala
65                  70                  75                  80

Leu Glu Met Val Phe Ala Ser Thr Arg Phe Glu Ala Val Ile His Phe
                85                  90                  95

Ala Gly Leu Lys Ala Val Gly Glu Ser Val Gln Lys Pro Leu Leu Tyr
            100                 105                 110

Tyr Asp Asn Asn Val Ile Gly Thr Ile Asn Leu Leu Glu Val Met Ser
        115                 120                 125

Val His Gly Cys Lys Lys Leu Val Phe Ser Ser Ser Ala Ala Val Tyr
    130                 135                 140

Gly Ser Pro Lys Asn Ser Pro Cys Thr Glu Asn Phe Pro Leu Thr Pro

145 150 155 160

Asn Asn Pro Tyr Gly Lys Thr Lys Leu Val Val Glu Asp Ile Cys Arg
165 170 175

Asp Ile Tyr Arg Ser Asp Pro Glu Trp Lys Ile Ile Leu Leu Arg Tyr
180 185 190

Phe Asn Pro Val Gly Ala His Pro Ser Gly Tyr Leu Gly Glu Asp Pro
195 200 205

Arg Gly Ile Pro Asn Asn Leu Met Pro Tyr Val Gln Gln Val Ala Val
210 215 220

Gly Arg Arg Pro Ala Leu Thr Val Leu Gly Asn Asp Tyr Ala Thr Arg
225 230 235 240

Asp Gly Thr Gly Val Arg Asp Tyr Ile His Val Val Asp Leu Ala Asp
245 250 255

Gly His Ile Ala Ala Leu Gln Lys Leu Phe Glu Asn Ser Ser Ile Gly
260 265 270

Cys Glu Ala Tyr Asn Leu Gly Thr Gly Arg Gly Thr Ser Val Leu Glu
275 280 285

Ile Val Lys Ala Phe Glu Lys Ala Ser Gly Lys Lys Ile Pro Leu Ile
290 295 300

Phe Gly Glu Arg Arg Pro Gly Asp Ala Glu Ile Leu Phe Ser Glu Thr
305 310 315 320

Thr Lys Ala Glu Arg Glu Leu Asn Trp Lys Ala Lys Tyr Gly Ile Glu
325 330 335

Glu Met Cys Arg Asp Gln Trp Asn Trp Ala Ser Lys Asn Pro Tyr Gly
340 345 350

Tyr Gly Ser Pro Asp Ser Ile Lys Gln Asn Gly His Gln Thr Asn Gly
355 360 365

Ser Ala Asp Ser Ser Lys Gln Asn Gly His Arg Thr Asn Gly Ser Thr
370 375 380

Asp Ser Pro Lys Arg Asn Gly His His Ala Tyr Gly Ser Ala Asp Ser
385 390 395 400

Pro Lys Arg Asn Gly His Cys Val Phe Gly Ser Ser Asp Leu Lys Pro
405 410 415

Asn Gly Asn Gly His Leu Arg
420

<210> 299
<211> 1068
<212> DNA
<213> Zea mays

<400> 299
atggtgtccg ccgtgctccg gaccatcctc gtgacgggcg gcgccgggta catcggcagc 60

cacacggtgc tgcagctgct gcagcagggc ttccgcgtcg tcgtcgtcga caacctcgac 120

aacgcctccg aggccgccct cgcccgcgtc gccgagctcg ccgggcacga cggcgccaac 180

ctcgtcttcc acaaggttga ccttcgcgac aggcacgcgt tggtggacat cttctcgtcg 240

cacaggttcg aggctgtcat tcactttgct gggctcaagg ctgttgggga gagcgtgcac 300

aagcccctac tttactacga caacaacctg gtcggcacca tcaccctcct ggaggtgatg 360

gctgcgaacg gctgcaagaa gctggtgttc tcgtcatctg caactgtcta tgggtggccc 420

aaggaagtac cctgcaccga agaattcccg ctctgcgcca ccaatcccta tgggcggaca 480

aagcttgtga ttgaagacat ctgccgcgac gtccaccgct ccgaccccga ctggaagatc 540

atactgctca ggtacttcaa ccccgttggc gctcatccaa gcgggtacat cggcgaagac 600

ccctgcggtg tcccgaacaa cctgatgccc tacgtgcagc aagtcgctgt tgggaagtta 660

cctcacctca cggtctacgg gacggactac agcaccaagg atgggactgg ggtgcgtgat 720

tacatccacg ttgtcgacct ggctgacggc cacatagcag ccctgaggaa gctctacgaa 780

gactccgaca aaataggctg tgaagtgtac aacttgggga ctggaaaggg gacgtccgtg 840

ttggaaatgg tggctgcatt cgagaaggct tctgggaaga aaatccctct ggtgttcgct 900

gggcgaagac ccggagacgc agagatcgtc tacgccgcaa ctgccaaggc agagaaggag 960

ctcaaatgga aggccaagta cgggatcgag gagatgtgca gagatctgtg gaactgggcg 1020

agcaagaacc cgtacgggta cgctgggtca cgcgacaaca gcaaatga 1068

<210> 300
<211> 355
<212> PRT
<213> Zea mays

<400> 300

Met Val Ser Ala Val Leu Arg Thr Ile Leu Val Thr Gly Gly Ala Gly
1 5 10 15

Tyr Ile Gly Ser His Thr Val Leu Gln Leu Leu Gln Gln Gly Phe Arg
20 25 30

Val Val Val Val Asp Asn Leu Asp Asn Ala Ser Glu Ala Ala Leu Ala
35 40 45

Arg Val Ala Glu Leu Ala Gly His Asp Gly Ala Asn Leu Val Phe His
50 55 60

```
Lys Val Asp Leu Arg Asp Arg His Ala Leu Val Asp Ile Phe Ser Ser
65                  70                  75                  80

His Arg Phe Glu Ala Val Ile His Phe Ala Gly Leu Lys Ala Val Gly
                85                  90                  95

Glu Ser Val His Lys Pro Leu Leu Tyr Tyr Asp Asn Asn Leu Val Gly
            100                 105                 110

Thr Ile Thr Leu Leu Glu Val Met Ala Ala Asn Gly Cys Lys Lys Leu
        115                 120                 125

Val Phe Ser Ser Ser Ala Thr Val Tyr Gly Trp Pro Lys Glu Val Pro
    130                 135                 140

Cys Thr Glu Glu Phe Pro Leu Cys Ala Thr Asn Pro Tyr Gly Arg Thr
145                 150                 155                 160

Lys Leu Val Ile Glu Asp Ile Cys Arg Asp Val His Arg Ser Asp Pro
                165                 170                 175

Asp Trp Lys Ile Ile Leu Leu Arg Tyr Phe Asn Pro Val Gly Ala His
            180                 185                 190

Pro Ser Gly Tyr Ile Gly Glu Asp Pro Cys Gly Val Pro Asn Asn Leu
        195                 200                 205

Met Pro Tyr Val Gln Gln Val Ala Val Gly Lys Leu Pro His Leu Thr
    210                 215                 220

Val Tyr Gly Thr Asp Tyr Ser Thr Lys Asp Gly Thr Gly Val Arg Asp
225                 230                 235                 240

Tyr Ile His Val Val Asp Leu Ala Asp Gly His Ile Ala Ala Leu Arg
                245                 250                 255

Lys Leu Tyr Glu Asp Ser Asp Lys Ile Gly Cys Glu Val Tyr Asn Leu
            260                 265                 270

Gly Thr Gly Lys Gly Thr Ser Val Leu Glu Met Val Ala Ala Phe Glu
        275                 280                 285

Lys Ala Ser Gly Lys Lys Ile Pro Leu Val Phe Ala Gly Arg Arg Pro
    290                 295                 300

Gly Asp Ala Glu Ile Val Tyr Ala Ala Thr Ala Lys Ala Glu Lys Glu
305                 310                 315                 320

Leu Lys Trp Lys Ala Lys Tyr Gly Ile Glu Glu Met Cys Arg Asp Leu
                325                 330                 335

Trp Asn Trp Ala Ser Lys Asn Pro Tyr Gly Tyr Ala Gly Ser Arg Asp
```

340 345 350

Asn Ser Lys
355

<210> 301
<211> 1065
<212> DNA
<213> Oryza sativa

<400> 301
atggtttcgg ccttgttgcg gacgatcctg gtgacgggcg gcgccggcta catcggcagc 60

cacaccgtcc tccagcttct ccaactcggc ttccgcgttg tcgtcctcga caacctcgac 120

aacgcctccg agctcgccat cctccgcgtc agggaactcg ccggacacaa cgccaacaac 180

ctcgacttcc gcaaggttga cctccgcgac aagcaagcgt tggaccaaat cttctcctct 240

caaaggtttg aggctgtcat ccattttgcc gggctgaaag ctgttggcga gagcgtgcag 300

aagcccctgc tttactacga caacaacctc atcggcacca tcactctcct gcaggtcatg 360

gccgcacatg gctgcaccaa gctggtgttc tcatcatccg caactgtcta cgggtggccc 420

aaggaggtgc cctgcactga agaatcccca ctttgtgcaa tgaaccccta cggcagaaca 480

aagctggtaa tcgaagacat gtgccgggat ctgcatgcct cagacccaaa ctggaagatc 540

atactgctcc gatacttcaa ccctgttgga gctcacccaa gcgggtacat tggtgaggac 600

ccctgcggca tcccaaacaa cctcatgccc ttcgtccagc aggtcgctgt tggcaggagg 660

ccggccctta ccgtctatgg aaccgactac aacaccaagg atggaactgg ggttcgtgac 720

tatatccatg ttgttgatct agcggatggt catatcgccg cgttaaggaa gctctatgaa 780

gattctgata gaataggatg tgaggtgtac aatctgggca ctggaaaggg gacatctgtg 840

ctggaaatgg ttgcagcatt cgagaaagct tctggaaaga aaatcccgct tgtatttgct 900

ggacgaaggc ctggagatgc cgagatcgtt tacgctcaaa ctgccaaagc tgagaaggaa 960

ctgaaatgga aggcaaaata cggggtagag gagatgtgca gggacctgtg gaattgggcg 1020

agcaagaacc cctacgggta tggatcgccg gacagtagca actga 1065

<210> 302
<211> 354
<212> PRT
<213> Oryza sativa

<400> 302

Met Val Ser Ala Leu Leu Arg Thr Ile Leu Val Thr Gly Gly Ala Gly
1 5 10 15

Tyr Ile Gly Ser His Thr Val Leu Gln Leu Leu Gln Leu Gly Phe Arg
20 25 30

Val Val Val Leu Asp Asn Leu Asp Asn Ala Ser Glu Leu Ala Ile Leu
35 40 45

```
Arg Val Arg Glu Leu Ala Gly His Asn Ala Asn Asn Leu Asp Phe Arg
    50                  55                  60

Lys Val Asp Leu Arg Asp Lys Gln Ala Leu Asp Gln Ile Phe Ser Ser
65                  70                  75                  80

Gln Arg Phe Glu Ala Val Ile His Phe Ala Gly Leu Lys Ala Val Gly
                85                  90                  95

Glu Ser Val Gln Lys Pro Leu Leu Tyr Tyr Asp Asn Asn Leu Ile Gly
            100                 105                 110

Thr Ile Thr Leu Leu Gln Val Met Ala Ala His Gly Cys Thr Lys Leu
        115                 120                 125

Val Phe Ser Ser Ser Ala Thr Val Tyr Gly Trp Pro Lys Glu Val Pro
    130                 135                 140

Cys Thr Glu Glu Ser Pro Leu Cys Ala Met Asn Pro Tyr Gly Arg Thr
145                 150                 155                 160

Lys Leu Val Ile Glu Asp Met Cys Arg Asp Leu His Ala Ser Asp Pro
                165                 170                 175

Asn Trp Lys Ile Ile Leu Leu Arg Tyr Phe Asn Pro Val Gly Ala His
            180                 185                 190

Pro Ser Gly Tyr Ile Gly Glu Asp Pro Cys Gly Ile Pro Asn Asn Leu
        195                 200                 205

Met Pro Phe Val Gln Gln Val Ala Val Gly Arg Arg Pro Ala Leu Thr
    210                 215                 220

Val Tyr Gly Thr Asp Tyr Asn Thr Lys Asp Gly Thr Gly Val Arg Asp
225                 230                 235                 240

Tyr Ile His Val Val Asp Leu Ala Asp Gly His Ile Ala Ala Leu Arg
                245                 250                 255

Lys Leu Tyr Glu Asp Ser Asp Arg Ile Gly Cys Glu Val Tyr Asn Leu
            260                 265                 270

Gly Thr Gly Lys Gly Thr Ser Val Leu Glu Met Val Ala Ala Phe Glu
        275                 280                 285

Lys Ala Ser Gly Lys Lys Ile Pro Leu Val Phe Ala Gly Arg Arg Pro
    290                 295                 300

Gly Asp Ala Glu Ile Val Tyr Ala Gln Thr Ala Lys Ala Glu Lys Glu
305                 310                 315                 320
```

Leu Lys Trp Lys Ala Lys Tyr Gly Val Glu Glu Met Cys Arg Asp Leu
325 330 335

Trp Asn Trp Ala Ser Lys Asn Pro Tyr Gly Tyr Gly Ser Pro Asp Ser
340 345 350

Ser Asn

<210> 303
<211> 1176
<212> DNA
<213> Glycine max

<400> 303
atggcatcgc gcgtcagcat tggcaacctt acctcctccg cgccgtatat taattcccct 60
cactttcgct caccacttaa gatttccaac aacccctctc tgcaaaacgc ttcgcataag 120
gtacttatgc gcgataagac tgtactggta accggcggag ccggttacat cggcagccac 180
accgttcttc agctcttgct cggaggtttc agagccgtcg tcctcgacaa cctcgaaaat 240
tcctccgagg ttgccatcca cagagtcagg gagctcgccg gcgaatttgg gaacaacctc 300
tcctttcaca aggtggacct acgggacaga gctgctctag accaaatatt ttcttccaca 360
caattcgatg ctgtcataca ttttgctgga ctgaaagcag taggagaaag tgtgcaaaaa 420
cctttactat actataacaa caacttgact gggacaatca ctctattgga agtcatggct 480
gcccatggat gcaagaagct cgtgttttca tcttcagcaa ctgtatatgg ttggccaaag 540
gaggttccat gcacagaaga gttccctctg tcagcaatga acccatatgg acgaactaag 600
cttatcattg aagaaatttg ccgtgatgtc cactgtgcag agccagattg taaaataatt 660
ttgttaagat acttcaaccc agttggtgca caccccagtg gttatattgg ggaggatcct 720
cgtggaattc caaacaatct catgccattt gttcagcaag tagcagttgg ccgacggcct 780
gcactgacag tttttggaaa tgattataat acaagtgatg gcactggggt tcgggattac 840
attcatgttg ttgatttagc agatgggcac attgctgcat tgcttaaact agatgaacct 900
aatataggtt gtgaggttta taacctggga acaggaaagg gaacatcagt tttggagatg 960
gttagagctt ttgaaatggc atctggaaag aaaattccac ttgtgatggc tggccgtaga 1020
cctggtgatg ctgaaattgt ttatgcatca acaaagaaag cggaaagaga gcttaaatgg 1080
aaggcaaaat atggcattga tgagatgtgc cgtgatcaat ggaattgggc tagcaaaaac 1140
ccttatggct atggagatca gggctccacc gattaa 1176

<210> 304
<211> 391
<212> PRT
<213> Glycine max

<400> 304

Met Ala Ser Arg Val Ser Ile Gly Asn Leu Thr Ser Ser Ala Pro Tyr
1 5 10 15

```
Ile Asn Ser Pro His Phe Arg Ser Pro Leu Lys Ile Ser Asn Asn Pro
            20                  25                  30

Ser Leu Gln Asn Ala Ser His Lys Val Leu Met Arg Asp Lys Thr Val
        35                  40                  45

Leu Val Thr Gly Gly Ala Gly Tyr Ile Gly Ser His Thr Val Leu Gln
    50                  55                  60

Leu Leu Leu Gly Gly Phe Arg Ala Val Val Leu Asp Asn Leu Glu Asn
65                  70                  75                  80

Ser Ser Glu Val Ala Ile His Arg Val Arg Glu Leu Ala Gly Glu Phe
                85                  90                  95

Gly Asn Asn Leu Ser Phe His Lys Val Asp Leu Arg Asp Arg Ala Ala
            100                 105                 110

Leu Asp Gln Ile Phe Ser Ser Thr Gln Phe Asp Ala Val Ile His Phe
        115                 120                 125

Ala Gly Leu Lys Ala Val Gly Glu Ser Val Gln Lys Pro Leu Leu Tyr
    130                 135                 140

Tyr Asn Asn Asn Leu Thr Gly Thr Ile Thr Leu Leu Glu Val Met Ala
145                 150                 155                 160

Ala His Gly Cys Lys Lys Leu Val Phe Ser Ser Ser Ala Thr Val Tyr
                165                 170                 175

Gly Trp Pro Lys Glu Val Pro Cys Thr Glu Glu Phe Pro Leu Ser Ala
            180                 185                 190

Met Asn Pro Tyr Gly Arg Thr Lys Leu Ile Ile Glu Glu Ile Cys Arg
        195                 200                 205

Asp Val His Cys Ala Glu Pro Asp Cys Lys Ile Ile Leu Leu Arg Tyr
    210                 215                 220

Phe Asn Pro Val Gly Ala His Pro Ser Gly Tyr Ile Gly Glu Asp Pro
225                 230                 235                 240

Arg Gly Ile Pro Asn Asn Leu Met Pro Phe Val Gln Gln Val Ala Val
                245                 250                 255

Gly Arg Arg Pro Ala Leu Thr Val Phe Gly Asn Asp Tyr Asn Thr Ser
            260                 265                 270

Asp Gly Thr Gly Val Arg Asp Tyr Ile His Val Val Asp Leu Ala Asp
        275                 280                 285
```

Gly His Ile Ala Ala Leu Leu Lys Leu Asp Glu Pro Asn Ile Gly Cys
    290                 295                 300

Glu Val Tyr Asn Leu Gly Thr Gly Lys Gly Thr Ser Val Leu Glu Met
305                 310                 315                 320

Val Arg Ala Phe Glu Met Ala Ser Gly Lys Lys Ile Pro Leu Val Met
                325                 330                 335

Ala Gly Arg Arg Pro Gly Asp Ala Glu Ile Val Tyr Ala Ser Thr Lys
            340                 345                 350

Lys Ala Glu Arg Glu Leu Lys Trp Lys Ala Lys Tyr Gly Ile Asp Glu
        355                 360                 365

Met Cys Arg Asp Gln Trp Asn Trp Ala Ser Lys Asn Pro Tyr Gly Tyr
    370                 375                 380

Gly Asp Gln Gly Ser Thr Asp
385                 390

<210> 305
<211> 1053
<212> DNA
<213> Glycine max

<400> 305
atgcctgccc aatcgattct ggttaccgga ggagccggtt acatcggtag ccacaccatc 60
cttcagcttc tcctcagcgg ttaccatgtc ttcgccgtcg acaacttcga caattcttcc 120
gaaaccgcca tcaacagagt caaggaactc gccggagaat tagcaaataa cctttccttt 180
tgcaagcttg acttgcggga cagagctgcc ctcgagaaaa tattttcaac cgtaaagttt 240
gatgccgtca tacattttgc tgggcttaaa gcagtgggtg aaagcgtgaa gaaaccactg 300
ctctactttg acaacaattt gatagggaca atcgttctat ttgaagtcat ggctgcccac 360
ggatgcaaga agcttgtgtt ctcatcttca gcaactgtat atgggtggcc aaaggaggtc 420
ccatgtaccg aagagttccc tttgtcagca acaaacccat atggacgaac caagcttatc 480
atcgaagaaa tttgtcgcga tatccaccgt gcagattcag attggacagt tatactattg 540
agatacttca acccagttgg tgcacatccc agtggttata ttggtgagga tcctcttgga 600
attccaaaca atctcatgcc ctttgttcaa caagtggcag ttggcagacg ccctgcattg 660
acagtttttg gaagcgatta taaaacaact gacggcactg gagttcgtga ttacattcat 720
gttcttgatt tagcagatgg gcatattgct gcgttgcgta aactggatga tcctaaaata 780
ggttgtgagg tttataactt gggaacagga aagggaacat cagttttgga gatggtaaac 840
gcttttgaac aggcctctgg aaagaaaatt ccacttgcaa tggcgggtcg gagacctgga 900
gatgctgaaa ttgtttatgc atcaacagaa aaagcggaaa gagaacttaa ttggaagact 960
aaatatagca tcgatgatat gtgccgcgat caatggaatt gggctagcaa aaacccttat 1020

ggctatggtg catctgcaga ttcctccaat tga 1053

<210> 306
<211> 350
<212> PRT
<213> Glycine max

<400> 306

Met Pro Ala Gln Ser Ile Leu Val Thr Gly Gly Ala Gly Tyr Ile Gly
1 5 10 15

Ser His Thr Ile Leu Gln Leu Leu Leu Ser Gly Tyr His Val Phe Ala
20 25 30

Val Asp Asn Phe Asp Asn Ser Ser Glu Thr Ala Ile Asn Arg Val Lys
35 40 45

Glu Leu Ala Gly Glu Leu Ala Asn Asn Leu Ser Phe Cys Lys Leu Asp
50 55 60

Leu Arg Asp Arg Ala Ala Leu Glu Lys Ile Phe Ser Thr Val Lys Phe
65 70 75 80

Asp Ala Val Ile His Phe Ala Gly Leu Lys Ala Val Gly Glu Ser Val
85 90 95

Lys Lys Pro Leu Leu Tyr Phe Asp Asn Asn Leu Ile Gly Thr Ile Val
100 105 110

Leu Phe Glu Val Met Ala Ala His Gly Cys Lys Lys Leu Val Phe Ser
115 120 125

Ser Ser Ala Thr Val Tyr Gly Trp Pro Lys Glu Val Pro Cys Thr Glu
130 135 140

Glu Phe Pro Leu Ser Ala Thr Asn Pro Tyr Gly Arg Thr Lys Leu Ile
145 150 155 160

Ile Glu Glu Ile Cys Arg Asp Ile His Arg Ala Asp Ser Asp Trp Thr
165 170 175

Val Ile Leu Leu Arg Tyr Phe Asn Pro Val Gly Ala His Pro Ser Gly
180 185 190

Tyr Ile Gly Glu Asp Pro Leu Gly Ile Pro Asn Asn Leu Met Pro Phe
195 200 205

Val Gln Gln Val Ala Val Gly Arg Arg Pro Ala Leu Thr Val Phe Gly
210 215 220

Ser Asp Tyr Lys Thr Thr Asp Gly Thr Gly Val Arg Asp Tyr Ile His
225 230 235 240

```
Val Leu Asp Leu Ala Asp Gly His Ile Ala Ala Leu Arg Lys Leu Asp
                245                 250                 255

Asp Pro Lys Ile Gly Cys Glu Val Tyr Asn Leu Gly Thr Gly Lys Gly
            260                 265                 270

Thr Ser Val Leu Glu Met Val Asn Ala Phe Glu Gln Ala Ser Gly Lys
        275                 280                 285

Lys Ile Pro Leu Ala Met Ala Gly Arg Arg Pro Gly Asp Ala Glu Ile
    290                 295                 300

Val Tyr Ala Ser Thr Glu Lys Ala Glu Arg Glu Leu Asn Trp Lys Thr
305                 310                 315                 320

Lys Tyr Ser Ile Asp Asp Met Cys Arg Asp Gln Trp Asn Trp Ala Ser
                325                 330                 335

Lys Asn Pro Tyr Gly Tyr Gly Ala Ser Ala Asp Ser Ser Asn
            340                 345                 350
```

<210> 307
<211> 1050
<212> DNA
<213> Glycine max

<400> 307

```
atgcgcgaca agactgtgct ggtaaccggc ggagccggtt acatcggcac ccacaccgtt      60
cttcagctct tgctcggagg ttgcagaacc gtcgtcgtcg acaatctcga caattcctcc     120
gaggtttcta tccaccgagt cagggagctt gccggcgaat ttgggaacaa cctctccttt     180
cacaaggtgg acctacggga cagggatgca ctagagcaaa tttttgtttc cacacaattt     240
gatgctgtca tacactttgc tggactgaaa gcagtaggag aaagtgtgca aaaaccttta     300
ctatactata acaacaactt gactgggaca atcactctat tggaagtcat ggctgcccat     360
ggatgcaaga agctcgtgtt ctcttcttca gcaactgtat atggttggcc aaaggaagtt     420
ccatgcacag aagagttccc tctgtcagca atgaacccat atggacgaac taagcttatc     480
attgaagaaa tttgccgtga tgtccactgt gcagagccag attgtaaaat aattttgtta     540
agatacttca acccagttgg tgcacacccc agcggttata ttggggagga tcctcgcgga     600
attccaaaca atctcatgcc atttgttcag caagtagcag ttggccgacg gcctgcactg     660
acagtttttg gaaatgatta taatacaagt gatggcactg ggttcggga ttacattcat      720
gttgttgatt tagcagatgg gcacattgct gcattgctta aactagatga acctaatata     780
ggttgtgagg tttataacct gggaacagga aagggaacat cagttttgga gatggttaga     840
gcttttgaaa tggcatctgg aaagaaaatt ccacttgtga tggctggccg tagacctggt     900
gatgctgaaa ttgtttatgc atcaacaaag aaagcggaaa gagagcttaa atggaaggca     960
aaatatggca ttgatgagat gtgccgtgat caatggaatt gggctagcaa aaacccttat    1020
```

ggctatggag atcagggctc caccgattaa 1050

<210> 308
<211> 349
<212> PRT
<213> Glycine max

<400> 308

Met Arg Asp Lys Thr Val Leu Val Thr Gly Gly Ala Gly Tyr Ile Gly
1 5 10 15

Thr His Thr Val Leu Gln Leu Leu Leu Gly Gly Cys Arg Thr Val Val
20 25 30

Val Asp Asn Leu Asp Asn Ser Ser Glu Val Ser Ile His Arg Val Arg
35 40 45

Glu Leu Ala Gly Glu Phe Gly Asn Asn Leu Ser Phe His Lys Val Asp
50 55 60

Leu Arg Asp Arg Asp Ala Leu Glu Gln Ile Phe Val Ser Thr Gln Phe
65 70 75 80

Asp Ala Val Ile His Phe Ala Gly Leu Lys Ala Val Gly Glu Ser Val
85 90 95

Gln Lys Pro Leu Leu Tyr Tyr Asn Asn Asn Leu Thr Gly Thr Ile Thr
100 105 110

Leu Leu Glu Val Met Ala Ala His Gly Cys Lys Lys Leu Val Phe Ser
115 120 125

Ser Ser Ala Thr Val Tyr Gly Trp Pro Lys Glu Val Pro Cys Thr Glu
130 135 140

Glu Phe Pro Leu Ser Ala Met Asn Pro Tyr Gly Arg Thr Lys Leu Ile
145 150 155 160

Ile Glu Glu Ile Cys Arg Asp Val His Cys Ala Glu Pro Asp Cys Lys
165 170 175

Ile Ile Leu Leu Arg Tyr Phe Asn Pro Val Gly Ala His Pro Ser Gly
180 185 190

Tyr Ile Gly Glu Asp Pro Arg Gly Ile Pro Asn Asn Leu Met Pro Phe
195 200 205

Val Gln Gln Val Ala Val Gly Arg Arg Pro Ala Leu Thr Val Phe Gly
210 215 220

Asn Asp Tyr Asn Thr Ser Asp Gly Thr Gly Val Arg Asp Tyr Ile His
225 230 235 240

```
Val Val Asp Leu Ala Asp Gly His Ile Ala Ala Leu Leu Lys Leu Asp
                245                 250                 255

Glu Pro Asn Ile Gly Cys Glu Val Tyr Asn Leu Gly Thr Gly Lys Gly
            260                 265                 270

Thr Ser Val Leu Glu Met Val Arg Ala Phe Glu Met Ala Ser Gly Lys
        275                 280                 285

Lys Ile Pro Leu Val Met Ala Gly Arg Arg Pro Gly Asp Ala Glu Ile
    290                 295                 300

Val Tyr Ala Ser Thr Lys Lys Ala Glu Arg Glu Leu Lys Trp Lys Ala
305                 310                 315                 320

Lys Tyr Gly Ile Asp Glu Met Cys Arg Asp Gln Trp Asn Trp Ala Ser
                325                 330                 335

Lys Asn Pro Tyr Gly Tyr Gly Asp Gln Gly Ser Thr Asp
            340                 345
```

<210> 309
<211> 1050
<212> DNA
<213> Glycine max

<400> 309

```
atgcgcgaca agactgtgct ggtaaccggc ggagccggtt acatcggcac ccacaccgtt     60
cttcagctct tgctcggagg ttgcagaacc gtcgtcgtcg acaatctcga caattcctcc    120
gaggtttcta tccaccgagt cagggagctt gccggcgaat ttgggaacaa cctctccttt    180
cacaaggtgg acctacggga cagggatgca ctagagcaaa tttttgtttc cacacaattt    240
gatgctgtca tacactttgc tggactgaaa gcagtaggag aaagtgtgca aaaaccttta    300
ctatactata acaacaactt gactgggaca atcactctat tggaagtcat ggctgcccat    360
ggatgcaaga agctcgtgtt ctcttcttca gcaactgtat atggttggcc aaaggaagtt    420
ccatgcacag aagagttccc tctgtcagca atgaacccat atggacgaac taagcttatc    480
attgaagaaa tttgtcgtga tgtccaccgt gcagagccag attggaaaat aatattgtta    540
agatacttca acccagtcgg tgcacacccc agcggttgta ttggggagga tccccgcgga    600
attccaaaca atctcatgcc atttgttcag caagtagcag ttggccgacg gcctgcactg    660
acagtttttg gaaatgatta taatacaact gatggcactg gggttcggga ttacattcat    720
gttgttgatt tagcagatgg gcacattgct gcattgctta aactagatga acctaatata    780
ggttgtgagg tttataacct gggaacagga aagggaacat cagttttgga gatggttaga    840
gcttttgaaa tggcatctgg aaagaaaatt ccacttgtga tggctggccg tagacctggt    900
gatgctgaaa ttgtttatgc atcaacaaag aaagcggaaa gagagcttaa atggaaggca    960
aaatatggca ttgatgagat gtgccgaaat caatggaatt gggctagcaa aaacccttat   1020
```

ggctatggag atcagggctc caccgattaa 1050

<210> 310
<211> 349
<212> PRT
<213> Glycine max

<400> 310

Met Arg Asp Lys Thr Val Leu Val Thr Gly Gly Ala Gly Tyr Ile Gly
1 5 10 15

Thr His Thr Val Leu Gln Leu Leu Leu Gly Gly Cys Arg Thr Val Val
20 25 30

Val Asp Asn Leu Asp Asn Ser Ser Glu Val Ser Ile His Arg Val Arg
35 40 45

Glu Leu Ala Gly Glu Phe Gly Asn Asn Leu Ser Phe His Lys Val Asp
50 55 60

Leu Arg Asp Arg Asp Ala Leu Glu Gln Ile Phe Val Ser Thr Gln Phe
65 70 75 80

Asp Ala Val Ile His Phe Ala Gly Leu Lys Ala Val Gly Glu Ser Val
85 90 95

Gln Lys Pro Leu Leu Tyr Tyr Asn Asn Asn Leu Thr Gly Thr Ile Thr
100 105 110

Leu Leu Glu Val Met Ala Ala His Gly Cys Lys Lys Leu Val Phe Ser
115 120 125

Ser Ser Ala Thr Val Tyr Gly Trp Pro Lys Glu Val Pro Cys Thr Glu
130 135 140

Glu Phe Pro Leu Ser Ala Met Asn Pro Tyr Gly Arg Thr Lys Leu Ile
145 150 155 160

Ile Glu Glu Ile Cys Arg Asp Val His Arg Ala Glu Pro Asp Trp Lys
165 170 175

Ile Ile Leu Leu Arg Tyr Phe Asn Pro Val Gly Ala His Pro Ser Gly
180 185 190

Cys Ile Gly Glu Asp Pro Arg Gly Ile Pro Asn Asn Leu Met Pro Phe
195 200 205

Val Gln Gln Val Ala Val Gly Arg Arg Pro Ala Leu Thr Val Phe Gly
210 215 220

Asn Asp Tyr Asn Thr Thr Asp Gly Thr Gly Val Arg Asp Tyr Ile His
225 230 235 240

Val Val Asp Leu Ala Asp Gly His Ile Ala Ala Leu Leu Lys Leu Asp
245 250 255

Glu Pro Asn Ile Gly Cys Glu Val Tyr Asn Leu Gly Thr Gly Lys Gly
260 265 270

Thr Ser Val Leu Glu Met Val Arg Ala Phe Glu Met Ala Ser Gly Lys
275 280 285

Lys Ile Pro Leu Val Met Ala Gly Arg Arg Pro Gly Asp Ala Glu Ile
290 295 300

Val Tyr Ala Ser Thr Lys Lys Ala Glu Arg Glu Leu Lys Trp Lys Ala
305 310 315 320

Lys Tyr Gly Ile Asp Glu Met Cys Arg Asn Gln Trp Asn Trp Ala Ser
325 330 335

Lys Asn Pro Tyr Gly Tyr Gly Asp Gln Gly Ser Thr Asp
340 345

<210> 311
<211> 1176
<212> DNA
<213> Glycine max

<400> 311
atggcatcgc gcgtcagcat tggcaacctt acctcctccg cgccgtatat taattcccct 60
cactttcgct caccacttaa gatttccaac aacccctctc tgcaaaacgc ttcgcataag 120
gtacttatgc gcgataagac tgtactggta accggcggag ccggttacat cggcagccac 180
accgttcttc agctcttgct cggaggtttc agagccgtcg tcctcgacaa cctcgaaaat 240
tcctccgagg ttgccatcca cagagtcagg gagctcgccg gcgaatttgg gaacaacctc 300
tcctttcaca aggtggacct acgggacaga gctgctctag accaaatatt ttcttccaca 360
caattcgatg ctgtcataca ttttgctgga ctgaaagcag taggagaaag tgtgcaaaaa 420
cctttactat actataacaa caacttgact gggacaatca ctctattgga agtcatggct 480
gcccatggat gcaagaagct cgtgttttca tcttcagcaa ctgtatatgg ttggccaaag 540
gaggttccat gcacagaaga gttccctctg tcagcaatga acccatatgg acgaactaag 600
cttatcattg aagaaatttg ccgtgatgtc cactgtgcag agccagattg taaaataatt 660
ttgttaagat acttcaaccc agttggtgca caccccagtg gttatattgg ggaggatcct 720
cgtggaattc caaacaatct catgccattt gttcagcaag tagcagttgg ccgacggcct 780
gcactgacag tttttggaaa tgattataat acaagtgatg gcactggggt tcgggattac 840
attcatgttg ttgatttagc agatgggcac attgctgcat tgcttaaact agatgaacct 900
aatataggtt gtgaggttta taacctggga acaggaaagg gaacatcagt tttggagatg 960
gttagagctt ttgaaatggc atctggaaag aaaattccac ttgtgatggc tggccgtaga 1020

cctggtgatg ctgaaattgt ttatgcatca acaaagaaag cggaaagaga gcttaaatgg 1080

aaggcaaaat atggcattga tgagatgtgc cgtgatcaat ggaattgggc tagcaaaaac 1140

ccttatggct atggagatca gggctccacc gattaa 1176

<210> 312
<211> 391
<212> PRT
<213> Glycine max

<400> 312

Met Ala Ser Arg Val Ser Ile Gly Asn Leu Thr Ser Ser Ala Pro Tyr
1 5 10 15

Ile Asn Ser Pro His Phe Arg Ser Pro Leu Lys Ile Ser Asn Asn Pro
20 25 30

Ser Leu Gln Asn Ala Ser His Lys Val Leu Met Arg Asp Lys Thr Val
35 40 45

Leu Val Thr Gly Gly Ala Gly Tyr Ile Gly Ser His Thr Val Leu Gln
50 55 60

Leu Leu Leu Gly Gly Phe Arg Ala Val Val Leu Asp Asn Leu Glu Asn
65 70 75 80

Ser Ser Glu Val Ala Ile His Arg Val Arg Glu Leu Ala Gly Glu Phe
85 90 95

Gly Asn Asn Leu Ser Phe His Lys Val Asp Leu Arg Asp Arg Ala Ala
100 105 110

Leu Asp Gln Ile Phe Ser Ser Thr Gln Phe Asp Ala Val Ile His Phe
115 120 125

Ala Gly Leu Lys Ala Val Gly Glu Ser Val Gln Lys Pro Leu Leu Tyr
130 135 140

Tyr Asn Asn Asn Leu Thr Gly Thr Ile Thr Leu Leu Glu Val Met Ala
145 150 155 160

Ala His Gly Cys Lys Lys Leu Val Phe Ser Ser Ser Ala Thr Val Tyr
165 170 175

Gly Trp Pro Lys Glu Val Pro Cys Thr Glu Glu Phe Pro Leu Ser Ala
180 185 190

Met Asn Pro Tyr Gly Arg Thr Lys Leu Ile Ile Glu Glu Ile Cys Arg
195 200 205

Asp Val His Cys Ala Glu Pro Asp Cys Lys Ile Ile Leu Leu Arg Tyr
210 215 220

Phe Asn Pro Val Gly Ala His Pro Ser Gly Tyr Ile Gly Glu Asp Pro
225 230 235 240

Arg Gly Ile Pro Asn Asn Leu Met Pro Phe Val Gln Gln Val Ala Val
245 250 255

Gly Arg Arg Pro Ala Leu Thr Val Phe Gly Asn Asp Tyr Asn Thr Ser
260 265 270

Asp Gly Thr Gly Val Arg Asp Tyr Ile His Val Val Asp Leu Ala Asp
275 280 285

Gly His Ile Ala Ala Leu Leu Lys Leu Asp Glu Pro Asn Ile Gly Cys
290 295 300

Glu Val Tyr Asn Leu Gly Thr Gly Lys Gly Thr Ser Val Leu Glu Met
305 310 315 320

Val Arg Ala Phe Glu Met Ala Ser Gly Lys Lys Ile Pro Leu Val Met

325 330 335

Ala Gly Arg Arg Pro Gly Asp Ala Glu Ile Val Tyr Ala Ser Thr Lys
340 345 350

Lys Ala Glu Arg Glu Leu Lys Trp Lys Ala Lys Tyr Gly Ile Asp Glu
355 360 365

Met Cys Arg Asp Gln Trp Asn Trp Ala Ser Lys Asn Pro Tyr Gly Tyr
370 375 380

Gly Asp Gln Gly Ser Thr Asp
385 390

<210> 313
<211> 1059
<212> DNA
<213> Helianthus annuus

<400> 313
atgacgcggc cgatgtgcat actggtgacc ggtggtgccg gttatatagg aagtcatact 60
gttttgcagt tgttgttaaa tggttacagt acggtggtgg ttgataattt ggataattcg 120
tcggaagttg ctatcagtcg ggttcgagaa ctcgctggtg atcgtggtcg taatctgtct 180
tttcacaaga tggatatccg ggataagcct gcactagagc agctttttgc ttctacaaag 240
tttgacgcgg ttatacattt tgctggatta aaggcagttg gtgaaagcgt gcagaagccg 300
ttgatgtatt acaacaataa tatcgttggt actataactt tattggaagt tatggctgcc 360
tatggctgca agaagcttgt gttttcatca tcggctactg tttatggctg gccaaaggag 420
ttgccgtgca cagaagagtt tcccttgtct gcagccaacc cgtatggacg aacaaagcta 480

```
atgatcgagg agatgtgccg agatgtatat gcttcagacc ctgagtggaa atttgtattg     540
ttgcggtatt ttaaccctgt tggtgcacat cccagtggcc gaattggcga agatcctcat     600
ggaattccaa acaatcttat gccttttatt cagcatgttg ctgttggtag acaacctgcg     660
cttaaagtgt tcggaactga ttactccaca aaagatggaa ctggggtacg tgattacatc     720
catgtcgtag atttagcaga cggacatact gcggcgttgg cgaaactttc tgatcccaaa     780
ataggttgtg aagtttataa cttggggact ggtaaaggga catctgtttt ggagatggta     840
tcagcctttg aaaaggcatc aggaaagaaa atcccattaa taaagactgc acgacgacct     900
ggtgatgctg agattgtata tgcgtcaaca acaaaggcag aacgcgagtt aaactggaag     960
gcaaagtatg gaatagagga gatgtgcaga gatcagtgga actgggctag caggaatcct    1020
tacggctatc agactgaagc aaagaaactt ggtgcatga                           1059
```

<210> 314
<211> 352
<212> PRT
<213> Helianthus annuus

<400> 314

```
Met Thr Arg Pro Met Cys Ile Leu Val Thr Gly Gly Ala Gly Tyr Ile
1               5                   10                  15

Gly Ser His Thr Val Leu Gln Leu Leu Leu Asn Gly Tyr Ser Thr Val
            20                  25                  30

Val Val Asp Asn Leu Asp Asn Ser Ser Glu Val Ala Ile Ser Arg Val
        35                  40                  45

Arg Glu Leu Ala Gly Asp Arg Gly Arg Asn Leu Ser Phe His Lys Met
    50                  55                  60

Asp Ile Arg Asp Lys Pro Ala Leu Glu Gln Leu Phe Ala Ser Thr Lys
65                  70                  75                  80

Phe Asp Ala Val Ile His Phe Ala Gly Leu Lys Ala Val Gly Glu Ser
                85                  90                  95

Val Gln Lys Pro Leu Met Tyr Tyr Asn Asn Asn Ile Val Gly Thr Ile
            100                 105                 110

Thr Leu Leu Glu Val Met Ala Ala Tyr Gly Cys Lys Lys Leu Val Phe
        115                 120                 125

Ser Ser Ser Ala Thr Val Tyr Gly Trp Pro Lys Glu Leu Pro Cys Thr
    130                 135                 140

Glu Glu Phe Pro Leu Ser Ala Ala Asn Pro Tyr Gly Arg Thr Lys Leu
145                 150                 155                 160

Met Ile Glu Glu Met Cys Arg Asp Val Tyr Ala Ser Asp Pro Glu Trp
```

165 170 175

Lys Phe Val Leu Leu Arg Tyr Phe Asn Pro Val Gly Ala His Pro Ser
180 185 190

Gly Arg Ile Gly Glu Asp Pro His Gly Ile Pro Asn Asn Leu Met Pro
195 200 205

Phe Ile Gln His Val Ala Val Gly Arg Gln Pro Ala Leu Lys Val Phe
210 215 220

Gly Thr Asp Tyr Ser Thr Lys Asp Gly Thr Gly Val Arg Asp Tyr Ile
225 230 235 240

His Val Val Asp Leu Ala Asp Gly His Thr Ala Ala Leu Ala Lys Leu
245 250 255

Ser Asp Pro Lys Ile Gly Cys Glu Val Tyr Asn Leu Gly Thr Gly Lys
260 265 270

Gly Thr Ser Val Leu Glu Met Val Ser Ala Phe Glu Lys Ala Ser Gly
275 280 285

Lys Lys Ile Pro Leu Ile Lys Thr Ala Arg Arg Pro Gly Asp Ala Glu
290 295 300

Ile Val Tyr Ala Ser Thr Thr Lys Ala Glu Arg Glu Leu Asn Trp Lys
305 310 315 320

Ala Lys Tyr Gly Ile Glu Glu Met Cys Arg Asp Gln Trp Asn Trp Ala
325 330 335

Ser Arg Asn Pro Tyr Gly Tyr Gln Thr Glu Ala Lys Lys Leu Gly Ala
340 345 350

<210> 315
<211> 1497
<212> DNA
<213> Zea mays

<400> 315
gaagcacggc aatctcgatc tcgttcgttc ccctgagcgg cagtcagtgc gagcgagcgc 60
gcacacagac aactcatcct ctcccctctc tctctcacac acacacatcc atcatcccat 120
ccctgtgacg ggcgatccga gggcctaccg acgatggtgt ccgccgtgct ccggaccatc 180
ctcgtgacgg gcggcgccgg gtacatcggc agccacacgg tgctgcagct gctgcagcag 240
ggcttccgcg tcgtcgtcgt cgacaacctc gacaacgcct ccgaggccgc cctcgcccgc 300
gtcgccgagc tcgccgggca cgacggcgcc aacctcgtct tccacaaggt tgaccttcgc 360
gacaggcacg cgttggtgga catcttctcg tcgcacaggt tcgaggctgt cattcacttt 420
gctgggctca aggctgttgg ggagagcgtg cacaagcccc tactttacta cgacaacaac 480

ctggtcggca ccatcaccct cctggaggtg atggctgcga acggctgcaa gaagctggtg 540
ttctcgtcat ctgcaactgt ctatgggtgg cccaaggaag taccctgcac cgaagaattc 600
ccgctctgcg ccaccaatcc ctatgggcgg acaaagcttg tgattgaaga catctgccgc 660
gacgtccacc gctccgaccc cgactggaag atcatactgc tcaggtactt caaccccgtt 720
ggcgctcatc caagcgggta catcggcgaa gacccctgcg gtgtcccgaa caacctgatg 780
ccctacgtgc agcaagtcgc tgttgggagg ttacctcacc tcacggtcta cgggacggac 840
tacagcacca aggatgggac tggggtgcgt gattacatcc acgttgtcga cctggctgac 900
ggccacatag cagccctgag gaagctctac gaagactccg acaaaatagg ctgtgaagtg 960
tacaacttgg ggactggaaa ggggacgtcc gtgttggaaa tggtggctgc attcgagaag 1020
gcttctggga agaaaatccc tctggtgttc gctgggcgaa gacccggaga cgcagagatc 1080
gtctacgccg caactgccaa ggcagagaag gagctcaaat ggaaggccaa gtacgggatc 1140
gaggagatgt gcagagatct gtggaactgg gcgagcaaga acccgtacgg gtacgctggg 1200
tcacgcgaca acagcaaatg aaccacccat ccatgcatcc catcctgcag taggagcaag 1260
cagcagcctt atagcctatg ctaccataat tagtaactca tcaatcatgc atacacataa 1320
ttagtaactc atcaatcatg catacataat cagccagtca tctgattagg ggtactgcga 1380
tgggcctcgc ttcttcagtg tatagaggga ggaggagggg gggtcatctt gatattcttt 1440
tttttttatc attcccgaat gattattatt agtagccagt tcgatgaaaa aaaaaaa 1497

<210> 316
<211> 355
<212> PRT
<213> Zea mays

<400> 316

Met Val Ser Ala Val Leu Arg Thr Ile Leu Val Thr Gly Gly Ala Gly
1 5 10 15

Tyr Ile Gly Ser His Thr Val Leu Gln Leu Leu Gln Gln Gly Phe Arg
20 25 30

Val Val Val Val Asp Asn Leu Asp Asn Ala Ser Glu Ala Ala Leu Ala
35 40 45

Arg Val Ala Glu Leu Ala Gly His Asp Gly Ala Asn Leu Val Phe His
50 55 60

Lys Val Asp Leu Arg Asp Arg His Ala Leu Val Asp Ile Phe Ser Ser
65 70 75 80

His Arg Phe Glu Ala Val Ile His Phe Ala Gly Leu Lys Ala Val Gly
85 90 95

Glu Ser Val His Lys Pro Leu Leu Tyr Tyr Asp Asn Asn Leu Val Gly
100 105 110

Thr Ile Thr Leu Leu Glu Val Met Ala Ala Asn Gly Cys Lys Lys Leu
115 120 125

Val Phe Ser Ser Ser Ala Thr Val Tyr Gly Trp Pro Lys Glu Val Pro
130 135 140

Cys Thr Glu Glu Phe Pro Leu Cys Ala Thr Asn Pro Tyr Gly Arg Thr
145 150 155 160

Lys Leu Val Ile Glu Asp Ile Cys Arg Asp Val His Arg Ser Asp Pro
165 170 175

Asp Trp Lys Ile Ile Leu Leu Arg Tyr Phe Asn Pro Val Gly Ala His
180 185 190

Pro Ser Gly Tyr Ile Gly Glu Asp Pro Cys Gly Val Pro Asn Asn Leu
195 200 205

Met Pro Tyr Val Gln Gln Val Ala Val Gly Arg Leu Pro His Leu Thr
210 215 220

Val Tyr Gly Thr Asp Tyr Ser Thr Lys Asp Gly Thr Gly Val Arg Asp
225 230 235 240

Tyr Ile His Val Val Asp Leu Ala Asp Gly His Ile Ala Ala Leu Arg
245 250 255

Lys Leu Tyr Glu Asp Ser Asp Lys Ile Gly Cys Glu Val Tyr Asn Leu
260 265 270

Gly Thr Gly Lys Gly Thr Ser Val Leu Glu Met Val Ala Ala Phe Glu
275 280 285

Lys Ala Ser Gly Lys Lys Ile Pro Leu Val Phe Ala Gly Arg Arg Pro
290 295 300

Gly Asp Ala Glu Ile Val Tyr Ala Ala Thr Ala Lys Ala Glu Lys Glu
305 310 315 320

Leu Lys Trp Lys Ala Lys Tyr Gly Ile Glu Glu Met Cys Arg Asp Leu
325 330 335

Trp Asn Trp Ala Ser Lys Asn Pro Tyr Gly Tyr Ala Gly Ser Arg Asp
340 345 350

Asn Ser Lys
355

<210> 317
<211> 1980
<212> DNA
<213> Zea mays

<400> 317

```
cattccatcg tcttcgcttc cactccccac gttccccgcc tgcttcctcc aggccccggc     60
cagatccgcg cgcccgcggg gaagagggcc tcctctcctc cctcctccct tggctcgcgc    120
tctggtcgag gaggcggagg ccggtgccgg cgggcgggcg gggaggtagg tagcagcttg    180
cggttgcggg agggaagctg cgcgcgctag gctgccatgg cggtggagaa gacgctgccc    240
ggcgcgagtg cggggaggac cgtgctggtc acgggcgggg cgggctacat cggtagccac    300
gccgtgctgc agctcctcct cgcgggcttc cgcgccgtcg tcatcgacaa cctcaacaac    360
tcctccgagc tggccgtccg ccgcgtcgcc gcgctcgcgg gggaccactc ccgcaacctc    420
tctttccaca agattgatct ccgtgacaag ggagcactgg aaatggtttt tgcttctaca    480
agatttgaag ctgtcattca cttcgctgga ttgaaagctg tgggtgaaag cgtacagaag    540
ccattacttt attatgacaa caacgtcatt ggcacgataa atcttctaga agttatgtct    600
gttcacggtt gcaagaagtt ggtgttctca tcatcagctg cagtttatgg atcacccaaa    660
aactcaccct gcacagaaaa ttttcctctt actccaaaca atccatatgg caaaacaaag    720
ctcgttgttg aagatatttg ccgggatatc taccgttcag atcctgaatg gaagatcatt    780
ttacttaggt acttcaatcc agttggtgct catcctagtg gatatcttgg cgaggaccca    840
cgaggaattc ccaacaatct tatgccctat gttcagcaag ttgcggttgg taggaggcca    900
gctctaacag ttttaggaaa tgactatgca acaagagatg ggactggggt ccgagattac    960
atccatgtgg ttgaccttgc tgacggacat attgctgcat tgcagaagct tttgagaac    1020
tctagcatag ggtgtgaagc gtacaacctt ggaaccggaa gaggtacatc tgtgctggag   1080
attgttaaag catttgagaa ggcttctggg aagaaaatac ctctgatttt tggtgaaaga   1140
cgcccaggtg atgcagagat tctgttttca gagactacta aagcagagag ggagcttaac   1200
tggaaagcaa aatacggtat tgaagagatg tgccgcgacc aatggaactg ggccagcaag   1260
aacccttatg gctatggatc acctgactct atcaagcaga atggtcacca aacaaacgga   1320
tccgctgact cctccaagca gaatggccac cgcacaaacg gttcaactga ctcacccaag   1380
cggaacggcc accatgcgta tgggtctgct gactcaccca agcgcaacgg gcactgcgtt   1440
tttggatcat cagacctcaa gccgaatggt aatggccacc tgcgctgagc agaactgttt   1500
ggcctgtgag ctccctgtac attcggttgc gatgtgagct ccctgcacgt tcggtcgagg   1560
tctatcgtga acccactatc cgagattgat gtggatcatt gggttgacag gtcatacagt   1620
atagagccgg tggcagagga attcctgttt gctgtgggta aagcttatct tctgctttcg   1680
tgtttttct tgcttctttc gattatggtg taggaatgtg gtcataatgt attagctgat    1740
tatcctttcc ctgctaattg gactttatta cgcttcatta agtttggctg attactgttt   1800
aacggggaaa agttgcacct gtgtacgatt atttatttgt acctgtaata tttatggaga   1860
actagcctcg ccgcaaatag ccctgatgaa ttcagctgca gtttattccc aaaaaaaaaa   1920
aaaaaaaaaa aaaaaaaaaa aaaaaaaaaa aaaaaaaaaa aaaaaaaaaa aaggggcccc   1980
```

<210> 318
<211> 423
<212> PRT
<213> Zea mays

<400> 318

```
Met Ala Val Glu Lys Thr Leu Pro Gly Ala Ser Ala Gly Arg Thr Val
1               5                   10                  15

Leu Val Thr Gly Gly Ala Gly Tyr Ile Gly Ser His Ala Val Leu Gln
            20                  25                  30

Leu Leu Leu Ala Gly Phe Arg Ala Val Val Ile Asp Asn Leu Asn Asn
        35                  40                  45

Ser Ser Glu Leu Ala Val Arg Arg Val Ala Ala Leu Ala Gly Asp His
    50                  55                  60

Ser Arg Asn Leu Ser Phe His Lys Ile Asp Leu Arg Asp Lys Gly Ala
65                  70                  75                  80

Leu Glu Met Val Phe Ala Ser Thr Arg Phe Glu Ala Val Ile His Phe
                85                  90                  95

Ala Gly Leu Lys Ala Val Gly Glu Ser Val Gln Lys Pro Leu Leu Tyr
            100                 105                 110

Tyr Asp Asn Asn Val Ile Gly Thr Ile Asn Leu Leu Glu Val Met Ser
        115                 120                 125

Val His Gly Cys Lys Lys Leu Val Phe Ser Ser Ser Ala Ala Val Tyr
    130                 135                 140

Gly Ser Pro Lys Asn Ser Pro Cys Thr Glu Asn Phe Pro Leu Thr Pro
145                 150                 155                 160

Asn Asn Pro Tyr Gly Lys Thr Lys Leu Val Val Glu Asp Ile Cys Arg
                165                 170                 175

Asp Ile Tyr Arg Ser Asp Pro Glu Trp Lys Ile Ile Leu Leu Arg Tyr
            180                 185                 190

Phe Asn Pro Val Gly Ala His Pro Ser Gly Tyr Leu Gly Glu Asp Pro
        195                 200                 205

Arg Gly Ile Pro Asn Asn Leu Met Pro Tyr Val Gln Gln Val Ala Val
    210                 215                 220

Gly Arg Arg Pro Ala Leu Thr Val Leu Gly Asn Asp Tyr Ala Thr Arg
225                 230                 235                 240

Asp Gly Thr Gly Val Arg Asp Tyr Ile His Val Val Asp Leu Ala Asp
```

245 250 255

Gly His Ile Ala Ala Leu Gln Lys Leu Phe Glu Asn Ser Ser Ile Gly
260 265 270

Cys Glu Ala Tyr Asn Leu Gly Thr Gly Arg Gly Thr Ser Val Leu Glu
275 280 285

Ile Val Lys Ala Phe Glu Lys Ala Ser Gly Lys Lys Ile Pro Leu Ile
290 295 300

Phe Gly Glu Arg Arg Pro Gly Asp Ala Glu Ile Leu Phe Ser Glu Thr
305 310 315 320

Thr Lys Ala Glu Arg Glu Leu Asn Trp Lys Ala Lys Tyr Gly Ile Glu
325 330 335

Glu Met Cys Arg Asp Gln Trp Asn Trp Ala Ser Lys Asn Pro Tyr Gly
340 345 350

Tyr Gly Ser Pro Asp Ser Ile Lys Gln Asn Gly His Gln Thr Asn Gly
355 360 365

Ser Ala Asp Ser Ser Lys Gln Asn Gly His Arg Thr Asn Gly Ser Thr
370 375 380

Asp Ser Pro Lys Arg Asn Gly His His Ala Tyr Gly Ser Ala Asp Ser
385 390 395 400

Pro Lys Arg Asn Gly His Cys Val Phe Gly Ser Ser Asp Leu Lys Pro
405 410 415

Asn Gly Asn Gly His Leu Arg
420

<210> 319
<211> 1444
<212> DNA
<213> Glycine max

<400> 319
acgtgtcaca gaattctcgc cttttccgaa tatggcatcg cgcgtcagca ttggcaatct 60
cacttcctcc gcgccgtata ttaattcccc tcattttcgc tcacaactta agctttccag 120
caactcctct ttgcacaagc cactcatgcg cgacaagact gtgctggtaa ccggcggagc 180
cggttacatc ggcacccaca ccgttcttca gctcttgctc ggaggttgca gaaccgtcgt 240
cgtcgacaat ctcgacaatt cctccgaggt ttctatccac cgagtcaggg agcttgccgg 300
cgaatttggg aacaacctct cctttcacaa ggtggaccta cgggacaggg atgcactaga 360

gcaaattttt gtttccacac aatttgatgc tgtcatacac tttgctggac tgaaagcagt 420
aggagaaagt gtgcaaaaac ctttactata ctataacaac aacttgactg ggacaatcac 480

```
tctattggaa gtcatggctg cccatggatg caagaagctc gtgttctctt cttcagcaac    540
tgtatatggt tggccaaagg aagttccatg cacagaagag ttccctctgt cagcaatgaa    600
cccatatgga cgaactaagc ttatcattga agaaatttgc cgtgatgtcc actgtgcaga    660
gccagattgt aaaataattt tgttaagata cttcaaccca gttggtgcac accccagcgg    720
ttatattggg gaggatcctc gcggaattcc aaacaatctc atgccatttg ttcagcaagt    780
agcagttggc cgacggcctg cactgacagt tttggaaat gattataata caagtgatgg    840
cactggggtt cgggattaca ttcatgttgt tgatttagca gatgggcaca ttgctgcatt    900
gcttaaacta gatgaaccta atataggttg tgaggtttat aacctgggaa caggaaaggg    960
aacatcagtt ttggagatgg ttagagcttt tgaaatggca tctggaaaga aaattccact   1020
tgtgatggct ggccgtagac ctggtgatgc tgaaattgtt tatgcatcaa caaagaaagc   1080
ggaaagagag cttaaatgga aggcaaaata tggcattgat gagatgtgcc gtgatcaatg   1140
gaattgggct agcaaaaacc cttatggcta tggagatcag ggctccaccg attaaccact   1200
tagttttctc tttgggttct tttctgaact cacccacacc gtagtccgta ggtcttgtga   1260
atttagtttt cccaaaagct tttctttctt tagtgatctt aaggtgacaa agtacttgta   1320
ttattactat tcatagttac atagtaagta agtagtggtt tactatactg taatttaaag   1380
gttctctagg ttccttctta caggttattg attattagat tcggattctc tcatgttcca   1440
catg                                                                1444
```

<210> 320
<211> 387
<212> PRT
<213> Glycine max

<400> 320

```
Met Ala Ser Arg Val Ser Ile Gly Asn Leu Thr Ser Ser Ala Pro Tyr
1               5                   10                  15

Ile Asn Ser Pro His Phe Arg Ser Gln Leu Lys Leu Ser Ser Asn Ser
            20                  25                  30

Ser Leu His Lys Pro Leu Met Arg Asp Lys Thr Val Leu Val Thr Gly
        35                  40                  45

Gly Ala Gly Tyr Ile Gly Thr His Thr Val Leu Gln Leu Leu Leu Gly
    50                  55                  60

Gly Cys Arg Thr Val Val Val Asp Asn Leu Asp Asn Ser Ser Glu Val
65                  70                  75                  80

Ser Ile His Arg Val Arg Glu Leu Ala Gly Glu Phe Gly Asn Asn Leu
                85                  90                  95

Ser Phe His Lys Val Asp Leu Arg Asp Arg Asp Ala Leu Glu Gln Ile
            100                 105                 110
```

```
Phe Val Ser Thr Gln Phe Asp Ala Val Ile His Phe Ala Gly Leu Lys
        115                 120                 125

Ala Val Gly Glu Ser Val Gln Lys Pro Leu Leu Tyr Tyr Asn Asn Asn
    130                 135                 140

Leu Thr Gly Thr Ile Thr Leu Leu Glu Val Met Ala Ala His Gly Cys
145                 150                 155                 160

Lys Lys Leu Val Phe Ser Ser Ser Ala Thr Val Tyr Gly Trp Pro Lys
                165                 170                 175

Glu Val Pro Cys Thr Glu Glu Phe Pro Leu Ser Ala Met Asn Pro Tyr
            180                 185                 190

Gly Arg Thr Lys Leu Ile Ile Glu Glu Ile Cys Arg Asp Val His Cys
        195                 200                 205

Ala Glu Pro Asp Cys Lys Ile Ile Leu Leu Arg Tyr Phe Asn Pro Val
    210                 215                 220

Gly Ala His Pro Ser Gly Tyr Ile Gly Glu Asp Pro Arg Gly Ile Pro
225                 230                 235                 240

Asn Asn Leu Met Pro Phe Val Gln Gln Val Ala Val Gly Arg Arg Pro
                245                 250                 255

Ala Leu Thr Val Phe Gly Asn Asp Tyr Asn Thr Ser Asp Gly Thr Gly
            260                 265                 270

Val Arg Asp Tyr Ile His Val Val Asp Leu Ala Asp Gly His Ile Ala
        275                 280                 285

Ala Leu Leu Lys Leu Asp Glu Pro Asn Ile Gly Cys Glu Val Tyr Asn
    290                 295                 300

Leu Gly Thr Gly Lys Gly Thr Ser Val Leu Glu Met Val Arg Ala Phe
305                 310                 315                 320

Glu Met Ala Ser Gly Lys Lys Ile Pro Leu Val Met Ala Gly Arg Arg
                325                 330                 335

Pro Gly Asp Ala Glu Ile Val Tyr Ala Ser Thr Lys Lys Ala Glu Arg
            340                 345                 350

Glu Leu Lys Trp Lys Ala Lys Tyr Gly Ile Asp Glu Met Cys Arg Asp
        355                 360                 365

Gln Trp Asn Trp Ala Ser Lys Asn Pro Tyr Gly Tyr Gly Asp Gln Gly
    370                 375                 380
```

Ser Thr Asp
385

## Description

The present invention relates generally to the field of molecular biology and concerns a method for enhancing various plant yield-related traits by modulating expression in a plant of a nucleic acid encoding a yield increasing polypeptide selected from the group consisting of:

- bHLH6-like (basic Helix-Loop-Helix 6-like) protein,
- a GRP (Growth Regulating Protein), wherein said GRP is selected from the group consisting of:
  - an RrmJ/FtsJ ribosomal RNA methyltransferase polypeptide (RrmJ/FtsJ polypeptide)
  - a basic-helix-loop-helix 4 (bHLH4) polypeptide
  - an isopentenyl transferase (IPT) polypeptide
- an STO (Salt Tolerance) protein
- a UGE (UDP-Glucose 4-Epimerase or UDP-Gal 4-Epimerase) polypeptide.

The present invention also concerns plants having modulated expression of a nucleic acid encoding a yield increasing polypeptide, which plants have enhanced yield-related relative to corresponding wild type plants or other control plants. The invention also provides constructs useful in the methods of the invention.

The ever-increasing world population and the dwindling supply of arable land available for agriculture fuels research towards increasing the efficiency of agriculture. Conventional means for crop and horticultural improvements utilise selective breeding techniques to identify plants having desirable characteristics. However, such selective breeding techniques have several drawbacks, namely that these techniques are typically labour intensive and result in plants that often contain heterogeneous genetic components that may not always result in the desirable trait being passed on from parent plants. Advances in molecular biology have allowed mankind to modify the germplasm of animals and plants. Genetic engineering of plants entails the isolation and manipulation of genetic material (typically in the form of DNA or RNA) and the subsequent introduction of that genetic material into a plant. Such technology has the capacity to deliver crops or plants having various improved economic, agronomic or horticultural traits.

A trait of particular economic interest is increased yield. Yield is normally defined as the measurable produce of economic value from a crop. This may be defined in terms of quantity and/or quality. Yield is directly dependent on several factors, for example, the number and size of the organs, plant architecture (for example, the number of branches), seed production, leaf senescence and more. Root development, nutrient uptake, stress tolerance and early vigour may also be important factors in determining yield. Optimizing the abovementioned factors may therefore contribute to increasing crop yield.

Seed yield is a particularly important trait, since the seeds of many plants are important for human and animal nutrition. Crops such as corn, rice, wheat, canola and soybean account for over half the total human caloric intake, whether through direct consumption of the seeds themselves or through consumption of meat products raised on processed seeds. They are also a source of sugars, oils and many kinds of metabolites used in industrial processes. Seeds contain an embryo (the source of new shoots and roots) and an endosperm (the source of nutrients for embryo growth during germination and during early growth of seedlings). The development of a seed involves many genes, and requires the transfer of metabolites from the roots, leaves and stems into the growing seed. The endosperm, in particular, assimilates the metabolic precursors of carbohydrates, oils and proteins and synthesizes them into storage macromolecules to fill out the grain.

A further interesting trait is the flowering time of a plant. The life span of a plant can be divided in phases such as germination, vegetative growth, reproductive growth and senescence. The flowering time is the time elapsed between sowing and start of reproductive growth. It is a crucial moment in the life of a plant that determines the transition from vegetative to reproductive growth, which in some plants coincides with the start of senescence. In many plants, this is the point in time at which the shoot apical meristem stops making leaves and starts making flowers which has a great impact on morphogenesis affecting, for example, the number of organs formed and the overall size and shape of the plant. The flowering time also impacts other yield-related traits in plants. Typically an early flowering variety shows less branching or tillering and therefore is less bushy. Such traits may be advantageous to the farmer to, for example, simplify crop management. On the other hand, delayed flowering may result in plants with more vegetative organs, for example more leaves which is a desirable trait in many crops, particularly in crops where the vegetative organs are harvested, such as lettuce. The relative duration of vegetative and reproductive phase of a plant directly affects its seed yield. In some plants, control of flowering time is a mechanism used to avoid negative impact of stresses such as drought. Flowering time may also affect quality traits of crops, for example herbage quality in forage crops, where delay in flowering may result in higher digestibility. The flowering time affects the length of the cultivation season. Modification of flowering time of a crop may result in the possibility to extend the geographic area of cultivation and therefore increase the cultivated acreage. It may also result in plants being more amenable to agriculture in a given environment, for example early flowering may allow late planting in areas where crop establishment may be negatively affected by low temperatures or may allow early harvest to avoid biotic and abiotic pressure at the end of the season, resulting therefore in an increase in the yield of the crop. Therefore, the ability to control flowering time is an important factor with many industrial applications in the field of agriculture.

Another important trait for many crops is early vigour. Improving early vigour is an important objective of modern rice breeding programs in both temperate and tropical rice cultivars. Long roots are important for proper soil anchorage in water-seeded rice. Where rice is sown directly into flooded fields, and where plants must emerge rapidly through water, longer shoots are associated with vigour. Where drill-seeding is practiced, longer mesocotyls and coleoptiles are important for good seedling emergence. The ability to engineer early vigour into plants would be of great importance in agriculture. For example, poor early vigour has been a limitation to the introduction of maize (Zea mays L.) hybrids based on Corn Belt germplasm in the European Atlantic.

A further important trait is that of improved abiotic stress tolerance. Abiotic stress is a primary cause of crop loss worldwide, reducing average yields for most major crop plants by more than 50% (Wang et al., Planta (2003) 218: 1-14). Abiotic stresses may be caused by drought, salinity, extremes of temperature, chemical toxicity, from 59348: excess or lack of nutrients (macroelements and/or microelemtens), radiation and oxidative stress. The ability to improve plant tolerance to abiotic stress would be of great economic advantage to farmers worldwide and would allow for the cultivation of crops during adverse conditions and in territories where cultivation of crops may not otherwise be possible.

Crop yield may therefore be increased by optimising one of the above-mentioned factors.

Depending on the end use, the modification of certain yield traits may be favoured over others. For example for applications such as forage or wood production, or bio-fuel resource, an increase in the vegetative parts of a plant may be desirable, and for applications such as flour, starch or oil production, an increase in seed parameters may be particularly desirable. Even amongst the seed parameters, some may be favoured over others, depending on the application. Various mechanisms may contribute to increasing seed yield, whether that is in the form of increased seed size or increased seed number.

One approach to increasing yield (seed yield and/or biomass) in plants may be through modification of the inherent growth mechanisms of a plant, such as the cell cycle or various signalling pathways involved in plant growth or in defense mechanisms.

It has now been found that various yield-related traits may be enhanced in plants by modulating expression in a plant of a nucleic acid encoding a yield increasing polypeptide selected from the group consisting of:

- bHLH6-like (basic Helix-Loop-Helix 6-like) protein,
- a GRP (Growth Regulating Protein), wherein said GRP is selected from the group consisting of:

    - an RrmJ/FtsJ ribosomal RNA methyltransferase polypeptide (RrmJ/FtsJ polypeptide)
    - a basic-helix-loop-helix 4 (bHLH4) polypeptide
    - an isopentenyl transferase (IPT) polypeptide

- an STO (Salt Tolerance) protein
- a UGE (UDP-Glucose 4-Epimerase or UDP-Gal 4-Epimerase) polypeptide.

## Background

bHLH6-like (basic Helix-Loop-Helix 6-like) protein

Transcription factors are usually defined as proteins that show sequence-specific DNA binding and that are capable of activating and/or repressing transcription. The basic Helix-Loop-Helix transcription factor family is one of the largest families of transcription factors that have been characterised in *Arabidopsis thaliana* (Toledo-Ortiz et al., Plant Cell 15, 1749-1770, 2003; Bailey et al., Plant Cell 15, 2497-2501, 2003) and in rice (Li et al Plant Physiol. 141, 1167-1184, 2006). The distinguishing characteristic of the bHLH transcription factor family is the presence of a bipartite domain consisting of approximately 60 amino acids. This bipartite domain is comprised of a DNA-binding basic region, which binds to a consensus hexanucleotide E-box and two $\alpha$-helices separated by a variable loop region, located C-terminally of the basic domain. The two $\alpha$-helices promote dimerisation, allowing the formation of homo- and heterodimers between different family members. While the bHLH domain is evolutionarily conserved, there is little sequence similarity between clades beyond the domain. Li et al. (2006) classify the rice and Arabidopsis bHLH transcription factors into 22 subfamilies, based on the sequence of the bHLH domains.

AtbHLH6 (AtMYC2) is a 68 kDa MYC-related transcriptional activator with a bHLH type DNA-binding domain (Abe et al, Plant Cell 9, 1859-1868, 1997). It is induced by dehydration stress and upon treatment with abscisic acid (ABA). AtMYC2 recognises a MYC recognition site in the promoter of the rd22 gene, an ABA responsive protein without an ABRE-element in the promoter. Plants overexpressing AtMYC2 have a higher sensitivity to ABA, with enhanced ABA-induced gene expression, whereas an insertion mutant showed the opposite (reduced ABA sensitivity and decreased ABA-induced gene expression); furthermore AtMYC2 overexpressing plants were more resistant to osmotic stress (Abe et al., Plant Cell 15, 63-78, 2003). Plants overexpressing AtMYC2 reportedly did not show morphological changes compared to wild type plants and also the cell shape in leaves was not affected. Furthermore, AtMYC2 was shown to be involved in pathogen and wound responses; in tryptophan and tryptophan-derived secondary metabolism and in tolerance to methyl viologen (Paraquat) (Dombrecht et al., Plant Cell 19, 2225-2245, 2007).

STO (Salt Tolerance) protein

STO protein, Salt Tolerance Protein, was first identified from Arabidopsis thaliana in a screen for genes suppressing defective salt response in a calcienurin mutant in yeast (Lippuner et a I. 1996). Methods for conferring salt tolerance in a plant by expressing the Arabidopsis STO have been previously disclosed (US5,859,337; Nagaoka and Takano 2003, J. Exp. Bot. 54, 391-396).

Structurally STO is characterized by the presence of a well-known domain called the B-box domain in which the structure CX2CX16CX2C is conserved. The B-box domain is a promiscuous domain present in proteins of archeobacterial, prokaryotic and eukaryotic origin. It is found essentially in transcription factors, ribonucleoproteins and protooncoproteins. The B-box domain in STO is structurally similar to that found in the Arabidopsis thaliana protein CONSTANS, a transcription factor involved in repression of flowering time. However STO and CONSTANs belong to different protein families, with CONSTANS being characterized by the presence of a plant-specific conserved domain, the so called the CCT box in addition to the B-box, while in the STO family of proteins only the B-box is present (Griffiths et al. 2003, Plant Phys, 131, 1855-1867; Lagercrants et al. 2000 Mol.Biol.Evol. 17, 1499-1507).

The biological function of STO in Arabidopsis has been further investigated using gain and loss of function mutants (Indorf, et al. 2007, Plant J. 51(4):563-74.). Data reported from those studies suggested a role for STO in light signalling where it was mentioned that STO could act as a negative regulator in phytochrome and blue light signalling. These studies were directed to understanding, the role of STO in the salt stress and light response.

UGE (UDP-Glucose 4-Epimerase or UDP-Gal 4-Epimerase) polypeptide

The biosynthesis of carbohydrates in plants requires specific glycosyl-transferases that act on activated sugars, typically uridine (UDP), adenosine or guanosine diphosphate hexoses and pentoses. Nucleotide sugars are modified at their glycosyl moities by nucleotide sugar interconversion enzymes to generate different sugars that are intermediates in the uptake of the free sugars released from the breakdown of nutritional or storage carbohydrates and other sources. One of the best-characterized nucleotide sugar interconversion enzymes is UGE (EC.5.1.3.2 according to the IUBMB - International Union of Biochemistry and Molecular Biology -Enzyme Nomenclature).

UGE, UDP-Glucose 4-Epimerase or UDP-Gal 4-Epimerase (EC.5.1.3.2) catalyses the interconversion of UDP-glucose and UDP-galactose. Varying concentrations of the cofactor NAD+/NADH (Nicotinamide Adenine Dinucleotide -oxidized/reduced-), can result in differential stimulation of the reaction. X-ray crystallography and other studies suggest that UGEs are dimeric, although active monomers and higher polymers have also been reported under certain experimental conditions (Thoden et al. 2005 J. Biol. Chem 280, 21900-21907).

UGE is essential for de novo biosynthesis of UDP-Gal, a precursor for the biosynthesis of numerous different carbohydrates, glycolipids and glycosides. UGE is also required for the catabolic uptake of galactose into the central metabolism, and therefore UGE deficiency exarcerbates galactose toxicity in plants (Dormann and Benning, 1998; Plant J. 13, 641-652) and in yeast and it also leads to different forms of human galactosemia.

UGE enzymes are encoded in the genome of microorganisms as well as of higher organisms. In the model plant *Arabidopsis thaliana* five different paralogous genes enconding UGE isoforms have been identified. The rice and poplar genomes encode at least seven predicted UGE isoforms.

The enzymatic properties and genetic role of UGE isoforms has been addressed in Arabidopsis thaliana in biochemical studies as well as by characterization of gain and loss of function mutants. All five Arabidopsis UGEs are enzymatically active in similar ranges of temperature (30 - 40 C) and PH (PH7-PH9). Homo and heterodimers can be formed between UGE isoforms. The five isoforms are functional in vivo and can complement the loss of function in the yeast GAL10 mutant lacking UGE function. The latter reflects the functional conservation of UGE enzymatic activities between UGEs of microorganisms and higher plants (Barber et al. 2006; JBC 281, 17276-17285). In Arabidopsis the 5 paralogous genes encoding UGEs are expressed preferentially in the roots. Mutations or loss of UGE function typically resulted in general growth defects of vegetative organs and flowers were smaller and abnormally shaped. Surprisingly the floral number and position was not altered, though sterility was reported (Rosti et al. 2007; The Plant Cell 19, 1565-1579). In accordance with UGE activity the cell wall galactose content in these mutants was altered. Patent US,6,9922,36 discloses plant-derived UGE genes and teaches methods to express them in plants to allow modification of carbohydrate metabolism in plants.

**Summary**

Surprisingly, it has now been found that modulating expression of a nucleic acid encoding a yield increasing polypeptide selected from the group consisting of:

- bHLH6-like (basic Helix-Loop-Helix 6-like) protein,
- a GRP (Growth Regulating Protein), wherein said GRP is selected from the group consisting of:

    - an RrmJ/FtsJ ribosomal RNA methyltransferase polypeptide (RrmJ/FtsJ polypeptide)
    - a basic-helix-loop-helix 4 (bHLH4) polypeptide
    - an isopentenyl transferase (IPT) polypeptide

- an STO (Salt Tolerance) protein
- a UGE (UDP-Glucose 4-Epimerase or UDP-Gal 4-Epimerase) polypeptide gives plants having enhanced yield-related traits relative to control plants.

According one embodiment, there is provided a method for enhancing yield related traits of a plant relative to control plants, comprising modulating expression of a nucleic acid encoding a bHLH6-like polypeptide in a plant. The enhanced yield related traits comprised increased increased yield and increased emergence vigour.

According one embodiment, there is provided a method for enhancing yield-related traits of a plant relative to control plants, comprising modulating expression of a nucleic acid sequence encoding a GRP polypeptide in a plant, wherein said GRP polypeptide is an RrmJ/FtsJ ribosomal RNA methyltransferase polypeptide (RrmJ/FtsJ polypeptide). The enhanced yield-related traits comprise increased seed yield, increased number of filled seeds, increased seed fill rate, and increased harvest index.

According to one embodiment, there is provided a method for enhancing yield-related traits of a plant relative to control plants, comprising modulating expression (preferably, increasing) of a nucleic acid sequence encoding a GRP polypeptide in a plant, wherein said GRP polypeptide is a basic-helix-loop-helix 4 (bHLH4) polypeptide. The enhanced yield-related traits are preferably enhanced seed yield-related traits, comprising one or more of: increased early vigour, increased greenness index, increased total seed yield per plant, increased number of filled seeds, increased seed fill rate, increased, and increased harvest index

According to one embodiment, there is provided a method for enhancing yield-related traits of a plant relative to control plants, comprising modulating expression (preferably, increasing) of a nucleic acid sequence encoding a GRP polypeptide in a plant, wherein said GRP polypeptide is an isopentenyl transferase (IPT) polypeptide. The enhanced yield-related traits are one or more of: increased total seed yield per plant, increased number of filled seeds, increased total number of seeds, and increased harvest index.

According one embodiment, there is provided a method for enhancing yield-related traits plants, particularly increasing early vigour and altering flowering time, particularly shortening flowering time relative to control plants, comprising modulating expression of a nucleic acid encoding an STO polypeptide in a plant.

According to one embodiment, there is provided a method for enhancing yield related traits of a plant relative to control plants, in particular increased yield, comprising modulating expression of a nucleic acid encoding a UGE polypeptide in a plant.

***Definitions***

The terms improving and/or enhancing and/or improved and/or enhanced plant yield-related traits are used herein encompass improving and/or enhancing and/or improved and/or enhanced plant growth characteristics.

Polypeptide(s)/Protein(s)

The terms "polypeptide" and "protein" are used interchangeably herein and refer to amino acids in a polymeric form of any length, linked together by peptide bonds.

Polynucleotide(s)/Nucleic acid(s)/Nucleic acid sequence(s)/nucleotide sequence(s)

The terms "polynucleotide(s)", "nucleic acid sequence(s)", "nucleotide sequence(s)", "nucleic acid(s)", "nucleic acid molecule" are used interchangeably herein and refer to nucleotides, either ribonucleotides or deoxyribonucleotides or a combination of both, in a polymeric unbranched form of any length.

Control plant(s)

The choice of suitable control plants is a routine part of an experimental setup and may include corresponding wild type plants or corresponding plants without the gene of interest. The control plant is typically of the same plant species or even of the same variety as the plant to be assessed. The control plant may also be a nullizygote of the plant to be assessed. Nullizygotes are individuals missing the transgene by segregation. A "control plant" as used herein refers not only to whole plants, but also to plant parts, including seeds and seed parts.

Homologue(s)

"Homologues" of a protein encompass peptides, oligopeptides, polypeptides, proteins and enzymes having amino acid substitutions, deletions and/or insertions relative to the unmodified protein in question and having similar biological and functional activity as the unmodified protein from which they are derived.

A deletion refers to removal of one or more amino acids from a protein.

An insertion refers to one or more amino acid residues being introduced into a predetermined site in a protein. Insertions may comprise N-terminal and/or C-terminal fusions as well as intra-sequence insertions of single or multiple amino acids. Generally, insertions within the amino acid sequence will be smaller than N- or C-terminal fusions, of the order of about 1 to 10 residues. Examples of N- or C-terminal fusion proteins or peptides include the binding domain or activation domain of a transcriptional activator as used in the yeast two-hybrid system, phage coat proteins, (histidine)-6-tag, glutathione S-transferase-tag, protein A, maltose-binding protein, dihydrofolate reductase, Tag•100 epitope, c-myc epitope, FLAG®-epitope, lacZ, CMP (calmodulin-binding peptide), HA epitope, protein C epitope and VSV epitope.

A substitution refers to replacement of amino acids of the protein with other amino acids having similar properties (such as similar hydrophobicity, hydrophilicity, antigenicity, propensity to form or break α-helical structures or β-sheet structures). Amino acid substitutions are typically of single residues, but may be clustered depending upon functional constraints placed upon the polypeptide; insertions will usually be of the order of about 1 to 10 amino acid residues. The amino acid substitutions are preferably conservative amino acid substitutions. Conservative substitution tables are well known in the art (see for example Creighton (1984) Proteins. W.H. Freeman and Company (Eds) and Table 1 below).

**Table 1:** Examples of conserved amino acid substitutions

| Residue | Conservative Substitutions | Residue | Conservative Substitutions |
|---|---|---|---|
| Ala | Ser | Leu | Ile; Val |
| Arg | Lys | Lys | Arg; Gln |
| Asn | Gln; His | Met | Leu; Ile |
| Asp | Glu | Phe | Met; Leu; Tyr |
| Gln | Asn | Ser | Thr; Gly |
| Cys | Ser | Thr | Ser; Val |
| Glu | Asp | Trp | Tyr |
| Gly | Pro | Tyr | Trp; Phe |
| His | Asn; Gln | Val | Ile; Leu |
| Ile | Leu, Val | | |

Amino acid substitutions, deletions and/or insertions may readily be made using peptide synthetic techniques well known in the art, such as solid phase peptide synthesis and the like, or by recombinant DNA manipulation. Methods for the manipulation of DNA sequences to produce substitution, insertion or deletion variants of a protein are well known in the art. For example, techniques for making substitution mutations at predetermined sites in DNA are well known to those skilled in the art and include M13 mutagenesis, T7-Gen in vitro mutagenesis (USB, Cleveland, OH), QuickChange Site Directed mutagenesis (Stratagene, San Diego, CA), PCR-mediated site-directed mutagenesis or other site-directed mutagenesis protocols.

Derivatives

"Derivatives" include peptides, oligopeptides, polypeptides which may, compared to the amino acid sequence of the naturally-occurring form of the protein, such as the protein of interest, comprise substitutions of amino acids with non-naturally occurring amino acid residues, or additions of non-naturally occurring amino acid residues. "Derivatives" of a protein also encompass peptides, oligopeptides, polypeptides which comprise naturally occurring altered (glycosylated, acylated, prenylated, phosphorylated, myristoylated, sulphated etc.) or non-naturally altered amino acid residues compared to the amino acid sequence of a naturally-occurring form of the polypeptide. A derivative may also comprise one or more non-amino acid substituents or additions compared to the amino acid sequence from which it is derived, for example a reporter molecule or other ligand, covalently or non-covalently bound to the amino acid sequence, such as a reporter molecule which is bound to facilitate its detection, and non-naturally occurring amino acid residues relative to the amino acid sequence of a naturally-occurring protein. Furthermore, "derivatives" also include fusions of the naturally-occurring form of the protein with tagging peptides such as FLAG, HIS6 or thioredoxin (for a review of tagging peptides, see Terpe, Appl. Microbiol. Biotechnol. 60, 523-533, 2003).

Orthologue(s)/Paralogue(s)

Orthologues and paralogues encompass evolutionary concepts used to describe the ancestral relationships of genes. Paralogues are genes within the same species that have originated through duplication of an ancestral gene; orthologues are genes from different organisms that have originated through speciation, and are also derived from a common ancestral gene.

Domain

The term "domain" refers to a set of amino acids conserved at specific positions along an alignment of sequences of evolutionarily related proteins. While amino acids at other positions can vary between homologues, amino acids that are highly conserved at specific positions indicate amino acids that are likely essential in the structure, stability or function of a protein. Identified by their high degree of conservation in aligned sequences of a family of protein homologues, they can be used as identifiers to determine if any polypeptide in question belongs to a previously identified polypeptide family.

Motif/Consensus sequence/Signature

The term "motif" or "consensus sequence" or "signature" refers to a short conserved region in the sequence of evolutionarily related proteins. Motifs are frequently highly conserved parts of domains, but may also include only part of the domain, or be located outside of conserved domain (if all of the amino acids of the motif fall outside of a defined domain).

Hybridisation

The term "hybridisation" as defined herein is a process wherein substantially homologous complementary nucleotide sequences anneal to each other. The hybridisation process can occur entirely in solution, i.e. both complementary nucleic acids are in solution. The hybridisation process can also occur with one of the complementary nucleic acids immobilised to a matrix such as magnetic beads, Sepharose beads or any other resin. The hybridisation process can furthermore occur with one of the complementary nucleic acids immobilised to a solid support such as a nitrocellulose or nylon membrane or immobilised by e.g. photolithography to, for example, a siliceous glass support (the latter known as nucleic acid arrays or microarrays or as nucleic acid chips). In order to allow hybridisation to occur, the nucleic acid molecules are generally thermally or chemically denatured to melt a double strand into two single strands and/or to remove hairpins or other secondary structures from single stranded nucleic acids.

The term "stringency" refers to the conditions under which a hybridisation takes place. The stringency of hybridisation is influenced by conditions such as temperature, salt concentration, ionic strength and hybridisation buffer composition. Generally, low stringency conditions are selected to be about 30°C lower than the thermal melting point ($T_m$) for the specific sequence at a defined ionic strength and pH. Medium stringency conditions are when the temperature is 20°C below $T_m$, and high stringency conditions are when the temperature is 10°C below $T_m$. High stringency hybridisation conditions are typically used for isolating hybridising sequences that have high sequence similarity to the target nucleic acid sequence. However, nucleic acids may deviate in sequence and still encode a substantially identical polypeptide, due to the degeneracy of the genetic code. Therefore medium stringency hybridisation conditions may sometimes be needed to identify such nucleic acid molecules.

The Tm is the temperature under defined ionic strength and pH, at which 50% of the target sequence hybridises to a perfectly matched probe. The $T_m$ is dependent upon the solution conditions and the base composition and length of the probe. For example, longer sequences hybridise specifically at higher temperatures. The maximum rate of hybridisation is obtained from about 16°C up to 32°C below $T_m$. The presence of monovalent cations in the hybridisation solution reduce the electrostatic repulsion between the two nucleic acid strands thereby promoting hybrid formation; this effect is visible for sodium concentrations of up to 0.4M (for higher concentrations, this effect may be ignored). Formamide reduces the melting temperature of DNA-DNA and DNA-RNA duplexes with 0.6 to 0.7°C for each percent formamide, and addition of 50% formamide allows hybridisation to be performed at 30 to 45°C, though the rate of hybridisation will be lowered. Base pair mismatches reduce the hybridisation rate and the thermal stability of the duplexes. On average and for large probes, the Tm decreases about 1°C per % base mismatch. The Tm may be calculated using the following equations, depending on the types of hybrids:

1) DNA-DNA hybrids (Meinkoth and Wahl, Anal. Biochem., 138: 267-284, 1984):

$$T_m = 81.5°C + 16.6x\log_{10}[Na^+]^a + 0.41x\%[G/C^b] - 500x[L^c]^{-1} - 0.61x\%\ formamide$$

2) DNA-RNA or RNA-RNA hybrids:

$$Tm = 79.8 + 18.5\ (\log_{10}[Na^+]^a) + 0.58\ (\%G/C^b) + 11.8\ (\%G/C^b)^2 - 820/L^c$$

3) oligo-DNA or oligo-RNA[d] hybrids:

For <20 nucleotides: $T_m$= 2 ($I_n$)
For 20-35 nucleotides: $T_m$= 22 + 1.46 (In)

[a] or for other monovalent cation, but only accurate in the 0.01-0.4 M range.
[b] only accurate for %GC in the 30% to 75% range.
[c] L = length of duplex in base pairs.
[d] oligo, oligonucleotide; $I_n$, = effective length of primer = 2×(no. of G/C)+(no. of A/T).

Non-specific binding may be controlled using any one of a number of known techniques such as, for example, blocking the membrane with protein containing solutions, additions of heterologous RNA, DNA, and SDS to the hybridisation buffer, and treatment with Rnase. For non-homologous probes, a series of hybridizations may be performed by varying one of (i) progressively lowering the annealing temperature (for example from 68°C to 42°C) or (ii) progressively lowering the formamide concentration (for example from 50% to 0%). The skilled artisan is aware of various parameters which may be altered during hybridisation and which will either maintain or change the stringency conditions.

Besides the hybridisation conditions, specificity of hybridisation typically also depends on the function of post-hybridisation washes. To remove background resulting from non-specific hybridisation, samples are washed with dilute salt solutions. Critical factors of such washes include the ionic strength and temperature of the final wash solution: the lower the salt concentration and the higher the wash temperature, the higher the stringency of the wash. Wash conditions are typically performed at or below hybridisation stringency. A positive hybridisation gives a signal that is at least twice of that of the background. Generally, suitable stringent conditions for nucleic acid hybridisation assays or gene amplification detection procedures are as set forth above. More or less stringent conditions may also be selected. The skilled artisan is aware of various parameters which may be altered during washing and which will either maintain or change the stringency conditions.

For example, typical high stringency hybridisation conditions for DNA hybrids longer than 50 nucleotides encompass hybridisation at 65°C in 1x SSC or at 42°C in 1x SSC and 50% formamide, followed by washing at 65°C in 0.3x SSC. Examples of medium stringency hybridisation conditions for DNA hybrids longer than 50 nucleotides encompass hybridisation at 50°C in 4x SSC or at 40°C in 6x SSC and 50% formamide, followed by washing at 50°C in 2x SSC. The length of the hybrid is the anticipated length for the hybridising nucleic acid. When nucleic acids of known sequence are hybridised, the hybrid length may be determined by aligning the sequences and identifying the conserved regions described herein. 1×SSC is 0.15M NaCl and 15mM sodium citrate; the hybridisation solution and wash solutions may additionally include 5x Denhardt's reagent, 0.5-1.0% SDS, 100 μg/ml denatured, fragmented salmon sperm DNA, 0.5% sodium pyrophosphate.

For the purposes of defining the level of stringency, reference can be made to Sambrook et al. (2001) Molecular Cloning: a laboratory manual, 3rd Edition, Cold Spring Harbor Laboratory Press, CSH, New York or to Current Protocols in Molecular Biology, John Wiley & Sons, N.Y. (1989 and yearly updates).

Splice variant

The term "splice variant" as used herein encompasses variants of a nucleic acid sequence in which selected introns and/or exons have been excised, replaced, displaced or added, or in which introns have been shortened or lengthened. Such variants will be ones in which the biological activity of the protein is substantially retained; this may be achieved by selectively retaining functional segments of the protein. Such splice variants may be found in nature or may be manmade. Methods for predicting and isolating such splice variants are well known in the art (see for example Foissac and Schiex (2005) BMC Bioinformatics 6: 25).

Allelic variant

Alleles or allelic variants are alternative forms of a given gene, located at the same chromosomal position. Allelic variants encompass Single Nucleotide Polymorphisms (SNPs), as well as Small Insertion/Deletion Polymorphisms (INDELs). The size of INDELs is usually less than 100 bp. SNPs and INDELs form the largest set of sequence variants in naturally occurring polymorphic strains of most organisms.

Gene shuffling/Directed evolution

Gene shuffling or directed evolution consists of iterations of DNA shuffling followed by appropriate screening and/or selection to generate variants of nucleic acids or portions thereof encoding proteins having a modified biological activity (Castle et al., (2004) Science 304(5674): 1151-4; US patents 5,811,238 and 6,395,547).

Regulatory element/Control sequence/Promoter

The terms "regulatory element", "control sequence" and "promoter" are all used interchangeably herein and are to be taken in a broad context to refer to regulatory nucleic acid sequences capable of effecting expression of the sequences to which they are ligated. The term "promoter" typically refers to a nucleic acid control sequence located upstream from the transcriptional start of a gene and which is involved in recognising and binding of RNA polymerase and other proteins, thereby directing transcription of an operably linked nucleic acid. Encompassed by the aforementioned terms are transcriptional regulatory sequences derived from a classical eukaryotic genomic gene (including the TATA box which is required for accurate transcription initiation, with or without a CCAAT box sequence) and additional regulatory elements (i.e. upstream activating sequences, enhancers and silencers) which alter gene expression in response to developmental and/or external stimuli, or in a tissue-specific manner. Also included within the term is a transcriptional regulatory sequence of a classical prokaryotic gene, in which case it may include a -35 box sequence and/or -10 box transcriptional regulatory sequences. The term "regulatory element" also encompasses a synthetic fusion molecule or derivative that confers, activates or enhances expression of a nucleic acid molecule in a cell, tissue or organ.

A "plant promoter" comprises regulatory elements, which mediate the expression of a coding sequence segment in plant cells. Accordingly, a plant promoter need not be of plant origin, but may originate from viruses or micro-organisms, for example from viruses which attack plant cells. The "plant promoter" can also originate from a plant cell, e.g. from the plant which is transformed with the nucleic acid sequence to be expressed in the inventive process and described herein. This also applies to other "plant" regulatory signals, such as "plant" terminators. The promoters upstream of the nucleotide sequences useful in the methods of the present invention can be modified by one or more nucleotide substitution(s), insertion(s) and/or deletion(s) without interfering with the functionality or activity of either the promoters, the open reading frame (ORF) or the 3'-regulatory region such as terminators or other 3' regulatory regions which are located away from the ORF. It is furthermore possible that the activity of the promoters is increased by modification of their sequence, or that they are replaced completely by more active promoters, even promoters from heterologous organisms. For expression in plants, the nucleic acid molecule must, as described above, be linked operably to or comprise a suitable promoter which expresses the gene at the right point in time and with the required spatial expression pattern.

For the identification of functionally equivalent promoters, the promoter strength and/or expression pattern of a candidate promoter may be analysed for example by operably linking the promoter to a reporter gene and assaying the expression level and pattern of the reporter gene in various tissues of the plant. Suitable well-known reporter genes include for example beta-glucuronidase or beta-galactosidase. The promoter activity is assayed by measuring the enzymatic activity of the beta-glucuronidase or beta-galactosidase. The promoter strength and/or expression pattern may then be compared to that of a reference promoter (such as the one used in the methods of the present invention). Alternatively, promoter strength may be assayed by quantifying mRNA levels or by comparing mRNA levels of the nucleic acid used in the methods of the present invention, with mRNA levels of housekeeping genes such as 18S rRNA, using methods known in the art, such as Northern blotting with densitometric analysis of autoradiograms, quantitative real-time PCR or RT-PCR (Heid et al., 1996 Genome Methods 6: 986-994). Generally by "weak promoter" is intended a promoter that drives expression of a coding sequence at a low level. By "low level" is intended at levels of about 1/10,000 transcripts to about 1/100,000 transcripts, to about 1/500,0000 transcripts per cell. Conversely, a "strong promoter" drives expression of a coding sequence at high level, or at about 1/10 transcripts to about 1/100 transcripts to about 1/1000 transcripts per cell. An example of a strong promoter is the CaMV 35S promoter.

Operably linked

The term "operably linked" as used herein refers to a functional linkage between the promoter sequence and the gene of interest, such that the promoter sequence is able to initiate transcription of the gene of interest.

Constitutive promoter

A "constitutive promoter" refers to a promoter that is transcriptionally active during most, but not necessarily all, phases of growth and development and under most environmental conditions, in at least one cell, tissue or organ. Table 2a below gives examples of constitutive promoters.

**Table 2a:** Examples of constitutive promoters

| Gene Source | Reference |
|---|---|
| Actin | McElroy et al, Plant Cell, 2: 163-171, 1990 |
| HMGP | WO 2004/070039 |
| CAMV 35S | Odell et al, Nature, 313: 810-812, 1985 |
| CaMV 19S | Nilsson et al., Physiol. Plant. 100:456-462, 1997 |
| GOS2 | de Pater et al, Plant J Nov;2(6):837-44, 1992, WO 2004/065596 |
| Ubiquitin | Christensen et al, Plant Mol. Biol. 18: 675-689, 1992 |
| Rice cyclophilin | Buchholz et al, Plant Mol Biol. 25(5): 837-43, 1994 |
| Maize H3 histone | Lepetit et al, Mol. Gen. Genet. 231:276-285, 1992 |
| Alfalfa H3 histone | Wu et al. Plant Mol. Biol. 11:641-649, 1988 |
| Actin 2 | An et al, Plant J. 10(1); 107-121, 1996 |
| 34S FMV | Sanger et al., Plant. Mol. Biol., 14, 1990: 433-443 |
| Rubisco small subunit | US 4,962,028 |
| OCS | Leisner (1988) Proc Natl Acad Sci USA 85(5): 2553 |
| SAD1 | Jain et al., Crop Science, 39 (6), 1999: 1696 |
| SAD2 | Jain et al., Crop Science, 39 (6), 1999: 1696 |
| nos | Shaw et al. (1984) Nucleic Acids Res. 12(20):7831-7846 |
| V-ATPase | WO 01/14572 |
| Super promoter | WO 95/14098 |
| G-box proteins | WO 94/12015 |

Ubiquitous promoter

A ubiquitous promoter is active in substantially all tissues or cells of an organism.

Developmentally-regulated promoter

A developmentally-regulated promoter is active during certain developmental stages or in parts of the plant that undergo developmental changes.

Inducible promoter

An inducible promoter has induced or increased transcription initiation in response to a chemical (for a review see Gatz 1997, Annu. Rev. Plant Physiol. Plant Mol. Biol., 48:89-108), environmental or physical stimulus, or may be "stress-inducible", i.e. activated when a plant is exposed to various stress conditions, or a "pathogen-inducible" i.e. activated when a plant is exposed to exposure to various pathogens.

Organ-specific/Tissue-specific promoter

An organ-specific or tissue-specific promoter is one that is capable of preferentially initiating transcription in certain organs or tissues, such as the leaves, roots, seed tissue etc. For example, a "root-specific promoter" is a promoter that is transcriptionally active predominantly in plant roots, substantially to the exclusion of any other parts of a plant, whilst still allowing for any leaky expression in these other plant parts. Promoters able to initiate transcription in certain cells only are referred to herein as "cell-specific".

A seed-specific promoter is transcriptionally active predominantly in seed tissue, but not necessarily exclusively in seed tissue (in cases of leaky expression). The seed-specific promoter may be active during seed development and/or during germination from 59392: Examples of seed-specific promoters are given in Qing Qu and Takaiwa (Plant Biotechnol. J. 2, 113-125, 2004).

From 59348:

For example, the seed-specific promoter may drive expression in one or more seed tissues such as endosperm, aleurone, embryo. Examples of seed-specific promoters are shown in Table 2b below. Further examples of seed-specific promoters are given in Qing Qu and Takaiwa (Plant Biotechnol. J. 2, 113-125, 2004), which disclosure is incorporated by reference herein as if fully set forth.

**Table 2b:** Examples of seed-specific promoters

| **Gene source** | **Reference** |
|---|---|
| seed-specific genes | Simon et al., Plant Mol. Biol. 5: 191, 1985; |
| | Scofield et al., J. Biol. Chem. 262: 12202, 1987.; |
| | Baszczynski et al., Plant Mol. Biol. 14: 633, 1990. |
| Brazil Nut albumin | Pearson et al., Plant Mol. Biol. 1’8: 235-245, 1992. |
| Legumin | Ellis et al., Plant Mol. Biol. 10: 203-214, 1988. |
| glutelin (rice) | Takaiwa et al., Mol. Gen. Genet. 208: 15-22, 1986; |
| | Takaiwa et al., FEBS Letts. 221: 43-47, 1987. |
| Zein | Matzke et al Plant Mol Biol, 14(3):323-32 1990 |
| NapA | Stalberg et al, Planta 199: 515-519, 1996. |
| Wheat LMW and HMW glutenin-1 | Mol Gen Genet 216:81-90, 1989; NAR 17:461-2, 1989 |
| Wheat SPA | Albani et al, Plant Cell, 9: 171-184, 1997 |
| Wheat $\alpha$, $\beta$, y-gliadins | EMBO J. 3:1409-15, 1984 |
| Barley Itr1 promoter | Diaz et al. (1995) Mol Gen Genet 248(5):592-8 |
| Barley B1, C, D, hordein | Theor Appl Gen 98:1253-62, 1999; Plant J 4:343-55, 1993; Mol Gen Genet 250:750-60, 1996 |
| Barley DOF | Mena et al, The Plant Journal, 116(1): 53-62, 1998 |
| blz2 | EP99106056.7 |
| Synthetic promoter | Vicente-Carbajosa et al., Plant J. 13: 629-640, 1998. |
| rice prolamin NRP33 | Wu et al, Plant Cell Physiology 39(8) 885-889, 1998 |
| rice a-globulin Glb-1 | Wu et al, Plant Cell Physiology 39(8) 885-889, 1998 |

(continued)

| Gene source | Reference |
|---|---|
| rice OSH1 | Sato et al, Proc. Natl. Acad. Sci. USA, 93: 8117-8122, 1996 |
| rice α-globulin REB/OHP-1 | Nakase et al. Plant Mol. Biol. 33: 513-522, 1997 |
| rice ADP-glucose pyrophos-phorylase | Trans Res 6:157-68, 1997 |
| Maize ESR gene family | Plant J 12:235-46, 1997 |
| Sorghum α-kafirin | DeRose et al., Plant Mol. Biol 32:1029-35, 1996 |
| KNOX | Postma-Haarsma et al, Plant Mol. Biol. 39:257-71, 1999 |
| rice oleosin | Wu et al, J. Biochem. 123:386, 1998 |
| sunflower oleosin | Cummins et al., Plant Mol. Biol. 19: 873-876, 1992 |
| PRO0117, putative rice 40S ribosomal protein | WO 2004/070039 |
| PRO0136, rice alanine aminotransferase | Unpublished |
| PRO0147 trypsin inhibitor ITR1 | Unpublished |
| (barley) | |
| PRO0151, rice WSI18 | WO 2004/070039 |
| PRO0175, rice RAB21 | WO 2004/070039 |
| PRO005 | WO 2004/070039 |
| PRO0095 | WO 2004/070039 |
| α-amylase (Amy32b) | Lanahan et al, Plant Cell 4:203-211, 1992; Skriver et al, Proc Natl Acad Sci USA 88:7266-7270, 1991 |
| Cathepsin β-like gene | Cejudo et al, Plant Mol Biol 20:849-856, 1992 |
| Barley Ltp2 | Kalla et al., Plant J. 6:849-60, 1994 |
| Chi26 | Leah et al., Plant J. 4:579-89, 1994 |
| Maize B-Peru | Selinger et al., Genetics 149;1125-38,1998 |

A green tissue-specific promoter as defined herein is a promoter that is transcriptionally active predominantly in green tissue, substantially to the exclusion of any other parts of a plant, whilst still allowing for any leaky expression in these other plant parts. From 59392: Examples of green tissue-specific promoters are given in Nomura et al., 2000 Plant Mol Biol. 44(1):99-106; WO 2004/070039.

Another example of a tissue-specific promoter is a meristem-specific promoter, which is transcriptionally active predominantly in meristematic tissue, substantially to the exclusion of any other parts of a plant, whilst still allowing for any leaky expression in these other plant parts.

Terminator

The term "terminator" encompasses a control sequence which is a DNA sequence at the end of a transcriptional unit which signals 3' processing and polyadenylation of a primary transcript and termination of transcription. The terminator can be derived from the natural gene, from a variety of other plant genes, or from T-DNA. The terminator to be added may be derived from, for example, the nopaline synthase or octopine synthase genes, or alternatively from another plant gene, or less preferably from any other eukaryotic gene.

Modulation

The term "modulation" means in relation to expression or gene expression, a process in which the expression level is changed by said gene expression in comparison to the control plant, the expression level may be increased or decreased. The original, unmodulated expression may be of any kind of expression of a structural RNA (rRNA, tRNA) or mRNA with subsequent translation. The term "modulating the activity" shall mean any change of the expression of the inventive nucleic acid sequences or encoded proteins, which leads to increased yield and/or increased growth of the plants.

Expression

The term "expression" or "gene expression" means the transcription of a specific gene or specific genes or specific genetic construct. The term "expression" or "gene expression" in particular means the transcription of a gene or genes or genetic construct into structural RNA (rRNA, tRNA) or mRNA with or without subsequent translation of the latter into a protein. The process includes transcription of DNA and processing of the resulting mRNA product.

Increased expression/overexpression

The term "increased expression" or "overexpression" as used herein means any form of expression that is additional to the original wild-type expression level.

Methods for increasing expression of genes or gene products are well documented in the art and include, for example, overexpression driven by appropriate promoters, the use of transcription enhancers or translation enhancers. Isolated nucleic acids which serve as promoter or enhancer elements may be introduced in an appropriate position (typically upstream) of a non-heterologous form of a polynucleotide so as to upregulate expression of a nucleic acid encoding the polypeptide of interest. For example, endogenous promoters may be altered in vivo by mutation, deletion, and/or substitution (see, Kmiec, US 5,565,350; Zarling et al., WO9322443), or isolated promoters may be introduced into a plant cell in the proper orientation and distance from a gene of the present invention so as to control the expression of the gene.

If polypeptide expression is desired, it is generally desirable to include a polyadenylation region at the 3'-end of a polynucleotide coding region. The polyadenylation region can be derived from the natural gene, from a variety of other plant genes, or from T-DNA. The 3' end sequence to be added may be derived from, for example, the nopaline synthase or octopine synthase genes, or alternatively from another plant gene, or less preferably from any other eukaryotic gene.

An intron sequence may also be added to the 5' untranslated region (UTR) or the coding sequence of the partial coding sequence to increase the amount of the mature message that accumulates in the cytosol. Inclusion of a spliceable intron in the transcription unit in both plant and animal expression constructs has been shown to increase gene expression at both the mRNA and protein levels up to 1000-fold (Buchman and Berg (1988) Mol. Cell biol. 8: 4395-4405; Callis et al. (1987) Genes Dev 1:1183-1200). Such intron enhancement of gene expression is typically greatest when placed near the 5' end of the transcription unit. Use of the maize introns Adh1-S intron 1, 2, and 6, the Bronze-1 intron are known in the art. For general information see: The Maize Handbook, Chapter 116, Freeling and Walbot, Eds., Springer, N.Y. (1994).

Endogenous gene

Reference herein to an "endogenous" gene not only refers to the gene in question as found in a plant in its natural form (i.e., without there being any human intervention), but also refers to that same gene (or a substantially homologous nucleic acid/gene) in an isolated form subsequently (re)introduced into a plant (a transgene). For example, a transgenic plant containing such a transgene may encounter a substantial reduction of the transgene expression and/or substantial reduction of expression of the endogenous gene. The isolated gene may be isolated from an organism or may be manmade, for example by chemical synthesis.

Decreased expression

Reference herein to "decreased expression" or "reduction or substantial elimination" of expression is taken to mean a decrease in endogenous gene expression and/or polypeptide levels and/or polypeptide activity relative to control plants. The reduction or substantial elimination is in increasing order of preference at least 10%, 20%, 30%, 40% or 50%, 60%, 70%, 80%, 85%, 90%, or 95%, 96%, 97%, 98%, 99% or more reduced compared to that of control plants.

For optimal performance, the gene silencing techniques used for reducing expression in a plant of an endogenous gene requires the use of nucleic acid sequences from monocotyledonous plants for transformation of monocotyledonous plants, and from dicotyledonous plants for transformation of dicotyledonous plants. Preferably, a nucleic acid sequence from any given plant species is introduced into that same species. For example, a nucleic acid sequence from rice is transformed into a rice plant. However, it is not an absolute requirement that the nucleic acid sequence to be introduced originates from the same plant species as the plant in which it will be introduced. It is sufficient that there is substantial homology between the endogenous target gene and the nucleic acid to be introduced.

From 59391:

For the reduction or substantial elimination of expression an endogenous gene in a plant, a sufficient length of substantially contiguous nucleotides of a nucleic acid sequence is required. In order to perform gene silencing, this may be as little as 20, 19, 18, 17, 16, 15, 14, 13, 12, 11, 10 or fewer nucleotides, alternatively this may be as much as the entire gene (including the 5' and/or 3' UTR, either in part or in whole). The stretch of substantially contiguous nucleotides may be derived from the nucleic acid encoding the protein of interest (target gene), or from any nucleic acid capable of encoding an orthologue, paralogue or homologue of the protein of interest. Preferably, the stretch of substantially contiguous nucleotides is capable of forming hydrogen bonds with the target gene (either sense or antisense strand), more preferably, the stretch of substantially contiguous nucleotides has, in increasing order of preference, 50%, 60%, 70%, 80%, 85%, 90%, 95%, 96%, 97%, 98%, 99%, 100% sequence identity to the target gene (either sense or antisense strand). A nucleic acid sequence encoding a (functional) polypeptide is not a requirement for the various methods discussed herein for the reduction or substantial elimination of expression of an endogenous gene.

This reduction or substantial elimination of expression may be achieved using routine tools and techniques. A preferred method for the reduction or substantial elimination of endogenous gene expression is by introducing and expressing in a plant a genetic construct into which the nucleic acid (in this case a stretch of substantially contiguous nucleotides derived from the gene of interest, or from any nucleic acid capable of encoding an orthologue, paralogue or homologue of any one of the protein of interest) is cloned as an inverted repeat (in part or completely), separated by a spacer (non-coding DNA).

In such a preferred method, expression of the endogenous gene is reduced or substantially eliminated through RNA-mediated silencing using an inverted repeat of a nucleic acid or a part thereof (in this case a stretch of substantially contiguous nucleotides derived from the gene of interest, or from any nucleic acid capable of encoding an orthologue, paralogue or homologue of the protein of interest), preferably capable of forming a hairpin structure. The inverted repeat is cloned in an expression vector comprising control sequences. A non-coding DNA nucleic acid sequence (a spacer, for example a matrix attachment region fragment (MAR), an intron, a polylinker, etc.) is located between the two inverted nucleic acids forming the inverted repeat. After transcription of the inverted repeat, a chimeric RNA with a self-complementary structure is formed (partial or complete). This double-stranded RNA structure is referred to as the hairpin RNA (hpRNA). The hpRNA is processed by the plant into siRNAs that are incorporated into an RNA-induced silencing complex (RISC). The RISC further cleaves the mRNA transcripts, thereby substantially reducing the number of mRNA transcripts to be translated into polypeptides. For further general details see for example, Grierson et al. (1998) WO 98/53083; Waterhouse et al. (1999) WO 99/53050).

Performance of the methods of the invention does not rely on introducing and expressing in a plant a genetic construct into which the nucleic acid is cloned as an inverted repeat, but any one or more of several well-known "gene silencing" methods may be used to achieve the same effects.

One such method for the reduction of endogenous gene expression is RNA-mediated silencing of gene expression (downregulation). Silencing in this case is triggered in a plant by a double stranded RNA sequence (dsRNA) that is substantially similar to the target endogenous gene. This dsRNA is further processed by the plant into about 20 to about 26 nucleotides called short interfering RNAs (siRNAs). The siRNAs are incorporated into an RNA-induced silencing complex (RISC) that cleaves the mRNA transcript of the endogenous target gene, thereby substantially reducing the number of mRNA transcripts to be translated into a polypeptide. Preferably, the double stranded RNA sequence corresponds to a target gene.

Another example of an RNA silencing method involves the introduction of nucleic acid sequences or parts thereof (in this case a stretch of substantially contiguous nucleotides derived from the gene of interest, or from any nucleic acid capable of encoding an orthologue, paralogue or homologue of the protein of interest) in a sense orientation into a plant. "Sense orientation" refers to a DNA sequence that is homologous to an mRNA transcript thereof. Introduced into a plant would therefore be at least one copy of the nucleic acid sequence. The additional nucleic acid sequence will reduce expression of the endogenous gene, giving rise to a phenomenon known as co-suppression. The reduction of gene expression will be more pronounced if several additional copies of a nucleic acid sequence are introduced into the plant, as there is a positive correlation between high transcript levels and the triggering of co-suppression.

Another example of an RNA silencing method involves the use of antisense nucleic acid sequences. An "antisense" nucleic acid sequence comprises a nucleotide sequence that is complementary to a "sense" nucleic acid sequence encoding a protein, i.e. complementary to the coding strand of a double-stranded cDNA molecule or complementary to an mRNA transcript sequence. The antisense nucleic acid sequence is preferably complementary to the endogenous gene to be silenced. The complementarity may be located in the "coding region" and/or in the "non-coding region" of a gene. The term "coding region" refers to a region of the nucleotide sequence comprising codons that are translated into amino acid residues. The term "non-coding region" refers to 5' and 3' sequences that flank the coding region that are transcribed but not translated into amino acids (also referred to as 5' and 3' untranslated regions).

Antisense nucleic acid sequences can be designed according to the rules of Watson and Crick base pairing.

The antisense nucleic acid sequence may be complementary to the entire nucleic acid sequence (in this case a stretch of substantially contiguous nucleotides derived from the gene of interest, or from any nucleic acid capable of encoding an orthologue, paralogue or homologue of the protein of interest), but may also be an oligonucleotide that is antisense to only a part of the nucleic acid sequence (including the mRNA 5' and 3' UTR). For example, the antisense oligonucleotide sequence may be complementary to the region surrounding the translation start site of an mRNA transcript encoding a polypeptide. The length of a suitable antisense oligonucleotide sequence is known in the art and may start from about 50, 45, 40, 35, 30, 25, 20, 15 or 10 nucleotides in length or less. An antisense nucleic acid sequence according to the invention may be constructed using chemical synthesis and enzymatic ligation reactions using methods known in the art. For example, an antisense nucleic acid sequence (e.g., an antisense oligonucleotide sequence) may be chemically synthesized using naturally occurring nucleotides or variously modified nucleotides designed to increase the biological stability of the molecules or to increase the physical stability of the duplex formed between the antisense and sense nucleic acid sequences, e.g., phosphorothioate derivatives and acridine substituted nucleotides may be used. Examples of modified nucleotides that may be used to generate the antisense nucleic acid sequences are well known in the art. Known nucleotide modifications include methylation, cyclization and 'caps' and substitution of one or more of the naturally occurring nucleotides with an analogue such as inosine. Other modifications of nucleotides are well known in the art.

The antisense nucleic acid sequence can be produced biologically using an expression vector into which a nucleic acid sequence has been subcloned in an antisense orientation (i.e., RNA transcribed from the inserted nucleic acid will be of an antisense orientation to a target nucleic acid of interest). Preferably, production of antisense nucleic acid sequences in plants occurs by means of a stably integrated nucleic acid construct comprising a promoter, an operably linked antisense oligonucleotide, and a terminator.

The nucleic acid molecules used for silencing in the methods of the invention (whether introduced into a plant or generated in situ) hybridize with or bind to mRNA transcripts and/or genomic DNA encoding a polypeptide to thereby inhibit expression of the protein, e.g., by inhibiting transcription and/or translation. The hybridization can be by conventional nucleotide complementarity to form a stable duplex, or, for example, in the case of an antisense nucleic acid sequence which binds to DNA duplexes, through specific interactions in the major groove of the double helix Antisense nucleic acid sequences may be introduced into a plant by transformation or direct injection at a specific tissue site. Alternatively, antisense nucleic acid sequences can be modified to target selected cells and then administered systemically. For example, for systemic administration, antisense nucleic acid sequences can be modified such that they specifically bind to receptors or antigens expressed on a selected cell surface, e.g., by linking the antisense nucleic acid sequence to peptides or antibodies which bind to cell surface receptors or antigens. The antisense nucleic acid sequences can also be delivered to cells using the vectors described herein.

According to a further aspect, the antisense nucleic acid sequence is an a-anomeric nucleic acid sequence. An a-anomeric nucleic acid sequence forms specific double-stranded hybrids with complementary RNA in which, contrary to the usual b-units, the strands run parallel to each other (Gaultier et al. (1987) Nucl Ac Res 15: 6625-6641). The antisense nucleic acid sequence may also comprise a 2'-o-methylribonucleotide (Inoue et al. (1987) Nucl Ac Res 15, 6131-6148) or a chimeric RNA-DNA analogue (Inoue et al. (1987) FEBS Lett. 215, 327-330).

The reduction or substantial elimination of endogenous gene expression may also be performed using ribozymes. Ribozymes are catalytic RNA molecules with ribonuclease activity that are capable of cleaving a single-stranded nucleic acid sequence, such as an mRNA, to which they have a complementary region. Thus, ribozymes (e.g., hammerhead ribozymes (described in Haselhoff and Gerlach (1988) Nature 334, 585-591) can be used to catalytically cleave mRNA transcripts encoding a polypeptide, thereby substantially reducing the number of mRNA transcripts to be translated into a polypeptide. A ribozyme having specificity for a nucleic acid sequence can be designed (see for example: Cech et al. U.S. Patent No. 4,987,071; and Cech et al. U.S. Patent No. 5,116,742). Alternatively, mRNA transcripts corresponding to a nucleic acid sequence can be used to select a catalytic RNA having a specific ribonuclease activity from a pool of RNA molecules (Bartel and Szostak (1993) Science 261, 1411-1418). The use of ribozymes for gene silencing in plants is known in the art (e.g., Atkins et al. (1994) WO 94/00012; Lenne et al. (1995) WO 95/03404; Lutziger et al. (2000) WO 00/00619; Prinsen et al. (1997) WO 97/13865 and Scott et al. (1997) WO 97/38116).

Gene silencing may also be achieved by insertion mutagenesis (for example, T-DNA insertion or transposon insertion) or by strategies as described by, among others, Angell and Baulcombe ((1999) Plant J 20(3): 357-62), (Amplicon VIGS WO 98/36083), or Baulcombe (WO 99/15682).

Gene silencing may also occur if there is a mutation on an endogenous gene and/or a mutation on an isolated gene/nucleic acid subsequently introduced into a plant. The reduction or substantial elimination may be caused by a non-functional polypeptide. For example, a polypeptide may bind to various interacting proteins; one or more mutation (s) and/or truncation(s) may therefore provide for a polypeptide that is still able to bind interacting proteins (such as receptor proteins) but that cannot exhibit its normal function (such as signalling ligand).

A further approach to gene silencing is by targeting nucleic acid sequences complementary to the regulatory region of the gene (e.g., the promoter and/or enhancers) to form triple helical structures that prevent transcription of the gene in target cells. See Helene, C., Anticancer Drug Res. 6, 569-84, 1991; Helene et al., Ann. N.Y. Acad. Sci. 660, 27-36 1992; and Maher, L.J. Bioassays 14, 807-15, 1992.

Other methods, such as the use of antibodies directed to an endogenous polypeptide for inhibiting its function in planta, or interference in the signalling pathway in which a polypeptide is involved, will be well known to the skilled man. In particular, it can be envisaged that manmade molecules may be useful for inhibiting the biological function of a target polypeptide, or for interfering with the signalling pathway in which the target polypeptide is involved.

Alternatively, a screening program may be set up to identify in a plant population natural variants of a gene, which variants encode polypeptides with reduced activity. Such natural variants may also be used for example, to perform homologous recombination.

Artificial and/or natural microRNAs (miRNAs) may be used to knock out gene expression and/or mRNA translation. Endogenous miRNAs are single stranded small RNAs of typically 19-24 nucleotides long. They function primarily to regulate gene expression and/ or mRNA translation. Most plant microRNAs (miRNAs) have perfect or near-perfect complementarity with their target sequences. However, there are natural targets with up to five mismatches. They are processed from longer non-coding RNAs with characteristic fold-back structures by double-strand specific RNases of the Dicer family. Upon processing, they are incorporated in the RNA-induced silencing complex (RISC) by binding to its main component, an Argonaute protein. MiRNAs serve as the specificity components of RISC, since they base-pair to target nucleic acids, mostly mRNAs, in the cytoplasm. Subsequent regulatory events include target mRNA cleavage and destruction and/or translational inhibition. Effects of miRNA overexpression are thus often reflected in decreased mRNA levels of target genes.

Artificial microRNAs (amiRNAs), which are typically 21 nucleotides in length, can be genetically engineered specifically to negatively regulate gene expression of single or multiple genes of interest. Determinants of plant microRNA target selection are well known in the art. Empirical parameters for target recognition have been defined and can be used to aid in the design of specific amiRNAs, (Schwab et al., Dev. Cell 8, 517-527, 2005). Convenient tools for design and generation of amiRNAs and their precursors are also available to the public (Schwab et al., Plant Cell 18, 1121-1133, 2006).

For optimal performance, the gene silencing techniques used for reducing expression in a plant of an endogenous gene requires the use of nucleic acid sequences from monocotyledonous plants for transformation of monocotyledonous plants, and from dicotyledonous plants for transformation of dicotyledonous plants. Preferably, a nucleic acid sequence from any given plant species is introduced into that same species. For example, a nucleic acid sequence from rice is transformed into a rice plant. However, it is not an absolute requirement that the nucleic acid sequence to be introduced originates from the same plant species as the plant in which it will be introduced. It is sufficient that there is substantial homology between the endogenous target gene and the nucleic acid to be introduced.

Described above are examples of various methods for the reduction or substantial elimination of expression in a plant of an endogenous gene. A person skilled in the art would readily be able to adapt the aforementioned methods for silencing so as to achieve reduction of expression of an endogenous gene in a whole plant or in parts thereof through the use of an appropriate promoter, for example.

A person skilled in the art is familiar with the various techniques for decreasing expression of a gene.

Selectable marker (gene)/Reporter gene

"Selectable marker", "selectable marker gene" or "reporter gene" includes any gene that confers a phenotype on a cell in which it is expressed to facilitate the identification and/or selection of cells that are transfected or transformed with a nucleic acid construct of the invention. These marker genes enable the identification of a successful transfer of the nucleic acid molecules via a series of different principles. Suitable markers may be selected from markers that confer antibiotic or herbicide resistance, that introduce a new metabolic trait or that allow visual selection. Examples of selectable marker genes include genes conferring resistance to antibiotics (such as nptII that phosphorylates neomycin and kanamycin, or hpt, phosphorylating hygromycin, or genes conferring resistance to, for example, bleomycin, streptomycin, tetracyclin, chloramphenicol, ampicillin, gentamycin, geneticin (G418), spectinomycin or blasticidin), to herbicides (for example bar which provides resistance to Basta®; aroA or gox providing resistance against glyphosate, or the genes conferring resistance to, for example, imidazolinone, phosphinothricin or sulfonylurea), or genes that provide a metabolic trait (such as manA that allows plants to use mannose as sole carbon source or xylose isomerase for the utilisation of xylose, or antinutritive markers such as the resistance to 2-deoxyglucose). Expression of visual marker genes results in the formation of colour (for example β-glucuronidase, GUS or β-galactosidase with its coloured substrates, for example X-Gal), luminescence (such as the luciferin/luceferase system) or fluorescence (Green Fluorescent Protein, GFP, and derivatives thereof). This list represents only a small number of possible markers. The skilled worker is familiar with such markers. Different markers are preferred, depending on the organism and the selection method.

It is known that upon stable or transient integration of nucleic acids into plant cells, only a minority of the cells takes up the foreign DNA and, if desired, integrates it into its genome, depending on the expression vector used and the transfection technique used. To identify and select these integrants, a gene coding for a selectable marker (such as the ones described above) is usually introduced into the host cells together with the gene of interest. These markers can for example be used in mutants in which these genes are not functional by, for example, deletion by conventional methods. Furthermore, nucleic acid molecules encoding a selectable marker can be introduced into a host cell on the same vector that comprises the sequence encoding the polypeptides of the invention or used in the methods of the invention, or else in a separate vector. Cells which have been stably transfected with the introduced nucleic acid can be identified for example by selection (for example, cells which have integrated the selectable marker survive whereas the other cells die).

Since the marker genes, particularly genes for resistance to antibiotics and herbicides, are no longer required or are undesired in the transgenic host cell once the nucleic acids have been introduced successfully, the process according to the invention for introducing the nucleic acids advantageously employs techniques which enable the removal or excision of these marker genes. One such a method is what is known as co-transformation. The co-transformation method employs two vectors simultaneously for the transformation, one vector bearing the nucleic acid according to the invention and a second bearing the marker gene(s). A large proportion of transformants receives or, in the case of plants, comprises (up to 40% or more of the transformants), both vectors. In case of transformation with Agrobacteria, the transformants usually receive only a part of the vector, i.e. the sequence flanked by the T-DNA, which usually represents the expression cassette. The marker genes can subsequently be removed from the transformed plant by performing crosses. In another method, marker genes integrated into a transposon are used for the transformation together with desired nucleic acid (known as the Ac/Ds technology). The transformants can be crossed with a transposase source or the transformants are transformed with a nucleic acid construct conferring expression of a transposase, transiently or stable. In some cases (approx. 10%), the transposon jumps out of the genome of the host cell once transformation has taken place successfully and is lost. In a further number of cases, the transposon jumps to a different location. In these cases the marker gene must be eliminated by performing crosses. In microbiology, techniques were developed which make possible, or facilitate, the detection of such events. A further advantageous method relies on what is known as recombination systems; whose advantage is that elimination by crossing can be dispensed with. The best-known system of this type is what is known as the Cre/lox system. Cre1 is a recombinase that removes the sequences located between the loxP sequences. If the marker gene is integrated between the loxP sequences, it is removed once transformation has taken place successfully, by expression of the recombinase. Further recombination systems are the HIN/HIX, FLP/FRT and REP/STB system (Tribble et al., J. Biol. Chem., 275, 2000: 22255-22267; Velmurugan et al., J. Cell Biol., 149, 2000: 553-566). A site-specific integration into the plant genome of the nucleic acid sequences according to the invention is possible. Naturally, these methods can also be applied to microorganisms such as yeast, fungi or bacteria.

Transgenic/Transgene/Recombinant

For the purposes of the invention, "transgenic", "transgene" or "recombinant" means with regard to, for example, a nucleic acid sequence, an expression cassette, gene construct or a vector comprising the nucleic acid sequence or an organism transformed with the nucleic acid sequences, expression cassettes or vectors according to the invention, all those constructions brought about by recombinant methods in which either

(a) the nucleic acid sequences encoding proteins useful in the methods of the invention, or
(b) genetic control sequence(s) which is operably linked with the nucleic acid sequence according to the invention, for example a promoter, or
(c) a) and b)

are not located in their natural genetic environment or have been modified by recombinant methods, it being possible for the modification to take the form of, for example, a substitution, addition, deletion, inversion or insertion of one or more nucleotide residues. The natural genetic environment is understood as meaning the natural genomic or chromosomal locus in the original plant or the presence in a genomic library. In the case of a genomic library, the natural genetic environment of the nucleic acid sequence is preferably retained, at least in part. The environment flanks the nucleic acid sequence at least on one side and has a sequence length of at least 50 bp, preferably at least 500 bp, especially preferably at least 1000 bp, most preferably at least 5000 bp. A naturally occurring expression cassette - for example the naturally occurring combination of the natural promoter of the nucleic acid sequences with the corresponding nucleic acid sequence encoding a polypeptide useful in the methods of the present invention, as defined above - becomes a transgenic expression cassette when this expression cassette is modified by non-natural, synthetic ("artificial") methods such as, for example, mutagenic treatment. Suitable methods are described, for example, in US 5,565,350 or WO 00/15815.

A transgenic plant for the purposes of the invention is thus understood as meaning, as above, that the nucleic acids used in the method of the invention are not at their natural locus in the genome of said plant, it being possible for the nucleic acids to be expressed homologously or heterologously. However, as mentioned, transgenic also means that, while the nucleic acids according to the invention or used in the inventive method are at their natural position in the genome of a plant, the sequence has been modified with regard to the natural sequence, and/or that the regulatory sequences of the natural sequences have been modified. Transgenic is preferably understood as meaning the expression of the nucleic acids according to the invention at an unnatural locus in the genome, i.e. homologous or, preferably, heterologous expression of the nucleic acids takes place. Preferred transgenic plants are mentioned herein.

Transformation

The term "introduction" or "transformation" as referred to herein encompasses the transfer of an exogenous polynucleotide into a host cell, irrespective of the method used for transfer. Plant tissue capable of subsequent clonal propagation, whether by organogenesis or embryogenesis, may be transformed with a genetic construct of the present invention and a whole plant regenerated there from. The particular tissue chosen will vary depending on the clonal propagation systems available for, and best suited to, the particular species being transformed. Exemplary tissue targets include leaf disks, pollen, embryos, cotyledons, hypocotyls, megagametophytes, callus tissue, existing meristematic tissue (e.g., apical meristem, axillary buds, and root meristems), and induced meristem tissue (e.g., cotyledon meristem and hypocotyl meristem). The polynucleotide may be transiently or stably introduced into a host cell and may be maintained non-integrated, for example, as a plasmid.

Alternatively, it may be integrated into the host genome. The resulting transformed plant cell may then be used to regenerate a transformed plant in a manner known to persons skilled in the art.

The transfer of foreign genes into the genome of a plant is called transformation. Transformation of plant species is now a fairly routine technique. Advantageously, any of several transformation methods may be used to introduce the gene of interest into a suitable ancestor cell. The methods described for the transformation and regeneration of plants from plant tissues or plant cells may be utilized for transient or for stable transformation. Transformation methods include the use of liposomes, electroporation, chemicals that increase free DNA uptake, injection of the DNA directly into the plant, particle gun bombardment, transformation using viruses or pollen and microprojection. Methods may be selected from the calcium/polyethylene glycol method for protoplasts (Krens, F.A. et al., (1982) Nature 296, 72-74; Negrutiu I et al. (1987) Plant Mol Biol 8: 363-373); electroporation of protoplasts (Shillito R.D. et al. (1985) Bio/Technol 3, 1099-1102); microinjection into plant material (Crossway A et al., (1986) Mol. Gen Genet 202: 179-185); DNA or RNA-coated particle bombardment (Klein TM et al., (1987) Nature 327: 70) infection with (non-integrative) viruses and the like. Transgenic plants, including transgenic crop plants, are preferably produced via Agrobacterium-mediated transformation. An advantageous transformation method is the transformation *in planta*. To this end, it is possible, for example, to allow the agrobacteria to act on plant seeds or to inoculate the plant meristem with agrobacteria. It has proved particularly expedient in accordance with the invention to allow a suspension of transformed agrobacteria to act on the intact plant or at least on the flower primordia. The plant is subsequently grown on until the seeds of the treated plant are obtained (Clough and Bent, Plant J. (1998) 16, 735-743). Methods for Agrobacterium-mediated transformation of rice include well known methods for rice transformation, such as those described in any of the following: European patent application EP 1198985 A1, Aldemita and Hodges (Planta 199: 612-617, 1996); Chan et al. (Plant Mol Biol 22 (3): 491-506, 1993), Hiei et al. (Plant J 6 (2): 271-282, 1994), which disclosures are incorporated by reference herein as if fully set forth. In the case of corn transformation, the preferred method is as described in either Ishida et al. (Nat. Biotechnol 14(6): 745-50, 1996) or Frame et al. (Plant Physiol 129(1): 13-22, 2002), which disclosures are incorporated by reference herein as if fully set forth. Said methods are further described by way of example in B. Jenes et al., Techniques for Gene Transfer, in: Transgenic Plants, Vol. 1, Engineering and Utilization, eds. S.D. Kung and R. Wu, Academic Press (1993) 128-143 and in Potrykus Annu. Rev. Plant Physiol. Plant Molec. Biol. 42 (1991) 205-225). The nucleic acids or the construct to be expressed is preferably cloned into a vector, which is suitable for transforming *Agrobacterium tumefaciens,* for example pBin19 (Bevan et al., Nucl. Acids Res. 12 (1984) 8711). Agrobacteria transformed by such a vector can then be used in known manner for the transformation of plants, such as plants used as a model, like *Arabidopsis (Arabidopsis thaliana* is within the scope of the present invention not considered as a crop plant), or crop plants such as, by way of example, tobacco plants, for example by immersing bruised leaves or chopped leaves in an agrobacterial solution and then culturing them in suitable media. The transformation of plants by means of *Agrobacterium tumefaciens* is described, for example, by Höfgen and Willmitzer in Nucl. Acid Res. (1988) 16, 9877 or is known inter alia from F.F. White, Vectors for Gene Transfer in Higher Plants; in Transgenic Plants, Vol. 1, Engineering and Utilization, eds. S.D. Kung and R. Wu, Academic Press, 1993, pp. 15-38.

In addition to the transformation of somatic cells, which then have to be regenerated into intact plants, it is also possible to transform the cells of plant meristems and in particular those cells which develop into gametes. In this case, the transformed gametes follow the natural plant development, giving rise to transgenic plants. Thus, for example, seeds of *Arabidopsis* are treated with agrobacteria and seeds are obtained from the developing plants of which a certain proportion is transformed and thus transgenic [Feldman, KA and Marks MD (1987). Mol Gen Genet 208:274-289; Feldmann K (1992). In: C Koncz, N-H Chua and J Shell, eds, Methods in Arabidopsis Research. Word Scientific, Singapore, pp. 274-289]. Alternative methods are based on the repeated removal of the inflorescences and incubation of the excision site in the center of the rosette with transformed agrobacteria, whereby transformed seeds can likewise be obtained at a later point in time (Chang (1994). Plant J. 5: 551-558; Katavic (1994). Mol Gen Genet, 245: 363-370). However, an especially effective method is the vacuum infiltration method with its modifications such as the "floral dip" method. In the case of vacuum infiltration of *Arabidopsis,* intact plants under reduced pressure are treated with an agrobacterial suspension [Bechthold, N (1993). C R Acad Sci Paris Life Sci, 316: 1194-1199], while in the case of the "floral dip" method the developing floral tissue is incubated briefly with a surfactant-treated agrobacterial suspension [Clough, SJ and Bent AF (1998) The Plant J. 16, 735-743]. A certain proportion of transgenic seeds are harvested in both cases, and these seeds can be distinguished from non-transgenic seeds by growing under the above-described selective conditions. In addition the stable transformation of plastids is of advantages because plastids are inherited maternally is most crops reducing or eliminating the risk of transgene flow through pollen. The transformation of the chloroplast genome is generally achieved by a process which has been schematically displayed in Klaus et al., 2004 [Nature Biotechnology 22 (2), 225-229]. Briefly the sequences to be transformed are cloned together with a selectable marker gene between flanking sequences homologous to the chloroplast genome. These homologous flanking sequences direct site specific integration into the plastome. Plastidal transformation has been described for many different plant species and an overview is given in Bock (2001) Transgenic plastids in basic research and plant biotechnology. J Mol Biol. 2001 Sep 21; 312 (3):425-38 or Maliga, P (2003) Progress towards commercialization of plastid transformation technology. Trends Biotechnol. 21, 20-28. Further biotechnological progress has recently been reported in form of marker free plastid transformants, which can be produced by a transient co-integrated maker gene (Klaus et al., 2004, Nature Biotechnology 22(2), 225-229).

T-DNA activation tagging

T-DNA activation tagging (Hayashi et al. Science (1992) 1350-1353), involves insertion of T-DNA, usually containing a promoter (may also be a translation enhancer or an intron), in the genomic region of the gene of interest or 10 kb up- or downstream of the coding region of a gene in a configuration such that the promoter directs expression of the targeted gene. Typically, regulation of expression of the targeted gene by its natural promoter is disrupted and the gene falls under the control of the newly introduced promoter. The promoter is typically embedded in a T-DNA. This T-DNA is randomly inserted into the plant genome, for example, through *Agrobacterium* infection and leads to modified expression of genes near the inserted T-DNA. The resulting transgenic plants show dominant phenotypes due to modified expression of genes close to the introduced promoter.

TILLING

The term "TILLING" is an abbreviation of "Targeted Induced Local Lesions In Genomes" and refers to a mutagenesis technology useful to generate and/or identify nucleic acids encoding proteins with modified expression and/or activity. TILLING also allows selection of plants carrying such mutant variants. These mutant variants may exhibit modified expression, either in strength or in location or in timing (if the mutations affect the promoter for example). These mutant variants may exhibit higher activity than that exhibited by the gene in its natural form. TILLING combines high-density mutagenesis with high-throughput screening methods. The steps typically followed in TILLING are: (a) EMS mutagenesis (Redei GP and Koncz C (1992) In Methods in Arabidopsis Research, Koncz C, Chua NH, Schell J, eds. Singapore, World Scientific Publishing Co, pp. 16-82; Feldmann et al., (1994) In Meyerowitz EM, Somerville CR, eds, Arabidopsis. Cold Spring Harbor Laboratory Press, Cold Spring Harbor, NY, pp 137-172; Lightner J and Caspar T (1998) In J Martinez-Zapater, J Salinas, eds, Methods on Molecular Biology, Vol. 82. Humana Press, Totowa, NJ, pp 91-104); (b) DNA preparation and pooling of individuals; (c) PCR amplification of a region of interest; (d) denaturation and annealing to allow formation of heteroduplexes; (e) DHPLC, where the presence of a heteroduplex in a pool is detected as an extra peak in the chromatogram; (f) identification of the mutant individual; and (g) sequencing of the mutant PCR product. Methods for TILLING are well known in the art (McCallum et al., (2000) Nat Biotechnol 18: 455-457; reviewed by Stemple (2004) Nat Rev Genet 5(2): 145-50).

Homologous recombination

Homologous recombination allows introduction in a genome of a selected nucleic acid at a defined selected position. Homologous recombination is a standard technology used routinely in biological sciences for lower organisms such as yeast or the moss *Physcomitrella.* Methods for performing homologous recombination in plants have been described not only for model plants (Offringa et al. (1990) EMBO J 9(10): 3077-84) but also for crop plants, for example rice (Terada et al. (2002) Nat Biotech 20(10): 1030-4; Iida and Terada (2004) Curr Opin Biotech 15(2): 132-8), and approaches exist that are generally applicable regardless of the target organism (Miller et al, Nature Biotechnol. 25, 778-785, 2007).

Yield

The term "yield" in general means a measurable produce of economic value, typically related to a specified crop, to an area, and to a period of time. Individual plant parts directly contribute to yield based on their number, size and/or weight, or the actual yield is the yield per square meter for a crop and year, which is determined by dividing total production (includes both harvested and appraised production) by planted square meters. The term "yield" of a plant may relate to vegetative biomass (root and/or shoot biomass), to reproductive organs, and/or to propagules (such as seeds) of that plant.

Early vigour

"Early vigour" refers to active healthy well-balanced growth especially during early stages of plant growth, and may result from increased plant fitness due to, for example, the plants being better adapted to their environment (i.e. optimizing the use of energy resources and partitioning between shoot and root). Plants having early vigour also show increased seedling survival and a better establishment of the crop, which often results in highly uniform fields (with the crop growing in uniform manner, i.e. with the majority of plants reaching the various stages of development at substantially the same time), and often better and higher yield. Therefore, early vigour may be determined by measuring various factors, such as thousand kernel weight, percentage germination, percentage emergence, seedling growth, seedling height, root length, root and shoot biomass and many more.

Increase/Improve/Enhance

The terms "increase", "improve" or "enhance" are interchangeable and shall mean in the sense of the application at least a 3%, 4%, 5%, 6%, 7%, 8%, 9% or 10%, preferably at least 15% or 20%, more preferably 25%, 30%, 35% or 40% more yield and/or growth in comparison to control plants as defined herein.

Seed yield

Increased seed yield may manifest itself as one or more of the following: a) an increase in seed biomass (total seed weight) which may be on an individual seed basis and/or per plant and/or per square meter; b) increased number of flowers per plant; c) increased number of (filled) seeds; d) increased seed filling rate (which is expressed as the ratio between the number of filled seeds divided by the total number of seeds); e) increased harvest index, which is expressed as a ratio of the yield of harvestable parts, such as seeds, divided by the total biomass; and f) increased thousand kernel weight (TKW), which is extrapolated from the number of filled seeds counted and their total weight. An increased TKW may result from an increased seed size and/or seed weight, and may also result from an increase in embryo and/or endosperm size.

An increase in seed yield may also be manifested as an increase in seed size and/or seed volume. Furthermore, an increase in seed yield may also manifest itself as an increase in seed area and/or seed length and/or seed width and/or seed perimeter. Increased yield may also result in modified architecture, or may occur because of modified architecture.

Greenness Index

The "greenness index" as used herein is calculated from digital images of plants. For each pixel belonging to the plant object on the image, the ratio of the green value versus the red value (in the RGB model for encoding color) is calculated. The greenness index is expressed as the percentage of pixels for which the green-to-red ratio exceeds a given threshold. Under normal growth conditions, under salt stress growth conditions, and under reduced nutrient availability growth conditions, the greenness index of plants is measured in the last imaging before flowering. In contrast, under drought stress growth conditions, the greenness index of plants is measured in the first imaging after drought.

Plant

The term "plant" as used herein encompasses whole plants, ancestors and progeny of the plants and plant parts, including seeds, shoots, stems, leaves, roots (including tubers), flowers, and tissues and organs, wherein each of the aforementioned comprise the gene/nucleic acid of interest. The term "plant" also encompasses plant cells, suspension cultures, callus tissue, embryos, meristematic regions, gametophytes, sporophytes, pollen and microspores, again wherein each of the aforementioned comprises the gene/nucleic acid of interest.

Plants that are particularly useful in the methods of the invention include all plants which belong to the superfamily Viridiplantae, in particular monocotyledonous and dicotyledonous plants including fodder or forage legumes, ornamental plants, food crops, trees or shrubs selected from the list comprising Acer spp., *Actinidia* spp., *Abelmoschus* spp., *Agave sisalana, Agropyron* spp., *Agrostis stolonifera, Allium* spp., *Amaranthus* spp., *Ammophila arenaria, Ananas comosus, Annona* spp., *Apium graveolens, Arachis spp, Artocarpus* spp., *Asparagus officinalis, Avena* spp. *(e.g. Avena sativa, Avena fatua, Avena byzantina, Avena fatua var. sativa, Avena hybrida), Averrhoa carambola, Bambusa sp., Benincasa hispida, Bertholletia excelsea, Beta vulgaris, Brassica* spp. *(e.g. Brassica napus, Brassica rapa* ssp. [canola, oilseed rape, turnip rape]), *Cadaba farinosa, Camellia sinensis, Canna indica, Cannabis sativa, Capsicum* spp., *Carex elata, Carica papaya, Carissa macrocarpa, Carya* spp., *Carthamus tinctorius, Castanea* spp., *Ceiba pentandra, Cichorium endivia, Cinnamomum* spp., *Citrullus lanatus, Citrus* spp., *Cocos* spp., *Coffea* spp., *Colocasia esculenta, Cola* spp., *Corchorus sp., Coriandrum sativum, Corylus* spp., *Crataegus* spp., *Crocus sativus, Cucurbita* spp., *Cucumis* spp., *Cynara* spp., *Daucus carota, Desmodium* spp., *Dimocarpus longan, Dioscorea* spp., *Diospyros* spp., *Echinochloa* spp., *Elaeis (e.g. Elaeis guineensis, Elaeis oleifera), Eleusine coracana, Eragrostis tef, Erianthus sp., Eriobotrya japonica, Eucalyptus sp., Eugenia uniflora, Fagopyrum* spp., *Fagus* spp., *Festuca arundinacea, Ficus carica, Fortunella* spp., *Fragaria* spp., *Ginkgo biloba, Glycine* spp. *(e.g. Glycine max, Soja hispida or Soja max), Gossypium hirsutum, Helianthus* spp. *(e.g. Helianthus annuus), Hemerocallis fulva, Hibiscus* spp., *Hordeum* spp. *(e.g. Hordeum vulgare), Ipomoea batatas, Juglans* spp., *Lactuca sativa, Lathyrus* spp., *Lens culinaris, Linum usitatissimum, Litchi chinensis, Lotus* spp., *Luffa acutangula, Lupinus* spp., *Luzula sylvatica, Lycopersicon* spp. *(e.g. Lycopersicon esculentum, Lycopersicon lycopersicum, Lycopersicon pyriforme), Macrotyloma* spp., *Malus* spp., *Malpighia emarginata, Mammea americana, Mangifera indica, Manihot* spp., *Manilkara zapota, Medicago sativa, Melilotus* spp., *Mentha* spp., *Miscanthus sinensis, Momordica* spp., *Morus nigra, Musa* spp., *Nicotiana* spp., Olea spp., *Opuntia* spp., *Ornithopus* spp., *Oryza* spp. *(e.g. Oryza sativa, Oryza latifolia), Panicum miliaceum, Panicum virgatum, Passiflora edulis, Pastinaca sativa, Pennisetum sp., Persea* spp., *Petroselinum crispum, Phalaris arundinacea, Phaseolus* spp., *Phleum pratense, Phoenix* spp., *Phragmites australis, Physalis* spp., *Pinus* spp., *Pistacia vera, Pisum* spp., *Poa* spp., *Populus* spp., *Prosopis* spp., *Prunus* spp., *Psidium* spp., *Punica granatum, Pyrus communis, Quercus* spp., *Raphanus sativus, Rheum rhabarbarum, Ribes* spp., *Ricinus communis, Rubus* spp., *Saccharum* spp., *Salix sp., Sambucus* spp., *Secale cereale, Sesamum* spp., *Sinapis sp., Solanum* spp. *(e.g. Solanum tuberosum, Solanum integrifolium or Solanum lycopersicum), Sorghum bicolor, Spinacia* spp., *Syzygium* spp., *Tagetes* spp., *Tamarindus indica, Theobroma cacao, Trifolium* spp., *Tripsacum dactyloides, Triticosecale rimpaui, Triticum* spp. *(e.g. Triticum aestivum, Triticum durum, Triticum turgidum, Triticum hybernum, Triticum macha, Triticum sativum, Triticum monococcum or Triticum vulgare), Tropaeolum minus, Tropaeolum majus, Vaccinium* spp., *Vicia* spp., *Vigna* spp., *Viola odorata, Vitis* spp., Zea *mays, Zizania palustris, Ziziphus* spp., amongst others.

**Detailed description of the invention**

Surprisingly, it has now been found that modulating expression in a plant of a nucleic acid encoding a bHLH6-like polypeptide gives plants having enhanced yield-related traits relative to control plants. According to a first embodiment, the present invention provides a method for enhancing yield-related traits in plants relative to control plants, comprising modulating expression in a plant of a nucleic acid encoding a bHLH6-like polypeptide.

A preferred method for modulating (preferably, increasing) expression of a nucleic acid encoding a bHLH6-like polypeptide is by introducing and expressing in a plant a nucleic acid encoding a bHLH6-like polypeptide.

In one embodiment any reference hereinafter to a "protein useful in the methods of the invention" is taken to mean a bHLH6-like polypeptide as defined herein. In the same embodiment any reference hereinafter to a "nucleic acid useful in the methods of the invention" is taken to mean a nucleic acid capable of encoding such a bHLH6-like polypeptide. In one embodiment the nucleic acid to be introduced into a plant (and therefore useful in performing the methods of the invention) is any nucleic acid encoding the type of protein which will now be described, hereafter also named *"bHLH6-like* nucleic acid" or *"bHLH6-like* gene".

A "bHLH6-like polypeptide" as defined herein refers to any polypeptide comprising a basic domain followed by a HLH domain (HMMPFam PF00010, ProfileScan PS50888, SMART SM00353) thereby forming a basic helix-loop-helix domain (Interpro IPR001092). Preferably, the bHLH6-like polypeptide comprises at least one, preferably two, more preferably three or more of the following motifs:

Motif 1 (SEQ ID NO: 3): L(G/E/T)WX(D/E)(G/S) X (Y/F) (K/N) G (E/D)
wherein X in position 4 can be any amino acid, preferably a turnlike amino acid, more preferably one of G, R, K, T, and S; and wherein X in position 7 can be any amino acid, preferably a hydrophobic or polar amino acid, more preferably one of Y, F, N, and H.

Motif 2 (SEQ ID NO: 4):

G(V/I)(V/I/L)E(V/L/I)(G/A)(S/V/T/A)(T/L/S)(E/D/S)

Motif 3 (SEQ ID NO: 5): (D/E) K(A/V/I) S(L/I/V) L (G/D/E/A)
Motif 4 (SEQ ID NO: 6): (T/N/S) LQ (Q/H) RLQ
Motif 5 (SEQ ID NO: 7):

(K/F)(I/F/V)(I/L/V/M/S)G(W/L/R/N/E)(E/D)AM(I/V)(G/R)(V/I)(Q/N/E/Y)

Motif 6 (SEQ ID NO: 8):

(H/Y)(A/S)(S/N)(V/C/M/L/T)(S/Q)(V/C/S)(V/MF)(K/N/S)(D/C/E)(L/Q/F/M)(M/R/L)(I/F/L)(Q/D/H)(Q/D/V)

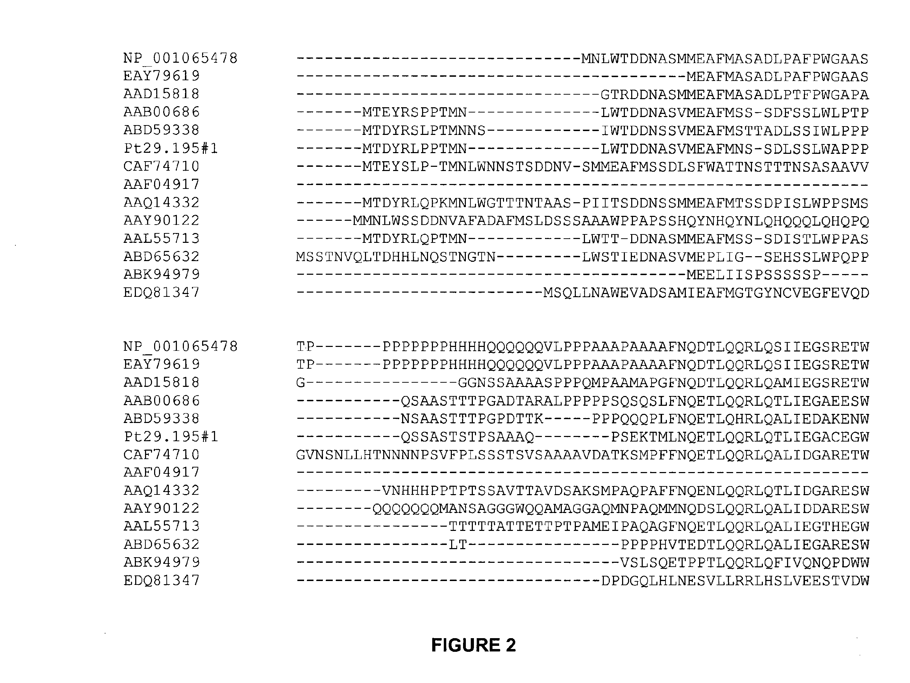

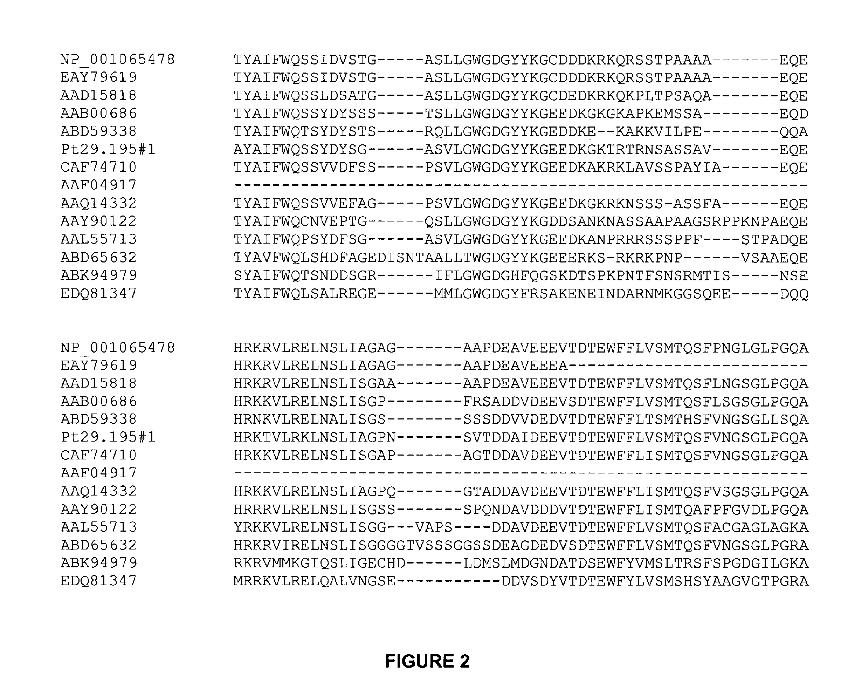

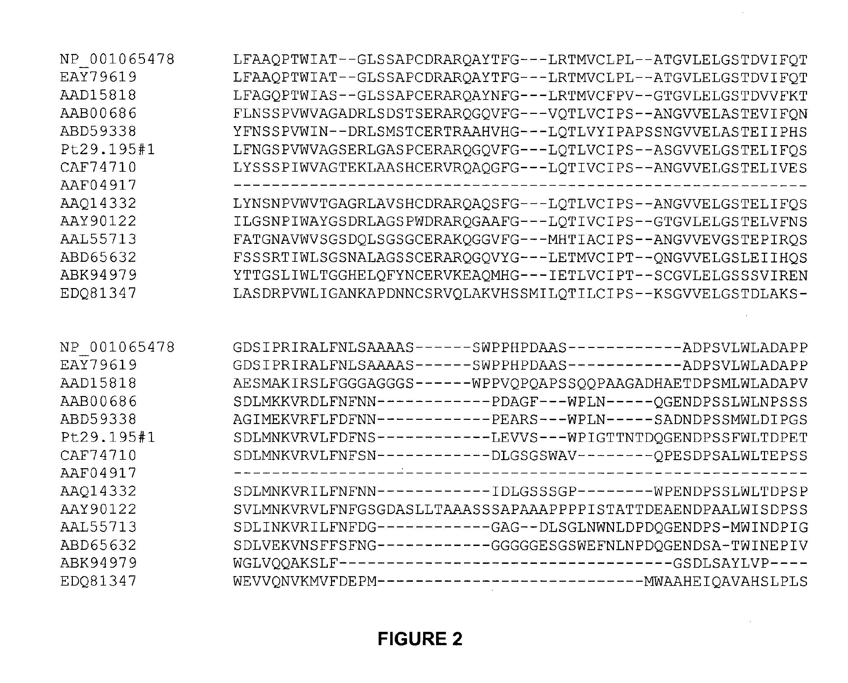

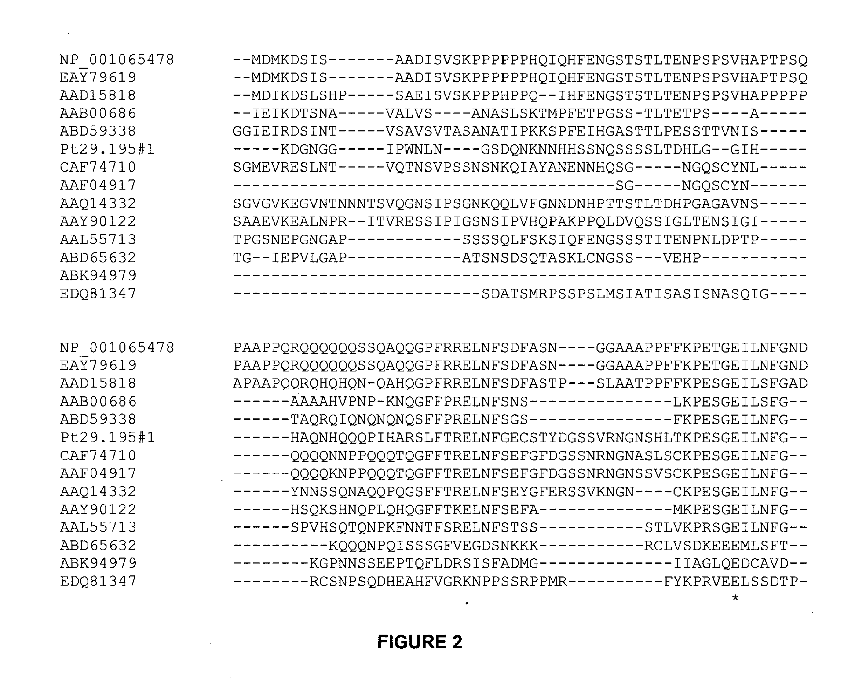

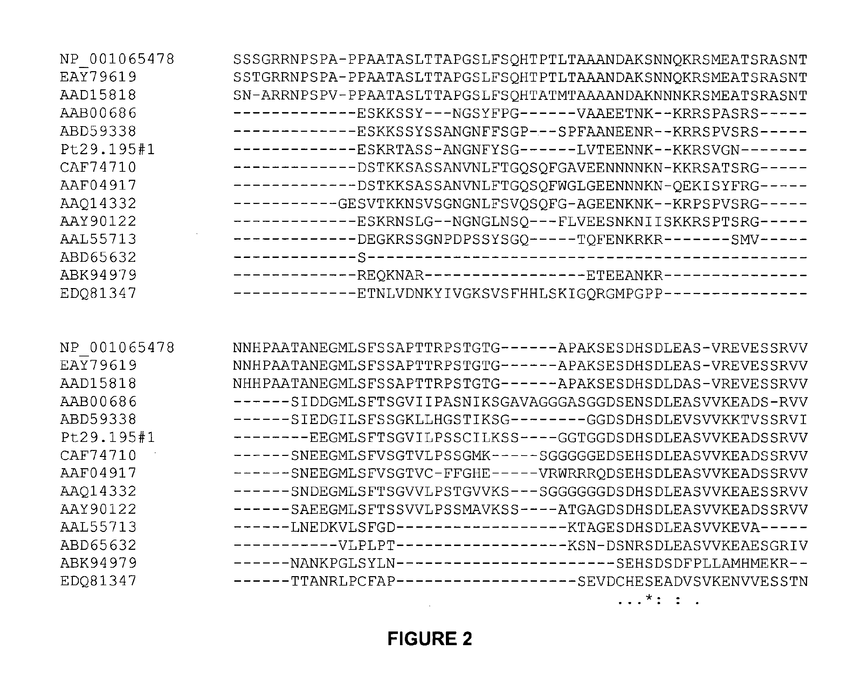

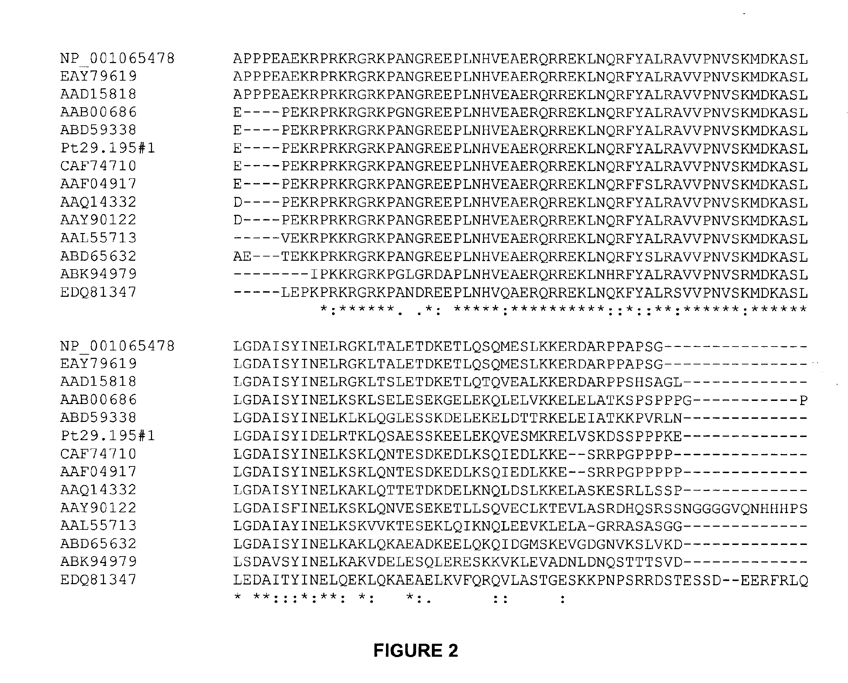

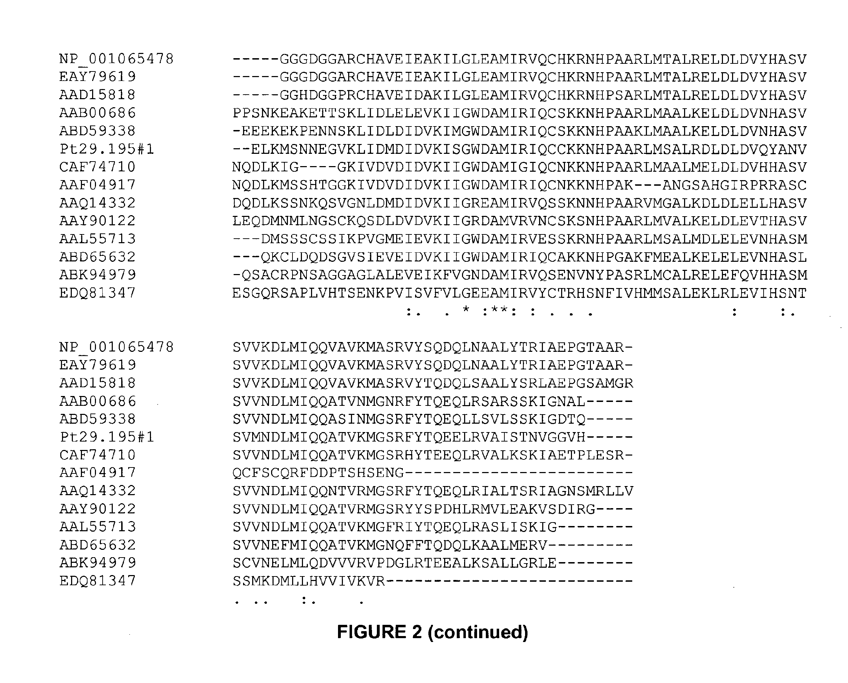

Alternatively, the homologue of a bHLH6-like protein has in increasing order of preference at least 25%, 26%, 27%, 28%, 29%, 30%, 31%, 32%, 33%, 34%, 35%, 36%, 37%, 38%, 39%, 40%, 41 %, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% overall sequence identity to the amino acid represented by SEQ ID NO: 2, provided that the homologous protein comprises the conserved motifs as outlined above. The overall sequence identity is determined using a global alignment algorithm, such as the Needleman Wunsch algorithm in the program GAP (GCG Wisconsin Package, Accelrys), preferably with default parameters. Compared to overall sequence identity, the sequence identity will generally be higher when only conserved domains or motifs are considered. The sequence conservation is much higher in the region of the bHLH domain (see Table B2 in Example 3 and Figure 2). Therefore the bHLH domain is a good criterion for the defining the group of bHLH6-like proteins. Preferably, the bHLH6-like polypeptide comprises the sequence of Motif 7 (SEQ ID NO: 9): PKKRGRKPAN GREEPLNHVEAERQRREKLNQRFYALRAWPNVSKMDKASLLGDAIAYINELKSKWKTE or a sequence that has, in increasing order of preference, at least 80%, 81%, 82%, 83%, 84%, 85%, 86%, 87%, 88%, 89%, 90%, 91%, 92%, 93%, 94%, 95%, 96%, 97%, 98%, or 99% sequence identity to SEQ ID NO: 9. The HLH domain as determined by SMART spans residue 454 to 503 in SEQ ID NO: 2 and is comprised in Motif 7.

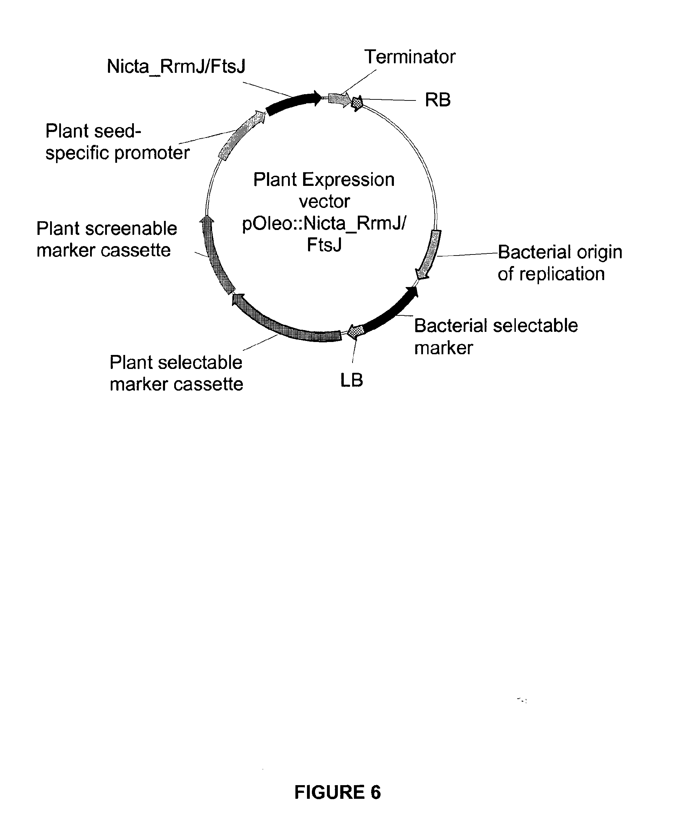

Preferably, the polypeptide sequence which when used in the construction of a phylogenetic tree, such as the one depicted in Figure 6 in Li et al. (2006), clusters within subgroup N (which comprises the amino acid sequence represented by SEQ ID NO: 2), rather than with any other group.

Preferably, the genomic DNA sequence encoding the bHLH6-like polypeptide does not comprise introns.

The terms "domain", "signature" and "motif" are defined in the "definitions" section herein. Specialist databases exist for the identification of domains, for example, SMART (Schultz et al. (1998) Proc. Natl. Acad. Sci. USA 95, 5857-5864; Letunic et al. (2002) Nucleic Acids Res 30, 242-244), InterPro (Mulder et al., (2003) Nucl. Acids. Res. 31, 315-318), Prosite (Bucher and Bairoch (1994), A generalized profile syntax for biomolecular sequences motifs and its function in automatic sequence interpretation. (In) ISMB-94; Proceedings 2nd International Conference on Intelligent Systems for Molecular Biology. Altman R., Brutlag D., Karp P., Lathrop R., Searls D., Eds., pp53-61, AAAI Press, Menlo Park; Hulo et al., Nucl. Acids. Res. 32:D134-D137, (2004)), or Pfam (Bateman et al., Nucleic Acids Research 30(1): 276-280 (2002)). A set of tools for *in silico* analysis of protein sequences is available on the ExPASy proteomics server (Swiss Institute of Bioinformatics (Gasteiger et al., ExPASy: the proteomics server for in-depth protein knowledge and analysis, Nucleic Acids Res. 31:3784-3788(2003)). Domains or motifs may also be identified using routine techniques, such as by sequence alignment.

Methods for the alignment of sequences for comparison are well known in the art, such methods include GAP, BESTFIT, BLAST, FASTA and TFASTA. GAP uses the algorithm of Needleman and Wunsch ((1970) J Mol Biol 48: 443-453) to find the global (i.e. spanning the complete sequences) alignment of two sequences that maximizes the number of matches and minimizes the number of gaps. The BLAST algorithm (Altschul et al. (1990) J Mol Biol 215: 403-10) calculates percent sequence identity and performs a statistical analysis of the similarity between the two sequences. The software for performing BLAST analysis is publicly available through the National Centre for Biotechnology Information (NCBI). Homologues may readily be identified using, for example, the ClustalW multiple sequence alignment algorithm (version 1.83), with the default pairwise alignment parameters, and a scoring method in percentage. Global percentages of similarity and identity may also be determined using one of the methods available in the MatGAT software package (Campanella et al., BMC Bioinformatics. 2003 Jul 10;4:29. MatGAT: an application that generates similarity/identity matrices using protein or DNA sequences.). Minor manual editing may be performed to optimise alignment between conserved motifs, as would be apparent to a person skilled in the art. Furthermore, instead of using full-length sequences for the identification of homologues, specific domains may also be used. The sequence identity values may be determined over the entire nucleic acid or amino acid sequence or over selected domains or conserved motif(s), using the programs mentioned above using the default parameters. For local alignments, the Smith-Waterman algorithm is particularly useful (Smith TF, Waterman MS (1981) J. Mol. Biol 147(1);195-7).

Furthermore, bHLH6-like polypeptides (at least in their native form) typically have DNA binding activity. Tools and techniques for measuring DNA binding activity are well known in the art. In addition, as shown in the present invention, a bHLH6-like protein, such as SEQ ID NO: 2, when overexpressed in rice, gives plants having enhanced yield-related traits, in particular emergence vigour and/or increased number of flowers per panicle. Other bioassays are provided in Dombrecht et al. (Plant Cell 19, 2225-2245, 2007). Further details are provided in Examples section.

The present invention is illustrated by transforming plants with the nucleic acid sequence represented by SEQ ID NO: 1, encoding the polypeptide sequence of SEQ ID NO: 2. However, performance of the invention is not restricted to these sequences; the methods of the invention may advantageously be performed using any bHLH6-like-encoding nucleic acid or bHLH6-like polypeptide as defined herein.

Examples of nucleic acids encoding bHLH6-like polypeptides are given in Table A1 of Example 1 herein. Such nucleic acids are useful in performing the methods of the invention. The amino acid sequences given in Table A1 of Example 1 are example sequences of orthologues and paralogues of the bHLH6-like polypeptide represented by SEQ ID NO: 2, the terms "orthologues" and "paralogues" being as defined herein. Further orthologues and paralogues may readily be identified by performing a so-called reciprocal blast search. Typically, this involves a first BLAST involving BLASTing a query sequence (for example using any of the sequences listed in Table A1 of Example 1) against any sequence database, such as the publicly available NCBI database. BLASTN or TBLASTX (using standard default values) are generally used when starting from a nucleotide sequence, and BLASTP or TBLASTN (using standard default values) when starting from a protein sequence. The BLAST results may optionally be filtered. The full-length sequences of either the filtered results or non-filtered results are then BLASTed back (second BLAST) against sequences from the organism from which the query sequence is derived (where the query sequence is SEQ ID NO: 1 or SEQ ID NO: 2, the second BLAST would therefore be against *Arabidopsis thaliana* sequences). The results of the first and second BLASTs are then compared. A paralogue is identified if a high-ranking hit from the first blast is from the same species as from which the query sequence is derived, a BLAST back then ideally results in the query sequence amongst the highest hits; an orthologue is identified if a high-ranking hit in the first BLAST is not from the same species as from which the query sequence is derived, and preferably results upon BLAST back in the query sequence being among the highest hits.

High-ranking hits are those having a low E-value. The lower the E-value, the more significant the score (or in other words the lower the chance that the hit was found by chance). Computation of the E-value is well known in the art. In addition to E-values, comparisons are also scored by percentage identity. Percentage identity refers to the number of identical nucleotides (or amino acids) between the two compared nucleic acid (or polypeptide) sequences over a particular length. In the case of large families, ClustalW may be used, followed by a neighbour joining tree, to help visualize clustering of related genes and to identify orthologues and paralogues.

Nucleic acid variants may also be useful in practising the methods of the invention. Examples of such variants include nucleic acids encoding homologues and derivatives of any one of the amino acid sequences given in Table A1 of Example 1, the terms "homologue" and "derivative" being as defined herein. Also useful in the methods of the invention are nucleic acids encoding homologues and derivatives of orthologues or paralogues of any one of the amino acid sequences given in Table A1 of Example 1. Homologues and derivatives useful in the methods of the present invention have substantially the same biological and functional activity as the unmodified protein from which they are derived.

Further nucleic acid variants useful in practising the methods of the invention include portions of nucleic acids encoding bHLH6-like polypeptides, nucleic acids hybridising to nucleic acids encoding bHLH6-like polypeptides, splice variants of nucleic acids encoding bHLH6-like polypeptides, allelic variants of nucleic acids encoding bHLH6-like polypeptides and variants of nucleic acids encoding bHLH6-like polypeptides obtained by gene shuffling. The terms hybridising sequence, splice variant, allelic variant and gene shuffling are as described herein.

Nucleic acids encoding bHLH6-like polypeptides need not be full-length nucleic acids, since performance of the methods of the invention does not rely on the use of full-length nucleic acid sequences. According to the present invention, there is provided a method for enhancing yield-related traits in plants, comprising introducing and expressing in a plant a portion of any one of the nucleic acid sequences given in Table A1 of Example 1, or a portion of a nucleic acid encoding an orthologue, paralogue or homologue of any of the amino acid sequences given in Table A1 of Example 1.

A portion of a nucleic acid may be prepared, for example, by making one or more deletions to the nucleic acid. The portions may be used in isolated form or they may be fused to other coding (or non-coding) sequences in order to, for example, produce a protein that combines several activities. When fused to other coding sequences, the resultant polypeptide produced upon translation may be bigger than that predicted for the protein portion.

Portions useful in the methods of the invention, encode a bHLH6-like polypeptide as defined herein, and have substantially the same biological activity as the amino acid sequences given in Table A1 of Example 1. Preferably, the portion is a portion of any one of the nucleic acids given in Table A1 of Example 1, or is a portion of a nucleic acid encoding an orthologue or paralogue of any one of the amino acid sequences given in Table A1 of Example 1. Preferably the portion is at least 500, 600, 700, 800, 900, 1000, 1100, 1200; 1300, 1400, 1500, 1600, 1700, 1800, 1900, 2000, 2100, 2200 consecutive nucleotides in length, the consecutive nucleotides being of any one of the nucleic acid sequences given in Table A1 of Example 1, or of a nucleic acid encoding an orthologue or paralogue of any one of the amino acid sequences given in Table A1 of Example 1. Most preferably the portion is a portion of the nucleic acid of SEQ ID NO: 1. Preferably, the portion encodes a fragment of an amino acid sequence which, when used in the construction of a phylogenetic tree, such as the one depicted in Figure 6 in Li et al. (2006), clusters within subgroup N (which comprises the amino acid sequence represented by SEQ ID NO: 2), rather than with any other group.

Another nucleic acid variant useful in the methods of the invention is a nucleic acid capable of hybridising, under reduced stringency conditions, preferably under stringent conditions, with a nucleic acid encoding a bHLH6-like polypeptide as defined herein, or with a portion as defined herein.

According to the present invention, there is provided a method for enhancing yield-related traits in plants, comprising introducing and expressing in a plant a nucleic acid capable of hybridizing to any one of the nucleic acids given in Table A1 of Example 1, or comprising introducing and expressing in a plant a nucleic acid capable of hybridising to a nucleic acid encoding an orthologue, paralogue or homologue of any of the nucleic acid sequences given in Table A1 of Example 1.

Hybridising sequences useful in the methods of the invention encode a bHLH6-like polypeptide as defined herein, having substantially the same biological activity as the amino acid sequences given in Table A1 of Example 1. Preferably, the hybridising sequence is capable of hybridising to any one of the nucleic acids given in Table A1 of Example 1, or to a portion of any of these sequences, a portion being as defined above, or the hybridising sequence is capable of hybridising to a nucleic acid encoding an orthologue or paralogue of any one of the amino acid sequences given in Table A1 of Example 1. Most preferably, the hybridising sequence is capable of hybridising to a nucleic acid as represented by SEQ ID NO: 1 or to a portion thereof.

Preferably, the hybridising sequence encodes a polypeptide with an amino acid sequence which, when full-length and used in the construction of a phylogenetic tree, such as the one depicted in Figure 6 in Li et al. (2006), clusters within subgroup N (which comprises the amino acid sequence represented by SEQ ID NO: 2), rather than with any other group.

Another nucleic acid variant useful in the methods of the invention is a splice variant encoding a bHLH6-like polypeptide as defined hereinabove, a splice variant being as defined herein.

According to the present invention, there is provided a method for enhancing yield-related traits in plants, comprising introducing and expressing in a plant a splice variant of any one of the nucleic acid sequences given in Table A1 of Example 1, or a splice variant of a nucleic acid encoding an orthologue, paralogue or homologue of any of the amino acid sequences given in Table A1 of Example 1.

Preferred splice variants are splice variants of a nucleic acid represented by SEQ ID NO: 1, or a splice variant of a nucleic acid encoding an orthologue or paralogue of SEQ ID NO: 2. Preferably, the amino acid sequence encoded by the splice variant, when used in the construction of a phylogenetic tree, such as the one depicted in Figure 6 in Li et al. (2006), clusters within subgroup N (which comprises the amino acid sequence represented by SEQ ID NO: 2), rather than with any other group.

Another nucleic acid variant useful in performing the methods of the invention is an allelic variant of a nucleic acid encoding a bHLH6-like polypeptide as defined hereinabove, an allelic variant being as defined herein.

According to the present invention, there is provided a method for enhancing yield-related traits in plants, comprising introducing and expressing in a plant an allelic variant of any one of the nucleic acids given in Table A1 of Example 1, or comprising introducing and expressing in a plant an allelic variant of a nucleic acid encoding an orthologue, paralogue or homologue of any of the amino acid sequences given in Table A1 of Example 1.

The polypeptides encoded by allelic variants useful in the methods of the present invention have substantially the same biological activity as the bHLH6-like polypeptide of SEQ ID NO: 2 and any of the amino acids depicted in Table A1 of Example 1. Allelic variants exist in nature, and encompassed within the methods of the present invention is the use of these natural alleles. Preferably, the allelic variant is an allelic variant of SEQ ID NO: 1 or an allelic variant of a nucleic acid encoding an orthologue or paralogue of SEQ ID NO: 2. Preferably, the amino acid sequence encoded by the allelic variant, when used in the construction of a phylogenetic tree, such as the one depicted in Figure 6 in Li et al. (2006), clusters within subgroup N (which comprises the amino acid sequence represented by SEQ ID NO: 2), rather than with any other group.

Gene shuffling or directed evolution may also be used to generate variants of nucleic acids encoding bHLH6-like polypeptides as defined above; the term "gene shuffling" being as defined herein. An example of optimisation of bHLH proteins is provided by Pattanaik et al. (BBA1759, 308-318, 2006). This approach may be used for selecting optimised bHLH6-like proteins for use in enhancing yield related traits in plants.

According to the present invention, there is provided a method for enhancing yield-related traits in plants, comprising introducing and expressing in a plant a variant of any one of the nucleic acid sequences given in Table A1 of Example 1, or comprising introducing and expressing in a plant a variant of a nucleic acid encoding an orthologue, paralogue or homologue of any of the amino acid sequences given in Table A1 of Example 1, which variant nucleic acid is obtained by gene shuffling.

Preferably, the amino acid sequence encoded by the variant nucleic acid obtained by gene shuffling, when used in the construction of a phylogenetic tree such as the one depicted in Figure 6 in Li et al. (2006), clusters within subgroup N (which comprises the amino acid sequence represented by SEQ ID NO: 2), rather than with any other group.

Furthermore, nucleic acid variants may also be obtained by site-directed mutagenesis. Several methods are available to achieve site-directed mutagenesis, the most common being PCR based methods (Current Protocols in Molecular Biology. Wiley Eds.).

Nucleic acids encoding bHLH6-like polypeptides may be derived from any natural or artificial source. The nucleic acid may be modified from its native form in composition and/or genomic environment through deliberate human manipulation. Preferably the bHLH6-like polypeptide-encoding nucleic acid is from a plant, further preferably from a dicotyledonous plant, more preferably from the family Brassicaceae, most preferably the nucleic acid is from *Arabidopsis thaliana.*

Performance of the methods of the invention gives plants having enhanced yield-related traits. In particular performance of the methods of the invention gives plants having increased yield, especially increased seed yield relative to control plants. The terms "yield" and "seed yield" are described in more detail in the "definitions" section herein.

Reference herein to enhanced yield-related traits is taken to mean an increase in biomass (weight) of one or more parts of a plant, which may include aboveground (harvestable) parts and/or (harvestable) parts below ground. In particular, such harvestable parts are seeds, and performance of the methods of the invention results in plants having increased seed yield relative to the seed yield of control plants. It should be noted that the enhanced yield related traits do not encompass increased resistance to osmotic stress, increased oxidative stress resistance or increased resistance to insects.

Taking corn as an example, a yield increase may be manifested as one or more of the following: increase in the number of plants established per square meter, an increase in the number of ears per plant, an increase in the number of rows, number of kernels per row, kernel weight, thousand kernel weight, ear length/diameter, increase in the seed filling rate (which is the number of filled seeds divided by the total number of seeds and multiplied by 100), among others. Taking rice as an example, a yield increase may manifest itself as an increase in one or more of the following: number of plants per square meter, number of panicles per plant, number of spikelets per panicle, number of flowers (florets) per panicle (which is expressed as a ratio of the number of filled seeds over the number of primary panicles), increase in the seed filling rate (which is the number of filled seeds divided by the total number of seeds and multiplied by 100), increase in thousand kernel weight, among others.

The present invention provides a method for increasing yield, especially seed yield of plants, relative to control plants, which method comprises modulating expression in a plant of a nucleic acid encoding a bHLH6-like polypeptide as defined herein.

Since the transgenic plants according to the present invention have increased yield, it is likely that these plants exhibit an increased growth rate (during at least part of their life cycle), relative to the growth rate of control plants at a corresponding stage in their life cycle.

The increased growth rate may be specific to one or more parts of a plant (including seeds), or may be throughout substantially the whole plant. Plants having an increased growth rate may have a shorter life cycle. The life cycle of a plant may be taken to mean the time needed to grow from a dry mature seed up to the stage where the plant has produced dry mature seeds, similar to the starting material. This life cycle may be influenced by factors such as early vigour, growth rate, greenness index, flowering time and speed of seed maturation. The increase in growth rate may take place at one or more stages in the life cycle of a plant or during substantially the whole plant life cycle. Increased growth rate during the early stages in the life cycle of a plant may reflect enhanced vigour. The increase in growth rate may alter the harvest cycle of a plant allowing plants to be sown later and/or harvested sooner than would otherwise be possible (a similar effect may be obtained with earlier flowering time). If the growth rate is sufficiently increased, it may allow for the further sowing of seeds of the same plant species (for example sowing and harvesting of rice plants followed by sowing and harvesting of further rice plants all within one conventional growing period). Similarly, if the growth rate is sufficiently increased, it may allow for the further sowing of seeds of different plants species (for example the sowing and harvesting of corn plants followed by, for example, the sowing and optional harvesting of soybean, potato or any other suitable plant). Harvesting additional times from the same rootstock in the case of some crop plants may also be possible. Altering the harvest cycle of a plant may lead to an increase in annual biomass production per square meter (due to an increase in the number of times (say in a year) that any particular plant may be grown and harvested). An increase in growth rate may also allow for the cultivation of transgenic plants in a wider geographical area than their wild-type counterparts, since the territorial limitations for growing a crop are often determined by adverse environmental conditions either at the time of planting (early season) or at the time of harvesting (late season). Such adverse conditions may be avoided if the harvest cycle is shortened. The growth rate may be determined by deriving various parameters from growth curves, such parameters may be: T-Mid (the time taken for plants to reach 50% of their maximal size) and T-90 (time taken for plants to reach 90% of their maximal size), amongst others.

According to a preferred feature of the present invention, performance of the methods of the invention gives plants having an increased growth rate relative to control plants. Therefore, according to the present invention, there is provided a method for increasing the growth rate of plants, which method comprises modulating expression in a plant of a nucleic acid encoding a bHLH6-like polypeptide as defined herein.

An increase in yield and/or growth rate occurs whether the plant is under non-stress conditions or whether the plant is exposed to various stresses compared to control plants. Plants typically respond to exposure to stress by growing more slowly. In conditions of severe stress, the plant may even stop growing altogether. Mild stress on the other hand is defined herein as being any stress to which a plant is exposed which does not result in the plant ceasing to grow altogether without the capacity to resume growth. Mild stress in the sense of the invention leads to a reduction in the growth of the stressed plants of less than 40%, 35% or 30%, preferably less than 25%, 20% or 15%, more preferably less than 14%, 13%, 12%, 11% or 10% or less in comparison to the control plant under non-stress conditions. Due to advances in agricultural practices (irrigation, fertilization, pesticide treatments) severe stresses are not often encountered in cultivated crop plants. As a consequence, the compromised growth induced by mild stress is often an undesirable feature for agriculture. Mild stresses are the everyday biotic and/or abiotic (environmental) stresses to which a plant is exposed. Abiotic stresses may be due to drought or excess water, anaerobic stress, salt stress, chemical toxicity, oxidative stress and hot, cold or freezing temperatures. The abiotic stress may be an osmotic stress caused by a water stress (particularly due to drought), salt stress, oxidative stress or an ionic stress. Biotic stresses are typically those stresses caused by pathogens, such as bacteria, viruses, fungi, nematodes and insects.

In particular, the methods of the present invention may be performed under non-stress conditions or under conditions of mild drought to give plants having increased yield relative to control plants. As reported in Wang et al. (Planta (2003) 218: 1-14), abiotic stress leads to a series of morphological, physiological, biochemical and molecular changes that adversely affect plant growth and productivity. Drought, salinity, extreme temperatures and oxidative stress are known to be interconnected and may induce growth and cellular damage through similar mechanisms. Rabbani et al. (Plant Physiol (2003) 133: 1755-1767) describes a particularly high degree of "cross talk" between drought stress and high-salinity stress. For example, drought and/or salinisation are manifested primarily as osmotic stress, resulting in the disruption of homeostasis and ion distribution in the cell. Oxidative stress, which frequently accompanies high or low temperature, salinity or drought stress, may cause denaturing of functional and structural proteins. As a consequence, these diverse environmental stresses often activate similar cell signalling pathways and cellular responses, such as the production of stress proteins, up-regulation of anti-oxidants, accumulation of compatible solutes and growth arrest. The term "non-stress" conditions as used herein are those environmental conditions that allow optimal growth of plants. Persons skilled in the art are aware of normal soil conditions and climatic conditions for a given location.

Performance of the methods of the invention gives plants grown under non-stress conditions or under mild drought conditions increased yield relative to control plants grown under comparable conditions. Therefore, according to the present invention, there is provided a method for increasing yield in plants grown under non-stress conditions or under mild drought conditions, which method comprises modulating expression in a plant of a nucleic acid encoding a bHLH6-like polypeptide.

Performance of the methods of the invention gives plants grown under conditions of nutrient deficiency, particularly under conditions of nitrogen deficiency, increased yield relative to control plants grown under comparable conditions. Therefore, according to the present invention, there is provided a method for increasing yield in plants grown under conditions of nutrient deficiency, which method comprises modulating expression in a plant of a nucleic acid encoding a bHLH6-like polypeptide. Nutrient deficiency may result from a lack of nutrients such as nitrogen, phosphates and other phosphorous-containing compounds, potassium, calcium, cadmium, magnesium, manganese, iron and boron, amongst others.

The present invention encompasses plants or parts thereof (including seeds) obtainable by the methods according to the present invention. The plants or parts thereof comprise a nucleic acid transgene encoding a bHLH6-like polypeptide as defined above.

The invention also provides genetic constructs and vectors to facilitate introduction and/or expression in plants of nucleic acids encoding bHLH6-like polypeptides. The gene constructs may be inserted into vectors, which may be commercially available, suitable for transforming into plants and suitable for expression of the gene of interest in the transformed cells. The invention also provides use of a gene construct as defined herein in the methods of the invention.

More specifically, the present invention provides a construct comprising:

(a) a nucleic acid encoding a bHLH6-like polypeptide as defined above;
(b) one or more control sequences capable of driving expression of the nucleic acid sequence of (a); and optionally

(c) a transcription termination sequence.

Preferably, the nucleic acid encoding a bHLH6-like polypeptide is as defined above. The term "control sequence" and "termination sequence" are as defined herein.

Plants are transformed with a vector comprising any of the nucleic acids described above. The skilled artisan is well aware of the genetic elements that must be present on the vector in order to successfully transform, select and propagate host cells containing the sequence of interest. The sequence of interest is operably linked to one or more control sequences (at least to a promoter).

Advantageously, any type of promoter, whether natural or synthetic, may be used to drive expression of the nucleic acid sequence. A constitutive promoter of plant origin is particularly useful in the methods. Preferably the constitutive promoter is also a ubiquitous promoter. See the "Definitions" section herein for definitions of the various promoter types. Preferably, the plant constitutive promoter is a medium strength promoter and is less strong than the CaMV 35S promoter.

It should be clear that the applicability of the present invention is not restricted to the bHLH6-like polypeptide-encoding nucleic acid represented by SEQ ID NO: 1, nor is the applicability of the invention restricted to expression of a bHLH6-like polypeptide-encoding nucleic acid when driven by a constitutive promoter.

The constitutive promoter is preferably a GOS2 promoter, preferably a GOS2 promoter from rice. Further preferably the constitutive promoter is represented by a nucleic acid sequence substantially similar to SEQ ID NO: 12, most preferably the constitutive promoter is as represented by SEQ ID NO: 12. See Table 2a in the "Definitions" section herein for further examples of constitutive promoters.

Optionally, one or more terminator sequences may be used in the construct introduced into a plant. Preferably, the construct comprises an expression cassette essentially similar or identical to SEQ ID NO 56, comprising the GOS2 promoter and the nucleic acid encoding the bHLH6-like polypeptide.

Additional regulatory elements may include transcriptional as well as translational enhancers. Those skilled in the art will be aware of terminator and enhancer sequences that may be suitable for use in performing the invention. An intron sequence may also be added to the 5' untranslated region (UTR) or in the coding sequence to increase the amount of the mature message that accumulates in the cytosol, as described in the definitions section. Other control sequences (besides promoter, enhancer, silencer, intron sequences, 3'UTR and/or 5'UTR regions) may be protein and/or RNA stabilizing elements. Such sequences would be known or may readily be obtained by a person skilled in the art.

The genetic constructs of the invention may further include an origin of replication sequence that is required for maintenance and/or replication in a specific cell type. One example is when a genetic construct is required to be maintained in a bacterial cell as an episomal genetic element (e.g. plasmid or cosmid molecule). Preferred origins of replication include, but are not limited to, the f1-ori and colE1.

For the detection of the successful transfer of the nucleic acid sequences as used in the methods of the invention and/or selection of transgenic plants comprising these nucleic acids, it is advantageous to use marker genes (or reporter genes). Therefore, the genetic construct may optionally comprise a selectable marker gene. Selectable markers are described in more detail in the "definitions" section herein. The marker genes may be removed or excised from the transgenic cell once they are no longer needed. Techniques for marker removal are known in the art, useful techniques are described above in the definitions section.

The invention also provides a method for the production of transgenic plants having enhanced yield-related traits relative to control plants, comprising introduction and expression in a plant of any nucleic acid encoding a bHLH6-like polypeptide as defined hereinabove.

More specifically, the present invention provides a method for the production of transgenic plants having increased enhanced yield-related traits, particularly increased emergence vigour and/or seed yield, which method comprises:

(i) introducing and expressing in a plant or plant cell a bHLH6-like polypeptide-encoding nucleic acid; and
(ii) cultivating the plant cell under conditions promoting plant growth and development.

The nucleic acid of (i) may be any of the nucleic acids capable of encoding a bHLH6-like polypeptide as defined herein.

The nucleic acid may be introduced directly into a plant cell or into the plant itself (including introduction into a tissue, organ or any other part of a plant). According to a preferred feature of the present invention, the nucleic acid is preferably introduced into a plant by transformation. The term "transformation" is described in more detail in the "definitions" section herein.

The genetically modified plant cells can be regenerated via all methods with which the skilled worker is familiar. Suitable methods can be found in the abovementioned publications by S.D. Kung and R. Wu, Potrykus or Höfgen and Willmitzer.

Generally after transformation, plant cells or cell groupings are selected for the presence of one or more markers which are encoded by plant-expressible genes co-transferred with the gene of interest, following which the transformed material is regenerated into a whole plant. To select transformed plants, the plant material obtained in the transformation is, as a rule, subjected to selective conditions so that transformed plants can be distinguished from untransformed plants. For example, the seeds obtained in the above-described manner can be planted and, after an initial growing period, subjected to a suitable selection by spraying. A further possibility consists in growing the seeds, if appropriate after sterilization, on agar plates using a suitable selection agent so that only the transformed seeds can grow into plants. Alternatively, the transformed plants are screened for the presence of a selectable marker such as the ones described above.

Following DNA transfer and regeneration, putatively transformed plants may also be evaluated, for instance using Southern analysis, for the presence of the gene of interest, copy number and/or genomic organisation. Alternatively or additionally, expression levels of the newly introduced DNA may be monitored using Northern and/or Western analysis, both techniques being well known to persons having ordinary skill in the art.

The generated transformed plants may be propagated by a variety of means, such as by clonal propagation or classical breeding techniques. For example, a first generation (or T1) transformed plant may be selfed and homozygous second-generation (or T2) transformants selected, and the T2 plants may then further be propagated through classical breeding techniques. The generated transformed organisms may take a variety of forms. For example, they may be chimeras of transformed cells and non-transformed cells; clonal transformants (e.g., all cells transformed to contain the expression cassette); grafts of transformed and untransformed tissues (e.g., in plants, a transformed rootstock grafted to an untransformed scion).

The present invention clearly extends to any plant cell or plant produced by any of the methods described herein, and to all plant parts and propagules thereof. The present invention extends further to encompass the progeny of a primary transformed or transfected cell, tissue, organ or whole plant that has been produced by any of the aforementioned methods, the only requirement being that progeny exhibit the same genotypic and/or phenotypic characteristic (s) as those produced by the parent in the methods according to the invention.

The invention also includes host cells containing an isolated nucleic acid encoding a bHLH6-like polypeptide as defined hereinabove. Preferred host cells according to the invention are plant cells. Host plants for the nucleic acids or the vector used in the method according to the invention, the expression cassette or construct or vector are, in principle, advantageously all plants, which are capable of synthesizing the polypeptides used in the inventive method.

The methods of the invention are advantageously applicable to any plant. Plants that are particularly useful in the methods of the invention include all plants which belong to the superfamily Viridiplantae, in particular monocotyledonous and dicotyledonous plants including fodder or forage legumes, ornamental plants, food crops, trees or shrubs. According to a preferred embodiment of the present invention, the plant is a crop plant. Examples of crop plants include soybean, sunflower, canola, alfalfa, rapeseed, linseed, cotton, tomato, potato and tobacco. Further preferably, the plant is a monocotyledonous plant. Examples of monocotyledonous plants include sugarcane. More preferably the plant is a cereal. Examples of cereals include rice, maize, wheat, barley, millet, rye, triticale, sorghum, emmer, spelt, secale, einkorn, teff, milo and oats.

The invention also extends to harvestable parts of a plant such as, but not limited to seeds, leaves, fruits, flowers, stems, roots, rhizomes, tubers and bulbs. The invention furthermore relates to products derived, preferably directly derived, from a harvestable part of such a plant, such as dry pellets or powders, oil, fat and fatty acids, starch or proteins.

According to a preferred feature of the invention, the modulated expression is increased expression. Methods for increasing expression of nucleic acids or genes, or gene products, are well documented in the art and examples are provided in the definitions section.

As mentioned above, a preferred method for modulating expression of a nucleic acid encoding a bHLH6-like polypeptide is by introducing and expressing in a plant a nucleic acid encoding a bHLH6-like polypeptide; however the effects of performing the method, i.e. enhancing yield-related traits may also be achieved using other well known techniques, including but not limited to T-DNA activation tagging, TILLING, homologous recombination. A description of these techniques is provided in the definitions section.

The present invention also encompasses use of nucleic acids encoding bHLH6-like polypeptides as described herein and use of these bHLH6-like polypeptides in enhancing any of the aforementioned yield-related traits in plants.

Nucleic acids encoding bHLH6-like polypeptide described herein, or the bHLH6-like polypeptides themselves, may find use in breeding programmes in which a DNA marker is identified which may be genetically linked to a bHLH6-like polypeptide-encoding gene. The nucleic acids/genes, or the bHLH6-like polypeptides themselves may be used to define a molecular marker. This DNA or protein marker may then be used in breeding programmes to select plants having enhanced yield-related traits as defined hereinabove in the methods of the invention.

Allelic variants of a bHLH6-like polypeptide-encoding nucleic acid/gene may also find use in marker-assisted breeding programmes. Such breeding programmes sometimes require introduction of allelic variation by mutagenic treatment of the plants, using for example EMS mutagenesis; alternatively, the programme may start with a collection of allelic variants of so called "natural" origin caused unintentionally. Identification of allelic variants then takes place, for example, by PCR. This is followed by a step for selection of superior allelic variants of the sequence in question and which give increased yield. Selection is typically carried out by monitoring growth performance of plants containing different allelic variants of the sequence in question. Growth performance may be monitored in a greenhouse or in the field. Further optional steps include crossing plants in which the superior allelic variant was identified with another plant. This could be used, for example, to make a combination of interesting phenotypic features.

Nucleic acids encoding bHLH6-like polypeptides may also be used as probes for genetically and physically mapping the genes that they are a part of, and as markers for traits linked to those genes. Such information may be useful in plant breeding in order to develop lines with desired phenotypes. Such use of bHLH6-like polypeptide-encoding nucleic acids requires only a nucleic acid sequence of at least 15 nucleotides in length. The bHLH6-like polypeptide-encoding nucleic acids may be used as restriction fragment length polymorphism (RFLP) markers. Southern blots (Sambrook J, Fritsch EF and Maniatis T (1989) Molecular Cloning, A Laboratory Manual) of restriction-digested plant genomic DNA may be probed with the bHLH6-like-encoding nucleic acids. The resulting banding patterns may then be subjected to genetic analyses using computer programs such as MapMaker (Lander et al. (1987) Genomics 1: 174-181) in order to construct a genetic map. In addition, the nucleic acids may be used to probe Southern blots containing restriction endonuclease-treated genomic DNAs of a set of individuals representing parent and progeny of a defined genetic cross. Segregation of the DNA polymorphisms is noted and used to calculate the position of the bHLH6-like polypeptide-encoding nucleic acid in the genetic map previously obtained using this population (Botstein et al. (1980) Am. J. Hum. Genet. 32:314-331).

The production and use of plant gene-derived probes for use in genetic mapping is described in Bernatzky and Tanksley (1986) Plant Mol. Biol. Reporter 4: 37-41. Numerous publications describe genetic mapping of specific cDNA clones using the methodology outlined above or variations thereof. For example, F2 intercross populations, backcross populations, randomly mated populations, near isogenic lines, and other sets of individuals may be used for mapping. Such methodologies are well known to those skilled in the art.

The nucleic acid probes may also be used for physical mapping (i.e., placement of sequences on physical maps; see Hoheisel et al. In: Non-mammalian Genomic Analysis: A Practical Guide, Academic press 1996, pp. 319-346, and references cited therein).

In another embodiment, the nucleic acid probes may be used in direct fluorescence in situ hybridisation (FISH) mapping (Trask (1991) Trends Genet. 7:149-154). Although current methods of FISH mapping favour use of large clones (several kb to several hundred kb; see Laan et al. (1995) Genome Res. 5:13-20), improvements in sensitivity may allow performance of FISH mapping using shorter probes.

A variety of nucleic acid amplification-based methods for genetic and physical mapping may be carried out using the nucleic acids. Examples include allele-specific amplification (Kazazian (1989) J. Lab. Clin. Med 11:95-96), polymorphism of PCR-amplified fragments (CAPS; Sheffield et al. (1993) Genomics 16:325-332), allele-specific ligation (Landegren et al. (1988) Science 241:1077-1080), nucleotide extension reactions (Sokolov (1990) Nucleic Acid Res. 18:3671), Radiation Hybrid Mapping (Walter et al. (1997) Nat. Genet. 7:22-28) and Happy Mapping (Dear and Cook (1989) Nucleic Acid Res. 17:6795-6807). For these methods, the sequence of a nucleic acid is used to design and produce primer pairs for use in the amplification reaction or in primer extension reactions. The design of such primers is well known to those skilled in the art. In methods employing PCR-based genetic mapping, it may be necessary to identify DNA sequence differences between the parents of the mapping cross in the region corresponding to the instant nucleic acid sequence. This, however, is generally not necessary for mapping methods.

The methods according to the present invention result in plants having enhanced yield-related traits, as described hereinbefore. These traits may also be combined with other economically advantageous traits, such as further yield-enhancing traits, tolerance to other abiotic and biotic stresses, traits modifying various architectural features and/or biochemical and/or physiological features.

In one embodiment the invention relates to subject mater summarized as follows:

Item 1: A method for enhancing yield-related traits in plants relative to control plants, comprising modulating expression in a plant of a nucleic acid encoding a bHLH6-like polypeptide, wherein said bHLH6-like polypeptide comprises a HLH domain.

Item 2: A method according to item 1, wherein said bHLH6-like polypeptide comprises one or more of the following motifs:

Motif 1 (SEQ ID NO: 3),
Motif 2 (SEQ ID NO: 4),
Motif 3: (SEQ ID NO: 5)

Motif 7 (SEQ ID NO: 9) or a sequence that has at least 80% sequence identity to SEQ ID NO: 9.

Item 3: Method according to item 1 or 2, wherein said modulated expression is effected by introducing and expressing in a plant a nucleic acid encoding a bHLH6-like polypeptide.

Item 4: Method according to any preceding claim, wherein said nucleic acid encoding a bHLH6-like polypeptide encodes any one of the proteins listed in Table A1 or is a portion of such a nucleic acid, or a nucleic acid capable of hybridising with such a nucleic acid.

Item 5: Method according to any preceding claim, wherein said nucleic acid sequence encodes an orthologue or paralogue of any of the proteins given in Table A1.

Item 6: Method according to any preceding claim, wherein said enhanced yield-related traits comprise increased yield, preferably increased emergence vigour and/or increased seed yield relative to control plants.

Item 7: Method according to any one of items 1 to 6, wherein said enhanced yield-related traits are obtained under non-stress conditions.

Item 8: Method according to any one of items 3 to 7, wherein said nucleic acid is operably linked to a constitutive promoter, preferably to a GOS2 promoter, most preferably to a GOS2 promoter from rice.

Item 9: Method according to any preceding item, wherein said nucleic acid encoding a bHLH6-like polypeptide is of plant origin, preferably from a dicotyledonous plant, further preferably from the family Brassicaceae, more preferably from the genus *Arabidopsis,* most preferably from *Arabidopsis thaliana.*

Item 10: Plant or part thereof, including seeds, obtainable by a method according to any preceding item, wherein said plant or part thereof comprises a recombinant nucleic acid encoding a bHLH6-like polypeptide.

Item 11: Construct comprising:

(a) nucleic acid encoding a bHLH6-like polypeptide as defined in items 1 or 2;
(b) one or more control sequences capable of driving expression of the nucleic acid sequence of (a); and optionally
(c) a transcription termination sequence.

Item 12: Construct according to item 11, wherein one of said control sequences is a constitutive promoter, preferably a GOS2 promoter, most preferably a GOS2 promoter from rice.

Item 13: Use of a construct according to item 11 or 12 in a method for making plants having increased yield, particularly increased emergence vigour and/or increased seed yield relative to control plants.

Item 14: Plant, plant part or plant cell transformed with a construct according to item 10 or 11.

Item 15: Method for the production of a transgenic plant having increased yield, particularly increased biomass and/or increased seed yield relative to control plants, comprising:

(i) introducing and expressing in a plant a nucleic acid encoding a bHLH6-like polypeptide as defined in item 1 or 2; and
(ii) cultivating the plant cell under conditions promoting plant growth and development.

Item 16: Transgenic plant having increased yield, particularly increased biomass and/or increased seed yield, relative to control plants, resulting from modulated expression of a nucleic acid encoding a bHLH6-like polypeptide as defined in item 1 or 2, or a transgenic plant cell derived from said transgenic plant.

Item 17: Transgenic plant according to item 10, 14 or 16, or a transgenic plant cell derived thereof, wherein said plant is a crop plant or a monocot or a cereal, such as rice, maize, wheat, barley, millet, rye, triticale, sorghum emmer, spelt, secale, einkorn, teff, milo and oats.

Item 18: Harvestable parts of a plant according to item 17, wherein said harvestable parts are preferably shoot biomass and/or seeds.

Item 19: Products derived from a plant according to item 17 and/or from harvestable parts of a plant according to item 19.

Item 20: Use of a nucleic acid encoding a bHLH6-like polypeptide in increasing yield, particularly in increasing seed yield and/or shoot biomass in plants, relative to control plants.

In another embodiment of the invention it has now been found that modulating expression in a plant of a nucleic acid sequence encoding a GRP polypeptide, wherein said GRP polypeptide is an RrmJ/FtsJ ribosomal RNA methyltransferase polypeptide, gives plants having enhanced yield-related traits relative to control plants. According to a first embodiment, the present invention provides a method for enhancing yield-related traits in plants relative to control plants, comprising modulating expression in a plant of a nucleic acid sequence encoding a GRP polypeptide, wherein said GRP polypeptide is an RrmJ/FtsJ ribosomal RNA methyltransferase polypeptide (RrmJ/FtsJ polypeptide).
A preferred method for modulating (preferably, increasing) expression of a nucleic acid sequence encoding a GRP polypeptide is by introducing and expressing in a plant a nucleic acid sequence encoding a GRP polypeptide.
In one embodiment any reference hereinafter to a "protein useful in the methods of the invention" is taken to mean a GRP polypeptide as defined herein. In the same embodiment any reference hereinafter to a "nucleic acid useful in the methods of the invention" is taken to mean a nucleic acid sequence capable of encoding such a GRP polypeptide. In one embodiment the nucleic acid sequence to be introduced into a plant (and therefore useful in performing the methods of the invention) is any nucleic acid sequence encoding the type of protein which will now be described, hereafter also named "*GRP* nucleic acid" or "*GRP* gene".
In one embodiment a "GRP polypeptide" as defined herein refers the proteins represented by SEQ ID NO: 58, by SEQ ID NO: 60, and to homologues (orthologues and paralogues) thereof. Table A2 of Example 1 herein presents such orthologues and paralogues.
Preferably, the homologues of SEQ ID NO: 58 or of SEQ ID NO: 60 have a ribosomal RNA methyltransferase RrmJ/FtsJ domain (InterPro entries IPR002877 and IPR015507; Pfam entry PF01728; PANTHER entry PTHR10920).
Alternatively or additionally, a "GRP polypeptide" as defined herein refers to any polypeptide having in increasing order of preference at least 50%, 55%, 60%, 65%, 70%, 75%, 80%, 85%, 90%, 95%, 98%, 99% or more polypeptide sequence identity to the GRP polypeptide as represented by SEQ ID NO: 58, or by SEQ ID NO: 60, or to any of the polypeptide sequences given in Table A2 of Example 1 herein.
In one embodiment the methods of the present invention make use of a partial GRP polypeptide, wherein the GRP polypeptide is an RrmJ/FtsJ polypeptide. GRP polypeptides may be identified by the presence of one or more of several well-known features (see above). Upon identification of a GRP polypeptide, a person skilled in the art could easily derive, using routine techniques, a corresponding partial GRP-encoding nucleic acid sequence useful in performing the methods of the invention.
The term "domain" and "motif" is defined in the "definitions" section herein. Specialist databases exist for the identification of domains, for example, SMART (Schultz et al. (1998) Proc. Natl. Acad. Sci. USA 95, 5857-5864; Letunic et al. (2002) Nucleic Acids Res 30, 242-244), InterPro (Mulder et al., (2003) Nucl. Acids. Res. 31, 315-318), Prosite (Bucher and Bairoch (1994), A generalized profile syntax for biomolecular sequences motifs and its function in automatic sequence interpretation. (In) ISMB-94; Proceedings 2nd International Conference on Intelligent Systems for Molecular Biology. Altman R., Brutlag D., Karp P., Lathrop R., Searls D., Eds., pp53-61, AAAI Press, Menlo Park; Hulo et al., Nucl. Acids. Res. 32:D134-D137, (2004)), or Pfam (Bateman et al., Nucleic Acids Research 30(1): 276-280 (2002)). A set of tools for *in silico* analysis of protein sequences is available on the ExPASy proteomics server (Swiss Institute of Bioinformatics (Gasteiger et al., ExPASy: the proteomics server for in-depth protein knowledge and analysis, Nucleic Acids Res. 31:3784-3788(2003)). Domains or motifs may also be identified using routine techniques, such as by sequence alignment.
Methods for the alignment of sequences for comparison are well known in the art, such methods include GAP, BESTFIT, BLAST, FASTA and TFASTA. GAP uses the algorithm of Needleman and Wunsch ((1970) J Mol Biol 48: 443-453) to find the global (i.e. spanning the complete sequences) alignment of two sequences that maximizes the number of matches and minimizes the number of gaps. The BLAST algorithm (Altschul et al. (1990) J Mol Biol 215: 403-10) calculates percent sequence identity and performs a statistical analysis of the similarity between the two sequences. The software for performing BLAST analysis is publicly available through the National Centre for Biotechnology Information (NCBI). Homologues may readily be identified using, for example, the ClustalW multiple sequence alignment algorithm (version 1.83), with the default pairwise alignment parameters, and a scoring method in percentage. Global percentages of similarity and identity may also be determined using one of the methods available in the MatGAT software package (Campanella et al., BMC Bioinformatics. 2003 Jul 10;4:29. MatGAT: an application that generates similarity/identity matrices using protein or DNA sequences). Minor manual editing may be performed to optimise alignment between conserved motifs, as would be apparent to a person skilled in the art. Furthermore, instead of using full-length sequences for the identification of homologues, specific domains may also be used. The sequence identity values may be determined over the entire nucleic acid sequence or polypeptide sequence or over selected domains or conserved motif(s), using the programs mentioned above using the default parameters.

Furthermore, GRP polypeptides, as far as SEQ ID NO: 58, SEQ ID NO: 60, and its homologues are concerned, typically have the capability to methylate 23 S ribosomal RNA at position U2552 in the aminoacyl (A)1-site of the ribosome, only when the 23 S rRNA is present in 50 S ribosomal subunits. Tools and techniques for assaying the rRNA methyltransferase activity are well known in the art, for example in Hager et al. (2004) J Bacteriol 186(19): 6634-6642.

The present invention is illustrated by transforming plants with the nucleic acid sequence represented by SEQ ID NO: 57, encoding the polypeptide sequence of SEQ ID NO: 58. However, performance of the invention is not restricted to these sequences; the methods of the invention may advantageously be performed using any GRP-encoding nucleic acid sequence or GRP polypeptide as defined herein.

Examples of nucleic acids encoding GRP polypeptides may be found in databases known in the art. Such nucleic acids are useful in performing the methods of the invention. Orthologues and paralogues, the terms "orthologues" and "paralogues" being as defined herein, may readily be identified by performing a so-called reciprocal blast search. Typically, this involves a first BLAST involving BLASTing a query sequence (for example using SEQ ID NO: 58 or SEQ ID NO: 60) against any sequence database, such as the publicly available NCBI database. BLASTN or TBLASTX (using standard default values) are generally used when starting from a nucleotide sequence, and BLASTP or TBLASTN (using standard default values) when starting from a protein sequence. The BLAST results may optionally be filtered. The full-length sequences of either the filtered results or non-filtered results are then BLASTed back (second BLAST) against sequences from the organism from which the query sequence is derived (where the query sequence is SEQ ID NO: 57, SEQ ID NO: 58, SEQ ID NO: 59, or SEQ ID NO: 60, the second BLAST would therefore be against *Nicotiana tabacum* sequences). The results of the first and second BLASTs are then compared. A paralogue is identified if a high-ranking hit from the first blast is from the same species as from which the query sequence is derived, a BLAST back then ideally results in the query sequence amongst the highest hits; an orthologue is identified if a high-ranking hit in the first BLAST is not from the same species as from which the query sequence is derived, and preferably results upon BLAST back in the query sequence being among the highest hits.

High-ranking hits are those having a low E-value. The lower the E-value, the more significant the score (or in other words the lower the chance that the hit was found by chance). Computation of the E-value is well known in the art. In addition to E-values, comparisons are also scored by percentage identity. Percentage identity refers to the number of identical nucleotides (or amino acids) between the two compared nucleic acid sequence (or polypeptide) sequences over a particular length. In the case of large families, ClustalW may be used, followed by a neighbour joining tree, to help visualize clustering of related genes and to identify orthologues and paralogues.

Nucleic acid variants encoding homologues and derivatives of SEQ ID NO: 58 or of SEQ ID NO: 60, may also be useful in practising the methods of the invention, the terms "homologue" and "derivative" being as defined herein. Also useful in the methods of the invention are nucleic acids encoding homologues and derivatives of orthologues or paralogues of SEQ ID NO: 58 or of SEQ ID NO: 60. Homologues and derivatives useful in the methods of the present invention have substantially the same biological and functional activity as the unmodified protein from which they are derived.

Further nucleic acid sequence variants useful in practising the methods of the invention include portions of nucleic acids encoding GRP polypeptides, nucleic acids hybridising to nucleic acids encoding GRP polypeptides, splice variants of nucleic acids encoding GRP polypeptides, allelic variants of nucleic acids encoding GRP polypeptides and variants of nucleic acids encoding GRP polypeptides obtained by gene shuffling. The terms hybridising sequence, splice variant, allelic variant and gene shuffling are as described herein.

Nucleic acids encoding GRP polypeptides need not be full-length nucleic acids, since performance of the methods of the invention does not rely on the use of full-length nucleic acid sequences. According to the present invention, there is provided a method for enhancing yield-related traits in plants, comprising introducing and expressing in a plant a portion of SEQ ID NO: 57, or a portion of SEQ ID NO: 59, or a portion of a nucleic acid sequence encoding an orthologue, paralogue or homologue of SEQ ID NO: 60.

A portion of a nucleic acid sequence may be prepared, for example, by making one or more deletions to the nucleic acid. The portions may be used in isolated form or they may be fused to other coding (or non-coding) sequences in order to, for example, produce a protein that combines several activities. When fused to other coding sequences, the resultant polypeptide produced upon translation may be bigger than that predicted for the protein portion.

Portions useful in the methods of the invention, encode a GRP polypeptide as defined herein, and have substantially the same biological activity as the polypeptide sequences given in SEQ ID NO: 58 or of SEQ ID NO: 60. Preferably, the portion is a portion of the nucleic acid given in SEQ ID NO: 57 or SEQ ID NO: 59, or is a portion of a nucleic acid sequence encoding an orthologue or paralogue of the polypeptide sequences given in SEQ ID NO: 58 or of SEQ ID NO: 60. Preferably the portion is at least 400, 450, 500, 550, 600, 650, 700, 750 consecutive nucleotides in length, the consecutive nucleotides being of SEQ ID NO: 57 or of SEQ ID NO: 59, or of a nucleic acid sequence encoding an orthologue or paralogue of SEQ ID NO: 58 or of SEQ ID NO: 60. Most preferably the portion is a portion of the nucleic acid sequence of SEQ ID NO: 57 or of SEQ ID NO: 59.

Another nucleic acid sequence variant useful in the methods of the invention is a nucleic acid sequence capable of hybridising, under reduced stringency conditions, preferably under stringent conditions, with a nucleic acid sequence encoding a GRP polypeptide as defined herein, or with a portion as defined herein.

According to the present invention, there is provided a method for enhancing yield-related traits in plants, comprising introducing and expressing in a plant a nucleic acid sequence capable of hybridizing to SEQ ID NO: 57 or to SEQ ID NO: 59, or comprising introducing and expressing in a plant a nucleic acid sequence capable of hybridising to a nucleic acid sequence encoding an orthologue, paralogue or homologue of SEQ ID NO: 58 or of SEQ ID NO: 60.

Hybridising sequences useful in the methods of the invention encode a GRP polypeptide as defined herein, having substantially the same biological activity as the polypeptide sequences given in SEQ ID NO: 58. Preferably, the hybridising sequence is capable of hybridising to SEQ ID NO: 57 or to SEQ ID NO: 59, or to a portion of any of these sequences, a portion being as defined above, or the hybridising sequence is capable of hybridising to a nucleic acid sequence encoding an orthologue or paralogue of SEQ ID NO: 58 or of SEQ ID NO: 60.

Another nucleic acid sequence variant useful in the methods of the invention is a splice variant encoding a GRP polypeptide as defined hereinabove, a splice variant being as defined herein.

According to the present invention, there is provided a method for enhancing yield-related traits in plants, comprising introducing and expressing in a plant a splice variant of SEQ ID NO: 57 or of SEQ ID NO: 59, or a splice variant of a nucleic acid sequence encoding an orthologue, paralogue or homologue of SEQ ID NO: 58 or of SEQ ID NO: 60.

Another nucleic acid sequence variant useful in performing the methods of the invention is an allelic variant of a nucleic acid sequence encoding a GRP polypeptide as defined hereinabove, an allelic variant being as defined herein.

According to the present invention, there is provided a method for enhancing yield-related traits in plants, comprising introducing and expressing in a plant an allelic variant of SEQ ID NO: 57 or of SEQ ID NO: 59, or comprising introducing and expressing in a plant an allelic variant of a nucleic acid sequence encoding an orthologue, paralogue or homologue of the polypeptide sequences represented by SEQ ID NO: 58 or of SEQ ID NO: 60.

The allelic variants useful in the methods of the present invention have substantially the same biological activity as the GRP polypeptide of SEQ ID NO: 58 or of SEQ ID NO: 60. Allelic variants exist in nature, and encompassed within the methods of the present invention is the use of these natural alleles. Gene shuffling or directed evolution may also be used to generate variants of nucleic acids encoding GRP polypeptides as defined above; the term "gene shuffling" being as defined herein.

According to the present invention, there is provided a method for enhancing yield-related traits in plants, comprising introducing and expressing in a plant a variant of SEQ ID NO: 57 or of SEQ ID NO: 59, or comprising introducing and expressing in a plant a variant of a nucleic acid sequence encoding an orthologue, paralogue or homologue of SEQ ID NO: 58 or of SEQ ID NO: 60, which variant nucleic acid sequence is obtained by gene shuffling.

Furthermore, nucleic acid sequence variants may also be obtained by site-directed mutagenesis. Several methods are available to achieve site-directed mutagenesis, the most common being PCR based methods (Current Protocols in Molecular Biology. Wiley Eds.).

Nucleic acids encoding GRP polypeptides may be derived from any natural or artificial source. The nucleic acid sequence may be modified from its native form in composition and/or genomic environment through deliberate human manipulation. Preferably the GRP polypeptide-encoding nucleic acid sequence is from a plant. In the case of SEQ ID NO: 57 or of SEQ ID NO: 59, the GRP polypeptide encoding nucleic acid sequence is preferably from a dicotyledonous plant, more preferably from the family Solanaceae, most preferably the nucleic acid sequence is from *Nicotiana tabacum.*

Performance of the methods of the invention gives plants having enhanced yield-related traits. In particular performance of the methods of the invention gives plants having increased early vigour and increased yield, especially increased biomass and increased seed yield relative to control plants. The terms "yield" and "seed yield" are described in more detail in the "definitions" section herein.

Reference herein to enhanced yield-related traits is taken to mean an increase in early vigour and/or in biomass (weight) of one or more parts of a plant, which may include aboveground (harvestable) parts and/or (harvestable) parts below ground. In particular, such harvestable parts are biomass and/or seeds, and performance of the methods of the invention results in plants having increased early vigour, biomass and/or seed yield relative to the early vigour, biomass or seed yield of control plants.

Taking corn as an example, a yield increase may be manifested as one or more of the following: increase in the number of plants established per hectare or acre, an increase in the number of ears per plant, an increase in the number of rows, number of kernels per row, kernel weight, thousand kernel weight, ear length/diameter, increase in the seed filling rate (which is the number of filled seeds divided by the total number of seeds and multiplied by 100), among others. Taking rice as an example, a yield increase may manifest itself as an increase in one or more of the following:

number of plants per hectare or acre, number of panicles per plant, number of spikelets per panicle, number of flowers (florets) per panicle (which is expressed as a ratio of the number of filled seeds over the number of primary panicles), increase in the seed filling rate (which is the number of filled seeds divided by the total number of seeds and multiplied by 100), increase in thousand kernel weight, among others.

The present invention provides a method for enhancing yield-related traits, especially biomass and/or seed yield of plants, relative to control plants, which method comprises modulating expression, preferably increasing expression, in a plant of a nucleic acid sequence encoding a GRP polypeptide as defined herein.

Since the transgenic plants according to the present invention have enhanced yield-related traits, it is likely that these plants exhibit an increased growth rate (during at least part of their life cycle), relative to the growth rate of control plants at a corresponding stage in their life cycle.

The increased growth rate may be specific to one or more parts of a plant (including seeds), or may be throughout substantially the whole plant. Plants having an increased growth rate may have a shorter life cycle. The life cycle of a plant may be taken to mean the time needed to grow from a dry mature seed up to the stage where the plant has produced dry mature seeds, similar to the starting material. This life cycle may be influenced by factors such as early vigour, growth rate, greenness index, flowering time and speed of seed maturation. The increase in growth rate may take place at one or more stages in the life cycle of a plant or during substantially the whole plant life cycle. Increased growth rate during the early stages in the life cycle of a plant may reflect enhanced vigour. The increase in growth rate may alter the harvest cycle of a plant allowing plants to be sown later and/or harvested sooner than would otherwise be possible (a similar effect may be obtained with earlier flowering time). If the growth rate is sufficiently increased, it may allow for the further sowing of seeds of the same plant species (for example sowing and harvesting of rice plants followed by sowing and harvesting of further rice plants all within one conventional growing period). Similarly, if the growth rate is sufficiently increased, it may allow for the further sowing of seeds of different plants species (for example the sowing and harvesting of corn plants followed by, for example, the sowing and optional harvesting of soybean, potato or any other suitable plant). Harvesting additional times from the same rootstock in the case of some crop plants may also be possible. Altering the harvest cycle of a plant may lead to an increase in annual biomass production per acre (due to an increase in the number of times (say in a year) that any particular plant may be grown and harvested). An increase in growth rate may also allow for the cultivation of transgenic plants in a wider geographical area than their wild-type counterparts, since the territorial limitations for growing a crop are often determined by adverse environmental conditions either at the time of planting (early season) or at the time of harvesting (late season). Such adverse conditions may be avoided if the harvest cycle is shortened. The growth rate may be determined by deriving various parameters from growth curves, such parameters may be: T-Mid (the time taken for plants to reach 50% of their maximal size) and T-90 (time taken for plants to reach 90% of their maximal size), amongst others.

According to a preferred feature of the present invention, performance of the methods of the invention gives plants having an increased growth rate relative to control plants. Therefore, according to the present invention, there is provided a method for increasing the growth rate of plants, which method comprises modulating expression, preferably increasing expression, in a plant of a nucleic acid sequence encoding a GRP polypeptide as defined herein.

An enhancement of yield-related traits (an increase in yield and/or growth rate) occurs whether the plant is under non-stress conditions or whether the plant is exposed to various stresses compared to control plants. Plants typically respond to exposure to stress by growing more slowly. In conditions of severe stress, the plant may even stop growing altogether. Mild stress on the other hand is defined herein as being any stress to which a plant is exposed which does not result in the plant ceasing to grow altogether without the capacity to resume growth. Mild stress in the sense of the invention leads to a reduction in the growth of the stressed plants of less than 40%, 35% or 30%, preferably less than 25%, 20% or 15%, more preferably less than 14%, 13%, 12%, 11 % or 10% or less in comparison to the control plant under non-stress conditions. Due to advances in agricultural practices (irrigation, fertilization, pesticide treatments) severe stresses are not often encountered in cultivated crop plants. As a consequence, the compromised growth induced by mild stress is often an undesirable feature for agriculture. Mild stresses are the everyday biotic and/or abiotic (environmental) stresses to which a plant is exposed. Abiotic stresses may be due to drought or excess water, anaerobic stress, salt stress, chemical toxicity, oxidative stress and hot, cold or freezing temperatures. The abiotic stress may be an osmotic stress caused by a water stress (particularly due to drought), salt stress, oxidative stress or an ionic stress. Biotic stresses are typically those stresses caused by pathogens, such as bacteria, viruses, fungi and insects. The term "non-stress" conditions as used herein are those environmental conditions that allow optimal growth of plants. Persons skilled in the art are aware of normal soil conditions and climatic conditions for a given location.

Performance of the methods of the invention gives plants grown under non-stress conditions or under mild drought conditions enhanced yield-related traits relative to control plants grown under comparable conditions. Therefore, according to the present invention, there is provided a method for enhancing yield-related traits in plants grown under non-stress conditions or under mild drought conditions, which method comprises modulating, preferably increasing, expression in a plant of a nucleic acid sequence encoding a GRP polypeptide.

Performance of the methods according to the present invention results in plants grown under abiotic stress conditions having enhanced yield-related traits, preferably enhanced seed yield-related traits, relative to control plants grown under comparable stress conditions. As reported in Wang et al. (Planta (2003) 218: 1-14), abiotic stress leads to a series of morphological, physiological, biochemical and molecular changes that adversely affect plant growth and productivity. Drought, salinity, extreme temperatures and oxidative stress are known to be interconnected and may induce growth and cellular damage through similar mechanisms. Rabbani et al. (Plant Physiol (2003) 133: 1755-1767) describes a particularly high degree of "cross talk" between drought stress and high-salinity stress. For example, drought and/or salinisation are manifested primarily as osmotic stress, resulting in the disruption of homeostasis and ion distribution in the cell. Oxidative stress, which frequently accompanies high or low temperature, salinity or drought stress, may cause denaturing of functional and structural proteins. As a consequence, these diverse environmental stresses often activate similar cell signalling pathways and cellular responses, such as the production of stress proteins, up-regulation of anti-oxidants, accumulation of compatible solutes and growth arrest. Since diverse environmental stresses activate similar pathways, the exemplification of the present invention with drought stress should not be seen as a limitation to drought stress, but more as a screen to indicate the involvement of GRP polypeptides as defined above, in enhancing yield-related traits, preferably enhancing seed yield-related traits, relative to control plants grown in comparable stress conditions, in abiotic stresses in general.

The term "abiotic stress" as defined herein is taken to mean any one or more of: water stress (due to drought or excess water), anaerobic stress, salt stress, temperature stress (due to hot, cold or freezing temperatures), chemical toxicity stress and oxidative stress. According to one aspect of the invention, the abiotic stress is an osmotic stress, selected from water stress, salt stress, oxidative stress and ionic stress. Preferably, the water stress is drought stress. The term salt stress is not restricted to common salt (NaCl), but may be any stress caused by one or more of: NaCl, KCl, LiCl, MgCl2, CaCl2, amongst others.

Performance of the methods of the invention gives plants having enhanced yield-related traits, preferably enhanced seed yield-related traits, under abiotic stress conditions relative to control plants grown in comparable stress conditions. Therefore, according to the present invention, there is provided a method for enhancing yield-related traits, preferably enhancing seed yield-related traits, in plants grown under abiotic stress conditions, which method comprises modulatingexpression in a plant of a nucleic acid sequence encoding a GRP polypeptide. According to one aspect of the invention, the abiotic stress is an osmotic stress, selected from one or more of the following: water stress, salt stress, oxidative stress and ionic stress.

Performance of the methods of the invention gives plants grown under conditions of nutrient deficiency, particularly under conditions of nitrogen deficiency, enhanced yield-related traits relative to control plants grown under comparable conditions. Therefore, according to the present invention, there is provided a method for enhancing yield-related traits in plants grown under conditions of nutrient deficiency, which method comprises modulating, preferably increasing, expression in a plant of a nucleic acid sequence encoding a GRP polypeptide. Nutrient deficiency may result from a lack of nutrients such as nitrogen, phosphates and other phosphorous-containing compounds, potassium, calcium, cadmium, magnesium, manganese, iron and boron, amongst others.

The present invention encompasses plants, parts thereof (including seeds), or plant cells obtainable by the methods according to the present invention. The plants, parts thereof, or plant cells comprise a nucleic acid transgene encoding a GRP polypeptide as defined above.

The invention also provides genetic constructs and vectors to facilitate introduction and/or expression in plants of nucleic acids encoding GRP polypeptides. The gene constructs may be inserted into vectors, which may be commercially available, suitable for transforming into plants and suitable for expression of the gene of interest in the transformed cells. The invention also provides use of a gene construct as defined herein in the methods of the invention.

More specifically, the present invention provides a construct comprising:

(a) a nucleic acid sequence encoding a GRP polypeptide as defined above;
(b) one or more control sequences capable of driving expression of the nucleic acid sequence of (a); and optionally
(c) a transcription termination sequence.

Preferably, the nucleic acid sequence encoding a GRP polypeptide is as defined above. The term "control sequence" and "termination sequence" are as defined herein.

Plants are transformed with a vector comprising any of the nucleic acids described above. The skilled artisan is well aware of the genetic elements that must be present on the vector in order to successfully transform, select and propagate host cells containing the sequence of interest. The sequence of interest is operably linked to one or more control sequences (at least to a promoter).

Advantageously, any type of promoter, whether natural or synthetic, may be used to drive expression of the nucleic acid sequence. A seed-specific promoter is particularly useful in the methods of the invention. See the "Definitions" section herein for definitions of the various promoter types.

It should be clear that the applicability of the present invention is not restricted to the GRP polypeptide-encoding nucleic acid sequence represented by SEQ ID NO: 57 or of SEQ ID NO: 59, nor is the applicability of the invention restricted to expression of a GRP polypeptide-encoding nucleic acid sequence when driven by a seed-specific promoter.

The seed-specific promoter is preferably an oleosin promoter, preferably a oleosin promoter from rice. Further preferably the oleosin promoter is represented by a nucleic acid sequence substantially similar to SEQ ID NO: 91, most preferably the oleosin promoter is as represented by SEQ ID NO: 91. See Table 2b in the "Definitions" section herein for further examples of seed-specific promoters.

Optionally, one or more terminator sequences may be used in the construct introduced into a plant. Additional regulatory elements may include transcriptional as well as translational enhancers. Those skilled in the art will be aware of terminator and enhancer sequences that may be suitable for use in performing the invention. An intron sequence may also be added to the 5' untranslated region (UTR) or in the coding sequence to increase the amount of the mature message that accumulates in the cytosol, as described in the definitions section. Other control sequences (besides promoter, enhancer, silencer, intron sequences, 3'UTR and/or 5'UTR regions) may be protein and/or RNA stabilizing elements. Such sequences would be known or may readily be obtained by a person skilled in the art.

The genetic constructs of the invention may further include an origin of replication sequence that is required for maintenance and/or replication in a specific cell type. One example is when a genetic construct is required to be maintained in a bacterial cell as an episomal genetic element (e.g. plasmid or cosmid molecule). Preferred origins of replication include, but are not limited to, the f1-ori and colE1.

For the detection of the successful transfer of the nucleic acid sequences as used in the methods of the invention and/or selection of transgenic plants comprising these nucleic acids, it is advantageous to use marker genes (or reporter genes). Therefore, the genetic construct may optionally comprise a selectable marker gene. Selectable markers are described in more detail in the "definitions" section herein. The marker genes may be removed or excised from the transgenic cell once they are no longer needed. Techniques for marker removal are known in the art, useful techniques are described above in the definitions section.

The invention also provides a method for the production of transgenic plants having enhanced yield-related traits relative to control plants, comprising introduction and expression in a plant of any nucleic acid sequence encoding a GRP polypeptide as defined hereinabove.

More specifically, the present invention provides a method for the production of transgenic plants having enhanced yield-related traits which method comprises:

(i) introducing and expressing in a plant, plant part, or plant cell, a nucleic acid sequence encoding a GRP polypeptide; and

(ii) cultivating the plant, plant part or plant cell under conditions promoting plant growth and development.

The nucleic acid sequence of (i) may be any of the nucleic acids capable of encoding a GRP polypeptide as defined herein.

The nucleic acid sequence may be introduced directly into a plant cell or into the plant itself (including introduction into a tissue, organ or any other part of a plant). According to a preferred feature of the present invention, the nucleic acid sequence is preferably introduced into a plant by transformation. The term "transformation" is described in more detail in the "definitions" section herein.

The genetically modified plant cells can be regenerated via all methods with which the skilled worker is familiar. Suitable methods can be found in the abovementioned publications by S.D. Kung and R. Wu, Potrykus or Höfgen and Willmitzer.

Generally after transformation, plant cells or cell groupings are selected for the presence of one or more markers which are encoded by plant-expressible genes co-transferred with the gene of interest, following which the transformed material is regenerated into a whole plant. To select transformed plants, the plant material obtained in the transformation is, as a rule, subjected to selective conditions so that transformed plants can be distinguished from untransformed plants. For example, the seeds obtained in the above-described manner can be planted and, after an initial growing period, subjected to a suitable selection by spraying. A further possibility consists in growing the seeds, if appropriate after sterilization, on agar plates using a suitable selection agent so that only the transformed seeds can grow into plants. Alternatively, the transformed plants are screened for the presence of a selectable marker such as the ones described above.

Following DNA transfer and regeneration, putatively transformed plants may also be evaluated, for instance using Southern analysis, for the presence of the gene of interest, copy number and/or genomic organisation. Alternatively or additionally, expression levels of the newly introduced DNA may be monitored using Northern and/or Western analysis, both techniques being well known to persons having ordinary skill in the art.

The generated transformed plants may be propagated by a variety of means, such as by clonal propagation or classical breeding techniques. For example, a first generation (or T1) transformed plant may be selfed and homozygous second-generation (or T2) transformants selected, and the T2 plants may then further be propagated through classical breeding techniques. The generated transformed organisms may take a variety of forms. For example, they may be chimeras of transformed cells and non-transformed cells; clonal transformants (e.g., all cells transformed to contain the expression cassette); grafts of transformed and untransformed tissues (e.g., in plants, a transformed rootstock grafted to an untransformed scion).

The present invention clearly extends to any plant cell or plant produced by any of the methods described herein, and to all plant parts and propagules thereof. The present invention extends further to encompass the progeny of a primary transformed or transfected cell, tissue, organ or whole plant that has been produced by any of the aforementioned methods, the only requirement being that progeny exhibit the same genotypic and/or phenotypic characteristic (s) as those produced by the parent in the methods according to the invention.

The invention also includes host cells containing an isolated nucleic acid sequence encoding a GRP polypeptide as defined hereinabove. Preferred host cells according to the invention are plant cells. Host plants for the nucleic acids or the vector used in the method according to the invention, the expression cassette or construct or vector are, in principle, advantageously all plants, which are capable of synthesizing the polypeptides used in the inventive method.

The methods of the invention are advantageously applicable to any plant. Plants that are particularly useful in the methods of the invention include all plants which belong to the superfamily Viridiplantae, in particular monocotyledonous and dicotyledonous plants including fodder or forage legumes, ornamental plants, food crops, trees or shrubs. According to a preferred embodiment of the present invention, the plant is a crop plant. Examples of crop plants include soybean, sunflower, canola, alfalfa, rapeseed, cotton, tomato, potato and tobacco. Further preferably, the plant is a monocotyledonous plant. Examples of monocotyledonous plants include sugarcane. More preferably the plant is a cereal. Examples of cereals include rice, maize, wheat, barley, millet, rye, triticale, sorghum and oats.

The invention also extends to harvestable parts of a plant such as, but not limited to seeds, leaves, fruits, flowers, stems, roots, rhizomes, tubers and bulbs. The invention furthermore relates to products derived, preferably directly derived, from a harvestable part of such a plant, such as dry pellets or powders, oil, fat and fatty acids, starch or proteins.

According to a preferred feature of the invention, the modulated expression is increased expression. Methods for increasing expression of nucleic acids or genes, or gene products, are well documented in the art and examples are provided in the definitions section.

As mentioned above, a preferred method for modulating (preferably, increasing) expression of a nucleic acid sequence encoding a GRP polypeptide is by introducing and expressing in a plant a nucleic acid sequence encoding a GRP polypeptide; however the effects of performing the method, i.e. enhancing yield-related traits may also be achieved using other well known techniques, including but not limited to T-DNA activation tagging, TILLING, homologous recombination. A description of these techniques is provided in the definitions section.

The present invention also encompasses use of nucleic acids encoding GRP polypeptides as described herein and use of these GRP polypeptides in enhancing any of the aforementioned yield-related traits in plants.

Nucleic acids encoding GRP polypeptide described herein, or the GRP polypeptides themselves, may find use in breeding programmes in which a DNA marker is identified which may be genetically linked to a GRP polypeptide-encoding gene. The nucleic acids/genes, or the GRP polypeptides themselves may be used to define a molecular marker. This DNA or protein marker may then be used in breeding programmes to select plants having enhanced yield-related traits as defined hereinabove in the methods of the invention.

Allelic variants of a GRP polypeptide-encoding nucleic acid/gene may also find use in marker-assisted breeding programmes. Such breeding programmes sometimes require introduction of allelic variation by mutagenic treatment of the plants, using for example EMS mutagenesis; alternatively, the programme may start with a collection of allelic variants of so called "natural" origin caused unintentionally. Identification of allelic variants then takes place, for example, by PCR. This is followed by a step for selection of superior allelic variants of the sequence in question and which give enhanced yield-related traits. Selection is typically carried out by monitoring growth performance of plants containing different allelic variants of the sequence in question. Growth performance may be monitored in a greenhouse or in the field. Further optional steps include crossing plants in which the superior allelic variant was identified with another plant. This could be used, for example, to make a combination of interesting phenotypic features.

Nucleic acids encoding GRP polypeptides may also be used as probes for genetically and physically mapping the genes that they are a part of, and as markers for traits linked to those genes. Such information may be useful in plant breeding in order to develop lines with desired phenotypes. Such use of GRP polypeptide-encoding nucleic acids requires only a nucleic acid sequence of at least 15 nucleotides in length. The GRP polypeptide-encoding nucleic acids may be used as restriction fragment length polymorphism (RFLP) markers. Southern blots (Sambrook J, Fritsch EF and Maniatis T (1989) Molecular Cloning, A Laboratory Manual) of restriction-digested plant genomic DNA may be probed with the GRP-encoding nucleic acids. The resulting banding patterns may then be subjected to genetic analyses using computer programs such as MapMaker (Lander et al. (1987) Genomics 1: 174-181) in order to construct a genetic map. In addition, the nucleic acids may be used to probe Southern blots containing restriction endonuclease-treated genomic DNAs of a set of individuals representing parent and progeny of a defined genetic cross. Segregation of the DNA polymorphisms is noted and used to calculate the position of the GRP polypeptide-encoding nucleic acid sequence in the genetic map previously obtained using this population (Botstein et al. (1980) Am. J. Hum. Genet. 32:314-331).

The production and use of plant gene-derived probes for use in genetic mapping is described in Bernatzky and Tanksley (1986) Plant Mol. Biol. Reporter 4: 37-41. Numerous publications describe genetic mapping of specific cDNA clones using the methodology outlined above or variations thereof. For example, F2 intercross populations, backcross populations, randomly mated populations, near isogenic lines, and other sets of individuals may be used for mapping. Such methodologies are well known to those skilled in the art.

The nucleic acid sequence probes may also be used for physical mapping (i.e., placement of sequences on physical maps; see Hoheisel et al. In: Non-mammalian Genomic Analysis: A Practical Guide, Academic press 1996, pp. 319-346, and references cited therein).

In another embodiment, the nucleic acid sequence probes may be used in direct fluorescence in situ hybridisation (FISH) mapping (Trask (1991) Trends Genet. 7:149-154). Although current methods of FISH mapping favour use of large clones (several kb to several hundred kb; see Laan et al. (1995) Genome Res. 5:13-20), improvements in sensitivity may allow performance of FISH mapping using shorter probes.

A variety of nucleic acid sequence amplification-based methods for genetic and physical mapping may be carried out using the nucleic acids. Examples include allele-specific amplification (Kazazian (1989) J. Lab. Clin. Med 11:95-96), polymorphism of PCR-amplified fragments (CAPS; Sheffield et al. (1993) Genomics 16:325-332), allele-specific ligation (Landegren et al. (1988) Science 241:1077-1080), nucleotide extension reactions (Sokolov (1990) nucleic acid sequence Res. 18:3671), Radiation Hybrid Mapping (Walter et al. (1997) Nat. Genet. 7:22-28) and Happy Mapping (Dear and Cook (1989) nucleic acid sequence Res. 17:6795-6807). For these methods, the sequence of a nucleic acid sequence is used to design and produce primer pairs for use in the amplification reaction or in primer extension reactions. The design of such primers is well known to those skilled in the art. In methods employing PCR-based genetic mapping, it may be necessary to identify DNA sequence differences between the parents of the mapping cross in the region corresponding to the instant nucleic acid sequence. This, however, is generally not necessary for mapping methods.

The methods according to the present invention result in plants having enhanced yield-related traits, as described hereinbefore. These traits may also be combined with other economically advantageous traits, such as further yield-enhancing traits, tolerance to other abiotic and biotic stresses, traits modifying various architectural features and/or biochemical and/or physiological features.

The term "table A2" used in this specification is to be taken to specify the content of table A2a and table A2b. The term "table A2a" used in this specification is to be taken to specify the content of table A2a. The term "table A2b" used in this specification is to be taken to specify the content of table A2b. In one preferred embodiment, the term "table A2" means table A2b.

In one embodiment the invention relates to subject mater summarized as follows:

Item 21: A method for enhancing yield-related traits in plants relative to control plants, comprising modulating expression in a plant of a nucleic acid sequence encoding a GRP, wherein said GRP is an RrmJ/FtsJ ribosomal RNA methyltransferase polypeptide (RrmJ/FtsJ polypeptide), and optionally selecting for plants having enhance yield-related traits.

Item 22: Method according to item 21, wherein said GRP polypeptide comprises a ribosomal RNA methyltransferase RrmJ/FtsJ domain (InterPro entries IPR002877 and IPR015507; Pfam entry PF01728; PANTHER entry PTHR10920).

Item 23: Method according to item 21 or 22, wherein said GRP polypeptide has in increasing order of preference at least 50%, 55%, 60%, 65%, 70%, 75%, 80%, 85%, 90%, 95%, 98%, 99% or more polypeptide sequence identity to the GRP polypeptide as represented by SEQ ID NO: 58, or by SEQ ID NO: 60, or to any of the polypeptide sequences given in Table A2 of Example 1.

Item 24: Method according to any of the items 21 to 23, wherein said nucleic acid sequence encoding a GRP polypeptide is represented by any one of the nucleic acid sequence SEQ ID NOs given in Table A2 or a portion thereof, or a sequence capable of hybridising with any one of the nucleic acid sequences SEQ ID NOs given in Table A2 of Example 1.

Item 25: Method according to any preceding item wherein said modulated expression is effected by introducing and expressing in a plant a nucleic acid sequence encoding said GRP polypeptide.

Item 26: Method according to any preceding item, wherein said enhanced yield-related traits comprise increased seed yield, increased number of filled seeds, increased seed fill rate, and increased harvest index, relative to control plants.

Item 27: Method according to any preceding item, wherein said modulated expression is increased expression.

Item 28: Method according to any preceding item, wherein said nucleic acid sequence is operably linked to a seed-specific promoter, preferably to an oleosin promoter, most preferably to an oleosin promoter from rice.

Item 29: Method according to any preceding item, wherein said nucleic acid sequence encoding a GRP polypeptide is of plant origin, preferably from a dicotyledonous plant, further preferably from the family Solanaceae, more preferably from *Nicotiana tabacum.*

Item 30: An isolated nucleic acid sequence comprising a nucleic acid sequence selected from the group consisting of:

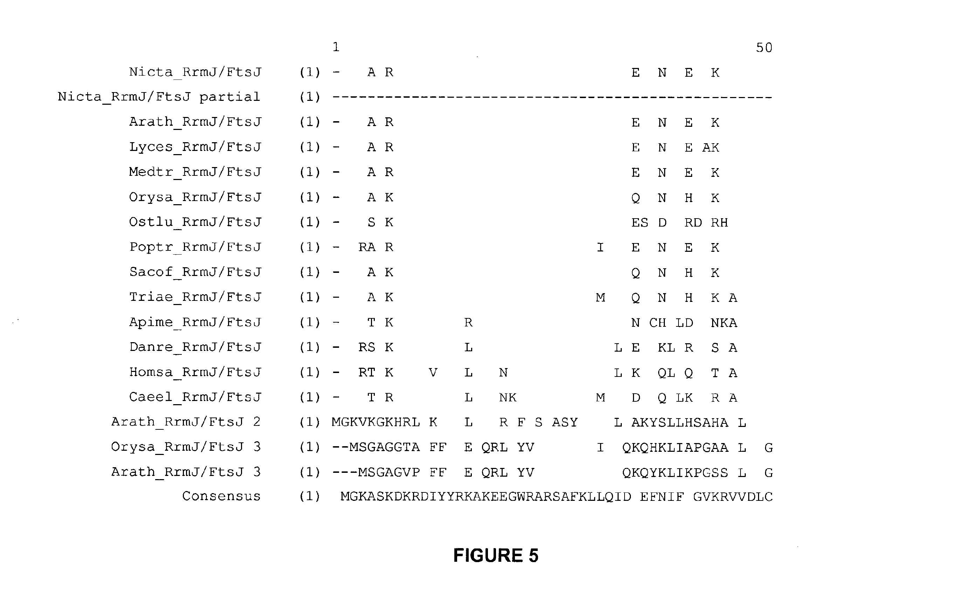

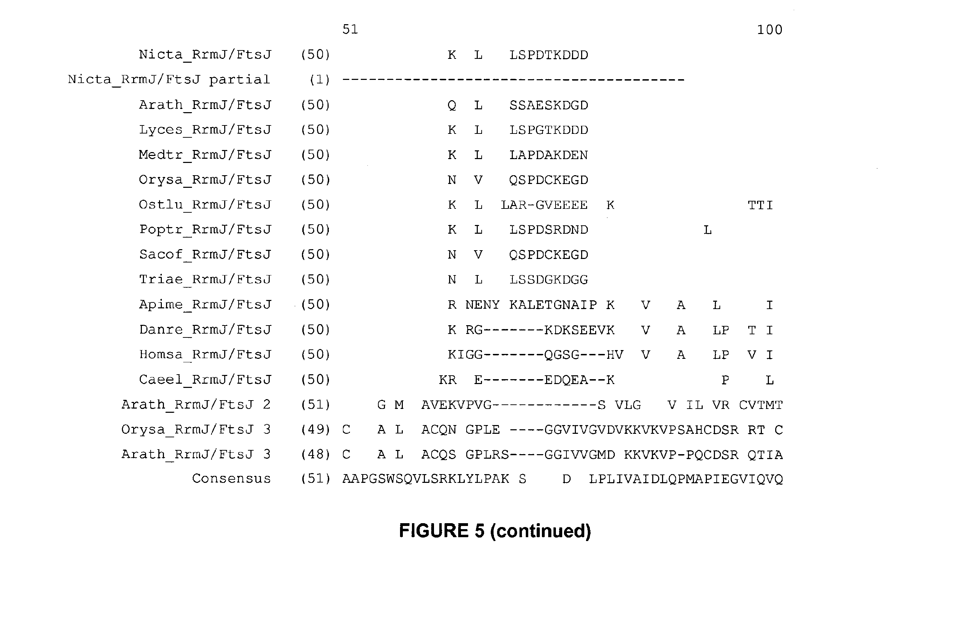

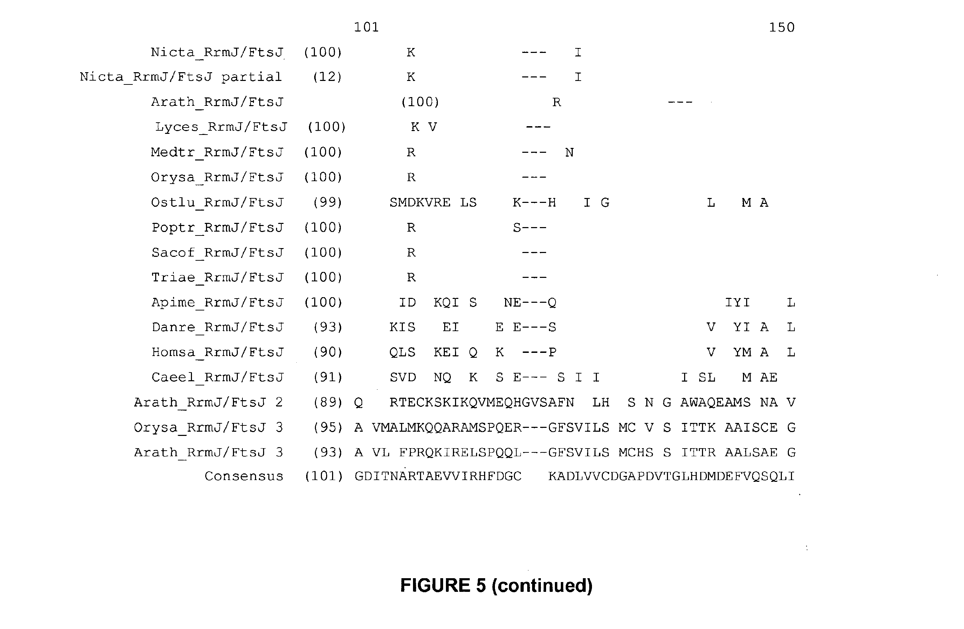

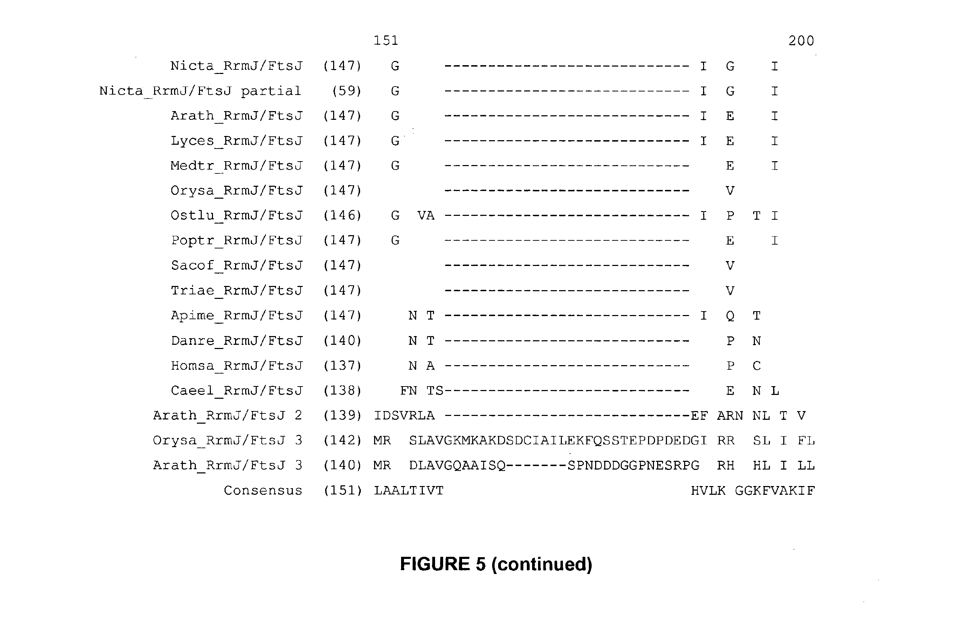

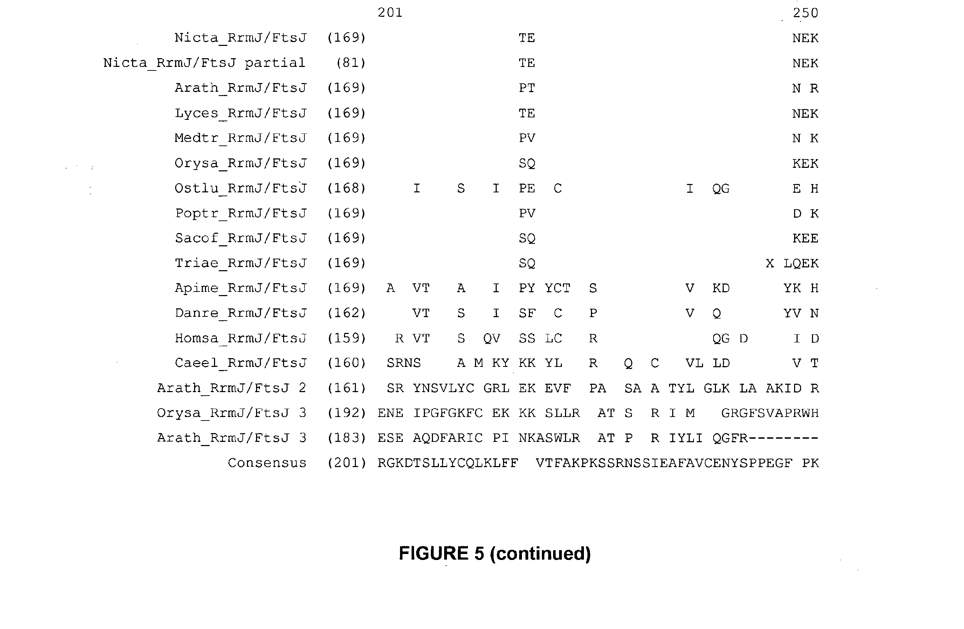

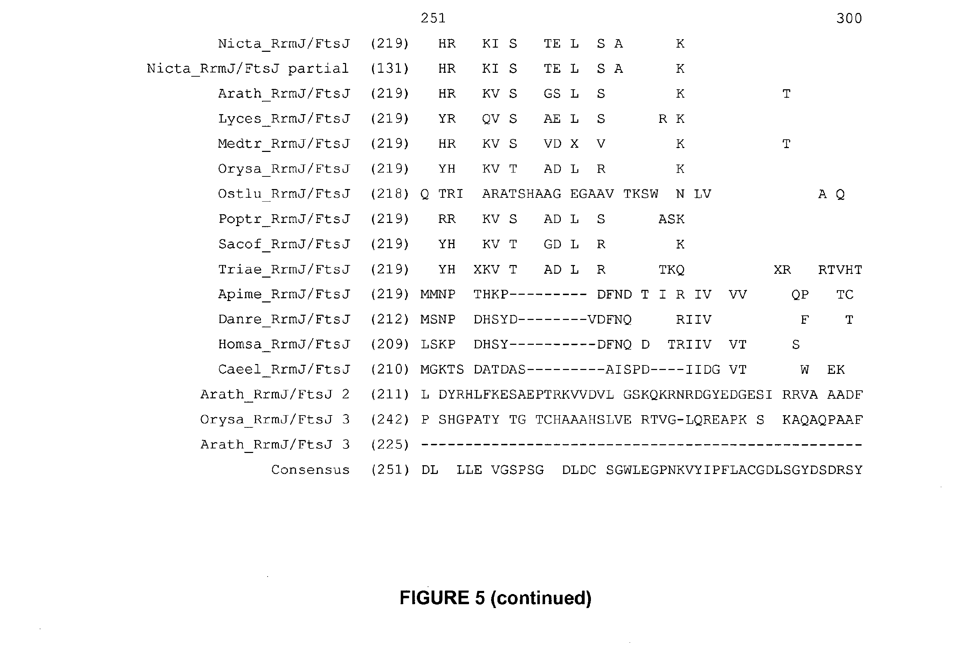

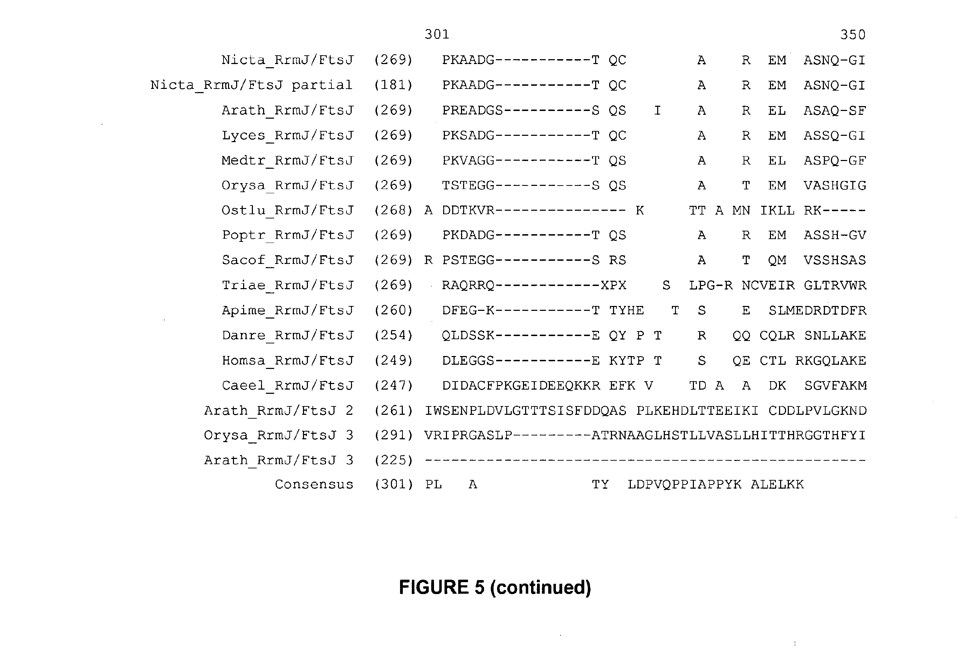

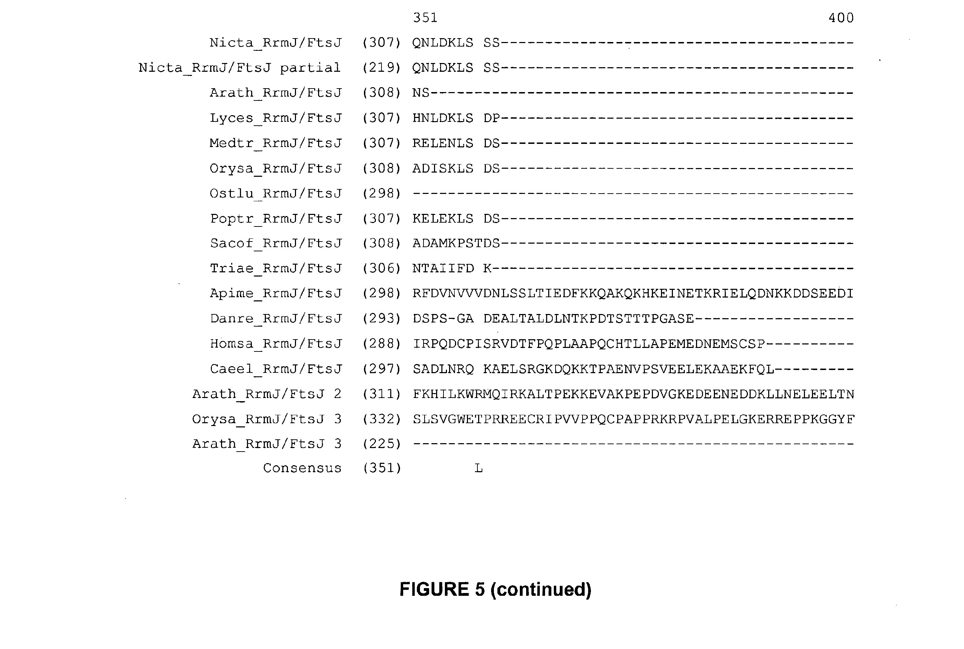

a) an isolated nucleic acid molecule encoding the polypeptide as depicted in SEQ ID NO: 97, 99, 101 and/or 103 and/or table A2b;
b) an isolated nucleic acid molecule as depicted in SEQ ID NO: 96, 98, 100 and/or 102 and/or table A2b;
c) an isolated nucleic acid molecule, which, as a result of the degeneracy of the genetic code, can be derived from a polypeptide sequence depicted in table A2 and confers enhanced yield-related traits relative to control plants;
d) an isolated nucleic acid molecule having at least 30 %, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity with the nucleic acid molecule sequence of a polynucleotide comprising the nucleic acid molecule depicted in table A2 and confers enhanced yield-related traits relative to control plants;
e) an isolated nucleic acid molecule encoding a polypeptide having at least 30 %, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity with the amino acid sequence of the polypeptide encoded by the nucleic acid molecule of (a) to (c) and confers enhanced yield-related traits relative to control plants;
f) an isolated nucleic acid molecule which hybridizes with a nucleic acid molecule of (a) to (c) under stringent hybridization conditions and confers enhanced yield-related traits relative to control plants;
g) an isolated nucleic acid molecule encoding a polypeptide comprising the consensus sequence or one or more polypeptide motifs as shown figure 5 and preferably having the activity represented by a nucleic acid molecule comprising a polynucleotide as depicted in table A2;
h) an isolated nucleic acid molecule encoding a polypeptide having the activity represented by a protein as depicted in table A2 and confering enhanced yield-related traits relative to control plants;
and
i) a nucleic acid molecule which is obtainable by screening a suitable nucleic acid library under stringent hybridization conditions with a probe comprising a complementary sequence of a nucleic acid molecule of (a) or (b) or with a fragment thereof, having at least 15 nt, preferably 20 nt, 30 nt, 50 nt, 100 nt, 200 nt or 500 nt of a nucleic acid molecule complementary to a nucleic acid molecule sequence characterized in (a) to (e) and encoding a polypeptide having the activity represented by a protein comprising a polypeptide as depicted in table A2;

whereby the nucleic acid molecule according to (a) to (i) is at least in one or more nucleotides different from the sequence depicted in table A2a and preferably which encodes a protein which differs at least in one or more amino acids from the protein sequences depicted in table A2a.

Item 31: Plant, part thereof (including seeds), or plant cell obtainable by a method according to any preceding item, wherein said plant, part thereof, or plant cell comprises a nucleic acid transgene encoding a GRP polypeptide, preferably according to item 30.

Tem 32: Construct comprising:

(i) nucleic acid sequence encoding a GRP polypeptide as defined in items 21 to 24 or of a polypeptide encoded by a nucleic acid molecule according to item 30;
(ii) one or more control sequences capable of driving expression of the nucleic acid sequence of (i); and optionally
(iii) a transcription termination sequence.

Item 33: Construct according to item 32, wherein one of said control sequences is a seed-specific promoter, preferably an oleosin promoter, most preferably a oleosin promoter from rice.

Item 34: Use of a construct according to any of litems 32 or 33 or of a nucleic acid molecule according to item 30 in a method for making plants having enhanced yield-related traits, particularly increased seed yield, increased number of filled seeds, increased seed fill rate, and increased harvest index, relative to control plants.

Item 35: Plant, plant part or plant cell transformed with a construct according to any of items 32 or 33.

Item 36: Method for the production of a transgenic plant having enhanced-yield related traits relative to control plants, comprising:

819 introducing and expressing in a plant, plant part, or plant cell, a nucleic acid sequence encoding a GRP polypeptide as defined in items 21 to 24 or of a polypeptide encoded by a nucleic acid molecule according to item 30; and
(ii) cultivating the plant, plant part, or plant cell under conditions promoting plant growth and development.

Item 37: Transgenic plant having enhanced yield-related traits, particularly increased seed yield, increased number of filled seeds, increased seed fill rate, and increased harvest index, relative to control plants, resulting from increased expression of a nucleic acid sequence encoding a GRP polypeptide as defined in items 21 to 24 or of a polypeptide encoded by a nucleic acid molecule according to item 30, or a transgenic plant cell derived from said transgenic plant.

Item 38: Transgenic plant according to item 31, 35 or 37, or a transgenic plant cell derived thereof, wherein said plant is a crop plant or a monocot or a cereal, such as rice, maize, wheat, barley, millet, rye, triticale, sorghum and oats.

Item 39: Harvestable parts of a plant comprising a nucleic acid sequence encoding a GRP polypeptide according to item 38, wherein said harvestable parts are preferably seeds.

Item 40: Products derived from a plant according to item 38 and/or from harvestable parts of a plant according to item 39.

Item 41: Use of a nucleic acid sequence encoding a GRP polypeptide in enhanced yield-related traits in plants, particularly in increasing particularly increased seed yield, increased number of filled seeds, increased seed fill rate, and increased harvest index, relative to control plants.

According to a further embodiment of the present invention, there is also provided an isolated polypeptide comprising a polypeptide sequence selected from the group consisting of:

(i) an amino acid sequence encoded by any one of the nucleic acids represented by Item 30;
(ii) an amino acid sequence having, in increasing order of preference, at least 50%, 55%, 60%, 65%, 70%, 75%, 80%, 85%, 90%, 95%, 96%, 97%, 98%, 99% or more sequence identity to the amino acid sequence of (i);
(iii) derivatives of any of the amino acid sequences given in (i) or (ii) above.

In an other embodiment of the invention it has now been found that modulating expression (preferably, increasing) in a plant of a nucleic acid sequence encoding a GRP polypeptide, wherein said GRP polypeptide is a basic-helix-loop-helix 4 (bHLH4) polypeptide, gives plants having enhanced yield-related traits, preferably enhanced seed yield-related traits, relative to control plants. According to a first embodiment, the present invention provides a method for enhancing yield-related traits, preferably enhanced seed yield-related traits, in plants relative to control plants, comprising modulating expression (preferably, increasing) in a plant of a nucleic acid sequence encoding a GRP polypeptide, wherein said GRP polypeptide is a basic-helix-loop-helix 4 (bHLH4) polypeptide.
A preferred method for modulating (preferably, increasing) expression of a nucleic acid sequence encoding a GRP polypeptide is by introducing and expressing in a plant a nucleic acid sequence encoding a GRP polypeptide.
Any reference hereinafter to a "protein useful in the methods of the invention" is taken to mean a GRP polypeptide as defined herein. Any reference hereinafter to a "nucleic acid sequence useful in the methods of the invention" is taken to mean a nucleic acid sequence capable of encoding such a GRP polypeptide. The nucleic acid sequence to be introduced into a plant (and therefore useful in performing the methods of the invention) is any nucleic acid sequence encoding the type of protein which will now be described, hereafter also named "GRP nucleic acid sequence" or "GRP gene".

In one embodiment of the invention a "GRP polypeptide" as defined herein refers the proteins represented by SEQ ID NO: 105, and to orthologues, paralogues, and homologues thereof. Table A3 of Example 1 herein presents such orthologues and paralogues.

Preferably, the orthologues, paralogues, and homologues of SEQ ID NO: 105 have a basic-helix-loop-helix dimerisation region with InterPro entry IPR001092, and helix-loop-helix DNA-binding with InterPro entry IPR011598.

Alternatively or additionally, a "GRP polypeptide" as defined herein refers to any polypeptide having in increasing order of preference at least 50%, 55%, 60%, 65%, 70%, 75%, 80%, 85%, 90%, 95%, 98%, 99% or more amino acid sequence identity to the GRP polypeptide as represented by SEQ ID NO: 105, or to any of the polypeptide sequences given in Table A3 of Example 1 herein.

The term "domain" and "motif" is defined in the "definitions" section herein. Specialist databases exist for the identification of domains, for example, SMART (Schultz et al. (1998) Proc. Natl. Acad. Sci. USA 95, 5857-5864; Letunic et al. (2002) Nucleic Acids Res 30, 242-244), InterPro (Mulder et al., (2003) Nucl. Acids. Res. 31, 315-318), Prosite (Bucher and Bairoch (1994), A generalized profile syntax for biomolecular sequences motifs and its function in automatic sequence interpretation. (In) ISMB-94; Proceedings 2nd International Conference on Intelligent Systems for Molecular Biology. Altman R., Brutlag D., Karp P., Lathrop R., Searls D., Eds., pp53-61, AAAI Press, Menlo Park; Hulo et al., Nucl. Acids. Res. 32:D134-D137, (2004)), or Pfam (Bateman et al., Nucleic Acids Research 30(1): 276-280 (2002)). A set of tools for *in silico* analysis of protein sequences is available on the ExPASy proteomics server (Swiss Institute of Bioinformatics (Gasteiger et al., ExPASy: the proteomics server for in-depth protein knowledge and analysis, Nucleic Acids Res. 31:3784-3788(2003)). Domains or motifs may also be identified using routine techniques, such as by sequence alignment.

Methods for the alignment of sequences for comparison are well known in the art, such methods include GAP, BESTFIT, BLAST, FASTA and TFASTA. GAP uses the algorithm of Needleman and Wunsch ((1970) J Mol Biol 48: 443-453) to find the global (i.e. spanning the complete sequences) alignment of two sequences that maximizes the number of matches and minimizes the number of gaps. The BLAST algorithm (Altschul et al. (1990) J Mol Biol 215: 403-10) calculates percent sequence identity and performs a statistical analysis of the similarity between the two sequences. The software for performing BLAST analysis is publicly available through the National Centre for Biotechnology Information (NCBI). Homologues may readily be identified using, for example, the ClustalW multiple sequence alignment algorithm (version 1.83), with the default pairwise alignment parameters, and a scoring method in percentage. Global percentages of similarity and identity may also be determined using one of the methods available in the MatGAT software package (Campanella et al., BMC Bioinformatics. 2003 Jul 10;4:29. MatGAT: an application that generates similarity/identity matrices using protein or DNA sequences). Minor manual editing may be performed to optimise alignment between conserved motifs, as would be apparent to a person skilled in the art. Furthermore, instead of using full-length sequences for the identification of homologues, specific domains may also be used. The sequence identity values may be determined over the entire nucleic acid sequence or polypeptide sequence or over selected domains or conserved motif(s), using the programs mentioned above using the default parameters.

Furthermore, GRP polypeptides, as far as SEQ ID NO: 105, and its orthologues, paralogues, and homologues are concerned, typically have the capability to bind DNA (at least in their native form), but not necessarily, have transcriptional regulatory activity and capacity to interact with other proteins. Therefore, GRP polypeptides with reduced transcriptional regulatory activity, without transcriptional regulatory activity, with reduced protein-protein interaction capacity, or with no protein-protein interaction capacity, may equally be useful in the methods of the present invention. DNA-binding activity and protein-protein interactions may readily be determined in vitro or in vivo using techniques well known in the art (for example in Current Protocols in Molecular Biology, Volumes 1 and 2, Ausubel et al. (1994), Current Protocols). To determine the DNA binding activity of GRP polypeptides, several assays are available, such as DNA binding gel-shift assays (or gel retardation assays; Korfhage et al. (1994) Plant C 6: 695-708), *in vitro* DNA binding assays (Schindler et al. (1993) Plant J 4(1): 137-150), or transcriptional activation of GRP polypeptides in yeast, animal and plant cells. GRP polypeptides typically bind to a consensus sequence called an E-box, CANNTG. The canonical E-box is CACGTG (palindromic), however some bHLH4 transcription factors bind to different sequences, which are often similar to the E-box. Specific DNA binding sequences can be determined using the random oligonucleotide selection technique (Viola & Gonzalez (May 26, 2007) Biochemistry).

The present invention is illustrated by transforming plants with the nucleic acid sequence represented by SEQ ID NO: 104, encoding the polypeptide sequence of SEQ ID NO: 105. However, performance of the invention is not restricted to these sequences; the methods of the invention may advantageously be performed using any GRP-encoding nucleic acid sequence or GRP polypeptide as defined herein.

Examples of nucleic acid sequences encoding GRP polypeptides may be found in databases known in the art.

Such nucleic acid sequences are useful in performing the methods of the invention. Orthologues and paralogues, the terms "orthologues" and "paralogues" being as defined herein, may readily be identified by performing a so-called reciprocal blast search. Typically, this involves a first BLAST involving BLASTing a query sequence (for example using SEQ ID NO: 105) against any sequence database, such as the publicly available NCBI database. BLASTN or TBLASTX (using standard default values) are generally used when starting from a nucleotide sequence, and BLASTP or TBLASTN (using standard default values) when starting from a protein sequence. The BLAST results may optionally be filtered. The full-length sequences of either the filtered results or non-filtered results are then BLASTed back (second BLAST) against sequences from the organism from which the query sequence is derived (where the query sequence is SEQ ID NO: 104 or SEQ ID NO: 105, the second BLAST would therefore be against *Arabidopsis thaliana* sequences). The results of the first and second BLASTs are then compared. A paralogue is identified if a high-ranking hit from the first blast is from the same species as from which the query sequence is derived, a BLAST back then ideally results in the query sequence amongst the highest hits; an orthologue is identified if a high-ranking hit in the first BLAST is not from the same species as from which the query sequence is derived, and preferably results upon BLAST back in the query sequence being among the highest hits.

High-ranking hits are those having a low E-value. The lower the E-value, the more significant the score (or in other words the lower the chance that the hit was found by chance). Computation of the E-value is well known in the art. In addition to E-values, comparisons are also scored by percentage identity. Percentage identity refers to the number of identical nucleotides (or amino acids) between the two compared nucleic acid sequence (or polypeptide) sequences over a particular length. In the case of large families, ClustalW may be used, followed by a neighbour joining tree, to help visualize clustering of related genes and to identify orthologues and paralogues.

Nucleic acid sequence variants encoding homologues and derivatives of SEQ ID NO: 105 may also be useful in practising the methods of the invention, the terms "homologue" and "derivative" being as defined herein. Also useful in the methods of the invention are nucleic acid sequences encoding homologues and derivatives of orthologues or paralogues of SEQ ID NO: 105. Homologues and derivatives useful in the methods of the present invention have substantially the same biological and functional activity as the unmodified protein from which they are derived.

Further nucleic acid sequence variants useful in practising the methods of the invention include portions of nucleic acid sequences encoding GRP polypeptides, nucleic acid sequences hybridising to nucleic acid sequences encoding GRP polypeptides, splice variants of nucleic acid sequences encoding GRP polypeptides, allelic variants of nucleic acid sequences encoding GRP polypeptides and variants of nucleic acid sequences encoding GRP polypeptides obtained by gene shuffling. The terms hybridising sequence, splice variant, allelic variant and gene shuffling are as described herein.

Nucleic acid sequences encoding GRP polypeptides need not be full-length nucleic acid sequences, since performance of the methods of the invention does not rely on the use of full-length nucleic acid sequences. According to the present invention, there is provided a method for enhancing yield-related traits in plants, preferably enhanced seed yield-related traits, comprising introducing and expressing in a plant a portion of SEQ ID NO: 104, or a portion of a nucleic acid sequence encoding an orthologue, paralogue or homologue of SEQ ID NO: 105.

A portion of a nucleic acid sequence may be prepared, for example, by making one or more deletions to the nucleic acid sequence. The portions may be used in isolated form or they may be fused to other coding (or non-coding) sequences in order to, for example, produce a protein that combines several activities. When fused to other coding sequences, the resultant polypeptide produced upon translation may be bigger than that predicted for the protein portion.

Portions useful in the methods of the invention, encode a GRP polypeptide as defined herein, and have substantially the same biological activity as the polypeptide sequence given in SEQ ID NO: 105. Preferably, the portion is a portion of the nucleic acid sequence given in SEQ ID NO: 104, or is a portion of a nucleic acid sequence encoding an orthologue or paralogue of the polypeptide sequence given in SEQ ID NO: 105. Preferably the portion is at least 500, 600, 700, 800, 850, 900, 950, 1000, 1050, 1100, 1150, 1200, 1250, 1300, 1350, 1400, 1450, 1500, 1550, 1600, 1650, 1700, 1750 or more consecutive nucleotides in length, the consecutive nucleotides being of SEQ ID NO: 104, or of a nucleic acid sequence encoding an orthologue or paralogue of SEQ ID NO: 105. Most preferably the portion is a portion of the nucleic acid sequence of SEQ ID NO: 104.

Another nucleic acid sequence variant useful in the methods of the invention is a nucleic acid sequence capable of hybridising, under reduced stringency conditions, preferably under stringent conditions, with a nucleic acid sequence encoding a GRP polypeptide as defined herein, or with a portion as defined herein.

According to the present invention, there is provided a method for enhancing yield-related traits in plants, preferably enhanced seed yield-related traits, comprising introducing and expressing in a plant a nucleic acid sequence capable of hybridizing to SEQ ID NO: 104, or comprising introducing and expressing in a plant a nucleic acid sequence capable of hybridising to a nucleic acid sequence encoding an orthologue, paralogue or homologue of SEQ ID NO: 105.

Hybridising sequences useful in the methods of the invention encode a GRP polypeptide as defined herein, having substantially the same biological activity as the polypeptide sequence given in SEQ ID NO: 105. Preferably, the hybridising sequence is capable of hybridising to SEQ ID NO: 104, or to a portion of this sequence, a portion being as defined above, or the hybridising sequence is capable of hybridising to a nucleic acid sequence encoding an orthologue or paralogue of SEQ ID NO: 105, or to a portion thereof.

Another nucleic acid sequence variant useful in the methods of the invention is a splice variant encoding a GRP polypeptide as defined hereinabove, a splice variant being as defined herein.

According to the present invention, there is provided a method for enhancing yield-related traits in plants, preferably enhancing seed yield-related traits, comprising introducing and expressing in a plant a splice variant of SEQ ID NO: 104, or a splice variant of a nucleic acid sequence encoding an orthologue, paralogue or homologue of SEQ ID NO: 105.

Another nucleic acid sequence variant useful in performing the methods of the invention is an allelic variant of a nucleic acid sequence encoding a GRP polypeptide as defined hereinabove, an allelic variant being as defined herein.

According to the present invention, there is provided a method for enhancing yield-related traits in plants, preferably enhanced seed yield-related traits, comprising introducing and expressing in a plant an allelic variant of SEQ ID NO: 104, or comprising introducing and expressing in a plant an allelic variant of a nucleic acid sequence encoding an orthologue, paralogue or homologue of the polypeptide sequence represented by SEQ ID NO: 105.

The allelic variants useful in the methods of the present invention have substantially the same biological activity as the GRP polypeptide of SEQ ID NO: 105. Allelic variants exist in nature, and encompassed within the methods of the present invention is the use of these natural alleles. Gene shuffling or directed evolution may also be used to generate variants of nucleic acid sequences encoding GRP polypeptides as defined above; the term "gene shuffling" being as defined herein.

According to the present invention, there is provided a method for enhancing yield-related traits in plants, preferably enhanced seed yield-related traits, comprising introducing and expressing in a plant a variant nucleic acid sequence of SEQ ID NO: 104, or comprising introducing and expressing in a plant a variant of a nucleic acid sequence encoding an orthologue, paralogue or homologue of SEQ ID NO: 105, which variant nucleic acid sequence is obtained by gene shuffling.

Furthermore, nucleic acid sequence variants may also be obtained by site-directed mutagenesis. Several methods are available to achieve site-directed mutagenesis, the most common being PCR based methods (Current Protocols in Molecular Biology. Wiley Eds.).

Nucleic acid sequences encoding GRP polypeptides may be derived from any natural or artificial source. The nucleic acid sequence may be modified from its native form in composition and/or genomic environment through deliberate human manipulation. Preferably the GRP polypeptide-encoding nucleic acid sequence is from a plant. In the case of SEQ ID NO: 104, the GRP polypeptide encoding nucleic acid sequence is preferably from a dicotyledonous plant, more preferably from the family Brassicaceae, most preferably the nucleic acid sequence is from *Arabidopsis thaliana.*

Performance of the methods of the invention gives plants having enhanced yield-related traits, in particular enhanced seed yield-related traits. The terms "yield" and "seed yield" are described in more detail in the "definitions" section herein.

Reference herein to enhanced yield-related traits is taken to mean an increase in early vigour and/or in biomass (weight) of one or more parts of a plant, which may include aboveground (harvestable) parts and/or (harvestable) parts below ground. In particular, such harvestable parts are biomass and/or seeds, and performance of the methods of the invention results in plants having increased early vigour, biomass and/or seed yield relative to the early vigour, biomass or seed yield of control plants.

Taking corn as an example, a yield increase may be manifested as one or more of the following: increase in the number of plants established per hectare or acre, an increase in the number of ears per plant, an increase in the number of rows, number of kernels per row, kernel weight, thousand kernel weight, ear length/diameter, increase in the seed filling rate (which is the number of filled seeds divided by the total number of seeds and multiplied by 100), among others. Taking rice as an example, a yield increase may manifest itself as an increase in one or more of the following: number of plants per hectare or acre, number of panicles per plant, number of spikelets per panicle, number of flowers (florets) per panicle (which is expressed as a ratio of the number of filled seeds over the number of primary panicles), increase in the seed filling rate (which is the number of filled seeds divided by the total number of seeds and multiplied by 100), increase in thousand kernel weight, among others.

The present invention provides a method for enhancing yield-related traits, especially biomass and/or seed yield of plants, relative to control plants, which method comprises modulating expression (preferably, increasing) in a plant of a nucleic acid sequence encoding a GRP polypeptide as defined herein.

Since the transgenic plants according to the present invention have enhanced yield-related traits, preferably enhanced seed yield-related traits, it is likely that these plants exhibit an increased growth rate (during at least part of their life cycle), relative to the growth rate of control plants at a corresponding stage in their life cycle.

The increased growth rate may be specific to one or more parts of a plant (including seeds), or may be throughout substantially the whole plant. Plants having an increased growth rate may have a shorter life cycle. The life cycle of a plant may be taken to mean the time needed to grow from a dry mature seed up to the stage where the plant has produced dry mature seeds, similar to the starting material. This life cycle may be influenced by factors such as early vigour, growth rate, greenness index, flowering time and speed of seed maturation. The increase in growth rate may take place at one or more stages in the life cycle of a plant or during substantially the whole plant life cycle. Increased growth rate during the early stages in the life cycle of a plant may reflect enhanced vigour. The increase in growth rate may alter the harvest cycle of a plant allowing plants to be sown later and/or harvested sooner than would otherwise be possible (a similar effect may be obtained with earlier flowering time). If the growth rate is sufficiently increased, it may allow for the further sowing of seeds of the same plant species (for example sowing and harvesting of rice plants followed by sowing and harvesting of further rice plants all within one conventional growing period). Similarly, if the growth rate is sufficiently increased, it may allow for the further sowing of seeds of different plants species (for example the sowing and harvesting of corn plants followed by, for example, the sowing and optional harvesting of soybean, potato or any other suitable plant). Harvesting additional times from the same rootstock in the case of some crop plants may also be possible. Altering the harvest cycle of a plant may lead to an increase in annual biomass production per acre (due to an increase in the number of times (say in a year) that any particular plant may be grown and harvested). An increase in growth rate may also allow for the cultivation of transgenic plants in a wider geographical area than their wild-type counterparts, since the territorial limitations for growing a crop are often determined by adverse environmental conditions either at the time of planting (early season) or at the time of harvesting (late season). Such adverse conditions may be avoided if the harvest cycle is shortened. The growth rate may be determined by deriving various parameters from growth curves, such parameters may be: T-Mid (the time taken for plants to reach 50% of their maximal size) and T-90 (time taken for plants to reach 90% of their maximal size), amongst others.

According to a preferred feature of the present invention, performance of the methods of the invention gives plants having an increased growth rate relative to control plants. Therefore, according to the present invention, there is provided a method for increasing the growth rate of plants, which method comprises modulating expression (preferably, increasing) in a plant of a nucleic acid sequence encoding a GRP polypeptide as defined herein.

An enhancement of yield-related traits (an increase in seed yield and/or growth rate) occurs whether the plant is under non-stress conditions or whether the plant is exposed to various stresses compared to control plants. Plants typically respond to exposure to stress by growing more slowly. In conditions of severe stress, the plant may even stop growing altogether. Mild stress on the other hand is defined herein as being any stress to which a plant is exposed which does not result in the plant ceasing to grow altogether without the capacity to resume growth. Mild stress in the sense of the invention leads to a reduction in the growth of the stressed plants of less than 40%, 35% or 30%, preferably less than 25%, 20% or 15%, more preferably less than 14%, 13%, 12%, 11 % or 10% or less in comparison to the control plant under non-stress conditions. Due to advances in agricultural practices (irrigation, fertilization, pesticide treatments) severe stresses are not often encountered in cultivated crop plants. As a consequence, the compromised growth induced by mild stress is often an undesirable feature for agriculture. Mild stresses are the everyday biotic and/or abiotic (environmental) stresses to which a plant is exposed. Abiotic stresses may be due to drought or excess water, anaerobic stress, salt stress, chemical toxicity, oxidative stress and hot, cold or freezing temperatures. The abiotic stress may be an osmotic stress caused by a water stress (particularly due to drought), salt stress, oxidative stress or an ionic stress. Biotic stresses are typically those stresses caused by pathogens, such as bacteria, viruses, nematodes, fungi and insects. The term "non-stress" conditions as used herein are those environmental conditions that allow optimal growth of plants. Persons skilled in the art are aware of normal soil conditions and climatic conditions for a given location.

Performance of the methods of the invention gives plants grown under non-stress conditions or under mild stress conditions having enhanced yield-related traits, preferably enhanced seed yield-related traits, relative to control plants grown under comparable conditions. Therefore, according to the present invention, there is provided a method for enhancing yield-related traits, preferably enhanced seed yield-related traits, in plants grown under non-stress conditions or under mild stress conditions, which method comprises modulating (preferably, increasing) expression in a plant of a nucleic acid sequence encoding a GRP polypeptide.

Performance of the methods according to the present invention results in plants grown under abiotic stress conditions having enhanced yield-related traits, preferably enhanced seed yield-related traits, relative to control plants grown under comparable stress conditions. As reported in Wang et al. (Planta (2003) 218: 1-14), abiotic stress leads to a series of morphological, physiological, biochemical and molecular changes that adversely affect plant growth and productivity. Drought, salinity, extreme temperatures and oxidative stress are known to be interconnected and may induce growth and cellular damage through similar mechanisms. Rabbani et al. (Plant Physiol (2003) 133: 1755-1767) describes a particularly high degree of "cross talk" between drought stress and high-salinity stress. For example, drought and/or salinisation are manifested primarily as osmotic stress, resulting in the disruption of homeostasis and ion distribution in the cell. Oxidative stress, which frequently accompanies high or low temperature, salinity or drought stress, may cause denaturing of functional and structural proteins. As a consequence, these diverse environmental stresses often activate similar cell signalling pathways and cellular responses, such as the production of stress proteins, up-regulation of anti-oxidants, accumulation of compatible solutes and growth arrest. Since diverse environmental stresses activate similar pathways, plants having enhanced yield-related traits, preferably enhanced seed yield-related traits, under one type of abiotic stress growth conditions, relative to control plants grown under comparable abiotic stress conditions, should not be seen as a limitation to that type of abiotic stress growth conditions, but more as a screen to indicate the involvement of GRP polypeptides as defined above, in enhancing yield-related traits in abiotic stress growth conditions in general.

The term "abiotic stress" as defined herein is taken to mean any one or more of: water stress (due to drought or excess water), anaerobic stress, salt stress, temperature stress (due to hot, cold or freezing temperatures), chemical toxicity stress and oxidative stress. According to one aspect of the invention, the abiotic stress is an osmotic stress, selected from water stress, salt stress, oxidative stress and ionic stress. Preferably, the water stress is drought stress. The term salt stress is not restricted to common salt (NaCl), but may be any stress caused by one or more of: NaCl, KCl, LiCl, MgCl2, CaCl2, amongst others.

Performance of the methods of the invention gives plants having enhanced yield-related traits, preferably enhanced seed yield-related traits, under abiotic stress growth conditions relative to control plants grown in comparable stress conditions. Therefore, according to the present invention, there is provided a method for enhancing yield-related traits, preferably enhancing seed yield-related traits, in plants grown under abiotic stress growth conditions, which method comprises modulating (preferably, increasing) expression in a plant of a nucleic acid sequence encoding a GRP polypeptide. According to one aspect of the invention, the abiotic stress is an osmotic stress, selected from one or more of the following: water stress, salt stress, oxidative stress and ionic stress.

Performance of the methods of the invention gives plants grown under conditions of reduced nutrient availability, particularly under conditions of reduced nitrogen availability, enhanced yield-related traits, preferably enhancing seed yield-related traits, relative to control plants grown under comparable conditions. Therefore, according to the present invention, there is provided a method for enhancing yield-related traits in plants grown under conditions of reduced nutrient availability, which method comprises modulating (preferably, increasing) expression in a plant of a nucleic acid sequence encoding a GRP polypeptide. Reduced nutrient availability may comprise reduced availability of nutrients such as nitrogen, phosphates and other phosphorous-containing compounds, potassium, calcium, cadmium, magnesium, manganese, iron and boron, amongst others.

The present invention encompasses plants, parts thereof (including seeds), or plant cells obtainable by the methods according to the present invention. The plants, parts thereof, or plant cells comprise a nucleic acid transgene encoding a GRP polypeptide as defined above, preferably a basic-helix-loop-helix 4 (bHLH4) polypeptide.

The invention also provides genetic constructs and vectors to facilitate introduction and/or expression in plants of nucleic acid sequences encoding GRP polypeptides. The gene constructs may be inserted into vectors, which may be commercially available, suitable for transforming into plants and suitable for expression of the gene of interest in the transformed cells. The invention also provides use of a gene construct as defined herein in the methods of the invention.

More specifically, the present invention provides a construct comprising:

(a) a nucleic acid sequence encoding a GRP polypeptide, preferably a basic-helix-loop-helix 4 (bHLH4) polypeptide as defined above;
(b) one or more control sequences capable of driving expression of the nucleic acid sequence of (a); and optionally
(c) a transcription termination sequence.

Preferably, the nucleic acid sequence encoding a GRP polypeptide is as defined above. The term "control sequence" and "termination sequence" are as defined herein.

Plants are transformed with a vector comprising any of the nucleic acid sequences described above. The skilled artisan is well aware of the genetic elements that must be present on the vector in order to successfully transform, select and propagate host cells containing the sequence of interest. The sequence of interest is operably linked to one or more control sequences (at least to a promoter).

Advantageously, any type of promoter, whether natural or synthetic, may be used to drive expression of the nucleic acid sequence. A constitutive promoter is particularly useful in the methods of the invention. See the "Definitions" section herein for definitions of the various promoter types.

It should be clear that the applicability of the present invention is not restricted to the GRP polypeptide-encoding nucleic acid sequence represented by SEQ ID NO: 104, nor is the applicability of the invention restricted to expression of a GRP polypeptide-encoding nucleic acid sequence when driven by a constitutive promoter.

The constitutive promoter is preferably a high mobility group B (HMGB) promoter, preferably an HMGB promoter from rice. Further preferably the HMGB promoter is represented by a nucleic acid sequence substantially similar to SEQ ID NO: 106, most preferably the HMGB promoter is as represented by SEQ ID NO: 106. See Table 2 in the "Definitions" section herein for further examples of constitutive promoters.

Optionally, one or more terminator sequences may be used in the construct introduced into a plant. Additional regulatory elements may include transcriptional as well as translational enhancers. Those skilled in the art will be aware of terminator and enhancer sequences that may be suitable for use in performing the invention. An intron sequence may also be added to the 5' untranslated region (UTR) or in the coding sequence to increase the amount of the mature message that accumulates in the cytosol, as described in the definitions section. Other control sequences (besides promoter, enhancer, silencer, intron sequences, 3'UTR and/or 5'UTR regions) may be protein and/or RNA stabilizing elements. Such sequences would be known or may readily be obtained by a person skilled in the art.

The genetic constructs of the invention may further include an origin of replication sequence that is required for maintenance and/or replication in a specific cell type. One example is when a genetic construct is required to be maintained in a bacterial cell as an episomal genetic element (e.g. plasmid or cosmid molecule). Preferred origins of replication include, but are not limited to, the f1-ori and colE1.

For the detection of the successful transfer of the nucleic acid sequences as used in the methods of the invention and/or selection of transgenic plants comprising these nucleic acid sequences, it is advantageous to use marker genes (or reporter genes). Therefore, the genetic construct may optionally comprise a selectable marker gene. Selectable markers are described in more detail in the "definitions" section herein. The marker genes may be removed or excised from the transgenic cell once they are no longer needed. Techniques for marker removal are known in the art, useful techniques are described above in the definitions section.

The invention also provides a method for the production of transgenic plants having enhanced yield-related traits, preferably enhanced seed yield-related traits, relative to control plants, comprising introduction and expression in a plant of a nucleic acid sequence encoding a GRP polypeptide as defined hereinabove.

More specifically, the present invention provides a method for the production of transgenic plants having enhanced yield-related traits, preferably enhanced seed yield-related traits, which method comprises:

(i) introducing and expressing in a plant, plant part, or plant cell, a nucleic acid sequence encoding a GRP polypeptide; and

(ii) cultivating the plant, plant part or plant cell under conditions promoting plant growth and development.

The nucleic acid sequence of (i) may be any of the nucleic acid sequences capable of encoding a GRP polypeptide as defined herein, in one embodiment having the activity of a basic-helix-loop-helix 4 (bHLH4) polypeptide.

The nucleic acid sequence may be introduced directly into a plant cell or into the plant itself (including introduction into a tissue, organ or any other part of a plant). According to a preferred feature of the present invention, the nucleic acid sequence is preferably introduced into a plant by transformation. The term "transformation" is described in more detail in the "definitions" section herein.

The genetically modified plant cells can be regenerated via all methods with which the skilled worker is familiar. Suitable methods can be found in the abovementioned publications by S.D. Kung and R. Wu, Potrykus or Höfgen and Willmitzer.

Generally after transformation, plant cells or cell groupings are selected for the presence of one or more markers which are encoded by plant-expressible genes co-transferred with the gene of interest, following which the transformed material is regenerated into a whole plant. To select transformed plants, the plant material obtained in the transformation is, as a rule, subjected to selective conditions so that transformed plants can be distinguished from untransformed plants. For example, the seeds obtained in the above-described manner can be planted and, after an initial growing period, subjected to a suitable selection by spraying. A further possibility consists in growing the seeds, if appropriate after sterilization, on agar plates using a suitable selection agent so that only the transformed seeds can grow into plants. Alternatively, the transformed plants are screened for the presence of a selectable marker such as the ones described above.

Following DNA transfer and regeneration, putatively transformed plants may also be evaluated, for instance using Southern analysis, for the presence of the gene of interest, copy number and/or genomic organisation. Alternatively or additionally, expression levels of the newly introduced DNA may be monitored using Northern and/or Western analysis, both techniques being well known to persons having ordinary skill in the art.

The generated transformed plants may be propagated by a variety of means, such as by clonal propagation or classical breeding techniques. For example, a first generation (or T1) transformed plant may be selfed and homozygous second-generation (or T2) transformants selected, and the T2 plants may then further be propagated through classical breeding techniques. The generated transformed organisms may take a variety of forms. For example, they may be chimeras of transformed cells and non-transformed cells; clonal transformants (e.g., all cells transformed to contain the expression cassette); grafts of transformed and untransformed tissues (e.g., in plants, a transformed rootstock grafted to an untransformed scion).

The present invention clearly extends to any plant cell or plant produced by any of the methods described herein, and to all plant parts and propagules thereof. The present invention extends further to encompass the progeny of a primary transformed or transfected cell, tissue, organ or whole plant that has been produced by any of the aforementioned methods, the only requirement being that progeny exhibit the same genotypic and/or phenotypic characteristic (s) as those produced by the parent in the methods according to the invention.

The invention also includes host cells containing an isolated nucleic acid sequence encoding a GRP polypeptide as defined hereinabove. Preferred host cells according to the invention are plant cells. Host plants for the nucleic acid transgene or the vector used in the method according to the invention, the expression cassette or construct or vector are, in principle, advantageously all plants, which are capable of synthesizing the polypeptides used in the inventive method.

The methods of the invention are advantageously applicable to any plant. Plants that are particularly useful in the methods of the invention include all plants which belong to the superfamily Viridiplantae, in particular monocotyledonous and dicotyledonous plants including fodder or forage legumes, ornamental plants, food crops, trees or shrubs. According to a preferred embodiment of the present invention, the plant is a crop plant. Examples of crop plants include soybean, sunflower, canola, alfalfa, rapeseed, cotton, tomato, potato and tobacco. Further preferably, the plant is a monocotyledonous plant. Examples of monocotyledonous plants include sugarcane. More preferably the plant is a cereal. Examples of cereals include rice, maize, wheat, barley, millet, rye, triticale, sorghum and oats.

The invention also extends to harvestable parts of a plant such as, but not limited to seeds, leaves, fruits, flowers, stems, roots, rhizomes, tubers and bulbs. The invention furthermore relates to products derived, preferably directly derived, from a harvestable part of such a plant, such as dry pellets or powders, oil, fat and fatty acids, starch or proteins.

According to a preferred feature of the invention, the modulated expression is increased expression. Methods for increasing expression of nucleic acid sequences or genes, or gene products, are well documented in the art and examples are provided in the definitions section.

As mentioned above, a preferred method for modulating (preferably, increasing) expression of a nucleic acid sequence encoding a GRP polypeptide is by introducing and expressing in a plant a nucleic acid sequence encoding a GRP polypeptide; however the effects of performing the method, i.e. enhancing yield-related traits, preferably enhancing seed yield-related traits, may also be achieved using other well known techniques, including but not limited to T-DNA activation tagging, TILLING, homologous recombination. A description of these techniques is provided in the definitions section.

The present invention also encompasses use of nucleic acid sequences encoding GRP polypeptides as described herein and use of these GRP polypeptides in enhancing any of the aforementioned yield-related traits, preferably seed yield-related traits, in plants.

Nucleic acid sequences encoding GRP polypeptide described herein, or the GRP polypeptides themselves, may find use in breeding programmes in which a DNA marker is identified which may be genetically linked to a GRP polypeptide-encoding gene. The genes/nucleic acid sequences, or the GRP polypeptides themselves may be used to define a molecular marker. This DNA or protein marker may then be used in breeding programmes to select plants having enhanced yield-related traits, preferably enhanced seed yield-related traits, as defined hereinabove in the methods of the invention.

Allelic variants of a GRP polypeptide-encoding nucleic acid sequence/gene may also find use in marker-assisted breeding programmes. Such breeding programmes sometimes require introduction of allelic variation by mutagenic treatment of the plants, using for example EMS mutagenesis; alternatively, the programme may start with a collection of allelic variants of so called "natural" origin caused unintentionally. Identification of allelic variants then takes place, for example, by PCR. This is followed by a step for selection of superior allelic variants of the sequence in question and which give enhanced yield-related traits, preferably enhanced seed yield-related traits. Selection is typically carried out by monitoring growth performance of plants containing different allelic variants of the sequence in question. Growth performance may be monitored in a greenhouse or in the field. Further optional steps include crossing plants in which the superior allelic variant was identified with another plant. This could be used, for example, to make a combination of interesting phenotypic features.

Nucleic acid sequences encoding GRP polypeptides may also be used as probes for genetically and physically mapping the genes that they are a part of, and as markers for traits linked to those genes. Such information may be useful in plant breeding in order to develop lines with desired phenotypes. Such use of GRP polypeptide-encoding nucleic acid sequences requires only a nucleic acid sequence of at least 15 nucleotides in length. The GRP polypeptide-encoding nucleic acid sequences may be used as restriction fragment length polymorphism (RFLP) markers. Southern blots (Sambrook J, Fritsch EF and Maniatis T (1989) Molecular Cloning, A Laboratory Manual) of restriction-digested plant genomic DNA may be probed with the GRP-encoding nucleic acid sequences. The resulting banding patterns may then be subjected to genetic analyses using computer programs such as MapMaker (Lander et al. (1987) Genomics 1: 174-181) in order to construct a genetic map. In addition, the nucleic acid sequences may be used to probe Southern blots containing restriction endonuclease-treated genomic DNAs of a set of individuals representing parent and progeny of a defined genetic cross. Segregation of the DNA polymorphisms is noted and used to calculate the position of the GRP polypeptide-encoding nucleic acid sequence in the genetic map previously obtained using this population (Botstein et al. (1980) Am. J. Hum. Genet. 32:314-331).

The production and use of plant gene-derived probes for use in genetic mapping is described in Bernatzky and Tanksley (1986) Plant Mol. Biol. Reporter 4: 37-41. Numerous publications describe genetic mapping of specific cDNA clones using the methodology outlined above or variations thereof. For example, F2 intercross populations, backcross populations, randomly mated populations, near isogenic lines, and other sets of individuals may be used for mapping. Such methodologies are well known to those skilled in the art.

The nucleic acid sequence probes may also be used for physical mapping (i.e., placement of sequences on physical maps; see Hoheisel et al. In: Non-mammalian Genomic Analysis: A Practical Guide, Academic press 1996, pp. 319-346, and references cited therein).

In another embodiment, the nucleic acid sequence probes may be used in direct fluorescence in situ hybridisation (FISH) mapping (Trask (1991) Trends Genet. 7:149-154). Although current methods of FISH mapping favour use of large clones (several kb to several hundred kb; see Laan et al. (1995) Genome Res. 5:13-20), improvements in sensitivity may allow performance of FISH mapping using shorter probes.

A variety of nucleic acid sequence amplification-based methods for genetic and physical mapping may be carried out using the nucleic acid sequences. Examples include allele-specific amplification (Kazazian (1989) J. Lab. Clin. Med 11:95-96), polymorphism of PCR-amplified fragments (CAPS; Sheffield et al. (1993) Genomics 16:325-332), allele-specific ligation (Landegren et al. (1988) Science 241:1077-1080), nucleotide extension reactions (Sokolov (1990) Nucleic Acids Res. 18:3671), Radiation Hybrid Mapping (Walter et al. (1997) Nat. Genet. 7:22-28) and Happy Mapping (Dear and Cook (1989) Nucleic Acids Res. 17:6795-6807). For these methods, the sequence of a nucleic acid sequence is used to design and produce primer pairs for use in the amplification reaction or in primer extension reactions. The design of such primers is well known to those skilled in the art. In methods employing PCR-based genetic mapping, it may be necessary to identify DNA sequence differences between the parents of the mapping cross in the region corresponding to the instant nucleic acid sequence. This, however, is generally not necessary for mapping methods.

The methods according to the present invention result in plants having enhanced yield-related traits, preferably enhanced seed yield-related traits, as described hereinbefore. These traits may also be combined with other economically advantageous traits, such as further yield-enhancing traits, tolerance to other abiotic and biotic stresses, traits modifying various architectural features and/or biochemical and/or physiological features.

The term "table A3" used in this specification is to be taken to specify the content of table A3a and table A3b. The term "table A3a" used in this specification is to be taken to specify the content of table A3a. The term "table A3b" used in this specification is to be taken to specify the content of table A3b. In one preferred embodiment, the term "table A3" means table A3b.

In one embodiment the invention relates to subject mater summarized as follows:

Item 42: A method for enhancing yield-related traits, preferably enhancing seed yield-related traits, in plants relative to control plants, comprising modulating expression (preferably, increasing) in a plant of a nucleic acid sequence encoding a GRP polypeptide, wherein said GRP polypeptide is a basic-helix-loop-helix 4 (bHLH4) polypeptide as represented by SEQ ID NO: 2 or any of the polypeptides given in table A3 or an orthologue, paralogue, or homologue thereof , and optionally selecting for plants having enhanced yield-related traits, preferably enhanced seed yield-related traits.

Item 43: A method according to item 42, wherein said GRP polypeptide as represented by SEQ ID NO: 105 and an orthologue, paralogue, or homologue thereof, have a basic-helix-loop-helix dimerisation region with InterPro entry IPR001092, and helix-loop-helix DNA-binding with InterPro entry IPR011598.

Item 44: Method according to item 42 or 43, wherein said GRP polypeptide has in increasing order of preference at least 50%, 55%, 60%, 65%, 70%, 75%, 80%, 85%, 90%, 95%, 98%, 99% or more amino acid sequence identity to the GRP polypeptide as represented by SEQ ID NO: 105 or to any of the polypeptide sequences given in Table A3 of Example 1.

Item 45: Method according to any preceding item 42 to 44, wherein said nucleic acid sequence encoding a GRP polypeptide is represented by the nucleic acid sequence of SEQ ID NO: 104 or a portion thereof, or a sequence capable of hybridising with the nucleic acid sequence of SEQ ID NO: 104 or a portion thereof or a sequence capable of hybridising with any one of the nucleic acid sequences SEQ ID NOs given in Table A3 of Example 1.

Item 46: Method according to any preceding item 42 to 45 wherein said modulated expression (preferably, increased) is effected by introducing and expressing in a plant a nucleic acid sequence encoding said GRP polypeptide.

Item 47: Method according to any preceding item 42 to 46, wherein said enhanced yield-related traits comprise one or more of: increased early vigour, increased greenness index, increased total seed yield per plant, increased number of filled seeds, increased seed fill rate, and increased harvest index, relative to control plants.

Item 48: Method according to any preceding item 42 to 47, wherein said modulated expression is increased expression.

Item 49: Method according to any preceding item 42 to 48, wherein said nucleic acid sequence is operably linked to a constitutive promoter, preferably to a high mobility group B (HMGB) promoter, most preferably to an HMGB promoter from rice.

Item 50: Method according to any preceding item 42 to 49, wherein said nucleic acid sequence encoding a GRP polypeptide is of plant origin, preferably from a dicotyledonous plant, further preferably from the family Brassicaceae, more preferably from *Arabidopsis thaliana.*

Item 51: Plant, part thereof (including seeds), or plant cell obtainable by a method according to any preceding item 42 to 45, wherein said plant, part thereof, or plant cell comprises a nucleic acid transgene encoding a GRP polypeptide.

Item 52: An isolated nucleic acid sequence comprising a nucleic acid sequence selected from the group consisting of:

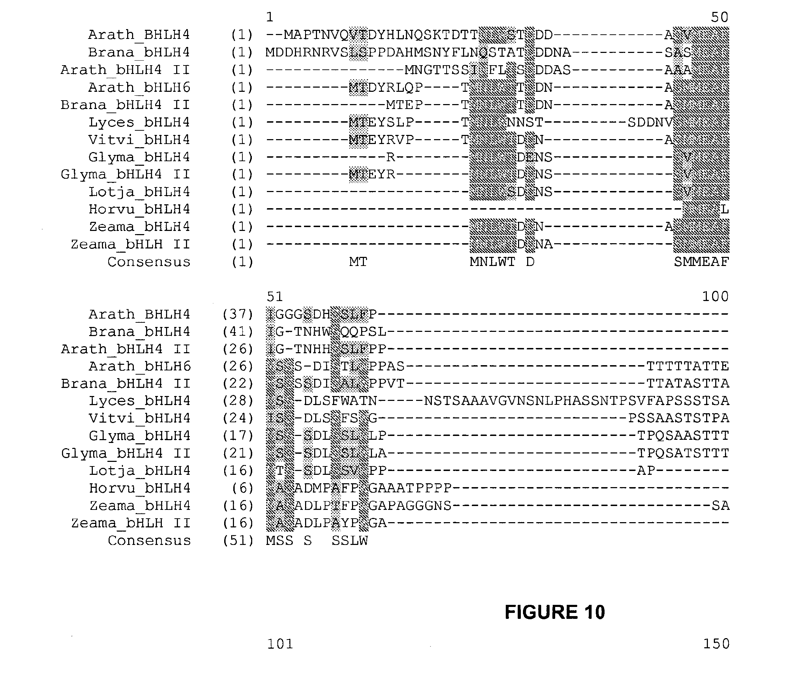

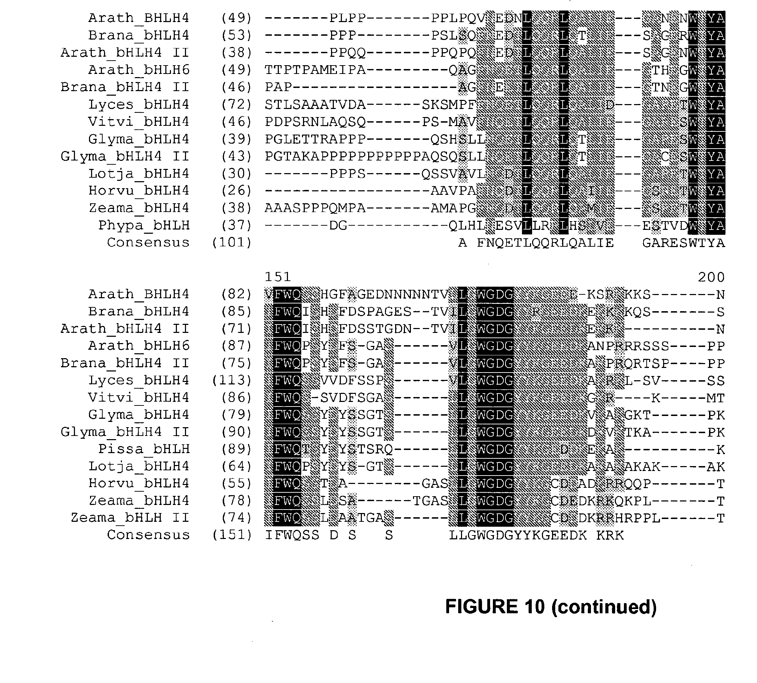

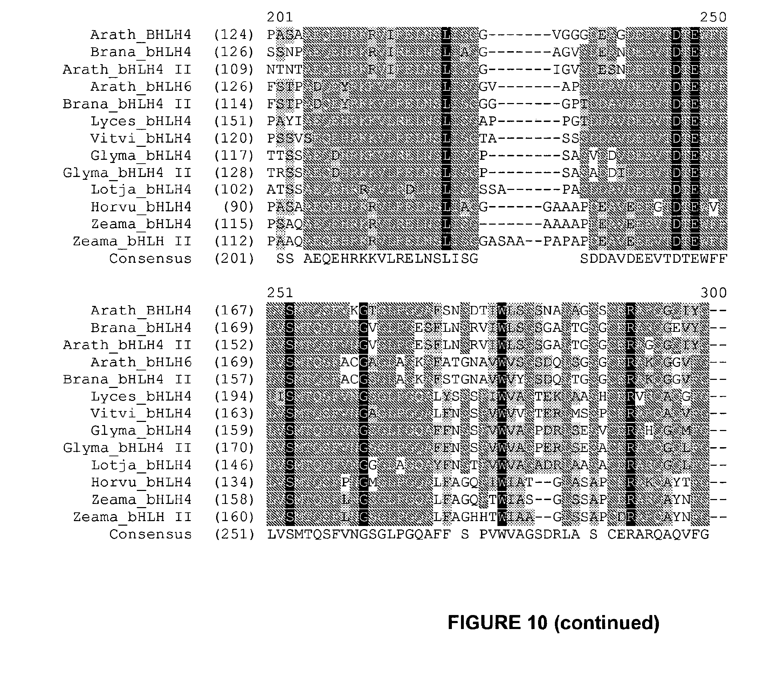

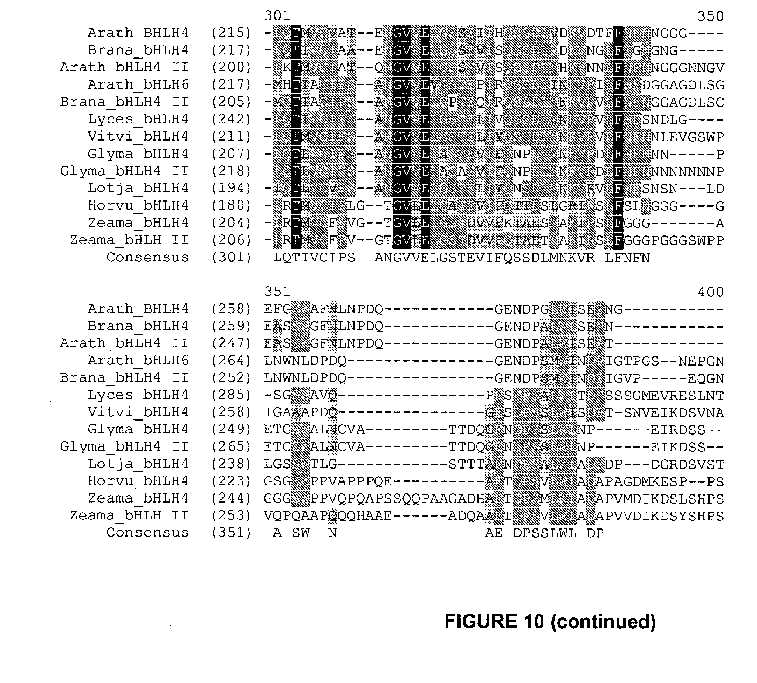

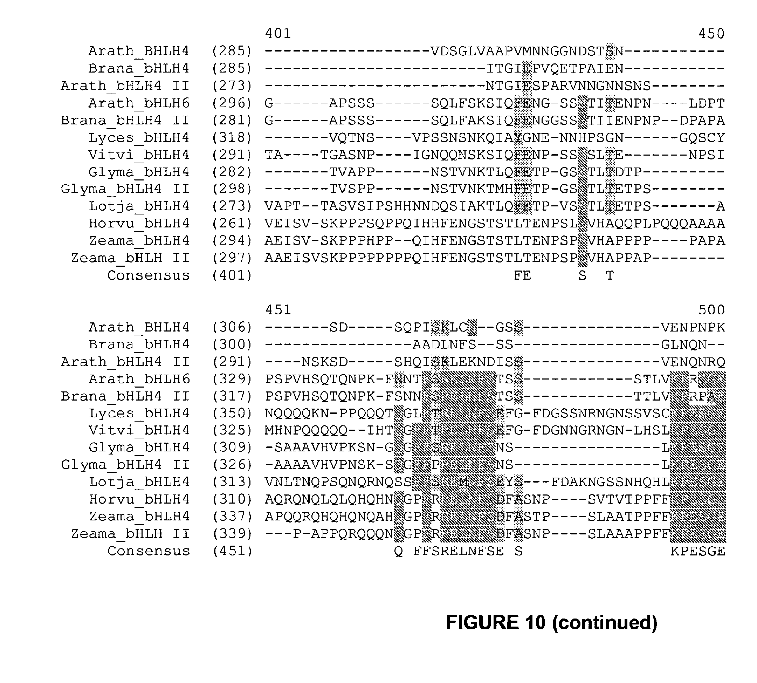

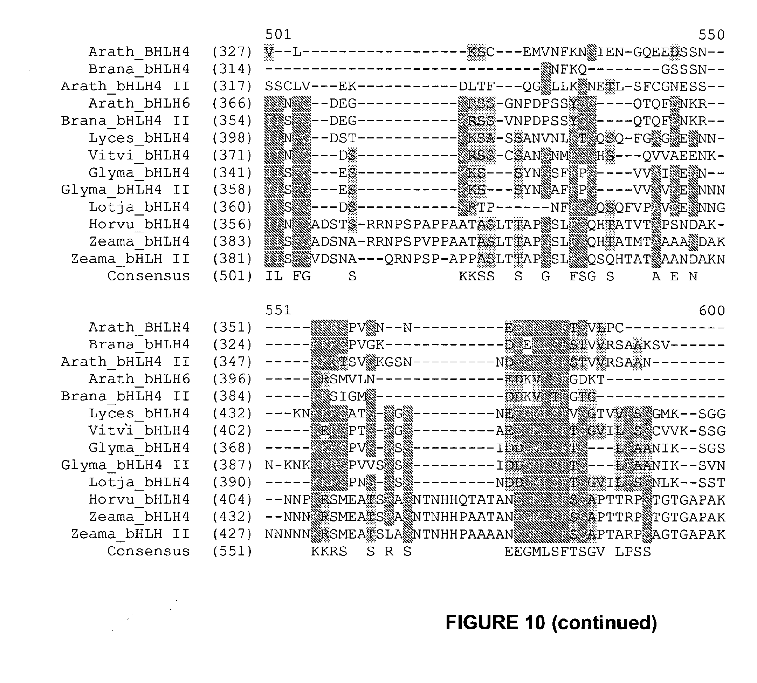

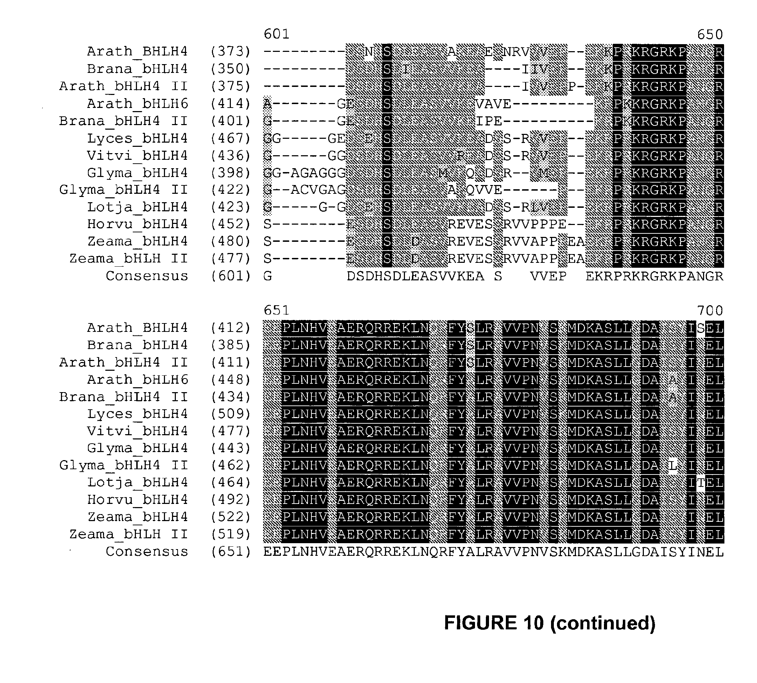

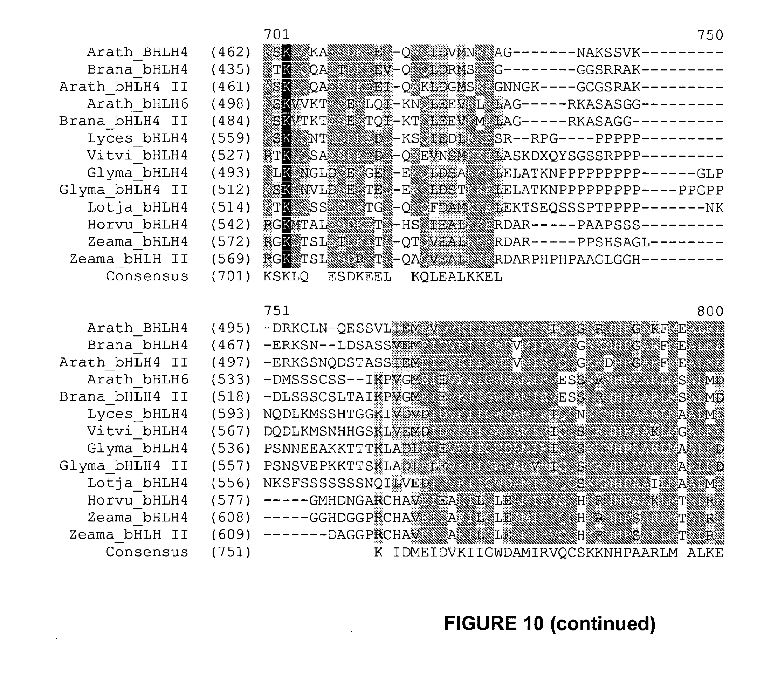

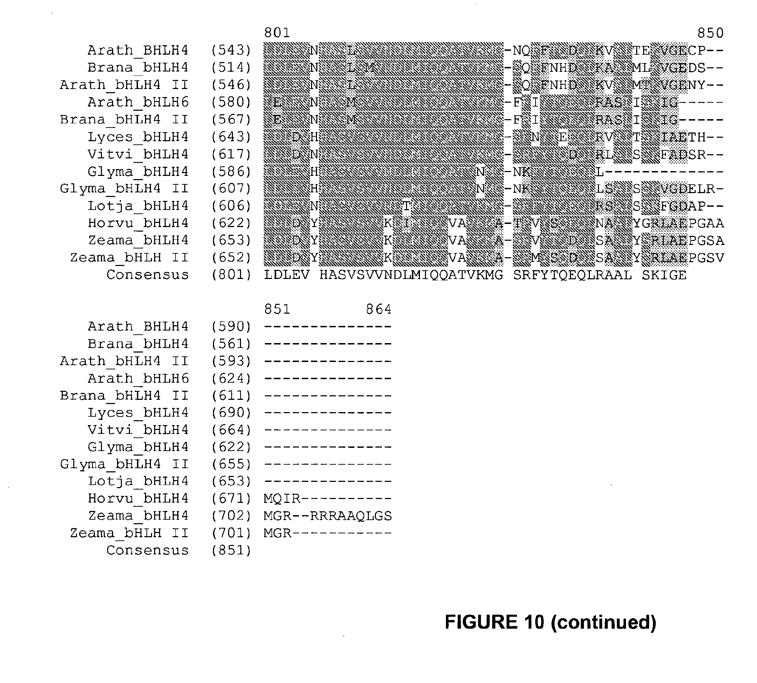

(a) an isolated nucleic acid molecule encoding the polypeptide as depicted in SEQ ID NO: 122, 124, 126, 128 and/or 130 and/or table A3b;
(b) an isolated nucleic acid molecule as depicted in SEQ ID NO:121, 123, 125, 127 and/or 129 and/or table A3b;
(c) an isolated nucleic acid molecule, which, as a result of the degeneracy of the genetic code, can be derived from a polypeptide sequence depicted in table A3 and confers enhanced yield-related traits relative to control plants;
(d) an isolated nucleic acid molecule having at least 30 %, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity with the nucleic acid molecule sequence of a polynucleotide comprising the nucleic acid molecule depicted in table A3 and confers enhanced yield-related traits relative to control plants;
(e) an isolated nucleic acid molecule encoding a polypeptide having at least 30 %, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity with the amino acid sequence of the polypeptide encoded by the nucleic acid molecule of (a) to (c) and confers enhanced yield-related traits relative to control plants;
(f) an isolated nucleic acid molecule which hybridizes with a nucleic acid molecule of (a) to (c) under stringent hybridization conditions and confers enhanced yield-related traits relative to control plants;
(g) an isolated nucleic acid molecule encoding a polypeptide comprising the consensus sequence or one or more polypeptide motifs as shown figure 10 and preferably having the activity represented by a nucleic acid molecule comprising a polynucleotide as depicted in table A3;
(h) an isolated nucleic acid molecule encoding a polypeptide having the activity represented by a protein as depicted in table A3 and confering enhanced yield-related traits relative to control plants;
and
(i) a nucleic acid molecule which is obtainable by screening a suitable nucleic acid library under stringent hybridization conditions with a probe comprising a complementary sequence of a nucleic acid molecule of (a) or (b) or with a fragment thereof, having at least 15 nt, preferably 20 nt, 30 nt, 50 nt, 100 nt, 200 nt or 500 nt of a nucleic acid molecule complementary to a nucleic acid molecule sequence characterized in (a) to (e) and encoding a polypeptide having the activity represented by a protein comprising a polypeptide as depicted in table A3;

whereby the nucleic acid molecule according to (a) to (i) is at least in one or more nucleotides different from the sequence depicted in table A3a and preferably which encodes a protein which differs at least in one or more amino acids from the protein sequences depicted in table A3a.

Item 53: Construct comprising:

(a) nucleic acid sequence encoding a GRP polypeptide as defined in item 42 to 44 or 52; one or more control sequences capable of driving expression of the nucleic acid sequence of (a); and optionally

(b) a transcription termination sequence.

Item 54: Construct according to item 53, wherein one of said control sequences is a constitutive promoter, preferably an HMGB promoter, most preferably an HMGB promoter from rice.

Item 55: Use of a construct according to any of items 53 to 54 in a method for making plants having enhanced yield-related traits, particularly increased early vigour, increased greenness index, increased total seed yield per plant, increased number of filled seeds, increased seed fill rate, and increased harvest index, relative to control plants.

Item 56: Plant, plant part or plant cell transformed with a construct according to any of items 53 to 54.

Item 57: Method for the production of a transgenic plant having enhanced-yield related traits relative to control plants, comprising:

(i) introducing and expressing in a plant, plant part, or plant cell, a nucleic acid sequence encoding a GRP polypeptide as defined in items 42 to 44 and 52; and
(ii) cultivating the plant, plant part, or plant cell under conditions promoting plant growth and development.

Item 58: Transgenic plant having enhanced yield-related traits, particularly increased early vigour, increased greenness index, increased total seed yield per plant, increased number of filled seeds, increased seed fill rate, and increased harvest index, relative to control plants, resulting from increased expression of a nucleic acid sequence encoding a GRP polypeptide as defined in items 42 to 44 and 52, or a transgenic plant cell derived from said transgenic plant.

Item 59: Transgenic plant according to items 51, 56 and 58, or a transgenic plant cell derived thereof, wherein said plant is a crop plant or a monocot or a cereal, such as rice, maize, wheat, barley, millet, rye, triticale, sorghum and oats.

Item 60: Harvestable parts of a plant comprising a nucleic acid sequence encoding a GRP polypeptide according to item 59, wherein said harvestable parts are preferably seeds.

Item 61: Products derived from a plant according to item 59 and/or from harvestable parts of a plant according to item 60.

Item 62: Use of a nucleic acid sequence encoding a GRP polypeptide in enhancing yield-related traits in plants, particularly in increasing early vigour, increasing greenness index, increasing total seed yield per plant, increasing number of filled seeds, increasing seed fill rate, and increasing harvest index, relative to control plants.

According to a further embodiment of the present invention, there is also provided an isolated polypeptide comprising a polypeptide sequence selected from the group consisting of:

(i) an amino acid sequence encoded by any one of the nucleic acids represented by Item 52;
(ii) an amino acid sequence having, in increasing order of preference, at least 50%, 55%, 60%, 65%, 70%, 75%, 80%, 85%, 90%, 95%, 96%, 97%, 98%, 99% or more sequence identity to the amino acid sequence of (i);
(iii) derivatives of any of the amino acid sequences given in (i) or (ii) above.

In one embodiment of the invention it has now been found that modulating expression (preferably, increasing) in a plant of a nucleic acid sequence encoding a GRP polypeptide, wherein said GRP polypeptide is an isopentenyl transferase (IPT) polypeptide, gives plants having enhanced yield-related traits relative to control plants. According to a first embodiment, the present invention provides a method for enhancing yield-related traits in plants relative to control plants, comprising modulating expression (preferably, increasing) in a plant of a nucleic acid sequence encoding a GRP polypeptide, wherein said GRP polypeptide is an isopentenyl transferase (IPT) polypeptide.
A preferred method for modulating (preferably, increasing) expression of a nucleic acid sequence encoding a GRP polypeptide is by introducing and expressing in a plant a nucleic acid sequence encoding a GRP polypeptide.
Any reference hereinafter to a "protein useful in the methods of the invention" is taken to mean a GRP polypeptide as defined herein. Any reference hereinafter to a "nucleic acid sequence useful in the methods of the invention" is taken to mean a nucleic acid sequence capable of encoding such a GRP polypeptide. The nucleic acid sequence to be introduced into a plant (and therefore useful in performing the methods of the invention) is any nucleic acid sequence encoding the type of protein which will now be described, hereafter also named "GRP nucleic acid sequence" or "GRP gene".

A "GRP polypeptide" as defined herein refers the polypeptides represented by SEQ ID NO: 132, and to orthologues, paralogues, and homologues thereof or any of the polypeptide sequences given in Table A4 of Example 1 herein.

Preferably, the orthologues, paralogues, and homologues of SEQ ID NO: 132 belong to the tRNA isopentenyltransferase family with InterPro entries IPR002627 and IPR011593.

Alternatively or additionally, a "GRP polypeptide" as defined herein refers to any polypeptide having in increasing order of preference at least 50%, 55%, 60%, 65%, 70%, 75%, 80%, 85%, 90%, 95%, 98%, 99% or more amino acid sequence identity to the GRP polypeptide as represented by SEQ ID NO: 132 or to any of the polypeptide sequences given in Table A4 of Example 1 herein.

The term "domain" and "motif" is defined in the "definitions" section herein. Specialist databases exist for the identification of domains, for example, SMART (Schultz et al. (1998) Proc. Natl. Acad. Sci. USA 95, 5857-5864; Letunic et al. (2002) Nucleic Acids Res 30, 242-244), InterPro (Mulder et al., (2003) Nucl. Acids. Res. 31, 315-318), Prosite (Bucher and Bairoch (1994), A generalized profile syntax for biomolecular sequences motifs and its function in automatic sequence interpretation. (In) ISMB-94; Proceedings 2nd International Conference on Intelligent Systems for Molecular Biology. Altman R., Brutlag D., Karp P., Lathrop R., Searls D., Eds., pp53-61, AAAI Press, Menlo Park; Hulo et al., Nucl. Acids. Res. 32:D134-D137, (2004)), or Pfam (Bateman et al., Nucleic Acids Research 30(1): 276-280 (2002)). A set of tools for *in silico* analysis of protein sequences is available on the ExPASy proteomics server (Swiss Institute of Bioinformatics (Gasteiger et al., ExPASy: the proteomics server for in-depth protein knowledge and analysis, Nucleic Acids Res. 31:3784-3788(2003)). Domains or motifs may also be identified using routine techniques, such as by sequence alignment.

Methods for the alignment of sequences for comparison are well known in the art, such methods include GAP, BESTFIT, BLAST, FASTA and TFASTA. GAP uses the algorithm of Needleman and Wunsch ((1970) J Mol Biol 48: 443-453) to find the global (i.e. spanning the complete sequences) alignment of two sequences that maximizes the number of matches and minimizes the number of gaps. The BLAST algorithm (Altschul et al. (1990) J Mol Biol 215: 403-10) calculates percent sequence identity and performs a statistical analysis of the similarity between the two sequences. The software for performing BLAST analysis is publicly available through the National Centre for Biotechnology Information (NCBI). Homologues may readily be identified using, for example, the ClustalW multiple sequence alignment algorithm (version 1.83), with the default pairwise alignment parameters, and a scoring method in percentage. Global percentages of similarity and identity may also be determined using one of the methods available in the MatGAT software package (Campanella et al., BMC Bioinformatics. 2003 Jul 10;4:29. MatGAT: an application that generates similarity/identity matrices using protein or DNA sequences). Minor manual editing may be performed to optimise alignment between conserved motifs, as would be apparent to a person skilled in the art. Furthermore, instead of using full-length sequences for the identification of homologues, specific domains may also be used. The sequence identity values may be determined over the entire nucleic acid sequence or polypeptide sequence or over selected domains or conserved motif(s), using the programs mentioned above using the default parameters.

Cytokinins, which are central regulators of cell division and differentiation in plants, are adenine derivatives carrying an isopentenyl side chain that may be hydroxylated. Plants have two classes of isopentenyltransferases (IPTs) acting on the adenine moiety: ATP/ADP isopentenyltransferases (in Arabidopsis thaliana, AtIPT1, 3, 4-8) and tRNA IPTs (in Arabidopsis, AtIPT2 and 9; Miyawaki et al. (2006) Proc Natl Acad Sci USA. 103(44): 16598-16603.). As described above, isopentenylation of ATP and ADP, by ATP/ADP IPTs, is the key step in the biosynthesis of iP- and tZ-type cytokinins. GRP polypeptides, as far as SEQ ID NO: 2, and its orthologues, paralogues, and homologues are concerned, typically are responsible for the isopentenylation of ATP and ADP in the biosynthesis of iP- and tZ-type cytokinins. Measuring such enzymatic activity is well known in the art (for example, Miyawaki *et al., supra).*

The present invention is illustrated by transforming plants with the nucleic acid sequence represented by SEQ ID NO: 131, encoding the polypeptide sequence of SEQ ID NO: 132. However, performance of the invention is not restricted to these sequences; the methods of the invention may advantageously be performed using any GRP-encoding nucleic acid sequence or GRP polypeptide as defined herein.

Examples of nucleic acid sequences encoding GRP polypeptides may be found in databases known in the art. Such nucleic acid sequences are useful in performing the methods of the invention. Orthologues and paralogues, the terms "orthologues" and "paralogues" being as defined herein, may readily be identified by performing a so-called reciprocal blast search. Typically, this involves a first BLAST involving BLASTing a query sequence (for example using SEQ ID NO: 132) against any sequence database, such as the publicly available NCBI database. BLASTN or TBLASTX (using standard default values) are generally used when starting from a nucleotide sequence, and BLASTP or TBLASTN (using standard default values) when starting from a protein sequence. The BLAST results may optionally be filtered. The full-length sequences of either the filtered results or non-filtered results are then BLASTed back (second BLAST) against sequences from the organism from which the query sequence is derived (where the query sequence is SEQ ID NO: 131 or SEQ ID NO: 132, the second BLAST would therefore be against *Arabidopsis thaliana* sequences). The results of the first and second BLASTs are then compared. A paralogue is identified if a high-ranking hit from the first blast is from the same species as from which the query sequence is derived, a BLAST back then ideally results in the query sequence amongst the highest hits; an orthologue is identified if a high-ranking hit in the first BLAST is not from the same species as from which the query sequence is derived, and preferably results upon BLAST back in the query sequence being among the highest hits.

High-ranking hits are those having a low E-value. The lower the E-value, the more significant the score (or in other words the lower the chance that the hit was found by chance). Computation of the E-value is well known in the art. In addition to E-values, comparisons are also scored by percentage identity. Percentage identity refers to the number of identical nucleotides (or amino acids) between the two compared nucleic acid sequence (or polypeptide) sequences over a particular length. In the case of large families, ClustalW may be used, followed by a neighbour joining tree, to help visualize clustering of related genes and to identify orthologues and paralogues.

Nucleic acid sequence variants encoding homologues and derivatives of SEQ ID NO: 132 may also be useful in practising the methods of the invention, the terms "homologue" and "derivative" being as defined herein. Also useful in the methods of the invention are nucleic acid sequences encoding homologues and derivatives of orthologues or paralogues of SEQ ID NO: 132 or of any of the polypeptide sequences given in Table A4 of Example 1. Homologues and derivatives useful in the methods of the present invention have substantially the same biological and functional activity as the unmodified protein from which they are derived.

Further nucleic acid sequence variants useful in practising the methods of the invention include portions of nucleic acid sequences encoding GRP polypeptides, nucleic acid sequences hybridising to nucleic acid sequences encoding GRP polypeptides, splice variants of nucleic acid sequences encoding GRP polypeptides, allelic variants of nucleic acid sequences encoding GRP polypeptides and variants of nucleic acid sequences encoding GRP polypeptides obtained by gene shuffling. The terms hybridising sequence, splice variant, allelic variant and gene shuffling are as described herein.

Nucleic acid sequences encoding GRP polypeptides need not be full-length nucleic acid sequences, since performance of the methods of the invention does not rely on the use of full-length nucleic acid sequences. According to the present invention, there is provided a method for enhancing yield-related traits in plants, comprising introducing and expressing in a plant a portion of SEQ ID NO: 131, or a portion of a nucleic acid sequence encoding an orthologue, paralogue, or homologue of SEQ ID NO: 132.

A portion of a nucleic acid sequence may be prepared, for example, by making one or more deletions to the nucleic acid sequence. The portions may be used in isolated form or they may be fused to other coding (or non-coding) sequences in order to, for example, produce a protein that combines several activities. When fused to other coding sequences, the resultant polypeptide produced upon translation may be bigger than that predicted for the polypeptide portion.

Portions useful in the methods of the invention, encode a GRP polypeptide as defined herein, and have substantially the same biological activity as the polypeptide sequence given in SEQ ID NO: 132 or as any of the polypeptide sequences given in Table A4 of Example 1. Preferably, the portion is a portion of the nucleic acid sequence given in SEQ ID NO: 131, or is a portion of a nucleic acid sequence encoding an orthologue or paralogue of the polypeptide sequence given in SEQ ID NO: 132. Preferably the portion is at least 300, 400, 500, 600, 700, 800, 850, 900, 950, 1000, 1050, or more consecutive nucleotides in length, the consecutive nucleotides being of SEQ ID NO: 131, or of a nucleic acid sequence encoding an orthologue or paralogue of SEQ ID NO: 132. Most preferably the portion is a portion of the nucleic acid sequence of SEQ ID NO: 131.

Another nucleic acid sequence variant useful in the methods of the invention is a nucleic acid sequence capable of hybridising, under reduced stringency conditions, preferably under stringent conditions, with a nucleic acid sequence encoding a GRP polypeptide as defined herein, or with a portion as defined herein.

According to the present invention, there is provided a method for enhancing yield-related traits in plants comprising introducing and expressing in a plant a nucleic acid sequence capable of hybridizing to SEQ ID NO: 131, or comprising introducing and expressing in a plant a nucleic acid sequence capable of hybridising to a nucleic acid sequence encoding an orthologue, paralogue, or homologue of SEQ ID NO: 132 or of any of the polypeptide sequences given in Table A of Example 1.

Hybridising sequences useful in the methods of the invention encode a GRP polypeptide as defined herein, having substantially the same biological activity as the polypeptide sequence given in SEQ ID NO: 132. Preferably, the hybridising sequence is capable of hybridising to SEQ ID NO: 131, or to a portion of this sequence, a portion being as defined above, or the hybridising sequence is capable of hybridising to a nucleic acid sequence encoding an orthologue or paralogue of SEQ ID NO: 132, or to a portion thereof or to any of the polypeptide sequences given in Table A of Example 1.

Another nucleic acid sequence variant useful in the methods of the invention is a splice variant encoding a GRP polypeptide as defined hereinabove, a splice variant being as defined herein.

According to the present invention, there is provided a method for enhancing yield-related traits in plants, comprising introducing and expressing in a plant a splice variant of SEQ ID NO: 131, or a splice variant of a nucleic acid sequence encoding an orthologue, paralogue, or homologue of SEQ ID NO: 132.

Another nucleic acid sequence variant useful in performing the methods of the invention is an allelic variant of a nucleic acid sequence encoding a GRP polypeptide as defined hereinabove, an allelic variant being as defined herein.

According to the present invention, there is provided a method for enhancing yield-related traits in plants comprising introducing and expressing in a plant an allelic variant of SEQ ID NO: 131, or comprising introducing and expressing in a plant an allelic variant of a nucleic acid sequence encoding an orthologue, paralogue, or homologue of the polypeptide sequence represented by SEQ ID NO: 132.

The allelic variants useful in the methods of the present invention have substantially the same biological activity as the GRP polypeptide of SEQ ID NO: 132. Allelic variants exist in nature, and encompassed within the methods of the present invention is the use of these natural alleles. Gene shuffling or directed evolution may also be used to generate variants of nucleic acid sequences encoding GRP polypeptides as defined above; the term "gene shuffling" being as defined herein.

According to the present invention, there is provided a method for enhancing yield-related traits in plants comprising introducing and expressing in a plant a variant nucleic acid sequence of SEQ ID NO: 131, or comprising introducing and expressing in a plant a variant of a nucleic acid sequence encoding an orthologue, paralogue, or homologue of SEQ ID NO: 132, which variant nucleic acid sequence is obtained by gene shuffling.

Furthermore, nucleic acid sequence variants may also be obtained by site-directed mutagenesis. Several methods are available to achieve site-directed mutagenesis, the most common being PCR based methods (Current Protocols in Molecular Biology. Wiley Eds.).

Nucleic acid sequences encoding GRP polypeptides may be derived from any natural or artificial source. The nucleic acid sequence may be modified from its native form in composition and/or genomic environment through deliberate human manipulation. Preferably the GRP polypeptide-encoding nucleic acid sequence is from a plant. In the case of SEQ ID NO: 131, the GRP polypeptide encoding nucleic acid sequence is preferably from a dicotyledonous plant, more preferably from the family Brassicaceae, most preferably the nucleic acid sequence is from *Arabidopsis thaliana.*

Performance of the methods of the invention gives plants having enhanced yield-related traits, preferably enhanced seed yield-related traits. The terms "yield" and "seed yield" are described in more detail in the "definitions" section herein.

Reference herein to enhanced yield-related traits is taken to mean an increase in early vigour and/or in biomass (weight) of one or more parts of a plant, which may include aboveground (harvestable) parts and/or (harvestable) parts below ground. In particular, such harvestable parts are biomass and/or seeds, and performance of the methods of the invention results in plants having increased early vigour, biomass and/or seed yield relative to the early vigour, biomass or seed yield of control plants.

Taking corn as an example, a yield increase may be manifested as one or more of the following: increase in the number of plants established per hectare or acre, an increase in the number of ears per plant, an increase in the number of rows, number of kernels per row, kernel weight, thousand kernel weight, ear length/diameter, increase in the seed filling rate (which is the number of filled seeds divided by the total number of seeds and multiplied by 100), among others. Taking rice as an example, a yield increase may manifest itself as an increase in one or more of the following: number of plants per hectare or acre, number of panicles per plant, number of spikelets per panicle, number of flowers (florets) per panicle (which is expressed as a ratio of the number of filled seeds over the number of primary panicles), increase in the seed filling rate (which is the number of filled seeds divided by the total number of seeds and multiplied by 100), increase in thousand kernel weight, among others.

The present invention provides a method for enhancing yield-related traits, especially biomass and/or seed yield of plants, relative to control plants, which method comprises modulating expression (preferably, increasing) in a plant of a nucleic acid sequence encoding a GRP polypeptide as defined herein.

Since the transgenic plants according to the present invention have enhanced yield-related traits, it is likely that these plants exhibit an increased growth rate (during at least part of their life cycle), relative to the growth rate of control plants at a corresponding stage in their life cycle.

The increased growth rate may be specific to one or more parts of a plant (including seeds), or may be throughout substantially the whole plant. Plants having an increased growth rate may have a shorter life cycle. The life cycle of a plant may be taken to mean the time needed to grow from a dry mature seed up to the stage where the plant has produced dry mature seeds, similar to the starting material. This life cycle may be influenced by factors such as early vigour, growth rate, greenness index, flowering time and speed of seed maturation. The increase in growth rate may take place at one or more stages in the life cycle of a plant or during substantially the whole plant life cycle. Increased growth rate during the early stages in the life cycle of a plant may reflect enhanced vigour. The increase in growth rate may alter the harvest cycle of a plant allowing plants to be sown later and/or harvested sooner than would otherwise be possible (a similar effect may be obtained with earlier flowering time). If the growth rate is sufficiently increased, it may allow for the further sowing of seeds of the same plant species (for example sowing and harvesting of rice plants followed by sowing and harvesting of further rice plants all within one conventional growing period). Similarly, if the growth rate is sufficiently increased, it may allow for the further sowing of seeds of different plants species (for example the sowing and harvesting of corn plants followed by, for example, the sowing and optional harvesting of soybean, potato or any other suitable plant). Harvesting additional times from the same rootstock in the case of some crop plants may also be possible. Altering the harvest cycle of a plant may lead to an increase in annual biomass production per acre (due to an increase in the number of times (say in a year) that any particular plant may be grown and harvested). An increase in growth rate may also allow for the cultivation of transgenic plants in a wider geographical area than their wild-type counterparts, since the territorial limitations for growing a crop are often determined by adverse environmental conditions either at the time of planting (early season) or at the time of harvesting (late season). Such adverse conditions may be avoided if the harvest cycle is shortened. The growth rate may be determined by deriving various parameters from growth curves, such parameters may be: T-Mid (the time taken for plants to reach 50% of their maximal size) and T-90 (time taken for plants to reach 90% of their maximal size), amongst others.

According to a preferred feature of the present invention, performance of the methods of the invention gives plants having an increased growth rate relative to control plants. Therefore, according to the present invention, there is provided a method for increasing the growth rate of plants, which method comprises modulating expression (preferably, increasing) in a plant of a nucleic acid sequence encoding a GRP polypeptide as defined herein.

An enhancement of yield-related traits (an increase in seed yield and/or growth rate) occurs whether the plant is under non-stress conditions or whether the plant is exposed to various stresses compared to control plants. Plants typically respond to exposure to stress by growing more slowly. In conditions of severe stress, the plant may even stop growing altogether. Mild stress on the other hand is defined herein as being any stress to which a plant is exposed which does not result in the plant ceasing to grow altogether without the capacity to resume growth. Mild stress in the sense of the invention leads to a reduction in the growth of the stressed plants of less than 40%, 35% or 30%, preferably less than 25%, 20% or 15%, more preferably less than 14%, 13%, 12%, 11% or 10% or less in comparison to the control plant under non-stress conditions. Due to advances in agricultural practices (irrigation, fertilization, pesticide treatments) severe stresses are not often encountered in cultivated crop plants. As a consequence, the compromised growth induced by mild stress is often an undesirable feature for agriculture. Mild stresses are the everyday biotic and/or abiotic (environmental) stresses to which a plant is exposed. Abiotic stresses may be due to drought or excess water, anaerobic stress, salt stress, chemical toxicity, oxidative stress and hot, cold or freezing temperatures. The abiotic stress may be an osmotic stress caused by a water stress (particularly due to drought), salt stress, oxidative stress or an ionic stress. Biotic stresses are typically those stresses caused by pathogens, such as bacteria, viruses, nematodes, fungi and insects. The term "non-stress" conditions as used herein are those environmental conditions that allow optimal growth of plants. Persons skilled in the art are aware of normal soil conditions and climatic conditions for a given location.

Performance of the methods of the invention gives plants grown under non-stress conditions or under mild stress conditions having enhanced yield-related traits relative to control plants grown under comparable conditions. Therefore, according to the present invention, there is provided a method for enhancing yield-related traits in plants grown under non-stress conditions or under mild stress conditions, which method comprises modulating (preferably, increasing) expression in a plant of a nucleic acid sequence encoding a GRP polypeptide.

Performance of the methods according to the present invention results in plants grown under abiotic stress conditions having enhanced yield-related traits relative to control plants grown under comparable stress conditions. As reported in Wang et al. (Planta (2003) 218: 1-14), abiotic stress leads to a series of morphological, physiological, biochemical and molecular changes that adversely affect plant growth and productivity. Drought, salinity, extreme temperatures and oxidative stress are known to be interconnected and may induce growth and cellular damage through similar mechanisms. Rabbani et al. (Plant Physiol (2003) 133: 1755-1767) describes a particularly high degree of "cross talk" between drought stress and high-salinity stress. For example, drought and/or salinisation are manifested primarily as osmotic stress, resulting in the disruption of homeostasis and ion distribution in the cell. Oxidative stress, which frequently accompanies high or low temperature, salinity or drought stress, may cause denaturing of functional and structural proteins. As a consequence, these diverse environmental stresses often activate similar cell signalling pathways and cellular responses, such as the production of stress proteins, up-regulation of anti-oxidants, accumulation of compatible solutes and growth arrest. Since diverse environmental stresses activate similar pathways, plants having enhanced yield-related traits, preferably enhanced seed yield-related traits, under one type of abiotic stress growth conditions, relative to control plants grown under comparable abiotic stress conditions, should not be seen as a limitation to that type of abiotic stress growth conditions, but more as a screen to indicate the involvement of GRP polypeptides as defined above, in enhancing yield-related traits in abiotic stress growth conditions in general.

The term "abiotic stress" as defined herein is taken to mean any one or more of: water stress (due to drought or excess water), anaerobic stress, salt stress, temperature stress (due to hot, cold or freezing temperatures), chemical toxicity stress and oxidative stress. According to one aspect of the invention, the abiotic stress is an osmotic stress, selected from water stress, salt stress, oxidative stress and ionic stress. Preferably, the water stress is drought stress. The term salt stress is not restricted to common salt (NaCl), but may be any stress caused by one or more of: NaCl, KCl, LiCl, MgCl2, CaCl2, amongst others.

Performance of the methods of the invention gives plants having enhanced yield-related traits under abiotic stress growth conditions relative to control plants grown in comparable stress conditions. Therefore, according to the present invention, there is provided a method for enhancing yield-related traits in plants grown under abiotic stress growth conditions, which method comprises modulating (preferably, increasing) expression in a plant of a nucleic acid sequence encoding a GRP polypeptide. According to one aspect of the invention, the abiotic stress is an osmotic stress, selected from one or more of the following: water stress, salt stress, oxidative stress and ionic stress.

Performance of the methods of the invention gives plants grown under conditions of reduced nutrient availability, particularly under conditions of reduced nitrogen availability, enhanced yield-related traits relative to control plants grown under comparable conditions. Therefore, according to the present invention, there is provided a method for enhancing yield-related traits in plants grown under conditions of reduced nutrient availability, which method comprises modulating (preferably, increasing) expression in a plant of a nucleic acid sequence encoding a GRP polypeptide. Reduced nutrient availability may comprise reduced availability of nutrients such as nitrogen, phosphates and other phosphorous-containing compounds, potassium, calcium, cadmium, magnesium, manganese, iron and boron, amongst others.

The present invention encompasses plants, parts thereof (including seeds), or plant cells obtainable by the methods according to the present invention. The plants, parts thereof, or plant cells comprise a nucleic acid transgene encoding a GRP polypeptide as defined above, preferably of isopentenyl transferase (IPT) polypeptide.

The invention also provides genetic constructs and vectors to facilitate introduction and/or expression in plants of nucleic acid sequences encoding GRP polypeptides. The gene constructs may be inserted into vectors, which may be commercially available, suitable for transforming into plants and suitable for expression of the gene of interest in the transformed cells. The invention also provides use of a gene construct as defined herein in the methods of the invention.

More specifically, the present invention provides a construct comprising:

(i) a nucleic acid sequence encoding a GRP polypeptide as defined above, preferably of isopentenyl transferase (IPT) polypeptide;
(ii) one or more control sequences capable of driving expression of the nucleic acid sequence of (a); and optionally
(iii) a transcription termination sequence.

Preferably, the nucleic acid sequence encoding a GRP polypeptide is as defined above. The term "control sequence" and "termination sequence" are as defined herein.

Plants are transformed with a vector comprising any of the nucleic acid sequences described above. The skilled artisan is well aware of the genetic elements that must be present on the vector in order to successfully transform, select and propagate host cells containing the sequence of interest. The sequence of interest is operably linked to one or more control sequences (at least to a promoter).

Advantageously, any type of promoter, whether natural or synthetic, may be used to drive expression of the nucleic acid sequence. A seed-specific promoter is particularly useful in the methods of the invention. See the "Definitions" section herein for definitions of the various promoter types.

It should be clear that the applicability of the present invention is not restricted to the GRP polypeptide-encoding nucleic acid sequence represented by SEQ ID NO: 131, nor is the applicability of the invention restricted to expression of a GRP polypeptide-encoding nucleic acid sequence when driven by a seed-specific promoter.

The seed-specific promoter is preferably a prolamin promoter, preferably a prolamin promoter from rice. Further preferably the prolamin promoter is represented by a nucleic acid sequence substantially similar to SEQ ID NO: 133, most preferably the prolamin promoter is as represented by SEQ ID NO: 133. See Table 2 in the "Definitions" section herein for further examples of seed-specific promoters.

Optionally, one or more terminator sequences may be used in the construct introduced into a plant. Additional regulatory elements may include transcriptional as well as translational enhancers. Those skilled in the art will be aware of terminator and enhancer sequences that may be suitable for use in performing the invention. An intron sequence may also be added to the 5' untranslated region (UTR) or in the coding sequence to increase the amount of the mature message that accumulates in the cytosol, as described in the definitions section. Other control sequences (besides promoter, enhancer, silencer, intron sequences, 3'UTR and/or 5'UTR regions) may be protein and/or RNA stabilizing elements. Such sequences would be known or may readily be obtained by a person skilled in the art.

The genetic constructs of the invention may further include an origin of replication sequence that is required for maintenance and/or replication in a specific cell type. One example is when a genetic construct is required to be maintained in a bacterial cell as an episomal genetic element (e.g. plasmid or cosmid molecule). Preferred origins of replication include, but are not limited to, the f1-ori and colE1.

For the detection of the successful transfer of the nucleic acid sequences as used in the methods of the invention and/or selection of transgenic plants comprising these nucleic acid sequences, it is advantageous to use marker genes (or reporter genes). Therefore, the genetic construct may optionally comprise a selectable marker gene. Selectable markers are described in more detail in the "definitions" section herein. The marker genes may be removed or excised from the transgenic cell once they are no longer needed. Techniques for marker removal are known in the art, useful techniques are described above in the definitions section.

The invention also provides a method for the production of transgenic plants having enhanced yield-related traits relative to control plants, comprising introduction and expression in a plant of a nucleic acid sequence encoding a GRP polypeptide as defined hereinabove, preferably of isopentenyl transferase (IPT) polypeptide.

More specifically, the present invention provides a method for the production of transgenic plants having enhanced yield-related traits, which method comprises:

(i) introducing and expressing in a plant, plant part, or plant cell, a nucleic acid sequence encoding a GRP polypeptide; and

(ii) cultivating the plant, plant part or plant cell under conditions promoting plant growth and development.

The nucleic acid sequence of (i) may be any of the nucleic acid sequences capable of encoding a GRP polypeptide as defined herein.

The nucleic acid sequence may be introduced directly into a plant cell or into the plant itself (including introduction into a tissue, organ or any other part of a plant). According to a preferred feature of the present invention, the nucleic acid sequence is preferably introduced into a plant by transformation. The term "transformation" is described in more detail in the "definitions" section herein.

The genetically modified plant cells can be regenerated via all methods with which the skilled worker is familiar. Suitable methods can be found in the abovementioned publications by S.D. Kung and R. Wu, Potrykus or Höfgen and Willmitzer.

Generally after transformation, plant cells or cell groupings are selected for the presence of one or more markers which are encoded by plant-expressible genes co-transferred with the gene of interest, following which the transformed material is regenerated into a whole plant. To select transformed plants, the plant material obtained in the transformation is, as a rule, subjected to selective conditions so that transformed plants can be distinguished from untransformed plants. For example, the seeds obtained in the above-described manner can be planted and, after an initial growing period, subjected to a suitable selection by spraying. A further possibility consists in growing the seeds, if appropriate after sterilization, on agar plates using a suitable selection agent so that only the transformed seeds can grow into plants. Alternatively, the transformed plants are screened for the presence of a selectable marker such as the ones described above.

Following DNA transfer and regeneration, putatively transformed plants may also be evaluated, for instance using Southern analysis, for the presence of the gene of interest, copy number and/or genomic organisation. Alternatively or additionally, expression levels of the newly introduced DNA may be monitored using Northern and/or Western analysis, both techniques being well known to persons having ordinary skill in the art.

The generated transformed plants may be propagated by a variety of means, such as by clonal propagation or classical breeding techniques. For example, a first generation (or T1) transformed plant may be selfed and homozygous second-generation (or T2) transformants selected, and the T2 plants may then further be propagated through classical breeding techniques. The generated transformed organisms may take a variety of forms. For example, they may be chimeras of transformed cells and non-transformed cells; clonal transformants (e.g., all cells transformed to contain the expression cassette); grafts of transformed and untransformed tissues (e.g., in plants, a transformed rootstock grafted to an untransformed scion).

The present invention clearly extends to any plant cell or plant produced by any of the methods described herein, and to all plant parts and propagules thereof. The present invention extends further to encompass the progeny of a primary transformed or transfected cell, tissue, organ or whole plant that has been produced by any of the aforementioned methods, the only requirement being that progeny exhibit the same genotypic and/or phenotypic characteristic (s) as those produced by the parent in the methods according to the invention.

The invention also includes host cells containing an isolated nucleic acid sequence encoding a GRP polypeptide as defined hereinabove. Preferred host cells according to the invention are plant cells. Host plants for the nucleic acid transgene or the vector used in the method according to the invention, the expression cassette or construct or vector are, in principle, advantageously all plants, which are capable of synthesizing the polypeptides used in the inventive method.

The methods of the invention are advantageously applicable to any plant. Plants that are particularly useful in the methods of the invention include all plants which belong to the superfamily Viridiplantae, in particular monocotyledonous and dicotyledonous plants including fodder or forage legumes, ornamental plants, food crops, trees or shrubs. According to a preferred embodiment of the present invention, the plant is a crop plant. Examples of crop plants include soybean, sunflower, canola, alfalfa, rapeseed, cotton, tomato, potato and tobacco. Further preferably, the plant is a monocotyledonous plant. Examples of monocotyledonous plants include sugarcane. More preferably the plant is a cereal. Examples of cereals include rice, maize, wheat, barley, millet, rye, triticale, sorghum and oats.

The invention also extends to harvestable parts of a plant such as, but not limited to seeds, leaves, fruits, flowers, stems, roots, rhizomes, tubers and bulbs. The invention furthermore relates to products derived, preferably directly derived, from a harvestable part of such a plant, such as dry pellets or powders, oil, fat and fatty acids, starch or proteins.

According to a preferred feature of the invention, the modulated expression is increased expression. Methods for increasing expression of nucleic acid sequences or genes, or gene products, are well documented in the art and examples are provided in the definitions section.

As mentioned above, a preferred method for modulating (preferably, increasing) expression of a nucleic acid sequence encoding a GRP polypeptide is by introducing and expressing in a plant a nucleic acid sequence encoding a GRP polypeptide; however the effects of performing the method, i.e. enhancing yield-related traits, may also be achieved using other well known techniques, including but not limited to T-DNA activation tagging, TILLING, homologous recombination. A description of these techniques is provided in the definitions section.

The present invention also encompasses use of nucleic acid sequences encoding GRP polypeptides as described herein and use of these GRP polypeptides in enhancing any of the aforementioned yield-related traits in plants.

Nucleic acid sequences encoding GRP polypeptide described herein, or the GRP polypeptides themselves, may find use in breeding programmes in which a DNA marker is identified which may be genetically linked to a GRP polypeptide-encoding gene. The genes/nucleic acid sequences, or the GRP polypeptides themselves may be used to define a molecular marker. This DNA or protein marker may then be used in breeding programmes to select plants having enhanced yield-related traits, as defined hereinabove in the methods of the invention.

Allelic variants of a GRP polypeptide-encoding nucleic acid sequence/gene may also find use in marker-assisted breeding programmes. Such breeding programmes sometimes require introduction of allelic variation by mutagenic treatment of the plants, using for example EMS mutagenesis; alternatively, the programme may start with a collection of allelic variants of so called "natural" origin caused unintentionally. Identification of allelic variants then takes place, for example, by PCR. This is followed by a step for selection of superior allelic variants of the sequence in question and which give enhanced yield-related traits. Selection is typically carried out by monitoring growth performance of plants containing different allelic variants of the sequence in question. Growth performance may be monitored in a greenhouse or in the field. Further optional steps include crossing plants in which the superior allelic variant was identified with another plant. This could be used, for example, to make a combination of interesting phenotypic features.

Nucleic acid sequences encoding GRP polypeptides may also be used as probes for genetically and physically mapping the genes that they are a part of, and as markers for traits linked to those genes. Such information may be useful in plant breeding in order to develop lines with desired phenotypes. Such use of GRP polypeptide-encoding nucleic acid sequences requires only a nucleic acid sequence of at least 15 nucleotides in length. The GRP polypeptide-encoding nucleic acid sequences may be used as restriction fragment length polymorphism (RFLP) markers. Southern blots (Sambrook J, Fritsch EF and Maniatis T (1989) Molecular Cloning, A Laboratory Manual) of restriction-digested plant genomic DNA may be probed with the GRP-encoding nucleic acid sequences. The resulting banding patterns may then be subjected to genetic analyses using computer programs such as MapMaker (Lander et al. (1987) Genomics 1: 174-181) in order to construct a genetic map. In addition, the nucleic acid sequences may be used to probe Southern blots containing restriction endonuclease-treated genomic DNAs of a set of individuals representing parent and progeny of a defined genetic cross. Segregation of the DNA polymorphisms is noted and used to calculate the position of the GRP polypeptide-encoding nucleic acid sequence in the genetic map previously obtained using this population (Botstein et al. (1980) Am. J. Hum. Genet. 32:314-331).

The production and use of plant gene-derived probes for use in genetic mapping is described in Bernatzky and Tanksley (1986) Plant Mol. Biol. Reporter 4: 37-41. Numerous publications describe genetic mapping of specific cDNA clones using the methodology outlined above or variations thereof. For example, F2 intercross populations, backcross populations, randomly mated populations, near isogenic lines, and other sets of individuals may be used for mapping. Such methodologies are well known to those skilled in the art.

The nucleic acid sequence probes may also be used for physical mapping (i.e., placement of sequences on physical maps; see Hoheisel et al. In: Non-mammalian Genomic Analysis: A Practical Guide, Academic press 1996, pp. 319-346, and references cited therein).

In another embodiment, the nucleic acid sequence probes may be used in direct fluorescence in situ hybridisation (FISH) mapping (Trask (1991) Trends Genet. 7:149-154). Although current methods of FISH mapping favour use of large clones (several kb to several hundred kb; see Laan et al. (1995) Genome Res. 5:13-20), improvements in sensitivity may allow performance of FISH mapping using shorter probes.

A variety of nucleic acid sequence amplification-based methods for genetic and physical mapping may be carried out using the nucleic acid sequences. Examples include allele-specific amplification (Kazazian (1989) J. Lab. Clin. Med 11:95-96), polymorphism of PCR-amplified fragments (CAPS; Sheffield et al. (1993) Genomics 16:325-332), allele-specific ligation (Landegren et al. (1988) Science 241:1077-1080), nucleotide extension reactions (Sokolov (1990) Nucleic Acids Res. 18:3671), Radiation Hybrid Mapping (Walter et al. (1997) Nat. Genet. 7:22-28) and Happy Mapping (Dear and Cook (1989) Nucleic Acids Res. 17:6795-6807). For these methods, the sequence of a nucleic acid sequence is used to design and produce primer pairs for use in the amplification reaction or in primer extension reactions. The design of such primers is well known to those skilled in the art. In methods employing PCR-based genetic mapping, it may be necessary to identify DNA sequence differences between the parents of the mapping cross in the region corresponding to the instant nucleic acid sequence. This, however, is generally not necessary for mapping methods.

The methods according to the present invention result in plants having enhanced yield-related traits, as described hereinbefore. These traits may also be combined with other economically advantageous traits, such as further yield-enhancing traits, tolerance to other abiotic and biotic stresses, traits modifying various architectural features and/or biochemical and/or physiological features.

.In one embodiment the invention relates to subject mater summarized as follows:

Item 63: A method for enhancing yield-related traits in plants relative to control plants, comprising modulating expression (preferably, increasing) in a plant of a nucleic acid sequence encoding a GRP polypeptide, wherein said GRP polypeptide is an isopentenyl transferase (IPT) polypeptide as represented by SEQ ID NO: 132 or an orthologue, paralogue, or homologue thereof, and optionally selecting for plants having enhanced yield-related traits.

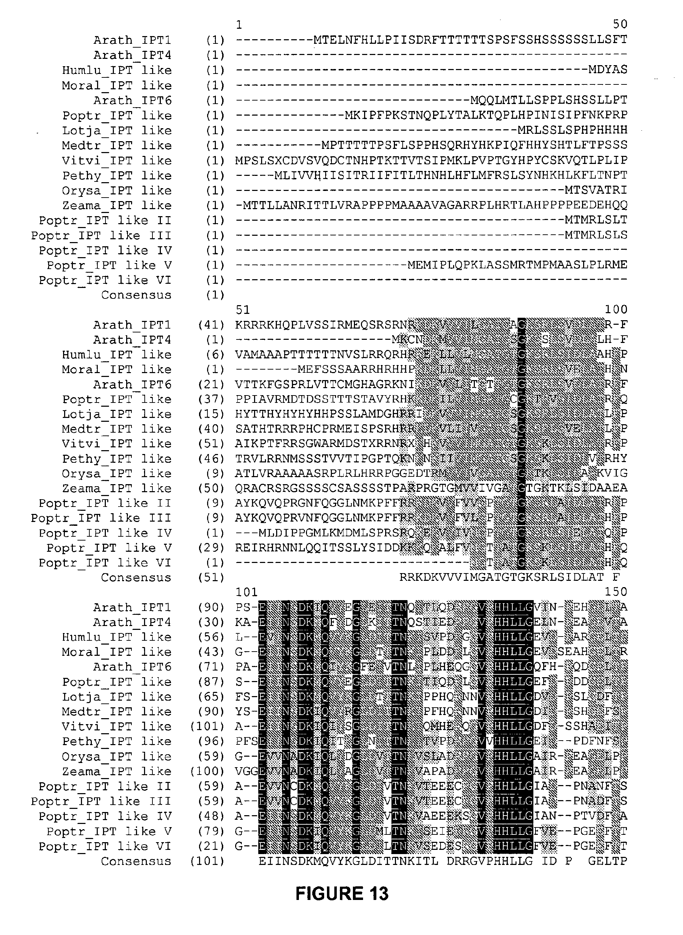

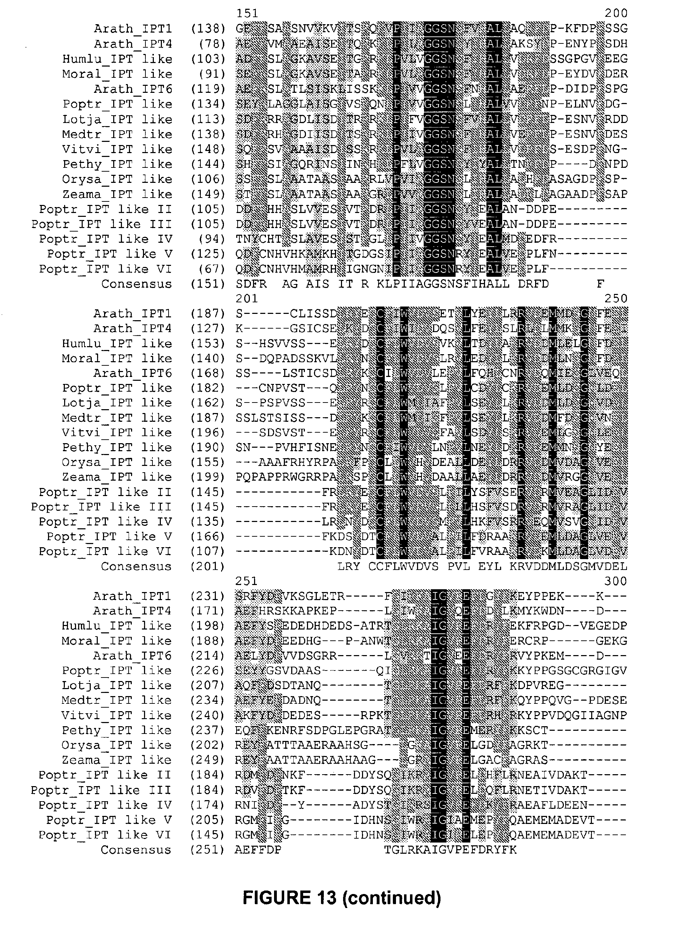

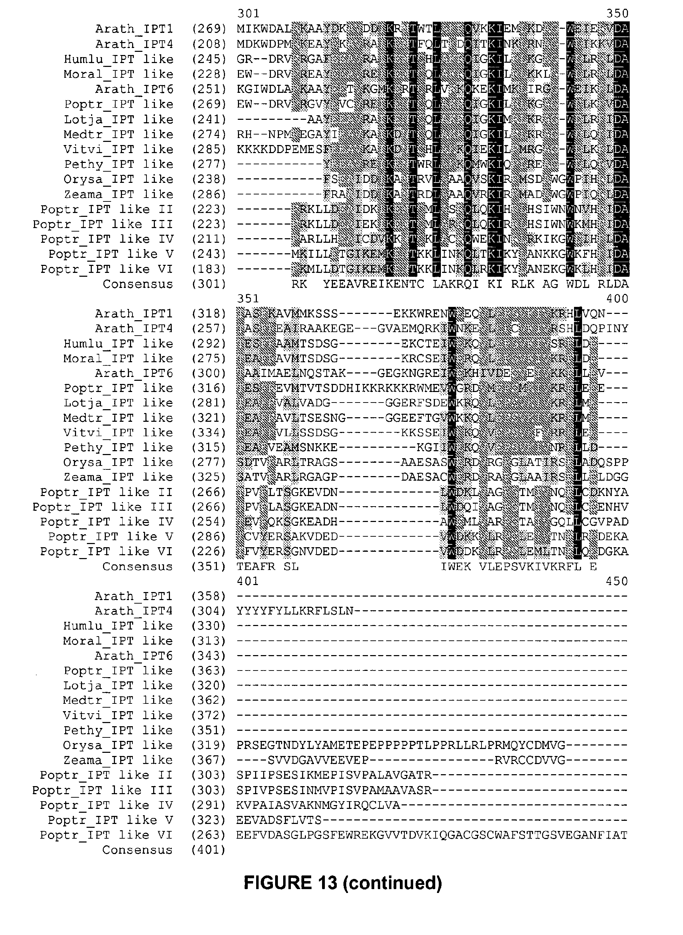

Item 64: A method according to item 63, wherein said GRP polypeptide as represented by SEQ ID NO: 132 and an orthologue, paralogue, or homologue thereof, belong to the tRNA isopentenyltransferase family with InterPro entries IPR002627 and IPR011593 or comprising a domain or motif according to the consensus sequence as depicted in figure 13.

Item 65: Method according to item 63 or 64, wherein said GRP polypeptide has in increasing order of preference at least 50%, 55%, 60%, 65%, 70%, 75%, 80%, 85%, 90%, 95%, 98%, 99% or more amino acid sequence identity to the GRP polypeptide as represented by SEQ ID NO: 132 or to any of the polypeptide sequences given in Table A4 of Example 1.

Item 66: Method according to any preceding item 63 to 65, wherein said nucleic acid sequence encoding a GRP polypeptide is represented by the nucleic acid sequence of SEQ ID NO: 131 or a portion thereof, or a sequence capable of hybridising with the nucleic acid sequence of SEQ ID NO: 131 or a portion thereof or a sequence capable of hybridising with any one of the nucleic acid sequences SEQ ID NOs given in Table A4 of Example 1.

Item 67: Method according to any preceding item 63 to 66wherein said modulated expression (preferably, increased) is effected by introducing and expressing in a plant a nucleic acid sequence encoding said GRP polypeptide.

Item 68: Method according to any preceding item 63 to 67, wherein said enhanced yield-related traits comprise one or more of: increased total seed yield per plant, increased number of filled seeds, increased total number of seeds, and increased harvest index, relative to control plants.

Item 69: Method according to any preceding item 63 to 68, wherein said modulated expression is increased expression.

Item 70: Method according to any preceding item 63 to 69, wherein said nucleic acid sequence is operably linked to a seed-specific promoter, preferably to a prolamin promoter, most preferably to a prolamin promoter from rice.

Item 71: Method according to any preceding item 63 to 70, wherein said nucleic acid sequence encoding a GRP polypeptide is of plant origin, preferably from a dicotyledonous plant, further preferably from the family Brassicaceae, more preferably from *Arabidopsis thaliana.*

Item 72: Plant, part thereof (including seeds), or plant cell obtainable by a method according to any preceding item 63 to 71, wherein said plant, part thereof, or plant cell comprises a nucleic acid transgene encoding a GRP polypeptide.

Item 73: Construct comprising:

(a) nucleic acid sequence encoding a GRP polypeptide as defined in item 63 to 65;
(b) one or more control sequences capable of driving expression of the nucleic acid sequence of (a); and optionally
(c) a transcription termination sequence.

Item 74: Construct according to item 73, wherein one of said control sequences is a seed-specific promoter, preferably a prolamin promoter, most preferably a prolamin promoter from rice.

Item 75: Use of a construct according to any of items 73 to 75 in a method for making plants having enhanced yield-related traits, particularly increased total seed yield per plant, increased number of filled seeds, increased total number of seeds, and increased harvest index, relative to control plants.

Item 76: Plant, plant part or plant cell transformed with a construct according to any of items 74 to 75.

Item 77: Method for the production of a transgenic plant having enhanced-yield related traits relative to control plants, comprising:

(i) introducing and expressing in a plant, plant part, or plant cell, a nucleic acid sequence encoding a GRP polypeptide as defined in item 63 to 65; and
(ii) cultivating the plant, plant part, or plant cell under conditions promoting plant growth and development.

Item 78: Transgenic plant having enhanced yield-related traits, particularly increased total seed yield per plant, increased number of filled seeds, increased total number of seeds, and increased harvest index, relative to control plants, resulting from increased expression of a nucleic acid sequence encoding a GRP polypeptide as defined in items 63 to 65, or a transgenic plant cell derived from said transgenic plant.

Item 79: Transgenic plant according to item 72, 76 or 78, or a transgenic plant cell derived thereof, wherein said plant is a crop plant or a monocot or a cereal, such as rice, maize, wheat, barley, millet, rye, triticale, sorghum and oats.

Item 80: Harvestable parts of a plant comprising a nucleic acid sequence encoding a GRP polypeptide according to item 79, wherein said harvestable parts are preferably seeds.

Item 81: Products derived from a plant according to item 79 and/or from harvestable parts of a plant according to item 80.

Item 81: Use of a nucleic acid sequence encoding a GRP polypeptide in enhancing yield-related traits in plants, particularly in increasing total seed yield per plant, increasing number of filled seeds, increasing total number of seeds, and increasing harvest index, relative to control plants.

In one embodiment of the invention it has now been found that modulating expression in a plant of a nucleic acid encoding an STO polypeptide gives plants having enhanced yield-related traits relative to control plants. According to a first embodiment, the present invention provides a method for enhancing yield-related traits in plants relative to control plants, comprising modulating expression in a plant of a nucleic acid encoding an STO polypeptide.

A preferred method for modulating (preferably, increasing) expression of a nucleic acid encoding an STO polypeptide is by introducing and expressing in a plant a nucleic acid encoding an STO polypeptide.

Any reference hereinafter to a "protein useful in the methods of the invention" is taken to mean an STO polypeptide as defined herein. Any reference hereinafter to a "nucleic acid useful in the methods of the invention" is taken to mean a nucleic acid capable of encoding such an STO polypeptide. The nucleic acid to be introduced into a plant (and therefore useful in performing the methods of the invention) is any nucleic acid encoding the type of protein which will now be described, hereinafter also named "*STO* nucleic acid" or "*STO* gene".

An "STO polypeptide" as defined herein refers to any polypeptide comprising at least one B-box domain. The B-box domain is an amino acid domain conserved in proteins of diverse origin including prokaryotic and eukaryotic organism. B-box domain is about 40 amino acids in length comprising conserved cysteine and/or histidine residues at key positions such that the domain can fall into a tertiary structure similar to the zinc fingers involved in the coordination of zinc ions. NMR analysis reveals that the B-box structure comprises two beta-strands, two helical turns and three extended loop regions different from any other zinc binding motif (Borden et al. 1995 EMBO J. 1995 Dec 1;14(23): 5947-56).

Proteins comprising B-box domains can be found in specific databases such as Pfam and Interpro. The accession number of the B-box domain in the release 15.1 of the Interpro database is IPR000315, and PF00643 in Pfam version 21.0.

Based on sequence comparison of B-box domains found in different proteins it is possible to define consensus sequences representing B-box domains. For example Reymond et al. (EMBO J. 2001 May 1;20(9):2140-51) were able to define consensus sequences to the two B-box domains present in TRIM proteins of Homo sapiens, as represented by CXX(C/D)X(7-12)CXXCXXXXCXX(C/H)X(3-4)HX(4-9)H for B-box1 and CXXHX(7-9)CXX(C/D/E/H)XXXXCXXCX(3-6)H(X-4)(H/C) for B-box2. A consensus sequence found within the B-box domains of many STO proteins of plant origin is represented by SEQ ID NO: 221 (CX2CX16CX2C). A signature characteristic of all B-box domains is the presence of at least 4 amino acid residues, typically Cysteine and/or Histidine residues with the putative capacity to bind zinc ions.

Preferred STO polypeptides useful in the methods of the invention comprise at least one, more preferably two B B-box domain having the consensus represented by CX2CX16CX2C (SEQ ID NO: 221).

The B-box domains found in SEQ ID NO: 169 are represented by SEQ ID NO: 219 and SEQ ID NO: 220. Further preferred STO polypeptides useful in the methods of the invention comprise at least one, more preferably two B-box domains having at least 50%, 55%, 60%, 65%, 70%, 75%, 80%, 85%, 90%, 95% or more sequence identity to SEQ ID NO: 219 or SEQ ID NO: 220.

Alternatively, a B-box domain in an STO polypeptide may be identified by performing a sequence comparison with known polypeptides comprising a B-box domain and establishing the similarity in the region of the B-box domain. The sequences may be aligned using any of the methods well known in the art such as Blast algorithms. The probability for the alignment to occur with a given sequence is taken as basis for identifying similar polypeptides. A parameter that is typically used to represent such probability is called e-value. The E-value is a measure of the reliability of the S score. The S score is a measure of the similarity of the query to the sequence shown. The e-value describes how often a given S score is expected to occur at random. The e-value cut-off may be as high as 1.0. The typical threshold for a trusted e-value from a BLAST search output using an STO polypeptide as query sequence can is lower than $1.e^{-5}$, $1.e^{-10}$, $1.e^{-15}$, $1.e^{-20}$, $1.e^{-25}$, $1.e^{-50}$, $1.e^{-75}$, $1.e^{-100}$, $1.e^{-200}$, $1.e^{-300}$, $1.e^{-400}$, $1.e^{-500}$, $1.e^{-600}$, $1.e^{-700}$ and $1.e^{-800}$. Preferred STO polypeptides useful in the methods of the invention comprise at least one, more preferably two B-box domains having a sequence having in increasing order of preference an e-value lower than $e^{-5}(=10^{-5})$, $1.e^{-10}$, $1.e^{-15}$, $1.e^{-20}$, $1.e^{-25}$, $1.e^{-50}$, $1.e^{-75}$, $1.e^{-100}$, $1.e^{-200}$, $1.e^{-300}$, $1.e^{-400}$, $1.e^{-500}$, $1.e^{-600}$, $1.e^{-700}$ and $1.e^{-800}$ in an alignment with SEQ ID NO: 219 or SEQ ID NO: 220 or any B-box domain as found in a known STO polypeptide, such as those listed in Table A.

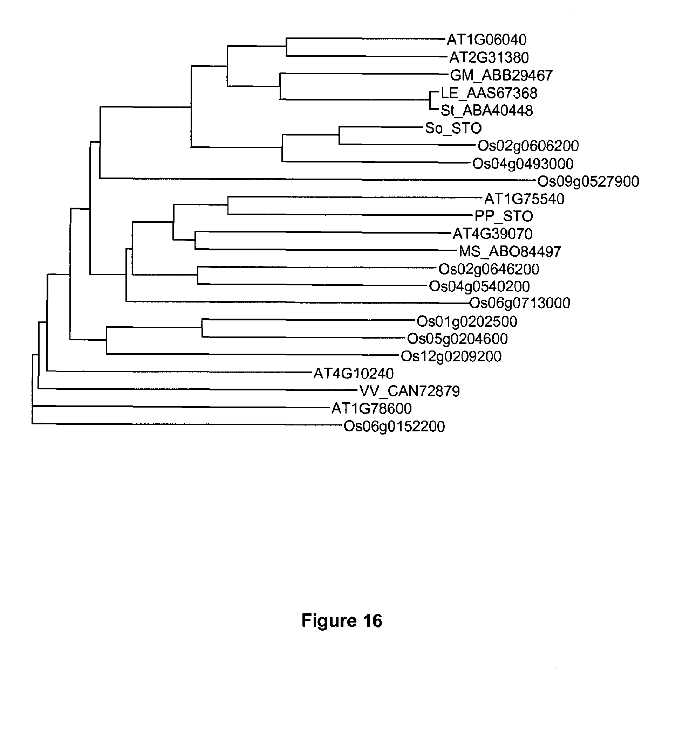

Preferably, the STO polypeptide sequence useful in the methods of the invention when used in the construction of a phylogenetic tree, such as the one depicted in Figure 16, clusters with the group of STO polypeptides comprising the amino acid sequence represented by SEQ ID NO: 169 (OS04g0540200 in the Figure) rather than with any other group.

Alternatively or additionally, a "STO polypeptide" as defined herein refers to any polypeptide having in increasing order of preference at least 50%, 55%, 60%, 65%, 70%, 75%, 80%, 85%, 90%, 95%, 98%, 99% or more polypeptide sequence identity to the STO polypeptide as represented by SEQ ID NO: 169, or by SEQ ID NO: 171, or to any of the polypeptide sequences given in Table A5 of Example 1 herein.

The term "domain" and "motif" is defined in the "definitions" section herein. Specialist databases exist for the identification of domains, for example, SMART (Schultz et al. (1998) Proc. Natl. Acad. Sci. USA 95, 5857-5864; Letunic et al. (2002) Nucleic Acids Res 30, 242-244), InterPro (Mulder et al., (2003) Nucl. Acids. Res. 31, 315-318), Prosite (Bucher and Bairoch (1994), A generalized profile syntax for biomolecular sequences motifs and its function in automatic sequence interpretation. (In) ISMB-94; Proceedings 2nd International Conference on Intelligent Systems for Molecular Biology. Altman R., Brutlag D., Karp P., Lathrop R., Searls D., Eds., pp53-61, AAAI Press, Menlo Park; Hulo et al., Nucl. Acids. Res. 32:D134-D137, (2004)), or Pfam (Bateman et al., Nucleic Acids Research 30(1): 276-280 (2002)). A set of tools for *in silico* analysis of protein sequences is available on the ExPASy proteomics server (Swiss Institute of Bioinformatics (Gasteiger et al., ExPASy: the proteomics server for in-depth protein knowledge and analysis, Nucleic Acids Res. 31:3784-3788(2003)). Domains or motifs may also be identified using routine techniques, such as by sequence alignment.

Methods for the alignment of sequences for comparison are well known in the art, such methods include GAP, BESTFIT, BLAST, FASTA and TFASTA. GAP uses the algorithm of Needleman and Wunsch ((1970) J Mol Biol 48: 443-453) to find the global (i.e. spanning the complete sequences) alignment of two sequences that maximizes the number of matches and minimizes the number of gaps. The BLAST algorithm (Altschul et al. (1990) J Mol Biol 215: 403-10) calculates percent sequence identity and performs a statistical analysis of the similarity between the two sequences. The software for performing BLAST analysis is publicly available through the National Centre for Biotechnology Information (NCBI). Homologues may readily be identified using, for example, the ClustalW multiple sequence alignment algorithm (version 1.83), with the default pairwise alignment parameters, and a scoring method in percentage. Global percentages of similarity and identity may also be determined using one of the methods available in the MatGAT software package (Campanella et al., BMC Bioinformatics. 2003 Jul 10;4:29. MatGAT: an application that generates similarity/identity matrices using protein or DNA sequences.). Minor manual editing may be performed to optimise alignment between conserved motifs, as would be apparent to a person skilled in the art. Furthermore, instead of using full-length sequences for the identification of homologues, specific domains may also be used. The sequence identity values may be determined over the entire nucleic acid or amino acid sequence or over selected domains or conserved motif(s), using the programs mentioned above using the default parameters.

Furthermore, STO polypeptides (at least in their native form) typically when expressed in a transgenic plant, preferably rice, under the control of a constitutive promoter result in modulation of flowering time, typically in shortening flowering time. Tools and techniques for producing transgenic plants and measuring flowering time are well known in the art. Further details are provided in the Examples section.

The present invention is illustrated by transforming plants with the nucleic acid sequence represented by SEQ ID NO: 168, encoding the polypeptide sequence of SEQ ID NO: 169. However, performance of the invention is not restricted to these sequences; the methods of the invention may advantageously be performed using any STO-encoding nucleic acid or STO polypeptide as defined herein.

Examples of nucleic acids encoding STO polypeptides are given in Table A5 of Example 1 herein. Such nucleic acids are useful in performing the methods of the invention. The amino acid sequences given in Table A5 of Example 1 are example sequences of orthologues and paralogues of the STO polypeptide represented by SEQ ID NO: 169, the terms "orthologues" and "paralogues" being as defined herein. Further orthologues and paralogues may readily be identified by performing a so-called reciprocal blast search. Typically, this involves a first BLAST involving BLASTing a query sequence (for example using any of the sequences listed in Table A5 of Example 1) against any sequence database, such as the publicly available NCBI database. BLASTN or TBLASTX (using standard default values) are generally used when starting from a nucleotide sequence, and BLASTP or TBLASTN (using standard default values) when starting from a protein sequence. The BLAST results may optionally be filtered. The full-length sequences of either the filtered results or non-filtered results are then BLASTed back (second BLAST) against sequences from the organism from which the query sequence is derived (where the query sequence is SEQ ID NO: 168 or SEQ ID NO: 169, the second BLAST would therefore be against rice sequences). The results of the first and second BLASTs are then compared. A paralogue is identified if a high-ranking hit from the first blast is from the same species as from which the query sequence is derived, a BLAST back then ideally results in the query sequence amongst the highest hits; an orthologue is identified if a high-ranking hit in the first BLAST is not from the same species as from which the query sequence is derived, and preferably results upon BLAST back in the query sequence being among the highest hits.

Preferred homologs

High-ranking hits are those having a low E-value. The lower the E-value, the more significant the score (or in other words the lower the chance that the hit was found by chance). Computation of the E-value is well known in the art. In addition to E-values, comparisons are also scored by percentage identity. Percentage identity refers to the number of identical nucleotides (or amino acids) between the two compared nucleic acid (or polypeptide) sequences over a particular length. In the case of large families, ClustalW may be used, followed by a neighbour joining tree, to help visualize clustering of related genes and to identify orthologues and paralogues.

Nucleic acid variants may also be useful in practising the methods of the invention. Examples of such variants include nucleic acids encoding homologues and derivatives of any one of the amino acid sequences given in Table A5 of Example 1, the terms "homologue" and "derivative" being as defined herein. Also useful in the methods of the invention are nucleic acids encoding homologues and derivatives of orthologues or paralogues of any one of the amino acid sequences given in Table A5 of Example 1. Homologues and derivatives useful in the methods of the present invention have substantially the same biological and functional activity as the unmodified protein from which they are derived.

Further nucleic acid variants useful in practising the methods of the invention include portions of nucleic acids encoding STO polypeptides, nucleic acids hybridising to nucleic acids encoding STO polypeptides, splice variants of nucleic acids encoding STO polypeptides, allelic variants of nucleic acids encoding STO polypeptides and variants of nucleic acids encoding STO polypeptides obtained by gene shuffling. The terms hybridising sequence, splice variant, allelic variant and gene shuffling are as described herein.

Nucleic acids encoding STO polypeptides need not be full-length nucleic acids, since performance of the methods of the invention does not rely on the use of full-length nucleic acid sequences. According to the present invention, there is provided a method for enhancing yield-related traits in plants, comprising introducing and expressing in a plant a portion of any one of the nucleic acid sequences given in Table A5 of Example 1, or a portion of a nucleic acid encoding an orthologue, paralogue or homologue of any of the amino acid sequences given in Table A5 of Example 1.

A portion of a nucleic acid may be prepared, for example, by making one or more deletions to the nucleic acid. The portions may be used in isolated form or they may be fused to other coding (or non-coding) sequences in order to, for example, produce a protein that combines several activities. When fused to other coding sequences, the resultant polypeptide produced upon translation may be bigger than that predicted for the protein portion.

Portions useful in the methods of the invention, encode an STO polypeptide as defined herein, and have substantially the same biological activity as the amino acid sequences given in Table A5 of Example 1. Preferably, the portion is a portion of any one of the nucleic acids given in Table A of Example 1, or is a portion of a nucleic acid encoding an orthologue or paralogue of any one of the amino acid sequences given in Table A5 of Example 1. Preferably the portion is at least 100, 200, 300, 400, 500, 550, 600, 650, 700, 750, 800, 850, 900, 950, 1000 consecutive nucleotides in length, the consecutive nucleotides being of any one of the nucleic acid sequences given in Table A5 of Example 1, or of a nucleic acid encoding an orthologue or paralogue of any one of the amino acid sequences given in Table A5 of Example 1. Most preferably the portion is a portion of the nucleic acid of SEQ ID NO: 168. Preferably, the portion encodes an amino acid sequence which, when used in the construction of a phylogenetic tree, such as the one depicted in Figure 16, Preferably, the STO polypeptide sequence useful in the methods of the invention when used in the construction of a phylogenetic tree, such as the one depicted in Figure 16, clusters with the group of STO polypeptides comprising the amino acid sequence represented by SEQ ID NO: 169 (OS04g0540200 in the Figure) rather than with any other group.

Another nucleic acid variant useful in the methods of the invention is a nucleic acid capable of hybridising, under reduced stringency conditions, preferably under stringent conditions, with a nucleic acid encoding an STO polypeptide as defined herein, or with a portion as defined herein.

According to the present invention, there is provided a method for enhancing yield-related traits in plants, comprising introducing and expressing in a plant a nucleic acid capable of hybridizing to any one of the nucleic acids given in Table A5 of Example 1, or comprising introducing and expressing in a plant a nucleic acid capable of hybridising to a nucleic acid encoding an orthologue, paralogue or homologue of any of the nucleic acid sequences given in Table A5 of Example 1.

Hybridising sequences useful in the methods of the invention encode an STO polypeptide as defined herein, having substantially the same biological activity as the amino acid sequences given in Table A5 of Example 1. Preferably, the hybridising sequence is capable of hybridising to any one of the nucleic acids given in Table A5 of Example 1, or to a portion of any of these sequences, a portion being as defined above, or the hybridising sequence is capable of hybridising to a nucleic acid encoding an orthologue or paralogue of any one of the amino acid sequences given in Table A5 of Example 1. Most preferably, the hybridising sequence is capable of hybridising to a nucleic acid as represented by SEQ ID NO: 168 or to a portion thereof.

Preferably, the hybridising sequence encodes a polypeptide with an amino acid sequence which, when full-length and used in the construction of a phylogenetic tree, such as the one depicted in Figure 16, clusters with the group of STO polypeptides comprising the amino acid sequence represented by SEQ ID NO: 169 (OS04g0540200 in the Figure) rather than with any other group.

Another nucleic acid variant useful in the methods of the invention is a splice variant encoding an STO polypeptide as defined hereinabove, a splice variant being as defined herein.

According to the present invention, there is provided a method for enhancing yield-related traits in plants, comprising introducing and expressing in a plant a splice variant of any one of the nucleic acid sequences given in Table A5 of Example 1, or a splice variant of a nucleic acid encoding an orthologue, paralogue or homologue of any of the amino acid sequences given in Table A5 of Example 1.

Preferred splice variants are splice variants of a nucleic acid represented by SEQ ID NO: 168, or a splice variant of a nucleic acid encoding an orthologue or paralogue of SEQ ID NO: 169. Preferably, the amino acid sequence encoded by the splice variant, when used in the construction of a phylogenetic tree, such as the one depicted in Figure 16, clusters with the group of STO polypeptides comprising the amino acid sequence represented by SEQ ID NO: 169 (OS04g0540200 in the Figure) rather than with any other group.

Another nucleic acid variant useful in performing the methods of the invention is an allelic variant of a nucleic acid encoding an STO polypeptide as defined hereinabove, an allelic variant being as defined herein.

According to the present invention, there is provided a method for enhancing yield-related traits in plants, comprising introducing and expressing in a plant an allelic variant of any one of the nucleic acids given in Table A5 of Example 1, or comprising introducing and expressing in a plant an allelic variant of a nucleic acid encoding an orthologue, paralogue or homologue of any of the amino acid sequences given in Table A5 of Example 1.

The allelic variants useful in the methods of the present invention have substantially the same biological activity as the STO polypeptide of SEQ ID NO: 169 and any of the amino acids depicted in Table A of Example 1. Allelic variants exist in nature, and encompassed within the methods of the present invention is the use of these natural alleles. Preferably, the allelic variant is an allelic variant of SEQ ID NO: 168 or an allelic variant of a nucleic acid encoding an orthologue or paralogue of SEQ ID NO: 169. Preferably, the amino acid sequence encoded by the allelic variant, when used in the construction of a phylogenetic tree, such as the one depicted in Figure 16, clusters with the group of STO polypeptides comprising the amino acid sequence represented by SEQ ID NO: 169 (OS04g0540200 in the Figure) rather than with any other group.

Gene shuffling or directed evolution may also be used to generate variants of nucleic acids encoding STO polypeptides as defined above; the term "gene shuffling" being as defined herein.

According to the present invention, there is provided a method for enhancing yield-related traits in plants, comprising introducing and expressing in a plant a variant of any one of the nucleic acid sequences given in Table A5 of Example 1, or comprising introducing and expressing in a plant a variant of a nucleic acid encoding an orthologue, paralogue or homologue of any of the amino acid sequences given in Table A5 of Example 1, which variant nucleic acid is obtained by gene shuffling.

Preferably, the amino acid sequence encoded by the variant nucleic acid directly or indirectly obtained by gene shuffling, when used in the construction of a phylogenetic tree such as the one depicted in Figure 16, clusters with the group of STO polypeptides comprising the amino acid sequence represented by SEQ ID NO: 169 (OS04g0540200 in the Figure) rather than with any other group.

Furthermore, nucleic acid variants may also be obtained by site-directed mutagenesis. Several methods are available to achieve site-directed mutagenesis, the most common being PCR based methods (Current Protocols in Molecular Biology. Wiley Eds.).

Nucleic acids encoding STO polypeptides may be derived from any natural or artificial source. The nucleic acid may be modified from its native form in composition and/or genomic environment through deliberate human manipulation. Preferably the STO polypeptide-encoding nucleic acid is from a plant, further preferably from a monocotyledonous plant, more preferably from the family Poaceae, most preferably the nucleic acid is from Oryza sativa.

Performance of the methods of the invention gives plants having enhanced yield-related traits. In particular performance of the methods of the invention gives plants having increased yield, especially increased seed yield relative to control plants. The terms "yield" and "seed yield" are described in more detail in the "definitions" section herein.

Reference herein to enhanced yield-related traits is taken to mean an increase in early (seedling) vigour and altered flowering time more particular shorter of flowering time relative to control plants. Preferably the shortening of the flowering time is at least of 1, 2, 3, 4, 5, 6, 7, 8, 9, 10, 11, 12, 13, 14, 15, 16, 17, 18, 19, 20, 21, 22, 23, 24 or 25 days or at least by 5%, 10%, 15%, 20%, or 25% of the time compared to relative to control plants

Taking corn as an example, a yield increase may be manifested as one or more of the following: increase in the number of plants established per hectare or acre, an increase in the number of ears per plant, an increase in the number of rows, number of kernels per row, kernel weight, thousand kernel weight, ear length/diameter, increase in the seed filling rate (which is the number of filled seeds divided by the total number of seeds and multiplied by 100), among others. Taking rice as an example, a yield increase may manifest itself as an increase in one or more of the following: number of plants per hectare or acre, number of panicles per plant, number of spikelets per panicle, number of flowers (florets) per panicle (which is expressed as a ratio of the number of filled seeds over the number of primary panicles), increase in the seed filling rate (which is the number of filled seeds divided by the total number of seeds and multiplied by 100), increase in thousand kernel weight, among others.

The present invention provides a method for increasing early (seedling) vigour and altering flowering time, particularly shortening of flowering time of plants, relative to control plants which method comprises modulating expression, preferably increasing expression, in a plant of a nucleic acid encoding an STO polypeptide as defined herein.

Since the transgenic plants according to the present invention have increased yield, it is likely that these plants exhibit an increased growth rate (during at least part of their life cycle), relative to the growth rate of control plants at a corresponding stage in their life cycle.

The increased growth rate may be specific to one or more parts of a plant (including seeds), or may be throughout substantially the whole plant. Plants having an increased growth rate may have a shorter life cycle. The life cycle of a plant may be taken to mean the time needed to grow from a dry mature seed up to the stage where the plant has produced dry mature seeds, similar to the starting material. This life cycle may be influenced by factors such as early vigour, growth rate, greenness index, flowering time and speed of seed maturation. The increase in growth rate may take place at one or more stages in the life cycle of a plant or during substantially the whole plant life cycle. Increased growth rate during the early stages in the life cycle of a plant may reflect enhanced or increased vigour. The increase in growth rate may alter the harvest cycle of a plant allowing plants to be sown later and/or harvested sooner than would otherwise be possible (a similar effect may be obtained with earlier flowering time). If the growth rate is sufficiently increased, it may allow for the further sowing of seeds of the same plant species (for example sowing and harvesting of rice plants followed by sowing and harvesting of further rice plants all within one conventional growing period). Similarly, if the growth rate is sufficiently increased, it may allow for the further sowing of seeds of different plants species (for example the sowing and harvesting of corn plants followed by, for example, the sowing and optional harvesting of soybean, potato or any other suitable plant). Harvesting additional times from the same rootstock in the case of some crop plants may also be possible. Altering the harvest cycle of a plant may lead to an increase in annual biomass production per acre (due to an increase in the number of times (say in a year) that any particular plant may be grown and harvested). An increase in growth rate may also allow for the cultivation of transgenic plants in a wider geographical area than their wild-type counterparts, since the territorial limitations for growing a crop are often determined by adverse environmental conditions either at the time of planting (early season) or at the time of harvesting (late season). Such adverse conditions may be avoided if the harvest cycle is shortened. The growth rate may be determined by deriving various parameters from growth curves, such parameters may be: T-Mid (the time taken for plants to reach 50% of their maximal size) and T-90 (time taken for plants to reach 90% of their maximal size), amongst others.

According to a preferred feature of the present invention, performance of the methods of the invention gives plants having an increased growth rate relative to control plants. Therefore, according to the present invention, there is provided a method for increasing the growth rate of plants, which method comprises modulating expression, preferably increasing expression, in a plant of a nucleic acid encoding an STO polypeptide as defined herein.

An increase in yield and/or growth rate occurs whether the plant is under non-stress conditions or whether the plant is exposed to various stresses compared to control plants. Plants typically respond to exposure to stress by growing more slowly. In conditions of severe stress, the plant may even stop growing altogether. Mild stress on the other hand is defined herein as being any stress to which a plant is exposed which does not result in the plant ceasing to grow altogether without the capacity to resume growth. Mild stress in the sense of the invention leads to a reduction in the growth of the stressed plants of less than 40%, 35% or 30%, preferably less than 25%, 20% or 15%, more preferably less than 14%, 13%, 12%, 11% or 10% or less in comparison to the control plant under non-stress conditions. Due to advances in agricultural practices (irrigation, fertilization, pesticide treatments) severe stresses are not often encountered in cultivated crop plants. As a consequence, the compromised growth induced by mild stress is often an undesirable feature for agriculture. Mild stresses are the everyday biotic and/or abiotic (environmental) stresses to which a plant is exposed. Abiotic stresses may be due to drought or excess water, anaerobic stress, salt stress, chemical toxicity, oxidative stress and hot, cold or freezing temperatures. The abiotic stress may be an osmotic stress caused by a water stress (particularly due to drought), salt stress, oxidative stress or an ionic stress. Biotic stresses are typically those stresses caused by pathogens, such as bacteria, viruses, fungi and insects.

In particular, the methods of the present invention may be performed under non-stress conditions or under conditions of mild drought to give plants having increased yield relative to control plants. As reported in Wang et al. (Planta (2003) 218: 1-14), abiotic stress leads to a series of morphological, physiological, biochemical and molecular changes that adversely affect plant growth and productivity. Drought, salinity, extreme temperatures and oxidative stress are known to be interconnected and may induce growth and cellular damage through similar mechanisms. Rabbani et al. (Plant Physiol (2003) 133: 1755-1767) describes a particularly high degree of "cross talk" between drought stress and high-salinity stress. For example, drought and/or salinisation are manifested primarily as osmotic stress, resulting in the disruption of homeostasis and ion distribution in the cell. Oxidative stress, which frequently accompanies high or low temperature, salinity or drought stress, may cause denaturing of functional and structural proteins. As a consequence, these diverse environmental stresses often activate similar cell signalling pathways and cellular responses, such as the production of stress proteins, up-regulation of anti-oxidants, accumulation of compatible solutes and growth arrest. The term "non-stress" conditions as used herein are those environmental conditions that allow optimal growth of plants. Persons skilled in the art are aware of normal soil conditions and climatic conditions for a given location.

Performance of the methods of the invention gives plants grown under non-stress conditions or under mild drought conditions increased yield relative to control plants grown under comparable conditions. Therefore, according to the present invention, there is provided a method for increasing yield in plants grown under non-stress conditions or under mild drought conditions, which method comprises increasing expression in a plant of a nucleic acid encoding an STO polypeptide.

Performance of the methods of the invention gives plants grown under conditions of nutrient deficiency, particularly under conditions of nitrogen deficiency, increased yield relative to control plants grown under comparable conditions. Therefore, according to the present invention, there is provided a method for increasing yield in plants grown under conditions of nutrient deficiency, which method comprises increasing expression in a plant of a nucleic acid encoding an STO polypeptide. Nutrient deficiency may result from a lack of nutrients such as nitrogen, phosphates and other phosphorous-containing compounds, potassium, calcium, cadmium, magnesium, manganese, iron and boron, amongst others.

The present invention encompasses plants or parts thereof (including seeds) obtainable by the methods according to the present invention. The plants or parts thereof comprise a nucleic acid transgene encoding an STO polypeptide as defined above.

The invention also provides genetic constructs and vectors to facilitate introduction and/or expression in plants of nucleic acids encoding STO polypeptides. The gene constructs may be inserted into vectors, which may be commercially available, suitable for transforming into plants and suitable for expression of the gene of interest in the transformed cells. The invention also provides use of a gene construct as defined herein in the methods of the invention.

More specifically, the present invention provides a construct comprising:

(a) a nucleic acid encoding an STO polypeptide as defined above;
(b) one or more control sequences capable of driving expression of the nucleic acid sequence of (a); and optionally
(c) a transcription termination sequence.

Preferably, the nucleic acid encoding an STO polypeptide is as defined above. The term "control sequence" and "termination sequence" are as defined herein.

Plants are transformed with a vector comprising any of the nucleic acids described above. The skilled artisan is well aware of the genetic elements that must be present on the vector in order to successfully transform, select and propagate host cells containing the sequence of interest. The sequence of interest is operably linked to one or more control sequences (at least to a promoter).

Advantageously, any type of promoter, whether natural or synthetic, may be used to drive expression of the nucleic acid sequence. A constitutive promoter is particularly useful in the methods. See the "Definitions" section herein for definitions of the various promoter types.

It should be clear that the applicability of the present invention is not restricted to the STO polypeptide-encoding nucleic acid represented by SEQ ID NO: 168, nor is the applicability of the invention restricted to expression of an STO polypeptide-encoding nucleic acid when driven by a constitutive promoter.

The constitutive promoter is preferably a GOS2 promoter, preferably a GOS2 promoter from rice. Further preferably the constitutive promoter is represented by a nucleic acid sequence substantially similar to SEQ ID NO: 218, most preferably the constitutive promoter is as represented by SEQ ID NO: 218. See Table 2 in the "Definitions" section herein for further examples of constitutive promoters.

Optionally, one or more terminator sequences may be used in the construct introduced into a plant. Additional regulatory elements may include transcriptional as well as translational enhancers. Those skilled in the art will be aware of terminator and enhancer sequences that may be suitable for use in performing the invention. An intron sequence may also be added to the 5' untranslated region (UTR) or in the coding sequence to increase the amount of the mature message that accumulates in the cytosol, as described in the definitions section. Other control sequences (besides promoter, enhancer, silencer, intron sequences, 3'UTR and/or 5'UTR regions) may be protein and/or RNA stabilizing elements. Such sequences would be known or may readily be obtained by a person skilled in the art.

The genetic constructs of the invention may further include an origin of replication sequence that is required for maintenance and/or replication in a specific cell type. One example is when a genetic construct is required to be maintained in a bacterial cell as an episomal genetic element (e.g. plasmid or cosmid molecule). Preferred origins of replication include, but are not limited to, the f1-ori and colE1.

For the detection of the successful transfer of the nucleic acid sequences as used in the methods of the invention and/or selection of transgenic plants comprising these nucleic acids, it is advantageous to use marker genes (or reporter genes). Therefore, the genetic construct may optionally comprise a selectable marker gene. Selectable markers are described in more detail in the "definitions" section herein. The marker genes may be removed or excised from the transgenic cell once they are no longer needed. Techniques for marker removal are known in the art, useful techniques are described above in the definitions section.

The invention also provides a method for the production of transgenic plants having enhanced yield-related traits relative to control plants, comprising introduction and expression in a plant of any nucleic acid encoding an STO polypeptide as defined hereinabove.

More specifically, the present invention provides a method for the production of transgenic plants having increased enhanced yield-related traits, particularly increased (early) seedling vigour and/or altered flowering time more particular shorter flowering time of plants, which method comprises:

(i) introducing and expressing in a plant or plant cell an STO polypeptide-encoding nucleic acid; and
(ii) cultivating the plant cell under conditions promoting plant growth and development.

The nucleic acid of (i) may be any of the nucleic acids capable of encoding an STO polypeptide as defined herein.

The nucleic acid may be introduced directly into a plant cell or into the plant itself (including introduction into a tissue, organ or any other part of a plant). According to a preferred feature of the present invention, the nucleic acid is preferably introduced into a plant by transformation. The term "transformation" is described in more detail in the "definitions" section herein.

The genetically modified plant cells can be regenerated via all methods with which the skilled worker is familiar. Suitable methods can be found in the abovementioned publications by S.D. Kung and R. Wu, Potrykus or Höfgen and Willmitzer.

Generally after transformation, plant cells or cell groupings are selected for the presence of one or more markers which are encoded by plant-expressible genes co-transferred with the gene of interest, following which the transformed material is regenerated into a whole plant. To select transformed plants, the plant material obtained in the transformation is, as a rule, subjected to selective conditions so that transformed plants can be distinguished from untransformed plants. For example, the seeds obtained in the above-described manner can be planted and, after an initial growing period, subjected to a suitable selection by spraying. A further possibility consists in growing the seeds, if appropriate after sterilization, on agar plates using a suitable selection agent so that only the transformed seeds can grow into plants. Alternatively, the transformed plants are screened for the presence of a selectable marker such as the ones described above.

Following DNA transfer and regeneration, putatively transformed plants may also be evaluated, for instance using Southern analysis, for the presence of the gene of interest, copy number and/or genomic organisation. Alternatively or additionally, expression levels of the newly introduced DNA may be monitored using Northern and/or Western analysis, both techniques being well known to persons having ordinary skill in the art.

The generated transformed plants may be propagated by a variety of means, such as by clonal propagation or classical breeding techniques. For example, a first generation (or T1) transformed plant may be selfed and homozygous second-generation (or T2) transformants selected, and the T2 plants may then further be propagated through classical breeding techniques. The generated transformed organisms may take a variety of forms. For example, they may be chimeras of transformed cells and non-transformed cells; clonal transformants (e.g., all cells transformed to contain the expression cassette); grafts of transformed and untransformed tissues (e.g., in plants, a transformed rootstock grafted to an untransformed scion).

The present invention clearly extends to any plant cell or plant produced by any of the methods described herein, and to all plant parts and propagules thereof. The present invention extends further to encompass the progeny of a primary transformed or transfected cell, tissue, organ or whole plant that has been produced by any of the aforementioned methods, the only requirement being that progeny exhibit the same genotypic and/or phenotypic characteristic (s) as those produced by the parent in the methods according to the invention.

The invention also includes host cells containing an isolated nucleic acid encoding an STO polypeptide as defined hereinabove. Preferred host cells according to the invention are plant cells. Host plants for the nucleic acids or the vector used in the method according to the invention, the expression cassette or construct or vector are, in principle, advantageously all plants, which are capable of synthesizing the polypeptides used in the inventive method.

The methods of the invention are advantageously applicable to any plant. Plants that are particularly useful in the methods of the invention include all plants which belong to the superfamily Viridiplantae, in particular monocotyledonous and dicotyledonous plants including fodder or forage legumes, ornamental plants, food crops, trees or shrubs. According to a preferred embodiment of the present invention, the plant is a crop plant. Examples of crop plants include soybean, sunflower, canola, alfalfa, rapeseed, cotton, tomato, potato and tobacco. Further preferably, the plant is a monocotyledonous plant. Examples of monocotyledonous plants include sugarcane. More preferably the plant is a cereal. Examples of cereals include rice, maize, wheat, barley, millet, rye, triticale, sorghum and oats.

The invention also extends to harvestable parts of a plant such as, but not limited to seeds, leaves, fruits, flowers, stems, roots, rhizomes, tubers and bulbs. The invention furthermore relates to products derived, preferably directly derived, from a harvestable part of such a plant, such as dry pellets or powders, oil, fat and fatty acids, starch or proteins.

According to a preferred feature of the invention, the modulated expression is increased expression. Methods for increasing expression of nucleic acids or genes, or gene products, are well documented in the art and examples are provided in the definitions section.

As mentioned above, a preferred method for modulating (preferably, increasing) expression of a nucleic acid encoding an STO polypeptide is by introducing and expressing in a plant a nucleic acid encoding an STO polypeptide; however the effects of performing the method, i.e. enhancing yield-related traits may also be achieved using other well known techniques, including, but not limited to, T-DNA activation tagging, TILLING, homologous recombination. A description of these techniques is provided in the definitions section.

The present invention also encompasses use of nucleic acids encoding STO polypeptides as described herein and use of these STO polypeptides in enhancing any of the aforementioned yield-related traits in plants.

Nucleic acids encoding STO polypeptide described herein, or the STO polypeptides themselves, may find use in breeding programmes in which a DNA marker is identified, which may be genetically linked to an STO polypeptide-encoding gene. The nucleic acids/genes, or the STO polypeptides themselves may be used to define a molecular marker. This DNA or protein marker may then be used in breeding programmes to select plants having enhanced yield-related traits as defined hereinabove in the methods of the invention.

Allelic variants of an STO polypeptide-encoding nucleic acid/gene may also find use in marker-assisted breeding programmes. Such breeding programmes sometimes require introduction of allelic variation by mutagenic treatment of the plants, using for example EMS mutagenesis; alternatively, the programme may start with a collection of allelic variants of so called "natural" origin caused unintentionally. Identification of allelic variants then takes place, for example, by PCR. This is followed by a step for selection of superior allelic variants of the sequence in question and which give increased yield. Selection is typically carried out by monitoring growth performance of plants containing different allelic variants of the sequence in question. Growth performance may be monitored in a greenhouse or in the field. Further optional steps include crossing plants in which the superior allelic variant was identified with another plant. This could be used, for example, to make a combination of interesting phenotypic features.

Nucleic acids encoding STO polypeptides may also be used as probes for genetically and physically mapping the genes that they are a part of, and as markers for traits linked to those genes. Such information may be useful in plant breeding in order to develop lines with desired phenotypes. Such use of STO polypeptide-encoding nucleic acids requires only a nucleic acid sequence of at least 15 nucleotides in length. The STO polypeptide-encoding nucleic acids may be used as restriction fragment length polymorphism (RFLP) markers. Southern blots (Sambrook J, Fritsch EF and Maniatis T (1989) Molecular Cloning, A Laboratory Manual) of restriction-digested plant genomic DNA may be probed with the STO-encoding nucleic acids. The resulting banding patterns may then be subjected to genetic analyses using computer programs such as MapMaker (Lander et al. (1987) Genomics 1: 174-181) in order to construct a genetic map.

In addition, the nucleic acids may be used to probe Southern blots containing restriction endonuclease-treated genomic DNAs of a set of individuals representing parent and progeny of a defined genetic cross. Segregation of the DNA polymorphisms is noted and used to calculate the position of the STO polypeptide-encoding nucleic acid in the genetic map previously obtained using this population (Botstein et al. (1980) Am. J. Hum. Genet. 32:314-331).

The production and use of plant gene-derived probes for use in genetic mapping is described in Bernatzky and Tanksley (1986) Plant Mol. Biol. Reporter 4: 37-41. Numerous publications describe genetic mapping of specific cDNA clones using the methodology outlined above or variations thereof. For example, F2 intercross populations, backcross populations, randomly mated populations, near isogenic lines, and other sets of individuals may be used for mapping. Such methodologies are well known to those skilled in the art.

The nucleic acid probes may also be used for physical mapping (i.e., placement of sequences on physical maps; see Hoheisel et al. In: Non-mammalian Genomic Analysis: A Practical Guide, Academic press 1996, pp. 319-346, and references cited therein).

In another embodiment, the nucleic acid probes may be used in direct fluorescence in situ hybridisation (FISH) mapping (Trask (1991) Trends Genet. 7:149-154). Although current methods of FISH mapping favour use of large clones (several kb to several hundred kb; see Laan et al. (1995) Genome Res. 5:13-20), improvements in sensitivity may allow performance of FISH mapping using shorter probes.

A variety of nucleic acid amplification-based methods for genetic and physical mapping may be carried out using the nucleic acids. Examples include allele-specific amplification (Kazazian (1989) J. Lab. Clin. Med 11:95-96), polymorphism of PCR-amplified fragments (CAPS; Sheffield et al. (1993) Genomics 16:325-332), allele-specific ligation (Landegren et al. (1988) Science 241:1077-1080), nucleotide extension reactions (Sokolov (1990) Nucleic Acid Res. 18:3671), Radiation Hybrid Mapping (Walter et al. (1997) Nat. Genet. 7:22-28) and Happy Mapping (Dear and Cook (1989) Nucleic Acid Res. 17:6795-6807). For these methods, the sequence of a nucleic acid is used to design and produce primer pairs for use in the amplification reaction or in primer extension reactions. The design of such primers is well known to those skilled in the art. In methods employing PCR-based genetic mapping, it may be necessary to identify DNA sequence differences between the parents of the mapping cross in the region corresponding to the instant nucleic acid sequence. This, however, is generally not necessary for mapping methods.

The methods according to the present invention result in plants having enhanced yield-related traits, as described hereinbefore. These traits may also be combined with other economically advantageous traits, such as further yield-enhancing traits, tolerance to other abiotic and biotic stresses, traits modifying various architectural features and/or biochemical and/or physiological features.

The term "table A5" used in this specification is to be taken to specify the content of table A5a and table A5b. The term "table A5a" used in this specification is to be taken to specify the content of table A5a. The term "table A5b" used in this specification is to be taken to specify the content of table A5b. In one preferred embodiment, the term "table A5" means table A5b.

In one embodiment the invention relates to subject mater summarized as follows:

Item 82: A method for enhancing yield-related traits in plants relative to control plants, comprising modulating expression in a plant of a nucleic acid encoding an STO polypeptide, wherein said STO polypeptide comprises a B-box domain.

Item 83: Method according to item 82, wherein said B-box domain comprises a consensus sequences as represented by SEQ ID NO: 221.

Item 84: Method according to items 82 or 83, wherein said STO polypeptides comprises a B-box domain having at least 50%, 55%, 60%, 65%, 70%, 75%, 80%, 85%, 90%, 95% or more sequence identity to SEQ ID NO: 219 or SEQ ID NO: 220.

Item 85: Method according to any of items 82 to 84, wherein said modulated expression is effected by introducing and expressing in a plant a nucleic acid encoding an STO polypeptide.

Item 86: Method according to any of items 82 to 85, wherein said nucleic acid encoding an STO polypeptide encodes any one of the proteins listed in Table A5 or is a portion of such a nucleic acid, or a nucleic acid capable of hybridising with such a nucleic acid.

Item 87: Method according to any of items 82 to 86, wherein said nucleic acid sequence encodes an orthologue or paralogue of any of the proteins given in Table A5.

Item 88: Method according to any of items 82 to 87, wherein said enhanced yield-related traits comprise increased seedling vigour and/or altered flowering time preferably shorter flowering time relative to control plants.

Item 89: Method according to any of items 82 to 88, wherein said enhanced yield-related traits are obtained under non-stress conditions.

Item 90: Method according to any of items 85 to 89, wherein said nucleic acid is operably linked to a constitutive promoter, preferably to a GOS2 promoter, most preferably to a GOS2 promoter from rice.

Item 91: Method according to any of items 82 to 90, wherein said nucleic acid encoding an STO polypeptide is of plant origin, preferably from a monocotyledonous plant, further preferably from the family Poaceae, more preferably from the genus Oryza, most preferably from Oryza sativa.

Item 92: Plant or part thereof, including seeds, obtainable by a method according to any preceding claim, wherein said plant or part thereof comprises a recombinant nucleic acid encoding an STO polypeptide.

Item 93: An isolated nucleic acid sequence comprising a nucleic acid sequence selected from the group consisting of:

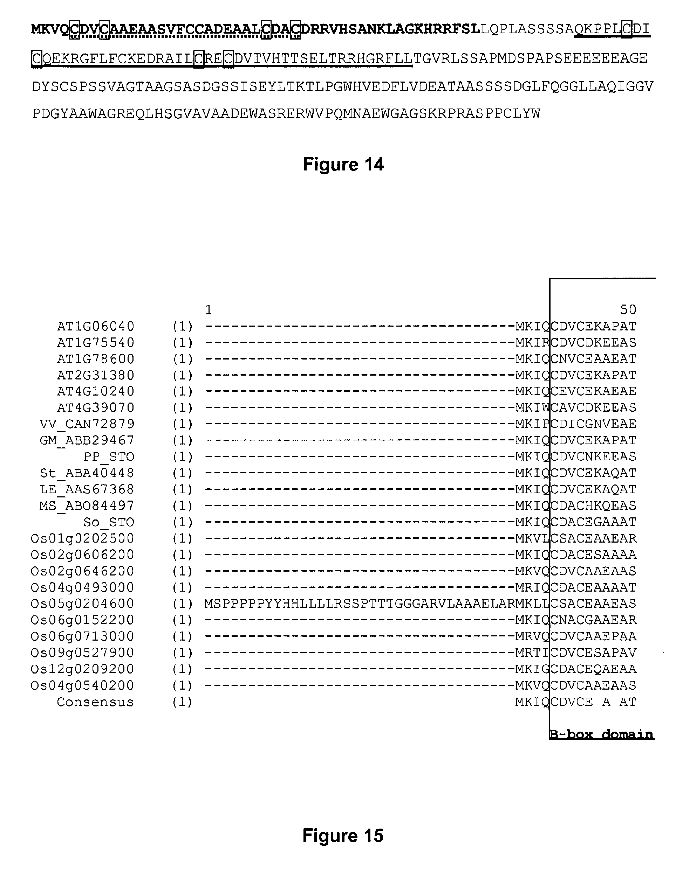

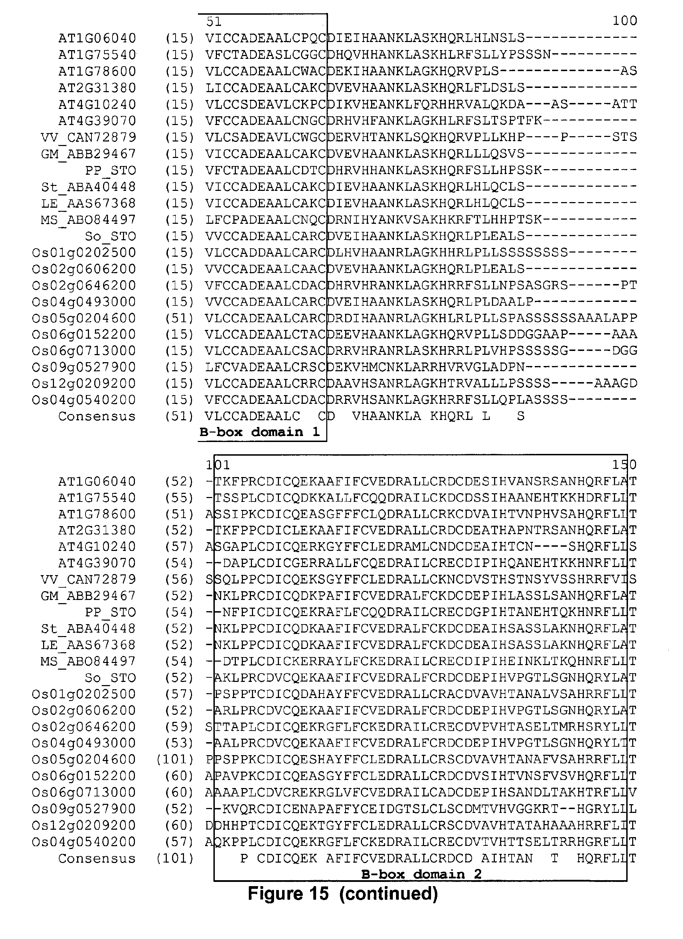

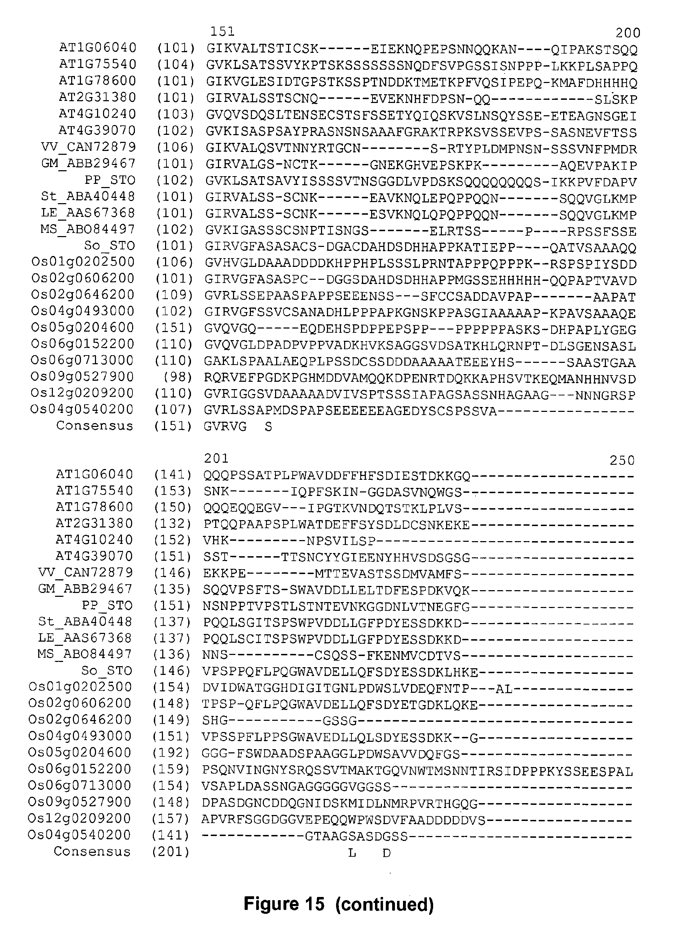

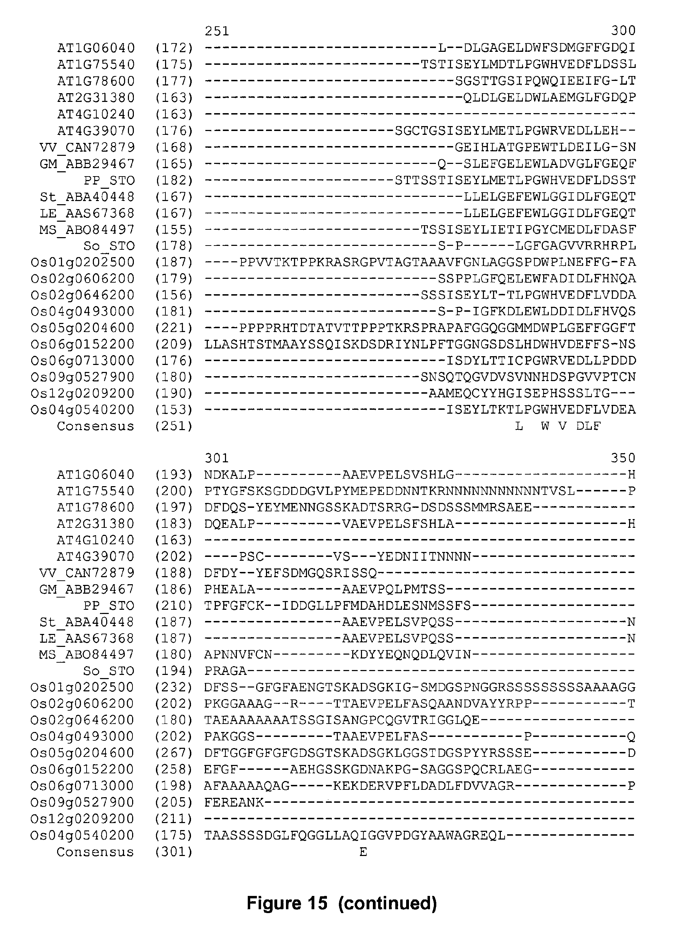

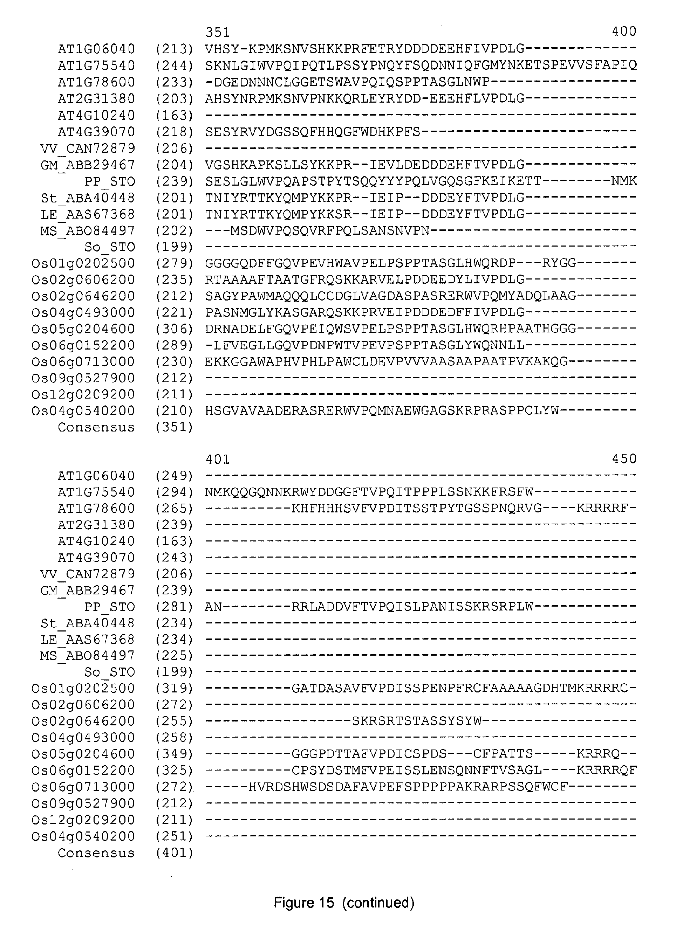

(a) an isolated nucleic acid molecule encoding the polypeptide as depicted in SEQ ID NO: 223 and/or table A5b;
(b) an isolated nucleic acid molecule as depicted in SEQ ID NO: 222 and/or table A5b;
(c) an isolated nucleic acid molecule, which, as a result of the degeneracy of the genetic code, can be derived from a polypeptide sequence depicted in table A5 and confers enhanced yield-related traits relative to control plants;
(d) an isolated nucleic acid molecule having at least 30 %, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity with the nucleic acid molecule sequence of a polynucleotide comprising the nucleic acid molecule depicted in table A5 and confers enhanced yield-related traits relative to control plants;
(e) an isolated nucleic acid molecule encoding a polypeptide having at least 30 %, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity with the amino acid sequence of the polypeptide encoded by the nucleic acid molecule of (a) to (c) and confers enhanced yield-related traits relative to control plants;
(f) an isolated nucleic acid molecule which hybridizes with a nucleic acid molecule of (a) to (c) under stringent hybridization conditions and confers enhanced yield-related traits relative to control plants;
(g) an isolated nucleic acid molecule encoding a polypeptide comprising the consensus sequence or one or more polypeptide motifs as shown figure 15 and preferably having the activity represented by a nucleic acid molecule comprising a polynucleotide as depicted in table A5;
(h) an isolated nucleic acid molecule encoding a polypeptide having the activity represented by a protein as depicted in table A5 and confering enhanced yield-related traits relative to control plants;
and
(i) a nucleic acid molecule which is obtainable by screening a suitable nucleic acid library under stringent hybridization conditions with a probe comprising a complementary sequence of a nucleic acid molecule of (a) or (b) or with a fragment thereof, having at least 15 nt, preferably 20 nt, 30 nt, 50 nt, 100 nt, 200 nt or 500 nt of a nucleic acid molecule complementary to a nucleic acid molecule sequence characterized in (a) to (e) and encoding a polypeptide having the activity represented by a protein comprising a polypeptide as depicted in table A5;

whereby the nucleic acid molecule according to (a) to (i) is at least in one or more nucleotides different from the sequence depicted in table A5a and preferably which encodes a protein which differs at least in one or more amino acids from the protein sequences depicted in table A5a.

Item 94: Construct comprising:

(a) nucleic acid encoding an STO polypeptide as defined in any of items 82 to 84 and 93;
(b) one or more control sequences capable of driving expression of the nucleic acid sequence of (a); and optionally

(c) a transcription termination sequence.

Item 95: Construct according to item 94, wherein one of said control sequences is a constitutive promoter, preferably a GOS2 promoter, most preferably a GOS2 promoter from rice.

Item 96:Use of a construct according to any of items 94 to 95 in a method for making plants having enhanced yield-related traits, preferably increased seedling vigour and/or altered flowering time, particularly shorter flowering time relative to control plants.

Item 97: Plant, plant part or plant cell transformed with a construct according to any of items 94 to 95.

Item 98: Method for the production of a transgenic plant having enhanced yield-related traits, preferably increased seedling vigour and/or altered flowering time, particularly shorter flowering time relative to control plants, comprising:

(i) introducing and expressing in a plant a nucleic acid encoding an STO polypeptide as defined in any of items 82 to 84 and 93; and
(ii) cultivating the plant cell under conditions promoting plant growth and development.

Item 99: Transgenic plant having enhanced yield-related traits, preferably increased seedling vigour and/or altered flowering time, particularly shorter flowering time relative to control plants, resulting from increased expression of a nucleic acid encoding an STO polypeptide as defined in any of items 82 to 84 and 93, or a transgenic plant cell derived from said transgenic plant.

Item 100: Transgenic plant according to item 92, 97 or 99, or a transgenic plant cell derived thereof, wherein said plant is a crop plant or a monocot or a cereal, such as rice, maize, wheat, barley, millet, rye, triticale, sorghum and oats.

Item 101: Harvestable parts of a plant according to item 100, wherein said harvestable parts are preferably shoot biomass and/or seeds.

Item 102: Products derived from a plant according to item 100 and/or from harvestable parts of a plant according to item 101.

Item 103: Use of a nucleic acid encoding an STO polypeptide in increasing seedling vigour and/or altering flowering time, particularly shortening flowering time relative to control plants.

According to a further embodiment of the present invention, there is also provided an isolated polypeptide comprising a polypeptide sequence selected from the group consisting of:

(i) an amino acid sequence encoded by any one of the nucleic acids represented by Item 93;
(ii) an amino acid sequence having, in increasing order of preference, at least 50%, 55%, 60%, 65%, 70%, 75%, 80%, 85%, 90%, 95%, 96%, 97%, 98%, 99% or more sequence identity to the amino acid sequence of (i);
(iii) derivatives of any of the amino acid sequences given in (i) or (ii) above.

In a further embodiment of the invention it has now been found that modulating expression in a plant of a nucleic acid encoding a UGE polypeptide gives plants having enhanced yield-related traits relative to control plants. According to a first embodiment, the present invention provides a method for enhancing yield-related traits in plants relative to control plants, comprising modulating expression in a plant of a nucleic acid encoding a UGE polypeptide.

A preferred method for modulating (preferably, increasing) expression of a nucleic acid encoding a UGE polypeptide is by introducing and expressing in a plant a nucleic acid encoding a UGE polypeptide.

Any reference hereinafter to a "protein useful in the methods of the invention" is taken to mean a UGE polypeptide. Any reference hereinafter to a "nucleic acid useful in the methods of the invention" is taken to mean a nucleic acid capable of encoding such a UGE polypeptide. The nucleic acid to be introduced into a plant (and therefore useful in performing the methods of the invention) is any nucleic acid encoding the type of protein which will now be described, hereinafter also named "UGE nucleic acid" or "UGE gene". UGE polypeptides and UGE nucleic acids have been described before (Maxwell and Robichon-Szulmajster (1960); J. Biol. Chem. 235 (1960) 308-312; Wilson, D.B. and Hogness, D.S. (1964). J. Biol. Chem. 239 2469-2481; reviewed by Frey FASEB J. 1996 Mar;10(4):461-70; Thoden et al. (1997). Biochemistry 36:6294-6304; Dormann and Benning 1998; Shaw et al. 2003. Mol. Biochem. Parasitol. 126, 173-180; Barber et al. 2006).

A "UGE polypeptide" comprises an epimerase domain and typically has UDP-Glucose-4-epimerase activity. Epimerase domain refers to a sequence of varying length, in average around 200 amino acids long, conserved amongst epimerases (InterPro reference IPR005886; Pfam reference: PF01370).

UGE polypeptides can be found by searching in specialized databases such as Pfam, (Finn et al. Nucleic Acids Research (2006) Database Issue 34:D247-D251). Pfam compiles a large collection of multiple sequence alignments and hidden Markov models (HMM) covering many common protein domains and families and is available through the Sanger Institute in the United Kingdom. Trusted matches as considered in the Pfam database are those sequences scoring higher than the gathering cut-off threshold. The gathering cutoff threshold of the epimerase domain in the Pfam HMM_fs method is -46.3 and in the Pfam HMM_ls method is 16.7. However potential matches, comprising true epimerase domains, may still fall under the gathering cut-off. Preferably a UGE polypeptide useful in the methods of the invention is a protein having one or more domains in their sequence that exceed the gathering cutoff of the Pfam protein domain family PF01370, known as epimerase or NAD dependent epimerase/dehydratase family domain.

Alternatively, an epimerase domain in a polypeptide may be identified by performing a sequence comparison with known polypeptides comprising a epimerase domain and establishing the similarity in the region of the epimerase domain. The sequences may be aligned using any of the methods well known in the art such as Blast algorithms. The probability for the alignment to occur with a given sequence is taken as basis for identifying similar polypeptides. A parameter that is typically used to represent such probability is called e-value. The E-value is a measure of the reliability of the S score. The S score is a measure of the similarity of the query to the sequence shown. The e-value describes how often a given S score is expected to occur at random. The e-value cut-off may be as high as 1.0. The typical threshold for a trusted e-value from a BLAST search output using an epimerase polypeptide as query sequence is lower than e5 (=10-5), 1.e-10, 1.e-15, 1.e-20, 1.e-25, 1.e-50, 1.e-75, 1.e-100, 1.e-200, 1.e-300, 1.e-400, 1.e-500, 1.e-600, 1.e-700 and 1.e-800. Preferably UGE polypeptides useful in the methods of the invention comprise a sequence having in increasing order of preference an e-value lower than e-5(=10-5), 1.e-10, 1.e-15, 1.e-20, 1.e-25, 1.e-50, 1.e-75, 1.e-100, 1.e-200, 1.e-300, 1.e-400, 1.e-500, 1.e-600, 1.e-700 and 1.e-800 in an alignment with an epimerase domain found in a known UGE polypeptide, such as for example SEQ ID NO: 225.

A preferred UGE polypeptide useful in the methods of the invention refers to a polypeptide comprising an epimerase domain, such domain having in increasing order of preference at least 70%, 75%, 80%, 85%, 90%, 95%, 97% or more sequence identity to SEQ ID NO: 275 (sequence representing the epimerase domain comprised in SEQ ID NO: 225).

Examples of UGE polypeptides useful in the methods of the invention are given in Table A6. The amino acid coordinates of the epimerase domain in the representative UGE protein as represented by SEQ ID NO: 225 are given in Table C. The sequence of the epimerase domain comprised in SEQ ID NO: 225 is given in SEQ ID NO: 275).

Preferred UGE polypeptides useful in the methods of the invention are those having in increasing order of preference at least 70%, 75%, 80%, 85%, 90%, 95%, 96%; 98% or more sequence identity to any of the polypeptides given in Table A6.

UGE polypeptides typically comprise conserved motifs relevant to their enzymatic activity such as i) Motif 8 involved in binding of the UGE protein to the cofactor NAD+ and represented by GXXGXXG (SEQ ID NO: 276) and ii) Motif 9 located in the active site as represented by YGRT/S (SEQ ID NO: 277), wherein the G can be substituted by any other non polar amino acid and the R by any other polar amino acid.

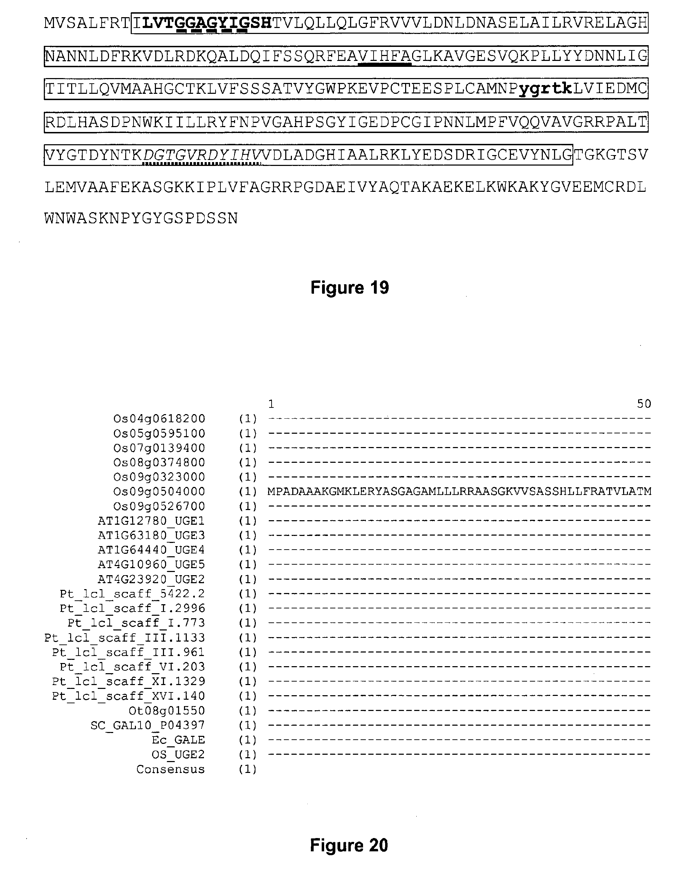

UGE polypeptides may comprise in addition to the epimerase domain one or more of the following conserved motifs: (i) Motif 10 as represented by SEQ ID NO: 278, which comprises the NAD+ binding motif, (ii) Motif 11 as represented by SEQ ID NO: 279 and iii) Motif 12 as represented by SEQ ID: 280. Figure 19 shows the conserved motifs Motif 8 to Motif 12 and their relative position in the UGE polypeptide sequence represented by SEQ ID NO: 225.

Therefore, preferred UGE polypeptides useful in the method of the invention, comprise in addition to the epimerase domain any one or more of the following conserved motifs:

(i) Motif 8 as represented by SEQ ID NO: 276;
(ii) Motif 9 as represented by SEQ ID NO: 277 wherein the G can be substituted by any other non polar amino acid and the R by any other polar amino acid ;
(iii) Motif 10 as represented by SEQ ID NO: 278, wherein 1, 2, 3 or 4 mismatches are allowed;
(iv) Motif 11 as represented by SEQ ID NO: 279,
(v) Motif 12 as represented by SEQ ID NO: 280, wherein 1, 2, 3 or 4 mismatches are allowed.

The polarity of the 20 essential amino acids in given in Table 3. Most frequently the amino acids comprised in polypeptides are alpha amino acids having an amine and a carboxyl group attached to the same carbon, the alpha carbon, which amino acid molecules often comprise a side chain attached to the alpha carbon. Table 3 shows the classification of amino acids based on the physical and biochemical properties of the side chain.

Table 3: Classification of amino acids according to the side chain properties.

| Amino Acid | 3-Letter | 1-Letter | Side chain polarity | Side chain acidity or basicity | Hydropathy index |
|---|---|---|---|---|---|
| Arginine | Arg | R | polar | basic | -4.5 |
| Asparagine | Asn | N | polar | neutral | -3.5 |
| Aspartic acid | Asp | D | polar | acidic | -3.5 |
| Cysteine | Cys | C | polar | neutral | 2.5 |
| Glutamic acid | Glu | E | polar | acidic | -3.5 |
| Glutamine | Gln | Q | polar | neutral | -3.5 |
| Histidine | His | H | polar | basic | -3.2 |
| Lysine | Lys | K | polar | basic | -3.9 |
| Serine | Ser | S | polar | neutral | -0.8 |
| Threonine | Thr | T | polar | neutral | -0.7 |
| Tyrosine | Tyr | Y | polar | neutral | -1.3 |
| Alanine | Ala | A | nonpolar | neutral | 1.8 |
| Glycine | Gly | G | nonpolar | neutral | -0.4 |
| Isoleucine | Ile | I | nonpolar | neutral | 4.5 |
| Leucine | Leu | L | nonpolar | neutral | 3.8 |
| Methionine | Met | M | nonpolar | neutral | 1.9 |
| Phenylalanine | Phe | F | nonpolar | neutral | 2.8 |
| Proline | Pro | P | nonpolar | neutral | -1.6 |
| Tryptophan | Trp | W | nonpolar | neutral | -0.9 |
| Valine | Val | V | nonpolar | neutral | 4.2 |

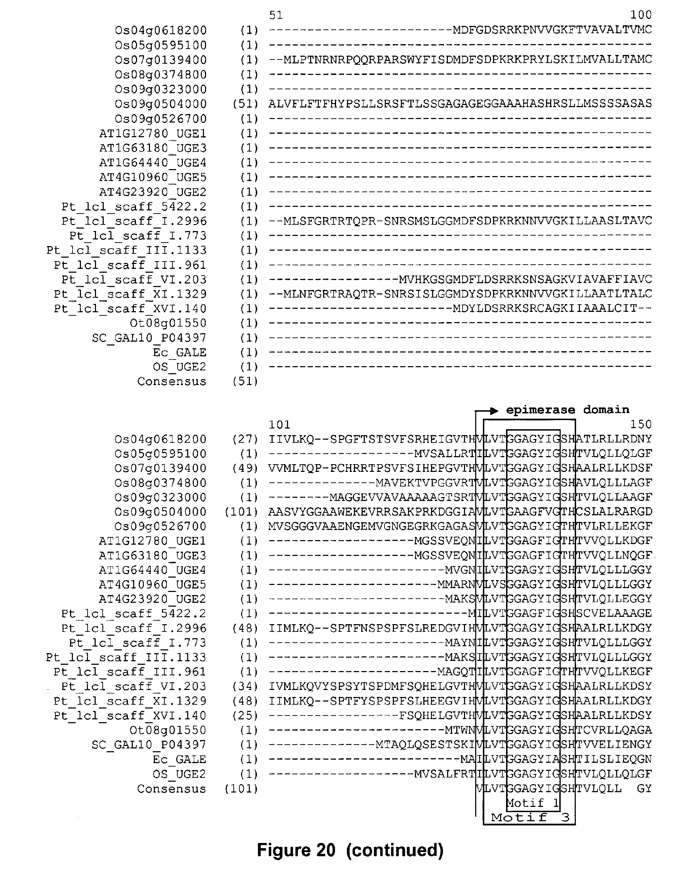

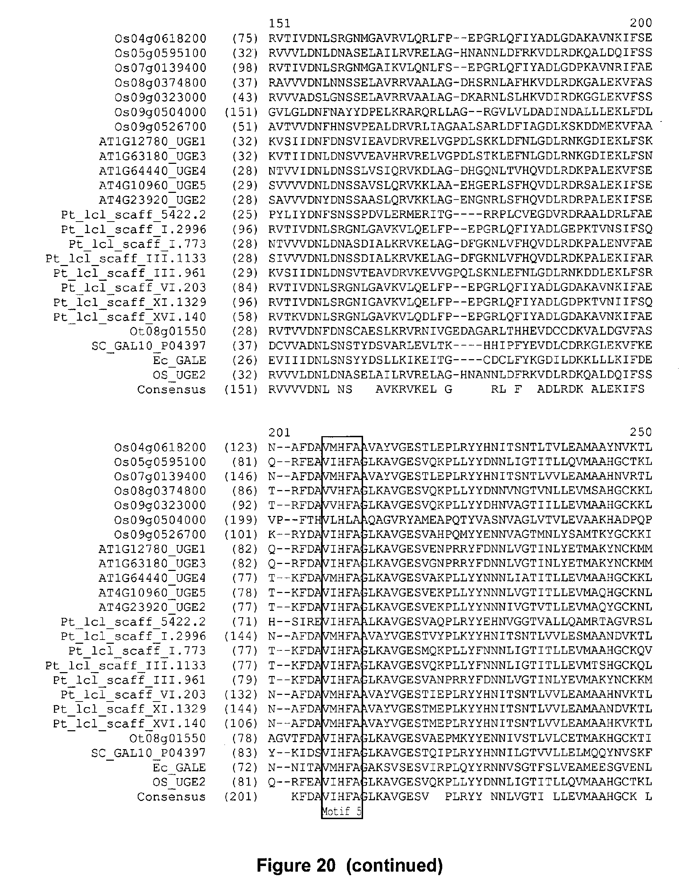

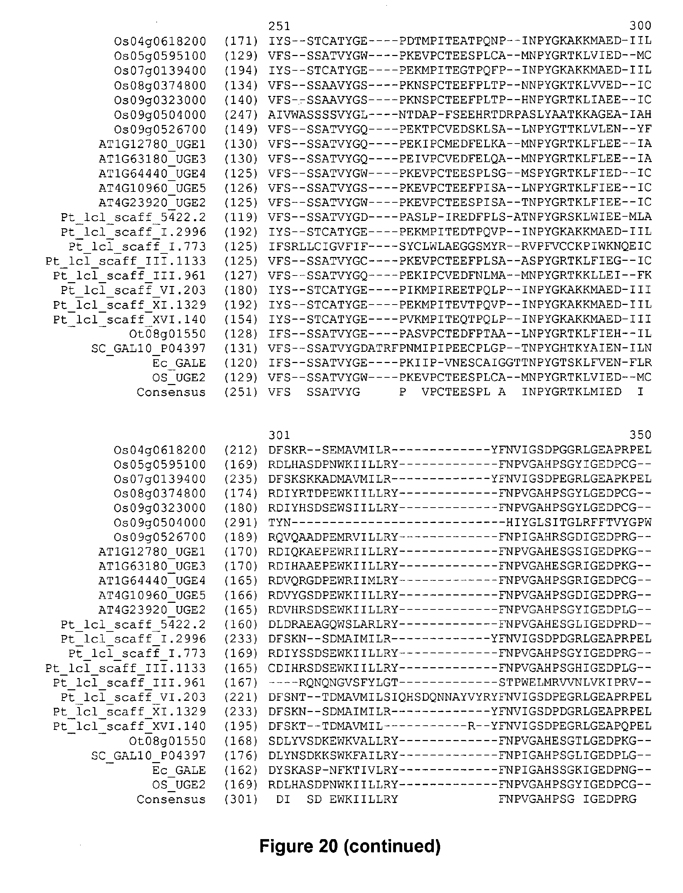

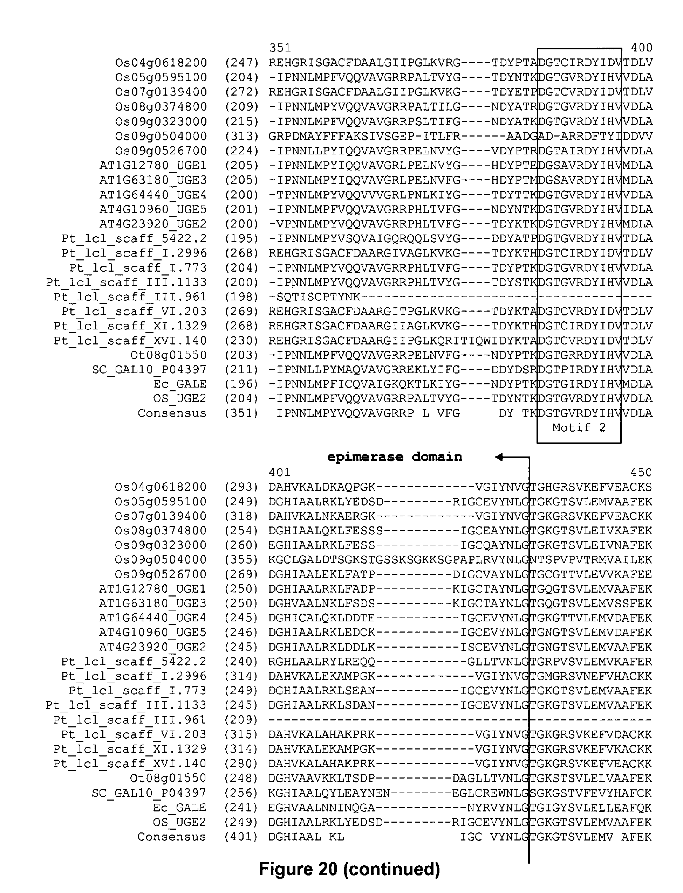

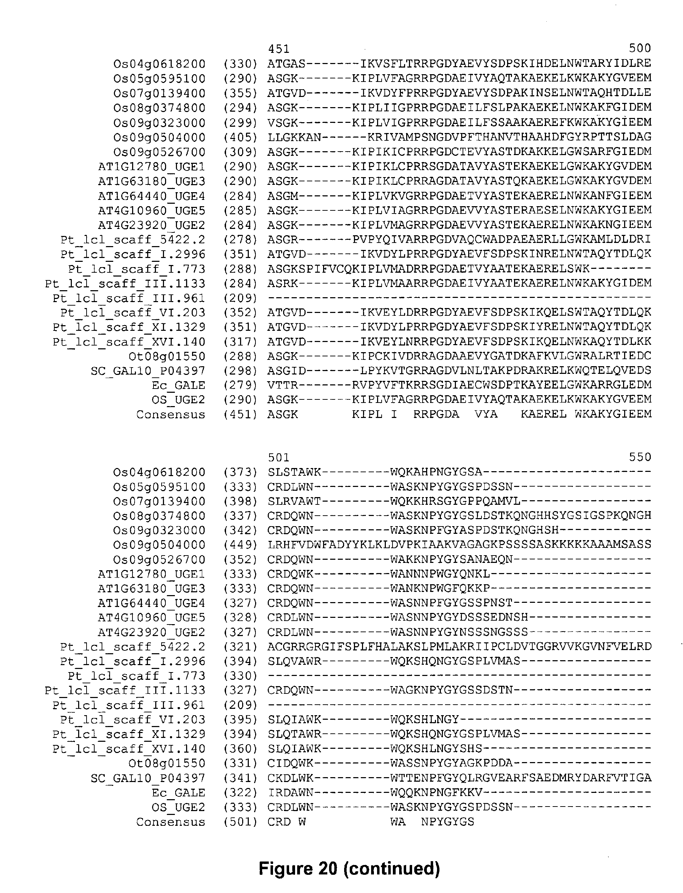

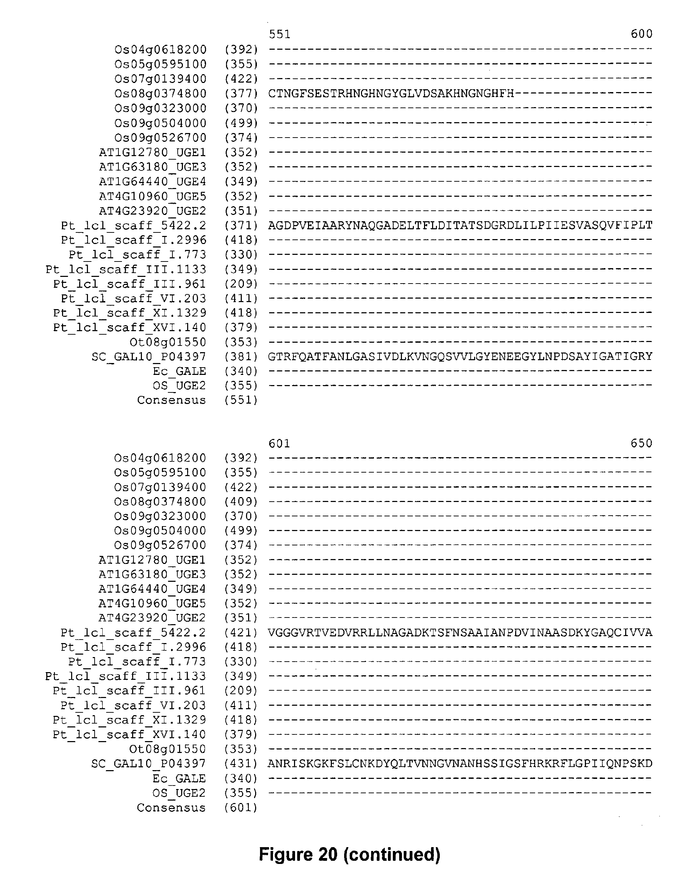

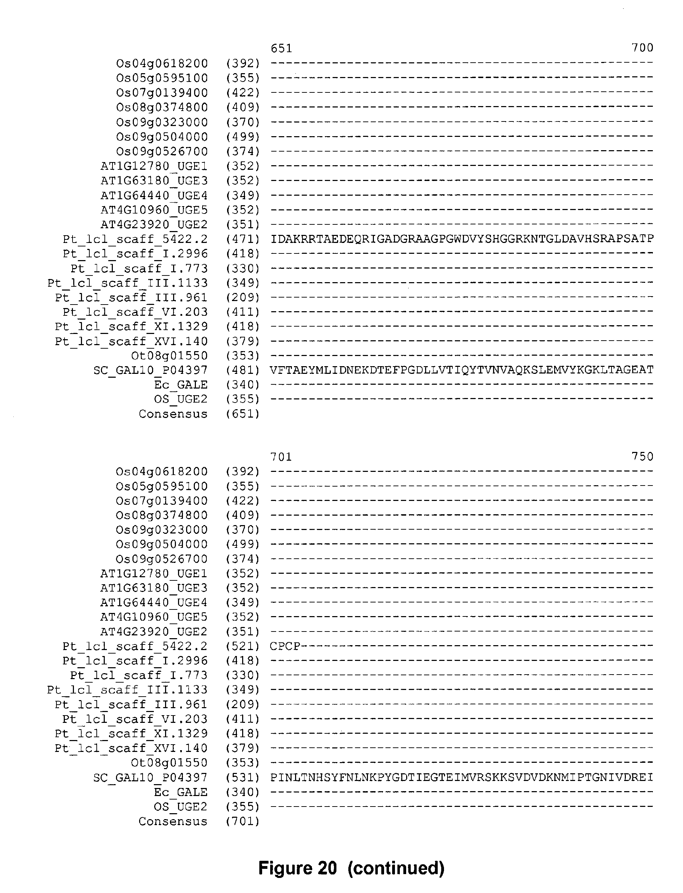

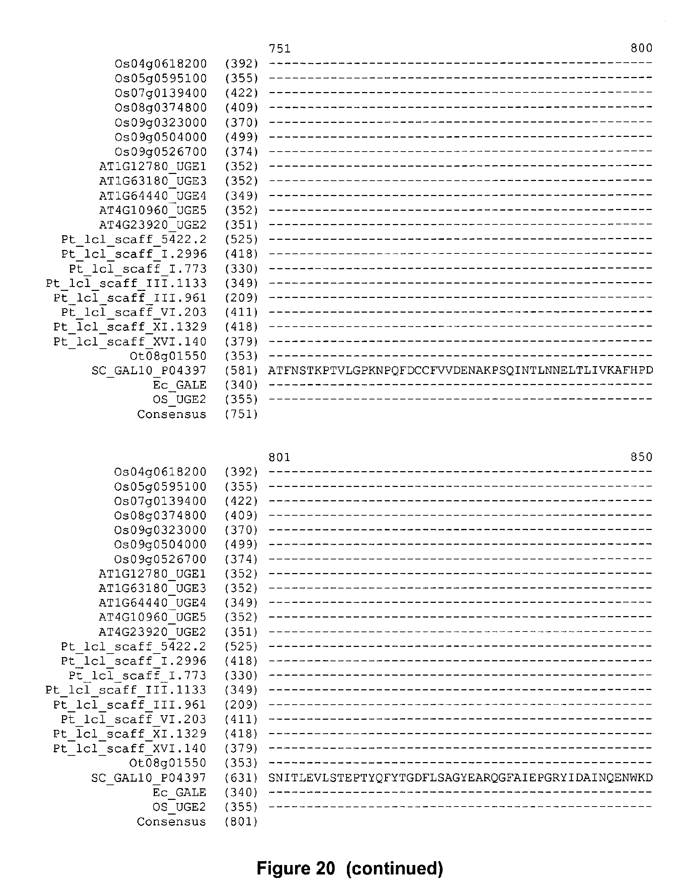

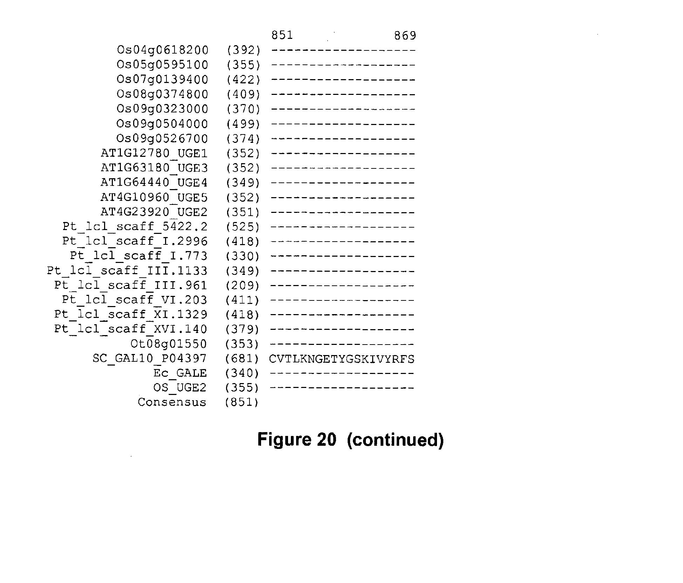

Examples of UGE polypeptides comprising one or more of the conserved motifs Motif 8 to Motif 12 are given in Example 1. Figure 20 shows the position of the conserved motifs in those UGE polypeptides.

Additionally UGE polypeptides may comprise a signal polypeptide functional in subcellular localisation of the protein in a cell. For example SEQ ID NO: 225 comprises a putative secretory pathway signal peptide located between amino acid 13 and 37, with a putative cleavage site between amino acid 36 and 37.

UGE polypeptides may be modified further in the cell, for example, by cleavage of signal peptides to produce a so-called, mature UGE polypeptide. A preferred derivative of a UGE polypeptide useful in the methods of the invention is that resulting from removal of the signalling peptide and corresponding to the mature UGE polypeptide.

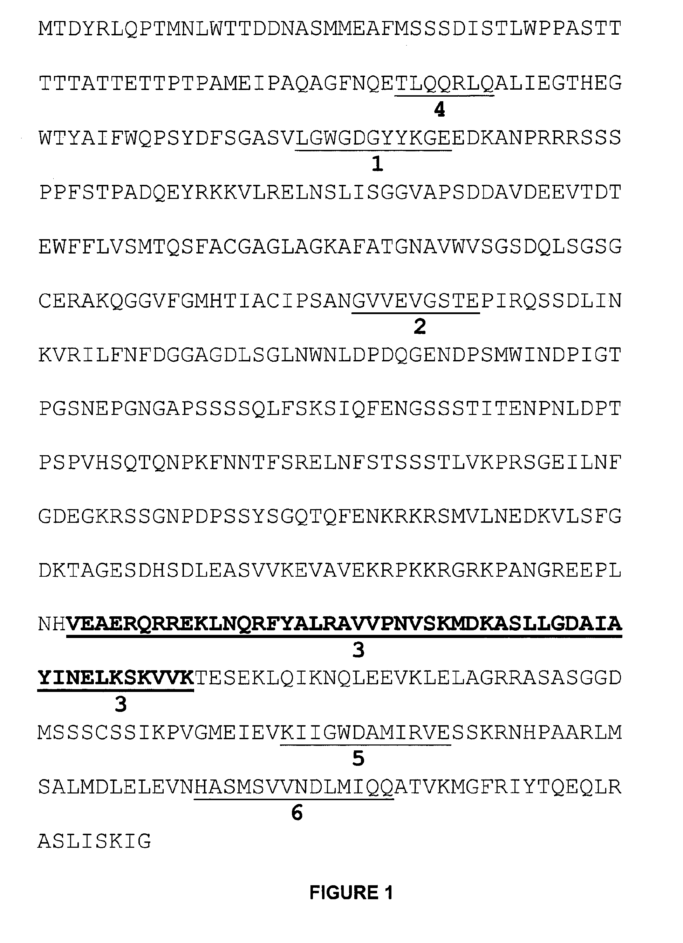

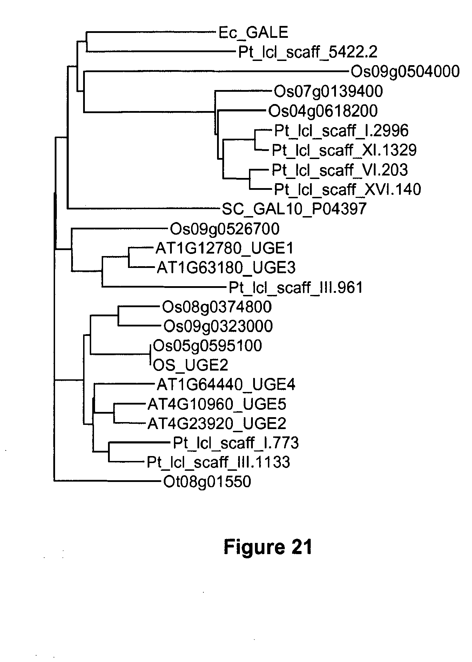

Preferably, the polypeptide sequence which when used in the construction of a phylogenetic tree, such as the one depicted in Figure 21, clusters with the group comprising the amino acid sequence represented by SEQ ID NO: 225 (Os_UGE2) or alternatively, when in a tree such as that of Figure 1 in Rosti et al. 2007 (The Plant Cell 19:1565-1579) clusters with group comprising AtUGE2.

The term "domain" and "motif" is defined in the "definitions" section herein. Specialist databases exist for the identification of domains, for example, SMART (Schultz et al. (1998) Proc. Natl. Acad. Sci. USA 95, 5857-5864; Letunic et al. (2002) Nucleic Acids Res 30, 242-244), InterPro (Mulder et al., (2003) Nucl. Acids. Res. 31, 315-318), Prosite (Bucher and Bairoch (1994), A generalized profile syntax for biomolecular sequences motifs and its function in automatic sequence interpretation. (In) ISMB-94; Proceedings 2nd International Conference on Intelligent Systems for Molecular Biology. Altman R., Brutlag D., Karp P., Lathrop R., Searls D., Eds., pp53-61, AAAI Press, Menlo Park; Hulo et al., Nucl. Acids. Res. 32:D134-D137, (2004)), or Pfam (Bateman et al., Nucleic Acids Research 30(1): 276-280 (2002)). A set of tools for *in silico* analysis of protein sequences is available on the ExPASy proteomics server (Swiss Institute of Bioinformatics (Gasteiger et al., ExPASy: the proteomics server for in-depth protein knowledge and analysis, Nucleic Acids Res. 31:3784-3788(2003)). Domains or motifs may also be identified using routine techniques, such as by sequence alignment.

Methods for the alignment of sequences for comparison are well known in the art, such methods include GAP, BESTFIT, BLAST, FASTA and TFASTA. GAP uses the algorithm of Needleman and Wunsch ((1970) J Mol Biol 48: 443-453) to find the global (i.e. spanning the complete sequences) alignment of two sequences that maximizes the number of matches and minimizes the number of gaps. The BLAST algorithm (Altschul et al. (1990) J Mol Biol 215: 403-10) calculates percent sequence identity and performs a statistical analysis of the similarity between the two sequences. The software for performing BLAST analysis is publicly available through the National Centre for Biotechnology Information (NCBI). Homologues may readily be identified using, for example, the ClustalW multiple sequence alignment algorithm (version 1.83), with the default pairwise alignment parameters, and a scoring method in percentage. Global percentages of similarity and identity may also be determined using one of the methods available in the MatGAT software package (Campanella et al., BMC Bioinformatics. 2003 Jul 10;4:29. MatGAT: an application that generates similarity/identity matrices using protein or DNA sequences.). Minor manual editing may be performed to optimise alignment between conserved motifs, as would be apparent to a person skilled in the art. Furthermore, instead of using full-length sequences for the identification of homologues, specific domains may also be used. The sequence identity values may be determined over the entire nucleic acid or amino acid sequence or over selected domains or conserved motif(s), using the programs mentioned above using the default parameters.

Furthermore, UGE polypeptides (at least in their native form) typically have UDP-Glucose 4-epimerase or UDP-Galactose 4-epimerase activity. In addition UGE polypeptides useful in the methods of the invention may also be enzymatically inactive. Tools and techniques for measuring UDP_Glucose 4-epimerase and UDP-Galactose 4-epimerase activity are well known in the art. Further details on the reaction properties or activity as well as examples of UGE polypeptides are well known in the art and can be found in specialized databases such as BRENDA (The Comprehensive Enzyme Information System) developed and maintained at the University of Cologne (Germany). In vivo assays include the complementation of defective UDP_Glucose 4- epimerase activity in microorganisms carrying mutations in genes encoding UGE polypeptides, e.g. the Scharomyces cerevisiae strain carrying a deficiency in the gene encoding the GAL10 0 protein (Dormann and Benning. 1996. Arch Biochem Biophys. 327(1):27-34). Methods to purify UGE polypeptides and to measure the epimearase activity in vitro have also been described (Barber 2006).

The present invention is illustrated by transforming plants with the nucleic acid sequence represented by SEQ ID NO: 224, encoding the polypeptide sequence of SEQ ID NO: 225. However, performance of the invention is not restricted to these sequences; the methods of the invention may advantageously be performed using any UGE-encoding nucleic acid or UGE polypeptide as defined herein.

Examples of nucleic acids encoding UGE polypeptides are given in Table A6 of Example 1 herein. Such nucleic acids are useful in performing the methods of the invention. The amino acid sequences given in Table A6 of Example 1 are example sequences of orthologues and paralogues of the UGE polypeptide represented by SEQ ID NO: 225, the terms "orthologues" and "paralogues" being as defined herein. Further orthologues and paralogues may readily be identified by performing a so-called reciprocal blast search. Typically, this involves a first BLAST involving BLASTing a query sequence (for example using any of the sequences listed in Table A6 of Example 1) against any sequence database, such as the publicly available NCBI database. BLASTN or TBLASTX (using standard default values) are generally used when starting from a nucleotide sequence, and BLASTP or TBLASTN (using standard default values) when starting from a protein sequence. The BLAST results may optionally be filtered. The full-length sequences of either the filtered results or non-filtered results are then BLASTed back (second BLAST) against sequences from the organism from which the query sequence is derived (where the query sequence is SEQ ID NO: 224 or SEQ ID NO: 225, the second BLAST would therefore be against rice sequences). The results of the first and second BLASTs are then compared. A paralogue is identified if a high-ranking hit from the first blast is from the same species as from which the query sequence is derived, a BLAST back then ideally results in the query sequence amongst the highest hits; an orthologue is identified if a high-ranking hit in the first BLAST is not from the same species as from which the query sequence is derived, and preferably results upon BLAST back in the query sequence being among the highest hits.

High-ranking hits are those having a low E-value. The lower the E-value, the more significant the score (or in other words the lower the chance that the hit was found by chance). Computation of the E-value is well known in the art. In addition to E-values, comparisons are also scored by percentage identity. Percentage identity refers to the number of identical nucleotides (or amino acids) between the two compared nucleic acid (or polypeptide) sequences over a particular length. In the case of large families, ClustalW may be used, followed by a neighbour joining tree, to help visualize clustering of related genes and to identify orthologues and paralogues.

Nucleic acid variants may also be useful in practising the methods of the invention. Examples of such variants include nucleic acids encoding homologues and derivatives of any one of the amino acid sequences given in Table A6 of Example 1, the terms "homologue" and "derivative" being as defined herein. Also useful in the methods of the invention are nucleic acids encoding homologues and derivatives of orthologues or paralogues of any one of the amino acid sequences given in Table A6 of Example 1. Homologues and derivatives useful in the methods of the present invention have substantially the same biological and functional activity as the unmodified protein from which they are derived.

Further nucleic acid variants useful in practising the methods of the invention include portions of nucleic acids encoding UGE polypeptides, nucleic acids hybridising to nucleic acids encoding UGE polypeptides, splice variants of nucleic acids encoding UGE polypeptides, allelic variants of nucleic acids encoding UGE polypeptides and variants of nucleic acids encoding UGE polypeptides obtained by gene shuffling. The terms hybridising sequence, splice variant, allelic variant and gene shuffling are as described herein.

Nucleic acids encoding UGE polypeptides need not be full-length nucleic acids, since performance of the methods of the invention does not rely on the use of full-length nucleic acid sequences. According to the present invention, there is provided a method for enhancing yield-related traits in plants, comprising introducing and expressing in a plant a portion of any one of the nucleic acid sequences given in Table A6 of Example 1, or a portion of a nucleic acid encoding an orthologue, paralogue or homologue of any of the amino acid sequences given in Table A6 of Example 1.

A portion of a nucleic acid may be prepared, for example, by making one or more deletions to the nucleic acid. The portions may be used in isolated form or they may be fused to other coding (or non-coding) sequences in order to, for example, produce a protein that combines several activities. When fused to other coding sequences, the resultant polypeptide produced upon translation may be bigger than that predicted for the protein portion.

Portions useful in the methods of the invention, encode a UGE polypeptide as defined herein, and have substantially the same biological activity as the amino acid sequences given in Table A6 of Example 1. Preferably, the portion is a portion of any one of the nucleic acids given in Table A of Example 1, or is a portion of a nucleic acid encoding an orthologue or paralogue of any one of the amino acid sequences given in Table A6 of Example 1. Preferably the portion is at least 250, 500, 550, 600, 650, 700, 750, 800, 850, 900, 950, 1000 consecutive nucleotides in length, the consecutive nucleotides being of any one of the nucleic acid sequences given in Table A6 of Example 1, or of a nucleic acid encoding an orthologue or paralogue of any one of the amino acid sequences given in Table A6 of Example 1. Most preferably the portion is a portion of the nucleic acid of SEQ ID NO: 224. Preferably, the portion encodes an amino acid sequence which when used in the construction of a phylogenetic tree, such as the one depicted in Figure 21, clusters with the group comprising the amino acid sequence represented by SEQ ID NO: 225 (Os_UGE2) or alternatively, when in a tree such as that of Figure 1 in Rosti et al. 2007 (The Plant Cell 19:1565-1579) clusters with group comprising AtUGE2.

Another nucleic acid variant useful in the methods of the invention is a nucleic acid capable of hybridising, under reduced stringency conditions, preferably under stringent conditions, with a nucleic acid encoding a UGE polypeptide as defined herein, or with a portion as defined herein.

According to the present invention, there is provided a method for enhancing yield-related traits in plants, comprising introducing and expressing in a plant a nucleic acid capable of hybridizing to any one of the nucleic acids given in Table A6 of Example 1, or comprising introducing and expressing in a plant a nucleic acid capable of hybridising to a nucleic acid encoding an orthologue, paralogue or homologue of any of the nucleic acid sequences given in Table A6 of Example 1.

Hybridising sequences useful in the methods of the invention encode a UGE polypeptide as defined herein, having substantially the same biological activity as the amino acid sequences given in Table A6 of Example 1. Preferably, the hybridising sequence is capable of hybridising to any one of the nucleic acids given in Table A6 of Example 1, or to a portion of any of these sequences, a portion being as defined above, or the hybridising sequence is capable of hybridising to a nucleic acid encoding an orthologue or paralogue of any one of the amino acid sequences given in Table A6 of Example 1. Most preferably, the hybridising sequence is capable of hybridising to a nucleic acid as represented by SEQ ID NO: 224 or to a portion thereof.

Preferably, the hybridising sequence encodes a polypeptide with an amino acid sequence which when used in the construction of a phylogenetic tree, such as the one depicted in Figure 21, clusters with the group comprising the amino acid sequence represented by SEQ ID NO: 225 (Os_UGE2) or alternatively when used in the construction of a phylogenetic tree such as that of Figure 1 in Rosti et al. 2007 (The Plant Cell 19:1565-1579) clusters with group comprising AtUGE2.

Another nucleic acid variant useful in the methods of the invention is a splice variant encoding a UGE polypeptide as defined hereinabove, a splice variant being as defined herein.

According to the present invention, there is provided a method for enhancing yield-related traits in plants, comprising introducing and expressing in a plant a splice variant of any one of the nucleic acid sequences given in Table A6 of Example 1, or a splice variant of a nucleic acid encoding an orthologue, paralogue or homologue of any of the amino acid sequences given in Table A6 of Example 1.

Preferred splice variants are splice variants of a nucleic acid represented by SEQ ID NO: 224, or a splice variant of a nucleic acid encoding an orthologue or paralogue of SEQ ID NO: 225. Preferably, the amino acid sequence encoded by the splice variant, when used in the construction of a phylogenetic tree, such as the one depicted in Figure 21, clusters with the group comprising the amino acid sequence represented by SEQ ID NO: 225 (Os_UGE2) or alternatively when used in the construction of a phylogenetic tree such as that of Figure 1 in Rosti et al. 2007 (The Plant Cell 19:1565-1579) clusters with group comprising AtUGE2.

Another nucleic acid variant useful in performing the methods of the invention is an allelic variant of a nucleic acid encoding a UGE polypeptide as defined hereinabove, an allelic variant being as defined herein.

According to the present invention, there is provided a method for enhancing yield-related traits in plants, comprising introducing and expressing in a plant an allelic variant of any one of the nucleic acids given in Table A6 of Example 1, or comprising introducing and expressing in a plant an allelic variant of a nucleic acid encoding an orthologue, paralogue or homologue of any of the amino acid sequences given in Table A6 of Example 1.

The allelic variants useful in the methods of the present invention have substantially the same biological activity as the UGE polypeptide of SEQ ID NO: 225 and any of the amino acids depicted in Table A6 of Example 1. Allelic variants exist in nature, and encompassed within the methods of the present invention is the use of these natural alleles. Preferably, the allelic variant is an allelic variant of SEQ ID NO: 224 or an allelic variant of a nucleic acid encoding an orthologue or paralogue of SEQ ID NO: 225. Preferably, the amino acid sequence encoded by the allelic variant, when used in the construction of a phylogenetic tree, such as the one depicted in Figure 21, clusters with the group comprising the amino acid sequence represented by SEQ ID NO: 225 (**Os_UGE2**) or alternatively when used in the construction of a phylogenetic tree such as that of Figure 1 in Rosti et al. 2007 (The Plant Cell 19:1565-1579) clusters with group comprising AtUGE2.

Gene shuffling or directed evolution may also be used to generate variants of nucleic acids encoding UGE polypeptides as defined above; the term "gene shuffling" being as defined herein.

According to the present invention, there is provided a method for enhancing yield-related traits in plants, comprising introducing and expressing in a plant a variant of any one of the nucleic acid sequences given in Table A6 of Example 1, or comprising introducing and expressing in a plant a variant of a nucleic acid encoding an orthologue, paralogue or homologue of any of the amino acid sequences given in Table A6 of Example 1, which variant nucleic acid is obtained by gene shuffling.

Preferably, the amino acid sequence encoded by the variant nucleic acid obtained by gene shuffling, when used in the construction of a phylogenetic tree such as the one depicted in Figure 21, clusters with the group comprising the amino acid sequence represented by SEQ ID NO: 225 (**Os_UGE2**) or alternatively when used in the construction of a phylogenetic tree such as that of Figure 1 in Rosti et al. 2007 (The Plant Cell 19:1565-1579) clusters with group comprising AtUGE2.

Furthermore, nucleic acid variants may also be obtained by site-directed mutagenesis. Several methods are available to achieve site-directed mutagenesis, the most common being PCR based methods (Current Protocols in Molecular Biology. Wiley Eds.).

Nucleic acids encoding UGE polypeptides may be derived from any natural or artificial source. The nucleic acid may be modified from its native form in composition and/or genomic environment through deliberate human manipulation. Preferably the UGE polypeptide-encoding nucleic acid is from a plant, further preferably from a monocotyledonous plant, more preferably from the family Poaceae, most preferably the nucleic acid is from Oryza sativa.

Performance of the methods of the invention gives plants having enhanced yield-related traits. In particular performance of the methods of the invention gives plants having increased yield, especially increased seed yield relative to control plants. The terms "yield" and "seed yield" are described in more detail in the "definitions" section herein.

Reference herein to enhanced yield-related traits is taken to mean an increase in biomass (weight) of one or more parts of a plant, which may include aboveground (harvestable) parts and/or (harvestable) parts below ground. In particular, such harvestable parts are seeds, and performance of the methods of the invention results in plants having increased seed yield relative to the seed yield of control plants.

Taking corn as an example, a yield increase may be manifested as one or more of the following: increase in the number of plants established per hectare or acre, an increase in the number of ears per plant, an increase in the number of rows, number of kernels per row, kernel weight, thousand kernel weight, ear length/diameter, increase in the seed filling rate (which is the number of filled seeds divided by the total number of seeds and multiplied by 100), among others. Taking rice as an example, a yield increase may manifest itself as an increase in one or more of the following: number of plants per hectare or acre, number of panicles per plant, number of spikelets per panicle, number of flowers (florets) per panicle (which is expressed as a ratio of the number of filled seeds over the number of primary panicles), increase in the seed filling rate (which is the number of filled seeds divided by the total number of seeds and multiplied by 100), increase in thousand kernel weight, among others.

The present invention provides a method for increasing yield, especially seed yield of plants, relative to control plants, which method comprises modulating expression, preferably increasing expression, in a plant of a nucleic acid encoding a UGE polypeptide as defined herein.

Since the transgenic plants according to the present invention have increased yield, it is likely that these plants exhibit an increased growth rate (during at least part of their life cycle), relative to the growth rate of control plants at a corresponding stage in their life cycle.

The increased growth rate may be specific to one or more parts of a plant (including seeds), or may be throughout substantially the whole plant. Plants having an increased growth rate may have a shorter life cycle. The life cycle of a plant may be taken to mean the time needed to grow from a dry mature seed up to the stage where the plant has produced dry mature seeds, similar to the starting material. This life cycle may be influenced by factors such as early vigour, growth rate, greenness index, flowering time and speed of seed maturation. The increase in growth rate may take place at one or more stages in the life cycle of a plant or during substantially the whole plant life cycle. Increased growth rate during the early stages in the life cycle of a plant may reflect enhanced vigour. The increase in growth rate may alter the harvest cycle of a plant allowing plants to be sown later and/or harvested sooner than would otherwise be possible (a similar effect may be obtained with earlier flowering time). If the growth rate is sufficiently increased, it may allow for the further sowing of seeds of the same plant species (for example sowing and harvesting of rice plants followed by sowing and harvesting of further rice plants all within one conventional growing period). Similarly, if the growth rate is sufficiently increased, it may allow for the further sowing of seeds of different plants species (for example the sowing and harvesting of corn plants followed by, for example, the sowing and optional harvesting of soybean, potato or any other suitable plant). Harvesting additional times from the same rootstock in the case of some crop plants may also be possible. Altering the harvest cycle of a plant may lead to an increase in annual biomass production per acre (due to an increase in the number of times (say in a year) that any particular plant may be grown and harvested). An increase in growth rate may also allow for the cultivation of transgenic plants in a wider geographical area than their wild-type counterparts, since the territorial limitations for growing a crop are often determined by adverse environmental conditions either at the time of planting (early season) or at the time of harvesting (late season). Such adverse conditions may be avoided if the harvest cycle is shortened. The growth rate may be determined by deriving various parameters from growth curves, such parameters may be: T-Mid (the time taken for plants to reach 50% of their maximal size) and T-90 (time taken for plants to reach 90% of their maximal size), amongst others.

According to a preferred feature of the present invention, performance of the methods of the invention gives plants having an increased growth rate relative to control plants. Therefore, according to the present invention, there is provided a method for increasing the growth rate of plants, which method comprises modulating expression, preferably increasing expression, in a plant of a nucleic acid encoding a UGE polypeptide as defined herein.

An increase in yield and/or growth rate occurs whether the plant is under non-stress conditions or whether the plant is exposed to various stresses compared to control plants. Plants typically respond to exposure to stress by growing more slowly. In conditions of severe stress, the plant may even stop growing altogether. Mild stress on the other hand is defined herein as being any stress to which a plant is exposed which does not result in the plant ceasing to grow altogether without the capacity to resume growth. Mild stress in the sense of the invention leads to a reduction in the growth of the stressed plants of less than 40%, 35% or 30%, preferably less than 25%, 20% or 15%, more preferably less than 14%, 13%, 12%, 11 % or 10% or less in comparison to the control plant under non-stress conditions. Due to advances in agricultural practices (irrigation, fertilization, pesticide treatments) severe stresses are not often encountered in cultivated crop plants. As a consequence, the compromised growth induced by mild stress is often an undesirable feature for agriculture. Mild stresses are the everyday biotic and/or abiotic (environmental) stresses to which a plant is exposed. Abiotic stresses may be due to drought or excess water, anaerobic stress, salt stress, chemical toxicity, oxidative stress and hot, cold or freezing temperatures. The abiotic stress may be an osmotic stress caused by a water stress (particularly due to drought), salt stress, oxidative stress or an ionic stress. Biotic stresses are typically those stresses caused by pathogens, such as bacteria, viruses, fungi and insects.

In particular, the methods of the present invention may be performed under non-stress conditions or under conditions of mild drought to give plants having increased yield relative to control plants. As reported in Wang et al. (Planta (2003) 218: 1-14), abiotic stress leads to a series of morphological, physiological, biochemical and molecular changes that adversely affect plant growth and productivity. Drought, salinity, extreme temperatures and oxidative stress are known to be interconnected and may induce growth and cellular damage through similar mechanisms. Rabbani et al. (Plant Physiol (2003) 133: 1755-1767) describes a particularly high degree of "cross talk" between drought stress and high-salinity stress. For example, drought and/or salinisation are manifested primarily as osmotic stress, resulting in the disruption of homeostasis and ion distribution in the cell. Oxidative stress, which frequently accompanies high or low temperature, salinity or drought stress, may cause denaturing of functional and structural proteins. As a consequence, these diverse environmental stresses often activate similar cell signalling pathways and cellular responses, such as the production of stress proteins, up-regulation of anti-oxidants, accumulation of compatible solutes and growth arrest. The term "non-stress" conditions as used herein are those environmental conditions that allow optimal growth of plants. Persons skilled in the art are aware of normal soil conditions and climatic conditions for a given location.

Performance of the methods of the invention gives plants grown under non-stress conditions or under mild drought conditions increased yield relative to control plants grown under comparable conditions. Therefore, according to the present invention, there is provided a method for increasing yield in plants grown under non-stress conditions or under mild drought conditions, which method comprises increasing expression in a plant of a nucleic acid encoding a UGE polypeptide.

Performance of the methods of the invention gives plants grown under conditions of nutrient deficiency, particularly under conditions of nitrogen deficiency, increased yield relative to control plants grown under comparable conditions. Therefore, according to the present invention, there is provided a method for increasing yield in plants grown under conditions of nutrient deficiency, which method comprises increasing expression in a plant of a nucleic acid encoding a UGE polypeptide. Nutrient deficiency may result from a lack of nutrients such as nitrogen, phosphates and other phosphorous-containing compounds, potassium, calcium, cadmium, magnesium, manganese, iron and boron, amongst others.

The present invention encompasses plants or parts thereof (including seeds) obtainable by the methods according to the present invention. The plants or parts thereof comprise a nucleic acid transgene encoding a UGE polypeptide as defined above.

The invention also provides genetic constructs and vectors to facilitate introduction and/or expression in plants of nucleic acids encoding UGE polypeptides. The gene constructs may be inserted into vectors, which may be commercially available, suitable for transforming into plants and suitable for expression of the gene of interest in the transformed cells. The invention also provides use of a gene construct as defined herein in the methods of the invention.

More specifically, the present invention provides a construct comprising:

(a) a nucleic acid encoding a UGE polypeptide as defined above;
(b) one or more control sequences capable of driving expression of the nucleic acid sequence of (a); and optionally
(c) a transcription termination sequence.

Preferably, the nucleic acid encoding a UGE polypeptide is as defined above. The term "control sequence" and "termination sequence" are as defined herein.

Plants are transformed with a vector comprising any of the nucleic acids described above. The skilled artisan is well aware of the genetic elements that must be present on the vector in order to successfully transform, select and propagate host cells containing the sequence of interest. The sequence of interest is operably linked to one or more control sequences (at least to a promoter).

Advantageously, any type of promoter, whether natural or synthetic, may be used to drive expression of the nucleic acid sequence. A constitutive promoter is particularly useful in the methods. See the "Definitions" section herein for definitions of the various promoter types.

It should be clear that the applicability of the present invention is not restricted to the UGE polypeptide-encoding nucleic acid represented by SEQ ID NO: 1, nor is the applicability of the invention restricted to expression of a UGE polypeptide-encoding nucleic acid when driven by a constitutive promoter, or when driven by a root-specific promoter.

The constitutive promoter is preferably a GOS2 promoter, preferably a GOS2 promoter from rice. Further preferably the constitutive promoter is represented by a nucleic acid sequence substantially similar to SEQ ID NO: 270, most preferably the constitutive promoter is as represented by SEQ ID NO: 270. See Table 2 in the "Definitions" section herein for further examples of constitutive promoters.

Optionally, one or more terminator sequences may be used in the construct introduced into a plant. Additional regulatory elements may include transcriptional as well as translational enhancers. Those skilled in the art will be aware of terminator and enhancer sequences that may be suitable for use in performing the invention. An intron sequence may also be added to the 5' untranslated region (UTR) or in the coding sequence to increase the amount of the mature message that accumulates in the cytosol, as described in the definitions section. Other control sequences (besides promoter, enhancer, silencer, intron sequences, 3'UTR and/or 5'UTR regions) may be protein and/or RNA stabilizing elements. Such sequences would be known or may readily be obtained by a person skilled in the art.

The genetic constructs of the invention may further include an origin of replication sequence that is required for maintenance and/or replication in a specific cell type. One example is when a genetic construct is required to be maintained in a bacterial cell as an episomal genetic element (e.g. plasmid or cosmid molecule). Preferred origins of replication include, but are not limited to, the f1-ori and colE1.

For the detection of the successful transfer of the nucleic acid sequences as used in the methods of the invention and/or selection of transgenic plants comprising these nucleic acids, it is advantageous to use marker genes (or reporter genes). Therefore, the genetic construct may optionally comprise a selectable marker gene. Selectable markers are described in more detail in the "definitions" section herein. The marker genes may be removed or excised from the transgenic cell once they are no longer needed. Techniques for marker removal are known in the art, useful techniques are described above in the definitions section.

The invention also provides a method for the production of transgenic plants having enhanced yield-related traits relative to control plants, comprising introduction and expression in a plant of any nucleic acid encoding a UGE polypeptide as defined hereinabove.

More specifically, the present invention provides a method for the production of transgenic plants having increased enhanced yield-related traits, particularly increased (seed) yield, which method comprises:

(i) introducing and expressing in a plant or plant cell a UGE polypeptide-encoding nucleic acid; and
(ii) cultivating the plant cell under conditions promoting plant growth and development.

The nucleic acid of (i) may be any of the nucleic acids capable of encoding a UGE polypeptide as defined herein.

The nucleic acid may be introduced directly into a plant cell or into the plant itself (including introduction into a tissue, organ or any other part of a plant). According to a preferred feature of the present invention, the nucleic acid is preferably introduced into a plant by transformation. The term "transformation" is described in more detail in the "definitions" section herein.

The genetically modified plant cells can be regenerated via all methods with which the skilled worker is familiar. Suitable methods can be found in the abovementioned publications by S.D. Kung and R. Wu, Potrykus or Höfgen and Willmitzer.

Generally after transformation, plant cells or cell groupings are selected for the presence of one or more markers which are encoded by plant-expressible genes co-transferred with the gene of interest, following which the transformed material is regenerated into a whole plant.

To select transformed plants, the plant material obtained in the transformation is, as a rule, subjected to selective conditions so that transformed plants can be distinguished from untransformed plants. For example, the seeds obtained in the above-described manner can be planted and, after an initial growing period, subjected to a suitable selection by spraying. A further possibility consists in growing the seeds, if appropriate after sterilization, on agar plates using a suitable selection agent so that only the transformed seeds can grow into plants. Alternatively, the transformed plants are screened for the presence of a selectable marker such as the ones described above.

Following DNA transfer and regeneration, putatively transformed plants may also be evaluated, for instance using Southern analysis, for the presence of the gene of interest, copy number and/or genomic organisation. Alternatively or additionally, expression levels of the newly introduced DNA may be monitored using Northern and/or Western analysis, both techniques being well known to persons having ordinary skill in the art.

The generated transformed plants may be propagated by a variety of means, such as by clonal propagation or classical breeding techniques. For example, a first generation (or T1) transformed plant may be selfed and homozygous second-generation (or T2) transformants selected, and the T2 plants may then further be propagated through classical breeding techniques. The generated transformed organisms may take a variety of forms. For example, they may be chimeras of transformed cells and non-transformed cells; clonal transformants (e.g., all cells transformed to contain the expression cassette); grafts of transformed and untransformed tissues (e.g., in plants, a transformed rootstock grafted to an untransformed scion).

The present invention clearly extends to any plant cell or plant produced by any of the methods described herein, and to all plant parts and propagules thereof. The present invention extends further to encompass the progeny of a primary transformed or transfected cell, tissue, organ or whole plant that has been produced by any of the aforementioned methods, the only requirement being that progeny exhibit the same genotypic and/or phenotypic characteristic (s) as those produced by the parent in the methods according to the invention.

The invention also includes host cells containing an isolated nucleic acid encoding a UGE polypeptide as defined hereinabove. Preferred host cells according to the invention are plant cells. Host plants for the nucleic acids or the vector used in the method according to the invention, the expression cassette or construct or vector are, in principle, advantageously all plants, which are capable of synthesizing the polypeptides used in the inventive method.

The methods of the invention are advantageously applicable to any plant. Plants that are particularly useful in the methods of the invention include all plants which belong to the superfamily Viridiplantae, in particular monocotyledonous and dicotyledonous plants including fodder or forage legumes, ornamental plants, food crops, trees or shrubs. According to a preferred embodiment of the present invention, the plant is a crop plant. Examples of crop plants include soybean, sunflower, canola, alfalfa, rapeseed, cotton, tomato, potato and tobacco. Further preferably, the plant is a monocotyledonous plant. Examples of monocotyledonous plants include sugarcane. More preferably the plant is a cereal. Examples of cereals include rice, maize, wheat, barley, millet, rye, triticale, sorghum and oats.

The invention also extends to harvestable parts of a plant such as, but not limited to seeds, leaves, fruits, flowers, stems, roots, rhizomes, tubers and bulbs. The invention furthermore relates to products derived, preferably directly derived, from a harvestable part of such a plant, such as dry pellets or powders, oil, fat and fatty acids, starch or proteins.

According to a preferred feature of the invention, the modulated expression is increased expression. Methods for increasing expression of nucleic acids or genes, or gene products, are well documented in the art and examples are provided in the definitions section.

As mentioned above, a preferred method for modulating (preferably, increasing) expression of a nucleic acid encoding a UGE polypeptide is by introducing and expressing in a plant a nucleic acid encoding a UGE polypeptide; however the effects of performing the method, i.e. enhancing yield-related traits may also be achieved using other well known techniques, including but not limited to T-DNA activation tagging, TILLING, homologous recombination. A description of these techniques is provided in the definitions section.

The present invention also encompasses use of nucleic acids encoding UGE polypeptides as described herein and use of these UGE polypeptides in enhancing any of the aforementioned yield-related traits in plants.

Nucleic acids encoding UGE polypeptide described herein, or the UGE polypeptides themselves, may find use in breeding programmes in which a DNA marker is identified which may be genetically linked to a UGE polypeptide-encoding gene. The nucleic acids/genes, or the UGE polypeptides themselves may be used to define a molecular marker. This DNA or protein marker may then be used in breeding programmes to select plants having enhanced yield-related traits as defined hereinabove in the methods of the invention.

Allelic variants of a UGE polypeptide-encoding nucleic acid/gene may also find use in marker-assisted breeding programmes. Such breeding programmes sometimes require introduction of allelic variation by mutagenic treatment of the plants, using for example EMS mutagenesis; alternatively, the programme may start with a collection of allelic variants of so called "natural" origin caused unintentionally. Identification of allelic variants then takes place, for example, by PCR. This is followed by a step for selection of superior allelic variants of the sequence in question and which give increased yield. Selection is typically carried out by monitoring growth performance of plants containing different allelic variants of the sequence in question. Growth performance may be monitored in a greenhouse or in the field. Further optional steps include crossing plants in which the superior allelic variant was identified with another plant. This could be used, for example, to make a combination of interesting phenotypic features.

Nucleic acids encoding UGE polypeptides may also be used as probes for genetically and physically mapping the genes that they are a part of, and as markers for traits linked to those genes. Such information may be useful in plant breeding in order to develop lines with desired phenotypes. Such use of UGE polypeptide-encoding nucleic acids requires only a nucleic acid sequence of at least 15 nucleotides in length. The UGE polypeptide-encoding nucleic acids may be used as restriction fragment length polymorphism (RFLP) markers. Southern blots (Sambrook J, Fritsch EF and Maniatis T (1989) Molecular Cloning, A Laboratory Manual) of restriction-digested plant genomic DNA may be probed with the UGE-encoding nucleic acids. The resulting banding patterns may then be subjected to genetic analyses using computer programs such as MapMaker (Lander et al. (1987) Genomics 1: 174-181) in order to construct a genetic map. In addition, the nucleic acids may be used to probe Southern blots containing restriction endonuclease-treated genomic DNAs of a set of individuals representing parent and progeny of a defined genetic cross. Segregation of the DNA polymorphisms is noted and used to calculate the position of the UGE polypeptide-encoding nucleic acid in the genetic map previously obtained using this population (Botstein et al. (1980) Am. J. Hum. Genet. 32:314-331).

The production and use of plant gene-derived probes for use in genetic mapping is described in Bernatzky and Tanksley (1986) Plant Mol. Biol. Reporter 4: 37-41. Numerous publications describe genetic mapping of specific cDNA clones using the methodology outlined above or variations thereof. For example, F2 intercross populations, backcross populations, randomly mated populations, near isogenic lines, and other sets of individuals may be used for mapping. Such methodologies are well known to those skilled in the art.

The nucleic acid probes may also be used for physical mapping (i.e., placement of sequences on physical maps; see Hoheisel et al. In: Non-mammalian Genomic Analysis: A Practical Guide, Academic press 1996, pp. 319-346, and references cited therein).

In another embodiment, the nucleic acid probes may be used in direct fluorescence in situ hybridisation (FISH) mapping (Trask (1991) Trends Genet. 7:149-154). Although current methods of FISH mapping favour use of large clones (several kb to several hundred kb; see Laan et al. (1995) Genome Res. 5:13-20), improvements in sensitivity may allow performance of FISH mapping using shorter probes.

A variety of nucleic acid amplification-based methods for genetic and physical mapping may be carried out using the nucleic acids. Examples include allele-specific amplification (Kazazian (1989) J. Lab. Clin. Med 11:95-96), polymorphism of PCR-amplified fragments (CAPS; Sheffield et al. (1993) Genomics 16:325-332), allele-specific ligation (Landegren et al. (1988) Science 241:1077-1080), nucleotide extension reactions (Sokolov (1990) Nucleic Acid Res. 18:3671), Radiation Hybrid Mapping (Walter et al. (1997) Nat. Genet. 7:22-28) and Happy Mapping (Dear and Cook (1989) Nucleic Acid Res. 17:6795-6807). For these methods, the sequence of a nucleic acid is used to design and produce primer pairs for use in the amplification reaction or in primer extension reactions. The design of such primers is well known to those skilled in the art. In methods employing PCR-based genetic mapping, it may be necessary to identify DNA sequence differences between the parents of the mapping cross in the region corresponding to the instant nucleic acid sequence. This, however, is generally not necessary for mapping methods.

The methods according to the present invention result in plants having enhanced yield-related traits, as described hereinbefore. These traits may also be combined with other economically advantageous traits, such as further yield-enhancing traits, tolerance to other abiotic and biotic stresses, traits modifying various architectural features and/or biochemical and/or physiological features.

The term "table A6" used in this specification is to be taken to specify the content of table A6a and table A6b. The term "table A6a" used in this specification is to be taken to specify the content of table A6a. The term "table A6b" used in this specification is to be taken to specify the content of table A6b. In one preferred embodiment, the term "table A6" means table A6b.

In one embodiment the invention relates to subject mater summarized as follows:

Item 104: A method for enhancing yield-related traits in plants relative to control plants, comprising modulating expression in a plant of a nucleic acid encoding a UGE polypeptide.

Item 105: Method according to item 104, wherein said UGE polypeptide comprises an epimerase domain having in increasing order of preference at least 70%, 75%, 80%, 85%, 90%, 95%, 97% or more sequence identity to SEQ ID NO: 275.

Item 106: Method according to item 104 or 105, wherein said UGE polypeptide comprises one or more of the following motifs:

Motif 8 as represented by SEQ ID NO: 276;
Motif 9 as represented by SEQ ID NO: 277 wherein the G can be substituted by any other non polar amino acid and the R by any other polar amino acid ;
Motif 10 as represented by SEQ ID NO: 278, wherein 1, 2, 3 or 4 mismatches are allowed;
Motif 11 as represented by SEQ ID NO: 279,
Motif 12 as represented by SEQ ID NO: 280, wherein 1, 2, 3 or 4 mismatches are allowed.

Item 107: Method according to item 104 to 106, wherein said modulated expression is effected by introducing and expressing in a plant a nucleic acid encoding a UGE polypeptide.

Item 108: Method according to any of the items 104 to 107, wherein said nucleic acid encoding a UGE polypeptide encodes any one of the proteins listed in Table A6 or is a portion of such a nucleic acid, or a nucleic acid capable of hybridising with such a nucleic acid.

Item 109: Method according to any of the items 104 to 108, wherein said nucleic acid sequence encodes an orthologue or paralogue of any of the proteins given in Table A6.

Item 110: Method according to any of the items 104 to 109, wherein said enhanced yield-related traits comprise increased yield, preferably increased seed yield relative to control plants.

Item 111: Method according to any of the items 104 to 110, wherein said enhanced yield-related traits are obtained under non-stress conditions.

Item 112: Method according to any of the items 104 to 111, wherein said enhanced yield-related traits are obtained under conditions of drought stress.

Item 113: Method according to any of the items 104 to 112, wherein said nucleic acid is operably linked to a constitutive promoter, preferably to a GOS2 promoter, most preferably to a GOS2 promoter from rice.

Item 114: Method according to any of the items 104 to 113, wherein said nucleic acid encoding a UGE polypeptide is of plant origin, preferably from a monocotyledonous plant, further preferably from the family Poaceae, more preferably from the genus Oryza, most preferably from Oryza sativa.

Item 115: Plant or part thereof, including seeds, obtainable by a method according to any of the items 104 to 114, wherein said plant or part thereof comprises a recombinant nucleic acid encoding a UGE polypeptide.

Item 116: An isolated nucleic acid sequence comprising a nucleic acid sequence selected from the group consisting of:

(a) an isolated nucleic acid molecule encoding the polypeptide as depicted in SEQ ID NO: 282, 284, 286, 288, 290, 292, 294, 296, 298, 300, 302, 304, 306, 308, 310, 312, 314, 316, 318 and/or 320 and/or table A6b;
(b) an isolated nucleic acid molecule as depicted in SEQ ID NO: 281, 283, 285, 287, 289, 291, 293, 295, 297, 299, 301, 303, 305, 307, 309, 311, 313, 315, 317 and/or 319 and/or table A6b;
(c) an isolated nucleic acid molecule, which, as a result of the degeneracy of the genetic code, can be derived from a polypeptide sequence depicted in table A6 and confers enhanced yield-related traits relative to control plants;
(d) an isolated nucleic acid molecule having at least 30 %, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity with the nucleic acid molecule sequence of a polynucleotide comprising the nucleic acid molecule depicted in table A6 and confers enhanced yield-related traits relative to control plants;
(e) an isolated nucleic acid molecule encoding a polypeptide having at least 30 %, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity with the amino acid sequence of the polypeptide encoded by the nucleic acid molecule of (a) to (c) and confers enhanced yield-related traits relative to control plants;
(f) an isolated nucleic acid molecule which hybridizes with a nucleic acid molecule of (a) to (c) under stringent hybridization conditions and confers enhanced yield-related traits relative to control plants;
(g) an isolated nucleic acid molecule encoding a polypeptide comprising the consensus sequence or one or more polypeptide motifs as shown figure 20 and preferably having the activity represented by a nucleic acid molecule comprising a polynucleotide as depicted in table A6;
(h) an isolated nucleic acid molecule encoding a polypeptide having the activity represented by a protein as depicted in table A6 and confering enhanced yield-related traits relative to control plants;
and
(i) a nucleic acid molecule which is obtainable by screening a suitable nucleic acid library under stringent hybridization conditions with a probe comprising a complementary sequence of a nucleic acid molecule of (a) or (b) or with a fragment thereof, having at least 15 nt, preferably 20 nt, 30 nt, 50 nt, 100 nt, 200 nt or 500 nt of a nucleic acid molecule complementary to a nucleic acid molecule sequence characterized in (a) to (e) and encoding a polypeptide having the activity represented by a protein comprising a polypeptide as depicted in table A6;

whereby the nucleic acid molecule according to (a) to (i) is at least in one or more nucleotides different from the sequence depicted in table A6a and preferably which encodes a protein which differs at least in one or more amino acids from the protein sequences depicted in table A6a.

Item 117: Construct comprising:

(a) nucleic acid encoding a UGE polypeptide as defined in items 104 to 106 or 116;
(b) one or more control sequences capable of driving expression of the nucleic acid sequence of (a); and optionally
(c) a transcription termination sequence.

Item 118: Construct according to item 117, wherein one of said control sequences is a constitutive promoter, preferably a GOS2 promoter, most preferably a GOS2 promoter from rice.

Item 119: Use of a construct according to any of the items 117 to 118 in a method for making plants having enhanced yield-related traits preferably increased yield, more preferably increased seed yield relative to control plants.

Item 120: Plant, plant part or plant cell transformed with a construct according to item 117 or 118.

Item 121: Method for the production of a transgenic plant having increased yield, particularly increased seed yield relative to control plants, comprising:

(i) introducing and expressing in a plant a nucleic acid encoding a UGE polypeptide as defined in items 104 to 106 or 116; and
(ii) cultivating the plant cell under conditions promoting plant growth and development.

Item 122: Transgenic plant having increased yield, particularly increased seed yield, relative to control plants, resulting from increased expression of a nucleic acid encoding a UGE polypeptide as defined in items 104 to 106 or 116, or a transgenic plant cell derived from said transgenic plant.

Item 123: Transgenic plant according to item 115, 120 or 122, or a transgenic plant cell derived thereof, wherein said plant is a crop plant or a monocot or a cereal, such as rice, maize, wheat, barley, millet, rye, triticale, sorghum and oats.

Item 124: Harvestable parts of a plant according to item 123, wherein said harvestable parts are preferably shoot biomass and/or seeds.

Item 125: Products derived from a plant according to item 123 and/or from harvestable parts of a plant according to item 124.

Item 126: Use of a nucleic acid encoding a UGE polypeptide enhancing yield-related traits preferably increased yield, more preferably increased seed yield relative to control plants.

According to a further embodiment of the present invention, there is also provided an isolated polypeptide comprising a polypeptide sequence selected from the group consisting of:

(i) an amino acid sequence encoded by any one of the nucleic acids represented by Item 116;
(ii) an amino acid sequence having, in increasing order of preference, at least 50%, 55%, 60%, 65%, 70%, 75%, 80%, 85%, 90%, 95%, 96%, 97%, 98%, 99% or more sequence identity to the amino acid sequence of (i);
(iii) derivatives of any of the amino acid sequences given in (i) or (ii) above.

**Description of figures**

The present invention will now be described with reference to the following figures in which:

**Fig. 1** represents the domain structure of SEQ ID NO: 2 with the conserved motifs indicated by underlining and their number. The HLH domain as determined by SMART is shown in bold underlined.

**Fig. 2** represents a multiple alignment of a number of bHLH6-like proteins. A dot indicates conserved residues, a colon indicates highly conserved residues and an asterisk stands for perfectly conserved residues. The highest degree of sequence conservation is found in the region of the bHLH domain and in the more N-terminal part of the protein sequence

**Fig. 3** represents the binary vector for increased expression in *Oryza sativa* of a bHLH6-like-encoding nucleic acid under the control of a rice GOS2 promoter (pGOS2)

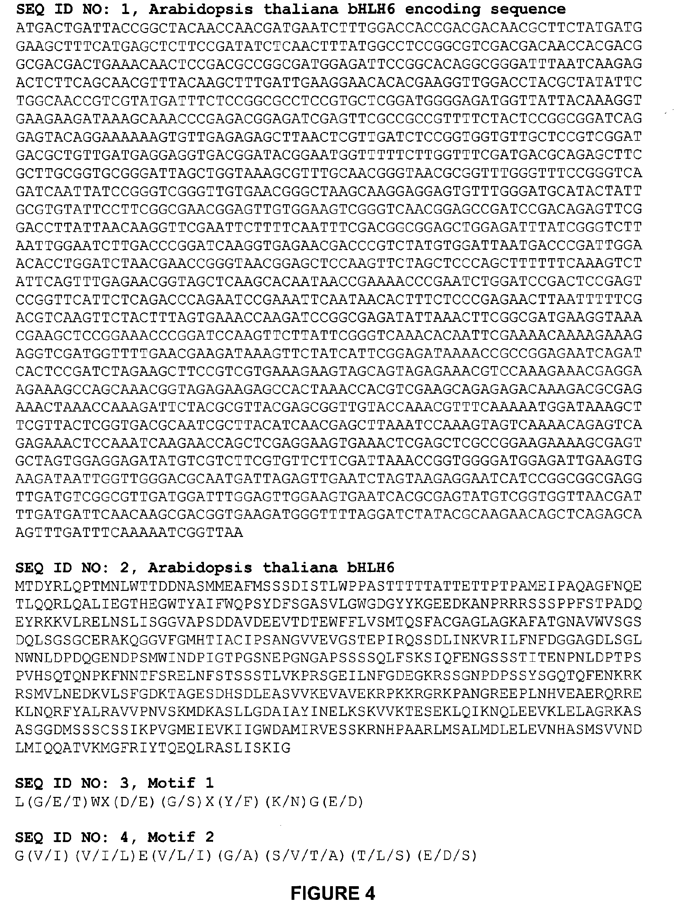

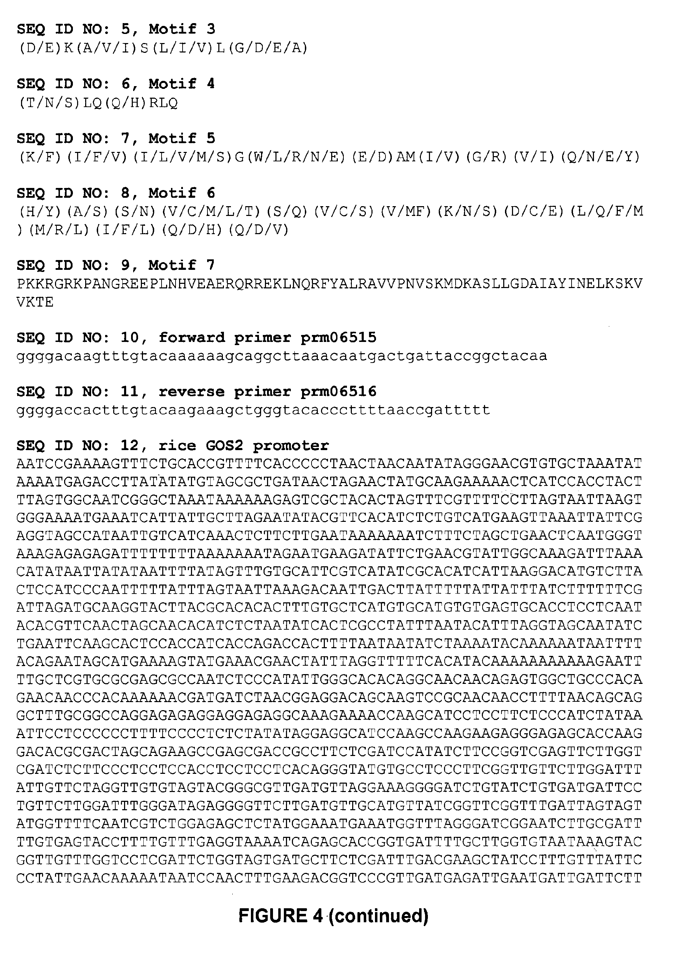

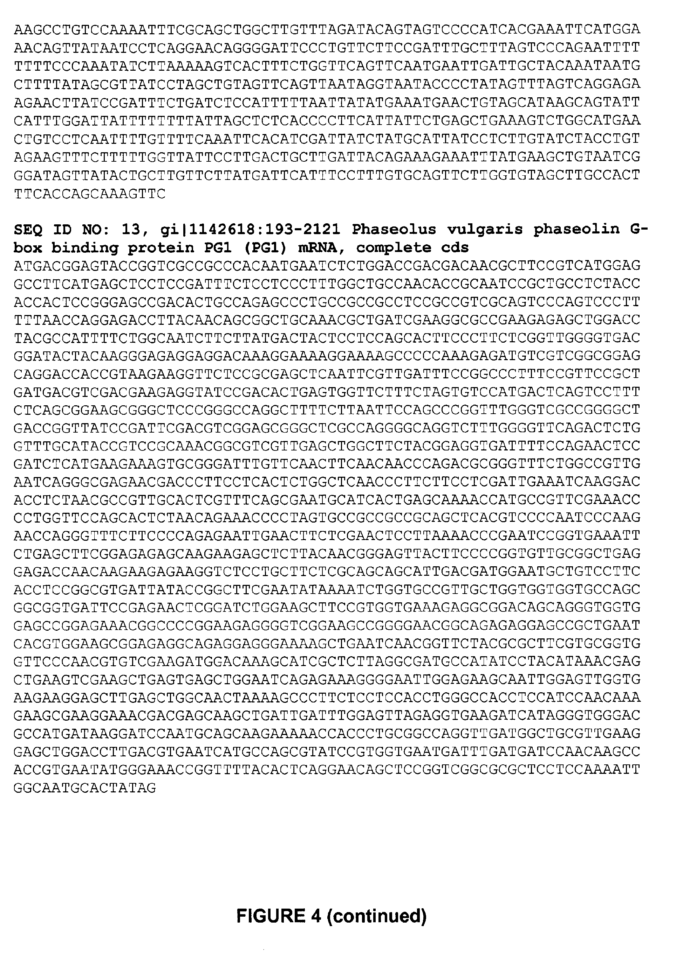

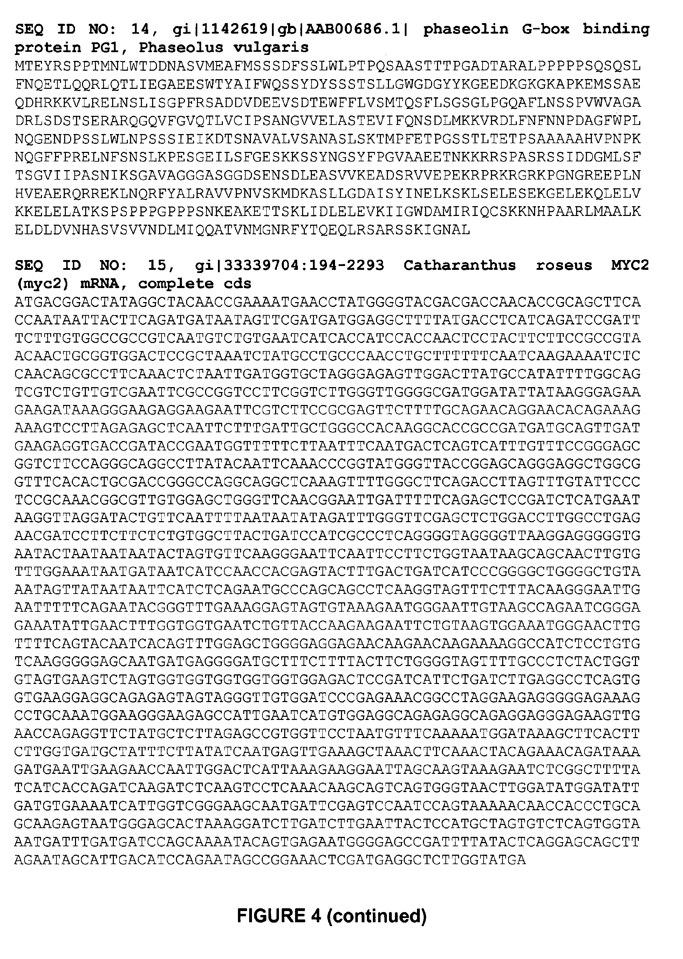

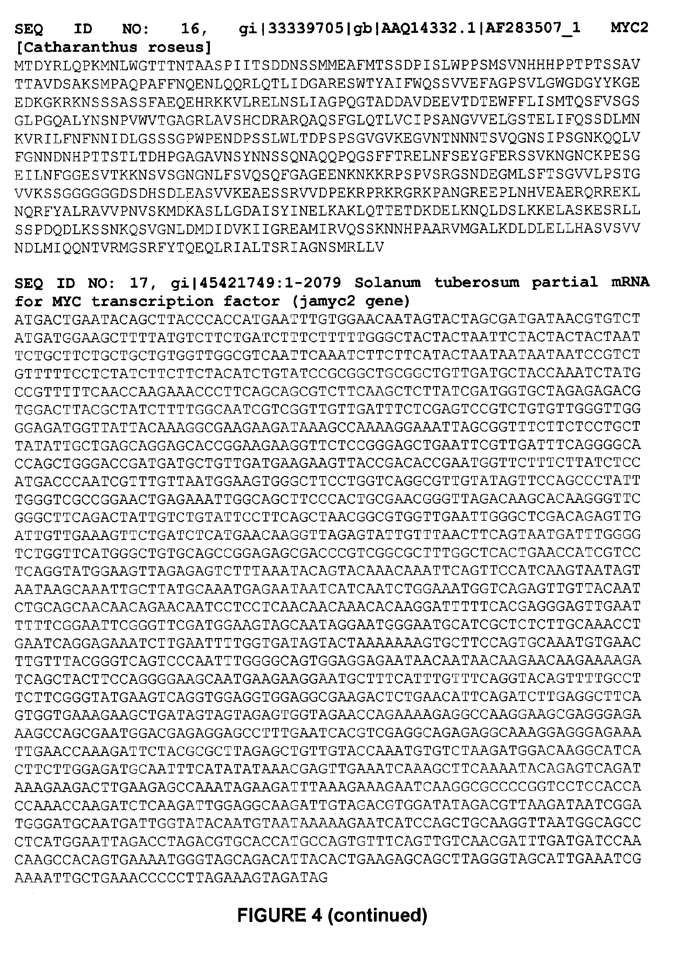

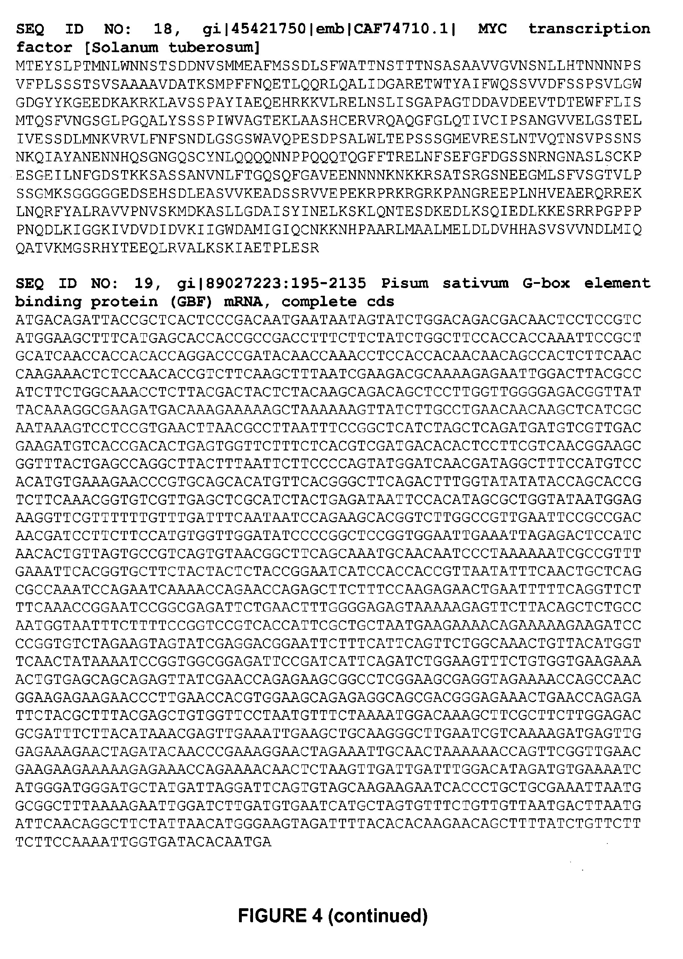

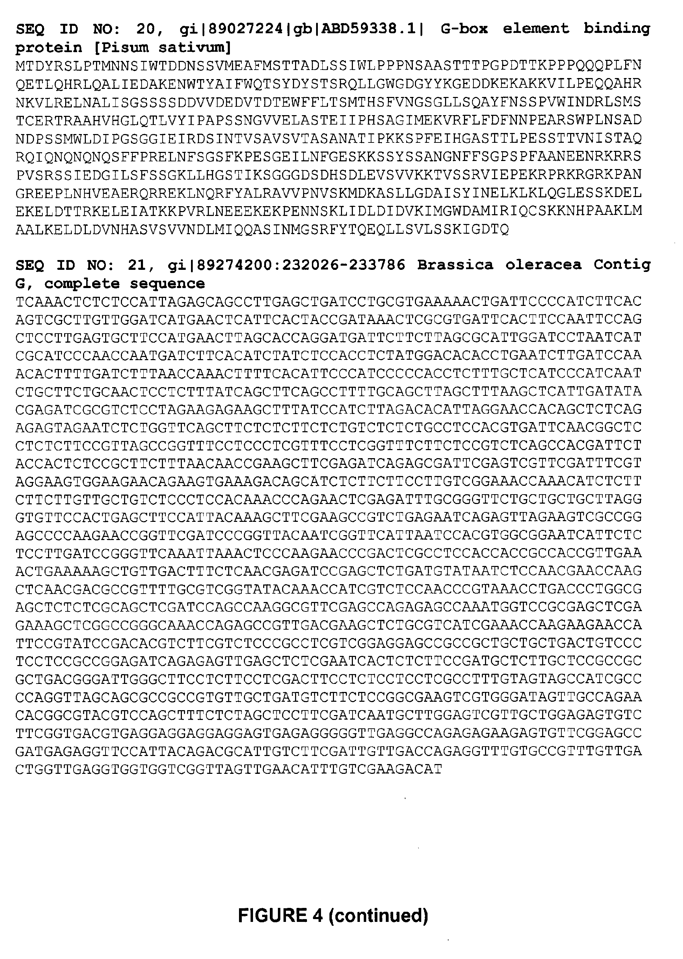

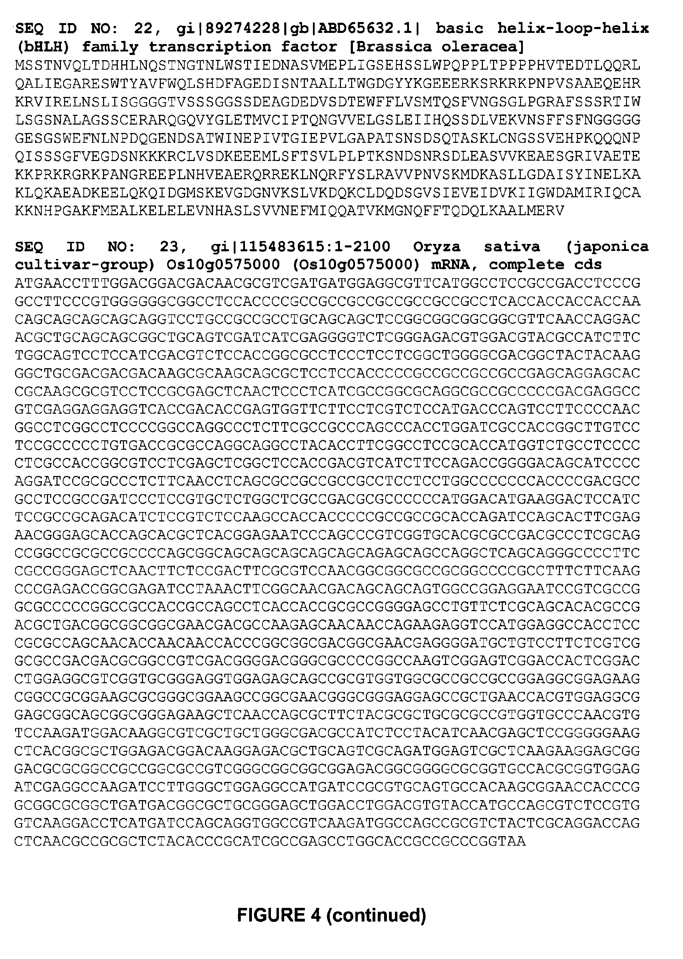

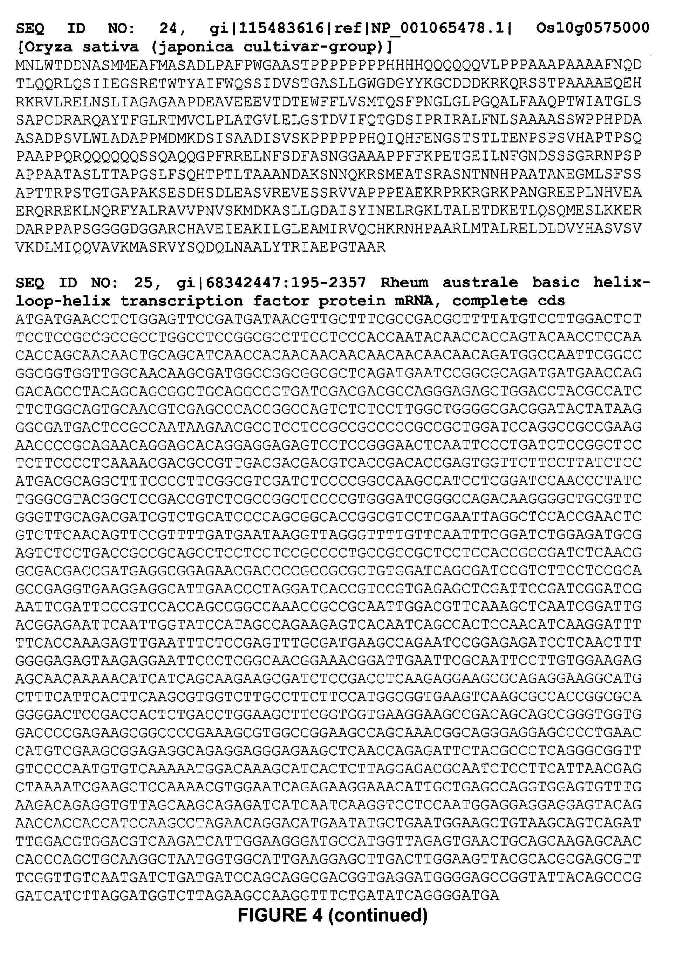

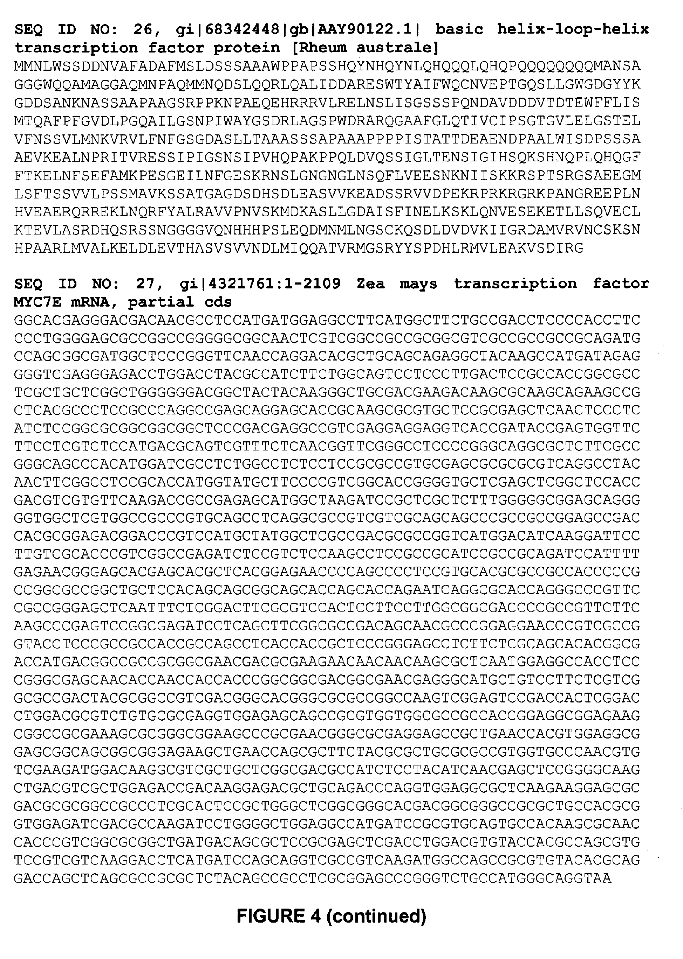

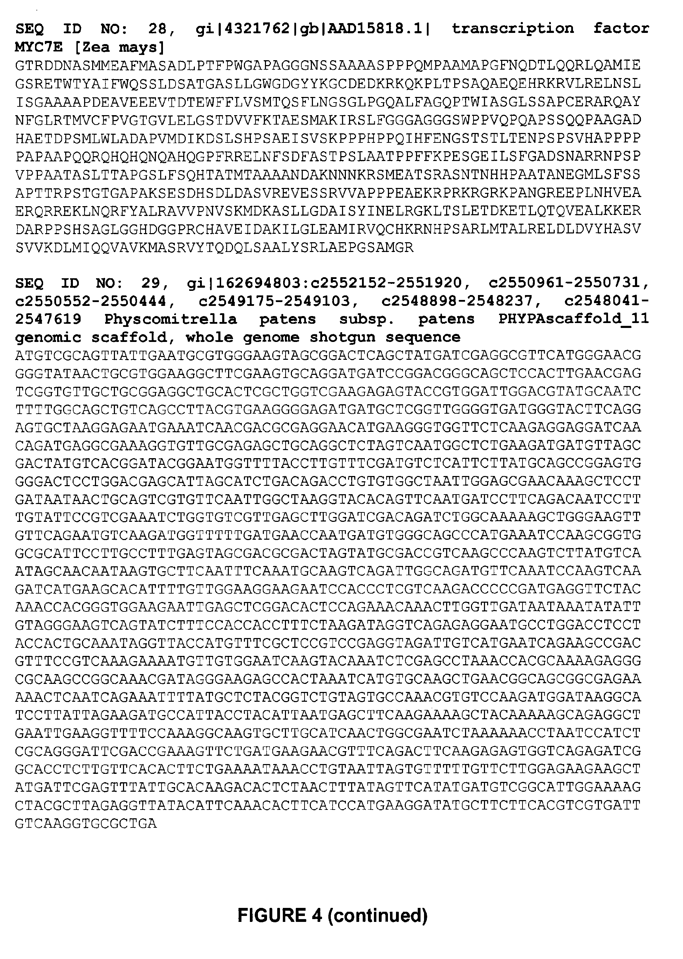

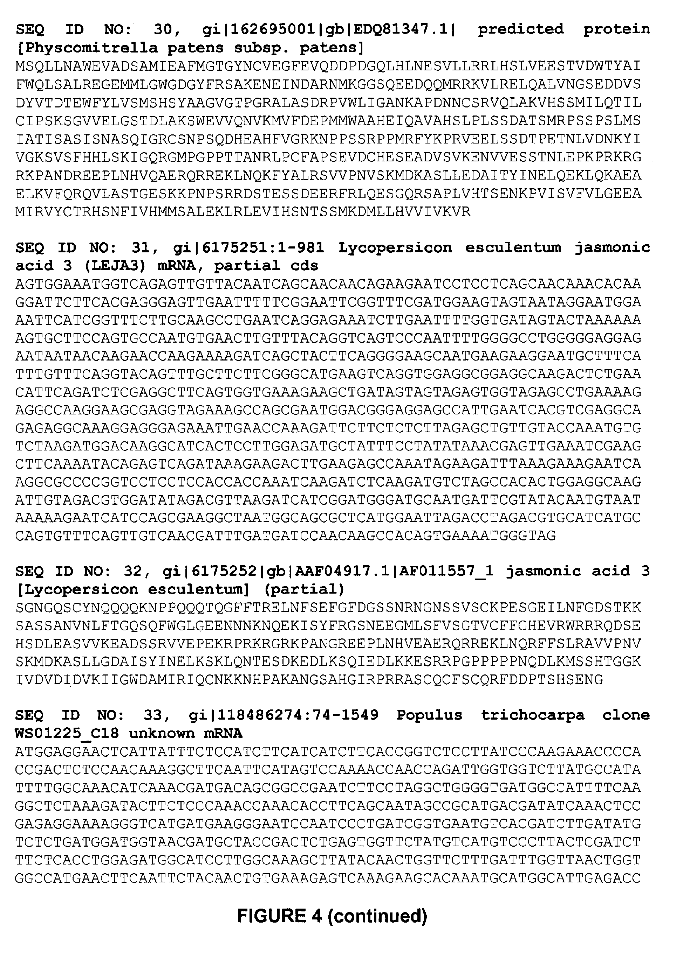

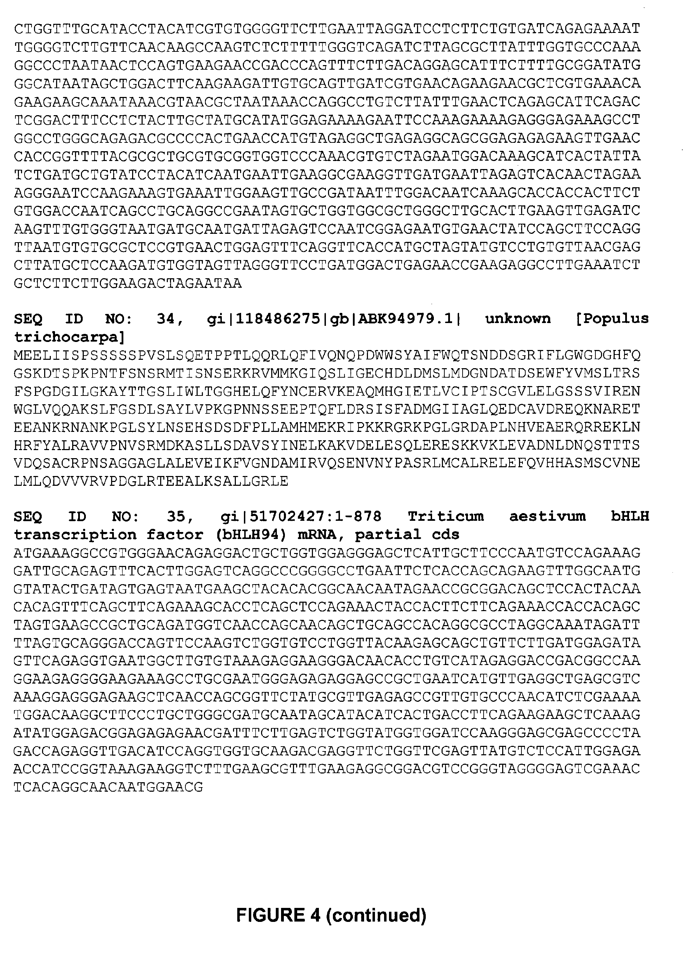

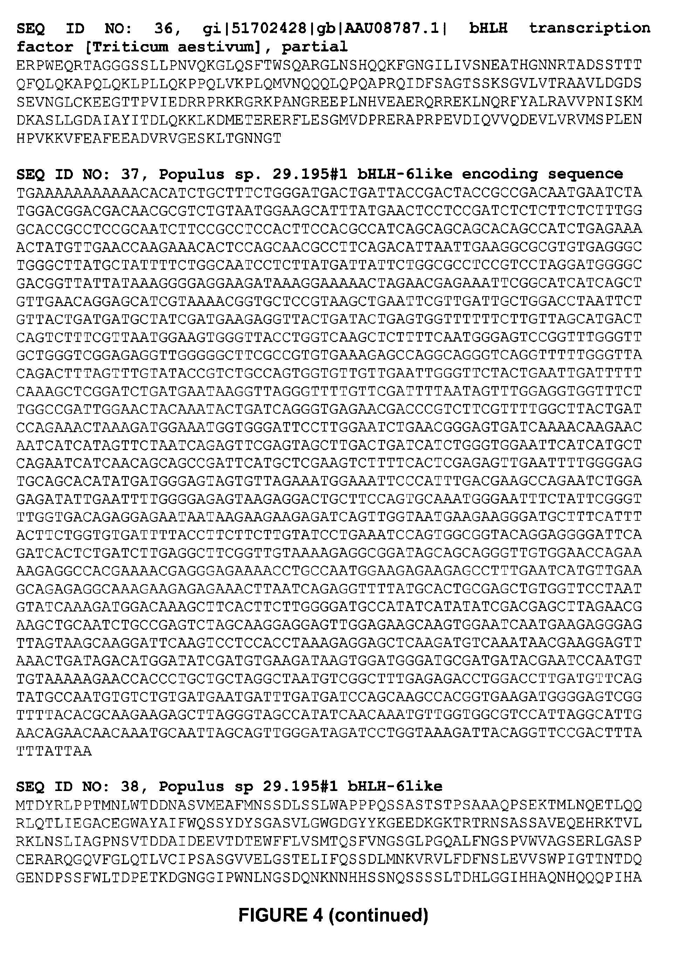

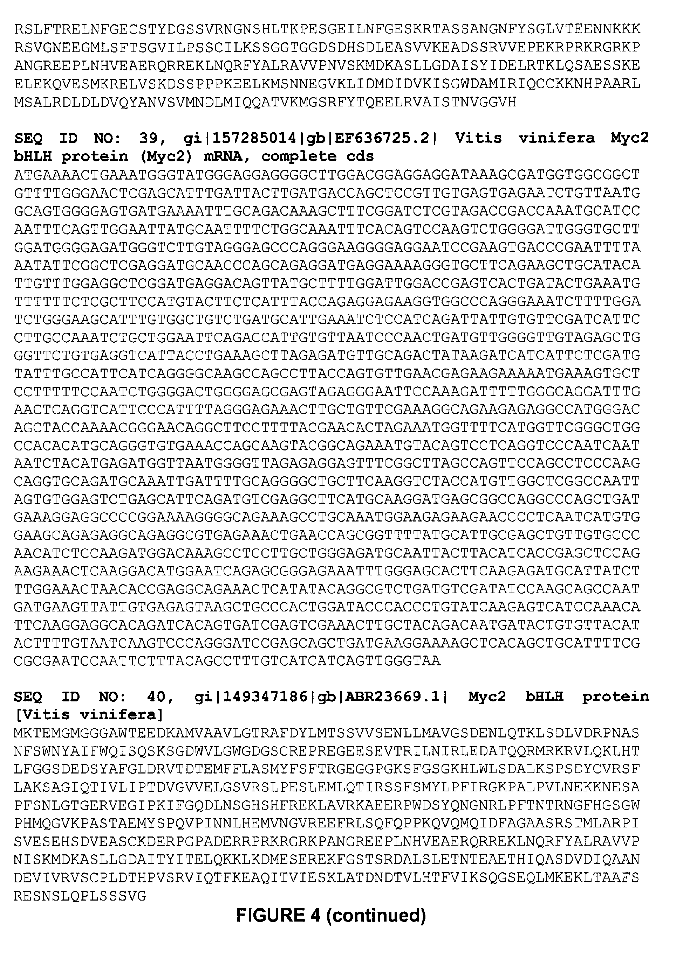

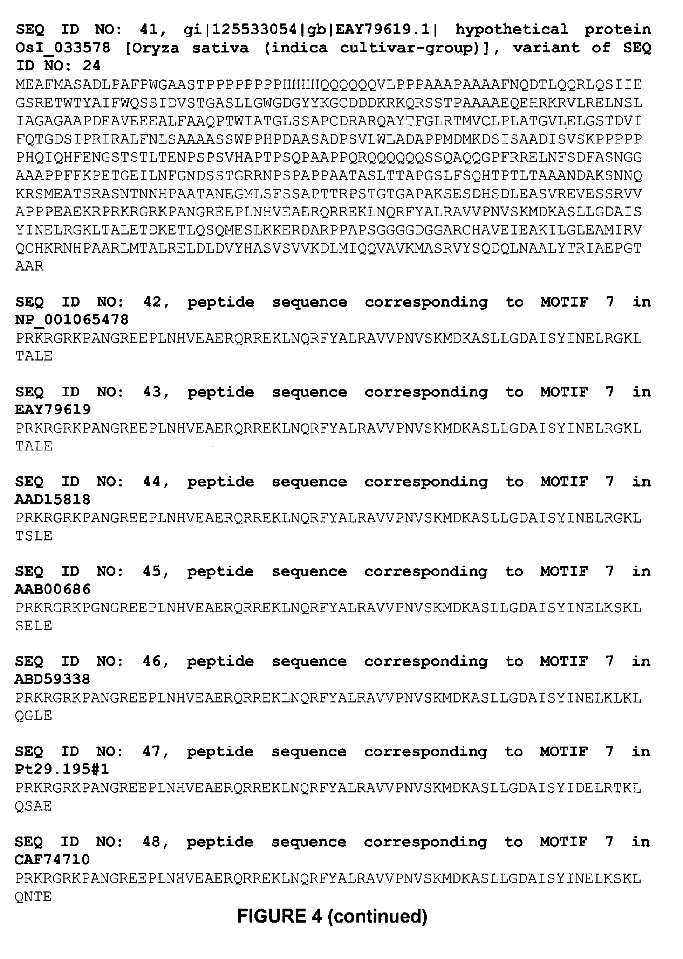

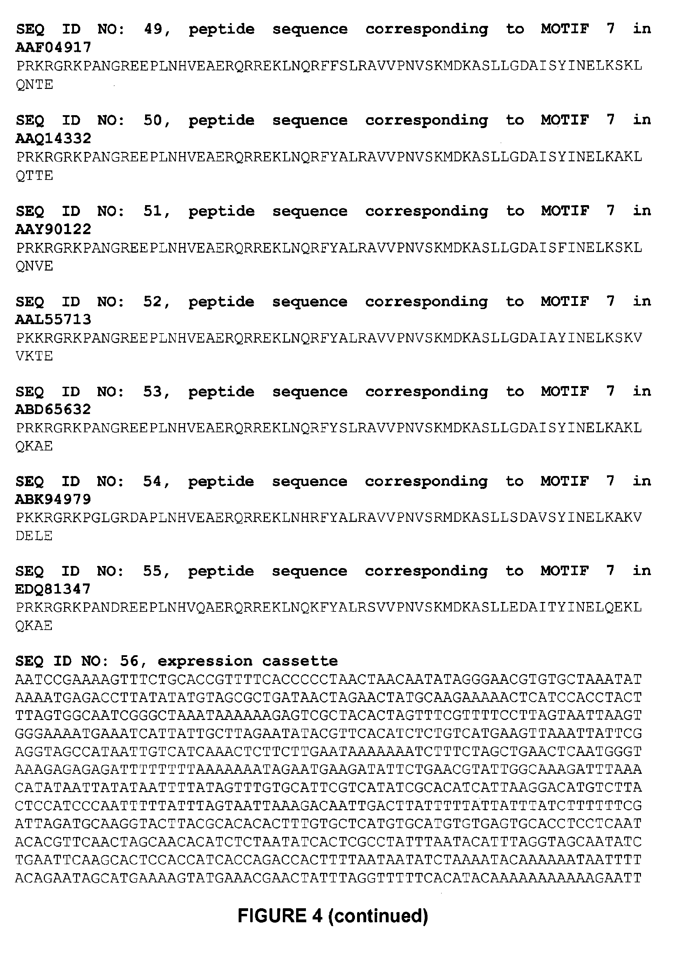

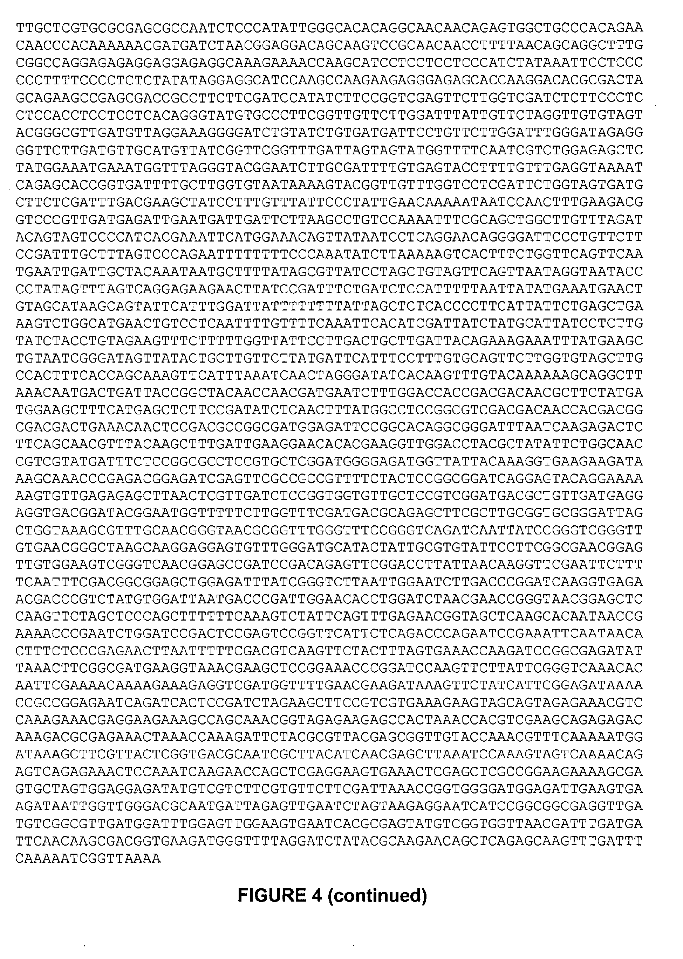

**Fig. 4** details examples of sequences useful in performing the methods according to the present invention.

**Fig. 5** represents a CLUSTAL W (1;83) multiple sequence alignment of RrmJ/FtsJ polypeptides from Table A2.

**Fig. 6** represents the binary vector for increased expression in *Oryza sativa* of a GRP-encoding nucleic acid sequence (wherein said GRP polypeptide is an RrmJ/FtsJ polypeptide) under the control of a rice oleosin promoter (pOleo:: GRP)

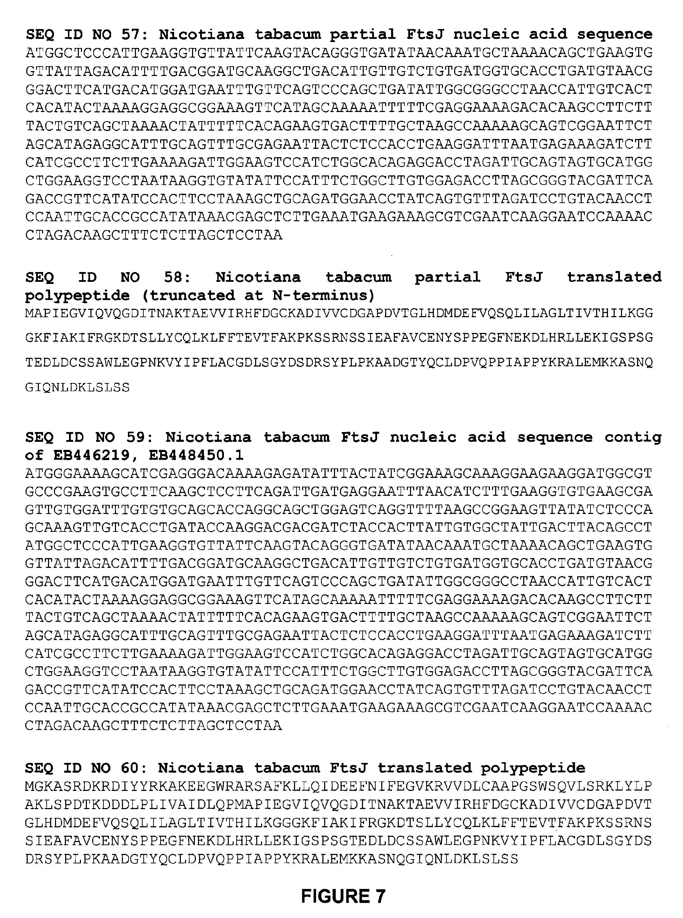

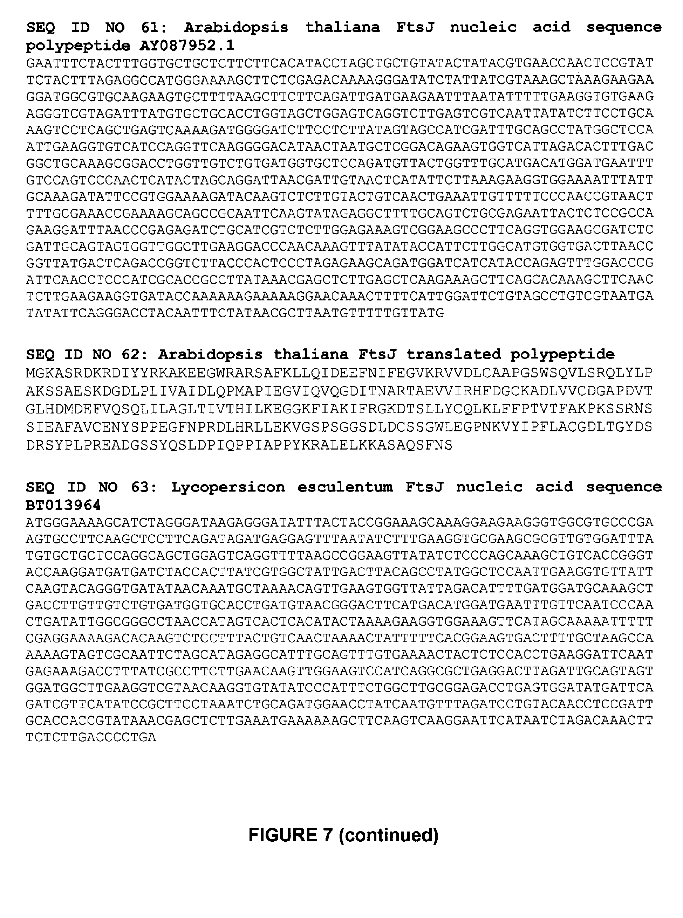

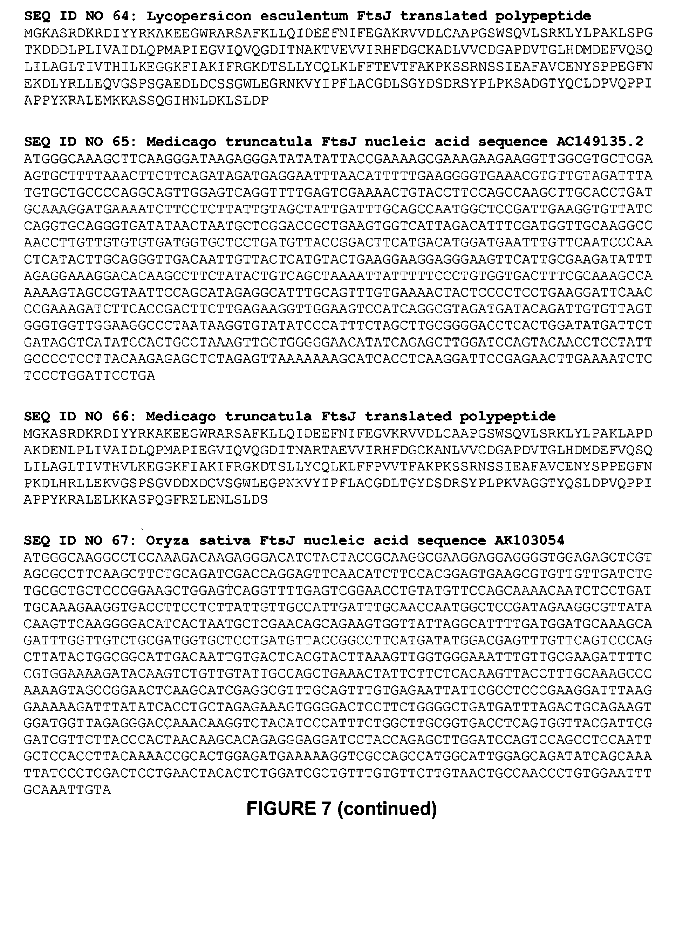

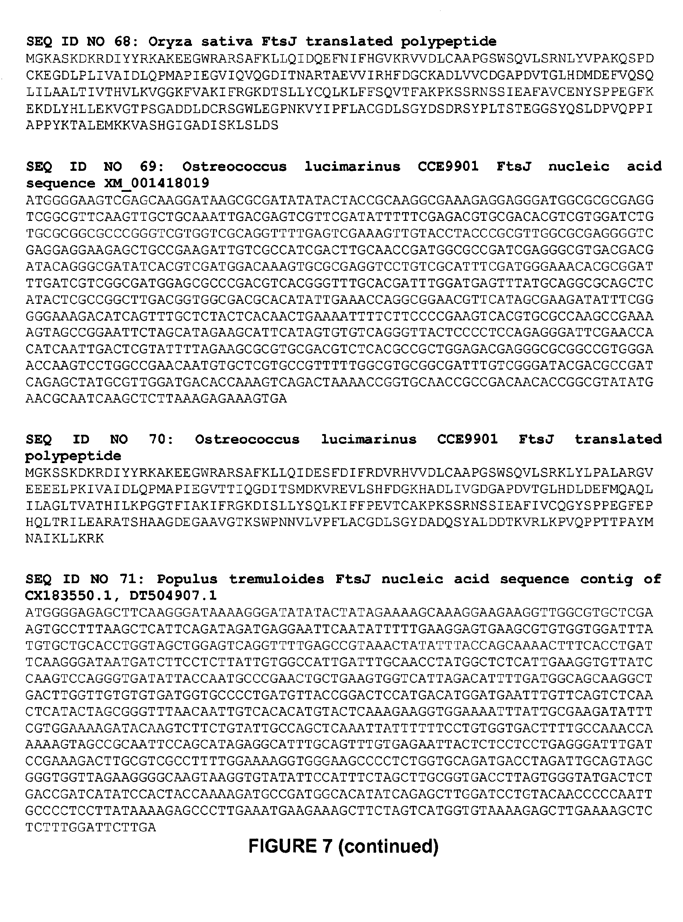

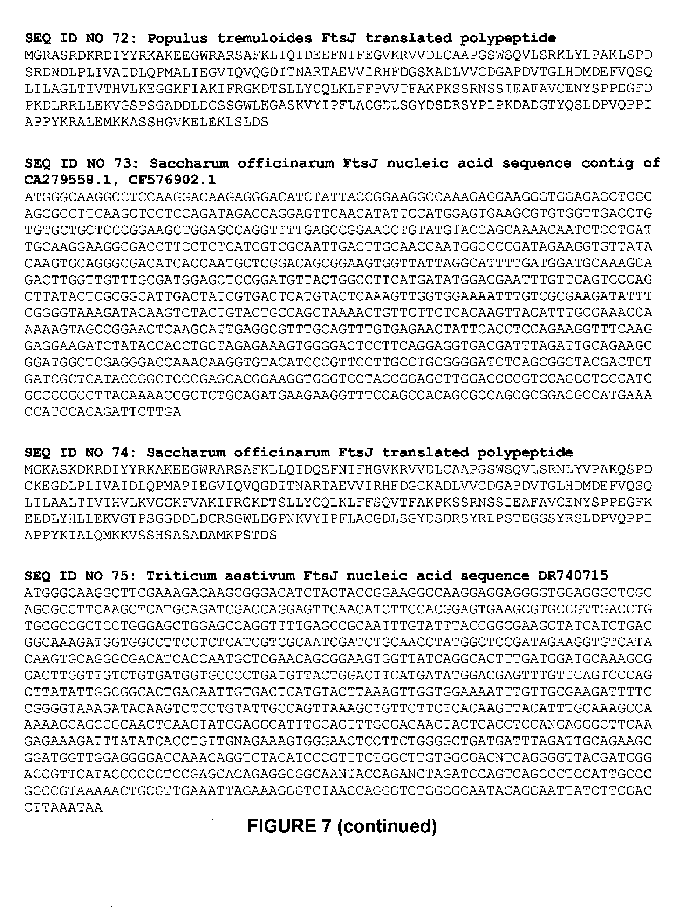

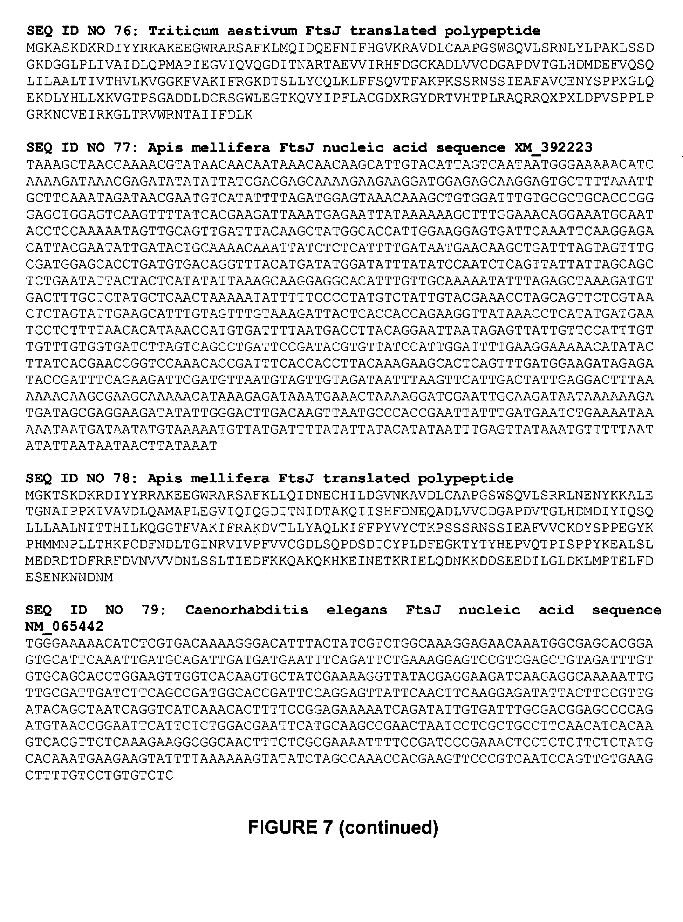

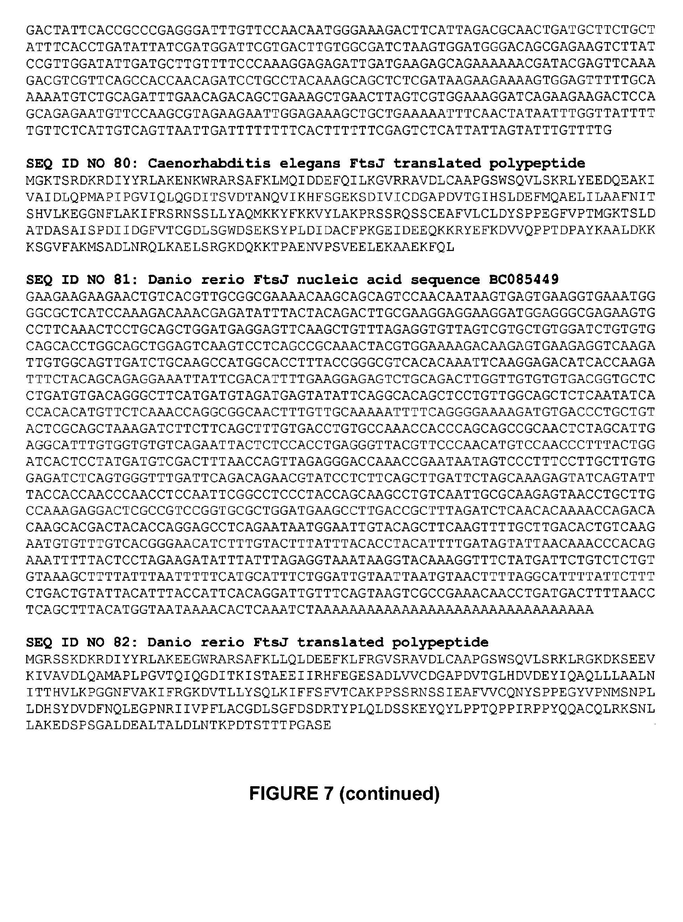

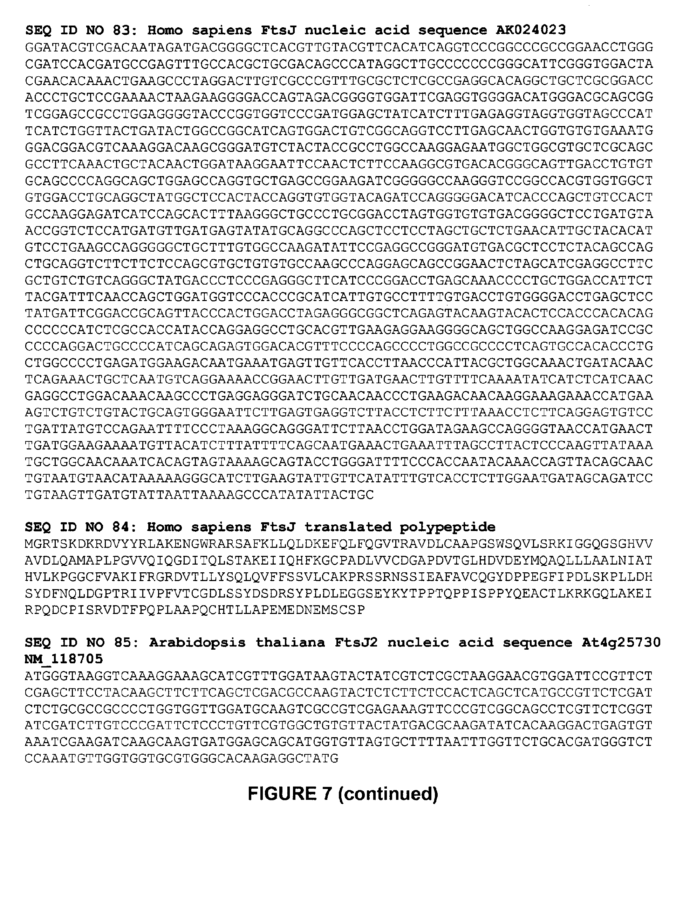

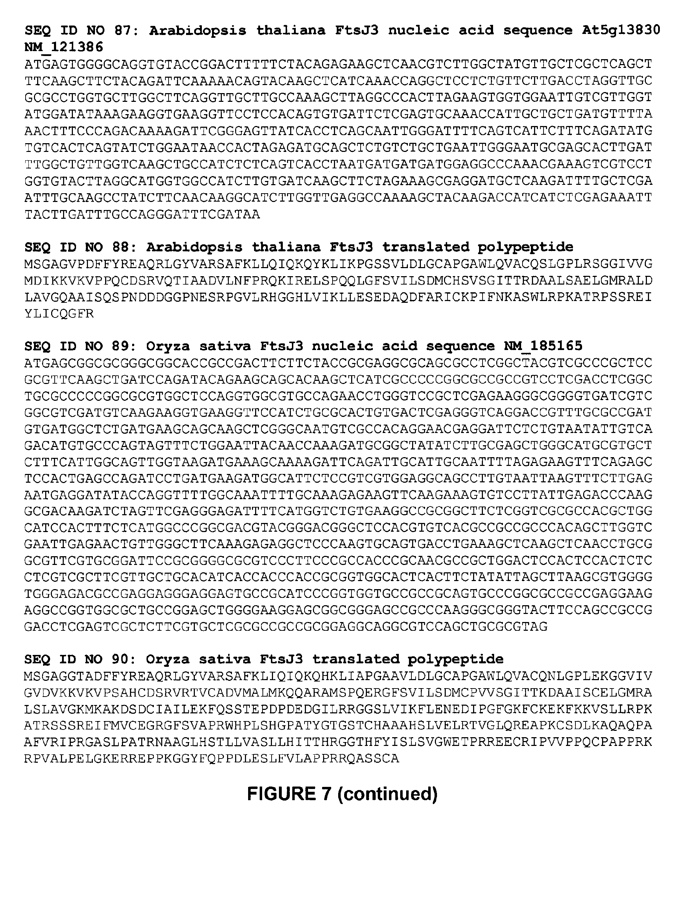

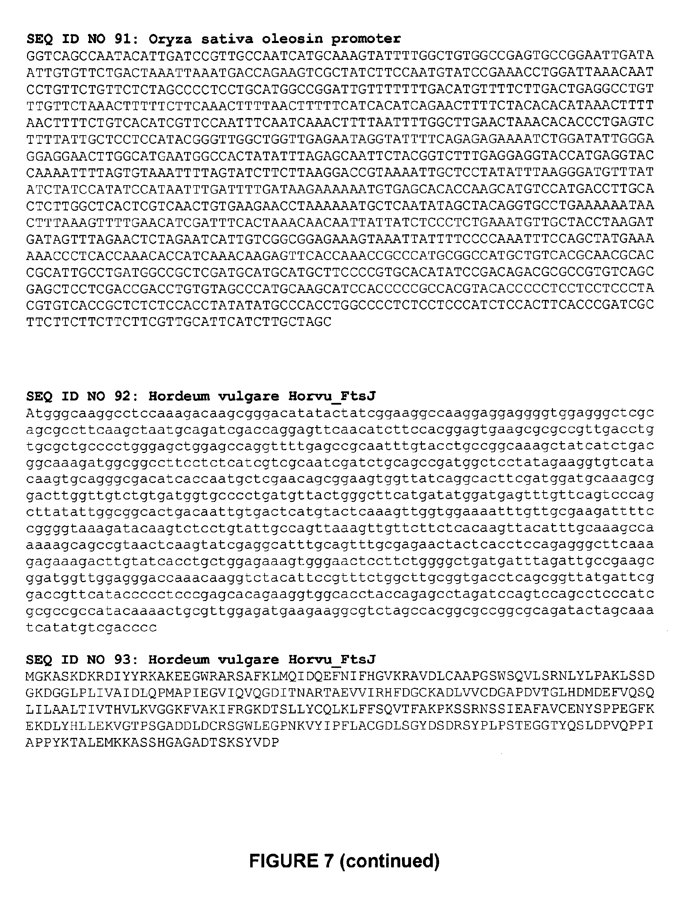

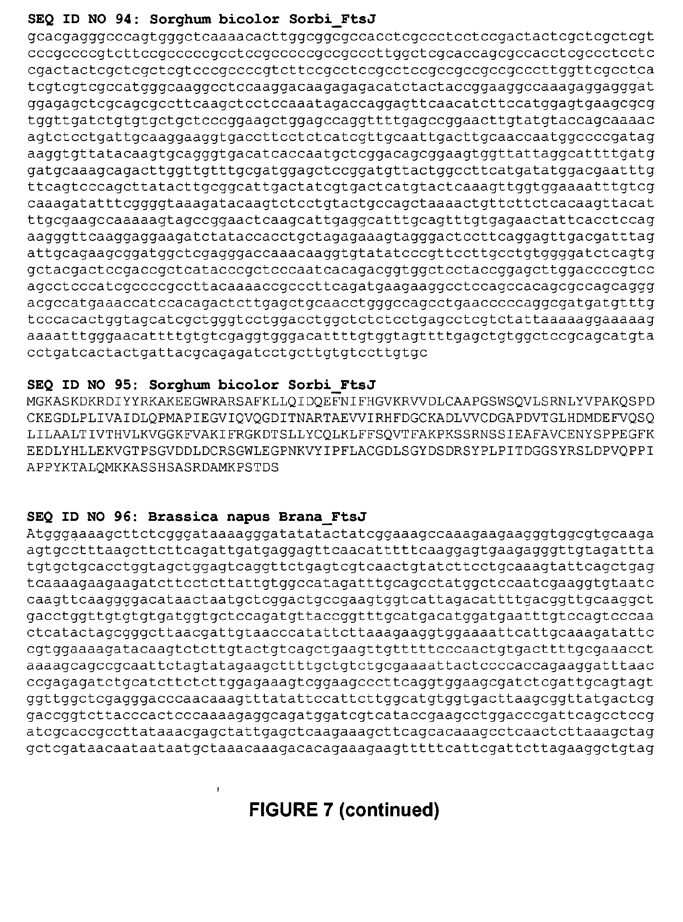

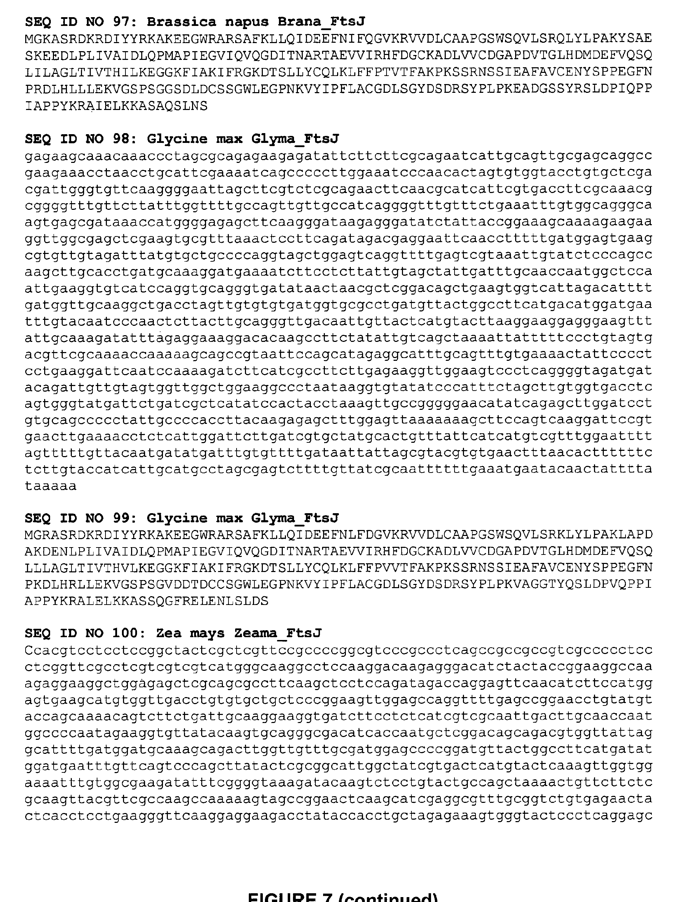

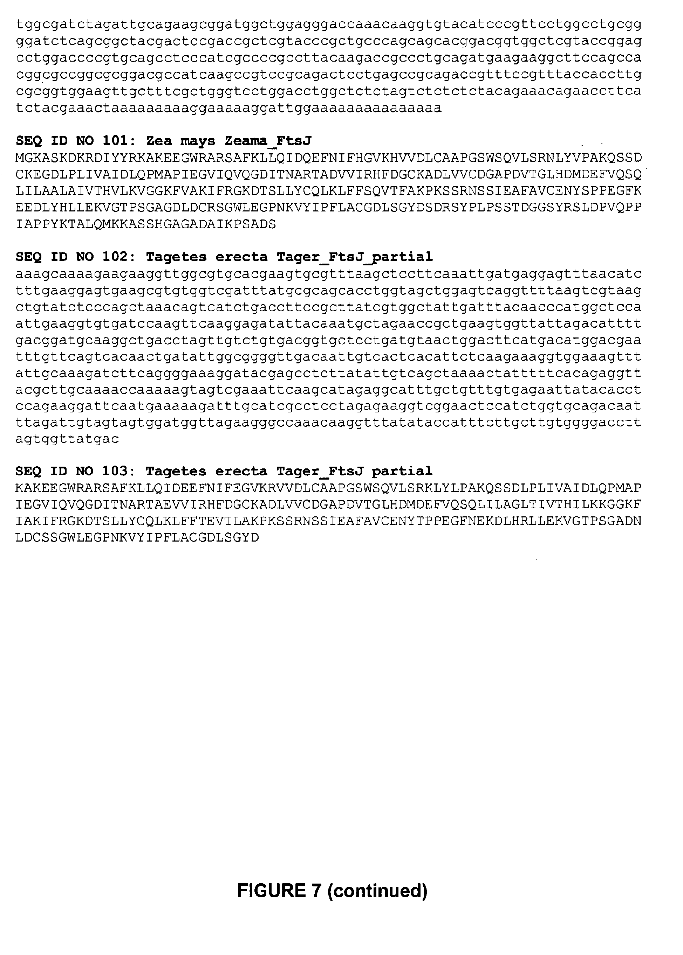

**Fig. 7** details examples of sequences useful in performing the methods according to the present invention.

**Fig. 8** represents the binary vector for increased expression in *Oryza sativa* of a GRP-encoding nucleic acid sequence (wherein said GRP polypeptide is a a basic-helix-loop-helix 4 (bHLH4) polypeptide) under the control of a rice HMGB promoter (pHMGB::GRP)

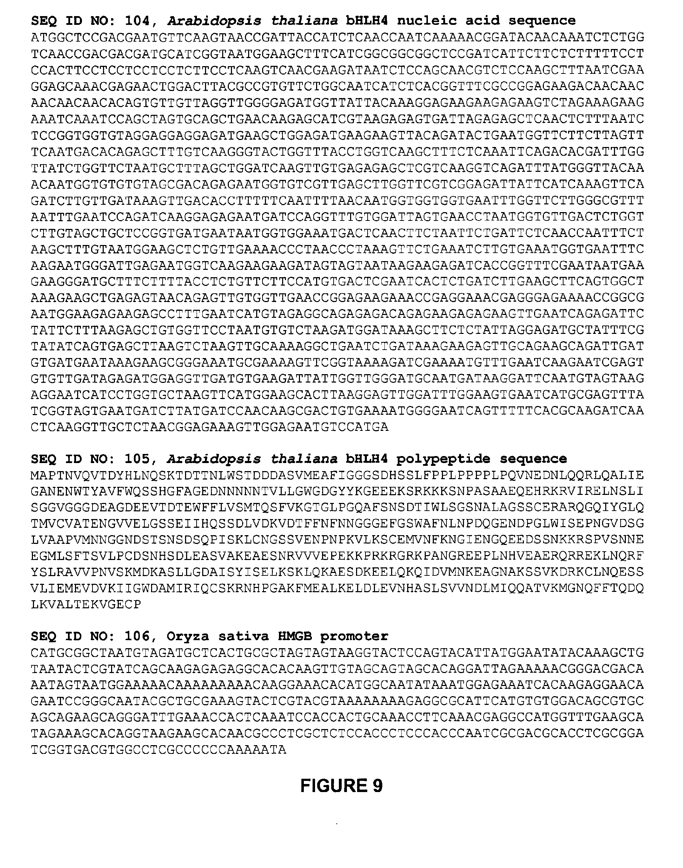

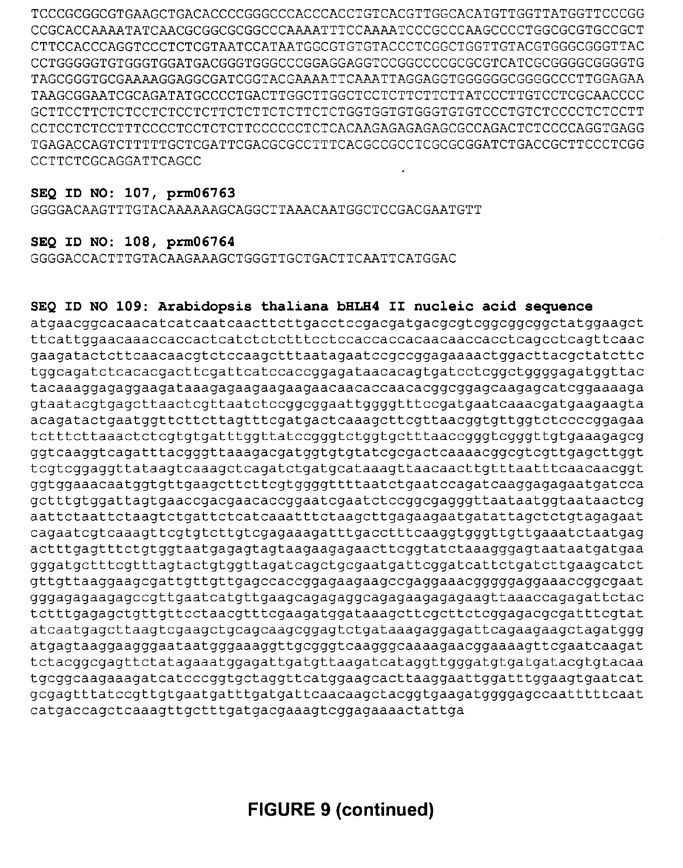

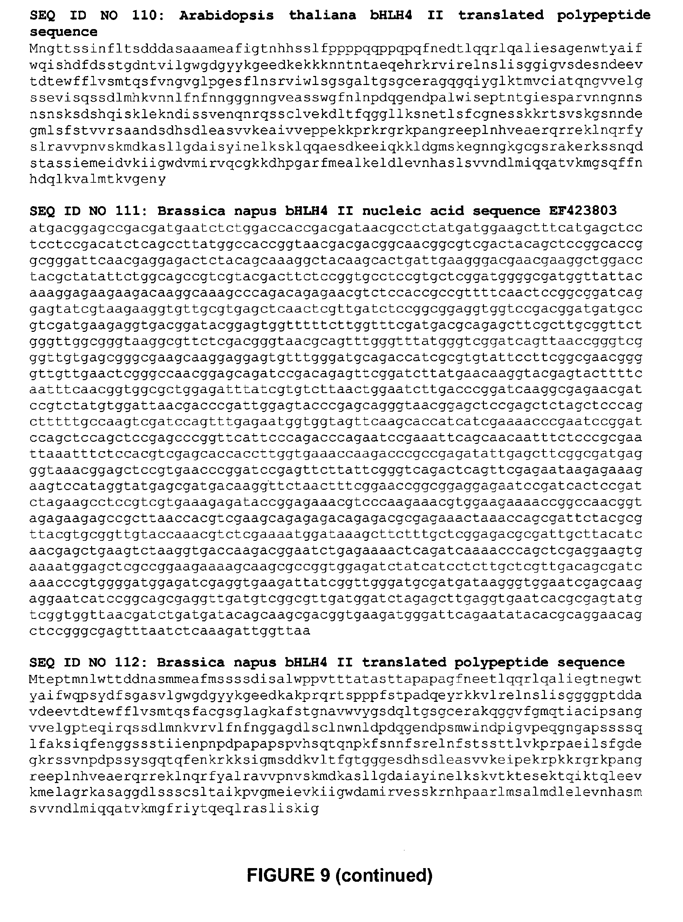

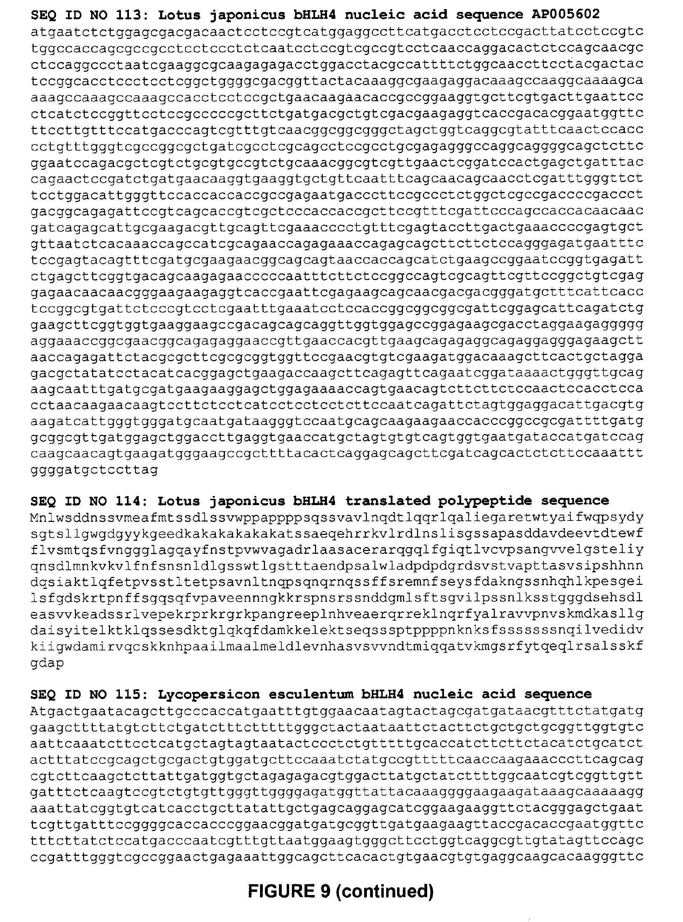

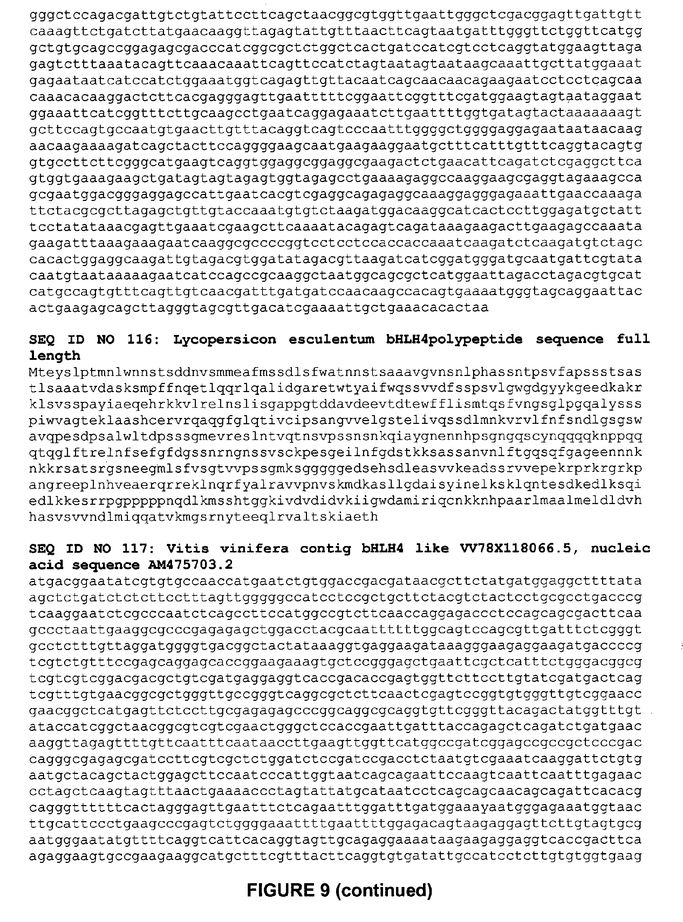

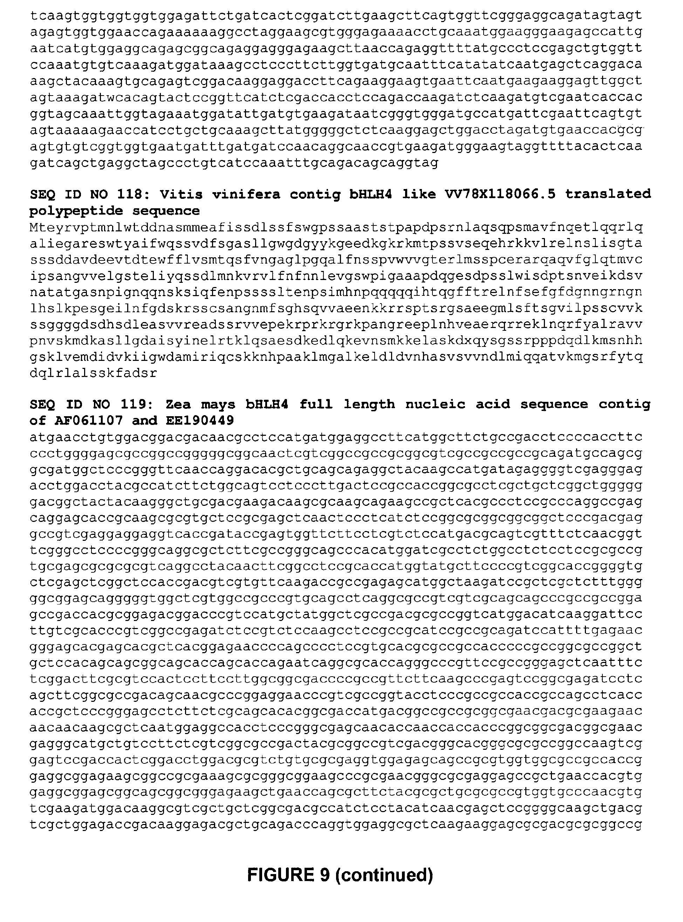

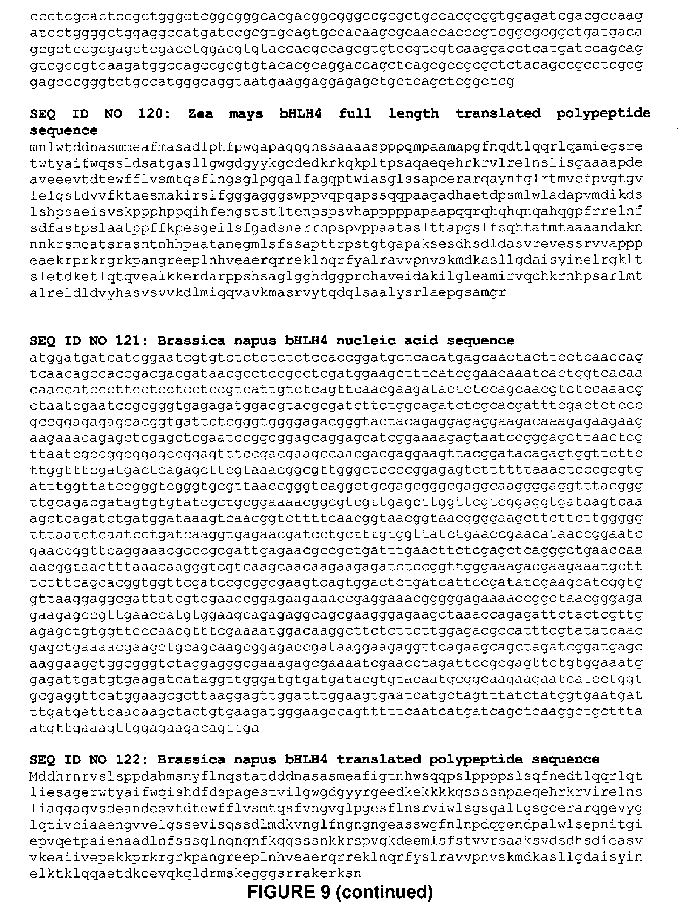

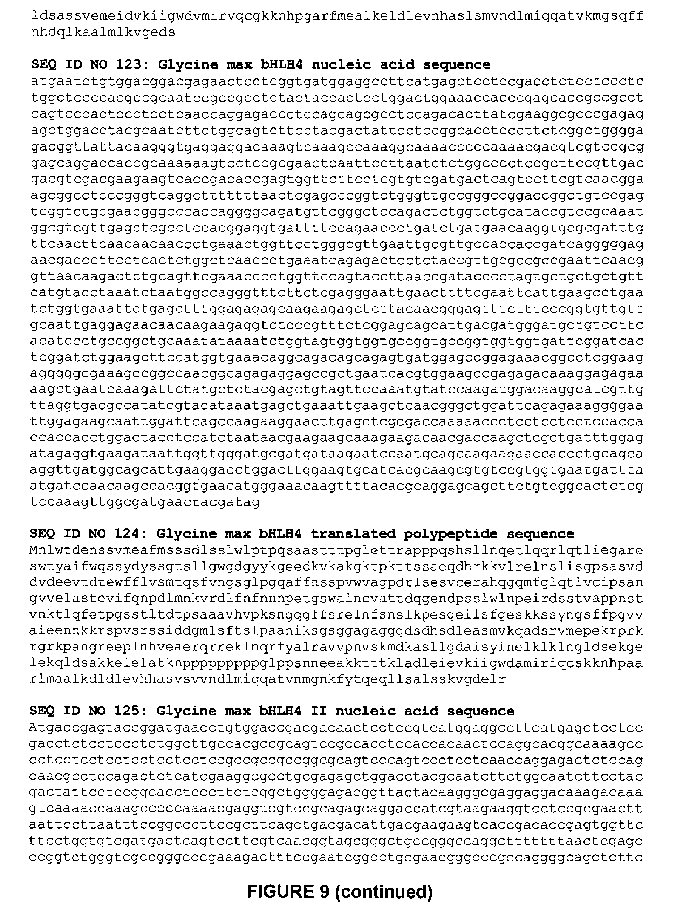

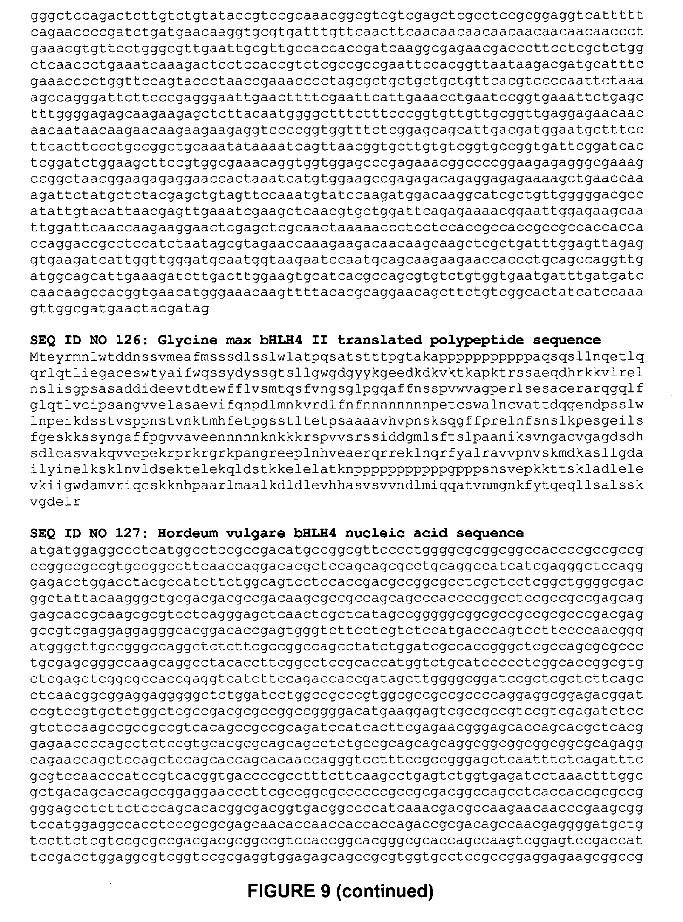

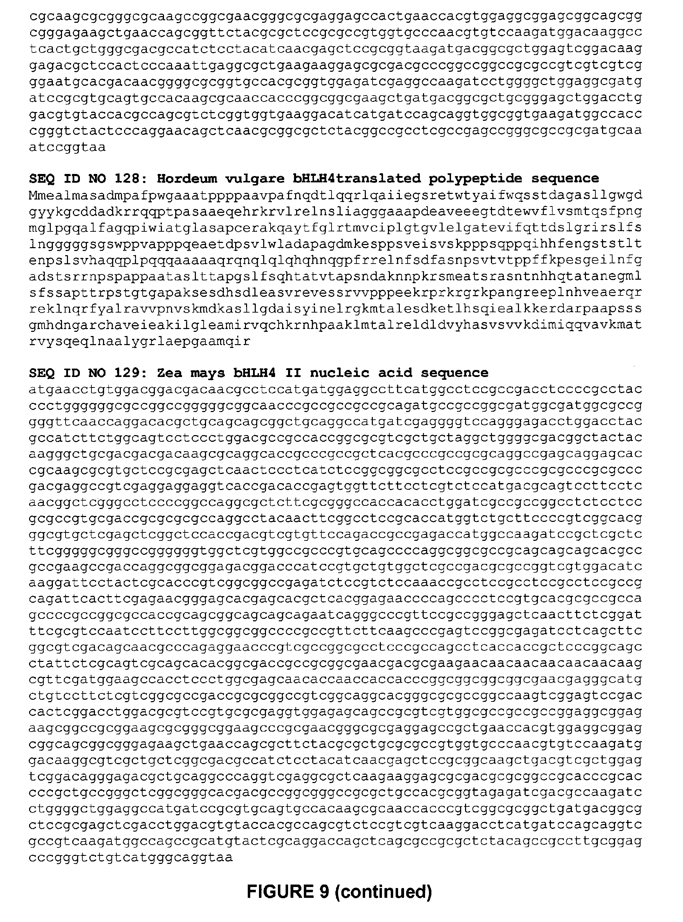

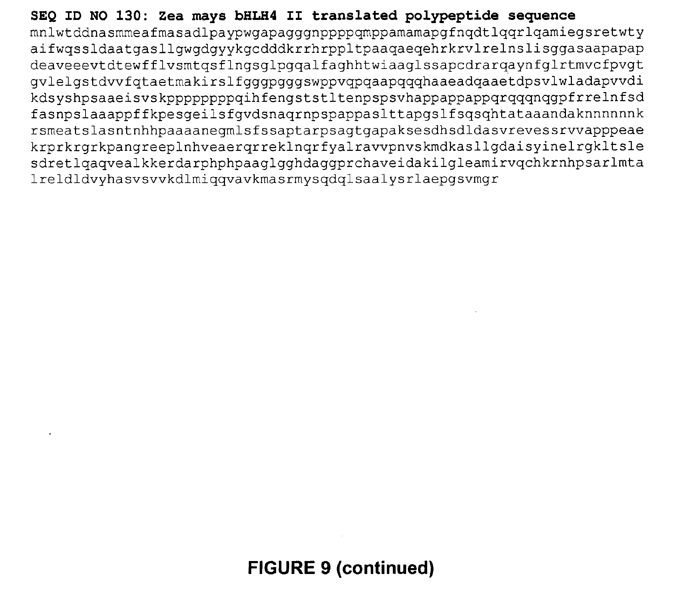

**Fig. 9** details examples of sequences useful in performing the methods according to the present invention.

**Fig. 10** represents a multiple sequence alignment of GRP-polypeptides (wherein said GRP polypeptide is a basic-helix-loop-helix 4 (bHLH4) polypeptide). A consensus sequence, domains and motifs representative of bHLH4-polypeptides are given. In the consensus sequence the highly conserved amino acids are provided; empty blank spaces in between represent any amino acid.

**Fig. 11** represents the binary vector for increased expression in *Oryza sativa* of a GRP-encoding nucleic acid sequence (wherein said GRP polypeptide is an isopentenyl transferase (IPT) polypeptide under the control of a rice prolamine promoter (pProl::GRP)

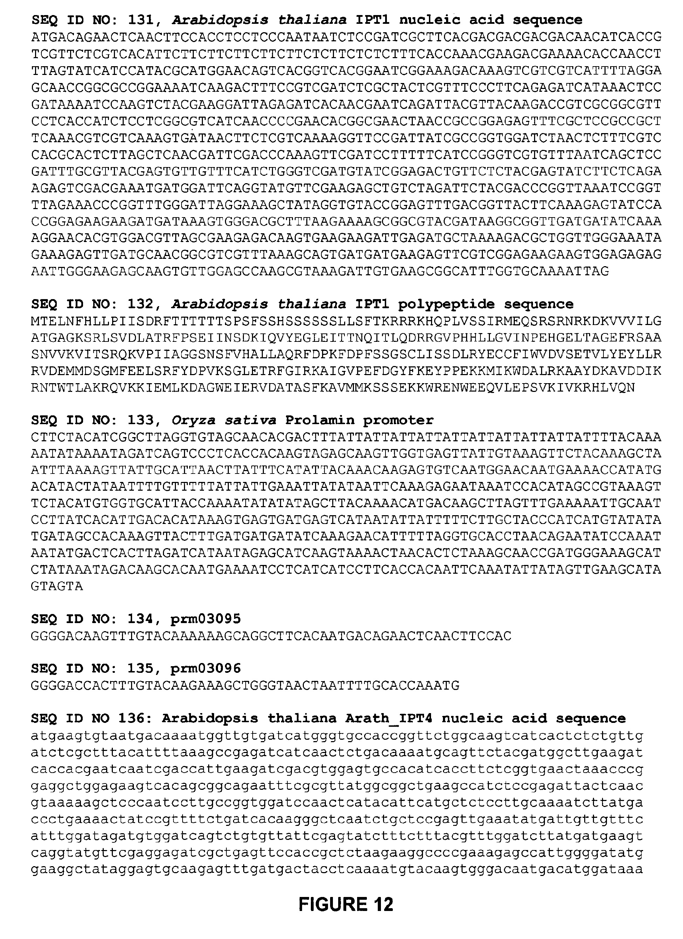

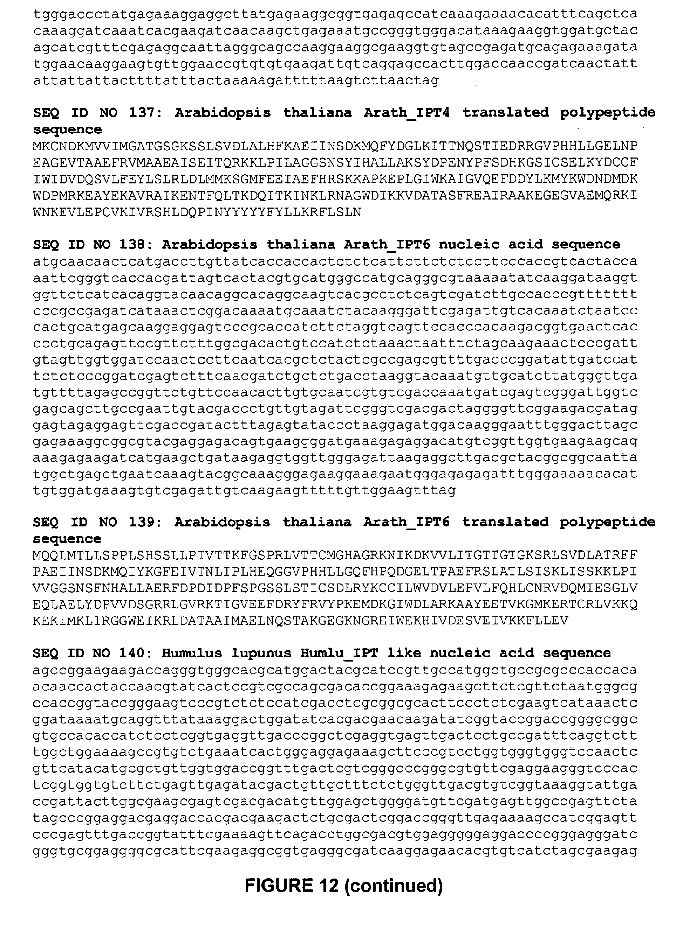

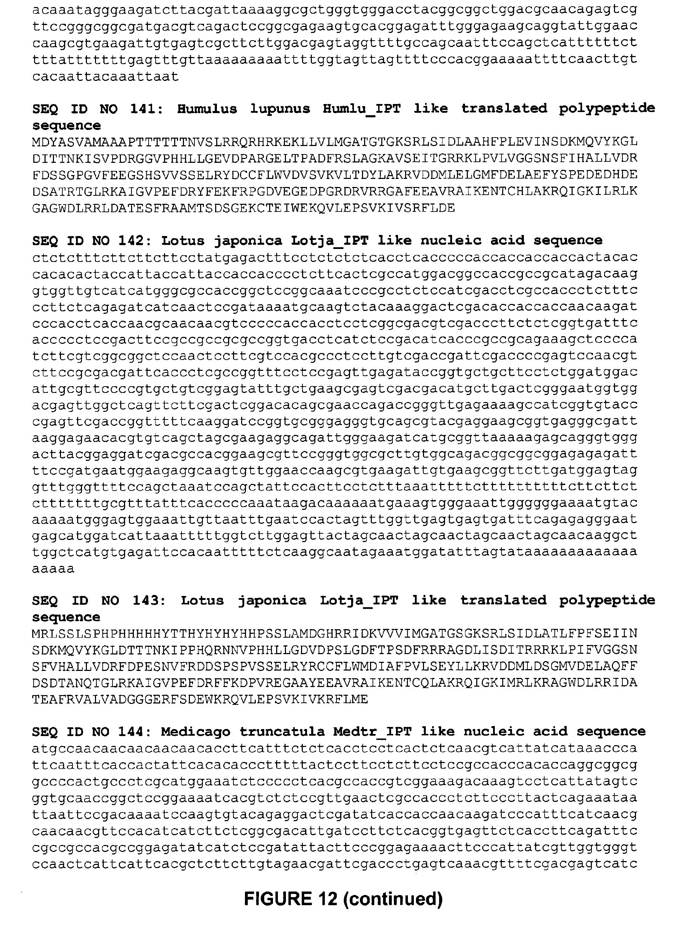

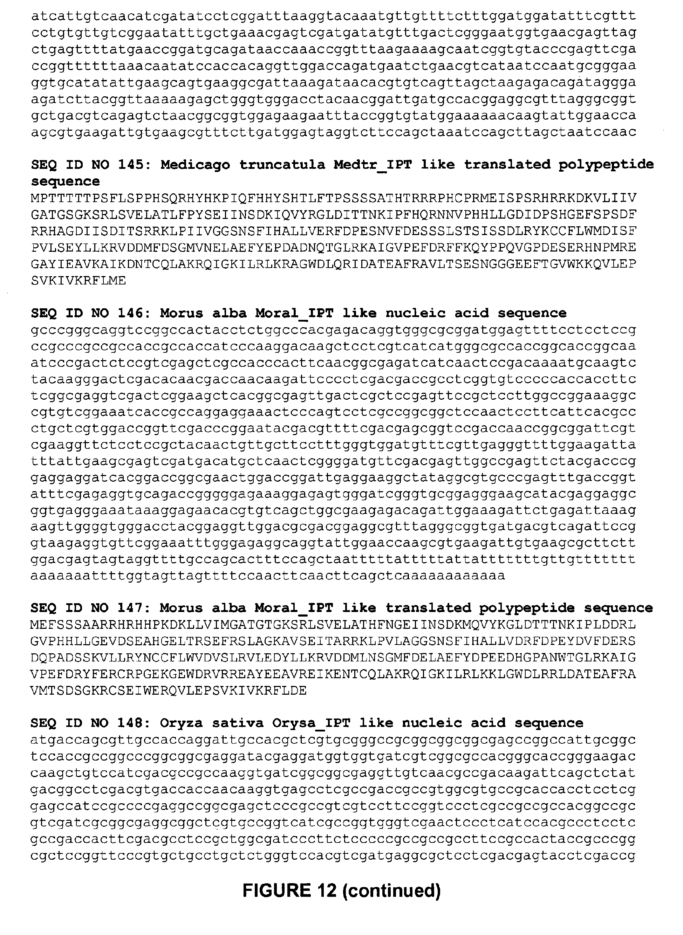

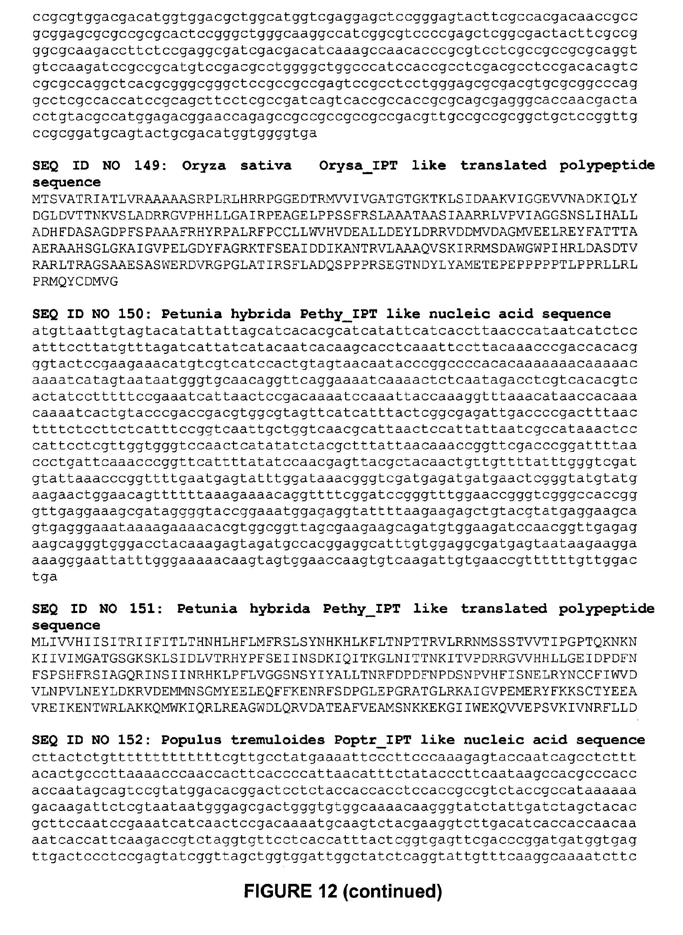

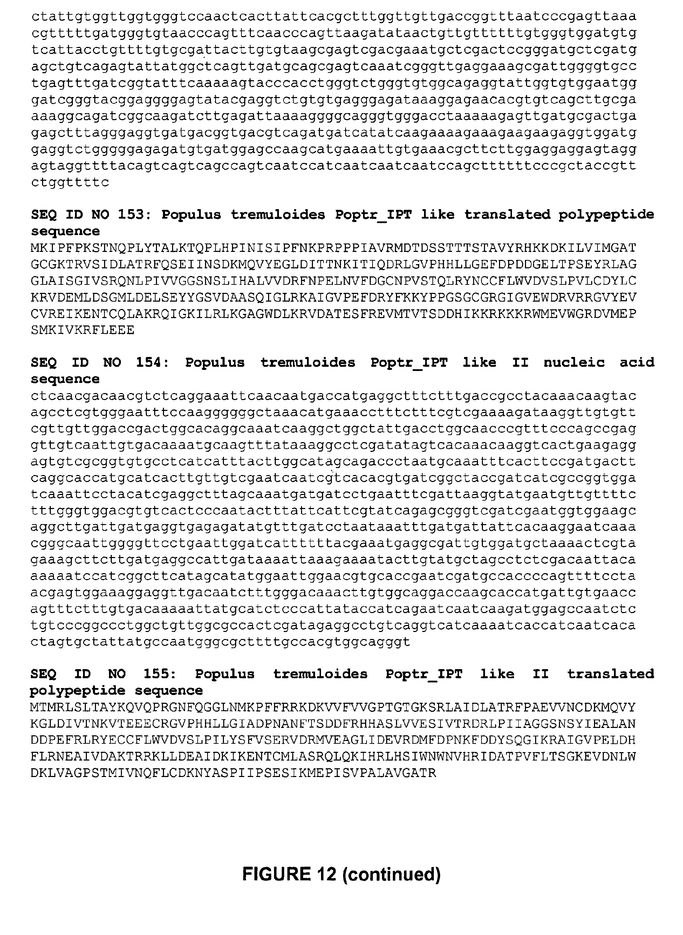

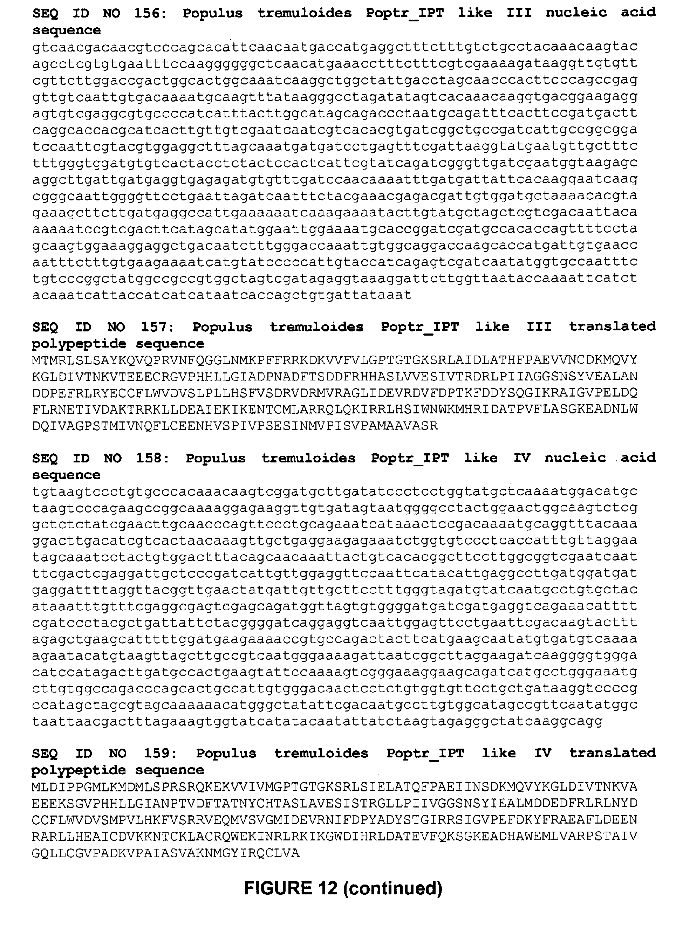

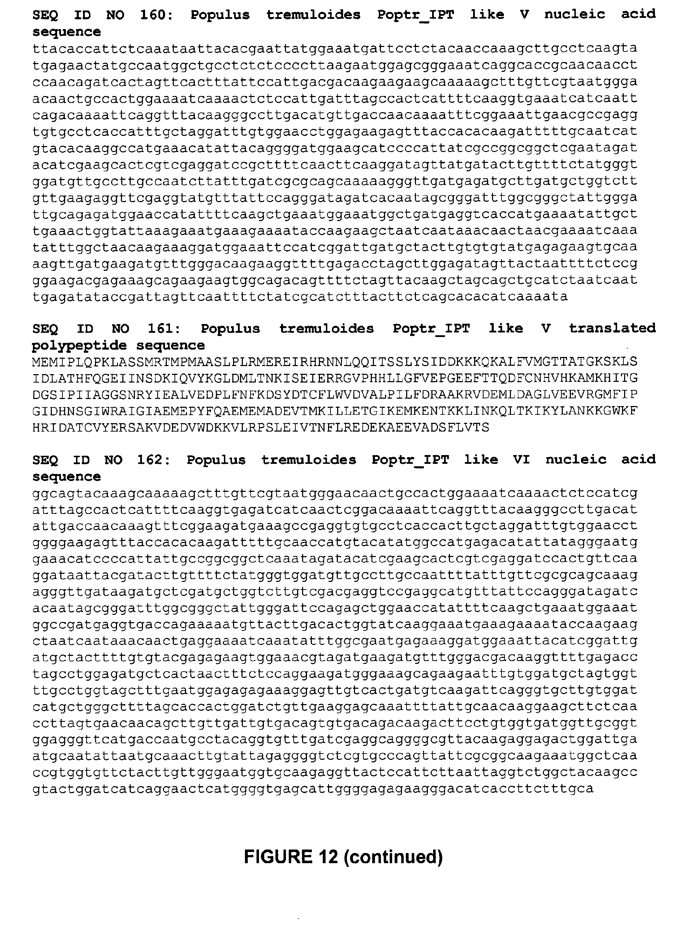

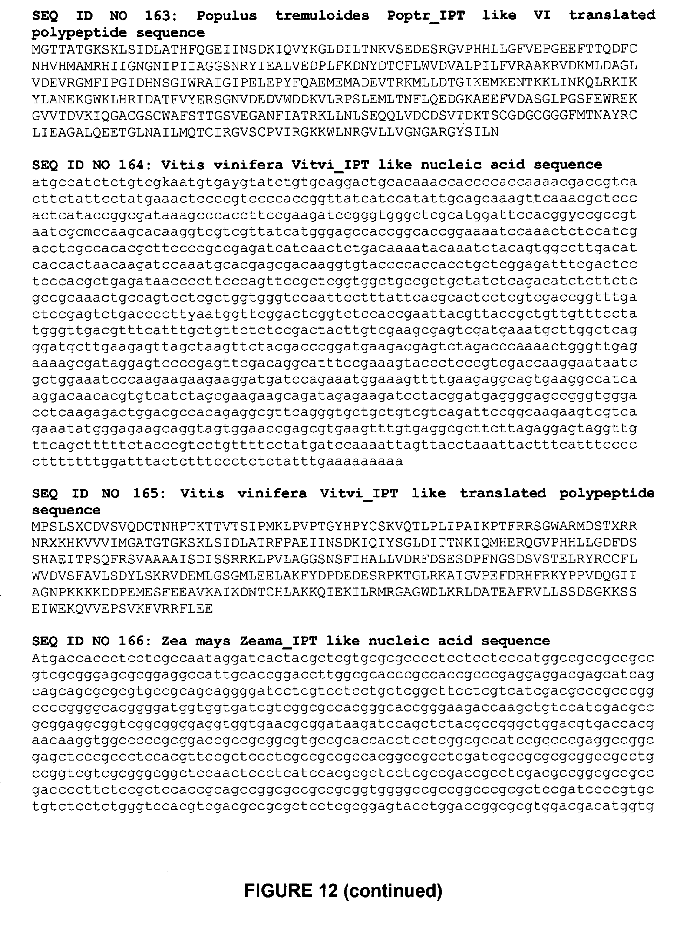

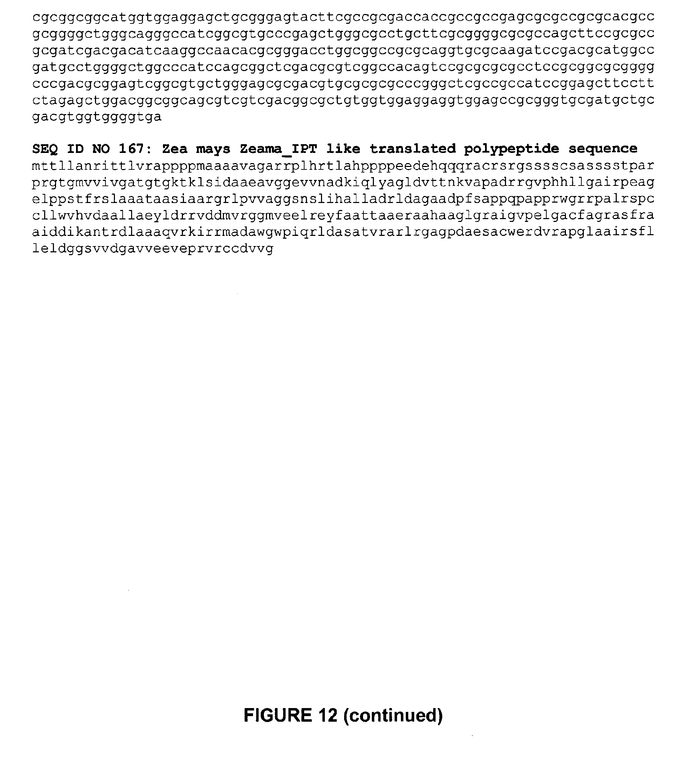

**Fig. 12** details examples of sequences useful in performing the methods according to the present invention.

**Fig. 13** represents a multiple sequence alignment of GRP-polypeptides (wherein said GRP polypeptide is a isopentenyl transferase (IPT) polypeptide). A consensus sequence, domains and motifs representative of IPT-polypeptides are given. In the consensus sequence the highly conserved amino acids are provided; empty blank spaces in between represent any amino acid.

**Fig. 14** represents the amino acid sequence and domain structure of SEQ ID NO: 169. The two conserved B-box domains in SEQ ID NO: 169 are indicated by dotted line (B-box domain 1) and by a continuous line (B-box domain 2). The four cysteine residues in each B-box domain with putative potential to bind zinc ions are indicated by boxes.

**Fig. 15** represents a multiple sequence alignment of STO polypeptides. Position of the conserved amino acid residues and domains corresponding to those described in Fig. 14 are indicated. A consensus sequence representative of STO polypeptides is given. In the consensus sequence the highly conserved amino acids are provided; empty blank spaces in between represent any amino acid.

**Fig. 16** shows phylogenetic tree of STO polypeptides. In the phylogenetic tree SEQ ID NO: 169 is represented by Os04g0540200.

**Fig. 17** represents the binary vector for increased expression in Oryza sativa of an STO-encoding nucleic acid under the control of a rice GOS2 promoter (pGOS2)

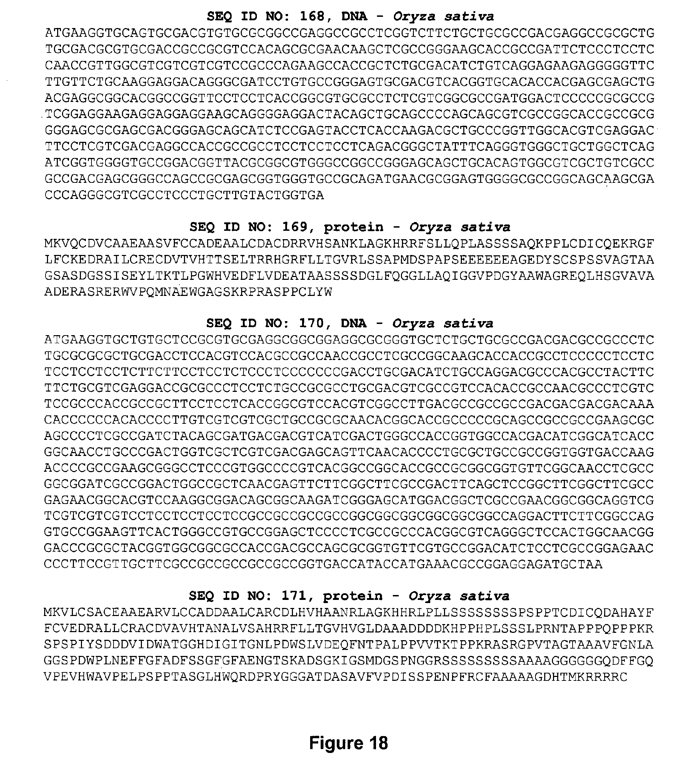

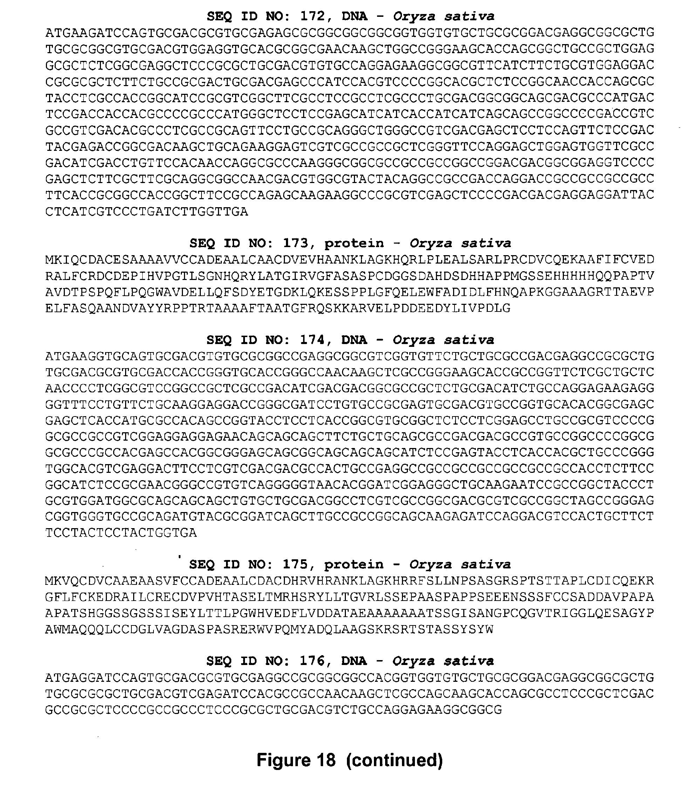

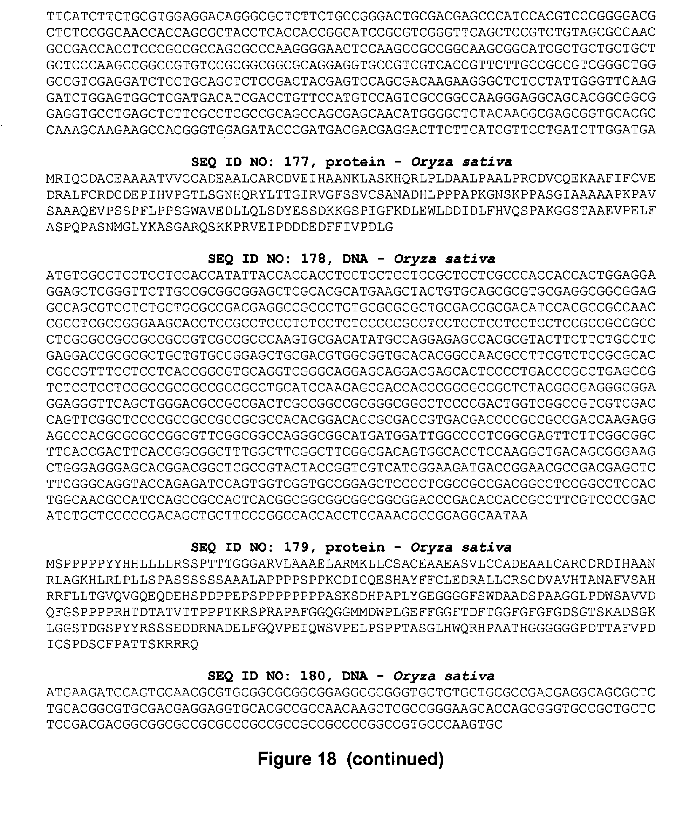

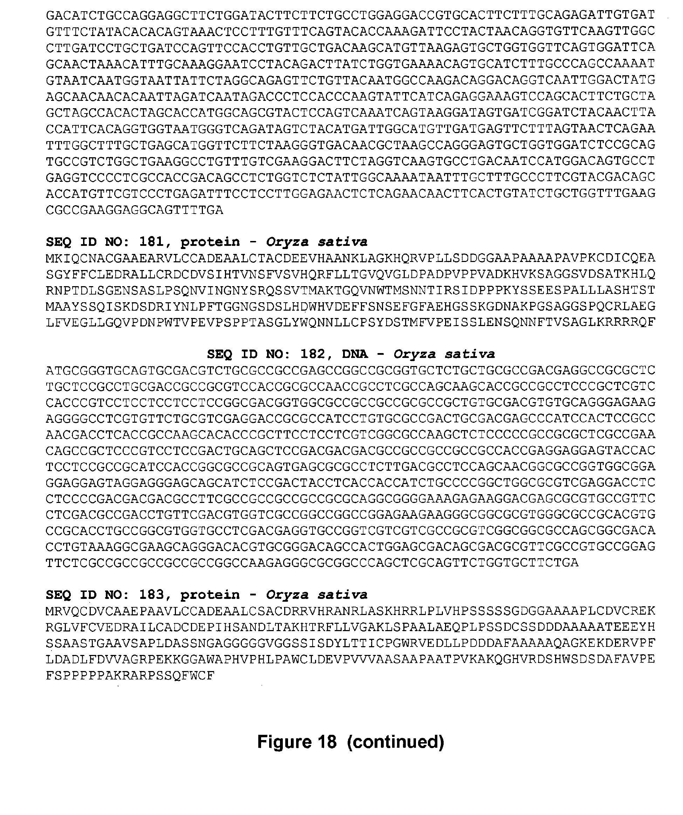

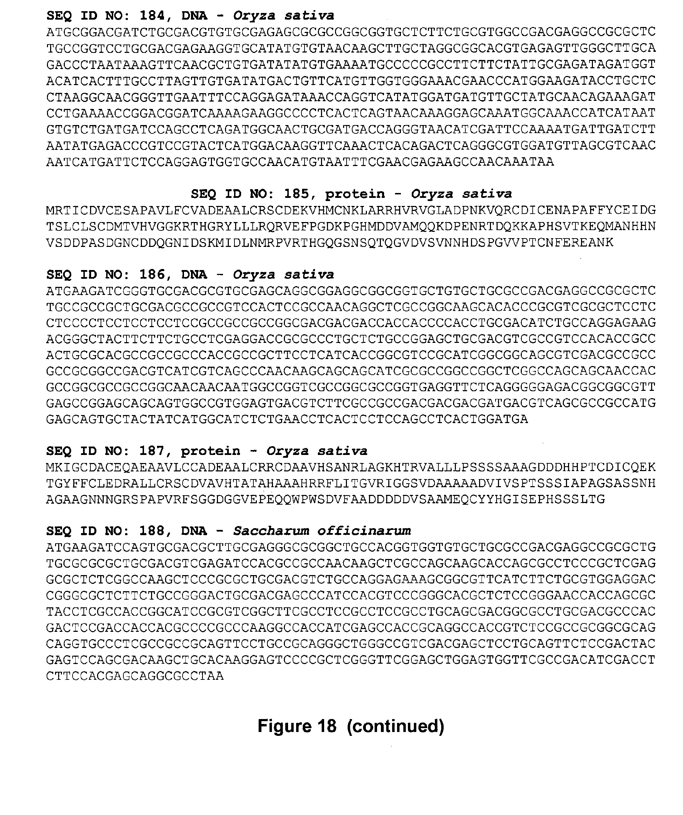

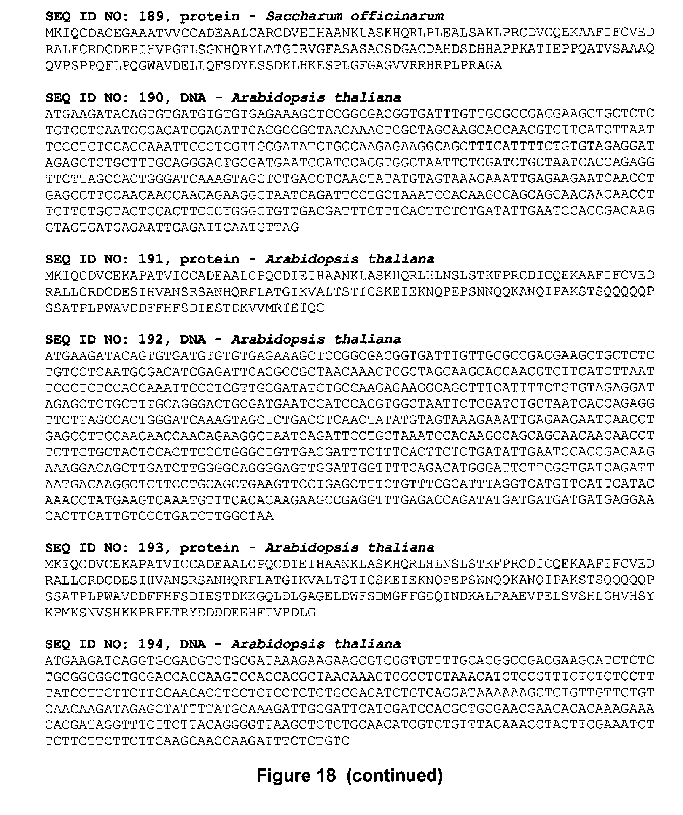

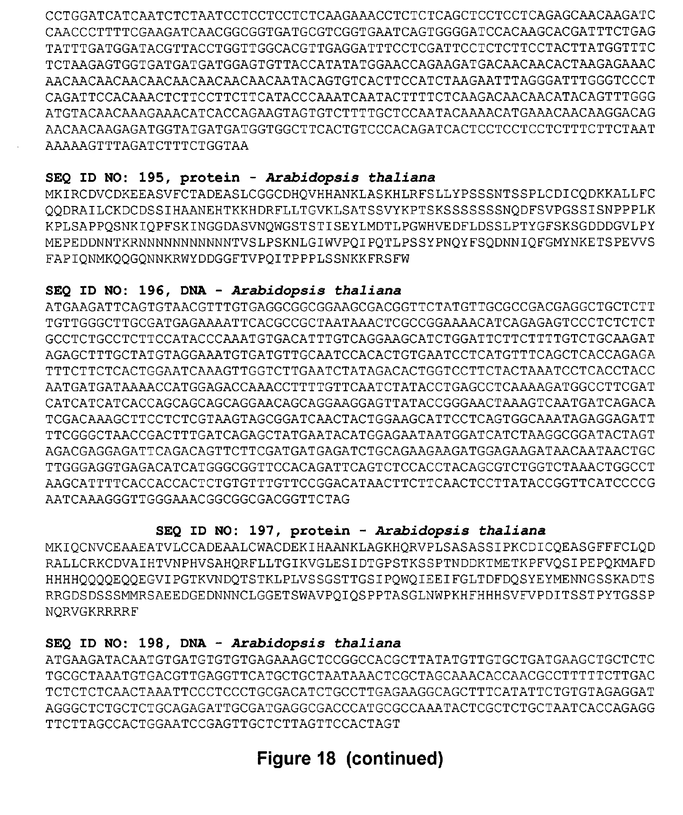

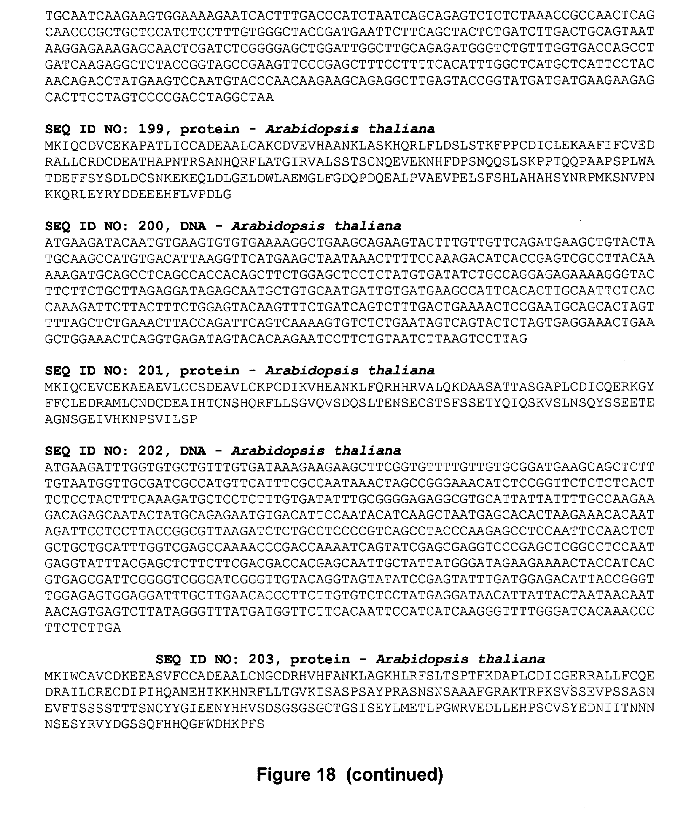

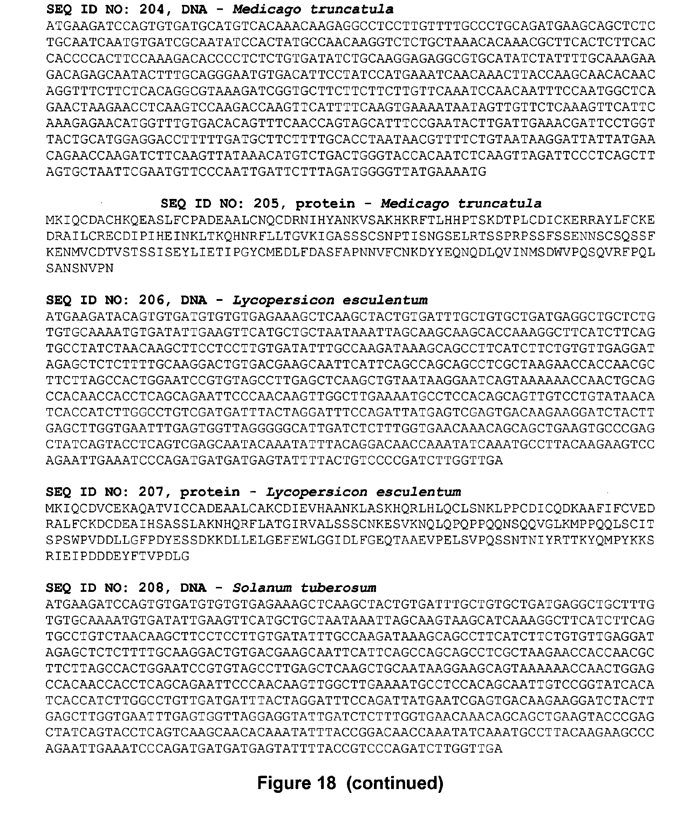

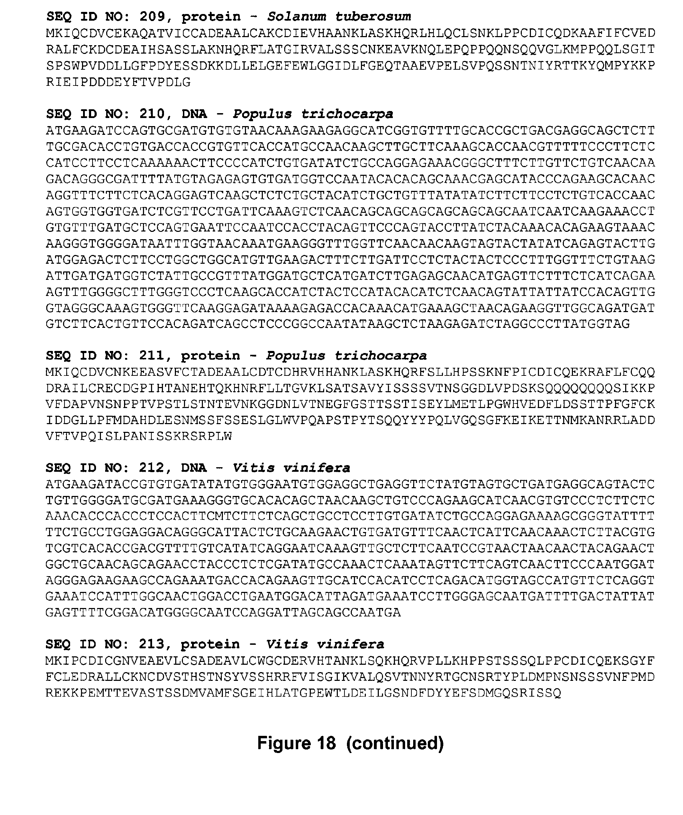

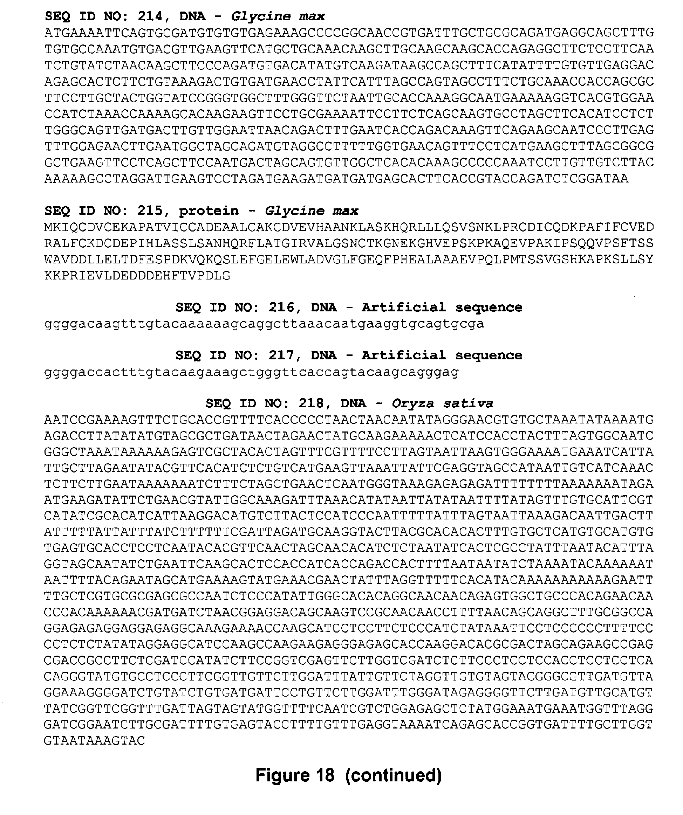

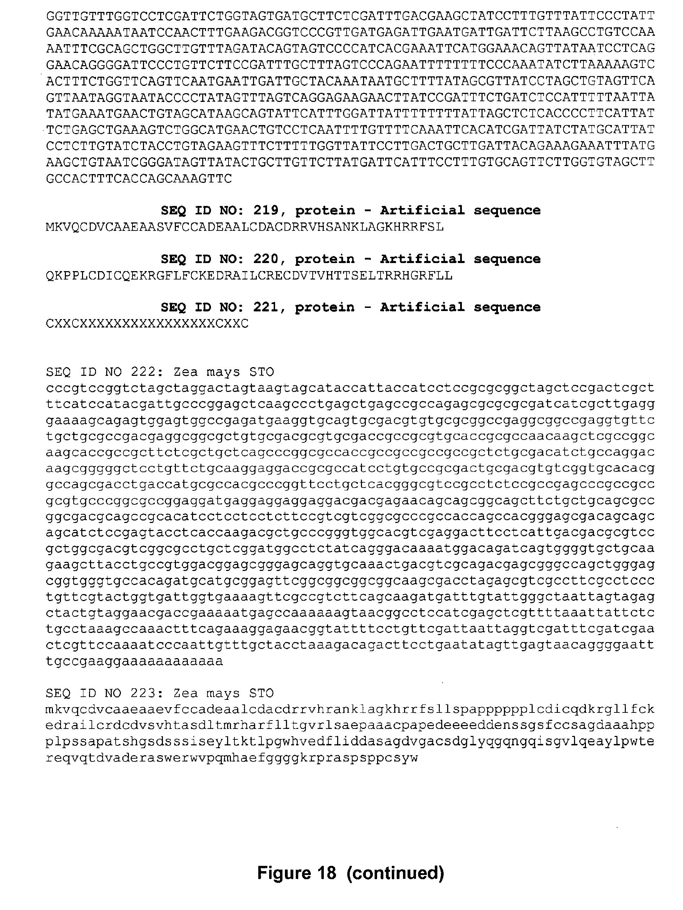

**Fig. 18** details examples of sequences useful in performing the methods according to the present invention.

**Fig. 19** represents the amino acid sequence of SEQ ID NO: 225 wherein relevant functional domains and conserved motifs are indicated. Epimerase domain (boxed region), Motif 8 (capital letters in bold, underlined with an interrupted line), Motif 9 (small letters in bold), Motif 10 (capital letter in bold), Motif 11 (capital letters underlined by dots) and Motif 12 (capital letters underlined with a continuous line) are indicated.

**Fig. 20** represents a multiple protein alignment of the UGE polypeptides of Table A6. A consensus sequences is given. Position of the conserved domain and motifs is indicated.

**Fig. 21** shows a phylogenetic tree of the of the UGE polypeptides of Table A6.

**Fig. 22** represents the binary vector for increased expression in Oryza sativa of a UGE-encoding nucleic acid under the control of a rice GOS2 promoter (pGOS2).

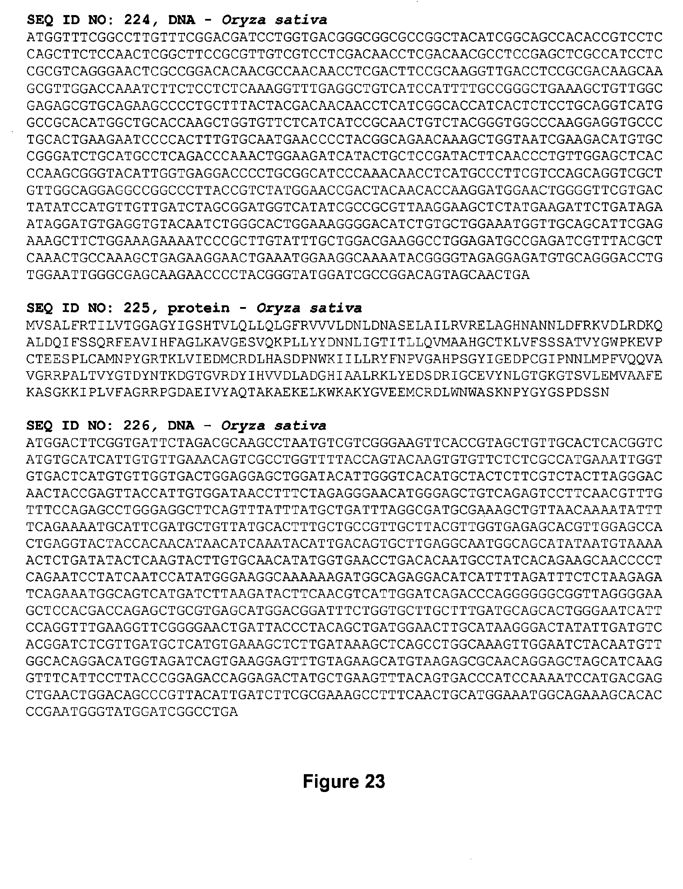

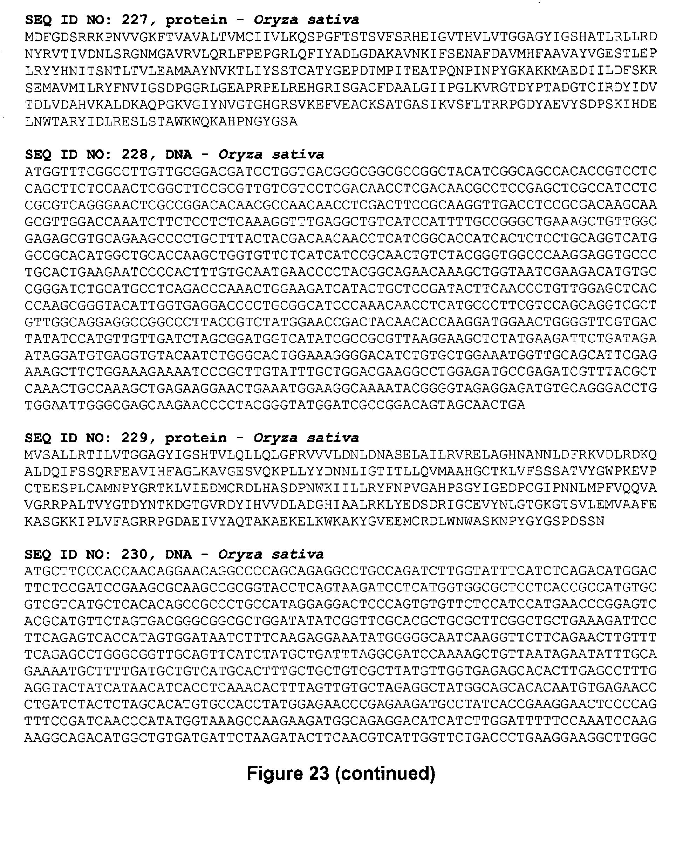

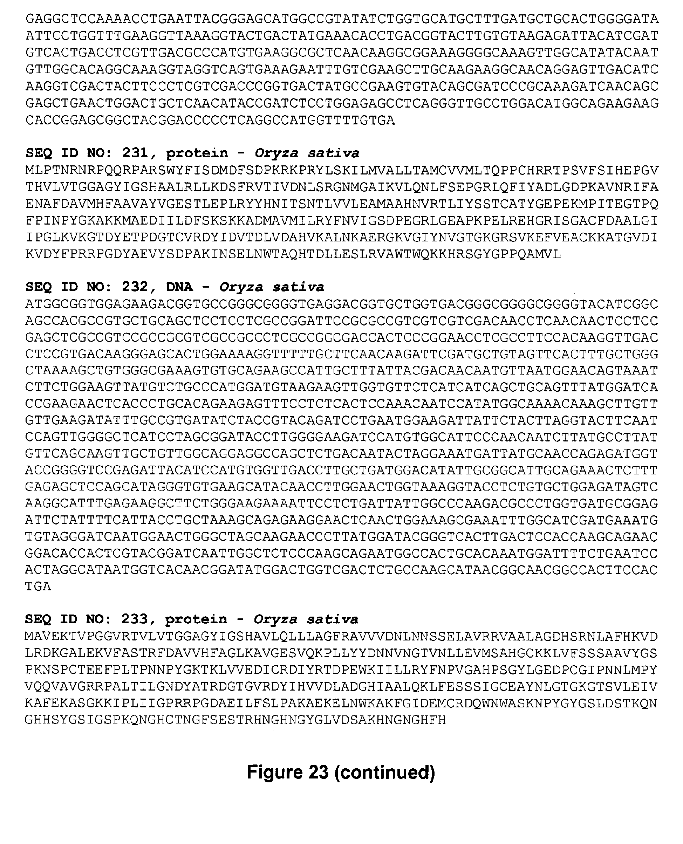

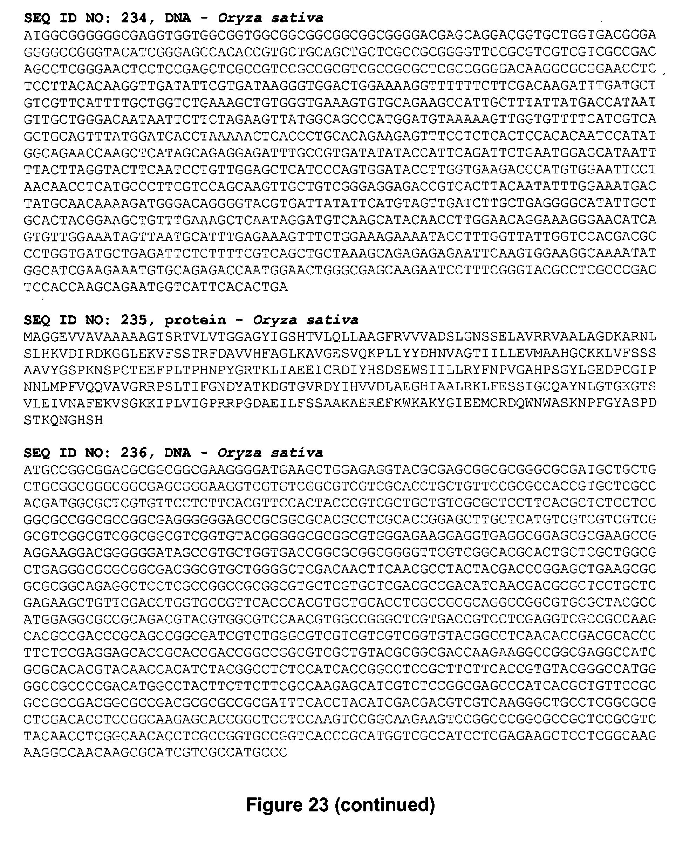

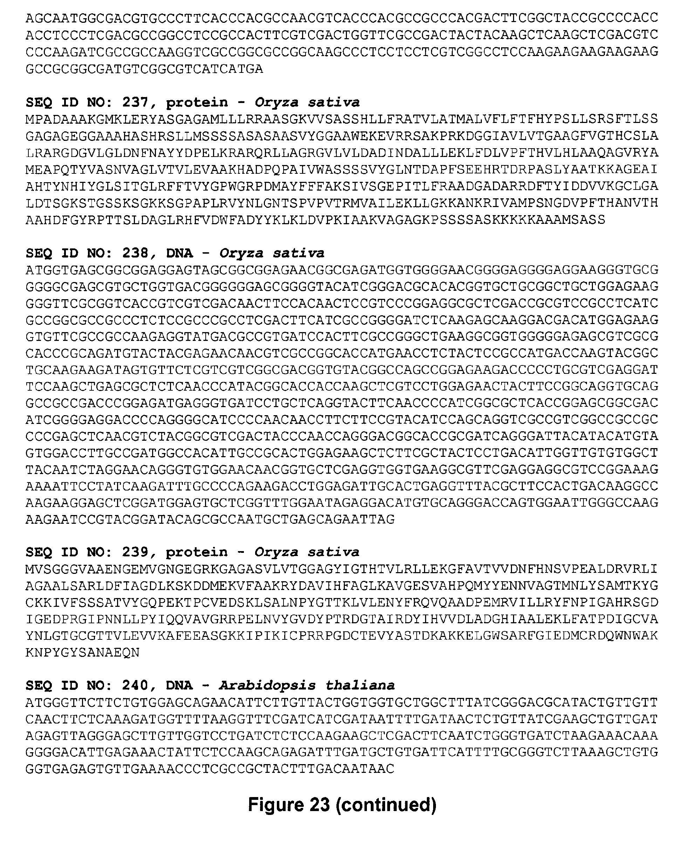

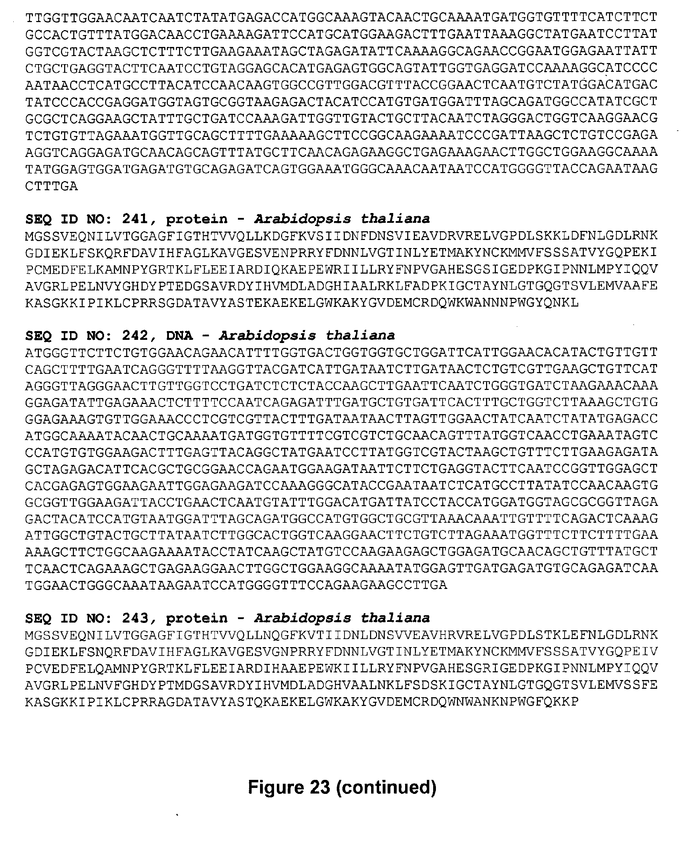

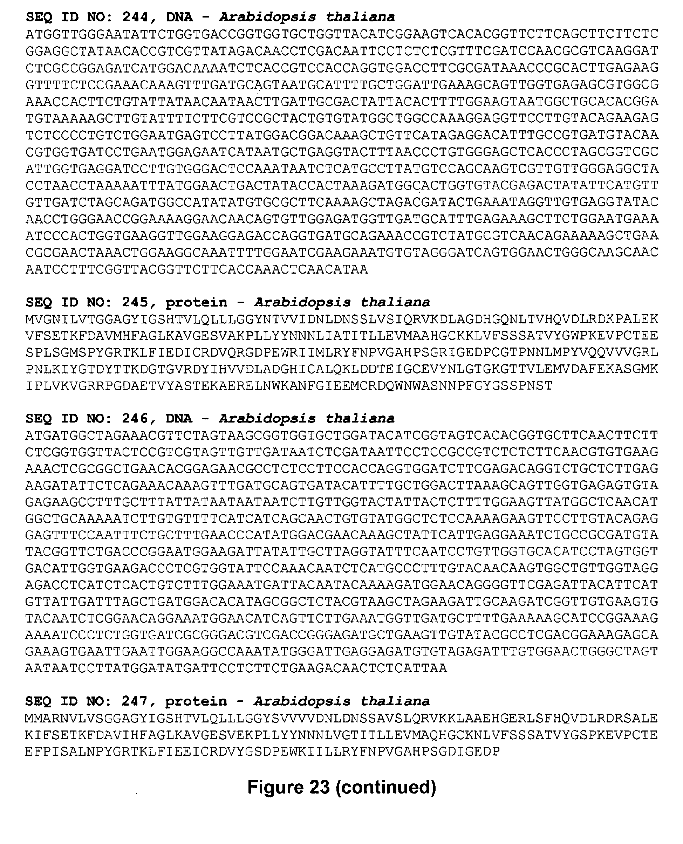

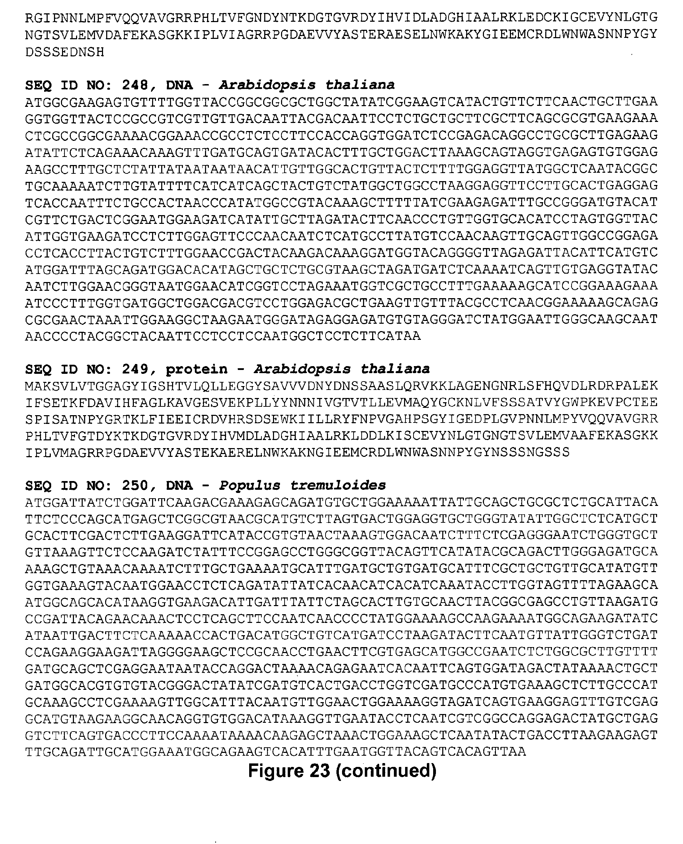

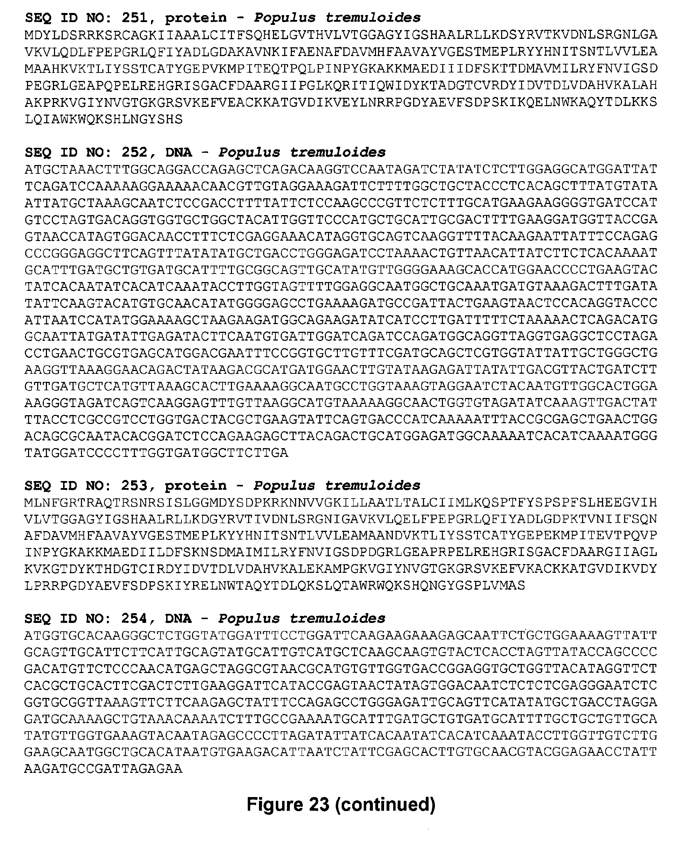

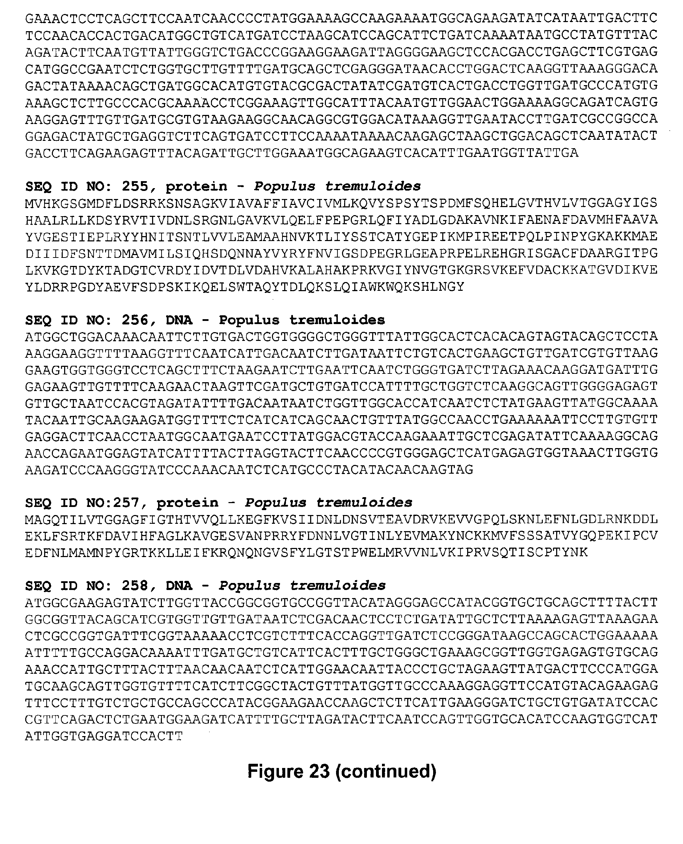

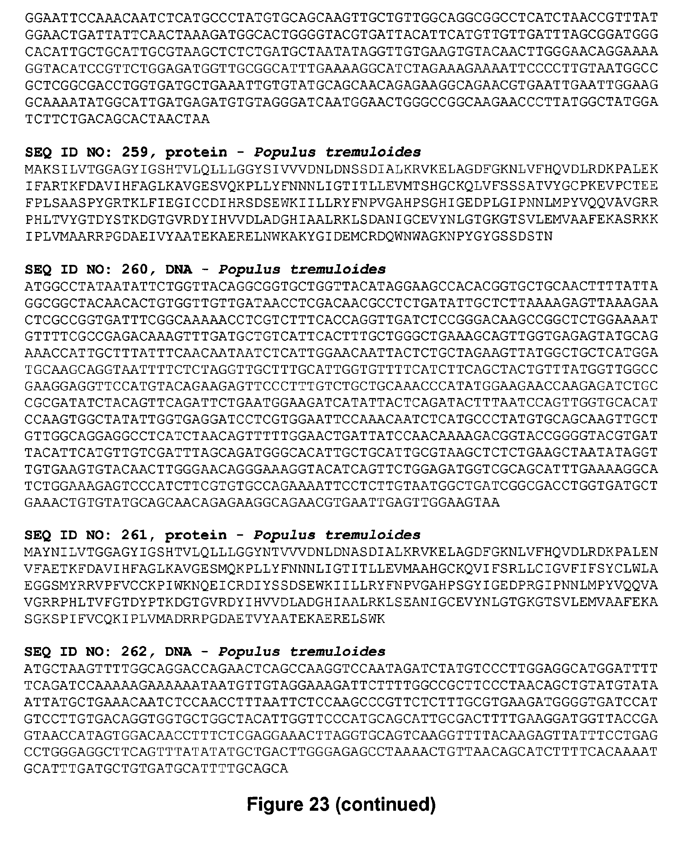

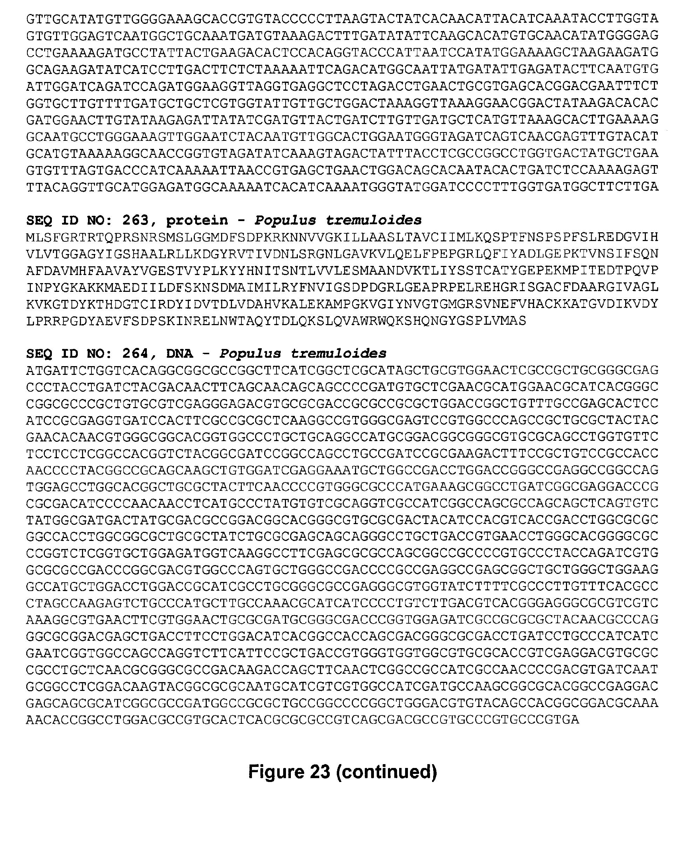

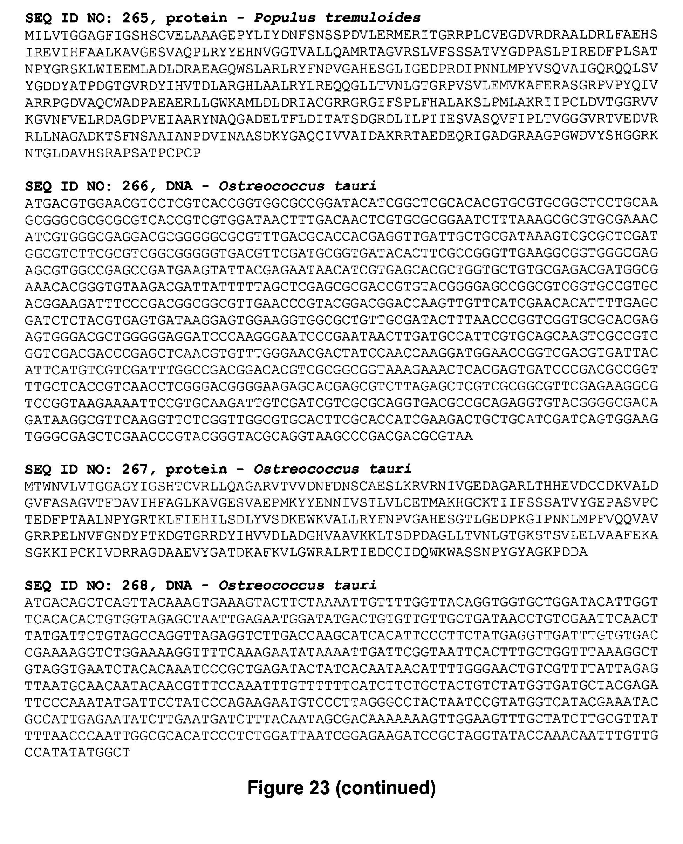

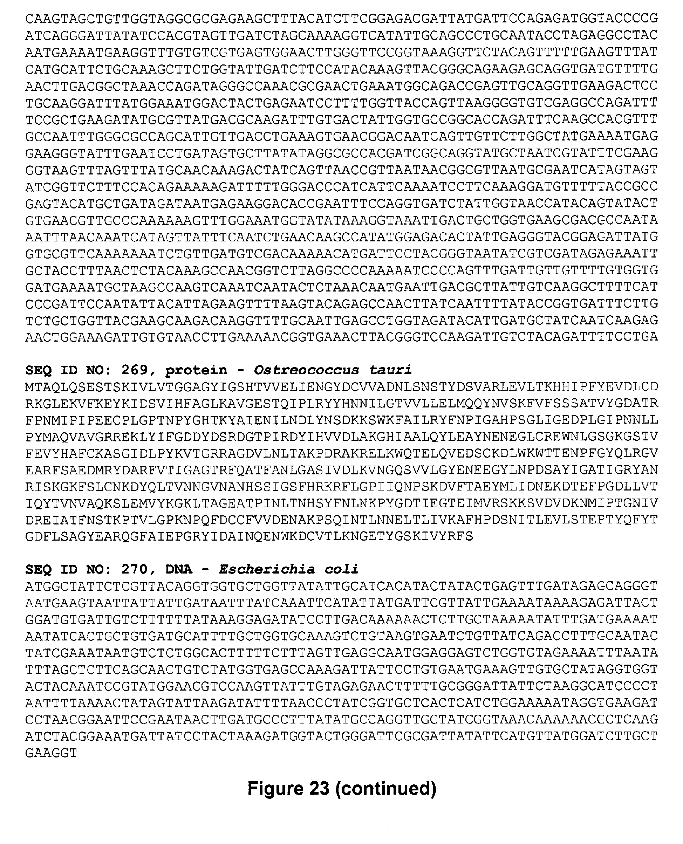

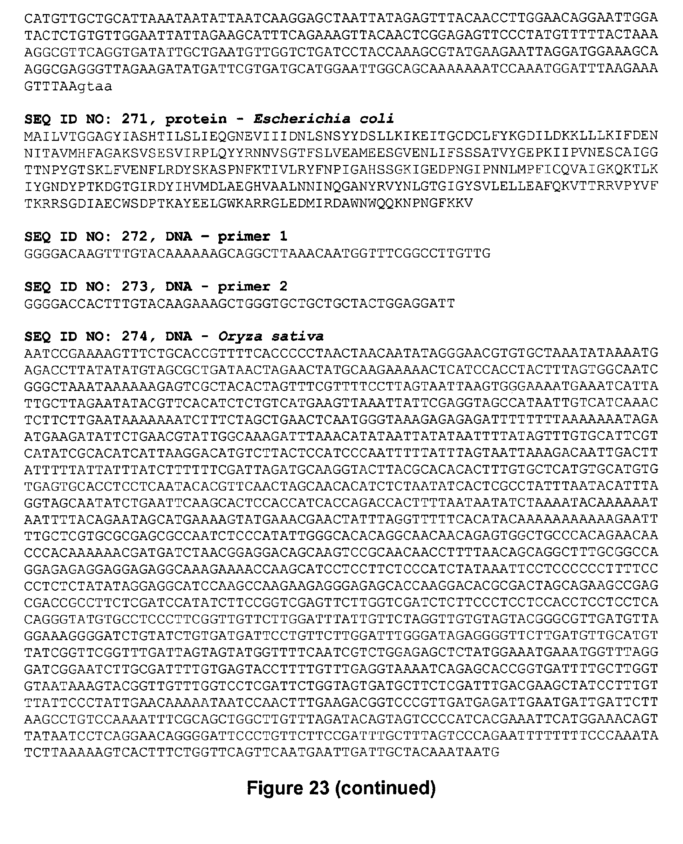

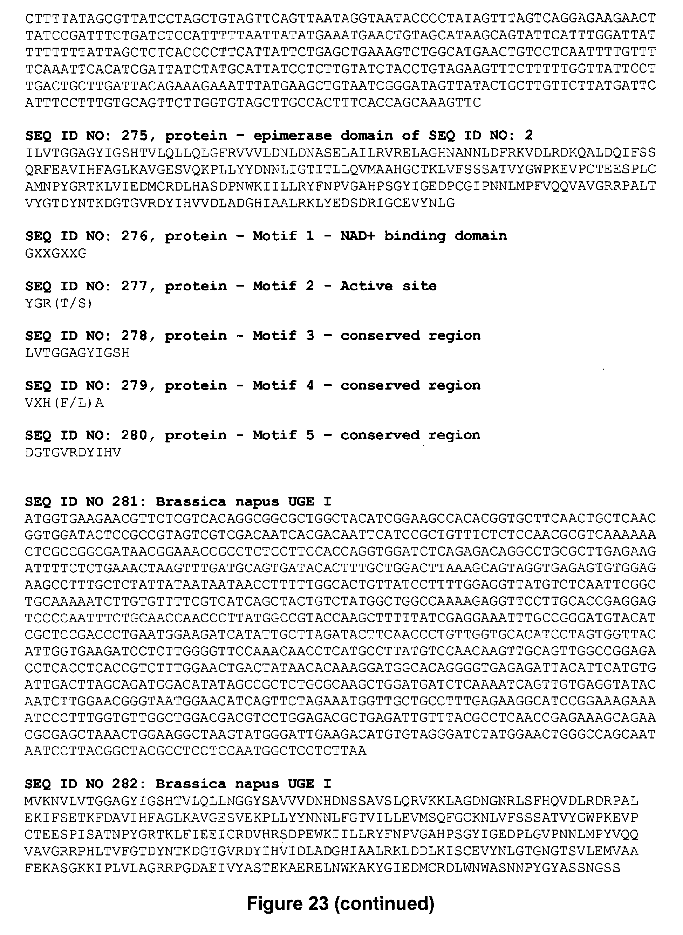

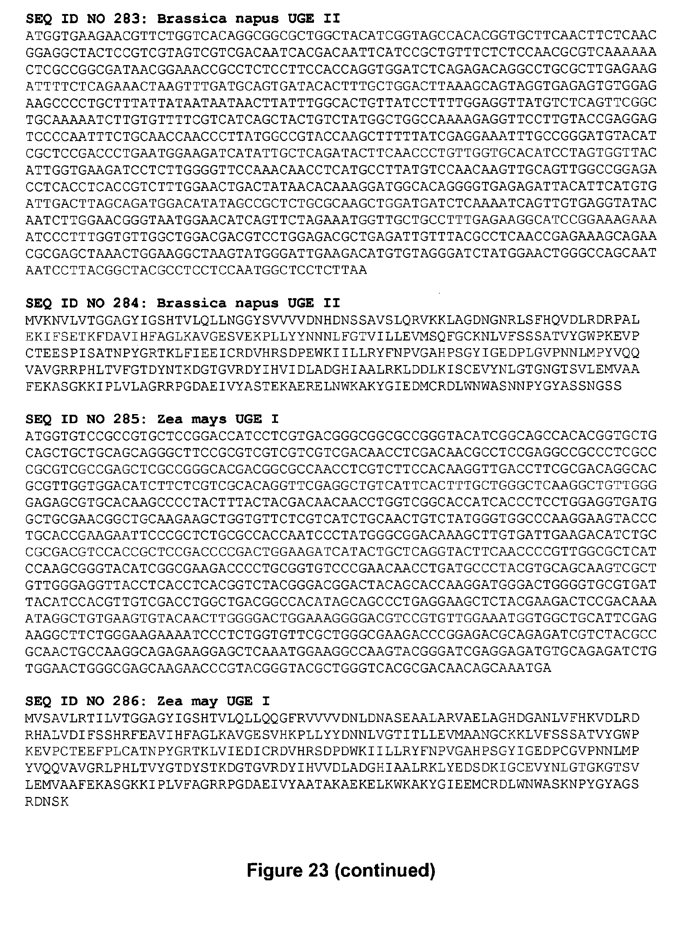

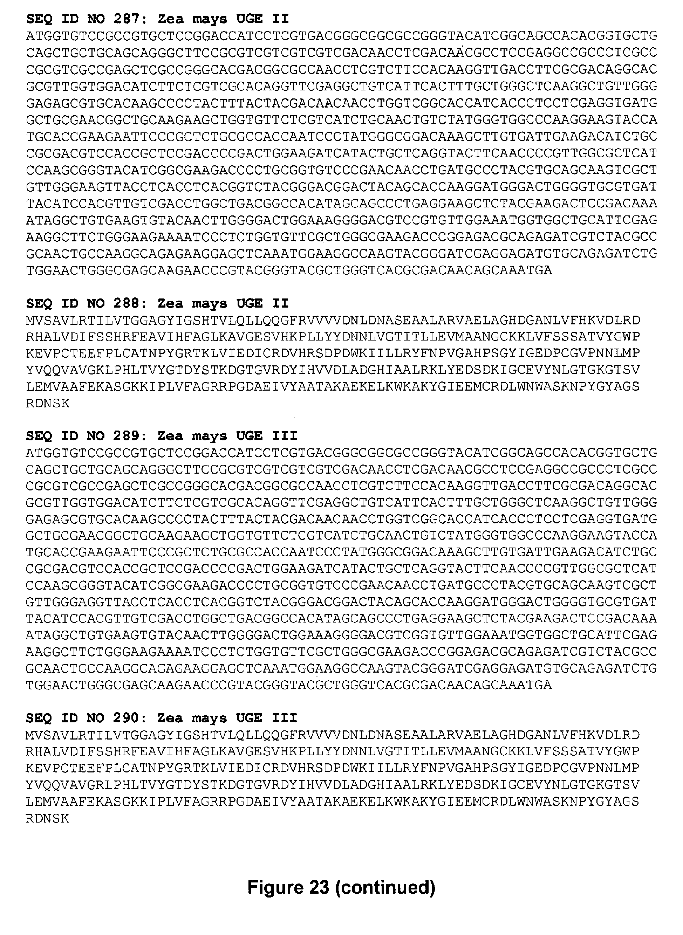

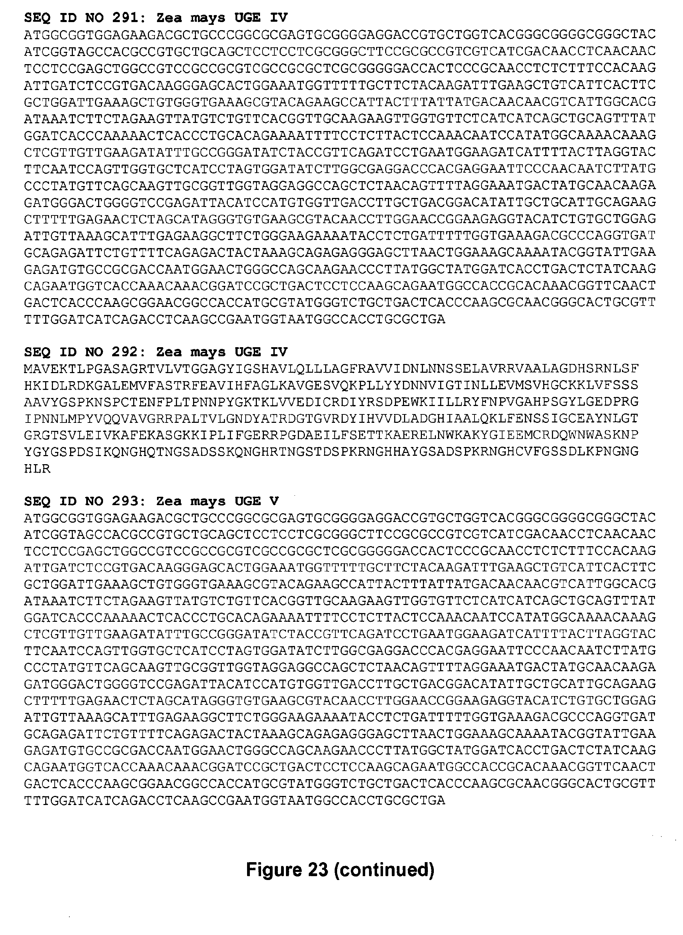

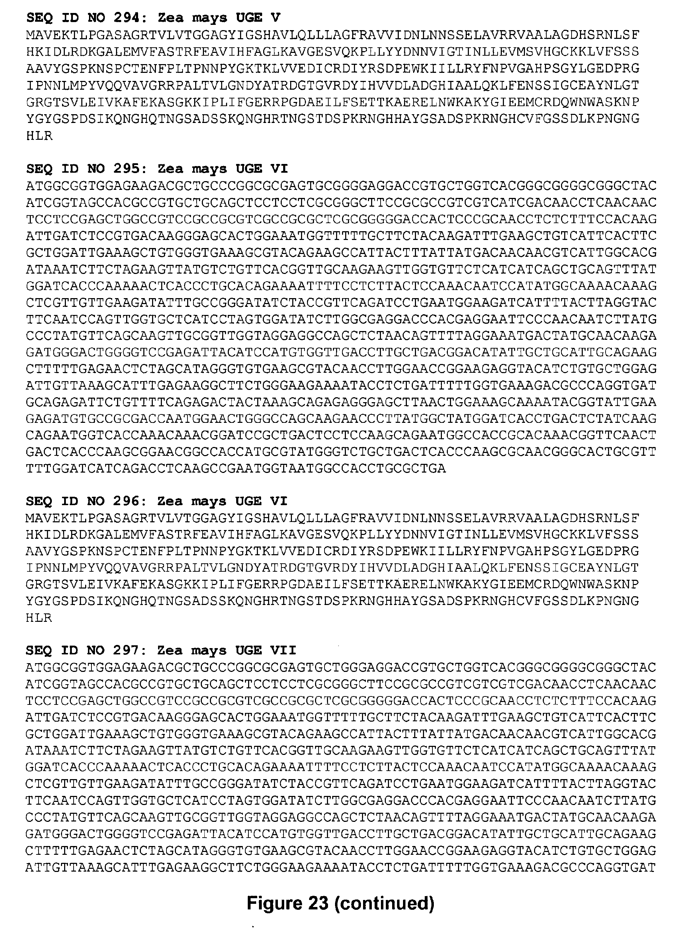

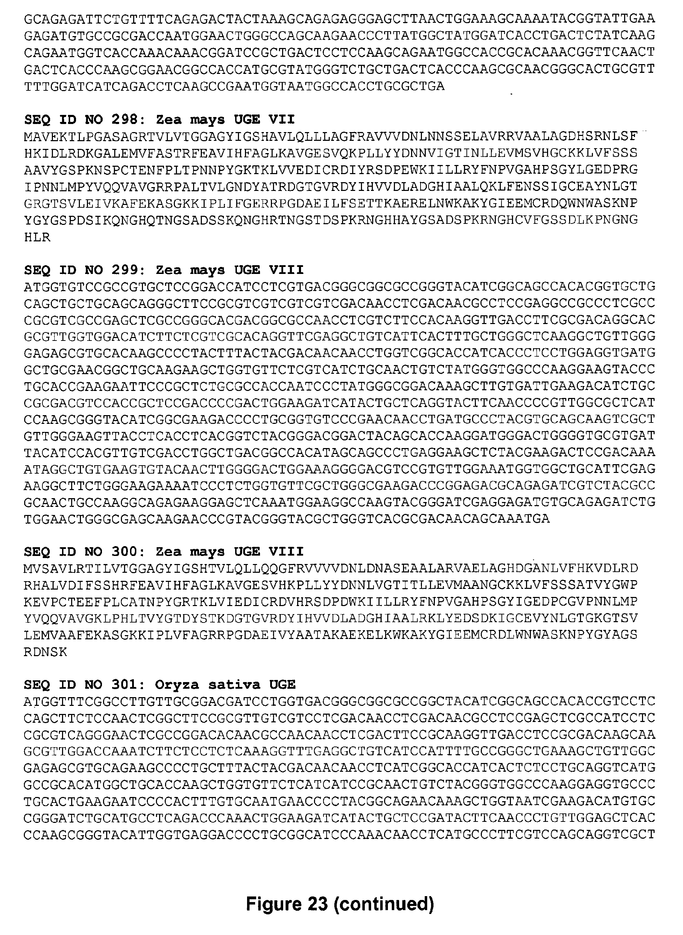

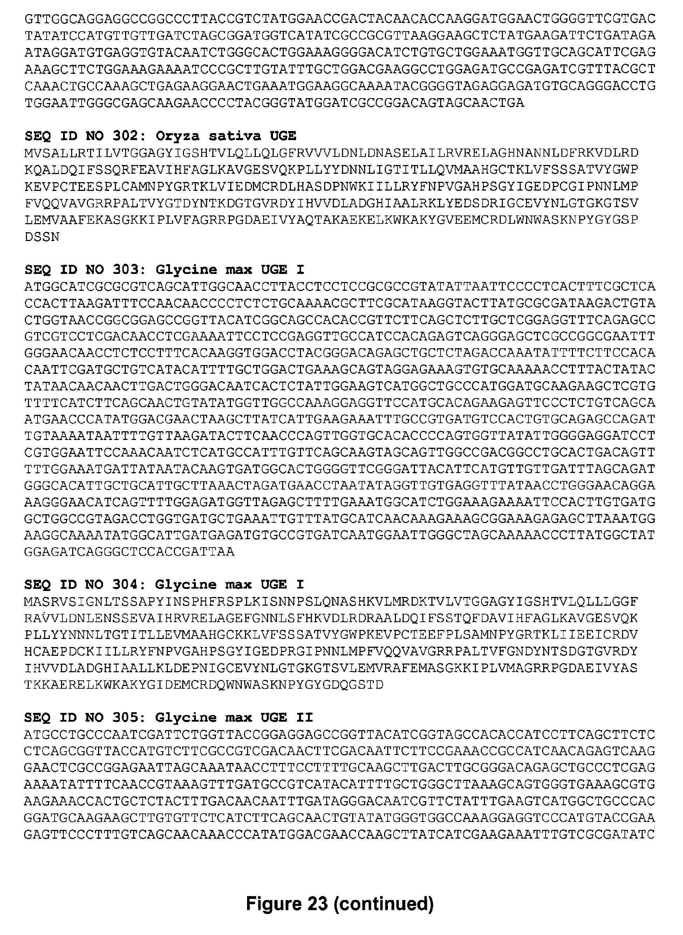

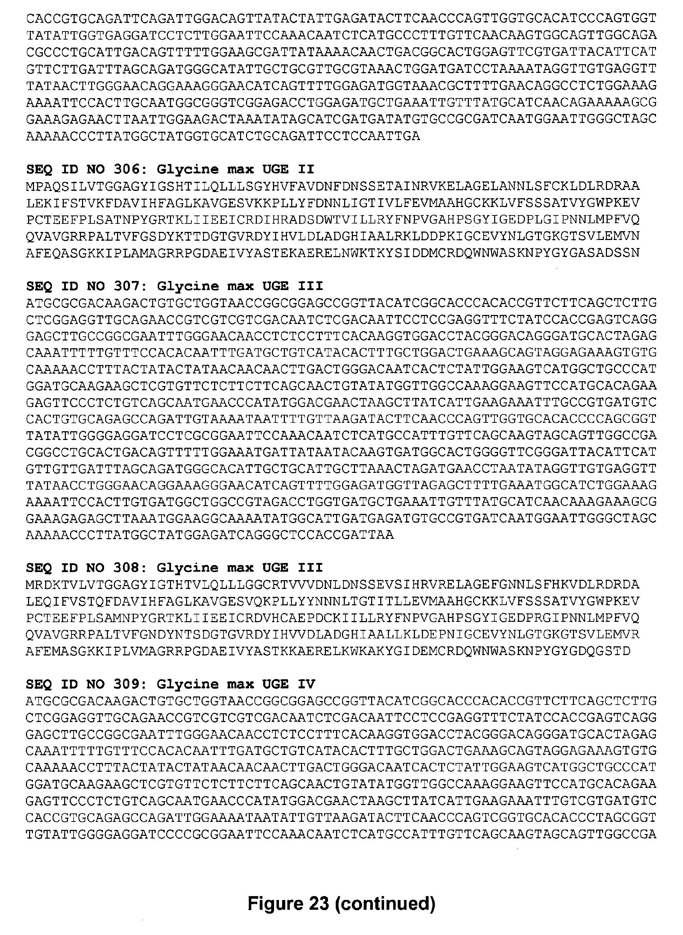

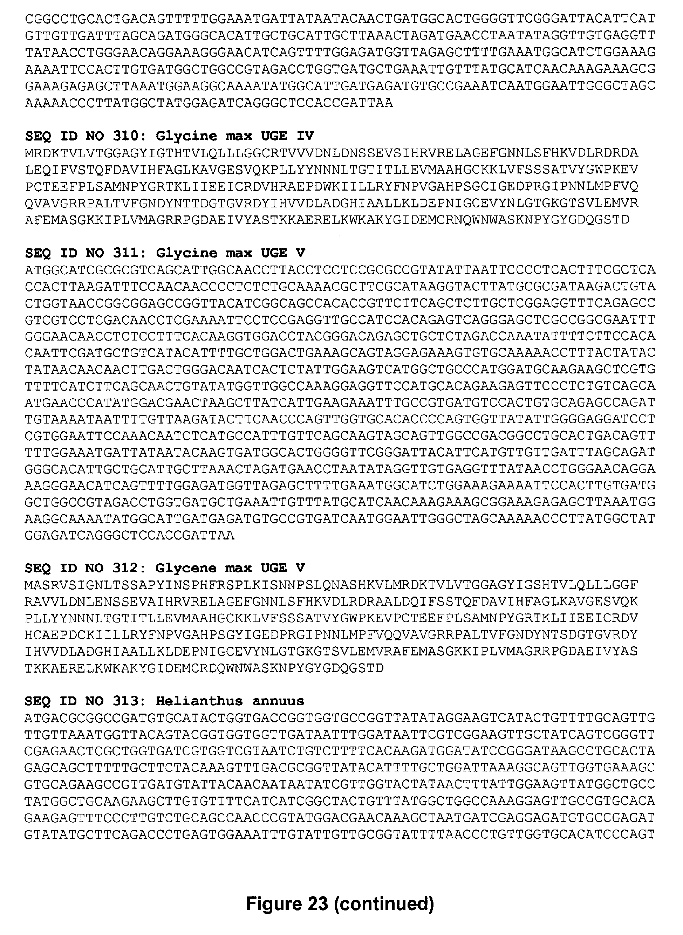

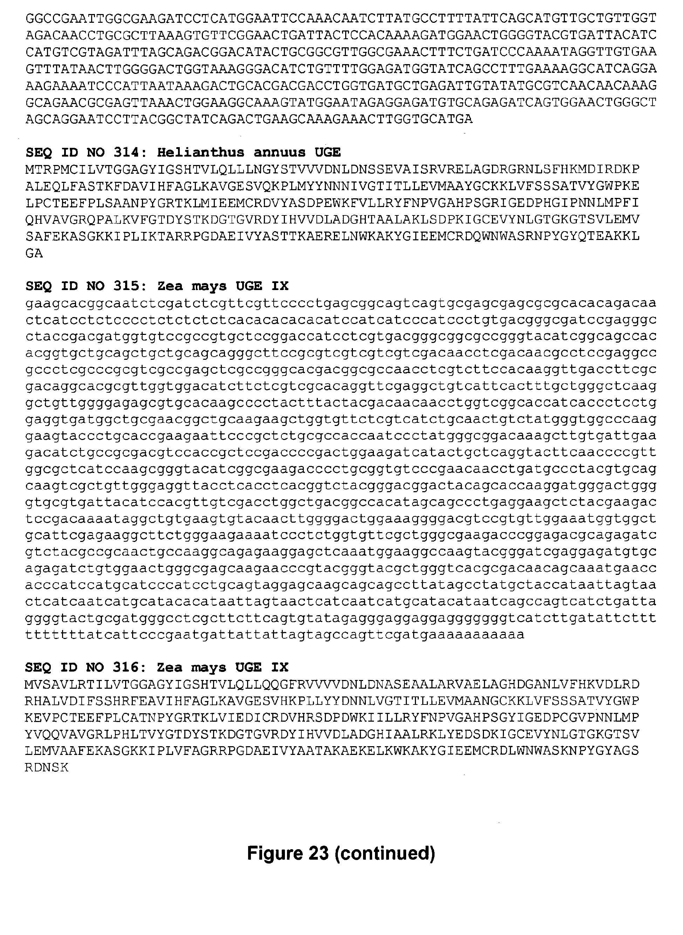

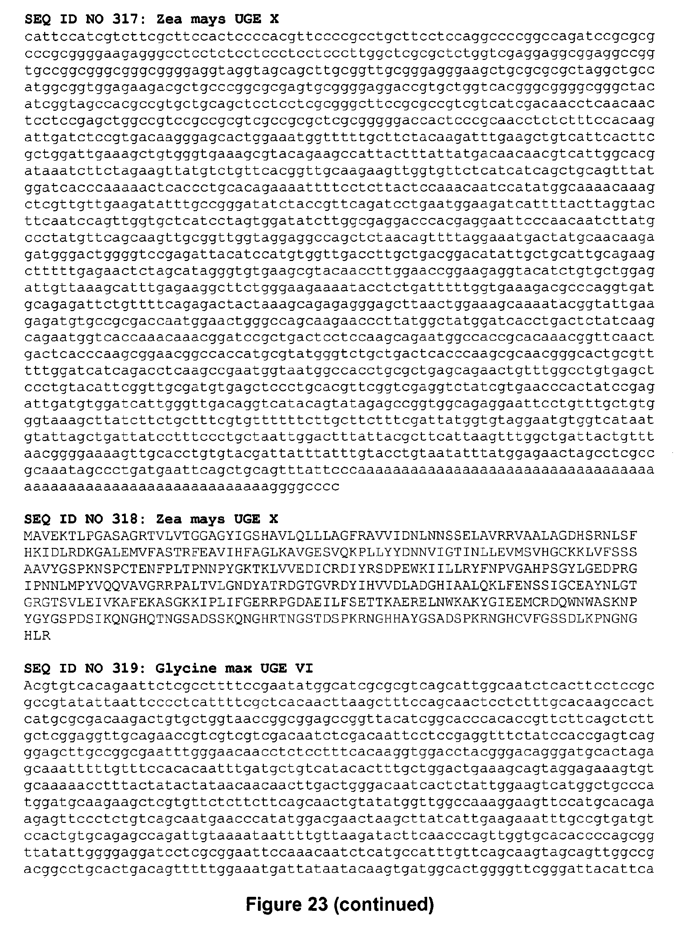

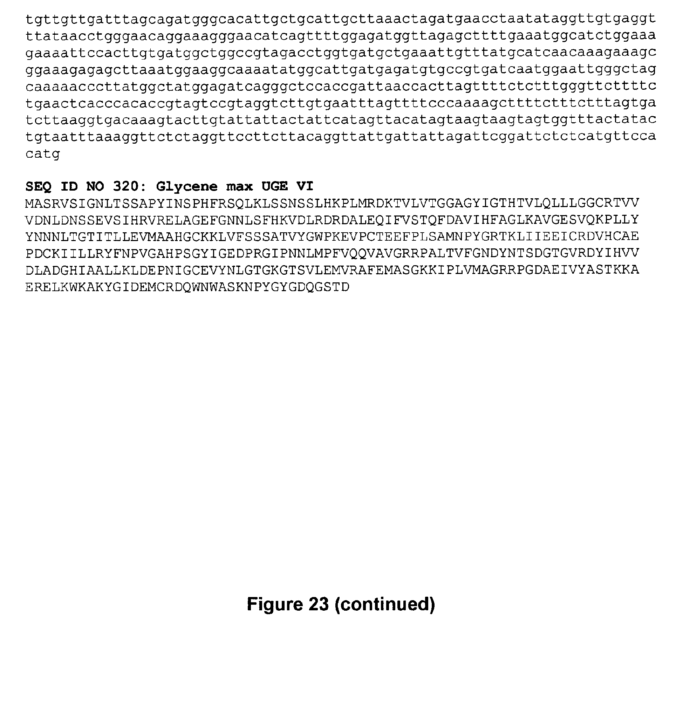

**Fig. 23** details examples of sequences useful in performing the methods according to the present invention.

## Examples

The present invention will now be described with reference to the following examples, which are by way of illustration alone. The following examples are not intended to completely define or otherwise limit the scope of the invention.

DNA manipulation: unless otherwise stated, recombinant DNA techniques are performed according to standard protocols described in (Sambrook (2001) Molecular Cloning: a laboratory manual, 3rd Edition Cold Spring Harbor Laboratory Press, CSH, New York) or in Volumes 1 and 2 of Ausubel et al. (1994), Current Protocols in Molecular Biology, Current Protocols. Standard materials and methods for plant molecular work are described in Plant Molecular Biology Labfax (1993) by R.D.D. Croy, published by BIOS Scientific Publications Ltd (UK) and Blackwell Scientific Publications (UK).

### *Example 1: Identification of sequences related to the nucleic acid sequence used in the methods of the invention*

Sequences (full length cDNA, ESTs or genomic) related to the nucleic acid sequence used in the methods of the present invention were identified amongst those maintained in the Entrez Nucleotides database at the National Center for Biotechnology Information (NCBI) using database sequence search tools, such as the Basic Local Alignment Tool (BLAST) (Altschul et al. (1990) J. Mol. Biol. 215:403-410; and Altschul et al. (1997) Nucleic Acids Res. 25:3389-3402). The program is used to find regions of local similarity between sequences by comparing nucleic acid or polypeptide sequences to sequence databases and by calculating the statistical significance of matches. For example, the polypeptide encoded by the nucleic acid used in the present invention was used for the TBLASTN algorithm, with default settings and the filter to ignore low complexity sequences set off. The output of the analysis was viewed by pairwise comparison, and ranked according to the probability score (E-value), where the score reflect the probability that a particular alignment occurs by chance (the lower the E-value, the more significant the hit). In addition to E-values, comparisons were also scored by percentage identity. Percentage identity refers to the number of identical nucleotides (or amino acids) between the two compared nucleic acid (or polypeptide) sequences over a particular length. In some instances, the default parameters may be adjusted to modify the stringency of the search. For example the E-value may be increased to show less stringent matches. This way, short nearly exact matches may be identified.

Table A provides a list of nucleic acid sequences related to the nucleic acid sequence used in the methods of the present invention.

**Table A1:** Examples of bHLH6-like polypeptides:

| **Plant Source** | **Nucleic acid SEQ ID NO:** | **Protein SEQ ID NO:** |
|---|---|---|
| *Arabidopsis thaliana* | 1 | 2 |
| *Phaseolus vulgaris* | 13 | 14 |
| *Catharanthus roseus* | 15 | 16 |
| *Solanum tuberosum* | 17 | 18 |
| *Pisum sativum* | 19 | 20 |
| *Brassica oleracea* | 21 | 22 |
| *Oryza sativa* | 23 | 24 |
| *Rheum australe* | 25 | 26 |
| *Zea mays* | 27 | 28 |
| *Physcomitrella patens* | 29 | 30 |
| *Lycopersicon esculentum* | 31 | 32 |
| *Populus trichocarpa* | 33 | 34 |
| *Triticum aestivum* | 35 | 36 |
| *Populus sp* | 37 | 38 |
| *Vitis vinifera* | 39 | 40 |
| *Oryza sativa* | | 41 |

Table A2a: Examples of GRP polypeptides, wherein the GRP polypeptides are RrmJ/FtsJ polypeptides:

| **Name** | **Source organism** | **SEQ ID NO nucleic acid sequence** | **SEQ ID NO polypeptide sequence** | **Status** | **Public database accession number** |
|---|---|---|---|---|---|
| Nicta_RrmJ/ FtsJ partial | *Nicotiana tabacum* | 57 | 58 | Partial | |
| Nicta_RrmJ/ FtsJ | *Nicotiana tabacum* | 59 | 60 | Full length | EB446219, EB448450.1 |
| Arath_RrmJ/ FtsJ | *Arabidopsis thaliana* | 61 | 62 | Full length | AY087952.1 |
| Lyces_RrmJ/ FtsJ | *Lycopersicon esculentum* | 63 | 64 | Full length | BT013964 |
| Medtr_RrmJ/ FtsJ | *Medicago truncatula* | 65 | 66 | Full length | AC149135.2 |
| Orysa_RrmJ/ FtsJ | *Oryza sativa* | 67 | 68 | Full length | AK103054 |

(continued)

| Name | Source organism | SEQ ID NO nucleic acid sequence | SEQ ID NO polypeptide sequence | Status | Public database accession number |
|---|---|---|---|---|---|
| Osta_RrmJ/F tsJ | *Ostreococcus lucimarinus CCE9901* | 69 | 70 | Full length | XM_001418019 |
| Poptr_RrmJ/ FtsJ | *Populus tremuloides* | 71 | 72 | Full length | contig of CX183550.1 DT504907.1 |
| Sacof_RrmJ/ FtsJ | *Saccharum officinarum* | 73 | 74 | Full length | contig of CA279558.1 CF576902.1 |
| Triae_RrmJ/ FtsJ | *Triticum aestivum* | 75 | 76 | Full length | DR740715 |
| Apime_RrmJ /FtsJ | *Apis mellifera* | 77 | 78 | Full length | XM_392223 |
| Caeel_RrmJ/ FtsJ | *Caenorhabditis elegans* | 79 | 80 | Full length | NM_065442 |
| Danre_RrmJ/ FtsJ | *Danio rerio* | 81 | 82 | Full length | BC085449 |
| Homsa_Rrm J/ FtsJ | *Homo sapiens* | 83 | 84 | Full length | AK024023 |
| Horvu_FtsJ | *Hordeum vulgare* | 92 | 93 | Full length | |
| Sorbi_FtsJ | *Sorghum bicolor* | 94 | 95 | Full length | |

Table A2b: Examples of GRP polypeptides, wherein the GRP polypeptides are RrmJ/FtsJ polypeptides:

| Name | Source organism | SEQ ID NO nucleic acid sequence | SEQ ID NO polypeptide sequence | Status |
|---|---|---|---|---|
| Brana_FtsJ | *Brassica napus* | 96 | 97 | Full length |
| Glyma_FtsJ | *Glycine max* | 98 | 99 | Full length |
| Zeama FtsJ | *Zea mays* | 100 | 101 | Full length |
| Tager_FtsJ_ partial | *Tagetes erecta* | | | Partial |

**Table A3a:** Examples of GRP polypeptides, wherein the GRP polypeptides are bHLH4 polypeptides:

| Name | Source organism | SEQ ID NO nucleic acid sequence | SEQ ID NO polypeptide sequence | Public database accession number |
|---|---|---|---|---|
| Arath_ bHLH4 | *Arabidopsis thaliana* | 104 | 105 | |
| Arath_bHLH 4 II | *Arabidopsis thaliana* | 109 | 110 | |
| Brana_bHLH 4 II | *Brassica napus* | 111 | 112 | |
| Lotja_ bHLH4 | *Lotus japonicus* | 113 | 114 | |
| Lyces-bHLH 4 | *Lycopersicon esculentum* | 115 | 116 | |

(continued)

| Name | Source organism | SEQ ID NO nucleic acid sequence | SEQ ID NO polypeptide sequence | Public database accession number |
|---|---|---|---|---|
| Vitvi_bHLH4 | *Vitis vinifera* | 117 | 118 | contig like VV78X118066.5 AM475703.2 |
| Zeama_bHL H4 | *Zea mays* | 119 | 120 | full length nucleic acid sequence contig of AF061107 and EE190449 |

**Table A3b:** Examples of GRP polypeptides, wherein the GRP polypeptides are bHLH4 polypeptides:

| Name | Source organism | SEQ ID NO nucleic acid sequence | SEQ ID NO polypeptide sequence | Public database accession number |
|---|---|---|---|---|
| Brana_bHLH 4 | *Brassica napus* | 121 | 122 | |
| Glyma_ bHLH4 | *Glycine max* | 123 | 124 | |
| Glyma_ bHLH4 II | *Glycine max* | 125 | 126 | |
| Horvu_ bHLH4 | *Hordeum vulgare* | 127 | 128 | |
| Zeama_bHLH4 II | *Zea mays* | 129 | 130 | |

**Table A4:** Examples of GRP polypeptides, wherein the GRP polypeptides are IPT polypeptides:

| Name | Source organism | SEQ ID NO nucleic acid sequence | SEQ ID NO polypeptide sequence |
|---|---|---|---|
| Arath_IPT | *Arabidopsis thaliana* | 131 | 132 |
| Arath_IPT4 | *Arabidopsis thaliana* | 136 | 137 |
| Arath_IPT6 | *Arabidopsis thaliana* | 138 | 139 |
| Humlu_IPT like | *Humulus lupunus* | 140 | 141 |
| Lotja_IPT like | *Lotus japonica* | 142 | 143 |
| Medtr_IPT like | *Medicago truncatula* | 144 | 145 |
| Moral_IPT like | *Morus alba* | 146 | 147 |
| Orysa_IPT like | *Oryza sativa* | 148 | 149 |
| Pethy_IPT like | *Petunia hybrida* | 150 | 151 |
| Poptr_IPT like | *Populus tremuloides* | 152 | 153 |
| Poptr_IPT like II | *Populus tremuloides* | 154 | 155 |
| Poptr_IPT like III | *Populus tremuloides* | 156 | 157 |
| Poptr IPT like IV | *Populus tremuloides* | 158 | 159 |
| Poptr_IPT like V | *Populus tremuloides* | 160 | 161 |
| Poptr_IPT like VI | *Populus tremuloides* | 162 | 163 |
| Vitvi IPT like | *Vitis vinifera* | 164 | 165 |
| Zeama_IPT like | *Zea mays* | 166 | 167 |

**Table A5a:** Examples of STO nucleic acids a and polypeptides:

| Name | Plant Source | Nucleic acid SEQ ID NO: | Protein SEQ ID NO: |
|---|---|---|---|
| Os04g0540200 cds3887 | *Oryza sativa* | 168 | 169 |
| Os01g0202500 | *Oryza sativa* | 170 | 171 |
| Os02g0606200 | *Oryza sativa* | 172 | 173 |
| Os02g0646200 | *Oryza sativa* | 174 | 175 |
| Os04g049300 | *Oryza sativa* | 176 | 177 |
| Os05g0204600 | *Oryza sativa* | 178 | 179 |
| Os06g0152200 | *Oryza sativa* | 180 | 181 |
| Os06g071300 | *Oryza sativa* | 182 | 183 |
| Os09g0527900 | *Oryza sativa* | 184 | 185 |
| Os12g0209200 | *Oryza sativa* | 186 | 187 |
| SO_STO | *Saccharum officinarum* | 188 | 189 |
| AT1 G06040_spliced variant1 | *Arabidopsis thaliana* | 190 | 191 |
| AT1G06040_spliced variant2 | *Arabidopsis thaliana* | 192 | 193 |
| AT1G75540 | *Arabidopsis thaliana* | 194 | 195 |
| AT1G78600 | *Arabidopsis thaliana* | 196 | 197 |
| AT2G31380 | *Arabidopsis thaliana* | 198 | 199 |
| AT4G10240 | *Arabidopsis thaliana* | 200 | 201 |
| AT4G39070 | *Arabidopsis thaliana* | 202 | 203 |
| Mt_ABO84497 | *Medicago truncatula* | 204 | 205 |
| LE_gi\|45544865 | *Lycopersicul esculentum* | 206 | 207 |
| ST_gi\|76160970 | *Solanum tuberosum* | 208 | 209 |
| PP_STO LIKE | *populus trichoparcca* | 210 | 211 |
| VV_CAN72879 | *Vitis vinifera* | 212 | 213 |
| GM_gi178173056 | *Glycine max* | 214 | 215 |

**Table A5b:** Examples of STO nucleic acids and polypeptides:

| Name | Plant Source | Nucleic acid SEQ ID NO: | Protein SEQ ID NO: |
|---|---|---|---|
| Zeama_STO | *Zea mays* | 222 | 223 |

**Table A6:** Examples of UGE nucleic acids and polypeptides:

| Name | Source organism | Nucleic acid SEQ ID NO: | Protein SEQ ID NO: |
|---|---|---|---|
| OS_UGE2 | *Oryza sativa* | 224 | 225 |
| Os04g0618200 | *Oryza sativa* | 226 | 227 |
| Os05g0595100 | *Oryza sativa* | 228 | 229 |
| Os07g0139400 | *Oryza sativa* | 230 | 231 |
| Os08g0374800 | *Oryza sativa* | 232 | 233 |

(continued)

| Name | Source organism | Nucleic acid SEQ ID NO: | Protein SEQ ID NO: |
|---|---|---|---|
| Os09g0323000 | *Oryza sativa* | 234 | 235 |
| Os09g0504000 | *Oryza sativa* | 236 | 237 |
| Os09g0526700 | *Oryza sativa* | 238 | 239 |
| AT1G12780_UGE1 | *Arabidopsis thaliana* | 240 | 241 |
| AT1G63180_UGE3 | *Arabidopsis thaliana* | 242 | 243 |
| AT1G64440_UGE4 | *Arabidopsis thaliana* | 244 | 245 |
| AT4G10960_UGE5 | *Arabidopsis thaliana* | 246 | 247 |
| AT4G23920_UGE2 | *Arabidopsis thaliana* | 248 | 249 |
| Pt_lcl\|scaff_XVI.140 | *Populus trichocarpa* | 250 | 251 |
| Pt_lcl\|scaff_XI.1329 | *Populus trichocarpa* | 252 | 253 |
| Pt_lcl\|scaff_VI.203 | *Populus trichocarpa* | 254 | 255 |
| Pt_lcl\|scaff_III.961 | *Populus trichocarpa* | 256 | 257 |
| Pt_lcl\|scaff_III.1133 | *Populus trichocarpa* | 258 | 259 |
| Pt_lcl\|scaff_I.773 | *Populus trichocarpa* | 260 | 261 |
| Pt_lcl\|scaff_I.2996 | *Populus trichocarpa* | 262 | 263 |
| Pt_lcl\|scaff_5422 | *Populus trichocarpa* | 264 | 265 |
| Ot08g015500 | *Ostreococcus tauri* | 266 | 267 |
| SC_GAL10_P04397 | *Ostreococcus tauri* | 268 | 269 |
| Ec_GALE_AAO37702 | *Escherichia coli* | 270 | 271 |

**Table A6b:** Examples of UGE nucleic acids and polypeptides:

| Name | Source organism | Nucleic acid SEQ ID NO: | Protein SEQ ID NO: |
|---|---|---|---|
| Brana_UGE I | *Brassica napus* | 281 | 282 |
| Brana_UGE II | *Brassica napus* | 283 | 284 |
| Zeama_UGE I | *Zea mays* | 285 | 286 |
| Zeama_UGE II | *Zea mays* | 287 | 288 |
| Zeama_UGE III | *Zea mays* | 289 | 290 |
| Zeama_UGE IV | *Zea mays* | 291 | 292 |
| zeama_UGE V | *Zea mays* | 293 | 294 |
| Zeama_UGE VI | *Zea mays* | 295 | 296 |
| Zeama_UGE VII | *Zea mays* | 297 | 298 |
| Zeama_UGE VIII | *Zea mays* | 299 | 300 |
| Orysa_UGE | *Oryza sativa* | 301 | 302 |
| Glyma_UGE I | *Glycine max* | 303 | 304 |
| Glyma_UGE II | *Glycine max* | 305 | 306 |
| Glyma_UGE III | *Glycine max* | 307 | 308 |
| Glyma_UGE IV | *Glycine max* | 309 | 310 |
| Glyma_UGE V | *Glycine max* | 311 | 312 |

(continued)

| Name | Source organism | Nucleic acid SEQ ID NO: | Protein SEQ ID NO: |
|---|---|---|---|
| Helan_UGE | *Helianthus annuus* | 313 | 314 |
| Zeama_UGE IX | *Zea mays* | 315 | 316 |
| Zeama_UGE X | *Zea mays* | 317 | 318 |
| Glyma_UGE VI | *Glycine max* | 319 | 320 |

In addition, in some instances, related sequences have tentatively been assembled and publicly disclosed by research institutions, such as The Institute for Genomic Research (TIGR). The Eukaryotic Gene Orthologs (EGO) database may be used to identify such related sequences, either by keyword search or by using the BLAST algorithm with the nucleic acid or polypeptide sequence of interest. Some of the listed sequences are partial; preferably full length sequences or functionally equivalent fragments are used in the methods of the present invention. Where only partial sequences are available from the databases, standard cloning techniques can be used by the skilled in the art for obtaining a full length gene sequence.

***Example 2: Alignment of polypeptide sequences of the invention***

Alignment of polypeptide sequences was performed using the AlignX programme from the Vector NTI (Invitrogen) which is based on the popular Clustal W algorithm of progressive alignment (Thompson et al. (1997) Nucleic Acids Res 25:4876-4882; Chenna et al. (2003). Nucleic Acids Res 31:3497-3500). Default values were for the gap open penalty of 10, for the gap extension penalty of 0,1 and the selected weight matrix was Blosum 62 (if polypeptides are aligned). Minor manual editing was done to further optimise the alignment.

***Alignment of bHLH6-like polypeptide sequences***

Sequence conservation among bHLH6-like polypeptides is essentially in the N-terminal domain and the C-terminal bHLH domain of the polypeptides, the central part usually being more variable in sequence length and composition. The bHLH6-like polypeptides are aligned in Figure 2.

***Alignment of RrmJ/FtsJ polypeptide sequences***

A CLUSTAL W (1 ;83) multiple sequence alignment of RrmJ/FtsJ polypeptides from Table A2 is shown in Figure 5.

A consensus sequence of bHLH4 polypeptides comprising highly conserved representative amino acids at each position is given; blanks in between given amino acids in the consensus sequence represent any amino acid.

The bHLH4 polypeptides are aligned in Figure 10.

Sequence conservation among bHLH4 polypeptides was found essentially along the bHLH domain. The consensus sequence represents highly conserved amino acid residues in bHLH4 polypetides; blanks in the consensus sequence represent highly variable regions.

*Alignment of IPT polypeptide sequences*

A consensus sequence of IPT polypeptides comprising highly conserved representative amino acids at each position is given; blanks in between given amino acids in the consensus sequence represent any amino acid.

The IPT polypeptides are aligned in Figure 13.

The consensus sequence represents highly conserved amino acid residues in IPT polypetides; blanks in the consensus sequence represent highly variable regions.

A phylogenetic tree of GRP polypeptides is constructed using a neighbour-joining clustering algorithm as provided in the AlignX programme from Vector NTI (Invitrogen).

Sequence conservation among STO polypeptides is essentially in the N-terminal region comprising the two B-box domains. The two B-box domains are separated by a linker region of variable length, typically between 2 and 20 amino acids.

A phylogenetic tree of STO polypeptides (Figure 16) was constructed using a neighbour-joining clustering algorithm as provided in the AlignX programme from the Vector NTI (Invitrogen).

A consensus sequence of UGE polypeptides comprising highly conserved representative amino acids at each position is given; blanks in between given amino acids in the consensus sequence represent any amino acid.

The UGE polypeptides are aligned in Figure 20.

Sequence conservation among UGE polypeptides is higher in the region located at the N-terminal of the epimerase domain. Regions wherein in the consensus sequence there are defined amino acids given represent the regions with high similarity; blanks in the consensus sequence represent highly variable regions.

A phylogenetic tree of UGE polypeptides (Figure 21) was constructed using a neighbour-joining clustering algorithm as provided in the AlignX programme from the Vector NTI (Invitrogen).

***Example 3: Calculation of global percentage identity between polypeptide sequences useful in performing the methods of the invention***

Global percentages of similarity and identity between full length polypeptide sequences useful in performing the methods of the invention were determined using one of the methods available in the art, the MatGAT (Matrix Global Alignment Tool) software (BMC Bioinformatics. 2003 4:29. MatGAT: an application that generates similarity/identity matrices using protein or DNA sequences. Campanella JJ, Bitincka L, Smalley J; software hosted by Ledion Bitincka). MatGAT software generates similarity/identity matrices for DNA or protein sequences without needing pre-alignment of the data. The program performs a series of pair-wise alignments using the Myers and Miller global alignment algorithm (with a gap opening penalty of 12, and a gap extension penalty of 2), calculates similarity and identity using for example Blosum 62 (for polypeptides), and then places the results in a distance matrix. Sequence similarity is shown in the bottom half of the dividing line and sequence identity is shown in the top half of the diagonal dividing line.

Parameters used in the comparison were:

| | |
|---|---|
| Scoring matrix: | Blosum62 |
| First Gap: | 12 |
| Extending gap: | 2 |

Results of the software analysis are shown in Table B1 for the global similarity and identity over the full length of the polypeptide sequences. Percentage identity is given above the diagonal in bold and percentage similarity is given below the diagonal (normal face).

The percentage identity between the bHLH6-like polypeptide sequences useful in performing the methods of the invention can be as low as 30 % amino acid identity compared to SEQ ID NO: 2 (AAL55713). However, when the alignment is made over the bHLH domain, the sequence identity becomes much higher among the different bHLH6-like proteins. In Table B2, values for the global similarity and identity between sequences that align with SEQ ID NO: 9, (comprising the bHLH domains of bHLH6-like polypeptide sequences) are listed. The sequence identity is about 80% or higher. The used peptide sequences are: SEQ ID NO: 42, NP_001065478; SEQ ID NO: 43, EAY79619; SEQ ID NO: 44, AAD15818; SEQ ID NO: 45, AAB00686; SEQ ID NO: 46, ABD59338; SEQ ID NO: 47, Pt29.195#1; SEQ ID NO: 48, CAF74710; SEQ ID NO: 49, AAF04917; SEQ ID NO: 50, AAQ14332; SEQ ID NO: 51, AAY90122; SEQ ID NO: 52, AAL55713; SEQ ID NO: 53, ABD65632; SEQ ID NO: 54, ABK94979; SEQ ID NO: 55, EDQ81347.

**Table B1:** MatGAT results for global similarity and identity over the full length of the polypeptide sequences.

| | 1 | 2 | 3 | 4 | 5 | 6 | 7 | 8 | 9 | 10 | 11 | 12 | 13 | 14 |
|---|---|---|---|---|---|---|---|---|---|---|---|---|---|---|
| 1. AAB00686 | | 66.5 | 59.1 | 56.6 | 58.3 | 28.2 | 49.1 | 48.4 | 48.0 | 43.7 | 47.8 | 31.9 | 30.5 | 62.6 |
| 2. ABD59338 | 79.1 | | 54.1 | 50.1 | 55.1 | 27.9 | 46.6 | 43.8 | 45.8 | 41.7 | 45.5 | 29.5 | 31.7 | 57.4 |
| 3.AAQ14332 | 71.1 | 67.7 | | 53.5 | 64.0 | 28.8 | 52.1 | 45.4 | 47.8 | 43.6 | 46.2 | 29.3 | 30.6 | 60.5 |
| 4. AAL55713 | 70.7 | 68.3 | 67.2 | | 52.0 | 24.7 | 46.3 | 47.6 | 47.2 | 42.6 | 45.8 | 33.6 | 33.2 | 55.4 |
| 5. CAF74710 | 70.2 | 68.8 | 78.4 | 66.8 | | 37.3 | 52.9 | 46.4 | 49.7 | 45.5 | 49.2 | 30.0 | 30.0 | 60.5 |
| 6. AAF04917 | 34.9 | 34.4 | 35.1 | 32.4 | 40.9 | | 26.0 | 27.8 | 23.3 | 24.4 | 24.2 | 23.2 | 20.7 | 29.5 |
| 7. AAY90122 | 63.6 | 62.1 | 68.1 | 59.6 | 67.6 | 30.7 | | 41.1 | 45.1 | 42.4 | 44.2 | 27.0 | 29.8 | 49.7 |
| 8. ABD65632 | 66.8 | 61.8 | 60.2 | 66.8 | 59.1 | 34.3 | 54.2 | | 39.3 | 36.8 | 40.0 | 33.5 | 32.2 | 49.2 |
| 9. NP001065478 | 63.8 | 63.5 | 64.8 | 60.8 | 65.5 | 30.9 | 61.4 | 53.5 | | 94.7 | 79.2 | 28.7 | 29.9 | 48.3 |
| 10. EAY79619 | 62.0 | 61.2 | 59.8 | 58.7 | 61.4 | 32.3 | 57.9 | 52.6 | 94.8 | | 75.3 | 27.9 | 27.9 | 43.2 |
| 11. AAD15818 | 62.3 | 62.4 | 63.4 | 59.0 | 65.7 | 31.1 | 62.1 | 54.1 | 87.6 | 83.3 | | 28.5 | 31.1 | 47.0 |
| 12. ABK94979 | 48.6 | 45.4 | 45.2 | 52.6 | 44.9 | 35.8 | 42.6 | 52.2 | 42.9 | 42.8 | 42.7 | | 27.3 | 30.4 |
| 13. EDQ81347 | 50.3 | 49.4 | 47.1 | 50.6 | 48.7 | 31.4 | 46.7 | 53.2 | 45.9 | 44.6 | 45.6 | 48.1 | | 32.2 |
| 14. Pt29.195#1 | 76.8 | 72.6 | 74.5 | 71.6 | 73.4 | 36.9 | 64.0 | 66.2 | 63.7 | 61.5 | 61.8 | 47.9 | 48.8 | |

**Table B2:** MatGAT results for global similarity and identity over the peptide sequences corresponding to Motif 7.

| | 1 | 2 | 3 | 4 | 5 | 6 | 7 | 8 | 9 | 10 | 11 | 12 | 13 | 14 |
|---|---|---|---|---|---|---|---|---|---|---|---|---|---|---|
| 1. AAL55713 | | 88.6 | 88.6 | 88.6 | 90.0 | 90.0 | 87.1 | 92.9 | 90.0 | 91.4 | 90.0 | 90.0 | 81.4 | 82.9 |
| 2. NP_001065478 | 94.3 | | 100.0 | 98.6 | 92.9 | 94.3 | 92.9 | 92.9 | 90.0 | 92.9 | 91.4 | 91.4 | 80.0 | 84.3 |
| 3. EAY79619 | 94.3 | 100.0 | | 98.6 | 92.9 | 94.3 | 92.9 | 92.9 | 90.0 | 92.9 | 91.4 | 91.4 | 80.0 | 84.3 |
| 4. AAD15818 | 94.3 | 100.0 | 100.0 | | 92.9 | 94.3 | 94.3 | 92.9 | 90.0 | 92.9 | 91.4 | 91.4 | 80.0 | 84.3 |
| 5. AAB00686 | 95.7 | 95.7 | 95.7 | 95.7 | | 94.3 | 90.0 | 94.3 | 91.4 | 92.9 | 92.9 | 91.4 | 84.3 | 82.9 |
| 6. ABD59338 | 94.3 | 95.7 | 95.7 | 95.7 | 94.3 | | 92.9 | 95.7 | 92.9 | 95.7 | 94.3 | 94.3 | 81.4 | 85.7 |
| 7. Pt29.195#1 | 95.7 | 95.7 | 95.7 | 95.7 | 94.3 | 95.7 | | 92.9 | 90.0 | 92.9 | 91.4 | 92.9 | 77.1 | 85.7 |
| 8. CAF74710 | 97.1 | 94.3 | 94.3 | 95.7 | 94.3 | 95.7 | 98.6 | | 97.1 | 97.1 | 97.1 | 94.3 | 80.0 | 85.7 |
| 9. AAF04917 | 97.1 | 94.3 | 94.3 | 95.7 | 94.3 | 95.7 | 98.6 | 100.0 | | 94.3 | 94.3 | 94.3 | 77.1 | 82.9 |
| 10. AAQ14332 | 97.1 | 94.3 | 94.3 | 95.7 | 94.3 | 95.7 | 97.1 | 98.6 | 98.6 | | 94.3 | 95.7 | 81.4 | 85.7 |
| 11. AAY90122 | 95.7 | 95.7 | 95.7 | 97.1 | 95.7 | 97.1 | 98.6 | 98.6 | 98.6 | 97.1 | | 92.9 | 78.6 | 84.3 |
| 12. ABD65632 | 97.1 | 94.3 | 94.3 | 94.3 | 95.7 | 95.7 | 97.1 | 97.1 | 97.1 | 97.1 | 97.1 | | 80.0 | 87.1 |
| 13. ABK94979 | 90.0 | 88.6 | 88.6 | 88.6 | 92.9 | 88.6 | 87.1 | 88.6 | 88.6 | 88.6 | 90.0 | 90.0 | | 71.4 |
| 14. EDQ81347 | 91.4 | 91.4 | 91.4 | 91.4 | 91.4 | 92.9 | 94.3 | 92.9 | 92.9 | 92.9 | 92.9 | 95.7 | 87.1 | |

The percentage identity between the STO polypeptide sequences useful in performing the methods of the invention can be as low as 15 % amino acid identity compared to SEQ ID NO: 2 (Os04g0540200)

**Table B3:** MatGAT results for global similarity and identity over the full length of the polypeptide sequence amongst rice STO proteins.

| | 1 | 2 | 3 | 4 | 5 | 6 | 7 | 8 | 9 | 10 |
|---|---|---|---|---|---|---|---|---|---|---|
| 1. Os04g0540200 | | 29.4 | 32.3 | 63.1 | 33.8 | 28 | 29.2 | 39.3 | 28 | 37.2 |
| 2. Os01g0202500 | 39.5 | | 27.1 | 27.4 | 26.5 | 50.1 | 39.1 | 28.6 | 17.9 | 30.9 |
| 3. Os02g0606200 | 49.1 | 38.4 | | 30.3 | 61.2 | 26.4 | 29.6 | 31.8 | 26.6 | 29.6 |
| 4. Os02g0646200 | 71.7 | 39.8 | 45.8 | | 30.5 | 27 | 29.1 | 41.1 | 25.3 | 33.3 |
| **5.** Os04g0493000 | 48.2 | 37.5 | 71.6 | 43.9 | | 28.2 | 30.4 | 34.6 | 25.7 | 31.7 |
| 6. Os05g0204600 | 36.2 | 63 | 36.2 | 36 | 36 | | 37.9 | 26.2 | 17.6 | 29.4 |
| 7. Os06g0152200 | 40 | 54.4 | 42.5 | 39.2 | 40.3 | 54.2 | | 28.8 | 21.1 | 28 |
| 8. Os06g0713000 | 52.3 | 39.8 | 43.8 | 51.9 | 45.5 | 37 | 41.4 | | 21.4 | 29.9 |
| 9. Os09g0527900 | 39.2 | 27.5 | 34.3 | 35.3 | 41.6 | 25.7 | 30.3 | 32.5 | | 25.1 |
| 10. Os12g0209200 | 46.8 | 37.8 | 42.4 | 44.6 | 43.6 | 34.1 | 36.1 | 38.6 | 38.4 | |

**Table B4:** MatGAT results for global similarity and identity over the full length of the polypeptide sequence amongst orthologous sequences to SEQ IC NO:2

| | 1 | 2 | 3 | 4 | 5 | 6 | 7 | 8 | 9 | 10 | 11 | 12 | 13 |
|---|---|---|---|---|---|---|---|---|---|---|---|---|---|
| **1. AT1G06040_SplicedVariant1** | | 68.5 | 29.5 | 32 | 69.2 | 29.1 | 31.5 | 31.6 | 58.9 | 59.7 | 32.3 | 30 | 62.5 |
| **2. AT1G06040_SplicedVariant2** | 69 | | 26.2 | 30.1 | 47.4 | 38.3 | 30.6 | 32.9 | 49.4 | 49.8 | 30 | 39.3 | 48.6 |
| **3. AT1G75540** | 43.2 | 35.6 | | 29.5 | 28.4 | 23.4 | 35.6 | 32.3 | 30.5 | 30.2 | 51.5 | 25.5 | 29 |
| **4. AT1 G78600** | 48.2 | 40.1 | 45.9 | | 32.1 | 28.2 | 29.7 | 28.9 | 32.1 | 32.1 | 30.9 | 30.5 | 30.6 |
| **5. AT2G31380** | 82.7 | 60.9 | 40.5 | 44.5 | | 29.5 | 34.6 | 32.6 | 58.2 | 58.2 | 32.2 | 29.6 | 58.3 |
| **6. AT4G10240** | 41.9 | 57.6 | 32.9 | 36.5 | 42 | | 30 | 31 | 31.4 | 29.3 | 23.8 | 39.9 | 30.3 |
| **7. AT4G39070** | 50.4 | 43 | 47.7 | 43.5 | 48.3 | 40.9 | | 46.8 | 30 | 30.4 | 38.7 | 29.6 | 30.9 |
| **8. MS_ABO84497** | 49.2 | 49.1 | 46.5 | 41.8 | 49.2 | 44.6 | 60.7 | | 32.6 | 30.8 | 37.5 | 35.1 | 30.6 |
| **9. LE_AAS67368** | 69.4 | 57.1 | 41.4 | 45.2 | 70.6 | 45.9 | 47.1 | 51.9 | | 98.3 | 33.4 | 33.2 | 65.8 |
| **10. ST_ABA40448.1** | 69.8 | 57.1 | 40.8 | 45.5 | 71 | 45.1 | 47.1 | 48.5 | 99.1 | | 33.1 | 32.8 | 66.7 |
| **11. PP_STO** | 43.5 | 37.1 | 65.3 | 43.5 | 42.6 | 33.9 | 50.3 | 53.2 | 46.1 | 46.1 | | 27.3 | 30.8 |
| **12. VV-CAN72879.1** | 45.6 | 53.2 | 35.6 | 45.2 | 49.2 | 54.6 | 44.2 | 47.3 | 49.8 | 48.9 | 37.1 | | 30.9 |
| **13. GM_ABB29467** | 74.6 | 57.1 | 40.2 | 44.1 | 73.5 | 43.7 | 45 | 50 | 76.5 | 76.5 | 42.3 | 45.4 | |

The percentage identity between the UGE polypeptide sequences useful in performing the methods of the invention can be as low as 15 % amino acid identity compared to SEQ ID NO: 222.

**Table B5:** MatGAT results for global similarity and identity over the full length of the polypeptide sequences representing UGE isoforms in rice.

| UGE protein Name | 1 | 2 | 3 | 4 | 5 | 6 | 7 | 8 |
|---|---|---|---|---|---|---|---|---|
| **1. Os04g0618200** | | 36.7 | 74.3 | 35.9 | 37 | 21.4 | 36.5 | 36.7 |
| **2. Os05g0595100** | 51.7 | | 36.2 | 65.8 | 72.2 | 21.1 | 58.6 | 99.7 |
| **3. Os07g0139400** | 81.7 | 50.6 | | 34.7 | 34.8 | 21.5 | 33.7 | 36 |
| **4. Os08g0374800** | 51 | 75.7 | 50.6 | | 74.4 | 21.7 | 53.4 | 65.8 |
| **5. Os09g0323000** | 51.9 | 82.4 | 49.6 | 82.1 | | 22.6 | 56.3 | 72.8 |
| **6. Os09g0504000** | 36.7 | 33.1 | 38.8 | 34.5 | 35.7 | | 21.4 | 21.6 |
| **7. Os09g0526700** | 51.4 | 74 | 48.2 | 67.6 | 72.9 | 34.5 | | 58.6 |
| **8. OS_UGE2** | 51.7 | 100 | 50.6 | 75.5 | 82.9 | 33.5 | 73.5 | |

**Table B6:** MatGAT results for global similarity and identity over the full length of the polypeptide sequences representing UGE isoforms originating form different organisms.

| **UGE protein Name** | 1 | 2 | 3 | 4 | 5 | 6 | 7 | 8 | 9 | 10 | 11 | 12 | 13 | 14 | 15 | 16 | 17 |
|---|---|---|---|---|---|---|---|---|---|---|---|---|---|---|---|---|---|
| 1. AT1G12780_UGE | | 90.6 | 63.3 | 54.6 | 64.3 | 33.9 | 33.1 | 32.9 | 43.6 | 65.8 | 54.3 | 33.1 | 33.5 | 24.5 | 59.6 | 47.7 | 65.1 |
| 2. AT1G63180_UGE | 95.4 | | 62.1 | 63.8 | 63.5 | 32.7 | 31.8 | 32.1 | 42.7 | 65.5 | 53.7 | 32 | 33.1 | 24 | 60.1 | 49.1 | 65.4 |
| 3. AT1G64440_UGE | 78.3 | 77.5 | | 79.2 | 79.4 | 37.6 | 34.6 | 34.2 | 32.3 | 79.6 | 64.6 | 34.4 | 33.9 | 26.9 | 59.9 | 49.3 | 74.9 |
| 4. AT4G10960_UGE | 81.5 | 80.1 | 88 | | 88.3 | 37.4 | 34.9 | 34.6 | 31 | 81.2 | 64.6 | 34.7 | 35.9 | 28.3 | 54.2 | 50.3 | 76.4 |
| 5. AT4G23920_UGE | 80.1 | 77.8 | 88 | 92.9 | | 37.1 | 35.3 | 34.8 | 31.3 | 83.1 | 65.9 | 35.3 | 35.6 | 28.5 | 62.4 | 50.4 | 75.8 |
| 6. Pt_lcl\|scaff_XVI.140 | 53.2 | 51.3 | 55 | 54.5 | 54 | | 75.3 | 82.7 | 21.1 | 36.8 | 32.9 | 73.2 | 22.5 | 19.6 | 36.1 | 35.2 | 36.1 |
| 7. Pt_lcl\|scaff_XI.1329 | 49.4 | 47.5 | 51.1 | 49.2 | 49.2 | 81.5 | | 76.2 | 18.9 | 34.7 | 31 | 94.5 | 21.1 | 19.9 | 33.3 | 33.1 | 34.3 |
| 8. Pt_lcl\|scaff_VI.203 | 49.5 | 48 | 50 | 50.2 | 48.8 | 87.6 | 85.9 | | 20.4 | 34.9 | 32.2 | 75.5 | 22 | 19.4 | 34.2 | 33 | 34.3 |
| 9. Pt_lcl\|scaff_III.961 | 47.9 | 46.4 | 42 | 41.9 | 42.6 | 33.3 | 30.9 | 31.7 | | 33.1 | 29.9 | 19.6 | 18.2 | 15.2 | 30 | 25.4 | 31.8 |
| 10. Pt_lcl\|scaff_III.1133 | 79.8 | 78.3 | 88.2 | 90.3 | 90.3 | 52.9 | 48.7 | 47.8 | 43.4 | | 73.1 | 34.4 | 34.7 | 27.2 | 52.5 | 50 | 78.2 |
| 11. Pt_lcl\|scaff_I.773 | 69.2 | 68.7 | 76.1 | 76.1 | 77.4 | 49.2 | 44.1 | 45.9 | 41.9 | 80.5 | | 30.8 | 28.6 | 23.7 | 52 | 41.8 | 62.5 |
| 12. Pt_lcl\|scaff_I.2996 | 49.4 | 47.5 | 50.8 | 49.4 | 48.9 | 80.6 | 97.1 | 85.1 | 30.9 | 48.2 | 44.1 | | 20.8 | 19.4 | 33.3 | 32.4 | 34 |
| 13. Pt_lcl\|scaff_5422 | 45 | 44.5 | 45.8 | 48.1 | 47.3 | 39.3 | 38.4 | 39.5 | 25 | 46.4 | 40.1 | 38.2 | | 32.5 | 35.5 | 32.9 | 35 |
| 14. Ot08g015500 | 34.9 | 34.5 | 35.5 | 37.1 | 36.8 | 28.9 | 29.8 | 29.8 | 20 | 36.3 | 32.2 | 29.8 | 45.8 | | 26.2 | 24.5 | 26.9 |
| 15. SC_ GAL10_P04397 | 77.8 | 76.4 | 73.6 | 75.6 | 75.3 | 52.1 | 47 | 47.3 | 40.9 | 73.9 | 65.1 | 47.2 | 48.7 | 34.6 | | 47.3 | 61.7 |
| 16. Ec_GALE_ AAO37702 | 67.5 | 67.8 | 65.5 | 66.7 | 67.4 | 51.1 | 46.5 | 46.1 | 39.5 | 67.5 | 61.9 | 46.3 | 44.5 | 34.2 | 63.6 | | 50 |
| 17. OS_UGE2 | 80.2 | 79.9 | 83.9 | 87.3 | 86.7 | 55.6 | 50.6 | 50.2 | 42.1 | 87.6 | 74.3 | 50.4 | 45 | 37.5 | 73.2 | 67.8 | |

A MATGAT table for local alignment of a specific domain, or data on % identity/similarity between specific domains may also be generated for the other polypeptides (or part thereof) of the invention.

*Example 4: Identification of domains comprised in polypeptide sequences useful* ***in*** *performing the methods of the invention*

The Integrated Resource of Protein Families, Domains and Sites (InterPro) database is an integrated interface for the commonly used signature databases for text- and sequence-based searches. The InterPro database combines these databases, which use different methodologies and varying degrees of biological information about well-characterized proteins to derive protein signatures. Collaborating databases include SWISS-PROT, PROSITE, TrEMBL, PRINTS, ProDom and Pfam, Smart and TIGRFAMs. Pfam is a large collection of multiple sequence alignments and hidden Markov models covering many common protein domains and families. Pfam is hosted at the Sanger institute server in the United Kingdom. Interpro is hosted at the European Bioinformatics Institute in the United Kingdom.

The results of the InterPro scan of the polypeptide sequence as represented by SEQ ID NO: 2 are presented in Table C1. The detected domains classify within the Interpro families IPR001092 (Basic helix-loop-helix dimerisation region bHLH) and IPR001092 (Basic helix-loop-helix dimerisation region bHLH).

**Table C1:** InterPro scan results (major accession numbers) of the polypeptide sequence as represented by SEQ ID NO: 2.

| Database | Accession number | Accession name | Amino acid coordinates on SEQ ID NO 2 |
|---|---|---|---|
| HMMPfam | PF00010 | HLH | 449-498 |
| ProfileScan | PS50888 | HLH | 438-498 |
| superfamily | SSF47459 | HLH_basic | 451-522 |

The protein sequences representing the GRP (RrmJ/FtsJ polypeptide) are used as query to search the InterPro database. GRP polypeptides useful in performing the methods of the invention have a ribosomal RNA methyltransferase RrmJ/FtsJ domain (InterPro entries IPR002877 and IPR015507; Pfam entry PF01728; PANTHER entry PTHR10920).

The GRP polypeptide (bHLH4 polypeptide) sequences are used as query to search the InterPro database. GRP polypeptides useful in performing the methods of the invention match two InterPro entries: (i) IPR001092 basic helix-loop-helix dimerisation region bHLH; and (ii) IPR011598 helix-loop-helix DNA-binding.

**Table C2:**

| InterPro accession number | Integrated database name | Integrated database accession number | Integrated database accession name |
|---|---|---|---|
| IPR001092 Basic helix-loop-helix dimerisation region bHLH | Pfam | PF00010 | HLH |
| " | SMART | SM00353 | HLH |
| " | Prosite | PS50888 | HLH |
| IPR011598 Helix-loop-helix DNA-binding | | SSF47459 | HLH_basic |
| unintegrated | Panther | PTHR12565 | |
| | Panther | PTHR12565 | SF7 |

The GRP polypeptide (IPT polypeptide) sequences are used as query to search the InterPro database. GRP polypeptides useful in performing the methods of the invention match InterPro entries: (i) IPR002627; and (ii) IPR011593, as shown in the table below:

**Table C3:**

| InterPro accession number | Integrated database name | Integrated database accession number | Integrated database accession name |
|---|---|---|---|
| IPR002627 Family tRNA isopentenyltransferase | ProDom | PD004674 | Q6GHD2_STAAR_Q6GHD2 |
| | Panther | PTHR11088 | TRNA DELTA (2)-ISOPENTENYLPYROPHOSPHATE TRANSFERASE-RELATED |
| | Pfam | PF01715 | IPPT |
| IPR011593 Domain isopentenyl transferase-like | ProDom | PD005388 | Q9CA35_ARATH_Q9CA35 |
| No IPR integrated | Panther | PTHR11088:SF20 | CYTOKININ SYNTHASE |

The results of the InterPro scan of the polypeptide sequence as represented by SEQ ID NO: 166 are presented in Table C4.

**Table C4:** InterPro scan results (major accession numbers) of the polypeptide sequence as represented by SEQ ID NO: 166.

| Interpro hit | Accession number | Accession number | Name Accession number | Amino acid coordinates of the B-box1 domain | E-value | Amino acid coordinates of the B-box2 domain | E-value |
|---|---|---|---|---|---|---|---|
| IPR000315 | PFAM | PF00643 | zf-B_box | [1-47] | 1.20E-10 | [58-105] | 8.00E-12 |
| | SMART | SM00336 | BBOX | [1-47] | 3.00E-11 | [58-105] | 4.80E-13 |
| | PROFILE | PS50119 | ZF_BBOX | [1-47] | 9.84 | [58-105] | 10.04 |

**Table C5:** Parameters used to build the HMMs for the B-box domain in pfam.

| | Pfam_ls [Download HMM] | Pfam_fs [Download HMM] |
|---|---|---|
| **Gathering cutoff** | 15.3 15.3; | 21.021.0 |
| **Trusted cutoff** | 15.5 15.5; | 21.021.0 |
| **Noise cutoff** | 15.2 15.2; | 20.8 |
| **Build method of HMM** | hmmbuild -F HMM ls SEED | hmmbuild -f -F HMM fs SEED |
| | hmmcalibrate --seed 0 HMM_ls | hmmcalibrate -seed 0 HMM_fs |

The results of the InterPro scan of the polypeptide sequence as represented by SEQ ID NO: 222 are presented in Table C6.

**Table C6:** InterPro scan results (major accession numbers) of the polypeptide sequence as represented by SEQ ID NO: 222.

| Database | Accession number | Accession name | Amino acid coordinates on SEQ ID NO 2 | e-value |
|---|---|---|---|---|
| INTERPRO | IPR001509 | NAD-dependent epimerase/ dehydratase | [9-273] | 4.20E-74 |
| PFAM | PF01370 | Epimerase | [9-273] | 4.20E-74 |

***Example 5: Topology prediction of the polypeptide sequences useful in performing the methods of the invention***

TargetP 1.1 predicts the subcellular location of eukaryotic proteins. The location assignment is based on the predicted presence of any of the N-terminal pre-sequences: chloroplast transit peptide (cTP), mitochondrial targeting peptide (mTP) or secretory pathway signal peptide (SP). Scores on which the final prediction is based are not really probabilities, and they do not necessarily add to one. However, the location with the highest score is the most likely according to TargetP, and the relationship between the scores (the reliability class) may be an indication of how certain the prediction is. The reliability class (RC) ranges from 1 to 5, where 1 indicates the strongest prediction. TargetP is maintained at the server of the Technical University of Denmark.

For the sequences predicted to contain an N-terminal presequence a potential cleavage site can also be predicted.

A number of parameters were selected, such as organism group (non-plant or plant), cutoff sets (none, predefined set of cutoffs, or user-specified set of cutoffs), and the calculation of prediction of cleavage sites (yes or no).

The results of TargetP 1.1 analysis of the polypeptide sequence as represented by SEQ ID NO: 2 are presented Table D. The "plant" organism group has been selected, no cutoffs defined, and the predicted length of the transit peptide requested. The subcellular localization of the polypeptide sequence as represented by SEQ ID NO: 2 may be the cytoplasm or nucleus, no transit peptide is predicted.

**Table D1:** TargetP 1.1 analysis of the polypeptide sequence as represented by SEQ ID NO: 2

| | |
|---|---|
| Length (AA) | 623 |
| Chloroplastic transit peptide | 0.155 |
| Mitochondrial transit peptide | 0.143 |
| Secretory pathway signal peptide | 0.099 |
| Other subcellular targeting | 0.816 |
| Predicted Location | / |
| Reliability class | 2 |
| Predicted transit peptide length | / |

When analysed with the PredictNLS algorithm, a nuclear localisation is predicted for SEQ ID NO: 2 with a probability of 98%:

| NLS | From | To | signal | Type |
|---|---|---|---|---|
| [KR][KR]x[KR][KR][KR]x[KR][KR] | 433 | 441 | KRPKKRGRK | Potential |

| NumWithNLS | **%NucProt** |
|---|---|
| 54 | **98.14** |

Many other algorithms can be used to perform such analyses, including:

- ChloroP 1.1 hosted on the server of the Technical University of Denmark;

- Protein Prowler Subcellular Localisation Predictor version 1.2 hosted on the server of the Institute for Molecular Bioscience, University of Queensland, Brisbane, Australia;
- PENCE Proteome Analyst PA-GOSUB 2.5 hosted on the server of the University of Alberta, Edmonton, Alberta, Canada;
- TMHMM, hosted on the server of the Technical University of Denmark

The protein sequences representing the GRP are used to query TargetP 1.1. The "plant" organism group is selected, no cutoffs defined, and the predicted length of the transit peptide requested.

The results of TargetP 1.1 analysis of the polypeptide sequence as represented by SEQ ID NO: 225 are presented Table D. The "plant" organism group has been selected, no cutoffs defined, and the predicted length of the transit peptide requested. The polypeptide sequence as represented by SEQ ID NO: 225 may be channelled through the secretory pathway to its final subcellular localization.

**Table D2:** TargetP 1.1 analysis of the polypeptide sequence as represented by SEQ ID NO: 222

| | |
|---|---|
| Length (AA) | 354 |
| Chloroplastic transit peptide | / |
| Mitochondrial transit peptide | 0.218 |
| Secretory pathway signal peptide | 0.451 |
| Other subcellular targeting | 0.268 |
| Predicted Location | S* |
| Reliability class | 5 |
| Predicted transit peptide length | / |
| *S: secretory pathway. | |

### *Example 6: Functional assay for the bHLH6-like polypeptide*

A DNA-binding assay for AtbHLH6 is provided in Dombrecht et al. (2007). Briefly, a 1000-bp fragment of the AtbHLH6 coding sequence encompassing codons 285 to 623 was amplified from genomic DNA and cloned into the NheI-BamHI-digested pTacLCELD6·His vector (Xue, Plant J. 41, 638-649, 2005). The resulting construct encoded the last 338 amino acids of AtbHLH6 (including the bHLH region) in-frame with the reporter protein CelD and a 6xHis tag. Correct amplification and cloning were verified by DNA sequencing.

Determination of the consensus sequence of the MYC2 DNA binding motif and the relative binding affinity of these sites was done according to Xue (2005), wherein a fusion of a DNA-binding protein (DBP) to 6-His-tagged cellulase D (CELD) serves both as a means for affinity purification of DBP-DNA complex in the selection of binding sites from a pool of biotinylated random-sequence oligonucleotides and as a reporter for measurement of DNA-binding activity. For the selection of binding-sites using cellulose paper as an affinity matrix, DBP-CELD was incubated at room temperature for 1 h with 20 ng of a biotin-labelled Bio-RS-Oligo in 40 μl of binding/washing buffer (described above) containing 1 mM EDTA, 0.25 μg $\mu l^{-1}$ poly d(AC-TG), 1 mg $ml^{-1}$ BSA and 10% glycerol. An appropriate amount of crude DBP-CELD used for binding-site selection was that achieving 20-30% relative cellulose binding efficiency (percentage cellulose activity bound to cellulose after washing). DBP-CELD/Bio-RS-Oligo mixtures were transferred to 96-well microplate wells containing Whatman 1 filter paper (4 $mm^2$) which was pre-soaked with 10 μl blocking solution [binding/washing buffer containing 0.5 μg $\mu l^{-1}$ poly d(AC-TG) and 10% glycerol]. After incubation at 0°C for 1 hr with gentle shaking, the cellulose paper was washed six times with binding/washing buffer containing 1 mM EDTA and 0.1 mg $ml^{-1}$ BSA. The use of extensive washing in the target site selection was based on observations of the relatively high stability of DBP-oligonucleotide complex immobilized on solid matrix. DBP-CELD carrying target binding sites were eluted at 40°C for 15 min with 40 μl of cellulase eluting buffer [10 mM HEPES, pH7, 50 mM KCl, 0.2 mM EDTA, 4 mM cellobiose, 0.05 mg $ml^{-1}$ BSA and 10 μg $ml^{-1}$ of a sense sequence-specific primer SP-S]. The eluate was used for PCR amplification of selected oligonucleotides. The PCR product (0.1 μl) without purification was used for the next round of site selection.

Alternatively, 6·His tagged DBP-CELD (10-25 μg crude protein) was incubated in 60 μl of PNT buffer (50 mM sodium phosphate, pH8.0, 300 mM NaCl and 0.05% Tween 20) containing 10 mM imidazole and 350 μg Ni-NTA magnetic agarose beads (Qiagen) at room temperature for 45-60 min with gentle shaking in a micro-tube mixer (Tomy Seiko Co., Tokyo, Japan). The Ni-NTA magnetic beads were collected at the side of the tubes by placing tubes in a 12-tube magnet (Qiagen). Unbound proteins were removed by washing twice with 150-200 μl of PNT containing 20 mM imidazole and

once with 60 μl of binding/washing buffer containing 2 mM $MgCl_2$, 1 mg $ml^{-1}$ BSA and 10% glycerol. Washed beads were suspended in 40 μl of binding/washing buffer containing 2 mM $MgCl_2$, 0.25 μg $\mu l^{-1}$ poly d(AC-TG), 1 mg $ml^{-1}$ BSA, 0.0025% Nonidet P-40, 10% glycerol and biotin-labelled oligonucleotides (50 ng Bio-RS-Oligo or 1 μl of PCR product amplified from previously selected oligonucleotides). The suspension was incubated at room temperature for 1.5 h with gentle shaking in the micro-tube mixer. After washing four times (3-4 min each washing) with 150-200 μl of binding/washing buffer containing 2 mM $MgCl_2$, 0.1 mg $ml^{-1}$ BSA and 0.0025% Nonidet P-40, the beads carrying target site-bound BDP-CELD were resuspended in 8 μl of 5mM Tris-Cl (pH 8.0)/0.5 mM EDTA containing 5 μg $ml^{-1}$ of a sense sequence-specific primer (SP-S) and the suspension was transferred to a clean tube and used for PCR amplification of selected oligonucleotides. The PCR product (1 μl) without purification was used for the next round of site selection.

For EMSA assays, 6·His-tagged DBP-CELD proteins were purified using Ni-NTA magnetic agarose beads using a high stringent binding buffer (50 mMsodium phosphate, pH8.0, 1 MNaCl, 10% glycerol, 1% Tween 20 and 10 mM imidazole) and the rest of the procedure followed the manufacturer's instruction. Double-stranded synthetic oligonucleotides (30-45 fmol) labelled with digoxigenin at the 3'-end were incubated with a purified DBP (30-75 ng) in 15 μl of binding buffer [25 mM HEPES/KOH, pH 7.0, 50 mM KCl, 0.5 mM DTT, 2 mM $MgCl_2$, 0.2 μg $\mu l^{-1}$ poly d(AC-TG), 0.3 mg $ml^{-1}$ BSA and 10% glycerol]. After incubation at room temperature for 30 min, DBP/DNA complexes were separated from free probes on a 6% polyacrylamide gel in a 40-mM Tris-acetate buffer (pH 7.5) containing 5 mM Na acetate, 0.5 mM EDTA and 5% glycerol. The DBP/DNA complexes and free probes in the gels after electrophoresis were transferred to a Hybond Nþ membrane. Alkaline phosphatase-conjugated anti-digoxigenin antibody and a chemiluminescent substrate, CDP-Star (Roche Diagnostics) was used for detection of digoxigenin according to the manufacturer's instructions.

Further details are provided in Xue (2005).

### *Example 7: Cloning of the nucleic acid sequence used in the methods of the invention*

The nucleic acid sequence used in the methods of the invention was amplified by PCR using as template a custom-made *Arabidopsis thaliana* seedlings cDNA library (in pCMV Sport 6.0; Invitrogen, Paisley, UK). PCR was performed using Hifi Taq DNA polymerase in standard conditions, using 200 ng of template in a 50 μl PCR mix The primers used were prm06515 (SEQ ID NO: 10; sense, start codon in bold):

5'-ggggacaagtttgtacaaaaaagcaggcttaaaca**atg**actgattaccggctacaa-**3'** and prm06516 (SEQ ID NO: 11; reverse, complementary):
5'-ggggaccactttgtacaagaaagctgggtacacccttttaaccgattttt-3',

which include the AttB sites for Gateway recombination. The amplified PCR fragment was purified also using standard methods. The first step of the Gateway procedure, the BP reaction, was then performed, during which the PCR fragment recombines in vivo with the pDONR201 plasmid to produce, according to the Gateway terminology, an "entry clone", pbHLH6-like. Plasmid pDONR201 was purchased from Invitrogen, as part of the Gateway® technology.

The entry clone comprising SEQ ID NO: 1 was then used in an LR reaction with a destination vector used for *Oryza sativa* transformation. This vector contained as functional elements within the T-DNA borders: a plant selectable marker; a screenable marker expression cassette; and a Gateway cassette intended for LR *in vivo* recombination with the nucleic acid sequence of interest already cloned in the entry clone. A rice GOS2 promoter (SEQ ID NO: 12) for root specific expression was located upstream of this Gateway cassette.

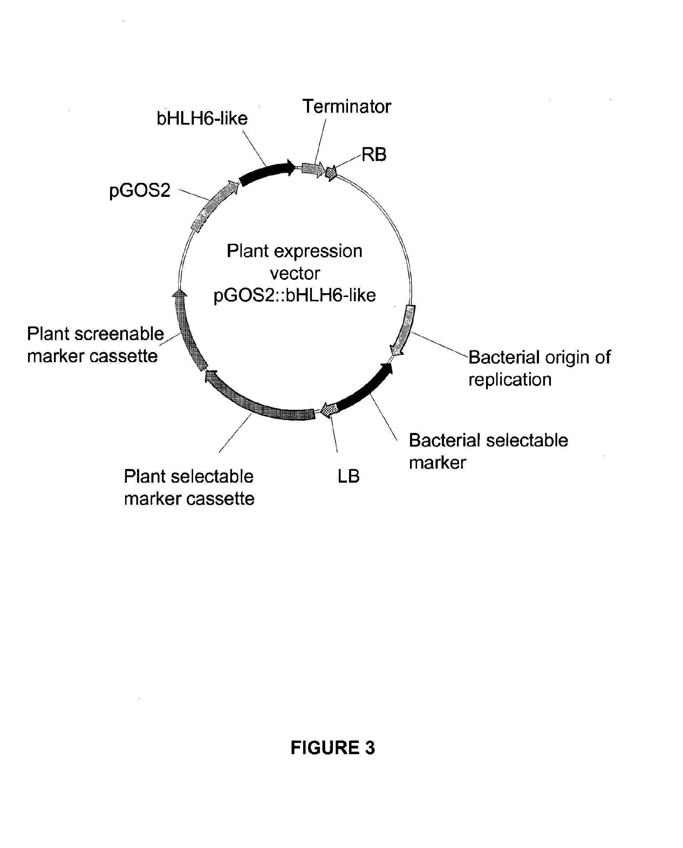

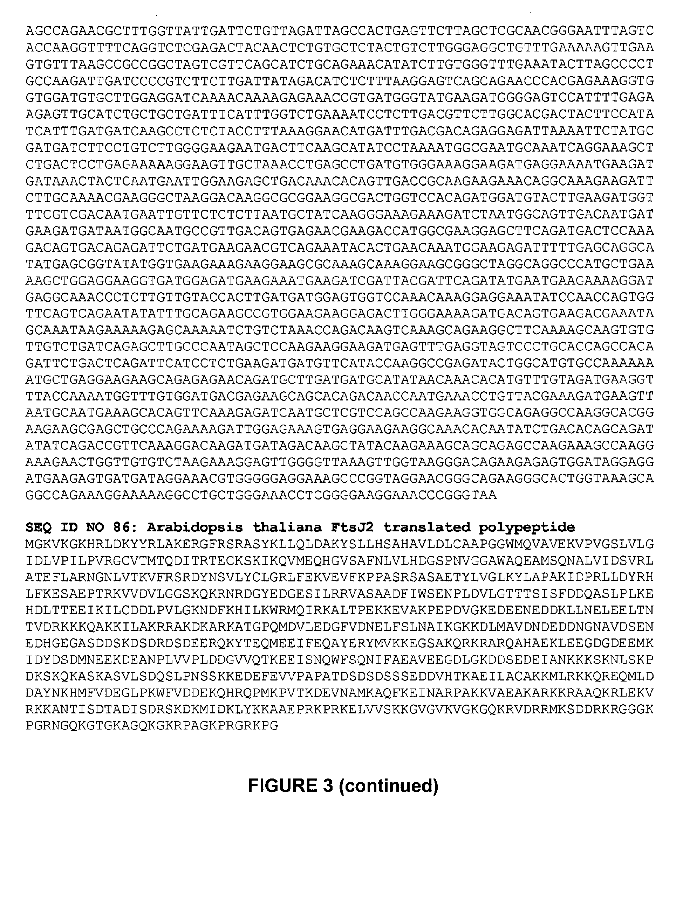

After the LR recombination step, the resulting expression vector pGOS2::bHLH6-like (Figure 3) was transformed into *Agrobacterium* strain LBA4044 according to methods well known in the art.

### *Example 8: Plant transformation*

*Rice transformation*

The *Agrobacterium* containing the expression vector was used to transform *Oryza sativa* plants. Mature dry seeds of the rice japonica cultivar Nipponbare were dehusked. Sterilization was carried out by incubating for one minute in 70% ethanol, followed by 30 minutes in 0.2% $HgCl_2$, followed by a 6 times 15 minutes wash with sterile distilled water. The sterile seeds were then germinated on a medium containing 2,4-D (callus induction medium). After incubation in the dark for four weeks, embryogenic, scutellum-derived calli were excised and propagated on the same medium. After two weeks, the calli were multiplied or propagated by subculture on the same medium for another 2 weeks. Embryogenic callus pieces were subcultured on fresh medium 3 days before co-cultivation (to boost cell division activity).

*Agrobacterium* strain LBA4404 containing the expression vector was used for co-cultivation. *Agrobacterium* was inoculated on AB medium with the appropriate antibiotics and cultured for 3 days at 28°C. The bacteria were then collected and suspended in liquid co-cultivation medium to a density ($OD_{600}$) of about 1. The suspension was then transferred to a Petri dish and the calli immersed in the suspension for 15 minutes. The callus tissues were then blotted dry on a filter paper and transferred to solidified, co-cultivation medium and incubated for 3 days in the dark at 25°C. Co-cultivated calli were grown on 2,4-D-containing medium for 4 weeks in the dark at 28°C in the presence of a selection agent. During this period, rapidly growing resistant callus islands developed. After transfer of this material to a regeneration medium and incubation in the light, the embryogenic potential was released and shoots developed in the next four to five weeks. Shoots were excised from the calli and incubated for 2 to 3 weeks on an auxin-containing medium from which they were transferred to soil. Hardened shoots were grown under high humidity and short days in a greenhouse.

Approximately 35 independent T0 rice transformants were generated for one construct. The primary transformants were transferred from a tissue culture chamber to a greenhouse. After a quantitative PCR analysis to verify copy number of the T-DNA insert, only single copy transgenic plants that exhibit tolerance to the selection agent were kept for harvest of T1 seed. Seeds were then harvested three to five months after transplanting. The method yielded single locus transformants at a rate of over 50 % (Aldemita and Hodges1996, Chan et al. 1993, Hiei et al. 1994).

*Corn transformation*

Transformation of maize *(Zea mays)* is performed with a modification of the method described by Ishida et al. (1996) Nature Biotech 14(6): 745-50. Transformation is genotype-dependent in corn and only specific genotypes are amenable to transformation and regeneration. The inbred line A188 (University of Minnesota) or hybrids with A188 as a parent are good sources of donor material for transformation, but other genotypes can be used successfully as well. Ears are harvested from corn plant approximately 11 days after pollination (DAP) when the length of the immature embryo is about 1 to 1.2 mm. Immature embryos are cocultivated with *Agrobacterium tumefaciens* containing the expression vector, and transgenic plants are recovered through organogenesis. Excised embryos are grown on callus induction medium, then maize regeneration medium, containing the selection agent (for example imidazolinone but various selection markers can be used). The Petri plates are incubated in the light at 25°C for 2-3 weeks, or until shoots develop. The green shoots are transferred from each embryo to maize rooting medium and incubated at 25 °C for 2-3 weeks, until roots develop. The rooted shoots are transplanted to soil in the greenhouse. T1 seeds are produced from plants that exhibit tolerance to the selection agent and that contain a single copy of the T-DNA insert.

*Wheat transformation*

Transformation of wheat is performed with the method described by Ishida et al. (1996) Nature Biotech 14(6): 745-50. The cultivar Bobwhite (available from CIMMYT, Mexico) is commonly used in transformation. Immature embryos are cocultivated with *Agrobacterium tumefaciens* containing the expression vector, and transgenic plants are recovered through organogenesis. After incubation with *Agrobacterium,* the embryos are grown in vitro on callus induction medium, then regeneration medium, containing the selection agent (for example imidazolinone but various selection markers can be used). The Petri plates are incubated in the light at 25 °C for 2-3 weeks, or until shoots develop. The green shoots are transferred from each embryo to rooting medium and incubated at 25 °C for 2-3 weeks, until roots develop. The rooted shoots are transplanted to soil in the greenhouse. T1 seeds are produced from plants that exhibit tolerance to the selection agent and that contain a single copy of the T-DNA insert.

*Soybean transformation*

Soybean is transformed according to a modification of the method described in the Texas A&M patent US 5,164,310. Several commercial soybean varieties are amenable to transformation by this method. The cultivar Jack (available from the Illinois Seed foundation) is commonly used for transformation. Soybean seeds are sterilised for *in vitro* sowing. The hypocotyl, the radicle and one cotyledon are excised from seven-day old young seedlings. The epicotyl and the remaining cotyledon are further grown to develop axillary nodes. These axillary nodes are excised and incubated with *Agrobacterium tumefaciens* containing the expression vector. After the cocultivation treatment, the explants are washed and transferred to selection media. Regenerated shoots are excised and placed on a shoot elongation medium. Shoots no longer than 1 cm are placed on rooting medium until roots develop. The rooted shoots are transplanted to soil in the greenhouse. T1 seeds are produced from plants that exhibit tolerance to the selection agent and that contain a single copy of the T-DNA insert.

*Rapeseed/canola transformation*

Cotyledonary petioles and hypocotyls of 5-6 day old young seedling are used as explants for tissue culture and transformed according to Babic et al. (1998, Plant Cell Rep 17: 183-188). The commercial cultivar Westar (Agriculture Canada) is the standard variety used for transformation, but other varieties can also be used. Canola seeds are surface-sterilized for in vitro sowing. The cotyledon petiole explants with the cotyledon attached are excised from the in vitro seedlings, and inoculated with *Agrobacterium* (containing the expression vector) by dipping the cut end of the petiole explant into the bacterial suspension. The explants are then cultured for 2 days on MSBAP-3 medium containing 3 mg/l BAP, 3 % sucrose, 0.7 % Phytagar at 23 °C, 16 hr light. After two days of co-cultivation with *Agrobacterium,* the petiole explants are transferred to MSBAP-3 medium containing 3 mg/l BAP, cefotaxime, carbenicillin, or timentin (300 mg/l) for 7 days, and then cultured on MSBAP-3 medium with cefotaxime, carbenicillin, or timentin and selection agent until shoot regeneration. When the shoots are 5 - 10 mm in length, they are cut and transferred to shoot elongation medium (MSBAP-0.5, containing 0.5 mg/l BAP). Shoots of about 2 cm in length are transferred to the rooting medium (MS0) for root induction. The rooted shoots are transplanted to soil in the greenhouse. T1 seeds are produced from plants that exhibit tolerance to the selection agent and that contain a single copy of the T-DNA insert.

*Alfalfa transformation*

A regenerating clone of alfalfa *(Medicago sativa)* is transformed using the method of (McKersie et al., 1999 Plant Physiol 119: 839-847). Regeneration and transformation of alfalfa is genotype dependent and therefore a regenerating plant is required. Methods to obtain regenerating plants have been described. For example, these can be selected from the cultivar Rangelander (Agriculture Canada) or any other commercial alfalfa variety as described by Brown DCW and A Atanassov (1985. Plant Cell Tissue Organ Culture 4: 111-112). Alternatively, the RA3 variety (University of Wisconsin) has been selected for use in tissue culture (Walker et al., 1978 Am J Bot 65:654-659). Petiole explants are cocultivated with an overnight culture of *Agrobacterium tumefaciens* C58C1 pMP90 (McKersie et al., 1999 Plant Physiol 119: 839-847) or LBA4404 containing the expression vector. The explants are cocultivated for 3 d in the dark on SH induction medium containing 288 mg/ L Pro, 53 mg/ L thioproline, 4.35 g/ L K2SO4, and 100 μm acetosyringinone. The explants are washed in half-strength Murashige-Skoog medium (Murashige and Skoog, 1962) and plated on the same SH induction medium without acetosyringinone but with a suitable selection agent and suitable antibiotic to inhibit *Agrobacterium* growth. After several weeks, somatic embryos are transferred to BOi2Y development medium containing no growth regulators, no antibiotics, and 50 g/ L sucrose. Somatic embryos are subsequently germinated on half-strength Murashige-Skoog medium. Rooted seedlings were transplanted into pots and grown in a greenhouse. T1 seeds are produced from plants that exhibit tolerance to the selection agent and that contain a single copy of the T-DNA insert.

*Cotton transformation*

Cotton is transformed using *Agrobacterium tumefaciens* according to the method described in US 5,159,135. Cotton seeds are surface sterilised in 3% sodium hypochlorite solution during 20 minutes and washed in distilled water with 500 μg/ml cefotaxime. The seeds are then transferred to SH-medium with 50μg/ml benomyl for germination. Hypocotyls of 4 to 6 days old seedlings are removed, cut into 0.5 cm pieces and are placed on 0.8% agar. An *Agrobacterium* suspension (approx 108 cells per ml, diluted from an overnight culture transformed with the gene of interest and suitable selection markers) is used for inoculation of the hypocotyl explants. After 3 days at room temperature and lighting, the tissues are transferred to a solid medium (1.6 g/l Gelrite) with Murashige and Skoog salts with B5 vitamins (Gamborg et al., Exp. Cell Res. 50:151-158 (1968)), 0.1 mg/l 2,4-D, 0.1 mg/l 6-furfurylaminopurine and 750 μg/ml MgCL2, and with 50 to 100 μg/ml cefotaxime and 400-500 μg/ml carbenicillin to kill residual bacteria. Individual cell lines are isolated after two to three months (with subcultures every four to six weeks) and are further cultivated on selective medium for tissue amplification (30°C, 16 hr photoperiod). Transformed tissues are subsequently further cultivated on non-selective medium during 2 to 3 months to give rise to somatic embryos. Healthy looking embryos of at least 4 mm length are transferred to tubes with SH medium in fine vermiculite, supplemented with 0.1 mg/l indole acetic acid, 6 furfurylaminopurine and gibberellic acid. The embryos are cultivated at 30°C with a photoperiod of 16 hrs, and plantlets at the 2 to 3 leaf stage are transferred to pots with vermiculite and nutrients. The plants are hardened and subsequently moved to the greenhouse for further cultivation.

***Example 9: Phenotypic evaluation procedure***

9.1 Evaluation setup

Approximately 35 independent T0 rice transformants were generated. The primary transformants were transferred from a tissue culture chamber to a greenhouse for growing and harvest of T1 seed. Six events, of which the T1 progeny segregated 3:1 for presence/absence of the transgene, were retained. For each of these events, approximately 10 T1 seedlings containing the transgene (hetero- and homo-zygotes) and approximately 10 T1 seedlings lacking the transgene (nullizygotes) were selected by monitoring visual marker expression. The transgenic plants and the corresponding nullizygotes were grown side-by-side at random positions. Greenhouse conditions were of shorts days (12 hours light), 28°C in the light and 22°C in the dark, and a relative humidity of 70%.

Four T1 events were further evaluated in the T2 generation following the same evaluation procedure as for the T1 generation but with more individuals per event. From the stage of sowing until the stage of maturity the plants were passed several times through a digital imaging cabinet. At each time point digital images (2048x1536 pixels, 16 million colours) were taken of each plant from at least 6 different angles.

*Drought screen*

Plants from T2 seeds are grown in potting soil under normal conditions until they approached the heading stage. They are then transferred to a "dry" section where irrigation is withheld. Humidity probes are inserted in randomly chosen pots to monitor the soil water content (SWC). When SWC goes below certain thresholds, the plants are automatically re-watered continuously until a normal level is reached again. The plants are then re-transferred again to normal conditions. The rest of the cultivation (plant maturation, seed harvest) is the same as for plants not grown under abiotic stress conditions. Growth and yield parameters are recorded as detailed for growth under normal conditions.

*Nitrogen use efficiency screen*

Rice plants from T2 seeds are grown in potting soil under normal conditions except for the nutrient solution. The pots are watered from transplantation to maturation with a specific nutrient solution containing reduced N nitrogen (N) content, usually between 7 to 8 times less. The rest of the cultivation (plant maturation, seed harvest) is the same as for plants not grown under abiotic stress. Growth and yield parameters are recorded as detailed for growth under normal conditions.

*Salt stress screen*

Plants are grown on a substrate made of coco fibers and argex (3 to 1 ratio). A normal nutrient solution is used during the first two weeks after transplanting the plantlets in the greenhouse. After the first two weeks, 25 mM of salt (NaCl) is added to the nutrient solution, until the plants were harvested. Seed-related parameters are then measured.

9.2 Statistical analysis: F test

A two factor ANOVA (analysis of variants) was used as a statistical model for the overall evaluation of plant phenotypic characteristics. An F test was carried out on all the parameters measured of all the plants of all the events transformed with the gene of the present invention. The F test was carried out to check for an effect of the gene over all the transformation events and to verify for an overall effect of the gene, also known as a global gene effect. The threshold for significance for a true global gene effect was set at a 5% probability level for the F test. A significant F test value points to a gene effect, meaning that it is not only the mere presence or position of the gene that is causing the differences in phenotype.

Because two experiments with overlapping events were carried out, a combined analysis was performed. This is useful to check consistency of the effects over the two experiments, and if this is the case, to accumulate evidence from both experiments in order to increase confidence in the conclusion. The method used was a mixed-model approach that takes into account the multilevel structure of the data (i.e. experiment - event - segregants). P values were obtained by comparing likelihood ratio test to chi square distributions.

9.3 Parameters measured

*Biomass-related parameter measurement*

From the stage of sowing until the stage of maturity the plants were passed several times through a digital imaging cabinet. At each time point digital images (2048x1536 pixels, 16 million colours) were taken of each plant from at least 6 different angles.

The plant aboveground area (or leafy biomass) was determined by counting the total number of pixels on the digital images from aboveground plant parts discriminated from the background. This value was averaged for the pictures taken on the same time point from the different angles and was converted to a physical surface value expressed in square mm by calibration. Experiments show that the aboveground plant area measured this way correlates with the biomass of plant parts above ground. The above ground area is the area measured at the time point at which the plant had reached its maximal leafy biomass. The early vigour is the plant (seedling) aboveground area three weeks post-germination.

Early vigour was determined by counting the total number of pixels from aboveground plant parts discriminated from the background. This value was averaged for the pictures taken on the same time point from different angles and was converted to a physical surface value expressed in square mm by calibration. The results described below are for plants three weeks post-germination.

*Seed-related parameter measurements*

The mature primary panicles were harvested, counted, bagged, barcode-labelled and then dried for three days in an oven at 37°C. The panicles were then threshed and all the seeds were collected and counted. The filled husks were separated from the empty ones using an air-blowing device. The empty husks were discarded and the remaining fraction was counted again. The filled husks were weighed on an analytical balance. The number of filled seeds was determined by counting the number of filled husks that remained after the separation step. The total seed yield was measured by weighing all filled husks harvested from a plant. Total seed number per plant was measured by counting the number of husks harvested from a plant. Thousand Kernel Weight (TKW) is extrapolated from the number of filled seeds counted and their total weight. The Harvest Index (HI) in the present invention is defined as the ratio between the total seed yield and the above ground area ($mm^2$), multiplied by a factor $10^6$. The total number of flowers per panicle as defined in the present invention is the ratio between the total number of seeds and the number of mature primary panicles. The seed fill rate as defined in the present invention is the proportion (expressed as a %) of the number of filled seeds over the total number of seeds (or florets). Number of flowers per panicle was calculated from the number of total seeds and the number of first panicles and is an estimation of the average number of florets per panicle on a plant.

***Example 10: Results of the phenotypic evaluation of the transgenic plants***

Transgenic rice plants expressing the bHLH6-like protein of SEQ ID NO: 2 grown under normal growth conditions showed compared to the control plants, an increase in emergence vigour and in seed yield (as demonstrated by number of flowers per panicle, see table below). These increases were measured in 2 experiments with T1 and T2 generation plants and were statistically significant.

| | **T1 generation** | **T2 generation** |
|---|---|---|
| **parameter** | **% increase** | **% increase** |
| Emergence vigour | 11.9 | 2.8 |
| number of flowers per panicle | 7.4 | 6.2 |

Furthermore positive effects were observed for total weight of seeds, number of filled seeds and harvest index, in the T1 generation as well as in T2 generation.

***Example 11: Cloning of the nucleic acid sequence used in the methods* of *the invention***

Cloning of SEQ ID NO: 57 (coding for RrmJ/FtsJ methyltransferase):

A cDNA-AFLP experiment performed on a synchronized tobacco BY2 cell culture (Nicotiana tabacum L. cv. Bright Yellow-2), and BY2 expressed sequence tags that were cell cycle modulated could be identified and were elected for further cloning. The expressed sequence tags were used to screen a tobacco cDNA library (in a Gateway compatible vector; Invitrogen, Paisley, UK) to isolate the full-length cDNA of interest, namely one coding for RrmJ/FtsJ methyltransferase nucleic acid sequence as represented by SEQ ID NO: 57.

The isolated plasmid comprising SEQ ID NO: 57 was then used in an LR reaction with a destination vector used for *Oryza sativa* transformation. This vector contained as functional elements within the T-DNA borders: a plant selectable marker; a screenable marker expression cassette; and a Gateway cassette intended for LR *in vivo* recombination with the nucleic acid sequence of interest already cloned in the entry clone. A rice oleosin promoter (SEQ ID NO: 91) for seed specific expression was located upstream of this Gateway cassette.

After the LR recombination step, the resulting expression vector pOleo::GRP (Figure 6) was transformed into *Agrobacterium* strain LBA4044 according to methods well known in the art.

### *Example 12: Plant transformation*

*Rice transformation*

The *Agrobacterium* containing the expression vector was used to transform Oryza *sativa* plants. Mature dry seeds of the rice japonica cultivar Nipponbare were dehusked. Sterilization was carried out by incubating for one minute in 70% ethanol, followed by 30 minutes in 0.2% $HgCl_2$, followed by a 6 times 15 minutes wash with sterile distilled water. The sterile seeds were then germinated on a medium containing 2,4-D (callus induction medium). After incubation in the dark for four weeks, embryogenic, scutellum-derived calli were excised and propagated on the same medium. After two weeks, the calli were multiplied or propagated by subculture on the same medium for another 2 weeks. Embryogenic callus pieces were subcultured on fresh medium 3 days before co-cultivation (to boost cell division activity).

*Agrobacterium* strain LBA4404 containing the expression vector was used for co-cultivation. *Agrobacterium* was inoculated on AB medium with the appropriate antibiotics and cultured for 3 days at 28°C. The bacteria were then collected and suspended in liquid co-cultivation medium to a density ($OD_{600}$) of about 1. The suspension was then transferred to a Petri dish and the calli immersed in the suspension for 15 minutes. The callus tissues were then blotted dry on a filter paper and transferred to solidified, cocultivation medium and incubated for 3 days in the dark at 25°C. Co-cultivated calli were grown on 2,4-D-containing medium for 4 weeks in the dark at 28°C in the presence of a selection agent. During this period, rapidly growing resistant callus islands developed. After transfer of this material to a regeneration medium and incubation in the light, the embryogenic potential was released and shoots developed in the next four to five weeks. Shoots were excised from the calli and incubated for 2 to 3 weeks on an auxin-containing medium from which they were transferred to soil. Hardened shoots were grown under high humidity and short days in a greenhouse.

Approximately 35 independent T0 rice transformants were generated for one construct. The primary transformants were transferred from a tissue culture chamber to a greenhouse. After a quantitative PCR analysis to verify copy number of the T-DNA insert, only single copy transgenic plants that exhibit tolerance to the selection agent were kept for harvest of T1 seed. Seeds were then harvested three to five months after transplanting. The method yielded single locus transformants at a rate of over 50 % (Aldemita and Hodges1996, Chan et al. 1993, Hiei et al. 1994).

### *Example 13: Phenotypic evaluation procedure*

<u>13.1 Evaluation setup</u>

Approximately 35 independent T0 rice transformants were generated. The primary transformants were transferred from a tissue culture chamber to a greenhouse for growing and harvest of T1 seed. Six events, of which the T1 progeny segregated 3:1 for presence/absence of the transgene, were retained. For each of these events, approximately 10 T1 seedlings containing the transgene (hetero- and homo-zygotes) and approximately 10 T1 seedlings lacking the transgene (nullizygotes) were selected by monitoring visual marker expression. The transgenic plants and the corresponding nullizygotes were grown side-by-side at random positions. Greenhouse conditions were of shorts days (12 hours light), 28°C in the light and 22°C in the dark, and a relative humidity of 70%.

Four T1 events were further evaluated in the T2 generation following the same evaluation procedure as for the T1 generation but with more individuals per event.

From the stage of sowing until the stage of maturity the plants were passed several times through a digital imaging cabinet. At each time point digital images (2048x1536 pixels, 16 million colours) were taken of each plant from at least 6 different angles.

*Drought screen*

Plants from T2 seeds are grown in potting soil under normal conditions until they approached the heading stage. They are then transferred to a "dry" section where irrigation is withheld. Humidity probes are inserted in randomly chosen pots to monitor the soil water content (SWC). When SWC goes below certain thresholds, the plants are automatically re-watered continuously until a normal level is reached again. The plants are then re-transferred again to normal conditions. The rest of the cultivation (plant maturation, seed harvest) is the same as for plants not grown under abiotic stress conditions. Growth and yield parameters are recorded as detailed for growth under normal conditions.

*Nitrogen use efficiency screen*

Rice plants from T2 seeds are grown in potting soil under normal conditions except for the nutrient solution. The pots are watered from transplantation to maturation with a specific nutrient solution containing reduced N nitrogen (N) content, usually between 7 to 8 times less. The rest of the cultivation (plant maturation, seed harvest) is the same as for plants not grown under abiotic stress. Growth and yield parameters are recorded as detailed for growth under normal conditions.

13.2 Statistical analysis: F test

A two factor ANOVA (analysis of variants) was used as a statistical model for the overall evaluation of plant phenotypic characteristics. An F test was carried out on all the parameters measured of all the plants of all the events transformed with the gene of the present invention. The F test was carried out to check for an effect of the gene over all the transformation events and to verify for an overall effect of the gene, also known as a global gene effect. The threshold for significance for a true global gene effect was set at a 5% probability level for the F test. A significant F test value points to a gene effect, meaning that it is not only the mere presence or position of the gene that is causing the differences in phenotype.

Because two experiments with overlapping events were carried out, a combined analysis is performed. This is useful to check consistency of the effects over the two experiments, and if this is the case, to accumulate evidence from both experiments in order to increase confidence in the conclusion. The method used is a mixed-model approach that takes into account the multilevel structure of the data (i.e. experiment - event - segregants). P values were obtained by comparing likelihood ratio test to chi square distributions.

13.3 Parameters measured

*Biomass-related parameter measurement*

The plant aboveground area (or leafy biomass) was determined by counting the total number of pixels on the digital images from aboveground plant parts discriminated from the background. This value was averaged for the pictures taken on the same time point from the different angles and was converted to a physical surface value expressed in square mm by calibration. Experiments show that the aboveground plant area measured this way correlates with the biomass of plant parts above ground. The above ground area is the area measured at the time point at which the plant had reached its maximal leafy biomass.

*Seed-related parameter measurements*

The mature primary panicles were harvested, counted, bagged, barcode-labelled and then dried for three days in an oven at 37°C. The panicles were then threshed and all the seeds were collected and counted. The filled husks were separated from the empty ones using an air-blowing device. The empty husks were discarded and the remaining fraction was counted again. The filled husks were weighed on an analytical balance. The number of filled seeds was determined by counting the number of filled husks that remained after the separation step. The total seed yield was measured by weighing all filled husks harvested from a plant. Total seed number per plant was measured by counting the number of husks harvested from a plant. Thousand Kernel Weight (TKW) is extrapolated from the number of filled seeds counted and their total weight. The Harvest Index (HI) in the present invention is defined as the ratio between the total seed yield and the above ground area ($mm^2$), multiplied by a factor $10^6$. The total number of flowers per panicle as defined in the present invention is the ratio between the total number of seeds and the number of mature primary panicles. The seed fill rate as defined in the present invention is the proportion (expressed as a %) of the number of filled seeds over the total number of seeds (or florets).

***Example 14: Results of the phenotypic evaluation* of *the transgenic plants***

The transgenic rice plants expressing the *GRP* nucleic acid sequence represented by SEQ ID NO: 57 under control of the oleosin promoter showed an increase of more than 5% for total weight of seeds, number of filled seeds, fill rate, and harvest index.

| | **Average % increase in 2 events in the T1 generation** | **Average % increase in 2 events in the T2 generation** |
|---|---|---|
| Total seed yield per plant | 48% | 46% |
| Total number of filled seeds | 46% | 44% |
| Fill rate | 39% | 45% |

(continued)

| | Average % increase in 2 events in the T1 generation | Average % increase in 2 events in the T2 generation |
|---|---|---|
| Harvest index | 40% | 40% |

***Example 15: Other crop transformation***

*Corn transformation*

Transformation of maize *(Zea mays)* is performed with a modification of the method described by Ishida et al. (1996) Nature Biotech 14(6): 745-50. Transformation is genotype-dependent in corn and only specific genotypes are amenable to transformation and regeneration. The inbred line A188 (University of Minnesota) or hybrids with A188 as a parent are good sources of donor material for transformation, but other genotypes can be used successfully as well. Ears are harvested from corn plant approximately 11 days after pollination (DAP) when the length of the immature embryo is about 1 to 1.2 mm. Immature embryos are cocultivated with *Agrobacterium tumefaciens* containing the expression vector, and transgenic plants are recovered through organogenesis. Excised embryos are grown on callus induction medium, then maize regeneration medium, containing the selection agent (for example imidazolinone but various selection markers can be used). The Petri plates are incubated in the light at 25 °C for 2-3 weeks, or until shoots develop. The green shoots are transferred from each embryo to maize rooting medium and incubated at 25 °C for 2-3 weeks, until roots develop. The rooted shoots are transplanted to soil in the greenhouse. T1 seeds are produced from plants that exhibit tolerance to the selection agent and that contain a single copy of the T-DNA insert.

*Wheat transformation*

Transformation of wheat is performed with the method described by Ishida et al. (1996) Nature Biotech 14(6): 745-50. The cultivar Bobwhite (available from CIMMYT, Mexico) is commonly used in transformation. Immature embryos are co-cultivated with *Agrobacterium tumefaciens* containing the expression vector, and transgenic plants are recovered through organogenesis. After incubation with *Agrobacterium,* the embryos are grown in vitro on callus induction medium, then regeneration medium, containing the selection agent (for example imidazolinone but various selection markers can be used). The Petri plates are incubated in the light at 25 °C for 2-3 weeks, or until shoots develop. The green shoots are transferred from each embryo to rooting medium and incubated at 25 °C for 2-3 weeks, until roots develop. The rooted shoots are transplanted to soil in the greenhouse. T1 seeds are produced from plants that exhibit tolerance to the selection agent and that contain a single copy of the T-DNA insert.

*Soybean transformation*

Soybean is transformed according to a modification of the method described in the Texas A&M patent US 5,164,310. Several commercial soybean varieties are amenable to transformation by this method. The cultivar Jack (available from the Illinois Seed foundation) is commonly used for transformation. Soybean seeds are sterilised for *in vitro* sowing. The hypocotyl, the radicle and one cotyledon are excised from seven-day old young seedlings. The epicotyl and the remaining cotyledon are further grown to develop axillary nodes. These axillary nodes are excised and incubated with *Agrobacterium tumefaciens* containing the expression vector. After the cocultivation treatment, the explants are washed and transferred to selection media. Regenerated shoots are excised and placed on a shoot elongation medium. Shoots no longer than 1 cm are placed on rooting medium until roots develop. The rooted shoots are transplanted to soil in the greenhouse. T1 seeds are produced from plants that exhibit tolerance to the selection agent and that contain a single copy of the T-DNA insert.

*Rapeseed/canola transformation*

Cotyledonary petioles and hypocotyls of 5-6 day old young seedling are used as explants for tissue culture and transformed according to Babic et al. (1998, Plant Cell Rep 17: 183-188). The commercial cultivar Westar (Agriculture Canada) is the standard variety used for transformation, but other varieties can also be used. Canola seeds are surface-sterilized for in vitro sowing. The cotyledon petiole explants with the cotyledon attached are excised from the in vitro seedlings, and inoculated with *Agrobacterium* (containing the expression vector) by dipping the cut end of the petiole explant into the bacterial suspension. The explants are then cultured for 2 days on MSBAP-3 medium containing 3 mg/l BAP, 3 % sucrose, 0.7 % Phytagar at 23 °C, 16 hr light. After two days of co-cultivation with *Agrobacterium,* the petiole explants are transferred to MSBAP-3 medium containing 3 mg/l BAP, cefotaxime, carbenicillin, or timentin (300 mg/l) for 7 days, and then cultured on MSBAP-3 medium with cefotaxime, carbenicillin, or timentin and selection agent until shoot regeneration. When the shoots are 5 - 10 mm in length, they are cut and transferred to shoot elongation medium (MSBAP-0.5, containing 0.5 mg/l BAP). Shoots of about 2 cm in length are transferred to the rooting medium (MS0) for root induction. The rooted shoots are transplanted to soil in the greenhouse. T1 seeds are produced from plants that exhibit tolerance to the selection agent and that contain a single copy of the T-DNA insert.

*Alfalfa transformation*

A regenerating clone of alfalfa *(Medicago sativa)* is transformed using the method of (McKersie et al., 1999 Plant Physiol 119: 839-847). Regeneration and transformation of alfalfa is genotype dependent and therefore a regenerating plant is required. Methods to obtain regenerating plants have been described. For example, these can be selected from the cultivar Rangelander (Agriculture Canada) or any other commercial alfalfa variety as described by Brown DCW and A Atanassov (1985. Plant Cell Tissue Organ Culture 4: 111-112). Alternatively, the RA3 variety (University of Wisconsin) has been selected for use in tissue culture (Walker et al., 1978 Am J Bot 65:654-659). Petiole explants are cocultivated with an overnight culture of *Agrobacterium tumefaciens* C58C1 pMP90 (McKersie et al., 1999 Plant Physiol 119: 839-847) or LBA4404 containing the expression vector. The explants are cocultivated for 3 d in the dark on SH induction medium containing 288 mg/ L Pro, 53 mg/ L thioproline, 4.35 g/ L K2S04, and 100 μm acetosyringinone. The explants are washed in half-strength Murashige-Skoog medium (Murashige and Skoog, 1962) and plated on the same SH induction medium without acetosyringinone but with a suitable selection agent and suitable antibiotic to inhibit *Agrobacterium* growth. After several weeks, somatic embryos are transferred to BOi2Y development medium containing no growth regulators, no antibiotics, and 50 g/ L sucrose. Somatic embryos are subsequently germinated on half-strength Murashige-Skoog medium. Rooted seedlings were transplanted into pots and grown in a greenhouse. T1 seeds are produced from plants that exhibit tolerance to the selection agent and that contain a single copy of the T-DNA insert.

**Cotton transformation**

Cotton *(Gossypium hirsutum* L.) transformation is performed using *Agrobacterium tumefaciens,* on hypocotyls explants. The commercial cultivars such as Coker 130 or Coker 312 (SeedCo, Lubbock, TX) are standard varieties used for transformation, but other varieties can also be used. The seeds are surface sterilized and germinated in the dark. Hypocotyl explants are cut from the germinated seedlings to lengths of about 1-1.5 centimeter. The hypotocyl explant is submersed in the *Agrobacterium tumefaciens* inoculum containing the expression vector, for 5 minutes then co-cultivated for about 48 hours on MS +1.8 mg/l KNO3 + 2% glucose at 24° C, in the dark. The explants are transferred the same medium containing appropriate bacterial and plant selectable markers (renewed several times), until embryogenic calli is seen. The calli are separated and subcultured until somatic embryos appear. Plantlets derived from the somatic embryos are matured on rooting medium until roots develop. The rooted shoots are transplanted to potting soil in the greenhouse. T1 seeds are produced from plants that exhibit tolerance to the selection agent and that contain a single copy of the T-DNA insert.

***Example 16: Cloning* of *the nucleic acid sequence used in the methods* of *the invention***

Cloning of SEQ ID NO: 104 (encoding a GRP, wherein said GRP polypeptide is a basic-helix-loop-helix4 (bHLH4) polypeptide):

The nucleic acid sequence SEQ ID NO: 104 used in the methods of the invention was amplified by PCR using as template a custom-made Arabidopsis thaliana mixed tissues cDNA library (in pCMV Sport 6.0; Invitrogen, Paisley, UK). PCR was performed using Hifi Taq DNA polymerase in standard conditions, using 200 ng of template in a 50 μl PCR mix. The primers used were prm06763 (SEQ ID NO: 107; sense:

5'ggggacaagtttgtacaaaaaagcaggcttaaacaatggctccgacgaatgtt 3'
and prm06764 (SEQ ID NO: 108; reverse, complementary):

5' ggggaccactttgtacaagaaagctgggttgctgacttcaattcatggac 3',
which include the AttB sites for Gateway recombination. The amplified PCR fragment was purified also using standard methods. The first step of the Gateway procedure, the BP reaction, was then performed, during which the PCR fragment recombines in vivo with the pDONR201 plasmid to produce, according to the Gateway terminology, an "entry clone". Plasmid pDONR201 was purchased from Invitrogen, as part of the Gateway® technology.

The entry clone comprising SEQ ID NO: 104 was then used in an LR reaction with a destination vector used for Oryza *sativa* transformation. This vector contained as functional elements within the T-DNA borders: a plant selectable marker; a screenable marker expression cassette; and a Gateway cassette intended for LR *in vivo* recombination with the nucleic acid sequence of interest already cloned in the entry clone. A rice HMGB promoter (SEQ ID NO: 106) for constitutive expression was located upstream of this Gateway cassette.

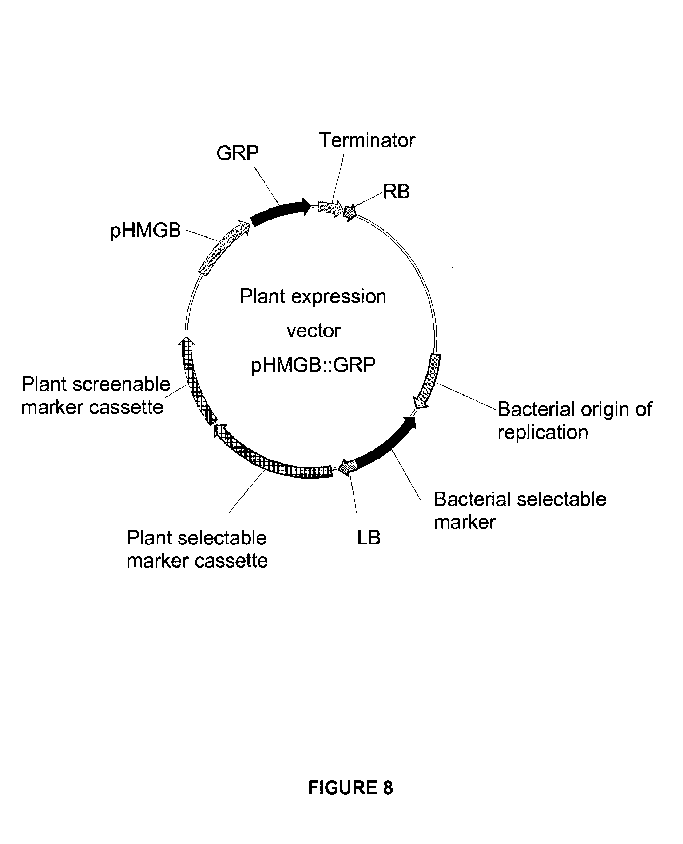

After the LR recombination step, the resulting expression vector pHMGB::GRP (Figure 8) was transformed into *Agrobacterium* strain LBA4044 according to methods well known in the art.

### *Example 17: Plant transformation*

*Rice transformation*

The *Agrobacterium* containing the expression vector was used to transform Oryza *sativa* plants. Mature dry seeds of the rice japonica cultivar Nipponbare were dehusked. Sterilization was carried out by incubating for one minute in 70% ethanol, followed by 30 minutes in 0.2% $HgCl_2$, followed by a 6 times 15 minutes wash with sterile distilled water. The sterile seeds were then germinated on a medium containing 2,4-D (callus induction medium). After incubation in the dark for four weeks, embryogenic, scutellum-derived calli were excised and propagated on the same medium. After two weeks, the calli were multiplied or propagated by subculture on the same medium for another 2 weeks. Embryogenic callus pieces were subcultured on fresh medium 3 days before co-cultivatin (to boost cell division activity).

*Agrobacterium* strain LBA4404 containing the expression vector was used for co-cultivation. *Agrobacterium* was inoculated on AB medium with the appropriate antibiotics and cultured for 3 days at 28°C. The bacteria were then collected and suspended in liquid co-cultivation medium to a density ($OD_{600}$) of about 1. The suspension was then transferred to a Petri dish and the calli immersed in the suspension for 15 minutes. The callus tissues were then blotted dry on a filter paper and transferred to solidified, co-cultivation medium and incubated for 3 days in the dark at 25°C. Co-cultivated calli were grown on 2,4-D-containing medium for 4 weeks in the dark at 28°C in the presence of a selection agent. During this period, rapidly growing resistant callus islands developed. After transfer of this material to a regeneration medium and incubation in the light, the embryogenic potential was released and shoots developed in the next four to five weeks. Shoots were excised from the calli and incubated for 2 to 3 weeks on an auxin-containing medium from which they were transferred to soil. Hardened shoots were grown under high humidity and short days in a greenhouse.

Approximately 35 independent T0 rice transformants were generated for one construct. The primary transformants were transferred from a tissue culture chamber to a greenhouse. After a quantitative PCR analysis to verify copy number of the T-DNA insert, only single copy transgenic plants that exhibit tolerance to the selection agent were kept for harvest of T1 seed. Seeds were then harvested three to five months after transplanting. The method yielded single locus transformants at a rate of over 50 % (Aldemita and Hodges1996, Chan et al. 1993, Hiei et al. 1994).

### *Example 18: Phenotypic evaluation procedure*

18.1 Evaluation setup

Approximately 35 independent T0 rice transformants were generated. The primary transformants were transferred from a tissue culture chamber to a greenhouse for growing and harvest of T1 seed. Five events, of which the T1 progeny segregated 3:1 for presence/absence of the transgene, were retained. For each of these events, approximately 10 T1 seedlings containing the transgene (hetero- and homo-zygotes) and approximately 10 T1 seedlings lacking the transgene (nullizygotes) were selected by monitoring visual marker expression. The transgenic plants and the corresponding nullizygotes were grown side-by-side at random positions. Greenhouse conditions were of shorts days (12 hours light), 28°C in the light and 22°C in the dark, and a relative humidity of 70%.

Four T1 events were further evaluated in the T2 generation following the same evaluation procedure as for the T1 generation but with more individuals per event.

From the stage of sowing until the stage of maturity the plants were passed several times through a digital imaging cabinet. At each time point digital images (2048x1536 pixels, 16 million colours) were taken of each plant from at least 6 different angles.

18.2 Statistical analysis: F test

A two factor ANOVA (analysis of variants) was used as a statistical model for the overall evaluation of plant phenotypic characteristics. An F test was carried out on all the parameters measured of all the plants of all the events transformed with the gene of the present invention. The F test was carried out to check for an effect of the gene over all the transformation events and to verify for an overall effect of the gene, also known as a global gene effect. The threshold for significance for a true global gene effect was set at a 5% probability level for the F test. A significant F test value points to a gene effect, meaning that it is not only the mere presence or position of the gene that is causing the differences in phenotype.

Because two experiments with overlapping events were carried out, a combined analysis is performed. This is useful to check consistency of the effects over the two experiments, and if this is the case, to accumulate evidence from both experiments in order to increase confidence in the conclusion. The method used is a mixed-model approach that takes into account the multilevel structure of the data (i.e. experiment - event - segregants). P values were obtained by comparing likelihood ratio test to chi square distributions.

18.3 Parameters measured

*Biomass-related parameter measurement*

The plant aboveground area (or leafy biomass) was determined by counting the total number of pixels on the digital images from aboveground plant parts discriminated from the background. This value was averaged for the pictures taken on the same time point from the different angles and was converted to a physical surface value expressed in square mm by calibration. Experiments show that the aboveground plant area measured this way correlates with the biomass of plant parts above ground. The above ground area is the area measured at the time point at which the plant had reached its maximal leafy biomass.

*Seed-related parameter measurements*

The mature primary panicles were harvested, counted, bagged, barcode-labelled and then dried for three days in an oven at 37°C. The panicles were then threshed and all the seeds were collected and counted. The filled husks were separated from the empty ones using an air-blowing device. The empty husks were discarded and the remaining fraction was counted again. The filled husks were weighed on an analytical balance. The number of filled seeds was determined by counting the number of filled husks that remained after the separation step. The total seed yield was measured by weighing all filled husks harvested from a plant. Total seed number per plant was measured by counting the number of husks harvested from a plant. Thousand Kernel Weight (TKW) is extrapolated from the number of filled seeds counted and their total weight. The Harvest Index (HI) in the present invention is defined as the ratio between the total seed yield and the above ground area ($mm^2$), multiplied by a factor $10^6$. The total number of flowers per panicle as defined in the present invention is the ratio between the total number of seeds and the number of mature primary panicles. The seed fill rate as defined in the present invention is the proportion (expressed as a %) of the number of filled seeds over the total number of seeds (or florets).

***Example 19: Results of the phenotypic evaluation of the transgenic plants***

The transgenic rice plants expressing the GRP nucleic acid sequence represented by SEQ ID NO: 104 under control of the HMGB promoter showed an increase of more than 5% for total weight of seeds, number of filled seeds, fill rate, and harvest index.

| | Overall average % increase in 5 events in the T1 generation | Average % increase in 2 events in the T2 generation |
|---|---|---|
| Early vigour | 21% | 7% |
| Total seed yield per plant | 23% | 27% |
| Total number of filled seeds | 22% | 28% |
| Seed fill rate | 12% | 15% |
| Harvest index | 18% | 17% |
| Greenness index | 4% | 9% |

***Example 20: Other crop transformation***

*Corn transformation*

Transformation of maize *(Zea mays)* is performed with a modification of the method described by Ishida et al. (1996) Nature Biotech 14(6): 745-50. Transformation is genotype-dependent in corn and only specific genotypes are amenable to transformation and regeneration. The inbred line A188 (University of Minnesota) or hybrids with A188 as a parent are good sources of donor material for transformation, but other genotypes can be used successfully as well. Ears are harvested from corn plant approximately 11 days after pollination (DAP) when the length of the immature embryo is about 1 to 1.2 mm. Immature embryos are cocultivated with *Agrobacterium tumefaciens* containing the expression vector, and transgenic plants are recovered through organogenesis. Excised embryos are grown on callus induction medium, then maize regeneration medium, containing the selection agent (for example imidazolinone but various selection markers can be used). The Petri plates are incubated in the light at 25 °C for 2-3 weeks, or until shoots develop. The green shoots are transferred from each embryo to maize rooting medium and incubated at 25 °C for 2-3 weeks, until roots develop. The rooted shoots are transplanted to soil in the greenhouse. T1 seeds are produced from plants that exhibit tolerance to the selection agent and that contain a single copy of the T-DNA insert.

*Wheat transformation*

Transformation of wheat is performed with the method described by Ishida et al. (1996) Nature Biotech 14(6): 745-50. The cultivar Bobwhite (available from CIMMYT, Mexico) is commonly used in transformation. Immature embryos are co-cultivated with *Agrobacterium tumefaciens* containing the expression vector, and transgenic plants are recovered through organogenesis. After incubation with *Agrobacterium,* the embryos are grown in vitro on callus induction medium, then regeneration medium, containing the selection agent (for example imidazolinone but various selection markers can be used). The Petri plates are incubated in the light at 25 °C for 2-3 weeks, or until shoots develop. The green shoots are transferred from each embryo to rooting medium and incubated at 25 °C for 2-3 weeks, until roots develop. The rooted shoots are transplanted to soil in the greenhouse. T1 seeds are produced from plants that exhibit tolerance to the selection agent and that contain a single copy of the T-DNA insert.

*Soybean transformation*

Soybean is transformed according to a modification of the method described in the Texas A&M patent US 5,164,310. Several commercial soybean varieties are amenable to transformation by this method. The cultivar Jack (available from the Illinois Seed foundation) is commonly used for transformation. Soybean seeds are sterilised for *in vitro* sowing. The hypocotyl, the radicle and one cotyledon are excised from seven-day old young seedlings. The epicotyl and the remaining cotyledon are further grown to develop axillary nodes. These axillary nodes are excised and incubated with *Agrobacterium tumefaciens* containing the expression vector. After the cocultivation treatment, the explants are washed and transferred to selection media. Regenerated shoots are excised and placed on a shoot elongation medium. Shoots no longer than 1 cm are placed on rooting medium until roots develop. The rooted shoots are transplanted to soil in the greenhouse. T1 seeds are produced from plants that exhibit tolerance to the selection agent and that contain a single copy of the T-DNA insert.

*Rapeseed/canola transformation*

Cotyledonary petioles and hypocotyls of 5-6 day old young seedling are used as explants for tissue culture and transformed according to Babic et al. (1998, Plant Cell Rep 17: 183-188). The commercial cultivar Westar (Agriculture Canada) is the standard variety used for transformation, but other varieties can also be used. Canola seeds are surface-sterilized for in vitro sowing. The cotyledon petiole explants with the cotyledon attached are excised from the in vitro seedlings, and inoculated with *Agrobacterium* (containing the expression vector) by dipping the cut end of the petiole explant into the bacterial suspension. The explants are then cultured for 2 days on MSBAP-3 medium containing 3 mg/l BAP, 3 % sucrose, 0.7 % Phytagar at 23 °C, 16 hr light. After two days of co-cultivation with *Agrobacterium,* the petiole explants are transferred to MSBAP-3 medium containing 3 mg/l BAP, cefotaxime, carbenicillin, or timentin (300 mg/l) for 7 days, and then cultured on MSBAP-3 medium with cefotaxime, carbenicillin, or timentin and selection agent until shoot regeneration. When the shoots are 5 - 10 mm in length, they are cut and transferred to shoot elongation medium (MSBAP-0.5, containing 0.5 mg/l BAP). Shoots of about 2 cm in length are transferred to the rooting medium (MS0) for root induction. The rooted shoots are transplanted to soil in the greenhouse. T1 seeds are produced from plants that exhibit tolerance to the selection agent and that contain a single copy of the T-DNA insert.

*Alfalfa transformation*

A regenerating clone of alfalfa *(Medicago sativa)* is transformed using the method of (McKersie et al., 1999 Plant Physiol 119: 839-847). Regeneration and transformation of alfalfa is genotype dependent and therefore a regenerating plant is required. Methods to obtain regenerating plants have been described. For example, these can be selected from the cultivar Rangelander (Agriculture Canada) or any other commercial alfalfa variety as described by Brown DCW and A Atanassov (1985. Plant Cell Tissue Organ Culture 4: 111-112). Alternatively, the RA3 variety (University of Wisconsin) has been selected for use in tissue culture (Walker et al., 1978 Am J Bot 65:654-659). Petiole explants are cocultivated with an overnight culture of *Agrobacterium tumefaciens* C58C1 pMP90 (McKersie et al., 1999 Plant Physiol 119: 839-847) or LBA4404 containing the expression vector. The explants are cocultivated for 3 d in the dark on SH induction medium containing 288 mg/ L Pro, 53 mg/ L thioproline, 4.35 g/ L K2SO4, and 100 μm acetosyringinone. The explants are washed in half-strength Murashige-Skoog medium (Murashige and Skoog, 1962) and plated on the same SH induction medium without acetosyringinone but with a suitable selection agent and suitable antibiotic to inhibit *Agrobacterium* growth. After several weeks, somatic embryos are transferred to BOi2Y development medium containing no growth regulators, no antibiotics, and 50 g/ L sucrose. Somatic embryos are subsequently germinated on half-strength Murashige-Skoog medium. Rooted seedlings were transplanted into pots and grown in a greenhouse. T1 seeds are produced from plants that exhibit tolerance to the selection agent and that contain a single copy of the T-DNA insert.

**Cotton transformation**

Cotton (*Gossypium hirsutum* L.) transformation is performed using *Agrobacterium tumefaciens,* on hypocotyls explants. The commercial cultivars such as Coker 130 or Coker 312 (SeedCo, Lubbock, TX) are standard varieties used for transformation, but other varieties can also be used. The seeds are surface sterilized and germinated in the dark. Hypocotyl explants are cut from the germinated seedlings to lengths of about 1-1.5 centimeter. The hypotocyl explant is submersed in the *Agrobacterium tumefaciens* inoculum containing the expression vector, for 5 minutes then co-cultivated for about 48 hours on MS +1.8 mg/l KNO3 + 2% glucose at 24° C, in the dark. The explants are transferred the same medium containing appropriate bacterial and plant selectable markers (renewed several times), until embryogenic calli is seen. The calli are separated and subcultured until somatic embryos appear. Plantlets derived from the somatic embryos are matured on rooting medium until roots develop. The rooted shoots are transplanted to potting soil in the greenhouse. T1 seeds are produced from plants that exhibit tolerance to the selection agent and that contain a single copy of the T-DNA insert.

***Example 21: Abiotic stress screens***

*Drought screen*

Rice plants from T1, T2 or further generations are grown in potting soil under normal conditions until they approached the heading stage. They are then transferred to a "dry" section where irrigation is withheld. Humidity probes are inserted in randomly chosen pots to monitor the soil water content (SWC). When SWC goes below certain thresholds, the plants are automatically re-watered continuously until a normal level is reached again. The plants are then re-transferred again to normal conditions. The rest of the cultivation (plant maturation, seed harvest) is the same as for plants not grown under abiotic stress conditions. Growth and yield parameters are recorded as detailed for growth under normal conditions.

*Salt stress screen*

Rice plants from T1, T2 or further generations are grown on a substrate made of coco fibers and argex (3 to 1 ratio). A normal nutrient solution is used during the first two weeks after transplanting the plantlets in the greenhouse. After the first two weeks, 25 mM of salt (NaCl) is added to the nutrient solution, until the plants are harvested. Growth and yield parameters are recorded as detailed for growth under normal conditions.

*Nitrogen use efficiency screen*

Rice plants from T1, T2 or further generations are grown in potting soil under normal conditions except for the nutrient solution. The pots are watered from transplantation to maturation with a specific nutrient solution containing reduced N nitrogen (N) content, usually between 7 to 8 times less. The rest of the cultivation (plant maturation, seed harvest) is the same as for plants not grown under abiotic stress. Growth and yield parameters are recorded as detailed for growth under normal conditions.

***Example 22: Cloning of the nucleic acid sequence used in the methods of the invention***

Cloning of SEQ ID NO: 131 (encoding a GRP, wherein said GRP polypeptide is an isopentenyl transferase (IPT) polypeptide):

The nucleic acid sequence SEQ ID NO: 131 used in the methods of the invention was amplified by PCR using as template a custom-made Arabidopsis thaliana mixed tissues cDNA library (in pCMV Sport 6.0; Invitrogen, Paisley, UK). PCR was performed using Hifi Taq DNA polymerase in standard conditions, using 200 ng of template in a 50 μl PCR mix. The primers used were prm03095 (SEQ ID NO: 134; sense:

5' ggggacaagtttgtacaaaaaagcaggcttcacaatgacagaactcaacttccac 3'
and prm03096 (SEQ ID NO: 135; reverse, complementary):

5' ggggaccactttgtacaagaaagctgggtaactaattttgcaccaaatg 3',
which include the AttB sites for Gateway recombination. The amplified PCR fragment was purified also using standard methods. The first step of the Gateway procedure, the BP reaction, was then performed, during which the PCR fragment recombines in vivo with the pDONR201 plasmid to produce, according to the Gateway terminology, an "entry clone". Plasmid pDONR201 was purchased from Invitrogen, as part of the Gateway® technology.

The entry clone comprising SEQ ID NO: 131 was then used in an LR reaction with a destination vector used for *Oryza sativa* transformation. This vector contained as functional elements within the T-DNA borders: a plant selectable marker; a screenable marker expression cassette; and a Gateway cassette intended for LR *in vivo* recombination with the nucleic acid sequence of interest already cloned in the entry clone. A rice prolamin promoter (SEQ ID NO: 133) for seed-specific expression was located upstream of this Gateway cassette.

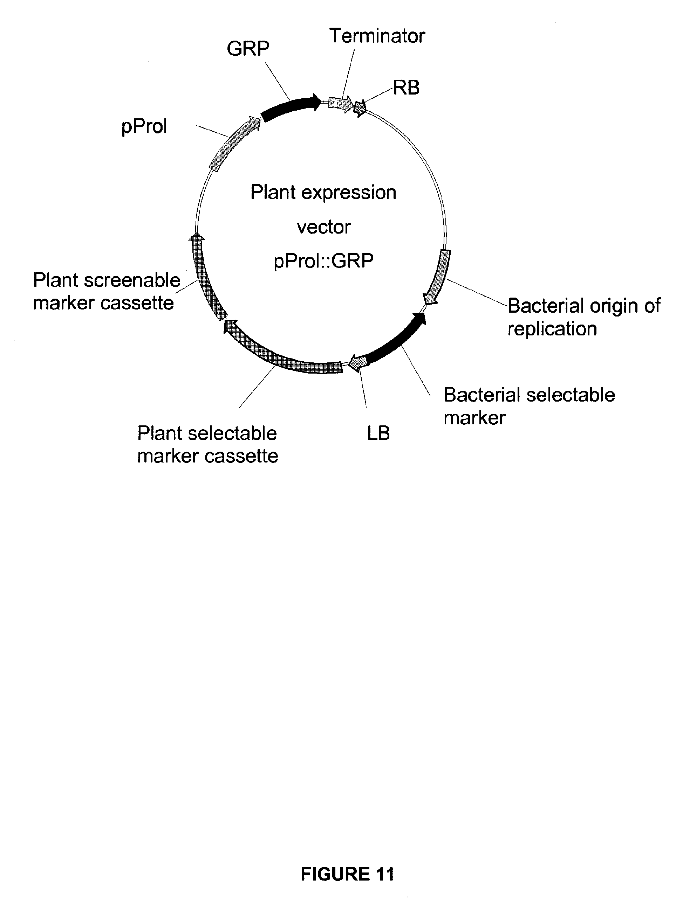

After the LR recombination step, the resulting expression vector pProl::GRP (Figure 11) was transformed into *Agrobacterium* strain LBA4044 according to methods well known in the art.

***Example 23: Plant transformation***

*Rice transformation*

The *Agrobacterium* containing the expression vector was used to transform *Oryza sativa* plants. Mature dry seeds of the rice japonica cultivar Nipponbare were dehusked. Sterilization was carried out by incubating for one minute in 70% ethanol, followed by 30 minutes in 0.2% $HgCl_2$, followed by a 6 times 15 minutes wash with sterile distilled water. The sterile seeds were then germinated on a medium containing 2,4-D (callus induction medium). After incubation in the dark for four weeks, embryogenic, scutellum-derived calli were excised and propagated on the same medium. After two weeks, the calli were multiplied or propagated by subculture on the same medium for another 2 weeks. Embryogenic callus pieces were subcultured on fresh medium 3 days before co-cultivatin (to boost cell division activity).

*Agrobacterium* strain LBA4404 containing the expression vector was used for co-cultivation. *Agrobacterium* was inoculated on AB medium with the appropriate antibiotics and cultured for 3 days at 28°C. The bacteria were then collected and suspended in liquid co-cultivation medium to a density ($OD_{600}$) of about 1. The suspension was then transferred to a Petri dish and the calli immersed in the suspension for 15 minutes. The callus tissues were then blotted dry on a filter paper and transferred to solidified, co-cultivation medium and incubated for 3 days in the dark at 25°C. Co-cultivated calli were grown on 2,4-D-containing medium for 4 weeks in the dark at 28°C in the presence of a selection agent. During this period, rapidly growing resistant callus islands developed. After transfer of this material to a regeneration medium and incubation in the light, the embryogenic potential was released and shoots developed in the next four to five weeks. Shoots were excised from the calli and incubated for 2 to 3 weeks on an auxin-containing medium from which they were transferred to soil. Hardened shoots were grown under high humidity and short days in a greenhouse.

Approximately 35 independent T0 rice transformants were generated for one construct. The primary transformants were transferred from a tissue culture chamber to a greenhouse. After a quantitative PCR analysis to verify copy number of the T-DNA insert, only single copy transgenic plants that exhibit tolerance to the selection agent were kept for harvest of T1 seed. Seeds were then harvested three to five months after transplanting. The method yielded single locus transformants at a rate of over 50 % (Aldemita and Hodges1996, Chan et al. 1993, Hiei et al. 1994).

***Example 24: Phenotypic evaluation procedure***

24.1 Evaluation setup

Approximately 35 independent T0 rice transformants were generated. The primary transformants were transferred from a tissue culture chamber to a greenhouse for growing and harvest of T1 seed. Six events, of which the T1 progeny segregated 3:1 for presence/absence of the transgene, were retained. For each of these events, approximately 10 T1 seedlings containing the transgene (hetero- and homo-zygotes) and approximately 10 T1 seedlings lacking the transgene (nullizygotes) were selected by monitoring visual marker expression. The transgenic plants and the corresponding nullizygotes were grown side-by-side at random positions. Greenhouse conditions were of shorts days (12 hours light), 28°C in the light and 22°C in the dark, and a relative humidity of 70%.

Four T1 events were further evaluated in the T2 generation following the same evaluation procedure as for the T1 generation but with more individuals per event.

From the stage of sowing until the stage of maturity the plants were passed several times through a digital imaging cabinet. At each time point digital images (2048x1536 pixels, 16 million colours) were taken of each plant from at least 6 different angles.

<u>24.2 Statistical analysis: F test</u>

A two factor ANOVA (analysis of variants) was used as a statistical model for the overall evaluation of plant phenotypic characteristics. An F test was carried out on all the parameters measured of all the plants of all the events transformed with the gene of the present invention. The F test was carried out to check for an effect of the gene over all the transformation events and to verify for an overall effect of the gene, also known as a global gene effect. The threshold for significance for a true global gene effect was set at a 5% probability level for the F test. A significant F test value points to a gene effect, meaning that it is not only the mere presence or position of the gene that is causing the differences in phenotype.

Because two experiments with overlapping events were carried out, a combined analysis is performed. This is useful to check consistency of the effects over the two experiments, and if this is the case, to accumulate evidence from both experiments in order to increase confidence in the conclusion. The method used is a mixed-model approach that takes into account the multilevel structure of the data (i.e. experiment - event - segregants). P values were obtained by comparing likelihood ratio test to chi square distributions.

<u>24.3 Parameters measured</u>

*Biomass-related parameter measurement*

The plant aboveground area (or leafy biomass) was determined by counting the total number of pixels on the digital images from aboveground plant parts discriminated from the background. This value was averaged for the pictures taken on the same time point from the different angles and was converted to a physical surface value expressed in square mm by calibration. Experiments show that the aboveground plant area measured this way correlates with the biomass of plant parts above ground. The above ground area is the area measured at the time point at which the plant had reached its maximal leafy biomass.

*Seed-related parameter measurements*

The mature primary panicles were harvested, counted, bagged, barcode-labelled and then dried for three days in an oven at 37°C. The panicles were then threshed and all the seeds were collected and counted. The filled husks were separated from the empty ones using an air-blowing device. The empty husks were discarded and the remaining fraction was counted again. The filled husks were weighed on an analytical balance. The number of filled seeds was determined by counting the number of filled husks that remained after the separation step. The total seed yield was measured by weighing all filled husks harvested from a plant. Total seed number per plant was measured by counting the number of husks harvested from a plant. Thousand Kernel Weight (TKW) is extrapolated from the number of filled seeds counted and their total weight. The Harvest Index (HI) in the present invention is defined as the ratio between the total seed yield and the above ground area ($mm^2$), multiplied by a factor $10^6$. The total number of flowers per panicle as defined in the present invention is the ratio between the total number of seeds and the number of mature primary panicles. The seed fill rate as defined in the present invention is the proportion (expressed as a %) of the number of filled seeds over the total number of seeds (or florets).

***Example 25: Results of the phenotypic evaluation of the transgenic plants***

The transgenic rice plants expressing the *GRP* nucleic acid sequence represented by SEQ ID NO: 131 under control of the prolamin promoter showed an increase of more than 5% for total seed yield per plant, number of filled seeds, total number of seeds, and harvest index.

| | Overall average % increase in 4 events in the T1 generation | Average % increase in 4 events in the T2 generation |
|---|---|---|
| Total seed yield per plant | 19% | 14% |
| Number of filled seeds | 20% | 16% |
| Total number of seeds | 16% | 8% |
| Harvest index | 9% | 15% |

***Example 26: Other crop transformation***

*Corn transformation*

Transformation of maize *(Zea mays)* is performed with a modification of the method described by Ishida et al. (1996) Nature Biotech 14(6): 745-50. Transformation is genotype-dependent in corn and only specific genotypes are amenable to transformation and regeneration. The inbred line A188 (University of Minnesota) or hybrids with A188 as a parent are good sources of donor material for transformation, but other genotypes can be used successfully as well. Ears are harvested from corn plant approximately 11 days after pollination (DAP) when the length of the immature embryo is about 1 to 1.2 mm. Immature embryos are cocultivated with *Agrobacterium tumefaciens* containing the expression vector, and transgenic plants are recovered through organogenesis. Excised embryos are grown on callus induction medium, then maize regeneration medium, containing the selection agent (for example imidazolinone but various selection markers can be used). The Petri plates are incubated in the light at 25 °C for 2-3 weeks, or until shoots develop. The green shoots are transferred from each embryo to maize rooting medium and incubated at 25 °C for 2-3 weeks, until roots develop. The rooted shoots are transplanted to soil in the greenhouse. T1 seeds are produced from plants that exhibit tolerance to the selection agent and that contain a single copy of the T-DNA insert.

*Wheat transformation*

Transformation of wheat is performed with the method described by Ishida et al. (1996) Nature Biotech 14(6): 745-50. The cultivar Bobwhite (available from CIMMYT, Mexico) is commonly used in transformation. Immature embryos are co-cultivated with *Agrobacterium tumefaciens* containing the expression vector, and transgenic plants are recovered through organogenesis. After incubation with *Agrobacterium,* the embryos are grown in vitro on callus induction medium, then regeneration medium, containing the selection agent (for example imidazolinone but various selection markers can be used). The Petri plates are incubated in the light at 25°C for 2-3 weeks, or until shoots develop. The green shoots are transferred from each embryo to rooting medium and incubated at 25 °C for 2-3 weeks, until roots develop. The rooted shoots are transplanted to soil in the greenhouse. T1 seeds are produced from plants that exhibit tolerance to the selection agent and that contain a single copy of the T-DNA insert.

*Soybean transformation*

Soybean is transformed according to a modification of the method described in the Texas A&M patent US 5,164,310. Several commercial soybean varieties are amenable to transformation by this method. The cultivar Jack (available from the Illinois Seed foundation) is commonly used for transformation. Soybean seeds are sterilised for *in vitro* sowing. The hypocotyl, the radicle and one cotyledon are excised from seven-day old young seedlings. The epicotyl and the remaining cotyledon are further grown to develop axillary nodes. These axillary nodes are excised and incubated with *Agrobacterium tumefaciens* containing the expression vector. After the cocultivation treatment, the explants are washed and transferred to selection media. Regenerated shoots are excised and placed on a shoot elongation medium. Shoots no longer than 1 cm are placed on rooting medium until roots develop. The rooted shoots are transplanted to soil in the greenhouse. T1 seeds are produced from plants that exhibit tolerance to the selection agent and that contain a single copy of the T-DNA insert.

Rapeseed/canola *transformation*

Cotyledonary petioles and hypocotyls of 5-6 day old young seedling are used as explants for tissue culture and transformed according to Babic et al. (1998, Plant Cell Rep 17: 183-188). The commercial cultivar Westar (Agriculture Canada) is the standard variety used for transformation, but other varieties can also be used. Canola seeds are surface-sterilized for in vitro sowing. The cotyledon petiole explants with the cotyledon attached are excised from the in vitro seedlings, and inoculated with *Agrobacterium* (containing the expression vector) by dipping the cut end of the petiole explant into the bacterial suspension. The explants are then cultured for 2 days on MSBAP-3 medium containing 3 mg/l BAP, 3 % sucrose, 0.7 % Phytagar at 23 °C, 16 hr light. After two days of co-cultivation with *Agrobacterium,* the petiole explants are transferred to MSBAP-3 medium containing 3 mg/l BAP, cefotaxime, carbenicillin, or timentin (300 mg/l) for 7 days, and then cultured on MSBAP-3 medium with cefotaxime, carbenicillin, or timentin and selection agent until shoot regeneration. When the shoots are 5 - 10 mm in length, they are cut and transferred to shoot elongation medium (MSBAP-0.5, containing 0.5 mg/l BAP). Shoots of about 2 cm in length are transferred to the rooting medium (MS0) for root induction. The rooted shoots are transplanted to soil in the greenhouse. T1 seeds are produced from plants that exhibit tolerance to the selection agent and that contain a single copy of the T-DNA insert.

*Alfalfa transformation*

A regenerating clone of alfalfa *(Medicago sativa)* is transformed using the method of (McKersie et al., 1999 Plant Physiol 119: 839-847). Regeneration and transformation of alfalfa is genotype dependent and therefore a regenerating plant is required. Methods to obtain regenerating plants have been described. For examples, these can be selected from the cultivar Rangelander (Agriculture Canada) or any other commercial alfalfa variety as described by Brown DCW and A Atanassov (1985. Plant Cell Tissue Organ Culture 4: 111-112). Alternatively, the RA3 variety (University of Wisconsin) has been selected for use in tissue culture (Walker et al., 1978 Am J Bot 65:654-659). Petiole explants are cocultivated with an overnight culture of *Agrobacterium tumefaciens* C58C1 pMP90 (McKersie et al., 1.999 Plant Physiol 119: 839-847) or LBA4404 containing the expression vector. The explants are cocultivated for 3 d in the dark on SH induction medium containing 288 mg/ L Pro, 53 mg/ L thioproline, 4.35 g/ L K2SO4, and 100 μm acetosyringinone. The explants are washed in half-strength Murashige-Skoog medium (Murashige and Skoog, 1962) and plated on the same SH induction medium without acetosyringinone but with a suitable selection agent and suitable antibiotic to inhibit *Agrobacterium* growth. After several weeks, somatic embryos are transferred to BOi2Y development medium containing no growth regulators, no antibiotics, and 50 g/ L sucrose. Somatic embryos are subsequently germinated on half-strength Murashige-Skoog medium. Rooted seedlings were transplanted into pots and grown in a greenhouse. T1 seeds are produced from plants that exhibit tolerance to the selection agent and that contain a single copy of the T-DNA insert.

**Cotton transformation**

Cotton *(Gossypium hirsutum* L.) transformation is performed using *Agrobacterium tumefaciens,* on hypocotyls explants. The commercial cultivars such as Coker 130 or Coker 312 (SeedCo, Lubbock, TX) are standard varieties used for transformation, but other varieties can also be used. The seeds are surface sterilized and germinated in the dark. Hypocotyl explants are cut from the germinated seedlings to lengths of about 1-1.5 centimeter. The hypotocyl explant is submersed in the *Agrobacterium tumefaciens* inoculum containing the expression vector, for 5 minutes then co-cultivated for about 48 hours on MS +1.8 mg/l KNO3 + 2% glucose at 24° C, in the dark. The explants are transferred the same medium containing appropriate bacterial and plant selectable markers (renewed several times), until embryogenic calli is seen. The calli are separated and subcultured until somatic embryos appear. Plantlets derived from the somatic embryos are matured on rooting medium until roots develop. The rooted shoots are transplanted to potting soil in the greenhouse. T1 seeds are produced from plants that exhibit tolerance to the selection agent and that contain a single copy of the T-DNA insert.

***Example 27: Abiotic stress screens***

*Drought screen*

Rice plants from T1, T2 or further generations are grown in potting soil under normal conditions until they approached the heading stage. They are then transferred to a "dry" section where irrigation is withheld. Humidity probes are inserted in randomly chosen pots to monitor the soil water content (SWC). When SWC goes below certain thresholds, the plants are automatically re-watered continuously until a normal level is reached again. The plants are then re-transferred again to normal conditions. The rest of the cultivation (plant maturation, seed harvest) is the same as for plants not grown under abiotic stress conditions. Growth and yield parameters are recorded as detailed for growth under normal conditions.

*Salt stress screen*

Rice plants from T1, T2 or further generations are grown on a substrate made of coco fibers and argex (3 to 1 ratio). A normal nutrient solution is used during the first two weeks after transplanting the plantlets in the greenhouse. After the first two weeks, 25 mM of salt (NaCl) is added to the nutrient solution, until the plants are harvested. Growth and yield parameters are recorded as detailed for growth under normal conditions.

*Nitrogen use efficiency screen*

Rice plants from T1, T2 or further generations are grown in potting soil under normal conditions except for the nutrient solution. The pots are watered from transplantation to maturation with a specific nutrient solution containing reduced N nitrogen (N) content, usually between 7 to 8 times less. The rest of the cultivation (plant maturation, seed harvest) is the same as for plants not grown under abiotic stress. Growth and yield parameters are recorded as detailed for growth under normal conditions.

***Example 28: Cloning of the nucleic acid sequence used in the methods of the invention, coding for* an STO (Salt Tolerance) protein**

The nucleic acid sequence used in the methods of the invention was amplified by PCR using as template a custom-made Oryza *sativa* seedlings cDNA library (in pCMV Sport 6.0; Invitrogen, Paisley, UK). PCR was performed using Hifi Taq DNA polymerase in standard conditions, using 200 ng of template in a 50 μl PCR mix. The primers used were prm1-STO (SEQ ID NO: 216; sense, start codon in bold): 5'-ggggacaagtttgtacaaaaaagcaggcttaaa caatgaaggtgcagtgcga-3' and prm2-STO (SEQ ID NO: 217; reverse, complementary): 5'-ggggacactttgtacaagaaagctgggttcaccagtacaagcagggag 3', which include the AttB sites for Gateway recombination. The amplified PCR fragment was purified also using standard methods. The first step of the Gateway procedure, the BP reaction, was then performed, during which the PCR fragment recombines in vivo with the pDONR201 plasmid to produce, according to the Gateway terminology, an "entry clone", pSTO. Plasmid pDONR201 was purchased from Invitrogen, as part of the Gateway® technology.

The entry clone comprising SEQ ID NO: 168 was then used in an LR reaction with a destination vector used for Oryza *sativa* transformation. This vector contained as functional elements within the T-DNA borders: a plant selectable marker; a screenable marker expression cassette; and a Gateway cassette intended for LR in vivo recombination with the nucleic acid sequence of interest already cloned in the entry clone. A rice GOS2 promoter (SEQ ID NO: 218) for root specific expression was located upstream of this Gateway cassette.

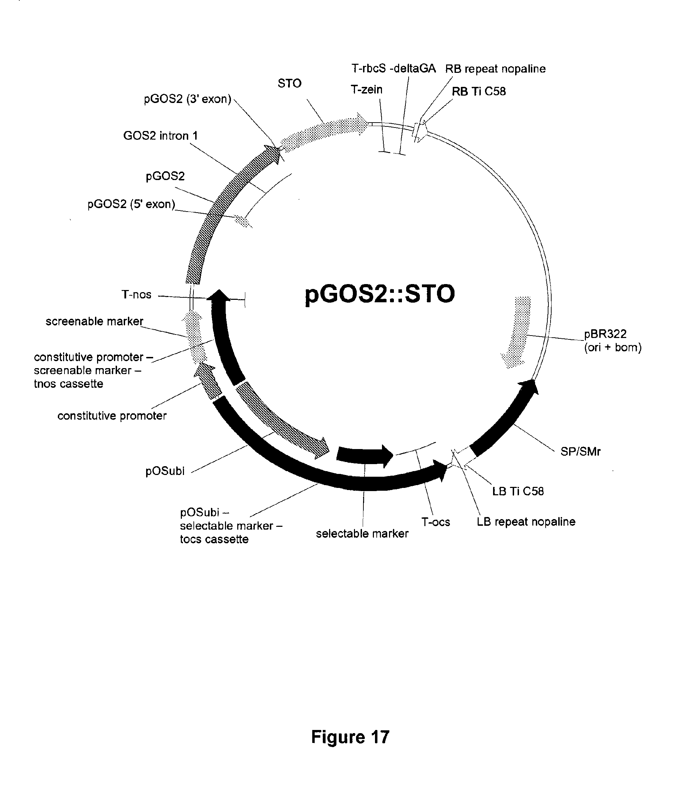

After the LR recombination step, the resulting expression vector pGOS2::STO (Figure 17) was transformed into Agrobacterium strain LBA4044 according to methods well known in the art.

***Example 29: Plant transformation***

*Rice transformation*

The *Agrobacterium* containing the expression vector was used to transform Oryza *sativa* plants. Mature dry seeds of the rice japonica cultivar Nipponbare were dehusked. Sterilization was carried out by incubating for one minute in 70% ethanol, followed by 30 minutes in 0.2% $HgCl_2$, followed by a 6 times 15 minutes wash with sterile distilled water. The sterile seeds were then germinated on a medium containing 2,4-D (callus induction medium). After incubation in the dark for four weeks, embryogenic, scutellum-derived calli were excised and propagated on the same medium. After two weeks, the calli were multiplied or propagated by subculture on the same medium for another 2 weeks. Embryogenic callus pieces were subcultured on fresh medium 3 days before co-cultivation (to boost cell division activity).

*Agrobacterium* strain LBA4404 containing the expression vector was used for co-cultivation. *Agrobacterium* was inoculated on AB medium with the appropriate antibiotics and cultured for 3 days at 28°C. The bacteria were then collected and suspended in liquid co-cultivation medium to a density ($OD_{600}$) of about 1. The suspension was then transferred to a Petri dish and the calli immersed in the suspension for 15 minutes. The callus tissues were then blotted dry on a filter paper and transferred to solidified, co-cultivation medium and incubated for 3 days in the dark at 25°C. Co-cultivated calli were grown on 2,4-D-containing medium for 4 weeks in the dark at 28°C in the presence of a selection agent. During this period, rapidly growing resistant callus islands developed. After transfer of this material to a regeneration medium and incubation in the light, the embryogenic potential was released and shoots developed in the next four to five weeks. Shoots were excised from the calli and incubated for 2 to 3 weeks on an auxin-containing medium from which they were transferred to soil. Hardened shoots were grown under high humidity and short days in a greenhouse.

Approximately 35 independent T0 rice transformants were generated for one construct. The primary transformants were transferred from a tissue culture chamber to a greenhouse. After a quantitative PCR analysis to verify copy number of the T-DNA insert, only single copy transgenic plants that exhibit tolerance to the selection agent were kept for harvest of T1 seed. Seeds were then harvested three to five months after transplanting. The method yielded single locus transformants at a rate of over 50 % (Aldemita and Hodges1996, Chan et al. 1993, Hiei et al. 1994).

*Corn transformation*

Transformation of maize *(Zea mays)* is performed with a modification of the method described by Ishida et al. (1996) Nature Biotech 14(6): 745-50. Transformation is genotype-dependent in corn and only specific genotypes are amenable to transformation and regeneration. The inbred line A188 (University of Minnesota) or hybrids with A188 as a parent are good sources of donor material for transformation, but other genotypes can be used successfully as well. Ears are harvested from corn plant approximately 11 days after pollination (DAP) when the length of the immature embryo is about 1 to 1.2 mm. Immature embryos are cocultivated with *Agrobacterium tumefaciens* containing the expression vector, and transgenic plants are recovered through organogenesis. Excised embryos are grown on callus induction medium, then maize regeneration medium, containing the selection agent (for example imidazolinone but various selection markers can be used). The Petri plates are incubated in the light at 25 °C for 2-3 weeks, or until shoots develop. The green shoots are transferred from each embryo to maize rooting medium and incubated at 25 °C for 2-3 weeks, until roots develop. The rooted shoots are transplanted to soil in the greenhouse. T1 seeds are produced from plants that exhibit tolerance to the selection agent and that contain a single copy of the T-DNA insert.

*Wheat transformation*

Transformation of wheat is performed with the method described by Ishida et al. (1996) Nature Biotech 14(6): 745-50. The cultivar Bobwhite (available from CIMMYT, Mexico) is commonly used in transformation. Immature embryos are co-cultivated with *Agrobacterium tumefaciens* containing the expression vector, and transgenic plants are recovered through organogenesis. After incubation with *Agrobacterium,* the embryos are grown in vitro on callus induction medium, then regeneration medium, containing the selection agent (for example imidazolinone but various selection markers can be used). The Petri plates are incubated in the light at 25 °C for 2-3 weeks, or until shoots develop. The green shoots are transferred from each embryo to rooting medium and incubated at 25 °C for 2-3 weeks, until roots develop. The rooted shoots are transplanted to soil in the greenhouse. T1 seeds are produced from plants that exhibit tolerance to the selection agent and that contain a single copy of the T-DNA insert.

*Soybean transformation*

Soybean is transformed according to a modification of the method described in the Texas A&M patent US 5,164,310. Several commercial soybean varieties are amenable to transformation by this method. The cultivar Jack (available from the Illinois Seed foundation) is commonly used for transformation. Soybean seeds are sterilised for *in vitro* sowing. The hypocotyl, the radicle and one cotyledon are excised from seven-day old young seedlings. The epicotyl and the remaining cotyledon are further grown to develop axillary nodes. These axillary nodes are excised and incubated with *Agrobacterium tumefaciens* containing the expression vector. After the cocultivation treatment, the explants are washed and transferred to selection media. Regenerated shoots are excised and placed on a shoot elongation medium. Shoots no longer than 1 cm are placed on rooting medium until roots develop. The rooted shoots are transplanted to soil in the greenhouse. T1 seeds are produced from plants that exhibit tolerance to the selection agent and that contain a single copy of the T-DNA insert.

*Rapeseed/canola transformation*

Cotyledonary petioles and hypocotyls of 5-6 day old young seedling are used as explants for tissue culture and transformed according to Babic et al. (1998, Plant Cell Rep 17: 183-188). The commercial cultivar Westar (Agriculture Canada) is the standard variety used for transformation, but other varieties can also be used. Canola seeds are surface-sterilized for in vitro sowing. The cotyledon petiole explants with the cotyledon attached are excised from the in vitro seedlings, and inoculated with *Agrobacterium* (containing the expression vector) by dipping the cut end of the petiole explant into the bacterial suspension. The explants are then cultured for 2 days on MSBAP-3 medium containing 3 mg/l BAP, 3 % sucrose, 0.7 % Phytagar at 23 °C, 16 hr light. After two days of co-cultivation with *Agrobacterium,* the petiole explants are transferred to MSBAP-3 medium containing 3 mg/l BAP, cefotaxime, carbenicillin, or timentin (300 mg/l) for 7 days, and then cultured on MSBAP-3 medium with cefotaxime, carbenicillin, or timentin and selection agent until shoot regeneration. When the shoots are 5 - 10 mm in length, they are cut and transferred to shoot elongation medium (MSBAP-0.5, containing 0.5 mg/l BAP). Shoots of about 2 cm in length are transferred to the rooting medium (MS0) for root induction. The rooted shoots are transplanted to soil in the greenhouse. T1 seeds are produced from plants that exhibit tolerance to the selection agent and that contain a single copy of the T-DNA insert.

*Alfalfa transformation*

A regenerating clone of alfalfa *(Medicago sativa)* is transformed using the method of (McKersie et al., 1999 Plant Physiol 119: 839-847). Regeneration and transformation of alfalfa is genotype dependent and therefore a regenerating plant is required. Methods to obtain regenerating plants have been described. For example, these can be selected from the cultivar Rangelander (Agriculture Canada) or any other commercial alfalfa variety as described by Brown DCW and A Atanassov (1985. Plant Cell Tissue Organ Culture 4: 111-112). Alternatively, the RA3 variety (University of Wisconsin) has been selected for use in tissue culture (Walker et al., 1978 Am J Bot 65:654-659). Petiole explants are cocultivated with an overnight culture of *Agrobacterium tumefaciens* C58C1 pMP90 (McKersie et al., 1999 Plant Physiol 119: 839-847) or LBA4404 containing the expression vector. The explants are cocultivated for 3 d in the dark on SH induction medium containing 288 mg/ L Pro, 53 mg/ L thioproline, 4.35 g/ L K2SO4, and 100 μm acetosyringinone. The explants are washed in half-strength Murashige-Skoog medium (Murashige and Skoog, 1962) and plated on the same SH induction medium without acetosyringinone but with a suitable selection agent and suitable antibiotic to inhibit *Agrobacterium* growth. After several weeks, somatic embryos are transferred to BOi2Y development medium containing no growth regulators, no antibiotics, and 50 g/ L sucrose. Somatic embryos are subsequently germinated on half-strength Murashige-Skoog medium. Rooted seedlings were transplanted into pots and grown in a greenhouse. T1 seeds are produced from plants that exhibit tolerance to the selection agent and that contain a single copy of the T-DNA insert.

***Example 30: Phenotypic evaluation procedure***

30.1 Evaluation setup

Approximately 35 independent T0 rice transformants were generated. The primary transformants were transferred from a tissue culture chamber to a greenhouse for growing and harvest of T1 seed. Six events, of which the T1 progeny segregated 3:1 for presence/absence of the transgene, were retained. For each of these events, approximately 10 T1 seedlings containing the transgene (hetero- and homo-zygotes) and approximately 10 T1 seedlings lacking the transgene (nullizygotes) were selected by monitoring visual marker expression. The transgenic plants and the corresponding nullizygotes were grown side-by-side at random positions. Greenhouse conditions were of shorts days (12 hours light), 28°C in the light and 22°C in the dark, and a relative humidity of 70%.

Four T1 events were further evaluated in the T2 generation following the same evaluation procedure as for the T1 generation but with more individuals per event. From the stage of sowing until the stage of maturity the plants were passed several times through a digital imaging cabinet. At each time Point digital images (2048x1536 pixels, 16 million colours) were taken of each plant from at least 6 different angles.

*Drought screen*

Plants from T2 seeds were grown in potting soil under normal conditions until they approached the heading stage. They were then transferred to a "dry" section where irrigation was withheld. Humidity probes were inserted in randomly chosen pots to monitor the soil water content (SWC). When SWC went below certain thresholds, the plants were automatically re-watered continuously until a normal level was reached again. The plants were then re-transferred again to normal conditions. The rest of the cultivation (plant maturation, seed harvest) was the same as for plants not grown under abiotic stress conditions. Growth and yield parameters were recorded as detailed for growth under normal conditions.

*Nitrogen use efficiency screen*

Rice plants from T2 seeds were grown in potting soil under normal conditions except for the nutrient solution. The pots were watered from transplantation to maturation with a specific nutrient solution containing reduced N nitrogen (N) content, usually between 7 to 8 times less. The rest of the cultivation (plant maturation, seed harvest) was the same as for plants not grown under abiotic stress. Growth and yield parameters are recorded as detailed for growth under normal conditions.

30.2 Statistical analysis: F test

A two factor ANOVA (analysis of variants) was used as a statistical model for the overall evaluation of plant phenotypic characteristics. An F test was carried out on all the parameters measured of all the plants of all the events transformed with the gene of the present invention. The F test was carried out to check for an effect of the gene over all the transformation events and to verify for an overall effect of the gene, also known as a global gene effect. The threshold for significance for a true global gene effect was set at a 5% probability level for the F test. A significant F test value Points to a gene effect, meaning that it is not only the mere presence or position of the gene that is causing the differences in phenotype.

Because two experiments with overlapping events were carried out, a combined analysis was performed. This is useful to check consistency of the effects over the two experiments, and if this is the case, to accumulate evidence from both experiments in order to increase confidence in the conclusion. The method used was a mixed-model approach that takes into account the multilevel structure of the data (i.e. experiment - event - segregants). P values were obtained by comparing likelihood ratio test to chi square distributions.

30.3 Parameters measured

*Biomass-related parameter measurement*

From the stage of sowing until the stage of maturity the plants were passed several times through a digital imaging cabinet. At each time Point digital images (2048x1536 pixels, 16 million colours) were taken of each plant from at least 6 different angles.

The plant aboveground area (or leafy biomass) was determined by counting the total number of pixels on the digital images from aboveground plant parts discriminated from the background. This value was averaged for the pictures taken on the same time Point from the different angles and was converted to a physical surface value expressed in square mm by calibration. Experiments show that the aboveground plant area measured this way correlates with the biomass of plant parts above ground. The above ground area is the area measured at the time Point at which the plant had reached its maximal leafy biomass. The early vigour is the plant (seedling) aboveground area three weeks post-germination. Increase in root biomass is expressed as an increase in total root biomass (measured as maximum biomass of roots observed during the lifespan of a plant); or as an increase in the root/shoot index (measured as the ratio between root mass and shoot mass in the period of active growth of root and shoot).

Early vigour was determined by counting the total number of pixels from aboveground plant parts discriminated from the background. This value was averaged for the pictures taken on the same time Point from different angles and was converted to a physical surface value expressed in square mm by calibration. The results described below are for plants three weeks post-germination.

Flowering time was calculated as previously described (PCT/EP2007/001422). Briefly, the time elapsed between sowing and the moment when the first panicle appeared in the plant was calculated. Appearance of the first panicle refers the point in time when the panicle is visible and detectable in the photographic images of the plants processed as described in (PCT/EP2007/001422).

*Seed-related parameter measurements*

The mature primary panicles were harvested, counted, bagged, barcode-labelled and then dried for three days in an oven at 37°C. The panicles were then threshed and all the seeds were collected and counted. The filled husks were separated from the empty ones using an air-blowing device. The empty husks were discarded and the remaining fraction was counted again. The filled husks were weighed on an analytical balance. The number of filled seeds was determined by counting the number of filled husks that remained after the separation step.

The total seed yield was measured by weighing all filled husks harvested from a plant. Total seed number per plant was measured by counting the number of husks harvested from a plant. Thousand Kernel Weight (TKW) is extrapolated from the number of filled seeds counted and their total weight. The Harvest Index (HI) in the present invention is defined as the ratio between the total seed yield and the above ground area ($mm^2$), multiplied by a factor $10^6$. The total number of flowers per panicle as defined in the present invention is the ratio between the total number of seeds and the number of mature primary panicles. The seed fill rate as defined in the present invention is the proportion (expressed as a %) of the number of filled seeds over the total number of seeds (or florets).

***Example 31: Results of the phenotypic evaluation of the transgenic plants***

The results of the evaluation of transgenic rice plants expressing an STO nucleic acid under non-stress conditions are presented below. An increase of at least 5 % was observed for emergence vigour (early vigour), and between 3 and 6 days (6-9%), depending on the specific transgenic line, earlier flowering was observed in the transgenic plants when compared to relative control plant.

The results of the evaluation of transgenic rice plants expressing an STO nucleic acid under drought-stress conditions are presented hereunder. An increase was observed for total seed weight, number of filled seeds, fill rate, harvest index and thousand-kernel weight (Table D).

**Table D:** Result phenotypic evaluation of the transgenic STO plants.

| Parameter | Percentage difference for the transgenic versus the control plants |
|---|---|
| Emergence vigour | 15 |
| Flowering time (early/shorter) | 7 |

***Example 32: Cloning of the nucleic acid sequence used in the methods of the invention* encoding a UGE (UDP-Glucose 4-Epimerase or UDP-Gal 4-Epimerase) polypeptide**

The nucleic acid sequence used in the methods of the invention was amplified by PCR using as template a custom-made *Oryza sativa* seedlings cDNA library (in pCMV Sport 6.0; Invitrogen, Paisley, UK). PCR was performed using Hifi Taq DNA polymerase in standard conditions, using 200 ng of template in a 50 μl PCR mix. The primers used were prm1 (SEQ ID NO: 272; sense, start codon in bold): 5'-ggggacaagtttgtacaaaaaagcaggcttaaacaatggtttc ggccttgttg-3' and prm2 (SEQ ID NO: 273; reverse, complementary): 5'-ggggaccactttgtacaag aaagctgggtgctgctgctactggaggatt-3', which include the AttB sites for Gateway recombination. The amplified PCR fragment was purified also using standard methods. The first step of the Gateway procedure, the BP reaction, was then performed, during which the PCR fragment recombines in vivo with the pDONR201 plasmid to produce, according to the Gateway terminology, an "entry clone", pUGE. Plasmid pDONR201 was purchased from Invitrogen, as part of the Gateway® technology.

The entry clone comprising SEQ ID NO: 224 was then used in an LR reaction with a destination vector used for *Oryza sativa* transformation. This vector contained as functional elements within the T-DNA borders: a plant selectable marker; a screenable marker expression cassette; and a Gateway cassette intended for LR *in vivo* recombination with the nucleic acid sequence of interest already cloned in the entry clone. A rice GOS2 promoter (SEQ ID NO: 274) for root specific expression was located upstream of this Gateway cassette.

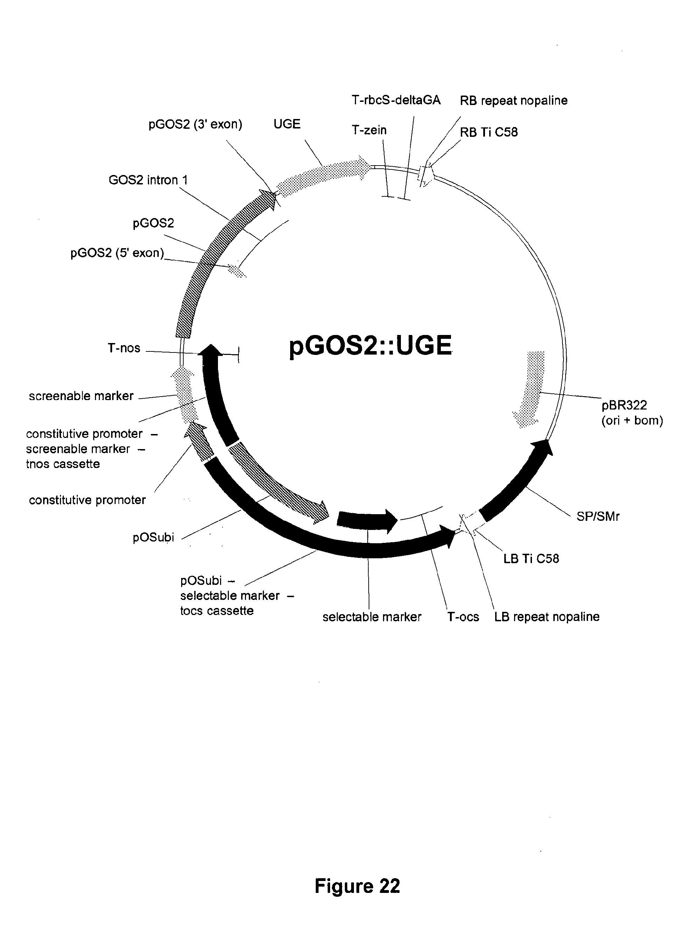

After the LR recombination step, the resulting expression vector pGOS2::UGE (Figure 22) was transformed into Agrobacterium strain LBA4044 according to methods well known in the art.

***Example 33: Plant transformation***

*Rice transformation*

The *Agrobacterium* containing the expression vector was used to transform *Oryza sativa* plants. Mature dry seeds of the rice japonica cultivar Nipponbare were dehusked. Sterilization was carried out by incubating for one minute in 70% ethanol, followed by 30 minutes in 0.2% $HgCl_2$, followed by a 6 times 15 minutes wash with sterile distilled water. The sterile seeds were then germinated on a medium containing 2,4-D (callus induction medium). After incubation in the dark for four weeks, embryogenic, scutellum-derived calli were excised and propagated on the same medium. After two weeks, the calli were multiplied or propagated by subculture on the same medium for another 2 weeks. Embryogenic callus pieces were subcultured on fresh medium 3 days before co-cultivation (to boost cell division activity).

*Agrobacterium* strain LBA4404 containing the expression vector was used for co-cultivation. *Agrobacterium* was inoculated on AB medium with the appropriate antibiotics and cultured for 3 days at 28°C. The bacteria were then collected and suspended in liquid co-cultivation medium to a density ($OD_{600}$) of about 1. The suspension was then transferred to a Petri dish and the calli immersed in the suspension for 15 minutes. The callus tissues were then blotted dry on a filter paper and transferred to solidified, co-cultivation medium and incubated for 3 days in the dark at 25°C. Co-cultivated calli were grown on 2,4-D-containing medium for 4 weeks in the dark at 28°C in the presence of a selection agent. During this period, rapidly growing resistant callus islands developed. After transfer of this material to a regeneration medium and incubation in the light, the embryogenic potential was released and shoots developed in the next four to five weeks. Shoots were excised from the calli and incubated for 2 to 3 weeks on an auxin-containing medium from which they were transferred to soil. Hardened shoots were grown under high humidity and short days in a greenhouse.

Approximately 35 independent T0 rice transformants were generated for one construct. The primary transformants were transferred from a tissue culture chamber to a greenhouse. After a quantitative PCR analysis to verify copy number of the T-DNA insert, only single copy transgenic plants that exhibit tolerance to the selection agent were kept for harvest of T1 seed. Seeds were then harvested three to five months after transplanting. The method yielded single locus transformants at a rate of over 50 % (Aldemita and Hodges1996, Chan et al. 1993, Hiei et al. 1994).

*Corn transformation*

Transformation of maize *(Zea mays)* is performed with a modification of the method described by Ishida et al. (1996) Nature Biotech 14(6): 745-50. Transformation is genotype-dependent in corn and only specific genotypes are amenable to transformation and regeneration. The inbred line A188 (University of Minnesota) or hybrids with A188 as a parent are good sources of donor material for transformation, but other genotypes can be used successfully as well. Ears are harvested from corn plant approximately 11 days after pollination (DAP) when the length of the immature embryo is about 1 to 1.2 mm. Immature embryos are cocultivated with *Agrobacterium tumefaciens* containing the expression vector, and transgenic plants are recovered through organogenesis. Excised embryos are grown on callus induction medium, then maize regeneration medium, containing the selection agent (for example imidazolinone but various selection markers can be used). The Petri plates are incubated in the light at 25 °C for 2-3 weeks, or until shoots develop. The green shoots are transferred from each embryo to maize rooting medium and incubated at 25 °C for 2-3 weeks, until roots develop. The rooted shoots are transplanted to soil in the greenhouse. T1 seeds are produced from plants that exhibit tolerance to the selection agent and that contain a single copy of the T-DNA insert.

*Wheat transformation*

Transformation of wheat is performed with the method described by Ishida et al. (1996) Nature Biotech 14(6): 745-50. The cultivar Bobwhite (available from CIMMYT, Mexico) is commonly used in transformation. Immature embryos are co-cultivated with *Agrobacterium tumefaciens* containing the expression vector, and transgenic plants are recovered through organogenesis. After incubation with *Agrobacterium,* the embryos are grown in vitro on callus induction medium, then regeneration medium, containing the selection agent (for example imidazolinone but various selection markers can be used). The Petri plates are incubated in the light at 25 °C for 2-3 weeks, or until shoots develop. The green shoots are transferred from each embryo to rooting medium and incubated at 25 °C for 2-3 weeks, until roots develop. The rooted shoots are transplanted to soil in the greenhouse. T1 seeds are produced from plants that exhibit tolerance to the selection agent and that contain a single copy of the T-DNA insert.

*Soybean transformation*

Soybean is transformed according to a modification of the method described in the Texas A&M patent US 5,164,310. Several commercial soybean varieties are amenable to transformation by this method. The cultivar Jack (available from the Illinois Seed foundation) is commonly used for transformation. Soybean seeds are sterilised for *in vitro* sowing. The hypocotyl, the radicle and one cotyledon are excised from seven-day old young seedlings. The epicotyl and the remaining cotyledon are further grown to develop axillary nodes. These axillary nodes are excised and incubated with *Agrobacterium tumefaciens* containing the expression vector. After the cocultivation treatment, the explants are washed and transferred to selection media. Regenerated shoots are excised and placed on a shoot elongation medium. Shoots no longer than 1 cm are placed on rooting medium until roots develop. The rooted shoots are transplanted to soil in the greenhouse. T1 seeds are produced from plants that exhibit tolerance to the selection agent and that contain a single copy of the T-DNA insert.

*Rapeseed/canola transformation*

Cotyledonary petioles and hypocotyls of 5-6 day old young seedling are used as explants for tissue culture and transformed according to Babic et al. (1998, Plant Cell Rep 17: 183-188). The commercial cultivar Westar (Agriculture Canada) is the standard variety used for transformation, but other varieties can also be used. Canola seeds are surface-sterilized for in vitro sowing. The cotyledon petiole explants with the cotyledon attached are excised from the in vitro seedlings, and inoculated with *Agrobacterium* (containing the expression vector) by dipping the cut end of the petiole explant into the bacterial suspension. The explants are then cultured for 2 days on MSBAP-3 medium containing 3 mg/l BAP, 3 % sucrose, 0.7 % Phytagar at 23 °C, 16 hr light. After two days of co-cultivation with *Agrobacterium,* the petiole explants are transferred to MSBAP-3 medium containing 3 mg/l BAP, cefotaxime, carbenicillin, or timentin (300 mg/l) for 7 days, and then cultured on MSBAP-3 medium with cefotaxime, carbenicillin, or timentin and selection agent until shoot regeneration. When the shoots are 5 - 10 mm in length, they are cut and transferred to shoot elongation medium (MSBAP-0.5, containing 0.5 mg/l BAP). Shoots of about 2 cm in length are transferred to the rooting medium (MS0) for root induction. The rooted shoots are transplanted to soil in the greenhouse. T1 seeds are produced from plants that exhibit tolerance to the selection agent and that contain a single copy of the T-DNA insert.

*Alfalfa transformation*

A regenerating clone of alfalfa *(Medicago sativa)* is transformed using the method of (McKersie et al., 1999 Plant Physiol 119: 839-847). Regeneration and transformation of alfalfa is genotype dependent and therefore a regenerating plant is required. Methods to obtain regenerating plants have been described. For example, these can be selected from the cultivar Rangelander (Agriculture Canada) or any other commercial alfalfa variety as described by Brown DCW and A Atanassov (1985. Plant Cell Tissue Organ Culture 4: 111-112). Alternatively, the RA3 variety (University of Wisconsin) has been selected for use in tissue culture (Walker et al., 1978 Am J Bot 65:654-659). Petiole explants are cocultivated with an overnight culture of *Agrobacterium tumefaciens* C58C1 pMP90 (McKersie et al., 1999 Plant Physiol 119: 839-847) or LBA4404 containing the expression vector. The explants are cocultivated for 3 d in the dark on SH induction medium containing 288 mg/ L Pro, 53 mg/ L thioproline, 4.35 g/ L K2SO4, and 100 μm acetosyringinone. The explants are washed in half-strength Murashige-Skoog medium (Murashige and Skoog, 1962) and plated on the same SH induction medium without acetosyringinone but with a suitable selection agent and suitable antibiotic to inhibit *Agrobacterium* growth. After several weeks, somatic embryos are transferred to BOi2Y development medium containing no growth regulators, no antibiotics, and 50 g/ L sucrose. Somatic embryos are subsequently germinated on half-strength Murashige-Skoog medium. Rooted seedlings were transplanted into pots and grown in a greenhouse. T1 seeds are produced from plants that exhibit tolerance to the selection agent and that contain a single copy of the T-DNA insert.

***Example 34: Phenotypic evaluation procedure***

34.1 Evaluation setup

Approximately 35 independent T0 rice transformants were generated. The primary transformants were transferred from a tissue culture chamber to a greenhouse for growing and harvest of T1 seed. Six events, of which the T1 progeny segregated 3:1 for presence/absence of the transgene, were retained. For each of these events, approximately 10 T1 seedlings containing the transgene (hetero- and homo-zygotes) and approximately 10 T1 seedlings lacking the transgene (nullizygotes) were selected by monitoring visual marker expression. The transgenic plants and the corresponding nullizygotes were grown side-by-side at random positions. Greenhouse conditions were of shorts days (12 hours light), 28°C in the light and 22°C in the dark, and a relative humidity of 70%.

Four T1 events were further evaluated in the T2 generation following the same evaluation procedure as for the T1 generation but with more individuals per event. From the stage of sowing until the stage of maturity the plants were passed several times through a digital imaging cabinet. At each time point digital images (2048x1536 pixels, 16 million colours) were taken of each plant from at least 6 different angles.

*Drought screen*

Plants from T2 seeds were grown in potting soil under normal conditions until they approached the heading stage. They were then transferred to a "dry" section where irrigation was withheld. Humidity probes were inserted in randomly chosen pots to monitor the soil water content (SWC). When SWC went below certain thresholds, the plants were automatically re-watered continuously until a normal level was reached again. The plants were then re-transferred again to normal conditions. The rest of the cultivation (plant maturation, seed harvest) was the same as for plants not grown under abiotic stress conditions. Growth and yield parameters are recorded as detailed for growth under normal conditions.

*Nitrogen use efficiency screen*

Rice plants from T2 seeds are grown in potting soil under normal conditions except for the nutrient solution. The pots were watered from transplantation to maturation with a specific nutrient solution containing reduced N nitrogen (N) content, usually between 7 to 8 times less. The rest of the cultivation (plant maturation, seed harvest) was the same as for plants not grown under abiotic stress. Growth and yield parameters are recorded as detailed for growth under normal conditions.

34.2 Statistical analysis: F test

A two factor ANOVA (analysis of variants) was used as a statistical model for the overall evaluation of plant phenotypic characteristics. An F test was carried out on all the parameters measured of all the plants of all the events transformed with the gene of the present invention. The F test was carried out to check for an effect of the gene over all the transformation events and to verify for an overall effect of the gene, also known as a global gene effect. The threshold for significance for a true global gene effect was set at a 5% probability level for the F test. A significant F test value points to a gene effect, meaning that it is not only the mere presence or position of the gene that is causing the differences in phenotype.

Because two experiments with overlapping events were carried out, a combined analysis was performed. This is useful to check consistency of the effects over the two experiments, and if this is the case, to accumulate evidence from both experiments in order to increase confidence in the conclusion. The method used was a mixed-model approach that takes into account the multilevel structure of the data (i.e. experiment - event - segregants). P values were obtained by comparing likelihood ratio test to chi square distributions.

34.3 Parameters measured

*Biomass-related parameter measurement*

From the stage of sowing until the stage of maturity the plants were passed several times through a digital imaging cabinet. At each time point digital images (2048x1536 pixels, 16 million colours) were taken of each plant from at least 6 different angles.

The plant aboveground area (or leafy biomass) was determined by counting the total number of pixels on the digital images from aboveground plant parts discriminated from the background. This value was averaged for the pictures taken on the same time point from the different angles and was converted to a physical surface value expressed in square mm by calibration. Experiments show that the aboveground plant area measured this way correlates with the biomass of plant parts above ground. The above ground area is the area measured at the time point at which the plant had reached its maximal leafy biomass. The early vigour is the plant (seedling) aboveground area three weeks post-germination. Increase in root biomass is expressed as an increase in total root biomass (measured as maximum biomass of roots observed during the lifespan of a plant); or as an increase in the root/shoot index (measured as the ratio between root mass and shoot mass in the period of active growth of root and shoot).

Early vigour was determined by counting the total number of pixels from aboveground plant parts discriminated from the background. This value was averaged for the pictures taken on the same time point from different angles and was converted to a physical surface value expressed in square mm by calibration. The results described below are for plants three weeks post-germination.

*Seed-related parameter measurements*

The mature primary panicles were harvested, counted, bagged, barcode-labelled and then dried for three days in an oven at 37°C. The panicles were then threshed and all the seeds were collected and counted. The filled husks were separated from the empty ones using an air-blowing device. The empty husks were discarded and the remaining fraction was counted again. The filled husks were weighed on an analytical balance. The number of filled seeds was determined by counting the number of filled husks that remained after the separation step. The total seed yield was measured by weighing all filled husks harvested from a plant. Total seed number per plant was measured by counting the number of husks harvested from a plant. Thousand Kernel Weight (TKW) is extrapolated from the number of filled seeds counted and their total weight. The Harvest Index (HI) in the present invention is defined as the ratio between the total seed yield and the above ground area ($mm^2$), multiplied by a factor $10^6$. The total number of flowers per panicle as defined in the present invention is the ratio between the total number of seeds and the number of mature primary panicles. The seed fill rate as defined in the present invention is the proportion (expressed as a %) of the number of filled seeds over the total number of seeds (or florets).

***Example 35: Results of the phenotypic evaluation of the transgenic plants***

The results of the evaluation of transgenic rice plants expressing a UGE nucleic acid under non-stress conditions and under drought-stress (water limiting) conditions are presented below. An increase of at least 5 % was observed for total seed yield, number of filled seeds, fill rate, and harvest index. Table E shows the results of the phenotypic evaluation.

**Table E:** Result of the phenotypic evaluation of transgenic plants.

| **Yield trait** | **non-stress** | **drought-stress** |
|---|---|---|
| | % Difference transgenic/ control* | % Difference transgenic/ control* |
| See yield (grams) | 15 | 45 |
| Nr of filled seed | 13 | 42 |

(continued)

| Yield trait | non-stress | drought-stress |
|---|---|---|
| | % Difference transgenic/ control* | % Difference transgenic/ control* |
| Fill rate | 5 | 45 |
| Harvest index | 10 | 49 |
| ** Increase measured in the transgenic plants versus the corresponding control nullyzygous plants, expressed in percentage (%).* | | |

## Claims

1. A method for enhancing yield-related traits in plants relative to control plants, comprising modulating expression (preferably, increasing) in a plant of a nucleic acid sequence encoding a GRP polypeptide, wherein said GRP polypeptide is an isopentenyl transferase (IPT) polypeptide as represented by SEQ ID NO: 132 or an orthologue, paralogue, or homologue thereof, and optionally selecting for plants having enhanced yield-related traits.

2. A method according to claim 1, wherein said GRP polypeptide as represented by SEQ ID NO: 132 and an orthologue, paralogue, or homologue thereof, belong to the tRNA isopentenyltransferase family with InterPro entries IPR002627 and IPR011593 and/or wherein said GRP polypeptide has in increasing order of preference at least 50%, 55%, 60%, 65%, 70%, 75%, 80%, 85%, 90%, 95%, 98%, 99% or more amino acid sequence identity to the GRP polypeptide as represented by SEQ ID NO: 132.

3. Method according to any preceding claim, wherein said nucleic acid sequence encoding a GRP polypeptide is represented by the nucleic acid sequence of SEQ ID NO: 131 or a portion thereof, or a sequence capable of hybridising with the nucleic acid sequence of SEQ ID NO: 131 or a portion thereof.

4. Method according to any preceding claim wherein said modulated expression (preferably, increased) is effected by introducing and expressing in a plant a nucleic acid sequence encoding said GRP polypeptide.

5. Method according to any preceding claim, wherein said enhanced yield-related traits comprise one or more of: increased total seed yield per plant, increased number of filled seeds, increased total number of seeds, and increased harvest index, relative to control plants.

6. Method according to any preceding claim, wherein said nucleic acid sequence is operably linked to a seed-specific promoter, preferably to a prolamin promoter, most preferably to a prolamin promoter from rice.

7. Method according to any preceding claim, wherein said nucleic acid sequence encoding a GRP polypeptide is of plant origin, preferably from a dicotyledonous plant, further preferably from the family Brassicaceae, more preferably from *Arabidopsis thaliana.*

8. Plant, part thereof (including seeds), or plant cell obtainable by a method according to any preceding claim, wherein said plant, part thereof, or plant cell comprises a nucleic acid transgene encoding a GRP polypeptide and preferably wherein said plant is a crop plant or a monocot or a cereal, such as rice, maize, wheat, barley, millet, rye, triticale, sorghum and oats.

9. Construct comprising:

   (i) nucleic acid sequence encoding a GRP polypeptide as defined in claims 1 or 2;
   (ii) one or more control sequences capable of driving expression of the nucleic acid sequence of (a), preferably wherein one of said control sequences is a seed-specific promoter, preferably a prolamin promoter, most preferably a prolamin promoter from rice; and optionally
   (iii) a transcription termination sequence.

10. Use of a construct according to claim 9 in a method for making plants having enhanced yield-related traits, particularly increased total seed yield per plant, increased number of filled seeds, increased total number of seeds, and increased harvest index, relative to control plants.

11. Plant, plant part or plant cell transformed with a construct according to claim 9 and preferably wherein said plant is a crop plant or a monocot or a cereal, such as rice, maize, wheat, barley, millet, rye, triticale, sorghum and oats.

12. Method for the production of a transgenic plant having enhanced-yield related traits relative to control plants, comprising:

(i) introducing and expressing in a plant, plant part, or plant cell, a nucleic acid sequence encoding a GRP polypeptide as defined in claim 1 or 2; and
(ii) cultivating the plant, plant part, or plant cell under conditions promoting plant growth and development.

**13.** Transgenic plant having enhanced yield-related traits, particularly increased total seed yield per plant, increased number of filled seeds, increased total number of seeds, and increased harvest index, relative to control plants, resulting from increased expression of a nucleic acid sequence encoding a GRP polypeptide as defined in claim 1 or 2, or a transgenic plant cell derived from said transgenic plant and preferably wherein said plant is a crop plant or a monocot or a cereal, such as rice, maize, wheat, barley, millet, rye, triticale, sorghum and oats.

**14.** Harvestable parts of a plant comprising a nucleic acid sequence encoding a GRP polypeptide according to claim 8, 11 or 13, wherein said harvestable parts are preferably seeds.

**15.** Products derived from a plant according to claim 8, 11 or 13 and/or from harvestable parts of a plant according to claim 14.

**16.** Use of a nucleic acid sequence encoding a GRP polypeptide in enhancing yield-related traits in plants, particularly in increasing total seed yield per plant, increasing number of filled seeds, increasing total number of seeds, and increasing harvest index, relative to control plants.

**17.** Use of a nucleic acid encoding a GRP polypeptide, wherein said GRP polypeptide is an isopentenyl transferase (IPT) polypeptide, for altering the harvest cycle of a plant.

**18.** Use of a nucleic acid encoding a GRP polypeptide, wherein said GRP polypeptide is an isopentenyl transferase (IPT) polypeptide, in breeding programmes to select plants having enhanced yield-related traits.

**19.** Use of a nucleic acid encoding a GRP polypeptide, wherein said GRP polypeptide is an isopentenyl transferase (IPT) polypeptide, as probes for genetically mapping the genes that they are a part of, and as markers for traits linked to those genes.

**20.** A method for combining enhanced yield-related traits in plants with tolerance to abiotic and biotic stresses, traits modifying various architectural features and/or biochemical and/or physiological features.

**21.** A method for growing plants under abiotic stress conditions by modulating expression of a nucleic acid sequence encoding a GRP polypeptide, wherein said GRP polypeptide is an isopentenyl transferase (IPT) polypeptide.

**22.** A method for growing plants under conditions of reduced nutrient availability by modulating expression of a nucleic acid sequence encoding a GRP polypeptide, wherein said GRP polypeptide is an isopentenyl transferase (IPT) polypeptide.

MTDYRLQPTMNLWTTDDNASMMEAFMSSSDISTLWPPASTT

TTTATTETTPTPAMEIPAQAGFNQETLQQRLQALIEGTHEG
4

WTYAIFWQPSYDFSGASVLGWGDGYYKGEEDKANPRRRSSS
1

PPFSTPADQEYRKKVLRELNSLISGGVAPSDDAVDEEVTDT

EWFFLVSMTQSFACGAGLAGKAFATGNAVWVSGSDQLSGSG

CERAKQGGVFGMHTIACIPSANGVVEVGSTEPIRQSSDLIN
2

KVRILFNFDGGAGDLSGLNWNLDPDQGENDPSMWINDPIGT

PGSNEPGNGAPSSSSQLFSKSIQFENGSSSTITENPNLDPT

PSPVHSQTQNPKFNNTFSRELNFSTSSSTLVKPRSGEILNF

GDEGKRSSGNPDPSSYSGQTQFENKRKRSMVLNEDKVLSFG

DKTAGESDHSDLEASVVKEVAVEKRPKKRGRKPANGREEPL

NH**VEAERQRREKLNQRFYALRAVVPNVSKMDKASLLGDAIA**
3

**YINELKSKVVK**TESEKLQIKNQLEEVKLELAGRRASASGGD
3

MSSSCSSIKPVGMEIEVKIIGWDAMIRVESSKRNHPAARLM
5

SALMDLELEVNHASMSVVNDLMIQQATVKMGFRIYTQEQLR
6

ASLISKIG

**FIGURE 1**

```
NP_001065478    ------------------------------MNLWTDDNASMMEAFMASADLPAFPWGAAS
EAY79619        -----------------------------------------MEAFMASADLPAFPWGAAS
AAD15818        --------------------------------GTRDDNASMMEAFMASADLPTFPWGAPA
AAB00686        -------MTEYRSPPTMN--------------LWTDDNASVMEAFMSS-SDFSSLWLPTP
ABD59338        -------MTDYRSLPTMNNS------------IWTDDNSSVMEAFMSTTADLSSIWLPPP
Pt29.195#1      -------MTDYRLPPTMN--------------LWTDDNASVMEAFMNS-SDLSSLWAPPP
CAF74710        -------MTEYSLP-TMNLWNNSTSDDNV-SMMEAFMSSDLSFWATTNSTTTNSASAAVV
AAF04917        ------------------------------------------------------------
AAQ14332        -------MTDYRLQPKMNLWGTTTNTAAS-PIITSDDNSSMMEAFMTSSDPISLWPPSMS
AAY90122        ------MMNLWSSDDNVAFADAFMSLDSSSAAAWPPAPSSHQYNHQYNLQHQQQLQHQPQ
AAL55713        -------MTDYRLQPTMN------------LWTT-DDNASMMEAFMSS-SDISTLWPPAS
ABD65632        MSSTNVQLTDHHLNQSTNGTN---------LWSTIEDNASVMEPLIG--SEHSSLWPQPP
ABK94979        -----------------------------------------MEELIISPSSSSSP-----
EDQ81347        --------------------------MSQLLNAWEVADSAMIEAFMGTGYNCVEGFEVQD

NP_001065478    TP-------PPPPPPPHHHQQQQQQVLPPPAAAPAAAAFNQDTLQQRLQSIIEGSRETW
EAY79619        TP-------PPPPPPPHHHQQQQQQVLPPPAAAPAAAAFNQDTLQQRLQSIIEGSRETW
AAD15818        G----------------GGNSSAAAASPPPQMPAAMAPGFNQDTLQQRLQAMIEGSRETW
AAB00686        -----------QSAASTTTPGADTARALPPPPPSQSQSLFNQETLQQRLQTLIEGAEESW
ABD59338        -----------NSAASTTTPGPDTTK-----PPPQQQPLFNQETLQHRLQALIEDAKENW
Pt29.195#1      -----------QSSASTSTPSAAAQ--------PSEKTMLNQETLQQRLQTLIEGACEGW
CAF74710        GVNSNLLHTNNNNPSVFPLSSSTSVSAAAAVDATKSMPFFNQETLQQRLQALIDGARETW
AAF04917        ------------------------------------------------------------
AAQ14332        ---------VNHHHPPTPTSSAVTTAVDSAKSMPAQPAFFNQENLQQRLQTLIDGARESW
AAY90122        --------QQQQQQMANSAGGGWQQAMAGGAQMNPAQMMNQDSLQQRLQALIDDARESW
AAL55713        ----------------TTTTTATTETTPTPAMEIPAQAGFNQETLQQRLQALIEGTHEGW
ABD65632        ----------------LT----------------PPPPHVTEDTLQQRLQALIEGARESW
ABK94979        ----------------------------------VSLSQETPPTLQQRLQFIVQNQPDWW
EDQ81347        --------------------------------DPDGQLHLNESVLLRRLHSLVEESTVDW
```

**FIGURE 2**

```
NP_001065478     TYAIFWQSSIDVSTG-----ASLLGWGDGYYKGCDDDKRKQRSSTPAAAA-------EQE
EAY79619         TYAIFWQSSIDVSTG-----ASLLGWGDGYYKGCDDDKRKQRSSTPAAAA-------EQE
AAD15818         TYAIFWQSSLDSATG-----ASLLGWGDGYYKGCDEDKRKQKPLTPSAQA-------EQE
AAB00686         TYAIFWQSSYDYSSS-----TSLLGWGDGYYKGEEDKGKGKAPKEMSSA--------EQD
ABD59338         TYAIFWQTSYDYSTS-----RQLLGWGDGYYKGEDDKE--KAKKVILPE--------QQA
Pt29.195#1       AYAIFWQSSYDYSG------ASVLGWGDGYYKGEEDKGKTRTRNSASSAV-------EQE
CAF74710         TYAIFWQSSVVDFSS-----PSVLGWGDGYYKGEEDKAKRKLAVSSPAYIA------EQE
AAF04917         ------------------------------------------------------------
AAQ14332         TYAIFWQSSVVEFAG-----PSVLGWGDGYYKGEEDKGKRKNSSS-ASSFA------EQE
AAY90122         TYAIFWQCNVEPTG------QSLLGWGDGYYKGDDSANKNASSAAPAAGSRPPKNPAEQE
AAL55713         TYAIFWQPSYDFSG------ASVLGWGDGYYKGEEDKANPRRRSSSPPF----STPADQE
ABD65632         TYAVFWQLSHDFAGEDISNTAALLTWGDGYYKGEEERKS-RKRKPNP------VSAAEQE
ABK94979         SYAIFWQTSNDDSGR------IFLGWGDGHFQGSKDTSPKPNTFSNSRMTIS-----NSE
EDQ81347         TYAIFWQLSALREGE------MMLGWGDGYFRSAKENEINDARNMKGGSQEE-----DQQ

NP_001065478     HRKRVLRELNSLIAGAG-------AAPDEAVEEEVTDTEWFFLVSMTQSFPNGLGLPGQA
EAY79619         HRKRVLRELNSLIAGAG-------AAPDEAVEEEA-------------------------
AAD15818         HRKRVLRELNSLISGAA-------AAPDEAVEEEVTDTEWFFLVSMTQSFLNGSGLPGQA
AAB00686         HRKKVLRELNSLISGP--------FRSADDVDEEVSDTEWFFLVSMTQSFLSGSGLPGQA
ABD59338         HRNKVLRELNALISGS--------SSSDDVVDEDVTDTEWFFLTSMTHSFVNGSGLLSQA
Pt29.195#1       HRKTVLRKLNSLIAGPN-------SVTDDAIDEEVTDTEWFFLVSMTQSFVNGSGLPGQA
CAF74710         HRKKVLRELNSLISGAP-------AGTDDAVDEEVTDTEWFFLISMTQSFVNGSGLPGQA
AAF04917         ------------------------------------------------------------
AAQ14332         HRKKVLRELNSLIAGPQ-------GTADDAVDEEVTDTEWFFLISMTQSFVSGSGLPGQA
AAY90122         HRRRVLRELNSLISGSS-------SPQNDAVDDDVTDTEWFFLISMTQAFPFGVDLPGQA
AAL55713         YRKKVLRELNSLISGG---VAPS----DDAVDEEVTDTEWFFLVSMTQSFACGAGLAGKA
ABD65632         HRKRVIRELNSLISGGGGTVSSSGGSSDEAGDEDVSDTEWFFLVSMTQSFVNGSGLPGRA
ABK94979         RKRVMMKGIQSLIGECHD------LDMSLMDGNDATDSEWFYVMSLTRSFSPGDGILGKA
EDQ81347         MRRKVLRELQALVNGSE-----------DDVSDYVTDTEWFYLVSMSHSYAAGVGTPGRA
```

**FIGURE 2**

```
NP_001065478    LFAAQPTWIAT--GLSSAPCDRARQAYTFG---LRTMVCLPL--ATGVLELGSTDVIFQT
EAY79619        LFAAQPTWIAT--GLSSAPCDRARQAYTFG---LRTMVCLPL--ATGVLELGSTDVIFQT
AAD15818        LFAGQPTWIAS--GLSSAPCERARQAYNFG---LRTMVCFPV--GTGVLELGSTDVVFKT
AAB00686        FLNSSPVWVAGADRLSDSTSERARQGQVFG---VQTLVCIPS--ANGVVELASTEVIFQN
ABD59338        YFNSSPVWIN--DRLSMSTCERTRAAHVHG---LQTLVYIPAPSSNGVVELASTEIIPHS
Pt29.195#1      LFNGSPVWVAGSERLGASPCERARQGQVFG---LQTLVCIPS--ASGVVELGSTELIFQS
CAF74710        LYSSSPIWVAGTEKLAASHCERVRQAQGFG---LQTIVCIPS--ANGVVELGSTELIVES
AAF04917        ------------------------------------------------------------
AAQ14332        LYNSNPVWVTGAGRLAVSHCDRARQAQSFG---LQTLVCIPS--ANGVVELGSTELIFQS
AAY90122        ILGSNPIWAYGSDRLAGSPWDRARQGAAFG---LQTIVCIPS--GTGVLELGSTELVFNS
AAL55713        FATGNAVWVSGSDQLSGSGCERAKQGGVFG---MHTIACIPS--ANGVVEVGSTEPIRQS
ABD65632        FSSSRTIWLSGSNALAGSSCERARQGQVYG---LETMVCIPT--QNGVVELGSLEIIHQS
ABK94979        YTTGSLIWLTGGHELQFYNCERVKEAQMHG---IETLVCIPT--SCGVLELGSSSVIREN
EDQ81347        LASDRPVWLIGANKAPDNNCSRVQLAKVHSSMILQTILCIPS--KSGVVELGSTDLAKS-

NP_001065478    GDSIPRIRALFNLSAAAAS------SWPPHPDAAS------------ADPSVLWLADAPP
EAY79619        GDSIPRIRALFNLSAAAAS------SWPPHPDAAS------------ADPSVLWLADAPP
AAD15818        AESMAKIRSLFGGGAGGGS------WPPVQPQAPSSQQPAAGADHAETDPSMLWLADAPV
AAB00686        SDLMKKVRDLFNFNN------------PDAGF---WPLN-----QGENDPSSLWLNPSSS
ABD59338        AGIMEKVRFLFDFNN------------PEARS---WPLN-----SADNDPSSMWLDIPGS
Pt29.195#1      SDLMNKVRVLFDFNS------------LEVVS---WPIGTTNTDQGENDPSSFWLTDPET
CAF74710        SDLMNKVRVLFNFSN------------DLGSGSWAV--------QPESDPSALWLTEPSS
AAF04917        ------------------------------------------------------------
AAQ14332        SDLMNKVRILFNFNN------------IDLGSSSGP--------WPENDPSSLWLTDPSP
AAY90122        SVLMNKVRVLFNFGSGDASLLTAAASSSAPAAAPPPPISTATTDEAENDPAALWISDPSS
AAL55713        SDLINKVRILFNFDG------------GAG--DLSGLNWNLDPDQGENDPS-MWINDPIG
ABD65632        SDLVEKVNSFFSFNG------------GGGGGESGSWEFNLNPDQGENDSA-TWINEPIV
ABK94979        WGLVQQAKSLF-----------------------------------GSDLSAYLVP----
EDQ81347        WEVVQNVKMVFDEPM----------------------------MWAAHEIQAVAHSLPLS
```

FIGURE 2

```
NP_001065478     --MDMKDSIS-------AADISVSKPPPPPPHQIQHFENGSTSTLTENPSPSVHAPTPSQ
EAY79619         --MDMKDSIS-------AADISVSKPPPPPPHQIQHFENGSTSTLTENPSPSVHAPTPSQ
AAD15818         --MDIKDSLSHP-----SAEISVSKPPPHPPQ--IHFENGSTSTLTENPSPSVHAPPPPP
AAB00686         --IEIKDTSNA-----VALVS----ANASLSKTMPFETPGSS-TLTETPS----A-----
ABD59338         GGIEIRDSINT-----VSAVSVTASANATIPKKSPFEIHGASTTLPESSTTVNIS-----
Pt29.195#1       -----KDGNGG-----IPWNLN----GSDQNKNNHHSSNQSSSSLTDHLG--GIH-----
CAF74710         SGMEVRESLNT-----VQTNSVPSSNSNKQIAYANENNHQSG-----NGQSCYNL-----
AAF04917         ----------------------------------------SG-----NGQSCYN------
AAQ14332         SGVGVKEGVNTNNNTSVQGNSIPSGNKQQLVFGNNDNHPTTSTLTDHPGAGAVNS-----
AAY90122         SAAEVKEALNPR--ITVRESSIPIGSNSIPVHQPAKPPQLDVQSSIGLTENSIGI-----
AAL55713         TPGSNEPGNGAP------------SSSSQLFSKSIQFENGSSSTITENPNLDPTP-----
ABD65632         TG--IEPVLGAP------------ATSNSDSQTASKLCNGSS---VEHP-----------
ABK94979         ------------------------------------------------------------
EDQ81347         --------------------------SDATSMRPSSPSLMSIATISASISNASQIG----

NP_001065478     PAAPPQRQQQQQQSSQAQQGPFRRELNFSDFASN----GGAAAPPFFKPETGEILNFGND
EAY79619         PAAPPQRQQQQQQSSQAQQGPFRRELNFSDFASN----GGAAAPPFFKPETGEILNFGND
AAD15818         APAAPQQRQHQHQN-QAHQGPFRRELNFSDFASTP---SLAATPPFFKPESGEILSFGAD
AAB00686         ------AAAAHVPNP-KNQGFFPRELNFSNS---------------LKPESGEILSFG--
ABD59338         ------TAQRQIQNQNQNQSFFPRELNFSGS---------------FKPESGEILNFG--
Pt29.195#1       ------HAQNHQQQPIHARSLFTRELNFGECSTYDGSSVRNGNSHLTKPESGEILNFG--
CAF74710         ------QQQQNNPPQQQTQGFFTRELNFSEFGFDGSSNRNGNASLSCKPESGEILNFG--
AAF04917         ------QQQQKNPPQQQTQGFFTRELNFSEFGFDGSSNRNGNSSVSCKPESGEILNFG--
AAQ14332         ------YNNSSQNAQQPQGSFFTRELNFSEYGFERSSVKNGN----CKPESGEILNFG--
AAY90122         ------HSQKSHNQPLQHQGFFTKELNFSEFA--------------MKPESGEILNFG--
AAL55713         ------SPVHSQTQNPKFNNTFSRELNFSTSS-----------STLVKPRSGEILNFG--
ABD65632         ----------KQQQNPQISSSGFVEGDSNKKK-----------RCLVSDKEEEMLSFT--
ABK94979         --------KGPNNSSEEPTQFLDRSISFADMG--------------IIAGLQEDCAVD--
EDQ81347         --------RCSNPSQDHEAHFVGRKNPPSSRPPMR----------FYKPRVEELSSDTP-
                                                                 *
```

**FIGURE 2**

```
NP_001065478    SSSGRRNPSPA-PPAATASLTTAPGSLFSQHTPTLTAAANDAKSNNQKRSMEATSRASNT
EAY79619        SSTGRRNPSPA-PPAATASLTTAPGSLFSQHTPTLTAAANDAKSNNQKRSMEATSRASNT
AAD15818        SN-ARRNPSPV-PPAATASLTTAPGSLFSQHTATMTAAAANDAKNNNKRSMEATSRASNT
AAB00686        -------------ESKKSSY---NGSYFPG------VAAEETNK--KRRSPASRS-----
ABD59338        -------------ESKKSSYSSANGNFFSGP---SPFAANEENR--KRRSPVSRS-----
Pt29.195#1      -------------ESKRTASS-ANGNFYSG------LVTEENNK--KKRSVGN-------
CAF74710        -------------DSTKKSASSANVNLFTGQSQFGAVEENNNNKN-KKRSATSRG-----
AAF04917        -------------DSTKKSASSANVNLFTGQSQFWGLGEENNNKN-QEKISYFRG-----
AAQ14332        -----------GESVTKKNSVSGNGNLFSVQSQFG-AGEENKNK--KRPSPVSRG-----
AAY90122        -------------ESKRNSLG--NGNGLNSQ---FLVEESNKNIISKKRSPTSRG-----
AAL55713        -------------DEGKRSSGNPDPSSYSGQ-----TQFENKRKR-------SMV-----
ABD65632        -------------S----------------------------------------------
ABK94979        -------------REQKNAR-----------------ETEEANKR---------------
EDQ81347        -------------ETNLVDNKYIVGKSVSFHHLSKIGQRGMPGPP---------------

NP_001065478    NNHPAATANEGMLSFSSAPTTRPSTGTG------APAKSESDHSDLEAS-VREVESSRVV
EAY79619        NNHPAATANEGMLSFSSAPTTRPSTGTG------APAKSESDHSDLEAS-VREVESSRVV
AAD15818        NHHPAATANEGMLSFSSAPTTRPSTGTG------APAKSESDHSDLDAS-VREVESSRVV
AAB00686        ------SIDDGMLSFTSGVIIPASNIKSGAVAGGGASGGDSENSDLEASVVKEADS-RVV
ABD59338        ------SIEDGILSFSSGKLLHGSTIKSG--------GGDSDHSDLEVSVVKKTVSSRVI
Pt29.195#1      --------EEGMLSFTSGVILPSSCILKSS----GGTGGDSDHSDLEASVVKEADSSRVV
CAF74710        ------SNEEGMLSFVSGTVLPSSGMK-----SGGGGGEDSEHSDLEASVVKEADSSRVV
AAF04917        ------SNEEGMLSFVSGTVC-FFGHE-----VRWRRRQDSEHSDLEASVVKEADSSRVV
AAQ14332        ------SNDEGMLSFTSGVVLPSTGVVKS---SGGGGGGDSDHSDLEASVVKEAESSRVV
AAY90122        ------SAEEGMLSFTSSVVLPSSMAVKSS----ATGAGDSDHSDLEASVVKEADSSRVV
AAL55713        ------LNEDKVLSFGD------------------KTAGESDHSDLEASVVKEVA-----
ABD65632        -----------VLPLPT------------------KSN-DSNRSDLEASVVKEAESGRIV
ABK94979        ------NANKPGLSYLN----------------------SEHSDSDFPLLAMHMEKR--
EDQ81347        ------TTANRLPCFAP-------------------SEVDCHESEADVSVKENVVESSTN
                                                          ...*: :  .
```

**FIGURE 2**

```
NP_001065478    APPPEAEKRPRKRGRKPANGREEPLNHVEAERQRREKLNQRFYALRAVVPNVSKMDKASL
EAY79619        APPPEAEKRPRKRGRKPANGREEPLNHVEAERQRREKLNQRFYALRAVVPNVSKMDKASL
AAD15818        APPPEAEKRPRKRGRKPANGREEPLNHVEAERQRREKLNQRFYALRAVVPNVSKMDKASL
AAB00686        E----PEKRPRKRGRKPGNGREEPLNHVEAERQRREKLNQRFYALRAVVPNVSKMDKASL
ABD59338        E----PEKRPRKRGRKPANGREEPLNHVEAERQRREKLNQRFYALRAVVPNVSKMDKASL
Pt29.195#1      E----PEKRPRKRGRKPANGREEPLNHVEAERQRREKLNQRFYALRAVVPNVSKMDKASL
CAF74710        E----PEKRPRKRGRKPANGREEPLNHVEAERQRREKLNQRFYALRAVVPNVSKMDKASL
AAF04917        E----PEKRPRKRGRKPANGREEPLNHVEAERQRREKLNQRFFSLRAVVPNVSKMDKASL
AAQ14332        D----PEKRPRKRGRKPANGREEPLNHVEAERQRREKLNQRFYALRAVVPNVSKMDKASL
AAY90122        D----PEKRPRKRGRKPANGREEPLNHVEAERQRREKLNQRFYALRAVVPNVSKMDKASL
AAL55713        -----VEKRPKKRGRKPANGREEPLNHVEAERQRREKLNQRFYALRAVVPNVSKMDKASL
ABD65632        AE---TEKKPRKRGRKPANGREEPLNHVEAERQRREKLNQRFYSLRAVVPNVSKMDKASL
ABK94979        --------IPKKRGRKPGLGRDAPLNHVEAERQRREKLNHRFYALRAVVPNVSRMDKASL
EDQ81347        -----LEPKPRKRGRKPANDREEPLNHVQAERQRREKLNQKFYALRSVVPNVSKMDKASL
                         *:******. .*: *****:**********::*::**:******:******

NP_001065478    LGDAISYINELRGKLTALETDKETLQSQMESLKKERDARPPAPSG---------------
EAY79619        LGDAISYINELRGKLTALETDKETLQSQMESLKKERDARPPAPSG---------------
AAD15818        LGDAISYINELRGKLTSLETDKETLQTQVEALKKERDARPPSHSAGL-------------
AAB00686        LGDAISYINELKSKLSELESEKGELEKQLELVKKELELATKSPSPPPG-----------P
ABD59338        LGDAISYINELKLKLQGLESSKDELEKELDTTRKELEIATKKPVRLN-------------
Pt29.195#1      LGDAISYIDELRTKLQSAESSKEELEKQVESMKRELVSKDSSPPPKE-------------
CAF74710        LGDAISYINELKSKLQNTESDKEDLKSQIEDLKKE--SRRPGPPPP--------------
AAF04917        LGDAISYINELKSKLQNTESDKEDLKSQIEDLKKE--SRRPGPPPPP-------------
AAQ14332        LGDAISYINELKAKLQTTETDKDELKNQLDSLKKELASKESRLLSSP-------------
AAY90122        LGDAISFINELKSKLQNVESEKETLLSQVECLKTEVLASRDHQSRSSNGGGGVQNHHHPS
AAL55713        LGDAIAYINELKSKVVKTESEKLQIKNQLEEVKLELA-GRRASASGG-------------
ABD65632        LGDAISYINELKAKLQKAEADKEELQKQIDGMSKEVGDGNVKSLVKD-------------
ABK94979        LSDAVSYINELKAKVDELESQLERESKKVKLEVADNLDNQSTTTSVD-------------
EDQ81347        LEDAITYINELQEKLQKAEAELKVFQRQVLASTGESKKPNPSRRDSTESSD--EERFRLQ
                * **:::*:**: *:    *:.        ::      :
```

FIGURE 2

```
NP_001065478    -----GGGDGGARCHAVEIEAKILGLEAMIRVQCHKRNHPAARLMTALRELDLDVYHASV
EAY79619        -----GGGDGGARCHAVEIEAKILGLEAMIRVQCHKRNHPAARLMTALRELDLDVYHASV
AAD15818        -----GGHDGGPRCHAVEIDAKILGLEAMIRVQCHKRNHPSARLMTALRELDLDVYHASV
AAB00686        PPSNKEAKETTSKLIDLELEVKIIGWDAMIRIQCSKKNHPAARLMAALKELDLDVNHASV
ABD59338        -EEEKEKPENNSKLIDLDIDVKIMGWDAMIRIQCSKKNHPAAKLMAALKELDLDVNHASV
Pt29.195#1      --ELKMSNNEGVKLIDMDIDVKISGWDAMIRIQCCKKNHPAARLMSALRDLDLDVQYANV
CAF74710        NQDLKIG----GKIVDVDIDVKIIGWDAMIGIQCNKKNHPAARLMAALMELDLDVHHASV
AAF04917        NQDLKMSSHTGGKIVDVDIDVKIIGWDAMIRIQCNKKNHPAK---ANGSAHGIRPRRASC
AAQ14332        DQDLKSSNKQSVGNLDMDIDVKIIGREAMIRVQSSKNNHPAARVMGALKDLDLELLHASV
AAY90122        LEQDMNMLNGSCKQSDLDVDVKIIGRDAMVRVNCSKSNHPAARLMVALKELDLEVTHASV
AAL55713        ---DMSSSCSSIKPVGMEIEVKIIGWDAMIRVESSKRNHPAARLMSALMDLELEVNHASM
ABD65632        ---QKCLDQDSGVSIEVEIDVKIIGWDAMIRIQCAKKNHPGAKFMEALKELELEVNHASL
ABK94979        -QSACRPNSAGGAGLALEVEIKFVGNDAMIRVQSENVNYPASRLMCALRELEFQVHHASM
EDQ81347        ESGQRSAPLVHTSENKPVISVFVLGEEAMIRVYCTRHSNFIVHMMSALEKLRLEVIHSNT
                                  :.  . * :**: : . . .                :     :.

NP_001065478    SVVKDLMIQQVAVKMASRVYSQDQLNAALYTRIAEPGTAAR-
EAY79619        SVVKDLMIQQVAVKMASRVYSQDQLNAALYTRIAEPGTAAR-
AAD15818        SVVKDLMIQQVAVKMASRVYTQDQLSAALYSRLAEPGSAMGR
AAB00686        SVVNDLMIQQATVNMGNRFYTQEQLRSARSSKIGNAL-----
ABD59338        SVVNDLMIQQASINMGSRFYTQEQLLSVLSSKIGDTQ-----
Pt29.195#1      SVMNDLMIQQATVKMGSRFYTQEELRVAISTNVGGVH-----
CAF74710        SVVNDLMIQQATVKMGSRHYTEEQLRVALKSKIAETPLESR-
AAF04917        QCFSCQRFDDPTSHSENG------------------------
AAQ14332        SVVNDLMIQQNTVRMGSRFYTQEQLRIALTSRIAGNSMRLLV
AAY90122        SVVNDLMIQQATVRMGSRYYSPDHLRMVLEAKVSDIRG----
AAL55713        SVVNDLMIQQATVKMGFRIYTQEQLRASLISKIG--------
ABD65632        SVVNEFMIQQATVKMGNQFFTQDQLKAALMERV---------
ABK94979        SCVNELMLQDVVVRVPDGLRTEEALKSALLGRLE--------
EDQ81347        SSMKDMLLHVVIVKVR--------------------------
                . ..   :.     .
```

FIGURE 2 (continued)

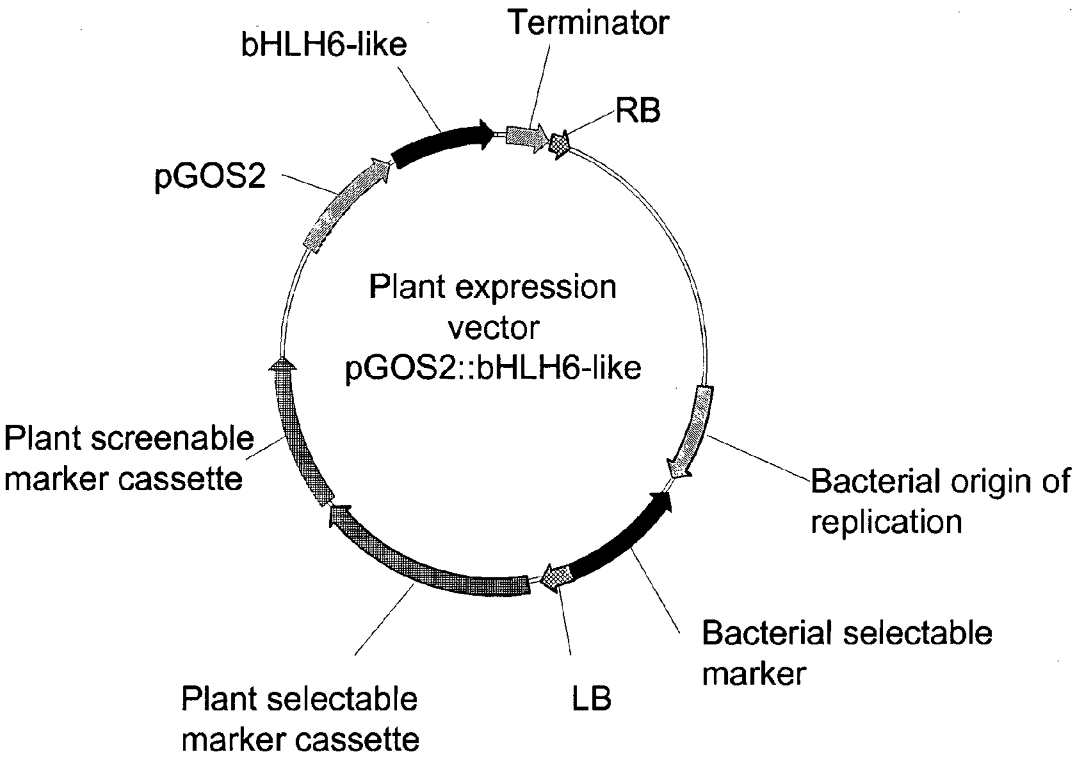
bHLH6-like
Terminator
RB
pGOS2
Plant expression vector pGOS2::bHLH6-like
Plant screenable marker cassette
Bacterial origin of replication
Bacterial selectable marker
Plant selectable marker cassette
LB

FIGURE 3

**SEQ ID NO: 1, Arabidopsis thaliana bHLH6 encoding sequence**
ATGACTGATTACCGGCTACAACCAACGATGAATCTTTGGACCACCGACGACAACGCTTCTATGATG
GAAGCTTTCATGAGCTCTTCCGATATCTCAACTTTATGGCCTCCGGCGTCGACGACAACCACGACG
GCGACGACTGAAACAACTCCGACGCCGGCGATGGAGATTCCGGCACAGGCGGGATTTAATCAAGAG
ACTCTTCAGCAACGTTTACAAGCTTTGATTGAAGGAACACACGAAGGTTGGACCTACGCTATATTC
TGGCAACCGTCGTATGATTTCTCCGGCGCCTCCGTGCTCGGATGGGGAGATGGTTATTACAAAGGT
GAAGAAGATAAAGCAAACCCGAGACGGAGATCGAGTTCGCCGCCGTTTTCTACTCCGGCGGATCAG
GAGTACAGGAAAAAAGTGTTGAGAGAGCTTAACTCGTTGATCTCCGGTGGTGTTGCTCCGTCGGAT
GACGCTGTTGATGAGGAGGTGACGGATACGGAATGGTTTTTCTTGGTTTCGATGACGCAGAGCTTC
GCTTGCGGTGCGGGATTAGCTGGTAAAGCGTTTGCAACGGGTAACGCGGTTTGGGTTTCCGGGTCA
GATCAATTATCCGGGTCGGGTTGTGAACGGGCTAAGCAAGGAGGAGTGTTTGGGATGCATACTATT
GCGTGTATTCCTTCGGCGAACGGAGTTGTGGAAGTCGGGTCAACGGAGCCGATCCGACAGAGTTCG
GACCTTATTAACAAGGTTCGAATTCTTTTCAATTTCGACGGCGGAGCTGGAGATTTATCGGGTCTT
AATTGGAATCTTGACCCGGATCAAGGTGAGAACGACCCGTCTATGTGGATTAATGACCCGATTGGA
ACACCTGGATCTAACGAACCGGGTAACGGAGCTCCAAGTTCTAGCTCCCAGCTTTTTTCAAAGTCT
ATTCAGTTTGAGAACGGTAGCTCAAGCACAATAACCGAAAACCCGAATCTGGATCCGACTCCGAGT
CCGGTTCATTCTCAGACCCAGAATCCGAAATTCAATAACACTTTCTCCCGAGAACTTAATTTTTCG
ACGTCAAGTTCTACTTTAGTGAAACCAAGATCCGGCGAGATATTAAACTTCGGCGATGAAGGTAAA
CGAAGCTCCGGAAACCCGGATCCAAGTTCTTATTCGGGTCAAACACAATTCGAAAACAAAAGAAAG
AGGTCGATGGTTTTGAACGAAGATAAAGTTCTATCATTCGGAGATAAAACCGCCGGAGAATCAGAT
CACTCCGATCTAGAAGCTTCCGTCGTGAAAGAAGTAGCAGTAGAGAAACGTCCAAAGAAACGAGGA
AGAAAGCCAGCAAACGGTAGAGAAGAGCCACTAAACCACGTCGAAGCAGAGAGACAAAGACGCGAG
AAACTAAACCAAAGATTCTACGCGTTACGAGCGGTTGTACCAAACGTTTCAAAAATGGATAAAGCT
TCGTTACTCGGTGACGCAATCGCTTACATCAACGAGCTTAAATCCAAAGTAGTCAAAACAGAGTCA
GAGAAACTCCAAATCAAGAACCAGCTCGAGGAAGTGAAACTCGAGCTCGCCGGAAGAAAAGCGAGT
GCTAGTGGAGGAGATATGTCGTCTTCGTGTTCTTCGATTAAACCGGTGGGGATGGAGATTGAAGTG
AAGATAATTGGTTGGGACGCAATGATTAGAGTTGAATCTAGTAAGAGGAATCATCCGGCGGCGAGG
TTGATGTCGGCGTTGATGGATTTGGAGTTGGAAGTGAATCACGCGAGTATGTCGGTGGTTAACGAT
TTGATGATTCAACAAGCGACGGTGAAGATGGGTTTTAGGATCTATACGCAAGAACAGCTCAGAGCA
AGTTTGATTTCAAAAATCGGTTAA

**SEQ ID NO: 2, Arabidopsis thaliana bHLH6**
MTDYRLQPTMNLWTTDDNASMMEAFMSSSDISTLWPPASTTTTATTETTPTPAMEIPAQAGFNQE
TLQQRLQALIEGTHEGWTYAIFWQPSYDFSGASVLGWGDGYYKGEEDKANPRRRSSSPPFSTPADQ
EYRKKVLRELNSLISGGVAPSDDAVDEEVTDTEWFFLVSMTQSFACGAGLAGKAFATGNAVWVSGS
DQLSGSGCERAKQGGVFGMHTIACIPSANGVVEVGSTEPIRQSSDLINKVRILFNFDGGAGDLSGL
NWNLDPDQGENDPSMWINDPIGTPGSNEPGNGAPSSSSQLFSKSIQFENGSSSTITENPNLDPTPS
PVHSQTQNPKFNNTFSRELNFSTSSSTLVKPRSGEILNFGDEGKRSSGNPDPSSYSGQTQFENKRK
RSMVLNEDKVLSFGDKTAGESDHSDLEASVVKEVAVEKRPKKRGRKPANGREEPLNHVEAERQRRE
KLNQRFYALRAVVPNVSKMDKASLLGDAIAYINELKSKVVKTESEKLQIKNQLEEVKLELAGRKAS
ASGGDMSSSCSSIKPVGMEIEVKIIGWDAMIRVESSKRNHPAARLMSALMDLELEVNHASMSVVND
LMIQQATVKMGFRIYTQEQLRASLISKIG

**SEQ ID NO: 3, Motif 1**
L(G/E/T)WX(D/E)(G/S)X(Y/F)(K/N)G(E/D)

**SEQ ID NO: 4, Motif 2**
G(V/I)(V/I/L)E(V/L/I)(G/A)(S/V/T/A)(T/L/S)(E/D/S)

## FIGURE 4

**SEQ ID NO: 5, Motif 3**
(D/E)K(A/V/I)S(L/I/V)L(G/D/E/A)

**SEQ ID NO: 6, Motif 4**
(T/N/S)LQ(Q/H)RLQ

**SEQ ID NO: 7, Motif 5**
(K/F)(I/F/V)(I/L/V/M/S)G(W/L/R/N/E)(E/D)AM(I/V)(G/R)(V/I)(Q/N/E/Y)

**SEQ ID NO: 8, Motif 6**
(H/Y)(A/S)(S/N)(V/C/M/L/T)(S/Q)(V/C/S)(V/MF)(K/N/S)(D/C/E)(L/Q/F/M
)(M/R/L)(I/F/L)(Q/D/H)(Q/D/V)

**SEQ ID NO: 9, Motif 7**
PKKRGRKPANGREEPLNHVEAERQRREKLNQRFYALRAVVPNVSKMDKASLLGDAIAYINELKSKV
VKTE

**SEQ ID NO: 10, forward primer prm06515**
ggggacaagtttgtacaaaaaagcaggcttaaacaatgactgattaccggctacaa

**SEQ ID NO: 11, reverse primer prm06516**
ggggaccactttgtacaagaaagctgggtacaccccttttaaccgatttt

**SEQ ID NO: 12, rice GOS2 promoter**
AATCCGAAAAGTTTCTGCACCGTTTTCACCCCCTAACTAACAATATAGGGAACGTGTGCTAAATAT
AAAATGAGACCTTATATATGTAGCGCTGATAACTAGAACTATGCAAGAAAAACTCATCCACCTACT
TTAGTGGCAATCGGGCTAAATAAAAAAGAGTCGCTACACTAGTTTCGTTTTCCTTAGTAATTAAGT
GGGAAAATGAAATCATTATTGCTTAGAATATACGTTCACATCTCTGTCATGAAGTTAAATTATTCG
AGGTAGCCATAATTGTCATCAAACTCTTCTTGAATAAAAAAATCTTTCTAGCTGAACTCAATGGGT
AAAGAGAGAGATTTTTTTTAAAAAAATAGAATGAAGATATTCTGAACGTATTGGCAAAGATTTAAA
CATATAATTATATAATTTTATAGTTTGTGCATTCGTCATATCGCACATCATTAAGGACATGTCTTA
CTCCATCCCAATTTTTATTTAGTAATTAAAGACAATTGACTTATTTTTATTATTTATCTTTTTTCG
ATTAGATGCAAGGTACTTACGCACACACTTTGTGCTCATGTGCATGTGTGAGTGCACCTCCTCAAT
ACACGTTCAACTAGCAACACATCTCTAATATCACTCGCCTATTTAATACATTTAGGTAGCAATATC
TGAATTCAAGCACTCCACCATCACCAGACCACTTTTAATAATATCTAAAATACAAAAAATAATTTT
ACAGAATAGCATGAAAAGTATGAAACGAACTATTTAGGTTTTTCACATACAAAAAAAAAAAGAATT
TTGCTCGTGCGCGAGCGCCAATCTCCCATATTGGGCACACAGGCAACAACAGAGTGGCTGCCCACA
GAACAACCCACAAAAAACGATGATCTAACGGAGGACAGCAAGTCCGCAACAACCTTTTAACAGCAG
GCTTTGCGGCCAGGAGAGAGGAGGAGAGGCAAAGAAAACCAAGCATCCTCCTTCTCCCATCTATAA
ATTCCTCCCCCCTTTTCCCCTCTCTATATAGGAGGCATCCAAGCCAAGAAGAGGGAGAGCACCAAG
GACACGCGACTAGCAGAAGCCGAGCGACCGCCTTCTCGATCCATATCTTCCGGTCGAGTTCTTGGT
CGATCTCTTCCCTCCTCCACCTCCTCCTCACAGGGTATGTGCCTCCCTTCGGTTGTTCTTGGATTT
ATTGTTCTAGGTTGTGTAGTACGGGCGTTGATGTTAGGAAAGGGGATCTGTATCTGTGATGATTCC
TGTTCTTGGATTTGGGATAGAGGGGTTCTTGATGTTGCATGTTATCGGTTCGGTTTGATTAGTAGT
ATGGTTTTCAATCGTCTGGAGAGCTCTATGGAAATGAAATGGTTTAGGGATCGGAATCTTGCGATT
TTGTGAGTACCTTTTGTTTGAGGTAAAATCAGAGCACCGGTGATTTTGCTTGGTGTAATAAAGTAC
GGTTGTTTGGTCCTCGATTCTGGTAGTGATGCTTCTCGATTTGACGAAGCTATCCTTTGTTTATTC
CCTATTGAACAAAAATAATCCAACTTTGAAGACGGTCCCGTTGATGAGATTGAATGATTGATTCTT

**FIGURE 4 (continued)**

AAGCCTGTCCAAAATTTCGCAGCTGGCTTGTTTAGATACAGTAGTCCCCATCACGAAATTCATGGA
AACAGTTATAATCCTCAGGAACAGGGGATTCCCTGTTCTTCCGATTTGCTTTAGTCCCAGAATTTT
TTTTCCCAAATATCTTAAAAAGTCACTTTCTGGTTCAGTTCAATGAATTGATTGCTACAAATAATG
CTTTTATAGCGTTATCCTAGCTGTAGTTCAGTTAATAGGTAATACCCCTATAGTTTAGTCAGGAGA
AGAACTTATCCGATTTCTGATCTCCATTTTTAATTATATGAAATGAACTGTAGCATAAGCAGTATT
CATTTGGATTATTTTTTTTATTAGCTCTCACCCCTTCATTATTCTGAGCTGAAAGTCTGGCATGAA
CTGTCCTCAATTTTGTTTTCAAATTCACATCGATTATCTATGCATTATCCTCTTGTATCTACCTGT
AGAAGTTTCTTTTTGGTTATTCCTTGACTGCTTGATTACAGAAAGAAATTTATGAAGCTGTAATCG
GGATAGTTATACTGCTTGTTCTTATGATTCATTTCCTTTGTGCAGTTCTTGGTGTAGCTTGCCACT
TTCACCAGCAAAGTTC

**SEQ ID NO: 13, gi|1142618:193-2121 Phaseolus vulgaris phaseolin G-box binding protein PG1 (PG1) mRNA, complete cds**
ATGACGGAGTACCGGTCGCCGCCCACAATGAATCTCTGGACCGACGACAACGCTTCCGTCATGGAG
GCCTTCATGAGCTCCTCCGATTTCTCCTCCCTTTGGCTGCCAACACCGCAATCCGCTGCCTCTACC
ACCACTCCGGGAGCCGACACTGCCAGAGCCCTGCCGCCGCCTCCGCCGTCGCAGTCCCAGTCCCTT
TTTAACCAGGAGACCTTACAACAGCGGCTGCAAACGCTGATCGAAGGCGCCGAAGAGAGCTGGACC
TACGCCATTTTCTGGCAATCTTCTTATGACTACTCCTCCAGCACTTCCCTTCTCGGTTGGGGTGAC
GGATACTACAAGGGAGAGGAGGACAAAGGAAAAGGAAAAGCCCCCAAAGAGATGTCGTCGGCGGAG
CAGGACCACCGTAAGAAGGTTCTCCGCGAGCTCAATTCGTTGATTTCCGGCCCTTTCCGTTCCGCT
GATGACGTCGACGAAGAGGTATCCGACACTGAGTGGTTCTTTCTAGTGTCCATGACTCAGTCCTTT
CTCAGCGGAAGCGGGCTCCCGGGCCAGGCTTTTCTTAATTCCAGCCCGGTTTGGGTCGCCGGGGCT
GACCGGTTATCCGATTCGACGTCGGAGCGGGCTCGCCAGGGGCAGGTCTTTGGGGTTCAGACTCTG
GTTTGCATACCGTCCGCAAACGGCGTCGTTGAGCTGGCTTCTACGGAGGTGATTTTCCAGAACTCC
GATCTCATGAAGAAAGTGCGGGATTTGTTCAACTTCAACAACCCAGACGCGGGTTTCTGGCCGTTG
AATCAGGGCGAGAACGACCCTTCCTCACTCTGGCTCAACCCTTCTTCCTCGATTGAAATCAAGGAC
ACCTCTAACGCCGTTGCACTCGTTTCAGCGAATGCATCACTGAGCAAAACCATGCCGTTCGAAACC
CCTGGTTCCAGCACTCTAACAGAAACCCCTAGTGCCGCCGCCGCAGCTCACGTCCCCAATCCCAAG
AACCAGGGTTTCTTCCCCAGAGAATTGAACTTCTCGAACTCCTTAAAACCCGAATCCGGTGAAATT
CTGAGCTTCGGAGAGAGCAAGAAGAGCTCTTACAACGGGAGTTACTTCCCCGGTGTTGCGGCTGAG
GAGACCAACAAGAAGAGAAGGTCTCCTGCTTCTCGCAGCAGCATTGACGATGGAATGCTGTCCTTC
ACCTCCGGCGTGATTATACCGGCTTCGAATATAAAATCTGGTGCCGTTGCTGGTGGTGGTGCCAGC
GGCGGTGATTCCGAGAACTCGGATCTGGAAGCTTCCGTGGTGAAAGAGGCGGACAGCAGGGTGGTG
GAGCCGGAGAAACGGCCCCGGAAGAGGGGTCGGAAGCCGGGGAACGGCAGAGAGGAGCCGCTGAAT
CACGTGGAAGCGGAGAGGCAGAGGAGGGAAAAGCTGAATCAACGGTTCTACGCGCTTCGTGCGGTG
GTTCCCAACGTGTCGAAGATGGACAAAGCATCGCTCTTAGGCGATGCCATATCCTACATAAACGAG
CTGAAGTCGAAGCTGAGTGAGCTGGAATCAGAGAAAGGGGAATTGGAGAAGCAATTGGAGTTGGTG
AAGAAGGAGCTTGAGCTGGCAACTAAAAGCCCTTCTCCTCCACCTGGGCCACCTCCATCCAACAAA
GAAGCGAAGGAAACGACGAGCAAGCTGATTGATTTGGAGTTAGAGGTGAAGATCATAGGGTGGGAC
GCCATGATAAGGATCCAATGCAGCAAGAAAAACCACCCTGCGGCCAGGTTGATGGCTGCGTTGAAG
GAGCTGGACCTTGACGTGAATCATGCCAGCGTATCCGTGGTGAATGATTTGATGATCCAACAAGCC
ACCGTGAATATGGGAAACCGGTTTTACACTCAGGAACAGCTCCGGTCGGCGCGCTCCTCCAAAATT
GGCAATGCACTATAG

FIGURE 4 (continued)

**SEQ ID NO: 14, gi|1142619|gb|AAB00686.1| phaseolin G-box binding protein PG1, Phaseolus vulgaris**

MTEYRSPPTMNLWTDDNASVMEAFMSSSDFSSLWLPTPQSAASTTTPGADTARALPPPPPSQSQSL
FNQETLQQRLQTLIEGAEESWTYAIFWQSSYDYSSSTSLLGWGDGYYKGEEDKGKGKAPKEMSSAE
QDHRKKVLRELNSLISGPFRSADDVDEEVSDTEWFFLVSMTQSFLSGSGLPGQAFLNSSPVWVAGA
DRLSDSTSERARQGQVFGVQTLVCIPSANGVVELASTEVIFQNSDLMKKVRDLFNFNNPDAGFWPL
NQGENDPSSLWLNPSSSIEIKDTSNAVALVSANASLSKTMPFETPGSSTLTETPSAAAAAHVPNPK
NQGFFPRELNFSNSLKPESGEILSFGESKKSSYNGSYFPGVAAEETNKKRRSPASRSSIDDGMLSF
TSGVIIPASNIKSGAVAGGGASGGDSENSDLEASVVKEADSRVVEPEKRPRKRGRKPGNGREEPLN
HVEAERQRREKLNQRFYALRAVVPNVSKMDKASLLGDAISYINELKSKLSELESEKGELEKQLELV
KKELELATKSPSPPPGPPPSNKEAKETTSKLIDLELEVKIIGWDAMIRIQCSKKNHPAARLMAALK
ELDLDVNHASVSVVNDLMIQQATVNMGNRFYTQEQLRSARSSKIGNAL

**SEQ ID NO: 15, gi|33339704:194-2293 Catharanthus roseus MYC2 (myc2) mRNA, complete cds**

ATGACGGACTATAGGCTACAACCGAAAATGAACCTATGGGGTACGACGACCAACACCGCAGCTTCA
CCAATAATTACTTCAGATGATAATAGTTCGATGATGGAGGCTTTTATGACCTCATCAGATCCGATT
TCTTTGTGGCCGCCGTCAATGTCTGTGAATCATCACCATCCACCAACTCCTACTTCTTCCGCCGTA
ACAACTGCGGTGGACTCCGCTAAATCTATGCCTGCCCAACCTGCTTTTTTCAATCAAGAAAATCTC
CAACAGCGCCTTCAAACTCTAATTGATGGTGCTAGGGAGAGTTGGACTTATGCCATATTTTGGCAG
TCGTCTGTTGTCGAATTCGCCGGTCCTTCGGTCTTGGGTTGGGGCGATGGATATTATAAGGGAGAA
GAAGATAAAGGGAAGAGGAAGAATTCGTCTTCCGCGAGTTCTTTTGCAGAACAGGAACACAGAAAG
AAAGTCCTTAGAGAGCTCAATTCTTTGATTGCTGGCCACAAGGCACCGCCGATGATGCAGTTGAT
GAAGAGGTGACCGATACCGAATGGTTTTTCTTAATTTCAATGACTCAGTCATTTGTTTCCGGGAGC
GGTCTTCCAGGGCAGGCCTTATACAATTCAAACCCGGTATGGGTTACCGGAGCAGGGAGGCTGGCG
GTTTCACACTGCGACCGGGCCAGGCAGGCTCAAAGTTTTGGGCTTCAGACCTTAGTTTGTATTCCC
TCCGCAAACGGCGTTGTGGAGCTGGGTTCAACGGAATTGATTTTTCAGAGCTCCGATCTCATGAAT
AAGGTTAGGATACTGTTCAATTTTAATAATATAGATTTGGGTTCGAGCTCTGGACCTTGGCCTGAG
AACGATCCTTCTTCTCTGTGGCTTACTGATCCATCGCCCTCAGGGGTAGGGGTTAAGGAGGGGGTG
AATACTAATAATAATACTAGTGTTCAAGGGAATTCAATTCCTTCTGGTAATAAGCAGCAACTTGTG
TTTGGAAATAATGATAATCATCCAACCACGAGTACTTTGACTGATCATCCCGGGGCTGGGGCTGTA
AATAGTTATAATAATTCATCTCAGAATGCCCAGCAGCCTCAAGGTAGTTTCTTTACAAGGGAATTG
AATTTTTCAGAATACGGGTTTGAAAGGAGTAGTGTAAAGAATGGGAATTGTAAGCCAGAATCGGGA
GAAATATTGAACTTTGGTGGTGAATCTGTTACCAAGAAGAATTCTGTAAGTGGAAATGGGAACTTG
TTTTCAGTACAATCACAGTTTGGAGCTGGGGAGGAGAACAAGAACAAGAAAAGGCCATCTCCTGTG
TCAAGGGGGAGCAATGATGAGGGGATGCTTTCTTTTACTTCTGGGGTAGTTTTGCCCTCTACTGGT
GTAGTGAAGTCTAGTGGTGGTGGTGGTGGTGGAGACTCCGATCATTCTGATCTTGAGGCCTCAGTG
GTGAAGGAGGCAGAGAGTAGTAGGGTTGTGGATCCCGAGAAACGGCCTAGGAAGAGGGGGAGAAAG
CCTGCAAATGGAAGGGAAGAGCCATTGAATCATGTGGAGGCAGAGAGGCAGAGGAGGGAGAAGTTG
AACCAGAGGTTCTATGCTCTTAGAGCCGTGGTTCCTAATGTTTCAAAAATGGATAAAGCTTCACTT
CTTGGTGATGCTATTTCTTATATCAATGAGTTGAAAGCTAAACTTCAAACTACAGAAACAGATAAA
GATGAATTGAAGAACCAATTGGACTCATTAAAGAAGGAATTAGCAAGTAAAGAATCTCGGCTTTTA
TCATCACCAGATCAAGATCTCAAGTCCTCAAACAAGCAGTCAGTGGGTAACTTGGATATGGATATT
GATGTGAAAATCATTGGTCGGGAAGCAATGATTCGAGTCCAATCCAGTAAAAACAACCACCCTGCA
GCAAGAGTAATGGGAGCACTAAAGGATCTTGATCTTGAATTACTCCATGCTAGTGTCTCAGTGGTA
AATGATTTGATGATCCAGCAAAATACAGTGAGAATGGGGAGCCGATTTTATACTCAGGAGCAGCTT
AGAATAGCATTGACATCCAGAATAGCCGGAAACTCGATGAGGCTCTTGGTATGA

## FIGURE 4 (continued)

**SEQ ID NO: 16, gi|33339705|gb|AAQ14332.1|AF283507_1 MYC2 [Catharanthus roseus]**

MTDYRLQPKMNLWGTTTNTAASPIITSDDNSSMMEAFMTSSDPISLWPPSMSVNHHHPPTPTSSAV
TTAVDSAKSMPAQPAFFNQENLQQRLQTLIDGARESWTYAIFWQSSVVEFAGPSVLGWGDGYYKGE
EDKGKRKNSSSASSFAEQEHRKKVLRELNSLIAGPQGTADDAVDEEVTDTEWFFLISMTQSFVSGS
GLPGQALYNSNPVWVTGAGRLAVSHCDRARQAQSFGLQTLVCIPSANGVVELGSTELIFQSSDLMN
KVRILFNFNNIDLGSSSGPWPENDPSSLWLTDPSPSGVGVKEGVNTNNNTSVQGNSIPSGNKQQLV
FGNNDNHPTTSTLTDHPGAGAVNSYNNSSQNAQQPQGSFFTRELNFSEYGFERSSVKNGNCKPESG
EILNFGGESVTKKNSVSGNGNLFSVQSQFGAGEENKNKKRPSPVSRGSNDEGMLSFTSGVVLPSTG
VVKSSGGGGGGDSDHSDLEASVVKEAESSRVVDPEKRPRKRGRKPANGREEPLNHVEAERQRREKL
NQRFYALRAVVPNVSKMDKASLLGDAISYINELKAKLQTTETDKDELKNQLDSLKKELASKESRLL
SSPDQDLKSSNKQSVGNLDMDIDVKIIGREAMIRVQSSKNNHPAARVMGALKDLDLELLHASVSVV
NDLMIQQNTVRMGSRFYTQEQLRIALTSRIAGNSMRLLV

**SEQ ID NO: 17, gi|45421749:1-2079 Solanum tuberosum partial mRNA for MYC transcription factor (jamyc2 gene)**

ATGACTGAATACAGCTTACCCACCATGAATTTGTGGAACAATAGTACTAGCGATGATAACGTGTCT
ATGATGGAAGCTTTTATGTCTTCTGATCTTTCTTTTTGGGCTACTACTAATTCTACTACTACTAAT
TCTGCTTCTGCTGCTGTGGTTGGCGTCAATTCAAATCTTCTTCATACTAATAATAATAATCCGTCT
GTTTTTCCTCTATCTTCTTCTACATCTGTATCCGCGGCTGCGGCTGTTGATGCTACCAAATCTATG
CCGTTTTTCAACCAAGAAACCCTTCAGCAGCGTCTTCAAGCTCTTATCGATGGTGCTAGAGAGACG
TGGACTTACGCTATCTTTTGGCAATCGTCGGTTGTTGATTTCTCGAGTCCGTCTGTGTTGGGTTGG
GGAGATGGTTATTACAAAGGCGAAGAAGATAAAGCCAAAAGGAAATTAGCGGTTTCTTCTCCTGCT
TATATTGCTGAGCAGGAGCACCGGAAGAAGGTTCTCCGGGAGCTGAATTCGTTGATTTCAGGGGCA
CCAGCTGGGACCGATGATGCTGTTGATGAAGAAGTTACCGACACCGAATGGTTCTTTCTTATCTCC
ATGACCCAATCGTTTGTTAATGGAAGTGGGCTTCCTGGTCAGGCGTTGTATAGTTCCAGCCCTATT
TGGGTCGCCGGAACTGAGAAATTGGCAGCTTCCCACTGCGAACGGGTTAGACAAGCACAAGGGTTC
GGGCTTCAGACTATTGTCTGTATTCCTTCAGCTAACGGCGTGGTTGAATTGGGCTCGACAGAGTTG
ATTGTTGAAAGTTCTGATCTCATGAACAAGGTTAGAGTATTGTTTAACTTCAGTAATGATTTGGGG
TCTGGTTCATGGGCTGTGCAGCCGGAGAGCGACCCGTCGGCGCTTTGGCTCACTGAACCATCGTCC
TCAGGTATGGAAGTTAGAGAGTCTTTAAATACAGTACAAACAAATTCAGTTCCATCAAGTAATAGT
AATAAGCAAATTGCTTATGCAAATGAGAATAATCATCAATCTGGAAATGGTCAGAGTTGTTACAAT
CTGCAGCAACAACAGAACAATCCTCCTCAACAACAAACACAAGGATTTTTCACGAGGGAGTTGAAT
TTTTCGGAATTCGGGTTCGATGGAAGTAGCAATAGGAATGGGAATGCATCGCTCTCTTGCAAACCT
GAATCAGGAGAAATCTTGAATTTTGGTGATAGTACTAAAAAAAGTGCTTCCAGTGCAAATGTGAAC
TTGTTTACGGGTCAGTCCCAATTTGGGGCAGTGGAGGAGAATAACAATAACAAGAACAAGAAAAGA
TCAGCTACTTCCAGGGGAAGCAATGAAGAAGGAATGCTTTCATTTGTTTCAGGTACAGTTTTGCCT
TCTTCGGGTATGAAGTCAGGTGGAGGTGGAGGCGAAGACTCTGAACATTCAGATCTTGAGGCTTCA
GTGGTGAAAGAAGCTGATAGTAGTAGAGTGGTAGAACCAGAAAAGAGGCCAAGGAAGCGAGGGAGA
AAGCCAGCGAATGGACGAGAGGAGCCTTTGAATCACGTCGAGGCAGAGAGGCAAAGGAGGGAGAAA
TTGAACCAAAGATTCTACGCGCTTAGAGCTGTTGTACCAAATGTGTCTAAGATGGACAAGGCATCA
CTTCTTGGAGATGCAATTTCATATATAAACGAGTTGAAATCAAAGCTTCAAAATACAGAGTCAGAT
AAAGAAGACTTGAAGAGCCAAATAGAAGATTTAAAGAAAGAATCAAGGCGCCCCGGTCCTCCACCA
CCAAACCAAGATCTCAAGATTGGAGGCAAGATTGTAGACGTGGATATAGACGTTAAGATAATCGGA
TGGGATGCAATGATTGGTATACAATGTAATAAAAAGAATCATCCAGCTGCAAGGTTAATGGCAGCC
CTCATGGAATTAGACCTAGACGTGCACCATGCCAGTGTTTCAGTTGTCAACGATTTGATGATCCAA
CAAGCCACAGTGAAAATGGGTAGCAGACATTACACTGAAGAGCAGCTTAGGGTAGCATTGAAATCG
AAAATTGCTGAAACCCCCTTAGAAAGTAGATAG

**FIGURE 4 (continued)**

**SEQ ID NO: 18, gi|45421750|emb|CAF74710.1| MYC transcription factor [Solanum tuberosum]**
MTEYSLPTMNLWNNSTSDDNVSMMEAFMSSDLSFWATTNSTTTNSASAAVVGVNSNLLHTNNNNPS
VFPLSSSTSVSAAAAVDATKSMPFFNQETLQQRLQALIDGARETWTYAIFWQSSVVDFSSPSVLGW
GDGYYKGEEDKAKRKLAVSSPAYIAEQEHRKKVLRELNSLISGAPAGTDDAVDEEVTDTEWFFLIS
MTQSFVNGSGLPGQALYSSSPIWVAGTEKLAASHCERVRQAQGFGLQTIVCIPSANGVVELGSTEL
IVESSDLMNKVRVLFNFSNDLGSGSWAVQPESDPSALWLTEPSSSGMEVRESLNTVQTNSVPSSNS
NKQIAYANENNHQSGNGQSCYNLQQQQNNPPQQQTQGFFTRELNFSEFGFDGSSNRNGNASLSCKP
ESGEILNFGDSTKKSASSANVNLFTGQSQFGAVEENNNNKNKKRSATSRGSNEEGMLSFVSGTVLP
SSGMKSGGGGGEDSEHSDLEASVVKEADSSRVVEPEKRPRKRGRKPANGREEPLNHVEAERQRREK
LNQRFYALRAVVPNVSKMDKASLLGDAISYINELKSKLQNTESDKEDLKSQIEDLKKESRRPGPPP
PNQDLKIGGKIVDVDIDVKIIGWDAMIGIQCNKKNHPAARLMAALMELDLDVHHASVSVVNDLMIQ
QATVKMGSRHYTEEQLRVALKSKIAETPLESR

**SEQ ID NO: 19, gi|89027223:195-2135 Pisum sativum G-box element binding protein (GBF) mRNA, complete cds**
ATGACAGATTACCGCTCACTCCCGACAATGAATAATAGTATCTGGACAGACGACAACTCCTCCGTC
ATGGAAGCTTTCATGAGCACCACCGCCGACCTTTCTTCTATCTGGCTTCCACCACCAAATTCCGCT
GCATCAACCACCACACCAGGACCCGATACAACCAAACCTCCACCACAACAACAGCCACTCTTCAAC
CAAGAAACTCTCCAACACCGTCTTCAAGCTTTAATCGAAGACGCAAAAGAGAATTGGACTTACGCC
ATCTTCTGGCAAACCTCTTACGACTACTCTACAAGCAGACAGCTCCTTGGTTGGGGAGACGGTTAT
TACAAAGGCGAAGATGACAAAGAAAAAGCTAAAAAAGTTATCTTGCCTGAACAACAAGCTCATCGC
AATAAAGTCCTCCGTGAACTTAACGCCTTAATTTCCGGCTCATCTAGCTCAGATGATGTCGTTGAC
GAAGATGTCACCGACACTGAGTGGTTCTTTCTCACGTCGATGACACACTCCTTCGTCAACGGAAGC
GGTTTACTGAGCCAGGCTTACTTTAATTCTTCCCCAGTATGGATCAACGATAGGCTTTCCATGTCC
ACATGTGAAAGAACCCGTGCAGCACATGTTCACGGGCTTCAGACTTTGGTATATATACCAGCACCG
TCTTCAAACGGTGTCGTTGAGCTCGCATCTACTGAGATAATTCCACATAGCGCTGGTATAATGGAG
AAGGTTCGTTTTTTGTTTGATTTCAATAATCCAGAAGCACGGTCTTGGCCGTTGAATTCCGCCGAC
AACGATCCTTCTTCCATGTGGTTGGATATCCCCGGCTCCGGTGGAATTGAAATTAGAGACTCCATC
AACACTGTTAGTGCCGTCAGTGTAACGGCTTCAGCAAATGCAACAATCCCTAAAAAATCGCCGTTT
GAAATTCACGGTGCTTCTACTACTCTACCGGAATCATCCACCACCGTTAATATTTCAACTGCTCAG
CGCCAAATCCAGAATCAAAACCAGAACCAGAGCTTCTTTCCAAGAGAACTGAATTTTTCAGGTTCT
TTCAAACCGGAATCCGGCGAGATTCTGAACTTTGGGGAGAGTAAAAAGAGTTCTTACAGCTCTGCC
AATGGTAATTTCTTTTCCGGTCCGTCACCATTCGCTGCTAATGAAGAAAACAGAAAAAGAAGATCC
CCGGTGTCTAGAAGTAGTATCGAGGACGGAATTCTTTCATTCAGTTCTGGCAAACTGTTACATGGT
TCAACTATAAAATCCGGTGGCGGAGATTCCGATCATTCAGATCTGGAAGTTTCTGTGGTGAAGAAA
ACTGTGAGCAGCAGAGTTATCGAACCAGAGAAGCGGCCTCGGAAGCGAGGTAGAAAACCAGCCAAC
GGAAGAGAAGAACCCTTGAACCACGTGGAAGCAGAGAGGCAGCGACGGGAGAAACTGAACCAGAGA
TTCTACGCTTTACGAGCTGTGGTTCCTAATGTTTCTAAAATGGACAAAGCTTCGCTTCTTGGAGAC
GCGATTTCTTACATAAACGAGTTGAAATTGAAGCTGCAAGGGCTTGAATCGTCAAAAGATGAGTTG
GAGAAAGAACTAGATACAACCCGAAAGGAACTAGAAATTGCAACTAAAAAACCAGTTCGGTTGAAC
GAAGAAGAAAAAGAGAAACCAGAAAACAACTCTAAGTTGATTGATTTGGACATAGATGTGAAAATC
ATGGGATGGGATGCTATGATTAGGATTCAGTGTAGCAAGAAGAATCACCCTGCTGCGAAATTAATG
GCGGCTTTAAAAGAATTGGATCTTGATGTGAATCATGCTAGTGTTTCTGTTGTTAATGACTTAATG
ATTCAACAGGCTTCTATTAACATGGGAAGTAGATTTTACACACAAGAACAGCTTTTATCTGTTCTT
TCTTCCAAAATTGGTGATACACAATGA

## FIGURE 4 (continued)

**SEQ ID NO: 20, gi|89027224|gb|ABD59338.1| G-box element binding protein [Pisum sativum]**

MTDYRSLPTMNNSIWTDDNSSVMEAFMSTTADLSSIWLPPPNSAASTTTPGPDTTKPPPQQQPLFN
QETLQHRLQALIEDAKENWTYAIFWQTSYDYSTSRQLLGWGDGYYKGEDDKEKAKKVILPEQQAHR
NKVLRELNALISGSSSSDDVVDEDVTDTEWFFLTSMTHSFVNGSGLLSQAYFNSSPVWINDRLSMS
TCERTRAAHVHGLQTLVYIPAPSSNGVVELASTEIIPHSAGIMEKVRFLFDFNNPEARSWPLNSAD
NDPSSMWLDIPGSGGIEIRDSINTVSAVSVTASANATIPKKSPFEIHGASTTLPESSTTVNISTAQ
RQIQNQNQNQSFFPRELNFSGSFKPESGEILNFGESKKSSYSSANGNFFSGPSPFAANEENRKRRS
PVSRSSIEDGILSFSSGKLLHGSTIKSGGGDSDHSDLEVSVVKKTVSSRVIEPEKRPRKRGRKPAN
GREEPLNHVEAERQRREKLNQRFYALRAVVPNVSKMDKASLLGDAISYINELKLKLQGLESSKDEL
EKELDTTRKELEIATKKPVRLNEEEKEKPENNSKLIDLDIDVKIMGWDAMIRIQCSKKNHPAAKLM
AALKELDLDVNHASVSVVNDLMIQQASINMGSRFYTQEQLLSVLSSKIGDTQ

**SEQ ID NO: 21, gi|89274200:232026-233786 Brassica oleracea Contig G, complete sequence**

TCAAACTCTCTCCATTAGAGCAGCCTTGAGCTGATCCTGCGTGAAAAACTGATTCCCCATCTTCAC
AGTCGCTTGTTGGATCATGAACTCATTCACTACCGATAAACTCGCGTGATTCACTTCCAATTCCAG
CTCCTTGAGTGCTTCCATGAACTTAGCACCAGGATGATTCTTCTTAGCGCATTGGATCCTAATCAT
CGCATCCCAACCAATGATCTTCACATCTATCTCCACCTCTATGGACACACCTGAATCTTGATCCAA
ACACTTTTGATCTTTAACCAAACTTTTCACATTCCCATCCCCCACCTCTTTGCTCATCCCATCAAT
CTGCTTCTGCAACTCCTCTTTATCAGCTTCAGCCTTTTGCAGCTTAGCTTTAAGCTCATTGATATA
CGAGATCGCGTCTCCTAGAAGAGAAGCTTTATCCATCTTAGACACATTAGGAACCACAGCTCTCAG
AGAGTAGAATCTCTGGTTCAGCTTCTCTCTTCTCTGTCTCTCTGCCTCCACGTGATTCAACGGCTC
CTCTCTTCCGTTAGCCGGTTTCCTCCCTCGTTTCCTCGGTTTCTTCTCCGTCTCAGCCACGATTCT
ACCACTCTCCGCTTCTTTAACAACCGAAGCTTCGAGATCAGAGCGATTCGAGTCGTTCGATTTCGT
AGGAAGTGGAAGAACAGAAGTGAAAGACAGCATCTCTTCTTCCTTGTCGGAAACCAAACATCTCTT
CTTCTTGTTGCTGTCTCCCTCCACAAACCCAGAACTCGAGATTTGCGGGTTCTGCTGCTGCTTAGG
GTGTTCCACTGAGCTTCCATTACAAAGCTTCGAAGCCGTCTGAGAATCAGAGTTAGAAGTCGCCGG
AGCCCCAAGAACCGGTTCGATCCCGGTTACAATCGGTTCATTAATCCACGTGGCGGAATCATTCTC
TCCTTGATCCGGGTTCAAATTAAACTCCCAAGAACCCGACTCGCCTCCACCACCGCCACCGTTGAA
ACTGAAAAAGCTGTTGACTTTCTCAACGAGATCCGAGCTCTGATGTATAATCTCCAACGAACCAAG
CTCAACGACGCCGTTTTGCGTCGGTATACAAACCATCGTCTCCAACCCGTAAACCTGACCCTGGCG
AGCTCTCTCGCAGCTCGATCCAGCCAAGGCGTTCGAGCCAGAGAGCCAAATGGTCCGCGAGCTCGA
GAAAGCTCGGCCGGGCAAACCAGAGCCGTTGACGAAGCTCTGCGTCATCGAAACCAAGAAGAACCA
TTCCGTATCCGACACGTCTTCGTCTCCCGCCTCGTCGGAGGAGCCGCCGCTGCTGCTGACTGTCCC
TCCTCCGCCGGAGATCAGAGAGTTGAGCTCTCGAATCACTCTCTTCCGATGCTCTTGCTCCGCCGC
GCTGACGGGATTGGGCTTCCTCTTCCTCGACTTCCTCTCCTCCTCGCCTTTGTAGTAGCCATCGCC
CCAGGTTAGCAGCGCCGCCGTGTTGCTGATGTCTTCTCCGGCGAAGTCGTGGGATAGTTGCCAGAA
CACGGCGTACGTCCAGCTTTCTCTAGCTCCTTCGATCAATGCTTGGAGTCGTTGCTGGAGAGTGTC
TTCGGTGACGTGAGGAGGAGGAGGAGTGAGAGGGGGTTGAGGCCAGAGAGAAGAGTGTTCGGAGCC
GATGAGAGGTTCCATTACAGACGCATTGTCTTCGATTGTTGACCAGAGGTTTGTGCCGTTTGTTGA
CTGGTTGAGGTGGTGGTCGGTTAGTTGAACATTTGTCGAAGACAT

**FIGURE 4 (continued)**

**SEQ ID NO: 22, gi|89274228|gb|ABD65632.1| basic helix-loop-helix (bHLH) family transcription factor [Brassica oleracea]**
MSSTNVQLTDHHLNQSTNGTNLWSTIEDNASVMEPLIGSEHSSLWPQPPLTPPPPHVTEDTLQQRL
QALIEGARESWTYAVFWQLSHDFAGEDISNTAALLTWGDGYYKGEEERKSRKRKPNPVSAAEQEHR
KRVIRELNSLISGGGGTVSSSGGSSDEAGDEDVSDTEWFFLVSMTQSFVNGSGLPGRAFSSSRTIW
LSGSNALAGSSCERARQGQVYGLETMVCIPTQNGVVELGSLEIIHQSSDLVEKVNSFFSFNGGGGG
GESGSWEFNLNPDQGENDSATWINEPIVTGIEPVLGAPATSNSDSQTASKLCNGSSVEHPKQQQNP
QISSSGFVEGDSNKKKRCLVSDKEEEMLSFTSVLPLPTKSNDSNRSDLEASVVKEAESGRIVAETE
KKPRKRGRKPANGREEPLNHVEAERQRREKLNQRFYSLRAVVPNVSKMDKASLLGDAISYINELKA
KLQKAEADKEELQKQIDGMSKEVGDGNVKSLVKDQKCLDQDSGVSIEVEIDVKIIGWDAMIRIQCA
KKNHPGAKFMEALKELELEVNHASLSVVNEFMIQQATVKMGNQFFTQDQLKAALMERV

**SEQ ID NO: 23, gi|115483615:1-2100 Oryza sativa (japonica cultivar-group) Os10g0575000 (Os10g0575000) mRNA, complete cds**
ATGAACCTTTGGACGGACGACAACGCGTCGATGATGGAGGCGTTCATGGCCTCCGCCGACCTCCCG
GCCTTCCCGTGGGGGGCGGCCTCCACCCCGCCGCCGCCGCCGCCGCCGCCTCACCACCACCACCAA
CAGCAGCAGCAGCAGGTCCTGCCGCCGCCTGCAGCAGCTCCGGCGGCGGCGGCGTTCAACCAGGAC
ACGCTGCAGCAGCGGCTGCAGTCGATCATCGAGGGGTCTCGGGAGACGTGGACGTACGCCATCTTC
TGGCAGTCCTCCATCGACGTCTCCACCGGCGCCTCCCTCCTCGGCTGGGGCGACGGCTACTACAAG
GGCTGCGACGACGACAAGCGCAAGCAGCGCTCCTCCACCCCCGCCGCCGCCGCCGAGCAGGAGCAC
CGCAAGCGCGTCCTCCGCGAGCTCAACTCCCTCATCGCCGGCGCAGGCGCCGCCCCCGACGAGGCC
GTCGAGGAGGAGGTCACCGACACCGAGTGGTTCTTCCTCGTCTCCATGACCCAGTCCTTCCCCAAC
GGCCTCGGCCTCCCCGGCCAGGCCCTCTTCGCCGCCCAGCCCACCTGGATCGCCACCGGCTTGTCC
TCCGCCCCCTGTGACCGCGCCAGGCAGGCCTACACCTTCGGCCTCCGCACCATGGTCTGCCTCCCC
CTCGCCACCGGCGTCCTCGAGCTCGGCTCCACCGACGTCATCTTCCAGACCGGGGACAGCATCCCC
AGGATCCGCGCCCTCTTCAACCTCAGCGCCGCCGCCGCCTCCTCCTGGCCCCCCCACCCCGACGCC
GCCTCCGCCGATCCCTCCGTGCTCTGGCTCGCCGACGCGCCCCCCATGGACATGAAGGACTCCATC
TCCGCCGCAGACATCTCCGTCTCCAAGCCACCACCCCCGCCGCCGCACCAGATCCAGCACTTCGAG
AACGGGAGCACCAGCACGCTCACGGAGAATCCCAGCCCGTCGGTGCACGCGCCGACGCCCTCGCAG
CCGGCCGCGCCGCCCCAGCGGCAGCAGCAGCAGCAGCAGAGCAGCCAGGCTCAGCAGGGCCCCTTC
CGCCGGGAGCTCAACTTCTCCGACTTCGCGTCCAACGGCGGCGCCGCGGCCCCGCCTTTCTTCAAG
CCCGAGACCGGCGAGATCCTAAACTTCGGCAACGACAGCAGCAGTGGCCGGAGGAATCCGTCGCCG
GCGCCCCCGGCCGCCACCGCCAGCCTCACCACCGCGCCGGGGAGCCTGTTCTCGCAGCACACGCCG
ACGCTGACGGCGGCGGCGAACGACGCCAAGAGCAACAACCAGAAGAGGTCCATGGAGGCCACCTCC
CGCGCCAGCAACACCAACAACCACCCGGCGGCGACGGCGAACGAGGGGATGCTGTCCTTCTCGTCG
GCGCCGACGACGCGGCCGTCGACGGGGACGGGCGCCCCGGCCAAGTCGGAGTCGGACCACTCGGAC
CTGGAGGCGTCGGTGCGGGAGGTGGAGAGCAGCCGCGTGGTGGCGCCGCCGCCGGAGGCGGAGAAG
CGGCCGCGGAAGCGCGGGCGGAAGCCGGCGAACGGGCGGGAGGAGCCGCTGAACCACGTGGAGGCG
GAGCGGCAGCGGCGGGAGAAGCTCAACCAGCGCTTCTACGCGCTGCGCGCCGTGGTGCCCAACGTG
TCCAAGATGGACAAGGCGTCGCTGCTGGGCGACGCCATCTCCTACATCAACGAGCTCCGGGGGAAG
CTCACGGCGCTGGAGACGGACAAGGAGACGCTGCAGTCGCAGATGGAGTCGCTCAAGAAGGAGCGG
GACGCGCGGCCGCCGGCGCCGTCGGGCGGCGGCGGAGACGGCGGGGCGCGGTGCCACGCGGTGGAG
ATCGAGGCCAAGATCCTTGGGCTGGAGGCCATGATCCGCGTGCAGTGCCACAAGCGGAACCACCCG
GCGGCGCGGCTGATGACGGCGCTGCGGGAGCTGGACCTGGACGTGTACCATGCCAGCGTCTCCGTG
GTCAAGGACCTCATGATCCAGCAGGTGGCCGTCAAGATGGCCAGCCGCGTCTACTCGCAGGACCAG
CTCAACGCCGCGCTCTACACCCGCATCGCCGAGCCTGGCACCGCCGCCCGGTAA

**FIGURE 4 (continued)**

**SEQ ID NO: 24, gi|115483616|ref|NP_001065478.1| Os10g0575000 [Oryza sativa (japonica cultivar-group)]**
MNLWTDDNASMMEAFMASADLPAFPWGAASTPPPPPPPPHHHHQQQQQQVLPPPAAAPAAAAFNQD
TLQQRLQSIIEGSRETWTYAIFWQSSIDVSTGASLLGWGDGYYKGCDDDKRKQRSSTPAAAAEQEH
RKRVLRELNSLIAGAGAAPDEAVEEEVTDTEWFFLVSMTQSFPNGLGLPGQALFAAQPTWIATGLS
SAPCDRARQAYTFGLRTMVCLPLATGVLELGSTDVIFQTGDSIPRIRALFNLSAAAASSWPPHPDA
ASADPSVLWLADAPPMDMKDSISAADISVSKPPPPPPHQIQHFENGSTSTLTENPSPSVHAPTPSQ
PAAPPQRQQQQQQSSQAQQGPFRRELNFSDFASNGGAAAPPFFKPETGEILNFGNDSSSGRRNPSP
APPAATASLTTAPGSLFSQHTPTLTAAANDAKSNNQKRSMEATSRASNTNNHPAATANEGMLSFSS
APTTRPSTGTGAPAKSESDHSDLEASVREVESSRVVAPPPEAEKRPRKRGRKPANGREEPLNHVEA
ERQRREKLNQRFYALRAVVPNVSKMDKASLLGDAISYINELRGKLTALETDKETLQSQMESLKKER
DARPPAPSGGGGDGGARCHAVEIEAKILGLEAMIRVQCHKRNHPAARLMTALRELDLDVYHASVSV
VKDLMIQQVAVKMASRVYSQDQLNAALYTRIAEPGTAAR

**SEQ ID NO: 25, gi|68342447:195-2357 Rheum australe basic helix-loop-helix transcription factor protein mRNA, complete cds**
ATGATGAACCTCTGGAGTTCCGATGATAACGTTGCTTTCGCCGACGCTTTTATGTCCTTGGACTCT
TCCTCCGCCGCCGCCTGGCCTCCGGCGCCTTCCTCCCACCAATACAACCACCAGTACAACCTCCAA
CACCAGCAACAACTGCAGCATCAACCACAACAACAACAACAACAACAACAGATGGCCAATTCGGCC
GGCGGTGGTTGGCAACAAGCGATGGCCGGCGGCGCTCAGATGAATCCGGCGCAGATGATGAACCAG
GACAGCCTACAGCAGCGGCTGCAGGCGCTGATCGACGACGCCAGGGAGAGCTGGACCTACGCCATC
TTCTGGCAGTGCAACGTCGAGCCCACCGGCCAGTCTCTCCTTGGCTGGGGCGACGGATACTATAAG
GGCGATGACTCCGCCAATAAGAACGCCTCCTCCGCCGCCCCCGCCGCTGGATCCAGGCCGCCGAAG
AACCCCGCAGAACAGGAGCACAGGAGGAGAGTCCTCCGGGAACTCAATTCCCTGATCTCCGGCTCC
TCTTCCCCTCAAAACGACGCCGTTGACGACGACGTCACCGACACCGAGTGGTTCTTCCTTATCTCC
ATGACGCAGGCTTTCCCCTTCGGCGTCGATCTCCCCGGCCAAGCCATCCTCGGATCCAACCCTATC
TGGGCGTACGGCTCCGACCGTCTCGCCGGCTCCCCGTGGGATCGGGCCAGACAAGGGGCTGCGTTC
GGGTTGCAGACGATCGTCTGCATCCCCAGCGGCACCGGCGTCCTCGAATTAGGCTCCACCGAACTC
GTCTTCAACAGTTCCGTTTTGATGAATAAGGTTAGGGTTTTGTTCAATTTCGGATCTGGAGATGCG
AGTCTCCTGACCGCCGCAGCCTCCTCCTCCGCCCCTGCCGCCGCTCCTCCACCGCCGATCTCAACG
GCGACGACCGATGAGGCGGAGAACGACCCCGCCGCGCTGTGGATCAGCGATCCGTCTTCCTCCGCA
GCCGAGGTGAAGGAGGCATTGAACCCTAGGATCACCGTCCGTGAGAGCTCGATTCCGATCGGATCG
AATTCGATTCCCGTCCACCAGCCGGCCAAACCGCCGCAATTGGACGTTCAAAGCTCAATCGGATTG
ACGGAGAATTCAATTGGTATCCATAGCCAGAAGAGTCACAATCAGCCACTCCAACATCAAGGATTT
TTCACCAAAGAGTTGAATTTCTCCGAGTTTGCGATGAAGCCAGAATCCGGAGAGATCCTCAACTTT
GGGGAGAGTAAGAGGAATTCCCTCGGCAACGGAAACGGATTGAATTCGCAATTCCTTGTGGAAGAG
AGCAACAAAAACATCATCAGCAAGAAGCGATCTCCGACCTCAAGAGGAAGCGCAGAGGAAGGCATG
CTTTCATTCACTTCAAGCGTGGTCTTGCCTTCTTCCATGGCGGTGAAGTCAAGCGCCACCGGCGCA
GGGGACTCCGACCACTCTGACCTGGAAGCTTCGGTGGTGAAGGAAGCCGACAGCAGCCGGGTGGTG
GACCCCGAGAAGCGGCCCCGAAAGCGTGGCCGGAAGCCAGCAAACGGCAGGGAGGAGCCCCTGAAC
CATGTCGAAGCGGAGAGGCAGAGGAGGGAGAAGCTCAACCAGAGATTCTACGCCCTCAGGGCGGTT
GTCCCCAATGTGTCAAAAATGGACAAAGCATCACTCTTAGGAGACGCAATCTCCTTCATTAACGAG
CTAAAATCGAAGCTCCAAAACGTGGAATCAGAGAAGGAAACATTGCTGAGCCAGGTGGAGTGTTTG
AAGACAGAGGTGTTAGCAAGCAGAGATCATCAATCAAGGTCCTCCAATGGAGGAGGAGGAGTACAG
AACCACCACCATCCAAGCCTAGAACAGGACATGAATATGCTGAATGGAAGCTGTAAGCAGTCAGAT
TTGGACGTGGACGTCAAGATCATTGGAAGGGATGCCATGGTTAGAGTGAACTGCAGCAAGAGCAAC
CACCCAGCTGCAAGGCTAATGGTGGCATTGAAGGAGCTTGACTTGGAAGTTACGCACGCGAGCGTT
TCGGTTGTCAATGATCTGATGATCCAGCAGGCGACGGTGAGGATGGGGAGCCGGTATTACAGCCCG
GATCATCTTAGGATGGTCTTAGAAGCCAAGGTTTCTGATATCAGGGGATGA

**FIGURE 4 (continued)**

**SEQ ID NO: 26, gi|68342448|gb|AAY90122.1| basic helix-loop-helix transcription factor protein [Rheum australe]**

MMNLWSSDDNVAFADAFMSLDSSSAAAWPPAPSSHQYNHQYNLQHQQQLQHQPQQQQQQQMANSA
GGGWQQAMAGGAQMNPAQMMNQDSLQQRLQALIDDARESWTYAIFWQCNVEPTGQSLLGWGDGYYK
GDDSANKNASSAAPAAGSRPPKNPAEQEHRRRVLRELNSLISGSSSPQNDAVDDDVTDTEWFFLIS
MTQAFPFGVDLPGQAILGSNPIWAYGSDRLAGSPWDRARQGAAFGLQTIVCIPSGTGVLELGSTEL
VFNSSVLMNKVRVLFNFGSGDASLLTAAASSSAPAAAPPPPISTATTDEAENDPAALWISDPSSSA
AEVKEALNPRITVRESSIPIGSNSIPVHQPAKPPQLDVQSSIGLTENSIGIHSQKSHNQPLQHQGF
FTKELNFSEFAMKPESGEILNFGESKRNSLGNGNGLNSQFLVEESNKNIISKKRSPTSRGSAEEGM
LSFTSSVVLPSSMAVKSSATGAGDSDHSDLEASVVKEADSSRVVDPEKRPRKRGRKPANGREEPLN
HVEAERQRREKLNQRFYALRAVVPNVSKMDKASLLGDAISFINELKSKLQNVESEKETLLSQVECL
KTEVLASRDHQSRSSNGGGGVQNHHHPSLEQDMNMLNGSCKQSDLDVDVKIIGRDAMVRVNCSKSN
HPAARLMVALKELDLEVTHASVSVVNDLMIQQATVRMGSRYYSPDHLRMVLEAKVSDIRG

**SEQ ID NO: 27, gi|4321761:1-2109 Zea mays transcription factor MYC7E mRNA, partial cds**

GGCACGAGGGACGACAACGCCTCCATGATGGAGGCCTTCATGGCTTCTGCCGACCTCCCCACCTTC
CCCTGGGGAGCGCCGGCCGGGGGCGGCAACTCGTCGGCCGCCGCGGCGTCGCCGCCGCCGCAGATG
CCAGCGGCGATGGCTCCCGGGTTCAACCAGGACACGCTGCAGCAGAGGCTACAAGCCATGATAGAG
GGGTCGAGGGAGACCTGGACCTACGCCATCTTCTGGCAGTCCTCCCTTGACTCCGCCACCGGCGCC
TCGCTGCTCGGCTGGGGGGACGGCTACTACAAGGGCTGCGACGAAGACAAGCGCAAGCAGAAGCCG
CTCACGCCCTCCGCCCAGGCCGAGCAGGAGCACCGCAAGCGCGTGCTCCGCGAGCTCAACTCCCTC
ATCTCCGGCGCGGCGGCGGCTCCCGACGAGGCCGTCGAGGAGGAGGTCACCGATACCGAGTGGTTC
TTCCTCGTCTCCATGACGCAGTCGTTTCTCAACGGTTCGGGCCTCCCCGGGCAGGCGCTCTTCGCC
GGGCAGCCCACATGGATCGCCTCTGGCCTCTCCTCCGCGCCGTGCGAGCGCGCGCGTCAGGCCTAC
AACTTCGGCCTCCGCACCATGGTATGCTTCCCCGTCGGCACCGGGGTGCTCGAGCTCGGCTCCACC
GACGTCGTGTTCAAGACCGCCGAGAGCATGGCTAAGATCCGCTCGCTCTTTGGGGGCGGAGCAGGG
GGTGGCTCGTGGCCGCCCGTGCAGCCTCAGGCGCCGTCGTCGCAGCAGCCCGCCGCCGGAGCCGAC
CACGCGGAGACGGACCCGTCCATGCTATGGCTCGCCGACGCGCCGGTCATGGACATCAAGGATTCC
TTGTCGCACCCGTCGGCCGAGATCTCCGTCTCCAAGCCTCCGCCGCATCCGCCGCAGATCCATTTT
GAGAACGGGAGCACGAGCACGCTCACGGAGAACCCCAGCCCCTCCGTGCACGCGCCGCCACCCCCG
CCGGCGCCGGCTGCTCCACAGCAGCGGCAGCACCAGCACCAGAATCAGGCGCACCAGGGCCCGTTC
CGCCGGGAGCTCAATTTCTCGGACTTCGCGTCCACTCCTTCCTTGGCGGCGACCCCGCCGTTCTTC
AAGCCCGAGTCCGGCGAGATCCTCAGCTTCGGCGCCGACAGCAACGCCCGGAGGAACCCGTCGCCG
GTACCTCCCGCCGCCACCGCCAGCCTCACCACCGCTCCCGGGAGCCTCTTCTCGCAGCACACGGCG
ACCATGACGGCCGCCGCGGCGAACGACGCGAAGAACAACAACAAGCGCTCAATGGAGGCCACCTCC
CGGGCGAGCAACACCAACCACCACCCGGCGGCGACGGCGAACGAGGGCATGCTGTCCTTCTCGTCG
GCGCCGACTACGCGGCCGTCGACGGGCACGGGCGCGCCGGCCAAGTCGGAGTCCGACCACTCGGAC
CTGGACGCGTCTGTGCGCGAGGTGGAGAGCAGCCGCGTGGTGGCGCCGCCACCGGAGGCGGAGAAG
CGGCCGCGAAAGCGCGGGCGGAAGCCCGCGAACGGGCGCGAGGAGCCGCTGAACCACGTGGAGGCG
GAGCGGCAGCGGCGGGAGAAGCTGAACCAGCGCTTCTACGCGCTGCGCGCCGTGGTGCCCAACGTG
TCGAAGATGGACAAGGCGTCGCTGCTCGGCGACGCCATCTCCTACATCAACGAGCTCCGGGGCAAG
CTGACGTCGCTGGAGACCGACAAGGAGACGCTGCAGACCCAGGTGGAGGCGCTCAAGAAGGAGCGC
GACGCGCGGCCGCCCTCGCACTCCGCTGGGCTCGGCGGCACGACGGCGGGCCGCGCTGCCACGCG
GTGGAGATCGACGCCAAGATCCTGGGGCTGGAGGCCATGATCCGCGTGCAGTGCCACAAGCGCAAC
CACCCGTCGGCGCGGCTGATGACAGCGCTCCGCGAGCTCGACCTGGACGTGTACCACGCCAGCGTG
TCCGTCGTCAAGGACCTCATGATCCAGCAGGTCGCCGTCAAGATGGCCAGCCGCGTGTACACGCAG
GACCAGCTCAGCGCCGCGCTCTACAGCCGCCTCGCGGAGCCCGGGTCTGCCATGGGCAGGTAA

**FIGURE 4 (continued)**

**SEQ ID NO: 28, gi|4321762|gb|AAD15818.1| transcription factor MYC7E [Zea mays]**

GTRDDNASMMEAFMASADLPTFPWGAPAGGGNSSAAAASPPPQMPAAMAPGFNQDTLQQRLQAMIE
GSRETWTYAIFWQSSLDSATGASLLGWGDGYYKGCDEDKRKQKPLTPSAQAEQEHRKRVLRELNSL
ISGAAAAPDEAVEEEVTDTEWFFLVSMTQSFLNGSGLPGQALFAGQPTWIASGLSSAPCERARQAY
NFGLRTMVCFPVGTGVLELGSTDVVFKTAESMAKIRSLFGGGAGGGSWPPVQPQAPSSQQPAAGAD
HAETDPSMLWLADAPVMDIKDSLSHPSAEISVSKPPPHPPQIHFENGSTSTLTENPSPSVHAPPPP
PAPAAPQQRQHQHQNQAHQGPFRRELNFSDFASTPSLAATPPFFKPESGEILSFGADSNARRNPSP
VPPAATASLTTAPGSLFSQHTATMTAAAANDAKNNNKRSMEATSRASNTNHHPAATANEGMLSFSS
APTTRPSTGTGAPAKSESDHSDLDASVREVESSRVVAPPPEAEKRPRKRGRKPANGREEPLNHVEA
ERQRREKLNQRFYALRAVVPNVSKMDKASLLGDAISYINELRGKLTSLETDKETLQTQVEALKKER
DARPPSHSAGLGGHDGGPRCHAVEIDAKILGLEAMIRVQCHKRNHPSARLMTALRELDLDVYHASV
SVVKDLMIQQVAVKMASRVYTQDQLSAALYSRLAEPGSAMGR

**SEQ ID NO: 29, gi|162694803:c2552152-2551920, c2550961-2550731, c2550552-2550444, c2549175-2549103, c2548898-2548237, c2548041-2547619 Physcomitrella patens subsp. patens PHYPAscaffold_11 genomic scaffold, whole genome shotgun sequence**

ATGTCGCAGTTATTGAATGCGTGGGAAGTAGCGGACTCAGCTATGATCGAGGCGTTCATGGGAACG
GGGTATAACTGCGTGGAAGGCTTCGAAGTGCAGGATGATCCGGACGGGCAGCTCCACTTGAACGAG
TCGGTGTTGCTGCGGAGGCTGCACTCGCTGGTCGAAGAGAGTACCGTGGATTGGACGTATGCAATC
TTTTGGCAGCTGTCAGCCTTACGTGAAGGGGAGATGATGCTCGGTTGGGGTGATGGGTACTTCAGG
AGTGCTAAGGAGAATGAAATCAACGACGCGAGGAACATGAAGGGTGGTTCTCAAGAGGAGGATCAA
CAGATGAGGCGAAAGGTGTTGCGAGAGCTGCAGGCTCTAGTCAATGGCTCTGAAGATGATGTTAGC
GACTATGTCACGGATACGGAATGGTTTTACCTTGTTTCGATGTCTCATTCTTATGCAGCCGGAGTG
GGGACTCCTGGACGAGCATTAGCATCTGACAGACCTGTGTGGCTAATTGGAGCGAACAAAGCTCCT
GATAATAACTGCAGTCGTGTTCAATTGGCTAAGGTACACAGTTCAATGATCCTTCAGACAATCCTT
TGTATTCCGTCGAAATCTGGTGTCGTTGAGCTTGGATCGACAGATCTGGCAAAAAGCTGGGAAGTT
GTTCAGAATGTCAAGATGGTTTTTGATGAACCAATGATGTGGGCAGCCCATGAAATCCAAGCGGTG
GCGCATTCCTTGCCTTTGAGTAGCGACGCGACTAGTATGCGACCGTCAAGCCCAAGTCTTATGTCA
ATAGCAACAATAAGTGCTTCAATTTCAAATGCAAGTCAGATTGGCAGATGTTCAAATCCAAGTCAA
GATCATGAAGCACATTTTGTTGGAAGGAAGAATCCACCCTCGTCAAGACCCCCGATGAGGTTCTAC
AAACCACGGGTGGAAGAATTGAGCTCGGACACTCCAGAAACAAACTTGGTTGATAATAAATATATT
GTAGGGAAGTCAGTATCTTTCCACCACCTTTCTAAGATAGGTCAGAGAGGAATGCCTGGACCTCCT
ACCACTGCAAATAGGTTACCATGTTTCGCTCCGTCCGAGGTAGATTGTCATGAATCAGAAGCCGAC
GTTTCCGTCAAAGAAAATGTTGTGGAATCAAGTACAAATCTCGAGCCTAAACCACGCAAAAGAGGG
CGCAAGCCGGCAAACGATAGGGAAGAGCCACTAAATCATGTGCAAGCTGAACGGCAGCGGCGAGAA
AAACTCAATCAGAAATTTTATGCTCTACGGTCTGTAGTGCCAAACGTGTCCAAGATGGATAAGGCA
TCCTTATTAGAAGATGCCATTACCTACATTAATGAGCTTCAAGAAAAGCTACAAAAAGCAGAGGCT
GAATTGAAGGTTTTCCAAAGGCAAGTGCTTGCATCAACTGGCGAATCTAAAAAACCTAATCCATCT
CGCAGGGATTCGACCGAAAGTTCTGATGAAGAACGTTTCAGACTTCAAGAGAGTGGTCAGAGATCG
GCACCTCTTGTTCACACTTCTGAAAATAAACCTGTAATTAGTGTTTTTGTTCTTGGAGAAGAAGCT
ATGATTCGAGTTTATTGCACAAGACACTCTAACTTTATAGTTCATATGATGTCGGCATTGGAAAAG
CTACGCTTAGAGGTTATACATTCAAACACTTCATCCATGAAGGATATGCTTCTTCACGTCGTGATT
GTCAAGGTGCGCTGA

FIGURE 4 (continued)

**SEQ ID NO: 30, gi|162695001|gb|EDQ81347.1| predicted protein [Physcomitrella patens subsp. patens]**
MSQLLNAWEVADSAMIEAFMGTGYNCVEGFEVQDDPDGQLHLNESVLLRRLHSLVEESTVDWTYAI
FWQLSALREGEMMLGWGDGYFRSAKENEINDARNMKGGSQEEDQQMRRKVLRELQALVNGSEDDVS
DYVTDTEWFYLVSMSHSYAAGVGTPGRALASDRPVWLIGANKAPDNNCSRVQLAKVHSSMILQTIL
CIPSKSGVVELGSTDLAKSWEVVQNVKMVFDEPMMWAAHEIQAVAHSLPLSSDATSMRPSSPSLMS
IATISASISNASQIGRCSNPSQDHEAHFVGRKNPPSSRPPMRFYKPRVEELSSDTPETNLVDNKYI
VGKSVSFHHLSKIGQRGMPGPPTTANRLPCFAPSEVDCHESEADVSVKENVVESSTNLEPKPRKRG
RKPANDREEPLNHVQAERQRREKLNQKFYALRSVVPNVSKMDKASLLEDAITYINELQEKLQKAEA
ELKVFQRQVLASTGESKKPNPSRRDSTESSDEERFRLQESGQRSAPLVHTSENKPVISVFVLGEEA
MIRVYCTRHSNFIVHMMSALEKLRLEVIHSNTSSMKDMLLHVVIVKVR

**SEQ ID NO: 31, gi|6175251:1-981 Lycopersicon esculentum jasmonic acid 3 (LEJA3) mRNA, partial cds**
AGTGGAAATGGTCAGAGTTGTTACAATCAGCAACAACAGAAGAATCCTCCTCAGCAACAAACACAA
GGATTCTTCACGAGGGAGTTGAATTTTTCGGAATTCGGTTTCGATGGAAGTAGTAATAGGAATGGA
AATTCATCGGTTTCTTGCAAGCCTGAATCAGGAGAAATCTTGAATTTTGGTGATAGTACTAAAAAA
AGTGCTTCCAGTGCCAATGTGAACTTGTTTACAGGTCAGTCCCAATTTTGGGGCCTGGGGGAGGAG
AATAATAACAAGAACCAAGAAAAGATCAGCTACTTCAGGGGAAGCAATGAAGAAGGAATGCTTTCA
TTTGTTTCAGGTACAGTTTGCTTCTTCGGGCATGAAGTCAGGTGGAGGCGGAGGCAAGACTCTGAA
CATTCAGATCTCGAGGCTTCAGTGGTGAAAGAAGCTGATAGTAGTAGAGTGGTAGAGCCTGAAAAG
AGGCCAAGGAAGCGAGGTAGAAAGCCAGCGAATGGACGGGAGGAGCCATTGAATCACGTCGAGGCA
GAGAGGCAAAGGAGGGAGAAATTGAACCAAAGATTCTTCTCTCTTAGAGCTGTTGTACCAAATGTG
TCTAAGATGGACAAGGCATCACTCCTTGGAGATGCTATTTCCTATATAAACGAGTTGAAATCGAAG
CTTCAAAATACAGAGTCAGATAAAGAAGACTTGAAGAGCCAAATAGAAGATTTAAAGAAAGAATCA
AGGCGCCCCGGTCCTCCTCCACCACCAAATCAAGATCTCAAGATGTCTAGCCACACTGGAGGCAAG
ATTGTAGACGTGGATATAGACGTTAAGATCATCGGATGGGATGCAATGATTCGTATACAATGTAAT
AAAAAGAATCATCCAGCGAAGGCTAATGGCAGCGCTCATGGAATTAGACCTAGACGTGCATCATGC
CAGTGTTTCAGTTGTCAACGATTTGATGATCCAACAAGCCACAGTGAAAATGGGTAG

**SEQ ID NO: 32, gi|6175252|gb|AAF04917.1|AF011557_1 jasmonic acid 3 [Lycopersicon esculentum] (partial)**
SGNGQSCYNQQQQKNPPQQQTQGFFTRELNFSEFGFDGSSNRNGNSSVSCKPESGEILNFGDSTKK
SASSANVNLFTGQSQFWGLGEENNNKNQEKISYFRGSNEEGMLSFVSGTVCFFGHEVRWRRRQDSE
HSDLEASVVKEADSSRVVEPEKRPRKRGRKPANGREEPLNHVEAERQRREKLNQRFFSLRAVVPNV
SKMDKASLLGDAISYINELKSKLQNTESDKEDLKSQIEDLKKESRRPGPPPPPNQDLKMSSHTGGK
IVDVDIDVKIIGWDAMIRIQCNKKNHPAKANGSAHGIRPRRASCQCFSCQRFDDPTSHSENG

**SEQ ID NO: 33, gi|118486274:74-1549 Populus trichocarpa clone WS01225_C18 unknown mRNA**
ATGGAGGAACTCATTATTTCTCCATCTTCATCATCTTCACCGGTCTCCTTATCCCAAGAAACCCCA
CCGACTCTCCAACAAAGGCTTCAATTCATAGTCCAAAACCAACCAGATTGGTGGTCTTATGCCATA
TTTTGGCAAACATCAAACGATGACAGCGGCCGAATCTTCCTAGGCTGGGGTGATGGCCATTTTCAA
GGCTCTAAAGATACTTCTCCCAAACCAAACACCTTCAGCAATAGCCGCATGACGATATCAAACTCC
GAGAGGAAAAGGGTCATGATGAAGGGAATCCAATCCCTGATCGGTGAATGTCACGATCTTGATATG
TCTCTGATGGATGGTAACGATGCTACCGACTCTGAGTGGTTCTATGTCATGTCCCTTACTCGATCT
TTCTCACCTGGAGATGGCATCCTTGGCAAAGCTTATACAACTGGTTCTTTGATTTGGTTAACTGGT
GGCCATGAACTTCAATTCTACAACTGTGAAAGAGTCAAAGAAGCACAAATGCATGGCATTGAGACC

## FIGURE 4 (continued)

CTGGTTTGCATACCTACATCGTGTGGGGTTCTTGAATTAGGATCCTCTTCTGTGATCAGAGAAAAT
TGGGGTCTTGTTCAACAAGCCAAGTCTCTTTTTGGGTCAGATCTTAGCGCTTATTTGGTGCCCAAA
GGCCCTAATAACTCCAGTGAAGAACCGACCCAGTTTCTTGACAGGAGCATTTCTTTTGCGGATATG
GGCATAATAGCTGGACTTCAAGAAGATTGTGCAGTTGATCGTGAACAGAAGAACGCTCGTGAAACA
GAAGAAGCAAATAAACGTAACGCTAATAAACCAGGCCTGTCTTATTTGAACTCAGAGCATTCAGAC
TCGGACTTTCCTCTACTTGCTATGCATATGGAGAAAAGAATTCCAAAGAAAAGAGGGAGAAAGCCT
GGCCTGGGCAGAGACGCCCCACTGAACCATGTAGAGGCTGAGAGGCAGCGGAGAGAGAAGTTGAAC
CACCGGTTTTACGCGCTGCGTGCGGTGGTCCCAAACGTGTCTAGAATGGACAAAGCATCACTATTA
TCTGATGCTGTATCCTACATCAATGAATTGAAGGCGAAGGTTGATGAATTAGAGTCACAACTAGAA
AGGGAATCCAAGAAAGTGAAATTGGAAGTTGCCGATAATTTGGACAATCAAAGCACCACCACTTCT
GTGGACCAATCAGCCTGCAGGCCGAATAGTGCTGGTGGCGCTGGGCTTGCACTTGAAGTTGAGATC
AAGTTTGTGGGTAATGATGCAATGATTAGAGTCCAATCGGAGAATGTGAACTATCCAGCTTCCAGG
TTAATGTGTGCGCTCCGTGAACTGGAGTTTCAGGTTCACCATGCTAGTATGTCCTGTGTTAACGAG
CTTATGCTCCAAGATGTGGTAGTTAGGGTTCCTGATGGACTGAGAACCGAAGAGGCCTTGAAATCT
GCTCTTCTTGGAAGACTAGAATAA

**SEQ ID NO: 34, gi|118486275|gb|ABK94979.1| unknown [Populus trichocarpa]**

MEELIISPSSSSSPVSLSQETPPTLQQRLQFIVQNQPDWWSYAIFWQTSNDDSGRIFLGWGDGHFQ
GSKDTSPKPNTFSNSRMTISNSERKRVMMKGIQSLIGECHDLDMSLMDGNDATDSEWFYVMSLTRS
FSPGDGILGKAYTTGSLIWLTGGHELQFYNCERVKEAQMHGIETLVCIPTSCGVLELGSSSVIREN
WGLVQQAKSLFGSDLSAYLVPKGPNNSSEEPTQFLDRSISFADMGIIAGLQEDCAVDREQKNARET
EEANKRNANKPGLSYLNSEHSDSDFPLLAMHMEKRIPKKRGRKPGLGRDAPLNHVEAERQRREKLN
HRFYALRAVVPNVSRMDKASLLSDAVSYINELKAKVDELESQLERESKKVKLEVADNLDNQSTTTS
VDQSACRPNSAGGAGLALEVEIKFVGNDAMIRVQSENVNYPASRLMCALRELEFQVHHASMSCVNE
LMLQDVVVRVPDGLRTEEALKSALLGRLE

**SEQ ID NO: 35, gi|51702427:1-878 Triticum aestivum bHLH transcription factor (bHLH94) mRNA, partial cds**

ATGAAAGGCCGTGGGAACAGAGGACTGCTGGTGGAGGGAGCTCATTGCTTCCCAATGTCCAGAAAG
GATTGCAGAGTTTCACTTGGAGTCAGGCCCGGGGCCTGAATTCTCACCAGCAGAAGTTTGGCAATG
GTATACTGATAGTGAGTAATGAAGCTACACACGGCAACAATAGAACCGCGGACAGCTCCACTACAA
CACAGTTTCAGCTTCAGAAAGCACCTCAGCTCCAGAAACTACCACTTCTTCAGAAACCACCACAGC
TAGTGAAGCCGCTGCAGATGGTCAACCAGCAACAGCTGCAGCCACAGGCGCCTAGGCAAATAGATT
TTAGTGCAGGGACCAGTTCCAAGTCTGGTGTCCTGGTTACAAGAGCAGCTGTTCTTGATGGAGATA
GTTCAGAGGTGAATGGCTTGTGTAAAGAGGAAGGGACAACACCTGTCATAGAGGACCGACGGCCAA
GGAAGAGGGGAAGAAAGCCTGCGAATGGGAGAGAGGAGCCGCTGAATCATGTTGAGGCTGAGCGTC
AAAGGAGGGAGAAGCTCAACCAGCGGTTCTATGCGTTGAGAGCCGTTGTGCCCAACATCTCGAAAA
TGGACAAGGCTTCCCTGCTGGGCGATGCAATAGCATACATCACTGACCTTCAGAAGAAGCTCAAAG
ATATGGAGACGGAGAGAGAACGATTTCTTGAGTCTGGTATGGTGGATCCAAGGGAGCGAGCCCCTA
GACCAGAGGTTGACATCCAGGTGGTGCAAGACGAGGTTCTGGTTCGAGTTATGTCTCCATTGGAGA
ACCATCCGGTAAAGAAGGTCTTTGAAGCGTTTGAAGAGGCGGACGTCCGGGTAGGGGAGTCGAAAC
TCACAGGCAACAATGGAACG

**FIGURE 4 (continued)**

**SEQ ID NO: 36, gi|51702428|gb|AAU08787.1| bHLH transcription factor [Triticum aestivum], partial**

ERPWEQRTAGGGSSLLPNVQKGLQSFTWSQARGLNSHQQKFGNGILIVSNEATHGNNRTADSSTTT
QFQLQKAPQLQKLPLLQKPPQLVKPLQMVNQQQLQPQAPRQIDFSAGTSSKSGVLVTRAAVLDGDS
SEVNGLCKEEGTTPVIEDRRPRKRGRKPANGREEPLNHVEAERQRREKLNQRFYALRAVVPNISKM
DKASLLGDAIAYITDLQKKLKDMETERERFLESGMVDPRERAPRPEVDIQVVQDEVLVRVMSPLEN
HPVKKVFEAFEEADVRVGESKLTGNNGT

**SEQ ID NO: 37, Populus sp. 29.195#1 bHLH-6like encoding sequence**

TGAAAAAAAAAAACACATCTGCTTTCTGGGATGACTGATTACCGACTACCGCCGACAATGAATCTA
TGGACGGACGACAACGCGTCTGTAATGGAAGCATTTATGAACTCCTCCGATCTCTCTTCTCTTTGG
GCACCGCCTCCGCAATCTTCCGCCTCCACTTCCACGCCATCAGCAGCAGCACAGCCATCTGAGAAA
ACTATGTTGAACCAAGAAACACTCCAGCAACGCCTTCAGACATTAATTGAAGGCGCGTGTGAGGGC
TGGGCTTATGCTATTTTCTGGCAATCCTCTTATGATTATTCTGGCGCCTCCGTCCTAGGATGGGGC
GACGGTTATTATAAAGGGGAGGAAGATAAAGGAAAAACTAGAACGAGAAATTCGGCATCATCAGCT
GTTGAACAGGAGCATCGTAAAACGGTGCTCCGTAAGCTGAATTCGTTGATTGCTGGACCTAATTCT
GTTACTGATGATGCTATCGATGAAGAGGTTACTGATACTGAGTGGTTTTTCTTGTTAGCATGACT
CAGTCTTTCGTTAATGGAAGTGGGTTACCTGGTCAAGCTCTTTTCAATGGGAGTCCGGTTTGGGTT
GCTGGGTCGGAGAGGTTGGGGGCTTCGCCGTGTGAAAGAGCCAGGCAGGGTCAGGTTTTTGGGTTA
CAGACTTTAGTTTGTATACCGTCTGCCAGTGGTGTTGTTGAATTGGGTTCTACTGAATTGATTTTT
CAAAGCTCGGATCTGATGAATAAGGTTAGGGTTTTGTTCGATTTTAATAGTTTGGAGGTGGTTTCT
TGGCCGATTGGAACTACAAATACTGATCAGGGTGAGAACGACCCGTCTTCGTTTTGGCTTACTGAT
CCAGAAACTAAAGATGGAAATGGTGGGATTCCTTGGAATCTGAACGGGAGTGATCAAAACAAGAAC
AATCATCATAGTTCTAATCAGAGTTCGAGTAGCTTGACTGATCATCTGGGTGGAATTCATCATGCT
CAGAATCATCAACAGCAGCCGATTCATGCTCGAAGTCTTTTCACTCGAGAGTTGAATTTTGGGGAG
TGCAGCACATATGATGGGAGTAGTGTTAGAAATGGAAATTCCCATTTGACGAAGCCAGAATCTGGA
GAGATATTGAATTTTGGGGAGAGTAAGAGGACTGCTTCCAGTGCAAATGGGAATTTCTATTCGGGT
TTGGTGACAGAGGAGAATAATAAGAAGAAGAGATCAGTTGGTAATGAAGAAGGGATGCTTTCATTT
ACTTCTGGTGTGATTTTACCTTCTTCTTGTATCCTGAAATCCAGTGGCGGTACAGGAGGGGATTCA
GATCACTCTGATCTTGAGGCTTCGGTTGTAAAAGAGGCGGATAGCAGCAGGGTTGTGGAACCAGAA
AAGAGGCCACGAAAACGAGGGAGAAAACCTGCCAATGGAAGAGAAGAGCCTTTGAATCATGTTGAA
GCAGAGAGGCAAAGAAGAGAGAAACTTAATCAGAGGTTTTATGCACTGCGAGCTGTGGTTCCTAAT
GTATCAAAGATGGACAAAGCTTCACTTCTTGGGGATGCCATATCATATATCGACGAGCTTAGAACG
AAGCTGCAATCTGCCGAGTCTAGCAAGGAGGAGTTGGAGAAGCAAGTGGAATCAATGAAGAGGGAG
TTAGTAAGCAAGGATTCAAGTCCTCCACCTAAAGAGGAGCTCAAGATGTCAAATAACGAAGGAGTT
AAACTGATAGACATGGATATCGATGTGAAGATAAGTGGATGGGATGCGATGATACGAATCCAATGT
TGTAAAAAGAACCACCCTGCTGCTAGGCTAATGTCGGCTTTGAGAGACCTGGACCTTGATGTTCAG
TATGCCAATGTGTCTGTGATGAATGATTTGATGATCCAGCAAGCCACGGTGAAGATGGGGAGTCGG
TTTTACACGCAAGAAGAGCTTAGGGTAGCCATATCAACAAATGTTGGTGGCGTCCATTAGGCATTG
AACAGAACAACAAATGCAATTAGCAGTTGGGATAGATCCTGGTAAAGATTACAGGTTCCGACTTTA
TTTATTAA

**SEQ ID NO: 38, Populus sp 29.195#1 bHLH-6like**

MTDYRLPPTMNLWTDDNASVMEAFMNSSDLSSLWAPPPQSSASTSTPSAAAQPSEKTMLNQETLQQ
RLQTLIEGACEGWAYAIFWQSSYDYSGASVLGWGDGYYKGEEDKGKTRTRNSASSAVEQEHRKTVL
RKLNSLIAGPNSVTDDAIDEEVTDTEWFFLVSMTQSFVNGSGLPGQALFNGSPVWVAGSERLGASP
CERARQGQVFGLQTLVCIPSASGVVELGSTELIFQSSDLMNKVRVLFDFNSLEVVSWPIGTTNTDQ
GENDPSSFWLTDPETKDGNGGIPWNLNGSDQNKNNHHSSNQSSSSLTDHLGGIHHAQNHQQQPIHA

# FIGURE 4 (continued)

RSLFTRELNFGECSTYDGSSVRNGNSHLTKPESGEILNFGESKRTASSANGNFYSGLVTEENNKKK
RSVGNEEGMLSFTSGVILPSSCILKSSGGTGGDSDHSDLEASVVKEADSSRVVEPEKRPRKRGRKP
ANGREEPLNHVEAERQRREKLNQRFYALRAVVPNVSKMDKASLLGDAISYIDELRTKLQSAESSKE
ELEKQVESMKRELVSKDSSPPPKEELKMSNNEGVKLIDMDIDVKISGWDAMIRIQCCKKNHPAARL
MSALRDLDLDVQYANVSVMNDLMIQQATVKMGSRFYTQEELRVAISTNVGGVH

**SEQ ID NO: 39, gi|157285014|gb|EF636725.2| Vitis vinifera Myc2 bHLH protein (Myc2) mRNA, complete cds**

ATGAAAACTGAAATGGGTATGGGAGGAGGGGCTTGGACGGAGGAGGATAAAGCGATGGTGGCGGCT
GTTTTGGGAACTCGAGCATTTGATTACTTGATGACCAGCTCCGTTGTGAGTGAGAATCTGTTAATG
GCAGTGGGGAGTGATGAAAATTTGCAGACAAAGCTTTCGGATCTCGTAGACCGACCAAATGCATCC
AATTTCAGTTGGAATTATGCAATTTTCTGGCAAATTTCACAGTCCAAGTCTGGGGATTGGGTGCTT
GGATGGGGAGATGGGTCTTGTAGGGAGCCCAGGGAAGGGGAGGAATCCGAAGTGACCCGAATTTTA
AATATTCGGCTCGAGGATGCAACCCAGCAGAGGATGAGGAAAAGGGTGCTTCAGAAGCTGCATACA
TTGTTTGGAGGCTCGGATGAGGACAGTTATGCTTTTGGATTGGACCGAGTCACTGATACTGAAATG
TTTTTTCTCGCTTCCATGTACTTCTCATTTACCAGAGGAGAAGGTGGCCCAGGGAAATCTTTTGGA
TCTGGGAAGCATTTGTGGCTGTCTGATGCATTGAAATCTCCATCAGATTATTGTGTTCGATCATTC
CTTGCCAAATCTGCTGGAATTCAGACCATTGTGTTAATCCCAACTGATGTTGGGGTTGTAGAGCTG
GGTTCTGTGAGGTCATTACCTGAAAGCTTAGAGATGTTGCAGACTATAAGATCATCATTCTCGATG
TATTTGCCATTCATCAGGGGCAAGCCAGCCTTACCAGTGTTGAACGAGAAGAAAAATGAAAGTGCT
CCTTTTTCCAATCTGGGGACTGGGGAGCGAGTAGAGGGAATTCCAAAGATTTTTGGGCAGGATTTG
AACTCAGGTCATTCCCATTTTAGGGAGAAACTTGCTGTTCGAAAGGCAGAAGAGAGGCCATGGGAC
AGCTACCAAAACGGGAACAGGCTTCCTTTTACGAACACTAGAAATGGTTTTCATGGTTCGGGCTGG
CCACACATGCAGGGTGTGAAACCAGCAAGTACGGCAGAAATGTACAGTCCTCAGGTCCCAATCAAT
AATCTACATGAGATGGTTAATGGGGTTAGAGAGGAGTTTCGGCTTAGCCAGTTCCAGCCTCCCAAG
CAGGTGCAGATGCAAATTGATTTTGCAGGGGCTGCTTCAAGGTCTACCATGTTGGCTCGGCCAATT
AGTGTGGAGTCTGAGCATTCAGATGTCGAGGCTTCATGCAAGGATGAGCGGCCAGGCCCAGCTGAT
GAAAGGAGGCCCCGGAAAAGGGGCAGAAAGCCTGCAAATGGAAGAGAAGAACCCCTCAATCATGTG
GAAGCAGAGAGGCAGAGGCGTGAGAAACTGAACCAGCGGTTTTATGCATTGCGAGCTGTTGTGCCC
AACATCTCCAAGATGGACAAAGCCTCCTTGCTGGGAGATGCAATTACTTACATCACCGAGCTCCAG
AAGAAACTCAAGGACATGGAATCAGAGCGGGAGAAATTTGGGAGCACTTCAAGAGATGCATTATCT
TTGGAAACTAACACCGAGGCAGAAACTCATATACAGGCGTCTGATGTCGATATCCAAGCAGCCAAT
GATGAAGTTATTGTGAGAGTAAGCTGCCCACTGGATACCCACCCTGTATCAAGAGTCATCCAAACA
TTCAAGGAGGCACAGATCACAGTGATCGAGTCGAAACTTGCTACAGACAATGATACTGTGTTACAT
ACTTTTGTAATCAAGTCCCAGGGATCCGAGCAGCTGATGAAGGAAAAGCTCACAGCTGCATTTTCG
CGCGAATCCAATTCTTTACAGCCTTTGTCATCATCAGTTGGGTAA

**SEQ ID NO: 40, gi|149347186|gb|ABR23669.1| Myc2 bHLH protein [Vitis vinifera]**

MKTEMGMGGGAWTEEDKAMVAAVLGTRAFDYLMTSSVVSENLLMAVGSDENLQTKLSDLVDRPNAS
NFSWNYAIFWQISQSKSGDWVLGWGDGSCREPREGEESEVTRILNIRLEDATQQRMRKRVLQKLHT
LFGGSDEDSYAFGLDRVTDTEMFFLASMYFSFTRGEGGPGKSFGSGKHLWLSDALKSPSDYCVRSF
LAKSAGIQTIVLIPTDVGVVELGSVRSLPESLEMLQTIRSSFSMYLPFIRGKPALPVLNEKKNESA
PFSNLGTGERVEGIPKIFGQDLNSGHSHFREKLAVRKAEERPWDSYQNGNRLPFTNTRNGFHGSGW
PHMQGVKPASTAEMYSPQVPINNLHEMVNGVREEFRLSQFQPPKQVQMQIDFAGAASRSTMLARPI
SVESEHSDVEASCKDERPGPADERRPRKRGRKPANGREEPLNHVEAERQRREKLNQRFYALRAVVP
NISKMDKASLLGDAITYITELQKKLKDMESEREKFGSTSRDALSLETNTEAETHIQASDVDIQAAN
DEVIVRVSCPLDTHPVSRVIQTFKEAQITVIESKLATDNDTVLHTFVIKSQGSEQLMKEKLTAAFS
RESNSLQPLSSSVG

## FIGURE 4 (continued)

**SEQ ID NO: 41, gi|125533054|gb|EAY79619.1| hypothetical protein OsI_033578 [Oryza sativa (indica cultivar-group)], variant of SEQ ID NO: 24**
MEAFMASADLPAFPWGAASTPPPPPPPPHHHHQQQQQVLPPPAAAPAAAAFNQDTLQQRLQSIIE
GSRETWTYAIFWQSSIDVSTGASLLGWGDGYYKGCDDDKRKQRSSTPAAAAEQEHRKRVLRELNSL
IAGAGAAPDEAVEEEALFAAQPTWIATGLSSAPCDRARQAYTFGLRTMVCLPLATGVLELGSTDVI
FQTGDSIPRIRALFNLSAAAASSWPPHPDAASADPSVLWLADAPPMDMKDSISAADISVSKPPPPP
PHQIQHFENGSTSTLTENPSPSVHAPTPSQPAAPPQRQQQQQSSQAQQGPFRRELNFSDFASNGG
AAAPPFFKPETGEILNFGNDSSTGRRNPSPAPPAATASLTTAPGSLFSQHTPTLTAAANDAKSNNQ
KRSMEATSRASNTNNHPAATANEGMLSFSSAPTTRPSTGTGAPAKSESDHSDLEASVREVESSRVV
APPPEAEKRPRKRGRKPANGREEPLNHVEAERQRREKLNQRFYALRAVVPNVSKMDKASLLGDAIS
YINELRGKLTALETDKETLQSQMESLKKERDARPPAPSGGGGDGGARCHAVEIEAKILGLEAMIRV
QCHKRNHPAARLMTALRELDLDVYHASVSVVKDLMIQQVAVKMASRVYSQDQLNAALYTRIAEPGT
AAR

**SEQ ID NO: 42, peptide sequence corresponding to MOTIF 7 in NP_001065478**
PRKRGRKPANGREEPLNHVEAERQRREKLNQRFYALRAVVPNVSKMDKASLLGDAISYINELRGKL
TALE

**SEQ ID NO: 43, peptide sequence corresponding to MOTIF 7 in EAY79619**
PRKRGRKPANGREEPLNHVEAERQRREKLNQRFYALRAVVPNVSKMDKASLLGDAISYINELRGKL
TALE

**SEQ ID NO: 44, peptide sequence corresponding to MOTIF 7 in AAD15818**
PRKRGRKPANGREEPLNHVEAERQRREKLNQRFYALRAVVPNVSKMDKASLLGDAISYINELRGKL
TSLE

**SEQ ID NO: 45, peptide sequence corresponding to MOTIF 7 in AAB00686**
PRKRGRKPGNGREEPLNHVEAERQRREKLNQRFYALRAVVPNVSKMDKASLLGDAISYINELKSKL
SELE

**SEQ ID NO: 46, peptide sequence corresponding to MOTIF 7 in ABD59338**
PRKRGRKPANGREEPLNHVEAERQRREKLNQRFYALRAVVPNVSKMDKASLLGDAISYINELKLKL
QGLE

**SEQ ID NO: 47, peptide sequence corresponding to MOTIF 7 in Pt29.195#1**
PRKRGRKPANGREEPLNHVEAERQRREKLNQRFYALRAVVPNVSKMDKASLLGDAISYIDELRTKL
QSAE

**SEQ ID NO: 48, peptide sequence corresponding to MOTIF 7 in CAF74710**
PRKRGRKPANGREEPLNHVEAERQRREKLNQRFYALRAVVPNVSKMDKASLLGDAISYINELKSKL
QNTE

**FIGURE 4 (continued)**

**SEQ ID NO: 49, peptide sequence corresponding to MOTIF 7 in AAF04917**
PRKRGRKPANGREEPLNHVEAERQRREKLNQRFFSLRAVVPNVSKMDKASLLGDAISYINELKSKL
QNTE

**SEQ ID NO: 50, peptide sequence corresponding to MOTIF 7 in AAQ14332**
PRKRGRKPANGREEPLNHVEAERQRREKLNQRFYALRAVVPNVSKMDKASLLGDAISYINELKAKL
QTTE

**SEQ ID NO: 51, peptide sequence corresponding to MOTIF 7 in AAY90122**
PRKRGRKPANGREEPLNHVEAERQRREKLNQRFYALRAVVPNVSKMDKASLLGDAISFINELKSKL
QNVE

**SEQ ID NO: 52, peptide sequence corresponding to MOTIF 7 in AAL55713**
PKKRGRKPANGREEPLNHVEAERQRREKLNQRFYALRAVVPNVSKMDKASLLGDAIAYINELKSKV
VKTE

**SEQ ID NO: 53, peptide sequence corresponding to MOTIF 7 in ABD65632**
PRKRGRKPANGREEPLNHVEAERQRREKLNQRFYSLRAVVPNVSKMDKASLLGDAISYINELKAKL
QKAE

**SEQ ID NO: 54, peptide sequence corresponding to MOTIF 7 in ABK94979**
PKKRGRKPGLGRDAPLNHVEAERQRREKLNHRFYALRAVVPNVSRMDKASLLSDAVSYINELKAKV
DELE

**SEQ ID NO: 55, peptide sequence corresponding to MOTIF 7 in EDQ81347**
PRKRGRKPANDREEPLNHVQAERQRREKLNQKFYALRSVVPNVSKMDKASLLEDAITYINELQEKL
QKAE

**SEQ ID NO: 56, expression cassette**
AATCCGAAAAGTTTCTGCACCGTTTTCACCCCCTAACTAACAATATAGGGAACGTGTGCTAAATAT
AAAATGAGACCTTATATATGTAGCGCTGATAACTAGAACTATGCAAGAAAAACTCATCCACCTACT
TTAGTGGCAATCGGGCTAAATAAAAAAGAGTCGCTACACTAGTTTCGTTTTCCTTAGTAATTAAGT
GGGAAAATGAAATCATTATTGCTTAGAATATACGTTCACATCTCTGTCATGAAGTTAAATTATTCG
AGGTAGCCATAATTGTCATCAAACTCTTCTTGAATAAAAAAATCTTTCTAGCTGAACTCAATGGGT
AAAGAGAGAGATTTTTTTAAAAAAATAGAATGAAGATATTCTGAACGTATTGGCAAAGATTTAAA
CATATAATTATATAATTTTATAGTTTGTGCATTCGTCATATCGCACATCATTAAGGACATGTCTTA
CTCCATCCCAATTTTTATTTAGTAATTAAAGACAATTGACTTATTTTTATTATTTATCTTTTTTCG
ATTAGATGCAAGGTACTTACGCACACACTTTGTGCTCATGTGCATGTGTGAGTGCACCTCCTCAAT
ACACGTTCAACTAGCAACACATCTCTAATATCACTCGCCTATTTAATACATTTAGGTAGCAATATC
TGAATTCAAGCACTCCACCATCACCAGACCACTTTTAATAATATCTAAAATACAAAAAATAATTTT
ACAGAATAGCATGAAAAGTATGAAACGAACTATTTAGGTTTTTCACATACAAAAAAAAAAAGAATT

**FIGURE 4 (continued)**

TTGCTCGTGCGCGAGCGCCAATCTCCCATATTGGGCACACAGGCAACAACAGAGTGGCTGCCCACAGAA
CAACCCACAAAAAACGATGATCTAACGGAGGACAGCAAGTCCGCAACAACCTTTTAACAGCAGGCTTTG
CGGCCAGGAGAGAGGAGGAGAGGCAAAGAAAACCAAGCATCCTCCTCCTCCCATCTATAAATTCCTCCC
CCCTTTTCCCCTCTCTATATAGGAGGCATCCAAGCCAAGAAGAGGGAGAGCACCAAGGACACGCGACTA
GCAGAAGCCGAGCGACCGCCTTCTTCGATCCATATCTTCCGGTCGAGTTCTTGGTCGATCTCTTCCCTC
CTCCACCTCCTCCTCACAGGGTATGTGCCCTTCGGTTGTTCTTGGATTTATTGTTCTAGGTTGTGTAGT
ACGGGCGTTGATGTTAGGAAAGGGGATCTGTATCTGTGATGATTCCTGTTCTTGGATTTGGGATAGAGG
GGTTCTTGATGTTGCATGTTATCGGTTCGGTTTGATTAGTAGTATGGTTTTCAATCGTCTGGAGAGCTC
TATGGAAATGAAATGGTTTAGGGTACGGAATCTTGCGATTTTGTGAGTACCTTTTGTTTGAGGTAAAAT
CAGAGCACCGGTGATTTTGCTTGGTGTAATAAAAGTACGGTTGTTTGGTCCTCGATTCTGGTAGTGATG
CTTCTCGATTTGACGAAGCTATCCTTTGTTTATTCCCTATTGAACAAAAATAATCCAACTTTGAAGACG
GTCCCGTTGATGAGATTGAATGATTGATTCTTAAGCCTGTCCAAAATTTCGCAGCTGGCTTGTTTAGAT
ACAGTAGTCCCCATCACGAAATTCATGGAAACAGTTATAATCCTCAGGAACAGGGGATTCCCTGTTCTT
CCGATTTGCTTTAGTCCCAGAATTTTTTTTCCCAAATATCTTAAAAAGTCACTTTCTGGTTCAGTTCAA
TGAATTGATTGCTACAAATAATGCTTTTATAGCGTTATCCTAGCTGTAGTTCAGTTAATAGGTAATACC
CCTATAGTTTAGTCAGGAGAAGAACTTATCCGATTTCTGATCTCCATTTTTAATTATATGAAATGAACT
GTAGCATAAGCAGTATTCATTTGGATTATTTTTTTATTAGCTCTCACCCCTTCATTATTCTGAGCTGA
AAGTCTGGCATGAACTGTCCTCAATTTTGTTTTCAAATTCACATCGATTATCTATGCATTATCCTCTTG
TATCTACCTGTAGAAGTTTCTTTTTGGTTATTCCTTGACTGCTTGATTACAGAAAGAAATTTATGAAGC
TGTAATCGGGATAGTTATACTGCTTGTTCTTATGATTCATTTCCTTTGTGCAGTTCTTGGTGTAGCTTG
CCACTTTCACCAGCAAAGTTCATTTAAATCAACTAGGGATATCACAAGTTTGTACAAAAAAGCAGGCTT
AAACAATGACTGATTACCGGCTACAACCAACGATGAATCTTTGGACCACCGACGACAACGCTTCTATGA
TGGAAGCTTTCATGAGCTCTTCCGATATCTCAACTTTATGGCCTCCGGCGTCGACGACAACCACGACGG
CGACGACTGAAACAACTCCGACGCCGGCGATGGAGATTCCGGCACAGGCGGGATTTAATCAAGAGACTC
TTCAGCAACGTTTACAAGCTTTGATTGAAGGAACACACGAAGGTTGGACCTACGCTATATTCTGGCAAC
CGTCGTATGATTTCTCCGGCGCCTCCGTGCTCGGATGGGGAGATGGTTATTACAAAGGTGAAGAAGATA
AAGCAAACCCGAGACGGAGATCGAGTTCGCCGCCGTTTTCTACTCCGGCGGATCAGGAGTACAGGAAAA
AAGTGTTGAGAGAGCTTAACTCGTTGATCTCCGGTGGTGTTGCTCCGTCGGATGACGCTGTTGATGAGG
AGGTGACGGATACGGAATGGTTTTTCTTGGTTTCGATGACGCAGAGCTTCGCTTGCGGTGCGGGATTAG
CTGGTAAAGCGTTTGCAACGGGTAACGCGGTTTGGGTTTCCGGGTCAGATCAATTATCCGGGTCGGGTT
GTGAACGGGCTAAGCAAGGAGGAGTGTTTGGGATGCATACTATTGCGTGTATTCCTTCGGCGAACGGAG
TTGTGGAAGTCGGGTCAACGGAGCCGATCCGACAGAGTTCGGACCTTATTAACAAGGTTCGAATTCTTT
TCAATTTCGACGGCGGAGCTGGAGATTTATCGGGTCTTAATTGGAATCTTGACCCGGATCAAGGTGAGA
ACGACCCGTCTATGTGGATTAATGACCCGATTGGAACACCTGGATCTAACGAACCGGGTAACGGAGCTC
CAAGTTCTAGCTCCCAGCTTTTTTCAAAGTCTATTCAGTTTGAGAACGGTAGCTCAAGCACAATAACCG
AAAACCCGAATCTGGATCCGACTCCGAGTCCGGTTCATTCTCAGACCCAGAATCCGAAATTCAATAACA
CTTTCTCCCGAGAACTTAATTTTTCGACGTCAAGTTCTACTTTAGTGAAACCAAGATCCGGCGAGATAT
TAAACTTCGGCGATGAAGGTAAACGAAGCTCCGGAAACCCGGATCCAAGTTCTTATTCGGGTCAAACAC
AATTCGAAAACAAAAGAAAGAGGTCGATGGTTTTGAACGAAGATAAAGTTCTATCATTCGGAGATAAAA
CCGCCGGAGAATCAGATCACTCCGATCTAGAAGCTTCCGTCGTGAAAGAAGTAGCAGTAGAGAAACGTC
CAAAGAAACGAGGAAGAAAGCCAGCAAACGGTAGAGAAGAGCCACTAAACCACGTCGAAGCAGAGAGAC
AAAGACGCGAGAAACTAAACCAAAGATTCTACGCGTTACGAGCGGTTGTACCAAACGTTTCAAAAATGG
ATAAAGCTTCGTTACTCGGTGACGCAATCGCTTACATCAACGAGCTTAAATCCAAAGTAGTCAAAACAG
AGTCAGAGAAACTCCAAATCAAGAACCAGCTCGAGGAAGTGAAACTCGAGCTCGCCGGAAGAAAAGCGA
GTGCTAGTGGAGGAGATATGTCGTCTTCGTGTTCTTCGATTAAACCGGTGGGGATGGAGATTGAAGTGA
AGATAATTGGTTGGGACGCAATGATTAGAGTTGAATCTAGTAAGAGGAATCATCCGGCGGCGAGGTTGA
TGTCGGCGTTGATGGATTTGGAGTTGGAAGTGAATCACGCGAGTATGTCGGTGGTTAACGATTTGATGA
TTCAACAAGCGACGGTGAAGATGGGTTTTAGGATCTATACGCAAGAACAGCTCAGAGCAAGTTTGATTT
CAAAAATCGGTTAAAA

**FIGURE 4 (continued)**

```
                                1                                                50
         Nicta_RrmJ/FtsJ   (1)  -   A R                           E  N  E  K
 Nicta_RrmJ/FtsJ partial   (1)  --------------------------------------------------
         Arath_RrmJ/FtsJ   (1)  -   A R                           E  N  E  K
         Lyces_RrmJ/FtsJ   (1)  -   A R                           E  N  E AK
         Medtr_RrmJ/FtsJ   (1)  -   A R                           E  N  E  K
         Orysa_RrmJ/FtsJ   (1)  -   A K                           Q  N  H  K
         Ostlu_RrmJ/FtsJ   (1)  -   S K                           ES D  RD RH
         Poptr_RrmJ/FtsJ   (1)  -  RA R                       I   E  N  E  K
         Sacof_RrmJ/FtsJ   (1)  -   A K                           Q  N  H  K
         Triae_RrmJ/FtsJ   (1)  -   A K                       M   Q  N  H  K A
         Apime_RrmJ/FtsJ   (1)  -   T K        R                  N CH LD  NKA
         Danre_RrmJ/FtsJ   (1)  -  RS K        L                L E KL  R  S A
         Homsa_RrmJ/FtsJ   (1)  -  RT K    V   L   N            L K QL  Q  T A
         Caeel_RrmJ/FtsJ   (1)  -   T R        L   NK         M   D  Q LK  R A
       Arath_RrmJ/FtsJ 2   (1)  MGKVKGKHRL K   L   R F S ASY    L AKYSLLHSAHA L
       Orysa_RrmJ/FtsJ 3   (1)  --MSGAGGTA FF  E QRL YV       I  QKQHKLIAPGAA L  G
       Arath_RrmJ/FtsJ 3   (1)  ---MSGAGVP FF  E QRL YV          QKQYKLIKPGSS L  G
               Consensus   (1)   MGKASKDKRDIYYRKAKEEGWRARSAFKLLQID EFNIF GVKRVVDLC
```

**FIGURE 5**

```
                                51                                                  100
           Nicta_RrmJ/FtsJ  (50)             K  L   LSPDTKDDD
   Nicta_RrmJ/FtsJ partial   (1) ---------------------------------------
           Arath_RrmJ/FtsJ  (50)             Q  L   SSAESKDGD
           Lyces_RrmJ/FtsJ  (50)             K  L   LSPGTKDDD
           Medtr_RrmJ/FtsJ  (50)             K  L   LAPDAKDEN
           Orysa_RrmJ/FtsJ  (50)             N  V   QSPDCKEGD
           Ostlu_RrmJ/FtsJ  (50)             K  L  LAR-GVEEEE  K                  TTI
           Poptr_RrmJ/FtsJ  (50)             K  L   LSPDSRDND             L
           Sacof_RrmJ/FtsJ  (50)             N  V   QSPDCKEGD
           Triae_RrmJ/FtsJ  (50)             N  L   LSSDGKDGG
           Apime_RrmJ/FtsJ  (50)             R NENY KALETGNAIP K   V   A   L     I
           Danre_RrmJ/FtsJ  (50)             K RG-------KDKSEEVK   V   A   LP  T I
           Homsa_RrmJ/FtsJ  (50)             KIGG-------QGSG---HV  V   A   LP  V I
           Caeel_RrmJ/FtsJ  (50)            KR  E-------EDQEA--K            P    L
         Arath_RrmJ/FtsJ 2  (51)     G M  AVEKVPVG------------S VLG   V IL VR CVTMT
         Orysa_RrmJ/FtsJ 3  (49) C   A L  ACQN GPLE ----GGVIVGVDVKKVKVPSAHCDSR RT C
         Arath_RrmJ/FtsJ 3  (48) C   A L  ACQS GPLRS----GGIVVGMD KKVKVP-PQCDSR QTIA
                 Consensus  (51) AAPGSWSQVLSRKLYLPAK S    D  LPLIVAIDLQPMAPIEGVIQVQ
```

FIGURE 5 (continued)

```
                                   101                                              150
      Nicta_RrmJ/FtsJ  (100)             K            ---   I
Nicta_RrmJ/FtsJ partial  (12)             K            ---   I
      Arath_RrmJ/FtsJ                   (100)             R            ---
      Lyces_RrmJ/FtsJ  (100)             K V          ---
      Medtr_RrmJ/FtsJ  (100)             R            ---  N
      Orysa_RrmJ/FtsJ  (100)             R            ---
      Ostlu_RrmJ/FtsJ   (99)           SMDKVRE LS    K---H   I G           L   M A
      Poptr_RrmJ/FtsJ  (100)             R           S---
      Sacof_RrmJ/FtsJ  (100)             R            ---
      Triae_RrmJ/FtsJ  (100)             R            ---
      Apime_RrmJ/FtsJ  (100)            ID  KQI S   NE---Q                   IYI    L
      Danre_RrmJ/FtsJ   (93)           KIS   EI    E E---S                 V  YI A  L
      Homsa_RrmJ/FtsJ   (90)           QLS  KEI Q  K  ---P                 V  YM A  L
      Caeel_RrmJ/FtsJ   (91)           SVD  NQ  K  S E--- S I I         I SL   M AE
    Arath_RrmJ/FtsJ 2   (89)       Q   RTECKSKIKQVMEQHGVSAFN  LH  S N G AWAQEAMS NA V
    Orysa_RrmJ/FtsJ 3   (95)       A VMALMKQQARAMSPQER---GFSVILS MC V S ITTK AAISCE G
    Arath_RrmJ/FtsJ 3   (93)       A VL FPRQKIRELSPQQL---GFSVILS MCHS S ITTR AALSAE G
            Consensus  (101)       GDITNARTAEVVIRHFDGC   KADLVVCDGAPDVTGLHDMDEFVQSQLI
```

**FIGURE 5 (continued)**

```
                                  151                                                  200
          Nicta_RrmJ/FtsJ  (147)  G     ---------------------------- I  G    I
  Nicta_RrmJ/FtsJ partial   (59)  G     ---------------------------- I  G    I
          Arath_RrmJ/FtsJ  (147)  G     ---------------------------- I  E    I
          Lyces_RrmJ/FtsJ  (147)  G     ---------------------------- I  E    I
          Medtr_RrmJ/FtsJ  (147)  G     ----------------------------    E    I
          Orysa_RrmJ/FtsJ  (147)        ----------------------------    V
          Ostlu_RrmJ/FtsJ  (146)  G  VA ---------------------------- I  P  T I
          Poptr_RrmJ/FtsJ  (147)  G     ----------------------------    E    I
          Sacof_RrmJ/FtsJ  (147)        ----------------------------    V
          Triae_RrmJ/FtsJ  (147)        ----------------------------    V
          Apime_RrmJ/FtsJ  (147)    N T ---------------------------- I  Q  T
          Danre_RrmJ/FtsJ  (140)    N T ----------------------------    P  N
          Homsa_RrmJ/FtsJ  (137)    N A ----------------------------    P  C
          Caeel_RrmJ/FtsJ  (138)   FN TS----------------------------    E  N L
        Arath_RrmJ/FtsJ 2  (139) IDSVRLA ----------------------------EF ARN NL T V
        Orysa_RrmJ/FtsJ 3  (142) MR  SLAVGKMKAKDSDCIAILEKFQSSTEPDPDEDGI RR  SL I FL
        Arath_RrmJ/FtsJ 3  (140) MR  DLAVGQAAISQ-------SPNDDDGGPNESRPG  RH  HL I LL
                Consensus  (151) LAALTIVT                            HVLK GGKFVAKIF
```

**FIGURE 5 (continued)**

```
                                 201                                              250
       Nicta_RrmJ/FtsJ  (169)                    TE                              NEK
Nicta_RrmJ/FtsJ partial  (81)                    TE                              NEK
       Arath_RrmJ/FtsJ  (169)                    PT                              N R
       Lyces_RrmJ/FtsJ  (169)                    TE                              NEK
       Medtr_RrmJ/FtsJ  (169)                    PV                              N K
       Orysa_RrmJ/FtsJ  (169)                    SQ                              KEK
       Ostlu_RrmJ/FtsJ  (168)     I    S   I  PE  C              I  QG       E H
       Poptr_RrmJ/FtsJ  (169)                    PV                              D K
       Sacof_RrmJ/FtsJ  (169)                    SQ                              KEE
       Triae_RrmJ/FtsJ  (169)                                                X LQEK
       Apime_RrmJ/FtsJ  (169)  A  VT   A   I  PY YCT  S          V  KD      YK H
       Danre_RrmJ/FtsJ  (162)     VT   S   I  SF  C   P          V  Q       YV N
       Homsa_RrmJ/FtsJ  (159)   R VT   S  QV  SS LC   R             QG D     I D
       Caeel_RrmJ/FtsJ  (160)  SRNS    A M KY KK YL   R   Q  C   VL LD       V T
     Arath_RrmJ/FtsJ 2  (161)  SR YNSVLYC GRL EK EVF  PA  SA A TYL GLK LA AKID R
     Orysa_RrmJ/FtsJ 3  (192) ENE IPGFGKFC EK KK SLLR  AT S  R I M   GRGFSVAPRWH
     Arath_RrmJ/FtsJ 3  (183) ESE AQDFARIC PI NKASWLR  AT P  R IYLI QGFR--------
             Consensus  (201) RGKDTSLLYCQLKLFF  VTFAKPKSSRNSSIEAFAVCENYSPPEGF PK
```

FIGURE 5 (continued)

```
                                  251                                                 300
         Nicta_RrmJ/FtsJ  (219)    HR    KI S    TE L   S A       K
 Nicta_RrmJ/FtsJ partial  (131)    HR    KI S    TE L   S A       K
         Arath_RrmJ/FtsJ  (219)    HR    KV S    GS L   S         K            T
         Lyces_RrmJ/FtsJ  (219)    YR    QV S    AE L   S       R K
         Medtr_RrmJ/FtsJ  (219)    HR    KV S    VD X   V         K            T
         Orysa_RrmJ/FtsJ  (219)    YH    KV T    AD L   R         K
         Ostlu_RrmJ/FtsJ  (218) Q TRI   ARATSHAAG EGAAV TKSW  N LV                A Q
         Poptr_RrmJ/FtsJ  (219)    RR    KV S    AD L   S       ASK
         Sacof_RrmJ/FtsJ  (219)    YH    KV T    GD L   R         K
         Triae_RrmJ/FtsJ  (219)    YH   XKV T    AD L   R       TKQ          XR    RTVHT
         Apime_RrmJ/FtsJ  (219) MMNP   THKP--------- DFND T I R IV  VV      QP    TC
         Danre_RrmJ/FtsJ  (212) MSNP   DHSYD--------VDFNQ     RIIV            F     T
         Homsa_RrmJ/FtsJ  (209) LSKP   DHSY----------DFNQ D  TRIIV  VT      S
         Caeel_RrmJ/FtsJ  (210) MGKTS DATDAS---------AISPD----IIDG VT       W   EK
       Arath_RrmJ/FtsJ 2  (211) L DYRHLFKESAEPTRKVVDVL GSKQKRNRDGYEDGESI RRVA AADF
       Orysa_RrmJ/FtsJ 3  (242) P SHGPATY TG TCHAAAHSLVE RTVG-LQREAPK S  KAQAQPAAF
       Arath_RrmJ/FtsJ 3  (225) --------------------------------------------------
               Consensus  (251) DL  LLE VGSPSG  DLDC SGWLEGPNKVYIPFLACGDLSGYDSDRSY
```

**FIGURE 5 (continued)**

```
                                 301                                                350
         Nicta_RrmJ/FtsJ  (269)    PKAADG-----------T QC        A    R  EM  ASNQ-GI
 Nicta_RrmJ/FtsJ partial  (181)    PKAADG-----------T QC        A    R  EM  ASNQ-GI
         Arath_RrmJ/FtsJ  (269)    PREADGS----------S QS   I    A    R  EL  ASAQ-SF
         Lyces_RrmJ/FtsJ  (269)    PKSADG-----------T QC        A    R  EM  ASSQ-GI
         Medtr_RrmJ/FtsJ  (269)    PKVAGG-----------T QS        A    R  EL  ASPQ-GF
         Orysa_RrmJ/FtsJ  (269)    TSTEGG-----------S QS        A    T  EM  VASHGIG
         Ostlu_RrmJ/FtsJ  (268)  A DDTKVR--------------- K      TT A MN IKLL RK-----
         Poptr_RrmJ/FtsJ  (269)    PKDADG-----------T QS        A    R  EM  ASSH-GV
         Sacof_RrmJ/FtsJ  (269)  R PSTEGG-----------S RS        A    T  QM  VSSHSAS
         Triae_RrmJ/FtsJ  (269)    RAQRRQ------------XPX    S  LPG-R NCVEIR GLTRVWR
         Apime_RrmJ/FtsJ  (260)    DFEG-K-----------T TYHE   T  S    E  SLMEDRDTDFR
         Danre_RrmJ/FtsJ  (254)    QLDSSK-----------E QY P T    R   QQ CQLR SNLLAKE
         Homsa_RrmJ/FtsJ  (249)    DLEGGS-----------E KYTP T    S   QE CTL RKGQLAKE
         Caeel_RrmJ/FtsJ  (247)    DIDACFPKGEIDEEQKKR EFK V    TD A  A  DK  SGVFAKM
       Arath_RrmJ/FtsJ 2  (261)  IWSENPLDVLGTTTSISFDDQAS PLKEHDLTTEEIKI CDDLPVLGKND
       Orysa_RrmJ/FtsJ 3  (291)  VRIPRGASLP---------ATRNAAGLHSTLLVASLLHITTHRGGTHFYI
       Arath_RrmJ/FtsJ 3  (225)  --------------------------------------------------
               Consensus  (301)  PL   A            TY  LDPVQPPIAPPYK ALELKK
```

**FIGURE 5 (continued)**

```
                                    351                                              400
         Nicta_RrmJ/FtsJ    (307)   QNLDKLS SS----------------------------------------
 Nicta_RrmJ/FtsJ partial    (219)   QNLDKLS SS----------------------------------------
         Arath_RrmJ/FtsJ    (308)   NS------------------------------------------------
         Lyces_RrmJ/FtsJ    (307)   HNLDKLS DP----------------------------------------
         Medtr_RrmJ/FtsJ    (307)   RELENLS DS----------------------------------------
         Orysa_RrmJ/FtsJ    (308)   ADISKLS DS----------------------------------------
         Ostlu_RrmJ/FtsJ    (298)   --------------------------------------------------
         Poptr_RrmJ/FtsJ    (307)   KELEKLS DS----------------------------------------
         Sacof_RrmJ/FtsJ    (308)   ADAMKPSTDS----------------------------------------
         Triae_RrmJ/FtsJ    (306)   NTAIIFD K-----------------------------------------
         Apime_RrmJ/FtsJ    (298)   RFDVNVVVDNLSSLTIEDFKKQAKQKHKEINETKRIELQDNKKDDSEEDI
         Danre_RrmJ/FtsJ    (293)   DSPS-GA DEALTALDLNTKPDTSTTTPGASE------------------
         Homsa_RrmJ/FtsJ    (288)   IRPQDCPISRVDTFPQPLAAPQCHTLLAPEMEDNEMSCSP----------
         Caeel_RrmJ/FtsJ    (297)   SADLNRQ KAELSRGKDQKKTPAENVPSVEELEKAAEKFQL---------
       Arath_RrmJ/FtsJ 2    (311)   FKHILKWRMQIRKALTPEKKEVAKPEPDVGKEDEENEDDKLLNELEELTN
       Orysa_RrmJ/FtsJ 3    (332)   SLSVGWETPRREECRIPVVPPQCPAPPRKRPVALPELGKERREPPKGGYF
       Arath_RrmJ/FtsJ 3    (225)   --------------------------------------------------
               Consensus    (351)           L
```

FIGURE 5 (continued)

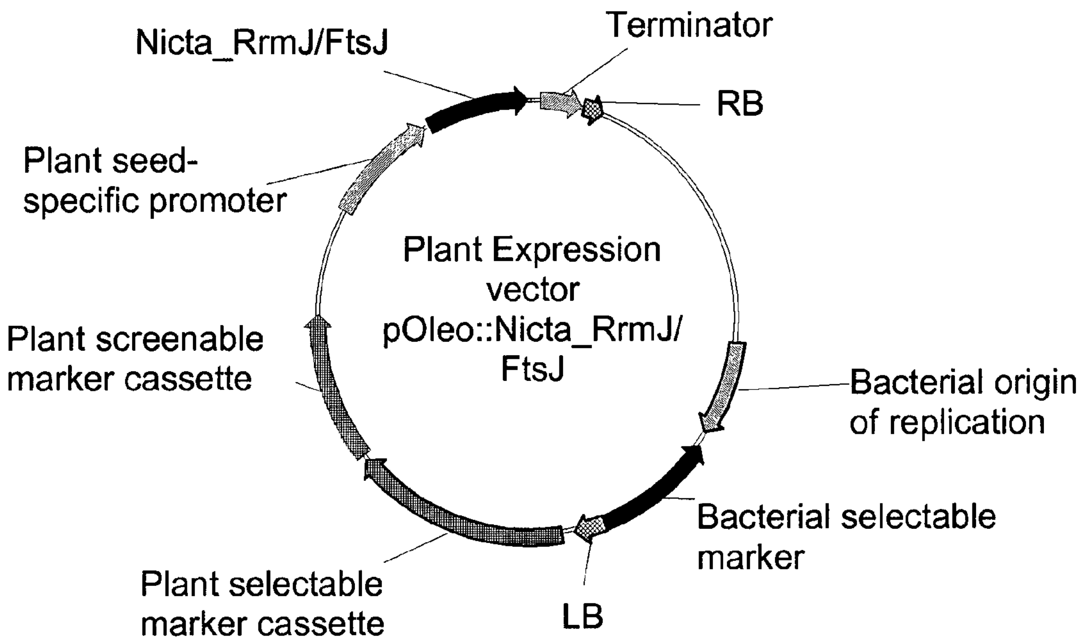
Nicta_RrmJ/FtsJ
Terminator
RB
Plant seed-specific promoter
Plant Expression vector pOleo::Nicta_RrmJ/FtsJ
Plant screenable marker cassette
Bacterial origin of replication
Bacterial selectable marker
Plant selectable marker cassette
LB

**FIGURE 6**

**SEQ ID NO 57: Nicotiana tabacum partial FtsJ nucleic acid sequence**

ATGGCTCCCATTGAAGGTGTTATTCAAGTACAGGGTGATATAACAAATGCTAAAACAGCTGAAGTG
GTTATTAGACATTTTGACGGATGCAAGGCTGACATTGTTGTCTGTGATGGTGCACCTGATGTAACG
GGACTTCATGACATGGATGAATTTGTTCAGTCCCAGCTGATATTGGCGGGCCTAACCATTGTCACT
CACATACTAAAAGGAGGCGGAAAGTTCATAGCAAAAATTTTTCGAGGAAAAGACACAAGCCTTCTT
TACTGTCAGCTAAAACTATTTTTCACAGAAGTGACTTTTGCTAAGCCAAAAAGCAGTCGGAATTCT
AGCATAGAGGCATTTGCAGTTTGCGAGAATTACTCTCCACCTGAAGGATTTAATGAGAAAGATCTT
CATCGCCTTCTTGAAAAGATTGGAAGTCCATCTGGCACAGAGGACCTAGATTGCAGTAGTGCATGG
CTGGAAGGTCCTAATAAGGTGTATATTCCATTTCTGGCTTGTGGAGACCTTAGCGGGTACGATTCA
GACCGTTCATATCCACTTCCTAAAGCTGCAGATGGAACCTATCAGTGTTTAGATCCTGTACAACCT
CCAATTGCACCGCCATATAAACGAGCTCTTGAAATGAAGAAAGCGTCGAATCAAGGAATCCAAAAC
CTAGACAAGCTTTCTCTTAGCTCCTAA

**SEQ ID NO 58: Nicotiana tabacum partial FtsJ translated polypeptide (truncated at N-terminus)**

MAPIEGVIQVQGDITNAKTAEVVIRHFDGCKADIVVCDGAPDVTGLHDMDEFVQSQLILAGLTIVTHILKGG
GKFIAKIFRGKDTSLLYCQLKLFFTEVTFAKPKSSRNSSIEAFAVCENYSPPEGFNEKDLHRLLEKIGSPSG
TEDLDCSSAWLEGPNKVYIPFLACGDLSGYDSDRSYPLPKAADGTYQCLDPVQPPIAPPYKRALEMKKASNQ
GIQNLDKLSLSS

**SEQ ID NO 59: Nicotiana tabacum FtsJ nucleic acid sequence contig of EB446219, EB448450.1**

ATGGGAAAAGCATCGAGGGACAAAAGAGATATTTACTATCGGAAAGCAAAGGAAGAAGGATGGCGT
GCCCGAAGTGCCTTCAAGCTCCTTCAGATTGATGAGGAATTTAACATCTTTGAAGGTGTGAAGCGA
GTTGTGGATTTGTGTGCAGCACCAGGCAGCTGGAGTCAGGTTTTAAGCCGGAAGTTATATCTCCCA
GCAAAGTTGTCACCTGATACCAAGGACGACGATCTACCACTTATTGTGGCTATTGACTTACAGCCT
ATGGCTCCCATTGAAGGTGTTATTCAAGTACAGGGTGATATAACAAATGCTAAAACAGCTGAAGTG
GTTATTAGACATTTTGACGGATGCAAGGCTGACATTGTTGTCTGTGATGGTGCACCTGATGTAACG
GGACTTCATGACATGGATGAATTTGTTCAGTCCCAGCTGATATTGGCGGGCCTAACCATTGTCACT
CACATACTAAAAGGAGGCGGAAAGTTCATAGCAAAAATTTTTCGAGGAAAAGACACAAGCCTTCTT
TACTGTCAGCTAAAACTATTTTTCACAGAAGTGACTTTTGCTAAGCCAAAAAGCAGTCGGAATTCT
AGCATAGAGGCATTTGCAGTTTGCGAGAATTACTCTCCACCTGAAGGATTTAATGAGAAAGATCTT
CATCGCCTTCTTGAAAAGATTGGAAGTCCATCTGGCACAGAGGACCTAGATTGCAGTAGTGCATGG
CTGGAAGGTCCTAATAAGGTGTATATTCCATTTCTGGCTTGTGGAGACCTTAGCGGGTACGATTCA
GACCGTTCATATCCACTTCCTAAAGCTGCAGATGGAACCTATCAGTGTTTAGATCCTGTACAACCT
CCAATTGCACCGCCATATAAACGAGCTCTTGAAATGAAGAAAGCGTCGAATCAAGGAATCCAAAAC
CTAGACAAGCTTTCTCTTAGCTCCTAA

**SEQ ID NO 60: Nicotiana tabacum FtsJ translated polypeptide**

MGKASRDKRDIYYRKAKEEGWRARSAFKLLQIDEEFNIFEGVKRVVDLCAAPGSWSQVLSRKLYLP
AKLSPDTKDDDLPLIVAIDLQPMAPIEGVIQVQGDITNAKTAEVVIRHFDGCKADIVVCDGAPDVT
GLHDMDEFVQSQLILAGLTIVTHILKGGGKFIAKIFRGKDTSLLYCQLKLFFTEVTFAKPKSSRNS
SIEAFAVCENYSPPEGFNEKDLHRLLEKIGSPSGTEDLDCSSAWLEGPNKVYIPFLACGDLSGYDS
DRSYPLPKAADGTYQCLDPVQPPIAPPYKRALEMKKASNQGIQNLDKLSLSS

**FIGURE 7**

**SEQ ID NO 61: Arabidopsis thaliana FtsJ nucleic acid sequence polypeptide AY087952.1**

GAATTTCTACTTTGGTGCTGCTCTTCTTCACATACCTAGCTGCTGTATACTATACGTGAACCAACTCCGTAT
TCTACTTTAGAGGCCATGGGAAAAGCTTCTCGAGACAAAAGGGATATCTATTATCGTAAAGCTAAAGAAGAA
GGATGGCGTGCAAGAAGTGCTTTTAAGCTTCTTCAGATTGATGAAGAATTTAATATTTTTGAAGGTGTGAAG
AGGGTCGTAGATTTATGTGCTGCACCTGGTAGCTGGAGTCAGGTCTTGAGTCGTCAATTATATCTTCCTGCA
AAGTCCTCAGCTGAGTCAAAAGATGGGGATCTTCCTCTTATAGTAGCCATCGATTTGCAGCCTATGGCTCCA
ATTGAAGGTGTCATCCAGGTTCAAGGGGACATAACTAATGCTCGGACAGAAGTGGTCATTAGACACTTTGAC
GGCTGCAAAGCGGACCTGGTTGTCTGTGATGGTGCTCCAGATGTTACTGGTTTGCATGACATGGATGAATTT
GTCCAGTCCCAACTCATACTAGCAGGATTAACGATTGTAACTCATATTCTTAAAGAAGGTGGAAAATTTATT
GCAAAGATATTCCGTGGAAAAGATACAAGTCTCTTGTACTGTCAACTGAAATTGTTTTTCCCAACCGTAACT
TTTGCGAAACCGAAAAGCAGCCGCAATTCAAGTATAGAGGCTTTTGCAGTCTGCGAGAATTACTCTCCGCCA
GAAGGATTTAACCCGAGAGATCTGCATCGTCTCTTGGAGAAAGTCGGAAGCCCTTCAGGTGGAAGCGATCTC
GATTGCAGTAGTGGTTGGCTTGAAGGACCCAACAAAGTTTATATACCATTCTTGGCATGTGGTGACTTAACC
GGTTATGACTCAGACCGGTCTTACCCACTCCCTAGAGAAGCAGATGGATCATCATACCAGAGTTTGGACCCG
ATTCAACCTCCCATCGCACCGCCTTATAAACGAGCTCTTGAGCTCAAGAAAGCTTCAGCACAAAGCTTCAAC
TCTTGAAGAAGGTGATACCAAAAAAGAAAAAGGAACAAACTTTTCATTGGATTCTGTAGCCTGTCGTAATGA
TATATTCAGGGACCTACAATTTCTATAACGCTTAATGTTTTTGTTATG

**SEQ ID NO 62: Arabidopsis thaliana FtsJ translated polypeptide**

MGKASRDKRDIYYRKAKEEGWRARSAFKLLQIDEEFNIFEGVKRVVDLCAAPGSWSQVLSRQLYLP
AKSSAESKDGDLPLIVAIDLQPMAPIEGVIQVQGDITNARTAEVVIRHFDGCKADLVVCDGAPDVT
GLHDMDEFVQSQLILAGLTIVTHILKEGGKFIAKIFRGKDTSLLYCQLKLFFPTVTFAKPKSSRNS
SIEAFAVCENYSPPEGFNPRDLHRLLEKVGSPSGGSDLDCSSGWLEGPNKVYIPFLACGDLTGYDS
DRSYPLPREADGSSYQSLDPIQPPIAPPYKRALELKKASAQSFNS

**SEQ ID NO 63: Lycopersicon esculentum FtsJ nucleic acid sequence BT013964**

ATGGGAAAAGCATCTAGGGATAAGAGGGATATTTACTACCGGAAAGCAAAGGAAGAAGGGTGGCGTGCCCGA
AGTGCCTTCAAGCTCCTTCAGATAGATGAGGAGTTTAATATCTTTGAAGGTGCGAAGCGCGTTGTGGATTTA
TGTGCTGCTCCAGGCAGCTGGAGTCAGGTTTTAAGCCGGAAGTTATATCTCCCAGCAAAGCTGTCACCGGGT
ACCAAGGATGATGATCTACCACTTATCGTGGCTATTGACTTACAGCCTATGGCTCCAATTGAAGGTGTTATT
CAAGTACAGGGTGATATAACAAATGCTAAAACAGTTGAAGTGGTTATTAGACATTTTGATGGATGCAAAGCT
GACCTTGTTGTCTGTGATGGTGCACCTGATGTAACGGGACTTCATGACATGGATGAATTTGTTCAATCCCAA
CTGATATTGGCGGGCCTAACCATAGTCACTCACATACTAAAAGAAGGTGGAAAGTTCATAGCAAAAATTTTT
CGAGGAAAAGACACAAGTCTCCTTTACTGTCAACTAAAACTATTTTTCACGGAAGTGACTTTTGCTAAGCCA
AAAGTAGTCGCAATTCTAGCATAGAGGCATTTGCAGTTTGTGAAAACTACTCTCCACCTGAAGGATTCAAT
GAGAAAGACCTTTATCGCCTTCTTGAACAAGTTGGAAGTCCATCAGGCGCTGAGGACTTAGATTGCAGTAGT
GGATGGCTTGAAGGTCGTAACAAGGTGTATATCCCATTTCTGGCTTGCGGAGACCTGAGTGGATATGATTCA
GATCGTTCATATCCGCTTCCTAAATCTGCAGATGGAACCTATCAATGTTTAGATCCTGTACAACCTCCGATT
GCACCACCGTATAAACGAGCTCTTGAAATGAAAAAAGCTTCAAGTCAAGGAATTCATAATCTAGACAAACTT
TCTCTTGACCCCTGA

## FIGURE 7 (continued)

**SEQ ID NO 64: Lycopersicon esculentum FtsJ translated polypeptide**
MGKASRDKRDIYYRKAKEEGWRARSAFKLLQIDEEFNIFEGAKRVVDLCAAPGSWSQVLSRKLYLPAKLSPG
TKDDDLPLIVAIDLQPMAPIEGVIQVQGDITNAKTVEVVIRHFDGCKADLVVCDGAPDVTGLHDMDEFVQSQ
LILAGLTIVTHILKEGGKFIAKIFRGKDTSLLYCQLKLFFTEVTFAKPKSSRNSSIEAFAVCENYSPPEGFN
EKDLYRLLEQVGSPSGAEDLDCSSGWLEGRNKVYIPFLACGDLSGYDSDRSYPLPKSADGTYQCLDPVQPPI
APPYKRALEMKKASSQGIHNLDKLSLDP

**SEQ ID NO 65: Medicago truncatula FtsJ nucleic acid sequence AC149135.2**
ATGGGCAAAGCTTCAAGGGATAAGAGGGATATATATTACCGAAAAGCGAAAGAAGAAGGTTGGCGTGCTCGA
AGTGCTTTTAAACTTCTTCAGATAGATGAGGAATTTAACATTTTTGAAGGGGTGAAACGTGTTGTAGATTTA
TGTGCTGCCCCAGGCAGTTGGAGTCAGGTTTTGAGTCGAAAACTGTACCTTCCAGCCAAGCTTGCACCTGAT
GCAAAGGATGAAAATCTTCCTCTTATTGTAGCTATTGATTTGCAGCCAATGGCTCCGATTGAAGGTGTTATC
CAGGTGCAGGGTGATATAACTAATGCTCGGACCGCTGAAGTGGTCATTAGACATTTCGATGGTTGCAAGGCC
AACCTTGTTGTGTGTGATGGTGCTCCTGATGTTACCGGACTTCATGACATGGATGAATTTGTTCAATCCCAA
CTCATACTTGCAGGGTTGACAATTGTTACTCATGTACTGAAGGAAGGAGGGAAGTTCATTGCGAAGATATTT
AGAGGAAAGGACACAAGCCTTCTATACTGTCAGCTAAAATTATTTTTCCCTGTGGTGACTTTCGCAAAGCCA
AAAAGTAGCCGTAATTCCAGCATAGAGGCATTTGCAGTTTGTGAAAACTACTCCCCTCCTGAAGGATTCAAC
CCGAAAGATCTTCACCGACTTCTTGAGAAGGTTGGAAGTCCATCAGGCGTAGATGATACAGATTGTGTTAGT
GGGTGGTTGGAAGGCCCTAATAAGGTGTATATCCCATTTCTAGCTTGCGGGGACCTCACTGGATATGATTCT
GATAGGTCATATCCACTGCCTAAAGTTGCTGGGGGAACATATCAGAGCTTGGATCCAGTACAACCTCCTATT
GCCCCTCCTTACAAGAGAGCTCTAGAGTTAAAAAAAGCATCACCTCAAGGATTCCGAGAACTTGAAAATCTC
TCCCTGGATTCCTGA

**SEQ ID NO 66: Medicago truncatula FtsJ translated polypeptide**
MGKASRDKRDIYYRKAKEEGWRARSAFKLLQIDEEFNIFEGVKRVVDLCAAPGSWSQVLSRKLYLPAKLAPD
AKDENLPLIVAIDLQPMAPIEGVIQVQGDITNARTAEVVIRHFDGCKANLVVCDGAPDVTGLHDMDEFVQSQ
LILAGLTIVTHVLKEGGKFIAKIFRGKDTSLLYCQLKLFFPVVTFAKPKSSRNSSIEAFAVCENYSPPEGFN
PKDLHRLLEKVGSPSGVDDXDCVSGWLEGPNKVYIPFLACGDLTGYDSDRSYPLPKVAGGTYQSLDPVQPPI
APPYKRALELKKASPQGFRELENLSLDS

**SEQ ID NO 67: Oryza sativa FtsJ nucleic acid sequence AK103054**
ATGGGCAAGGCCTCCAAAGACAAGAGGGACATCTACTACCGCAAGGCGAAGGAGGAGGGGTGGAGAGCTCGT
AGCGCCTTCAAGCTTCTGCAGATCGACCAGGAGTTCAACATCTTCCACGGAGTGAAGCGTGTTGTTGATCTG
TGCGCTGCTCCCGGAAGCTGGAGTCAGGTTTTGAGTCGGAACCTGTATGTTCCAGCAAAACAATCTCCTGAT
TGCAAAGAAGGTGACCTTCCTCTTATTGTTGCCATTGATTTGCAACCAATGGCTCCGATAGAAGGCGTTATA
CAAGTTCAAGGGGACATCACTAATGCTCGAACAGCAGAAGTGGTTATTAGGCATTTTGATGGATGCAAAGCA
GATTTGGTTGTCTGCGATGGTGCTCCTGATGTTACCGGCCTTCATGATATGGACGAGTTTGTTCAGTCCCAG
CTTATACTGGCGGCATTGACAATTGTGACTCACGTACTTAAAGTTGGTGGGAAATTTGTTGCGAAGATTTTC
CGTGGAAAAGATACAAGTCTGTTGTATTGCCAGCTGAAACTATTCTTCTCACAAGTTACCTTTGCAAAGCCC
AAAAGTAGCCGGAACTCAAGCATCGAGGCGTTTGCAGTTTGTGAGAATTATTCGCCTCCCGAAGGATTTAAG
GAAAAAGATTTATATCACCTGCTAGAGAAAGTGGGGACTCCTTCTGGGGCTGATGATTTAGACTGCAGAAGT
GGATGGTTAGAGGGACCAAACAAGGTCTACATCCCATTTCTGGCTTGCGGTGACCTCAGTGGTTACGATTCG
GATCGTTCTTACCCACTAACAAGCACAGAGGGAGGATCCTACCAGAGCTTGGATCCAGTCCAGCCTCCAATT
GCTCCACCTTACAAAACCGCACTGGAGATGAAAAAGGTCGCCAGCCATGGCATTGGAGCAGATATCAGCAAA
TTATCCCTCGACTCCTGAACTACACTCTGGATCGCTGTTTGTGTTCTTGTAACTGCCAACCCTGTGGAATTT
GCAAATTGTA

**FIGURE 7 (continued)**

**SEQ ID NO 68: Oryza sativa FtsJ translated polypeptide**
MGKASKDKRDIYYRKAKEEGWRARSAFKLLQIDQEFNIFHGVKRVVDLCAAPGSWSQVLSRNLYVPAKQSPD
CKEGDLPLIVAIDLQPMAPIEGVIQVQGDITNARTAEVVIRHFDGCKADLVVCDGAPDVTGLHDMDEFVQSQ
LILAALTIVTHVLKVGGKFVAKIFRGKDTSLLYCQLKLFFSQVTFAKPKSSRNSSIEAFAVCENYSPPEGFK
EKDLYHLLEKVGTPSGADDLDCRSGWLEGPNKVYIPFLACGDLSGYDSDRSYPLTSTEGGSYQSLDPVQPPI
APPYKTALEMKKVASHGIGADISKLSLDS

**SEQ ID NO 69: Ostreococcus lucimarinus CCE9901 FtsJ nucleic acid sequence XM_001418019**
ATGGGGAAGTCGAGCAAGGATAAGCGCGATATATACTACCGCAAGGCGAAAGAGGAGGGATGGCGCGCGAGG
TCGGCGTTCAAGTTGCTGCAAATTGACGAGTCGTTCGATATTTTTCGAGACGTGCGACACGTCGTGGATCTG
TGCGCGGCGCCCGGGTCGTGGTCGCAGGTTTTGAGTCGAAAGTTGTACCTACCCGCGTTGGCGCGAGGGGTC
GAGGAGGAAGAGCTGCCGAAGATTGTCGCCATCGACTTGCAACCGATGGCGCCGATCGAGGGCGTGACGACG
ATACAGGGCGATATCACGTCGATGGACAAAGTGCGCGAGGTCCTGTCGCATTTCGATGGGAAACACGCGGAT
TTGATCGTCGGCGATGGAGCGCCCGACGTCACGGGTTTGCACGATTTGGATGAGTTTATGCAGGCGCAGCTC
ATACTCGCCGGCTTGACGGTGGCGACGCACATATTGAAACCAGGCGGAACGTTCATAGCGAAGATATTTCGG
GGGAAAGACATCAGTTTGCTCTACTCACAACTGAAAATTTTCTTCCCCGAAGTCACGTGCGCCAAGCCGAAA
AGTAGCCGGAATTCTAGCATAGAAGCATTCATAGTGTGTCAGGGTTACTCCCCTCCAGAGGGATTCGAACCA
CATCAATTGACTCGTATTTTAGAAGCGCGTGCGACGTCTCACGCCGCTGGAGACGAGGGCGCGGCCGTGGGA
ACCAAGTCCTGGCCGAACAATGTGCTCGTGCCGTTTTTGGCGTGCGGCGATTTGTCGGGATACGACGCCGAT
CAGAGCTATGCGTTGGATGACACCAAAGTCAGACTAAAACCGGTGCAACCGCCGACAACACCGGCGTATATG
AACGCAATCAAGCTCTTAAAGAGAAAGTGA

**SEQ ID NO 70: Ostreococcus lucimarinus CCE9901 FtsJ translated polypeptide**
MGKSSKDKRDIYYRKAKEEGWRARSAFKLLQIDESFDIFRDVRHVVDLCAAPGSWSQVLSRKLYLPALARGV
EEEELPKIVAIDLQPMAPIEGVTTIQGDITSMDKVREVLSHFDGKHADLIVGDGAPDVTGLHDLDEFMQAQL
ILAGLTVATHILKPGGTFIAKIFRGKDISLLYSQLKIFFPEVTCAKPKSSRNSSIEAFIVCQGYSPPEGFEP
HQLTRILEARATSHAAGDEGAAVGTKSWPNNVLVPFLACGDLSGYDADQSYALDDTKVRLKPVQPPTTPAYM
NAIKLLKRK

**SEQ ID NO 71: Populus tremuloides FtsJ nucleic acid sequence contig of CX183550.1, DT504907.1**
ATGGGGAGAGCTTCAAGGGATAAAAGGGATATATACTATAGAAAAGCAAAGGAAGAAGGTTGGCGTGCTCGA
AGTGCCTTTAAGCTCATTCAGATAGATGAGGAATTCAATATTTTTGAAGGAGTGAAGCGTGTGGTGGATTTA
TGTGCTGCACCTGGTAGCTGGAGTCAGGTTTTGAGCCGTAAACTATATTTACCAGCAAAACTTTCACCTGAT
TCAAGGGATAATGATCTTCCTCTTATTGTGGCCATTGATTTGCAACCTATGGCTCTCATTGAAGGTGTTATC
CAAGTCCAGGGTGATATTACCAATGCCCGAACTGCTGAAGTGGTCATTAGACATTTTGATGGCAGCAAGGCT
GACTTGGTTGTGTGTGATGGTGCCCCTGATGTTACCGGACTCCATGACATGGATGAATTTGTTCAGTCTCAA
CTCATACTAGCGGGTTTAACAATTGTCACACATGTACTCAAAGAAGGTGGAAAATTTATTGCGAAGATATTT
CGTGGAAAAGATACAAGTCTTCTGTATTGCCAGCTCAAATTATTTTTTCCTGTGGTGACTTTTGCCAAACCA
AAAAGTAGCCGCAATTCCAGCATAGAGGCATTTGCAGTTTGTGAGAATTACTCTCCTCCTGAGGGATTTGAT
CCGAAAGACTTGCGTCGCCTTTTGGAAAAGGTGGGAAGCCCCTCTGGTGCAGATGACCTAGATTGCAGTAGC
GGGTGGTTAGAAGGGGCAAGTAAGGTGTATATTCCATTTCTAGCTTGCGGTGACCTTAGTGGGTATGACTCT
GACCGATCATATCCACTACCAAAAGATGCCGATGGCACATATCAGAGCTTGGATCCTGTACAACCCCCAATT
GCCCCTCCTTATAAAAGAGCCCTTGAAATGAAGAAAGCTTCTAGTCATGGTGTAAAAGAGCTTGAAAAGCTC
TCTTTGGATTCTTGA

## FIGURE 7 (continued)

**SEQ ID NO 72: Populus tremuloides FtsJ translated polypeptide**
MGRASRDKRDIYYRKAKEEGWRARSAFKLIQIDEEFNIFEGVKRVVDLCAAPGSWSQVLSRKLYLPAKLSPD
SRDNDLPLIVAIDLQPMALIEGVIQVQGDITNARTAEVVIRHFDGSKADLVVCDGAPDVTGLHDMDEFVQSQ
LILAGLTIVTHVLKEGGKFIAKIFRGKDTSLLYCQLKLFFPVVTFAKPKSSRNSSIEAFAVCENYSPPEGFD
PKDLRRLLEKVGSPSGADDLDCSSGWLEGASKVYIPFLACGDLSGYDSDRSYPLPKDADGTYQSLDPVQPPI
APPYKRALEMKKASSHGVKELEKLSLDS

**SEQ ID NO 73: Saccharum officinarum FtsJ nucleic acid sequence contig of CA279558.1, CF576902.1**
ATGGGCAAGGCCTCCAAGGACAAGAGGGACATCTATTACCGGAAGGCCAAAGAGGAAGGGTGGAGAGCTCGC
AGCGCCTTCAAGCTCCTCCAGATAGACCAGGAGTTCAACATATTCCATGGAGTGAAGCGTGTGGTTGACCTG
TGTGCTGCTCCCGGAAGCTGGAGCCAGGTTTTGAGCCGGAACCTGTATGTACCAGCAAAACAATCTCCTGAT
TGCAAGGAAGGCGACCTTCCTCTCATCGTCGCAATTGACTTGCAACCAATGGCCCCGATAGAAGGTGTTATA
CAAGTGCAGGGCGACATCACCAATGCTCGGACAGCGGAAGTGGTTATTAGGCATTTTGATGGATGCAAAGCA
GACTTGGTTGTTTGCGATGGAGCTCCGGATGTTACTGGCCTTCATGATATGGACGAATTTGTTCAGTCCCAG
CTTATACTCGCGGCATTGACTATCGTGACTCATGTACTCAAAGTTGGTGGAAAATTTGTCGCGAAGATATTT
CGGGGTAAAGATACAAGTCTACTGTACTGCCAGCTAAAACTGTTCTTCTCACAAGTTACATTTGCGAAACCA
AAAAGTAGCCGGAACTCAAGCATTGAGGCGTTTGCAGTTTGTGAGAACTATTCACCTCCAGAAGGTTTCAAG
GAGGAAGATCTATACCACCTGCTAGAGAAAGTGGGGACTCCTTCAGGAGGTGACGATTTAGATTGCAGAAGC
GGATGGCTCGAGGGACCAAACAAGGTGTACATCCCGTTCCTTGCCTGCGGGGATCTCAGCGGCTACGACTCT
GATCGCTCATACCGGCTCCCGAGCACGGAAGGTGGGTCCTACCGGAGCTTGGACCCCGTCCAGCCTCCCATC
GCCCCGCCTTACAAAACCGCTCTGCAGATGAAGAAGGTTTCCAGCCACAGCGCCAGCGCGGACGCCATGAAA
CCATCCACAGATTCTTGA

**SEQ ID NO 74: Saccharum officinarum FtsJ translated polypeptide**
MGKASKDKRDIYYRKAKEEGWRARSAFKLLQIDQEFNIFHGVKRVVDLCAAPGSWSQVLSRNLYVPAKQSPD
CKEGDLPLIVAIDLQPMAPIEGVIQVQGDITNARTAEVVIRHFDGCKADLVVCDGAPDVTGLHDMDEFVQSQ
LILAALTIVTHVLKVGGKFVAKIFRGKDTSLLYCQLKLFFSQVTFAKPKSSRNSSIEAFAVCENYSPPEGFK
EEDLYHLLEKVGTPSGGDDLDCRSGWLEGPNKVYIPFLACGDLSGYDSDRSYRLPSTEGGSYRSLDPVQPPI
APPYKTALQMKKVSSHSASADAMKPSTDS

**SEQ ID NO 75: Triticum aestivum FtsJ nucleic acid sequence DR740715**
ATGGGCAAGGCTTCGAAAGACAAGCGGGACATCTACTACCGGAAGGCCAAGGAGGAGGGGTGGAGGGCTCGC
AGCGCCTTCAAGCTCATGCAGATCGACCAGGAGTTCAACATCTTCCACGGAGTGAAGCGTGCCGTTGACCTG
TGCGCCGCTCCTGGGAGCTGGAGCCAGGTTTTGAGCCGCAATTTGTATTTACCGGCGAAGCTATCATCTGAC
GGCAAAGATGGTGGCCTTCCTCTCATCGTCGCAATCGATCTGCAACCTATGGCTCCGATAGAAGGTGTCATA
CAAGTGCAGGGCGACATCACCAATGCTCGAACAGCGGAAGTGGTTATCAGGCACTTTGATGGATGCAAAGCG
GACTTGGTTGTCTGTGATGGTGCCCCTGATGTTACTGGACTTCATGATATGGACGAGTTTGTTCAGTCCCAG
CTTATATTGGCGGCACTGACAATTGTGACTCATGTACTTAAAGTTGGTGGAAAATTTGTTGCGAAGATTTTC
CGGGGTAAAGATACAAGTCTCCTGTATTGCCAGTTAAAGCTGTTCTTCTCACAAGTTACATTTGCAAAGCCA
AAAAGCAGCCGCAACTCAAGTATCGAGGCATTTGCAGTTTGCGAGAACTACTCACCTCCANGAGGGCTTCAA
GAGAAAGATTTATATCACCTGTTGNAGAAAGTGGGAACTCCTTCTGGGGCTGATGATTTAGATTGCAGAAGC
GGATGGTTGGAGGGGACCAAACAGGTCTACATCCCGTTTCTGGCTTGTGGCGACNTCAGGGGTTACGATCGG
ACCGTTCATACCCCCCTCCGAGCACAGAGGCGGCAANTACCAGANCTAGATCCAGTCAGCCCTCCATTGCCC
GGCCGTAAAAACTGCGTTGAAATTAGAAAGGGTCTAACCAGGGTCTGGCGCAATACAGCAATTATCTTCGAC
CTTAAATAA

**FIGURE 7 (continued)**

**SEQ ID NO 76: Triticum aestivum FtsJ translated polypeptide**
MGKASKDKRDIYYRKAKEEGWRARSAFKLMQIDQEFNIFHGVKRAVDLCAAPGSWSQVLSRNLYLPAKLSSD
GKDGGLPLIVAIDLQPMAPIEGVIQVQGDITNARTAEVVIRHFDGCKADLVVCDGAPDVTGLHDMDEFVQSQ
LILAALTIVTHVLKVGGKFVAKIFRGKDTSLLYCQLKLFFSQVTFAKPKSSRNSSIEAFAVCENYSPPXGLQ
EKDLYHLLXKVGTPSGADDLDCRSGWLEGTKQVYIPFLACGDXRGYDRTVHTPLRAQRRQXPXLDPVSPPLP
GRKNCVEIRKGLTRVWRNTAIIFDLK

**SEQ ID NO 77: Apis mellifera FtsJ nucleic acid sequence XM_392223**
TAAAGCTAACCAAAACGTATAACAACAATAAACAACAAGCATTGTACATTAGTCAATAATGGGAAAAACATC
AAAAGATAAACGAGATATATATTATCGACGAGCAAAAGAAGAAGGATGGAGAGCAAGGAGTGCTTTTAAATT
GCTTCAAATAGATAACGAATGTCATATTTTAGATGGAGTAAACAAAGCTGTGGATTTGTGCGCTGCACCCGG
GAGCTGGAGTCAAGTTTTATCACGAAGATTAAATGAGAATTATAAAAAAGCTTTGGAAACAGGAAATGCAAT
ACCTCCAAAAATAGTTGCAGTTGATTTACAAGCTATGGCACCATTGGAAGGAGTGATTCAAATTCAAGGAGA
CATTACGAATATTGATACTGCAAAACAAATTATCTCTCATTTTGATAATGAACAAGCTGATTTAGTAGTTTG
CGATGGAGCACCTGATGTGACAGGTTTACATGATATGGATATTTATATCCAATCTCAGTTATTATTAGCAGC
TCTGAATATTACTACTCATATATTAAAGCAAGGAGGCACATTTGTTGCAAAAATATTTAGAGCTAAAGATGT
GACTTTGCTCTATGCTCAACTAAAAATATTTTTCCCCTATGTCTATTGTACGAAACCTAGCAGTTCTCGTAA
CTCTAGTATTGAAGCATTTGTAGTTTGTAAAGATTACTCACCACCAGAAGGTTATAAACCTCATATGATGAA
TCCTCTTTTAACACATAAACCATGTGATTTTAATGACCTTACAGGAATTAATAGAGTTATTGTTCCATTTGT
TGTTTGTGGTGATCTTAGTCAGCCTGATTCCGATACGTGTTATCCATTGGATTTTGAAGGAAAAACATATAC
TTATCACGAACCGGTCCAAACACCGATTTCACCACCTTACAAAGAAGCACTCAGTTTGATGGAAGATAGAGA
TACCGATTTCAGAAGATTCGATGTTAATGTAGTTGTAGATAATTTAAGTTCATTGACTATTGAGGACTTTAA
AAAACAAGCGAAGCAAAAACATAAAGAGATAAATGAAACTAAAAGGATCGAATTGCAAGATAATAAAAAAGA
TGATAGCGAGGAAGATATATTGGGACTTGACAAGTTAATGCCCACCGAATTATTTGATGAATCTGAAAATAA
AAATAATGATAATATGTAAAAATGTTATGATTTTATATTATACATATAATTTGAGTTATAAATGTTTTTAAT
ATATTAATAATAACTTATAAAT

**SEQ ID NO 78: Apis mellifera FtsJ translated polypeptide**
MGKTSKDKRDIYYRRAKEEGWRARSAFKLLQIDNECHILDGVNKAVDLCAAPGSWSQVLSRRLNENYKKALE
TGNAIPPKIVAVDLQAMAPLEGVIQIQGDITNIDTAKQIISHFDNEQADLVVCDGAPDVTGLHDMDIYIQSQ
LLLAALNITTHILKQGGTFVAKIFRAKDVTLLYAQLKIFFPYVYCTKPSSSRNSSIEAFVVCKDYSPPEGYK
PHMMNPLLTHKPCDFNDLTGINRVIVPFVVCGDLSQPDSDTCYPLDFEGKTYTYHEPVQTPISPPYKEALSL
MEDRDTDFRRFDVNVVVDNLSSLTIEDFKKQAKQKHKEINETKRIELQDNKKDDSEEDILGLDKLMPTELFD
ESENKNNDNM

**SEQ ID NO 79: Caenorhabditis elegans FtsJ nucleic acid sequence NM_065442**
TGGGAAAAACATCTCGTGACAAAAGGGACATTTACTATCGTCTGGCAAAGGAGAACAAATGGCGAGCACGGA
GTGCATTCAAATTGATGCAGATTGATGATGAATTTCAGATTCTGAAAGGAGTCCGTCGAGCTGTAGATTTGT
GTGCAGCACCTGGAAGTTGGTCACAAGTGCTATCGAAAAGGTTATACGAGGAAGATCAAGAGGCAAAAATTG
TTGCGATTGATCTTCAGCCGATGGCACCGATTCCAGGAGTTATTCAACTTCAAGGAGATATTACTTCCGTTG
ATACAGCTAATCAGGTCATCAAACACTTTTCCGGAGAAAAATCAGATATTGTGATTTGCGACGGAGCCCCAG
ATGTAACCGGAATTCATTCTCTGGACGAATTCATGCAAGCCGAACTAATCCTCGCTGCCTTCAACATCACAA
GTCACGTTCTCAAAGAAGGCGGCAACTTTCTCGCGAAAATTTTCCGATCCCGAAACTCCTCTCTTCTCTATG
CACAAATGAAGAAGTATTTTAAAAAAGTATATCTAGCCAAACCACGAAGTTCCCGTCAATCCAGTTGTGAAG
CTTTTGTCCTGTGTCTC

## FIGURE 7 (continued)

GACTATTCACCGCCCGAGGGATTTGTTCCAACAATGGGAAAGACTTCATTAGACGCAACTGATGCTTCTGCT
ATTTCACCTGATATTATCGATGGATTCGTGACTTGTGGCGATCTAAGTGGATGGGACAGCGAGAAGTCTTAT
CCGTTGGATATTGATGCTTGTTTTCCCAAAGGAGAGATTGATGAAGAGCAGAAAAAACGATACGAGTTCAAA
GACGTCGTTCAGCCACCAACAGATCCTGCCTACAAAGCAGCTCTCGATAAGAAGAAAAGTGGAGTTTTTGCA
AAAATGTCTGCAGATTTGAACAGACAGCTGAAAGCTGAACTTAGTCGTGGAAAGGATCAGAAGAAGACTCCA
GCAGAGAATGTTCCAAGCGTAGAAGAATTGGAGAAAGCTGCTGAAAAATTTCAACTATAATTTGGTTATTTT
TGTTCTCATTGTCAGTTAATTGATTTTTTTTCACTTTTTTCGAGTCTCATTATTAGTATTTGTTTTG

**SEQ ID NO 80: Caenorhabditis elegans FtsJ translated polypeptide**
MGKTSRDKRDIYYRLAKENKWRARSAFKLMQIDDEFQILKGVRRAVDLCAAPGSWSQVLSKRLYEEDQEAKI
VAIDLQPMAPIPGVIQLQGDITSVDTANQVIKHFSGEKSDIVICDGAPDVTGIHSLDEFMQAELILAAFNIT
SHVLKEGGNFLAKIFRSRNSSLLYAQMKKYFKKVYLAKPRSSRQSSCEAFVLCLDYSPPEGFVPTMGKTSLD
ATDASAISPDIIDGFVTCGDLSGWDSEKSYPLDIDACFPKGEIDEEQKKRYEFKDVVQPPTDPAYKAALDKK
KSGVFAKMSADLNRQLKAELSRGKDQKKTPAENVPSVEELEKAAEKFQL

**SEQ ID NO 81: Danio rerio FtsJ nucleic acid sequence BC085449**
GAAGAAGAAGAACTGTCACGTTGCGGCGAAAACAAGCAGCAGTCCAACAATAAGTGAGTGAAGGTGAAATGG
GGCGCTCATCCAAAGACAAACGAGATATTTACTACAGACTTGCGAAGGAGGAAGGATGGAGGGCGAGAAGTG
CCTTCAAACTCCTGCAGCTGGATGAGGAGTTCAAGCTGTTTAGAGGTGTTAGTCGTGCTGTGGATCTGTGTG
CAGCACCTGGCAGCTGGAGTCAAGTCCTCAGCCGCAAACTACGTGGAAAAGACAAGAGTGAAGAGGTCAAGA
TTGTGGCAGTTGATCTGCAAGCCATGGCACCTTTACCGGGCGTCACACAAATTCAAGGAGACATCACCAAGA
TTTCTACAGCAGAGGAAATTATTCGACATTTTGAAGGAGAGTCTGCAGACTTGGTTGTGTGTGACGGTGCTC
CTGATGTGACAGGGCTTCATGATGTAGATGAGTATATTCAGGCACAGCTCCTGTTGGCAGCTCTCAATATCA
CCACACATGTTCTCAAACCAGGCGGCAACTTTGTTGCAAAAATTTTCAGGGGAAAAGATGTGACCCTGCTGT
ACTCGCAGCTAAAGATCTTCTTCAGCTTTGTGACCTGTGCCAAACCACCCAGCAGCCGCAACTCTAGCATTG
AGGCATTTGTGGTGTGTCAGAATTACTCTCCACCTGAGGGTTACGTTCCCAACATGTCCAACCCTTTACTGG
ATCACTCCTATGATGTCGACTTTAACCAGTTAGAGGGACCAAACCGAATAATAGTCCCTTTCCTTGCTTGTG
GAGATCTCAGTGGGTTTGATTCAGACAGAACGTATCCTCTTCAGCTTGATTCTAGCAAAGAGTATCAGTATT
TACCACCAACCCAACCTCCAATTCGGCCTCCCTACCAGCAAGCCTGTCAATTGCGCAAGAGTAACCTGCTTG
CCAAAGAGGACTCGCCGTCCGGTGCGCTGGATGAAGCCTTGACCGCTTTAGATCTCAACACAAAACCAGACA
CAAGCACGACTACACCAGGAGCCTCAGAATAATGGAATTGTACAGCTTCAAGTTTTGCTTGACACTGTCAAG
AATGTGTTTGTCACGGGAACATCTTTGTACTTTATTTACACCTACATTTTGATAGTATTAACAAACCCACAG
AAATTTTTACTCCTAGAAGATATTTATTTAGAGGTAAATAAGGTACAAAGGTTTCTATGATTCTGTCTCTGT
GTAAAGCTTTTATTTAATTTTTCATGCATTTCTGGATTGTAATTAATGTAACTTTTAGGCATTTTATTCTTT
CTGACTGTATTACATTTACCATTCACAGGATTGTTTCAGTAAGTCGCCGAAACAACCTGATGACTTTTAACC
TCAGCTTTACATGGTAATAAAACACTCAAATCTAAAAAAAAAAAAAAAAAAAAAAAAAAAAAAAA

**SEQ ID NO 82: Danio rerio FtsJ translated polypeptide**
MGRSSKDKRDIYYRLAKEEGWRARSAFKLLQLDEEFKLFRGVSRAVDLCAAPGSWSQVLSRKLRGKDKSEEV
KIVAVDLQAMAPLPGVTQIQGDITKISTAEEIIRHFEGESADLVVCDGAPDVTGLHDVDEYIQAQLLLAALN
ITTHVLKPGGNFVAKIFRGKDVTLLYSQLKIFFSFVTCAKPPSSRNSSIEAFVVCQNYSPPEGYVPNMSNPL
LDHSYDVDFNQLEGPNRIIVPFLACGDLSGFDSDRTYPLQLDSSKEYQYLPPTQPPIRPPYQQACQLRKSNL
LAKEDSPSGALDEALTALDLNTKPDTSTTTPGASE

## FIGURE 7 (continued)

**SEQ ID NO 83: Homo sapiens FtsJ nucleic acid sequence AK024023**
GGATACGTCGACAATAGATGACGGGGCTCACGTTGTACGTTCACATCAGGTCCCGGCCCGCCGGAACCTGGG
CGATCCACGATGCCGAGTTTGCCACGCTGCGACAGCCCATAGGCTTGCCCCCCCGGGCATTCGGGTGGACTA
CGAACACAAACTGAAGCCCTAGGACTTGTCGCCCGTTTGCGCTCTCGCCGAGGCACAGGCTGCTCGCGGACC
ACCCTGCTCCGAAAACTAAGAAGGGGACCAGTAGACGGGGTGGATTCGAGGTGGGGACATGGGACGCAGCGG
TCGGAGCCGCCTGGAGGGGTACCCGGTGGTCCCGATGGAGCTATCATCTTTGAGAGGTAGGTGGTAGCCCAT
TCATCTGGTTACTGATACTGGCCGGCATCAGTGGACTGTCGGCAGGTCCTTGAGCAACTGGTGTGTGAAATG
GGACGGACGTCAAAGGACAAGCGGGATGTCTACTACCGCCTGGCCAAGGAGAATGGCTGGCGTGCTCGCAGC
GCCTTCAAACTGCTACAACTGGATAAGGAATTCCAACTCTTCCAAGGCGTGACACGGGCAGTTGACCTGTGT
GCAGCCCCAGGCAGCTGGAGCCAGGTGCTGAGCCGGAAGATCGGGGGCCAAGGGTCCGGCCACGTGGTGGCT
GTGGACCTGCAGGCTATGGCTCCACTACCAGGTGTGGTACAGATCCAGGGGGACATCACCCAGCTGTCCACT
GCCAAGGAGATCATCCAGCACTTTAAGGGCTGCCCTGCGGACCTAGTGGTGTGTGACGGGGCTCCTGATGTA
ACCGGTCTCCATGATGTTGATGAGTATATGCAGGCCCAGCTCCTCCTAGCTGCTCTGAACATTGCTACACAT
GTCCTGAAGCCAGGGGGCTGCTTTGTGGCCAAGATATTCCGAGGCCGGGATGTGACGCTCCTCTACAGCCAG
CTGCAGGTCTTCTTCTCCAGCGTGCTGTGTGCCAAGCCCAGGAGCAGCCGGAACTCTAGCATCGAGGCCTTC
GCTGTCTGTCAGGGCTATGACCCTCCCGAGGGCTTCATCCCGGACCTGAGCAAACCCCTGCTGGACCATTCT
TACGATTTCAACCAGCTGGATGGTCCCACCCGCATCATTGTGCCTTTTGTGACCTGTGGGGACCTGAGCTCC
TATGATTCGGACCGCAGTTACCCACTGGACCTAGAGGGCGGCTCAGAGTACAAGTACACTCCACCCACACAG
CCCCCCATCTCGCCACCATACCAGGAGGCCTGCACGTTGAAGAGGAAGGGGCAGCTGGCCAAGGAGATCCGC
CCCCAGGACTGCCCCATCAGCAGAGTGGACACGTTTCCCCAGCCCCTGGCCGCCCCTCAGTGCCACACCCTG
CTGGCCCCTGAGATGGAAGACAATGAAATGAGTTGTTCACCTTAACCCATTACGCTGGCAAACTGATACAAC
TCAGAAACTGCTCAATGTCAGGAAAACCGGAACTTGTTGATGAACTTGTTTTCAAAATATCATCTCATCAAC
GAGGCCTGGACAAACAAGCCCTGAGGAGGGATCTGCAACAACCCTGAAGACAACAAGGAAAGAAACCATGAA
AGTCTGTCTGTACTGCAGTGGGAATTCTTGAGTGAGGTCTTACCTCTTCTTTAAACCTCTTCAGGAGTGTCC
TGATTATGTCCAGAATTTTCCCTAAAGGCAGGGATTCTTAACCTGGATAGAAGCCAGGGGTAACCATGAACT
TGATGGAAGAAAATGTTACATCTTTATTTTCAGCAATGAAACTGAAATTTAGCCTTACTCCCAAGTTATAAA
TGCTGGCAACAAATCACAGTAGTAAAAGCAGTACCTGGGATTTTCCCACCAATACAAACCAGTTACAGCAAC
TGTAATGTAACATAAAAAGGGCATCTTGAAGTATTGTTCATATTTGTCACCTCTTGGAATGATAGCAGATCC
TGTAAGTTGATGTATTAATTAAAAGCCCATATATTACTGC

**SEQ ID NO 84: Homo sapiens FtsJ translated polypeptide**
MGRTSKDKRDVYYRLAKENGWRARSAFKLLQLDKEFQLFQGVTRAVDLCAAPGSWSQVLSRKIGGQGSGHVV
AVDLQAMAPLPGVVQIQGDITQLSTAKEIIQHFKGCPADLVVCDGAPDVTGLHDVDEYMQAQLLLAALNIAT
HVLKPGGCFVAKIFRGRDVTLLYSQLQVFFSSVLCAKPRSSRNSSIEAFAVCQGYDPPEGFIPDLSKPLLDH
SYDFNQLDGPTRIIVPFVTCGDLSSYDSDRSYPLDLEGGSEYKYTPPTQPPISPPYQEACTLKRKGQLAKEI
RPQDCPISRVDTFPQPLAAPQCHTLLAPEMEDNEMSCSP

**SEQ ID NO 85: Arabidopsis thaliana FtsJ2 nucleic acid sequence At4g25730 NM_118705**
ATGGGTAAGGTCAAAGGAAAGCATCGTTTGGATAAGTACTATCGTCTCGCTAAGGAACGTGGATTCCGTTCT
CGAGCTTCCTACAAGCTTCTTCAGCTCGACGCCAAGTACTCTCTTCTCCACTCAGCTCATGCCGTTCTCGAT
CTCTGCGCCGCCCCTGGTGGTTGGATGCAAGTCGCCGTCGAGAAAGTTCCCGTCGGCAGCCTCGTTCTCGGT
ATCGATCTTGTCCCGATTCTCCCTGTTCGTGGCTGTGTTACTATGACGCAAGATATCACAAGGACTGAGTGT
AAATCGAAGATCAAGCAAGTGATGGAGCAGCATGGTGTTAGTGCTTTTAATTTGGTTCTGCACGATGGGTCT
CCAAATGTTGGTGGTGCGTGGGCACAAGAGGCTATG

## FIGURE 7 (continued)

AGCCAGAACGCTTTGGTTATTGATTCTGTTAGATTAGCCACTGAGTTCTTAGCTCGCAACGGGAATTTAGTC
ACCAAGGTTTTCAGGTCTCGAGACTACAACTCTGTGCTCTACTGTCTTGGGAGGCTGTTTGAAAAAGTTGAA
GTGTTTAAGCCGCCGGCTAGTCGTTCAGCATCTGCAGAAACATATCTTGTGGGTTTGAAATACTTAGCCCCT
GCCAAGATTGATCCCCGTCTTCTTGATTATAGACATCTCTTTAAGGAGTCAGCAGAACCCACGAGAAAGGTG
GTGGATGTGCTTGGAGGATCAAAACAAAAGAGAAACCGTGATGGGTATGAAGATGGGGAGTCCATTTTGAGA
AGAGTTGCATCTGCTGCTGATTTCATTTGGTCTGAAAATCCTCTTGACGTTCTTGGCACGACTACTTCCATA
TCATTTGATGATCAAGCCTCTCTACCTTTAAAGGAACATGATTTGACGACAGAGGAGATTAAAATTCTATGC
GATGATCTTCCTGTCTTGGGGAAGAATGACTTCAAGCATATCCTAAAATGGCGAATGCAAATCAGGAAAGCT
CTGACTCCTGAGAAAAAGGAAGTTGCTAAACCTGAGCCTGATGTGGGAAAGGAAGATGAGGAAAATGAAGAT
GATAAACTACTCAATGAATTGGAAGAGCTGACAAACACAGTTGACCGCAAGAAGAAACAGGCAAAGAAGATT
CTTGCAAAACGAAGGGCTAAGGACAAGGCGCGGAAGGCGACTGGTCCACAGATGGATGTACTTGAAGATGGT
TTCGTCGACAATGAATTGTTCTCTCTTAATGCTATCAAGGGAAAGAAAGATCTAATGGCAGTTGACAATGAT
GAAGATGATAATGGCAATGCCGTTGACAGTGAGAACGAAGACCATGGCGAAGGAGCTTCAGATGACTCCAAA
GACAGTGACAGAGATTCTGATGAAGAACGTCAGAAATACACTGAACAAATGGAAGAGATTTTTGAGCAGGCA
TATGAGCGGTATATGGTGAAGAAAGAAGGAAGCGCAAAGCAAAGGAAGCGGGCTAGGCAGGCCCATGCTGAA
AAGCTGGAGGAAGGTGATGGAGATGAAGAAATGAAGATCGATTACGATTCAGATATGAATGAAGAAAAGGAT
GAGGCAAACCCTCTTGTTGTACCACTTGATGATGGAGTGGTCCAAACAAAGGAGGAAATATCCAACCAGTGG
TTCAGTCAGAATATATTTGCAGAAGCCGTGGAAGAAGGAGACTTGGGAAAAGATGACAGTGAAGACGAAATA
GCAAATAAGAAAAAGAGCAAAAATCTGTCTAAACCAGACAAGTCAAAGCAGAAGGCTTCAAAAGCAAGTGTG
TTGTCTGATCAGAGCTTGCCCAATAGCTCCAAGAAGGAAGATGAGTTTGAGGTAGTCCCTGCACCAGCCACA
GATTCTGACTCAGATTCATCCTCTGAAGATGATGTTCATACCAAGGCCGAGATACTGGCATGTGCCAAAAAA
ATGCTGAGGAAGAAGCAGAGAGAACAGATGCTTGATGATGCATATAACAAACACATGTTTGTAGATGAAGGT
TTACCAAAATGGTTTGTGGATGACGAGAAGCAGCACAGACAACCAATGAAACCTGTTACGAAAGATGAAGTT
AATGCAATGAAAGCACAGTTCAAAGAGATCAATGCTCGTCCAGCCAAGAAGGTGGCAGAGGCCAAGGCACGG
AAGAAGCGAGCTGCCCAGAAAAGATTGGAGAAAGTGAGGAAGAAGGCAAACACAATATCTGACACAGCAGAT
ATATCAGACCGTTCAAAGGACAAGATGATAGACAAGCTATACAAGAAAGCAGCAGAGCCAAGAAAGCCAAGG
AAAGAACTGGTTGTGTCTAAGAAAGGAGTTGGGGTTAAAGTTGGTAAGGGACAGAAGAGAGTGGATAGGAGG
ATGAAGAGTGATGATAGGAAACGTGGGGGAGGAAAGCCCGGTAGGAACGGGCAGAAGGGCACTGGTAAAGCA
GGCCAGAAAGGAAAAAGGCCTGCTGGGAAACCTCGGGGAAGGAAACCCGGGTAA

**SEQ ID NO 86: Arabidopsis thaliana FtsJ2 translated polypeptide**

MGKVKGKHRLDKYYRLAKERGFRSRASYKLLQLDAKYSLLHSAHAVLDLCAAPGGWMQVAVEKVPVGSLVLG
IDLVPILPVRGCVTMTQDITRTECKSKIKQVMEQHGVSAFNLVLHDGSPNVGGAWAQEAMSQNALVIDSVRL
ATEFLARNGNLVTKVFRSRDYNSVLYCLGRLFEKVEVFKPPASRSASAETYLVGLKYLAPAKIDPRLLDYRH
LFKESAEPTRKVVDVLGGSKQKRNRDGYEDGESILRRVASAADFIWSENPLDVLGTTTSISFDDQASLPLKE
HDLTTEEIKILCDDLPVLGKNDFKHILKWRMQIRKALTPEKKEVAKPEPDVGKEDEENEDDKLLNELEELTN
TVDRKKKQAKKILAKRRAKDKARKATGPQMDVLEDGFVDNELFSLNAIKGKKDLMAVDNDEDDNGNAVDSEN
EDHGEGASDDSKDSDRDSDEERQKYTEQMEEIFEQAYERYMVKKEGSAKQRKRARQAHAEKLEEGDGDEEMK
IDYDSDMNEEKDEANPLVVPLDDGVVQTKEEISNQWFSQNIFAEAVEEGDLGKDDSEDEIANKKKSKNLSKP
DKSKQKASKASVLSDQSLPNSSKKEDEFEVVPAPATDSDSDSSSEDDVHTKAEILACAKKMLRKKQREQMLD
DAYNKHMFVDEGLPKWFVDDEKQHRQPMKPVTKDEVNAMKAQFKEINARPAKKVAEAKARKKRAAQKRLEKV
RKKANTISDTADISDRSKDKMIDKLYKKAAEPRKPRKELVVSKKGVGVKVGKGQKRVDRRMKSDDRKRGGGK
PGRNGQKGTGKAGQKGKRPAGKPRGRKPG

## FIGURE 3 (continued)

**SEQ ID NO 87: Arabidopsis thaliana FtsJ3 nucleic acid sequence At5g13830 NM_121386**
ATGAGTGGGGCAGGTGTACCGGACTTTTTCTACAGAGAAGCTCAACGTCTTGGCTATGTTGCTCGCTCAGCT
TTCAAGCTTCTACAGATTCAAAAACAGTACAAGCTCATCAAACCAGGCTCCTCTGTTCTTGACCTAGGTTGC
GCGCCTGGTGCTTGGCTTCAGGTTGCTTGCCAAAGCTTAGGCCCACTTAGAAGTGGTGGAATTGTCGTTGGT
ATGGATATAAAGAAGGTGAAGGTTCCTCCACAGTGTGATTCTCGAGTGCAAACCATTGCTGCTGATGTTTTA
AACTTTCCCAGACAAAAGATTCGGGAGTTATCACCTCAGCAATTGGGATTTTCAGTCATTCTTTCAGATATG
TGTCACTCAGTATCTGGAATAACCACTAGAGATGCAGCTCTGTCTGCTGAATTGGGAATGCGAGCACTTGAT
TTGGCTGTTGGTCAAGCTGCCATCTCTCAGTCACCTAATGATGATGATGGAGGCCCAAACGAAAGTCGTCCT
GGTGTACTTAGGCATGGTGGCCATCTTGTGATCAAGCTTCTAGAAAGCGAGGATGCTCAAGATTTTGCTCGA
ATTTGCAAGCCTATCTTCAACAAGGCATCTTGGTTGAGGCCAAAAGCTACAAGACCATCATCTCGAGAAATT
TACTTGATTTGCCAGGGATTTCGATAA

**SEQ ID NO 88: Arabidopsis thaliana FtsJ3 translated polypeptide**
MSGAGVPDFFYREAQRLGYVARSAFKLLQIQKQYKLIKPGSSVLDLGCAPGAWLQVACQSLGPLRSGGIVVG
MDIKKVKVPPQCDSRVQTIAADVLNFPRQKIRELSPQQLGFSVILSDMCHSVSGITTRDAALSAELGMRALD
LAVGQAAISQSPNDDDGGPNESRPGVLRHGGHLVIKLLESEDAQDFARICKPIFNKASWLRPKATRPSSREI
YLICQGFR

**SEQ ID NO 89: Oryza sativa FtsJ3 nucleic acid sequence NM_185165**
ATGAGCGGCGCGGGCGGCACCGCCGACTTCTTCTACCGCGAGGCGCAGCGCCTCGGCTACGTCGCCCGCTCC
GCGTTCAAGCTGATCCAGATACAGAAGCAGCACAAGCTCATCGCCCCCGGCGCCGCCGTCCTCGACCTCGGC
TGCGCCCCCGGCGCGTGGCTCCAGGTGGCGTGCCAGAACCTGGGTCCGCTCGAGAAGGGCGGGGTGATCGTC
GGCGTCGATGTCAAGAAGGTGAAGGTTCCATCTGCGCACTGTGACTCGAGGGTCAGGACCGTTTGCGCCGAT
GTGATGGCTCTGATGAAGCAGCAAGCTCGGGCAATGTCGCCACAGGAACGAGGATTCTCTGTAATATTGTCA
GACATGTGCCCAGTAGTTTCTGGAATTACAACCAAAGATGCGGCTATATCTTGCGAGCTGGGCATGCGTGCT
CTTTCATTGGCAGTTGGTAAGATGAAAGCAAAAGATTCAGATTGCATTGCAATTTTAGAGAAGTTTCAGAGC
TCCACTGAGCCAGATCCTGATGAAGATGGCATTCTCCGTCGTGGAGGCAGCCTTGTAATTAAGTTTCTTGAG
AATGAGGATATACCAGGTTTTGGCAAATTTTGCAAAGAGAAGTTCAAGAAAGTGTCCTTATTGAGACCCAAG
GCGACAAGATCTAGTTCGAGGGAGATTTTCATGGTCTGTGAAGGCCGCGGCTTCTCGGTCGCGCCACGCTGG
CATCCACTTTCTCATGGCCCGGCGACGTACGGGACGGGCTCCACGTGTCACGCCGCCGCCCACAGCTTGGTC
GAATTGAGAACTGTTGGGCTTCAAAGAGAGGCTCCCAAGTGCAGTGACCTGAAAGCTCAAGCTCAACCTGCG
GCGTTCGTGCGGATTCCGCGGGGCGCGTCCCTTCCCGCCACCCGCAACGCCGCTGGACTCCACTCCACTCTC
CTCGTCGCTTCGTTGCTGCACATCACCACCCACCGCGGTGGCACTCACTTCTATATTAGCTTAAGCGTGGGG
TGGGAGACGCCGAGGAGGGAGGAGTGCCGCATCCCGGTGGTGCCGCCGCAGTGCCCGGCGCCGCCGAGGAAG
AGGCCGGTGGCGCTGCCGGAGCTGGGGAAGGAGCGGCGGGAGCCGCCCAAGGGCGGGTACTTCCAGCCGCCG
GACCTCGAGTCGCTCTTCGTGCTCGCGCCGCCGCGGAGGCAGGCGTCCAGCTGCGCGTAG

**SEQ ID NO 90: Oryza sativa FtsJ3 translated polypeptide**
MSGAGGTADFFYREAQRLGYVARSAFKLIQIQKQHKLIAPGAAVLDLGCAPGAWLQVACQNLGPLEKGGVIV
GVDVKKVKVPSAHCDSRVRTVCADVMALMKQQARAMSPQERGFSVILSDMCPVVSGITTKDAAISCELGMRA
LSLAVGKMKAKDSDCIAILEKFQSSTEPDPDEDGILRRGGSLVIKFLENEDIPGFGKFCKEKFKKVSLLRPK
ATRSSSREIFMVCEGRGFSVAPRWHPLSHGPATYGTGSTCHAAAHSLVELRTVGLQREAPKCSDLKAQAQPA
AFVRIPRGASLPATRNAAGLHSTLLVASLLHITTHRGGTHFYISLSVGWETPRREECRIPVVPPQCPAPPRK
RPVALPELGKERREPPKGGYFQPPDLESLFVLAPPRRQASSCA

## FIGURE 7 (continued)

**SEQ ID NO 91: Oryza sativa oleosin promoter**
GGTCAGCCAATACATTGATCCGTTGCCAATCATGCAAAGTATTTTGGCTGTGGCCGAGTGCCGGAATTGATA
ATTGTGTTCTGACTAAATTAAATGACCAGAAGTCGCTATCTTCCAATGTATCCGAAACCTGGATTAAACAAT
CCTGTTCTGTTCTCTAGCCCCTCCTGCATGGCCGGATTGTTTTTTTGACATGTTTTCTTGACTGAGGCCTGT
TTGTTCTAAACTTTTTCTTCAAACTTTTAACTTTTTCATCACATCAGAACTTTTCTACACACATAAACTTTT
AACTTTTCTGTCACATCGTTCCAATTTCAATCAAACTTTTAATTTTGGCTTGAACTAAACACACCCTGAGTC
TTTTATTGCTCCTCCATACGGGTTGGCTGGTTGAGAATAGGTATTTTCAGAGAGAAAATCTGGATATTGGGA
GGAGGAACTTGGCATGAATGGCCACTATATTTAGAGCAATTCTACGGTCTTTGAGGAGGTACCATGAGGTAC
CAAAATTTTAGTGTAAATTTTAGTATCTTCTTAAGGACCGTAAAATTGCTCCTATATTTAAGGGATGTTTAT
ATCTATCCATATCCATAATTTGATTTTGATAAGAAAAAATGTGAGCACACCAAGCATGTCCATGACCTTGCA
CTCTTGGCTCACTCGTCAACTGTGAAGAACCTAAAAAATGCTCAATATAGCTACAGGTGCCTGAAAAAAATAA
CTTTAAAGTTTTGAACATCGATTTCACTAAACAACAATTATTATCTCCCTCTGAAATGTTGCTACCTAAGAT
GATAGTTTAGAACTCTAGAATCATTGTCGGCGGAGAAAGTAAATTATTTTCCCCAAATTTCCAGCTATGAAA
AAACCCTCACCAAACACCATCAAACAAGAGTTCACCAAACCGCCCATGCGGCCATGCTGTCACGCAACGCAC
CGCATTGCCTGATGGCCGCTCGATGCATGCATGCTTCCCCGTGCACATATCCGACAGACGCGCCGTGTCAGC
GAGCTCCTCGACCGACCTGTGTAGCCCATGCAAGCATCCACCCCCGCCACGTACACCCCCTCCTCCTCCCTA
CGTGTCACCGCTCTCTCCACCTATATATGCCCACCTGGCCCCTCTCCTCCCATCTCCACTTCACCCGATCGC
TTCTTCTTCTTCTTCGTTGCATTCATCTTGCTAGC

**SEQ ID NO 92: Hordeum vulgare Horvu_FtsJ**
Atgggcaaggcctccaaagacaagcgggacatatactatcggaaggccaaggaggaggggtggagggctcgc
agcgccttcaagctaatgcagatcgaccaggagttcaacatcttccacggagtgaagcgcgccgttgacctg
tgcgctgcccctgggagctggagccaggttttgagccgcaatttgtacctgccggcaaagctatcatctgac
ggcaaagatggcggccttcctctcatcgtcgcaatcgatctgcagccgatggctcctatagaaggtgtcata
caagtgcagggcgacatcaccaatgctcgaacagcggaagtggttatcaggcacttcgatggatgcaaagcg
gacttggttgtctgtgatggtgcccctgatgttactgggcttcatgatatggatgagtttgttcagtcccag
cttatattggcggcactgacaattgtgactcatgtactcaaagttggtggaaaatttgttgcgaagattttc
cggggtaaagatacaagtctcctgtattgccagttaaagttgttcttctcacaagttacatttgcaaagcca
aaaagcagccgtaactcaagtatcgaggcatttgcagtttgcgagaactactcacctccagagggcttcaaa
gagaaagacttgtatcacctgctggagaaagtgggaactccttctggggctgatgatttagattgccgaagc
ggatggttggagggaccaaacaaggtctacattccgtttctggcttgcggtgacctcagcggttatgattcg
gaccgttcataccccctcccgagcacagaaggtggcacctaccagagcctagatccagtccagcctcccatc
gcgccgccatacaaaactgcgttggagatgaagaaggcgtctagccacggcgccggcgcagatactagcaaa
tcatatgtcgacccc

**SEQ ID NO 93: Hordeum vulgare Horvu_FtsJ**
MGKASKDKRDIYYRKAKEEGWRARSAFKLMQIDQEFNIFHGVKRAVDLCAAPGSWSQVLSRNLYLPAKLSSD
GKDGGLPLIVAIDLQPMAPIEGVIQVQGDITNARTAEVVIRHFDGCKADLVVCDGAPDVTGLHDMDEFVQSQ
LILAALTIVTHVLKVGGKFVAKIFRGKDTSLLYCQLKLFFSQVTFAKPKSSRNSSIEAFAVCENYSPPEGFK
EKDLYHLLEKVGTPSGADDLDCRSGWLEGPNKVYIPFLACGDLSGYDSDRSYPLPSTEGGTYQSLDPVQPPI
APPYKTALEMKKASSHGAGADTSKSYVDP

## FIGURE 7 (continued)

**SEQ ID NO 94: Sorghum bicolor Sorbi_FtsJ**
gcacgagggcccagtgggctcaaaacacttggcggcgccacctcgccctcctccgactactcgctcgctcgt
cccgccccgtcttccgcccccgcctccgcccccgccgcccttggctcgcaccagcgccacctcgccctcctc
cgactactcgctcgctcgtcccgccccgtcttccgcctccgcctccgccgccgccgcccttggttcgcctca
tcgtcgtcgccatgggcaaggcctccaaggacaagagagacatctactaccggaaggccaaagaggagggat
ggagagctcgcagcgccttcaagctcctccaaatagaccaggagttcaacatcttccatggagtgaagcgcg
tggttgatctgtgtgctgctcccggaagctggagccaggttttgagccggaacttgtatgtaccagcaaaac
agtctcctgattgcaaggaaggtgaccttcctctcatcgttgcaattgacttgcaaccaatggccccgatag
aaggtgttatacaagtgcagggtgacatcaccaatgctcggacagcggaagtggttattaggcattttgatg
gatgcaaagcagacttggttgtttgcgatggagctccggatgttactggccttcatgatatggacgaatttg
ttcagtcccagcttatacttgcggcattgactatcgtgactcatgtactcaaagttggtggaaaatttgtcg
caaagatatttcggggtaaagatacaagtctcctgtactgccagctaaaactgttcttctcacaagttacat
ttgcgaagccaaaaagtagccggaactcaagcattgaggcatttgcagtttgtgagaactattcacctccag
aagggttcaaggaggaagatctataccacctgctagagaaagtagggactccttcaggagttgacgatttag
attgcagaagcggatggctcgagggaccaaacaaggtgtatatcccgttccttgcctgtggggatctcagtg
gctacgactccgaccgctcatacccgctcccaatcacagacggtggctcctaccggagcttggaccccgtcc
agcctcccatcgccccgccttacaaaaccgcccttcagatgaagaaggcctccagccacagcgccagcaggg
acgccatgaaaccatccacagactcttgagctgcaacctgggccagcctgaaccccccaggcgatgatgtttg
tcccacactggtagcatcgctgggtcctggacctggctctctcctgagcctcgtctattaaaaaggaaaaag
aaaatttgggaacattttgtgtcgaggtgggacattttgtggtagttttgagctgtggctccgcagcatgta
cctgatcactactgattacgcagagatcctgcttgtgtccttgtgc

**SEQ ID NO 95: Sorghum bicolor Sorbi_FtsJ**
MGKASKDKRDIYYRKAKEEGWRARSAFKLLQIDQEFNIFHGVKRVVDLCAAPGSWSQVLSRNLYVPAKQSPD
CKEGDLPLIVAIDLQPMAPIEGVIQVQGDITNARTAEVVIRHFDGCKADLVVCDGAPDVTGLHDMDEFVQSQ
LILAALTIVTHVLKVGGKFVAKIFRGKDTSLLYCQLKLFFSQVTFAKPKSSRNSSIEAFAVCENYSPPEGFK
EEDLYHLLEKVGTPSGVDDLDCRSGWLEGPNKVYIPFLACGDLSGYDSDRSYPLPITDGGSYRSLDPVQPPI
APPYKTALQMKKASSHSASRDAMKPSTDS

**SEQ ID NO 96: Brassica napus Brana_FtsJ**
Atgggaaaagcttctcgggataaaagggatatatactatcggaaagccaaagaagaagggtggcgtgcaaga
agtgcctttaagcttcttcagattgatgaggagttcaacatttttcaaggagtgaagagggttgtagattta
tgtgctgcacctggtagctggagtcaggttctgagtcgtcaactgtatcttcctgcaaagtattcagctgag
tcaaaagaagaagatcttcctcttattgtggccatagatttgcagcctatggctccaatcgaaggtgtaatc
caagttcaaggggacataactaatgctcggactgccgaagtggtcattagacattttgacggttgcaaggct
gacctggttgtgtgtgatggtgctccagatgttaccggtttgcatgacatggatgaatttgtccagtcccaa
ctcatactagcgggcttaacgattgtaacccatattcttaaagaaggtggaaaattcattgcaaagatattc
cgtggaaaagatacaagtctcttgtactgtcagctgaagttgtttttcccaactgtgacttttgcgaaacct
aaaagcagccgcaattctagtatagaagcttttgctgtctgcgaaaattactccccaccagaaggatttaac
ccgagagatctgcatcttctcttggagaaagtcggaagcccttcaggtggaagcgatctcgattgcagtagt
ggttggctcgagggacccaacaaagtttatattccattcttggcatgtggtgacttaagcggttatgactcg
gaccggtcttacccactcccaaaagaggcagatggatcgtcataccgaagcctggacccgattcagcctccg
atcgcaccgccttataaacgagctattgagctcaagaaagcttcagcacaaagcctcaactcttaaagctag
gctcgataacaataataatgctaaacaaagacacagaaagaagtttttcattcgattcttagaaggctgtag

FIGURE 7 (continued)

**SEQ ID NO 97: Brassica napus Brana_FtsJ**
MGKASRDKRDIYYRKAKEEGWRARSAFKLLQIDEEFNIFQGVKRVVDLCAAPGSWSQVLSRQLYLPAKYSAE
SKEEDLPLIVAIDLQPMAPIEGVIQVQGDITNARTAEVVIRHFDGCKADLVVCDGAPDVTGLHDMDEFVQSQ
LILAGLTIVTHILKEGGKFIAKIFRGKDTSLLYCQLKLFFPTVTFAKPKSSRNSSIEAFAVCENYSPPEGFN
PRDLHLLLEKVGSPSGGSDLDCSSGWLEGPNKVYIPFLACGDLSGYDSDRSYPLPKEADGSSYRSLDPIQPP
IAPPYKRAIELKKASAQSLNS

**SEQ ID NO 98: Glycine max Glyma_FtsJ**
gagaagcaaacaaaccctagcgcagagaagagatattcttcttcgcagaatcattgcagttgcgagcaggcc
gaagaaacctaacctgcattcgaaaatcagccccccttggaaatcccaacactagtgtggtacctgtgctcga
cgattgggtgttcaaggggaattagcttcgtctcgcagaacttcaacgcatcattcgtgaccttcgcaaacg
cggggtttgttcttatttggttttgccagttgttgccatcaggggtttgtttctgaaatttgtggcagggca
agtgagcgataaaccatggggagagcttcaagggataagagggatatctattaccggaaagcaaaagaagaa
ggttggcgagctcgaagtgcgtttaaactccttcagatagacgaggaattcaacctttttgatggagtgaag
cgtgttgtagatttatgtgctgccccaggtagctggagtcaggttttgagtcgtaaattgtatctcccagcc
aagcttgcacctgatgcaaaggatgaaaatcttcctcttattgtagctattgatttgcaaccaatggctcca
attgaaggtgtcatccaggtgcagggtgatataactaacgctcggacagctgaagtggtcattagacatttt
gatggttgcaaggctgacctagttgtgtgtgatggtgcgcctgatgttactggccttcatgacatggatgaa
tttgtacaatcccaactcttacttgcagggttgacaattgttactcatgtacttaaggaaggagggaagttt
attgcaaagatatttagaggaaaggacacaagccttctatattgtcagctaaaattatttttccctgtagtg
acgttcgcaaaaccaaaaagcagccgtaattccagcatagaggcatttgcagtttgtgaaaactattcccct
cctgaaggattcaatccaaaagatcttcatcgccttcttgagaaggttggaagtccctcaggggtagatgat
acagattgttgtagtggttggctggaaggccctaataaggtgtatatcccatttctagcttgtggtgacctc
agtgggtatgattctgatcgctcatatccactacctaaagttgccggggggaacatatcagagcttggatcct
gtgcagccccctattgccccaccttacaagagagctttggagttaaaaaaagcttccagtcaaggattccgt
gaacttgaaaacctctcattggattcttgatcgtgctatgcactgtttattcatcatgtcgtttggaatttt
agtttttgttacaatgatatgatttgtgttttgataattattagcgtacgtgtgaactttaacactttttc
tcttgtaccatcattgcatgcctagcgagtcttttgttatcgcaatttttgaaatgaatacaactatttta
taaaaa

**SEQ ID NO 99: Glycine max Glyma_FtsJ**
MGRASRDKRDIYYRKAKEEGWRARSAFKLLQIDEEFNLFDGVKRVVDLCAAPGSWSQVLSRKLYLPAKLAPD
AKDENLPLIVAIDLQPMAPIEGVIQVQGDITNARTAEVVIRHFDGCKADLVVCDGAPDVTGLHDMDEFVQSQ
LLLAGLTIVTHVLKEGGKFIAKIFRGKDTSLLYCQLKLFFPVVTFAKPKSSRNSSIEAFAVCENYSPPEGFN
PKDLHRLLEKVGSPSGVDDTDCCSGWLEGPNKVYIPFLACGDLSGYDSDRSYPLPKVAGGTYQSLDPVQPPI
APPYKRALELKKASSQGFRELENLSLDS

**SEQ ID NO 100: Zea mays Zeama_FtsJ**
Ccacgtcctcctccggctactcgctcgttccgccccggcgtcccgccctcagccgccgccgtcgcccccctcc
ctcggttcgcctcgtcgtcgtcatgggcaaggcctccaaggacaagagggacatctactaccggaaggccaa
agaggaaggctggagagctcgcagcgccttcaagctcctccagatagaccaggagttcaacatcttccatgg
agtgaagcatgtggttgacctgtgtgctgctcccggaagttggagccaggttttgagccggaacctgtatgt
accagcaaaacagtcttctgattgcaaggaaggtgatcttcctctcatcgtcgcaattgacttgcaaccaat
ggccccaatagaaggtgttatacaagtgcagggcgacatcaccaatgctcggacagcagacgtggttattag
gcattttgatggatgcaaagcagacttggttgtttgcgatggagccccggatgttactggccttcatgatat
ggatgaatttgttcagtcccagcttatactcgcggcattggctatcgtgactcatgtactcaaagttggtgg
aaaatttgtggcgaagatatttcggggtaaagatacaagtctcctgtactgccagctaaaactgttcttctc
gcaagttacgttcgccaagccaaaaagtagccggaactcaagcatcgaggcgtttgcggtctgtgagaacta
ctcacctcctgaagggttcaaggaggaagacctataccacctgctagagaaagtgggtactccctcaggagc

FIGURE 7 (continued)

tggcgatctagattgcagaagcggatggctggagggaccaaacaaggtgtacatcccgttcctggcctgcgg
ggatctcagcggctacgactccgaccgctcgtacccgctgcccagcagcacggacggtggctcgtaccggag
cctggaccccgtgcagcctcccatcgccccgccttacaagaccgccctgcagatgaagaaggcttccagcca
cggcgccggcgcggacgccatcaagccgtccgcagactcctgagccgcagaccgtttccgtttaccaccttg
cgcggtggaagttgctttcgctgggtcctggacctggctctctagtctctctacagaaacagaaccttca
tctacgaaactaaaaaaaaaggaaaaaggattggaaaaaaaaaaaaaaa

**SEQ ID NO 101: Zea mays Zeama_FtsJ**
MGKASKDKRDIYYRKAKEEGWRARSAFKLLQIDQEFNIFHGVKHVVDLCAAPGSWSQVLSRNLYVPAKQSSD
CKEGDLPLIVAIDLQPMAPIEGVIQVQGDITNARTADVVIRHFDGCKADLVVCDGAPDVTGLHDMDEFVQSQ
LILAALAIVTHVLKVGGKFVAKIFRGKDTSLLYCQLKLFFSQVTFAKPKSSRNSSIEAFAVCENYSPPEGFK
EEDLYHLLEKVGTPSGAGDLDCRSGWLEGPNKVYIPFLACGDLSGYDSDRSYPLPSSTDGGSYRSLDPVQPP
IAPPYKTALQMKKASSHGAGADAIKPSADS

**SEQ ID NO 102: Tagetes erecta Tager_FtsJ_partial**
aaagcaaaagaagaaggttggcgtgcacgaagtgcgtttaagctccttcaaattgatgaggagtttaacatc
tttgaaggagtgaagcgtgtggtcgatttatgcgcagcacctggtagctggagtcaggttttaagtcgtaag
ctgtatctcccagctaaacagtcatctgaccttccgcttatcgtggctattgatttacaacccatggctcca
attgaaggtgtgatccaagttcaaggagatattacaaatgctagaaccgctgaagtggttattagacatttt
gacggatgcaaggctgacctagttgtctgtgacggtgctcctgatgtaactggacttcatgacatggacgaa
tttgttcagtcacaactgatattggcggggttgacaattgtcactcacattctcaagaaaggtggaaagttt
attgcaaagatcttcaggggaaaggatacgagcctcttatattgtcagctaaaactatttttcacagaggtt
acgcttgcaaaaccaaaaagtagtcgaaattcaagcatagaggcatttgctgtttgtgagaattatacacct
ccagaaggattcaatgaaaaagatttgcatcgcctcctagagaaggtcggaactccatctggtgcagacaat
ttagattgtagtagtggatggttagaagggccaaacaaggtttatataccatttcttgcttgtggggacctt
agtggttatgac

**SEQ ID NO 103: Tagetes erecta Tager_FtsJ partial**
KAKEEGWRARSAFKLLQIDEEFNIFEGVKRVVDLCAAPGSWSQVLSRKLYLPAKQSSDLPLIVAIDLQPMAP
IEGVIQVQGDITNARTAEVVIRHFDGCKADLVVCDGAPDVTGLHDMDEFVQSQLILAGLTIVTHILKKGGKF
IAKIFRGKDTSLLYCQLKLFFTEVTLAKPKSSRNSSIEAFAVCENYTPPEGFNEKDLHRLLEKVGTPSGADN
LDCSSGWLEGPNKVYIPFLACGDLSGYD

FIGURE 7 (continued)

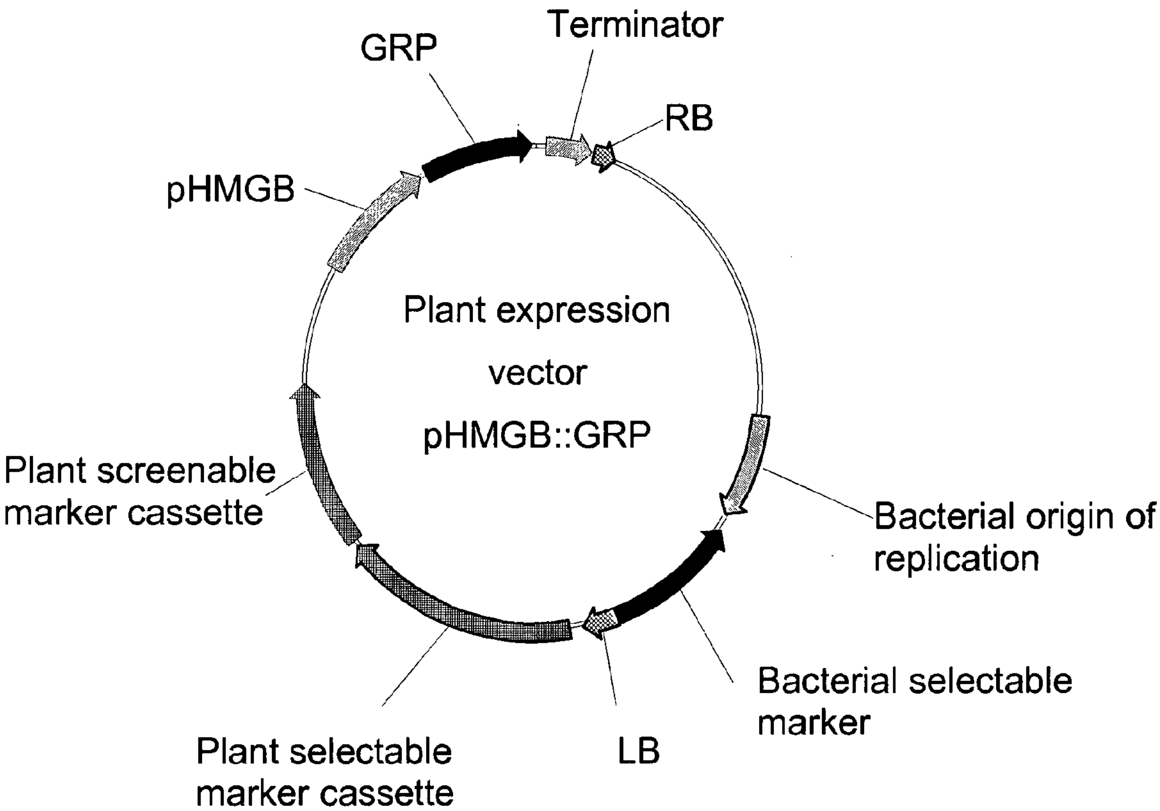
Terminator
GRP
RB
pHMGB
Plant expression
vector
pHMGB::GRP
Plant screenable
marker cassette
Bacterial origin of
replication
Bacterial selectable
marker
LB
Plant selectable
marker cassette

FIGURE 8

**SEQ ID NO: 104, *Arabidopsis thaliana* bHLH4 nucleic acid sequence**
ATGGCTCCGACGAATGTTCAAGTAACCGATTACCATCTCAACCAATCAAAAACGGATACAACAAATCTCTGG
TCAACCGACGACGATGCATCGGTAATGGAAGCTTTCATCGGCGGCGGCTCCGATCATTCTTCTCTTTTTCCT
CCACTTCCTCCTCCTCCTCTTCCTCAAGTCAACGAAGATAATCTCCAGCAACGTCTCCAAGCTTTAATCGAA
GGAGCAAACGAGAACTGGACTTACGCCGTGTTCTGGCAATCATCTCACGGTTTCGCCGGAGAAGACAACAAC
AACAACAACACAGTGTTGTTAGGTTGGGGAGATGGTTATTACAAAGGAGAAGAAGAGAAGTCTAGAAAGAAG
AAATCAAATCCAGCTAGTGCAGCTGAACAAGAGCATCGTAAGAGAGTGATTAGAGAGCTCAACTCTTTAATC
TCCGGTGGTGTAGGAGGAGGAGATGAAGCTGGAGATGAAGAAGTTACAGATACTGAATGGTTCTTCTTAGTT
TCAATGACACAGAGCTTTGTCAAGGGTACTGGTTTACCTGGTCAAGCTTTCTCAAATTCAGACACGATTTGG
TTATCTGGTTCTAATGCTTTAGCTGGATCAAGTTGTGAGAGAGCTCGTCAAGGTCAGATTTATGGGTTACAA
ACAATGGTGTGTGTAGCGACAGAGAATGGTGTCGTTGAGCTTGGTTCGTCGGAGATTATTCATCAAAGTTCA
GATCTTGTTGATAAAGTTGACACCTTTTTCAATTTTAACAATGGTGGTGGTGAATTTGGTTCTTGGGCGTTT
AATTTGAATCCAGATCAAGGAGAGAATGATCCAGGTTTGTGGATTAGTGAACCTAATGGTGTTGACTCTGGT
CTTGTAGCTGCTCCGGTGATGAATAATGGTGGAAATGACTCAACTTCTAATTCTGATTCTCAACCAATTTCT
AAGCTTTGTAATGGAAGCTCTGTTGAAAACCCTAACCCTAAAGTTCTGAAATCTTGTGAAATGGTGAATTTC
AAGAATGGGATTGAGAATGGTCAAGAAGAAGATAGTAGTAATAAGAAGAGATCACCGGTTTCGAATAATGAA
GAAGGGATGCTTTCTTTTACCTCTGTTCTTCCATGTGACTCGAATCACTCTGATCTTGAAGCTTCAGTGGCT
AAAGAAGCTGAGAGTAACAGAGTTGTGGTTGAACCGGAGAAGAAACCGAGGAAACGAGGGAGAAAACCGGCG
AATGGAAGAGAAGAGCCTTTGAATCATGTAGAGGCAGAGAGACAGAGAAGAGAGAAGTTGAATCAGAGATTC
TATTCTTTAAGAGCTGTGGTTCCTAATGTGTCTAAGATGGATAAAGCTTCTCTATTAGGAGATGCTATTTCG
TATATCAGTGAGCTTAAGTCTAAGTTGCAAAAGGCTGAATCTGATAAAGAAGAGTTGCAGAAGCAGATTGAT
GTGATGAATAAAGAAGCGGGAAATGCGAAAAGTTCGGTAAAAGATCGAAAATGTTTGAATCAAGAATCGAGT
GTGTTGATAGAGATGGAGGTTGATGTGAAGATTATTGGTTGGGATGCAATGATAAGGATTCAATGTAGTAAG
AGGAATCATCCTGGTGCTAAGTTCATGGAAGCACTTAAGGAGTTGGATTTGGAAGTGAATCATGCGAGTTTA
TCGGTAGTGAATGATCTTATGATCCAACAAGCGACTGTGAAAATGGGGAATCAGTTTTTCACGCAAGATCAA
CTCAAGGTTGCTCTAACGGAGAAAGTTGGAGAATGTCCATGA

**SEQ ID NO: 105, *Arabidopsis thaliana* bHLH4 polypeptide sequence**
MAPTNVQVTDYHLNQSKTDTTNLWSTDDDASVMEAFIGGGSDHSSLFPPLPPPPLPQVNEDNLQQRLQALIE
GANENWTYAVFWQSSHGFAGEDNNNNNTVLLGWGDGYYKGEEEKSRKKKSNPASAAEQEHRKRVIRELNSLI
SGGVGGGDEAGDEEVTDTEWFFLVSMTQSFVKGTGLPGQAFSNSDTIWLSGSNALAGSSCERARQGQIYGLQ
TMVCVATENGVVELGSSEIIHQSSDLVDKVDTFFNFNNGGGEFGSWAFNLNPDQGENDPGLWISEPNGVDSG
LVAAPVMNNGGNDSTSNSDSQPISKLCNGSSVENPNPKVLKSCEMVNFKNGIENGQEEDSSNKKRSPVSNNE
EGMLSFTSVLPCDSNHSDLEASVAKEAESNRVVVEPEKKPRKRGRKPANGREEPLNHVEAERQRREKLNQRF
YSLRAVVPNVSKMDKASLLGDAISYISELKSKLQKAESDKEELQKQIDVMNKEAGNAKSSVKDRKCLNQESS
VLIEMEVDVKIIGWDAMIRIQCSKRNHPGAKFMEALKELDLEVNHASLSVVNDLMIQQATVKMGNQFFTQDQ
LKVALTEKVGECP

**SEQ ID NO: 106, Oryza sativa HMGB promoter**
CATGCGGCTAATGTAGATGCTCACTGCGCTAGTAGTAAGGTACTCCAGTACATTATGGAATATACAAAGCTG
TAATACTCGTATCAGCAAGAGAGAGGCACACAAGTTGTAGCAGTAGCACAGGATTAGAAAAACGGGACGACA
AATAGTAATGGAAAAACAAAAAAAAACAAGGAAACACATGGCAATATAAATGGAGAAATCACAAGAGGAACA
GAATCCGGGCAATACGCTGCGAAAGTACTCGTACGTAAAAAAAAGAGGCGCATTCATGTGTGGACAGCGTGC
AGCAGAAGCAGGGATTTGAAACCACTCAAATCCACCACTGCAAACCTTCAAACGAGGCCATGGTTTGAAGCA
TAGAAAGCACAGGTAAGAAGCACAACGCCCTCGCTCTCCACCCTCCCACCCAATCGCGACGCACCTCGCGGA
TCGGTGACGTGGCCTCGCCCCCCAAAAATA

## FIGURE 9

TCCCGCGGCGTGAAGCTGACACCCCGGGCCCACCCACCTGTCACGTTGGCACATGTTGGTTATGGTTCCCGG
CCGCACCAAAATATCAACGCGGCGCGGCCCAAAATTTCCAAAATCCCGCCCAAGCCCCTGGCGCGTGCCGCT
CTTCCACCCAGGTCCCTCTCGTAATCCATAATGGCGTGTGTACCCTCGGCTGGTTGTACGTGGGCGGGTTAC
CCTGGGGGTGTGGGTGGATGACGGGTGGGCCCGGAGGAGGTCCGGCCCCGCGCGTCATCGCGGGGCGGGGTG
TAGCGGGTGCGAAAAGGAGGCGATCGGTACGAAAATTCAAATTAGGAGGTGGGGGGCGGGGCCCTTGGAGAA
TAAGCGGAATCGCAGATATGCCCCTGACTTGGCTTGGCTCCTCTTCTTCTTATCCCTTGTCCTCGCAACCCC
GCTTCCTTCTCTCCTCTCCTCTTCTCTTCTCTTCTCTGGTGGTGTGGGTGTGTCCCTGTCTCCCCTCTCCTT
CCTCCTCTCCTTTCCCCTCCTCTCTTCCCCCCTCTCACAAGAGAGAGAGCGCCAGACTCTCCCCAGGTGAGG
TGAGACCAGTCTTTTTGCTCGATTCGACGCGCCTTTCACGCCGCCTCGCGCGGATCTGACCGCTTCCCTCGG
CCTTCTCGCAGGATTCAGCC

**SEQ ID NO: 107, prm06763**
GGGGACAAGTTTGTACAAAAAAGCAGGCTTAAACAATGGCTCCGACGAATGTT

**SEQ ID NO: 108, prm06764**
GGGGACCACTTTGTACAAGAAAGCTGGGTTGCTGACTTCAATTCATGGAC

**SEQ ID NO 109: Arabidopsis thaliana bHLH4 II nucleic acid sequence**
atgaacggcacaacatcatcaatcaacttcttgacctccgacgatgacgcgtcggcggcggctatggaagct
ttcattggaacaaaccaccactcatctctctttcctccaccaccacaacaaccacctcagcctcagttcaac
gaagatactcttcaacaacgtctccaagctttaatagaatccgccggagaaaactggacttacgctatcttc
tggcagatctcacacgacttcgattcatccaccggagataacacagtgatcctcggctggggagatggttac
tacaaaggagaggaagataaagagaagaagaagaacaacaccaacacggcggagcaagagcatcggaaaaga
gtaatacgtgagcttaactcgttaatctccggcggaattggggtttccgatgaatcaaacgatgaagaagta
acagatactgaatggttcttcttagtttcgatgactcaaagcttcgttaacggtgttggtctccccggagaa
tctttcttaaactctcgtgtgatttggttatccgggtctggtgctttaaccgggtcgggttgtgaaagagcg
ggtcaaggtcagatttacgggttaaagacgatggtgtgtatcgcgactcaaaacggcgtcgttgagcttggt
tcgtcggaggttataagtcaaagctcagatctgatgcataaagttaacaacttgtttaatttcaacaacggt
ggtggaaacaatggtgttgaagcttcttcgtggggttttaatctgaatccagatcaaggagagaatgatcca
gctttgtggattagtgaaccgacgaacaccggaatcgaatctccggcgagggttaataatggtaataactcg
aattctaattctaagtctgattctcatcaaatttctaagcttgagaagaatgatattagctctgtagagaat
cagaatcgtcaaagttcgtgtcttgtcgagaaagatttgacctttcaaggtgggttgttgaaatctaatgag
actttgagtttctgtggtaatgagagtagtaagaagagaacttcggtatctaaagggagtaataatgatgaa
gggatgctttcgtttagtactgtggttagatcagctgcgaatgattcggatcattctgatcttgaagcatct
gttgttaaggaagcgattgttgttgagccaccggagaagaagccgaggaaacggggggaggaaaccggcgaat
gggagagaagagccgttgaatcatgttgaagcagagaggcagagaagagagaagttaaaccagagattctac
tctttgagagctgttgttcctaacgtttcgaagatggataaagcttcgcttctcggagacgcgatttcgtat
atcaatgagcttaagtcgaagctgcagcaagcggagtctgataaagaggagattcagaagaagctagatggg
atgagtaaggaagggaataatgggaaaggttgcgggtcaagggcaaaagaacggaaaagttcgaatcaagat
tctacggcgagttctatagaaatggagattgatgttaagatcataggttgggatgtgatgatacgtgtacaa
tgcggcaagaaagatcatcccggtgctaggttcatggaagcacttaaggaattggatttggaagtgaatcat
gcgagtttatccgttgtgaatgatttgatgattcaacaagctacggtgaagatggggagccaatttttcaat
catgaccagctcaaagttgctttgatgacgaaagtcggagaaaactattga

## FIGURE 9 (continued)

**SEQ ID NO 110: Arabidopsis thaliana bHLH4 II translated polypeptide sequence**
Mngttssinfltsdddasaaameafigtnhhsslfppppqqppqpqfnedtlqqrlqaliesagenwtyaif
wqishdfdsstgdntvilgwgdgyykgeedkekkknntntaeqehrkrvirelnslisggigvsdesndeev
tdtewfflvsmtqsfvngvglpgesflnsrviwlsgsgaltgsgceragqgqiyglktmvciatqngvvelg
ssevisqssdlmhkvnnlfnfnngggnngveasswgfnlnpdqgendpalwiseptntgiesparvnngnns
nsnsksdshqiskleknd issvenqnrqssclvekdltfqggllksnetlsfcgnesskkrtsvskgsnnde
gmlsfstvvrsaandsdhsdleasvvkeaivveppekkprkrgrkpangreeplnhveaerqrreklnqrfy
slravvpnvskmdkasllgdaisyinelksklqqaesdkeeiqkkldgmskegnngkgcgsrakerkssnqd
stassiemeidvkiigwdvmirvqcgkkdhpgarfmealkeldlevnhaslsvvndlmiqqatvkmgsqffn
hdqlkvalmtkvgeny

**SEQ ID NO 111: Brassica napus bHLH4 II nucleic acid sequence EF423803**
atgacggagccgacgatgaatctctggaccaccgacgataacgcctctatgatggaagctttcatgagctcc
tcctccgacatctcagccttatggccaccggtaacgacgacggcaacggcgtcgactacagctccggcaccg
gcgggattcaacgaggagactctacagcaaaggctacaagcactgattgaagggacgaacgaaggctggacc
tacgctatattctggcagccgtcgtacgacttctccggtgcctccgtgctcggatggggcgatggttattac
aaaggagaagaagacaaggcaaagcccagacagagaacgtctccaccgccgttttcaactccggcggatcag
gagtatcgtaagaaggtgttgcgtgagctcaactcgttgatctccggcggaggtggtccgacggatgatgcc
gtcgatgaagaggtgacggatacggagtggtttttcttggtttcgatgacgcagagcttcgcttgcggttct
gggttggcgggtaaggcgttctcgacgggtaacgcagtttgggtttatgggtcggatcagttaaccgggtcg
ggttgtgagcgggcgaagcaaggaggagtgtttgggatgcagaccatcgcgtgtattccttcggcgaacggg
gttgttgaactcgggccaacggagcagatccgacagagttcggatcttatgaacaaggtacgagtacttttc
aatttcaacggtggcgctggagatttatcgtgtcttaactggaatcttgacccggatcaaggcgagaacgat
ccgtctatgtggattaacgacccgattggagtacccgagcagggtaacggagctccgagctctagctcccag
ctttttgccaagtcgatccagtttgagaatggtggtagttcaagcaccatcatcgaaaacccgaatccggat
ccagctccagctccgagcccggttcattcccagacccagaatccgaaattcagcaacaatttctcccgcgaa
ttaaatttctccacgtcgagcaccaccttggtgaaaccaagacccgccgagatattgagcttcggcgatgag
ggtaaacggagctccgtgaacccggatccgagttcttattcgggtcagactcagttcgagaataagagaaag
aagtccataggtatgagcgatgacaaggttctaactttcggaaccggcggaggagaatccgatcactccgat
ctagaagcctccgtcgtgaaagagataccggagaaacgtcccaagaaacgtggaagaaaaccggccaacggt
agagaagagccgcttaaccacgtcgaagcagagagacagagacgcgagaaactaaaccagcgattctacgcg
ttacgtgcggttgtaccaaacgtctcgaaaatggataaagcttctttgctcggagacgcgattgcttacatc
aacgagctgaagtctaaggtgaccaagacggaatctgagaaaactcagatcaaaacccagctcgaggaagtg
aaaatggagctcgccggaagaaaagcaagcgccggtggagatctatcatcctcttgctcgttgacagcgatc
aaacccgtggggatggagatcgaggtgaagattatcggttgggatgcgatgataagggtggaatcgagcaag
aggaatcatccggcagcgaggttgatgtcggcgttgatggatctagagcttgaggtgaatcacgcgagtatg
tcggtggttaacgatctgatgatacagcaagcgacggtgaagatgggattcagaatatacacgcaggaacag
ctccgggcgagtttaatctcaaagattggttaa

**SEQ ID NO 112: Brassica napus bHLH4 II translated polypeptide sequence**
Mteptmnlwttddnasmmeafmssssdisalwppvtttatasttapapagfneetlqqrlqaliegtnegwt
yaifwqpsydfsgasvlgwgdgyykgeedkakprqrtspppfstpadqeyrkkvlrelnslisggggptdda
vdeevtdtewfflvsmtqsfacgsglagkafstgnavwvygsdqltgsgcerakqggvfgmqtiacipsang
vvelgpteqirqssdlmnkvrvlfnfnggagdlsclnwnldpdqgendpsmwindpigvpeqgngapssssq
lfaksiqfenggssstiienpnpdpapapspvhsqtqnpkfsnnfsrelnfstssttlvkprpaeilsfgde
gkrssvnpdpssysgqtqfenkrkksigmsddkvltfgtgggesdhsdleasvvkeipekrpkkrgrkpang
reeplnhveaerqrreklnqrfyalravvpnvskmdkasllgdaiayinelkskvtktesektqiktqleev
kmelagrkasaggdlssscsltaikpvgmeievkiigwdamirvesskrnhpaarlmsalmdlelevnhasm
svvndlmiqqatvkmgfriytqeqlrasliskig

## FIGURE 9 (continued)

**SEQ ID NO 113: Lotus japonicus bHLH4 nucleic acid sequence AP005602**
atgaatctctggagcgacgacaactcctccgtcatggaggccttcatgacctcctccgacttatcctccgtc
tggccaccagcgccgcctcctccctctcaatcctccgtcgccgtcctcaaccaggacactctccagcaacgc
ctccaggccctaatcgaaggcgcaagagagacctggacctacgccattttctggcaaccttcctacgactac
tccggcacctccctcctcggctggggcgacggttactacaaaggcgaagaggacaaagccaaggcaaaagca
aaagccaaagccaaagccacctcctccgctgaacaagaacaccgccggaaggtgcttcgtgacttgaattcc
ctcatctccggttcctccgcccccgcttctgatgacgctgtcgacgaagaggtcaccgacacggaatggttc
ttccttgtttccatgacccagtcgtttgtcaacggcggcgggctagctggtcaggcgtatttcaactccacc
cctgtttgggtcgccggcgctgatcgcctcgcagcctccgcctgcgagagggccaggcaggggcagctcttc
ggaatccagacgctcgtctgcgtgccgtctgcaaacggcgtcgttgaactcggatccactgagctgatttac
cagaactccgatctgatgaacaaggtgaaggtgctgttcaatttcagcaacagcaacctcgatttgggttct
tcctggacattgggttccaccaccaccgccgagaatgacccttccgccctctggctcgccgaccccgaccct
gacggcagagattccgtcagcaccgtcgctcccaccaccgcttccgtttcgattcccagccaccacaacaac
gatcagagcattgcgaagacgttgcagttcgaaacccctgtttcgagtaccttgactgaaaccccgagtgct
gttaatctcacaaaccagccatcgcagaaccagagaaaccagagcagcttcttctccagggagatgaatttc
tccgagtacagtttcgatgcgaagaacggcagcagtaaccaccagcatctgaagccggaatccggtgagatt
ctgagcttcggtgacagcaagagaacccccaatttcttctccggccagtcgcagttcgttccggctgtcgag
gagaacaacaacgggaagaagaggtcaccgaattcgagaagcagcaacgacgacgggatgctttcattcacc
tccggcgtgattctcccgtcctcgaatttgaaatcctccaccggcggcggcgattcggagcattcagatctg
gaagcttcggtggtgaaggaagccgacagcagcaggttggtggagccggagaagcgacctaggaagaggggg
aggaaaccggcgaacggcagagaggaaccgttgaaccacgttgaagcagagaggcagaggagggagaagctt
aaccagagattctacgcgcttcgcgcggtggttccgaacgtgtcgaagatggacaaagcttcactgctagga
gacgctatatcctacatcacggagctgaagaccaagcttcagagttcagaatcggataaaactgggttgcag
aagcaatttgatgcgatgaagaaggagctggagaaaaccagtgaacagtcttcttctccaactccacctcca
cctaacaagaacaagtccttctcctcatcctcctcctcttccaatcagattctagtggaggacattgacgtg
aagatcattgggtgggatgcaatgataagggtccaatgcagcaagaagaaccacccggccgcgattttgatg
gcggcgttgatggagctggaccttgaggtgaaccatgctagtgtgtcagtggtgaatgataccatgatccag
caagcaacagtgaagatgggaagccgcttttacactcaggagcagcttcgatcagcactctcttccaaattt
ggggatgctccttag

**SEQ ID NO 114: Lotus japonicus bHLH4 translated polypeptide sequence**
Mnlwsddnssvmeafmtssdlssvwppappppsqssvavlnqdtlqqrlqaliegaretwtyaifwqpsydy
sgtsllgwgdgyykgeedkakakakakakatssaeqehrrkvlrdlnslisgssapasddavdeevtdtewf
flvsmtqsfvngggglagqayfnstpvwvagadrlaasacerarqgqlfgiqtlvcvpsangvvelgsteliy
qnsdlmnkvkvlfnfsnsnldlgsswtlgstttaendpsalwladpdpdgrdsvstvapttasvsipshhnn
dqsiaktlqfetpvsstltetpsavnltnqpsqnqrnqssffsremnfseysfdakngssnhqhlkpesgei
lsfgdskrtpnffsgqsqfvpaveennngkkrspnsrssnddgmlsftsgvilpssnlkssstgggdsehsdl
easvvkeadssrlvepekrprkrgrkpangreeplnhveaerqrreklnqrfyalravvpnvskmdkasllg
daisyitelktklqssesdktglqkqfdamkkelektseqssptppppnknksfsssssssnqilvedidv
kiigwdamirvqcskknhpaailmaalmeldlevnhasvsvvndtmiqqatvkmgsrfytqeqlrsalsskf
gdap

**SEQ ID NO 115: Lycopersicon esculentum bHLH4 nucleic acid sequence**
Atgactgaatacagcttgcccaccatgaatttgtggaacaatagtactagcgatgataacgtttctatgatg
gaagcttttatgtcttctgatctttcttttgggctactaataattctacttctgctgctgcggttggtgtc
aattcaaatcttcctcatgctagtagtaatactccctctgttttgcaccatcttcttctacatctgcatct
actttatccgcagctgcgactgtggatgcttccaaatctatgccgtttttcaaccaagaaacccttcagcag
cgtcttcaagctcttattgatggtgctagagagacgtggacttatgctatcttttggcaatcgtcggttgtt
gatttctcaagtccgtctgtgttgggttggggagatggttattacaaaggggaagaagataaagcaaaaagg
aaattatcggtgtcatcacctgcttatattgctgagcaggagcatcggaagaaggttctacgggagctgaat
tcgttgatttccggggcaccacccggaacggatgatgcggttgatgaagaagttaccgacaccgaatggttc
tttcttatctccatgacccaatcgtttgttaatggaagtgggcttcctggtcaggcgttgtatagttccagc
ccgatttgggtcgccggaactgagaaattggcagcttcacactgtgaacgtgtgaggcaagcacaaggttc

## FIGURE 9 (continued)

gggctccagacgattgtctgtattccttcagctaacggcgtggttgaattgggctcgacggagttgattgtt
caaagttctgatcttatgaacaaggttagagtattgtttaacttcagtaatgatttgggttctggttcatgg
gctgtgcagccggagagcgacccatcggcgctctggctcactgatccatcgtcctcaggtatggaagttaga
gagtctttaaatacagttcaaacaaattcagttccatctagtaatagtaataagcaaattgcttatggaaat
gagaataatcatccatctggaaatggtcagagttgttacaatcagcaacaacagaagaatcctcctcagcaa
caaacacaaggactcttcacgagggagttgaattttcggaattcggtttcgatggaagtagtaataggaat
ggaaattcatcggtttcttgcaagcctgaatcaggagaaatcttgaattttggtgatagtactaaaaaaagt
gcttccagtgccaatgtgaacttgtttacaggtcagtcccaatttggggctggggaggagaataataacaag
aacaagaaaagatcagctacttccaggggaagcaatgaagaaggaatgctttcatttgtttcaggtacagtg
gtgccttcttcgggcatgaagtcaggtggaggcggaggcgaagactctgaacattcagatctcgaggcttca
gtggtgaaagaagctgatagtagtagagtggtagagcctgaaaagaggccaaggaagcgaggtagaaagcca
gcgaatggacgggaggagccattgaatcacgtcgaggcagagaggcaaaggagggagaaattgaaccaaaga
ttctacgcgcttagagctgttgtaccaaatgtgtctaagatggacaaggcatcactccttggagatgctatt
tcctatataaacgagttgaaatcgaagcttcaaaatacagagtcagataaagaagacttgaagagccaaata
gaagatttaaagaaagaatcaaggcgccccggtcctcctccaccaccaaatcaagatctcaagatgtctagc
cacactggaggcaagattgtagacgtggatatagacgttaagatcatcggatgggatgcaatgattcgtata
caatgtaataaaaagaatcatccagccgcaaggctaatggcagcgctcatggaattagacctagacgtgcat
catgccagtgtttcagttgtcaacgatttgatgatccaacaagccacagtgaaaatgggtagcaggaattac
actgaagagcagcttagggtagcgttgacatcgaaaattgctgaaacacactaa

**SEQ ID NO 116: Lycopersicon esculentum bHLH4polypeptide sequence full length**

Mteyslptmnlwnnstsddnvsmmeafmssdlsfwatnnstsaaavgvnsnlphassntpsvfapssstsas
tlsaaatvdasksmpffnqetlqqrlqalidgaretwtyaifwqssvvdfsspsvlgwgdgyykgeedkakr
klsvsspayiaeqehrkkvlrelnslisgappgtddavdeevtdtewfflismtqsfvngsglpgqalysss
piwvagteklaashcervrqaqgfglqtivcipsangvvelgstelivqssdlmnkvrvlfnfsndlgsgsw
avqpesdpsalwltdpsssgmevreslntvqtnsvpssnsnkqiaygnennhpsgngqscynqqqknppqq
qtqglftrelnfsefgfdgssnrngnssvsckpesgeilnfgdstkksassanvnlftgqsqfgageennnk
nkkrsatsrgsneegmlsfvsgtvvpssgmksgggggedsehsdleasvvkeadssrvvepekrprkrgrkp
angreeplnhveaerqrreklnqrfyalravvpnvskmdkasllgdaisyinelksklqntesdkedlksqi
edlkkesrrpgppppnqdlkmsshtggkivdvdidvkiigwdamiriqcnkknhpaarlmaalmeldldvh
hasvsvvndlmiqqatvkmgsrnyteeqlrvaltskiaeth

**SEQ ID NO 117: Vitis vinifera contig bHLH4 like VV78X118066.5, nucleic acid sequence AM475703.2**

atgacggaatatcgtgtgccaaccatgaatctgtggaccgacgataacgcttctatgatggaggcttttata
agctctgatctctcttcctttagttgggggccatcctccgctgcttctacgtctactcctgcgcctgacccg
tcaaggaatctcgcccaatctcagccttccatggccgtcttcaaccaggagaccctccagcagcgacttcaa
gccctaattgaaggcgcccgagagagctggacctacgcaatttttggcagtccagcgttgatttctcgggt
gcctctttgttaggatggggtgacggctactataaaggtgaggaagataaagggaagaggaagatgaccccg
tcgtctgtttccgagcaggagcaccggaagaaagtgctccgggagctgaattcgctcatttctgggacggcg
tcgtcgtcggacgacgctgtcgatgaggaggtcaccgacaccgagtggttcttccttgtatcgatgactcag
tcgtttgtgaacggcgctgggttgccgggtcaggcgctcttcaactcgagtccggtgtgggttgtcggaacc
gaacggctcatgagttctccttgcgagagagcccggcaggcgcaggtgttcgggttacagactatggtttgt
ataccatcggctaacggcgtcgtcgaactgggctccaccgaattgatttaccagagctcagatctgatgaac
aaggttagagtttgttcaatttcaataaccttgaagttggttcatggccgatcggagccgccgctcccgac
cagggcgagagcgatccttcgtcgctctggatctccgatccgacctctaatgtcgaaatcaaggattctgtg
aatgctacagctactggagcttccaatcccattggtaatcagcagaattccaagtcaattcaatttgagaac
cctagctcaagtagtttaactgaaaaccctagtattatgcataatcctcagcagcaacagcagattcacacg
cagggtttttttcactagggagttgaatttctcagaatttggatttgatggaaayaatgggagaaatggtaac
ttgcattccctgaagcccgagtctggggaaattttgaattttggagacagtaagaggagttcttgtagtgcg
aatgggaatatgttttcaggtcattcacaggtagttgcagaggaaaataagaagaggaggtcaccgacttca
agaggaagtgccgaagaaggcatgctttcgtttacttcaggtgtgatattgccatcctcttgtgtggtgaag

# FIGURE 9 (continued)

tcaagtggtggtggtggagattctgatcactcggatcttgaagcttcagtggttcgggaggcagatagtagt
agagtggtggaaccagaaaaaaggcctaggaagcgtgggagaaaacctgcaaatggaagggaagagccattg
aatcatgtggaggcagagcggcagaggagggagaagcttaaccagaggttttatgccctccgagctgtggtt
ccaaatgtgtcaaagatggataaagcctcccttcttggtgatgcaatttcatatatcaatgagctcaggaca
aagctacaaagtgcagagtcggacaaggaggaccttcagaaggaagtgaattcaatgaagaaggagttggct
agtaaagatwcacagtactccggttcatctcgaccacctccagaccaagatctcaagatgtcgaatcaccac
ggtagcaaattggtagaaatggatattgatgtgaagataatcgggtgggatgccatgattcgaattcagtgt
agtaaaaagaaccatcctgctgcaaagcttatggggctctcaaggagctggacctagatgtgaaccacgcg
agtgtgtcggtggtgaatgatttgatgatccaacaggcaaccgtgaagatgggaagtaggttttacactcaa
gatcagctgaggctagccctgtcatccaaatttgcagacagcaggtag

**SEQ ID NO 118: Vitis vinifera contig bHLH4 like VV78X118066.5 translated polypeptide sequence**

Mteyrvptmnlwtddnasmmeafissdlssfswgpssaaststpapdpsrnlaqsqpsmavfnqetlqqrlq
aliegareswtyaifwqssvdfsgasllgwgdgyykgeedkgkrkmtpssvseqehrkkvlrelnslisgta
sssddavdeevtdtewfflvsmtqsfvngaglpgqalfnsspvwvvgterlmsspcerarqaqvfglqtmvc
ipsangvvelgsteliyqssdlmnkvrvlfnfnnlevgswpigaaapdqgesdpsslwisdptsnveikdsv
natatgasnpignqqnsksiqfenpsssltenpsimhnpqqqqihtqgfftrelnfsefgfdgnngrngn
lhslkpesgeilnfgdskrsscsangnmfsghsqvvaeenkkrrsptsrgsaeegmlsftsgvilpsscvvk
ssggggdsdhsdleasvvreadssrvvepekrprkrgrkpangreeplnhveaerqrreklnqrfyalravv
pnvskmdkasllgdaisyinelrtklqsaesdkedlqkevnsmkkelaskdxqysgssrpppdqdlkmsnhh
gsklvemdidvkiigwdamiriqcskknhpaaklmgalkeldldvnhasvsvvndlmiqqatvkmgsrfytq
dqlrlalsskfadsr

**SEQ ID NO 119: Zea mays bHLH4 full length nucleic acid sequence contig of AF061107 and EE190449**

atgaacctgtggacggacgacaacgcctccatgatggaggccttcatggcttctgccgacctccccaccttc
ccctggggagcgccggccgggggcggcaactcgtcggccgccgcggcgtcgccgccgccgcagatgccagcg
gcgatggctcccgggttcaaccaggacacgctgcagcagaggctacaagccatgatagaggggtcgagggag
acctggacctacgccatcttctggcagtcctcccttgactccgccaccggcgcctcgctgctcggctgggg
gacggctactacaagggctgcgacgaagacaagcgcaagcagaagccgctcacgccctccgcccaggccgag
caggagcaccgcaagcgcgtgctccgcgagctcaactccctcatctccggcgcggcggcggctcccgacgag
gccgtcgaggaggaggtcaccgataccgagtggttcttcctcgtctccatgacgcagtcgtttctcaacggt
tcgggcctccccgggcaggcgctcttcgccgggcagcccacatggatcgcctctggcctctcctccgcgccg
tgcgagcgcgcgcgtcaggcctacaacttcggcctccgcaccatggtatgcttccccgtcggcaccggggtg
ctcgagctcggctccaccgacgtcgtgttcaagaccgccgagagcatggctaagatccgctcgctctttggg
ggcggagcagggggtggctcgtggccgcccgtgcagcctcaggcgccgtcgtcgcagcagcccgccgccgga
gccgaccacgcggagacggacccgtccatgctatggctcgccgacgcgccggtcatggacatcaaggattcc
ttgtcgcacccgtcggccgagatctccgtctccaagcctccgccgcatccgccgcagatccatttgagaac
gggagcacgagcacgctcacggagaaccccagcccctccgtgcacgcgccgccaccccgccggcgccggct
gctccacagcagcggcagcaccagcaccagaatcaggcgcaccagggcccgttccgccgggagctcaatttc
tcggacttcgcgtccactccttccttggcggcgaccccgccgttcttcaagcccgagtccggcgagatcctc
agcttcggcgccgacagcaacgcccggaggaacccgtcgccggtacctcccgccgccaccgccagcctcacc
accgctcccgggagcctcttctcgcagcacacggcgaccatgacggccgccgcggcgaacgacgcgaagaac
aacaacaagcgctcaatggaggccacctcccgggcgagcaacaccaaccaccacccggcggcgacggcgaac
gagggcatgctgtccttctcgtcggcgccgactacgcggccgtcgacgggcacgggcgcgccggccaagtcg
gagtccgaccactcggacctggacgcgtctgtgcgcgaggtggagagcagccgcgtggtggcgccgccaccg
gaggcggagaagcggccgcgaaagcgcgggcggaagcccgcgaacgggcgcgaggagccgctgaaccacgtg
gaggcggagcggcagcggcgggagaagctgaaccagcgcttctacgcgctgcgcgccgtggtgcccaacgtg
tcgaagatggacaaggcgtcgctgctcggcgacgccatctcctacatcaacgagctccggggcaagctgacg
tcgctggagaccgacaaggagacgctgcagacccaggtggaggcgctcaagaaggagcgcgacgcgcggccg

FIGURE 9 (continued)

ccctcgcactccgctgggctcggcgggcacgacggcgggccgcgctgccacgcggtggagatcgacgccaag
atcctggggctggaggccatgatccgcgtgcagtgccacaagcgcaaccacccgtcggcgcggctgatgaca
gcgctccgcgagctcgacctggacgtgtaccacgccagcgtgtccgtcgtcaaggacctcatgatccagcag
gtcgccgtcaagatggccagccgcgtgtacacgcaggaccagctcagcgccgcgctctacagccgcctcgcg
gagcccgggtctgccatgggcaggtaatgaaggaggagagctgctcagctcggctcg

**SEQ ID NO 120: Zea mays bHLH4 full length translated polypeptide sequence**
mnlwtddnasmmeafmasadlptfpwgapagggnssaaaasppqmpaamapgfnqdtlqqrlqamiegsre
twtyaifwqssldsatgasllgwgdgyykgcdedkrkqkpltpsaqaeqehrkrvlrelnslisgaaaapde
aveeevtdtewfflvsmtqsflngsglpgqalfagqptwiasglssapcerarqaynfglrtmvcfpvgtgv
lelgstdvvfktaesmakirslfgggagggswppvqpqapssqqpaagadhaetdpsmlwladapvmdikds
lshpsaeisvskppphppqihfengststltenpspsvhapppppapaapqqrqhqhqnqahqgpfrrelnf
sdfastpslaatppffkpesgeilsfgadsnarrnpspvppaataslttapgslfsqhtatmtaaaandakn
nnkrsmeatsrasntnhhpaatanegmlsfssapttrpstgtgapaksesdhsdldasvrevessrvvappp
eaekrprkrgrkpangreeplnhveaerqrreklnqrfyalravvpnvskmdkasllgdaisyinelrgklt
sletdketlqtqvealkkerdarppshsaglgghdggprchaveidakilgleamirvqchkrnhpsarlmt
alreldldvyhasvsvvkdlmiqqvavkmasrvytqdqlsaalysrlaepgsamgr

**SEQ ID NO 121: Brassica napus bHLH4 nucleic acid sequence**
atggatgatcatcggaatcgtgtctctctctctccaccggatgctcacatgagcaactacttcctcaaccag
tcaacagccaccgacgacgataacgcctccgcctcgatggaagctttcatcggaacaaatcactggtcacaa
caaccatcccttcctcctcctccgtcattgtctcagttcaacgaagatactctccagcaacgtctccaaacg
ctaatcgaatccgcgggtgagagatggacgtacgcgatcttctggcagatctcgcacgatttcgactctccc
gccggagagagcacggtgattctcgggtggggagacgggtactacagaggagaggaagacaaagagaagaag
aagaaacagagctcgagctcgaatccggcggagcaggagcatcggaaaagagtaatccgggagcttaactcg
ttaatcgccggcggagccggagtttccgacgaagccaacgacgaggaagttacggatacagagtggttcttc
ttggtttcgatgactcagagcttcgtaaacggcgttgggctccccggagagtctttttaaactcccgcgtg
atttggttatccgggtcgggtgcgttaaccgggtcaggctgcgagcggcgaggcaaggggaggtttacggg
ttgcagacgatagtgtgtatcgctgcggaaaacggcgtcgttgagcttggttcgtcggaggtgataagtcaa
agctcagatctgatggataaagtcaacggtctttcaacggtaacggtaacggggaagcttcttcttgggg
tttaatctcaatcctgatcaaggtgagaacgatcctgctttgtggttatctgaaccgaacataaccggaatc
gaaccggttcaggaaacgcccgcgattgagaacgccgctgatttgaacttctcgagctcagggctgaaccaa
aacggtaactttaaacaagggtcgtcaagcaacaagaagagatctccggttgggaaagacgaagaaatgctt
tctttcagcacggtggttcgatccgcggcgaagtcagtggactctgatcattccgatatcgaagcatcggtg
gttaaggaggcgattatcgtcgaaccggagaagaaaccgaggaaacgggggagaaaaccggctaacgggaga
gaagagccgttgaaccatgtggaagcagagaggcagcgaagggagaagctaaaccagagattctactcgttg
agagctgtggttcccaacgtttcgaaaatggacaaggcttctcttcttggagacgccatttcgtatatcaac
gagctgaaaacgaagctgcagcaagcggagaccgataaggaagaggttcagaagcagctagatcggatgagc
aaggaaggtggcgggtctaggagggcgaaagagcgaaaatcgaacctagattccgcgagttctgtggaaatg
gagattgatgtgaagatcataggttgggatgtgatgatacgtgtacaatgcggcaagaagaatcatcctggt
gcgaggttcatggaagcgcttaaggagttggatttggaagtgaatcatgctagtttatctatggtgaatgat
ttgatgattcaacaagctactgtgaagatgggaagccagtttttcaatcatgatcagctcaaggctgcttta
atgttgaaagttggagaagacagttga

**SEQ ID NO 122: Brassica napus bHLH4 translated polypeptide sequence**
Mddhrnrvslsppdahmsnyflnqstatdddnasasmeafigtnhwsqqpslppppslsqfnedtlqqrlqt
liesagerwtyaifwqishdfdspagestvilgwgdgyyrgeedkekkkkqssssnpaeqehrkrvirelns
liaggagvsdeandeevtdtewfflvsmtqsfvngvglpgesflnsrviwlsgsgaltgsgcerarqgevyg
lqtivciaaengvvelgssevisqssdlmdkvnglfngngngeasswgfnlnpdqgendpalwlsepnitgi
epvqetpaienaadlnfsssglnqngnfkqgsssnkkrspvgkdeemlsfstvvrsaaksvdsdhsdieasv
vkeaiivepekkprkrgrkpangreeplnhveaerqrreklnqrfyslravvpnvskmdkasllgdaisyin
elktklqqaetdkeevqkqldrmskegggsrrakerksn

**FIGURE 9 (continued)**

ldsassvemeidvkiigwdvmirvqcgkknhpgarfmealkeldlevnhaslsmvndlmiqqatvkmgsqff
nhdqlkaalmlkvgeds

**SEQ ID NO 123: Glycine max bHLH4 nucleic acid sequence**
atgaatctgtggacggacgagaactcctcggtgatggaggccttcatgagctcctccgacctctcctccctc
tggctccccacgccgcaatccgccgcctctactaccactcctggactggaaaccacccgagcaccgccgcct
cagtcccactccctcctcaaccaggagaccctccagcagcgcctccagacacttatcgaaggcgcccgagag
agctggacctacgcaatcttctggcagtcttcctacgactattcctccggcacctcccttctcggctgggga
gacggttattacaagggtgaggaggacaaagtcaaagccaaaggcaaaacccccaaaacgacgtcgtccgcg
gagcaggaccaccgcaaaaaagtcctccgcgaactcaattccttaatctctggcccctccgcttccgttgac
gacgtcgacgaagaagtcaccgacaccgagtggttcttcctcgtgtcgatgactcagtccttcgtcaacgga
agcggcctcccgggtcaggcttttttaactcgagcccggtctgggttgccgggccggaccggctgtccgag
tcggtctgcgaacgggcccaccaggggcagatgttcgggctccagactctggtctgcataccgtccgcaaat
ggcgtcgttgagctcgcctccacggaggtgattttccagaaccctgatctgatgaacaaggtgcgcgatttg
ttcaacttcaacaacaaccctgaaactggttcctgggcgttgaattgcgttgccaccaccgatcagggggag
aacgacccttcctcactctggctcaaccctgaaatcagagactcctctaccgttgcgccgccgaattcaacg
gttaacaagactctgcagttcgaaacccctggttccagtaccttaaccgataccctagtgctgctgctgtt
catgtacctaaatctaatggccagggtttcttctcgagggaattgaacttttcgaattcattgaagcctgaa
tctggtgaaattctgagctttggagagagcaagaagagctcttacaacgggagtttctttcccggtgttgtt
gcaattgaggagaacaacaagaagaggtctcccgtttctcggagcagcattgacgatgggatgctgtccttc
acatccctgccggctgcaaatataaaatctggtagtggtggtgccggtgccggtggtggtgattcggatcac
tcggatctggaagcttccatggtgaaacaggcagacagcagagtgatggagccggagaaacggcctcggaag
aggggcgaaagccggccaacggcagagaggagccgctgaatcacgtggaagccgagagacaaaggagagaa
aagctgaatcaaagattctatgctctacgagctgtagttccaaatgtatccaagatggacaaggcatcgttg
ttaggtgacgccatatcgtacataaatgagctgaaattgaagctcaacgggctggattcagagaaaggggaa
ttggagaagcaattggattcagccaagaaggaacttgagctcgcgaccaaaaaccctcctcctcctccacca
ccaccacctggactacctccatctaataacgaagaagcaaagaagacaacgaccaagctcgctgatttggag
atagaggtgaagataattggttgggatgcgatgataagaatccaatgcagcaagaagaaccaccctgcagca
aggttgatggcagcattgaaggacctggacttggaagtgcatcacgcaagcgtgtccgtggtgaatgattta
atgatccaacaagccacggtgaacatgggaaacaagttttacacgcaggagcagcttctgtcggcactctcg
tccaaagttggcgatgaactacgatag

**SEQ ID NO 124: Glycine max bHLH4 translated polypeptide sequence**
Mnlwtdenssvmeafmsssdlsslwlptpqsaastttpglettrapppqshsllnqetlqqrlqtliegare
swtyaifwqssydyssgtsllgwgdgyykgeedkvkakgktpkttssaeqdhrkkvlrelnslisgpsasvd
dvdeevtdtewfflvsmtqsfvngsglpgqaffnsspvwvagpdrlsesvcerahqgqmfglqtlvcipsan
gvvelastevifqnpdlmnkvrdlfnfnnnpetgswalncvattdqgendpsslwlnpeirdsstvappnst
vnktlqfetpgsstltdtpsaaavhvpksngqgffsrelnfsnslkpesgeilsfgeskkssyngsffpgvv
aieennkkrspvsrssiddgmlsftslpaaniksgsggagagggdsdhsdleasmvkqadsrvmepekrprk
rgrkpangreeplnhveaerqrreklnqrfyalravvpnvskmdkasllgdaisyinelklklngldsekge
lekqldsakkelelatknpppppppppglppsnneeakktttkladleievkiigwdamiriqcskknhpaa
rlmaalkdldlevhhasvsvvndlmiqqatvnmgnkfytqeqllsalsskvgdelr

**SEQ ID NO 125: Glycine max bHLH4 II nucleic acid sequence**
Atgaccgagtaccggatgaacctgtggaccgacgacaactcctccgtcatggaggccttcatgagctcctcc
gacctctcctccctctggcttgccacgccgcagtccgccacctccaccacaactccaggcacggcaaaagcc
cctcctcctcctcctcctcctccgccgccgccggcgcagtcccagtccctcctcaaccaggagactctccag
caacgcctccagactctcatcgaaggcgcctgcgagagctggacctacgcaatcttctggcaatcttcctac
gactattcctccggcacctcccttctcggctggggagacggttactacaagggcgaggaggacaaagacaaa
gtcaaaaccaaagcccccaaaacgaggtcgtccgcagagcaggaccatcgtaagaaggtcctccgcgaactt
aattccttaatttccggcccttccgcttcagctgacgacattgacgaagaagtcaccgacaccgagtggttc
ttcctggtgtcgatgactcagtccttcgtcaacggtagcgggctgccgggccaggcttttttaactcgagc
ccggtctgggtcgccgggcccgaaagactttccgaatcggcctgcgaacgggcccgccaggggcagctcttc

**FIGURE 9 (continued)**

gggctccagactcttgtctgtataccgtccgcaaacggcgtcgtcgagctcgcctccgcggaggtcatttt
cagaaccccgatctgatgaacaaggtgcgtgatttgttcaacttcaacaacaacaacaacaacaacaaccct
gaaacgtgttcctgggcgttgaattgcgttgccaccaccgatcaaggcgagaacgacccttcctcgctctgg
ctcaaccctgaaatcaaagactcctccaccgtctcgccgccgaattccacggttaataagacgatgcatttc
gaaacccctggttccagtaccctaaccgaaacccctagcgctgctgctgctgttcacgtccccaattctaaa
agccagggattcttcccgagggaattgaacttttcgaattcattgaaacctgaatccggtgaaattctgagc
tttggggagagcaagaagagctcttacaatggggctttctttcccggtgttgttgcggttgaggagaacaac
aacaataacaagaacaagaagaagaggtccccggtggtttctcggagcagcattgacgatggaatgctttcc
ttcacttccctgccggctgcaaatataaaatcagttaacggtgcttgtgtcggtgccggtgattcggatcac
tcggatctggaagcttccgtggcgaaacaggtggtggagcccgagaaacggccccggaagagagggcgaaag
ccggctaacggaagagaggaaccactaaatcatgtggaagccgagagacagaggagagaaaagctgaaccaa
agattctatgctctacgagctgtagttccaaatgtatccaagatggacaaggcatcgctgttgggggacgcc
atattgtacattaacgagttgaaatcgaagctcaacgtgctggattcagagaaaacggaattggagaagcaa
ttggattcaaccaagaaggaactcgagctcgcaactaaaaaccctcctccaccgccaccgccgccaccacca
ccaggaccgcctccatctaatagcgtagaaccaaagaagacaacaagcaagctcgctgatttggagttagag
gtgaagatcattggttgggatgcaatggtaagaatccaatgcagcaagaagaaccaccctgcagccaggttg
atggcagcattgaaagatcttgacttggaagtgcatcacgccagcgtgtctgtggtgaatgatttgatgatc
caacaagccacggtgaacatgggaaacaagttttacacgcaggaacagcttctgtcggcactatcatccaaa
gttggcgatgaactacgatag

**SEQ ID NO 126: Glycine max bHLH4 II translated polypeptide sequence**
Mteyrmnlwtddnssvmeafmsssdlsslwlatpqsatstttpgtakappppppppppppaqsqsllnqetlq
qrlqtliegaceswtyaifwqssydyssgtsllgwgdgyykgeedkdkvktkapktrssaeqdhrkkvlrel
nslisgpsasaddideevtdtewfflvsmtqsfvngsglpgqaffnsspvwvagperlsesacerarqgqlf
glqtlvcipsangvvelasaevifqnpdlmnkvrdlfnfnnnnnnnnpetcswalncvattdqgendpsslw
lnpeikdsstvsppnstvnktmhfetpgsstltetpsaaaavhvpnsksqgffprelnfsnslkpesgeils
fgeskkssyngaffpgvvaveennnnknkkkrspvvsrssiddgmlsftslpaaniksvngacvgagdsdh
sdleasvakqvvepekrprkrgrkpangreeplnhveaerqrreklnqrfyalravvpnvskmdkasllgda
ilyinelksklnvldsektelekqldstkkelelatknpppppppppppgpppsnsvepkkttskladlele
vkiigwdamvriqcskknhpaarlmaalkdldlevhhasvsvvndlmiqqatvnmgnkfytqeqllsalssk
vgdelr

**SEQ ID NO 127: Hordeum vulgare bHLH4 nucleic acid sequence**
atgatggaggccctcatggcctccgccgacatgccggcgttcccctggggcgcggcggccaccccgccgccg
ccggccgccgtgccggccttcaaccaggacacgctccagcagcgcctgcaggccatcatcgagggctccagg
gagacctggacctacgccatcttctggcagtcctccaccgacgccggcgcctcgctcctcggctggggcgac
ggctattacaagggctgcgacgacgccgacaagcgccgccagcagcccaccccggcctccgccgccgagcag
gagcaccgcaagcgcgtcctcagggagctcaactcgctcatagccgggggcggcgccgccgcgcccgacgag
gccgtcgaggaggagggcacggacaccgagtgggtcttcctcgtctccatgacccagtccttccccaacggg
atgggcttgccgggccaggctctcttcgccggccagcctatctggatcgccaccgggctcgccagcgcgccc
tgcgagcgggccaagcaggcctacaccttcggcctccgcaccatggtctgcatcccccctcggcaccggcgtg
ctcgagctcggcgccaccgaggtcatcttccagaccaccgatagcttggggcggatccgctcgctcttcagc
ctcaacggcggaggaggggggctctggatcctggccgcccgtggcgccgccgccccaggaggcggagacggat
ccgtccgtgctctggctcgccgacgcgccggccggggacatgaaggagtcgccgccgtccgtcgagatctcc
gtctccaagccgccgccgtcacagccgccgcagatccatcacttcgagaacgggagcaccagcacgctcacg
gagaaccccagcctctccgtgcacgcgcagcagcctctgccgcagcagcaggcggcggcggcggcgcagagg
cagaaccagctccagctccagcaccagcacaaccagggtcctttccgccgggagctcaatttctcagatttc
gcgtccaacccatccgtcacggtgaccccgcctttcttcaagcctgagtctggtgagatcctaaactttggc
gctgacagcaccagccggaggaaccccttcgccggcgcccccccgccgcgacggccagcctcaccaccgcgccg
gggagcctcttctcccagcacacggcgacggtgacggccccatcaaacgacgccaagaacaacccgaagcgg
tccatggaggccacctcccgcgcgagcaacaccaaccaccaccagaccgcgacagccaacgaggggatgctg
tccttctcgtccgcgccgacgacgcggccgtccaccggcacgggcgcaccagccaagtcggagtccgaccat
tccgacctggaggcgtcggtccgcgaggtggagagcagccgcgtggtgcctccgccggaggagaagcggccg

FIGURE 9 (continued)

cgcaagcgcgggcgcaagccggcgaacgggcgcgaggagccactgaaccacgtggaggcggagcggcagcgg
cgggagaagctgaaccagcggttctacgcgctccgcgccgtggtgcccaacgtgtccaagatggacaaggcc
tcactgctgggcgacgccatctcctacatcaacgagctccgcggtaagatgacggcgctggagtcggacaag
gagacgctccactcccaaattgaggcgctgaagaaggagcgcgacgcccggccggccgcgccgtcgtcgtcg
ggaatgcacgacaacggggcgcggtgccacgcggtggagatcgaggccaagatcctggggctggaggcgatg
atccgcgtgcagtgccacaagcgcaaccacccggcggcgaagctgatgacggcgctgcgggagctggacctg
gacgtgtaccacgccagcgtctcggtggtgaaggacatcatgatccagcaggtggcggtgaagatggccacc
cgggtctactcccaggaacagctcaacgcggcgctctacggccgcctcgccgagccgggcgccgcgatgcaa
atccggtaa

**SEQ ID NO 128: Hordeum vulgare bHLH4translated polypeptide sequence**
Mmealmasadmpafpwgaaatppppaavpafnqdtlqqrlqaiiegsretwtyaifwqsstdagasllgwgd
gyykgcddadkrrqqptpasaaeqehrkrvlrelnsliagggaaapdeaveeegtdtewvflvsmtqsfpng
mglpgqalfagqpiwiatglasapcerakqaytfglrtmvciplgtgvlelgatevifqttdslgrirslfs
lngggggsgswppvapppqeaetdpsvlwladapagdmkesppsveisvskpppsqppqihhfengststlt
enpslsvhaqqplpqqqaaaaaqrqnqlqlqhqhnqgpfrrelnfsdfasnpsvtvtppffkpesgeilnfg
adstsrrnpspappaatasltt apgslfsqhtatvtapsndaknnpkrsmeatsrasntnhhqtatanegml
sfssapttrpstgtgapaksesdhsdleasvrevessrvvpppeekrprkrgrkpangreeplnhveaerqr
reklnqrfyalravvpnvskmdkasllgdaisyinelrgkmtalesdketlhsqiealkkerdarpaapsss
gmhdngarchaveieakilgleamirvqchkrnhpaaklmtalreldldvyhasvsvvkdimiqqvavkmat
rvysqeqlnaalygrlaepgaamqir

**SEQ ID NO 129: Zea mays bHLH4 II nucleic acid sequence**
atgaacctgtggacggacgacaacgcctccatgatggaggccttcatggcctccgccgacctccccgcctac
ccctgggggcgccggccgggggcggcaacccgccgccgccgcagatgccgccggcgatggcgatggcgccg
gggttcaaccaggacacgctgcagcagcggctgcaggccatgatcgaggggtccagggagacctggacctac
gccatcttctggcagtcctccctggacgccgccaccggcgcgtcgctgctaggctggggcgacggctactac
aagggctgcgacgacgacaagcgcaggcaccgcccgccgctcacgcccgccgcgcaggccgagcaggagcac
cgcaagcgcgtgctccgcgagctcaactccctcatctccggcggcgcctccgccgcgcccgcgcccgcgccc
gacgaggccgtcgaggaggaggtcaccgacaccgagtggttcttcctcgtctccatgacgcagtccttcctc
aacggctcgggcctccccggccaggcgctcttcgcgggccaccacacctggatcgccgccggcctctcctcc
gcgccgtgcgaccgcgcgcgccaggcctacaacttcggcctccgcaccatggtctgcttccccgtcggcacg
ggcgtgctcgagctcggctccaccgacgtcgtgttccagaccgccgagaccatggccaagatccgctcgctc
ttcgggggcgggccgggggtggctcgtggccgcccgtgcagccccaggcggcgccgcagcagcagcacgcc
gccgaagccgaccaggcggcggagacggacccatccgtgctgtggctcgccgacgcgccggtcgtggacatc
aaggattcctactcgcacccgtcggcggccgagatctccgtctccaaaccgcctccgcctccgcctccgccg
cagattcacttcgagaacgggagcacgagcacgctcacggagaaccccagcccctccgtgcacgcgccgcca
gccccgccggcgccaccgcagcggcagcagcagaatcagggcccgttccgccgggagctcaacttctcggat
ttcgcgtccaatccttccttggcggcggccccgccgttcttcaagcccgagtccggcgagatcctcagcttc
ggcgtcgacagcaacgcccagaggaacccgtcgccggcgcctcccgccagcctcaccaccgctcccggcagc
ctattctcgcagtcgcagcacacggcgaccgccgcggcgaacgacgcgaagaacaacaacaacaacaacaag
cgttcgatggaagccacctccctggcgagcaacaccaaccaccacccggcggcggcggcgaacgagggcatg
ctgtccttctcgtcggcgccgaccgcgcggccgtcggcaggcacgggcgcgccggccaagtcggagtccgac
cactcggacctggacgcgtccgtgcgcgaggtggagagcagccgcgtcgtggcgccgccgccggaggcggag
aagcggccgcggaagcgcgggcggaagcccgcgaacgggcgcgaggagccgctgaaccacgtggaggcggag
cggcagcggcgggagaagctgaaccagcgcttctacgcgctgcgcgccgtggtgcccaacgtgtccaagatg
gacaaggcgtcgctgctcggcgacgccatctcctacatcaacgagctccgcggcaagctgacgtcgctggag
tcggacagggagacgctgcaggcccaggtcgaggcgctcaagaaggagcgcgacgcgcggccgcacccgcac
cccgctgccgggctcggcgggcacgacgccggcgggccgcgctgccacgcggtagagatcgacgccaagatc
ctggggctggaggccatgatccgcgtgcagtgccacaagcgcaaccacccgtcggcgcggctgatgacggcg
ctccgcgagctcgacctggacgtgtaccacgccagcgtctccgtcgtcaaggacctcatgatccagcaggtc
gccgtcaagatggccagccgcatgtactcgcaggaccagctcagcgccgcgctctacagccgccttgcggag
cccgggtctgtcatgggcaggtaa

FIGURE 9 (continued)

**SEQ ID NO 130: Zea mays bHLH4 II translated polypeptide sequence**

mnlwtddnasmmeafmasadlpaypwgapagggnppppqmppamamapgfnqdtlqqrlqamiegsretwty
aifwqssldaatgasllgwgdgyykgcdddkrrhrppltpaaqaeqehrkrvlrelnslisggasaapapap
deaveeevtdtewfflvsmtqsflngsglpgqalfaghhtwiaaglssapcdrarqaynfglrtmvcfpvgt
gvlelgstdvvfqtaetmakirslfgggpgggswppvqpqaapqqqhaaeadqaaetdpsvlwladapvvdi
kdsyshpsaaeisvskppppppppqihfengststltenpspsvhappappappqrqqqnqgpfrrelnfsd
fasnpslaaappffkpesgeilsfgvdsnaqrnpspappaslttapgslfsqsqhtataaandaknnnnnnk
rsmeatslasntnhhpaaaanegmlsfssaptarpsagtgapaksesdhsdldasvrevessrvvapppeae
krprkrgrkpangreeplnhveaerqrreklnqrfyalravvpnvskmdkasllgdaisyinelrgkltsle
sdretlqaqvealkkerdarphphpaaglgghdaggprchaveidakilgleamirvqchkrnhpsarlmta
lreldldvyhasvsvvkdlmiqqvavkmasrmysqdqlsaalysrlaepgsvmgr

## FIGURE 9 (continued)

```
                        1                                                50
     Arath_BHLH4   (1)  --MAPTNVQVTDYHLNQSKTDTTNLWSTDDD------------ASVMEAF
     Brana_bHLH4   (1)  MDDHRNRVSLSPPDAHMSNYFLNQSTATDDDNA----------SASMEAF
  Arath_bHLH4 II   (1)  ---------------MNGTTSSINFLTSDDDAS----------AAAMEAF
     Arath_bHLH6   (1)  ---------MTDYRLQP----TMNLWTTDDN------------ASMMEAF
  Brana_bHLH4 II   (1)  -------------MTEP----TMNLWTTDDN------------ASMMEAF
     Lyces_bHLH4   (1)  ---------MTEYSLP-----TMNLWNNST---------SDDNVSMMEAF
     Vitvi_bHLH4   (1)  ---------MTEYRVP-----TMNLWTDDN-------------ASMMEAF
     Glyma_bHLH4   (1)  -------------R--------MNLWTDENS-------------SVMEAF
  Glyma_bHLH4 II   (1)  ---------MTEYR--------MNLWTDDNS-------------SVMEAF
     Lotja_bHLH4   (1)  ----------------------MNLWSDDNS-------------SVMEAF
     Horvu_bHLH4   (1)  ---------------------------------------------MMEAL
     Zeama_bHLH4   (1)  ----------------------MNLWTDDN-------------ASMMEAF
   Zeama_bHLH II   (1)  ----------------------MNLWTDDNA-------------SMMEAF
       Consensus   (1)           MT           MNLWT D               SMMEAF

                        51                                              100
     Arath_BHLH4  (37)  IGGGSDHSSLFP--------------------------------------
     Brana_bHLH4  (41)  IG-TNHWSQQPSL-------------------------------------
  Arath_bHLH4 II  (26)  IG-TNHHSSLFPP-------------------------------------
     Arath_bHLH6  (26)  MSSS-DISTLWPPAS--------------------------TTTTTATTE
  Brana_bHLH4 II  (22)  MSSSSDISALWPPVT--------------------------TTATASTTA
     Lyces_bHLH4  (28)  MSS-DLSFWATN-----NSTSAAAVGVNSNLPHASSNTPSVFAPSSSTSA
     Vitvi_bHLH4  (24)  ISS-DLSSFSWG---------------------------PSSAASTSTPA
     Glyma_bHLH4  (17)  MSS-SDLSSLWLP---------------------------TPQSAASTTT
  Glyma_bHLH4 II  (21)  MSS-SDLSSLWLA---------------------------TPQSATSTTT
     Lotja_bHLH4  (16)  MTS-SDLSSVWPP---------------------------AP--------
     Horvu_bHLH4   (6)  MASADMPAFPWGAAATPPPP------------------------------
     Zeama_bHLH4  (16)  MASADLPTFPWGAPAGGGNS----------------------------SA
   Zeama_bHLH II  (16)  MASADLPAYPWGA-------------------------------------
       Consensus  (51)  MSS S  SSLW

                        101                                             150
```

**FIGURE 10**

```
     Arath_BHLH4   (49) -------PLPP-------PPLPQV EDNL   L     ---  N NW YA
     Brana_bHLH4   (53) -------PPP--------PSLSQ  ED L   LT    ---S G RW YA
  Arath_bHLH4 II   (38) -------PPQQ-------PPQPQ  ED L   L     ---S G NW YA
     Arath_bHLH6   (49) TTPTPAMEIPA---------QAG     L   L     --- TH GW YA
  Brana_bHLH4 II   (46) PAP------------------AG  E  L   L     --- TN GW YA
     Lyces_bHLH4   (72) STLSAAATVDA------SKSMPF     L   L    D---    TW YA
     Vitvi_bHLH4   (46) PDPSRNLAQSQ------PS-MAV     L   L     ---    SW YA
     Glyma_bHLH4   (39) PGLETTRAPPP-------QSHSLL    L   LT    ---    SW YA
  Glyma_bHLH4 II   (43) PGTAKAPPPPPPPPPPPAQSQSLL    L   LT    ---  C SW YA
     Lotja_bHLH4   (30) -------PPPS------QSSVAVL  D L   L     ---    TW YA
     Horvu_bHLH4   (26) ------------------AAVPA   D L   L  I  --- S  TW YA
     Zeama_bHLH4   (38) AAASPPPQMPA-------AMAPG   D L   L  M  --- S  TW YA
      Phypa_bHLH   (37) -------DG-----------QLHL ESVLLR LHS V ---ESTVDW YA
       Consensus  (101)                      A FNQETLQQRLQALIE   GARESWTYA

                        151                                              200
     Arath_BHLH4   (82) VFWQ  HGFAGEDNNNNNTV L WDGD      E-KSR KKS-------N
     Brana_bHLH4   (85)  FWQI H FDSPAGES--TVIL WGDG  R     E K KQS-------S
  Arath_bHLH4 II   (71)  FWQI H FDSSTGDN--TVIL WGDG        E K ----------N
     Arath_bHLH6   (87)  FWQP Y FS-GA ------VL WGDG        ANPRRRSSS----PP
  Brana_bHLH4 II   (75)  FWQP Y FS-GA ------VL WGDG        A PRQRTSP----PP
     Lyces_bHLH4  (113)  FWQ  VVDFSSP ------VL WGDG        A R L-SV-----SS
     Vitvi_bHLH4   (86)  FWQ -SVDFSGA ------ L WGDG        G R----K-----MT
     Glyma_bHLH4   (79)  FWQ  Y YSSGT ------ L WGDG        V A GKT------PK
  Glyma_bHLH4 II   (90)  FWQ  Y YSSGT ------ L WGDG        D V TKA------PK
      Pissa_bHLH   (89)  FWQT Y YSTSRQ------ L WGDG     D  E A ----------K
     Lotja_bHLH4   (64)  FWQP Y YS-GT ------ L WGDG        A A AKAK-----AK
     Horvu_bHLH4   (55)  FWQ  T A--------GAS L WGDG     CD AD RRQQP-------T
     Zeama_bHLH4   (78)  FWQ  L SA------TGAS L WGDG    CDEDKRKQKPL-------T
   Zeama_bHLH II   (74)  FWQ  L AATGA ------ L WGDG    CD DKRRHRPPL------T
       Consensus  (151) IFWQSS D S   S       LLGWDGYYKGEEDK KRK
```

FIGURE 10 (continued)

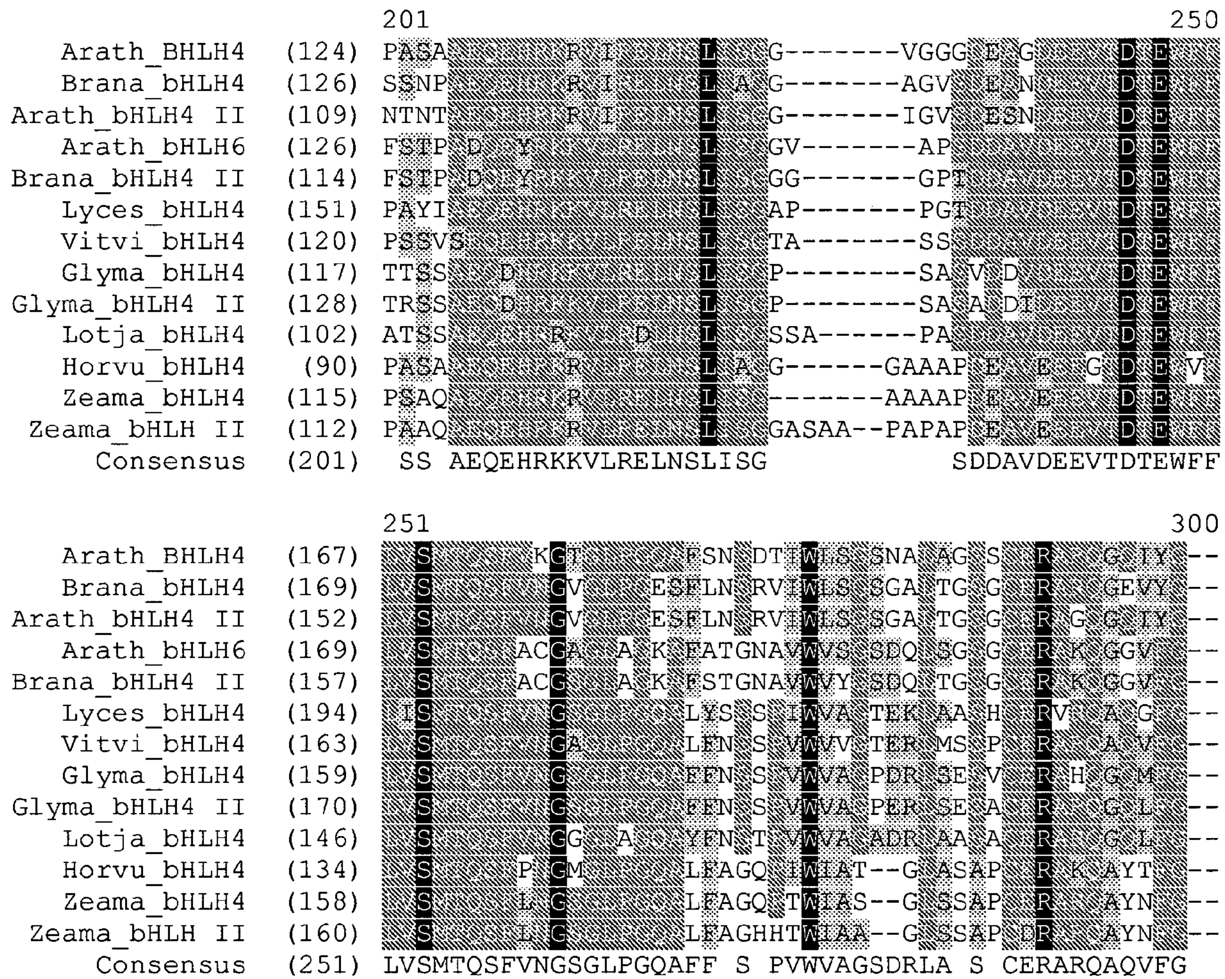


FIGURE 10 (continued)

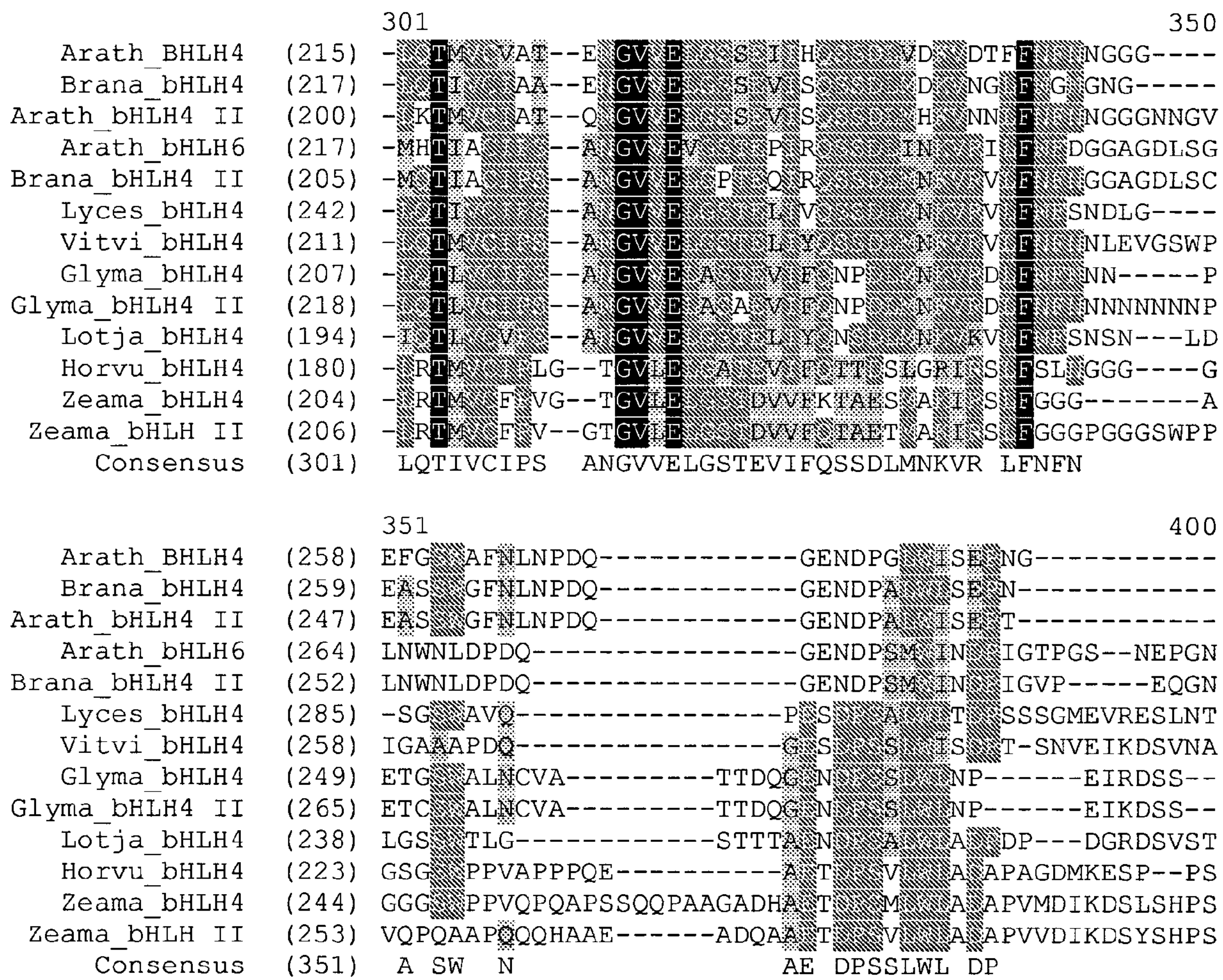


FIGURE 10 (continued)

```
                                401                                               450
      Arath_BHLH4   (285) ------------------VDSGLVAAPVMNNGGNDSTSN-----------
      Brana_bHLH4   (285) ------------------------ITGIEPVQETPAIEN-----------
  Arath_bHLH4 II   (273) ------------------------NTGIESPARVNNGNNSNS--------
      Arath_bHLH6   (296) G------APSSS------SQLFSKSIQFENG-SSSTITENPN----LDPT
   Brana_bHLH4 II   (281) G------APSSS------SQLFAKSIQFENGGSSSTIIENPNP--DPAPA
      Lyces_bHLH4   (318) -------VQTNS----VPSSNSNKQIAYGNE-NNHPSGN------GQSCY
      Vitvi_bHLH4   (291) TA----TGASNP----IGNQQNSKSIQFENP-SSSSLTE-------NPSI
      Glyma_bHLH4   (282) -------TVAPP------NSTVNKTLQFETP-GSSTLTDTP---------
   Glyma_bHLH4 II   (298) -------TVSPP------NSTVNKTMHFETP-GSSTLTETPS--------
      Lotja_bHLH4   (273) VAPT--TASVSIPSHHNNDQSIAKTLQFETP-VSSTLTETPS-------A
      Horvu_bHLH4   (261) VEISV-SKPPPSQPPQIHHFENGSTSTLTENPSLSVHAQQPLPQQQAAAA
      Zeama_bHLH4   (294) AEISV-SKPPPHPP--QIHFENGSTSTLTENPSPSVHAPPPP----PAPA
    Zeama_bHLH II   (297) AAEISVSKPPPPPPPPQIHFENGSTSTLTENPSPSVHAPPAP--------
        Consensus   (401)                                   FE     S  T

                                451                                               500
      Arath_BHLH4   (306) -------SD-----SQPISKLCS--GSS---------------VENPNPK
      Brana_bHLH4   (300) ----------------AADLNFS--SS----------------GLNQN--
  Arath_bHLH4 II   (291) ----NSKSD-----SHQISKLEKNDISS---------------VENQNRQ
      Arath_bHLH6   (329) PSPVHSQTQNPK-FNNTFSRELNFSTSS------------STLVKPRSGE
   Brana_bHLH4 II   (317) PSPVHSQTQNPK-FSNNFSRELNFSTSS------------TTLVKPRPAE
      Lyces_bHLH4   (350) NQQQQKN-PPQQQTQGLFTRELNFSEFG-FDGSSNRGNSSSVSCKPESGE
      Vitvi_bHLH4   (325) MHNPQQQQQ--IHTQGFFTRELNFSEFG-FDGNNGRNGN-LHSLKPESGE
      Glyma_bHLH4   (309) -SAAAVHVPKSN-GQGFFSRELNFSNS----------------LKPESGE
   Glyma_bHLH4 II   (326) -AAAAVHVPNSK-SQGFFPRELNFSNS----------------LKPESGE
      Lotja_bHLH4   (313) VNLTNQPSQNQRNQSSFFSREMNFSEYS---FDAKNGSSNHQHLKPESGE
      Horvu_bHLH4   (310) AQRQNQLQLQHQHNQGPFRRELNFSDFASNP----SVTVTPPFFKPESGE
      Zeama_bHLH4   (337) APQQRQHQHQNQAHQGPFRRELNFSDFASTP----SLAATPPFFKPESGE
    Zeama_bHLH II   (339) ---P-APPQRQQQNQGPFRRELNFSDFASNP----SLAAAPPFFKPESGE
        Consensus   (451)               Q FFSRELNFSE S                KPESGE
```

FIGURE 10 (continued)

```
                          501                                               550
     Arath_BHLH4 (327)    V--L------------------KSC---EMVNFKNGIEN-GQEEDSSN--
     Brana_bHLH4 (314)    ------------------------------GNFKQ--------GSSSN--
  Arath_bHLH4 II (317)    SSCLV---EK-----------DLTF---QGGLLKSNETL-SFCGNESS--
     Arath_bHLH6 (366)    ILNFG--DEG-----------KRSS-GNPDPSSYSG----QTQFENKR--
  Brana_bHLH4 II (354)    ILSFG--DEG-----------KRSS-VNPDPSSYSG----QTQFENKR--
     Lyces_bHLH4 (398)    ILNFG--DST-----------KKSA-SSANVNLFTGQSQ-FGAGEENNN-
     Vitvi_bHLH4 (371)    ILNFG---DS-----------KRSS-CSANGNMFSGHS---QVVAEENK-
     Glyma_bHLH4 (341)    ILSFG---ES-----------KKS---SYNGSFFPG----VVAIEENN--
  Glyma_bHLH4 II (358)    ILSFG---ES-----------KKS---SYNGAFFPG----VVAVEENNNN
     Lotja_bHLH4 (360)    ILSFG---DS-----------KRTP------NFFSGQSQFVPAVEENNNG
     Horvu_bHLH4 (356)    ILNFGADSTS-RRNPSPAPPAATASLTTAPGSLFSQHTATVTAPSNDAK-
     Zeama_bHLH4 (383)    ILSFGADSNA-RRNPSPVPPAATASLTTAPGSLFSQHTATMTAAAANDAK
   Zeama_bHLH II (381)    ILSFGVDSNA---QRNPSP-APPASLTTAPGSLFSQSQHTATAAANDAKN
       Consensus (501)    IL FG    S           KKSS  S  G  FSG S    A E N

                          551                                               600
     Arath_BHLH4 (351)    -----KKRSPVSN--N----------EEGMLSFTSVLPC-----------
     Brana_bHLH4 (324)    -----KKRSPVGK-------------DEEMLSFSTVVRSAAKSV------
  Arath_bHLH4 II (347)    -----KKRTSVSKGSN---------NDEGMLSFSTVVRSAAN--------
     Arath_bHLH6 (396)    -----KRSMVLN--------------EDKVLSFGDKT-------------
  Brana_bHLH4 II (384)    -----KKSIGMS--------------DDKVLTFGTG--------------
     Lyces_bHLH4 (432)    ---KNKKRSATS-RGS---------NEEGMLSFVSGTVVPSSGMK--SGG
     Vitvi_bHLH4 (402)    -----KRRSPTS-RGS---------AEEGMLSFTSGVILPSSCVVK-SSG
     Glyma_bHLH4 (368)    -----KKRSPVS-RSS---------IDDGMLSFTS---LPAANIK--SGS
  Glyma_bHLH4 II (387)    N-KNKKKRSPVVSRSS---------IDDGMLSFTS---LPAANIK--SVN
     Lotja_bHLH4 (390)    -----KKRSPNS-RSS---------NDDGMLSFTSGVILPSSNLK--SST
     Horvu_bHLH4 (404)    --NNPKRSMEATSRASNTNHHQTATANEGMLSFSSAPTTRPSTGTGAPAK
     Zeama_bHLH4 (432)    --NNNKRSMEATSRASNTNHHPAATANEGMLSFSSAPTTRPSTGTGAPAK
   Zeama_bHLH II (427)    NNNNNKRSMEATSLASNTNHHPAAAANEGMLSFSSAPTARPSAGTGAPAK
       Consensus (551)         KKRS  S R S          EEGMLSFTSGV LPSS
```

**FIGURE 10 (continued)**

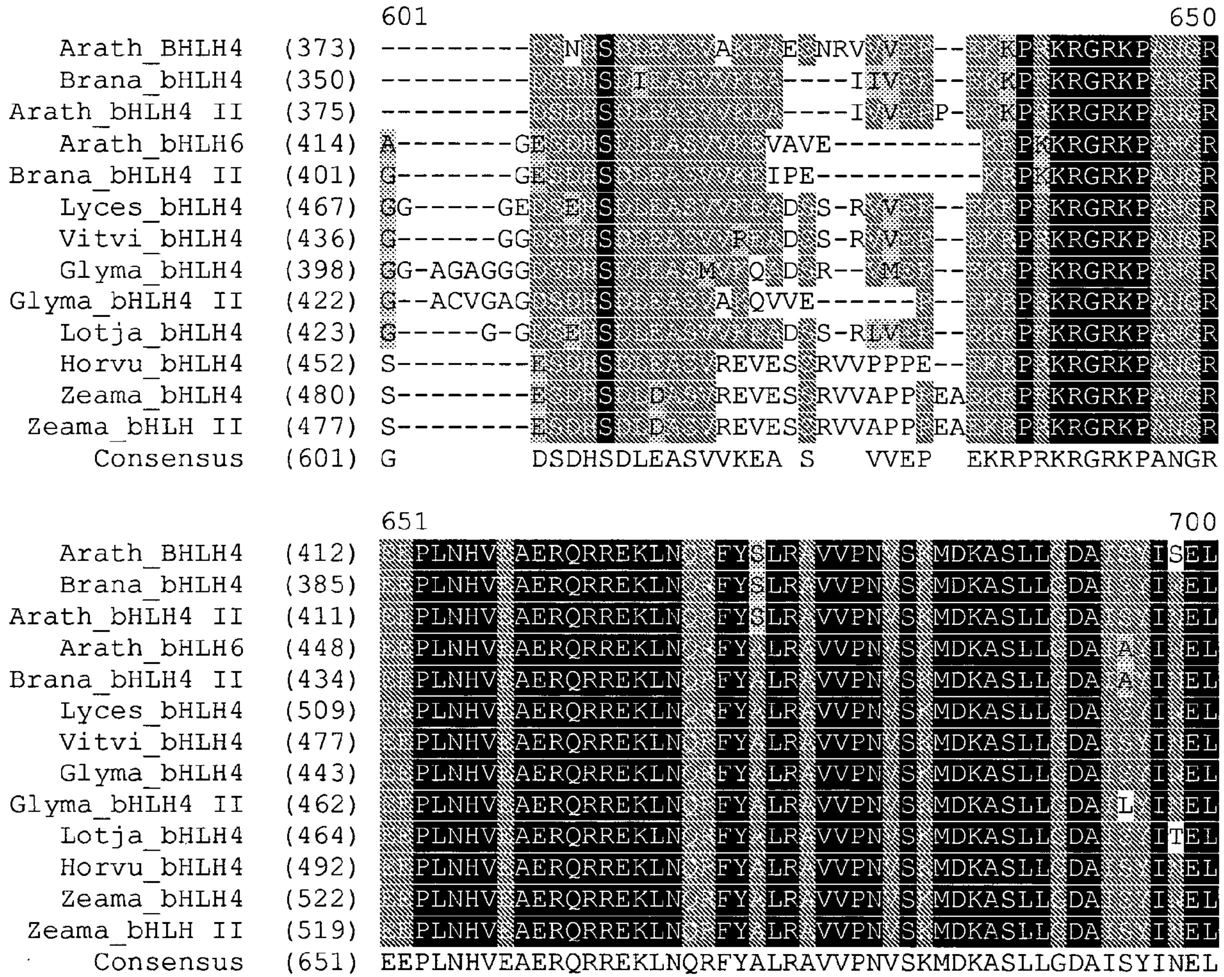


FIGURE 10 (continued)

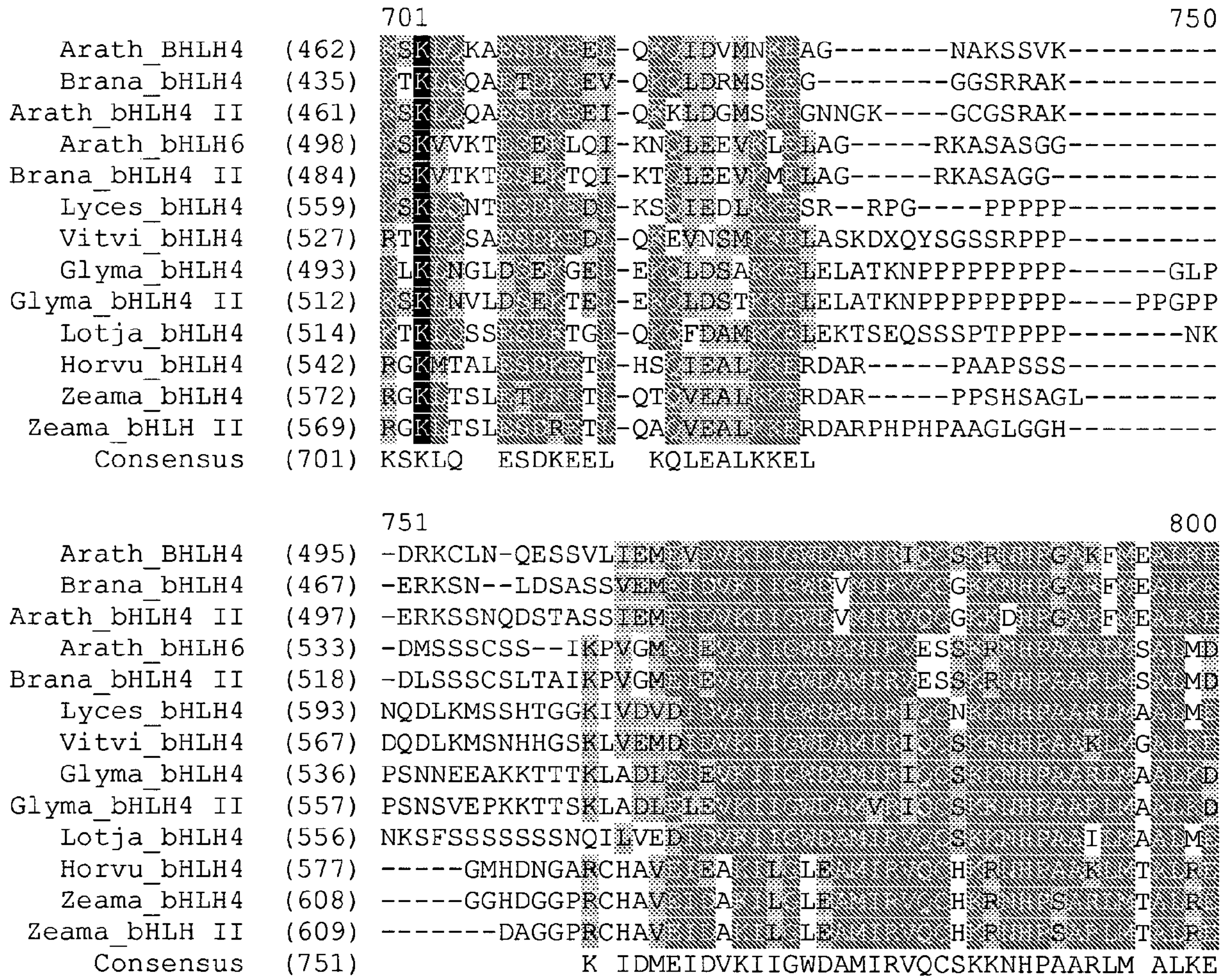


FIGURE 10 (continued)

```
                                801                                              850
     Arath_BHLH4   (543)             N   L                -NQ F  D  KV  TE VGECP--
     Brana_bHLH4   (514)             N   L M              - Q FNHD  KA  ML VGEDS--
  Arath_bHLH4 II   (546)             N   L                - Q FNHD  KV  MT VGENY--
     Arath_bHLH6   (580)        E   N   M                -F I      RAS I  IG-----
  Brana_bHLH4 II   (567)        E   N   M                -F I      RAS I  IG-----
     Lyces_bHLH4   (643)          D H                    -  N  E   RV  T  IAETH--
     Vitvi_bHLH4   (617)          D N                    -      D  RL  S  FADSR--
     Glyma_bHLH4   (586)            H                 N  -NK       L-------------
  Glyma_bHLH4 II   (607)            H                 N  -NK       LS  S  VGDELR-
     Lotja_bHLH4   (606)            N         T          -          RS  S  FGDAP--
     Horvu_bHLH4   (622)          D Y       K I    VA   A-T V S    NA  YGRLAEPGAA
     Zeama_bHLH4   (653)          D Y       K      VA   A-  V   D  SA  Y RLAEPGSA
   Zeama_bHLH II   (652)          D Y       K      VA   A-  M S D  SA  Y RLAEPGSV
       Consensus   (801)   LDLEV HASVSVVNDLMIQQATVKMG SRFYTQEQLRAAL SKIGE

                                851          864
     Arath_BHLH4   (590)   --------------
     Brana_bHLH4   (561)   --------------
  Arath_bHLH4 II   (593)   --------------
     Arath_bHLH6   (624)   --------------
  Brana_bHLH4 II   (611)   --------------
     Lyces_bHLH4   (690)   --------------
     Vitvi_bHLH4   (664)   --------------
     Glyma_bHLH4   (622)   --------------
  Glyma_bHLH4 II   (655)   --------------
     Lotja_bHLH4   (653)   --------------
     Horvu_bHLH4   (671)   MQIR----------
     Zeama_bHLH4   (702)   MGR--RRRAAQLGS
   Zeama_bHLH II   (701)   MGR-----------
       Consensus   (851)
```

**FIGURE 10 (continued)**

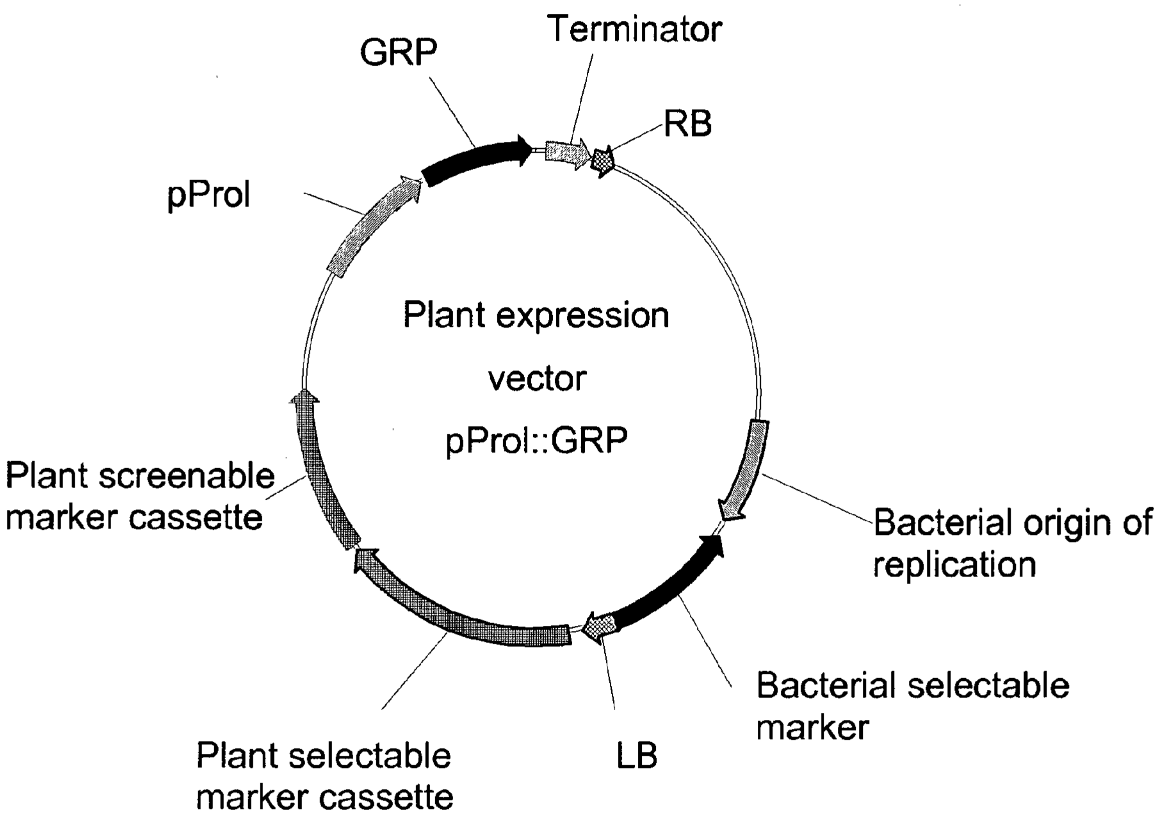
GRP
Terminator
RB
pProl
Plant expression
vector
pProl::GRP
Plant screenable
marker cassette
Bacterial origin of
replication
Bacterial selectable
marker
Plant selectable
marker cassette
LB

FIGURE 11

**SEQ ID NO: 131, *Arabidopsis thaliana* IPT1 nucleic acid sequence**
ATGACAGAACTCAACTTCCACCTCCTCCCAATAATCTCCGATCGCTTCACGACGACGACGACAACATCACCG
TCGTTCTCGTCACATTCTTCTTCTTCTTCTTCTCTTCTCTCTTTCACCAAACGAAGACGAAAACACCAACCT
TTAGTATCATCCATACGCATGGAACAGTCACGGTCACGGAATCGGAAAGACAAAGTCGTCGTCATTTTAGGA
GCAACCGGCGCCGGAAAATCAAGACTTTCCGTCGATCTCGCTACTCGTTTCCCTTCAGAGATCATAAACTCC
GATAAAATCCAAGTCTACGAAGGATTAGAGATCACAACGAATCAGATTACGTTACAAGACCGTCGCGGCGTT
CCTCACCATCTCCTCGGCGTCATCAACCCCGAACACGGCGAACTAACCGCCGGAGAGTTTCGCTCCGCCGCT
TCAAACGTCGTCAAAGTGATAACTTCTCGTCAAAAGGTTCCGATTATCGCCGGTGGATCTAACTCTTTCGTC
CACGCACTCTTAGCTCAACGATTCGACCCAAAGTTCGATCCTTTTTCATCCGGGTCGTGTTTAATCAGCTCC
GATTTGCGTTACGAGTGTTGTTTCATCTGGGTCGATGTATCGGAGACTGTTCTCTACGAGTATCTTCTCAGA
AGAGTCGACGAAATGATGGATTCAGGTATGTTCGAAGAGCTGTCTAGATTCTACGACCCGGTTAAATCCGGT
TTAGAAACCCGGTTTGGGATTAGGAAAGCTATAGGTGTACCGGAGTTTGACGGTTACTTCAAAGAGTATCCA
CCGGAGAAGAAGATGATAAAGTGGGACGCTTTAAGAAAAGCGGCGTACGATAAGGCGGTTGATGATATCAAA
AGGAACACGTGGACGTTAGCGAAGAGACAAGTGAAGAAGATTGAGATGCTAAAAGACGCTGGTTGGGAAATA
GAAAGAGTTGATGCAACGGCGTCGTTTAAAGCAGTGATGATGAAGAGTTCGTCGGAGAAGAAGTGGAGAGAG
AATTGGGAAGAGCAAGTGTTGGAGCCAAGCGTAAAGATTGTGAAGCGGCATTTGGTGCAAAATTAG

**SEQ ID NO: 132, *Arabidopsis thaliana* IPT1 polypeptide sequence**
MTELNFHLLPIISDRFTTTTTTSPSFSSHSSSSSSLLSFTKRRRKHQPLVSSIRMEQSRSRNRKDKVVVILG
ATGAGKSRLSVDLATRFPSEIINSDKIQVYEGLEITTNQITLQDRRGVPHHLLGVINPEHGELTAGEFRSAA
SNVVKVITSRQKVPIIAGGSNSFVHALLAQRFDPKFDPFSSGSCLISSDLRYECCFIWVDVSETVLYEYLLR
RVDEMMDSGMFEELSRFYDPVKSGLETRFGIRKAIGVPEFDGYFKEYPPEKKMIKWDALRKAAYDKAVDDIK
RNTWTLAKRQVKKIEMLKDAGWEIERVDATASFKAVMMKSSSEKKWRENWEEQVLEPSVKIVKRHLVQN

**SEQ ID NO: 133, *Oryza sativa* Prolamin promoter**
CTTCTACATCGGCTTAGGTGTAGCAACACGACTTTATTATTATTATTATTATTATTATTATTTTACAAA
AATATAAAATAGATCAGTCCCTCACCACAAGTAGAGCAAGTTGGTGAGTTATTGTAAAGTTCTACAAAGCTA
ATTTAAAAGTTATTGCATTAACTTATTTCATATTACAAACAAGAGTGTCAATGGAACAATGAAAACCATATG
ACATACTATAATTTTGTTTTTATTATTGAAATTATATAATTCAAAGAGAATAAATCCACATAGCCGTAAAGT
TCTACATGTGGTGCATTACCAAAATATATATAGCTTACAAAACATGACAAGCTTAGTTTGAAAAATTGCAAT
CCTTATCACATTGACACATAAAGTGAGTGATGAGTCATAATATTATTTTCTTGCTACCCATCATGTATATA
TGATAGCCACAAAGTTACTTTGATGATGATATCAAAGAACATTTTTAGGTGCACCTAACAGAATATCCAAAT
AATATGACTCACTTAGATCATAATAGAGCATCAAGTAAAACTAACACTCTAAAGCAACCGATGGGAAAGCAT
CTATAAATAGACAAGCACAATGAAAATCCTCATCATCCTTCACCACAATTCAAATATTATAGTTGAAGCATA
GTAGTA

**SEQ ID NO: 134, prm03095**
GGGGACAAGTTTGTACAAAAAAGCAGGCTTCACAATGACAGAACTCAACTTCCAC

**SEQ ID NO: 135, prm03096**
GGGGACCACTTTGTACAAGAAAGCTGGGTAACTAATTTTGCACCAAATG

**SEQ ID NO 136: Arabidopsis thaliana Arath_IPT4 nucleic acid sequence**
atgaagtgtaatgacaaaatggttgtgatcatgggtgccaccggttctggcaagtcatcactctctgttg
atctcgctttacatttaaagccgagatcatcaactctgacaaaatgcagttctacgatggcttgaagat
caccacgaatcaatcgaccattgaagatcgacgtggagtgccacatcaccttctcggtgaactaaacccg
gaggctggagaagtcacagcggcagaatttcgcgttatggcggctgaagccatctccgagattactcaac
gtaaaaagctcccaatccttgccggtggatccaactcatacattcatgctctccttgcaaaatcttatga
ccctgaaaactatccgttttctgatcacaagggctcaatctgctccgagttgaaatatgattgttgtttc
atttggatagatgtggatcagtctgtgttattcgagtatctttctttacgtttggatcttatgatgaagt
caggtatgttcgaggagatcgctgagttccaccgctctaagaaggccccgaaagagccattggggatatg
gaaggctataggagtgcaagagtttgatgactacctcaaaatgtacaagtgggacaatgacatggataaa

**FIGURE 12**

tgggaccctatgagaaaggaggcttatgagaaggcggtgagagccatcaaagaaaacacatttcagctca
caaaggatcaaatcacgaagatcaacaagctgagaaatgccgggtgggacataaagaaggtggatgctac
agcatcgtttcgagaggcaattagggcagccaaggaaggcgaaggtgtagccgagatgcagagaaagata
tggaacaaggaagtgttggaaccgtgtgtgaagattgtcaggagccacttggaccaaccgatcaactatt
attattattactttatttactaaaaagatttttaagtcttaactag

**SEQ ID NO 137: Arabidopsis thaliana Arath_IPT4 translated polypeptide sequence**
MKCNDKMVVIMGATGSGKSSLSVDLALHFKAEIINSDKMQFYDGLKITTNQSTIEDRRGVPHHLLGELNP
EAGEVTAAEFRVMAAEAISEITQRKKLPILAGGSNSYIHALLAKSYDPENYPFSDHKGSICSELKYDCCF
IWIDVDQSVLFEYLSLRLDLMMKSGMFEEIAEFHRSKKAPKEPLGIWKAIGVQEFDDYLKMYKWDNDMDK
WDPMRKEAYEKAVRAIKENTFQLTKDQITKINKLRNAGWDIKKVDATASFREAIRAAKEGEGVAEMQRKI
WNKEVLEPCVKIVRSHLDQPINYYYYYFYLLKRFLSLN

**SEQ ID NO 138: Arabidopsis thaliana Arath_IPT6 nucleic acid sequence**
atgcaacaactcatgaccttgttatcaccaccactctctcattcttctctccttcccaccgtcactacca
aattcgggtcaccacgattagtcactacgtgcatgggccatgcagggcgtaaaaatatcaaggataaggt
ggttctcatcacaggtacaacaggcacaggcaagtcacgcctctcagtcgatcttgccacccgtttttt
cccgccgagatcataaactcggacaaaatgcaaatctacaagggattcgagattgtcacaaatctaatcc
cactgcatgagcaaggaggagtcccgcaccatcttctaggtcagttccacccacaagacggtgaactcac
ccctgcagagttccgttctttggcgacactgtccatctctaaactaatttctagcaagaaactcccgatt
gtagttggtggatccaactccttcaatcacgctctactcgccgagcgttttgacccggatattgatccat
tctctcccggatcgagtctttcaacgatctgctctgacctaaggtacaaatgttgcatcttatgggttga
tgttttagagccggttctgttccaacacttgtgcaatcgtgtcgaccaaatgatcgagtcgggattggtc
gagcagcttgccgaattgtacgaccctgttgtagattcgggtcgacgactaggggttcggaagacgatag
gagtagaggagttcgaccgatactttagagtataccctaaggagatggacaagggaatttgggacttagc
gagaaaggcggcgtacgaggagacagtgaaggggatgaaagagaggacatgtcggttggtgaagaagcag
aaagagaagatcatgaagctgataagaggtggttgggagattaagaggcttgacgctacggcggcaatta
tggctgagctgaatcaaagtacggcaaagggagaaggaaagaatgggagagagatttgggaaaaacacat
tgtggatgaaagtgtcgagattgtcaagaagttttgttggaagtttag

**SEQ ID NO 139: Arabidopsis thaliana Arath_IPT6 translated polypeptide sequence**
MQQLMTLLSPPLSHSSLLPTVTTKFGSPRLVTTCMGHAGRKNIKDKVVLITGTTGTGKSRLSVDLATRFF
PAEIINSDKMQIYKGFEIVTNLIPLHEQGGVPHHLLGQFHPQDGELTPAEFRSLATLSISKLISSKKLPI
VVGGSNSFNHALLAERFDPDIDPFSPGSSLSTICSDLRYKCCILWVDVLEPVLFQHLCNRVDQMIESGLV
EQLAELYDPVVDSGRRLGVRKTIGVEEFDRYFRVYPKEMDKGIWDLARKAAYEETVKGMKERTCRLVKKQ
KEKIMKLIRGGWEIKRLDATAAIMAELNQSTAKGEGKNGREIWEKHIVDESVEIVKKFLLEV

**SEQ ID NO 140: Humulus lupunus Humlu_IPT like nucleic acid sequence**
agccggaagaagaccagggtgggcacgcatggactacgcatccgttgccatggctgccgcgcccaccaca
acaaccactaccaacgtatcactccgtcgccagcgacaccggaaagagaagcttctcgttctaatgggcg
ccaccggtaccgggaagtcccgtctctccatcgacctcgcggcgcacttccctctcgaagtcataaactc
ggataaaatgcaggtttataaaggactggatatcacgacgaacaagatatcggtaccggaccggggcggc
gtgccacaccatctcctcggtgaggttgacccggctcgaggtgagttgactcctgccgatttcaggtctt
tggctggaaaagccgtgtctgaaatcactgggaggagaaagcttcccgtcctggtgggtgggtccaactc
gttcatacatgcgctgttggtggaccggtttgactcgtcgggcccgggcgtgttcgaggaagggtcccac
tcggtggtgtcttctgagttgagatacgactgttgctttctctgggttgacgtgtcggtaaaggtattga
ccgattacttggcgaagcgagtcgacgacatgttggagctggggatgttcgatgagttggccgagttcta
tagcccggaggacgaggaccacgacgaagactctgcgactcggaccgggttgagaaaagccatcggagtt
cccgagtttgaccggtatttcgaaaagttcagacctggcgacgtggaggggggaggaccccgggagggatc
gggtgcggaggggcgcattcgaagaggcggtgagggcgatcaaggagaacacgtgtcatctagcgaagag

## FIGURE 12 (continued)

acaaatagggaagatcttacgattaaaaggcgctgggtgggacctacggcggctggacgcaacagagtcg
ttccgggcggcgatgacgtcagactccggcgagaagtgcacggagatttgggagaagcaggtattggaac
caagcgtgaagattgtgagtcgcttcttggacgagtaggttttgccagcaatttccagctcatttttct
tttatttttttgagtttgttaaaaaaaaattttggtagttagttttcccacggaaaaattttcaacttgt
cacaattacaaattaat

**SEQ ID NO 141: Humulus lupunus Humlu_IPT like translated polypeptide sequence**
MDYASVAMAAAPTTTTTTNVSLRRQRHRKEKLLVLMGATGTGKSRLSIDLAAHFPLEVINSDKMQVYKGL
DITTNKISVPDRGGVPHHLLGEVDPARGELTPADFRSLAGKAVSEITGRRKLPVLVGGSNSFIHALLVDR
FDSSGPGVFEEGSHSVVSSELRYDCCFLWVDVSVKVLTDYLAKRVDDMLELGMFDELAEFYSPEDEDHDE
DSATRTGLRKAIGVPEFDRYFEKFRPGDVEGEDPGRDRVRRGAFEEAVRAIKENTCHLAKRQIGKILRLK
GAGWDLRRLDATESFRAAMTSDSGEKCTEIWEKQVLEPSVKIVSRFLDE

**SEQ ID NO 142: Lotus japonica Lotja_IPT like nucleic acid sequence**
ctctctttcttcttcttcctatgagactttcctctctctcacctcacccccaccaccaccaccactacac
cacacactaccattaccattaccaccacccctcttcactcgccatggacggccaccgccgcatagacaag
gtggttgtcatcatgggcgccaccggctccggcaaatcccgcctctccatcgacctcgccaccctctttc
ccttctcagagatcatcaactccgataaaatgcaagtctacaaaggactcgacaccaccaccaacaagat
cccacctcaccaacgcaacaacgtcccccaccacctcctcggcgacgtcgacccttctctcggtgatttc
acccccctccgacttccgccgccgcgccggtgacctcatctccgacatcacccgccgcagaaagctcccca
tcttcgtcggcggctccaactccttcgtccacgccctccttgtcgaccgattcgaccccgagtccaacgt
cttccgcgacgattcaccctcgccggtttcctccgagttgagataccggtgctgcttcctctggatggac
attgcgttccccgtgctgtcggagtatttgctgaagcgagtcgacgacatgcttgactcgggaatggtgg
acgagttggctcagttcttcgactcggacacagcgaaccagaccgggttgagaaaagccatcggtgtacc
cgagttcgaccggtttttcaaggatccggtgcgggagggtgcagcgtacgaggaagcggtgagggcgatt
aaggagaacacgtgtcagctagcgaagaggcagattgggaagatcatgcggttaaaaagagcagggtggg
acttacggaggatcgacgccacggaagcgttccgggtggcgcttgtggcagacggcggcggagagagatt
ttccgatgaatggaagaggcaagtgttggaaccaagcgtgaagattgtgaagcggttcttgatggagtag
gtttgggttttccagctaaatccagctattccacttcctctttaaattttctttttttttcttcttct
ctttttttgcgtttatttcacccccaaataagacaaaaaatgaaagtgggaaattgggggaaaatgtac
aaaaatgggagtggaaattgttaatttgaatccactagtttggttgagtgagtgatttcagagagggaat
gagcatggatcattaaatttttggtcttggagttactagcaactagcaactagcaactagcaacaaggct
tggctcatgtgagattccacaattttctcaaggcaatagaaatggatatttagtataaaaaaaaaaaaa
aaaaa

**SEQ ID NO 143: Lotus japonica Lotja_IPT like translated polypeptide sequence**
MRLSSLSPHPHHHHHYTTHYHYHYHHPSSLAMDGHRRIDKVVVIMGATGSGKSRLSIDLATLFPFSEIIN
SDKMQVYKGLDTTTNKIPPHQRNNVPHHLLGDVDPSLGDFTPSDFRRRAGDLISDITRRRKLPIFVGGSN
SFVHALLVDRFDPESNVFRDDSPSPVSSELRYRCCFLWMDIAFPVLSEYLLKRVDDMLDSGMVDELAQFF
DSDTANQTGLRKAIGVPEFDRFFKDPVREGAAYEEAVRAIKENTCQLAKRQIGKIMRLKRAGWDLRRIDA
TEAFRVALVADGGGERFSDEWKRQVLEPSVKIVKRFLME

**SEQ ID NO 144: Medicago truncatula Medtr_IPT like nucleic acid sequence**
atgccaacaacaacaacaacaccttcatttctctcacctcctcactctcaacgtcattatcataaaccca
ttcaatttcaccactattcacacacccttttactccttcctcttcctccgccacccacaccaggcggcg
gccccactgccctcgcatggaaatctccccctcacgccaccgtcggaaagacaaagtcctcattatagtc
ggtgcaaccggctccggaaaatcacgtctctccgttgaactcgccaccctcttcccttactcagaaataa
ttaattccgacaaaatccaagtgtacagaggactcgatatcaccaccaacaagatcccatttcatcaacg
caacaacgttccacatcatcttctcggcgacattgatccttctcacggtgagttctcaccttcagatttc
cgccgccacgccggagatatcatctccgatattacttcccggagaaaacttcccattatcgttggtgggt
ccaactcattcattcacgctcttcttgtagaacgattcgaccctgagtcaaacgttttcgacgagtcatc

**FIGURE 12 (continued)**

atcattgtcaacatcgatatcctcggatttaaggtacaaatgttgtttctttggatggatatttcgttt
cctgtgttgtcggaatatttgctgaaacgagtcgatgatatgtttgactcgggaatggtgaacgagttag
ctgagttttatgaaccggatgcagataaccaaaccggtttaagaaaagcaatcggtgtacccgagttcga
ccggtttttaaacaatatccaccacaggttggaccagatgaatctgaacgtcataatccaatgcgggaa
ggtgcatatattgaagcagtgaaggcgattaaagataacacgtgtcagttagctaagagacagataggga
agatcttacggttaaaaagagctgggtgggacctacaacggattgatgccacggaggcgtttagggcggt
gctgacgtcagagtctaacggcggtggagaagaatttaccggtgtatggaaaaaacaagtattggaacca
agcgtgaagattgtgaagcgtttcttgatggagtaggtcttccagctaaatccagcttagctaatccaac

**SEQ ID NO 145: Medicago truncatula Medtr_IPT like translated polypeptide sequence**
MPTTTTTPSFLSPPHSQRHYHKPIQFHHYSHTLFTPSSSSATHTRRRPHCPRMEISPSRHRRKDKVLIIV
GATGSGKSRLSVELATLFPYSEIINSDKIQVYRGLDITTNKIPFHQRNNVPHHLLGDIDPSHGEFSPSDF
RRHAGDIISDITSRRKLPIIVGGSNSFIHALLVERFDPESNVFDESSSLSTSISSDLRYKCCFLWMDISF
PVLSEYLLKRVDDMFDSGMVNELAEFYEPDADNQTGLRKAIGVPEFDRFFKQYPPQVGPDESERHNPMRE
GAYIEAVKAIKDNTCQLAKRQIGKILRLKRAGWDLQRIDATEAFRAVLTSESNGGGEEFTGVWKKQVLEP
SVKIVKRFLME

**SEQ ID NO 146: Morus alba Moral_IPT like nucleic acid sequence**
gcccgggcaggtccggccactacctctggcccacgagacaggtgggcgcggatggagttttcctcctccg
ccgcccgccgccaccgccaccatcccaaggacaagctcctcgtcatcatgggcgccaccggcaccggcaa
atcccgactctccgtcgagctcgccacccacttcaacggcgagatcatcaactccgacaaaatgcaagtc
tacaagggactcgacacaacgaccaacaagattcccctcgacgaccgcctcggtgtcccccaccaccttc
tcggcgaggtcgactcggaagctcacggcgagttgactcgctccgagttccgctccttggccggaaaggc
cgtgtcggaaatcaccgccaggaggaaactcccagtcctcgccggcggctccaactccttcattcacgcc
ctgctcgtggaccggttcgacccggaatacgacgttttcgacgagcggtccgaccaaccggcggattcgt
cgaaggttctcctccgctacaactgttgcttcctttgggtggatgtttcgttgagggttttggaagatta
tttattgaagcgagtcgatgacatgctcaactcggggatgttcgacgagttggccgagttctacgacccg
gaggaggatcacggaccggcgaactggaccggattgaggaaggctataggcgtgcccgagtttgaccggt
atttcgagaggtgcagaccgggggagaaaggagagtgggatcgggtgcggagggaagcatacgaggaggc
ggtgagggaaataaaggagaacacgtgtcagctggcgaagagacagattggaaagattctgagattaaag
aagttggggtgggacctacggaggttggacgcgacggaggcgtttagggcggtgatgacgtcagattccg
gtaagaggtgttcggaaatttgggagaggcaggtattggaaccaagcgtgaagattgtgaagcgcttctt
ggacgagtagtaggttttgccagcactttccagctaatttttatttttattatttttttgttgttttttt
aaaaaaatttggtagttagttttccaacttcaacttcagctcaaaaaaaaaaaa

**SEQ ID NO 147: Morus alba Moral_IPT like translated polypeptide sequence**
MEFSSSAARRHRHHPKDKLLVIMGATGTGKSRLSVELATHFNGEIINSDKMQVYKGLDTTTNKIPLDDRL
GVPHHLLGEVDSEAHGELTRSEFRSLAGKAVSEITARRKLPVLAGGSNSFIHALLVDRFDPEYDVFDERS
DQPADSSKVLLRYNCCFLWVDVSLRVLEDYLLKRVDDMLNSGMFDELAEFYDPEEDHGPANWTGLRKAIG
VPEFDRYFERCRPGEKGEWDRVRREAYEEAVREIKENTCQLAKRQIGKILRLKKLGWDLRRLDATEAFRA
VMTSDSGKRCSEIWERQVLEPSVKIVKRFLDE

**SEQ ID NO 148: Oryza sativa Orysa_IPT like nucleic acid sequence**
atgaccagcgttgccaccaggattgccacgctcgtgcgggccgcggcggcggcgagccggccattgcggc
tccaccgccggcccggcggcgaggatacgaggatggtggtgatcgtcggcgccacgggcaccgggaagac
caagctgtccatcgacgccgccaaggtgatcggcggcgaggttgtcaacgccgacaagattcagctctat
gacggcctcgacgtgaccaccaacaaggtgagcctcgccgaccgccgtggcgtgccgcaccacctcctcg
gagccatccgccccgaggccggcgagctcccgccgtcgtccttccggtccctcgccgccgccacggccgc
gtcgatcgcggcgaggcggctcgtgccggtcatcgccggtgggtcgaactccctcatccacgccctcctc
gccgaccacttcgacgcctccgctggcgatcccttctcccccgccgccgccttccgccactaccgcccgg
cgctccggttcccgtgctgcctgctctgggtccacgtcgatgaggcgctcctcgacgagtacctcgaccg

## FIGURE 12 (continued)

ccgcgtggacgacatggtggacgctggcatggtcgaggagctccgggagtacttcgccacgacaaccgcc
gcggagcgcgccgcgcactccgggctgggcaaggccatcggcgtccccgagctcggcgactacttcgccg
ggcgcaagaccttctccgaggcgatcgacgacatcaaagccaacacccgcgtcctcgccgccgcgcaggt
gtccaagatccgccgcatgtccgacgcctggggctggcccatccaccgcctcgacgcctccgacacagtc
cgcgccaggctcacgcgggcgggctccgccgccgagtccgcctcctgggagcgcgacgtgcgcggcccag
gcctcgccaccatccgcagcttcctcgccgatcagtcaccgccaccgcgcagcgagggcaccaacgacta
cctgtacgccatggagacggaaccagagccgccgccgccgccgacgttgccgccgcggctgctccggttg
ccgcggatgcagtactgcgacatggtggggtga

**SEQ ID NO 149: Oryza sativa Orysa_IPT like translated polypeptide sequence**

MTSVATRIATLVRAAAAASRPLRLHRRPGGEDTRMVVIVGATGTGKTKLSIDAAKVIGGEVVNADKIQLY
DGLDVTTNKVSLADRRGVPHHLLGAIRPEAGELPPSSFRSLAAATAASIAARRLVPVIAGGSNSLIHALL
ADHFDASAGDPFSPAAAFRHYRPALRFPCCLLWVHVDEALLDEYLDRRVDDMVDAGMVEELREYFATTTA
AERAAHSGLGKAIGVPELGDYFAGRKTFSEAIDDIKANTRVLAAAQVSKIRRMSDAWGWPIHRLDASDTV
RARLTRAGSAAESASWERDVRGPGLATIRSFLADQSPPPRSEGTNDYLYAMETEPEPPPPPTLPPRLLRL
PRMQYCDMVG

**SEQ ID NO 150: Petunia hybrida Pethy_IPT like nucleic acid sequence**

atgttaattgtagtacatattattagcatcacacgcatcatattcatcaccttaacccataatcatctcc
atttccttatgtttagatcattatcatacaatcacaagcacctcaaattccttacaaacccgaccacacg
ggtactccgaagaaacatgtcgtcatccactgtagtaacaatacccggccccacacaaaaaaacaaaaac
aaaatcatagtaataatgggtgcaacaggttcaggaaaatcaaaactctcaatagacctcgtcacacgtc
actatcctttttccgaaatcattaactccgacaaaatccaaattaccaaaggtttaaacataaccacaaa
caaaatcactgtacccgaccgacgtggcgtagttcatcatttactcggcgagattgaccccgactttaac
ttttctccttctcatttccggtcaattgctggtcaacgcattaactccattattaatcgccataaactcc
cattcctcgttggtgggtccaactcatatatctacgctttattaacaaaccggttcgacccggattttaa
ccctgattcaaacccggttcattttatatccaacgagttacgctacaactgttgttttatttgggtcgat
gtattaaacccggttttgaatgagtatttggataaacgggtcgatgagatgatgaactcgggtatgtatg
aagaactggaacagtttttaaagaaaacaggttttcggatccgggtttggaaccgggtcgggccaccgg
gttgaggaaagcgataggggtaccggaaatggagaggtatttaagaagagctgtacgtatgaggaagca
gtgagggaaataaaagaaaacacgtggcggttagcgaagaagcagatgtggaagatccaacggttgagag
aagcagggtgggacctacaaagagtagatgccacggaggcatttgtggaggcgatgagtaataagaagga
aaagggaattatttgggaaaaacaagtagtggaaccaagtgtcaagattgtgaaccgtttttgttggac
tga

**SEQ ID NO 151: Petunia hybrida Pethy_IPT like translated polypeptide sequence**

MLIVVHIISITRIIFITLTHNHLHFLMFRSLSYNHKHLKFLTNPTTRVLRRNMSSSTVVTIPGPTQKNKN
KIIVIMGATGSGKSKLSIDLVTRHYPFSEIINSDKIQITKGLNITTNKITVPDRRGVVHHLLGEIDPDFN
FSPSHFRSIAGQRINSIINRHKLPFLVGGSNSYIYALLTNRFDPDFNPDSNPVHFISNELRYNCCFIWVD
VLNPVLNEYLDKRVDEMMNSGMYEELEQFFKENRFSDPGLEPGRATGLRKAIGVPEMERYFKKSCTYEEA
VREIKENTWRLAKKQMWKIQRLREAGWDLQRVDATEAFVEAMSNKKEKGIIWEKQVVEPSVKIVNRFLLD

**SEQ ID NO 152: Populus tremuloides Poptr_IPT like nucleic acid sequence**

cttactctgtttttttttttttcgttgcctatgaaaattcccttcccaaagagtaccaatcagcctcttt
acactgcccttaaaacccaaccacttcaccccattaacatttctatacccttcaataagccacgcccacc
accaatagcagtccgtatggacacggactcctctaccaccacctccaccgccgtctaccgccataaaaaa
gacaagattctcgtaataatgggagcgactgggtgtggcaaaacaagggtatctattgatctagctacac
gcttccaatccgaaatcatcaactccgacaaaatgcaagtctacgaaggtcttgacatcaccaccaacaa
aatcaccattcaagaccgtctaggtgttcctcaccatttactcggtgagttcgacccggatgatggtgag
ttgactccctccgagtatcggttagctggtggattggctatctcaggtattgtttcaaggcaaaatcttc

FIGURE 12 (continued)

ctattgtggttggtgggtccaactcacttattcacgctttggttgttgaccggtttaatcccgagttaaa
cgtttttgatgggtgtaacccagtttcaacccagttaagatataactgttgtttttgtgggtggatgtg
tcattacctgttttgtgcgattacttgtgtaagcgagtcgacgaaatgctcgactccgggatgctcgatg
agctgtcagagtattatggctcagttgatgcagcgagtcaaatcgggttgaggaaagcgattggggtgcc
tgagtttgatcggtatttcaaaaagtacccacctgggtctgggtgtggcagaggtattggtgtggaatgg
gatcgggtacggaggggagtatacgaggtctgtgtgagggagataaaggagaacacgtgtcagcttgcga
aaaggcagatcggcaagatcttgagattaaaagggcagggtgggacctaaaaagagttgatgcgactga
gagctttagggaggtgatgacggtgacgtcagatgatcatatcaagaaaagaaagaagaagaggtggatg
gaggtctgggggagagatgtgatggagccaagcatgaaaattgtgaaacgcttcttggaggaggagtagg
agtaggttttacagtcagtcagccagtcaatccatcaatcaatcaatccagctttttcccgctaccgtt
ctggttttc

**SEQ ID NO 153: Populus tremuloides Poptr_IPT like translated polypeptide sequence**

MKIPFPKSTNQPLYTALKTQPLHPINISIPFNKPRPPPIAVRMDTDSSTTTSTAVYRHKKDKILVIMGAT
GCGKTRVSIDLATRFQSEIINSDKMQVYEGLDITTNKITIQDRLGVPHHLLGEFDPDDGELTPSEYRLAG
GLAISGIVSRQNLPIVVGGSNSLIHALVVDRFNPELNVFDGCNPVSTQLRYNCCFLWVDVSLPVLCDYLC
KRVDEMLDSGMLDELSEYYGSVDAASQIGLRKAIGVPEFDRYFKKYPPGSGCGRGIGVEWDRVRRGVYEV
CVREIKENTCQLAKRQIGKILRLKGAGWDLKRVDATESFREVMTVTSDDHIKKRKKKRWMEVWGRDVMEP
SMKIVKRFLEEE

**SEQ ID NO 154: Populus tremuloides Poptr_IPT like II nucleic acid sequence**

ctcaacgacaacgtctcaggaaattcaacaatgaccatgaggctttctttgaccgcctacaaacaagtac
agcctcgtgggaatttccaagggggctaaacatgaaacctttctttcgtcgaaaagataaggttgtgtt
cgttgttggaccgactggcacaggcaaatcaaggctggctattgacctggcaacccgtttcccagccgag
gttgtcaattgtgacaaaatgcaagtttataaaggcctcgatatagtcacaaacaaggtcactgaagagg
agtgtcgcggtgtgcctcatcatttacttggcatagcagaccctaatgcaaatttcacttccgatgactt
caggcaccatgcatcacttgttgtcgaatcaatcgtcacacgtgatcggctaccgatcatcgccggtgga
tcaaattcctacatcgaggctttagcaaatgatgatcctgaatttcgattaaggtatgaatgttgttttc
tttgggtggacgtgtcactcccaatactttattcattcgtatcagagcgggtcgatcgaatggtggaagc
aggcttgattgatgaggtgagagatatgtttgatcctaataaatttgatgattattcacaaggaatcaaa
cgggcaattggggttcctgaattggatcatttttacgaaatgaggcgattgtggatgctaaaactcgta
gaaagcttcttgatgaggccattgataaaattaaagaaaatacttgtatgctagcctctcgacaattaca
aaaaatccatcggcttcatagcatatggaattggaacgtgcaccgaatcgatgccaccccagttttccta
acgagtggaaaggaggttgacaatctttgggacaaacttgtggcaggaccaagcaccatgattgtgaacc
agtttctttgtgacaaaaattatgcatctcccattataccatcagaatcaatcaagatggagccaatctc
tgtcccggccctggctgttggcgccactcgatagaggcctgtcaggtcatcaaaatcaccatcaatcaca
ctagtgctattatgccaatgggcgcttttgccacgtggcagggt

**SEQ ID NO 155: Populus tremuloides Poptr_IPT like II translated polypeptide sequence**

MTMRLSLTAYKQVQPRGNFQGGLNMKPFFRRKDKVVFVVGPTGTGKSRLAIDLATRFPAEVVNCDKMQVY
KGLDIVTNKVTEEECRGVPHHLLGIADPNANFTSDDFRHHASLVVESIVTRDRLPIIAGGSNSYIEALAN
DDPEFRLRYECCFLWVDVSLPILYSFVSERVDRMVEAGLIDEVRDMFDPNKFDDYSQGIKRAIGVPELDH
FLRNEAIVDAKTRRKLLDEAIDKIKENTCMLASRQLQKIHRLHSIWNWNVHRIDATPVFLTSGKEVDNLW
DKLVAGPSTMIVNQFLCDKNYASPIIPSESIKMEPISVPALAVGATR

**FIGURE 12 (continued)**

**SEQ ID NO 156: Populus tremuloides Poptr_IPT like III nucleic acid sequence**

gtcaacgacaacgtcccagcacattcaacaatgaccatgaggctttctttgtctgcctacaaacaagtac
agcctcgtgtgaatttccaagggggctcaacatgaaacctttctttcgtcgaaaagataaggttgtgtt
cgttcttggaccgactggcactggcaaatcaaggctggctattgacctagcaacccacttcccagccgag
gttgtcaattgtgacaaaatgcaagtttataagggcctagatatagtcacaaacaaggtgacggaagagg
agtgtcgaggcgtgccccatcatttacttggcatagcagaccctaatgcagatttcacttccgatgactt
caggcaccacgcatcacttgttgtcgaatcaatcgtcacacgtgatcggctgccgatcattgccggcgga
tccaattcgtacgtggaggctttagcaaatgatgatcctgagtttcgattaaggtatgaatgttgctttc
tttgggtggatgtgtcactacctctactccactcattcgtatcagatcgggttgatcgaatggtaagagc
aggcttgattgatgaggtgagagatgtgtttgatccaacaaaatttgatgattattcacaaggaatcaag
cgggcaattggggttcctgaattagatcaatttctacgaaacgagacgattgtggatgctaaaacacgta
gaaagcttcttgatgaggccattgaaaaaatcaaagaaaatacttgtatgctagctcgtcgacaattaca
aaaaatccgtcgacttcatagcatatggaattggaaaatgcaccggatcgatgccacaccagttttccta
gcaagtggaaaggaggctgacaatctttgggaccaaattgtggcaggaccaagcaccatgattgtgaacc
aatttctttgtgaagaaaatcatgtatcccccattgtaccatcagagtcgatcaatatggtgccaatttc
tgtcccggctatggccgccgtggctagtcgatagaggtaaaggattcttggttaataccaaaattcatct
acaaatcattaccatcatcataatcaccagctgtgattataaat

**SEQ ID NO 157: Populus tremuloides Poptr_IPT like III translated polypeptide sequence**

MTMRLSLSAYKQVQPRVNFQGGLNMKPFFRRKDKVVFVLGPTGTGKSRLAIDLATHFPAEVVNCDKMQVY
KGLDIVTNKVTEEECRGVPHHLLGIADPNADFTSDDFRHHASLVVESIVTRDRLPIIAGGSNSYVEALAN
DDPEFRLRYECCFLWVDVSLPLLHSFVSDRVDRMVRAGLIDEVRDVFDPTKFDDYSQGIKRAIGVPELDQ
FLRNETIVDAKTRRKLLDEAIEKIKENTCMLARRQLQKIRRLHSIWNWKMHRIDATPVFLASGKEADNLW
DQIVAGPSTMIVNQFLCEENHVSPIVPSESINMVPISVPAMAAVASR

**SEQ ID NO 158: Populus tremuloides Poptr_IPT like IV nucleic acid sequence**

tgtaagtccctgtgcccacaaacaagtcggatgcttgatatccctcctggtatgctcaaaatggacatgc
taagtcccagaagccggcaaaaggagaaggttgtgatagtaatggggcctactggaactggcaagtctcg
gctctctatcgaacttgcaacccagttccctgcagaaatcataaactccgacaaaatgcaggtttacaaa
ggacttgacatcgtcactaacaaagttgctgaggaagagaaatctggtgtccctcaccatttgttaggaa
tagcaaatcctactgtggactttacagcaacaaattactgtcacacggcttccttggcggtcgaatcaat
ttcgactcgaggattgctcccgatcattgttggaggttccaattcatacattgaggccttgatggatgat
gaggatttaggttacggttgaactatgattgttgcttcctttgggtagatgtatcaatgcctgtgctac
ataaatttgtttcgaggcgagtcgagcagatggttagtgtggggatgatcgatgaggtcagaaacatttt
cgatccctacgctgattattctacggggatcaggaggtcaattggagttcctgaattcgacaagtacttt
agagctgaagcatttttggatgaagaaaaccgtgccagactacttcatgaagcaatatgtgatgtcaaaa
agaatacatgtaagttagcttgccgtcaatgggaaaagattaatcggcttaggaagatcaaggggtggga
catccatagacttgatgccactgaagtattccaaaagtcgggaaaggaagcagatcatgcctgggaaatg
cttgtggccagacccagcactgccattgtgggacaactcctctgtggtgttcctgctgataaggtccccg
ccatagctagcgtagcaaaaaacatgggctatattcgacaatgccttgtggcatagccgttcaatatggc
taattaacgactttagaaagtggtatcatatacaatattatctaagtagagggctatcaaggcagg

**SEQ ID NO 159: Populus tremuloides Poptr_IPT like IV translated polypeptide sequence**

MLDIPPGMLKMDMLSPRSRQKEKVVIVMGPTGTGKSRLSIELATQFPAEIINSDKMQVYKGLDIVTNKVA
EEEKSGVPHHLLGIANPTVDFTATNYCHTASLAVESISTRGLLPIIVGGSNSYIEALMDDEDFRLRLNYD
CCFLWVDVSMPVLHKFVSRRVEQMVSVGMIDEVRNIFDPYADYSTGIRRSIGVPEFDKYFRAEAFLDEEN
RARLLHEAICDVKKNTCKLACRQWEKINRLRKIKGWDIHRLDATEVFQKSGKEADHAWEMLVARPSTAIV
GQLLCGVPADKVPAIASVAKNMGYIRQCLVA

## FIGURE 12 (continued)

**SEQ ID NO 160: Populus tremuloides Poptr_IPT like V nucleic acid sequence**

ttacaccattctcaaataattacacgaattatggaaatgattcctctacaaccaaagcttgcctcaagta
tgagaactatgccaatggctgcctctctccccttaagaatggagcgggaaatcaggcaccgcaacaacct
ccaacagatcactagttcactttattccattgacgacaagaagaagcaaaaagctttgttcgtaatggga
acaactgccactggaaaatcaaaactctccattgatttagccactcattttcaaggtgaaatcatcaatt
cagacaaaattcaggtttacaagggccttgacatgttgaccaacaaaatttcggaaattgaacgccgagg
tgtgcctcaccatttgctaggatttgtggaacctggagaagagtttaccacacaagatttttgcaatcat
gtacacaaggccatgaaacatattacaggggatggaagcatccccattatcgccggcggctcgaatagat
acatcgaagcactcgtcgaggatccgctttcaacttcaaggatagttatgatacttgttttctatgggt
ggatgttgccttgccaatcttatttgatcgcgcagcaaaaagggttgatgagatgcttgatgctggtctt
gttgaagaggttcgaggtatgtttattccagggatagatcacaatagcgggatttggcgggctattggga
ttgcagagatggaaccatattttcaagctgaaatggaaatggctgatgaggtcaccatgaaaatattgct
tgaaactggtattaaagaaatgaaagaaaataccaagaagctaatcaataaacaactaacgaaaatcaaa
tatttggctaacaagaaaggatggaaattccatcggattgatgctacttgtgtgtatgagagaagtgcaa
aagttgatgaagatgtttgggacaagaaggttttgagacctagcttggagatagttactaattttctccg
ggaagacgagaaagcagaagaagtggcagacagttttctagttacaagctagcagctgcatctaatcaat
tgagatataccgattagttcaattttctatcgcatctttacttctcagcacacatcaaaata

**SEQ ID NO 161: Populus tremuloides Poptr_IPT like V translated polypeptide sequence**

MEMIPLQPKLASSMRTMPMAASLPLRMEREIRHRNNLQQITSSLYSIDDKKKQKALFVMGTTATGKSKLS
IDLATHFQGEIINSDKIQVYKGLDMLTNKISEIERRGVPHHLLGFVEPGEEFTTQDFCNHVHKAMKHITG
DGSIPIIAGGSNRYIEALVEDPLFNFKDSYDTCFLWVDVALPILFDRAAKRVDEMLDAGLVEEVRGMFIP
GIDHNSGIWRAIGIAEMEPYFQAEMEMADEVTMKILLETGIKEMKENTKKLINKQLTKIKYLANKKGWKF
HRIDATCVYERSAKVDEDVWDKKVLRPSLEIVTNFLREDEKAEEVADSFLVTS

**SEQ ID NO 162: Populus tremuloides Poptr_IPT like VI nucleic acid sequence**

ggcagtacaaagcaaaaagctttgttcgtaatgggaacaactgccactggaaaatcaaaactctccatcg
atttagccactcattttcaaggtgagatcatcaactcggacaaaattcaggtttacaagggccttgacat
attgaccaacaaagtttcggaagatgaaagccgaggtgtgcctcaccacttgctaggatttgtggaacct
ggggaagagtttaccacacaagatttttgcaaccatgtacatatggccatgagacatattatagggaatg
gaaacatccccattattgccggcggctcaaatagatacatcgaagcactcgtcgaggatccactgttcaa
ggataattacgatacttgttttctatgggtggatgttgccttgccaatttatttgttcgcgcagcaaag
agggttgataagatgctcgatgctggtcttgtcgacgaggtccgaggcatgtttattccagggatagatc
acaatagcgggatttggcgggctattgggattccagagctggaaccatattttcaagctgaaatggaaat
ggccgatgaggtgaccagaaaaatgttacttgacactggtatcaaggaaatgaaagaaaataccaagaag
ctaatcaataaacaactgaggaaaatcaaatatttggcgaatgagaaaggatggaaattacatcggattg
atgctacttttgtgtacgagagaagtggaaacgtagatgaagatgtttgggacgacaaggttttgagacc
tagcctggagatgctcactaactttctccaggaagatgggaaagcagaagaatttgtggatgctagtggt
ttgcctggtagctttgaatggagagagaaaggagttgtcactgatgtcaagattcagggtgcttgtggat
catgctgggcttttagcaccactggatctgttgaaggagcaaattttattgcaacaaggaagcttctcaa
ccttagtgaacaacagcttgttgattgtgacagtgtgacagacaagacttcctgtggtgatggttgcggt
ggagggttcatgaccaatgcctacaggtgtttgatcgaggcaggggcgttacaagaggagactggattga
atgcaatattaatgcaaacttgtattagaggggtctcgtgcccagttattcgcggcaagaaatggctcaa
ccgtggtgttctacttgttgggaatggtgcaagaggttactccattcttaattaggtctggctacaagcc
gtactggatcatcaggaactcatggggtgagcattggggagagaagggacatcaccttctttgca

FIGURE 12 (continued)

**SEQ ID NO 163: Populus tremuloides Poptr_IPT like VI translated polypeptide sequence**
MGTTATGKSKLSIDLATHFQGEIINSDKIQVYKGLDILTNKVSEDESRGVPHHLLGFVEPGEEFTTQDFC
NHVHMAMRHIIGNGNIPIIAGGSNRYIEALVEDPLFKDNYDTCFLWVDVALPILFVRAAKRVDKMLDAGL
VDEVRGMFIPGIDHNSGIWRAIGIPELEPYFQAEMEMADEVTRKMLLDTGIKEMKENTKKLINKQLRKIK
YLANEKGWKLHRIDATFVYERSGNVDEDVWDDKVLRPSLEMLTNFLQEDGKAEEFVDASGLPGSFEWREK
GVVTDVKIQGACGSCWAFSTTGSVEGANFIATRKLLNLSEQQLVDCDSVTDKTSCGDGCGGGFMTNAYRC
LIEAGALQEETGLNAILMQTCIRGVSCPVIRGKKWLNRGVLLVGNGARGYSILN

**SEQ ID NO 164: Vitis vinifera Vitvi_IPT like nucleic acid sequence**
atgccatctctgtcgkaatgtgaygtatctgtgcaggactgcacaaaccaccccaccaaaacgaccgtca
cttctattcctatgaaactccccgtccccaccggttatcatccatattgcagcaaagttcaaacgctccc
actcataccggcgataaagcccaccttccgaagatccgggtgggctcgcatggattccacggyccgccgt
aatcgcmccaagcacaaggtcgtcgttatcatgggagccaccggcaccggaaaatccaaactctccatcg
acctcgccacacgcttccccgccgagatcatcaactctgacaaaatacaaatctacagtggccttgacat
caccactaacaagatccaaatgcacgagcgacaaggtgtaccccaccacctgctcggagatttcgactcc
tcccacgctgagataaccccttcccagttccgctcggtggctgccgctgctatctcagacatctcttctc
gccgcaaactgccagtcctcgctggtgggtccaattcctttattcacgcactcctcgtcgaccggtttga
ctccgagtctgaccccttyaatggttcggactcggtctccaccgaattacgttaccgctgttgtttccta
tgggttgacgtttcatttgctgttctctccgactacttgtcgaagcgagtcgatgaaatgcttggctcag
ggatgcttgaagagttagctaagttctacgacccggatgaagacgagtctagacccaaaactgggttgag
aaaagcgataggagtccccgagttcgacaggcatttccgaaagtaccctcccgtcgaccaaggaataatc
gctggaaatcccaagaagaagaaggatgatccagaaatggaaagttttgaagaggcagtgaaggccatca
aggacaacacgtgtcatctagcgaagaagcagatagagaagatcctacggatgaggggagccgggtggga
cctcaagagactggacgccacagaggcgttcagggtgctgctgtcgtcagattccggcaagaagtcgtca
gaaatatgggagaagcaggtagtggaaccgagcgtgaagtttgtgaggcgcttcttagaggagtaggttg
ttcagctttttctacccgtcctgttttcctatgatccaaaattagttacctaaattactttcatttcccc
ctttttttggatttactctttccctctctatttgaaaaaaaaa

**SEQ ID NO 165: Vitis vinifera Vitvi_IPT like translated polypeptide sequence**
MPSLSXCDVSVQDCTNHPTKTTVTSIPMKLPVPTGYHPYCSKVQTLPLIPAIKPTFRRSGWARMDSTXRR
NRXKHKVVVIMGATGTGKSKLSIDLATRFPAEIINSDKIQIYSGLDITTNKIQMHERQGVPHHLLGDFDS
SHAEITPSQFRSVAAAAISDISSRRKLPVLAGGSNSFIHALLVDRFDSESDPFNGSDSVSTELRYRCCFL
WVDVSFAVLSDYLSKRVDEMLGSGMLEELAKFYDPDEDESRPKTGLRKAIGVPEFDRHFRKYPPVDQGII
AGNPKKKKDDPEMESFEEAVKAIKDNTCHLAKKQIEKILRMRGAGWDLKRLDATEAFRVLLSSDSGKKSS
EIWEKQVVEPSVKFVRRFLEE

**SEQ ID NO 166: Zea mays Zeama_IPT like nucleic acid sequence**
Atgaccaccctcctcgccaataggatcactacgctcgtgcgcgcccctcctcctcccatggccgccgccgcc
gtcgcgggagcgcggaggccattgcaccggaccttggcgcacccgccaccgcccgaggaggacgagcatcag
cagcagcgcgcgtgccgcagcaggggatcctcgtcctcctgctcggcttcctcgtcatcgacgcccgcccgg
ccccgggcacggggatggtggtgatcgtcggcgccacgggcaccgggaagaccaagctgtccatcgacgcc
gcggaggcggtcggcggggaggtggtgaacgcggataagatccagctctacgccgggctggacgtgaccacg
aacaaggtggcccccgcggaccgccgcggcgtgccgcaccacctcctcggcgccatccgccccgaggccggc
gagctcccgccctccacgttccgctccctcgccgccgccacggccgcctcgatcgccgcgcgcggccgcctg
ccggtcgtcgcgggcggctccaactccctcatccacgcgctcctcgccgaccgcctcgacgccggcgccgcc
gaccccttctccgctccaccgcagccggcgccgccgcggtgggccgccggcccgcgctccgatccccgtgc
tgtctcctctgggtccacgtcgacgccgcgctcctcgcggagtacctggaccggcgcgtggacgacatggtg

FIGURE 12 (continued)

cgcggcggcatggtggaggagctgcgggagtacttcgccgcgaccaccgccgccgagcgcgccgcgcacgcc
gcggggctgggcagggccatcggcgtgcccgagctgggcgcctgcttcgcggggcgcgccagcttccgcgcc
gcgatcgacgacatcaaggccaacacgcgggacctggcggccgcgcaggtgcgcaagatccgacgcatggcc
gatgcctggggctggcccatccagcggctcgacgcgtcggccacagtccgcgcgcgcctccgcggcgcgggg
cccgacgcggagtcggcgtgctgggagcgcgacgtgcgcgcgcccgggctcgccgccatccggagcttcctt
ctagagctggacggcggcagcgtcgtcgacggcgctgtggtggaggaggtggagccgcgggtgcgatgctgc
gacgtggtggggtga

**SEQ ID NO 167: Zea mays Zeama_IPT like translated polypeptide sequence**
mttllanrittlvrappppmaaaavagarrplhrtlahppppeedehqqracrsrgsssscsasssstpar
prgtgmvvivgatgtgktklsidaaeavggevvnadkiqlyagldvttnkvapadrrgvphhllgairpeag
elppstfrslaaataasiaargrlpvvaggsnslihalladrldagaadpfsappqpapprwgrrpalrspc
cllwvhvdaallaeyldrrvddmvrggmveelreyfaattaaeraahaaglgraigvpelgacfagrasfra
aiddikantrdlaaaqvrkirrmadawgwpiqrldasatvrarlrgagpdaesacwerdvrapglaairsfl
leldggsvvdgavveeveprvrccdvvg

FIGURE 12 (continued)

```
                                   1                                                50
          Arath_IPT1        (1)    ----------MTELNFHLLPIISDRFTTTTTTSPSFSSHSSSSSSLLSFT
          Arath_IPT4        (1)    --------------------------------------------------
      Humlu_IPT like        (1)    ---------------------------------------------MDYAS
      Moral_IPT like        (1)    --------------------------------------------------
          Arath_IPT6        (1)    ------------------------------MQQLMTLLSPPLSHSSLLPT
      Poptr_IPT like        (1)    --------------MKIPFPKSTNQPLYTALKTQPLHPINISIPFNKPRP
      Lotja_IPT like        (1)    -------------------------------------MRLSSLSPHPHHHH
      Medtr_IPT like        (1)    -----------MPTTTTTPSFLSPPHSQRHYHKPIQFHHYSHTLFTPSSS
      Vitvi_IPT like        (1)    MPSLSXCDVSVQDCTNHPTKTTVTSIPMKLPVPTGYHPYCSKVQTLPLIP
      Pethy_IPT like        (1)    -----MLIVVHIISITRIIFITLTHNHLHFLMFRSLSYNHKHLKFLTNPT
      Orysa_IPT like        (1)    ------------------------------------------MTSVATRI
      Zeama_IPT like        (1)    -MTTLLANRITTLVRAPPPPMAAAAVAGARRPLHRTLAHPPPPEEDEHQQ
   Poptr_IPT like II        (1)    ------------------------------------------MTMRLSLT
  Poptr_IPT like III        (1)    ------------------------------------------MTMRLSLS
   Poptr_IPT like IV        (1)    --------------------------------------------------
    Poptr_IPT like V        (1)    ---------------------MEMIPLPQPKLASSMRTMPMAASLPLRME
   Poptr_IPT like VI        (1)    --------------------------------------------------
           Consensus        (1)
                                   51                                              100
          Arath_IPT1       (41)    KRRRKHQPLVSSIRMEQSRSRNR    V    L     A G    V     R-F
          Arath_IPT4        (1)    --------------------MKCN  M         S    S  V    LH-F
      Humlu_IPT like        (6)    VAMAAAPTTTTTTNVSLRRQRHR E LL L      G            AH P
      Moral_IPT like        (1)    --------MEFSSSAARRHRHHP   LL        G        VE   H N
          Arath_IPT6       (21)    VTTKFGSPRLVTTCMGHAGRKNI   V L T T   G        V    R F
      Poptr_IPT like       (37)    PPIAVRMDTDSSTTTSTAVYRHK   IL        C  T V        R Q
      Lotja_IPT like       (15)    HYTTHYHYHYHHPSSLAMDGHRRI   V        S              L P
      Medtr_IPT like       (40)    SATHTRRRPHCPRMEISPSRHRR   VLI V     S        VE   L P
      Vitvi_IPT like       (51)    AIKPTFRRSGWARMDSTXRRNRX H  V        G    K         R P
      Pethy_IPT like       (46)    TRVLRRNMSSSTVVTIPGPTQKN N  II       S    K        V RHY
      Orysa_IPT like        (9)    ATLVRAAAAASRPLRLHRRPGGEDTRM   V      G  TK      A KVIG
      Zeama_IPT like       (50)    QRACRSRGSSSSCSASSSSTPARPRGTGMVVIVGA GTGKTKLSIDAAEA
   Poptr_IPT like II        (9)    AYKQVQPRGNFQGGLNMKPFFRR   V FVV P    G      A      R P
  Poptr_IPT like III        (9)    AYKQVQPRVNFQGGLNMKPFFRR   V FVL P    G      A      H P
   Poptr_IPT like IV        (1)    ---MLDIPPGMLKMDMLSPRSRQ E V IV  P    G          E   Q P
    Poptr_IPT like V       (29)    REIRHRNNLQQITSSLYSIDDKK Q ALFV  T A  G   K         H Q
   Poptr_IPT like VI        (1)    ------------------------------  T A  G   K         H Q
           Consensus       (51)                        RRKDKVVVIMGATGTGKSRLSIDLAT F
                                   101                                             150
          Arath_IPT1       (90)    PS-E  N DK  Q  E G E  TNQ TIQD   V HHLLGVIN- EH  L A
          Arath_IPT4       (30)    KA-E  N DK  QF DG K   TNQSTIED   V HHLLGEIN- EA  V A
      Humlu_IPT like       (56)    L--EV N DK  Q  G      TN SVPD G V HHLLGEV - AR  L
      Moral_IPT like       (43)    G--E  N DK  Q  G   T TN PLDD L V HHLLGEV SEAH  L R
          Arath_IPT6       (71)    PA-E  N DK  QI  CFE  VTNL PLHEQG V HHLLGQFH- QD  L
      Poptr_IPT like       (87)    S--E  N DK  Q  EG     TN TIQD L V HHLLGEF - DD  L
      Lotja_IPT like       (65)    FS-E  N DK  Q  G   T TN PPHQ NN V HHLLGDV - SL DF
      Medtr_IPT like       (90)    YS-E  N DK  Q  R      TN PFHQ NN V HHLLGDI - SH  FS
      Vitvi_IPT like      (101)    A--E  N DK  Q I SG     TN QMHE Q V HHLLGDF -SSHA I
      Pethy_IPT like       (96)    PFSE  N DK  Q T  G N   TN TVPD    VVHHLLGEI --PDFNFS
      Orysa_IPT like       (59)    G--EV NADK  QL DG   V TN VSLAD   V HHLLGAIR- EA  LP
      Zeama_IPT like      (100)    VGGEV NADK  QL AG   V TN VAPAD   V HHLLGAIR- EA  LP
   Poptr_IPT like II       (59)    A--EV NCDK  Q  G    VTN VTEEEC  V HHLLGIA --PNANF S
  Poptr_IPT like III       (59)    A--EV NCDK  Q  G    VTN VTEEEC  V HHLLGIA --PNADF S
   Poptr_IPT like IV       (48)    A--E  N DK  Q  G    VTN VAEEEKS V HHLLGIAN--PTVDF A
    Poptr_IPT like V       (79)    G--E  N DK  Q  G   MLTN SEIE    V HHLLGFVE--PGE F T
   Poptr_IPT like VI       (21)    G--E  N DK  Q  G    LTN VSEDES  V HHLLGFVE--PGE F T
           Consensus      (101)      EIINSDKMQVYKGLDITTNKITL DRRGVPHHLLG ID P  GELTP
```

**FIGURE 13**

```
                       151                                            200
         Arath_IPT1 (138) GE  SA SNVVKKV TS Q VP I GGSN FV AL AQ   P-KFDP SSG
         Arath_IPT4  (78) AE  VM AEAISE TQ K  P L GGSN Y  AL AKSY P-ENYP SDH
     Humlu_IPT like (103) AD  SL GKAVSE TG R  PVLVGGSN F  AL V    SSGPGV EEG
     Moral_IPT like  (91) SE  SL GKAVSE TA R  PVL GGSN F  AL V    P-EYDV DER
         Arath_IPT6 (119) AE  SL TLSISKLISSK P VVGGSN FN AL AE   P-DIDP SPG
     Poptr_IPT like (134) SEY LAGGLAISG VS QN P VVGGSN L  ALVV  NP-ELNV DG-
     Lotja_IPT like (113) SD  RR GDLISD TR R  P FVGGSN FV AL V    P-ESNV RDD
     Medtr_IPT like (138) SD  RH GDIISD TS R  P IVGGSN F  AL VE   P-ESNV DES
     Vitvi_IPT like (148) SQ  SV AAAISD SS R  PVL GGSN F  AL V    S-ESDP NG-
     Pethy_IPT like (144) SH  SI GQRINS IN H  PFLVGGSN Y YAL TN   P----D NPD
     Orysa_IPT like (106) SS  SL AATAAS AA RLVPVI GGSN L  AL A H  ASAGDP SP-
     Zeama_IPT like (149) ST  SL AATAAS AA GR PVV GGSN L  AL A  L AGAADP SAP
  Poptr_IPT like II (105) DD  HH SLVVES VT DR P I GGSN Y EALAN-DDPE---------
 Poptr_IPT like III (105) DD  HH SLVVES VT DR P I GGSN YVEALAN-DDPE---------
  Poptr_IPT like IV  (94) TNYCHT SLAVES ST GL P IVGGSN Y EALMD EDFR---------
   Poptr_IPT like V (125) QD CNHVHKAMKH TGDGSIP I GGSNRY EALVE PLFN---------
  Poptr_IPT like VI  (67) QD CNHVHMAMRH IGNGNIP I GGSNRY EALVE PLF----------
          Consensus (151) SDFR  AG AIS IT R KLPIIAGGSNSFIHALL DRFD       F
                       201                                            250
         Arath_IPT1 (187) S------CLISSD   E C IW   ET LYE  LRR  EMMD G FE
         Arath_IPT4 (127) K------GSICSE K D C IWI  DQS LFE  SLR  LMMK G FE I
     Humlu_IPT like (153) S--HSVVSS---E   D C  W   VK LTD  A R  DMLEL G FD
     Moral_IPT like (140) S--DQPADSSKVL   N C  W   LR LED  L R  DMLN G FD
         Arath_IPT6 (168) SS----LSTICSD   K CI W  LE  LFQH CN R  QMIE G VEQ
     Poptr_IPT like (182) ---CNPVST---Q   N C  W   L  LCD  C R  EMLD G LD
     Lotja_IPT like (162) S--PSPVSS---E   R C  WM IAF  LSE  L R  IMLD G VD
     Medtr_IPT like (187) SSLSTSISS---D   K C  WM I F  LSE  L R  DMFD G VN
     Vitvi_IPT like (196) ---SDSVST---E   R C  W    FA LSD  S R  EMLG G LE
     Pethy_IPT like (190) SN---PVHFISNE   N C IW  LN  LNE  D R  EMMN G YE
     Orysa_IPT like (155) ---AAAFRHYRPA  FP CL  W H DEAL DE  DR R  DMVDA  VE
     Zeama_IPT like (199) PQPAPPRWGRRPA  SP CL  W H DAAL AE  DR R  DMVRG  VE
  Poptr_IPT like II (145) -----------FR   E C  W   L  LYSFVSE R  RMVEAGLID V
 Poptr_IPT like III (145) -----------FR   E C  W   L  LHSFVSD R  RMVRAGLID V
  Poptr_IPT like IV (135) -----------LR N D C  W   M  LHKFVSR R  EQMVSV  ID V
   Poptr_IPT like V (166) -----------FKDS DT C  W  AL  LFDRAA  R  EMLDAGLVE V
  Poptr_IPT like VI (107) ------------KDN DT C  W  AL  LFVRAA  R  KMLDAGLVD V
          Consensus (201)               LRY CCFLWVDVS PVL EYL KRVDDMLDSGMVDEL
                       251                                            300
         Arath_IPT1 (231) SRFYD VKSGLETR-----F  I    IG  E  G  KEYPPEK----K---
         Arath_IPT4 (171) AEFHRSKKAPKEP------L  IW   IG QE  D LKMYKWDN----D---
     Humlu_IPT like (198) AEFYS EDEDHDEDS-ATRT      IG  E  R  EKFRPGD--VEGEDP
     Moral_IPT like (188) AEFYD EEDHG---P-ANWT      IG  E  R  ERCRP------GEKG
         Arath_IPT6 (214) AELYD VVDSGRR------L  V   TIG  EE R  RVYPKEM----D---
     Poptr_IPT like (226) SEYYGSVDAAS-------QI      IG  E  R  KKYPPGSGCGRGIGV
     Lotja_IPT like (207) AQF DSDTANQ--------T      IG  E  RF KDPVREG--------
     Medtr_IPT like (234) AEFYE DADNQ--------T      IG  E  RF KQYPPQVG--PDESE
     Vitvi_IPT like (240) AKFYD DEDES-----RPKT      IG  E  RH RKYPPVDQGIIAGNP
     Pethy_IPT like (237) EQF KENRFSDPGLEPGRAT      IG  EMEP  KKSCT----------
     Orysa_IPT like (202) REY ATTTAAERAAHSG----G  G IG  ELGD  AGRKT----------
     Zeama_IPT like (249) REY AATTAAERAAHAAG---GR   IG  ELGAC AGRAS----------
  Poptr_IPT like II (184) RDM D NKF------DDYSQ IKR IG  EL HFLRNEAIVDAKT-----
 Poptr_IPT like III (184) RDV D TKF------DDYSQ IKR IG  EL QFLRNETIVDAKT-----
  Poptr_IPT like IV (174) RNI D --Y------ADYST I RSIG  E  K  RAEAFLDEEN-----
   Poptr_IPT like V (205) RGM I G---------IDHNS IWR IGIAEMEP  QAEMEMADEVT----
  Poptr_IPT like VI (145) RGM I G---------IDHNS IWR IGI ELEP  QAEMEMADEVT----
          Consensus (251) AEFFDP                TGLRKAIGVPEFDRYFK
```

## FIGURE 13 (continued)

```
                                 301                                                350
           Arath_IPT1    (269)   MIKWDAL KAAYDK  DD KR TWTL   QVKKIEM KD  -WEIE VDA
           Arath_IPT4    (208)   MDKWDPM KEAY K  RA K  TFQLT DQITKINK RN  -W IKKVDA
        Humlu_IPT like   (245)   GR--DRV RGAF    RA K  T HL   QIGKIL  KG  -W LR LDA
        Moral_IPT like   (228)   EW--DRV REAY    RE K  T QL   QIGKIL  KKL -W LR LDA
           Arath_IPT6    (251)   KGIWDLA KAAY  T KGMK RT RLV KQKEKIMK IRG -WEIK LDA
        Poptr_IPT like   (269)   EW--DRV RGVY VC RE K  T QL   QIGKIL  KG  -W LK VDA
        Lotja_IPT like   (241)   ---------AAY    RA K  T QL   QIGKIM  KR  -W LR IDA
        Medtr_IPT like   (274)   RH--NPM EGAYI   KA KD T QL   QIGKIL  KR  -W LQ IDA
        Vitvi_IPT like   (285)   KKKKDDPEMESF    KA KD T HL  KQIEKIL MRG  -W LK LDA
        Pethy_IPT like   (277)   -----------Y    RE K  TWRL  KQMWKIQ  RE  -W LQ VDA
        Orysa_IPT like   (238)   -----------FS  IDD KA TRVL AAQVSKIR MSD WGWPIH LDA
        Zeama_IPT like   (286)   -----------FRA IDD KA TRDL AAQVRKIR MAD WGWPIQ LDA
     Poptr_IPT like II   (223)   ------- RKLLD  IDK K  T ML S QLQKIH  HSIWNWNVH IDA
    Poptr_IPT like III   (223)   ------- RKLLD  IEK K  T ML R QLQKIR  HSIWNWKMH IDA
     Poptr_IPT like IV   (211)   ------- ARLLH  ICDVKK T KL C QWEKIN  RKIKGW IH LDA
      Poptr_IPT like V   (243)   -------MKILL TGIKEMK  TKKLINKQLTKIKY ANKKGWKFH IDA
     Poptr_IPT like VI   (183)   ------- KMLLDTGIKEMK  TKKLINKQLRKIKY ANEKGWKLH IDA
             Consensus   (301)          RK  YEEAVREIKENTC LAKRQI KI RLK AG WDL RLDA
                                 351                                                400
           Arath_IPT1    (318)    AS KAVMMKSSS-------EKKWRENW EQ L          KRHLVQN---
           Arath_IPT4    (257)    AS  EAIRAAKEGE---GVAEMQRKIWNKE L  C     RSHLDQPINY
        Humlu_IPT like   (292)    ES  AAMTSDSG--------EKCTEIW KQ L         SR LD ----
        Moral_IPT like   (275)    EA  AVMTSDSG--------KRCSEIW RQ L         KR LD ----
           Arath_IPT6    (300)    AAIMAELNQSTAK----GEGKNGREIW KHIVDE  E  KK LL V---
        Poptr_IPT like   (316)    ES  EVMTVTSDDHIKKRKKKRWMEVWGRD M   M   KR LE E---
        Lotja_IPT like   (281)    EA  VALVADG-------GGERFSDEWKRQ L         KR LM ----
        Medtr_IPT like   (321)    EA  AVLTSESNG-----GGEEFTGVWKKQ L         KR LM ----
        Vitvi_IPT like   (334)    EA  VLLSSDSG--------KKSSEIW KQ V       F RR LE ----
        Pethy_IPT like   (315)    EA VEAMSNKKE----------KGIIW KQ V         NR LLD----
        Orysa_IPT like   (277)   SDTV ARLTRAGS--------AAESASW RD RG GLATIRS LADQSPP
        Zeama_IPT like   (325)   SATV ARLRGAGP--------DAESACW RD RA GLAAIRS LL LDGG
     Poptr_IPT like II   (266)    PV LTSGKEVDN-------------LWDKL AG  TM  NQ LCDKNYA
    Poptr_IPT like III   (266)    PV LASGKEADN-------------LWDQI AG  TM  NQ LC ENHV
     Poptr_IPT like IV   (254)    EV QKSGKEADH-------------AW ML AR  TA  GQLLCGVPAD
      Poptr_IPT like V   (286)    CVYERSAKVDED-------------VWDKK LR  LE  TN LR DEKA
     Poptr_IPT like VI   (226)    FVYERSGNVDED-------------VWDDK LR  LEMLTN LQ DGKA
             Consensus   (351)   TEAFR SL                  IWEK VLEPSVKIVKRFL E
                                 401                                                450
           Arath_IPT1    (358)   --------------------------------------------------
           Arath_IPT4    (304)   YYYYFYLLKRFLSLN-----------------------------------
        Humlu_IPT like   (330)   --------------------------------------------------
        Moral_IPT like   (313)   --------------------------------------------------
           Arath_IPT6    (343)   --------------------------------------------------
        Poptr_IPT like   (363)   --------------------------------------------------
        Lotja_IPT like   (320)   --------------------------------------------------
        Medtr_IPT like   (362)   --------------------------------------------------
        Vitvi_IPT like   (372)   --------------------------------------------------
        Pethy_IPT like   (351)   --------------------------------------------------
        Orysa_IPT like   (319)   PRSEGTNDYLYAMETEPEPPPPPTLPPRLLRLPRMQYCDMVG--------
        Zeama_IPT like   (367)   ----SVVDGAVVEEVEP----------------RVRCCDVVG--------
     Poptr_IPT like II   (303)   SPIIPSESIKMEPISVPALAVGATR-------------------------
    Poptr_IPT like III   (303)   SPIVPSESINMVPISVPAMAAVASR-------------------------
     Poptr_IPT like IV   (291)   KVPAIASVAKNMGYIRQCLVA-----------------------------
      Poptr_IPT like V   (323)   EEVADSFLVTS---------------------------------------
     Poptr_IPT like VI   (263)   EEFVDASGLPGSFEWREKGVVTDVKIQGACGSCWAFSTTGSVEGANFIAT
             Consensus   (401)
```

**FIGURE 13 (continued)**

**MKVQCDVCAAEAASVFCCADEAALCDACDRRVHSANKLAGKHRRFSL**LQPLASSSSAQKPPLCDI
CQEKRGFLFCKEDRAILCRECDVTVHTTSELTRRHGRFLLTGVRLSSAPMDSPAPSEEEEEEAGE
DYSCSPSSVAGTAAGSASDGSSISEYLTKTLPGWHVEDFLVDEATAASSSSDGLFQGGLLAQIGGV
PDGYAAWAGREQLHSGVAVAADEWASRERWVPQMNAEWGAGSKRPRASPPCLYW

Figure 14

```
                       1                                              50
     AT1G06040   (1) -------------------------------------MKIQCDVCEKAPAT
     AT1G75540   (1) -------------------------------------MKIRCDVCDKEEAS
     AT1G78600   (1) -------------------------------------MKIQCNVCEAAEAT
     AT2G31380   (1) -------------------------------------MKIQCDVCEKAPAT
     AT4G10240   (1) -------------------------------------MKIQCEVCEKAEAE
     AT4G39070   (1) -------------------------------------MKIWCAVCDKEEAS
   VV_CAN72879   (1) -------------------------------------MKIPCDICGNVEAE
   GM_ABB29467   (1) -------------------------------------MKIQCDVCEKAPAT
        PP_STO   (1) -------------------------------------MKIQCDVCNKEEAS
   St_ABA40448   (1) -------------------------------------MKIQCDVCEKAQAT
   LE_AAS67368   (1) -------------------------------------MKIQCDVCEKAQAT
   MS_ABO84497   (1) -------------------------------------MKIQCDACHKQEAS
        So_STO   (1) -------------------------------------MKIQCDACEGAAAT
  Os01g0202500   (1) -------------------------------------MKVLCSACEAAEAR
  Os02g0606200   (1) -------------------------------------MKIQCDACESAAAA
  Os02g0646200   (1) -------------------------------------MKVQCDVCAAEAAS
  Os04g0493000   (1) -------------------------------------MRIQCDACEAAAAT
  Os05g0204600   (1) MSPPPPPYYHHLLLLRSSPTTTGGGARVLAAAELARMKLLCSACEAAEAS
  Os06g0152200   (1) -------------------------------------MKIQCNACGAAEAR
  Os06g0713000   (1) -------------------------------------MRVQCDVCAAEPAA
  Os09g0527900   (1) -------------------------------------MRTICDVCESAPAV
  Os12g0209200   (1) -------------------------------------MKIGCDACEQAEAA
  Os04g0540200   (1) -------------------------------------MKVQCDVCAAEAAS
     Consensus   (1)                                      MKIQCDVCE A AT
                                                              B-box domain
```

Figure 15

```
                      51                                                100
AT1G06040      (15)   VICCADEAALCPQCDIEIHAANKLASKHQRLHLNSLS-------------
AT1G75540      (15)   VFCTADEASLCGGCDHQVHHANKLASKHLRFSLLYPSSSN----------
AT1G78600      (15)   VLCCADEAALCWACDEKIHAANKLAGKHQRVPLS--------------AS
AT2G31380      (15)   LICCADEAALCAKCDVEVHAANKLASKHQRLFLDSLS-------------
AT4G10240      (15)   VLCCSDEAVLCKPCDIKVHEANKLFQRHHRVALQKDA---AS-----ATT
AT4G39070      (15)   VFCCADEAALCNGCDRHVHFANKLAGKHLRFSLTSPTFK-----------
VV_CAN72879    (15)   VLCSADEAVLCWGCDERVHTANKLSQKHQRVPLLKHP----P-----STS
GM_ABB29467    (15)   VICCADEAALCAKCDVEVHAANKLASKHQRLLLQSVS-------------
PP_STO         (15)   VFCTADEAALCDTCDHRVHHANKLASKHQRFSLLHPSSK-----------
St_ABA40448    (15)   VICCADEAALCAKCDIEVHAANKLASKHQRLHLQCLS-------------
LE_AAS67368    (15)   VICCADEAALCAKCDIEVHAANKLASKHQRLHLQCLS-------------
MS_ABO84497    (15)   LFCPADEAALCNQCDRNIHYANKVSAKHKRFTLHHPTSK-----------
So_STO         (15)   VVCCADEAALCARCDVEIHAANKLASKHQRLPLEALS-------------
Os01g0202500   (15)   VLCCADDAALCARCDLHVHAANRLAGKHHRLPLLSSSSSSS---------
Os02g0606200   (15)   VVCCADEAALCAACDVEVHAANKLAGKHQRLPLEALS-------------
Os02g0646200   (15)   VFCCADEAALCDACDHRVHRANKLAGKHRRFSLLNPSASGRS------PT
Os04g0493000   (15)   VVCCADEAALCARCDVEIHAANKLASKHQRLPLDAALP------------
Os05g0204600   (51)   VLCCADEAALCARCDRDIHAANRLAGKHLRLPLLSPASSSSSAAALAPP
Os06g0152200   (15)   VLCCADEAALCTACDEEVHAANKLAGKHQRVPLLSDDGGAAP-----AAA
Os06g0713000   (15)   VLCCADEAALCSACDRRVHRANRLASKHRRLPLVHPSSSSSG-----DGG
Os09g0527900   (15)   LFCVADEAALCRSCDEKVHMCNKLARRHVRVGLADPN-------------
Os12g0209200   (15)   VLCCADEAALCRRCDAAVHSANRLAGKHTRVALLLPSSSS-----AAAGD
Os04g0540200   (15)   VFCCADEAALCDACDRRVHSANKLAGKHRRFSLLQPLASSSS--------
Consensus      (51)   VLCCADEAALC  CD  VHAANKLA KHQRL L   S
                      B-box domain 1

                      101                                                150
AT1G06040      (52)   -TKFPRCDICQEKAAFIFCVEDRALLCRDCDESIHVANSRSANHQRFLAT
AT1G75540      (55)   -TSSPLCDICQDKKALLFCQQDRAILCKDCDSSIHAANEHTKKHDRFLLT
AT1G78600      (51)   ASSIPKCDICQEASGFFFCLQDRALLCRKCDVAIHTVNPHVSAHQRFLLT
AT2G31380      (52)   -TKFPPCDICLEKAAFIFCVEDRALLCRDCDEATHAPNTRSANHQRFLAT
AT4G10240      (57)   ASGAPLCDICQERKGYFFCLEDRAMLCNDCDEAIHTCN----SHQRFLLS
AT4G39070      (54)   --DAPLCDICGERRALLFCQEDRAILCRECDIPIHQANEHTKKHNRFLLT
VV_CAN72879    (56)   SSQLPPCDICQEKSGYFFCLEDRALLCKNCDVSTHSTNSYVSSHRRFVIS
GM_ABB29467    (52)   -NKLPRCDICQDKPAFIFCVEDRALFCKDCDEPIHLASSLSANHQRFLAT
PP_STO         (54)   --NFPICDICQEKRAFLFCQQDRAILCRECDGPIHTANEHTQKHNRFLLT
St_ABA40448    (52)   -NKLPPCDICQDKAAFIFCVEDRALFCKDCDEAIHSASSLAKNHQRFLAT
LE_AAS67368    (52)   -NKLPPCDICQDKAAFIFCVEDRALFCKDCDEAIHSASSLAKNHQRFLAT
MS_ABO84497    (54)   --DTPLCDICKERRAYLFCKEDRAILCRECDIPIHEINKLTKQHNRFLLT
So_STO         (52)   -AKLPRCDVCQEKAAFIFCVEDRALFCRDCDEPIHVPGTLSGNHQRYLAT
Os01g0202500   (57)   -PSPPTCDICQDAHAYFFCVEDRALLCRACDVAVHTANALVSAHRRFLLT
Os02g0606200   (52)   -ARLPRCDVCQEKAAFIFCVEDRALFCRDCDEPIHVPGTLSGNHQRYLAT
Os02g0646200   (59)   STTAPLCDICQEKRGFLFCKEDRAILCRECDVPVHTASELTMRHSRYLLT
Os04g0493000   (53)   -AALPRCDVCQEKAAFIFCVEDRALFCRDCDEPIHVPGTLSGNHQRYLTT
Os05g0204600  (101)   PPSPPKCDICQESHAYFFCLEDRALLCRSCDVAVHTANAFVSAHRRFLLT
Os06g0152200   (60)   APAVPKCDICQEASGYFFCLEDRALLCRDCDVSIHTVNSFVSVHQRFLLT
Os06g0713000   (60)   AAAAPLCDVCREKRGLVFCVEDRAILCADCDEPIHSANDLTAKHTRFLLV
Os09g0527900   (52)   --KVQRCDICENAPAFFYCEIDGTSLCLSCDMTVHVGGKRT--HGRYLLL
Os12g0209200   (60)   DDHHPTCDICQEKTGYFFCLEDRALLCRSCDVAVHTATAHAAAHRRFLLT
Os04g0540200   (57)   AQKPPLCDICQEKRGFLFCKEDRAILCRECDVTVHTTSELTRRHGRFLLT
Consensus     (101)       P CDICQEK AFIFCVEDRALLCRDCD AIHTAN  T  HQRFLLT
                                B-box domain 2
```

Figure 15 (continued)

```
                         151                                               200
     AT1G06040  (101)  GIKVALTSTICSK------EIEKNQPEPSNNQQKAN----QIPAKSTSQQ
     AT1G75540  (104)  GVKLSATSSVYKPTSKSSSSSSSNQDFSVPGSSISNPPP-LKKPLSAPPQ
     AT1G78600  (101)  GIKVGLESIDTGPSTKSSPTNDDKTMETKPFVQSIPEPQ-KMAFDHHHHQ
     AT2G31380  (101)  GIRVALSSTSCNQ------EVEKNHFDPSN-QQ------------SLSKP
     AT4G10240  (103)  GVQVSDQSLTENSECSTSFSSETYQIQSKVSLNSQYSSE-ETEAGNSGEI
     AT4G39070  (102)  GVKISASPSAYPRASNSNSAAAFGRAKTRPKSVSSEVPS-SASNEVFTSS
    VV_CAN72879  (106)  GIKVALQSVTNNYRTGCN--------S-RTYPLDMPNSN-SSSVNFPMDR
    GM_ABB29467  (101)  GIRVALGS-NCTK------GNEKGHVEPSKPK---------AQEVPAKIP
         PP_STO  (102)  GVKLSATSAVYISSSSVTNSGGDLVPDSKSQQQQQQQS-IKKPVFDAPV
    St_ABA40448  (101)  GIRVALSS-SCNK------EAVKNQLEPQPPQQN-------SQQVGLKMP
    LE_AAS67368  (101)  GIRVALSS-SCNK------ESVKNQLQPQPPQQN-------SQQVGLKMP
    MS_ABO84497  (102)  GVKIGASSSCSNPTISNGS-------ELRTSS-----P----RPSSFSSE
         So_STO  (101)  GIRVGFASASACS-DGACDAHDSDHHAPPKATIEPP----QATVSAAAQQ
   Os01g0202500  (106)  GVHVGLDAAADDDDKHPPHPLSSSLPRNTAPPPQPPPK--RSPSPIYSDD
   Os02g0606200  (101)  GIRVGFASASPC--DGGSDAHDSDHHAPPMGSSEHHHHH-QQPAPTVAVD
   Os02g0646200  (109)  GVRLSSEPAASPAPPSEEENSS---SFCCSADDAVPAP-------AAPAT
   Os04g0493000  (102)  GIRVGFSSVCSANADHLPPPAPKGNSKPPASGIAAAAAP-KPAVSAAAQE
   Os05g0204600  (151)  GVQVGQ-----EQDEHSPDPPEPSPP---PPPPPPASKS-DHPAPLYGEG
   Os06g0152200  (110)  GVQVGLDPADPVPPVADKHVKSAGGSVDSATKHLQRNPT-DLSGENSASL
   Os06g0713000  (110)  GAKLSPAALAEQPLPSSDCSSDDDAAAAATEEEYHS------SAASTGAA
   Os09g0527900   (98)  RQRVEFPGDKPGHMDDVAMQQKDPENRTDQKKAPHSVTKEQMANHHNVSD
   Os12g0209200  (110)  GVRIGGSVDAAAAADVIVSPTSSSIAPAGSASSNHAGAAG---NNNGRSP
   Os04g0540200  (107)  GVRLSSAPMDSPAPSEEEEEEAGEDYSCSPSSVA----------------
      Consensus  (151)  GVRVG  S

                        201                                               250
     AT1G06040  (141)  QQQPSSATPLPWAVDDFFHFSDIESTDKKGQ-------------------
     AT1G75540  (153)  SNK-------IQPFSKIN-GGDASVNQWGS--------------------
     AT1G78600  (150)  QQQEQQEGV---IPGTKVNDQTSTKLPLVS--------------------
     AT2G31380  (132)  PTQQPAAPSPLWATDEFFSYSDLDCSNKEKE-------------------
     AT4G10240  (152)  VHK---------NPSVILSP------------------------------
     AT4G39070  (151)  SST------TTSNCYYGIEENYHHVSDSGSG-------------------
    VV_CAN72879  (146)  EKKPE--------MTTEVASTSSDMVAMFS--------------------
    GM_ABB29467  (135)  SQQVPSFTS-SWAVDDLLELTDFESPDKVQK-------------------
         PP_STO  (151)  NSNPPTVPSTLSTNTEVNKGGDNLVTNEGFG-------------------
    St_ABA40448  (137)  PQQLSGITSPSWPVDDLLGFPDYESSDKKD--------------------
    LE_AAS67368  (137)  PQQLSCITSPSWPVDDLLGFPDYESSDKKD--------------------
    MS_ABO84497  (136)  NNS----------CSQSS-FKENMVCDTVS--------------------
         So_STO  (146)  VPSPPQFLPQGWAVDELLQFSDYESSDKLHKE------------------
   Os01g0202500  (154)  DVIDWATGGHDIGITGNLPDWSLVDEQFNTP---AL--------------
   Os02g0606200  (148)  TPSP-QFLPQGWAVDELLQFSDYETGDKLQKE------------------
   Os02g0646200  (149)  SHG-----------GSSG--------------------------------
   Os04g0493000  (151)  VPSSPFLPPSGWAVEDLLQLSDYESSDKK--G------------------
   Os05g0204600  (192)  GGG-FSWDAADSPAAGGLPDWSAVVDQFGS--------------------
   Os06g0152200  (159)  PSQNVINGNYSRQSSVTMAKTGQVNWTMSNNTIRSIDPPPKYSSEESPAL
   Os06g0713000  (154)  VSAPLDASSNGAGGGGGVGGSS----------------------------
   Os09g0527900  (148)  DPASDGNCDDQGNIDSKMIDLNMRPVRTHGQG------------------
   Os12g0209200  (157)  APVRFSGGDGGVEPEQQWPWSDVFAADDDDDVS-----------------
   Os04g0540200  (141)  ------------GTAAGSASDGSS--------------------------
      Consensus  (201)                    L    D
```

## Figure 15 (continued)

```
                        251                                              300
   AT1G06040   (172)  ---------------------------L--DLGAGELDWFSDMGFFGDQI
   AT1G75540   (175)  -------------------------TSTISEYLMDTLPGWHVEDFLDSSL
   AT1G78600   (177)  -----------------------------SGSTTGSIPQWQIEEIFG-LT
   AT2G31380   (163)  ------------------------------QLDLGELDWLAEMGLFGDQP
   AT4G10240   (163)  --------------------------------------------------
   AT4G39070   (176)  ----------------------SGCTGSISEYLMETLPGWRVEDLLEH--
 VV_CAN72879   (168)  -----------------------------GEIHLATGPEWTLDEILG-SN
 GM_ABB29467   (165)  ---------------------------Q--SLEFGELEWLADVGLFGEQF
      PP_STO   (182)  ----------------------STTSSTISEYLMETLPGWHVEDFLDSST
 St_ABA40448   (167)  ------------------------------LLELGEFEWLGGIDLFGEQT
 LE_AAS67368   (167)  ------------------------------LLELGEFEWLGGIDLFGEQT
 MS_ABO84497   (155)  -------------------------TSSISEYLIETIPGYCMEDLFDASF
      So_STO   (178)  ---------------------------S-P------LGFGAGVVRRHRPL
Os01g0202500   (187)  ----PPVVTKTPPKRASRGPVTAGTAAAVFGNLAGGSPDWPLNEFFG-FA
Os02g0606200   (179)  ---------------------------SSPPLGFQELEWFADIDLFHNQA
Os02g0646200   (156)  -------------------------SSSISEYLT-TLPGWHVEDFLVDDA
Os04g0493000   (181)  ---------------------------S-P-IGFKDLEWLDDIDLFHVQS
Os05g0204600   (221)  ----PPPPRHTDTATVTTPPPTKRSPRAPAFGGQGGMMDWPLGEFFGGFT
Os06g0152200   (209)  LLASHTSTMAAYSSQISKDSDRIYNLPFTGGNGSDSLHDWHVDEFFS-NS
Os06g0713000   (176)  ----------------------------ISDYLTTICPGWRVEDLLPDDD
Os09g0527900   (180)  -------------------------SNSQTQGVDVSVNNHDSPGVVPTCN
Os12g0209200   (190)  --------------------------AAMEQCYYHGISEPHSSSLTG---
Os04g0540200   (153)  ----------------------------ISEYLTKTLPGWHVEDFLVDEA
   Consensus   (251)                                      L  W V DLF

                        301                                              350
   AT1G06040   (193)  NDKALP----------AAEVPELSVSHLG--------------------H
   AT1G75540   (200)  PTYGFSKSGDDDGVLPYMEPEDDNNTKRNNNNNNNNNNNTVSL------P
   AT1G78600   (197)  DFDQS-YEYMENNGSSKADTSRRG-DSDSSSMMRSAEE------------
   AT2G31380   (183)  DQEALP----------VAEVPELSFSHLA--------------------H
   AT4G10240   (163)  --------------------------------------------------
   AT4G39070   (202)  ----PSC--------VS---YEDNIITNNNN-------------------
 VV_CAN72879   (188)  DFDY--YEFSDMGQSRISSQ------------------------------
 GM_ABB29467   (186)  PHEALA----------AAEVPQLPMTSS----------------------
      PP_STO   (210)  TPFGFCK--IDDGLLPFMDAHDLESNMSSFS-------------------
 St_ABA40448   (187)  ----------------AAEVPELSVPQSS--------------------N
 LE_AAS67368   (187)  ----------------AAEVPELSVPQSS--------------------N
 MS_ABO84497   (180)  APNNVFCN---------KDYYEQNQDLQVIN-------------------
      So_STO   (194)  PRAGA---------------------------------------------
Os01g0202500   (232)  DFSS--GFGFAENGTSKADSGKIG-SMDGSPNGGRSSSSSSSSSAAAAGG
Os02g0606200   (202)  PKGGAAAG--R----TTAEVPELFASQAANDVAYYRPP-----------T
Os02g0646200   (180)  TAEAAAAAAATSSGISANGPCQGVTRIGGLQE------------------
Os04g0493000   (202)  PAKGGS---------TAAEVPELFAS-----------P-----------Q
Os05g0204600   (267)  DFTGGFGFGFGDSGTSKADSGKLGGSTDGSPYYRSSSE-----------D
Os06g0152200   (258)  EFGF------AEHGSSKGDNAKPG-SAGGSPQCRLAEG------------
Os06g0713000   (198)  AFAAAAAQAG-----KEKDERVPFLDADLFDVVAGR-------------P
Os09g0527900   (205)  FEREANK-------------------------------------------
Os12g0209200   (211)  --------------------------------------------------
Os04g0540200   (175)  TAASSSSDGLFQGGLLAQIGGVPDGYAAWAGREQL---------------
   Consensus   (301)                    E
```

**Figure 15 (continued)**

```
                            351                                              400
     AT1G06040    (213)  VHSY-KPMKSNVSHKKPRFETRYDDDDEEHFIVPDLG-------------
     AT1G75540    (244)  SKNLGIWVPQIPQTLPSSYPNQYFSQDNNIQFGMYNKETSPEVVSFAPIQ
     AT1G78600    (233)  -DGEDNNNCLGGETSWAVPQIQSPPTASGLNWP-----------------
     AT2G31380    (203)  AHSYNRPMKSNVPNKKQRLEYRYDD-EEEHFLVPDLG-------------
     AT4G10240    (163)  --------------------------------------------------
     AT4G39070    (218)  SESYRVYDGSSQFHHQGFWDHKPFS-------------------------
   VV_CAN72879    (206)  --------------------------------------------------
   GM_ABB29467    (204)  VGSHKAPKSLLSYKKPR--IEVLDEDDDEHFTVPDLG-------------
        PP_STO    (239)  SESLGLWVPQAPSTPYTSQQYYYPQLVGQSGFKEIKETT--------NMK
   St_ABA40448    (201)  TNIYRTTKYQMPYKKPR--IEIP--DDDEYFTVPDLG-------------
   LE_AAS67368    (201)  TNIYRTTKYQMPYKKSR--IEIP--DDDEYFTVPDLG-------------
   MS_ABO84497    (202)  ---MSDWVPQSQVRFPQLSANSNVPN------------------------
        So_STO    (199)  --------------------------------------------------
  Os01g0202500    (279)  GGGGQDFFGQVPEVHWAVPELPSPPTASGLHWQRDP---RYGG-------
  Os02g0606200    (235)  RTAAAAFTAATGFRQSKKARVELPDDEEDYLIVPDLG-------------
  Os02g0646200    (212)  SAGYPAWMAQQQLCCDGLVAGDASPASRERWVPQMYADQLAAG-------
  Os04g0493000    (221)  PASNMGLYKASGARQSKKPRVEIPDDDEDFFIVPDLG-------------
  Os05g0204600    (306)  DRNADELFGQVPEIQWSVPELPSPPTASGLHWQRHPAATHGGG-------
  Os06g0152200    (289)  -LFVEGLLGQVPDNPWTVPEVPSPPTASGLYWQNNLL-------------
  Os06g0713000    (230)  EKKGGAWAPHVPHLPAWCLDEVPVVVAASAAPAATPVKAKQG--------
  Os09g0527900    (212)  --------------------------------------------------
  Os12g0209200    (211)  --------------------------------------------------
  Os04g0540200    (210)  HSGVAVAADERASRERWVPQMNAEWGAGSKRPRASPPCLYW---------
     Consensus    (351)

                            401                                              450
     AT1G06040    (249)  --------------------------------------------------
     AT1G75540    (294)  NMKQQGQNNKRWYDDGGFTVPQITPPPLSSNKKFRSFW------------
     AT1G78600    (265)  ----------KHFHHHSVFVPDITSSTPYTGSSPNQRVG----KRRRRF-
     AT2G31380    (239)  --------------------------------------------------
     AT4G10240    (163)  --------------------------------------------------
     AT4G39070    (243)  --------------------------------------------------
   VV_CAN72879    (206)  --------------------------------------------------
   GM_ABB29467    (239)  --------------------------------------------------
        PP_STO    (281)  AN--------RRLADDVFTVPQISLPANISSKRSRPLW------------
   St_ABA40448    (234)  --------------------------------------------------
   LE_AAS67368    (234)  --------------------------------------------------
   MS_ABO84497    (225)  --------------------------------------------------
        So_STO    (199)  --------------------------------------------------
  Os01g0202500    (319)  ----------GATDASAVFVPDISSPENPFRCFAAAAAGDHTMKRRRRC-
  Os02g0606200    (272)  --------------------------------------------------
  Os02g0646200    (255)  -----------------SKRSRTSTASSYSYW------------------
  Os04g0493000    (258)  --------------------------------------------------
  Os05g0204600    (349)  ----------GGGPDTTAFVPDICSPDS---CFPATTS-----KRRRQ--
  Os06g0152200    (325)  ----------CPSYDSTMFVPEISSLENSQNNFTVSAGL----KRRRRQF
  Os06g0713000    (272)  -----HVRDSHWSDSDAFAVPEFSPPPPPAKRARPSSQFWCF--------
  Os09g0527900    (212)  --------------------------------------------------
  Os12g0209200    (211)  --------------------------------------------------
  Os04g0540200    (251)  --------------------------------------------------
     Consensus    (401)
```

Figure 15 (continued)

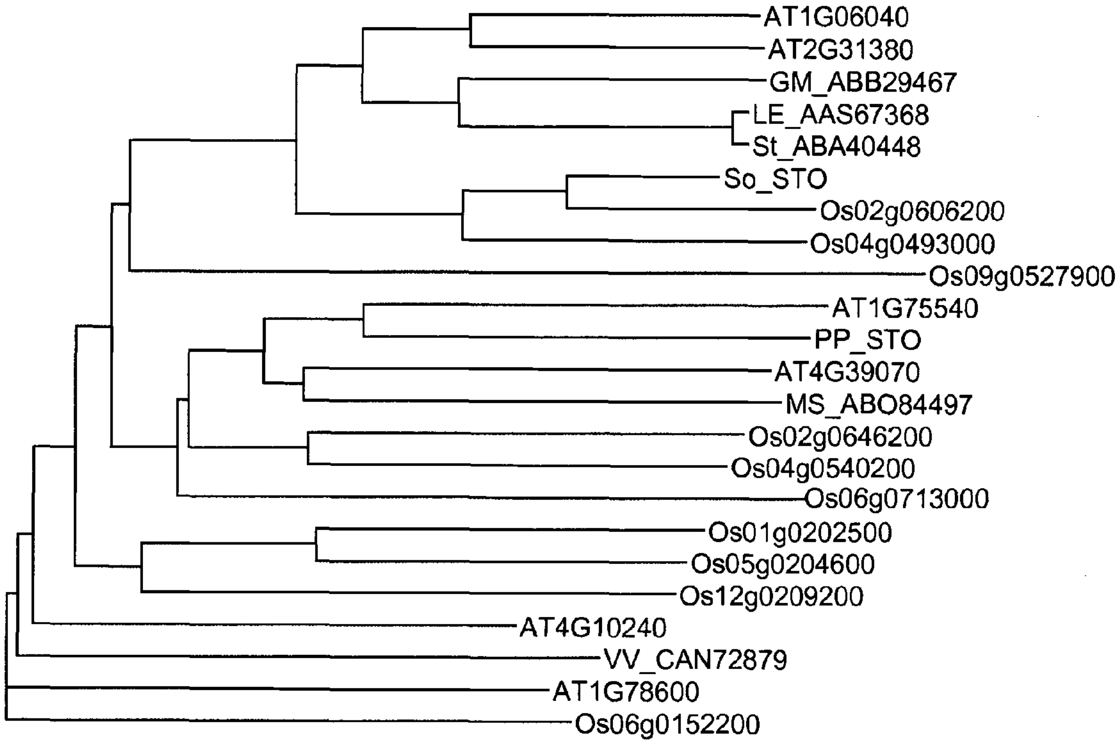
AT1G06040
AT2G31380
GM_ABB29467
LE_AAS67368
St_ABA40448
So_STO
Os02g0606200
Os04g0493000
Os09g0527900
AT1G75540
PP_STO
AT4G39070
MS_ABO84497
Os02g0646200
Os04g0540200
Os06g0713000
Os01g0202500
Os05g0204600
Os12g0209200
AT4G10240
VV_CAN72879
AT1G78600
Os06g0152200

Figure 16

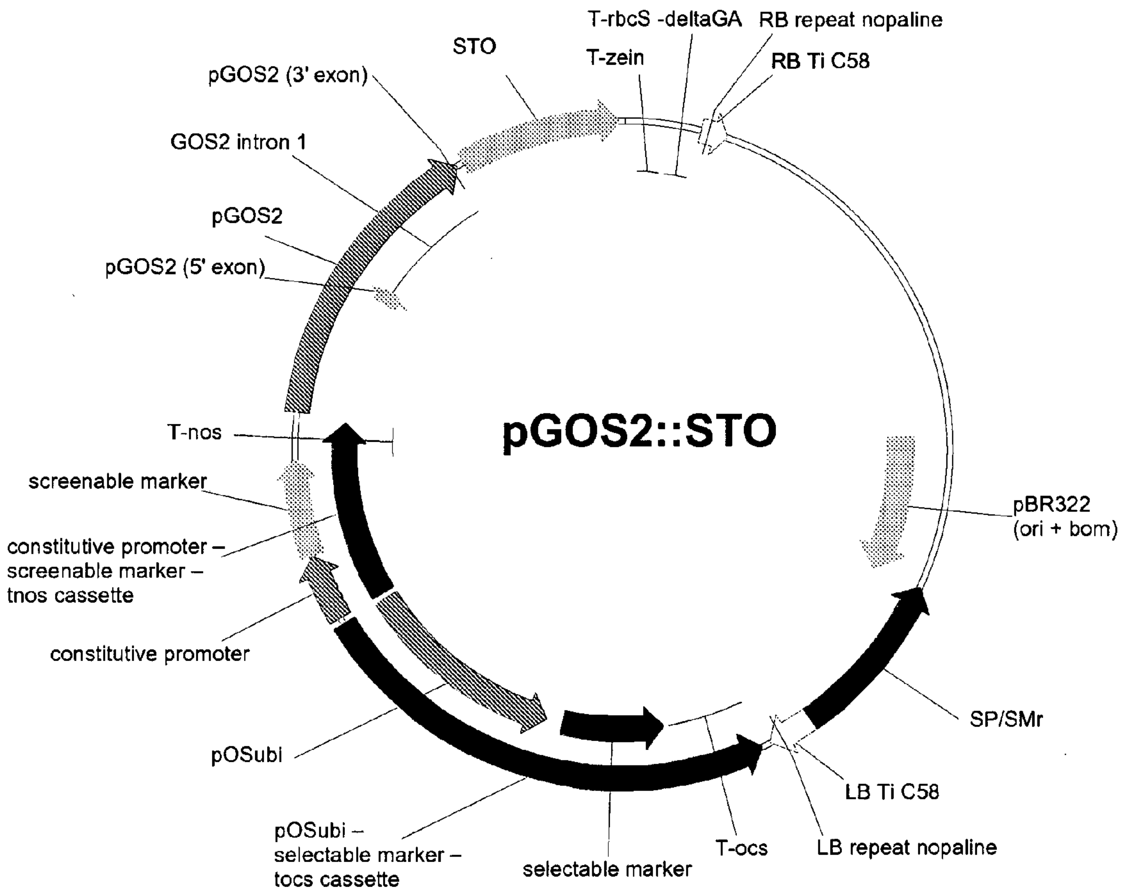
pGOS2::STO
STO
T-rbcS -deltaGA
RB repeat nopaline
T-zein
RB Ti C58
pGOS2 (3' exon)
GOS2 intron 1
pGOS2
pGOS2 (5' exon)
T-nos
screenable marker
constitutive promoter – screenable marker – tnos cassette
constitutive promoter
pOSubi
pOSubi – selectable marker – tocs cassette
selectable marker
T-ocs
LB repeat nopaline
LB Ti C58
SP/SMr
pBR322 (ori + bom)

**Figure 17**

**SEQ ID NO: 168, DNA - *Oryza sativa***

ATGAAGGTGCAGTGCGACGTGTGCGCGGCCGAGGCCGCCTCGGTCTTCTGCTGCGCCGACGAGGCCGCGCTG
TGCGACGCGTGCGACCGCCGCGTCCACAGCGCGAACAAGCTCGCCGGGAAGCACCGCCGATTCTCCCTCCTC
CAACCGTTGGCGTCGTCGTCGTCCGCCCAGAAGCCACCGCTCTGCGACATCTGTCAGGAGAAGAGGGGGTTC
TTGTTCTGCAAGGAGGACAGGGCGATCCTGTGCCGGGAGTGCGACGTCACGGTGCACACCACGAGCGAGCTG
ACGAGGCGGCACGGCCGGTTCCTCCTCACCGGCGTGCGCCTCTCGTCGGCGCCGATGGACTCCCCCGCGCCG
TCGGAGGAAGAGGAGGAGGAAGCAGGGGAGGACTACAGCTGCAGCCCCAGCAGCGTCGCCGGCACCGCCGCG
GGGAGCGCGAGCGACGGGAGCAGCATCTCCGAGTACCTCACCAAGACGCTGCCCGGTTGGCACGTCGAGGAC
TTCCTCGTCGACGAGGCCACCGCCGCCTCCTCCTCCTCAGACGGGCTATTTCAGGGTGGGCTGCTGGCTCAG
ATCGGTGGGGTGCCGGACGGTTACGCGGCGTGGGCCGGCCGGGAGCAGCTGCACAGTGGCGTCGCTGTCGCC
GCCGACGAGCGGGCCAGCCGCGAGCGGTGGGTGCCGCAGATGAACGCGGAGTGGGGCGCCGGCAGCAAGCGA
CCCAGGGCGTCGCCTCCCTGCTTGTACTGGTGA

**SEQ ID NO: 169, protein - *Oryza sativa***

MKVQCDVCAAEAASVFCCADEAALCDACDRRVHSANKLAGKHRRFSLLQPLASSSSAQKPPLCDICQEKRGF
LFCKEDRAILCRECDVTVHTTSELTRRHGRFLLTGVRLSSAPMDSPAPSEEEEEEAGEDYSCSPSSVAGTAA
GSASDGSSISEYLTKTLPGWHVEDFLVDEATAASSSSDGLFQGGLLAQIGGVPDGYAAWAGREQLHSGVAVA
ADERASRERWVPQMNAEWGAGSKRPRASPPCLYW

**SEQ ID NO: 170, DNA - *Oryza sativa***

ATGAAGGTGCTGTGCTCCGCGTGCGAGGCGGCGGAGGCGCGGGTGCTCTGCTGCGCCGACGACGCCGCCCTC
TGCGCGCGCTGCGACCTCCACGTCCACGCCGCCAACCGCCTCGCCGGCAAGCACCACCGCCTCCCCCTCCTC
TCCTCCTCCTCTTCTTCCTCCTCTCCCTCCCCCCCGACCTGCGACATCTGCCAGGACGCCCACGCCTACTTC
TTCTGCGTCGAGGACCGCGCCCTCCTCTGCCGCGCCTGCGACGTCGCCGTCCACACCGCCAACGCCCTCGTC
TCCGCCCACCGCCGCTTCCTCCTCACCGGCGTCCACGTCGGCCTTGACGCCGCCGCCGACGACGACGACAAA
CACCCCCCACACCCCTTGTCGTCGTCGCTGCCGCGCAACACGGCACCGCCCCCGCAGCCGCCGCCGAAGCGC
AGCCCCTCGCCGATCTACAGCGATGACGACGTCATCGACTGGGCCACCGGTGGCCACGACATCGGCATCACC
GGCAACCTGCCCGACTGGTCGCTCGTCGACGAGCAGTTCAACACCCCTGCGCTGCCGCCGGTGGTGACCAAG
ACCCCGCCGAAGCGGGCCTCCCGTGGCCCCGTCACGGCCGGCACCGCCGCGGCGGTGTTCGGCAACCTCGCC
GGCGGATCGCCGGACTGGCCGCTCAACGAGTTCTTCGGCTTCGCCGACTTCAGCTCCGGCTTCGGCTTCGCC
GAGAACGGCACGTCCAAGGCGGACAGCGGCAAGATCGGGAGCATGGACGGCTCGCCGAACGGCGGCAGGTCG
TCGTCGTCGTCCTCCTCCTCCTCCGCCGCCGCCGCCGGCGGCGGCGGCGGCGGCCAGGACTTCTTCGGCCAG
GTGCCGGAAGTTCACTGGGCCGTGCCGGAGCTCCCCTCGCCGCCCACGGCGTCAGGGCTCCACTGGCAACGG
GACCCGCGCTACGGTGGCGGCGCCACCGACGCCAGCGCGGTGTTCGTGCCGGACATCTCCTCGCCGGAGAAC
CCCTTCCGTTGCTTCGCCGCCGCCGCCGCCGGTGACCATACCATGAAACGCCGGAGGAGATGCTAA

**SEQ ID NO: 171, protein - *Oryza sativa***

MKVLCSACEAAEARVLCCADDAALCARCDLHVHAANRLAGKHHRLPLLSSSSSSSPSPPTCDICQDAHAYF
FCVEDRALLCRACDVAVHTANALVSAHRRFLLTGVHVGLDAAADDDDKHPPHPLSSSLPRNTAPPPQPPPKR
SPSPIYSDDDVIDWATGGHDIGITGNLPDWSLVDEQFNTPALPPVVTKTPPKRASRGPVTAGTAAAVFGNLA
GGSPDWPLNEFFGFADFSSGFGFAENGTSKADSGKIGSMDGSPNGGRSSSSSSSSAAAAGGGGGQDFFGQ
VPEVHWAVPELPSPPTASGLHWQRDPRYGGGATDASAVFVPDISSPENPFRCFAAAAAGDHTMKRRRRC

Figure 18

**SEQ ID NO: 172, DNA - *Oryza sativa***

ATGAAGATCCAGTGCGACGCGTGCGAGAGCGCGGCGGCGGCGGTGGTGTGCTGCGCGGACGAGGCGGCGCTG
TGCGCGGCGTGCGACGTGGAGGTGCACGCGGCGAACAAGCTGGCCGGGAAGCACCAGCGGCTGCCGCTGGAG
GCGCTCTCGGCGAGGCTCCCGCGCTGCGACGTGTGCCAGGAGAAGGCGGCGTTCATCTTCTGCGTGGAGGAC
CGCGCGCTCTTCTGCCGCGACTGCGACGAGCCCATCCACGTCCCCGGCACGCTCTCCGGCAACCACCAGCGC
TACCTCGCCACCGGCATCCGCGTCGGCTTCGCCTCCGCCTCGCCCTGCGACGGCGGCAGCGACGCCCATGAC
TCCGACCACCACGCCCCGCCCATGGGCTCCTCCGAGCATCATCACCATCATCAGCAGCCGGCCCCGACCGTC
GCCGTCGACACGCCCTCGCCGCAGTTCCTGCCGCAGGGCTGGGCCGTCGACGAGCTCCTCCAGTTCTCCGAC
TACGAGACCGGCGACAAGCTGCAGAAGGAGTCGTCGCCGCCGCTCGGGTTCCAGGAGCTGGAGTGGTTCGCC
GACATCGACCTGTTCCACAACCAGGCGCCCAAGGGCGGCGCCGCCGCCGGCCGGACGACGGCGGAGGTCCCC
GAGCTCTTCGCTTCGCAGGCGGCCAACGACGTGGCGTACTACAGGCCGCCGACCAGGACCGCCGCCGCCGCC
TTCACCGCGGCCACCGGCTTCCGCCAGAGCAAGAAGGCCCGCGTCGAGCTCCCCGACGACGAGGAGGATTAC
CTCATCGTCCCTGATCTTGGTTGA

**SEQ ID NO: 173, protein - *Oryza sativa***

MKIQCDACESAAAAVVCCADEAALCAACDVEVHAANKLAGKHQRLPLEALSARLPRCDVCQEKAAFIFCVED
RALFCRDCDEPIHVPGTLSGNHQRYLATGIRVGFASASPCDGGSDAHDSDHHAPPMGSSEHHHHHQQPAPTV
AVDTPSPQFLPQGWAVDELLQFSDYETGDKLQKESSPPLGFQELEWFADIDLFHNQAPKGGAAAGRTTAEVP
ELFASQAANDVAYYRPPTRTAAAAFTAATGFRQSKKARVELPDDEEDYLIVPDLG

**SEQ ID NO: 174, DNA - *Oryza sativa***

ATGAAGGTGCAGTGCGACGTGTGCGCGGCCGAGGCGGCGTCGGTGTTCTGCTGCGCCGACGAGGCCGCGCTG
TGCGACGCGTGCGACCACCGGGTGCACCGGGCCAACAAGCTCGCCGGGAAGCACCGCCGGTTCTCGCTGCTC
AACCCCTCGGCGTCCGGCCGCTCGCCGACATCGACGACGGCGCCGCTCTGCGACATCTGCCAGGAGAAGAGG
GGTTTCCTGTTCTGCAAGGAGGACCGGGCGATCCTGTGCCGCGAGTGCGACGTGCCGGTGCACACGGCGAGC
GAGCTCACCATGCGCCACAGCCGGTACCTCCTCACCGGCGTGCGGCTCTCCTCGGAGCCTGCCGCGTCCCCG
GCGCCGCCGTCGGAGGAGGAGAACAGCAGCAGCTTCTGCTGCAGCGCCGACGACGCCGTGCCGGCCCCGGCG
GCGCCCGCCACGAGCCACGGCGGGAGCAGCGGCAGCAGCAGCATCTCCGAGTACCTCACCACGCTGCCCGGG
TGGCACGTCGAGGACTTCCTCGTCGACGACGCCACTGCCGAGGCCGCCGCCGCCGCCGCCGCCACCTCTTCC
GGCATCTCCGCGAACGGGCCGTGTCAGGGGGTAACACGGATCGGAGGGCTGCAAGAATCCGCCGGCTACCCT
GCGTGGATGGCGCAGCAGCAGCTGTGCTGCGACGGCCTCGTCGCCGGCGACGCGTCGCCGGCTAGCCGGGAG
CGGTGGGTGCCGCAGATGTACGCGGATCAGCTTGCCGCCGGCAGCAAGAGATCCAGGACGTCCACTGCTTCT
TCCTACTCCTACTGGTGA

**SEQ ID NO: 175, protein - *Oryza sativa***

MKVQCDVCAAEAASVFCCADEAALCDACDHRVHRANKLAGKHRRFSLLNPSASGRSPTSTTAPLCDICQEKR
GFLFCKEDRAILCRECDVPVHTASELTMRHSRYLLTGVRLSSEPAASPAPPSEEENSSSFCCSADDAVPAPA
APATSHGGSSGSSSISEYLTTLPGWHVEDFLVDDATAEAAAAAAATSSGISANGPCQGVTRIGGLQESAGYP
AWMAQQQLCCDGLVAGDASPASRERWVPQMYADQLAAGSKRSRTSTASSYSYW

**SEQ ID NO: 176, DNA - *Oryza sativa***

ATGAGGATCCAGTGCGACGCGTGCGAGGCCGCGGCGGCCACGGTGGTGTGCTGCGCGGACGAGGCGGCGCTG
TGCGCGCGCTGCGACGTCGAGATCCACGCCGCCAACAAGCTCGCCAGCAAGCACCAGCGCCTCCCGCTCGAC
GCCGCGCTCCCCGCCGCCCTCCCGCGCTGCGACGTCTGCCAGGAGAAGGCGGCG

## Figure 18 (continued)

TTCATCTTCTGCGTGGAGGACAGGGCGCTCTTCTGCCGGGACTGCGACGAGCCCATCCACGTCCCGGGGACG
CTCTCCGGCAACCACCAGCGCTACCTCACCACCGGCATCCGCGTCGGGTTCAGCTCCGTCTGTAGCGCCAAC
GCCGACCACCTCCCGCCGCCAGCGCCCAAGGGGAACTCCAAGCCGCCGGCAAGCGGCATCGCTGCTGCTGCT
GCTCCCAAGCCGGCCGTGTCCGCGGCGGCGCAGGAGGTGCCGTCGTCACCGTTCTTGCCGCCGTCGGGCTGG
GCCGTCGAGGATCTCCTGCAGCTCTCCGACTACGAGTCCAGCGACAAGAAGGGCTCTCCTATTGGGTTCAAG
GATCTGGAGTGGCTCGATGACATCGACCTGTTCCATGTCCAGTCGCCGGCCAAGGGAGGCAGCACGGCGGCG
GAGGTGCCTGAGCTCTTCGCCTCGCCGCAGCCAGCGAGCAACATGGGGCTCTACAAGGCGAGCGGTGCACGC
CAAAGCAAGAAGCCACGGGTGGAGATACCCGATGACGACGAGGACTTCTTCATCGTTCCTGATCTTGGATGA

**SEQ ID NO: 177, protein - *Oryza sativa***

MRIQCDACEAAAATVVCCADEAALCARCDVEIHAANKLASKHQRLPLDAALPAALPRCDVCQEKAAFIFCVE
DRALFCRDCDEPIHVPGTLSGNHQRYLTTGIRVGFSSVCSANADHLPPPAPKGNSKPPASGIAAAAAPKPAV
SAAAQEVPSSPFLPPSGWAVEDLLQLSDYESSDKKGSPIGFKDLEWLDDIDLFHVQSPAKGGSTAAEVPELF
ASPQPASNMGLYKASGARQSKKPRVEIPDDDEDFFIVPDLG

**SEQ ID NO: 178, DNA - *Oryza sativa***

ATGTCGCCTCCTCCTCCACCATATTACCACCACCTCCTCCTCCTCCGCTCCTCGCCCACCACCACTGGAGGA
GGAGCTCGGGTTCTTGCCGCGGCGGAGCTCGCACGCATGAAGCTACTGTGCAGCGCGTGCGAGGCGGCGGAG
GCCAGCGTCCTCTGCTGCGCCGACGAGGCCGCCCTGTGCGCGCGCTGCGACCGCGACATCCACGCCGCCAAC
CGCCTCGCCGGGAAGCACCTCCGCCTCCCTCTCCTCTCCCCCGCCTCCTCCTCCTCCTCCGCCGCCGCC
CTCGCGCCGCCGCCGCCGTCGCCGCCCAAGTGCGACATATGCCAGGAGAGCCACGCGTACTTCTTCTGCCTC
GAGGACCGCGCGCTGCTGTGCCGGAGCTGCGACGTGGCGGTGCACACGGCCAACGCCTTCGTCTCCGCGCAC
CGCCGTTTCCTCCTCACCGGCGTGCAGGTCGGGCAGGAGCAGGACGAGCACTCCCCTGACCCGCCTGAGCCG
TCTCCTCCTCCGCCGCCGCCGCCGCCTGCATCCAAGAGCGACCACCCGGCGCCGCTCTACGGCGAGGGCGGA
GGAGGGTTCAGCTGGGACGCCGCCGACTCGCCGGCCGCGGGCGGCCTCCCCGACTGGTCGGCCGTCGTCGAC
CAGTTCGGCTCCCCGCCGCCGCCGCGCCACACGGACACCGCGACCGTGACGACCCCGCCGCCGACCAAGAGG
AGCCCACGCGCGCCGGCGTTCGGCGGCCAGGGCGGCATGATGGATTGGCCCCTCGGCGAGTTCTTCGGCGGC
TTCACCGACTTCACCGGCGGCTTTGGCTTCGGCTTCGGCGACAGTGGCACCTCCAAGGCTGACAGCGGGAAG
CTGGGAGGGAGCACGGACGGCTCGCCGTACTACCGGTCGTCATCGGAAGATGACCGGAACGCCGACGAGCTC
TTCGGGCAGGTACCAGAGATCCAGTGGTCGGTGCCGGAGCTCCCCTCGCCGCCGACGGCCTCCGGCCTCCAC
TGGCAACGCCATCCAGCCGCCACTCACGGCGGCGGCGGCGGCGGACCCGACACCACCGCCTTCGTCCCCGAC
ATCTGCTCCCCCGACAGCTGCTTCCCGGCCACCACCTCCAAACGCCGGAGGCAATAA

**SEQ ID NO: 179, protein - *Oryza sativa***

MSPPPPPYYHHLLLLRSSPTTTGGGARVLAAAELARMKLLCSACEAAEASVLCCADEAALCARCDRDIHAAN
RLAGKHLRLPLLSPASSSSSSAAALAPPPPSPPKCDICQESHAYFFCLEDRALLCRSCDVAVHTANAFVSAH
RRFLLTGVQVGQEQDEHSPDPPEPSPPPPPPPPASKSDHPAPLYGEGGGGFSWDAADSPAAGGLPDWSAVVD
QFGSPPPPRHTDTATVTTPPPTKRSPRAPAFGGQGGMMDWPLGEFFGGFTDFTGGFGFGFGDSGTSKADSGK
LGGSTDGSPYYRSSSEDDRNADELFGQVPEIQWSVPELPSPPTASGLHWQRHPAATHGGGGGGPDTTAFVPD
ICSPDSCFPATTSKRRRQ

**SEQ ID NO: 180, DNA - *Oryza sativa***

ATGAAGATCCAGTGCAACGCGTGCGGCGCGGCGGAGGCGCGGGTGCTGTGCTGCGCCGACGAGGCAGCGCTC
TGCACGGCGTGCGACGAGGAGGTGCACGCCGCCAACAAGCTCGCCGGGAAGCACCAGCGGGTGCCGCTGCTC
TCCGACGACGGCGGCGCCGCGCCCGCCGCCGCCGCCCCGGCCGTGCCCAAGTGC

## Figure 18 (continued)

GACATCTGCCAGGAGGCTTCTGGATACTTCTTCTGCCTGGAGGACCGTGCACTTCTTTGCAGAGATTGTGAT
GTTTCTATACACACAGTAAACTCCTTTGTTTCAGTACACCAAAGATTCCTACTAACAGGTGTTCAAGTTGGC
CTTGATCCTGCTGATCCAGTTCCACCTGTTGCTGACAAGCATGTTAAGAGTGCTGGTGGTTCAGTGGATTCA
GCAACTAAACATTTGCAAAGGAATCCTACAGACTTATCTGGTGAAAACAGTGCATCTTTGCCCAGCCAAAAT
GTAATCAATGGTAATTATTCTAGGCAGAGTTCTGTTACAATGGCCAAGACAGGACAGGTCAATTGGACTATG
AGCAACAACACAATTAGATCAATAGACCCTCCACCCAAGTATTCATCAGAGGAAAGTCCAGCACTTCTGCTA
GCTAGCCACACTAGCACCATGGCAGCGTACTCCAGTCAAATCAGTAAGGATAGTGATCGGATCTACAACTTA
CCATTCACAGGTGGTAATGGGTCAGATAGTCTACATGATTGGCATGTTGATGAGTTCTTTAGTAACTCAGAA
TTTGGCTTTGCTGAGCATGGTTCTTCTAAGGGTGACAACGCTAAGCCAGGGAGTGCTGGTGGATCTCCGCAG
TGCCGTCTGGCTGAAGGCCTGTTTGTCGAAGGACTTCTAGGTCAAGTGCCTGACAATCCATGGACAGTGCCT
GAGGTCCCCTCGCCACCGACAGCCTCTGGTCTCTATTGGCAAAATAATTTGCTTTGCCCTTCGTACGACAGC
ACCATGTTCGTCCCTGAGATTTCCTCCTTGGAGAACTCTCAGAACAACTTCACTGTATCTGCTGGTTTGAAG
CGCCGAAGGAGGCAGTTTTGA

**SEQ ID NO: 181, protein - *Oryza sativa***
MKIQCNACGAAEARVLCCADEAALCTACDEEVHAANKLAGKHQRVPLLSDDGGAAPAAAAPAVPKCDICQEA
SGYFFCLEDRALLCRDCDVSIHTVNSFVSVHQRFLLTGVQVGLDPADPVPPVADKHVKSAGGSVDSATKHLQ
RNPTDLSGENSASLPSQNVINGNYSRQSSVTMAKTGQVNWTMSNNTIRSIDPPPKYSSEESPALLLASHTST
MAAYSSQISKDSDRIYNLPFTGGNGSDSLHDWHVDEFFSNSEFGFAEHGSSKGDNAKPGSAGGSPQCRLAEG
LFVEGLLGQVPDNPWTVPEVPSPPTASGLYWQNNLLCPSYDSTMFVPEISSLENSQNNFTVSAGLKRRRRQF

**SEQ ID NO: 182, DNA - *Oryza sativa***
ATGCGGGTGCAGTGCGACGTCTGCGCCGCCGAGCCGGCCGCGGTGCTCTGCTGCGCCGACGAGGCCGCGCTC
TGCTCCGCCTGCGACCGCCGCGTCCACCGCGCCAACCGCCTCGCCAGCAAGCACCGCCGCCTCCCGCTCGTC
CACCCGTCCTCCTCCTCCTCCGGCGACGGTGGCGCCGCCGCCGCGCCGCTGTGCGACGTGTGCAGGGAGAAG
AGGGGCCTCGTGTTCTGCGTCGAGGACCGCGCCATCCTGTGCGCCGACTGCGACGAGCCCATCCACTCCGCC
AACGACCTCACCGCCAAGCACACCCGCTTCCTCCTCGTCGGCGCCAAGCTCTCCCCCGCCGCGCTCGCCGAA
CAGCCGCTCCCGTCCTCCGACTGCAGCTCCGACGACGACGCCGCCGCCGCCGCCGCCACCGAGGAGGAGTACCAC
TCCTCCGCCGCATCCACCGGCGCCGCAGTGAGCGCGCCTCTTGACGCCTCCAGCAACGGCGCCGGTGGCGGA
GGAGGAGTAGGAGGGAGCAGCATCTCCGACTACCTCACCACCATCTGCCCCGGCTGGCGCGTCGAGGACCTC
CTCCCCGACGACGACGCCTTCGCCGCCGCCGCCGCGCAGGCGGGGAAAGAGAAGGACGAGCGCGTGCCGTTC
CTCGACGCCGACCTGTTCGACGTGGTCGCCGGCCGGCCGGAGAAGAAGGGCGGCGCGTGGGCGCCGCACGTG
CCGCACCTGCCGGCGTGGTGCCTCGACGAGGTGCCGGTCGTCGTCGCCGCGTCGGCGGCGCCAGCGGCGACA
CCTGTAAAGGCGAAGCAGGGACACGTGCGGGACAGCCACTGGAGCGACAGCGACGCGTTCGCCGTGCCGGAG
TTCTCGCCGCCGCCGCCGCCGGCCAAGAGGGCGCGGCCCAGCTCGCAGTTCTGGTGCTTCTGA

**SEQ ID NO: 183, protein - *Oryza sativa***
MRVQCDVCAAEPAAVLCCADEAALCSACDRRVHRANRLASKHRRLPLVHPSSSSSGDGGAAAAPLCDVCREK
RGLVFCVEDRAILCADCDEPIHSANDLTAKHTRFLLVGAKLSPAALAEQPLPSSDCSSDDDAAAAATEEEYH
SSAASTGAAVSAPLDASSNGAGGGGGVGGSSISDYLTTICPGWRVEDLLPDDDAFAAAAAQAGKEKDERVPF
LDADLFDVVAGRPEKKGGAWAPHVPHLPAWCLDEVPVVVAASAAPAATPVKAKQGHVRDSHWSDSDAFAVPE
FSPPPPPAKRARPSSQFWCF

## Figure 18 (continued)

**SEQ ID NO: 184, DNA - *Oryza sativa***
ATGCGGACGATCTGCGACGTGTGCGAGAGCGCGCCGGCGGTGCTCTTCTGCGTGGCCGACGAGGCCGCGCTC
TGCCGGTCCTGCGACGAGAAGGTGCATATGTGTAACAAGCTTGCTAGGCGGCACGTGAGAGTTGGGCTTGCA
GACCCTAATAAAGTTCAACGCTGTGATATATGTGAAAATGCCCCCGCCTTCTTCTATTGCGAGATAGATGGT
ACATCACTTTGCCTTAGTTGTGATATGACTGTTCATGTTGGTGGGAAACGAACCCATGGAAGATACCTGCTC
CTAAGGCAACGGGTTGAATTTCCAGGAGATAAACCAGGTCATATGGATGATGTTGCTATGCAACAGAAAGAT
CCTGAAAACCGGACGGATCAAAAGAAGGCCCCTCACTCAGTAACAAAGGAGCAAATGGCAAACCATCATAAT
GTGTCTGATGATCCAGCCTCAGATGGCAACTGCGATGACCAGGGTAACATCGATTCCAAAATGATTGATCTT
AATATGAGACCCGTCCGTACTCATGGACAAGGTTCAAACTCACAGACTCAGGGCGTGGATGTTAGCGTCAAC
AATCATGATTCTCCAGGAGTGGTGCCAACATGTAATTTCGAACGAGAAGCCAACAAATAA

**SEQ ID NO: 185, protein - *Oryza sativa***
MRTICDVCESAPAVLFCVADEAALCRSCDEKVHMCNKLARRHVRVGLADPNKVQRCDICENAPAFFYCEIDG
TSLCLSCDMTVHVGGKRTHGRYLLLRQRVEFPGDKPGHMDDVAMQQKDPENRTDQKKAPHSVTKEQMANHHN
VSDDPASDGNCDDQGNIDSKMIDLNMRPVRTHGQGSNSQTQGVDVSVNNHDSPGVVPTCNFEREANK

**SEQ ID NO: 186, DNA - *Oryza sativa***
ATGAAGATCGGGTGCGACGCGTGCGAGCAGGCGGAGGCGGCGGTGCTGTGCTGCGCCGACGAGGCCGCGCTC
TGCCGCCGCTGCGACGCCGCCGTCCACTCCGCCAACAGGCTCGCCGGCAAGCACACCCGCGTCGCGCTCCTC
CTCCCCTCCTCCTCCTCCGCCGCCGCCGGCGACGACGACCACCACCCCACCTGCGACATCTGCCAGGAGAAG
ACGGGCTACTTCTTCTGCCTCGAGGACCGCGCCCTGCTCTGCCGGAGCTGCGACGTCGCCGTCCACACCGCC
ACTGCGCACGCCGCCGCCCACCGCCGCTTCCTCATCACCGGCGTCCGCATCGGCGGCAGCGTCGACGCCGCC
GCCGCGGCCGACGTCATCGTCAGCCCAACAAGCAGCAGCATCGCGCCGGCCGGCTCGGCCAGCAGCAACCAC
GCCGGCGCCGCCGGCAACAACAATGGCCGGTCGCCGGCGCCGGTGAGGTTCTCAGGGGGAGACGGCGGCGTT
GAGCCGGAGCAGCAGTGGCCGTGGAGTGACGTCTTCGCCGCCGACGACGACGATGACGTCAGCGCCGCCATG
GAGCAGTGCTACTATCATGGCATCTCTGAACCTCACTCCTCCAGCCTCACTGGATGA

**SEQ ID NO: 187, protein - *Oryza sativa***
MKIGCDACEQAEAAVLCCADEAALCRRCDAAVHSANRLAGKHTRVALLLPSSSSAAAGDDDHHPTCDICQEK
TGYFFCLEDRALLCRSCDVAVHTATAHAAAHRRFLITGVRIGGSVDAAAAADVIVSPTSSSIAPAGSASSNH
AGAAGNNNGRSPAPVRFSGGDGGVEPEQQWPWSDVFAADDDDDVSAAMEQCYYHGISEPHSSSLTG

**SEQ ID NO: 188, DNA - *Saccharum officinarum***
ATGAAGATCCAGTGCGACGCTTGCGAGGGCGCGGCTGCCACGGTGGTGTGCTGCGCCGACGAGGCCGCGCTG
TGCGCGCGCTGCGACGTCGAGATCCACGCCGCCAACAAGCTCGCCAGCAAGCACCAGCGCCTCCCGCTCGAG
GCGCTCTCGGCCAAGCTCCCGCGCTGCGACGTCTGCCAGGAGAAAGCGGCGTTCATCTTCTGCGTGGAGGAC
CGGGCGCTCTTCTGCCGGGACTGCGACGAGCCCATCCACGTCCCGGGCACGCTCTCCGGGAACCACCAGCGC
TACCTCGCCACCGGCATCCGCGTCGGCTTCGCCTCCGCCTCCGCCTGCAGCGACGGCGCCTGCGACGCCCAC
GACTCCGACCACCACGCCCCGCCCAAGGCCACCATCGAGCCACCGCAGGCCACCGTCTCCGCCGCGGCGCAG
CAGGTGCCCTCGCCGCCGCAGTTCCTGCCGCAGGGCTGGGCCGTCGACGAGCTCCTGCAGTTCTCCGACTAC
GAGTCCAGCGACAAGCTGCACAAGGAGTCCCCGCTCGGGTTCGGAGCTGGAGTGGTTCGCCGACATCGACCT
CTTCCACGAGCAGGCGCCTAA

## Figure 18 (continued)

**SEQ ID NO: 189, protein - *Saccharum officinarum***
MKIQCDACEGAAATVVCCADEAALCARCDVEIHAANKLASKHQRLPLEALSAKLPRCDVCQEKAAFIFCVED
RALFCRDCDEPIHVPGTLSGNHQRYLATGIRVGFASASACSDGACDAHDSDHHAPPKATIEPPQATVSAAAQ
QVPSPPQFLPQGWAVDELLQFSDYESSDKLHKESPLGFGAGVVRRHRPLPRAGA

**SEQ ID NO: 190, DNA - *Arabidopsis thaliana***
ATGAAGATACAGTGTGATGTGTGTGAGAAAGCTCCGGCGACGGTGATTTGTTGCGCCGACGAAGCTGCTCTC
TGTCCTCAATGCGACATCGAGATTCACGCCGCTAACAAACTCGCTAGCAAGCACCAACGTCTTCATCTTAAT
TCCCTCTCCACCAAATTCCCTCGTTGCGATATCTGCCAAGAGAAGGCAGCTTTCATTTTCTGTGTAGAGGAT
AGAGCTCTGCTTTGCAGGGACTGCGATGAATCCATCCACGTGGCTAATTCTCGATCTGCTAATCACCAGAGG
TTCTTAGCCACTGGGATCAAAGTAGCTCTGACCTCAACTATATGTAGTAAAGAAATTGAGAAGAATCAACCT
GAGCCTTCCAACAACCAACAGAAGGCTAATCAGATTCCTGCTAAATCCACAAGCCAGCAGCAACAACAACCT
TCTTCTGCTACTCCACTTCCCTGGGCTGTTGACGATTTCTTTCACTTCTCTGATATTGAATCCACCGACAAG
GTAGTGATGAGAATTGAGATTCAATGTTAG

**SEQ ID NO: 191, protein - *Arabidopsis thaliana***
MKIQCDVCEKAPATVICCADEAALCPQCDIEIHAANKLASKHQRLHLNSLSTKFPRCDICQEKAAFIFCVED
RALLCRDCDESIHVANSRSANHQRFLATGIKVALTSTICSKEIEKNQPEPSNNQQKANQIPAKSTSQQQQP
SSATPLPWAVDDFFHFSDIESTDKVVMRIEIQC

**SEQ ID NO: 192, DNA - *Arabidopsis thaliana***
ATGAAGATACAGTGTGATGTGTGTGAGAAAGCTCCGGCGACGGTGATTTGTTGCGCCGACGAAGCTGCTCTC
TGTCCTCAATGCGACATCGAGATTCACGCCGCTAACAAACTCGCTAGCAAGCACCAACGTCTTCATCTTAAT
TCCCTCTCCACCAAATTCCCTCGTTGCGATATCTGCCAAGAGAAGGCAGCTTTCATTTTCTGTGTAGAGGAT
AGAGCTCTGCTTTGCAGGGACTGCGATGAATCCATCCACGTGGCTAATTCTCGATCTGCTAATCACCAGAGG
TTCTTAGCCACTGGGATCAAAGTAGCTCTGACCTCAACTATATGTAGTAAAGAAATTGAGAAGAATCAACCT
GAGCCTTCCAACAACCAACAGAAGGCTAATCAGATTCCTGCTAAATCCACAAGCCAGCAGCAACAACAACCT
TCTTCTGCTACTCCACTTCCCTGGGCTGTTGACGATTTCTTTCACTTCTCTGATATTGAATCCACCGACAAG
AAAGGACAGCTTGATCTTGGGGCAGGGGAGTTGGATTGGTTTTCAGACATGGGATTCTTCGGTGATCAGATT
AATGACAAGGCTCTTCCTGCAGCTGAAGTTCCTGAGCTTTCTGTTTCGCATTTAGGTCATGTTCATTCATAC
AAACCTATGAAGTCAAATGTTTCACACAAGAAGCCGAGGTTTGAGACCAGATATGATGATGATGATGAGGAA
CACTTCATTGTCCCTGATCTTGGCTAA

**SEQ ID NO: 193, protein - *Arabidopsis thaliana***
MKIQCDVCEKAPATVICCADEAALCPQCDIEIHAANKLASKHQRLHLNSLSTKFPRCDICQEKAAFIFCVED
RALLCRDCDESIHVANSRSANHQRFLATGIKVALTSTICSKEIEKNQPEPSNNQQKANQIPAKSTSQQQQP
SSATPLPWAVDDFFHFSDIESTDKKGQLDLGAGELDWFSDMGFFGDQINDKALPAAEVPELSVSHLGHVHSY
KPMKSNVSHKKPRFETRYDDDDEEHFIVPDLG

**SEQ ID NO: 194, DNA - *Arabidopsis thaliana***
ATGAAGATCAGGTGCGACGTCTGCGATAAAGAAGAAGCGTCGGTGTTTTGCACGGCCGACGAAGCATCTCTC
TGCGGCGGCTGCGACCACCAAGTCCACCACGCTAACAAACTCGCCTCTAAACATCTCCGTTTCTCTCTCCTT
TATCCTTCTTCTTCCAACACCTCCTCTCCTCTCTGCGACATCTGTCAGGATAAAAAAGCTCTGTTGTTCTGT
CAACAAGATAGAGCTATTTTATGCAAAGATTGCGATTCATCGATCCACGCTGCGAACGAACACACAAAGAAA
CACGATAGGTTTCTTCTTACAGGGGTTAAGCTCTCTGCAACATCGTCTGTTTACAAACCTACTTCGAAATCT
TCTTCTTCTTCTTCAAGCAACCAAGATTTCTCTGTC

## Figure 18 (continued)

CCTGGATCATCAATCTCTAATCCTCCTCCTCTCAAGAAACCTCTCTCAGCTCCTCCTCAGAGCAACAAGATC
CAACCCTTTTCGAAGATCAACGGCGGTGATGCGTCGGTGAATCAGTGGGGATCCACAAGCACGATTTCTGAG
TATTTGATGGATACGTTACCTGGTTGGCACGTTGAGGATTTCCTCGATTCCTCTCTTCCTACTTATGGTTTC
TCTAAGAGTGGTGATGATGATGGAGTGTTACCATATATGGAACCAGAAGATGACAACAACACTAAGAGAAAC
AACAACAACAACAACAACAACAACAACAATACAGTGTCACTTCCATCTAAGAATTTAGGGATTTGGGTCCCT
CAGATTCCACAAACTCTTCCTTCTTCATACCCAAATCAATACTTTTCTCAAGACAACAACATACAGTTTGGG
ATGTACAACAAAGAAACATCACCAGAAGTAGTGTCTTTTGCTCCAATACAAAACATGAAACAACAAGGACAG
AACAACAAGAGATGGTATGATGATGGTGGCTTCACTGTCCCACAGATCACTCCTCCTCCTCTTTCTTCTAAT
AAAAAGTTTAGATCTTTCTGGTAA

**SEQ ID NO: 195, protein - *Arabidopsis thaliana***
MKIRCDVCDKEEASVFCTADEASLCGGCDHQVHHANKLASKHLRFSLLYPSSSNTSSPLCDICQDKKALLFC
QQDRAILCKDCDSSIHAANEHTKKHDRFLLTGVKLSATSSVYKPTSKSSSSSSSNQDFSVPGSSISNPPPLK
KPLSAPPQSNKIQPFSKINGGDASVNQWGSTSTISEYLMDTLPGWHVEDFLDSSLPTYGFSKSGDDDGVLPY
MEPEDDNNTKRNNNNNNNNNNNNTVSLPSKNLGIWVPQIPQTLPSSYPNQYFSQDNNIQFGMYNKETSPEVVS
FAPIQNMKQQGQNNKRWYDDGGFTVPQITPPPLSSNKKFRSFW

**SEQ ID NO: 196, DNA - *Arabidopsis thaliana***
ATGAAGATTCAGTGTAACGTTTGTGAGGCGGCGGAAGCGACGGTTCTATGTTGCGCCGACGAGGCTGCTCTT
TGTTGGGCTTGCGATGAGAAAATTCACGCCGCTAATAAACTCGCCGGAAAACATCAGAGAGTCCCTCTCTCT
GCCTCTGCCTCTTCCATACCCAAATGTGACATTTGTCAGGAAGCATCTGGATTCTTCTTTTGTCTGCAAGAT
AGAGCTTTGCTATGTAGGAAATGTGATGTTGCAATCCACACTGTGAATCCTCATGTTTCAGCTCACCAGAGA
TTTCTTCTCACTGGAATCAAAGTTGGTCTTGAATCTATAGACACTGGTCCTTCTACTAAATCCTCACCTACC
AATGATGATAAAACCATGGAGACCAAACCTTTTGTTCAATCTATACCTGAGCCTCAAAAGATGGCCTTCGAT
CATCATCATCACCAGCAGCAGCAGGAACAGCAGGAAGGAGTTATACCGGGAACTAAAGTCAATGATCAGACA
TCGACAAAGCTTCCTCTCGTAAGTAGCGGATCAACTACTGGAAGCATTCCTCAGTGGCAAATAGAGGAGATT
TTCGGGCTAACCGACTTTGATCAGAGCTATGAATACATGGAGAATAATGGATCATCTAAGGCGGATACTAGT
AGACGAGGAGATTCAGACAGTTCTTCGATGATGAGATCTGCAGAAGAAGATGGAGAAGATAACAATAACTGC
TTGGGAGGTGAGACATCATGGGCGGTTCCACAGATTCAGTCTCCACCTACAGCGTCTGGTCTAAACTGGCCT
AAGCATTTTCACCACCACTCTGTGTTTGTTCCGGACATAACTTCTTCAACTCCTTATACCGGTTCATCCCCG
AATCAAAGGGTTGGGAAACGGCGGCGACGGTTCTAG

**SEQ ID NO: 197, protein - *Arabidopsis thaliana***
MKIQCNVCEAAEATVLCCADEAALCWACDEKIHAANKLAGKHQRVPLSASASSIPKCDICQEASGFFFCLQD
RALLCRKCDVAIHTVNPHVSAHQRFLLTGIKVGLESIDTGPSTKSSPTNDDKTMETKPFVQSIPEPQKMAFD
HHHHQQQQEQQEGVIPGTKVNDQTSTKLPLVSSGSTTGSIPQWQIEEIFGLTDFDQSYEYMENNGSSKADTS
RRGDSDSSSMMRSAEEDGEDNNNCLGGETSWAVPQIQSPPTASGLNWPKHFHHHSVFVPDITSSTPYTGSSP
NQRVGKRRRRF

**SEQ ID NO: 198, DNA - *Arabidopsis thaliana***
ATGAAGATACAATGTGATGTGTGTGAGAAAGCTCCGGCCACGCTTATATGTTGTGCTGATGAAGCTGCTCTC
TGCGCTAAATGTGACGTTGAGGTTCATGCTGCTAATAAACTCGCTAGCAAACACCAACGCCTTTTTCTTGAC
TCTCTCTCAACTAAATTCCCTCCCTGCGACATCTGCCTTGAGAAGGCAGCTTTCATATTCTGTGTAGAGGAT
AGGGCTCTGCTCTGCAGAGATTGCGATGAGGCGACCCATGCGCCAAATACTCGCTCTGCTAATCACCAGAGG
TTCTTAGCCACTGGAATCCGAGTTGCTCTTAGTTCCACTAGT

## Figure 18 (continued)

TGCAATCAAGAAGTGGAAAAGAATCACTTTGACCCATCTAATCAGCAGAGTCTCTCTAAACCGCCAACTCAG
CAACCCGCTGCTCCATCTCCTTTGTGGGCTACCGATGAATTCTTCAGCTACTCTGATCTTGACTGCAGTAAT
AAGGAGAAAGAGCAACTCGATCTCGGGGAGCTGGATTGGCTTGCAGAGATGGGTCTGTTTGGTGACCAGCCT
GATCAAGAGGCTCTACCGGTAGCCGAAGTTCCCGAGCTTTCCTTTTCACATTTGGCTCATGCTCATTCCTAC
AACAGACCTATGAAGTCCAATGTACCCAACAAGAAGCAGAGGCTTGAGTACCGGTATGATGATGAAGAAGAG
CACTTCCTAGTCCCCGACCTAGGCTAA

**SEQ ID NO: 199, protein - *Arabidopsis thaliana***
MKIQCDVCEKAPATLICCADEAALCAKCDVEVHAANKLASKHQRLFLDSLSTKFPPCDICLEKAAFIFCVED
RALLCRDCDEATHAPNTRSANHQRFLATGIRVALSSTSCNQEVEKNHFDPSNQQSLSKPPTQQPAAPSPLWA
TDEFFSYSDLDCSNKEKEQLDLGELDWLAEMGLFGDQPDQEALPVAEVPELSFSHLAHAHSYNRPMKSNVPN
KKQRLEYRYDDEEEHFLVPDLG

**SEQ ID NO: 200, DNA - *Arabidopsis thaliana***
ATGAAGATACAATGTGAAGTGTGTGAAAAGGCTGAAGCAGAAGTACTTTGTTGTTCAGATGAAGCTGTACTA
TGCAAGCCATGTGACATTAAGGTTCATGAAGCTAATAAACTTTTCCAAAGACATCACCGAGTCGCCTTACAA
AAAGATGCAGCCTCAGCCACCACAGCTTCTGGAGCTCCTCTATGTGATATCTGCCAGGAGAGAAAAGGGTAC
TTCTTCTGCTTAGAGGATAGAGCAATGCTGTGCAATGATTGTGATGAAGCCATTCACACTTGCAATTCTCAC
CAAAGATTCTTACTTTCTGGAGTACAAGTTTCTGATCAGTCTTTGACTGAAAACTCCGAATGCAGCACTAGT
TTTAGCTCTGAAACTTACCAGATTCAGTCAAAAGTGTCTCTGAATAGTCAGTACTCTAGTGAGGAAACTGAA
GCTGGAAACTCAGGTGAGATAGTACACAAGAATCCTTCTGTAATCTTAAGTCCTTAG

**SEQ ID NO: 201, protein - *Arabidopsis thaliana***
MKIQCEVCEKAEAEVLCCSDEAVLCKPCDIKVHEANKLFQRHHRVALQKDAASATTASGAPLCDICQERKGY
FFCLEDRAMLCNDCDEAIHTCNSHQRFLLSGVQVSDQSLTENSECSTSFSSETYQIQSKVSLNSQYSSEETE
AGNSGEIVHKNPSVILSP

**SEQ ID NO: 202, DNA - *Arabidopsis thaliana***
ATGAAGATTTGGTGTGCTGTTTGTGATAAAGAAGAAGCTTCGGTGTTTTGTTGTGCGGATGAAGCAGCTCTT
TGTAATGGTTGCGATCGCCATGTTCATTTCGCCAATAAACTAGCCGGGAAACATCTCCGGTTCTCTCTCACT
TCTCCTACTTTCAAAGATGCTCCTCTTTGTGATATTTGCGGGGAGAGGCGTGCATTATTATTTTGCCAAGAA
GACAGAGCAATACTATGCAGAGAATGTGACATTCCAATACATCAAGCTAATGAGCACACTAAGAAACACAAT
AGATTCCTCCTTACCGGCGTTAAGATCTCTGCCTCCCCGTCAGCCTACCCAAGAGCCTCCAATTCCAACTCT
GCTGCTGCATTTGGTCGAGCCAAAACCCGACCAAAATCAGTATCGAGCGAGGTCCCGAGCTCGGCCTCCAAT
GAGGTATTTACGAGCTCTTCTTCGACGACCACGAGCAATTGCTATTATGGGATAGAAGAAAACTACCATCAC
GTGAGCGATTCGGGGTCGGGATCGGGTTGTACAGGTAGTATATCCGAGTATTTGATGGAGACATTACCGGGT
TGGAGAGTGGAGGATTTGCTTGAACACCCTTCTTGTGTCTCCTATGAGGATAACATTATTACTAATAACAAT
AACAGTGAGTCTTATAGGGTTTATGATGGTTCTTCACAATTCCATCATCAAGGGTTTTGGGATCACAAACCC
TTCTCTTGA

**SEQ ID NO: 203, protein - *Arabidopsis thaliana***
MKIWCAVCDKEEASVFCCADEAALCNGCDRHVHFANKLAGKHLRFSLTSPTFKDAPLCDICGERRALLFCQE
DRAILCRECDIPIHQANEHTKKHNRFLLTGVKISASPSAYPRASNSNSAAAFGRAKTRPKSVSSEVPSSASN
EVFTSSSSTTTSNCYYGIEENYHHVSDSGSGSGCTGSISEYLMETLPGWRVEDLLEHPSCVSYEDNIITNNN
NSESYRVYDGSSQFHHQGFWDHKPFS

## Figure 18 (continued)

**SEQ ID NO: 204, DNA - *Medicago truncatula***
ATGAAGATCCAGTGTGATGCATGTCACAAACAAGAGGCCTCCTTGTTTTGCCCTGCAGATGAAGCAGCTCTC
TGCAATCAATGTGATCGCAATATCCACTATGCCAACAAGGTCTCTGCTAAACACAAACGCTTCACTCTTCAC
CACCCCACTTCCAAAGACACCCCTCTCTGTGATATCTGCAAGGAGAGGCGTGCATATCTATTTTGCAAAGAA
GACAGAGCAATACTTTGCAGGGAATGTGACATTCCTATCCATGAAATCAACAAACTTACCAAGCAACACAAC
AGGTTTCTTCTCACAGGCGTAAAGATCGGTGCTTCTTCTTCTTGTTCAAATCCAACAATTTCCAATGGCTCA
GAACTAAGAACCTCAAGTCCAAGACCAAGTTCATTTTCAAGTGAAAATAATAGTTGTTCTCAAAGTTCATTC
AAAGAGAACATGGTTTGTGACACAGTTTCAACCAGTAGCATTTCCGAATACTTGATTGAAACGATTCCTGGT
TACTGCATGGAGGACCTTTTTGATGCTTCTTTTGCACCTAATAACGTTTTCTGTAATAAGGATTATTATGAA
CAGAACCAAGATCTTCAAGTTATAAACATGTCTGACTGGGTACCACAATCTCAAGTTAGATTCCCTCAGCTT
AGTGCTAATTCGAATGTTCCCAATTGATTCTTTAGATGGGGTTATGAAAATG

**SEQ ID NO: 205, protein - *Medicago truncatula***
MKIQCDACHKQEASLFCPADEAALCNQCDRNIHYANKVSAKHKRFTLHHPTSKDTPLCDICKERRAYLFCKE
DRAILCRECDIPIHEINKLTKQHNRFLLTGVKIGASSSCSNPTISNGSELRTSSPRPSSFSSENNSCSQSSF
KENMVCDTVSTSSISEYLIETIPGYCMEDLFDASFAPNNVFCNKDYYEQNQDLQVINMSDWVPQSQVRFPQL
SANSNVPN

**SEQ ID NO: 206, DNA - *Lycopersicon esculentum***
ATGAAGATACAGTGTGATGTGTGTGAGAAAGCTCAAGCTACTGTGATTTGCTGTGCTGATGAGGCTGCTCTG
TGTGCAAAATGTGATATTGAAGTTCATGCTGCTAATAAATTAGCAAGCAAGCACCAAAGGCTTCATCTTCAG
TGCCTATCTAACAAGCTTCCTCCTTGTGATATTTGCCAAGATAAAGCAGCCTTCATCTTCTGTGTTGAGGAT
AGAGCTCTCTTTTGCAAGGACTGTGACGAAGCAATTCATTCAGCCAGCAGCCTCGCTAAGAACCACCAACGC
TTCTTAGCCACTGGAATCCGTGTAGCCTTGAGCTCAAGCTGTAATAAGGAATCAGTAAAAAACCAACTGCAG
CCACAACCACCTCAGCAGAATTCCCAACAAGTTGGCTTGAAAATGCCTCCACAGCAGTTGTCCTGTATAACA
TCACCATCTTGGCCTGTCGATGATTTACTAGGATTTCCAGATTATGAGTCGAGTGACAAGAAGGATCTACTT
GAGCTTGGTGAATTTGAGTGGTTAGGGGGCATTGATCTCTTTGGTGAACAAACAGCAGCTGAAGTGCCCGAG
CTATCAGTACCTCAGTCGAGCAATACAAATATTTACAGGACAACCAAATATCAAATGCCTTACAAGAAGTCC
AGAATTGAAATCCCAGATGATGATGAGTATTTTACTGTCCCCGATCTTGGTTGA

**SEQ ID NO: 207, protein - *Lycopersicon esculentum***
MKIQCDVCEKAQATVICCADEAALCAKCDIEVHAANKLASKHQRLHLQCLSNKLPPCDICQDKAAFIFCVED
RALFCKDCDEAIHSASSLAKNHQRFLATGIRVALSSSCNKESVKNQLQPQPPQQNSQQVGLKMPPQQLSCIT
SPSWPVDDLLGFPDYESSDKKDLLELGEFEWLGGIDLFGEQTAAEVPELSVPQSSNTNIYRTTKYQMPYKKS
RIEIPDDDEYFTVPDLG

**SEQ ID NO: 208, DNA - *Solanum tuberosum***
ATGAAGATCCAGTGTGATGTGTGTGAGAAAGCTCAAGCTACTGTGATTTGCTGTGCTGATGAGGCTGCTTTG
TGTGCAAAATGTGATATTGAAGTTCATGCTGCTAATAAATTAGCAAGTAAGCATCAAAGGCTTCATCTTCAG
TGCCTGTCTAACAAGCTTCCTCCTTGTGATATTTGCCAAGATAAAGCAGCCTTCATCTTCTGTGTTGAGGAT
AGAGCTCTCTTTTGCAAGGACTGTGACGAAGCAATTCATTCAGCCAGCAGCCTCGCTAAGAACCACCAACGC
TTCTTAGCCACTGGAATCCGTGTAGCCTTGAGCTCAAGCTGCAATAAGGAAGCAGTAAAAAACCAACTGGAG
CCACAACCACCTCAGCAGAATTCCCAACAAGTTGGCTTGAAAATGCCTCCACAGCAATTGTCCGGTATCACA
TCACCATCTTGGCCTGTTGATGATTTACTAGGATTTCCAGATTATGAATCGAGTGACAAGAAGGATCTACTT
GAGCTTGGTGAATTTGAGTGGTTAGGAGGTATTGATCTCTTTGGTGAACAAACAGCAGCTGAAGTACCCGAG
CTATCAGTACCTCAGTCAAGCAACACAAATATTTACCGGACAACCAAATATCAAATGCCTTACAAGAAGCCC
AGAATTGAAATCCCAGATGATGATGAGTATTTTACCGTCCCAGATCTTGGTTGA

## Figure 18 (continued)

**SEQ ID NO: 209, protein - *Solanum tuberosum***
MKIQCDVCEKAQATVICCADEAALCAKCDIEVHAANKLASKHQRLHLQCLSNKLPPCDICQDKAAFIFCVED
RALFCKDCDEAIHSASSLAKNHQRFLATGIRVALSSSCNKEAVKNQLEPQPPQQNSQQVGLKMPPQQLSGIT
SPSWPVDDLLGFPDYESSDKKDLLELGEFEWLGGIDLFGEQTAAEVPELSVPQSSNTNIYRTTKYQMPYKKP
RIEIPDDDEYFTVPDLG

**SEQ ID NO: 210, DNA - *Populus trichocarpa***
ATGAAGATCCAGTGCGATGTGTGTAACAAAGAAGAGGCATCGGTGTTTTGCACCGCTGACGAGGCAGCTCTT
TGCGACACCTGTGACCACCGTGTTCACCATGCCAACAAGCTTGCTTCAAAGCACCAACGTTTTTCCCTTCTC
CATCCTTCCTCAAAAAACTTCCCCATCTGTGATATCTGCCAGGAGAAACGGGCTTTCTTGTTCTGTCAACAA
GACAGGGCGATTTTATGTAGAGAGTGTGATGGTCCAATACACACAGCAAACGAGCATACCCAGAAGCACAAC
AGGTTTCTTCTCACAGGAGTCAAGCTCTCTGCTACATCTGCTGTTTATATATCTTCTTCCTCTGTCACCAAC
AGTGGTGGTGATCTCGTTCCTGATTCAAAGTCTCAACAGCAGCAGCAGCAGCAGCAATCAATCAAGAAACCT
GTGTTTGATGCTCCAGTGAATTCCAATCCACCTACAGTTCCCAGTACCTTATCTACAAACACAGAAGTAAAC
AAGGGTGGGGATAATTTGGTAACAAATGAAGGGTTTGGTTCAACAACAAGTAGTACTATATCAGAGTACTTG
ATGGAGACTCTTCCTGGCTGGCATGTTGAAGACTTTCTTGATTCCTCTACTACTCCCTTTGGTTTCTGTAAG
ATTGATGATGGTCTATTGCCGTTTATGGATGCTCATGATCTTGAGAGCAACATGAGTTCTTTCTCATCAGAA
AGTTTGGGGCTTTGGGTCCCTCAAGCACCATCTACTCCATACACATCTCAACAGTATTATTATCCACAGTTG
GTAGGGCAAAGTGGGTTCAAGGAGATAAAAGAGACCACAAACATGAAAGCTAACAGAAGGTTGGCAGATGAT
GTCTTCACTGTTCCACAGATCAGCCTCCCGGCCAATATAAGCTCTAAGAGATCTAGGCCCTTATGGTAG

**SEQ ID NO: 211, protein - *Populus trichocarpa***
MKIQCDVCNKEEASVFCTADEAALCDTCDHRVHHANKLASKHQRFSLLHPSSKNFPICDICQEKRAFLFCQQ
DRAILCRECDGPIHTANEHTQKHNRFLLTGVKLSATSAVYISSSSVTNSGGDLVPDSKSQQQQQQQSIKKP
VFDAPVNSNPPTVPSTLSTNTEVNKGGDNLVTNEGFGSTTSSTISEYLMETLPGWHVEDFLDSSTTPFGFCK
IDDGLLPFMDAHDLESNMSSFSSESLGLWVPQAPSTPYTSQQYYYPQLVGQSGFKEIKETTNMKANRRLADD
VFTVPQISLPANISSKRSRPLW

**SEQ ID NO: 212, DNA - *Vitis vinifera***
ATGAAGATACCGTGTGATATATGTGGGAATGTGGAGGCTGAGGTTCTATGTAGTGCTGATGAGGCAGTACTC
TGTTGGGGATGCGATGAAAGGGTGCACACAGCTAACAAGCTGTCCCAGAAGCATCAACGTGTCCCTCTTCTC
AAACACCCACCCTCCACTTCMTCTTCTCAGCTGCCTCCTTGTGATATCTGCCAGGAGAAAAGCGGGTATTTT
TTCTGCCTGGAGGACAGGGCATTACTCTGCAAGAACTGTGATGTTTCAACTCATTCAACAAACTCTTACGTG
TCGTCACACCGACGTTTTGTCATATCAGGAATCAAAGTTGCTCTTCAATCCGTAACTAACAACTACAGAACT
GGCTGCAACAGCAGAACCTACCCTCTCGATATGCCAAACTCAAATAGTTCTTCAGTCAACTTCCCAATGGAT
AGGGAGAAGAAGCCAGAAATGACCACAGAAGTTGCATCCACATCCTCAGACATGGTAGCCATGTTCTCAGGT
GAAATCCATTTGGCAACTGGACCTGAATGGACATTAGATGAAATCCTTGGGAGCAATGATTTTGACTATTAT
GAGTTTTCGGACATGGGGCAATCCAGGATTAGCAGCCAATGA

**SEQ ID NO: 213, protein - *Vitis vinifera***
MKIPCDICGNVEAEVLCSADEAVLCWGCDERVHTANKLSQKHQRVPLLKHPPSTSSSQLPPCDICQEKSGYF
FCLEDRALLCKNCDVSTHSTNSYVSSHRRFVISGIKVALQSVTNNYRTGCNSRTYPLDMPNSNSSSVNFPMD
REKKPEMTTEVASTSSDMVAMFSGEIHLATGPEWTLDEILGSNDFDYYEFSDMGQSRISSQ

## Figure 18 (continued)

**SEQ ID NO: 214, DNA - *Glycine max***
ATGAAAATTCAGTGCGATGTGTGTGAGAAAGCCCCGGCAACCGTGATTTGCTGCGCAGATGAGGCAGCTTTG
TGTGCCAAATGTGACGTTGAAGTTCATGCTGCAAACAAGCTTGCAAGCAAGCACCAGAGGCTTCTCCTTCAA
TCTGTATCTAACAAGCTTCCCAGATGTGACATATGTCAAGATAAGCCAGCTTTCATATTTTGTGTTGAGGAC
AGAGCACTCTTCTGTAAAGACTGTGATGAACCTATTCATTTAGCCAGTAGCCTTTCTGCAAACCACCAGCGC
TTCCTTGCTACTGGTATCCGGGTGGCTTTGGGTTCTAATTGCACCAAAGGCAATGAAAAAGGTCACGTGGAA
CCATCTAAACCAAAAGCACAAGAAGTTCCTGCGAAAATTCCTTCTCAGCAAGTGCCTAGCTTCACATCCTCT
TGGGCAGTTGATGACTTGTTGGAATTAACAGACTTTGAATCACCAGACAAAGTTCAGAAGCAATCCCTTGAG
TTTGGAGAACTTGAATGGCTAGCAGATGTAGGCCTTTTTGGTGAACAGTTTCCTCATGAAGCTTTAGCGGCG
GCTGAAGTTCCTCAGCTTCCAATGACTAGCAGTGTTGGCTCACACAAAGCCCCCAAATCCTTGTTGTCTTAC
AAAAAGCCTAGGATTGAAGTCCTAGATGAAGATGATGATGAGCACTTCACCGTACCAGATCTCGGATAA

**SEQ ID NO: 215, protein - *Glycine max***
MKIQCDVCEKAPATVICCADEAALCAKCDVEVHAANKLASKHQRLLLQSVSNKLPRCDICQDKPAFIFCVED
RALFCKDCDEPIHLASSLSANHQRFLATGIRVALGSNCTKGNEKGHVEPSKPKAQEVPAKIPSQQVPSFTSS
WAVDDLLELTDFESPDKVQKQSLEFGELEWLADVGLFGEQFPHEALAAAEVPQLPMTSSVGSHKAPKSLLSY
KKPRIEVLDEDDDEHFTVPDLG

**SEQ ID NO: 216, DNA - Artificial sequence**
ggggacaagtttgtacaaaaaagcaggcttaaacaatgaaggtgcagtgcga

**SEQ ID NO: 217, DNA - Artificial sequence**
ggggaccactttgtacaagaaagctgggttcaccagtacaagcagggag

**SEQ ID NO: 218, DNA - *Oryza sativa***
AATCCGAAAAGTTTCTGCACCGTTTTCACCCCCTAACTAACAATATAGGGAACGTGTGCTAAATATAAAATG
AGACCTTATATATGTAGCGCTGATAACTAGAACTATGCAAGAAAAACTCATCCACCTACTTTAGTGGCAATC
GGGCTAAATAAAAAAGAGTCGCTACACTAGTTTCGTTTTCCTTAGTAATTAAGTGGGAAAATGAAATCATTA
TTGCTTAGAATATACGTTCACATCTCTGTCATGAAGTTAAATTATTCGAGGTAGCCATAATTGTCATCAAAC
TCTTCTTGAATAAAAAAATCTTTCTAGCTGAACTCAATGGGTAAAGAGAGAGATTTTTTTTAAAAAAATAGA
ATGAAGATATTCTGAACGTATTGGCAAAGATTTAAACATATAATTATATAATTTTATAGTTTGTGCATTCGT
CATATCGCACATCATTAAGGACATGTCTTACTCCATCCCAATTTTTATTTAGTAATTAAAGACAATTGACTT
ATTTTTATTATTTATCTTTTTTCGATTAGATGCAAGGTACTTACGCACACACTTTGTGCTCATGTGCATGTG
TGAGTGCACCTCCTCAATACACGTTCAACTAGCAACACATCTCTAATATCACTCGCCTATTTAATACATTTA
GGTAGCAATATCTGAATTCAAGCACTCCACCATCACCAGACCACTTTTAATAATATCTAAAATACAAAAAAT
AATTTTACAGAATAGCATGAAAAGTATGAAACGAACTATTTAGGTTTTTCACATACAAAAAAAAAAAGAATT
TTGCTCGTGCGCGAGCGCCAATCTCCCATATTGGGCACACAGGCAACAACAGAGTGGCTGCCCACAGAACAA
CCCACAAAAAACGATGATCTAACGGAGGACAGCAAGTCCGCAACAACCTTTTAACAGCAGGCTTTGCGGCCA
GGAGAGAGGAGGAGAGGCAAAGAAAACCAAGCATCCTCCTTCTCCCATCTATAAATTCCTCCCCCCTTTTCC
CCTCTCTATATAGGAGGCATCCAAGCCAAGAAGAGGGAGAGCACCAAGGACACGCGACTAGCAGAAGCCGAG
CGACCGCCTTCTCGATCCATATCTTCCGGTCGAGTTCTTGGTCGATCTCTTCCCTCCTCCACCTCCTCCTCA
CAGGGTATGTGCCTCCCTTCGGTTGTTCTTGGATTTATTGTTCTAGGTTGTGTAGTACGGGCGTTGATGTTA
GGAAAGGGGATCTGTATCTGTGATGATTCCTGTTCTTGGATTTGGGATAGAGGGGTTCTTGATGTTGCATGT
TATCGGTTCGGTTTGATTAGTAGTATGGTTTTCAATCGTCTGGAGAGCTCTATGGAAATGAAATGGTTTAGG
GATCGGAATCTTGCGATTTTGTGAGTACCTTTTGTTTGAGGTAAAATCAGAGCACCGGTGATTTTGCTTGGT
GTAATAAAGTAC

## Figure 18 (continued)

GGTTGTTTGGTCCTCGATTCTGGTAGTGATGCTTCTCGATTTGACGAAGCTATCCTTTGTTTATTCCCTATT
GAACAAAAATAATCCAACTTTGAAGACGGTCCCGTTGATGAGATTGAATGATTGATTCTTAAGCCTGTCCAA
AATTTCGCAGCTGGCTTGTTTAGATACAGTAGTCCCCATCACGAAATTCATGGAAACAGTTATAATCCTCAG
GAACAGGGGATTCCCTGTTCTTCCGATTTGCTTTAGTCCCAGAATTTTTTTCCCAAATATCTTAAAAAGTC
ACTTTCTGGTTCAGTTCAATGAATTGATTGCTACAAATAATGCTTTTATAGCGTTATCCTAGCTGTAGTTCA
GTTAATAGGTAATACCCCTATAGTTTAGTCAGGAGAAGAACTTATCCGATTTCTGATCTCCATTTTTAATTA
TATGAAATGAACTGTAGCATAAGCAGTATTCATTTGGATTATTTTTTTATTAGCTCTCACCCCTTCATTAT
TCTGAGCTGAAAGTCTGGCATGAACTGTCCTCAATTTTGTTTTCAAATTCACATCGATTATCTATGCATTAT
CCTCTTGTATCTACCTGTAGAAGTTTCTTTTTGGTTATTCCTTGACTGCTTGATTACAGAAAGAAATTTATG
AAGCTGTAATCGGGATAGTTATACTGCTTGTTCTTATGATTCATTTCCTTTGTGCAGTTCTTGGTGTAGCTT
GCCACTTTCACCAGCAAAGTTC

**SEQ ID NO: 219, protein - Artificial sequence**
MKVQCDVCAAEAASVFCCADEAALCDACDRRVHSANKLAGKHRRFSL

**SEQ ID NO: 220, protein - Artificial sequence**
QKPPLCDICQEKRGFLFCKEDRAILCRECDVTVHTTSELTRRHGRFLL

**SEQ ID NO: 221, protein - Artificial sequence**
CXXCXXXXXXXXXXXXXXXXCXXC

SEQ ID NO 222: Zea mays STO
cccgtccggtctagctaggactagtaagtagcataccattaccatcctccgcgcggctagctccgactcgct
ttcatccatacgattgcccggagctcaagccctgagctgagccgccagagcgcgcgcgatcatcgcttgagg
gaaaagcagagtggagtggccgagatgaaggtgcagtgcgacgtgtgcgcggccgaggcggccgaggtgttc
tgctgcgccgacgaggcggcgctgtgcgacgcgtgcgaccgccgcgtgcaccgcgccaacaagctcgccggc
aagcaccgccgcttctcgctgctcagcccggcgccaccgccgccgccgccgctctgcgacatctgccaggac
aagcgggggctcctgttctgcaaggaggaccgcgccatcctgtgccgcgactgcgacgtgtcggtgcacacg
gccagcgacctgaccatgcgccacgcccggttcctgctcacgggcgtccgcctctccgccgagcccgccgcc
gcgtgcccggcgccggaggatgaggaggaggaggacgacgagaacagcagcggcagcttctgctgcagcgcc
ggcgacgcagccgcacatcctcctcctcttccgtcgtcggcgcccgccaccagccacgggagcgacagcagc
agcatctccgagtacctcaccaagacgctgcccgggtggcacgtcgaggacttcctcattgacgacgcgtcc
gctggcgacgtcggcgcctgctcggatggcctctatcagggacaaaatggacagatcagtggggtgctgcaa
gaagcttacctgccgtggacggagcgggagcaggtgcaaactgacgtcgcagacgagcgggccagctgggag
cggtgggtgccacagatgcatgcggagttcggcggcggcggcaagcgacctagagcgtcgccttcgcctccc
tgttcgtactggtgattggtgaaaagttcgccgtcttcagcaagatgatttgtattgggctaattagtagag
ctactgtaggaacgaccgaaaaatgagccaaaaaagtaacggcctccatcgagctcgttttaaattattctc
tgcctaaagccaaactttcagaaaggagaacggtattttcctgttcgattaattaggtcgatttcgatcgaa
ctcgttccaaaatcccaattgtttgctacctaaagacagacttcctgaatatagttgagtaacaggggaatt
tgccgaaggaaaaaaaaaaaa

SEQ ID NO 223: Zea mays STO
mkvqcdvcaaeaaevfccadeaalcdacdrrvhranklagkhrrfsllspappppplcdicqdkrgllfck
edrailcrdcdvsvhtasdltmrharflltgvrlsaepaaacpapedeeeeddenssgsfccsagdaaahpp
plpssapatshgsdsssiseyltktlpgwhvedfliddasagdvgacsdglyqgqngqisgvlqeaylpwte
reqvqtdvaderaswerwvpqmhaefggggkrpraspsppcsyw

Figure 18 (continued)

MVSALFRTI**LVTGGAGYIGSH**TVLQLLQLGFRVVVLDNLDNASELAILRVRELAGH
NANNLDFRKVDLRDKQALDQIFSSQRFEAVIHFAGLKAVGESVQKPLLYYDNNLIG
TITLLQVMAAHGCTKLVFSSSATVYGWPKEVPCTEESPLCAMNP**ygrtk**LVIEDMC
RDLHASDPNWKIILLRYFNPVGAHPSGYIGEDPCGIPNNLMPFVQQVAVGRRPALT
VYGTDYNTK*DGTGVRDYIHV*VDLADGHIAALRKLYEDSDRIGCEVYNLGTGKGTSV
LEMVAAFEKASGKKIPLVFAGRRPGDAEIVYAQTAKAEKELKWKAKYGVEEMCRDL
WNWASKNPYGYGSPDSSN

**Figure 19**

| | | 1 50 |
|---|---|---|
| Os04g0618200 | (1) | -------------------------------------------------- |
| Os05g0595100 | (1) | -------------------------------------------------- |
| Os07g0139400 | (1) | -------------------------------------------------- |
| Os08g0374800 | (1) | -------------------------------------------------- |
| Os09g0323000 | (1) | -------------------------------------------------- |
| Os09g0504000 | (1) | MPADAAAKGMKLERYASGAGAMLLLRRAASGKVVSASSHLLFRATVLATM |
| Os09g0526700 | (1) | -------------------------------------------------- |
| AT1G12780_UGE1 | (1) | -------------------------------------------------- |
| AT1G63180_UGE3 | (1) | -------------------------------------------------- |
| AT1G64440_UGE4 | (1) | -------------------------------------------------- |
| AT4G10960_UGE5 | (1) | -------------------------------------------------- |
| AT4G23920_UGE2 | (1) | -------------------------------------------------- |
| Pt_lcl_scaff_5422.2 | (1) | -------------------------------------------------- |
| Pt_lcl_scaff_I.2996 | (1) | -------------------------------------------------- |
| Pt_lcl_scaff_I.773 | (1) | -------------------------------------------------- |
| Pt_lcl_scaff_III.1133 | (1) | -------------------------------------------------- |
| Pt_lcl_scaff_III.961 | (1) | -------------------------------------------------- |
| Pt_lcl_scaff_VI.203 | (1) | -------------------------------------------------- |
| Pt_lcl_scaff_XI.1329 | (1) | -------------------------------------------------- |
| Pt_lcl_scaff_XVI.140 | (1) | -------------------------------------------------- |
| Ot08g01550 | (1) | -------------------------------------------------- |
| SC_GAL10_P04397 | (1) | -------------------------------------------------- |
| Ec_GALE | (1) | -------------------------------------------------- |
| OS_UGE2 | (1) | -------------------------------------------------- |
| Consensus | (1) | |

**Figure 20**

```
                                51                                               100
           Os04g0618200   (1)  ------------------------MDFGDSRRKPNVVGKFTVAVALTVMC
           Os05g0595100   (1)  --------------------------------------------------
           Os07g0139400   (1)  --MLPTNRNRPQQRPARSWYFISDMDFSDPKRKPRYLSKILMVALLTAMC
           Os08g0374800   (1)  --------------------------------------------------
           Os09g0323000   (1)  --------------------------------------------------
           Os09g0504000  (51)  ALVFLFTFHYPSLLSRSFTLSSGAGAGEGGAAAHASHRSLLMSSSSASAS
           Os09g0526700   (1)  --------------------------------------------------
         AT1G12780_UGE1   (1)  --------------------------------------------------
         AT1G63180_UGE3   (1)  --------------------------------------------------
         AT1G64440_UGE4   (1)  --------------------------------------------------
         AT4G10960_UGE5   (1)  --------------------------------------------------
         AT4G23920_UGE2   (1)  --------------------------------------------------
     Pt_lcl_scaff_5422.2  (1)  --------------------------------------------------
     Pt_lcl_scaff_I.2996  (1)  --MLSFGRTRTQPR-SNRSMSLGGMDFSDPKRKNNVVGKILLAASLTAVC
      Pt_lcl_scaff_I.773  (1)  --------------------------------------------------
   Pt_lcl_scaff_III.1133  (1)  --------------------------------------------------
    Pt_lcl_scaff_III.961  (1)  --------------------------------------------------
     Pt_lcl_scaff_VI.203  (1)  -----------------MVHKGSGMDFLDSRRKSNSAGKVIAVAFFIAVC
    Pt_lcl_scaff_XI.1329  (1)  --MLNFGRTRAQTR-SNRSISLGGMDYSDPKRKNNVVGKILLAATLTALC
    Pt_lcl_scaff_XVI.140  (1)  ------------------------MDYLDSRRKSRCAGKIIAAALCIT--
             Ot08g01550   (1)  --------------------------------------------------
        SC_GAL10_P04397   (1)  --------------------------------------------------
                Ec_GALE   (1)  --------------------------------------------------
                OS_UGE2   (1)  --------------------------------------------------
              Consensus  (51)

                                                           epimerase domain
                                101                                              150
           Os04g0618200  (27)  IIVLKQ--SPGFTSTSVFSRHEIGVTHVLVTGGAGYIGSHATLRLLRDNY
           Os05g0595100   (1)  -------------------MVSALLRTILVTGGAGYIGSHTVLQLLQLGF
           Os07g0139400  (49)  VVMLTQP-PCHRRTPSVFSIHEPGVTHVLVTGGAGYIGSHAALRLLKDSF
           Os08g0374800   (1)  --------------MAVEKTVPGGVRTVLVTGGAGYIGSHAVLQLLLAGF
           Os09g0323000   (1)  --------MAGGEVVAVAAAAAGTSRTVLVTGGAGYIGSHTVLQLLAAGF
           Os09g0504000 (101)  AASVYGGAAWEKEVRRSAKPRKDGGIAVLVTGAAGFVGTHCSLALRARGD
           Os09g0526700   (1)  MVSGGGVAAENGEMVGNGEGRKGAGASVLVTGGAGYIGTHTVLRLLEKGF
         AT1G12780_UGE1   (1)  -------------------MGSSVEQNILVTGGAGFIGTHTVVQLLKDGF
         AT1G63180_UGE3   (1)  -------------------MGSSVEQNILVTGGAGFIGTHTVVQLLNQGF
         AT1G64440_UGE4   (1)  -----------------------MVGNILVTGGAGYIGSHTVLQLLLGGY
         AT4G10960_UGE5   (1)  ----------------------MMARNVLVSGGAGYIGSHTVLQLLLGGY
         AT4G23920_UGE2   (1)  -----------------------MAKSVLVTGGAGYIGSHTVLQLLEGGY
     Pt_lcl_scaff_5422.2  (1)  --------------------------MILVTGGAGFIGSHSCVELAAAGE
     Pt_lcl_scaff_I.2996 (48)  IIMLKQ--SPTFNSPSPFSLREDGVIHVLVTGGAGYIGSHAALRLLKDGY
      Pt_lcl_scaff_I.773  (1)  -----------------------MAYNILVTGGAGYIGSHTVLQLLLGGY
   Pt_lcl_scaff_III.1133  (1)  -----------------------MAKSILVTGGAGYIGSHTVLQLLLGGY
    Pt_lcl_scaff_III.961  (1)  ----------------------MAGQTILVTGGAGFIGTHTVVQLLKEGF
     Pt_lcl_scaff_VI.203 (34)  IVMLKQVYSPSYTSPDMFSQHELGVTHVLVTGGAGYIGSHAALRLLKDSY
    Pt_lcl_scaff_XI.1329 (48)  IIMLKQ--SPTFYSPSPFSLHEEGVIHVLVTGGAGYIGSHAALRLLKDGY
    Pt_lcl_scaff_XVI.140 (25)  -----------------FSQHELGVTHVLVTGGAGYIGSHAALRLLKDSY
             Ot08g01550   (1)  -----------------------MTWNVLVTGGAGYIGSHTCVRLLQAGA
        SC_GAL10_P04397   (1)  --------------MTAQLQSESTSKIVLVTGGAGYIGSHTVVELIENGY
                Ec_GALE   (1)  -------------------------MAILVTGGAGYIASHTILSLIEQGN
                OS_UGE2   (1)  -------------------MVSALFRTILVTGGAGYIGSHTVLQLLQLGF
              Consensus (101)                             VLVTGGAGYIGSHTVLQLL  GY
                                                                 Motif 1
                                                              Motif 3
```

Figure 20 (continued)

```
                                151                                              200
           Os04g0618200   (75)  RVTIVDNLSRGNMGAVRVLQRLFP--EPGRLQFIYADLGDAKAVNKIFSE
           Os05g0595100   (32)  RVVVLDNLDNASELAILRVRELAG-HNANNLDFRKVDLRDKQALDQIFSS
           Os07g0139400   (98)  RVTIVDNLSRGNMGAIKVLQNLFS--EPGRLQFIYADLGDPKAVNRIFAE
           Os08g0374800   (37)  RAVVVDNLNNSSELAVRRVAALAG-DHSRNLAFHKVDLRDKGALEKVFAS
           Os09g0323000   (43)  RVVVADSLGNSSELAVRRVAALAG-DKARNLSLHKVDIRDKGGLEKVFSS
           Os09g0504000  (151)  GVLGLDNFNAYYDPELKRARQRLLAG--RGVLVLDADINDALLLEKLFDL
           Os09g0526700   (51)  AVTVVDNFHNSVPEALDRVRLIAGAALSARLDFIAGDLKSKDDMEKVFAA
        AT1G12780_UGE1    (32)  KVSIIDNFDNSVIEAVDRVRELVGPDLSKKLDFNLGDLRNKGDIEKLFSK
        AT1G63180_UGE3    (32)  KVTIIDNLDNSVVEAVHRVRELVGPDLSTKLEFNLGDLRNKGDIEKLFSN
        AT1G64440_UGE4    (28)  NTVVIDNLDNSSLVSIQRVKDLAG-DHGQNLTVHQVDLRDKPALEKVFSE
        AT4G10960_UGE5    (29)  SVVVVDNLDNSSAVSLQRVKKLAA-EHGERLSFHQVDLRDRSALEKIFSE
        AT4G23920_UGE2    (28)  SAVVVDNYDNSSAASLQRVKKLAG-ENGNRLSFHQVDLRDRPALEKIFSE
    Pt_lcl_scaff_5422.2   (25)  PYLIYDNFSNSSPDVLERMERITG----RRPLCVEGDVRDRAALDRLFAE
    Pt_lcl_scaff_I.2996   (96)  RVTIVDNLSRGNLGAVKVLQELFP--EPGRLQFIYADLGEPKTVNSIFSQ
     Pt_lcl_scaff_I.773   (28)  NTVVVDNLDNASDIALKRVKELAG-DFGKNLVFHQVDLRDKPALENVFAE
  Pt_lcl_scaff_III.1133   (28)  SIVVVDNLDNSSDIALKRVKELAG-DFGKNLVFHQVDLRDKPALEKIFAR
   Pt_lcl_scaff_III.961   (29)  KVSIIDNLDNSVTEAVDRVKEVVGPQLSKNLEFNLGDLRNKDDLEKLFSR
    Pt_lcl_scaff_VI.203   (84)  RVTIVDNLSRGNLGAVKVLQELFP--EPGRLQFIYADLGDAKAVNKIFAE
   Pt_lcl_scaff_XI.1329   (96)  RVTIVDNLSRGNIGAVKVLQELFP--EPGRLQFIYADLGDPKTVNIIFSQ
   Pt_lcl_scaff_XVI.140   (58)  RVTKVDNLSRGNLGAVKVLQDLFP--EPGRLQFIYADLGDAKAVNKIFAE
            Ot08g01550    (28)  RVTVVDNFDNSCAESLKRVRNIVGEDAGARLTHHEVDCCDKVALDGVFAS
       SC_GAL10_P04397    (37)  DCVVADNLSNSTYDSVARLEVLTK----HHIPFYEVDLCDRKGLEKVFKE
               Ec_GALE    (26)  EVIIIDNLSNSYYDSLLKIKEITG----CDCLFYKGDILDKKLLLKIFDE
               OS_UGE2    (32)  RVVVLDNLDNASELAILRVRELAG-HNANNLDFRKVDLRDKQALDQIFSS
             Consensus   (151)  RVVVVDNL NS   AVKRVKEL G     RL F  ADLRDK ALEKIFS
```

```
                                201                                              250
           Os04g0618200  (123)  N--AFDAVMHFAAVAYVGESTLEPLRYYHNITSNTLTVLEAMAAYNVKTL
           Os05g0595100   (81)  Q--RFEAVIHFAGLKAVGESVQKPLLYYDNNLIGTITLLQVMAAHGCTKL
           Os07g0139400  (146)  N--AFDAVMHFAAVAYVGESTLEPLRYYHNITSNTLVVLEAMAAHNVRTL
           Os08g0374800   (86)  T--RFDAVVHFAGLKAVGESVQKPLLYYDNNVNGTVNLLEVMSAHGCKKL
           Os09g0323000   (92)  T--RFDAVVHFAGLKAVGESVQKPLLYYDHNVAGTIILLEVMAAHGCKKL
           Os09g0504000  (199)  VP--FTHVLHLAAQAGVRYAMEAPQTYVASNVAGLVTVLEVAAKHADPQP
           Os09g0526700  (101)  K--RYDAVIHFAGLKAVGESVAHPQMYYENNVAGTMNLYSAMTKYGCKKI
        AT1G12780_UGE1    (82)  Q--RFDAVIHFAGLKAVGESVENPRRYFDNNLVGTINLYETMAKYNCKMM
        AT1G63180_UGE3    (82)  Q--RFDAVIHFAGLKAVGESVGNPRRYFDNNLVGTINLYETMAKYNCKMM
        AT1G64440_UGE4    (77)  T--KFDAVMHFAGLKAVGESVAKPLLYYNNNLIATITLLEVMAAHGCKKL
        AT4G10960_UGE5    (78)  T--KFDAVIHFAGLKAVGESVEKPLLYYNNNLVGTITLLEVMAQHGCKNL
        AT4G23920_UGE2    (77)  T--KFDAVIHFAGLKAVGESVEKPLLYYNNNIVGTVTLLEVMAQYGCKNL
    Pt_lcl_scaff_5422.2   (71)  H--SIREVIHFAALKAVGESVAQPLRYYEHNVGGTVALLQAMRTAGVRSL
    Pt_lcl_scaff_I.2996  (144)  N--AFDAVMHFAAVAYVGESTVYPLKYYHNITSNTLVVLESMAANDVKTL
     Pt_lcl_scaff_I.773   (77)  T--KFDAVIHFAGLKAVGESMQKPLLYFNNNLIGTITLLEVMAAHGCKQV
  Pt_lcl_scaff_III.1133   (77)  T--KFDAVIHFAGLKAVGESVQKPLLYFNNNLIGTITLLEVMTSHGCKQL
   Pt_lcl_scaff_III.961   (79)  T--KFDAVIHFAGLKAVGESVANPRRYFDNNLVGTINLYEVMAKYNCKKM
    Pt_lcl_scaff_VI.203  (132)  N--AFDAVMHFAAVAYVGESTIEPLRYYHNITSNTLVVLEAMAAHNVKTL
   Pt_lcl_scaff_XI.1329  (144)  N--AFDAVMHFAAVAYVGESTMEPLKYYHNITSNTLVVLEAMAANDVKTL
   Pt_lcl_scaff_XVI.140  (106)  N--AFDAVMHFAAVAYVGESTMEPLRYYHNITSNTLVVLEAMAAHKVKTL
            Ot08g01550    (78)  AGVTFDAVIHFAGLKAVGESVAEPMKYYENNIVSTLVLCETMAKHGCKTI
       SC_GAL10_P04397    (83)  Y--KIDSVIHFAGLKAVGESTQIPLRYYHNNILGTVVLLELMQQYNVSKF
               Ec_GALE    (72)  N--NITAVMHFAGAKSVSESVIRPLQYYRNNVSGTFSLVEAMEESGVENL
               OS_UGE2    (81)  Q--RFEAVIHFAGLKAVGESVQKPLLYYDNNLIGTITLLQVMAAHGCTKL
             Consensus   (201)     KFDAVIHFAGLKAVGESV  PLRYY NNLVGTI LLEVMAAHGCK L
                                       Motif 5
```

## Figure 20 (continued)

```
                                251                                               300
           Os04g0618200  (171)  IYS--STCATYGE----PDTMPITEATPQNP--INPYGKAKKMAED-IIL
           Os05g0595100  (129)  VFS--SSATVYGW----PKEVPCTEESPLCA--MNPYGRTKLVIED--MC
           Os07g0139400  (194)  IYS--STCATYGE----PEKMPITEGTPQFP--INPYGKAKKMAED-IIL
           Os08g0374800  (134)  VFS--SSAAVYGS----PKNSPCTEEFPLTP--NNPYGKTKLVVED--IC
           Os09g0323000  (140)  VFS--SSAAVYGS----PKNSPCTEEFPLTP--HNPYGRTKLIAEE--IC
           Os09g0504000  (247)  AIVWASSSSVYGL----NTDAP-FSEEHRTDRPASLYAATKKAGEA-IAH
           Os09g0526700  (149)  VFS--SSATVYGQ----PEKTPCVEDSKLSA--LNPYGTTKLVLEN--YF
         AT1G12780_UGE1  (130)  VFS--SSATVYGQ----PEKIPCMEDFELKA--MNPYGRTKLFLEE--IA
         AT1G63180_UGE3  (130)  VFS--SSATVYGQ----PEIVPCVEDFELQA--MNPYGRTKLFLEE--IA
         AT1G64440_UGE4  (125)  VFS--SSATVYGW----PKEVPCTEESPLSG--MSPYGRTKLFIED--IC
         AT4G10960_UGE5  (126)  VFS--SSATVYGS----PKEVPCTEEFPISA--LNPYGRTKLFIEE--IC
         AT4G23920_UGE2  (125)  VFS--SSATVYGW----PKEVPCTEESPISA--TNPYGRTKLFIEE--IC
     Pt_lcl_scaff_5422.2  (119)  VFS--SSATVYGD----PASLP-IREDFPLS-ATNPYGRSKLWIEE-MLA
     Pt_lcl_scaff_I.2996  (192)  IYS--STCATYGE----PEKMPITEDTPQVP--INPYGKAKKMAED-IIL
      Pt_lcl_scaff_I.773  (125)  IFSRLLCIGVFIF----SYCLWLAEGGSMYR--RVPFVCCKPIWKNQEIC
   Pt_lcl_scaff_III.1133  (125)  VFS--SSATVYGC----PKEVPCTEEFPLSA--ASPYGRTKLFIEG--IC
    Pt_lcl_scaff_III.961  (127)  VFS--SSATVYGQ----PEKIPCVEDFNLMA--MNPYGRTKKLLEI--FK
     Pt_lcl_scaff_VI.203  (180)  IYS--STCATYGE----PIKMPIREETPQLP--INPYGKAKKMAED-III
    Pt_lcl_scaff_XI.1329  (192)  IYS--STCATYGE----PEKMPITEVTPQVP--INPYGKAKKMAED-IIL
    Pt_lcl_scaff_XVI.140  (154)  IYS--STCATYGE----PVKMPITEQTPQLP--INPYGKAKKMAED-III
             Ot08g01550  (128)  IFS--SSATVYGE----PASVPCTEDFPTAA--LNPYGRTKLFIEH--IL
        SC_GAL10_P04397  (131)  VFS--SSATVYGDATRFPNMIPIPEECPLGP--TNPYGHTKYAIEN-ILN
                Ec_GALE  (120)  IFS--SSATVYGE----PKIIP-VNESCAIGGTTNPYGTSKLFVEN-FLR
                OS_UGE2  (129)  VFS--SSATVYGW----PKEVPCTEESPLCA--MNPYGRTKLVIED--MC
              Consensus  (251)  VFS  SSATVYG     P  VPCTEESPL A  INPYGRTKLMIED  I

                                301                                               350
           Os04g0618200  (212)  DFSKR--SEMAVMILR-------------YFNVIGSDPGGRLGEAPRPEL
           Os05g0595100  (169)  RDLHASDPNWKIILLRY-------------FNPVGAHPSGYIGEDPCG--
           Os07g0139400  (235)  DFSKSKKADMAVMILR-------------YFNVIGSDPEGRLGEAPKPEL
           Os08g0374800  (174)  RDIYRTDPEWKIILLRY-------------FNPVGAHPSGYLGEDPCG--
           Os09g0323000  (180)  RDIYHSDSEWSIILLRY-------------FNPVGAHPSGYLGEDPCG--
           Os09g0504000  (291)  TYN----------------------------HIYGLSITGLRFFTVYGPW
           Os09g0526700  (189)  RQVQAADPEMRVILLRY-------------FNPIGAHRSGDIGEDPRG--
         AT1G12780_UGE1  (170)  RDIQKAEPEWRIILLRY-------------FNPVGAHESGSIGEDPKG--
         AT1G63180_UGE3  (170)  RDIHAAEPEWKIILLRY-------------FNPVGAHESGRIGEDPKG--
         AT1G64440_UGE4  (165)  RDVQRGDPEWRIIMLRY-------------FNPVGAHPSGRIGEDPCG--
         AT4G10960_UGE5  (166)  RDVYGSDPEWKIILLRY-------------FNPVGAHPSGDIGEDPRG--
         AT4G23920_UGE2  (165)  RDVHRSDSEWKIILLRY-------------FNPVGAHPSGYIGEDPLG--
     Pt_lcl_scaff_5422.2  (160)  DLDRAEAGQWSLARLRY-------------FNPVGAHESGLIGEDPRD--
     Pt_lcl_scaff_I.2996  (233)  DFSKN--SDMAIMILR-------------YFNVIGSDPDGRLGEAPRPEL
      Pt_lcl_scaff_I.773  (169)  RDIYSSDSEWKIILLRY-------------FNPVGAHPSGYIGEDPRG--
   Pt_lcl_scaff_III.1133  (165)  CDIHRSDSEWKIILLRY-------------FNPVGAHPSGHIGEDPLG--
    Pt_lcl_scaff_III.961  (167)  ----RQNQNGVSFYLGT-------------STPWELMRVVNLVKIPRV--
     Pt_lcl_scaff_VI.203  (221)  DFSNT--TDMAVMILSIQHSDQNNAYVYRYFNVIGSDPEGRLGEAPRPEL
    Pt_lcl_scaff_XI.1329  (233)  DFSKN--SDMAIMILR-------------YFNVIGSDPDGRLGEAPRPEL
    Pt_lcl_scaff_XVI.140  (195)  DFSKT--TDMAVMIL-----------R--YFNVIGSDPEGRLGEAPQPEL
             Ot08g01550  (168)  SDLYVSDKEWKVALLRY-------------FNPVGAHESGTLGEDPKG--
        SC_GAL10_P04397  (176)  DLYNSDKKSWKFAILRY-------------FNPIGAHPSGLIGEDPLG--
                Ec_GALE  (162)  DYSKASP-NFKTIVLRY-------------FNPIGAHSSGKIGEDPNG--
                OS_UGE2  (169)  RDLHASDPNWKIILLRY-------------FNPVGAHPSGYIGEDPCG--
              Consensus  (301)   DI  SD EWKIILLRY             FNPVGAHPSG IGEDPRG
```

## Figure 20 (continued)

```
                                   351                                               400
       Os04g0618200     (247)  REHGRISGACFDAALGIIPGLKVRG----TDYPTADGTCIRDYIDVTDLV
       Os05g0595100     (204)  -IPNNLMPFVQQVAVGRRPALTVYG----TDYNTKDGTGVRDYIHVVDLA
       Os07g0139400     (272)  REHGRISGACFDAALGIIPGLKVKG----TDYETPDGTCVRDYIDVTDLV
       Os08g0374800     (209)  -IPNNLMPYVQQVAVGRRPALTILG----NDYATRDGTGVRDYIHVVDLA
       Os09g0323000     (215)  -IPNNLMPFVQQVAVGRRPSLTIFG----NDYATKDGTGVRDYIHVVDLA
       Os09g0504000     (313)  GRPDMAYFFFAKSIVSGEP-ITLFR------AADGAD-ARRDFTYIDDVV
       Os09g0526700     (224)  -IPNNLLPYIQQVAVGRRPELNVYG----VDYPTRDGTAIRDYIHVVDLA
      AT1G12780_UGE1    (205)  -IPNNLMPYIQQVAVGRLPELNVYG----HDYPTEDGSAVRDYIHVMDLA
      AT1G63180_UGE3    (205)  -IPNNLMPYIQQVAVGRLPELNVFG----HDYPTMDGSAVRDYIHVMDLA
      AT1G64440_UGE4    (200)  -TPNNLMPYVQQVVVGRLPNLKIYG----TDYTTKDGTGVRDYIHVVDLA
      AT4G10960_UGE5    (201)  -IPNNLMPFVQQVAVGRRPHLTVFG----NDYNTKDGTGVRDYIHVIDLA
      AT4G23920_UGE2    (200)  -VPNNLMPYVQQVAVGRRPHLTVFG----TDYKTKDGTGVRDYIHVMDLA
  Pt_lcl_scaff_5422.2   (195)  -IPNNLMPYVSQVAIGQRQQLSVYG----DDYATPDGTGVRDYIHVTDLA
  Pt_lcl_scaff_I.2996   (268)  REHGRISGACFDAARGIVAGLKVKG----TDYKTHDGTCIRDYIDVTDLV
   Pt_lcl_scaff_I.773   (204)  -IPNNLMPYVQQVAVGRRPHLTVFG----TDYPTKDGTGVRDYIHVVDLA
Pt_lcl_scaff_III.1133   (200)  -IPNNLMPYVQQVAVGRRPHLTVYG----TDYSTKDGTGVRDYIHVVDLA
 Pt_lcl_scaff_III.961   (198)  -SQTISCPTYNK--------------------------------------
  Pt_lcl_scaff_VI.203   (269)  REHGRISGACFDAARGITPGLKVKG----TDYKTADGTCVRDYIDVTDLV
 Pt_lcl_scaff_XI.1329   (268)  REHGRISGACFDAARGIIAGLKVKG----TDYKTHDGTCIRDYIDVTDLV
 Pt_lcl_scaff_XVI.140   (230)  REHGRISGACFDAARGIIPGLKQRITIQWIDYKTADGTCVRDYIDVTDLV
         Ot08g01550     (203)  -IPNNLMPFVQQVAVGRRPELNVFG----NDYPTKDGTGRRDYIHVVDLA
     SC_GAL10_P04397    (211)  -IPNNLLPYMAQVAVGRREKLYIFG----DDYDSRDGTPIRDYIHVVDLA
            Ec_GALE     (196)  -IPNNLMPFICQVAIGKQKTLKIYG----NDYPTKDGTGIRDYIHVMDLA
            OS_UGE2     (204)  -IPNNLMPFVQQVAVGRRPALTVYG----TDYNTKDGTGVRDYIHVVDLA
          Consensus     (351)   IPNNLMPYVQQVAVGRRP L VFG     DY TKDGTGVRDYIHVVDLA
                                                                 Motif 2

                                           epimerase domain  <--
                               401                                               450
       Os04g0618200     (293)  DAHVKALDKAQPGK-------------VGIYNVGTGHGRSVKEFVEACKS
       Os05g0595100     (249)  DGHIAALRKLYEDSD---------RIGCEVYNLGTGKGTSVLEMVAAFEK
       Os07g0139400     (318)  DAHVKALNKAERGK-------------VGIYNVGTGKGRSVKEFVEACKK
       Os08g0374800     (254)  DGHIAALQKLFESSS----------IGCEAYNLGTGKGTSVLEIVKAFEK
       Os09g0323000     (260)  EGHIAALRKLFESS-----------IGCQAYNLGTGKGTSVLEIVNAFEK
       Os09g0504000     (355)  KGCLGALDTSGKSTGSSKSGKKSGPAPLRVYNLGNTSPVPVTRMVAILEK
       Os09g0526700     (269)  DGHIAALEKLFATP----------DIGCVAYNLGTGCGTTVLEVVKAFEE
      AT1G12780_UGE1    (250)  DGHIAALRKLFADP----------KIGCTAYNLGTGQGTSVLEMVAAFEK
      AT1G63180_UGE3    (250)  DGHVAALNKLFSDS----------KIGCTAYNLGTGQGTSVLEMVSSFEK
      AT1G64440_UGE4    (245)  DGHICALQKLDDTE-----------IGCEVYNLGTGKGTTVLEMVDAFEK
      AT4G10960_UGE5    (246)  DGHIAALRKLEDCK-----------IGCEVYNLGTGNGTSVLEMVDAFEK
      AT4G23920_UGE2    (245)  DGHIAALRKLDDLK-----------ISCEVYNLGTGNGTSVLEMVAAFEK
  Pt_lcl_scaff_5422.2   (240)  RGHLAALRYLREQQ------------GLLTVNLGTGRPVSVLEMVKAFER
  Pt_lcl_scaff_I.2996   (314)  DAHVKALEKAMPGK-------------VGIYNVGTGMGRSVNEFVHACKK
   Pt_lcl_scaff_I.773   (249)  DGHIAALRKLSEAN-----------IGCEVYNLGTGKGTSVLEMVAAFEK
Pt_lcl_scaff_III.1133   (245)  DGHIAALRKLSDAN-----------IGCEVYNLGTGKGTSVLEMVAAFEK
 Pt_lcl_scaff_III.961   (209)  --------------------------------------------------
  Pt_lcl_scaff_VI.203   (315)  DAHVKALAHAKPRK-------------VGIYNVGTGKGRSVKEFVDACKK
 Pt_lcl_scaff_XI.1329   (314)  DAHVKALEKAMPGK-------------VGIYNVGTGKGRSVKEFVKACKK
 Pt_lcl_scaff_XVI.140   (280)  DAHVKALAHAKPRK-------------VGIYNVGTGKGRSVKEFVEACKK
         Ot08g01550     (248)  DGHVAAVKKLTSDP----------DAGLLTVNLGTGKSTSVLELVAAFEK
     SC_GAL10_P04397    (256)  KGHIAALQYLEAYNEN--------EGLCREWNLGSGKGSTVFEVYHAFCK
            Ec_GALE     (241)  EGHVAALNNINQGA------------NYRVYNLGTGIGYSVLELLEAFQK
            OS_UGE2     (249)  DGHIAALRKLYEDSD---------RIGCEVYNLGTGKGTSVLEMVAAFEK
          Consensus     (401)  DGHIAAL KL               IGC VYNLGTGKGTSVLEMV AFEK
```

**Figure 20 (continued)**

```
                              451                                              500
         Os04g0618200   (330) ATGAS-------IKVSFLTRRPGDYAEVYSDPSKIHDELNWTARYIDLRE
         Os05g0595100   (290) ASGK-------KIPLVFAGRRPGDAEIVYAQTAKAEKELKWKAKYGVEEM
         Os07g0139400   (355) ATGVD-------IKVDYFPRRPGDYAEVYSDPAKINSELNWTAQHTDLLE
         Os08g0374800   (294) ASGK-------KIPLIIGPRRPGDAEILFSLPAKAEKELNWKAKFGIDEM
         Os09g0323000   (299) VSGK-------KIPLVIGPRRPGDAEILFSSAAKAEREFKWKAKYGIEEM
         Os09g0504000   (405) LLGKKAN------KRIVAMPSNGDVPFTHANVTHAAHDFGYRPTTSLDAG
         Os09g0526700   (309) ASGK-------KIPIKICPRRPGDCTEVYASTDKAKKELGWSARFGIEDM
        AT1G12780_UGE1  (290) ASGK-------KIPIKLCPRRSGDATAVYASTEKAEKELGWKAKYGVDEM
        AT1G63180_UGE3  (290) ASGK-------KIPIKLCPRRAGDATAVYASTQKAEKELGWKAKYGVDEM
        AT1G64440_UGE4  (284) ASGM-------KIPLVKVGRRPGDAETVYASTEKAERELNWKANFGIEEM
        AT4G10960_UGE5  (285) ASGK-------KIPLVIAGRRPGDAEVVYASTERAESELNWKAKYGIEEM
        AT4G23920_UGE2  (284) ASGK-------KIPLVMAGRRPGDAEVVYASTEKAERELNWKAKNGIEEM
   Pt_lcl_scaff_5422.2  (278) ASGR-------PVPYQIVARRPGDVAQCWADPAEAERLLGWKAMLDLDRI
   Pt_lcl_scaff_I.2996  (351) ATGVD-------IKVDYLPRRPGDYAEVFSDPSKINRELNWTAQYTDLQK
    Pt_lcl_scaff_I.773  (288) ASGKSPIFVCQKIPLVMADRRPGDAETVYAATEKAERELSWK--------
 Pt_lcl_scaff_III.1133  (284) ASRK-------KIPLVMAARRPGDAEIVYAATEKAERELNWKAKYGIDEM
  Pt_lcl_scaff_III.961  (209) --------------------------------------------------
   Pt_lcl_scaff_VI.203  (352) ATGVD-------IKVEYLDRRPGDYAEVFSDPSKIKQELSWTAQYTDLQK
  Pt_lcl_scaff_XI.1329  (351) ATGVD-------IKVDYLPRRPGDYAEVFSDPSKIYRELNWTAQYTDLQK
  Pt_lcl_scaff_XVI.140  (317) ATGVD-------IKVEYLNRRPGDYAEVFSDPSKIKQELNWKAQYTDLKK
           Ot08g01550   (288) ASGK-------KIPCKIVDRRAGDAAEVYGATDKAFKVLGWRALRTIEDC
      SC_GAL10_P04397   (298) ASGID-------LPYKVTGRRAGDVLNLTAKPDRAKRELKWQTELQVEDS
              Ec_GALE   (279) VTTR-------RVPYVFTKRRSGDIAECWSDPTKAYEELGWKARRGLEDM
              OS_UGE2   (290) ASGK-------KIPLVFAGRRPGDAEIVYAQTAKAEKELKWKAKYGVEEM
            Consensus   (451) ASGK       KIPL I  RRPGDA  VYA   KAEREL WKAKYGIEEM
```

```
                              501                                              550
         Os04g0618200   (373) SLSTAWK---------WQKAHPNGYGSA----------------------
         Os05g0595100   (333) CRDLWN----------WASKNPYGYGSPDSSN------------------
         Os07g0139400   (398) SLRVAWT---------WQKKHRSGYGPPQAMVL-----------------
         Os08g0374800   (337) CRDQWN----------WASKNPYGYGSLDSTKQNGHHSYGSIGSPKQNGH
         Os09g0323000   (342) CRDQWN----------WASKNPFGYASPDSTKQNGHSH------------
         Os09g0504000   (449) LRHFVDWFADYYKLKLDVPKIAAKVAGAGKPSSSSASKKKKKAAAMSASS
         Os09g0526700   (352) CRDQWN----------WAKKNPYGYSANAEQN------------------
        AT1G12780_UGE1  (333) CRDQWK----------WANNNPWGYQNKL---------------------
        AT1G63180_UGE3  (333) CRDQWN----------WANKNPWGFQKKP---------------------
        AT1G64440_UGE4  (327) CRDQWN----------WASNNPFGYGSSPNST------------------
        AT4G10960_UGE5  (328) CRDLWN----------WASNNPYGYDSSSEDNSH----------------
        AT4G23920_UGE2  (327) CRDLWN----------WASNNPYGYNSSSNGSSS----------------
   Pt_lcl_scaff_5422.2  (321) ACGRRGRGIFSPLFHALAKSLPMLAKRIIPCLDVTGGRVVKGVNFVELRD
   Pt_lcl_scaff_I.2996  (394) SLQVAWR---------WQKSHQNGYGSPLVMAS-----------------
    Pt_lcl_scaff_I.773  (330) --------------------------------------------------
 Pt_lcl_scaff_III.1133  (327) CRDQWN----------WAGKNPYGYGSSDSTN------------------
  Pt_lcl_scaff_III.961  (209) --------------------------------------------------
   Pt_lcl_scaff_VI.203  (395) SLQIAWK---------WQKSHLNGY-------------------------
  Pt_lcl_scaff_XI.1329  (394) SLQTAWR---------WQKSHQNGYGSPLVMAS-----------------
  Pt_lcl_scaff_XVI.140  (360) SLQIAWK---------WQKSHLNGYSHS----------------------
           Ot08g01550   (331) CIDQWK----------WASSNPYGYAGKPDDA------------------
      SC_GAL10_P04397   (341) CKDLWK----------WTTENPFGYQLRGVEARFSAEDMRYDARFVTIGA
              Ec_GALE   (322) IRDAWN----------WQQKNPNGFKKV----------------------
              OS_UGE2   (333) CRDLWN----------WASKNPYGYGSPDSSN------------------
            Consensus   (501) CRD W           WA  NPYGYGS
```

Figure 20 (continued)

```
                                  551                                              600
            Os04g0618200   (392)  --------------------------------------------------
            Os05g0595100   (355)  --------------------------------------------------
            Os07g0139400   (422)  --------------------------------------------------
            Os08g0374800   (377)  CTNGFSESTRHNGHNGYGLVDSAKHNGNGHFH------------------
            Os09g0323000   (370)  --------------------------------------------------
            Os09g0504000   (499)  --------------------------------------------------
            Os09g0526700   (374)  --------------------------------------------------
           AT1G12780_UGE1  (352)  --------------------------------------------------
           AT1G63180_UGE3  (352)  --------------------------------------------------
           AT1G64440_UGE4  (349)  --------------------------------------------------
           AT4G10960_UGE5  (352)  --------------------------------------------------
           AT4G23920_UGE2  (351)  --------------------------------------------------
       Pt_lcl_scaff_5422.2 (371)  AGDPVEIAARYNAQGADELTFLDITATSDGRDLILPIIESVASQVFIPLT
       Pt_lcl_scaff_I.2996 (418)  --------------------------------------------------
        Pt_lcl_scaff_I.773 (330)  --------------------------------------------------
     Pt_lcl_scaff_III.1133 (349)  --------------------------------------------------
      Pt_lcl_scaff_III.961 (209)  --------------------------------------------------
       Pt_lcl_scaff_VI.203 (411)  --------------------------------------------------
      Pt_lcl_scaff_XI.1329 (418)  --------------------------------------------------
      Pt_lcl_scaff_XVI.140 (379)  --------------------------------------------------
             Ot08g01550    (353)  --------------------------------------------------
         SC_GAL10_P04397   (381)  GTRFQATFANLGASIVDLKVNGQSVVLGYENEEGYLNPDSAYIGATIGRY
                 Ec_GALE   (340)  --------------------------------------------------
                 OS_UGE2   (355)  --------------------------------------------------
               Consensus   (551)

                                  601                                              650
            Os04g0618200   (392)  --------------------------------------------------
            Os05g0595100   (355)  --------------------------------------------------
            Os07g0139400   (422)  --------------------------------------------------
            Os08g0374800   (409)  --------------------------------------------------
            Os09g0323000   (370)  --------------------------------------------------
            Os09g0504000   (499)  --------------------------------------------------
            Os09g0526700   (374)  --------------------------------------------------
           AT1G12780_UGE1  (352)  --------------------------------------------------
           AT1G63180_UGE3  (352)  --------------------------------------------------
           AT1G64440_UGE4  (349)  --------------------------------------------------
           AT4G10960_UGE5  (352)  --------------------------------------------------
           AT4G23920_UGE2  (351)  --------------------------------------------------
       Pt_lcl_scaff_5422.2 (421)  VGGGVRTVEDVRRLLNAGADKTSFNSAAIANPDVINAASDKYGAQCIVVA
       Pt_lcl_scaff_I.2996 (418)  --------------------------------------------------
        Pt_lcl_scaff_I.773 (330)  --------------------------------------------------
     Pt_lcl_scaff_III.1133 (349)  --------------------------------------------------
      Pt_lcl_scaff_III.961 (209)  --------------------------------------------------
       Pt_lcl_scaff_VI.203 (411)  --------------------------------------------------
      Pt_lcl_scaff_XI.1329 (418)  --------------------------------------------------
      Pt_lcl_scaff_XVI.140 (379)  --------------------------------------------------
             Ot08g01550    (353)  --------------------------------------------------
         SC_GAL10_P04397   (431)  ANRISKGKFSLCNKDYQLTVNNGVNANHSSIGSFHRKRFLGPIIQNPSKD
                 Ec_GALE   (340)  --------------------------------------------------
                 OS_UGE2   (355)  --------------------------------------------------
               Consensus   (601)
```

**Figure 20 (continued)**

| | | 651 ... 700 |
|---|---|---|
| Os04g0618200 | (392) | -------------------------------------------------- |
| Os05g0595100 | (355) | -------------------------------------------------- |
| Os07g0139400 | (422) | -------------------------------------------------- |
| Os08g0374800 | (409) | -------------------------------------------------- |
| Os09g0323000 | (370) | -------------------------------------------------- |
| Os09g0504000 | (499) | -------------------------------------------------- |
| Os09g0526700 | (374) | -------------------------------------------------- |
| AT1G12780_UGE1 | (352) | -------------------------------------------------- |
| AT1G63180_UGE3 | (352) | -------------------------------------------------- |
| AT1G64440_UGE4 | (349) | -------------------------------------------------- |
| AT4G10960_UGE5 | (352) | -------------------------------------------------- |
| AT4G23920_UGE2 | (351) | -------------------------------------------------- |
| Pt_lcl_scaff_5422.2 | (471) | IDAKRRTAEDEQRIGADGRAAGPGWDVYSHGGRKNTGLDAVHSRAPSATP |
| Pt_lcl_scaff_I.2996 | (418) | -------------------------------------------------- |
| Pt_lcl_scaff_I.773 | (330) | -------------------------------------------------- |
| Pt_lcl_scaff_III.1133 | (349) | -------------------------------------------------- |
| Pt_lcl_scaff_III.961 | (209) | -------------------------------------------------- |
| Pt_lcl_scaff_VI.203 | (411) | -------------------------------------------------- |
| Pt_lcl_scaff_XI.1329 | (418) | -------------------------------------------------- |
| Pt_lcl_scaff_XVI.140 | (379) | -------------------------------------------------- |
| Ot08g01550 | (353) | -------------------------------------------------- |
| SC_GAL10_P04397 | (481) | VFTAEYMLIDNEKDTEFPGDLLVTIQYTVNVAQKSLEMVYKGKLTAGEAT |
| Ec_GALE | (340) | -------------------------------------------------- |
| OS_UGE2 | (355) | -------------------------------------------------- |
| Consensus | (651) | |

| | | 701 ... 750 |
|---|---|---|
| Os04g0618200 | (392) | -------------------------------------------------- |
| Os05g0595100 | (355) | -------------------------------------------------- |
| Os07g0139400 | (422) | -------------------------------------------------- |
| Os08g0374800 | (409) | -------------------------------------------------- |
| Os09g0323000 | (370) | -------------------------------------------------- |
| Os09g0504000 | (499) | -------------------------------------------------- |
| Os09g0526700 | (374) | -------------------------------------------------- |
| AT1G12780_UGE1 | (352) | -------------------------------------------------- |
| AT1G63180_UGE3 | (352) | -------------------------------------------------- |
| AT1G64440_UGE4 | (349) | -------------------------------------------------- |
| AT4G10960_UGE5 | (352) | -------------------------------------------------- |
| AT4G23920_UGE2 | (351) | -------------------------------------------------- |
| Pt_lcl_scaff_5422.2 | (521) | CPCP---------------------------------------------- |
| Pt_lcl_scaff_I.2996 | (418) | -------------------------------------------------- |
| Pt_lcl_scaff_I.773 | (330) | -------------------------------------------------- |
| Pt_lcl_scaff_III.1133 | (349) | -------------------------------------------------- |
| Pt_lcl_scaff_III.961 | (209) | -------------------------------------------------- |
| Pt_lcl_scaff_VI.203 | (411) | -------------------------------------------------- |
| Pt_lcl_scaff_XI.1329 | (418) | -------------------------------------------------- |
| Pt_lcl_scaff_XVI.140 | (379) | -------------------------------------------------- |
| Ot08g01550 | (353) | -------------------------------------------------- |
| SC_GAL10_P04397 | (531) | PINLTNHSYFNLNKPYGDTIEGTEIMVRSKKSVDVDKNMIPTGNIVDREI |
| Ec_GALE | (340) | -------------------------------------------------- |
| OS_UGE2 | (355) | -------------------------------------------------- |
| Consensus | (701) | |

**Figure 20 (continued)**

```
                                   751                                              800
           Os04g0618200   (392)   --------------------------------------------------
           Os05g0595100   (355)   --------------------------------------------------
           Os07g0139400   (422)   --------------------------------------------------
           Os08g0374800   (409)   --------------------------------------------------
           Os09g0323000   (370)   --------------------------------------------------
           Os09g0504000   (499)   --------------------------------------------------
           Os09g0526700   (374)   --------------------------------------------------
         AT1G12780_UGE1   (352)   --------------------------------------------------
         AT1G63180_UGE3   (352)   --------------------------------------------------
         AT1G64440_UGE4   (349)   --------------------------------------------------
         AT4G10960_UGE5   (352)   --------------------------------------------------
         AT4G23920_UGE2   (351)   --------------------------------------------------
    Pt_lcl_scaff_5422.2   (525)   --------------------------------------------------
    Pt_lcl_scaff_I.2996   (418)   --------------------------------------------------
     Pt_lcl_scaff_I.773   (330)   --------------------------------------------------
  Pt_lcl_scaff_III.1133   (349)   --------------------------------------------------
   Pt_lcl_scaff_III.961   (209)   --------------------------------------------------
    Pt_lcl_scaff_VI.203   (411)   --------------------------------------------------
   Pt_lcl_scaff_XI.1329   (418)   --------------------------------------------------
   Pt_lcl_scaff_XVI.140   (379)   --------------------------------------------------
            Ot08g01550   (353)   --------------------------------------------------
      SC_GAL10_P04397   (581)   ATFNSTKPTVLGPKNPQFDCCFVVDENAKPSQINTLNNELTLIVKAFHPD
               Ec_GALE   (340)   --------------------------------------------------
               OS_UGE2   (355)   --------------------------------------------------
             Consensus   (751)

                                   801                                              850
           Os04g0618200   (392)   --------------------------------------------------
           Os05g0595100   (355)   --------------------------------------------------
           Os07g0139400   (422)   --------------------------------------------------
           Os08g0374800   (409)   --------------------------------------------------
           Os09g0323000   (370)   --------------------------------------------------
           Os09g0504000   (499)   --------------------------------------------------
           Os09g0526700   (374)   --------------------------------------------------
         AT1G12780_UGE1   (352)   --------------------------------------------------
         AT1G63180_UGE3   (352)   --------------------------------------------------
         AT1G64440_UGE4   (349)   --------------------------------------------------
         AT4G10960_UGE5   (352)   --------------------------------------------------
         AT4G23920_UGE2   (351)   --------------------------------------------------
    Pt_lcl_scaff_5422.2   (525)   --------------------------------------------------
    Pt_lcl_scaff_I.2996   (418)   --------------------------------------------------
     Pt_lcl_scaff_I.773   (330)   --------------------------------------------------
  Pt_lcl_scaff_III.1133   (349)   --------------------------------------------------
   Pt_lcl_scaff_III.961   (209)   --------------------------------------------------
    Pt_lcl_scaff_VI.203   (411)   --------------------------------------------------
   Pt_lcl_scaff_XI.1329   (418)   --------------------------------------------------
   Pt_lcl_scaff_XVI.140   (379)   --------------------------------------------------
            Ot08g01550   (353)   --------------------------------------------------
      SC_GAL10_P04397   (631)   SNITLEVLSTEPTYQFYTGDFLSAGYEARQGFAIEPGRYIDAINQENWKD
               Ec_GALE   (340)   --------------------------------------------------
               OS_UGE2   (355)   --------------------------------------------------
             Consensus   (801)
```

**Figure 20 (continued)**

| | | 851 869 |
|---|---|---|
| Os04g0618200 | (392) | ------------------- |
| Os05g0595100 | (355) | ------------------- |
| Os07g0139400 | (422) | ------------------- |
| Os08g0374800 | (409) | ------------------- |
| Os09g0323000 | (370) | ------------------- |
| Os09g0504000 | (499) | ------------------- |
| Os09g0526700 | (374) | ------------------- |
| AT1G12780_UGE1 | (352) | ------------------- |
| AT1G63180_UGE3 | (352) | ------------------- |
| AT1G64440_UGE4 | (349) | ------------------- |
| AT4G10960_UGE5 | (352) | ------------------- |
| AT4G23920_UGE2 | (351) | ------------------- |
| Pt_lcl_scaff_5422.2 | (525) | ------------------- |
| Pt_lcl_scaff_I.2996 | (418) | ------------------- |
| Pt_lcl_scaff_I.773 | (330) | ------------------- |
| Pt_lcl_scaff_III.1133 | (349) | ------------------- |
| Pt_lcl_scaff_III.961 | (209) | ------------------- |
| Pt_lcl_scaff_VI.203 | (411) | ------------------- |
| Pt_lcl_scaff_XI.1329 | (418) | ------------------- |
| Pt_lcl_scaff_XVI.140 | (379) | ------------------- |
| Ot08g01550 | (353) | ------------------- |
| SC_GAL10_P04397 | (681) | CVTLKNGETYGSKIVYRFS |
| Ec_GALE | (340) | ------------------- |
| OS_UGE2 | (355) | ------------------- |
| Consensus | (851) | |

**Figure 20 (continued)**

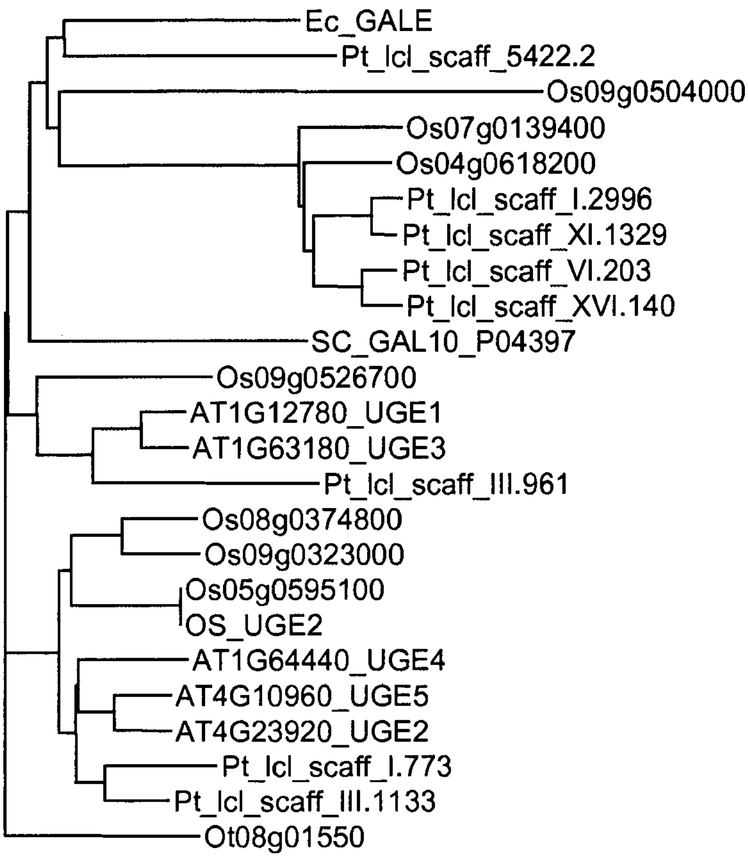
Ec_GALE
Pt_lcl_scaff_5422.2
Os09g0504000
Os07g0139400
Os04g0618200
Pt_lcl_scaff_I.2996
Pt_lcl_scaff_XI.1329
Pt_lcl_scaff_VI.203
Pt_lcl_scaff_XVI.140
SC_GAL10_P04397
Os09g0526700
AT1G12780_UGE1
AT1G63180_UGE3
Pt_lcl_scaff_III.961
Os08g0374800
Os09g0323000
Os05g0595100
OS_UGE2
AT1G64440_UGE4
AT4G10960_UGE5
AT4G23920_UGE2
Pt_lcl_scaff_I.773
Pt_lcl_scaff_III.1133
Ot08g01550

**Figure 21**

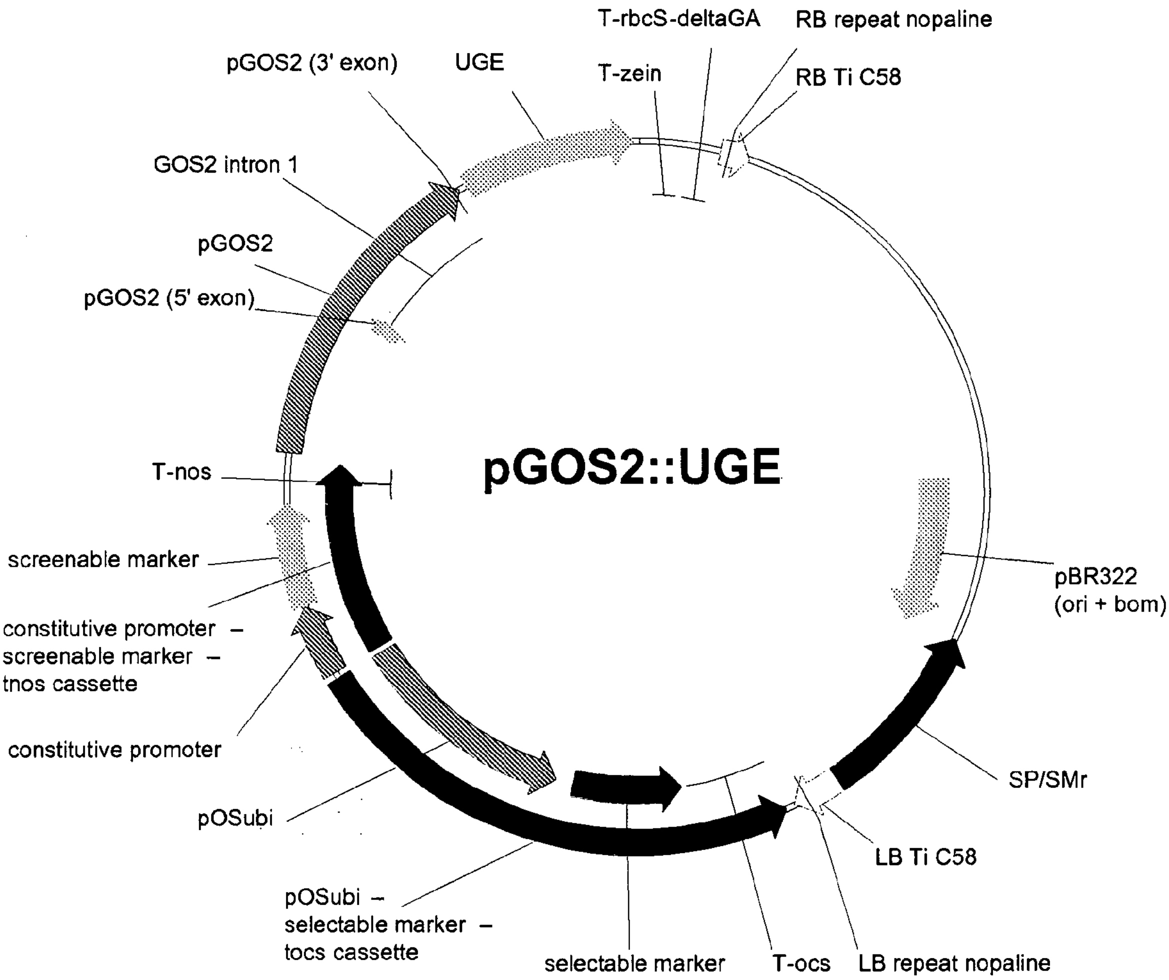
pGOS2::UGE
T-rbcS-deltaGA
RB repeat nopaline
pGOS2 (3' exon)
UGE
T-zein
RB Ti C58
GOS2 intron 1
pGOS2
pGOS2 (5' exon)
T-nos
screenable marker
constitutive promoter – screenable marker – tnos cassette
constitutive promoter
pOSubi
pOSubi – selectable marker – tocs cassette
selectable marker
T-ocs
LB repeat nopaline
LB Ti C58
SP/SMr
pBR322 (ori + bom)

**Figure 22**

**SEQ ID NO: 224, DNA - *Oryza sativa***
ATGGTTTCGGCCTTGTTTCGGACGATCCTGGTGACGGGCGGCGCCGGCTACATCGGCAGCCACACCGTCCTC
CAGCTTCTCCAACTCGGCTTCCGCGTTGTCGTCCTCGACAACCTCGACAACGCCTCCGAGCTCGCCATCCTC
CGCGTCAGGGAACTCGCCGGACACAACGCCAACAACCTCGACTTCCGCAAGGTTGACCTCCGCGACAAGCAA
GCGTTGGACCAAATCTTCTCCTCTCAAAGGTTTGAGGCTGTCATCCATTTTGCCGGGCTGAAAGCTGTTGGC
GAGAGCGTGCAGAAGCCCCTGCTTTACTACGACAACAACCTCATCGGCACCATCACTCTCCTGCAGGTCATG
GCCGCACATGGCTGCACCAAGCTGGTGTTCTCATCATCCGCAACTGTCTACGGGTGGCCCAAGGAGGTGCCC
TGCACTGAAGAATCCCCACTTTGTGCAATGAACCCCTACGGCAGAACAAAGCTGGTAATCGAAGACATGTGC
CGGGATCTGCATGCCTCAGACCCAAACTGGAAGATCATACTGCTCCGATACTTCAACCCTGTTGGAGCTCAC
CCAAGCGGGTACATTGGTGAGGACCCCTGCGGCATCCCAAACAACCTCATGCCCTTCGTCCAGCAGGTCGCT
GTTGGCAGGAGGCCGGCCCTTACCGTCTATGGAACCGACTACAACACCAAGGATGGAACTGGGGTTCGTGAC
TATATCCATGTTGTTGATCTAGCGGATGGTCATATCGCCGCGTTAAGGAAGCTCTATGAAGATTCTGATAGA
ATAGGATGTGAGGTGTACAATCTGGGCACTGGAAAGGGGACATCTGTGCTGGAAATGGTTGCAGCATTCGAG
AAAGCTTCTGGAAAGAAAATCCCGCTTGTATTTGCTGGACGAAGGCCTGGAGATGCCGAGATCGTTTACGCT
CAAACTGCCAAAGCTGAGAAGGAACTGAAATGGAAGGCAAAATACGGGGTAGAGGAGATGTGCAGGGACCTG
TGGAATTGGGCGAGCAAGAACCCCTACGGGTATGGATCGCCGGACAGTAGCAACTGA

**SEQ ID NO: 225, protein - *Oryza sativa***
MVSALFRTILVTGGAGYIGSHTVLQLLQLGFRVVVLDNLDNASELAILRVRELAGHNANNLDFRKVDLRDKQ
ALDQIFSSQRFEAVIHFAGLKAVGESVQKPLLYYDNNLIGTITLLQVMAAHGCTKLVFSSSATVYGWPKEVP
CTEESPLCAMNPYGRTKLVIEDMCRDLHASDPNWKIILLRYFNPVGAHPSGYIGEDPCGIPNNLMPFVQQVA
VGRRPALTVYGTDYNTKDGTGVRDYIHVVDLADGHIAALRKLYEDSDRIGCEVYNLGTGKGTSVLEMVAAFE
KASGKKIPLVFAGRRPGDAEIVYAQTAKAEKELKWKAKYGVEEMCRDLWNWASKNPYGYGSPDSSN

**SEQ ID NO: 226, DNA - *Oryza sativa***
ATGGACTTCGGTGATTCTAGACGCAAGCCTAATGTCGTCGGGAAGTTCACCGTAGCTGTTGCACTCACGGTC
ATGTGCATCATTGTGTTGAAACAGTCGCCTGGTTTTACCAGTACAAGTGTGTTCTCTCGCCATGAAATTGGT
GTGACTCATGTGTTGGTGACTGGAGGAGCTGGATACATTGGGTCACATGCTACTCTTCGTCTACTTAGGGAC
AACTACCGAGTTACCATTGTGGATAACCTTTCTAGAGGGAACATGGGAGCTGTCAGAGTCCTTCAACGTTTG
TTTCCAGAGCCTGGGAGGCTTCAGTTTATTTATGCTGATTTAGGCGATGCGAAAGCTGTTAACAAAATATTT
TCAGAAAATGCATTCGATGCTGTTATGCACTTTGCTGCCGTTGCTTACGTTGGTGAGAGCACGTTGGAGCCA
CTGAGGTACTACCACAACATAACATCAAATACATTGACAGTGCTTGAGGCAATGGCAGCATATAATGTAAAA
ACTCTGATATACTCAAGTACTTGTGCAACATATGGTGAACCTGACACAATGCCTATCACAGAAGCAACCCCT
CAGAATCCTATCAATCCATATGGGAAGGCAAAAAAGATGGCAGAGGACATCATTTTAGATTTCTCTAAGAGA
TCAGAAATGGCAGTCATGATCTTAAGATACTTCAACGTCATTGGATCAGACCCAGGGGGGCGGTTAGGGGAA
GCTCCACGACCAGAGCTGCGTGAGCATGGACGGATTTCTGGTGCTTGCTTTGATGCAGCACTGGGAATCATT
CCAGGTTTGAAGGTTCGGGGAACTGATTACCCTACAGCTGATGGAACTTGCATAAGGGACTATATTGATGTC
ACGGATCTCGTTGATGCTCATGTGAAAGCTCTTGATAAAGCTCAGCCTGGCAAAGTTGGAATCTACAATGTT
GGCACAGGACATGGTAGATCAGTGAAGGAGTTTGTAGAAGCATGTAAGAGCGCAACAGGAGCTAGCATCAAG
GTTTCATTCCTTACCCGGAGACCAGGAGACTATGCTGAAGTTTACAGTGACCCATCCAAAATCCATGACGAG
CTGAACTGGACAGCCCGTTACATTGATCTTCGCGAAAGCCTTTCAACTGCATGGAAATGGCAGAAAGCACAC
CCGAATGGGTATGGATCGGCCTGA

**Figure 23**

**SEQ ID NO: 227, protein - *Oryza sativa***
MDFGDSRRKPNVVGKFTVAVALTVMCIIVLKQSPGFTSTSVFSRHEIGVTHVLVTGGAGYIGSHATLRLLRD
NYRVTIVDNLSRGNMGAVRVLQRLFPEPGRLQFIYADLGDAKAVNKIFSENAFDAVMHFAAVAYVGESTLEP
LRYYHNITSNTLTVLEAMAAYNVKTLIYSSTCATYGEPDTMPITEATPQNPINPYGKAKKMAEDIILDFSKR
SEMAVMILRYFNVIGSDPGGRLGEAPRPELREHGRISGACFDAALGIIPGLKVRGTDYPTADGTCIRDYIDV
TDLVDAHVKALDKAQPGKVGIYNVGTGHGRSVKEFVEACKSATGASIKVSFLTRRPGDYAEVYSDPSKIHDE
LNWTARYIDLRESLSTAWKWQKAHPNGYGSA

**SEQ ID NO: 228, DNA - *Oryza sativa***
ATGGTTTCGGCCTTGTTGCGGACGATCCTGGTGACGGGCGGCGCCGGCTACATCGGCAGCCACACCGTCCTC
CAGCTTCTCCAACTCGGCTTCCGCGTTGTCGTCCTCGACAACCTCGACAACGCCTCCGAGCTCGCCATCCTC
CGCGTCAGGGAACTCGCCGGACACAACGCCAACAACCTCGACTTCCGCAAGGTTGACCTCCGCGACAAGCAA
GCGTTGGACCAAATCTTCTCCTCTCAAAGGTTTGAGGCTGTCATCCATTTTGCCGGGCTGAAAGCTGTTGGC
GAGAGCGTGCAGAAGCCCCTGCTTTACTACGACAACAACCTCATCGGCACCATCACTCTCCTGCAGGTCATG
GCCGCACATGGCTGCACCAAGCTGGTGTTCTCATCATCCGCAACTGTCTACGGGTGGCCCAAGGAGGTGCCC
TGCACTGAAGAATCCCCACTTTGTGCAATGAACCCCTACGGCAGAACAAAGCTGGTAATCGAAGACATGTGC
CGGGATCTGCATGCCTCAGACCCAAACTGGAAGATCATACTGCTCCGATACTTCAACCCTGTTGGAGCTCAC
CCAAGCGGGTACATTGGTGAGGACCCCTGCGGCATCCCAAACAACCTCATGCCCTTCGTCCAGCAGGTCGCT
GTTGGCAGGAGGCCGGCCCTTACCGTCTATGGAACCGACTACAACACCAAGGATGGAACTGGGGTTCGTGAC
TATATCCATGTTGTTGATCTAGCGGATGGTCATATCGCCGCGTTAAGGAAGCTCTATGAAGATTCTGATAGA
ATAGGATGTGAGGTGTACAATCTGGGCACTGGAAAGGGGACATCTGTGCTGGAAATGGTTGCAGCATTCGAG
AAAGCTTCTGGAAAGAAAATCCCGCTTGTATTTGCTGGACGAAGGCCTGGAGATGCCGAGATCGTTTACGCT
CAAACTGCCAAAGCTGAGAAGGAACTGAAATGGAAGGCAAAATACGGGGTAGAGGAGATGTGCAGGGACCTG
TGGAATTGGGCGAGCAAGAACCCCTACGGGTATGGATCGCCGGACAGTAGCAACTGA

**SEQ ID NO: 229, protein - *Oryza sativa***
MVSALLRTILVTGGAGYIGSHTVLQLLQLGFRVVVLDNLDNASELAILRVRELAGHNANNLDFRKVDLRDKQ
ALDQIFSSQRFEAVIHFAGLKAVGESVQKPLLYYDNNLIGTITLLQVMAAHGCTKLVFSSSATVYGWPKEVP
CTEESPLCAMNPYGRTKLVIEDMCRDLHASDPNWKIILLRYFNPVGAHPSGYIGEDPCGIPNNLMPFVQQVA
VGRRPALTVYGTDYNTKDGTGVRDYIHVVDLADGHIAALRKLYEDSDRIGCEVYNLGTGKGTSVLEMVAAFE
KASGKKIPLVFAGRRPGDAEIVYAQTAKAEKELKWKAKYGVEEMCRDLWNWASKNPYGYGSPDSSN

**SEQ ID NO: 230, DNA - *Oryza sativa***
ATGCTTCCCACCAACAGGAACAGGCCCCAGCAGAGGCCTGCCAGATCTTGGTATTTCATCTCAGACATGGAC
TTCTCCGATCCGAAGCGCAAGCCGCGGTACCTCAGTAAGATCCTCATGGTGGCGCTCCTCACCGCCATGTGC
GTCGTCATGCTCACACAGCCGCCCTGCCATAGGAGGACTCCCAGTGTGTTCTCCATCCATGAACCCGGAGTC
ACGCATGTTCTAGTGACGGGCGGCGCTGGATATATCGGTTCGCACGCTGCGCTTCGGCTGCTGAAAGATTCC
TTCAGAGTCACCATAGTGGATAATCTTTCAAGAGGAAATATGGGGGCAATCAAGGTTCTTCAGAACTTGTTT
TCAGAGCCTGGGCGGTTGCAGTTCATCTATGCTGATTTAGGCGATCCAAAAGCTGTTAATAGAATATTTGCA
GAAAATGCTTTTGATGCTGTCATGCACTTTGCTGCTGTCGCTTATGTTGGTGAGAGCACACTTGAGCCTTTG
AGGTACTATCATAACATCACCTCAAACACTTTAGTTGTGCTAGAGGCTATGGCAGCACACAATGTGAGAACC
CTGATCTACTCTAGCACATGTGCCACCTATGGAGAACCCGAGAAGATGCCTATCACCGAAGGAACTCCCCAG
TTTCCGATCAACCCATATGGTAAAGCCAAGAAGATGGCAGAGGACATCATCTTGGATTTTTCCAAATCCAAG
AAGGCAGACATGGCTGTGATGATTCTAAGATACTTCAACGTCATTGGTTCTGACCCTGAAGGAAGGCTTGGC

## Figure 23 (continued)

GAGGCTCCAAAACCTGAATTACGGGAGCATGGCCGTATATCTGGTGCATGCTTTGATGCTGCACTGGGGATA
ATTCCTGGTTTGAAGGTTAAAGGTACTGACTATGAAACACCTGACGGTACTTGTGTAAGAGATTACATCGAT
GTCACTGACCTCGTTGACGCCCATGTGAAGGCGCTCAACAAGGCGGAAAGGGGCAAAGTTGGCATATACAAT
GTTGGCACAGGCAAAGGTAGGTCAGTGAAAGAATTTGTCGAAGCTTGCAAGAAGGCAACAGGAGTTGACATC
AAGGTCGACTACTTCCCTCGTCGACCCGGTGACTATGCCGAAGTGTACAGCGATCCCGCAAAGATCAACAGC
GAGCTGAACTGGACTGCTCAACATACCGATCTCCTGGAGAGCCTCAGGGTTGCCTGGACATGGCAGAAGAAG
CACCGGAGCGGCTACGGACCCCCTCAGGCCATGGTTTTGTGA

**SEQ ID NO: 231, protein - *Oryza sativa***
MLPTNRNRPQQRPARSWYFISDMDFSDPKRKPRYLSKILMVALLTAMCVVMLTQPPCHRRTPSVFSIHEPGV
THVLVTGGAGYIGSHAALRLLKDSFRVTIVDNLSRGNMGAIKVLQNLFSEPGRLQFIYADLGDPKAVNRIFA
ENAFDAVMHFAAVAYVGESTLEPLRYYHNITSNTLVVLEAMAAHNVRTLIYSSTCATYGEPEKMPITEGTPQ
FPINPYGKAKKMAEDIILDFSKSKKADMAVMILRYFNVIGSDPEGRLGEAPKPELREHGRISGACFDAALGI
IPGLKVKGTDYETPDGTCVRDYIDVTDLVDAHVKALNKAERGKVGIYNVGTGKGRSVKEFVEACKKATGVDI
KVDYFPRRPGDYAEVYSDPAKINSELNWTAQHTDLLESLRVAWTWQKKHRSGYGPPQAMVL

**SEQ ID NO: 232, DNA - *Oryza sativa***
ATGGCGGTGGAGAAGACGGTGCCGGGCGGGGTGAGGACGGTGCTGGTGACGGGCGGGGCGGGGTACATCGGC
AGCCACGCCGTGCTGCAGCTCCTCCTCGCCGGATTCCGCGCCGTCGTCGTCGACAACCTCAACAACTCCTCC
GAGCTCGCCGTCCGCCGCGTCGCCGCCCTCGCCGGCGACCACTCCCGGAACCTCGCCTTCCACAAGGTTGAC
CTCCGTGACAAGGGAGCACTGGAAAAGGTTTTTGCTTCAACAAGATTCGATGCTGTAGTTCACTTTGCTGGG
CTAAAAGCTGTGGGCGAAAGTGTGCAGAAGCCATTGCTTTATTACGACAACAATGTTAATGGAACAGTAAAT
CTTCTGGAAGTTATGTCTGCCCATGGATGTAAGAAGTTGGTGTTCTCATCATCAGCTGCAGTTTATGGATCA
CCGAAGAACTCACCCTGCACAGAAGAGTTTCCTCTCACTCCAAACAATCCATATGGCAAAACAAAGCTTGTT
GTTGAAGATATTTGCCGTGATATCTACCGTACAGATCCTGAATGGAAGATTATTCTACTTAGGTACTTCAAT
CCAGTTGGGGCTCATCCTAGCGGATACCTTGGGGAAGATCCATGTGGCATTCCCAACAATCTTATGCCTTAT
GTTCAGCAAGTTGCTGTTGGCAGGAGGCCAGCTCTGACAATACTAGGAAATGATTATGCAACCAGAGATGGT
ACCGGGGTCCGAGATTACATCCATGTGGTTGACCTTGCTGATGGACATATTGCGGCATTGCAGAAACTCTTT
GAGAGCTCCAGCATAGGGTGTGAAGCATACAACCTTGGAACTGGTAAAGGTACCTCTGTGCTGGAGATAGTC
AAGGCATTTGAGAAGGCTTCTGGGAAGAAAATTCCTCTGATTATTGGCCCAAGACGCCCTGGTGATGCGGAG
ATTCTATTTTCATTACCTGCTAAAGCAGAGAAGGAACTCAACTGGAAAGCGAAATTTGGCATCGATGAAATG
TGTAGGGATCAATGGAACTGGGCTAGCAAGAACCCTTATGGATACGGGTCACTTGACTCCACCAAGCAGAAC
GGACACCACTCGTACGGATCAATTGGCTCTCCCAAGCAGAATGGCCACTGCACAAATGGATTTTCTGAATCC
ACTAGGCATAATGGTCACAACGGATATGGACTGGTCGACTCTGCCAAGCATAACGGCAACGGCCACTTCCAC
TGA

**SEQ ID NO: 233, protein - *Oryza sativa***
MAVEKTVPGGVRTVLVTGGAGYIGSHAVLQLLLAGFRAVVVDNLNNSSELAVRRVAALAGDHSRNLAFHKVD
LRDKGALEKVFASTRFDAVVHFAGLKAVGESVQKPLLYYDNNVNGTVNLLEVMSAHGCKKLVFSSSAAVYGS
PKNSPCTEEFPLTPNNPYGKTKLVVEDICRDIYRTDPEWKIILLRYFNPVGAHPSGYLGEDPCGIPNNLMPY
VQQVAVGRRPALTILGNDYATRDGTGVRDYIHVVDLADGHIAALQKLFESSSIGCEAYNLGTGKGTSVLEIV
KAFEKASGKKIPLIIGPRRPGDAEILFSLPAKAEKELNWKAKFGIDEMCRDQWNWASKNPYGYGSLDSTKQN
GHHSYGSIGSPKQNGHCTNGFSESTRHNGHNGYGLVDSAKHNGNGHFH

**Figure 23 (continued)**

**SEQ ID NO: 234, DNA - *Oryza sativa***

ATGGCGGGGGGCGAGGTGGTGGCGGTGGCGGCGGCGGCGGCGGGGACGAGCAGGACGGTGCTGGTGACGGGA
GGGGCCGGGTACATCGGGAGCCACACCGTGCTGCAGCTGCTCGCCGCGGGGTTCCGCGTCGTCGTCGCCGAC
AGCCTCGGGAACTCCTCCGAGCTCGCCGTCCGCCGCGTCGCCGCGCTCGCCGGGGACAAGGCGCGGAACCTC
TCCTTACACAAGGTTGATATTCGTGATAAGGGTGGACTGGAAAAGGTTTTTTCTTCGACAAGATTTGATGCT
GTCGTTCATTTTGCTGGTCTGAAAGCTGTGGGTGAAAGTGTGCAGAAGCCATTGCTTTATTATGACCATAAT
GTTGCTGGGACAATAATTCTTCTAGAAGTTATGGCAGCCCATGGATGTAAAAAGTTGGTGTTTTCATCGTCA
GCTGCAGTTTATGGATCACCTAAAAACTCACCCTGCACAGAAGAGTTTCCTCTCACTCCACACAATCCATAT
GGCAGAACCAAGCTCATAGCAGAGGAGATTTGCCGTGATATATACCATTCAGATTCTGAATGGAGCATAATT
TTACTTAGGTACTTCAATCCTGTTGGAGCTCATCCCAGTGGATACCTTGGTGAAGACCCATGTGGAATTCCT
AACAACCTCATGCCCTTCGTCCAGCAAGTTGCTGTCGGGAGGAGACCGTCACTTACAATATTTGGAAATGAC
TATGCAACAAAAGATGGGACAGGGGTACGTGATTATATTCATGTAGTTGATCTTGCTGAGGGGCATATTGCT
GCACTACGGAAGCTGTTTGAAAGCTCAATAGGATGTCAAGCATACAACCTTGGAACAGGAAAGGGAACATCA
GTGTTGGAAATAGTTAATGCATTTGAGAAAGTTTCTGGAAAGAAAATACCTTTGGTTATTGGTCCACGACGC
CCTGGTGATGCTGAGATTCTCTTTTCGTCAGCTGCTAAAGCAGAGAGAGAATTCAAGTGGAAGGCAAAATAT
GGCATCGAAGAAATGTGCAGAGACCAATGGAACTGGGCGAGCAAGAATCCTTTCGGGTACGCCTCGCCCGAC
TCCACCAAGCAGAATGGTCATTCACACTGA

**SEQ ID NO: 235, protein - *Oryza sativa***

MAGGEVVAVAAAAAGTSRTVLVTGGAGYIGSHTVLQLLAAGFRVVVADSLGNSSELAVRRVAALAGDKARNL
SLHKVDIRDKGGLEKVFSSTRFDAVVHFAGLKAVGESVQKPLLYYDHNVAGTIILLEVMAAHGCKKLVFSSS
AAVYGSPKNSPCTEEFPLTPHNPYGRTKLIAEEICRDIYHSDSEWSIILLRYFNPVGAHPSGYLGEDPCGIP
NNLMPFVQQVAVGRRPSLTIFGNDYATKDGTGVRDYIHVVDLAEGHIAALRKLFESSIGCQAYNLGTGKGTS
VLEIVNAFEKVSGKKIPLVIGPRRPGDAEILFSSAAKAEREFKWKAKYGIEEMCRDQWNWASKNPFGYASPD
STKQNGHSH

**SEQ ID NO: 236, DNA - *Oryza sativa***

ATGCCGGCGGACGCGGCGGCGAAGGGGATGAAGCTGGAGAGGTACGCGAGCGGCGCGGGCGCGATGCTGCTG
CTGCGGCGGGCGGCGAGCGGGAAGGTCGTGTCGGCGTCGTCGCACCTGCTGTTCCGCGCCACCGTGCTCGCC
ACGATGGCGCTCGTGTTCCTCTTCACGTTCCACTACCCGTCGCTGCTGTCGCGCTCCTTCACGCTCTCCTCC
GGCGCCGGCGCCGGCGAGGGGGGAGCCGCGGCGCACGCCTCGCACCGGAGCTTGCTCATGTCGTCGTCGTCG
GCGTCGGCGTCGGCGGCGTCGGTGTACGGGGGCGCGGCGTGGGAGAAGGAGGTGAGGCGGAGCGCGAAGCCG
AGGAAGGACGGGGGGATAGCCGTGCTGGTGACCGGCGCGGCGGGGTTCGTCGGCACGCACTGCTCGCTGGCG
CTGAGGGCGCGCGGCGACGGCGTGCTGGGGCTCGACAACTTCAACGCCTACTACGACCCGGAGCTGAAGCGC
GCGCGGCAGAGGCTCCTCGCCGGCCGCGGCGTGCTCGTGCTCGACGCCGACATCAACGACGCGCTCCTGCTC
GAGAAGCTGTTCGACCTGGTGCCGTTCACCCACGTGCTGCACCTCGCCGCGCAGGCCGGCGTGCGCTACGCC
ATGGAGGCGCCGCAGACGTACGTGGCGTCCAACGTGGCCGGGCTCGTGACCGTCCTCGAGGTCGCCGCCAAG
CACGCCGACCCGCAGCCGGCGATCGTCTGGGCGTCGTCGTCGTCGGTGTACGGCCTCAACACCGACGCACCC
TTCTCCGAGGAGCACCGCACCGACCGGCCGGCGTCGCTGTACGCGGCGACCAAGAAGGCCGGCGAGGCCATC
GCGCACACGTACAACCACATCTACGGCCTCTCCATCACCGGCCTCCGCTTCTTCACCGTGTACGGGCCATGG
GGCCGCCCCGACATGGCCTACTTCTTCTTCGCCAAGAGCATCGTCTCCGGCGAGCCCATCACGCTGTTCCGC
GCCGCCGACGGCGCCGACGCGCGCCGCGATTTCACCTACATCGACGACGTCGTCAAGGGCTGCCTCGGCGCG
CTCGACACCTCCGGCAAGAGCACCGGCTCCTCCAAGTCCGGCAAGAAGTCCGGCCCGGCGCCGCTCCGCGTC
TACAACCTCGGCAACACCTCGCCGGTGCCGGTCACCCGCATGGTCGCCATCCTCGAGAAGCTCCTCGGCAAG
AAGGCCAACAAGCGCATCGTCGCCATGCCC

## Figure 23 (continued)

AGCAATGGCGACGTGCCCTTCACCCACGCCAACGTCACCCACGCCGCCCACGACTTCGGCTACCGCCCCACC
ACCTCCCTCGACGCCGGCCTCCGCCACTTCGTCGACTGGTTCGCCGACTACTACAAGCTCAAGCTCGACGTC
CCCAAGATCGCCGCCAAGGTCGCCGGCGCCGGCAAGCCCTCCTCCTCGTCGGCCTCCAAGAAGAAGAAGAAG
GCCGCGGCGATGTCGGCGTCATCATGA

**SEQ ID NO: 237, protein - *Oryza sativa***
MPADAAAKGMKLERYASGAGAMLLLRRAASGKVVSASSHLLFRATVLATMALVFLFTFHYPSLLSRSFTLSS
GAGAGEGGAAAHASHRSLLMSSSSASASAASVYGGAAWEKEVRRSAKPRKDGGIAVLVTGAAGFVGTHCSLA
LRARGDGVLGLDNFNAYYDPELKRARQRLLAGRGVLVLDADINDALLLEKLFDLVPFTHVLHLAAQAGVRYA
MEAPQTYVASNVAGLVTVLEVAAKHADPQPAIVWASSSSVYGLNTDAPFSEEHRTDRPASLYAATKKAGEAI
AHTYNHIYGLSITGLRFFTVYGPWGRPDMAYFFFAKSIVSGEPITLFRAADGADARRDFTYIDDVVKGCLGA
LDTSGKSTGSSKSGKKSGPAPLRVYNLGNTSPVPVTRMVAILEKLLGKKANKRIVAMPSNGDVPFTHANVTH
AAHDFGYRPTTSLDAGLRHFVDWFADYYKLKLDVPKIAAKVAGAGKPSSSSASKKKKKAAAMSASS

**SEQ ID NO: 238, DNA - *Oryza sativa***
ATGGTGAGCGGCGGAGGAGTAGCGGCGGAGAACGGCGAGATGGTGGGGAACGGGGAGGGGAGGAAGGGTGCG
GGGGCGAGCGTGCTGGTGACGGGGGGAGCGGGGTACATCGGGACGCACACGGTGCTGCGGCTGCTGGAGAAG
GGGTTCGCGGTCACCGTCGTCGACAACTTCCACAACTCCGTCCCGGAGGCGCTCGACCGCGTCCGCCTCATC
GCCGGCGCCGCCCTCTCCGCCCGCCTCGACTTCATCGCCGGGGATCTCAAGAGCAAGGACGACATGGAGAAG
GTGTTCGCCGCCAAGAGGTATGACGCCGTGATCCACTTCGCCGGGCTGAAGGCGGTGGGGGAGAGCGTCGCG
CACCCGCAGATGTACTACGAGAACAACGTCGCCGGCACCATGAACCTCTACTCCGCCATGACCAAGTACGGC
TGCAAGAAGATAGTGTTCTCGTCGTCGGCGACGGTGTACGGCCAGCCGGAGAAGACCCCCTGCGTCGAGGAT
TCCAAGCTGAGCGCTCTCAACCCATACGGCACCACCAAGCTCGTCCTGGAGAACTACTTCCGGCAGGTGCAG
GCCGCCGACCCGGAGATGAGGGTGATCCTGCTCAGGTACTTCAACCCCATCGGCGCTCACCGGAGCGGCGAC
ATCGGGGAGGACCCCAGGGGCATCCCCAACAACCTTCTTCCGTACATCCAGCAGGTCGCCGTCGGCCGCCGC
CCCGAGCTCAACGTCTACGGCGTCGACTACCCAACCAGGGACGGCACCGCGATCAGGGATTACATACATGTA
GTGGACCTTGCCGATGGCCACATTGCCGCACTGGAGAAGCTCTTCGCTACTCCTGACATTGGTTGTGTGGCT
TACAATCTAGGAACAGGGTGTGGAACAACGGTGCTCGAGGTGGTGAAGGCGTTCGAGGAGGCGTCCGGAAAG
AAAATTCCTATCAAGATTTGCCCCAGAAGACCTGGAGATTGCACTGAGGTTTACGCTTCCACTGACAAGGCC
AAGAAGGAGCTCGGATGGAGTGCTCGGTTTGGAATAGAGGACATGTGCAGGGACCAGTGGAATTGGGCCAAG
AAGAATCCGTACGGATACAGCGCCAATGCTGAGCAGAATTAG

**SEQ ID NO: 239, protein - *Oryza sativa***
MVSGGGVAAENGEMVGNGEGRKGAGASVLVTGGAGYIGTHTVLRLLEKGFAVTVVDNFHNSVPEALDRVRLI
AGAALSARLDFIAGDLKSKDDMEKVFAAKRYDAVIHFAGLKAVGESVAHPQMYYENNVAGTMNLYSAMTKYG
CKKIVFSSSATVYGQPEKTPCVEDSKLSALNPYGTTKLVLENYFRQVQAADPEMRVILLRYFNPIGAHRSGD
IGEDPRGIPNNLLPYIQQVAVGRRPELNVYGVDYPTRDGTAIRDYIHVVDLADGHIAALEKLFATPDIGCVA
YNLGTGCGTTVLEVVKAFEEASGKKIPIKICPRRPGDCTEVYASTDKAKKELGWSARFGIEDMCRDQWNWAK
KNPYGYSANAEQN

**SEQ ID NO: 240, DNA - *Arabidopsis thaliana***
ATGGGTTCTTCTGTGGAGCAGAACATTCTTGTTACTGGTGGTGCTGGCTTTATCGGGACGCATACTGTTGTT
CAACTTCTCAAAGATGGTTTTAAGGTTTCGATCATCGATAATTTTGATAACTCTGTTATCGAAGCTGTTGAT
AGAGTTAGGGAGCTTGTTGGTCCTGATCTCTCCAAGAAGCTCGACTTCAATCTGGGTGATCTAAGAAACAAA
GGGGACATTGAGAAACTATTCTCCAAGCAGAGAGATTTGATGCTGTGATTCATTTTGCGGGTCTTAAAGCTGTG
GGTGAGAGTGTTGAAAACCCTCGCCGCTACTTTGACAATAAC

## Figure 23 (continued)

TTGGTTGGAACAATCAATCTATATGAGACCATGGCAAAGTACAACTGCAAAATGATGGTGTTTTCATCTTCT
GCCACTGTTTATGGACAACCTGAAAAGATTCCATGCATGGAAGACTTTGAATTAAAGGCTATGAATCCTTAT
GGTCGTACTAAGCTCTTTCTTGAAGAAATAGCTAGAGATATTCAAAAGGCAGAACCGGAATGGAGAATTATT
CTGCTGAGGTACTTCAATCCTGTAGGAGCACATGAGAGTGGCAGTATTGGTGAGGATCCAAAAGGCATCCCC
AATAACCTCATGCCTTACATCCAACAAGTGGCCGTTGGACGTTTACCGGAACTCAATGTCTATGGACATGAC
TATCCCACCGAGGATGGTAGTGCGGTAAGAGACTACATCCATGTGATGGATTTAGCAGATGGCCATATCGCT
GCGCTCAGGAAGCTATTTGCTGATCCAAAGATTGGTTGTACTGCTTACAATCTAGGGACTGGTCAAGGAACG
TCTGTGTTAGAAATGGTTGCAGCTTTTGAAAAAGCTTCCGGCAAGAAAATCCCGATTAAGCTCTGTCCGAGA
AGGTCAGGAGATGCAACAGCAGTTTATGCTTCAACAGAGAAGGCTGAGAAAGAACTTGGCTGGAAGGCAAAA
TATGGAGTGGATGAGATGTGCAGAGATCAGTGGAAATGGGCAAACAATAATCCATGGGGTTACCAGAATAAG
CTTTGA

**SEQ ID NO: 241, protein - *Arabidopsis thaliana***
MGSSVEQNILVTGGAGFIGTHTVVQLLKDGFKVSIIDNFDNSVIEAVDRVRELVGPDLSKKLDFNLGDLRNK
GDIEKLFSKQRFDAVIHFAGLKAVGESVENPRRYFDNNLVGTINLYETMAKYNCKMMVFSSSATVYGQPEKI
PCMEDFELKAMNPYGRTKLFLEEIARDIQKAEPEWRIILLRYFNPVGAHESGSIGEDPKGIPNNLMPYIQQV
AVGRLPELNVYGHDYPTEDGSAVRDYIHVMDLADGHIAALRKLFADPKIGCTAYNLGTGQGTSVLEMVAAFE
KASGKKIPIKLCPRRSGDATAVYASTEKAEKELGWKAKYGVDEMCRDQWKWANNNPWGYQNKL

**SEQ ID NO: 242, DNA - *Arabidopsis thaliana***
ATGGGTTCTTCTGTGGAACAGAACATTTTGGTGACTGGTGGTGCTGGATTCATTGGAACACATACTGTTGTT
CAGCTTTTGAATCAGGGTTTTAAGGTTACGATCATTGATAATCTTGATAACTCTGTCGTTGAAGCTGTTCAT
AGGGTTAGGGAACTTGTTGGTCCTGATCTCTCTACCAAGCTTGAATTCAATCTGGGTGATCTAAGAAACAAA
GGAGATATTGAGAAACTCTTTTCCAATCAGAGATTTGATGCTGTGATTCACTTTGCTGGTCTTAAAGCTGTG
GGAGAAAGTGTTGGAAACCCTCGTCGTTACTTTGATAATAACTTAGTTGGAACTATCAATCTATATGAGACC
ATGGCAAAATACAACTGCAAAATGATGGTGTTTTCGTCGTCTGCAACAGTTTATGGTCAACCTGAAATAGTC
CCATGTGTGGAAGACTTTGAGTTACAGGCTATGAATCCTTATGGTCGTACTAAGCTGTTTCTTGAAGAGATA
GCTAGAGACATTCACGCTGCGGAACCAGAATGGAAGATAATTCTTCTGAGGTACTTCAATCCGGTTGGAGCT
CACGAGAGTGGAAGAATTGGAGAAGATCCAAAGGGCATACCGAATAATCTCATGCCTTATATCCAACAAGTG
GCGGTTGGAAGATTACCTGAACTCAATGTATTTGGACATGATTATCCTACCATGGATGGTAGCGCGGTTAGA
GACTACATCCATGTAATGGATTTAGCAGATGGCCATGTGGCTGCGTTAAACAAATTGTTTTCAGACTCAAAG
ATTGGCTGTACTGCTTATAATCTTGGCACTGGTCAAGGAACTTCTGTCTTAGAAATGGTTTCTTCTTTTGAA
AAAGCTTCTGGCAAGAAAATACCTATCAAGCTATGTCCAAGAAGAGCTGGAGATGCAACAGCTGTTTATGCT
TCAACTCAGAAAGCTGAGAAGGAACTTGGCTGGAAGGCAAAATATGGAGTTGATGAGATGTGCAGAGATCAA
TGGAACTGGGCAAATAAGAATCCATGGGGTTTCCAGAAGAAGCCTTGA

**SEQ ID NO: 243, protein - *Arabidopsis thaliana***
MGSSVEQNILVTGGAGFIGTHTVVQLLNQGFKVTIIDNLDNSVVEAVHRVRELVGPDLSTKLEFNLGDLRNK
GDIEKLFSNQRFDAVIHFAGLKAVGESVGNPRRYFDNNLVGTINLYETMAKYNCKMMVFSSSATVYGQPEIV
PCVEDFELQAMNPYGRTKLFLEEIARDIHAAEPEWKIILLRYFNPVGAHESGRIGEDPKGIPNNLMPYIQQV
AVGRLPELNVFGHDYPTMDGSAVRDYIHVMDLADGHVAALNKLFSDSKIGCTAYNLGTGQGTSVLEMVSSFE
KASGKKIPIKLCPRRAGDATAVYASTQKAEKELGWKAKYGVDEMCRDQWNWANKNPWGFQKKP

## Figure 23 (continued)

**SEQ ID NO: 244, DNA - *Arabidopsis thaliana***
ATGGTTGGGAATATTCTGGTGACCGGTGGTGCTGGTTACATCGGAAGTCACACGGTTCTTCAGCTTCTTCTC
GGAGGCTATAACACCGTCGTTATAGACAACCTCGACAATTCCTCTCTCGTTTCGATCCAACGCGTCAAGGAT
CTCGCCGGAGATCATGGACAAAATCTCACCGTCCACCAGGTGGACCTTCGCGATAAACCCGCACTTGAGAAG
GTTTTCTCCGAAACAAAGTTTGATGCAGTAATGCATTTTGCTGGATTGAAAGCAGTTGGTGAGAGCGTGGCG
AAACCACTTCTGTATTATAACAATAACTTGATTGCGACTATTACACTTTTGGAAGTAATGGCTGCACACGGA
TGTAAAAAGCTTGTATTTTCTTCGTCCGCTACTGTGTATGGCTGGCCAAAGGAGGTTCCTTGTACAGAAGAG
TCTCCCCTGTCTGGAATGAGTCCTTATGGACGGACAAAGCTGTTCATAGAGGACATTTGCCGTGATGTACAA
CGTGGTGATCCTGAATGGAGAATCATAATGCTGAGGTACTTTAACCCTGTGGGAGCTCACCCTAGCGGTCGC
ATTGGTGAGGATCCTTGTGGGACTCCAAATAATCTCATGCCTTATGTCCAGCAAGTCGTTGTTGGGAGGCTA
CCTAACCTAAAAATTTATGGAACTGACTATACCACTAAAGATGGCACTGGTGTACGAGACTATATTCATGTT
GTTGATCTAGCAGATGGCCATATATGTGCGCTTCAAAAGCTAGACGATACTGAAATAGGTTGTGAGGTATAC
AACCTGGGAACCGGAAAAGGAACAACAGTGTTGGAGATGGTTGATGCATTTGAGAAAGCTTCTGGAATGAAA
ATCCCACTGGTGAAGGTTGGAAGGAGACCAGGTGATGCAGAAACCGTCTATGCGTCAACAGAAAAAGCTGAA
CGCGAACTAAACTGGAAGGCAAATTTTGGAATCGAAGAAATGTGTAGGGATCAGTGGAACTGGGCAAGCAAC
AATCCTTTCGGTTACGGTTCTTCACCAAACTCAACATAA

**SEQ ID NO: 245, protein - *Arabidopsis thaliana***
MVGNILVTGGAGYIGSHTVLQLLLGGYNTVVIDNLDNSSLVSIQRVKDLAGDHGQNLTVHQVDLRDKPALEK
VFSETKFDAVMHFAGLKAVGESVAKPLLYYNNNLIATITLLEVMAAHGCKKLVFSSSATVYGWPKEVPCTEE
SPLSGMSPYGRTKLFIEDICRDVQRGDPEWRIIMLRYFNPVGAHPSGRIGEDPCGTPNNLMPYVQQVVVGRL
PNLKIYGTDYTTKDGTGVRDYIHVVDLADGHICALQKLDDTEIGCEVYNLGTGKGTTVLEMVDAFEKASGMK
IPLVKVGRRPGDAETVYASTEKAERELNWKANFGIEEMCRDQWNWASNNPFGYGSSPNST

**SEQ ID NO: 246, DNA - *Arabidopsis thaliana***
ATGATGGCTAGAAACGTTCTAGTAAGCGGTGGTGCTGGATACATCGGTAGTCACACGGTGCTTCAACTTCTT
CTCGGTGGTTACTCCGTCGTAGTTGTTGATAATCTCGATAATTCCTCCGCCGTCTCTCTTCAACGTGTGAAG
AAACTCGCGGCTGAACACGGAGAACGCCTCTCCTTCCACCAGGTGGATCTTCGAGACAGGTCTGCTCTTGAG
AAGATATTCTCAGAAACAAAGTTTGATGCAGTGATACATTTTGCTGGACTTAAAGCAGTTGGTGAGAGTGTA
GAGAAGCCTTTGCTTTATTATAATAATAATCTTGTTGGTACTATTACTCTTTTGGAAGTTATGGCTCAACAT
GGCTGCAAAAATCTTGTGTTTTCATCATCAGCAACTGTGTATGGCTCTCCAAAAGAAGTTCCTTGTACAGAG
GAGTTTCCAATTTCTGCTTTGAACCCATATGGACGAACAAAGCTATTCATTGAGGAAATCTGCCGCGATGTA
TACGGTTCTGACCCGGAATGGAAGATTATATTGCTTAGGTATTTCAATCCTGTTGGTGCACATCCTAGTGGT
GACATTGGTGAAGACCCTCGTGGTATTCCAAACAATCTCATGCCCTTTGTACAACAAGTGGCTGTTGGTAGG
AGACCTCATCTCACTGTCTTTGGAAATGATTACAATACAAAAGATGGAACAGGGGTTCGAGATTACATTCAT
GTTATTGATTTAGCTGATGGACACATAGCGGCTCTACGTAAGCTAGAAGATTGCAAGATCGGTTGTGAAGTG
TACAATCTCGGAACAGGAAATGGAACATCAGTTCTTGAAATGGTTGATGCTTTTGAAAAAGCATCCGGAAAG
AAAATCCCTCTGGTGATCGCGGGACGTCGACCGGGAGATGCTGAAGTTGTATACGCCTCGACGGAAAGAGCA
GAAAGTGAATTGAATTGGAAGGCCAAATATGGGATTGAGGAGATGTGTAGAGATTTGTGGAACTGGGCTAGT
AATAATCCTTATGGATATGATTCCTCTTCTGAAGACAACTCTCATTAA

**SEQ ID NO: 247, protein - *Arabidopsis thaliana***
MMARNVLVSGGAGYIGSHTVLQLLLGGYSVVVVDNLDNSSAVSLQRVKKLAAEHGERLSFHQVDLRDRSALE
KIFSETKFDAVIHFAGLKAVGESVEKPLLYYNNNLVGTITLLEVMAQHGCKNLVFSSSATVYGSPKEVPCTE
EFPISALNPYGRTKLFIEEICRDVYGSDPEWKIILLRYFNPVGAHPSGDIGEDP

## Figure 23 (continued)

RGIPNNLMPFVQQVAVGRRPHLTVFGNDYNTKDGTGVRDYIHVIDLADGHIAALRKLEDCKIGCEVYNLGTG
NGTSVLEMVDAFEKASGKKIPLVIAGRRPGDAEVVYASTERAESELNWKAKYGIEEMCRDLWNWASNNPYGY
DSSSEDNSH

**SEQ ID NO: 248, DNA - *Arabidopsis thaliana***
ATGGCGAAGAGTGTTTTGGTTACCGGCGGCGCTGGCTATATCGGAAGTCATACTGTTCTTCAACTGCTTGAA
GGTGGTTACTCCGCCGTCGTTGTTGACAATTACGACAATTCCTCTGCTGCTTCGCTTCAGCGCGTGAAGAAA
CTCGCCGGCGAAAACGGAAACCGCCTCTCCTTCCACCAGGTGGATCTCCGAGACAGGCCTGCGCTTGAGAAG
ATATTCTCAGAAACAAAGTTTGATGCAGTGATACACTTTGCTGGACTTAAAGCAGTAGGTGAGAGTGTGGAG
AAGCCTTTGCTCTATTATAATAATAACATTGTTGGCACTGTTACTCTTTTGGAGGTTATGGCTCAATACGGC
TGCAAAAATCTTGTATTTTCATCATCAGCTACTGTCTATGGCTGGCCTAAGGAGGTTCCTTGCACTGAGGAG
TCACCAATTTCTGCCACTAACCCATATGGCCGTACAAAGCTTTTTATCGAAGAGATTTGCCGGGATGTACAT
CGTTCTGACTCGGAATGGAAGATCATATTGCTTAGATACTTCAACCCTGTTGGTGCACATCCTAGTGGTTAC
ATTGGTGAAGATCCTCTTGGAGTTCCCAACAATCTCATGCCTTATGTCCAACAAGTTGCAGTTGGCCGGAGA
CCTCACCTTACTGTCTTTGGAACCGACTACAAGACAAAGGATGGTACAGGGGTTAGAGATTACATTCATGTC
ATGGATTTAGCAGATGGACACATAGCTGCTCTGCGTAAGCTAGATGATCTCAAAATCAGTTGTGAGGTATAC
AATCTTGGAACGGGTAATGGAACATCGGTCCTAGAAATGGTCGCTGCCTTTGAAAAAGCATCCGGAAAGAAA
ATCCCTTTGGTGATGGCTGGACGACGTCCTGGAGACGCTGAAGTTGTTTACGCCTCAACGGAAAAAGCAGAG
CGCGAACTAAATTGGAAGGCTAAGAATGGGATAGAGGAGATGTGTAGGGATCTATGGAATTGGGCAAGCAAT
AACCCCTACGGCTACAATTCCTCCTCCAATGGCTCCTCTTCATAA

**SEQ ID NO: 249, protein - *Arabidopsis thaliana***
MAKSVLVTGGAGYIGSHTVLQLLEGGYSAVVVDNYDNSSAASLQRVKKLAGENGNRLSFHQVDLRDRPALEK
IFSETKFDAVIHFAGLKAVGESVEKPLLYYNNNIVGTVTLLEVMAQYGCKNLVFSSSATVYGWPKEVPCTEE
SPISATNPYGRTKLFIEEICRDVHRSDSEWKIILLRYFNPVGAHPSGYIGEDPLGVPNNLMPYVQQVAVGRR
PHLTVFGTDYKTKDGTGVRDYIHVMDLADGHIAALRKLDDLKISCEVYNLGTGNGTSVLEMVAAFEKASGKK
IPLVMAGRRPGDAEVVYASTEKAERELNWKAKNGIEEMCRDLWNWASNNPYGYNSSSNGSSS

**SEQ ID NO: 250, DNA - *Populus tremuloides***
ATGGATTATCTGGATTCAAGACGAAAGAGCAGATGTGCTGGAAAAATTATTGCAGCTGCGCTCTGCATTACA
TTCTCCCAGCATGAGCTCGGCGTAACGCATGTCTTAGTGACTGGAGGTGCTGGGTATATTGGCTCTCATGCT
GCACTTCGACTCTTGAAGGATTCATACCGTGTAACTAAAGTGGACAATCTTTCTCGAGGGAATCTGGGTGCT
GTTAAAGTTCTCCAAGATCTATTTCCGGAGCCTGGGCGGTTACAGTTCATATACGCAGACTTGGGAGATGCA
AAAGCTGTAAACAAAATCTTTGCTGAAAATGCATTTGATGCTGTGATGCATTTCGCTGCTGTTGCATATGTT
GGTGAAAGTACAATGGAACCTCTCAGATATTATCACAACATCACATCAAATACCTTGGTAGTTTTAGAAGCA
ATGGCAGCACATAAGGTGAAGACATTGATTTATTCTAGCACTTGTGCAACTTACGGCGAGCCTGTTAAGATG
CCGATTACAGAACAAACTCCTCAGCTTCCAATCAACCCCTATGGAAAAGCCAAGAAAATGGCAGAAGATATC
ATAATTGACTTCTCAAAAACCACTGACATGGCTGTCATGATCCTAAGATACTTCAATGTTATTGGGTCTGAT
CCAGAAGGAAGATTAGGGGAAGCTCCGCAACCTGAACTTCGTGAGCATGGCCGAATCTCTGGCGCTTGTTTT
GATGCAGCTCGAGGAATAATACCAGGACTAAAACAGAGAATCACAATTCAGTGGATAGACTATAAAACTGCT
GATGGCACGTGTGTACGGGACTATATCGATGTCACTGACCTGGTCGATGCCCATGTGAAAGCTCTTGCCCAT
GCAAAGCCTCGAAAAGTTGGCATTTACAATGTTGGAACTGGAAAAGGTAGATCAGTGAAGGAGTTTGTCGAG
GCATGTAAGAAGGCAACAGGTGTGGACATAAAGGTTGAATACCTCAATCGTCGGCCAGGAGACTATGCTGAG
GTCTTCAGTGACCCTTCCAAAATAAAACAAGAGCTAAACTGGAAAGCTCAATATACTGACCTTAAGAAGAGT
TTGCAGATTGCATGGAAATGGCAGAAGTCACATTTGAATGGTTACAGTCACAGTTAA

**Figure 23 (continued)**

**SEQ ID NO: 251, protein - *Populus tremuloides***
MDYLDSRRKSRCAGKIIAAALCITFSQHELGVTHVLVTGGAGYIGSHAALRLLKDSYRVTKVDNLSRGNLGA
VKVLQDLFPEPGRLQFIYADLGDAKAVNKIFAENAFDAVMHFAAVAYVGESTMEPLRYYHNITSNTLVVLEA
MAAHKVKTLIYSSTCATYGEPVKMPITEQTPQLPINPYGKAKKMAEDIIIDFSKTTDMAVMILRYFNVIGSD
PEGRLGEAPQPELREHGRISGACFDAARGIIPGLKQRITIQWIDYKTADGTCVRDYIDVTDLVDAHVKALAH
AKPRKVGIYNVGTGKGRSVKEFVEACKKATGVDIKVEYLNRRPGDYAEVFSDPSKIKQELNWKAQYTDLKKS
LQIAWKWQKSHLNGYSHS

**SEQ ID NO: 252, DNA - *Populus tremuloides***
ATGCTAAACTTTGGCAGGACCAGAGCTCAGACAAGGTCCAATAGATCTATATCTCTTGGAGGCATGGATTAT
TCAGATCCAAAAAGGAAAAACAACGTTGTAGGAAAGATTCTTTTGGCTGCTACCCTCACAGCTTTATGTATA
ATTATGCTAAAGCAATCTCCGACCTTTTATTCTCCAAGCCCGTTCTCTTTGCATGAAGAAGGGGTGATCCAT
GTCCTAGTGACAGGTGGTGCTGGCTACATTGGTTCCCATGCTGCATTGCGACTTTTGAAGGATGGTTACCGA
GTAACCATAGTGGACAACCTTTCTCGAGGAAACATAGGTGCAGTCAAGGTTTTACAAGAATTATTTCCAGAG
CCCGGGAGGCTTCAGTTTATATATGCTGACCTGGGAGATCCTAAAACTGTTAACATTATCTTCTCACAAAAT
GCATTTGATGCTGTGATGCATTTTGCGGCAGTTGCATATGTTGGGGAAAGCACCATGGAACCCCTGAAGTAC
TATCACAATATCACATCAAATACCTTGGTAGTTTTGGAGGCAATGGCTGCAAATGATGTAAAGACTTTGATA
TATTCAAGTACATGTGCAACATATGGGGAGCCTGAAAAGATGCCGATTACTGAAGTAACTCCACAGGTACCC
ATTAATCCATATGGAAAAGCTAAGAAGATGGCAGAAGATATCATCCTTGATTTTTCTAAAAACTCAGACATG
GCAATTATGATATTGAGATACTTCAATGTGATTGGATCAGATCCAGATGGCAGGTTAGGTGAGGCTCCTAGA
CCTGAACTGCGTGAGCATGGACGAATTTCCGGTGCTTGTTTCGATGCAGCTCGTGGTATTATTGCTGGGCTG
AAGGTTAAAGGAACAGACTATAAGACGCATGATGGAACTTGTATAAGAGATTATATTGACGTTACTGATCTT
GTTGATGCTCATGTTAAAGCACTTGAAAAGGCAATGCCTGGTAAAGTAGGAATCTACAATGTTGGCACTGGA
AAGGGTAGATCAGTCAAGGAGTTTGTTAAGGCATGTAAAAAGGCAACTGGTGTAGATATCAAAGTTGACTAT
TTACCTCGCCGTCCTGGTGACTACGCTGAAGTATTCAGTGACCCATCAAAAATTTACCGCGAGCTGAACTGG
ACAGCGCAATACACGGATCTCCAGAAGAGCTTACAGACTGCATGGAGATGGCAAAAATCACATCAAAATGGG
TATGGATCCCCTTTGGTGATGGCTTCTTGA

**SEQ ID NO: 253, protein - *Populus tremuloides***
MLNFGRTRAQTRSNRSISLGGMDYSDPKRKNNVVGKILLAATLTALCIIMLKQSPTFYSPSPFSLHEEGVIH
VLVTGGAGYIGSHAALRLLKDGYRVTIVDNLSRGNIGAVKVLQELFPEPGRLQFIYADLGDPKTVNIIFSQN
AFDAVMHFAAVAYVGESTMEPLKYYHNITSNTLVVLEAMAANDVKTLIYSSTCATYGEPEKMPITEVTPQVP
INPYGKAKKMAEDIILDFSKNSDMAIMILRYFNVIGSDPDGRLGEAPRPELREHGRISGACFDAARGIIAGL
KVKGTDYKTHDGTCIRDYIDVTDLVDAHVKALEKAMPGKVGIYNVGTGKGRSVKEFVKACKKATGVDIKVDY
LPRRPGDYAEVFSDPSKIYRELNWTAQYTDLQKSLQTAWRWQKSHQNGYGSPLVMAS

**SEQ ID NO: 254, DNA - *Populus tremuloides***
ATGGTGCACAAGGGCTCTGGTATGGATTTCCTGGATTCAAGAAGAAAGAGCAATTCTGCTGGAAAAGTTATT
GCAGTTGCATTCTTCATTGCAGTATGCATTGTCATGCTCAAGCAAGTGTACTCACCTAGTTATACCAGCCCC
GACATGTTCTCCCAACATGAGCTAGGCGTAACGCATGTGTTGGTGACCGGAGGTGCTGGTTACATAGGTTCT
CACGCTGCACTTCGACTCTTGAAGGATTCATACCGAGTAACTATAGTGGACAATCTCTCTCGAGGGAATCTC
GGTGCGGTTAAAGTTCTTCAAGAGCTATTTCCAGAGCCTGGGAGATTGCAGTTCATATATGCTGACCTAGGA
GATGCAAAAGCTGTAAACAAAATCTTTGCCGAAAATGCATTTGATGCTGTGATGCATTTTGCTGCTGTTGCA
TATGTTGGTGAAAGTACAATAGAGCCCCTTAGATATTATCACAATATCACATCAAATACCTTGGTTGTCTTG
GAAGCAATGGCTGCACATAATGTGAAGACATTAATCTATTCGAGCACTTGTGCAACGTACGGAGAACCTATT
AAGATGCCGATTAGAGAA

## Figure 23 (continued)

GAAACTCCTCAGCTTCCAATCAACCCCTATGGAAAAGCCAAGAAAATGGCAGAAGATATCATAATTGACTTC
TCCAACACCACTGACATGGCTGTCATGATCCTAAGCATCCAGCATTCTGATCAAAATAATGCCTATGTTTAC
AGATACTTCAATGTTATTGGGTCTGACCCGGAAGGAAGATTAGGGGAAGCTCCACGACCTGAGCTTCGTGAG
CATGGCCGAATCTCTGGTGCTTGTTTTGATGCAGCTCGAGGGATAACACCTGGACTCAAGGTTAAAGGGACA
GACTATAAAACAGCTGATGGCACATGTGTACGCGACTATATCGATGTCACTGACCTGGTTGATGCCCATGTG
AAAGCTCTTGCCCACGCAAAACCTCGGAAAGTTGGCATTTACAATGTTGGAACTGGAAAAGGCAGATCAGTG
AAGGAGTTTGTTGATGCGTGTAAGAAGGCAACAGGCGTGGACATAAAGGTTGAATACCTTGATCGCCGGCCA
GGAGACTATGCTGAGGTCTTCAGTGATCCTTCCAAAATAAAACAAGAGCTAAGCTGGACAGCTCAATATACT
GACCTTCAGAAGAGTTTACAGATTGCTTGGAAATGGCAGAAGTCACATTTGAATGGTTATTGA

**SEQ ID NO: 255, protein - *Populus tremuloides***
MVHKGSGMDFLDSRRKSNSAGKVIAVAFFIAVCIVMLKQVYSPSYTSPDMFSQHELGVTHVLVTGGAGYIGS
HAALRLLKDSYRVTIVDNLSRGNLGAVKVLQELFPEPGRLQFIYADLGDAKAVNKIFAENAFDAVMHFAAVA
YVGESTIEPLRYYHNITSNTLVVLEAMAAHNVKTLIYSSTCATYGEPIKMPIREETPQLPINPYGKAKKMAE
DIIIDFSNTTDMAVMILSIQHSDQNNAYVYRYFNVIGSDPEGRLGEAPRPELREHGRISGACFDAARGITPG
LKVKGTDYKTADGTCVRDYIDVTDLVDAHVKALAHAKPRKVGIYNVGTGKGRSVKEFVDACKKATGVDIKVE
YLDRRPGDYAEVFSDPSKIKQELSWTAQYTDLQKSLQIAWKWQKSHLNGY

**SEQ ID NO: 256, DNA - Populus tremuloides**
ATGGCTGGACAAACAATTCTTGTGACTGGTGGGGCTGGGTTTATTGGCACTCACACAGTAGTACAGCTCCTA
AAGGAAGGTTTTAAGGTTTCAATCATTGACAATCTTGATAATTCTGTCACTGAAGCTGTTGATCGTGTTAAG
GAAGTGGTGGGTCCTCAGCTTTCTAAGAATCTTGAATTCAATCTGGGTGATCTTAGAAACAAGGATGATTTG
GAGAAGTTGTTTTCAAGAACTAAGTTCGATGCTGTGATCCATTTTGCTGGTCTCAAGGCAGTTGGGGAGAGT
GTTGCTAATCCACGTAGATATTTTGACAATAATCTGGTTGGCACCATCAATCTCTATGAAGTTATGGCAAAA
TACAATTGCAAGAAGATGGTTTTCTCATCATCAGCAACTGTTTATGGCCAACCTGAAAAAATTCCTTGTGTT
GAGGACTTCAACCTAATGGCAATGAATCCTTATGGACGTACCAAGAAATTGCTCGAGATATTCAAAAGGCAG
AACCAGAATGGAGTATCATTTTACTTAGGTACTTCAACCCCGTGGGAGCTCATGAGAGTGGTAAACTTGGTG
AAGATCCCAAGGGTATCCCAAACAATCTCATGCCCTACATACAACAAGTAG

**SEQ ID NO:257, protein - *Populus tremuloides***
MAGQTILVTGGAGFIGTHTVVQLLKEGFKVSIIDNLDNSVTEAVDRVKEVVGPQLSKNLEFNLGDLRNKDDL
EKLFSRTKFDAVIHFAGLKAVGESVANPRRYFDNNLVGTINLYEVMAKYNCKKMVFSSSATVYGQPEKIPCV
EDFNLMAMNPYGRTKKLLEIFKRQNQNGVSFYLGTSTPWELMRVVNLVKIPRVSQTISCPTYNK

**SEQ ID NO: 258, DNA - *Populus tremuloides***
ATGGCGAAGAGTATCTTGGTTACCGGCGGTGCCGGTTACATAGGGAGCCATACGGTGCTGCAGCTTTTACTT
GGCGGTTACAGCATCGTGGTTGTTGATAATCTCGACAACTCCTCTGATATTGCTCTTAAAAGAGTTAAAGAA
CTCGCCGGTGATTTCGGTAAAAACCTCGTCTTTCACCAGGTTGATCTCCGGGATAAGCCAGCACTGGAAAAA
ATTTTTGCCAGGACAAAATTTGATGCTGTCATTCACTTTGCTGGGCTGAAAGCGGTTGGTGAGAGTGTGCAG
AAACCATTGCTTTACTTTAACAACAATCTCATTGGAACAATTACCCTGCTAGAAGTTATGACTTCCCATGGA
TGCAAGCAGTTGGTGTTTTCATCTTCGGCTACTGTTTATGGTTGCCCAAAGGAGGTTCCATGTACAGAAGAG
TTTCCTTTGTCTGCTGCCAGCCCATACGGAAGAACCAAGCTCTTCATTGAAGGGATCTGCTGTGATATCCAC
CGTTCAGACTCTGAATGGAAGATCATTTTGCTTAGATACTTCAATCCAGTTGGTGCACATCCAAGTGGTCAT
ATTGGTGAGGATCCACTT

## Figure 23 (continued)

GGAATTCCAAACAATCTCATGCCCTATGTGCAGCAAGTTGCTGTTGGCAGGCGGCCTCATCTAACCGTTTAT
GGAACTGATTATTCAACTAAAGATGGCACTGGGGTACGTGATTACATTCATGTTGTTGATTTAGCGGATGGG
CACATTGCTGCATTGCGTAAGCTCTCTGATGCTAATATAGGTTGTGAAGTGTACAACTTGGGAACAGGAAAA
GGTACATCCGTTCTGGAGATGGTTGCGGCATTTGAAAAGGCATCTAGAAAGAAAATTCCCCTTGTAATGGCC
GCTCGGCGACCTGGTGATGCTGAAATTGTGTATGCAGCAACAGAGAAGGCAGAACGTGAATTGAATTGGAAG
GCAAAATATGGCATTGATGAGATGTGTAGGGATCAATGGAACTGGGCCGGCAAGAACCCTTATGGCTATGGA
TCTTCTGACAGCACTAACTAA

**SEQ ID NO: 259, protein - *Populus tremuloides***
MAKSILVTGGAGYIGSHTVLQLLLGGYSIVVVDNLDNSSDIALKRVKELAGDFGKNLVFHQVDLRDKPALEK
IFARTKFDAVIHFAGLKAVGESVQKPLLYFNNNLIGTITLLEVMTSHGCKQLVFSSSATVYGCPKEVPCTEE
FPLSAASPYGRTKLFIEGICCDIHRSDSEWKIILLRYFNPVGAHPSGHIGEDPLGIPNNLMPYVQQVAVGRR
PHLTVYGTDYSTKDGTGVRDYIHVVDLADGHIAALRKLSDANIGCEVYNLGTGKGTSVLEMVAAFEKASRKK
IPLVMAARRPGDAEIVYAATEKAERELNWKAKYGIDEMCRDQWNWAGKNPYGYGSSDSTN

**SEQ ID NO: 260, DNA - *Populus tremuloides***
ATGGCCTATAATATTCTGGTTACAGGCGGTGCTGGTTACATAGGAAGCCACACGGTGCTGCAACTTTTATTA
GGCGGCTACAACACTGTGGTTGTTGATAACCTCGACAACGCCTCTGATATTGCTCTTAAAAGAGTTAAAGAA
CTCGCCGGTGATTTCGGCAAAAACCTCGTCTTTCACCAGGTTGATCTCCGGGACAAGCCGGCTCTGGAAAAT
GTTTTCGCCGAGACAAAGTTTGATGCTGTCATTCACTTTGCTGGGCTGAAAGCAGTTGGTGAGAGTATGCAG
AAACCATTGCTTTATTTCAACAATAATCTCATTGGAACAATTACTCTGCTAGAAGTTATGGCTGCTCATGGA
TGCAAGCAGGTAATTTTCTCTAGGTTGCTTTGCATTGGTGTTTTCATCTTCAGCTACTGTTTATGGTTGGCC
GAAGGAGGTTCCATGTACAGAAGAGTTCCCTTTGTCTGCTGCAAACCCATATGGAAGAACCAAGAGATCTGC
CGCGATATCTACAGTTCAGATTCTGAATGGAAGATCATATTACTCAGATACTTTAATCCAGTTGGTGCACAT
CCAAGTGGCTATATTGGTGAGGATCCTCGTGGAATTCCAAACAATCTCATGCCCTATGTGCAGCAAGTTGCT
GTTGGCAGGAGGCCTCATCTAACAGTTTTTGGAACTGATTATCCAACAAAAGACGGTACCGGGGTACGTGAT
TACATTCATGTTGTCGATTTAGCAGATGGGCACATTGCTGCATTGCGTAAGCTCTCTGAAGCTAATATAGGT
TGTGAAGTGTACAACTTGGGAACAGGGAAAGGTACATCAGTTCTGGAGATGGTCGCAGCATTTGAAAAGGCA
TCTGGAAAGAGTCCCATCTTCGTGTGCCAGAAAATTCCTCTTGTAATGGCTGATCGGCGACCTGGTGATGCT
GAAACTGTGTATGCAGCAACAGAGAAGGCAGAACGTGAATTGAGTTGGAAGTAA

**SEQ ID NO: 261, protein - *Populus tremuloides***
MAYNILVTGGAGYIGSHTVLQLLLGGYNTVVVDNLDNASDIALKRVKELAGDFGKNLVFHQVDLRDKPALEN
VFAETKFDAVIHFAGLKAVGESMQKPLLYFNNNLIGTITLLEVMAAHGCKQVIFSRLLCIGVFIFSYCLWLA
EGGSMYRRVPFVCCKPIWKNQEICRDIYSSDSEWKIILLRYFNPVGAHPSGYIGEDPRGIPNNLMPYVQQVA
VGRRPHLTVFGTDYPTKDGTGVRDYIHVVDLADGHIAALRKLSEANIGCEVYNLGTGKGTSVLEMVAAFEKA
SGKSPIFVCQKIPLVMADRRPGDAETVYAATEKAERELSWK

**SEQ ID NO: 262, DNA - *Populus tremuloides***
ATGCTAAGTTTTGGCAGGACCAGAACTCAGCCAAGGTCCAATAGATCTATGTCCCTTGGAGGCATGGATTTT
TCAGATCCAAAAAGAAAAAATAATGTTGTAGGAAAGATTCTTTTGGCCGCTTCCCTAACAGCTGTATGTATA
ATTATGCTGAAACAATCTCCAACCTTTAATTCTCCAAGCCCGTTCTCTTTGCGTGAAGATGGGGTGATCCAT
GTCCTTGTGACAGGTGGTGCTGGCTACATTGGTTCCCATGCAGCATTGCGACTTTTGAAGGATGGTTACCGA
GTAACCATAGTGGACAACCTTTCTCGAGGAAACTTAGGTGCAGTCAAGGTTTTACAAGAGTTATTTCCTGAG
CCTGGGAGGCTTCAGTTTATATATGCTGACTTGGGAGAGCCTAAAACTGTTAACAGCATCTTTTCACAAAAT
GCATTTGATGCTGTGATGCATTTTGCAGCA

## Figure 23 (continued)

GTTGCATATGTTGGGGAAAGCACCGTGTACCCCCTTAAGTACTATCACAACATTACATCAAATACCTTGGTA
GTGTTGGAGTCAATGGCTGCAAATGATGTAAAGACTTTGATATATTCAAGCACATGTGCAACATATGGGGAG
CCTGAAAAGATGCCTATTACTGAAGACACTCCACAGGTACCCATTAATCCATATGGAAAAGCTAAGAAGATG
GCAGAAGATATCATCCTTGACTTCTCTAAAAATTCAGACATGGCAATTATGATATTGAGATACTTCAATGTG
ATTGGATCAGATCCAGATGGAAGGTTAGGTGAGGCTCCTAGACCTGAACTGCGTGAGCACGGACGAATTTCT
GGTGCTTGTTTTGATGCTGCTCGTGGTATTGTTGCTGGACTAAAGGTTAAAGGAACGGACTATAAGACACAC
GATGGAACTTGTATAAGAGATTATATCGATGTTACTGATCTTGTTGATGCTCATGTTAAAGCACTTGAAAAG
GCAATGCCTGGGAAAGTTGGAATCTACAATGTTGGCACTGGAATGGGTAGATCAGTCAACGAGTTTGTACAT
GCATGTAAAAAGGCAACCGGTGTAGATATCAAAGTAGACTATTTACCTCGCCGGCCTGGTGACTATGCTGAA
GTGTTTAGTGACCCATCAAAAATTAACCGTGAGCTGAACTGGACAGCACAATACACTGATCTCCAAAAGAGT
TTACAGGTTGCATGGAGATGGCAAAAATCACATCAAAATGGGTATGGATCCCCTTTGGTGATGGCTTCTTGA

**SEQ ID NO: 263, protein - *Populus tremuloides***
MLSFGRTRTQPRSNRSMSLGGMDFSDPKRKNNVVGKILLAASLTAVCIIMLKQSPTFNSPSPFSLREDGVIH
VLVTGGAGYIGSHAALRLLKDGYRVTIVDNLSRGNLGAVKVLQELFPEPGRLQFIYADLGEPKTVNSIFSQN
AFDAVMHFAAVAYVGESTVYPLKYYHNITSNTLVVLESMAANDVKTLIYSSTCATYGEPEKMPITEDTPQVP
INPYGKAKKMAEDIILDFSKNSDMAIMILRYFNVIGSDPDGRLGEAPRPELREHGRISGACFDAARGIVAGL
KVKGTDYKTHDGTCIRDYIDVTDLVDAHVKALEKAMPGKVGIYNVGTGMGRSVNEFVHACKKATGVDIKVDY
LPRRPGDYAEVFSDPSKINRELNWTAQYTDLQKSLQVAWRWQKSHQNGYGSPLVMAS

**SEQ ID NO: 264, DNA - *Populus tremuloides***
ATGATTCTGGTCACAGGCGGCGCCGGCTTCATCGGCTCGCATAGCTGCGTGGAACTCGCCGCTGCGGGCGAG
CCCTACCTGATCTACGACAACTTCAGCAACAGCAGCCCCGATGTGCTCGAACGCATGGAACGCATCACGGGC
CGGCGCCCGCTGTGCGTCGAGGGAGACGTGCGCGACCGCGCCGCGCTGGACCGGCTGTTTGCCGAGCACTCC
ATCCGCGAGGTGATCCACTTCGCCGCGCTCAAGGCCGTGGGCGAGTCCGTGGCCCAGCCGCTGCGCTACTAC
GAACACAACGTGGGCGGCACGGTGGCCCTGCTGCAGGCCATGCGGACGGCGGGCGTGCGCAGCCTGGTGTTC
TCCTCCTCGGCCACGGTCTACGGCGATCCGGCCAGCCTGCCGATCCGCGAAGACTTTCCGCTGTCCGCCACC
AACCCCTACGGCCGCAGCAAGCTGTGGATCGAGGAAATGCTGGCCGACCTGGACCGGGCCGAGGCCGGCCAG
TGGAGCCTGGCACGGCTGCGCTACTTCAACCCCGTGGGCGCCCATGAAAGCGGCCTGATCGGCGAGGACCCG
CGCGACATCCCCAACAACCTCATGCCCTATGTGTCGCAGGTCGCCATCGGCCAGCGCCAGCAGCTCAGTGTC
TATGGCGATGACTATGCGACGCCGGACGGCACGGGCGTGCGCGACTACATCCACGTCACCGACCTGGCGCGC
GGCCACCTGGCGGCGCTGCGCTATCTGCGCGAGCAGCAGGGCCTGCTGACCGTGAACCTGGGCACGGGGCGC
CCGGTCTCGGTGCTGGAGATGGTCAAGGCCTTCGAGCGCGCCAGCGGCCGCCCCGTGCCCTACCAGATCGTG
GCGCGCCGACCCGGCGACGTGGCCCAGTGCTGGGCCGACCCCGCCGAGGCCGAGCGGCTGCTGGGCTGGAAG
GCCATGCTGGACCTGGACCGCATCGCCTGCGGGCGCCGAGGGCGTGGTATCTTTTCGCCCTTGTTTCACGCC
CTAGCCAAGAGTCTGCCCATGCTTGCCAAACGCATCATCCCCTGTCTTGACGTCACGGGAGGGCGCGTCGTC
AAAGGCGTGAACTTCGTGGAACTGCGCGATGCGGGCGACCCGGTGGAGATCGCCGCGCGCTACAACGCCCAG
GGCGCGGACGAGCTGACCTTCCTGGACATCACGGCCACCAGCGACGGGCGCGACCTGATCCTGCCCATCATC
GAATCGGTGGCCAGCCAGGTCTTCATTCCGCTGACCGTGGGTGGTGGCGTGCGCACCGTCGAGGACGTGCGC
CGCCTGCTCAACGCGGGCGCCGACAAGACCAGCTTCAACTCGGCCGCCATCGCCAACCCCGACGTGATCAAT
GCGGCCTCGGACAAGTACGGCGCGCAATGCATCGTCGTGGCCATCGATGCCAAGCGGCGCACGGCCGAGGAC
GAGCAGCGCATCGGCGCCGATGGCCGCGCTGCCGGCCCCGGCTGGACGTGTACAGCCACGGCGGACGCAAA
AACACCGGCCTGGACGCCGTGCACTCACGCGCGCCGTCAGCGACGCCGTGCCCGTGCCCGTGA

## Figure 23 (continued)

**SEQ ID NO: 265, protein - *Populus tremuloides***
MILVTGGAGFIGSHSCVELAAAGEPYLIYDNFSNSSPDVLERMERITGRRPLCVEGDVRDRAALDRLFAEHS
IREVIHFAALKAVGESVAQPLRYYEHNVGGTVALLQAMRTAGVRSLVFSSSATVYGDPASLPIREDFPLSAT
NPYGRSKLWIEEMLADLDRAEAGQWSLARLRYFNPVGAHESGLIGEDPRDIPNNLMPYVSQVAIGQRQQLSV
YGDDYATPDGTGVRDYIHVTDLARGHLAALRYLREQQGLLTVNLGTGRPVSVLEMVKAFERASGRPVPYQIV
ARRPGDVAQCWADPAEAERLLGWKAMLDLDRIACGRRGRGIFSPLFHALAKSLPMLAKRIIPCLDVTGGRVV
KGVNFVELRDAGDPVEIAARYNAQGADELTFLDITATSDGRDLILPIIESVASQVFIPLTVGGGVRTVEDVR
RLLNAGADKTSFNSAAIANPDVINAASDKYGAQCIVVAIDAKRRTAEDEQRIGADGRAAGPGWDVYSHGGRK
NTGLDAVHSRAPSATPCPCP

**SEQ ID NO: 266, DNA - *Ostreococcus tauri***
ATGACGTGGAACGTCCTCGTCACCGGTGGCGCCGGATACATCGGCTCGCACACGTGCGTGCGGCTCCTGCAA
GCGGGCGCGCGCGTCACCGTCGTGGATAACTTTGACAACTCGTGCGCGGAATCTTTAAAGCGCGTGCGAAAC
ATCGTGGGCGAGGACGCGGGGGCGCGTTTGACGCACCACGAGGTTGATTGCTGCGATAAAGTCGCGCTCGAT
GGCGTCTTCGCGTCGGCGGGGGTGACGTTCGATGCGGTGATACACTTCGCCGGGTTGAAGGCGGTGGGCGAG
AGCGTGGCCGAGCCGATGAAGTATTACGAGAATAACATCGTGAGCACGCTGGTGCTGTGCGAGACGATGGCG
AAACACGGGTGTAAGACGATTATTTTTAGCTCGAGCGCGACCGTGTACGGGGAGCCGGCGTCGGTGCCGTGC
ACGGAAGATTTCCCGACGGCGGCGTTGAACCCGTACGGACGGACCAAGTTGTTCATCGAACACATTTTGAGC
GATCTCTACGTGAGTGATAAGGAGTGGAAGGTGGCGCTGTTGCGATACTTTAACCCGGTCGGTGCGCACGAG
AGTGGGACGCTGGGGGAGGATCCCAAGGGAATCCCGAATAACTTGATGCCATTCGTGCAGCAAGTCGCCGTC
GGTCGACGACCCGAGCTCAACGTGTTTGGGAACGACTATCCAACCAAGGATGGAACCGGTCGACGTGATTAC
ATTCATGTCGTCGATTTGGCCGACGGACACGTCGCGGCGGTAAAGAAACTCACGAGTGATCCCGACGCCGGT
TTGCTCACCGTCAACCTCGGGACGGGGAAGAGCACGAGCGTCTTAGAGCTCGTCGCGGCGTTCGAGAAGGCG
TCCGGTAAGAAAATTCCGTGCAAGATTGTCGATCGTCGCGCAGGTGACGCCGCAGAGGTGTACGGGGCGACA
GATAAGGCGTTCAAGGTTCTCGGTTGGCGTGCACTTCGCACCATCGAAGACTGCTGCATCGATCAGTGGAAG
TGGGCGAGCTCGAACCCGTACGGGTACGCAGGTAAGCCCGACGACGCGTAA

**SEQ ID NO: 267, protein - *Ostreococcus tauri***
MTWNVLVTGGAGYIGSHTCVRLLQAGARVTVVDNFDNSCAESLKRVRNIVGEDAGARLTHHEVDCCDKVALD
GVFASAGVTFDAVIHFAGLKAVGESVAEPMKYYENNIVSTLVLCETMAKHGCKTIIFSSSATVYGEPASVPC
TEDFPTAALNPYGRTKLFIEHILSDLYVSDKEWKVALLRYFNPVGAHESGTLGEDPKGIPNNLMPFVQQVAV
GRRPELNVFGNDYPTKDGTGRRDYIHVVDLADGHVAAVKKLTSDPDAGLLTVNLGTGKSTSVLELVAAFEKA
SGKKIPCKIVDRRAGDAAEVYGATDKAFKVLGWRALRTIEDCCIDQWKWASSNPYGYAGKPDDA

**SEQ ID NO: 268, DNA - *Ostreococcus tauri***
ATGACAGCTCAGTTACAAAGTGAAAGTACTTCTAAAATTGTTTTGGTTACAGGTGGTGCTGGATACATTGGT
TCACACACTGTGGTAGAGCTAATTGAGAATGGATATGACTGTGTTGTTGCTGATAACCTGTCGAATTCAACT
TATGATTCTGTAGCCAGGTTAGAGGTCTTGACCAAGCATCACATTCCCTTCTATGAGGTTGATTTGTGTGAC
CGAAAAGGTCTGGAAAAGGTTTTCAAAGAATATAAAATTGATTCGGTAATTCACTTTGCTGGTTTAAAGGCT
GTAGGTGAATCTACACAAATCCCGCTGAGATACTATCACAATAACATTTTGGGAACTGTCGTTTTATTAGAG
TTAATGCAACAATACAACGTTTCCAAATTTGTTTTTCATCTTCTGCTACTGTCTATGGTGATGCTACGAGA
TTCCCAAATATGATTCCTATCCCAGAAGAATGTCCCTTAGGGCCTACTAATCCGTATGGTCATACGAAATAC
GCCATTGAGAATATCTTGAATGATCTTTACAATAGCGACAAAAAAAGTTGGAAGTTTGCTATCTTGCGTTAT
TTTAACCCAATTGGCGCACATCCCTCTGGATTAATCGGAGAAGATCCGCTAGGTATACCAAACAATTTGTTG
CCATATATGGCT

## Figure 23 (continued)

CAAGTAGCTGTTGGTAGGCGCGAGAAGCTTTACATCTTCGGAGACGATTATGATTCCAGAGATGGTACCCCG
ATCAGGGATTATATCCACGTAGTTGATCTAGCAAAAGGTCATATTGCAGCCCTGCAATACCTAGAGGCCTAC
AATGAAAATGAAGGTTTGTGTCGTGAGTGGAACTTGGGTTCCGGTAAAGGTTCTACAGTTTTTGAAGTTTAT
CATGCATTCTGCAAAGCTTCTGGTATTGATCTTCCATACAAAGTTACGGGCAGAAGAGCAGGTGATGTTTTG
AACTTGACGGCTAAACCAGATAGGGCCAAACGCGAACTGAAATGGCAGACCGAGTTGCAGGTTGAAGACTCC
TGCAAGGATTTATGGAAATGGACTACTGAGAATCCTTTTGGTTACCAGTTAAGGGGTGTCGAGGCCAGATTT
TCCGCTGAAGATATGCGTTATGACGCAAGATTTGTGACTATTGGTGCCGGCACCAGATTTCAAGCCACGTTT
GCCAATTTGGGCGCCAGCATTGTTGACCTGAAAGTGAACGGACAATCAGTTGTTCTTGGCTATGAAAATGAG
GAAGGGTATTTGAATCCTGATAGTGCTTATATAGGCGCCACGATCGGCAGGTATGCTAATCGTATTTCGAAG
GGTAAGTTTAGTTTATGCAACAAAGACTATCAGTTAACCGTTAATAACGGCGTTAATGCGAATCATAGTAGT
ATCGGTTCTTTCCACAGAAAAAGATTTTTGGGACCCATCATTCAAAATCCTTCAAAGGATGTTTTTACCGCC
GAGTACATGCTGATAGATAATGAGAAGGACACCGAATTTCCAGGTGATCTATTGGTAACCATACAGTATACT
GTGAACGTTGCCCAAAAAAGTTTGGAAATGGTATATAAAGGTAAATTGACTGCTGGTGAAGCGACGCCAATA
AATTTAACAAATCATAGTTATTTCAATCTGAACAAGCCATATGGAGACACTATTGAGGGTACGGAGATTATG
GTGCGTTCAAAAAAATCTGTTGATGTCGACAAAAACATGATTCCTACGGGTAATATCGTCGATAGAGAAATT
GCTACCTTTAACTCTACAAAGCCAACGGTCTTAGGCCCCAAAAATCCCCAGTTTGATTGTTGTTTTGTGGTG
GATGAAAATGCTAAGCCAAGTCAAATCAATACTCTAAACAATGAATTGACGCTTATTGTCAAGGCTTTTCAT
CCCGATTCCAATATTACATTAGAAGTTTTAAGTACAGAGCCAACTTATCAATTTTATACCGGTGATTTCTTG
TCTGCTGGTTACGAAGCAAGACAAGGTTTTGCAATTGAGCCTGGTAGATACATTGATGCTATCAATCAAGAG
AACTGGAAAGATTGTGTAACCTTGAAAAACGGTGAAACTTACGGGTCCAAGATTGTCTACAGATTTTCCTGA

**SEQ ID NO: 269, protein - *Ostreococcus tauri***
MTAQLQSESTSKIVLVTGGAGYIGSHTVVELIENGYDCVVADNLSNSTYDSVARLEVLTKHHIPFYEVDLCD
RKGLEKVFKEYKIDSVIHFAGLKAVGESTQIPLRYYHNNILGTVVLLELMQQYNVSKFVFSSSATVYGDATR
FPNMIPIPEECPLGPTNPYGHTKYAIENILNDLYNSDKKSWKFAILRYFNPIGAHPSGLIGEDPLGIPNNLL
PYMAQVAVGRREKLYIFGDDYDSRDGTPIRDYIHVVDLAKGHIAALQYLEAYNENEGLCREWNLGSGKGSTV
FEVYHAFCKASGIDLPYKVTGRRAGDVLNLTAKPDRAKRELKWQTELQVEDSCKDLWKWTTENPFGYQLRGV
EARFSAEDMRYDARFVTIGAGTRFQATFANLGASIVDLKVNGQSVVLGYENEEGYLNPDSAYIGATIGRYAN
RISKGKFSLCNKDYQLTVNNGVNANHSSIGSFHRKRFLGPIIQNPSKDVFTAEYMLIDNEKDTEFPGDLLVT
IQYTVNVAQKSLEMVYKGKLTAGEATPINLTNHSYFNLNKPYGDTIEGTEIMVRSKKSVDVDKNMIPTGNIV
DREIATFNSTKPTVLGPKNPQFDCCFVVDENAKPSQINTLNNELTLIVKAFHPDSNITLEVLSTEPTYQFYT
GDFLSAGYEARQGFAIEPGRYIDAINQENWKDCVTLKNGETYGSKIVYRFS

**SEQ ID NO: 270, DNA - *Escherichia coli***
ATGGCTATTCTCGTTACAGGTGGTGCTGGTTATATTGCATCACATACTATACTGAGTTTGATAGAGCAGGGT
AATGAAGTAATTATTATTGATAATTTATCAAATTCATATTATGATTCGTTATTGAAAATAAAAGAGATTACT
GGATGTGATTGTCTTTTTTATAAAGGAGATATCCTTGACAAAAAACTCTTGCTAAAAATATTTGATGAAAAT
AATATCACTGCTGTGATGCATTTTGCTGGTGCAAAGTCTGTAAGTGAATCTGTTATCAGACCTTTGCAATAC
TATCGAAATAATGTCTCTGGCACTTTTTCTTTAGTTGAGGCAATGGAGGAGTCTGGTGTAGAAAATTTAATA
TTTAGCTCTTCAGCAACTGTCTATGGTGAGCCAAAGATTATTCCTGTGAATGAAAGTTGTGCTATAGGTGGT
ACTACAAATCCGTATGGAACGTCCAAGTTATTTGTAGAGAACTTTTTGCGGGATTATTCTAAGGCATCCCCT
AATTTTAAAACTATAGTATTAAGATATTTTAACCCTATCGGTGCTCACTCATCTGGAAAAATAGGTGAAGAT
CCTAACGGAATTCCGAATAACTTGATGCCCTTTATATGCCAGGTTGCTATCGGTAAACAAAAAACGCTCAAG
ATCTACGGAAATGATTATCCTACTAAAGATGGTACTGGGATTCGCGATTATATTCATGTTATGGATCTTGCT
GAAGGT

## Figure 23 (continued)

CATGTTGCTGCATTAAATAATATTAATCAAGGAGCTAATTATAGAGTTTACAACCTTGGAACAGGAATTGGA
TACTCTGTGTTGGAATTATTAGAAGCATTTCAGAAAGTTACAACTCGGAGAGTTCCCTATGTTTTTACTAAA
AGGCGTTCAGGTGATATTGCTGAATGTTGGTCTGATCCTACCAAAGCGTATGAAGAATTAGGATGGAAAGCA
AGGCGAGGGTTAGAAGATATGATTCGTGATGCATGGAATTGGCAGCAAAAAAATCCAAATGGATTTAAGAAA
GTTTAAgtaa

**SEQ ID NO: 271, protein - *Escherichia coli***
MAILVTGGAGYIASHTILSLIEQGNEVIIIDNLSNSYYDSLLKIKEITGCDCLFYKGDILDKKLLLKIFDEN
NITAVMHFAGAKSVSESVIRPLQYYRNNVSGTFSLVEAMEESGVENLIFSSSATVYGEPKIIPVNESCAIGG
TTNPYGTSKLFVENFLRDYSKASPNFKTIVLRYFNPIGAHSSGKIGEDPNGIPNNLMPFICQVAIGKQKTLK
IYGNDYPTKDGTGIRDYIHVMDLAEGHVAALNNINQGANYRVYNLGTGIGYSVLELLEAFQKVTTRRVPYVF
TKRRSGDIAECWSDPTKAYEELGWKARRGLEDMIRDAWNWQQKNPNGFKKV

**SEQ ID NO: 272, DNA - primer 1**
GGGGACAAGTTTGTACAAAAAAGCAGGCTTAAACAATGGTTTCGGCCTTGTTG

**SEQ ID NO: 273, DNA - primer 2**
GGGGACCACTTTGTACAAGAAAGCTGGGTGCTGCTGCTACTGGAGGATT

**SEQ ID NO: 274, DNA - *Oryza sativa***
AATCCGAAAAGTTTCTGCACCGTTTTCACCCCCTAACTAACAATATAGGGAACGTGTGCTAAATATAAAATG
AGACCTTATATATGTAGCGCTGATAACTAGAACTATGCAAGAAAAACTCATCCACCTACTTTAGTGGCAATC
GGGCTAAATAAAAAAGAGTCGCTACACTAGTTTCGTTTTCCTTAGTAATTAAGTGGGAAAATGAAATCATTA
TTGCTTAGAATATACGTTCACATCTCTGTCATGAAGTTAAATTATTCGAGGTAGCCATAATTGTCATCAAAC
TCTTCTTGAATAAAAAAATCTTTCTAGCTGAACTCAATGGGTAAAGAGAGAGATTTTTTTAAAAAAATAGA
ATGAAGATATTCTGAACGTATTGGCAAAGATTTAAACATATAATTATATAATTTTATAGTTTGTGCATTCGT
CATATCGCACATCATTAAGGACATGTCTTACTCCATCCCAATTTTTATTTAGTAATTAAAGACAATTGACTT
ATTTTTATTATTTATCTTTTTTCGATTAGATGCAAGGTACTTACGCACACACTTTGTGCTCATGTGCATGTG
TGAGTGCACCTCCTCAATACACGTTCAACTAGCAACACATCTCTAATATCACTCGCCTATTTAATACATTTA
GGTAGCAATATCTGAATTCAAGCACTCCACCATCACCAGACCACTTTTAATAATATCTAAAATACAAAAAAT
AATTTTACAGAATAGCATGAAAAGTATGAAACGAACTATTTAGGTTTTTCACATACAAAAAAAAAAAGAATT
TTGCTCGTGCGCGAGCGCCAATCTCCCATATTGGGCACACAGGCAACAACAGAGTGGCTGCCCACAGAACAA
CCCACAAAAAACGATGATCTAACGGAGGACAGCAAGTCCGCAACAACCTTTTAACAGCAGGCTTTGCGGCCA
GGAGAGAGGAGGAGAGGCAAAGAAAACCAAGCATCCTCCTTCTCCCATCTATAAATTCCTCCCCCCTTTTCC
CCTCTCTATATAGGAGGCATCCAAGCCAAGAAGAGGGAGAGCACCAAGGACACGCGACTAGCAGAAGCCGAG
CGACCGCCTTCTCGATCCATATCTTCCGGTCGAGTTCTTGGTCGATCTCTTCCCTCCTCCACCTCCTCCTCA
CAGGGTATGTGCCTCCCTTCGGTTGTTCTTGGATTTATTGTTCTAGGTTGTGTAGTACGGGCGTTGATGTTA
GGAAAGGGGATCTGTATCTGTGATGATTCCTGTTCTTGGATTTGGGATAGAGGGGTTCTTGATGTTGCATGT
TATCGGTTCGGTTTGATTAGTAGTATGGTTTTCAATCGTCTGGAGAGCTCTATGGAAATGAAATGGTTTAGG
GATCGGAATCTTGCGATTTTGTGAGTACCTTTTGTTTGAGGTAAAATCAGAGCACCGGTGATTTTGCTTGGT
GTAATAAAGTACGGTTGTTTGGTCCTCGATTCTGGTAGTGATGCTTCTCGATTTGACGAAGCTATCCTTTGT
TTATTCCCTATTGAACAAAAATAATCCAACTTTGAAGACGGTCCCGTTGATGAGATTGAATGATTGATTCTT
AAGCCTGTCCAAAATTTCGCAGCTGGCTTGTTTAGATACAGTAGTCCCCATCACGAAATTCATGGAAACAGT
TATAATCCTCAGGAACAGGGGATTCCCTGTTCTTCCGATTTGCTTTAGTCCCAGAATTTTTTTCCCAAATA
TCTTAAAAAGTCACTTTCTGGTTCAGTTCAATGAATTGATTGCTACAAATAATG

**Figure 23 (continued)**

CTTTTATAGCGTTATCCTAGCTGTAGTTCAGTTAATAGGTAATACCCCTATAGTTTAGTCAGGAGAAGAACT
TATCCGATTTCTGATCTCCATTTTTAATTATATGAAATGAACTGTAGCATAAGCAGTATTCATTTGGATTAT
TTTTTTTATTAGCTCTCACCCCTTCATTATTCTGAGCTGAAAGTCTGGCATGAACTGTCCTCAATTTTGTTT
TCAAATTCACATCGATTATCTATGCATTATCCTCTTGTATCTACCTGTAGAAGTTTCTTTTTGGTTATTCCT
TGACTGCTTGATTACAGAAAGAAATTTATGAAGCTGTAATCGGGATAGTTATACTGCTTGTTCTTATGATTC
ATTTCCTTTGTGCAGTTCTTGGTGTAGCTTGCCACTTTCACCAGCAAAGTTC

**SEQ ID NO: 275, protein - epimerase domain of SEQ ID NO: 2**
ILVTGGAGYIGSHTVLQLLQLGFRVVVLDNLDNASELAILRVRELAGHNANNLDFRKVDLRDKQALDQIFSS
QRFEAVIHFAGLKAVGESVQKPLLYYDNNLIGTITLLQVMAAHGCTKLVFSSSATVYGWPKEVPCTEESPLC
AMNPYGRTKLVIEDMCRDLHASDPNWKIILLRYFNPVGAHPSGYIGEDPCGIPNNLMPFVQQVAVGRRPALT
VYGTDYNTKDGTGVRDYIHVVDLADGHIAALRKLYEDSDRIGCEVYNLG

**SEQ ID NO: 276, protein - Motif 1 - NAD+ binding domain**
GXXGXXG

**SEQ ID NO: 277, protein - Motif 2 - Active site**
YGR(T/S)

**SEQ ID NO: 278, protein - Motif 3 - conserved region**
LVTGGAGYIGSH

**SEQ ID NO: 279, protein - Motif 4 - conserved region**
VXH(F/L)A

**SEQ ID NO: 280, protein - Motif 5 - conserved region**
DGTGVRDYIHV

**SEQ ID NO 281: Brassica napus UGE I**
ATGGTGAAGAACGTTCTCGTCACAGGCGGCGCTGGCTACATCGGAAGCCACACGGTGCTTCAACTGCTCAAC
GGTGGATACTCCGCCGTAGTCGTCGACAATCACGACAATTCATCCGCTGTTTCTCTCCAACGCGTCAAAAAA
CTCGCCGGCGATAACGGAAACCGCCTCTCCTTCCACCAGGTGGATCTCAGAGACAGGCCTGCGCTTGAGAAG
ATTTTCTCTGAAACTAAGTTTGATGCAGTGATACACTTTGCTGGACTTAAAGCAGTAGGTGAGAGTGTGGAG
AAGCCTTTGCTCTATTATAATAATAACCTTTTTGGCACTGTTATCCTTTTGGAGGTTATGTCTCAATTCGGC
TGCAAAAATCTTGTGTTTTCGTCATCAGCTACTGTCTATGGCTGGCCAAAAGAGGTTCCTTGCACCGAGGAG
TCCCCAATTTCTGCAACCAACCCTTATGGCCGTACCAAGCTTTTTATCGAGGAAATTTGCCGGGATGTACAT
CGCTCCGACCCTGAATGGAAGATCATATTGCTTAGATACTTCAACCCTGTTGGTGCACATCCTAGTGGTTAC
ATTGGTGAAGATCCTCTTGGGGTTCCAAACAACCTCATGCCTTATGTCCAACAAGTTGCAGTTGGCCGGAGA
CCTCACCTCACCGTCTTTGGAACTGACTATAACACAAAGGATGGCACAGGGGTGAGAGATTACATTCATGTG
ATTGACTTAGCAGATGGACATATAGCCGCTCTGCGCAAGCTGGATGATCTCAAAATCAGTTGTGAGGTATAC
AATCTTGGAACGGGTAATGGAACATCAGTTCTAGAAATGGTTGCTGCCTTTGAGAAGGCATCCGGAAAGAAA
ATCCCTTTGGTGTTGGCTGGACGACGTCCTGGAGACGCTGAGATTGTTTACGCCTCAACCGAGAAAGCAGAA
CGCGAGCTAAACTGGAAGGCTAAGTATGGGATTGAAGACATGTGTAGGGATCTATGGAACTGGGCCAGCAAT
AATCCTTACGGCTACGCCTCCTCCAATGGCTCCTCTTAA

**SEQ ID NO 282: Brassica napus UGE I**
MVKNVLVTGGAGYIGSHTVLQLLNGGYSAVVVDNHDNSSAVSLQRVKKLAGDNGNRLSFHQVDLRDRPAL
EKIFSETKFDAVIHFAGLKAVGESVEKPLLYYNNNLFGTVILLEVMSQFGCKNLVFSSSATVYGWPKEVP
CTEESPISATNPYGRTKLFIEEICRDVHRSDPEWKIILLRYFNPVGAHPSGYIGEDPLGVPNNLMPYVQQ
VAVGRRPHLTVFGTDYNTKDGTGVRDYIHVIDLADGHIAALRKLDDLKISCEVYNLGTGNGTSVLEMVAA
FEKASGKKIPLVLAGRRPGDAEIVYASTEKAERELNWKAKYGIEDMCRDLWNWASNNPYGYASSNGSS

## Figure 23 (continued)

**SEQ ID NO 283: Brassica napus UGE II**
ATGGTGAAGAACGTTCTGGTCACAGGCGGCGCTGGCTACATCGGTAGCCACACGGTGCTTCAACTTCTCAAC
GGAGGCTACTCCGTCGTAGTCGTCGACAATCACGACAATTCATCCGCTGTTTCTCTCCAACGCGTCAAAAAA
CTCGCCGGCGATAACGGAAACCGCCTCTCCTTCCACCAGGTGGATCTCAGAGACAGGCCTGCGCTTGAGAAG
ATTTTCTCAGAAACTAAGTTTGATGCAGTGATACACTTTGCTGGACTTAAAGCAGTAGGTGAGAGTGTGGAG
AAGCCCCTGCTTTATTATAATAATAACTTATTTGGCACTGTTATCCTTTTGGAGGTTATGTCTCAGTTCGGC
TGCAAAAATCTTGTGTTTTCGTCATCAGCTACTGTCTATGGCTGGCCAAAAGAGGTTCCTTGTACCGAGGAG
TCCCCAATTTCTGCAACCAACCCTTATGGCCGTACCAAGCTTTTTATCGAGGAAATTTGCCGGGATGTACAT
CGCTCCGACCCTGAATGGAAGATCATATTGCTCAGATACTTCAACCCTGTTGGTGCACATCCTAGTGGTTAC
ATTGGTGAAGATCCTCTTGGGGTTCCAAACAACCTCATGCCTTATGTCCAACAAGTTGCAGTTGGCCGGAGA
CCTCACCTCACCGTCTTTGGAACTGACTATAACACAAAGGATGGCACAGGGGTGAGAGATTACATTCATGTG
ATTGACTTAGCAGATGGACATATAGCCGCTCTGCGCAAGCTGGATGATCTCAAAATCAGTTGTGAGGTATAC
AATCTTGGAACGGGTAATGGAACATCAGTTCTAGAAATGGTTGCTGCCTTTGAGAAGGCATCCGGAAAGAAA
ATCCCTTTGGTGTTGGCTGGACGACGTCCTGGAGACGCTGAGATTGTTTACGCCTCAACCGAGAAAGCAGAA
CGCGAGCTAAACTGGAAGGCTAAGTATGGGATTGAAGACATGTGTAGGGATCTATGGAACTGGGCCAGCAAT
AATCCTTACGGCTACGCCTCCTCCAATGGCTCCTCTTAA

**SEQ ID NO 284: Brassica napus UGE II**
MVKNVLVTGGAGYIGSHTVLQLLNGGYSVVVVDNHDNSSAVSLQRVKKLAGDNGNRLSFHQVDLRDRPAL
EKIFSETKFDAVIHFAGLKAVGESVEKPLLYYNNNLFGTVILLEVMSQFGCKNLVFSSSATVYGWPKEVP
CTEESPISATNPYGRTKLFIEEICRDVHRSDPEWKIILLRYFNPVGAHPSGYIGEDPLGVPNNLMPYVQQ
VAVGRRPHLTVFGTDYNTKDGTGVRDYIHVIDLADGHIAALRKLDDLKISCEVYNLGTGNGTSVLEMVAA
FEKASGKKIPLVLAGRRPGDAEIVYASTEKAERELNWKAKYGIEDMCRDLWNWASNNPYGYASSNGSS

**SEQ ID NO 285: Zea mays UGE I**
ATGGTGTCCGCCGTGCTCCGGACCATCCTCGTGACGGGCGGCGCCGGGTACATCGGCAGCCACACGGTGCTG
CAGCTGCTGCAGCAGGGCTTCCGCGTCGTCGTCGTCGACAACCTCGACAACGCCTCCGAGGCCGCCCTCGCC
CGCGTCGCCGAGCTCGCCGGGCACGACGGCGCCAACCTCGTCTTCCACAAGGTTGACCTTCGCGACAGGCAC
GCGTTGGTGGACATCTTCTCGTCGCACAGGTTCGAGGCTGTCATTCACTTTGCTGGGCTCAAGGCTGTTGGG
GAGAGCGTGCACAAGCCCCTACTTTACTACGACAACAACCTGGTCGGCACCATCACCCTCCTGGAGGTGATG
GCTGCGAACGGCTGCAAGAAGCTGGTGTTCTCGTCATCTGCAACTGTCTATGGGTGGCCCAAGGAAGTACCC
TGCACCGAAGAATTCCCGCTCTGCGCCACCAATCCCTATGGGCGGACAAAGCTTGTGATTGAAGACATCTGC
CGCGACGTCCACCGCTCCGACCCCGACTGGAAGATCATACTGCTCAGGTACTTCAACCCCGTTGGCGCTCAT
CCAAGCGGGTACATCGGCGAAGACCCCTGCGGTGTCCCGAACAACCTGATGCCCTACGTGCAGCAAGTCGCT
GTTGGGAGGTTACCTCACCTCACGGTCTACGGGACGGACTACAGCACCAAGGATGGGACTGGGGTGCGTGAT
TACATCCACGTTGTCGACCTGGCTGACGGCCACATAGCAGCCCTGAGGAAGCTCTACGAAGACTCCGACAAA
ATAGGCTGTGAAGTGTACAACTTGGGGACTGGAAAGGGGACGTCCGTGTTGGAAATGGTGGCTGCATTCGAG
AAGGCTTCTGGGAAGAAAATCCCTCTGGTGTTCGCTGGGCGAAGACCCGGAGACGCAGAGATCGTCTACGCC
GCAACTGCCAAGGCAGAGAAGGAGCTCAAATGGAAGGCCAAGTACGGGATCGAGGAGATGTGCAGAGATCTG
TGGAACTGGGCGAGCAAGAACCCGTACGGGTACGCTGGGTCACGCGACAACAGCAAATGA

**SEQ ID NO 286: Zea may UGE I**
MVSAVLRTILVTGGAGYIGSHTVLQLLQQGFRVVVVDNLDNASEAALARVAELAGHDGANLVFHKVDLRD
RHALVDIFSSHRFEAVIHFAGLKAVGESVHKPLLYYDNNLVGTITLLEVMAANGCKKLVFSSSATVYGWP
KEVPCTEEFPLCATNPYGRTKLVIEDICRDVHRSDPDWKIILLRYFNPVGAHPSGYIGEDPCGVPNNLMP
YVQQVAVGRLPHLTVYGTDYSTKDGTGVRDYIHVVDLADGHIAALRKLYEDSDKIGCEVYNLGTGKGTSV
LEMVAAFEKASGKKIPLVFAGRRPGDAEIVYAATAKAEKELKWKAKYGIEEMCRDLWNWASKNPYGYAGS
RDNSK

## Figure 23 (continued)

**SEQ ID NO 287: Zea mays UGE II**
ATGGTGTCCGCCGTGCTCCGGACCATCCTCGTGACGGGCGGCGCCGGGTACATCGGCAGCCACACGGTGCTG
CAGCTGCTGCAGCAGGGCTTCCGCGTCGTCGTCGTCGACAACCTCGACAACGCCTCCGAGGCCGCCCTCGCC
CGCGTCGCCGAGCTCGCCGGGCACGACGGCGCCAACCTCGTCTTCCACAAGGTTGACCTTCGCGACAGGCAC
GCGTTGGTGGACATCTTCTCGTCGCACAGGTTCGAGGCTGTCATTCACTTTGCTGGGCTCAAGGCTGTTGGG
GAGAGCGTGCACAAGCCCCTACTTTACTACGACAACAACCTGGTCGGCACCATCACCCTCCTCGAGGTGATG
GCTGCGAACGGCTGCAAGAAGCTGGTGTTCTCGTCATCTGCAACTGTCTATGGGTGGCCCAAGGAAGTACCA
TGCACCGAAGAATTCCCGCTCTGCGCCACCAATCCCTATGGGCGGACAAAGCTTGTGATTGAAGACATCTGC
CGCGACGTCCACCGCTCCGACCCCGACTGGAAGATCATACTGCTCAGGTACTTCAACCCCGTTGGCGCTCAT
CCAAGCGGGTACATCGGCGAAGACCCCTGCGGTGTCCCGAACAACCTGATGCCCTACGTGCAGCAAGTCGCT
GTTGGGAAGTTACCTCACCTCACGGTCTACGGGACGGACTACAGCACCAAGGATGGGACTGGGGTGCGTGAT
TACATCCACGTTGTCGACCTGGCTGACGGCCACATAGCAGCCCTGAGGAAGCTCTACGAAGACTCCGACAAA
ATAGGCTGTGAAGTGTACAACTTGGGGACTGGAAAGGGGACGTCCGTGTTGGAAATGGTGGCTGCATTCGAG
AAGGCTTCTGGGAAGAAAATCCCTCTGGTGTTCGCTGGGCGAAGACCCGGAGACGCAGAGATCGTCTACGCC
GCAACTGCCAAGGCAGAGAAGGAGCTCAAATGGAAGGCCAAGTACGGGATCGAGGAGATGTGCAGAGATCTG
TGGAACTGGGCGAGCAAGAACCCGTACGGGTACGCTGGGTCACGCGACAACAGCAAATGA

**SEQ ID NO 288: Zea mays UGE II**
MVSAVLRTILVTGGAGYIGSHTVLQLLQQGFRVVVVDNLDNASEAALARVAELAGHDGANLVFHKVDLRD
RHALVDIFSSHRFEAVIHFAGLKAVGESVHKPLLYYDNNLVGTITLLEVMAANGCKKLVFSSSATVYGWP
KEVPCTEEFPLCATNPYGRTKLVIEDICRDVHRSDPDWKIILLRYFNPVGAHPSGYIGEDPCGVPNNLMP
YVQQVAVGKLPHLTVYGTDYSTKDGTGVRDYIHVVDLADGHIAALRKLYEDSDKIGCEVYNLGTGKGTSV
LEMVAAFEKASGKKIPLVFAGRRPGDAEIVYAATAKAEKELKWKAKYGIEEMCRDLWNWASKNPYGYAGS
RDNSK

**SEQ ID NO 289: Zea mays UGE III**
ATGGTGTCCGCCGTGCTCCGGACCATCCTCGTGACGGGCGGCGCCGGGTACATCGGCAGCCACACGGTGCTG
CAGCTGCTGCAGCAGGGCTTCCGCGTCGTCGTCGTCGACAACCTCGACAACGCCTCCGAGGCCGCCCTCGCC
CGCGTCGCCGAGCTCGCCGGGCACGACGGCGCCAACCTCGTCTTCCACAAGGTTGACCTTCGCGACAGGCAC
GCGTTGGTGGACATCTTCTCGTCGCACAGGTTCGAGGCTGTCATTCACTTTGCTGGGCTCAAGGCTGTTGGG
GAGAGCGTGCACAAGCCCCTACTTTACTACGACAACAACCTGGTCGGCACCATCACCCTCCTCGAGGTGATG
GCTGCGAACGGCTGCAAGAAGCTGGTGTTCTCGTCATCTGCAACTGTCTATGGGTGGCCCAAGGAAGTACCA
TGCACCGAAGAATTCCCGCTCTGCGCCACCAATCCCTATGGGCGGACAAAGCTTGTGATTGAAGACATCTGC
CGCGACGTCCACCGCTCCGACCCCGACTGGAAGATCATACTGCTCAGGTACTTCAACCCCGTTGGCGCTCAT
CCAAGCGGGTACATCGGCGAAGACCCCTGCGGTGTCCCGAACAACCTGATGCCCTACGTGCAGCAAGTCGCT
GTTGGGAGGTTACCTCACCTCACGGTCTACGGGACGGACTACAGCACCAAGGATGGGACTGGGGTGCGTGAT
TACATCCACGTTGTCGACCTGGCTGACGGCCACATAGCAGCCCTGAGGAAGCTCTACGAAGACTCCGACAAA
ATAGGCTGTGAAGTGTACAACTTGGGGACTGGAAAGGGGACGTCGGTGTTGGAAATGGTGGCTGCATTCGAG
AAGGCTTCTGGGAAGAAAATCCCTCTGGTGTTCGCTGGGCGAAGACCCGGAGACGCAGAGATCGTCTACGCC
GCAACTGCCAAGGCAGAGAAGGAGCTCAAATGGAAGGCCAAGTACGGGATCGAGGAGATGTGCAGAGATCTG
TGGAACTGGGCGAGCAAGAACCCGTACGGGTACGCTGGGTCACGCGACAACAGCAAATGA

**SEQ ID NO 290: Zea mays UGE III**
MVSAVLRTILVTGGAGYIGSHTVLQLLQQGFRVVVVDNLDNASEAALARVAELAGHDGANLVFHKVDLRD
RHALVDIFSSHRFEAVIHFAGLKAVGESVHKPLLYYDNNLVGTITLLEVMAANGCKKLVFSSSATVYGWP
KEVPCTEEFPLCATNPYGRTKLVIEDICRDVHRSDPDWKIILLRYFNPVGAHPSGYIGEDPCGVPNNLMP
YVQQVAVGRLPHLTVYGTDYSTKDGTGVRDYIHVVDLADGHIAALRKLYEDSDKIGCEVYNLGTGKGTSV
LEMVAAFEKASGKKIPLVFAGRRPGDAEIVYAATAKAEKELKWKAKYGIEEMCRDLWNWASKNPYGYAGS
RDNSK

## Figure 23 (continued)

**SEQ ID NO 291: Zea mays UGE IV**
ATGGCGGTGGAGAAGACGCTGCCCGGCGCGAGTGCGGGGAGGACCGTGCTGGTCACGGGCGGGGCGGGCTAC
ATCGGTAGCCACGCCGTGCTGCAGCTCCTCCTCGCGGGCTTCCGCGCCGTCGTCATCGACAACCTCAACAAC
TCCTCCGAGCTGGCCGTCCGCCGCGTCGCCGCGCTCGCGGGGGACCACTCCCGCAACCTCTCTTTCCACAAG
ATTGATCTCCGTGACAAGGGAGCACTGGAAATGGTTTTTGCTTCTACAAGATTTGAAGCTGTCATTCACTTC
GCTGGATTGAAAGCTGTGGGTGAAAGCGTACAGAAGCCATTACTTTATTATGACAACAACGTCATTGGCACG
ATAAATCTTCTAGAAGTTATGTCTGTTCACGGTTGCAAGAAGTTGGTGTTCTCATCATCAGCTGCAGTTTAT
GGATCACCCAAAAACTCACCCTGCACAGAAAATTTTCCTCTTACTCCAAACAATCCATATGGCAAAACAAAG
CTCGTTGTTGAAGATATTTGCCGGGATATCTACCGTTCAGATCCTGAATGGAAGATCATTTTACTTAGGTAC
TTCAATCCAGTTGGTGCTCATCCTAGTGGATATCTTGGCGAGGACCCACGAGGAATTCCCAACAATCTTATG
CCCTATGTTCAGCAAGTTGCGGTTGGTAGGAGGCCAGCTCTAACAGTTTTAGGAAATGACTATGCAACAAGA
GATGGGACTGGGGTCCGAGATTACATCCATGTGGTTGACCTTGCTGACGGACATATTGCTGCATTGCAGAAG
CTTTTTGAGAACTCTAGCATAGGGTGTGAAGCGTACAACCTTGGAACCGGAAGAGGTACATCTGTGCTGGAG
ATTGTTAAAGCATTTGAGAAGGCTTCTGGGAAGAAAATACCTCTGATTTTTGGTGAAAGACGCCCAGGTGAT
GCAGAGATTCTGTTTTCAGAGACTACTAAAGCAGAGAGGGAGCTTAACTGGAAAGCAAAATACGGTATTGAA
GAGATGTGCCGCGACCAATGGAACTGGGCCAGCAAGAACCCTTATGGCTATGGATCACCTGACTCTATCAAG
CAGAATGGTCACCAAACAAACGGATCCGCTGACTCCTCCAAGCAGAATGGCCACCGCACAAACGGTTCAACT
GACTCACCCAAGCGGAACGGCCACCATGCGTATGGGTCTGCTGACTCACCCAAGCGCAACGGGCACTGCGTT
TTTGGATCATCAGACCTCAAGCCGAATGGTAATGGCCACCTGCGCTGA

**SEQ ID NO 292: Zea mays UGE IV**
MAVEKTLPGASAGRTVLVTGGAGYIGSHAVLQLLLAGFRAVVIDNLNNSSELAVRRVAALAGDHSRNLSF
HKIDLRDKGALEMVFASTRFEAVIHFAGLKAVGESVQKPLLYYDNNVIGTINLLEVMSVHGCKKLVFSSS
AAVYGSPKNSPCTENFPLTPNNPYGKTKLVVEDICRDIYRSDPEWKIILLRYFNPVGAHPSGYLGEDPRG
IPNNLMPYVQQVAVGRRPALTVLGNDYATRDGTGVRDYIHVVDLADGHIAALQKLFENSSIGCEAYNLGT
GRGTSVLEIVKAFEKASGKKIPLIFGERRPGDAEILFSETTKAERELNWKAKYGIEEMCRDQWNWASKNP
YGYGSPDSIKQNGHQTNGSADSSKQNGHRTNGSTDSPKRNGHHAYGSADSPKRNGHCVFGSSDLKPNGNG
HLR

**SEQ ID NO 293: Zea mays UGE V**
ATGGCGGTGGAGAAGACGCTGCCCGGCGCGAGTGCGGGGAGGACCGTGCTGGTCACGGGCGGGGCGGGCTAC
ATCGGTAGCCACGCCGTGCTGCAGCTCCTCCTCGCGGGCTTCCGCGCCGTCGTCATCGACAACCTCAACAAC
TCCTCCGAGCTGGCCGTCCGCCGCGTCGCCGCGCTCGCGGGGGACCACTCCCGCAACCTCTCTTTCCACAAG
ATTGATCTCCGTGACAAGGGAGCACTGGAAATGGTTTTTGCTTCTACAAGATTTGAAGCTGTCATTCACTTC
GCTGGATTGAAAGCTGTGGGTGAAAGCGTACAGAAGCCATTACTTTATTATGACAACAACGTCATTGGCACG
ATAAATCTTCTAGAAGTTATGTCTGTTCACGGTTGCAAGAAGTTGGTGTTCTCATCATCAGCTGCAGTTTAT
GGATCACCCAAAAACTCACCCTGCACAGAAAATTTTCCTCTTACTCCAAACAATCCATATGGCAAAACAAAG
CTCGTTGTTGAAGATATTTGCCGGGATATCTACCGTTCAGATCCTGAATGGAAGATCATTTTACTTAGGTAC
TTCAATCCAGTTGGTGCTCATCCTAGTGGATATCTTGGCGAGGACCCACGAGGAATTCCCAACAATCTTATG
CCCTATGTTCAGCAAGTTGCGGTTGGTAGGAGGCCAGCTCTAACAGTTTTAGGAAATGACTATGCAACAAGA
GATGGGACTGGGGTCCGAGATTACATCCATGTGGTTGACCTTGCTGACGGACATATTGCTGCATTGCAGAAG
CTTTTTGAGAACTCTAGCATAGGGTGTGAAGCGTACAACCTTGGAACCGGAAGAGGTACATCTGTGCTGGAG
ATTGTTAAAGCATTTGAGAAGGCTTCTGGGAAGAAAATACCTCTGATTTTTGGTGAAAGACGCCCAGGTGAT
GCAGAGATTCTGTTTTCAGAGACTACTAAAGCAGAGAGGGAGCTTAACTGGAAAGCAAAATACGGTATTGAA
GAGATGTGCCGCGACCAATGGAACTGGGCCAGCAAGAACCCTTATGGCTATGGATCACCTGACTCTATCAAG
CAGAATGGTCACCAAACAAACGGATCCGCTGACTCCTCCAAGCAGAATGGCCACCGCACAAACGGTTCAACT
GACTCACCCAAGCGGAACGGCCACCATGCGTATGGGTCTGCTGACTCACCCAAGCGCAACGGGCACTGCGTT
TTTGGATCATCAGACCTCAAGCCGAATGGTAATGGCCACCTGCGCTGA

## Figure 23 (continued)

**SEQ ID NO 294: Zea mays UGE V**
MAVEKTLPGASAGRTVLVTGGAGYIGSHAVLQLLLAGFRAVVIDNLNNSSELAVRRVAALAGDHSRNLSF
HKIDLRDKGALEMVFASTRFEAVIHFAGLKAVGESVQKPLLYYDNNVIGTINLLEVMSVHGCKKLVFSSS
AAVYGSPKNSPCTENFPLTPNNPYGKTKLVVEDICRDIYRSDPEWKIILLRYFNPVGAHPSGYLGEDPRG
IPNNLMPYVQQVAVGRRPALTVLGNDYATRDGTVRDYIHVVDLADGHIAALQKLFENSSIGCEAYNLGT
GRGTSVLEIVKAFEKASGKKIPLIFGERRPGDAEILFSETTKAERELNWKAKYGIEEMCRDQWNWASKNP
YGYGSPDSIKQNGHQTNGSADSSKQNGHRTNGSTDSPKRNGHHAYGSADSPKRNGHCVFGSSDLKPNGNG
HLR

**SEQ ID NO 295: Zea mays UGE VI**
ATGGCGGTGGAGAAGACGCTGCCCGGCGCGAGTGCGGGGAGGACCGTGCTGGTCACGGGCGGGGCGGGCTAC
ATCGGTAGCCACGCCGTGCTGCAGCTCCTCCTCGCGGGCTTCCGCGCCGTCGTCATCGACAACCTCAACAAC
TCCTCCGAGCTGGCCGTCCGCCGCGTCGCCGCGCTCGCGGGGGACCACTCCCGCAACCTCTCTTTCCACAAG
ATTGATCTCCGTGACAAGGGAGCACTGGAAATGGTTTTTGCTTCTACAAGATTTGAAGCTGTCATTCACTTC
GCTGGATTGAAAGCTGTGGGTGAAAGCGTACAGAAGCCATTACTTTATTATGACAACAACGTCATTGGCACG
ATAAATCTTCTAGAAGTTATGTCTGTTCACGGTTGCAAGAAGTTGGTGTTCTCATCATCAGCTGCAGTTTAT
GGATCACCCAAAAACTCACCCTGCACAGAAAATTTTCCTCTTACTCCAAACAATCCATATGGCAAAACAAAG
CTCGTTGTTGAAGATATTTGCCGGGATATCTACCGTTCAGATCCTGAATGGAAGATCATTTTACTTAGGTAC
TTCAATCCAGTTGGTGCTCATCCTAGTGGATATCTTGGCGAGGACCCACGAGGAATTCCCAACAATCTTATG
CCCTATGTTCAGCAAGTTGCGGTTGGTAGGAGGCCAGCTCTAACAGTTTTAGGAAATGACTATGCAACAAGA
GATGGGACTGGGGTCCGAGATTACATCCATGTGGTTGACCTTGCTGACGGACATATTGCTGCATTGCAGAAG
CTTTTTGAGAACTCTAGCATAGGGTGTGAAGCGTACAACCTTGGAACCGGAAGAGGTACATCTGTGCTGGAG
ATTGTTAAAGCATTTGAGAAGGCTTCTGGGAAGAAAATACCTCTGATTTTTGGTGAAAGACGCCCAGGTGAT
GCAGAGATTCTGTTTTCAGAGACTACTAAAGCAGAGAGGGAGCTTAACTGGAAAGCAAAATACGGTATTGAA
GAGATGTGCCGCGACCAATGGAACTGGGCCAGCAAGAACCCTTATGGCTATGGATCACCTGACTCTATCAAG
CAGAATGGTCACCAAACAAACGGATCCGCTGACTCCTCCAAGCAGAATGGCCACCGCACAAACGGTTCAACT
GACTCACCCAAGCGGAACGGCCACCATGCGTATGGGTCTGCTGACTCACCCAAGCGCAACGGGCACTGCGTT
TTTGGATCATCAGACCTCAAGCCGAATGGTAATGGCCACCTGCGCTGA

**SEQ ID NO 296: Zea mays UGE VI**
MAVEKTLPGASAGRTVLVTGGAGYIGSHAVLQLLLAGFRAVVIDNLNNSSELAVRRVAALAGDHSRNLSF
HKIDLRDKGALEMVFASTRFEAVIHFAGLKAVGESVQKPLLYYDNNVIGTINLLEVMSVHGCKKLVFSSS
AAVYGSPKNSPCTENFPLTPNNPYGKTKLVVEDICRDIYRSDPEWKIILLRYFNPVGAHPSGYLGEDPRG
IPNNLMPYVQQVAVGRRPALTVLGNDYATRDGTVRDYIHVVDLADGHIAALQKLFENSSIGCEAYNLGT
GRGTSVLEIVKAFEKASGKKIPLIFGERRPGDAEILFSETTKAERELNWKAKYGIEEMCRDQWNWASKNP
YGYGSPDSIKQNGHQTNGSADSSKQNGHRTNGSTDSPKRNGHHAYGSADSPKRNGHCVFGSSDLKPNGNG
HLR

**SEQ ID NO 297: Zea mays UGE VII**
ATGGCGGTGGAGAAGACGCTGCCCGGCGCGAGTGCTGGGAGGACCGTGCTGGTCACGGGCGGGGCGGGCTAC
ATCGGTAGCCACGCCGTGCTGCAGCTCCTCCTCGCGGGCTTCCGCGCCGTCGTCGTCGACAACCTCAACAAC
TCCTCCGAGCTGGCCGTCCGCCGCGTCGCCGCGCTCGCGGGGGACCACTCCCGCAACCTCTCTTTCCACAAG
ATTGATCTCCGTGACAAGGGAGCACTGGAAATGGTTTTTGCTTCTACAAGATTTGAAGCTGTCATTCACTTC
GCTGGATTGAAAGCTGTGGGTGAAAGCGTACAGAAGCCATTACTTTATTATGACAACAACGTCATTGGCACG
ATAAATCTTCTAGAAGTTATGTCTGTTCACGGTTGCAAGAAGTTGGTGTTCTCATCATCAGCTGCAGTTTAT
GGATCACCCAAAAACTCACCCTGCACAGAAAATTTTCCTCTTACTCCAAACAATCCATATGGCAAAACAAAG
CTCGTTGTTGAAGATATTTGCCGGGATATCTACCGTTCAGATCCTGAATGGAAGATCATTTTACTTAGGTAC
TTCAATCCAGTTGGTGCTCATCCTAGTGGATATCTTGGCGAGGACCCACGAGGAATTCCCAACAATCTTATG
CCCTATGTTCAGCAAGTTGCGGTTGGTAGGAGGCCAGCTCTAACAGTTTTAGGAAATGACTATGCAACAAGA
GATGGGACTGGGGTCCGAGATTACATCCATGTGGTTGACCTTGCTGACGGACATATTGCTGCATTGCAGAAG
CTTTTTGAGAACTCTAGCATAGGGTGTGAAGCGTACAACCTTGGAACCGGAAGAGGTACATCTGTGCTGGAG
ATTGTTAAAGCATTTGAGAAGGCTTCTGGGAAGAAAATACCTCTGATTTTTGGTGAAAGACGCCCAGGTGAT

## Figure 23 (continued)

GCAGAGATTCTGTTTTCAGAGACTACTAAAGCAGAGAGGGAGCTTAACTGGAAAGCAAAATACGGTATTGAA
GAGATGTGCCGCGACCAATGGAACTGGGCCAGCAAGAACCCTTATGGCTATGGATCACCTGACTCTATCAAG
CAGAATGGTCACCAAACAAACGGATCCGCTGACTCCTCCAAGCAGAATGGCCACCGCACAAACGGTTCAACT
GACTCACCCAAGCGGAACGGCCACCATGCGTATGGGTCTGCTGACTCACCCAAGCGCAACGGGCACTGCGTT
TTTGGATCATCAGACCTCAAGCCGAATGGTAATGGCCACCTGCGCTGA

**SEQ ID NO 298: Zea mays UGE VII**
MAVEKTLPGASAGRTVLVTGGAGYIGSHAVLQLLLAGFRAVVVDNLNNSSELAVRRVAALAGDHSRNLSF
HKIDLRDKGALEMVFASTRFEAVIHFAGLKAVGESVQKPLLYYDNNVIGTINLLEVMSVHGCKKLVFSSS
AAVYGSPKNSPCTENFPLTPNNPYGKTKLVVEDICRDIYRSDPEWKIILLRYFNPVGAHPSGYLGEDPRG
IPNNLMPYVQQVAVGRRPALTVLGNDYATRDGTGVRDYIHVVDLADGHIAALQKLFENSSIGCEAYNLGT
GRGTSVLEIVKAFEKASGKKIPLIFGERRPGDAEILFSETTKAERELNWKAKYGIEEMCRDQWNWASKNP
YGYGSPDSIKQNGHQTNGSADSSKQNGHRTNGSTDSPKRNGHHAYGSADSPKRNGHCVFGSSDLKPNGNG
HLR

**SEQ ID NO 299: Zea mays UGE VIII**
ATGGTGTCCGCCGTGCTCCGGACCATCCTCGTGACGGGCGGCGCCGGGTACATCGGCAGCCACACGGTGCTG
CAGCTGCTGCAGCAGGGCTTCCGCGTCGTCGTCGTCGACAACCTCGACAACGCCTCCGAGGCCGCCCTCGCC
CGCGTCGCCGAGCTCGCCGGGCACGACGGCGCCAACCTCGTCTTCCACAAGGTTGACCTTCGCGACAGGCAC
GCGTTGGTGGACATCTTCTCGTCGCACAGGTTCGAGGCTGTCATTCACTTTGCTGGGCTCAAGGCTGTTGGG
GAGAGCGTGCACAAGCCCCTACTTTACTACGACAACAACCTGGTCGGCACCATCACCCTCCTGGAGGTGATG
GCTGCGAACGGCTGCAAGAAGCTGGTGTTCTCGTCATCTGCAACTGTCTATGGGTGGCCCAAGGAAGTACCC
TGCACCGAAGAATTCCCGCTCTGCGCCACCAATCCCTATGGGCGGACAAAGCTTGTGATTGAAGACATCTGC
CGCGACGTCCACCGCTCCGACCCCGACTGGAAGATCATACTGCTCAGGTACTTCAACCCCGTTGGCGCTCAT
CCAAGCGGGTACATCGGCGAAGACCCCTGCGGTGTCCCGAACAACCTGATGCCCTACGTGCAGCAAGTCGCT
GTTGGGAAGTTACCTCACCTCACGGTCTACGGGACGGACTACAGCACCAAGGATGGGACTGGGGTGCGTGAT
TACATCCACGTTGTCGACCTGGCTGACGGCCACATAGCAGCCCTGAGGAAGCTCTACGAAGACTCCGACAAA
ATAGGCTGTGAAGTGTACAACTTGGGGACTGGAAAGGGGACGTCCGTGTTGGAAATGGTGGCTGCATTCGAG
AAGGCTTCTGGGAAGAAAATCCCTCTGGTGTTCGCTGGGCGAAGACCCGGAGACGCAGAGATCGTCTACGCC
GCAACTGCCAAGGCAGAGAAGGAGCTCAAATGGAAGGCCAAGTACGGGATCGAGGAGATGTGCAGAGATCTG
TGGAACTGGGCGAGCAAGAACCCGTACGGGTACGCTGGGTCACGCGACAACAGCAAATGA

**SEQ ID NO 300: Zea mays UGE VIII**
MVSAVLRTILVTGGAGYIGSHTVLQLLQQGFRVVVVDNLDNASEAALARVAELAGHDGANLVFHKVDLRD
RHALVDIFSSHRFEAVIHFAGLKAVGESVHKPLLYYDNNLVGTITLLEVMAANGCKKLVFSSSATVYGWP
KEVPCTEEFPLCATNPYGRTKLVIEDICRDVHRSDPDWKIILLRYFNPVGAHPSGYIGEDPCGVPNNLMP
YVQQVAVGKLPHLTVYGTDYSTKDGTGVRDYIHVVDLADGHIAALRKLYEDSDKIGCEVYNLGTGKGTSV
LEMVAAFEKASGKKIPLVFAGRRPGDAEIVYAATAKAEKELKWKAKYGIEEMCRDLWNWASKNPYGYAGS
RDNSK

**SEQ ID NO 301: Oryza sativa UGE**
ATGGTTTCGGCCTTGTTGCGGACGATCCTGGTGACGGGCGGCGCCGGCTACATCGGCAGCCACACCGTCCTC
CAGCTTCTCCAACTCGGCTTCCGCGTTGTCGTCCTCGACAACCTCGACAACGCCTCCGAGCTCGCCATCCTC
CGCGTCAGGGAACTCGCCGGACACAACGCCAACAACCTCGACTTCCGCAAGGTTGACCTCCGCGACAAGCAA
GCGTTGGACCAAATCTTCTCCTCTCAAAGGTTTGAGGCTGTCATCCATTTTGCCGGGCTGAAAGCTGTTGGC
GAGAGCGTGCAGAAGCCCCTGCTTTACTACGACAACAACCTCATCGGCACCATCACTCTCCTGCAGGTCATG
GCCGCACATGGCTGCACCAAGCTGGTGTTCTCATCATCCGCAACTGTCTACGGGTGGCCCAAGGAGGTGCCC
TGCACTGAAGAATCCCCACTTTGTGCAATGAACCCCTACGGCAGAACAAAGCTGGTAATCGAAGACATGTGC
CGGGATCTGCATGCCTCAGACCCAAACTGGAAGATCATACTGCTCCGATACTTCAACCCTGTTGGAGCTCAC
CCAAGCGGGTACATTGGTGAGGACCCCTGCGGCATCCCAAACAACCTCATGCCCTTCGTCCAGCAGGTCGCT

Figure 23 (continued)

GTTGGCAGGAGGCCGGCCCTTACCGTCTATGGAACCGACTACAACACCAAGGATGGAACTGGGGTTCGTGAC
TATATCCATGTTGTTGATCTAGCGGATGGTCATATCGCCGCGTTAAGGAAGCTCTATGAAGATTCTGATAGA
ATAGGATGTGAGGTGTACAATCTGGGCACTGGAAAGGGGACATCTGTGCTGGAAATGGTTGCAGCATTCGAG
AAAGCTTCTGGAAAGAAAATCCCGCTTGTATTTGCTGGACGAAGGCCTGGAGATGCCGAGATCGTTTACGCT
CAAACTGCCAAAGCTGAGAAGGAACTGAAATGGAAGGCAAAATACGGGGTAGAGGAGATGTGCAGGGACCTG
TGGAATTGGGCGAGCAAGAACCCCTACGGGTATGGATCGCCGGACAGTAGCAACTGA

**SEQ ID NO 302: Oryza sativa UGE**
MVSALLRTILVTGGAGYIGSHTVLQLLQLGFRVVVLDNLDNASELAILRVRELAGHNANNLDFRKVDLRD
KQALDQIFSSQRFEAVIHFAGLKAVGESVQKPLLYYDNNLIGTITLLQVMAAHGCTKLVFSSSATVYGWP
KEVPCTEESPLCAMNPYGRTKLVIEDMCRDLHASDPNWKIILLRYFNPVGAHPSGYIGEDPCGIPNNLMP
FVQQVAVGRRPALTVYGTDYNTKDGTGVRDYIHVVDLADGHIAALRKLYEDSDRIGCEVYNLGTGKGTSV
LEMVAAFEKASGKKIPLVFAGRRPGDAEIVYAQTAKAEKELKWKAKYGVEEMCRDLWNWASKNPYGYGSP
DSSN

**SEQ ID NO 303: Glycine max UGE I**
ATGGCATCGCGCGTCAGCATTGGCAACCTTACCTCCTCCGCGCCGTATATTAATTCCCCTCACTTTCGCTCA
CCACTTAAGATTTCCAACAACCCCTCTCTGCAAAACGCTTCGCATAAGGTACTTATGCGCGATAAGACTGTA
CTGGTAACCGGCGGAGCCGGTTACATCGGCAGCCACACCGTTCTTCAGCTCTTGCTCGGAGGTTTCAGAGCC
GTCGTCCTCGACAACCTCGAAAATTCCTCCGAGGTTGCCATCCACAGAGTCAGGGAGCTCGCCGGCGAATTT
GGGAACAACCTCTCCTTTCACAAGGTGGACCTACGGGACAGAGCTGCTCTAGACCAAATATTTTCTTCCACA
CAATTCGATGCTGTCATACATTTTGCTGGACTGAAAGCAGTAGGAGAAAGTGTGCAAAAACCTTTACTATAC
TATAACAACAACTTGACTGGGACAATCACTCTATTGGAAGTCATGGCTGCCCATGGATGCAAGAAGCTCGTG
TTTTCATCTTCAGCAACTGTATATGGTTGGCCAAAGGAGGTTCCATGCACAGAAGAGTTCCCTCTGTCAGCA
ATGAACCCATATGGACGAACTAAGCTTATCATTGAAGAAATTTGCCGTGATGTCCACTGTGCAGAGCCAGAT
TGTAAAATAATTTTGTTAAGATACTTCAACCCAGTTGGTGCACACCCCAGTGGTTATATTGGGGAGGATCCT
CGTGGAATTCCAAACAATCTCATGCCATTTGTTCAGCAAGTAGCAGTTGGCCGACGGCCTGCACTGACAGTT
TTTGGAAATGATTATAATACAAGTGATGGCACTGGGGTTCGGGATTACATTCATGTTGTTGATTTAGCAGAT
GGGCACATTGCTGCATTGCTTAAACTAGATGAACCTAATATAGGTTGTGAGGTTTATAACCTGGGAACAGGA
AAGGGAACATCAGTTTTGGAGATGGTTAGAGCTTTTGAAATGGCATCTGGAAAGAAAATTCCACTTGTGATG
GCTGGCCGTAGACCTGGTGATGCTGAAATTGTTTATGCATCAACAAAGAAAGCGGAAAGAGAGCTTAAATGG
AAGGCAAAATATGGCATTGATGAGATGTGCCGTGATCAATGGAATTGGGCTAGCAAAAACCCTTATGGCTAT
GGAGATCAGGGCTCCACCGATTAA

**SEQ ID NO 304: Glycine max UGE I**
MASRVSIGNLTSSAPYINSPHFRSPLKISNNPSLQNASHKVLMRDKTVLVTGGAGYIGSHTVLQLLLGGF
RAVVLDNLENSSEVAIHRVRELAGEFGNNLSFHKVDLRDRAALDQIFSSTQFDAVIHFAGLKAVGESVQK
PLLYYNNNLTGTITLLEVMAAHGCKKLVFSSSATVYGWPKEVPCTEEFPLSAMNPYGRTKLIIEEICRDV
HCAEPDCKIILLRYFNPVGAHPSGYIGEDPRGIPNNLMPFVQQVAVGRRPALTVFGNDYNTSDGTGVRDY
IHVVDLADGHIAALLKLDEPNIGCEVYNLGTGKGTSVLEMVRAFEMASGKKIPLVMAGRRPGDAEIVYAS
TKKAERELKWKAKYGIDEMCRDQWNWASKNPYGYGDQGSTD

**SEQ ID NO 305: Glycine max UGE II**
ATGCCTGCCCAATCGATTCTGGTTACCGGAGGAGCCGGTTACATCGGTAGCCACACCATCCTTCAGCTTCTC
CTCAGCGGTTACCATGTCTTCGCCGTCGACAACTTCGACAATTCTTCCGAAACCGCCATCAACAGAGTCAAG
GAACTCGCCGGAGAATTAGCAAATAACCTTTCCTTTTGCAAGCTTGACTTGCGGGACAGAGCTGCCCTCGAG
AAAATATTTTCAACCGTAAAGTTTGATGCCGTCATACATTTTGCTGGGCTTAAAGCAGTGGGTGAAAGCGTG
AAGAAACCACTGCTCTACTTTGACAACAATTTGATAGGGACAATCGTTCTATTTGAAGTCATGGCTGCCCAC
GGATGCAAGAAGCTTGTGTTCTCATCTTCAGCAACTGTATATGGGTGGCCAAAGGAGGTCCCATGTACCGAA
GAGTTCCCTTTGTCAGCAACAAACCCATATGGACGAACCAAGCTTATCATCGAAGAAATTTGTCGCGATATC

## Figure 23 (continued)

CACCGTGCAGATTCAGATTGGACAGTTATACTATTGAGATACTTCAACCCAGTTGGTGCACATCCCAGTGGT
TATATTGGTGAGGATCCTCTTGGAATTCCAAACAATCTCATGCCCTTTGTTCAACAAGTGGCAGTTGGCAGA
CGCCCTGCATTGACAGTTTTTGGAAGCGATTATAAAACAACTGACGGCACTGGAGTTCGTGATTACATTCAT
GTTCTTGATTTAGCAGATGGGCATATTGCTGCGTTGCGTAAACTGGATGATCCTAAAATAGGTTGTGAGGTT
TATAACTTGGGAACAGGAAAGGGAACATCAGTTTTGGAGATGGTAAACGCTTTTGAACAGGCCTCTGGAAAG
AAAATTCCACTTGCAATGGCGGGTCGGAGACCTGGAGATGCTGAAATTGTTTATGCATCAACAGAAAAAGCG
GAAAGAGAACTTAATTGGAAGACTAAATATAGCATCGATGATATGTGCCGCGATCAATGGAATTGGGCTAGC
AAAAACCCTTATGGCTATGGTGCATCTGCAGATTCCTCCAATTGA

**SEQ ID NO 306: Glycine max UGE II**
MPAQSILVTGGAGYIGSHTILQLLLSGYHVFAVDNFDNSSETAINRVKELAGELANNLSFCKLDLRDRAA
LEKIFSTVKFDAVIHFAGLKAVGESVKKPLLYFDNNLIGTIVLFEVMAAHGCKKLVFSSSATVYGWPKEV
PCTEEFPLSATNPYGRTKLIIEEICRDIHRADSDWTVILLRYFNPVGAHPSGYIGEDPLGIPNNLMPFVQ
QVAVGRRPALTVFGSDYKTTDGTGVRDYIHVLDLADGHIAALRKLDDPKIGCEVYNLGTGKGTSVLEMVN
AFEQASGKKIPLAMAGRRPGDAEIVYASTEKAERELNWKTKYSIDDMCRDQWNWASKNPYGYGASADSSN

**SEQ ID NO 307: Glycine max UGE III**
ATGCGCGACAAGACTGTGCTGGTAACCGGCGGAGCCGGTTACATCGGCACCCACACCGTTCTTCAGCTCTTG
CTCGGAGGTTGCAGAACCGTCGTCGTCGACAATCTCGACAATTCCTCCGAGGTTTCTATCCACCGAGTCAGG
GAGCTTGCCGGCGAATTTGGGAACAACCTCTCCTTTCACAAGGTGGACCTACGGGACAGGGATGCACTAGAG
CAAATTTTTGTTTCCACACAATTTGATGCTGTCATACACTTTGCTGGACTGAAAGCAGTAGGAGAAAGTGTG
CAAAAACCTTTACTATACTATAACAACAACTTGACTGGGACAATCACTCTATTGGAAGTCATGGCTGCCCAT
GGATGCAAGAAGCTCGTGTTCTCTTCTTCAGCAACTGTATATGGTTGGCCAAAGGAAGTTCCATGCACAGAA
GAGTTCCCTCTGTCAGCAATGAACCCATATGGACGAACTAAGCTTATCATTGAAGAAATTTGCCGTGATGTC
CACTGTGCAGAGCCAGATTGTAAAATAATTTTGTTAAGATACTTCAACCCAGTTGGTGCACACCCCAGCGGT
TATATTGGGGAGGATCCTCGCGGAATTCCAAACAATCTCATGCCATTTGTTCAGCAAGTAGCAGTTGGCCGA
CGGCCTGCACTGACAGTTTTTGGAAATGATTATAATACAAGTGATGGCACTGGGGTTCGGGATTACATTCAT
GTTGTTGATTTAGCAGATGGGCACATTGCTGCATTGCTTAAACTAGATGAACCTAATATAGGTTGTGAGGTT
TATAACCTGGGAACAGGAAAGGGAACATCAGTTTTGGAGATGGTTAGAGCTTTTGAAATGGCATCTGGAAAG
AAAATTCCACTTGTGATGGCTGGCCGTAGACCTGGTGATGCTGAAATTGTTTATGCATCAACAAAGAAAGCG
GAAAGAGAGCTTAAATGGAAGGCAAAATATGGCATTGATGAGATGTGCCGTGATCAATGGAATTGGGCTAGC
AAAAACCCTTATGGCTATGGAGATCAGGGCTCCACCGATTAA

**SEQ ID NO 308: Glycine max UGE III**
MRDKTVLVTGGAGYIGTHTVLQLLLGGCRTVVVDNLDNSSEVSIHRVRELAGEFGNNLSFHKVDLRDRDA
LEQIFVSTQFDAVIHFAGLKAVGESVQKPLLYYNNNLTGTITLLEVMAAHGCKKLVFSSSATVYGWPKEV
PCTEEFPLSAMNPYGRTKLIIEEICRDVHCAEPDCKIILLRYFNPVGAHPSGYIGEDPRGIPNNLMPFVQ
QVAVGRRPALTVFGNDYNTSDGTGVRDYIHVVDLADGHIAALLKLDEPNIGCEVYNLGTGKGTSVLEMVR
AFEMASGKKIPLVMAGRRPGDAEIVYASTKKAERELKWKAKYGIDEMCRDQWNWASKNPYGYGDQGSTD

**SEQ ID NO 309: Glycine max UGE IV**
ATGCGCGACAAGACTGTGCTGGTAACCGGCGGAGCCGGTTACATCGGCACCCACACCGTTCTTCAGCTCTTG
CTCGGAGGTTGCAGAACCGTCGTCGTCGACAATCTCGACAATTCCTCCGAGGTTTCTATCCACCGAGTCAGG
GAGCTTGCCGGCGAATTTGGGAACAACCTCTCCTTTCACAAGGTGGACCTACGGGACAGGGATGCACTAGAG
CAAATTTTTGTTTCCACACAATTTGATGCTGTCATACACTTTGCTGGACTGAAAGCAGTAGGAGAAAGTGTG
CAAAAACCTTTACTATACTATAACAACAACTTGACTGGGACAATCACTCTATTGGAAGTCATGGCTGCCCAT
GGATGCAAGAAGCTCGTGTTCTCTTCTTCAGCAACTGTATATGGTTGGCCAAAGGAAGTTCCATGCACAGAA
GAGTTCCCTCTGTCAGCAATGAACCCATATGGACGAACTAAGCTTATCATTGAAGAAATTTGTCGTGATGTC
CACCGTGCAGAGCCAGATTGGAAAATAATATTGTTAAGATACTTCAACCCAGTCGGTGCACACCCTAGCGGT
TGTATTGGGGAGGATCCCCGCGGAATTCCAAACAATCTCATGCCATTTGTTCAGCAAGTAGCAGTTGGCCGA

**Figure 23 (continued)**

CGGCCTGCACTGACAGTTTTGGAAATGATTATAATACAACTGATGGCACTGGGGTTCGGGATTACATTCAT
GTTGTTGATTTAGCAGATGGGCACATTGCTGCATTGCTTAAACTAGATGAACCTAATATAGGTTGTGAGGTT
TATAACCTGGGAACAGGAAAGGGAACATCAGTTTTGGAGATGGTTAGAGCTTTTGAAATGGCATCTGGAAAG
AAAATTCCACTTGTGATGGCTGGCCGTAGACCTGGTGATGCTGAAATTGTTTATGCATCAACAAAGAAAGCG
GAAAGAGAGCTTAAATGGAAGGCAAAATATGGCATTGATGAGATGTGCCGAAATCAATGGAATTGGGCTAGC
AAAAACCCTTATGGCTATGGAGATCAGGGCTCCACCGATTAA

**SEQ ID NO 310: Glycine max UGE IV**
MRDKTVLVTGGAGYIGTHTVLQLLLGGCRTVVVDNLDNSSEVSIHRVRELAGEFGNNLSFHKVDLRDRDA
LEQIFVSTQFDAVIHFAGLKAVGESVQKPLLYYNNNLTGTITLLEVMAAHGCKKLVFSSSATVYGWPKEV
PCTEEFPLSAMNPYGRTKLIIEEICRDVHRAEPDWKIILLRYFNPVGAHPSGCIGEDPRGIPNNLMPFVQ
QVAVGRRPALTVFGNDYNTTDGTGVRDYIHVVDLADGHIAALLKLDEPNIGCEVYNLGTGKGTSVLEMVR
AFEMASGKKIPLVMAGRRPGDAEIVYASTKKAERELKWKAKYGIDEMCRNQWNWASKNPYGYGDQGSTD

**SEQ ID NO 311: Glycine max UGE V**
ATGGCATCGCGCGTCAGCATTGGCAACCTTACCTCCTCCGCGCCGTATATTAATTCCCCTCACTTTCGCTCA
CCACTTAAGATTTCCAACAACCCCTCTCTGCAAAACGCTTCGCATAAGGTACTTATGCGCGATAAGACTGTA
CTGGTAACCGGCGGAGCCGGTTACATCGGCAGCCACACCGTTCTTCAGCTCTTGCTCGGAGGTTTCAGAGCC
GTCGTCCTCGACAACCTCGAAAATTCCTCCGAGGTTGCCATCCACAGAGTCAGGGAGCTCGCCGGCGAATTT
GGGAACAACCTCTCCTTTCACAAGGTGGACCTACGGGACAGAGCTGCTCTAGACCAAATATTTTCTTCCACA
CAATTCGATGCTGTCATACATTTTGCTGGACTGAAAGCAGTAGGAGAAAGTGTGCAAAAACCTTTACTATAC
TATAACAACAACTTGACTGGGACAATCACTCTATTGGAAGTCATGGCTGCCCATGGATGCAAGAAGCTCGTG
TTTTCATCTTCAGCAACTGTATATGGTTGGCCAAAGGAGGTTCCATGCACAGAAGAGTTCCCTCTGTCAGCA
ATGAACCCATATGGACGAACTAAGCTTATCATTGAAGAAATTTGCCGTGATGTCCACTGTGCAGAGCCAGAT
TGTAAAATAATTTTGTTAAGATACTTCAACCCAGTTGGTGCACACCCCAGTGGTTATATTGGGGAGGATCCT
CGTGGAATTCCAAACAATCTCATGCCATTTGTTCAGCAAGTAGCAGTTGGCCGACGGCCTGCACTGACAGTT
TTTGGAAATGATTATAATACAAGTGATGGCACTGGGGTTCGGGATTACATTCATGTTGTTGATTTAGCAGAT
GGGCACATTGCTGCATTGCTTAAACTAGATGAACCTAATATAGGTTGTGAGGTTTATAACCTGGGAACAGGA
AAGGGAACATCAGTTTTGGAGATGGTTAGAGCTTTTGAAATGGCATCTGGAAAGAAAATTCCACTTGTGATG
GCTGGCCGTAGACCTGGTGATGCTGAAATTGTTTATGCATCAACAAAGAAAGCGGAAAGAGAGCTTAAATGG
AAGGCAAAATATGGCATTGATGAGATGTGCCGTGATCAATGGAATTGGGCTAGCAAAAACCCTTATGGCTAT
GGAGATCAGGGCTCCACCGATTAA

**SEQ ID NO 312: Glycene max UGE V**
MASRVSIGNLTSSAPYINSPHFRSPLKISNNPSLQNASHKVLMRDKTVLVTGGAGYIGSHTVLQLLLGGF
RAVVLDNLENSSEVAIHRVRELAGEFGNNLSFHKVDLRDRAALDQIFSSTQFDAVIHFAGLKAVGESVQK
PLLYYNNNLTGTITLLEVMAAHGCKKLVFSSSATVYGWPKEVPCTEEFPLSAMNPYGRTKLIIEEICRDV
HCAEPDCKIILLRYFNPVGAHPSGYIGEDPRGIPNNLMPFVQQVAVGRRPALTVFGNDYNTSDGTGVRDY
IHVVDLADGHIAALLKLDEPNIGCEVYNLGTGKGTSVLEMVRAFEMASGKKIPLVMAGRRPGDAEIVYAS
TKKAERELKWKAKYGIDEMCRDQWNWASKNPYGYGDQGSTD

**SEQ ID NO 313: Helianthus annuus**
ATGACGCGGCCGATGTGCATACTGGTGACCGGTGGTGCCGGTTATATAGGAAGTCATACTGTTTTGCAGTTG
TTGTTAAATGGTTACAGTACGGTGGTGGTTGATAATTTGGATAATTCGTCGGAAGTTGCTATCAGTCGGGTT
CGAGAACTCGCTGGTGATCGTGGTCGTAATCTGTCTTTTCACAAGATGGATATCCGGGATAAGCCTGCACTA
GAGCAGCTTTTTGCTTCTACAAAGTTTGACGCGGTTATACATTTTGCTGGATTAAAGGCAGTTGGTGAAAGC
GTGCAGAAGCCGTTGATGTATTACAACAATAATATCGTTGGTACTATAACTTTATTGGAAGTTATGGCTGCC
TATGGCTGCAAGAAGCTTGTGTTTTCATCATCGGCTACTGTTTATGGCTGGCCAAAGGAGTTGCCGTGCACA
GAAGAGTTTCCCTTGTCTGCAGCCAACCCGTATGGACGAACAAAGCTAATGATCGAGGAGATGTGCCGAGAT
GTATATGCTTCAGACCCTGAGTGGAAATTTGTATTGTTGCGGTATTTTAACCCTGTTGGTGCACATCCCAGT

## Figure 23 (continued)

GGCCGAATTGGCGAAGATCCTCATGGAATTCCAAACAATCTTATGCCTTTTATTCAGCATGTTGCTGTTGGT
AGACAACCTGCGCTTAAAGTGTTCGGAACTGATTACTCCACAAAAGATGGAACTGGGGTACGTGATTACATC
CATGTCGTAGATTTAGCAGACGGACATACTGCGGCGTTGGCGAAACTTTCTGATCCCAAAATAGGTTGTGAA
GTTTATAACTTGGGGACTGGTAAAGGGACATCTGTTTTGGAGATGGTATCAGCCTTTGAAAAGGCATCAGGA
AAGAAAATCCCATTAATAAAGACTGCACGACGACCTGGTGATGCTGAGATTGTATATGCGTCAACAACAAAG
GCAGAACGCGAGTTAAACTGGAAGGCAAAGTATGGAATAGAGGAGATGTGCAGAGATCAGTGGAACTGGGCT
AGCAGGAATCCTTACGGCTATCAGACTGAAGCAAAGAAACTTGGTGCATGA

**SEQ ID NO 314: Helianthus annuus UGE**
MTRPMCILVTGGAGYIGSHTVLQLLLNGYSTVVVDNLDNSSEVAISRVRELAGDRGRNLSFHKMDIRDKP
ALEQLFASTKFDAVIHFAGLKAVGESVQKPLMYYNNNIVGTITLLEVMAAYGCKKLVFSSSATVYGWPKE
LPCTEEFPLSAANPYGRTKLMIEEMCRDVYASDPEWKFVLLRYFNPVGAHPSGRIGEDPHGIPNNLMPFI
QHVAVGRQPALKVFGTDYSTKDGTGVRDYIHVVDLADGHTAALAKLSDPKIGCEVYNLGTGKGTSVLEMV
SAFEKASGKKIPLIKTARRPGDAEIVYASTTKAERELNWKAKYGIEEMCRDQWNWASRNPYGYQTEAKKL
GA

**SEQ ID NO 315: Zea mays UGE IX**
gaagcacggcaatctcgatctcgttcgttcccctgagcggcagtcagtgcgagcgagcgcgcacacagacaa
ctcatcctctcccctctctctctcacacacacacatccatcatcccatccctgtgacgggcgatccgagggc
ctaccgacgatggtgtccgccgtgctccggaccatcctcgtgacgggcggcgccgggtacatcggcagccac
acggtgctgcagctgctgcagcagggcttccgcgtcgtcgtcgtcgacaacctcgacaacgcctccgaggcc
gccctcgcccgcgtcgccgagctcgccgggcacgacggcgccaacctcgtcttccacaaggttgaccttcgc
gacaggcacgcgttggtggacatcttctcgtcgcacaggttcgaggctgtcattcactttgctgggctcaag
gctgttggggagagcgtgcacaagcccctactttactacgacaacaacctggtcggcaccatcaccctcctg
gaggtgatggctgcgaacggctgcaagaagctggtgttctcgtcatctgcaactgtctatgggtggcccaag
gaagtaccctgcaccgaagaattcccgctctgcgccaccaatccctatgggcggacaaagcttgtgattgaa
gacatctgccgcgacgtccaccgctccgaccccgactggaagatcatactgctcaggtacttcaaccccgtt
ggcgctcatccaagcgggtacatcggcgaagacccctgcggtgtcccgaacaacctgatgccctacgtgcag
caagtcgctgttgggaggttacctcacctcacggtctacgggacggactacagcaccaaggatgggactggg
gtgcgtgattacatccacgttgtcgacctggctgacggccacatagcagccctgaggaagctctacgaagac
tccgacaaaataggctgtgaagtgtacaacttggggactggaaaggggacgtccgtgttggaaatggtggct
gcattcgagaaggcttctgggaagaaaatccctctggtgttcgctgggcgaagacccggagacgcagagatc
gtctacgccgcaactgccaaggcagagaaggagctcaaatggaaggccaagtacgggatcgaggagatgtgc
agagatctgtggaactgggcgagcaagaacccgtacgggtacgctgggtcacgcgacaacagcaaatgaacc
acccatccatgcatcccatcctgcagtaggagcaagcagcagccttatagcctatgctaccataattagtaa
ctcatcaatcatgcatacacataattagtaactcatcaatcatgcatacataatcagccagtcatctgatta
ggggtactgcgatgggcctcgcttcttcagtgtatagagggaggaggaggggggtcatcttgatattcttt
tttttttatcattcccgaatgattattattagtagccagttcgatgaaaaaaaaaa

**SEQ ID NO 316: Zea mays UGE IX**
MVSAVLRTILVTGGAGYIGSHTVLQLLQQGFRVVVVDNLDNASEAALARVAELAGHDGANLVFHKVDLRD
RHALVDIFSSHRFEAVIHFAGLKAVGESVHKPLLYYDNNLVGTITLLEVMAANGCKKLVFSSSATVYGWP
KEVPCTEEFPLCATNPYGRTKLVIEDICRDVHRSDPDWKIILLRYFNPVGAHPSGYIGEDPCGVPNNLMP
YVQQVAVGRLPHLTVYGTDYSTKDGTGVRDYIHVVDLADGHIAALRKLYEDSDKIGCEVYNLGTGKGTSV
LEMVAAFEKASGKKIPLVFAGRRPGDAEIVYAATAKAEKELKWKAKYGIEEMCRDLWNWASKNPYGYAGS
RDNSK

## Figure 23 (continued)

**SEQ ID NO 317: Zea mays UGE X**

cattccatcgtcttcgcttccactccccacgttccccgcctgcttcctccaggccccggccagatccgcgcg
cccgcggggaagagggcctcctctcctccctcctcccttggctcgcgctctggtcgaggaggcggaggccgg
tgccggcgggcgggcggggaggtaggtagcagcttgcggttgcgggagggaagctgcgcgcgctaggctgcc
atggcggtggagaagacgctgcccggcgcgagtgcggggaggaccgtgctggtcacgggcggggcgggctac
atcggtagccacgccgtgctgcagctcctcctcgcgggcttccgcgccgtcgtcatcgacaacctcaacaac
tcctccgagctggccgtccgccgcgtcgccgcgctcgcgggggaccactcccgcaacctctctttccacaag
attgatctccgtgacaagggagcactggaaatggtttttgcttctacaagatttgaagctgtcattcacttc
gctggattgaaagctgtgggtgaaagcgtacagaagccattactttattatgacaacaacgtcattggcacg
ataaatcttctagaagttatgtctgttcacggttgcaagaagttggtgttctcatcatcagctgcagtttat
ggatcacccaaaaactcaccctgcacagaaaattttcctcttactccaaacaatccatatggcaaaacaaag
ctcgttgttgaagatatttgccgggatatctaccgttcagatcctgaatggaagatcattttacttaggtac
ttcaatccagttggtgctcatcctagtggatatcttggcgaggacccacgaggaattcccaacaatcttatg
ccctatgttcagcaagttgcggttggtaggaggccagctctaacagttttaggaaatgactatgcaacaaga
gatgggactggggtccgagattacatccatgtggttgaccttgctgacggacatattgctgcattgcagaag
ctttttgagaactctagcatagggtgtgaagcgtacaaccttggaaccggaagaggtacatctgtgctggag
attgttaaagcatttgagaaggcttctgggaagaaaatacctctgattttggtgaaagacgcccaggtgat
gcagagattctgttttcagagactactaaagcagagagggagcttaactggaaagcaaaatacggtattgaa
gagatgtgccgcgaccaatggaactgggccagcaagaacccttatggctatggatcacctgactctatcaag
cagaatggtcaccaaacaaacggatccgctgactcctccaagcagaatggccaccgcacaaacggttcaact
gactcacccaagcggaacggccaccatgcgtatgggtctgctgactcacccaagcgcaacgggcactgcgtt
tttggatcatcagacctcaagccgaatggtaatggccacctgcgctgagcagaactgtttggcctgtgagct
ccctgtacattcggttgcgatgtgagctccctgcacgttcggtcgaggtctatcgtgaacccactatccgag
attgatgtggatcattgggttgacaggtcatacagtatagagccggtggcagaggaattcctgtttgctgtg
ggtaaagcttatcttctgctttcgtgtttttcttgcttctttcgattatggtgtaggaatgtggtcataat
gtattagctgattatcctttccctgctaattggactttattacgcttcattaagtttggctgattactgttt
aacggggaaaagttgcacctgtgtacgattatttatttgtacctgtaatatttatggagaactagcctcgcc
gcaaatagccctgatgaattcagctgcagtttattcccaaaaaaaaaaaaaaaaaaaaaaaaaaaaaaaaaa
aaaaaaaaaaaaaaaaaaaaaaaaaaaggggcccc

**SEQ ID NO 318: Zea mays UGE X**

MAVEKTLPGASAGRTVLVTGGAGYIGSHAVLQLLLAGFRAVVIDNLNNSSELAVRRVAALAGDHSRNLSF
HKIDLRDKGALEMVFASTRFEAVIHFAGLKAVGESVQKPLLYYDNNVIGTINLLEVMSVHGCKKLVFSSS
AAVYGSPKNSPCTENFPLTPNNPYGKTKLVVEDICRDIYRSDPEWKIILLRYFNPVGAHPSGYLGEDPRG
IPNNLMPYVQQVAVGRRPALTVLGNDYATRDGTGVRDYIHVVDLADGHIAALQKLFENSSIGCEAYNLGT
GRGTSVLEIVKAFEKASGKKIPLIFGERRPGDAEILFSETTKAERELNWKAKYGIEEMCRDQWNWASKNP
YGYGSPDSIKQNGHQTNGSADSSKQNGHRTNGSTDSPKRNGHHAYGSADSPKRNGHCVFGSSDLKPNGNG
HLR

**SEQ ID NO 319: Glycine max UGE VI**

Acgtgtcacagaattctcgccttttccgaatatggcatcgcgcgtcagcattggcaatctcacttcctccgc
gccgtatattaattcccctcattttcgctcacaacttaagctttccagcaactcctctttgcacaagccact
catgcgcgacaagactgtgctggtaaccggcggagccggttacatcggcacccacaccgttcttcagctctt
gctcggaggttgcagaaccgtcgtcgtcgacaatctcgacaattcctccgaggtttctatccaccgagtcag
ggagcttgccggcgaatttgggaacaacctctcctttcacaaggtggacctacgggacagggatgcactaga
gcaaatttttgtttccacacaatttgatgctgtcatacactttgctggactgaaagcagtaggagaaagtgt
gcaaaaacctttactatactataacaacaacttgactgggacaatcactctattggaagtcatggctgccca
tggatgcaagaagctcgtgttctcttcttcagcaactgtatatggttggccaaaggaagttccatgcacaga
agagttccctctgtcagcaatgaacccatatggacgaactaagcttatcattgaagaaatttgccgtgatgt
ccactgtgcagagccagattgtaaaataattttgttaagatacttcaacccagttggtgcacaccccagcgg
ttatattggggaggatcctcgcggaattccaaacaatctcatgccatttgttcagcaagtagcagttggccg
acggcctgcactgacagtttttggaaatgattataatacaagtgatggcactggggttcgggattacattca

## Figure 23 (continued)

tgttgttgatttagcagatgggcacattgctgcattgcttaaactagatgaacctaatataggttgtgaggt
ttataacctgggaacaggaaagggaacatcagttttggagatggttagagcttttgaaatggcatctggaaa
gaaaattccacttgtgatggctggccgtagacctggtgatgctgaaattgtttatgcatcaacaaagaaagc
ggaaagagagcttaaatggaaggcaaaatatggcattgatgagatgtgccgtgatcaatggaattgggctag
caaaaacccttatggctatggagatcagggctccaccgattaaccacttagttttctctttgggttcttttc
tgaactcacccacaccgtagtccgtaggtcttgtgaatttagttttcccaaaagcttttctttctttagtga
tcttaaggtgacaaagtacttgtattattactattcatagttacatagtaagtaagtagtggtttactatac
tgtaatttaaaggttctctaggttccttcttacaggttattgattattagattcggattctctcatgttcca
catg

**SEQ ID NO 320: Glycene max UGE VI**
MASRVSIGNLTSSAPYINSPHFRSQLKLSSNSSLHKPLMRDKTVLVTGGAGYIGTHTVLQLLLGGCRTVV
VDNLDNSSEVSIHRVRELAGEFGNNLSFHKVDLRDRDALEQIFVSTQFDAVIHFAGLKAVGESVQKPLLY
YNNNLTGTITLLEVMAAHGCKKLVFSSSATVYGWPKEVPCTEEFPLSAMNPYGRTKLIIEEICRDVHCAE
PDCKIILLRYFNPVGAHPSGYIGEDPRGIPNNLMPFVQQVAVGRRPALTVFGNDYNTSDGTGVRDYIHVV
DLADGHIAALLKLDEPNIGCEVYNLGTGKGTSVLEMVRAFEMASGKKIPLVMAGRRPGDAEIVYASTKKA
ERELKWKAKYGIDEMCRDQWNWASKNPYGYGDQGSTD

## Figure 23 (continued)

Europäisches Patentamt
European Patent Office
Office européen des brevets

# EUROPEAN SEARCH REPORT

Application Number
EP 12 16 8728

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2006/034286 A2 (PIONEER HI BRED INT [US]; BRUGIERE NORBERT [US]) 30 March 2006 (2006-03-30) * the whole document * ----- | 1-21 | INV. C12N15/82 A01H5/00 |
| X | MCCABE MATTHEW S ET AL: "Effects of PSAG12-IPT gene expression on development and senescence in transgenic lettuce", PLANT PHYSIOLOGY, AMERICAN SOCIETY OF PLANT PHYSIOLOGISTS, ROCKVILLE, MD, US, vol. 127, no. 2, 1 October 2001 (2001-10-01), pages 505-516, XP002521725, ISSN: 0032-0889, DOI: 10.1104/PP.010244 * the whole document * ----- | 21,22 | |
| A | WO 2005/049646 A2 (CROPDESIGN NV [BE]; FRANKARD VALERIE [BE]; MIRONOV VLADIMIR [BE]) 2 June 2005 (2005-06-02) ----- | 1-22 | |
| A | WO 2004/074442 A2 (MONSANTO TECHNOLOGY LLC [US]; BHAT DEEPTI G [US]; DENG MOLIAN [US]; EI) 2 September 2004 (2004-09-02) ----- | 1-22 | TECHNICAL FIELDS SEARCHED (IPC) C12N |
| A | EP 1 384 776 A1 (SUNTORY LTD [JP]; JUJO PAPER CO LTD [JP] SUNTORY HOLDINGS LTD [JP]) 28 January 2004 (2004-01-28) ----- | 1-22 | |
| A | MARTINEAU B ET AL: "PRODUCTION OF HIGH SOLIDS TOMATOES THROUGH MOLECULAR MODIFICATION OF LEVELS OF THE PLANT GROWTH REGULATOR CYTOKININ", BIO/TECHNOLOGY, NATURE PUBLISHING CO. NEW YORK, US, vol. 13, 1 March 1995 (1995-03-01), pages 250-254, XP002910461, ISSN: 0733-222X, DOI: 10.1038/NBT0395-250 ----- -/-- | 1-22 | |

The present search report has been drawn up for all claims

1

| Place of search | Date of completion of the search | Examiner |
|---|---|---|
| Munich | 20 August 2012 | Puonti-Kaerlas, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 12 16 8728

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | ROECKEL P ET AL: "EFFECTS OF SEED-SPECIFIC EXPRESSION OF A CYTOKININ BIOSYNTHETIC GENE ON CANOLA AND TOBACCO PHENOTYPES", TRANSGENIC RESEARCH, LONDON, GB, vol. 6, no. 2, 1 March 1997 (1997-03-01), pages 133-141, XP000938801, ISSN: 0962-8819, DOI: 10.1023/A:1018425720949 ----- | 1-22 | |
| | | | TECHNICAL FIELDS SEARCHED (IPC) |

The present search report has been drawn up for all claims

1

| Place of search | Date of completion of the search | Examiner |
|---|---|---|
| Munich | 20 August 2012 | Puonti-Kaerlas, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 12 16 8728

This annex lists the patent family members relating to the patent documents cited in the above-mentioned European search report.
The members are as contained in the European Patent Office EDP file on
The European Patent Office is in no way liable for these particulars which are merely given for the purpose of information.

20-08-2012

| Patent document cited in search report | Publication date | Patent family member(s) | Publication date |
|---|---|---|---|
| WO 2006034286 A2 | 30-03-2006 | AR 051633 A1 | 31-01-2007 |
| | | AU 2005286853 A1 | 30-03-2006 |
| | | BR PI0515424 A | 22-07-2008 |
| | | CA 2580761 A1 | 30-03-2006 |
| | | EP 1799832 A2 | 27-06-2007 |
| | | NZ 553864 A | 27-11-2009 |
| | | US 2006064786 A1 | 23-03-2006 |
| | | US 2009165177 A1 | 25-06-2009 |
| | | WO 2006034286 A2 | 30-03-2006 |
| WO 2005049646 A2 | 02-06-2005 | AU 2004291348 A1 | 02-06-2005 |
| | | BR PI0416676 A | 13-02-2007 |
| | | CA 2546674 A1 | 02-06-2005 |
| | | US 2009235393 A1 | 17-09-2009 |
| | | WO 2005049646 A2 | 02-06-2005 |
| WO 2004074442 A2 | 02-09-2004 | AR 043185 A1 | 20-07-2005 |
| | | US 2006212970 A1 | 21-09-2006 |
| | | WO 2004074442 A2 | 02-09-2004 |
| EP 1384776 A1 | 28-01-2004 | AT 441708 T | 15-09-2009 |
| | | AU 2002237573 B2 | 28-02-2008 |
| | | CA 2441791 A1 | 19-09-2002 |
| | | CN 1496401 A | 12-05-2004 |
| | | EP 1384776 A1 | 28-01-2004 |
| | | JP 4334228 B2 | 30-09-2009 |
| | | NZ 528152 A | 30-06-2008 |
| | | US 2004177403 A1 | 09-09-2004 |
| | | US 2006236431 A1 | 19-10-2006 |
| | | WO 02072818 A1 | 19-09-2002 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5859337 A
- US 6992236 B
- US 5811238 A
- US 6395547 A
- WO 2004070039 A
- WO 2004065596 A
- US 4962028 A
- WO 0114572 A
- WO 9514098 A
- WO 9412015 A
- EP 99106056 A
- US 5565350 A, Kmiec
- WO 9322443 A, Zarling
- WO 1998 A, Grierson
- WO 9853083 A
- WO 1999 A, Waterhouse
- WO 9953050 A
- US 4987071 A, Cech
- US 5116742 A, Cech
- WO 1994 A, Atkins
- WO 9400012 A
- WO 1995 A, Lenne
- WO 9503404 A
- WO 2000 A, Lutziger
- WO 0000619 A
- WO 1997 A, Prinsen
- WO 9713865 A
- WO 9738116 A
- WO 9836083 A
- WO 9915682 A, Baulcombe
- WO 0015815 A
- EP 1198985 A1
- US 5164310 A
- US 5159135 A
- EP 2007001422 W


### Non-patent literature cited in the description


- **WANG et al.** *Planta,* 2003, vol. 218, 1-14
- **TOLEDO-ORTIZ et al.** *Plant Cell,* 2003, vol. 15, 1749-1770
- **BAILEY et al.** *Plant Cell,* 2003, vol. 15, 2497-2501
- **LI et al.** *Plant Physiol.,* 2006, vol. 141, 1167-1184
- **ABE et al.** *Plant Cell,* 1997, vol. 9, 1859-1868
- **ABE et al.** *Plant Cell,* vol. 15, 63-78
- **DOMBRECHT et al.** *Plant Cell,* 2007, vol. 19, 2225-2245
- **NAGAOKA ; TAKANO.** *J. Exp. Bot.,* 2003, vol. 54, 391-396
- **GRIFFITHS et al.** *Plant Phys,* 2003, vol. 131, 1855-1867
- **LAGERCRANTS et al.** *Mol.Biol.Evol.,* 2000, vol. 17, 1499-1507
- **INDORF et al.** *Plant J.,* 2007, vol. 51 (4), 563-74
- **THODEN et al.** *J. Biol. Chem,* 2005, vol. 280, 21900-21907
- **DORMANN ; BENNING.** *Plant J.,* 1998, vol. 13, 641-652
- **BARBER et al.** *JBC,* 2006, vol. 281, 17276-17285
- **ROSTI et al.** *The Plant Cell,* 2007, vol. 19, 1565-1579
- **CREIGHTON.** Proteins. W.H. Freeman and Company, 1984
- **TERPE.** *Appl. Microbiol. Biotechnol.,* 2003, vol. 60, 523-533
- **MEINKOTH ; WAHL.** *Anal. Biochem.,* 1984, vol. 138, 267-284
- **SAMBROOK et al.** Molecular Cloning: a laboratory manual. Cold Spring Harbor Laboratory Press, 2001
- Current Protocols in Molecular Biology. John Wiley & Sons, 1989
- **FOISSAC ; SCHIEX.** *BMC Bioinformatics,* 2005, vol. 6, 25
- **CASTLE et al.** *Science,* 2004, vol. 304 (5674), 1151-4
- **HEID et al.** *Genome Methods,* 1996, vol. 6, 986-994
- **MCELROY et al.** *Plant Cell,* 1990, vol. 2, 163-171
- **ODELL et al.** *Nature,* 1985, vol. 313, 810-812
- **NILSSON et al.** *Physiol. Plant.,* 1997, vol. 100, 456-462
- **DE PATER et al.** *Plant J,* 1992, vol. 2 (6), 837-44

- **CHRISTENSEN et al.** *Plant Mol. Biol.,* 1992, vol. 18, 675-689
- **BUCHHOLZ et al.** *Plant Mol Biol.,* 1994, vol. 25 (5), 837-43
- **LEPETIT et al.** *Mol. Gen. Genet.,* 1992, vol. 231, 276-285
- **WU et al.** *Plant Mol. Biol.,* 1988, vol. 11, 641-649
- **AN et al.** *Plant J.,* 1996, vol. 10 (1), 107-121
- **SANGER et al.** *Plant. Mol. Biol.,* 1990, vol. 14, 433-443
- **LEISNER.** *Proc Natl Acad Sci USA,* 1988, vol. 85 (5), 2553
- **JAIN et al.** *Crop Science,* 1999, vol. 39 (6), 1696
- **SHAW et al.** *Nucleic Acids Res.,* 1984, vol. 12 (20), 7831-7846
- **GATZ.** *Annu. Rev. Plant Physiol. Plant Mol. Biol.,* 1997, vol. 48, 89-108
- **QING QU ; TAKAIWA.** *Plant Biotechnol. J.,* 2004, vol. 2, 113-125
- **SIMON et al.** *Plant Mol. Biol.,* 1985, vol. 5, 191
- **SCOFIELD et al.** *J. Biol. Chem.,* 1987, vol. 262, 12202
- **BASZCZYNSKI et al.** *Plant Mol. Biol.,* 1990, vol. 14, 633
- **PEARSON et al.** *Plant Mol. Biol.,* 1992, vol. 1'8, 235-245
- **ELLIS et al.** *Plant Mol. Biol.,* 1988, vol. 10, 203-214
- **TAKAIWA et al.** *Mol. Gen. Genet.,* 1986, vol. 208, 15-22
- **TAKAIWA et al.** *FEBS Letts.,* 1987, vol. 221, 43-47
- **MATZKE et al.** *Plant Mol Biol,* 1990, vol. 14 (3), 323-32
- **STALBERG et al.** *Planta,* 1996, vol. 199, 515-519
- *Mol Gen Genet,* 1989, vol. 216, 81-90
- *NAR,* 1989, vol. 17, 461-2
- **ALBANI et al.** *Plant Cell,* 1997, vol. 9, 171-184
- *EMBO J.,* 1984, vol. 3, 1409-15
- **DIAZ et al.** *Mol Gen Genet,* 1995, vol. 248 (5), 592-8
- *Theor Appl Gen,* 1999, vol. 98, 1253-62
- *Plant J,* 1993, vol. 4, 343-55
- *Mol Gen Genet,* 1996, vol. 250, 750-60
- **MENA et al.** *The Plant Journal,* 1998, vol. 116 (1), 53-62
- **VICENTE-CARBAJOSA et al.** *Plant J.,* 1998, vol. 13, 629-640
- **WU et al.** *Plant Cell Physiology,* 1998, vol. 39 (8), 885-889
- **SATO et al.** *Proc. Natl. Acad. Sci. USA,* 1996, vol. 93, 8117-8122
- **NAKASE et al.** *Plant Mol. Biol.,* 1997, vol. 33, 513-522
- *Trans Res,* 1997, vol. 6, 157-68
- *Plant J,* 1997, vol. 12, 235-46
- **DEROSE et al.** *Plant Mol. Biol,* 1996, vol. 32, 1029-35
- **POSTMA-HAARSMA et al.** *Plant Mol. Biol.,* 1999, vol. 39, 257-71
- **WU et al.** *J. Biochem.,* 1998, vol. 123, 386
- **CUMMINS et al.** *Plant Mol. Biol.,* 1992, vol. 19, 873-876
- **LANAHAN et al.** *Plant Cell,* 1992, vol. 4, 203-211
- **SKRIVER et al.** *Proc Natl Acad Sci USA,* 1991, vol. 88, 7266-7270
- **CEJUDO et al.** *Plant Mol Biol,* 1992, vol. 20, 849-856
- **KALLA et al.** *Plant J.,* 1994, vol. 6, 849-60
- **LEAH et al.** *Plant J.,* 1994, vol. 4, 579-89
- **SELINGER et al.** *Genetics,* 1998, vol. 149, 1125-38
- **NOMURA et al.** *Plant Mol Biol.,* 2000, vol. 44 (1), 99-106
- **BUCHMAN ; BERG.** *Mol. Cell biol.,* 1988, vol. 8, 4395-4405
- **CALLIS et al.** *Genes Dev,* 1987, vol. 1, 1183-1200
- The Maize Handbook. Springer, 1994
- **GAULTIER et al.** *Nucl Ac Res,* 1987, vol. 15, 6625-6641
- **INOUE et al.** *Nucl Ac Res,* 1987, vol. 15, 6131-6148
- **INOUE et al.** *FEBS Lett.,* 1987, vol. 215, 327-330
- **HASELHOFF ; GERLACH.** *Nature,* 1988, vol. 334, 585-591
- **BARTEL ; SZOSTAK.** *Science,* 1993, vol. 261, 1411-1418
- **ANGELL ; BAULCOMBE.** *Plant J,* 1999, vol. 20 (3), 357-62
- **HELENE, C.** *Anticancer Drug Res.,* 1991, vol. 6, 569-84
- **HELENE et al.** *Ann. N.Y. Acad. Sci.,* 1992, vol. 660, 27-36
- **MAHER, L.J.** *Bioassays,* 1992, vol. 14, 807-15
- **SCHWAB et al.** *Dev. Cell,* 2005, vol. 8, 517-527
- **SCHWAB et al.** *Plant Cell,* 2006, vol. 18, 1121-1133
- **TRIBBLE et al.** *J. Biol. Chem.,* 2000, vol. 275, 22255-22267
- **VELMURUGAN et al.** *J. Cell Biol.,* 2000, vol. 149, 553-566
- **KRENS, F.A. et al.** *Nature,* 1982, vol. 296, 72-74
- **NEGRUTIU I et al.** *Plant Mol Biol,* 1987, vol. 8, 363-373
- **SHILLITO R.D. et al.** *Bio/Technol,* 1985, vol. 3, 1099-1102

- **CROSSWAY A et al.** *Mol. Gen Genet,* 1986, vol. 202, 179-185
- **KLEIN TM et al.** *Nature,* 1987, vol. 327, 70
- **CLOUGH ; BENT.** *Plant J.,* 1998, vol. 16, 735-743
- **ALDEMITA ; HODGES.** *Planta,* 1996, vol. 199, 612-617
- **CHAN et al.** *Plant Mol Biol,* 1993, vol. 22 (3), 491-506
- **HIEI et al.** *Plant J,* 1994, vol. 6 (2), 271-282
- **ISHIDA et al.** *Nat. Biotechnol,* 1996, vol. 14 (6), 745-50
- **FRAME et al.** *Plant Physiol,* 2002, vol. 129 (1), 13-22
- Techniques for Gene Transfer. **B. JENES et al.** Transgenic Plants, Vol. 1, Engineering and Utilization. Academic Press, 1993, vol. 1, 128-143
- **POTRYKUS.** *Annu. Rev. Plant Physiol. Plant Molec. Biol.,* 1991, vol. 42, 205-225
- **BEVAN et al.** *Nucl. Acids Res.,* 1984, vol. 12, 8711
- **HÖFGEN ; WILLMITZER.** *Nucl. Acid Res.,* 1998, vol. 16, 9877
- Vectors for Gene Transfer in Higher Plants. **F.F. WHITE.** Transgenic Plants, Vol. 1, Engineering and Utilization. Academic Press, 1993, vol. 1, 15-38
- **FELDMAN, KA ; MARKS MD.** *Mol Gen Genet,* 1987, vol. 208, 274-289
- **FELDMANN K.** *Methods in Arabidopsis Research. Word Scientific,* 1992, 274-289
- **CHANG.** *Plant J.,* 1994, vol. 5, 551-558
- **KATAVIC.** *Mol Gen Genet,* 1994, vol. 245, 363-370
- **BECHTHOLD, N.** *C R Acad Sci Paris Life Sci,* 1993, vol. 316, 1194-1199
- **CLOUGH, SJ ; BENT AF.** *The Plant J.,* 1998, vol. 16, 735-743
- **KLAUS et al.** *Nature Biotechnology,* 2004, vol. 22 (2), 225-229
- **BOCK.** Transgenic plastids in basic research and plant biotechnology. *J Mol Biol.,* 21 September 2001, vol. 312 (3), 425-38
- **MALIGA, P.** Progress towards commercialization of plastid transformation technology. *Trends Biotechnol.,* 2003, vol. 21, 20-28
- **HAYASHI et al.** *Science,* 1992, 1350-1353
- **REDEI GP ; KONCZ C.** Methods in Arabidopsis Research. World Scientific Publishing Co, 1992, 16-82
- **FELDMANN et al.** Arabidopsis. Cold Spring Harbor Laboratory Press, 1994, 137-172
- **LIGHTNER J ; CASPAR T.** Methods on Molecular Biology. Humana Press, 1998, vol. 82, 91-104
- **MCCALLUM et al.** *Nat Biotechnol,* 2000, vol. 18, 455-457
- **STEMPLE.** *Nat Rev Genet,* 2004, vol. 5 (2), 145-50
- **OFFRINGA et al.** *EMBO J,* 1990, vol. 9 (10), 3077-84
- **TERADA et al.** *Nat Biotech,* 2002, vol. 20 (10), 1030-4
- **LIDA ; TERADA.** *Curr Opin Biotech,* 2004, vol. 15 (2), 132-8
- **MILLER et al.** *Nature Biotechnol.,* 2007, vol. 25, 778-785
- **SCHULTZ et al.** *Proc. Natl. Acad. Sci. USA,* 1998, vol. 95, 5857-5864
- **LETUNIC et al.** *Nucleic Acids Res,* 2002, vol. 30, 242-244
- **MULDER et al.** *Nucl. Acids. Res.,* 2003, vol. 31, 315-318
- **BUCHER ; BAIROCH.** A generalized profile syntax for biomolecular sequences motifs and its function in automatic sequence interpretation. *Proceedings 2nd International Conference on Intelligent Systems for Molecular Biology,* 1994, 53-61
- **HULO et al.** *Nucl. Acids. Res.,* 2004, vol. 32, D134-D137
- **BATEMAN et al.** *Nucleic Acids Research,* 2002, vol. 30 (1), 276-280
- **GASTEIGER et al.** ExPASy: the proteomics server for in-depth protein knowledge and analysis. *Nucleic Acids Res.,* 2003, vol. 31, 3784-3788
- **NEEDLEMAN ; WUNSCH.** *J Mol Biol,* 1970, vol. 48, 443-453
- **ALTSCHUL et al.** *J Mol Biol,* 1990, vol. 215, 403-10
- **CAMPANELLA et al.** MatGAT: an application that generates similarity/identity matrices using protein or DNA sequences. *BMC Bioinformatics,* 10 July 2003, vol. 4, 29
- **SMITH TF ; WATERMAN MS.** *J. Mol. Biol,* 1981, vol. 147 (1), 195-7
- **PATTANAIK et al.** *BBA1759,* 2006, 308-318
- Current Protocols in Molecular Biology
- **RABBANI et al.** *Plant Physiol,* 2003, vol. 133, 1755-1767
- **SAMBROOK J ; FRITSCH EF ; MANIATIS T.** Molecular Cloning, A Laboratory Manual. 1989
- **LANDER et al.** *Genomics,* 1987, vol. 1, 174-181
- **BOTSTEIN et al.** *Am. J. Hum. Genet.,* 1980, vol. 32, 314-331
- **BERNATZKY ; TANKSLEY.** *Plant Mol. Biol. Reporter,* 1986, vol. 4, 37-41
- **HOHEISEL et al.** Non-mammalian Genomic Analysis: A Practical Guide. Academic press, 1996, 319-346

- **TRASK.** *Trends Genet.,* 1991, vol. 7, 149-154
- **LAAN et al.** *Genome Res.,* 1995, vol. 5, 13-20
- **KAZAZIAN.** *J. Lab. Clin. Med,* 1989, vol. 11, 95-96
- **SHEFFIELD et al.** *Genomics,* 1993, vol. 16, 325-332
- **LANDEGREN et al.** *Science,* 1988, vol. 241, 1077-1080
- **SOKOLOV.** *Nucleic Acid Res.,* 1990, vol. 18, 3671
- **WALTER et al.** *Nat. Genet.,* 1997, vol. 7, 22-28
- **DEAR ; COOK.** *Nucleic Acid Res.,* 1989, vol. 17, 6795-6807
- A generalized profile syntax for biomolecular sequences motifs and its function in automatic sequence interpretation. **BUCHER ; BAIROCH.** ISMB-94; Proceedings 2nd International Conference on Intelligent Systems for Molecular Biology. AAAI Press, 1994, 53-61
- **CAMPANELLA et al.** *BMC Bioinformatics,* 10 July 2003, vol. 4, 29
- **HAGER et al.** *J Bacteriol,* vol. 186 (19), 6634-6642
- Current Protocols in Molecular Biology. Wiley
- **SOKOLOV.** *nucleic acid sequence Res.,* 1990, vol. 18, 3671
- **DEAR ; COOK.** *nucleic acid sequence Res.,* 1989, vol. 17, 6795-6807
- **AUSUBEL et al.** Current Protocols. *Current Protocols in Molecular Biology,* 1994, vol. 1, 2
- **KORFHAGE et al.** *Plant C,* 1994, vol. 6, 695-708
- **SCHINDLER et al.** *Plant J,* 1993, vol. 4 (1), 137-150
- **VIOLA ; GONZALEZ.** *Biochemistry,* 26 May 2007
- **SOKOLOV.** *Nucleic Acids Res.,* 1990, vol. 18, 3671
- **DEAR ; COOK.** *Nucleic Acids Res.,* 1989, vol. 17, 6795-6807
- **MIYAWAKI et al.** *Proc Natl Acad Sci USA.,* 2006, vol. 103 (44), 16598-16603
- **WANG et al.** *Planta,* vol. 218, 1-14
- **BORDEN et al.** *EMBO J.,* 01 December 1995, vol. 14 (23), 5947-56
- **REYMOND et al.** *EMBO J.,* 01 May 2001, vol. 20 (9), 2140-51
- A generalized profile syntax for biomolecular sequences motifs and its function in automatic sequence interpretation. **BUCHER ; BAIROCH.** Proceedings 2nd International Conference on Intelligent Systems for Molecular Biology. AAAI Press, 1994, 53-61
- **HULO et al.** *Nucl. Acids. Res.,* vol. 32, D134-D137
- **MAXWELL ; ROBICHON-SZULMAJSTER.** *J. Biol. Chem.,* 1960, vol. 235, 308-312
- **WILSON, D.B. ; HOGNESS, D.S.** *J. Biol. Chem.,* 1964, vol. 239, 2469-2481
- **FREY.** *FASEB J.,* March 1996, vol. 10 (4), 461-70
- **THODEN et al.** *Biochemistry,* 1997, vol. 36, 6294-6304
- **SHAW et al.** *Mol. Biochem. Parasitol.,* 2003, vol. 126, 173-180
- **FINN et al.** *Nucleic Acids Research,* 2006, D247-D251
- **DORMANN ; BENNING.** *Arch Biochem Biophys.,* 1996, vol. 327 (1), 27-34
- **SAMBROOK.** Molecular Cloning: a laboratory manual. Cold Spring Harbor Laboratory Press, 2001
- Current Protocols in Molecular Biology, Current Protocols. 1994, vol. 1, 2
- **R.D.D. CROY.** Plant Molecular Biology Labfax. BIOS Scientific Publications Ltd, 1993
- **ALTSCHUL et al.** *J. Mol. Biol.,* 1990, vol. 215, 403-410
- **ALTSCHUL et al.** *Nucleic Acids Res.,* 1997, vol. 25, 3389-3402
- **THOMPSON et al.** *Nucleic Acids Res,* 1997, vol. 25, 4876-4882
- **CHENNA et al.** *Nucleic Acids Res,* 2003, vol. 31, 3497-3500
- *BMC Bioinformatics,* 2003, vol. 4, 29
- **XUE.** *Plant J.,* 2005, vol. 41, 638-649
- **ISHIDA et al.** *Nature Biotech,* 1996, vol. 14 (6), 745-50
- **BABIC et al.** *Plant Cell Rep,* 1998, vol. 17, 183-188
- **MCKERSIE et al.** *Plant Physiol,* 1999, vol. 119, 839-847
- **BROWN DCW ; A ATANASSOV.** *Plant Cell Tissue Organ Culture,* 1985, vol. 4, 111-112
- **WALKER et al.** *Am J Bot,* 1978, vol. 65, 654-659
- **GAMBORG et al.** *Exp. Cell Res.,* 1968, vol. 50, 151-158